# EUROPEAN PATENT APPLICATION

(11) **EP 4 628 486 A1**
(43) Date of publication of application: **08.10.2025**
(21) Application number: 23896929.9
(22) Date of filing: 01.12.2023
(51) Int. Cl.: C07D 401/12, C07D 401/14, C07D 413/14, C07D 413/12, A61K 31/506, A61K 31/4545, A61K 31/517, A61P 35/00

(54) **AMIDE OR UREA COMPOUND**

(30) Priority: 02.12.2022 CN 202211538155; 09.05.2023 CN 202310522117; 23.08.2023 CN 202311071943; 24.10.2023 CN 202311385406
(71) Applicant: Shenzhen Zhongge Biological Technology Co., Ltd., Futian District Shenzhen, Guangdong 518017 (CN)
(72) Inventor: CHEN, Jie, Shenzhen, Guangdong 518017 (CN); LIU, Zhiqiang, Shenzhen, Guangdong 518017 (CN); WEI, Lin, Shenzhen, Guangdong 518017 (CN); ZHU, Xinying, Shenzhen, Guangdong 518017 (CN); WU, Yuhang, Shenzhen, Guangdong 518017 (CN); XIE, Dewei, Shenzhen, Guangdong 518017 (CN); HAN, Yu, Shenzhen, Guangdong 518017 (CN); PAN, Wei, Shenzhen, Guangdong 518017 (CN); WU, Qiang, Shenzhen, Guangdong 518017 (CN); CHEN, Bin, Shenzhen, Guangdong 518017 (CN); WANG, Shaohui, Shenzhen, Guangdong 518017 (CN); ZHANG, Peiyu, Shenzhen, Guangdong 518017 (CN)
(74) Representative: Vossius & Partner Patentanwälte Rechtsanwälte mbB
(86) International application number: PCT/CN2023/135823
(87) International publication number: WO 2024/114787

(57) **Abstract**

Provided in the present application is a compound as represented by formula (I). The compound can regulate the KIF18A protein, thereby affecting the cell cycle and the cell proliferation process to treat cancer and cancer-associated diseases. The present disclosure further comprises a pharmaceutical composition containing the compound, and a method for treating conditions associated with the KIF18A activity.

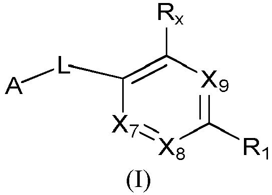

## Description

The related application document is entitled to the priority for Chinese disclosure patent application 202211538155.1, filed on December 2, 2022, Chinese disclosure patent application 202310522117.5, filed on May 09, 2023, Chinese disclosure patent application 202311071943.9, filed on August 23, 2023 and Chinese disclosure patent application 202311385406.1, filed on October 24, 2023, the entire contents of which are incorporated herein by reference in their entireties.

### Technical Field

The present application belongs to the field of pharmaceutical chemistry and specifically relates to an amide or urea compound.

### Background Art

CIN, defined as chromosomal instability, is caused by a chromosomal segregation error during mitosis. CIN is an important feature of recurrent, metastatic advanced malignancies, occurring in about 90% of solid tumors and affecting about 25% of the solid tumor cell genome. CIN can be caused by a variety of causes, including mitotic errors, replication stress, homologous recombination repair, and breakage-fusion-bridge cycles. CIN is closely associated with cell transformation, tumor progression and relapse, chemotherapy resistance, and poor prognosis.

Kinesins are molecular motors that play an important role in cell division and intracellular vesicle and organelle transport. Mitotic kinesins play a role in many aspects of spindle assembly, chromosome separation, centrosome separation, and kinetics. Based on sequence homology within the "motor domain", human kinesins are classified into 14 subfamilies. KIF18A belongs to the kinesin-8 family and is a protein which is specifically expressed in mitotic G2/M phase and that plays a key role in maintaining bipolar spindle integrity during cell division, thereby regulating chromosomal localization. KIF18A is able to move in the positive direction along the microtubule orbit by utilizing the energy released by ATP hydrolysis in a cell. Positioning at the positive end of the microtubule also regulates the dynamic instability of the microtubule and plays an activity similar to that of microtubule depolymerase. During mitosis, KIF18A is able to regulate spindle microtubule kinetics and chromosome amplitudes, which are critical to the timely completion of entire columns of mitotic chromosomes, the maintenance of genome stability, and the successful completion of mitosis.

Studies have shown that kinesin KIF18A is an important factor in the proliferation of CIN tumor cells, and after KIF18A knockout, tumor cells with the CIN feature exhibit mitotic vulnerability associated with spindle assembly checkpoint (SAC) activation, multipolar spindle formation and induction of apoptosis. This in turn leads to mitotic arrest, cell cycle arrest, and apoptosis. KIF18A is a non-essential gene for normal somatic cell division, but CIN tumor cells are highly sensitive to KIF18A knockout. Thus, small molecule inhibitors of KIF18A can selectively target tumor cells with the CIN feature, KIF18A inhibition does not affect normal cell proliferation compared with other drugs targeting mitotic mechanisms, and there is currently no effective therapy for targeting CIN. KIF18A is a new anti-tumor target with great potential.

### Summary of the Invention

It is an objective of the present disclosure to provide amide or urea compounds which have KIF18A inhibitory activity. To this end, the present disclosure further provides the use of these compounds and pharmaceutically acceptable salts thereof in the preparation and manufacture of a pharmaceutical composition or medicament for the therapeutic, prophylactic, acute or chronic treatment of KIF18A-mediated diseases and disorders, including but not limited to cancer. Thus, the compounds of the present disclosure can be used in the manufacture of anticancer drugs. The present disclosure further provides a method for the preparation of a compound of formula I, as well as intermediates useful in such methods.

In a first aspect, the present application provides a compound represented by formula (I), or a stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof, wherein 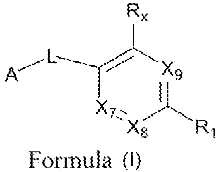
A is monocyclic aryl, monocyclic heteroaryl, bicyclic aryl, bicyclic heteroaryl or fused heterocyclyl, and optionally, the monocyclic aryl, monocyclic heteroaryl, bicyclic aryl, bicyclic heteroaryl or fused heterocyclyl is optionally substituted with one or more R_{z};
L is selected from -NR³-CO- or -NR³CONR³-, and each R³ is independently H, deuterium, C1-C6 alkylene or C1-C6 haloalkylene;
Rₓ is selected from -OR₄ or -NR₅R₆, wherein R₄ is selected from 3- to 8-membered cycloalkyl, 3- to 8-membered cycloalkenyl or 3- to 8-membered heterocyclyl, and the 3- to 8-membered heterocyclyl comprises at least one heteroatom selected from O, S and N; R₅ is selected from 3- to 8-membered cycloalkyl, 3- to 8-membered cycloalkenyl or 3- to 8-membered heterocyclyl, R₆ is selected from H or C1-C6 alkyl, or R₅ and R₆, together with the N atom to which they are attached, form 4- to 8-membered heterocyclyl; the 3- to 8-membered cycloalkyl, 3- to 8-membered cycloalkenyl, 3- to 8-membered heterocyclyl or 4- to 8-membered heterocyclyl comprises 0, 1, 2 or 3 heteroatoms selected from O, S and N, and is optionally substituted with one or more R_{z};
R₁ is -CN or -Z-R₁₂, wherein Z is a direct bond, -C1-C6 alkylene-, -C1-C6 alkylene-O-, -O-, -S-, -S(=O)-, -SO₂-, -NR₁₁-, -NR₁₁SO₂-, -SO₂NR₁₁-, -NR₁₁-S(=O)(=NH)-, -S(=O)(=NH)-, -C1-C6 alkylene-SO₂-, -C1-C6 alkylene-SO₂R₁₁-, - (C=O)-, -(C=O)NR₁₁-, -C=N(OH)- or -NR₁₁(C=O)-; or -Z-R₁₂ is -N=S(=O)-(R₁₂)₂, wherein a pair of two R₁₂, together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
X₇ is N or CR₇; X₈ is N or CR₈; X₉ is N or CR₉;
R₇, R₈ and R₉ are independently H, halogen, C1-C8 alkyl, C1-C6 haloalkyl, - OH, -O-R₈ₐ, -O-R_{8b} or -NRₐRₐ; where both R₇ and R₈ are present, R₇ and R₈, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S; where both Rₓ and R₉ are present, Rₓ and R₉, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
R₁₁ is H, R₁₁ₐ or R_{11b}; R₁₂ is H, R₁₂ₐ or R_{12b};
R₈ₐ, R₁₁ₐ and R₁₂ₐ are independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, - S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, - N(Rₐ)C(=NRₐ)NRₐRₐ, -N(Rₐ)S(=O)₂R_{b}, -N(Rₐ)S(=O)₂NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, - C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, R₁₄ or oxo;
R_{8b}, R_{11b} and R_{12b} are independently selected from the group consisting of: C1-C6 alkyl substituted with 0, 1, 2, 3, 4 or 5 groups selected from F, Cl, Br, -ORₐ, - OC1-C6 haloalkyl or CN;
R₁₄, in each case, is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0 or 1 atom selected from O and S, wherein the monocyclic ring or bicyclic ring is substituted with 0, 1, 2 or 3 groups selected from: F, Cl, Br, C1-C6 alkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-6 alkylene NRₐRₐ, -OC2-6 alkylene ORₐ, -SRₐ, -S(=O)R_{b}, -S(=O)₂R_{b}, -S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, -N(Rₐ)C(=NRₐ)NRₐRₐ, -N(Rₐ)S(=O)₂R_{b}, - N(Rₐ)S(=O)₂NRₐRₐ, -NRₐC2-C6 alkylene NR^{a}R^{a}, -NR^{a}C2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ or oxo;
each Rₐ is independently H or R_{b};
each R_{b} is independently C1-C6 alkyl, phenyl or benzyl, wherein C1-C6 alkyl is substituted with 0, 1, 2, or 3 substituents selected from: halogen, -OH, -O-C1-C6 alkyl, -NH₂, -NH-C1-C6 alkyl, -OC(=O)C1-C6 alkyl or -N(C1-C6 alkyl)(C1-C6 alkyl); and phenyl or benzyl is substituted with 0, 1, 2, or 3 substituents selected from: halogen, C1-C6 alkyl, C1-C6 haloalkyl, -OH, -O-C1-C6 alkyl, -NH₂, -NH-C1-C6 alkyl, -OC(=O)C1-C6 alkyl or -N(C1-C6 alkyl)C1-C6 alkyl;
each R_{z} is independently selected from the following groups: deuterium, halogen, C1-C6 alkyl, C3-C8 cycloalkyl, -C1-C6 alkylene-O-C1-C6 alkyl, C3-C8 cycloalkyl-C1-C6 alkylene, -OH, -CN, -O-C1-C6 alkyl, -O-C3-C8 cycloalkyl, -NH₂, -NH-C1-C6 alkyl, -OC(=O)C1-C6 alkyl, -N(C1-C6 alkyl)(C1-C6 alkyl), -S(O)-R_{z1}, -S(O)₂-R_{z1}, -C(O)-R_{z2}, -C(O)-NR_{z1}R_{z2}, phenyl, 5- to 6-membered monocyclic heteroaryl comprising 0, 1, 2, or 3 N atoms, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two R_{z} attached to the same carbon atom may form 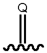 or two R_{z} attached to the same carbon atom, together with the carbon atom to which they are attached, may form C3-C8 cycloalkyl; the C1-C6 alkyl, C3-C8 cycloalkyl, -C1-C6 alkylene-O-C1-C6 alkyl, -O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl;
each R_{z1} is independently selected from the following groups: H, C1-C6 alkyl, or C1-C6 haloalkyl;
each R_{z2} is independently selected from the following groups: H, C1-C6 alkyl, C1-C6 haloalkyl, C1-C6 deuteroalkyl, or C3-C8 cycloalkyl;
Q is O or CR_{Q1}R_{Q2};
R_{Q1} and R_{Q2} are independently selected from the following groups: H, deuterium, halogen, C1-C6 alkyl, or C1-C6 haloalkyl.

In some embodiments, the present application provides the compound represented by formula (I), or the stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof, wherein
A is monocyclic aryl, monocyclic heteroaryl, bicyclic aryl, bicyclic heteroaryl or fused heterocyclyl, and optionally, the monocyclic aryl, monocyclic heteroaryl, bicyclic aryl, bicyclic heteroaryl or fused heterocyclyl is optionally substituted with one or more R_{z};
L is selected from -NR³-CO- or -NR³CONR³-, and each R³ is independently H, deuterium, C1-C6 alkylene or C1-C6 haloalkylene;
Rₓ is selected from -OR₄ or -NR₅R₆, wherein R₄ is selected from 3- to 8-membered cycloalkyl, 3- to 8-membered cycloalkenyl or 3- to 8-membered heterocyclyl, and the 3- to 8-membered heterocyclyl comprises at least one heteroatom selected from O, S and N; R₅ is selected from 3- to 8-membered cycloalkyl, 3- to 8-membered cycloalkenyl or 3- to 8-membered heterocyclyl, R₆ is selected from H or C1-C6 alkyl, or R₅ and R₆, together with the N atom to which they are attached, form 4- to 8-membered heterocyclyl; the 3- to 8-membered cycloalkyl, 3- to 8-membered cycloalkenyl, 3- to 8-membered heterocyclyl or 4- to 8-membered heterocyclyl comprises 0, 1, 2 or 3 heteroatoms selected from O, S and N, and is optionally substituted with one or more R_{z};
R₁ is -CN or -Z-R₁₂, wherein Z is a direct bond, -C1-C6 alkylene-, -C1-C6 alkylene-O-, -O-, -S-, -S(=O)-, -SO₂-, -NR₁₁-, -NR₁₁SO₂-, -SO₂NR₁₁-, -NR₁₁-S(=O)(=NH)-, -S(=O)(=NH)-, -C1-C6 alkylene-SO₂-, -C1-C6 alkylene-SO₂R₁₁-, - (C=O)-, -(C=O)NR₁₁-, -C=N(OH)- or -NR₁₁(C=O)-; or -Z-R₁₂ is -N=S(=O)-(R₁₂)₂, wherein a pair of two R₁₂, together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
X₇ is N or CR₇; X₈ is N or CR₈; X₉ is N or CR₉;
R₇, R₈ and R₉ are independently H, halogen, C1-C8 alkyl, C1-C6 haloalkyl, - OH, -O-R₈ₐ, -O-R_{8b} or -NRₐRₐ; where both R₇ and R₈ are present, R₇ and R₈, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S; where both Rₓ and R₉ are present, Rₓ and R₉, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
R₁₁ is H, R₁₁ₐ or R_{11b}; R₁₂ is H, R₁₂ₐ or R_{12b};
R₈ₐ, R₁₁ₐ and R₁₂ₐ are independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, - S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, - N(Rₐ)C(=NRₐ)NRₐRₐ, -N(Rₐ)S(=O)₂R_{b}, -N(Rₐ)S(=O)₂NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, - C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, R₁₄ or oxo;
R_{8b}, R_{11b} and R_{12b} are independently selected from the group consisting of: C1-C6 alkyl substituted with 0, 1, 2, 3, 4 or 5 groups selected from F, Cl, Br, -ORₐ, - OC1-C6 haloalkyl or CN;
R₁₄, in each case, is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0 or 1 atom selected from O and S, wherein the monocyclic ring or bicyclic ring is substituted with 0, 1, 2 or 3 groups selected from: F, Cl, Br, C1-C6 alkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-6 alkylene NRₐRₐ, -OC2-6 alkylene ORₐ, -SRₐ, -S(=O)R_{b}, -S(=O)₂R_{b}, -S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, -N(Rₐ)C(=NRₐ)NRₐRₐ, -N(Rₐ)S(=O)₂R_{b}, - N(Rₐ)S(=O)₂NRₐRₐ, -NRₐC2-C6 alkylene NR^{a}R^{a}, -NR^{a}C2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ or oxo;
each Rₐ is independently H or R_{b};
each R_{b} is independently C1-C6 alkyl, phenyl or benzyl, wherein C1-C6 alkyl is substituted with 0, 1, 2, or 3 substituents selected from: halogen, -OH, -O-C1-C6 alkyl, -NH₂, -NH-C1-C6 alkyl, -OC(=O)C1-C6 alkyl or -N(C1-C6 alkyl)(C1-C6 alkyl); and phenyl or benzyl is substituted with 0, 1, 2, or 3 substituents selected from: halogen, C1-C6 alkyl, C1-C6 haloalkyl, -OH, -O-C1-C6 alkyl, -NH₂, -NH-C1-C6 alkyl, -OC(=O)C1-C6 alkyl or -N(C1-C6 alkyl)C1-C6 alkyl;
each R_{z} is independently selected from the following groups: deuterium, halogen, C1-C6 alkyl, C3-C8 cycloalkyl, -C1-C6 alkylene-O-C1-C6 alkyl, C3-C8 cycloalkyl-C1-C6 alkylene, -OH, -CN, -O-C1-C6 alkyl, -O-C3-C8 cycloalkyl, -NH₂, -NH-C1-C6 alkyl, -OC(=O)C1-C6 alkyl, -N(C1-C6 alkyl)(C1-C6 alkyl), -S(O)-R_{z1}, -S(O)₂-R_{z1}, -C(O)-R_{z2}, -C(O)-NR_{z1}R_{z2}, 5- to 6-membered monocyclic heteroaryl comprising 0, 1, 2, or 3 N atoms, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two R_{z} attached to the same carbon atom may form 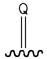 or two R_{z} attached to the same carbon atom, together with the carbon atom to which they are attached, may form C3-C8 cycloalkyl; the C1-C6 alkyl, C3-C8 cycloalkyl, -C1-C6 alkylene-O-C1-C6 alkyl, -O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl;
each R_{z1} is independently selected from the following groups: H, C1-C6 alkyl, or C1-C6 haloalkyl;
each R_{z2} is independently selected from the following groups: H, C1-C6 alkyl, C1-C6 haloalkyl, C1-C6 deuteroalkyl, or C3-C8 cycloalkyl;
Q is O or CR_{Q1}R_{Q2};
R_{Q1} and R_{Q2} are independently selected from the following groups: H, deuterium, halogen, C1-C6 alkyl, or C1-C6 haloalkyl.

In some embodiments, the present application provides the compound represented by formula (I), or the stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof, wherein
each R_{z} is independently selected from the following groups: deuterium, halogen, C1-C6 alkyl, C3-C8 cycloalkyl, -C1-C6 alkylene-O-C1-C6 alkyl, C3-C8 cycloalkyl-C1-C6 alkylene, -OH, -CN, -O-C1-C6 alkyl, -O-C3-C8 cycloalkyl, -NH₂, -NH-C1-C6 alkyl, -OC(=O)C1-C6 alkyl, -N(C1-C6 alkyl)(C1-C6 alkyl), -S(O)-R_{z1}, -S(O)₂-R_{z1}, -C(O)-R_{z2}, -C(O)-NR_{z1}R_{z2}, 5- to 6-membered monocyclic heteroaryl comprising 0, 1, 2, or 3 N atoms, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, - OC(=O)C1-C6 alkyl or -N(C1-C6 alkyl)(C1-C6 alkyl); and two R_{z} attached to the same carbon atom may form 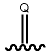 or two R_{z} attached to the same carbon atom, together with the carbon atom to which they are attached, may form C3-C8 cycloalkyl; the C1-C6 alkyl, C3-C8 cycloalkyl, -O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl.

In some embodiments, the present application provides the compound represented by formula (I), or the stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof, wherein
A is monocyclic aryl, monocyclic heteroaryl, bicyclic aryl, bicyclic heteroaryl or fused heterocyclyl, and optionally, the monocyclic aryl, monocyclic heteroaryl, bicyclic aryl, bicyclic heteroaryl or fused heterocyclyl is optionally substituted with one or more R_{z};
L is selected from -NR³-CO- or -NR³CONR³-, and each R³ is independently H, deuterium, C1-C6 alkylene or C1-C6 haloalkylene;
Rₓ is selected from -OR₄ or -NR₅R₆, wherein R₄ is selected from 3- to 8-membered cycloalkyl, 3- to 8-membered cycloalkenyl or 3- to 8-membered heterocyclyl, and the 3- to 8-membered heterocyclyl comprises at least one heteroatom selected from O, S and N; R₅ is selected from 3- to 8-membered cycloalkyl, 3- to 8-membered cycloalkenyl or 3- to 8-membered heterocyclyl, R₆ is selected from H or C1-C6 alkyl, or R₅ and R₆, together with the N atom to which they are attached, form 4- to 8-membered heterocyclyl; the 3- to 8-membered cycloalkyl, 3- to 8-membered cycloalkenyl, 3- to 8-membered heterocyclyl or 4- to 8-membered heterocyclyl comprises 0, 1, 2 or 3 heteroatoms selected from O, S and N, and is optionally substituted with one or more R_{z};
R₁ is -CN or -Z-R₁₂, wherein Z is a direct bond, -C1-C6 alkylene-, -C1-C6 alkylene-O-, -O-, -S-, -S(=O)-, -SO₂-, -NR₁₁-, -NR₁₁SO₂-, -SO₂NR₁₁-, -NR₁₁-S(=O)(=NH)-, -S(=O)(=NH)-, -C1-C6 alkylene-SO₂-, -C1-C6 alkylene-SO₂R₁₁-, - (C=O)-, -(C=O)NR₁₁-, -C=N(OH)- or -NR₁₁(C=O)-; or -Z-R₁₂ is -N=S(=O)-(R₁₂)₂, wherein a pair of two R₁₂, together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
X₇ is N or CR₇; X₈ is N or CR₈; X₉ is N or CR₉;
R₇, R₈ and R₉ are independently H, halogen, C1-C8 alkyl, C1-C6 haloalkyl, - OH, -O-R₈ₐ, -O-R_{8b} or -NRₐRₐ; where both R₇ and R₈ are present, R₇ and R₈, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S; where both Rₓ and R₉ are present, Rₓ and R₉, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
R₁₁ is H, R₁₁ₐ or R_{11b}; R₁₂ is H, R₁₂ₐ or R_{12b};
R₈ₐ, R₁₁ₐ and R₁₂ₐ are independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, - S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, - N(Rₐ)C(=NRₐ)NRₐRₐ, -N(Rₐ)S(=O)₂R_{b}, -N(Rₐ)S(=O)₂NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, - C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, R₁₄ or oxo;
R_{8b}, R_{11b} and R_{12b} are independently selected from the group consisting of: C1-C6 alkyl substituted with 0, 1, 2, 3, 4 or 5 groups selected from F, Cl, Br, -ORₐ, - OC1-C6 haloalkyl or CN;
R₁₄, in each case, is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0 or 1 atom selected from O and S, wherein the monocyclic ring or bicyclic ring is substituted with 0, 1, 2 or 3 groups selected from: F, Cl, Br, C1-C6 alkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-6 alkylene NRₐRₐ, -OC2-6 alkylene ORₐ, -SRₐ, -S(=O)R_{b}, -S(=O)₂R_{b}, -S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, -N(Rₐ)C(=NRₐ)NRₐRₐ, -N(Rₐ)S(=O)₂R_{b}, - N(Rₐ)S(=O)₂NRₐRₐ, -NRₐC2-C6 alkylene NR^{a}R^{a}, -NR^{a}C2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ or oxo;
each Rₐ is independently H or R_{b};
each R_{b} is independently C1-C6 alkyl, phenyl or benzyl, wherein C1-C6 alkyl is substituted with 0, 1, 2, or 3 substituents selected from: halogen, -OH, -O-C1-C6 alkyl, -NH₂, -NH-C1-C6 alkyl, -OC(=O)C1-C6 alkyl or -N(C1-C6 alkyl)(C1-C6 alkyl); and phenyl or benzyl is substituted with 0, 1, 2, or 3 substituents selected from: halogen, C1-C6 alkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl);
each R_{z} is independently selected from the following groups: deuterium, halogen, C1-C6 alkyl, C3-C8 cycloalkyl, C1-C6 haloalkyl, C3-C8 cycloalkyl-C1-C6 alkylene, -OH, -CN, -O-C1-C6 alkyl, -NH₂, -NH-C1-C6 alkyl, -OC(=O)C1-C6 alkyl, -N(C1-C6 alkyl)(C1-C6 alkyl), -S(O)-R_{z1}, -S(O)₂-R_{z1}, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two R_{z} attached to the same carbon atom may form 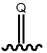 or two R_{z} attached to the same carbon atom, together with the carbon atom to which they are attached, may form C3-C8 cycloalkyl; the C1-C6 alkyl, C3-C8 cycloalkyl, -O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl;
each R_{z1} is independently selected from the following groups: C1-C6 alkyl, or C1-C6 haloalkyl;
Q is O or CR_{Q1}R_{Q2};
R_{Q1} and R_{Q2} are independently selected from the following groups: H, deuterium, halogen, C1-C6 alkyl, or C1-C6 haloalkyl.

In some embodiments, each R_{z} is independently selected from the following groups: hydrogen, deuterium, halogen, C1-C6 alkyl, C3-C8 cycloalkyl, C1-C6 haloalkyl, C3-C8 cycloalkyl-C1-C6 alkylene, -OH, -CN, -O-C1-C6 alkyl, -NH₂, - NH-C1-C6 alkyl, -OC(=O)C1-C6 alkyl, -N(C1-C6 alkyl)(C1-C6 alkyl), -S(O)-R_{z1}, -S(O)₂-R_{z1}, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two R_{z} attached to the same carbon atom may form 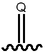 or two R_{z} attached to the same carbon atom, together with the carbon atom to which they are attached, may form C3-C8 cycloalkyl; the C1-C6 alkyl, C3-C8 cycloalkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl.

In some embodiments, each R_{z} is independently selected from the following groups: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two or more of the substituents, together with the carbon atom to which they are attached, may form C3-C8 cycloalkyl; the C1-C6 alkyl, C3-C8 cycloalkyl, or -O-C1-C6 alkyl may be optionally substituted with substituents selected from deuterium, F, Cl, Br, I, C1-C6 alkyl, C3-C8 cycloalkyl, C1-C6 alkoxy or C1-C4 haloalkyl.

In some embodiments, formula (I) is as represented by formula (I) -1, formula (I) -2, or formula (I) -3:

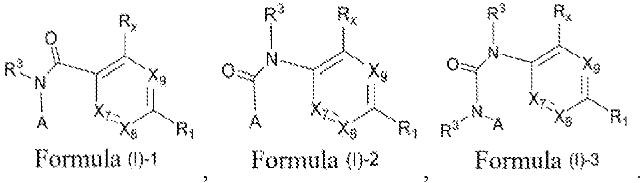

The symbols of formula (I)-1 to formula (I)-3 are as defined in formula (I).

In some embodiments, Rₓ is selected from the group consisting of: 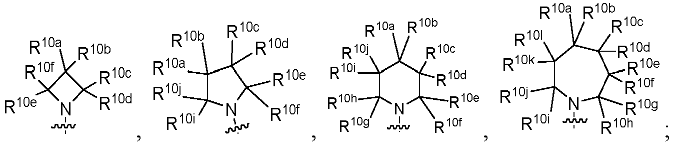
preferably, Rₓ is 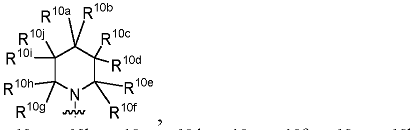
wherein each of R^{10a}, R^{10b}, R^{10c}, R^{10d}, R^{10e}, R^{10f}, R^{10g}, R^{10h}, R¹⁰ⁱ, R^{10j}, R^{10k} and R¹⁰¹ is H, deuterium, halogen, -CN, -OH, C1-C6 alkyl, C1-C6 alkoxy, C1-C6 haloalkyl, C3-C8 cycloalkyl, -NH₂, -NH(C1-C6 alkyl), -N(C1-C6 alkyl)(C1-C6 alkyl), or saturated, partially saturated or unsaturated 3-, 4-, 5-, 6- or 7-membered monocyclic ring or 5-, 6-, 7-, 8-, 9-, 10-, 11- or 12-membered bicyclic ring, which comprises 1-3 heteroatoms selected from N, O and S, and the C1-C6 alkyl, C1-C6 alkoxy, C1-C6 haloalkyl, or C3-C8 cycloalkyl is optionally substituted with 1-5 substituents selected from deuterium, halogen, -OH, -CN, C1-C6 alkyl, C1-C6 haloalkyl, C1-C6 alkoxy, or -O-C1-C6 haloalkyl; the monocyclic ring or bicyclic ring is substituted with 0, 1, 2, or 3 groups selected from: F, Cl, Br, C1-C6 alkyl, C1-C6 haloalkyl, -ORₘ, -OC1-C6 haloalkyl, CN, -C(=O)Rₙ, -C(=O)ORₘ, - C(=O)NRₘRₘ, -C(=NRₘ)NRₘRₘ, -OC(=O)Rₙ, -OC(=O)NRₘRₘ, -OC2-C6 alkylene NRₘRₘ, -OC2-C6 alkylene ORₘ, -SRₘ, -S(=O)Rₙ, -S(=O)₂Rₙ, - S(=O)₂NRₘRₘ, -NRₘRₘ, -N(Rₘ)C(=O)Rₙ, -N(Rₘ)C(=O)ORₙ, - N(Rₘ)C(=O)NRₘRₘ, -N(Rₘ)C(=NRₘ)NRₘRₘ, -N(Rₘ)S(=O)₂Rₙ, - N(Rₘ)S(=O)₂NRₘRₘ, -NRₘC2-6 alkylene NRₘRₘ, -NRₘC2-C6 alkylene ORₘ, -C1-C6 alkylene NRₘRₘ, -C1-C6 alkylene ORₘ, -C1-C6 alkylene N(Rₘ)C(=O)Rₙ, -C1-C6 alkylene OC(=O)Rₙ, -C1-C6 alkylene C(=O)NRₘRₘ, -C1-C6 alkylene C(=O)ORₘ or oxo;
or alternatively, each of the R^{10a} and R^{10b} pair, R^{10c} and R^{10d} pair, R^{10e} and R^{10f} pair, R^{10g} and R^{10h} pair, R¹⁰ⁱ and R^{10j} pair, and R^{10k} and R^{10l} pair, independently together with the carbon atom to which they are each attached, may form saturated or partially saturated 3-, 4-, 5-, or 6-membered monocyclic ring spiro-connected to the Rₓ ring, wherein the 3-, 4-, 5-, or 6-membered monocyclic ring comprises 0, 1, 2 or 3 N atoms and 0, 1 or 2 atoms selected from O and S, and further, the 3-, 4-, 5-, or 6-membered monocyclic ring is substituted with 0, 1, 2, or 3 groups selected from: F, Cl, Br, C1-C6 alkyl, C1-C4 haloalkyl, -ORₘ, -OC1-C4 haloalkyl, CN, -NRₘRₘ or oxo;
Rₘ and Rₙ are each independently selected from H or -C1-C6 alkyl;
or alternatively, the R^{10a} and R^{10b} pair, R^{10c} and R^{10d} pair, R^{10e} and R^{10f} pair, R^{10g} and R¹⁰h pair, R¹⁰ⁱ and R^{10j} pair, and R^{10k} and R^{10l} pair may form 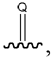 , wherein Q is CR_{Q1}R_{Q2}; R_{Q1} and R_{Q2} are independently selected from the following groups: H, deuterium, halogen, C1-C6 alkyl, or C1-C6 haloalkyl.

In some embodiments, Rₓ is

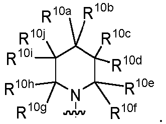

In some preferred embodiments, each of R^{10c}, R^{10d}, R^{10e}, R^{10f}, R^{10g}, R^{10h} R¹⁰ⁱ and R^{10j} is H, deuterium, halogen, -CN, -OH, C1-C6 alkyl, C1-C6 alkoxy, C1-C6 haloalkyl, -NH2, -NH(C1-C6 alkyl) or -N(C1-C6 alkyl)(C1-C6 alkyl), and the C1-C6 alkyl, C1-C6 alkoxy, or C1-C6 haloalkyl is optionally substituted with 1-5 substituents selected from deuterium, halogen, -OH, -CN, C1-C6 alkyl, C1-C6 haloalkyl, C1-C6 alkoxy, or -O-C1-C6 haloalkyl; the R10a and R10b pair forms 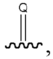 and Q is CR_{Q1}R_{Q2}; R_{Q1} and R_{Q2} are independently selected from the following groups: H, deuterium, halogen, C1-C6 alkyl, or C1-C6 haloalkyl, or the R^{10a} and R^{10b} pair, together with the carbon atom to which they are attached, forms C3-C8 cycloalkyl spiro-connected to the Rx ring, and further, the C3-C8 cycloalkyl is substituted with 0, 1, 2, or 3 groups selected from: F, Cl, Br, C1-C6 alkyl, C1-C4 haloalkyl, -OH, C1-C6 alkoxy, -OC1-C4 haloalkyl, -CN, -NH2, -NH(C1-C6 alkyl), -N(C1-C6 alkyl)(C1-C6 alkyl) or oxo.

In some embodiments, Rₓ is selected from: 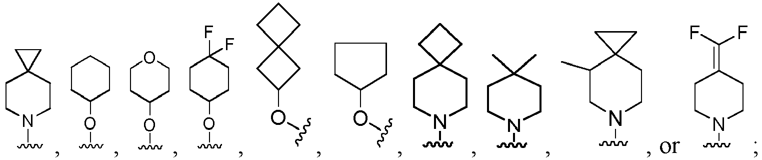
preferably, Rₓ is selected from: 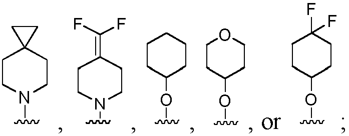
more preferably, Rₓ is selected from:

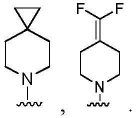

In some embodiments, formula (I) is as represented by formula (1)-4-1, formula (I)-4-2, formula (I)-4-3, formula (I)-4-4, formula (1)-5-1, formula (I)-5-2, formula (I)-6-1 or formula (I)-6-2:

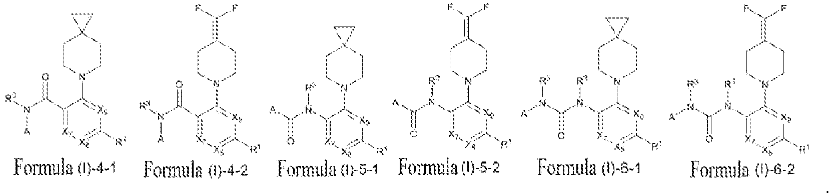

In some embodiments, R₁ is group -Z-R₁₂, wherein -Z is a single bond, -C1-C4 alkylene-, -C1-C4 alkylene-O-, -O-, -S-, -S(=O)-, -SO₂-, -NH-, -NHSO₂-, -SO₂NH-, -NH-S(=O)(=NH)-, -S(=O)(=NH)-, -C1-C4 alkylene-SO₂-, -(C=O)-, -(C=O)NH-, - C=N(OH)- or -NH(C=O)-; and/or
(a) R₁₂ is H;
(b) R₁₂ is oxetanyl or cyclopropyl substituted with 0, 1, 2 or 3 groups selected from OH, halogen (such as F or Cl), or C1-C6 alkoxy; or
(c) R₁₂ is C1-C6 alkyl substituted with 0, 1, 2 or 3 groups selected from OH, halogen (such as F), or C1-C6 alkoxy.

preferably, the group -Z-R₁₂ is -N=S(=O)-(R₁₂)₂, wherein a pair of two R₁₂, alternatively together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
or in the group -Z-R₁₂, Z is a single bond, and R₁₂ is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, -S(=O)₂NRₐRₐ, -NRₐRₐ, -N(Rₐ)C(=O)R_{b}, -N(Rₐ)C(=O)OR_{b}, - N(Rₐ)C(=O)NRₐRₐ, -N(Rₐ)C(=NRₐ)NRₐRₐ, -N(Rₐ)S(=O)₂R_{b}, -N(Rₐ)S(=O)₂NRₐRₐ, - NRₐC2-6 alkylene NRₐRₐ, -NRₐC2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, - C1-C6 alkylene C(=O)NRₐRₐ, -C1-C6 alkylene C(=O)ORₐ, or oxo, and Rₐ and R_{b} are independently selected from H or -C1-C6 alkyl.

More preferably, R₁ is group -Z-R₁₂, wherein Z is -NHSO₂- or -SO₂NH-; and R₁₂ is oxetanyl or cyclopropyl substituted with 0, 1, 2 or 3 groups selected from OH, halogen (such as F), or C1-C6 alkoxy, or R₁₂ is C1-C6 alkyl substituted with 0, 1, 2 or 3 groups selected from OH, halogen (such as F), or C1-C6 alkoxy.

Further preferably, R₁ is selected from the following groups:

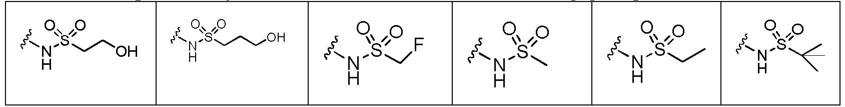

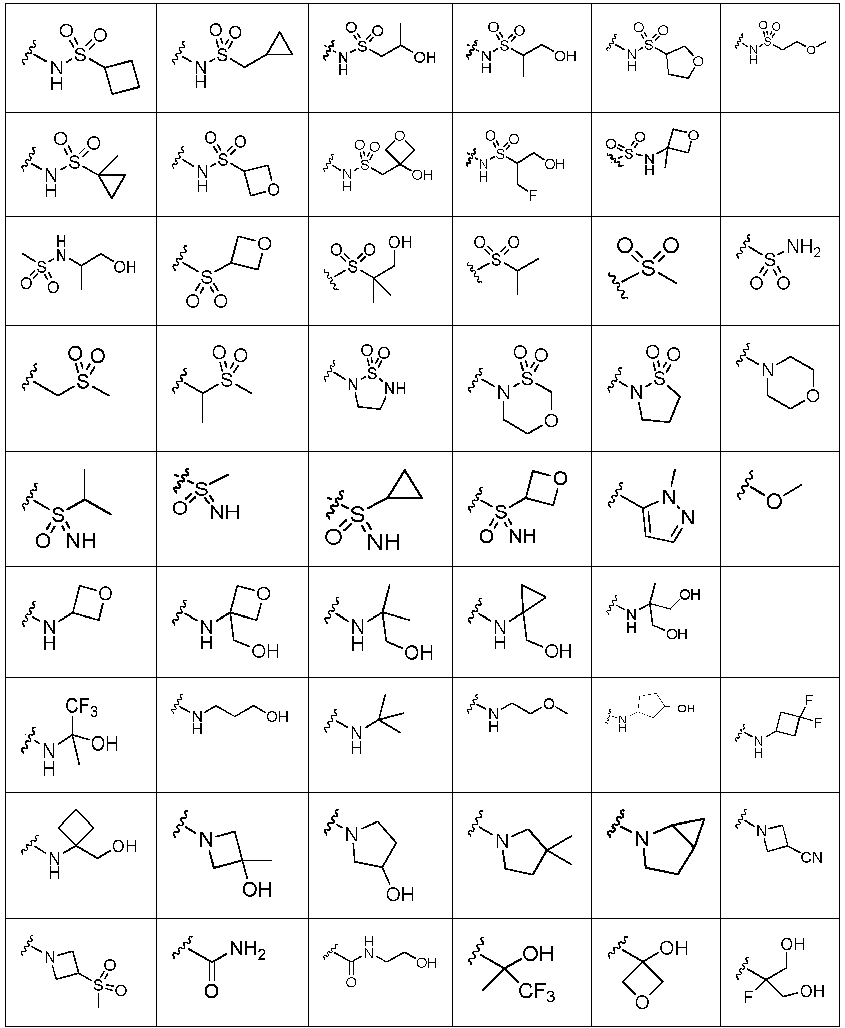

Still further preferably, R₁ is selected from the following groups:

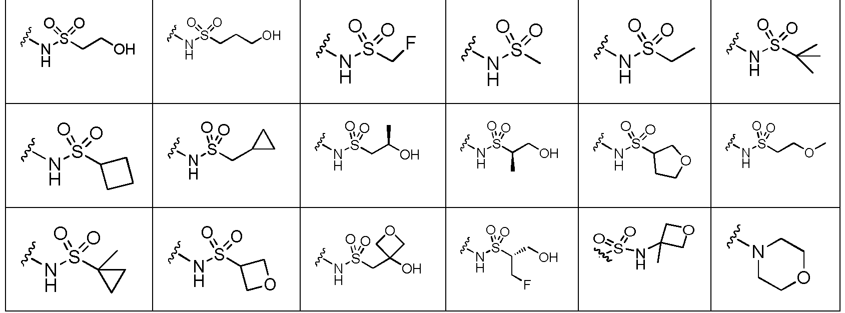

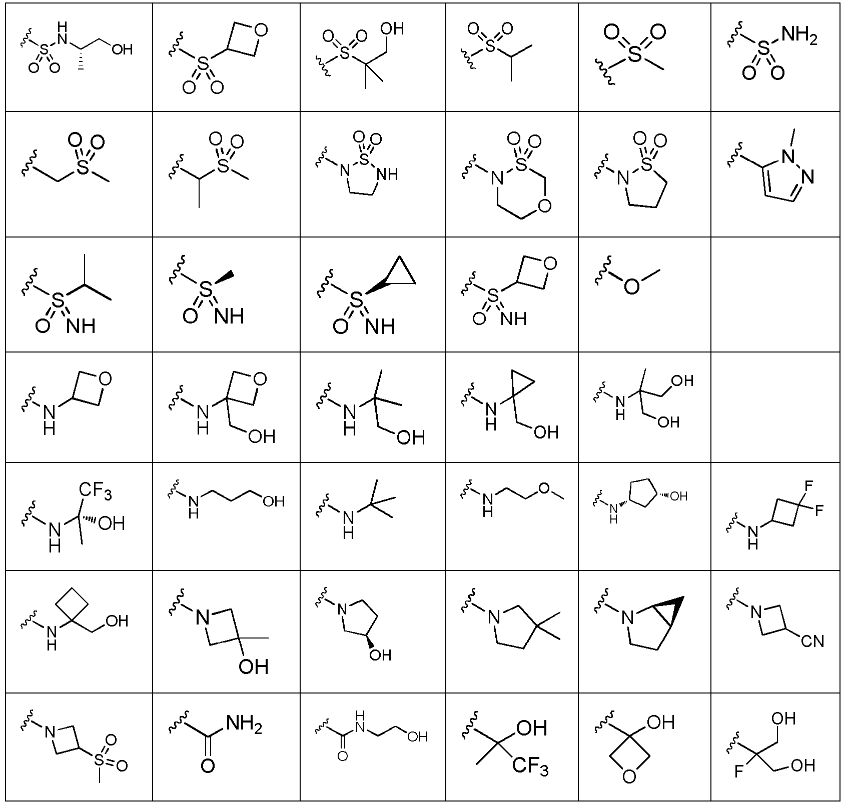

most preferably, R₁ is

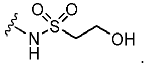

In some embodiments, formula (I) is as represented by formula (I)-1-1, formula (I)-1-2, formula (I)-2-1, formula (I)-2-2, formula (1)-3-1 or formula (I)-3-2:

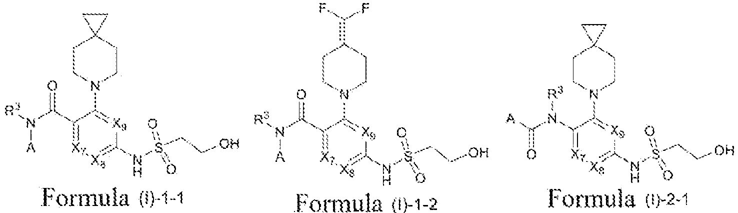

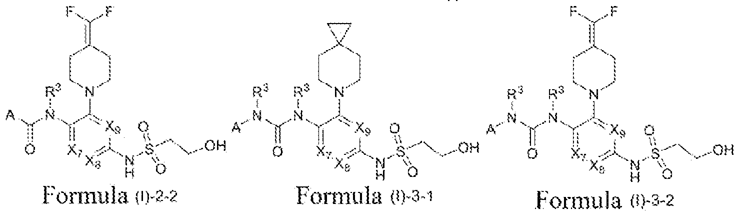

Preferably, formula (I) is as represented by formula (I)-1-1, formula (I)-1-2, formula (1)-2-1 or formula (I)-2-2:

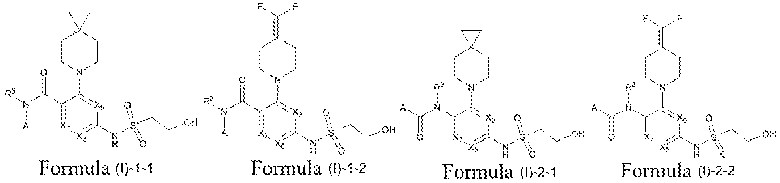

In some embodiments, each R³ is independently H, C1-C4 alkyl or C1-C4 haloalkyl;
preferably, each R³ is independently H.

In some embodiments, X₇ is CR₇.

In some embodiments, X₇ is N.

In some embodiments, X₈ is CR₈.

In some embodiments, X₈ is N.

In some embodiments, X₉ is CR₉.

In some embodiments, X₉ is N.

In some embodiments, X₇ is CR₇, X₈ is CR₈, and X₉ is CR₉.

In some embodiments, X₇ is CR₇, X₈ is CR₈, and X₉ is N.

In some embodiments, X₇ is CR₇, X₈ is N, and X₉ is CR₉.

In some embodiments, X₇ is CR₇, X₈ is N, and X₉ is N.

In some embodiments, R₇ is H, halogen, C1-C8 alkyl, C1-C6 haloalkyl, -OH, - O-R₈ₐ, -O-R_{8b} or -NRₐRₐ;
preferably, R₇ is H, halogen, C1-C8 alkyl, or -NRₐRₐ;
more preferably, R₇ is H or -NRₐRₐ;
still further preferably, R₇ is H, -NH₂ or -NH-C1-C6 alkyl.

In some embodiments, R₇ is H.

In some embodiments, R₇ is -NRₐRₐ.

In some embodiments, R₇ is -NH₂ or -NH-C1-C6 alkyl; preferably, R₇ is -NH₂ or -NHCH₃.

In some embodiments, X₇ is CR₇ and R₇ is -NH₂ or -NH-C1-C6 alkyl, X₈ is CH, and X₉ is CH.

In some embodiments, X₇ is CH, X₈ is N or CR₈, and X₉ is N or CR₉; preferably, X₇ is CH, X₈ is CR₈, and X₉ is CR₉; more preferably, X₇ is CH, X₈ is CH, and X₉ is CH.

In some embodiments, X₇ is N and/or X₈ is N.

In some embodiments, R₈ and R₉ are independently H, halogen, C1-C8 alkyl, C1-C6 haloalkyl, -OH, -O-R₈ₐ, -O-R_{8b} or -NRₐRₐ; preferably, R₈ and R₉ are independently H, halogen, C1-C8 alkyl, -OH or -NRₐRₐ; more preferably, R₈ and R₉ are independently H or halogen; most preferably, R₈ and R₉ are H.

In some embodiments, A is selected from phenyl, 5- to 6-membered nitrogen-containing heteroaryl, benzomonoheterocyclyl, benzobisheterocyclyl, 5- to 6-membered nitrogen-containing heteroaryl fused monoheterocyclyl, 5- to 6-membered nitrogen-containing heteroaryl fused bisheterocyclyl, benzomonoheteroaryl, or 5- to 6-membered nitrogen-containing heteroaryl fused monoheteroaryl;
optionally, the phenyl, 5- to 6-membered nitrogen-containing heteroaryl, benzomonoheterocyclyl, benzobisheterocyclyl, 5- to 6-membered nitrogen-containing heteroaryl fused monoheterocyclyl, 5- to 6-membered nitrogen-containing heteroaryl fused bisheterocyclyl, benzomonoheteroaryl, or 5- to 6-membered nitrogen-containing heteroaryl fused monoheteroaryl is substituted with one or more R_{z},
monoheterocyclyl in the benzomonoheterocyclyl, or 5- to 6-membered nitrogen-containing heteroaryl fused monoheterocyclyl contains 0, 1, 2 or 3 atoms selected from N, O and S;
monoheteroaryl in the benzomonoheteroaryl, or 5- to 6-membered nitrogen-containing heteroaryl fused monoheteroaryl contains 0, 1, 2 or 3 atoms selected from N, O and S;
bisheterocyclyl in the benzobisheterocyclyl and 5- to 6-membered nitrogen-containing heteroaryl fused bisheterocyclyl contains 0, 1, 2 or 3 atoms selected from N, O and S.

In some embodiments, A is a group selected from group A1, group A2 or group A3, wherein
the group A1 includes the following groups: 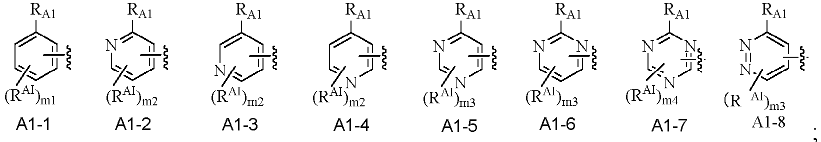
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -CN, -O-C1-C6 alkyl, C1-C6 alkylene-O-C1-C6 alkyl, C1-C6 haloalkoxy, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 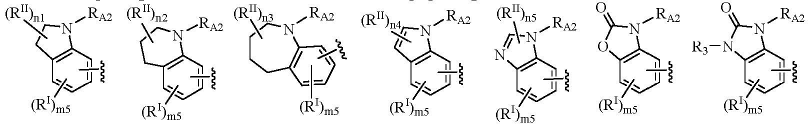 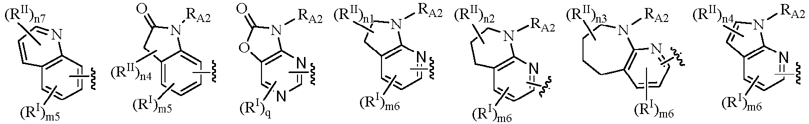 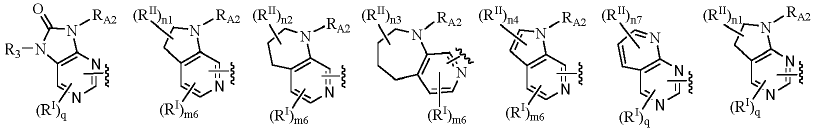 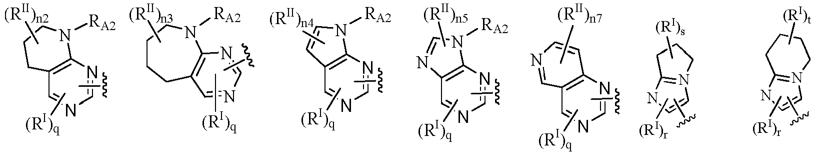 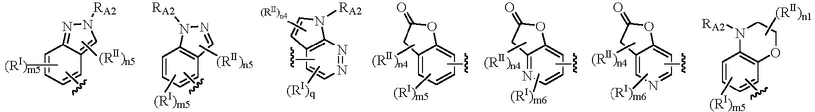 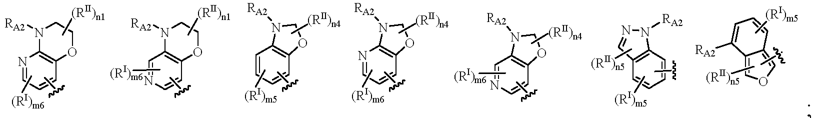
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, C3-C8 cycloalkyl-C1-C6 alkylene, -OH, C1-C6 alkoxy, -NH₂, -NH-C1-C6 alkyl, -OC(=O)C1-C6 alkyl, -N(C1-C6 alkyl)(C1-C6 alkyl), -S(O)-R_{z1} or -S(O)₂-R_{z1}, and the C1-C6 alkyl, C3-C8 cycloalkyl, C1-C6 haloalkyl, C1-C6 alkoxy, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkyl, C1-C6 alkoxy or C1-C4 haloalkyl;
each R_{z1} is independently selected from the following groups: C1-C6 alkyl, or C1-C6 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 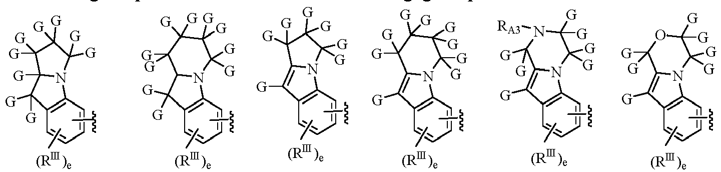 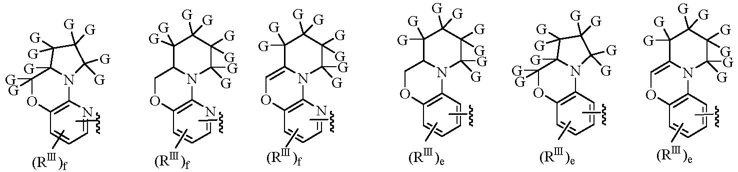 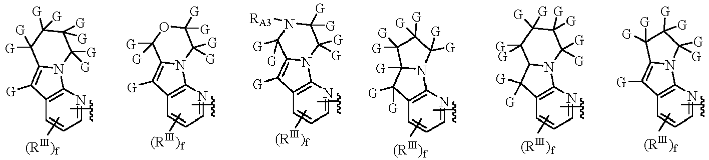 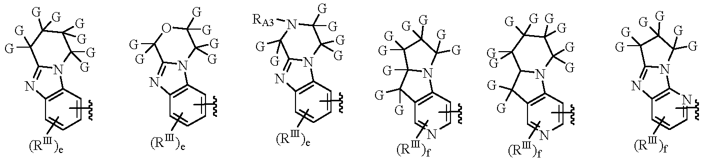 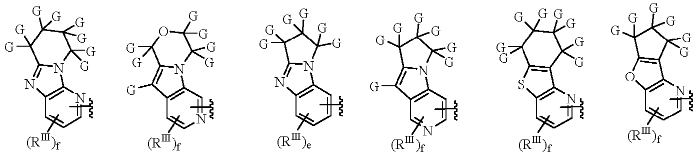 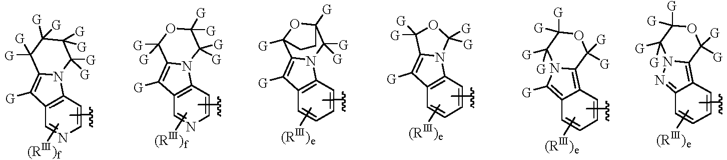 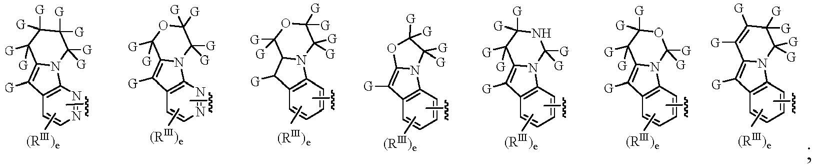
in the group A3,
each G is independently selected from hydrogen, deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 haloalkyl, and C1-C6 alkoxy may be optionally substituted with substituents selected from deuterium, halogen, C1-C6 alkyl, C3-C8 cycloalkyl, C1-C6 alkoxy or C1-C4 haloalkyl, or two G attached to the same carbon atom form an oxo group (=O) or two G attached to the same carbon atom, together with the carbon atom to which they are attached, may form C3-C8 cycloalkyl;
R_{A3} is independently selected from hydrogen, deuterium, halogen, C1-C6 alkyl, C3-C8 cycloalkyl, C1-C6 haloalkyl, -OH, -CN, -O-C3-C8 cycloalkyl, C1-C6 alkoxy, -NH₂, -NH-C1-C6 alkyl, -C(=O)NH-C1-C6 alkyl, -OC(=O)C1-C6 alkyl, -C(=O)C3-C8 cycloalkyl, -N(C1-C6 alkyl)(C1-C6 alkyl), -C(=O)N(C1-C6 alkyl)(C1-C6 alkyl), phenyl, 5- to 6-membered monocyclic heteroaryl comprising 1, 2 or 3 N atoms, or 5- to 6-membered monocyclic heteroaryl comprising 0, 1, 2 or 3 N atoms and 1 or 2 atoms selected from O, and the C1-C6 alkyl, C3-C8 cycloalkyl, C1-C6 alkoxy, or 5-to 6-membered monocyclic heteroaryl may be optionally substituted with substituents selected from deuterium, F, Cl, Br, I, C1-C6 alkyl, C3-C8 cycloalkyl, C1-C6 alkoxy or C1-C4 haloalkyl;
R^{III} is independently selected from deuterium, halogen, C1-C6 alkyl, C3-C8 cycloalkyl, C1-C6 haloalkyl, -OH, -CN, -O-C3-C8 cycloalkyl, C1-C6 alkoxy, -NH₂, -NH-C1-C6 alkyl, -C(=O)NH-C1-C6 alkyl, -OC(=O)C1-C6 alkyl, -C(=O)C3-C8 cycloalkyl, -N(C1-C6 alkyl)(C1-C6 alkyl), -C(=O)N(C1-C6 alkyl)(C1-C6 alkyl), phenyl, -5- to 6-membered monocyclic heteroaryl-C1-C6 alkyl comprising 1, 2 or 3 N atoms, 5- to 6-membered monocyclic heteroaryl comprising 1, 2 or 3 N atoms, or 5- to 6-membered monocyclic heteroaryl comprising 0, 1, 2 or 3 N atoms and 1 or 2 atoms selected from O, and the C1-C6 alkyl, C3-C8 cycloalkyl, C1-C6 alkoxy, or 5-to 6-membered monocyclic heteroaryl may be optionally substituted with substituents selected from deuterium, halogen, C1-C6 alkyl, C3-C8 cycloalkyl, C1-C6 alkoxy or C1-C4 haloalkyl;
e is selected from 0, 1, 2 or 3; f is selected from 0, 1 or 2.

In some embodiments, each G is independently selected from hydrogen, deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 haloalkyl, and C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl, or two G attached to the same carbon atom form an oxo group (=O) or two G attached to the same carbon atom, together with the carbon atom to which they are attached, may form C3-C8 cycloalkyl.

In some embodiments, R_{A3} and R^{III} are each independently selected from deuterium, halogen, C1-C6 alkyl, C3-C8 cycloalkyl, C1-C6 haloalkyl, -OH, -CN, - O-C3-C8 cycloalkyl, C1-C6 alkoxy, -NH₂, -NH-C1-C6 alkyl, -C(=O)NH-C1-C6 alkyl, -OC(=O)C1-C6 alkyl, -C(=O)C3-C8 cycloalkyl, -N(C1-C6 alkyl)(C1-C6 alkyl), -C(=O)N(C1-C6 alkyl)(C1-C6 alkyl), phenyl, 5- to 6-membered monocyclic heteroaryl comprising 1, 2 or 3 N atoms, or 5- to 6-membered monocyclic heteroaryl comprising 0, 1, 2 or 3 N atoms and 1 or 2 atoms selected from O, and the C1-C6 alkyl, C3-C8 cycloalkyl, C1-C6 alkoxy, or 5- to 6-membered monocyclic heteroaryl may be optionally substituted with substituents selected from deuterium, F, Cl, Br, I, C1-C6 alkyl, C3-C8 cycloalkyl, C1-C6 alkoxy or C1-C4 haloalkyl.

In other embodiments, R_{A3} is independently selected from hydrogen, deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 alkyl, or C1-C6 alkoxy may be optionally substituted with substituents selected from deuterium, F, Cl, Br, I, C1-C6 alkyl, C3-C8 cycloalkyl, C1-C6 alkyl, C1-C6 alkoxy or C1-C4 haloalkyl.

In some embodiments, R_{A3} and R^{III} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 alkyl, or C1-C6 alkoxy may be optionally substituted with substituents selected from deuterium, F, Cl, Br, I, C1-C6 alkyl, C3-C8 cycloalkyl, C1-C6 alkyl, C1-C6 alkoxy or C1-C4 haloalkyl.

In some embodiments, A is a group selected from group A1, group A2 or group A3, wherein
the group A1 includes the following groups: 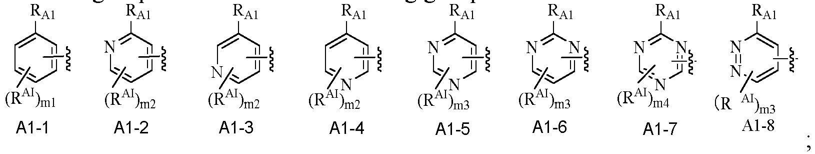
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -CN, -O-C1-C6 alkyl, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 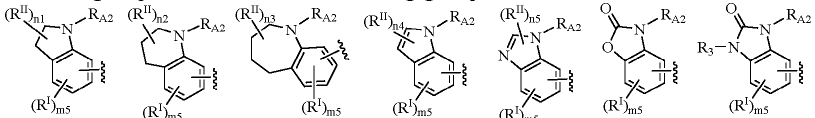 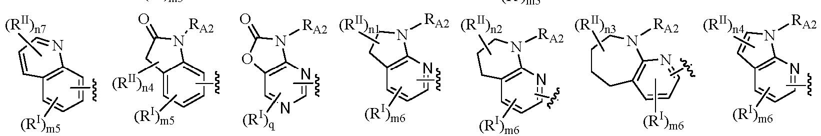 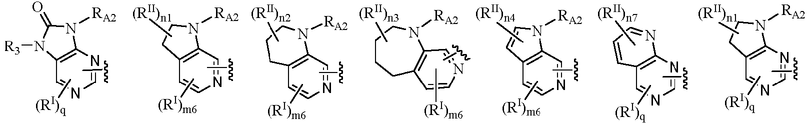 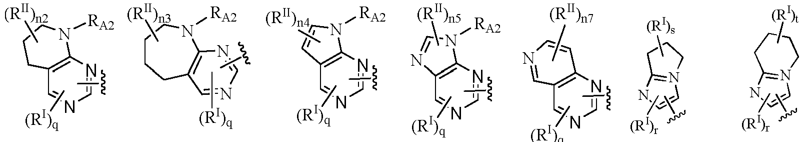 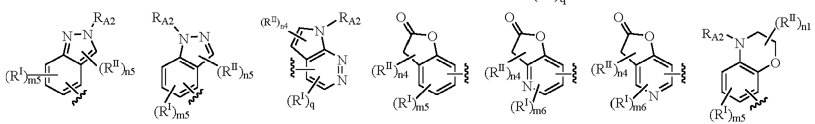 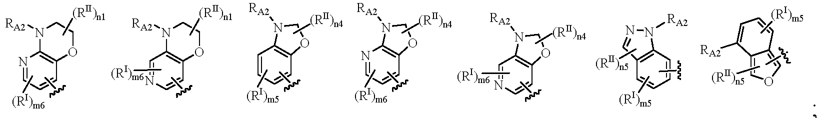
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, C3-C8 cycloalkyl-C1-C6 alkylene, -OH, C1-C6 alkoxy, -NH₂, -NH-C1-C6 alkyl, -OC(=O)C1-C6 alkyl, -N(C1-C6 alkyl)(C1-C6 alkyl), -S(O)-R_{z1} or -S(O)₂-R_{z1}, and the C1-C6 alkyl, C3-C8 cycloalkyl, C1-C6 haloalkyl, C1-C6 alkoxy, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy or C1-C4 haloalkyl;
each R_{z1} is independently selected from the following groups: C1-C6 alkyl, or C1-C6 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 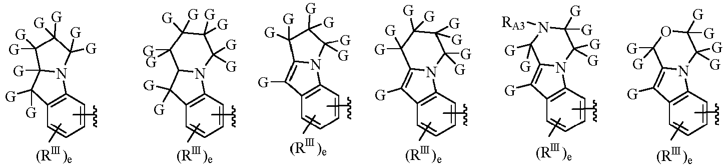 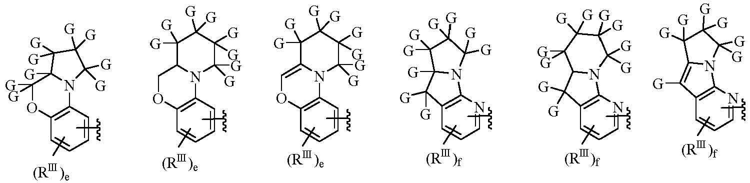 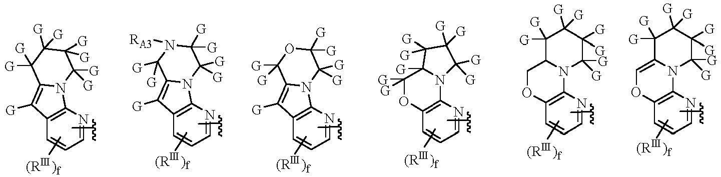 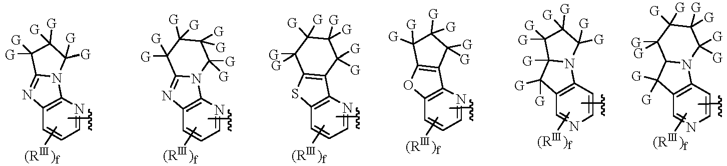 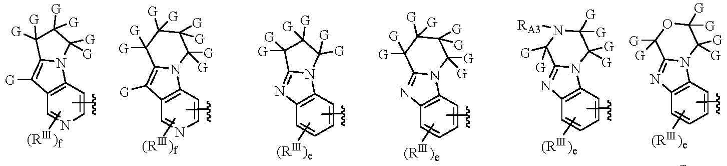 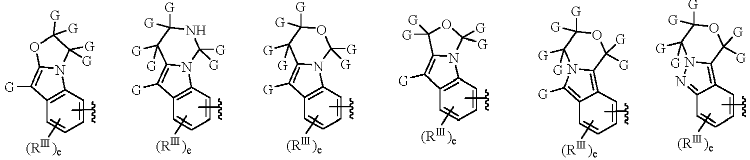 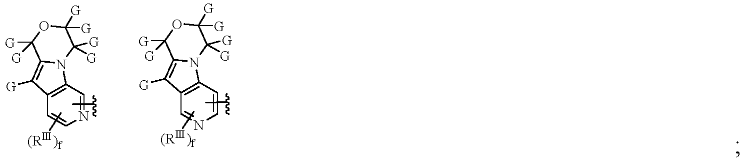
in the group A3,
G, R_{A3} and R^{III} are each independently as defined above; for example, in some embodiments, each G is independently selected from hydrogen, deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 haloalkyl, and C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl, or two G attached to the same carbon atom form an oxo group (=O); and/or, in some embodiments, R_{A3} is independently selected from hydrogen, deuterium, halogen, C1-C6 alkyl, C3-C8 cycloalkyl, C1-C6 haloalkyl, -OH, -CN, C1-C6 alkoxy, -NH₂, -NH-C1-C6 alkyl, - OC(=O)C1-C6 alkyl or -N(C1-C6 alkyl)(C1-C6 alkyl), and the C1-C6 alkyl, C3-C8 cycloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl, and R^{III} is independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl; in other embodiments, R_{A3} and R^{III} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
e is selected from 0, 1, 2 or 3;
f is selected from 0, 1 or 2.

In some embodiments, A is a group selected from group A1, group A2 or group A3, wherein
the group A1 includes the following groups: 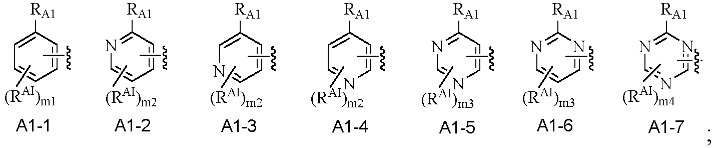
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 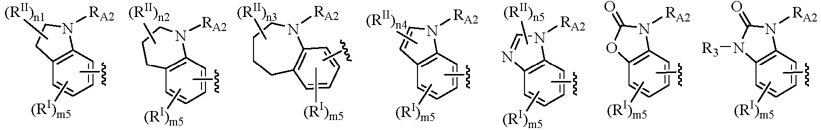 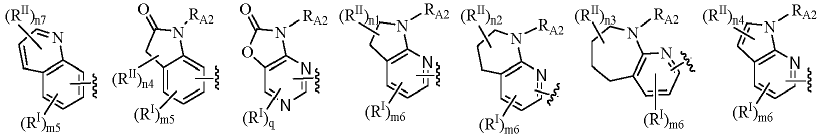 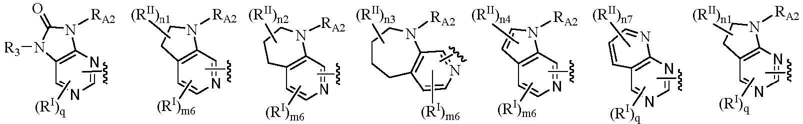 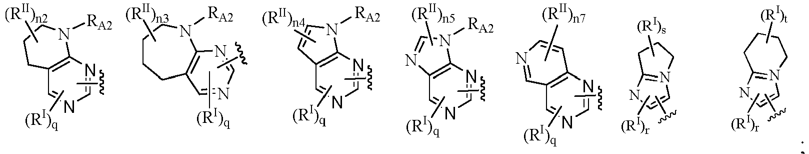
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, -OH, C1-C6 alkoxy, -NH₂, -NH-C1-C6 alkyl, -OC(=O)C1-C6 alkyl, -N(C1-C6 alkyl)(C1-C6 alkyl) or C3-C8 cycloalkyl-C1-C6 alkylene, and the C1-C6 alkyl, C3-C8 cycloalkyl, C1-C6 haloalkyl, C1-C6 alkoxy, or C3-C8 cycloalkyl-C1-C6 alkylene may be optionally substituted with substituents selected from deuterium, F, Cl, Br, I, -OH, C1-C6 alkyl, C3-C8 cycloalkyl, C1-C6 alkoxy or C1-C4 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 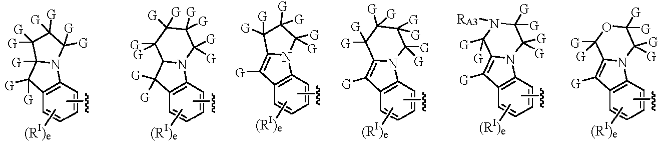 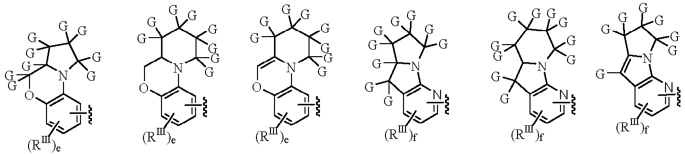 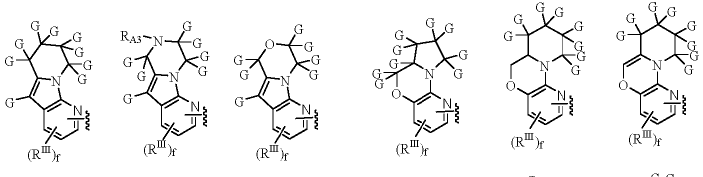 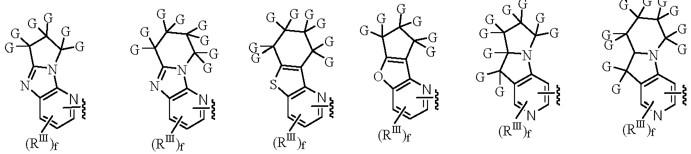 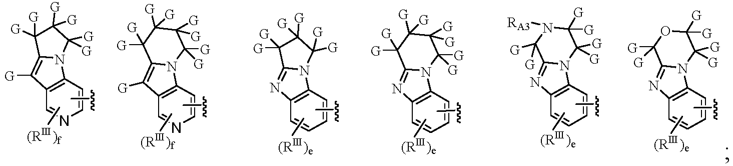
in the group A3,
G, R_{A3} and R^{III} are each independently as defined above; for example, in some embodiments, each G is independently selected from hydrogen, deuterium, halogen, C1-C6 alkyl, C3-C8 cycloalkyl, C1-C6 haloalkyl, -OH, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl; and/or, in some embodiments, R_{A3} is independently selected from hydrogen, deuterium, halogen, C1-C6 alkyl, C3-C8 cycloalkyl, C1-C6 haloalkyl, -OH, C1-C6 alkoxy, -NH₂, -NH-C1-C6 alkyl, - OC(=O)C1-C6 alkyl or -N(C1-C6 alkyl)(C1-C6 alkyl), and the C1-C6 alkyl, C3-C8 cycloalkyl, C1-C6 alkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl, and R^{III} is independently selected from deuterium, halogen, C1-C6 alkyl, C3-C8 cycloalkyl, C1-C6 haloalkyl, -OH, C1-C6 alkoxy, - NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 alkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl; in other embodiments, R_{A3} and R^{III} are each independently selected from deuterium, halogen, C1-C6 alkyl, C3-C8 cycloalkyl, C1-C6 haloalkyl, -OH, C1-C6 alkoxy, -NH₂, -NH-C1-C6 alkyl, - OC(=O)C1-C6 alkyl or -N(C1-C6 alkyl)(C1-C6 alkyl), and the C1-C6 alkyl, C3-C8 cycloalkyl, C1-C6 alkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
e is selected from 0, 1, 2 or 3; f is selected from 0, 1 or 2.

In some embodiments, A is a group selected from the A1 group, and in the A1 group, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, -CN, C1-C6 alkyl, C3-C8 cycloalkyl, C1-C6 haloalkyl, -OH, -O-C1-C6 alkyl, -NH₂, -NH-C1-C6 alkyl, or -N(C1-C6 alkyl)(C1-C6 alkyl);
each R^{AI} is independently selected from deuterium, halogen, -CN, C1-C6 alkyl, C1-C6 haloalkyl, -OH, -O-C1-C6 alkyl, -C1-C6 alkylene-O-C1-C6 alkyl, C1-C6 haloalkoxy, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

In some embodiments, A is a group selected from the A1 group, and in the A1 group, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, -CN, C1-C6 alkyl, C3-C8 cycloalkyl, C1-C6 haloalkyl, -OH, -O-C1-C6 alkyl, -NH₂, -NH-C1-C6 alkyl, or -N(C1-C6 alkyl)(C1-C6 alkyl);
each R^{AI} is independently selected from deuterium, halogen, -CN, C1-C6 alkyl, C1-C6 haloalkyl, -OH, -O-C1-C6 alkyl, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

In some embodiments, A is a group selected from the A1 group,
and in the A1 group, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, or -N(C1-C6 alkyl)(C1-C6 alkyl);
each R^{AI} is independently selected from deuterium, halogen, C1-C6 alkyl, C1-C6 haloalkyl, -OH, -O-C1-C6 alkyl, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

In some embodiments, A is a group selected from the A1 group.

In some embodiments, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, or -N(C1-C6 alkyl)(C1-C6 alkyl); preferably, each R_{A1} is independently selected from saturated 5- to 6-membered monocyclic ring, which comprises 0 or 1 N atom, which monocyclic ring is optionally substituted with 0, 1, 2 or 3 groups selected from: deuterium, halogen, C1-C6 alkyl, C1-C6 haloalkyl, -OH, -O-C1-C6 alkyl, -NH₂, - NH-C1-C6 alkyl, or -N(C1-C6 alkyl)(C1-C6 alkyl).

In some embodiments, each R^{AI} is independently selected from deuterium, halogen, C1-C6 alkyl, C1-C6 haloalkyl, -OH, -O-C1-C6 alkyl, -NH₂, -NH-C1-C6 alkyl, or -N(C1-C6 alkyl)(C1-C6 alkyl); preferably, each R^{AI} is independently selected from deuterium, halogen, C1-C6 alkyl, -OH, -O-C1-C6 alkyl, -NH₂, -NH-C1-C6 alkyl, or -N(C1-C6 alkyl)(C1-C6 alkyl).

In some embodiments, m1 is selected from 0, 1 or 2; m2 is selected from 0, 1 or 2; m3 is selected from 0, 1 or 2; m4 is selected from 0 or 1.

In some embodiments, A is a group selected from the A1 group, and in the A1 group, each R_{A1} is independently selected from saturated 5- to 6-membered monocyclic ring, which comprises 0 or 1 N atom, which monocyclic ring is optionally substituted with 0, 1, 2 or 3 groups selected from: deuterium, halogen, C1-C6 alkyl, C1-C6 haloalkyl, -OH, -O-C1-C6 alkyl, -NH₂, -NH-C1-C6 alkyl, or - N(C1-C6 alkyl)(C1-C6 alkyl); each R^{AI} is independently selected from deuterium, halogen, -CN, C1-C6 alkyl, -OH, -O-C1-C6 alkyl, -NH₂, -NH-C1-C6 alkyl, or - N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

In some embodiments, A is a group selected from the A1 group, and in the A1 group, each R_{A1} is independently selected from saturated 5- to 6-membered monocyclic ring, which comprises 0 or 1 N atom, which monocyclic ring is optionally substituted with 0, 1, 2 or 3 groups selected from: deuterium, halogen, C1-C6 alkyl, C1-C6 haloalkyl, -OH, -O-C1-C6 alkyl, -NH₂, -NH-C1-C6 alkyl, or - N(C1-C6 alkyl)(C1-C6 alkyl); each R^{AI} is independently selected from deuterium, halogen, C1-C6 alkyl, -OH, -O-C1-C6 alkyl, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

In some embodiments, A is selected from: 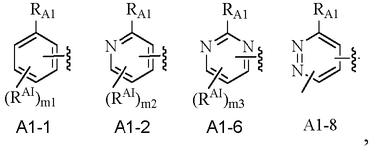 wherein the optional ranges of R_{A1}, R^{AI}, m1, and m3 are as defined above; preferably, A is selected from: 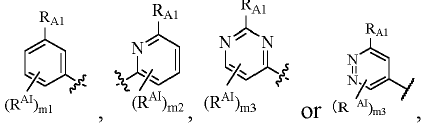 wherein the optional ranges of R_{A1}, R^{AI}, m1, and m3 are as defined above.

In some embodiments, A is selected from: 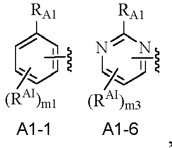 , wherein the optional ranges of R_{A1}, R^{AI}, m1, and m3 are as defined above; preferably, A is selected from: 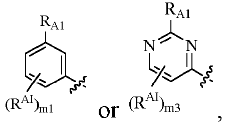 wherein the optional ranges of R_{A1}, R^{AI}, m1, and m3 are as defined above.

In some embodiments, A is a group selected from the A2 group.

In some embodiments, A is selected from: 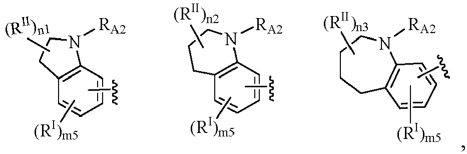 wherein the optional ranges of R^{I}, R^{II}, R_{A2}, n1, n2, n3, and m5 are as defined above; preferably, R^{I} and R^{II} are each independently selected from deuterium, halogen, C1-C6 alkyl, C1-C6 haloalkyl, - OH, C1-C6 alkoxy, -NH₂, -NH-C1-C6 alkyl, or -N(C1-C6 alkyl)(C1-C6 alkyl), and two R^{II}, together with the carbon atom to which they are attached, may form C3-C6 cycloalkyl, each R_{A2} is independently selected from hydrogen, deuterium, C1-C6 alkyl, C3-C6 cycloalkyl, or C1-C6 haloalkyl, and the C1-C6 alkyl may be optionally substituted with substituents selected from deuterium, F, Cl, Br, C1-C6 alkyl, C3-C6 cycloalkyl, or C1-C6 alkoxy; m5 is selected from 0, 1, 2 or 3, n1 is an integer selected from 0-2, n2 is an integer selected from 0-2, and n3 is an integer selected from 0-2.

In some embodiments, A is selected from: 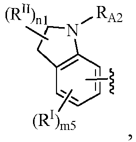 wherein the optional ranges of R^{I}, R^{II}, R_{A2}, n1, and m5 are as defined above; preferably, R^{I} and R^{II} are each independently selected from deuterium, halogen, C1-C6 alkyl, C1-C6 haloalkyl, -OH, C1-C6 alkoxy, -NH₂, -NH-C1-C6 alkyl, or -N(C1-C6 alkyl)(C1-C6 alkyl), and two R^{II}, together with the carbon atom to which they are attached, may form C3-C6 cycloalkyl, each R_{A2} is independently selected from hydrogen, deuterium, C1-C6 alkyl, C3-C6 cycloalkyl, or C1-C6 haloalkyl, and the C1-C6 alkyl may be optionally substituted with substituents selected from deuterium, F, Cl, Br, C1-C6 alkyl, C3-C6 cycloalkyl, or C1-C6 alkoxy; m5 is selected from 0, 1, 2 or 3; n1 is an integer selected from 0-2.

In some embodiments, A is selected from 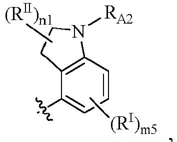 wherein the optional ranges of R^{I}, R^{II}, R_{A2}, n1, and m5 are as defined above; preferably, R^{I} and R^{II} are each independently selected from deuterium, halogen, C1-C6 alkyl, C1-C6 haloalkyl, -OH, C1-C6 alkoxy, -NH₂, -NH-C1-C6 alkyl, or -N(C1-C6 alkyl)(C1-C6 alkyl), and two R^{II}, together with the carbon atom to which they are attached, may form C3-C6 cycloalkyl, each R_{A2} is independently selected from hydrogen, deuterium, C1-C6 alkyl, C3-C6 cycloalkyl, or C1-C6 haloalkyl, and the C1-C6 alkyl may be optionally substituted with substituents selected from deuterium, F, Cl, Br, C1-C6 alkyl, C3-C6 cycloalkyl, or C1-C6 alkoxy, m5 is selected from 0, 1, 2 or 3, and n1 is an integer selected from 0-2.

In some embodiments, A is selected from 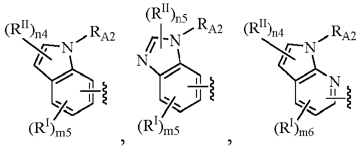 or 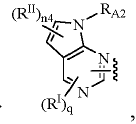 wherein the optional ranges of R^{I}, R^{II}, R_{A2}, n4, n5, m5, m6, and q are as defined above;
preferably, R^{I} and R^{II} are each independently selected from deuterium, halogen, C1-C6 alkyl, -OH, -CN, C1-C6 alkoxy, -NH₂, -NH-C1-C6 alkyl or -N(C1-C6 alkyl)(C1-C6 alkyl); each R_{A2} is independently selected from C1-C6 alkyl, C3-C6 cycloalkyl, C1-C6 haloalkyl, or C3-C6 cycloalkyl-C1-C4 alkyl, and the C1-C6 alkyl, C3-C6 cycloalkyl, C1-C6 haloalkyl, or C3-C6 cycloalkyl-C1-C4 alkyl may be optionally substituted with one or more substituents selected from deuterium, F, Cl, Br, I, -OH, C1-C6 alkyl, C3-C6 cycloalkyl, C1-C6 alkoxy or C1-C4 haloalkyl; m5 is selected from 0 or 1; m6 is selected from 0 or 1; n4 is selected from 0 or 1; n5 is selected from 0 or 1; q is selected from 0 or 1;
more preferably, each R_{A2} is independently selected from C1-C6 alkyl or C3-C6 cycloalkyl-C1-C2 alkyl, and the C1-C6 alkyl or C3-C6 cycloalkyl-C1-C2 alkyl may be optionally substituted with 1-3 substituents selected from deuterium, F, Cl, Br, -OH, C1-C4 alkyl, C3-C6 cycloalkyl, or C1-C4 haloalkyl;
further preferably, each R_{A2} is independently selected from methyl, 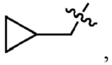 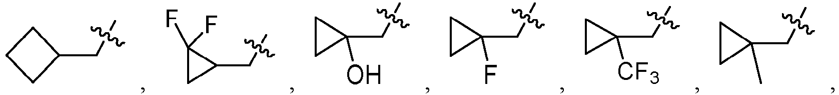 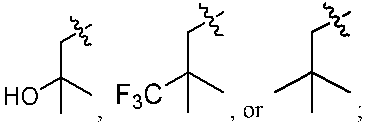
still further preferably, each R_{A2} is independently selected from

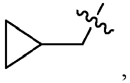

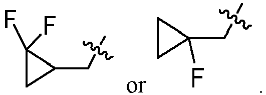

In some embodiments, A is selected from 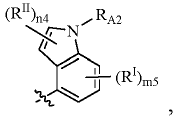 wherein the optional ranges of R^{I}, R^{II}, R_{A2}, n4, and m5 are as defined above;
preferably, R^{I} and R^{II} are each independently selected from deuterium, halogen, C1-C6 alkyl, -OH, -CN, C1-C6 alkoxy, -NH₂, -NH-C1-C6 alkyl or -N(C1-C6 alkyl)(C1-C6 alkyl); each R_{A2} is independently selected from C1-C6 alkyl, C3-C6 cycloalkyl, C1-C6 haloalkyl, or C3-C6 cycloalkyl-C1-C4 alkyl, and the C1-C6 alkyl, C3-C6 cycloalkyl, C1-C6 haloalkyl, or C3-C6 cycloalkyl-C1-C4 alkyl may be optionally substituted with one or more substituents selected from deuterium, F, Cl, Br, I, -OH, C1-C6 alkyl, C3-C6 cycloalkyl, C1-C6 alkoxy or C1-C4 haloalkyl; m5 is selected from 0 or 1; n4 is selected from 0 or 1;
more preferably, each R_{A2} is independently selected from C1-C6 alkyl or C3-C6 cycloalkyl-C1-C2 alkyl, and the C1-C6 alkyl or C3-C6 cycloalkyl-C1-C2 alkyl may be optionally substituted with 1-3 substituents selected from deuterium, F, Cl, Br, -OH, C1-C4 alkyl, C3-C6 cycloalkyl, or C1-C4 haloalkyl;
further preferably, each R_{A2} is independently selected from methyl, 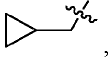 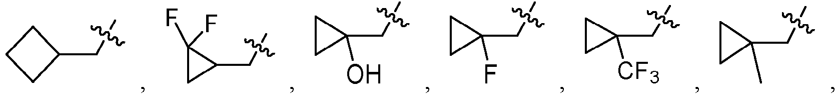 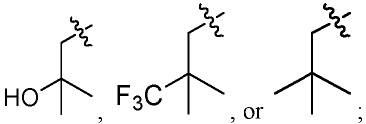
still further preferably, each R_{A2} is independently selected from

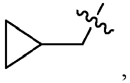

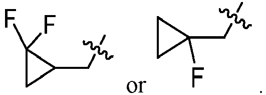

In some embodiments, A is selected from 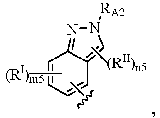 wherein the optional ranges of R^{I}, R^{II}, R_{A2}, n5, and m5 are as defined above;
preferably, R^{I} and R^{II} are each independently selected from deuterium, halogen, C1-C6 alkyl, -OH, -CN, C1-C6 alkoxy, -NH₂, -NH-C1-C6 alkyl or -N(C1-C6 alkyl)(C1-C6 alkyl); each R_{A2} is independently selected from C1-C6 alkyl, C3-C6 cycloalkyl, C1-C6 haloalkyl, C3-C6 cycloalkyl-C1-C4 alkyl, -S(O)-R_{z1} or -S(O)₂-R_{z1}, and the C1-C6 alkyl, C3-C6 cycloalkyl, C1-C6 haloalkyl, or C3-C6 cycloalkyl-C1-C4 alkyl may be optionally substituted with 1-3 substituents selected from deuterium, F, Cl, Br, I, -OH, C1-C6 alkyl, C3-C6 cycloalkyl, C1-C6 alkoxy or C1-C4 haloalkyl; each R_{z1} is independently selected from C1-C6 alkyl or C1-C6 haloalkyl; m5 is selected from 0 or 1, and n5 is selected from 0 or 1;
more preferably, each R_{A2} is independently selected from 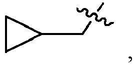 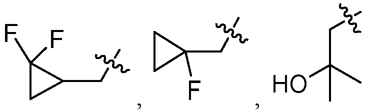 or -S(O)₂-CF₃.

In some embodiments, A is selected from 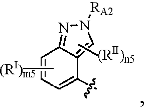 wherein the optional ranges of R^{I}, R^{II}, R_{A2}, n5, and m5 are as defined above;
preferably, R^{I} and R^{II} are each independently selected from deuterium, halogen, C1-C6 alkyl, -OH, -CN, C1-C6 alkoxy, -NH₂, -NH-C1-C6 alkyl or -N(C1-C6 alkyl)(C1-C6 alkyl); each R_{A2} is independently selected from C1-C6 alkyl, C3-C6 cycloalkyl, C1-C6 haloalkyl, C3-C6 cycloalkyl-C1-C4 alkyl, -S(O)-R_{z1} or -S(O)₂-R_{z1}, and the C1-C6 alkyl, C3-C6 cycloalkyl, C1-C6 haloalkyl, or C3-C6 cycloalkyl-C1-C4 alkyl may be optionally substituted with 1-3 substituents selected from deuterium, F, Cl, Br, I, -OH, C1-C6 alkyl, C3-C6 cycloalkyl, C1-C6 alkoxy or C1-C4 haloalkyl; each R_{z1} is independently selected from C1-C6 alkyl or C1-C6 haloalkyl; m5 is selected from 0 or 1, and n5 is selected from 0 or 1;
more preferably, each R_{A2} is independently selected from 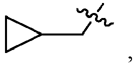 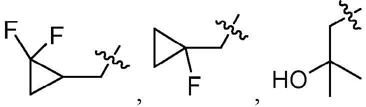 or -S(O)₂-CF₃.

In some embodiments, A is selected from 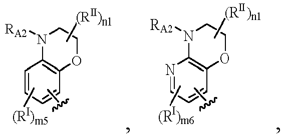 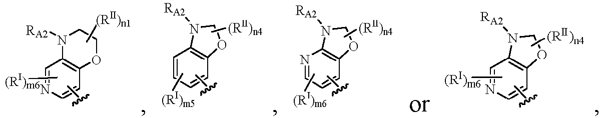 wherein the optional ranges of R^{I}, R^{II}, R_{A2}, n1, m5, and m6 are as defined above.

In some embodiments, wherein A is selected from 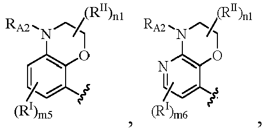 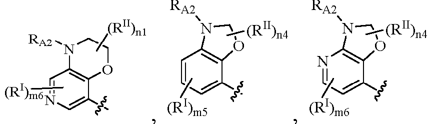 or, wherein the optional ranges of R^{I}, R^{II} R_{A2}, n1, m5, and m6 are as defined above.

In some embodiments, A is selected from 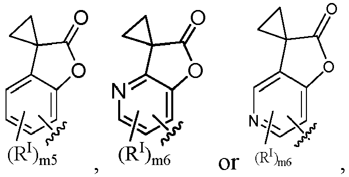 wherein the optional ranges of R^{I}, m5, and m6 are as defined above.

In some embodiments, A is selected from 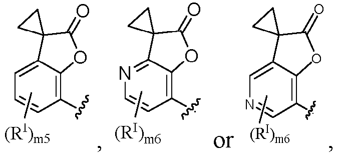 wherein the optional ranges of R^{I}, m5, and m6 are as defined above.

In some embodiments, A is a group selected from the A3 group.

In some embodiments, each G is independently selected from hydrogen, deuterium, halogen, C1-C6 alkyl, deuterated C1-C6 alkyl, C1-C6 haloalkyl, -OH, C1-C6 alkoxy, C1-C6 haloalkoxy, C1-C6 deuteroalkoxy, -NH₂, -NH-C1-C6 alkyl or -N(C1-C6 alkyl)(C1-C6 alkyl), or two G attached to the same carbon atom form an oxo group (=O).

In some embodiments, R_{A3} is independently selected from hydrogen, deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH₂, - NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl).

In some embodiments, R^{III} is independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH₂, -NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl).

In some embodiments, R_{A3} and R^{III} are each independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, - NH₂, -NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl).

In some embodiments, each G is independently selected from hydrogen, deuterium, halogen, C1-C6 alkyl, deuterated C1-C6 alkyl, C1-C6 haloalkyl, -OH, C1-C6 alkoxy, -NH₂, -NH-C1-C6 alkyl or -N(C1-C6 alkyl)(C1-C6 alkyl), or two G attached to the same carbon atom form an oxo group (=O).

In some embodiments, R_{A3} is independently selected from hydrogen, deuterium, halogen, C1-C6 alkyl, C1-C6 haloalkyl, -OH, C1-C6 alkoxy, -NH₂, -NH-C1-C6 alkyl or -N(C1-C6 alkyl)(C1-C6 alkyl), and R^{III} is independently selected from deuterium, halogen, C1-C6 alkyl, C1-C6 haloalkyl, -OH, C1-C6 alkoxy, -NH₂, -NH-C1-C6 alkyl or -N(C1-C6 alkyl)(C1-C6 alkyl).

In some embodiments, R_{A3} and R^{III} are each independently selected from deuterium, halogen, C1-C6 alkyl, C1-C6 haloalkyl, -OH, C1-C6 alkoxy, -NH₂, -NH-C1-C6 alkyl, or -N(C1-C6 alkyl)(C1-C6 alkyl).

In some embodiments, e is selected from 0, 1 or 2.

In some embodiments, f is selected from 0 or 1.

In some embodiments, A is selected from any one of the following groups:

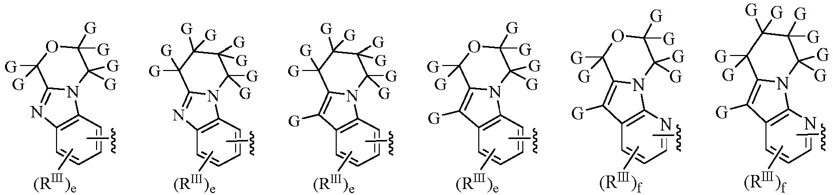

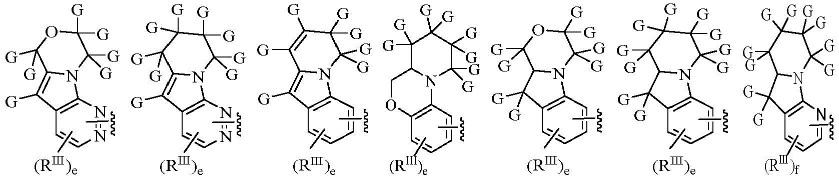

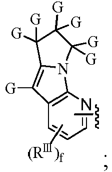

A is preferably selected from any one of the following groups:

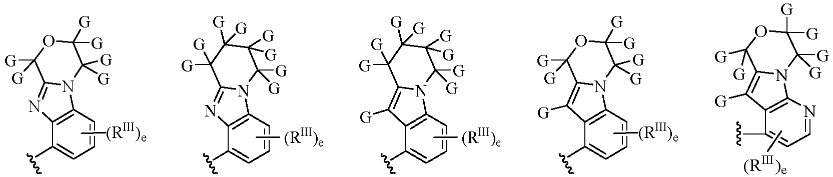

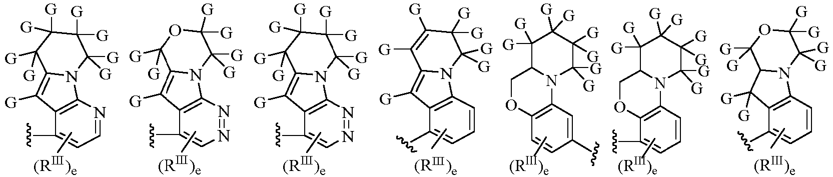

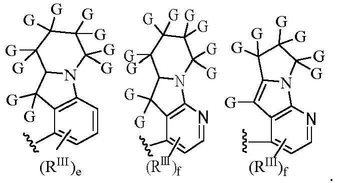

In some embodiments, A is selected from any one of the following groups:

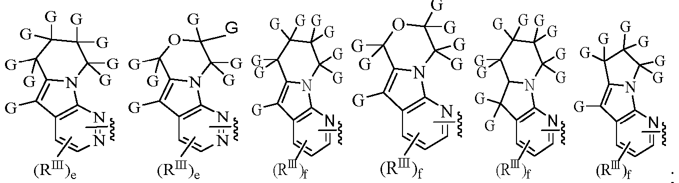

A is preferably selected from any one of the following groups:

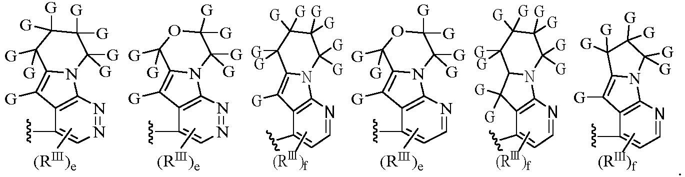

In some embodiments, A is selected from any one of the following groups:

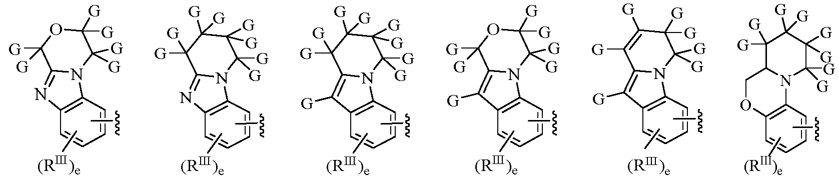

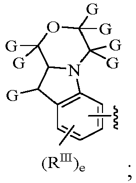

A is preferably selected from any one of the following groups:

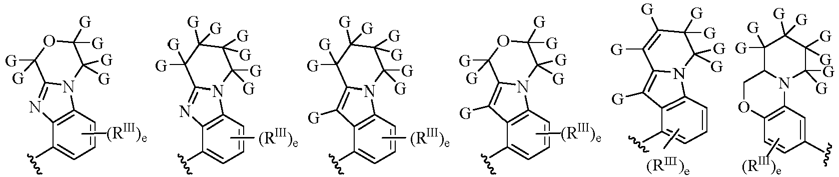

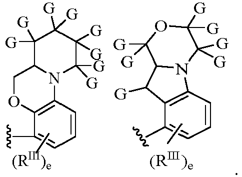

In some embodiments, A is selected from any one of the following groups: 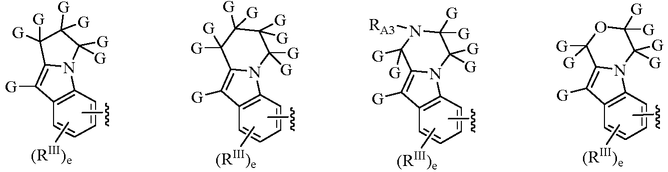 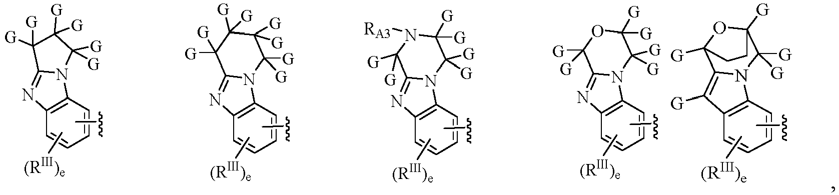 wherein the optional ranges of G, R^{III}, R_{A3}, and e are as defined above.

In some embodiments, A is selected from any one of the following groups: 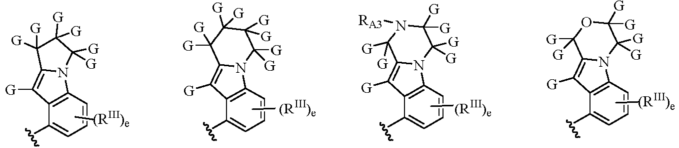 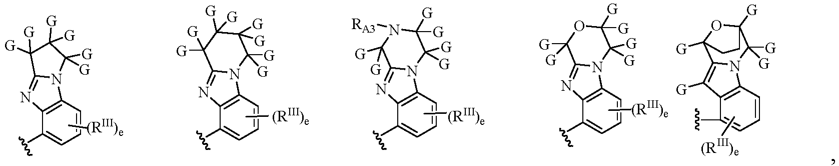 wherein the optional ranges of G, R^{III}, R_{A3}, and e are as defined above.

In some embodiments, A is selected from the following groups: 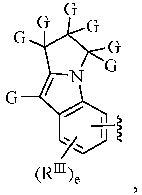 wherein the optional ranges of G, R^{III}, and e are as defined above; preferably, A is selected from

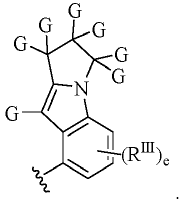

In some embodiments, A is selected from the following groups: 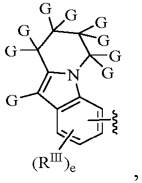 wherein the optional ranges of G, R^{III}, and e are as defined above; preferably, A is

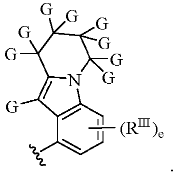

In some embodiments, A is selected from the following groups: 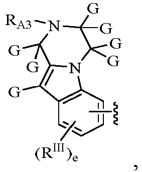 wherein the optional ranges of G, R^{III}, R_{A3}, and e are as defined above; preferably, A is

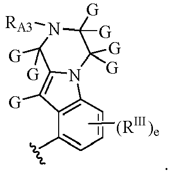

In some embodiments, each G is independently selected from hydrogen, deuterium, halogen, C1-C6 alkyl, C1-C6 haloalkyl, C1-C6 deuteroalkyl, -OH, or C1-C6 alkoxy, or two G attached to the same carbon atom form 3- to 6-membered cycloalkyl, and the C1-C6 alkyl, C1-C6 haloalkyl, C1-C6 alkoxy, or 3- to 6-membered cycloalkyl may be optionally substituted with 1-2 substituents selected from deuterium, F, Cl, or Br;
each R^{III} is independently selected from deuterium, halogen, -CN, C1-C6 alkyl, C1-C6 alkoxy, C1-C6 haloalkyl, C1-C6 deuteroalkyl, -OH, -O-C3-C8 cycloalkyl, C1-C6 haloalkoxy, C1-C6 deuteroalkoxy, -NH₂, -NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
e is selected from 0, 1 or 2; f is selected from 0 or 1.

In some embodiments, each G is independently selected from hydrogen, deuterium, halogen, C1-C6 alkyl, C1-C6 haloalkyl, -OH, or C1-C6 alkoxy, and the C1-C6 alkyl, C1-C6 haloalkyl, or C1-C6 alkoxy may be optionally substituted with 1-2 substituents selected from deuterium, F, Cl, or Br;
each R^{III} is independently selected from deuterium, halogen, -CN, C1-C6 alkyl or C1-C6 alkoxy;
e is selected from 0, 1 or 2; f is selected from 0 or 1.

In some embodiments, A is selected from the following groups: 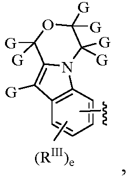 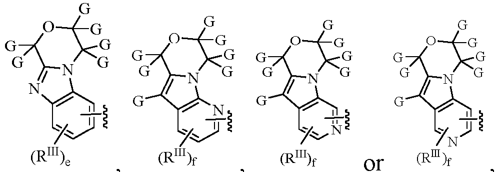 wherein the optional ranges of G, R^{III}, e, and f are as defined above; preferably, wherein each G is independently selected from hydrogen, deuterium, halogen, C1-C6 alkyl, C1-C6 haloalkyl, -OH, or C1-C6 alkoxy, and the C1-C6 alkyl, C1-C6 haloalkyl, or C1-C6 alkoxy may be optionally substituted with 1-2 substituents selected from deuterium, F, Cl, or Br, each R^{III} is independently selected from deuterium, halogen, -CN, C1-C6 alkyl, or C1-C6 alkoxy, e is selected from 0, 1 or 2, and f is selected from 0 or 1.

In some embodiments, A is selected from the following groups: 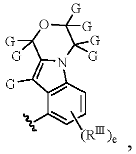 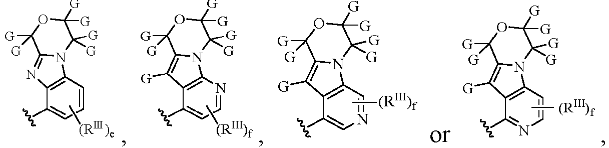 wherein the optional ranges of G, R^{III}, e, and f are as defined above; preferably, wherein each G is independently selected from hydrogen, deuterium, halogen, C1-C6 alkyl, C1-C6 haloalkyl, -OH, or C1-C6 alkoxy, and the C1-C6 alkyl, C1-C6 haloalkyl, or C1-C6 alkoxy may be optionally substituted with 1-2 substituents selected from deuterium, F, Cl, or Br, each R^{III} is independently selected from deuterium, halogen, -CN, C1-C6 alkyl, or C1-C6 alkoxy, e is selected from 0, 1 or 2, and f is selected from 0 or 1.

In some embodiments, A is selected from the following groups: 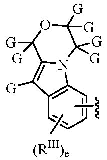 or 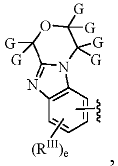 wherein the optional ranges of G, R^{III}, and e are as defined above; preferably, wherein each G is independently selected from hydrogen, deuterium, halogen, C1-C6 alkyl, C1-C6 haloalkyl, -OH, or C1-C6 alkoxy, and the C1-C6 alkyl, C1-C6 haloalkyl, or C1-C6 alkoxy may be optionally substituted with 1-2 substituents selected from deuterium, F, Cl, or Br, each R^{III} is independently selected from deuterium, halogen, -CN, C1-C6 alkyl, or C1-C6 alkoxy, e is selected from 0, 1 or 2, and f is selected from 0 or 1.

In some embodiments, A is selected from the following groups: 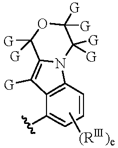 or 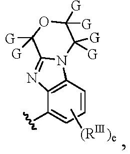 wherein the optional ranges of G, R^{III}, and e are as defined above; preferably, wherein each G is independently selected from hydrogen, deuterium, halogen, C1-C6 alkyl, C1-C6 haloalkyl, -OH, or C1-C6 alkoxy, and the C1-C6 alkyl, C1-C6 haloalkyl, or C1-C6 alkoxy may be optionally substituted with 1-2 substituents selected from deuterium, F, Cl, or Br, each R^{III} is independently selected from deuterium, halogen, -CN, C1-C6 alkyl, or C1-C6 alkoxy, e is selected from 0, 1 or 2, and f is selected from 0 or 1.

In some embodiments, A is selected from the following groups: 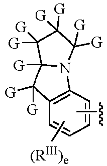 or 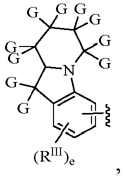 wherein the optional ranges of G, R^{III}, and e are as defined above.

In some embodiments, A is selected from the following groups: 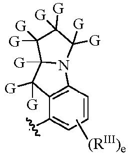 or 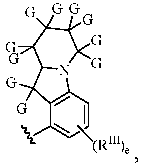 wherein the optional ranges of G, R^{III}, and e are as defined above.

In some embodiments, A is selected from the following groups: 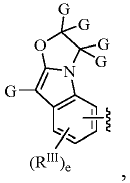 wherein the optional ranges of G, R^{III}, and e are as defined above.

In some embodiments, A is selected from the following groups: 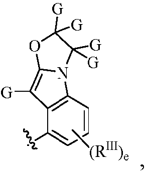 wherein the optional ranges of G, R^{III}, and e are as defined above.

In some embodiments, A is selected from the following groups: 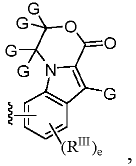 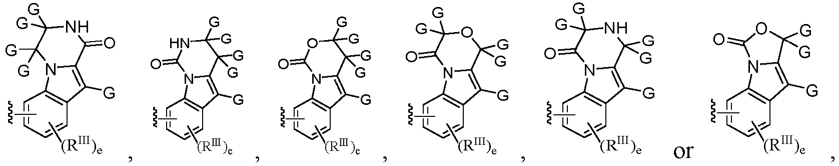 wherein the optional ranges of G, R^{III}, and e are as defined above.

In some embodiments, A is selected from the following groups: 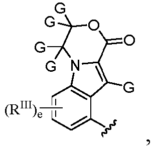 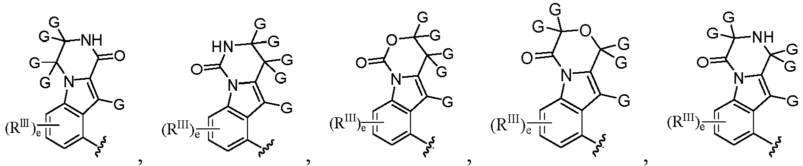 or 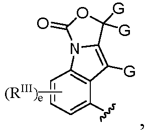 wherein the optional ranges of G, RIII, and e are as defined above.

In some embodiments, A is selected from the following groups: 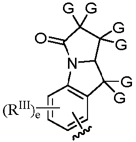 or 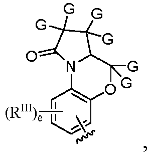 wherein the optional ranges of G, R^{III}, and e are as defined above.

In some embodiments, A is selected from the following groups: 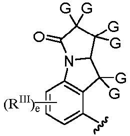 or 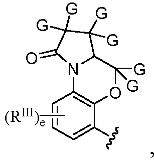 wherein the optional ranges of G, R^{III}, and e are as defined above.

In some embodiments, A is selected from the following groups: 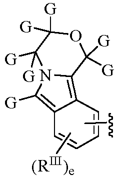 or 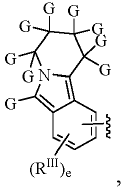 wherein the optional ranges of G, R^{III}, and e are as defined above.

In some embodiments, A is selected from the following groups: 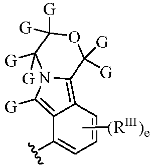 or 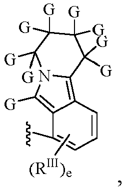 wherein the optional ranges of G, R^{III}, and e are as defined above.

In some embodiments, A is selected from any one of the following groups:

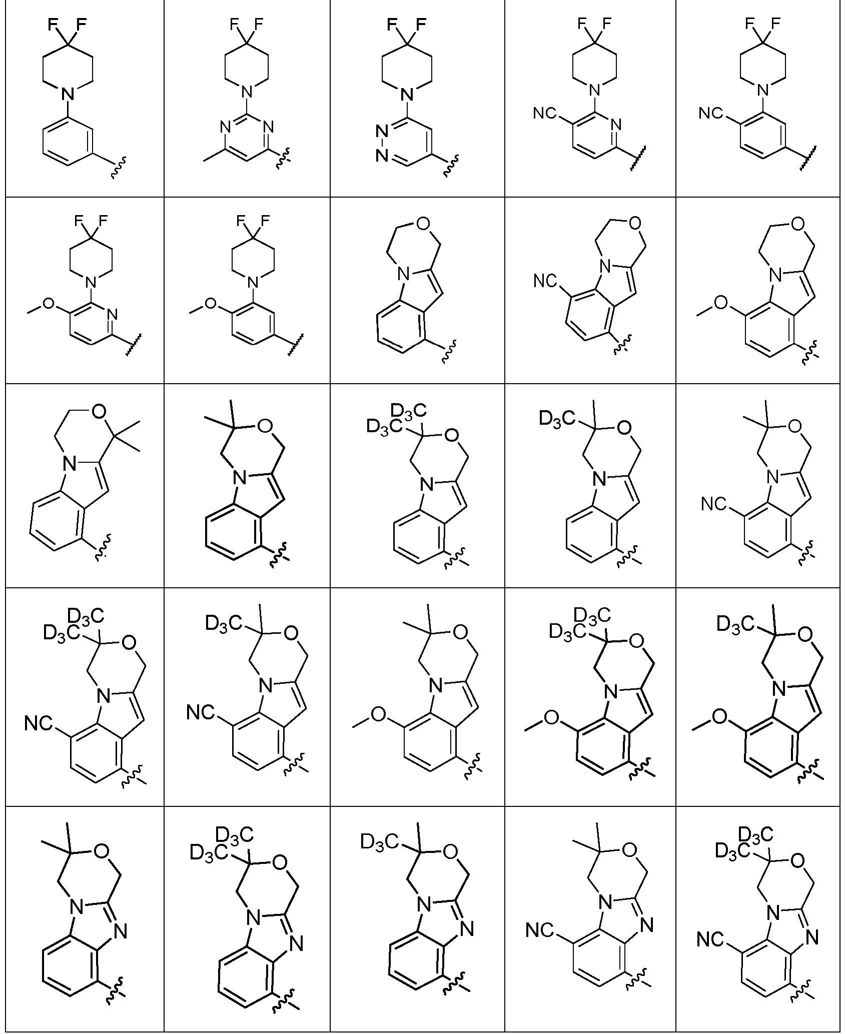

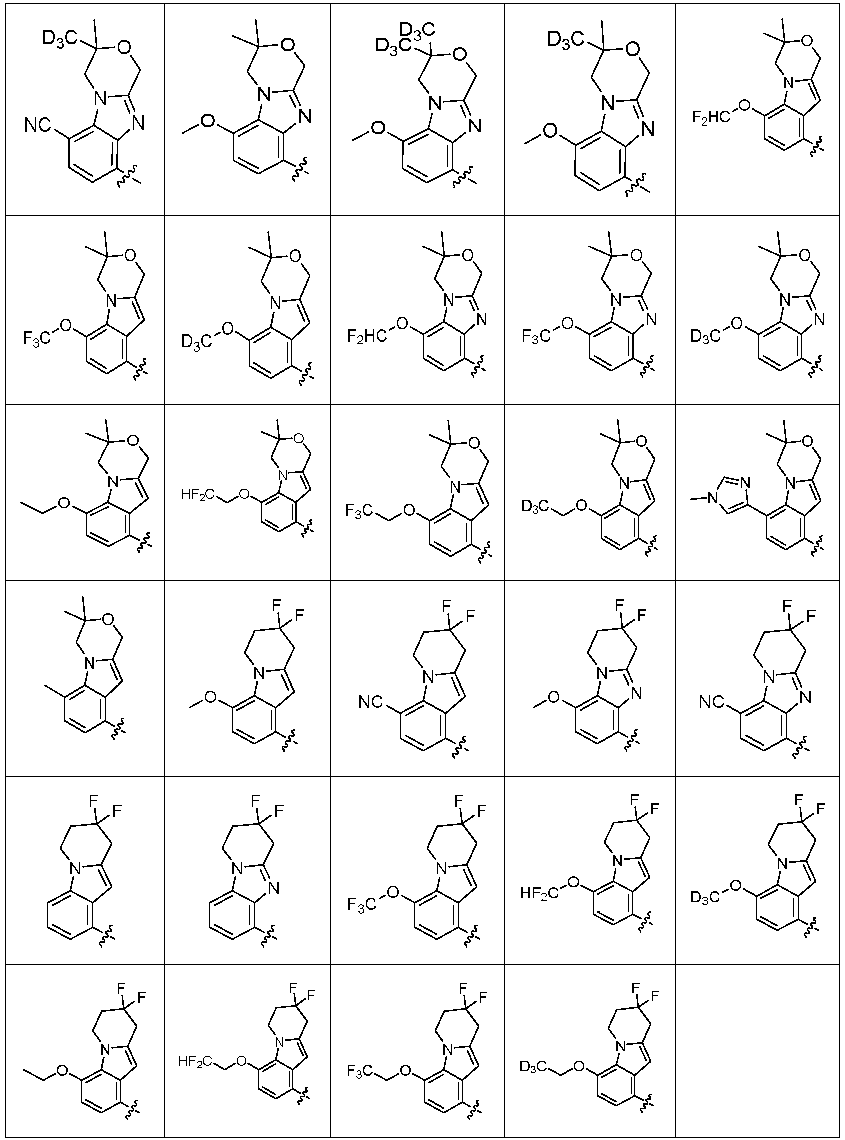

In some embodiments, A is selected from any one of the following groups:

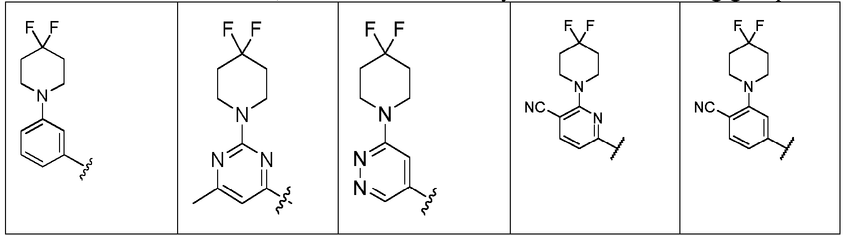

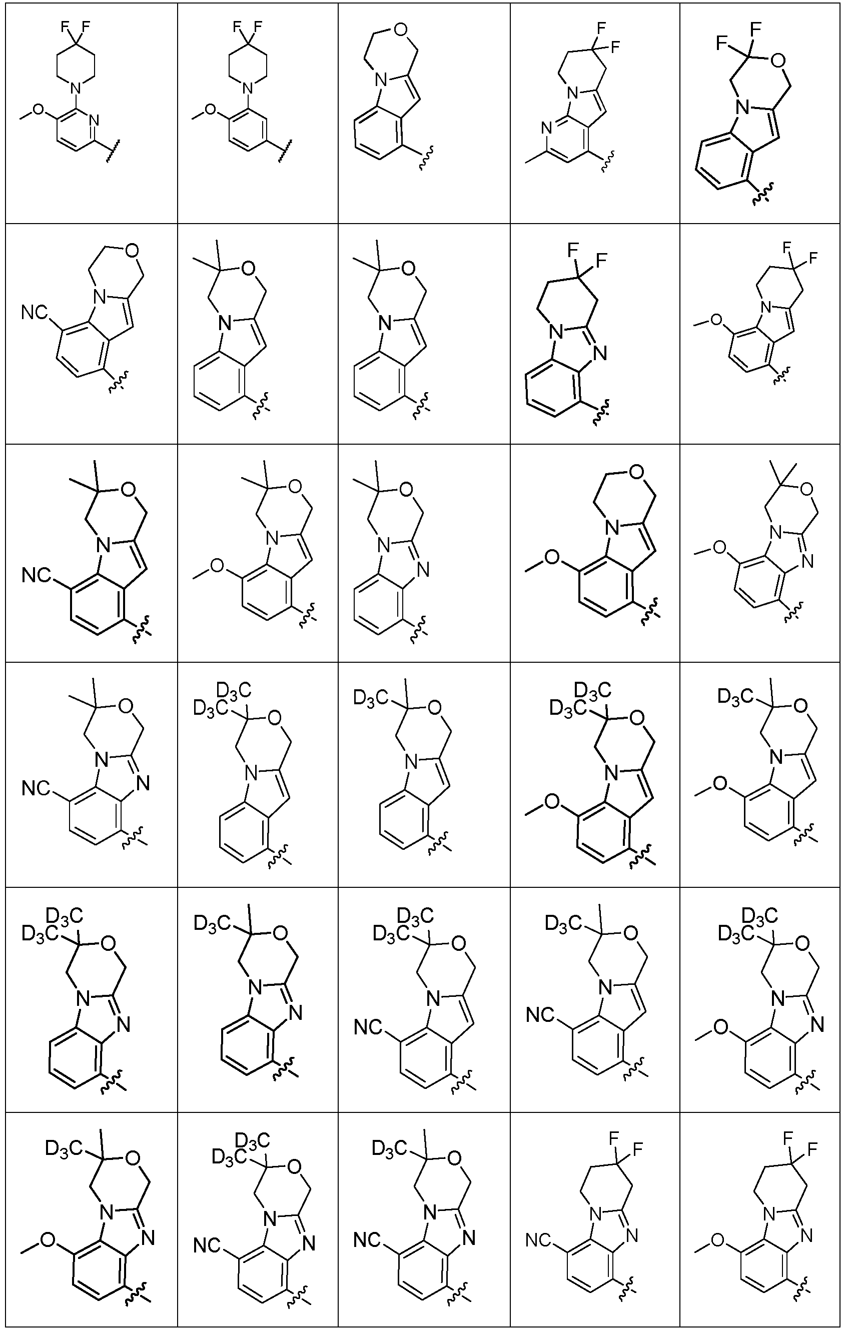

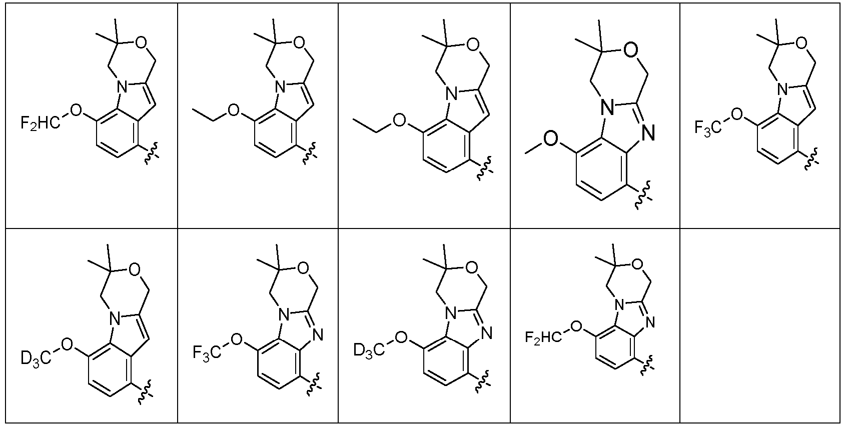

In some embodiments, A is selected from any one of the following groups:

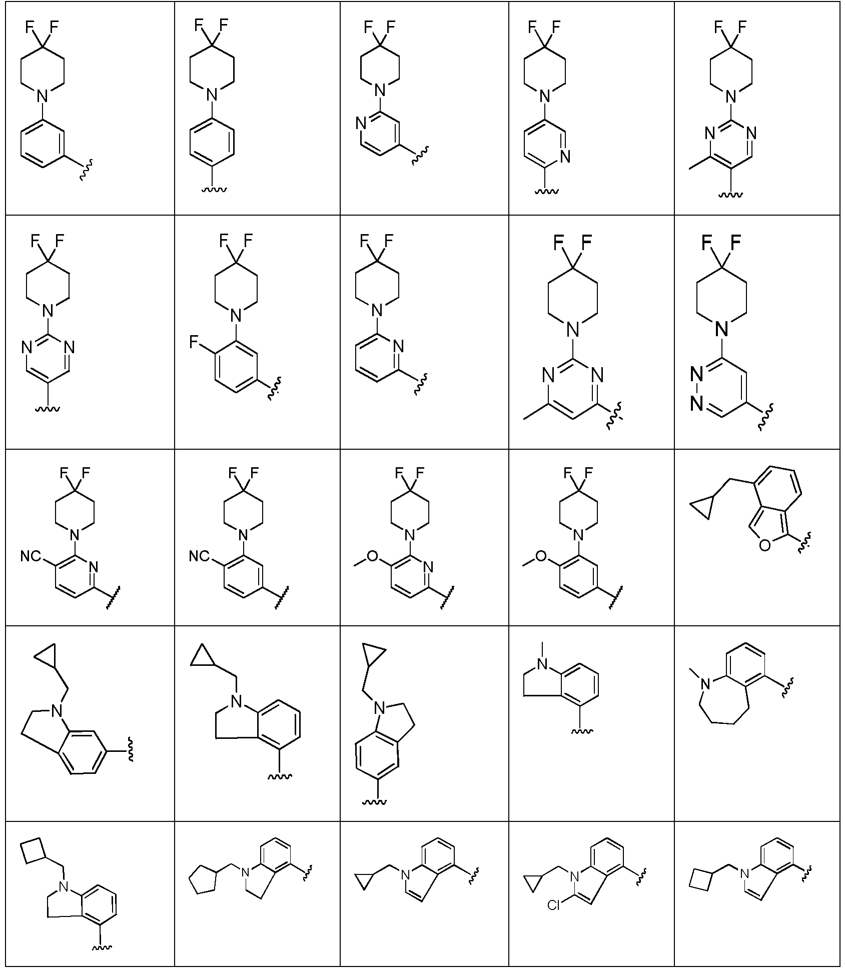

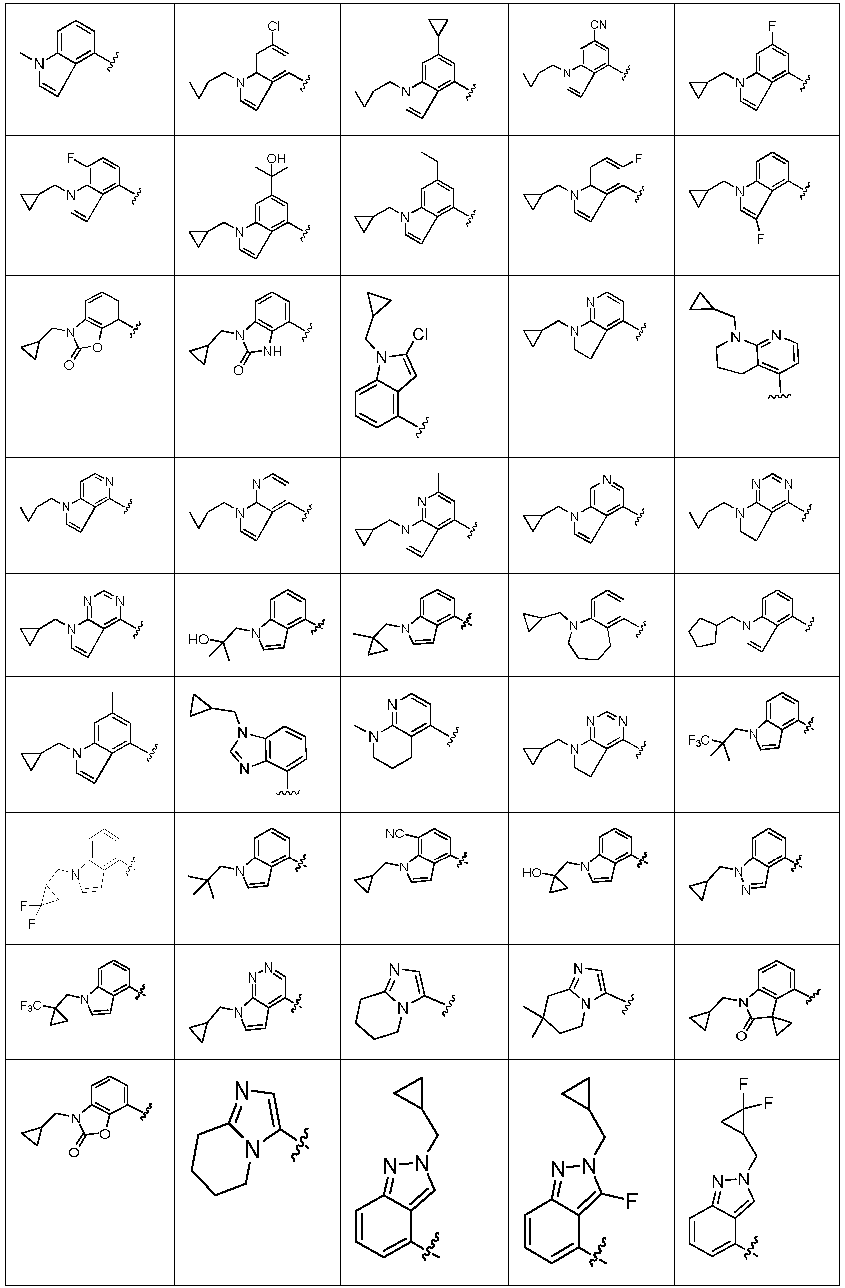

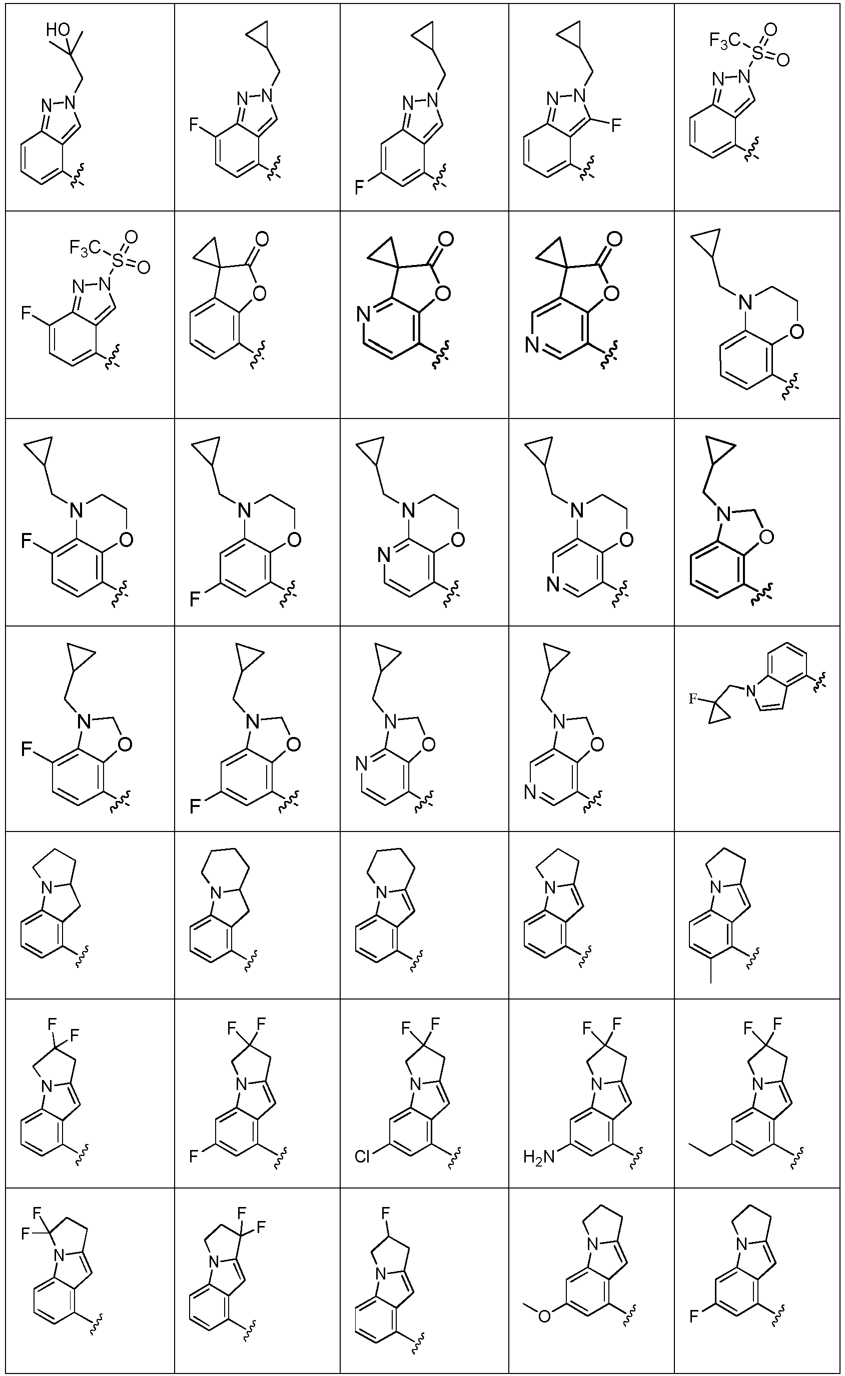

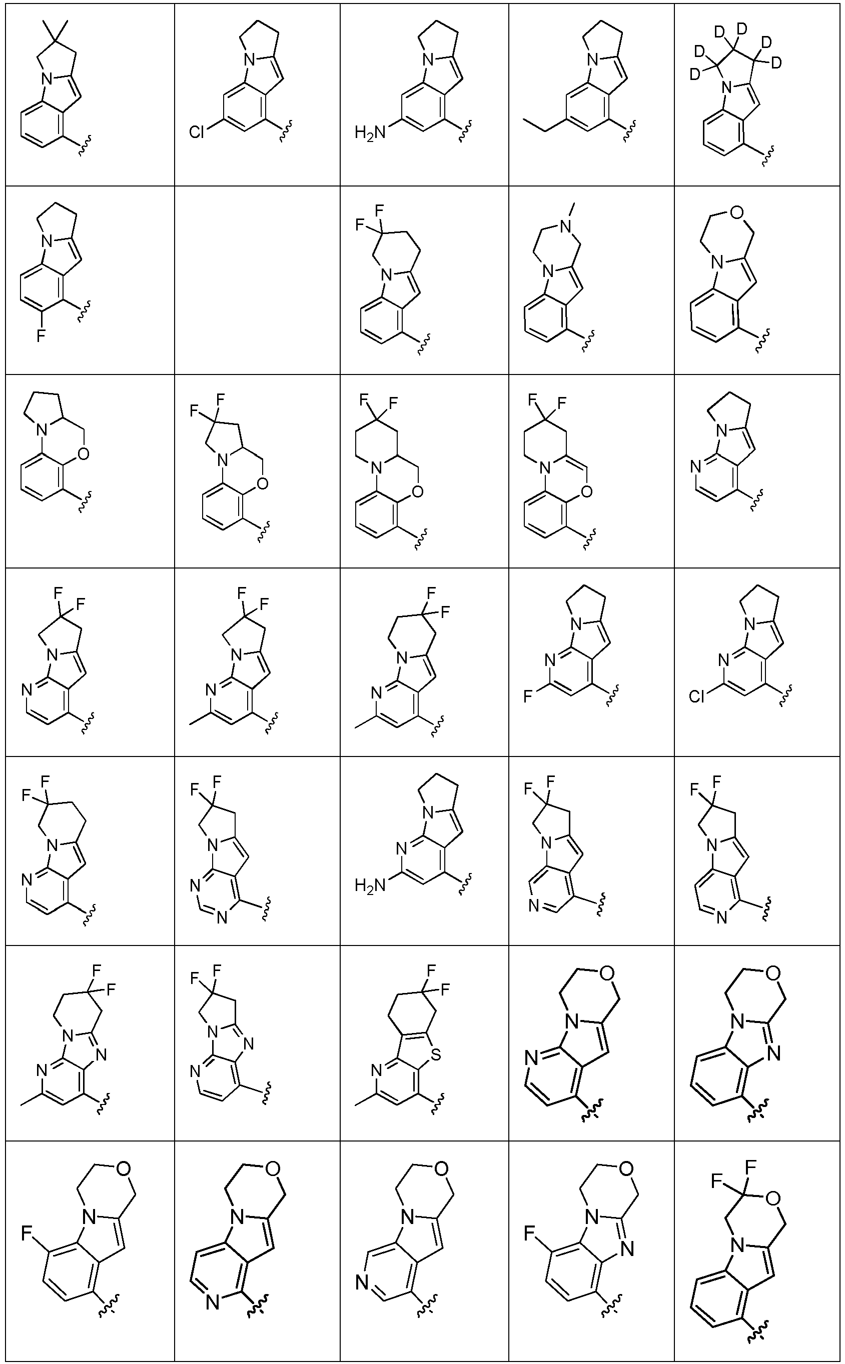

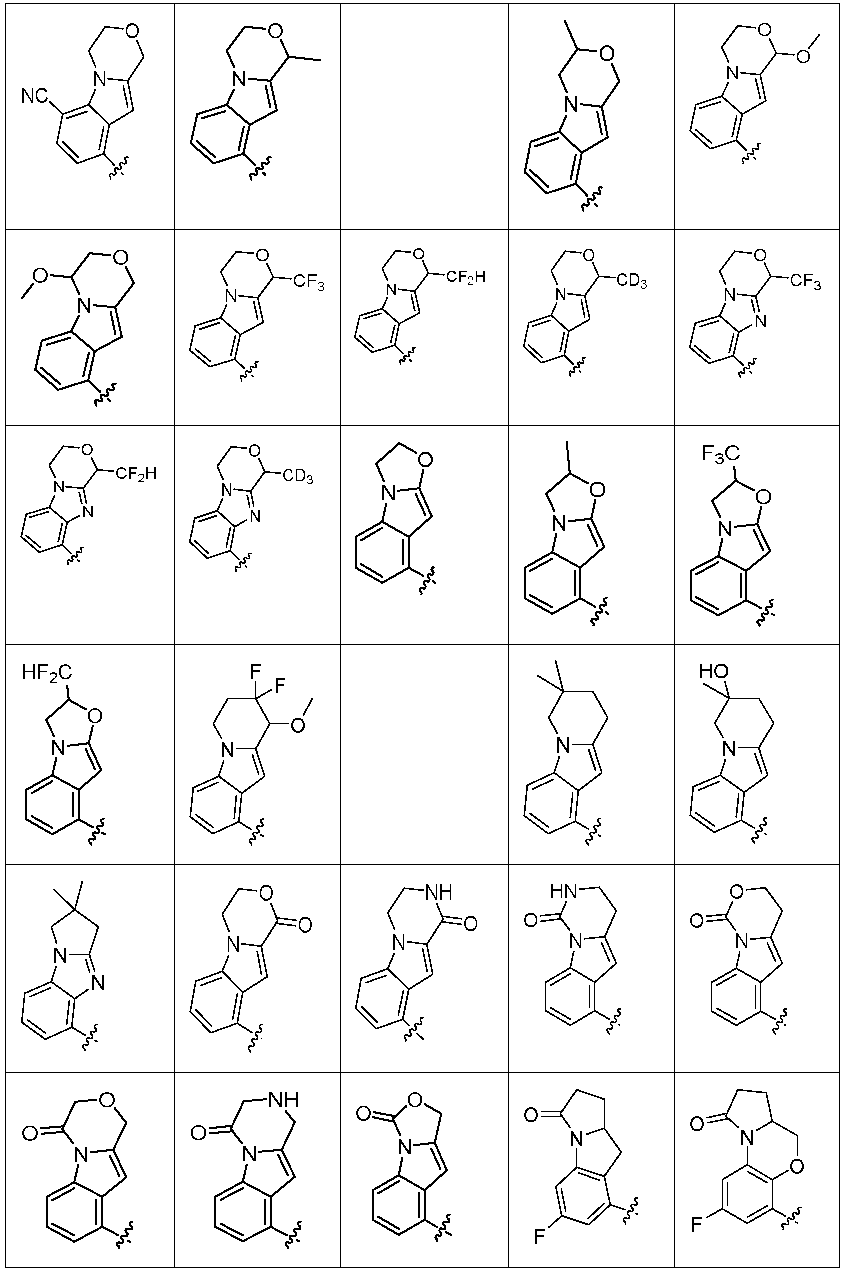

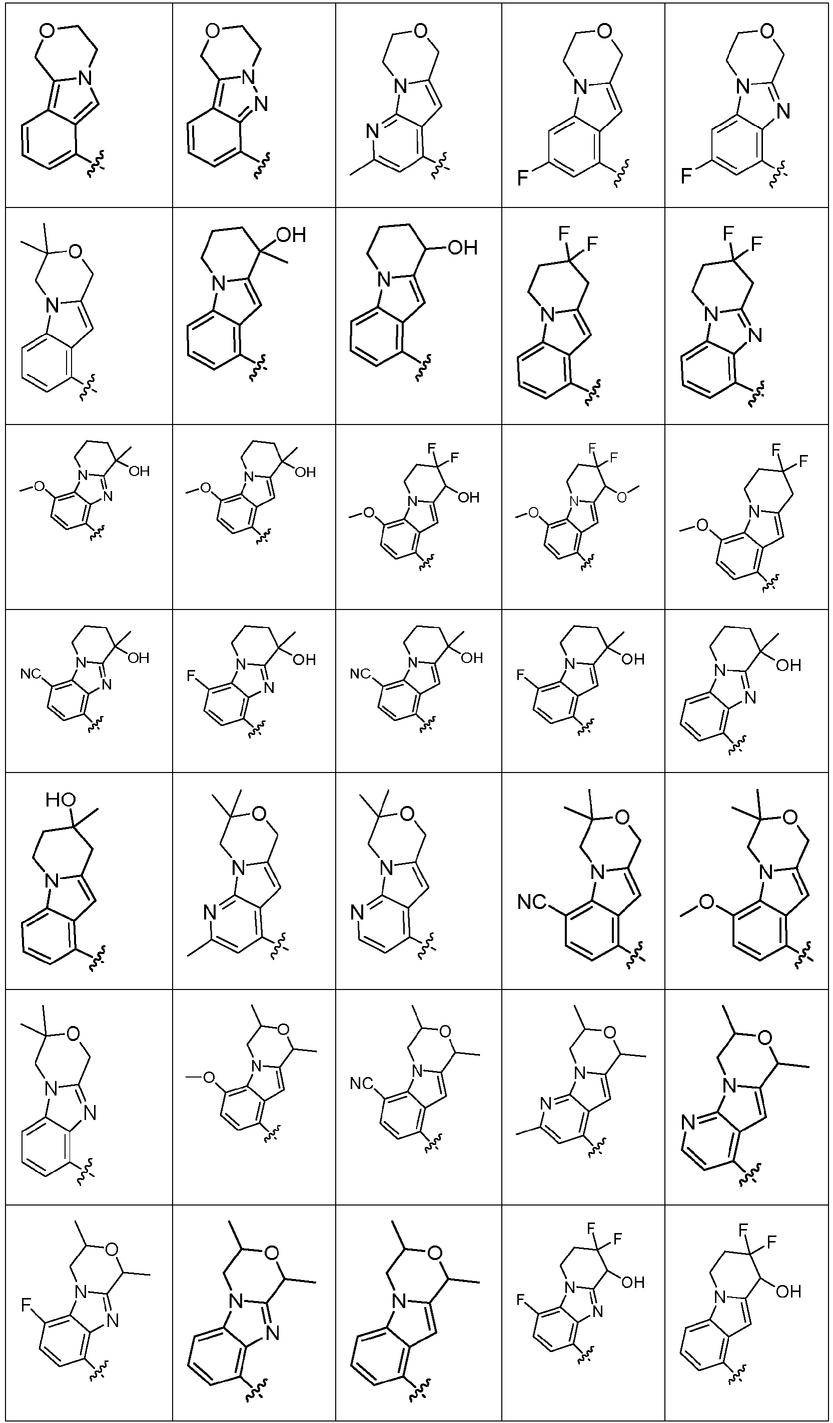

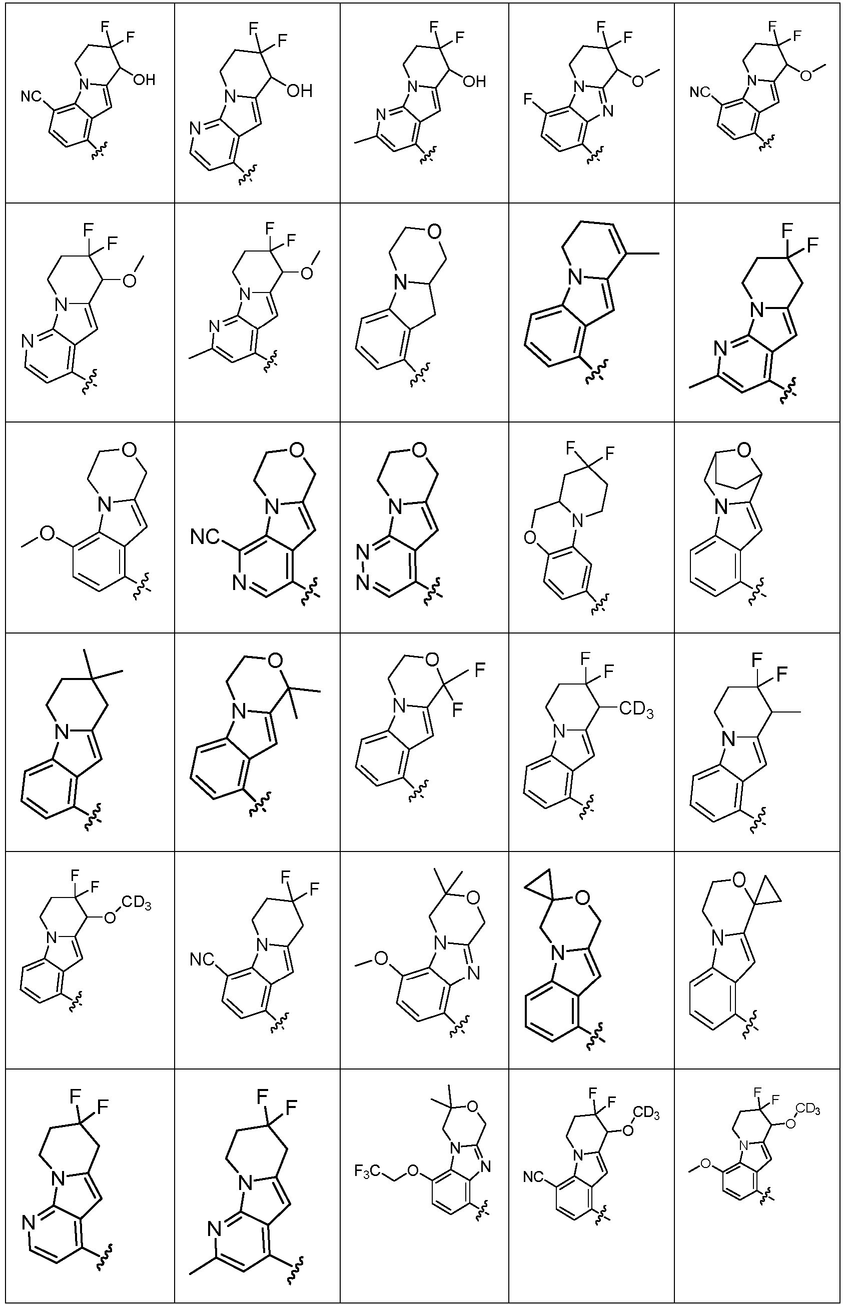

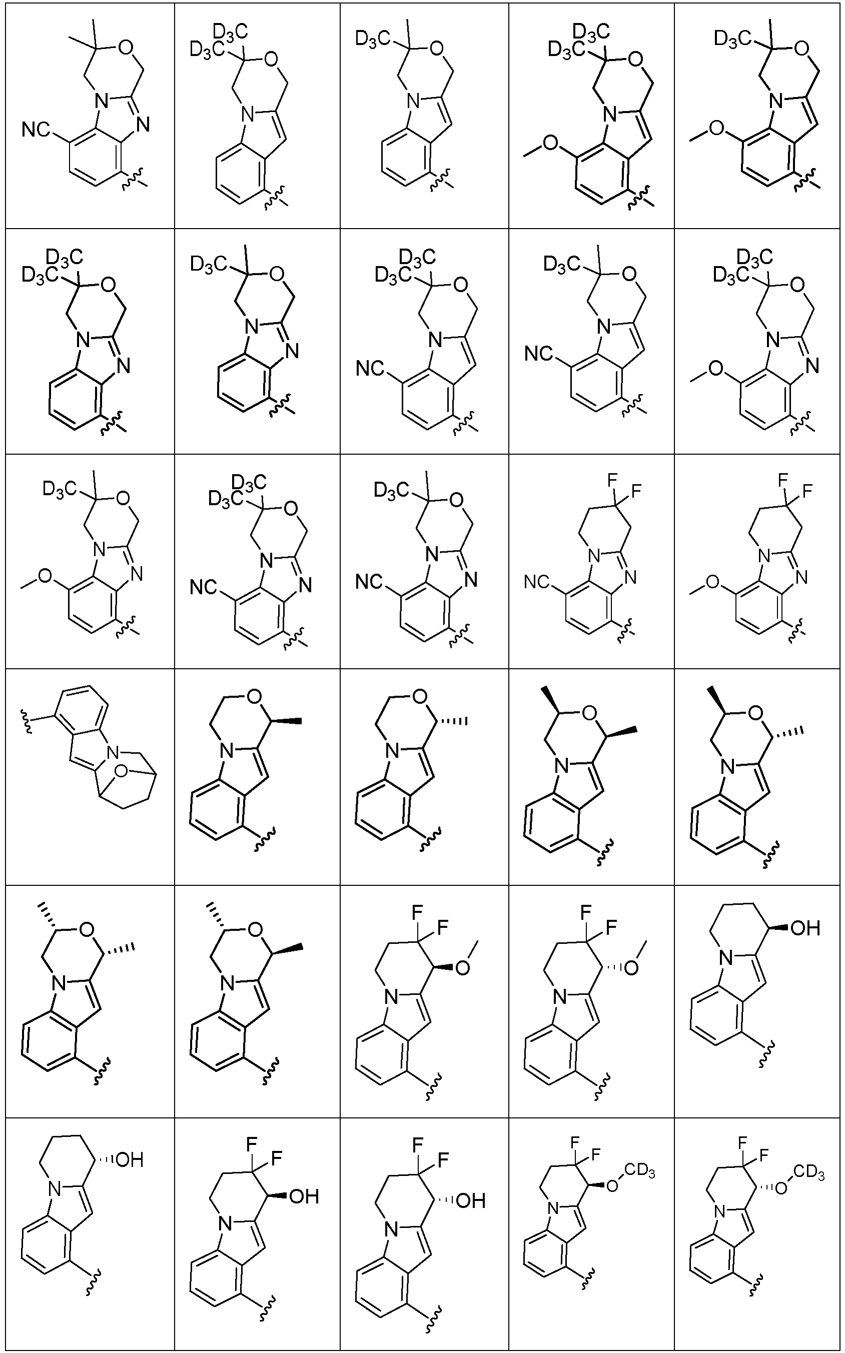

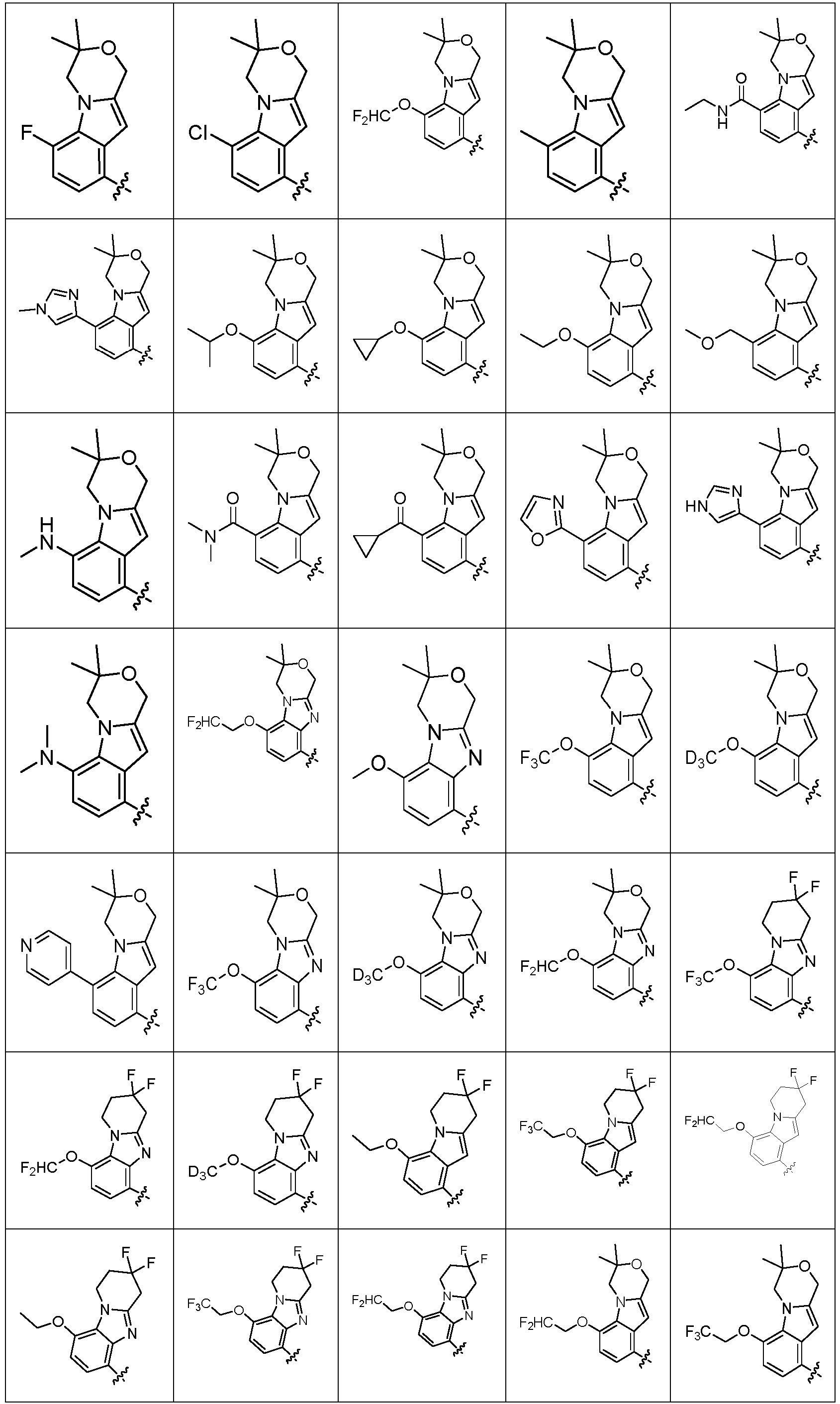

In some embodiments, A is selected from any one of the following groups:

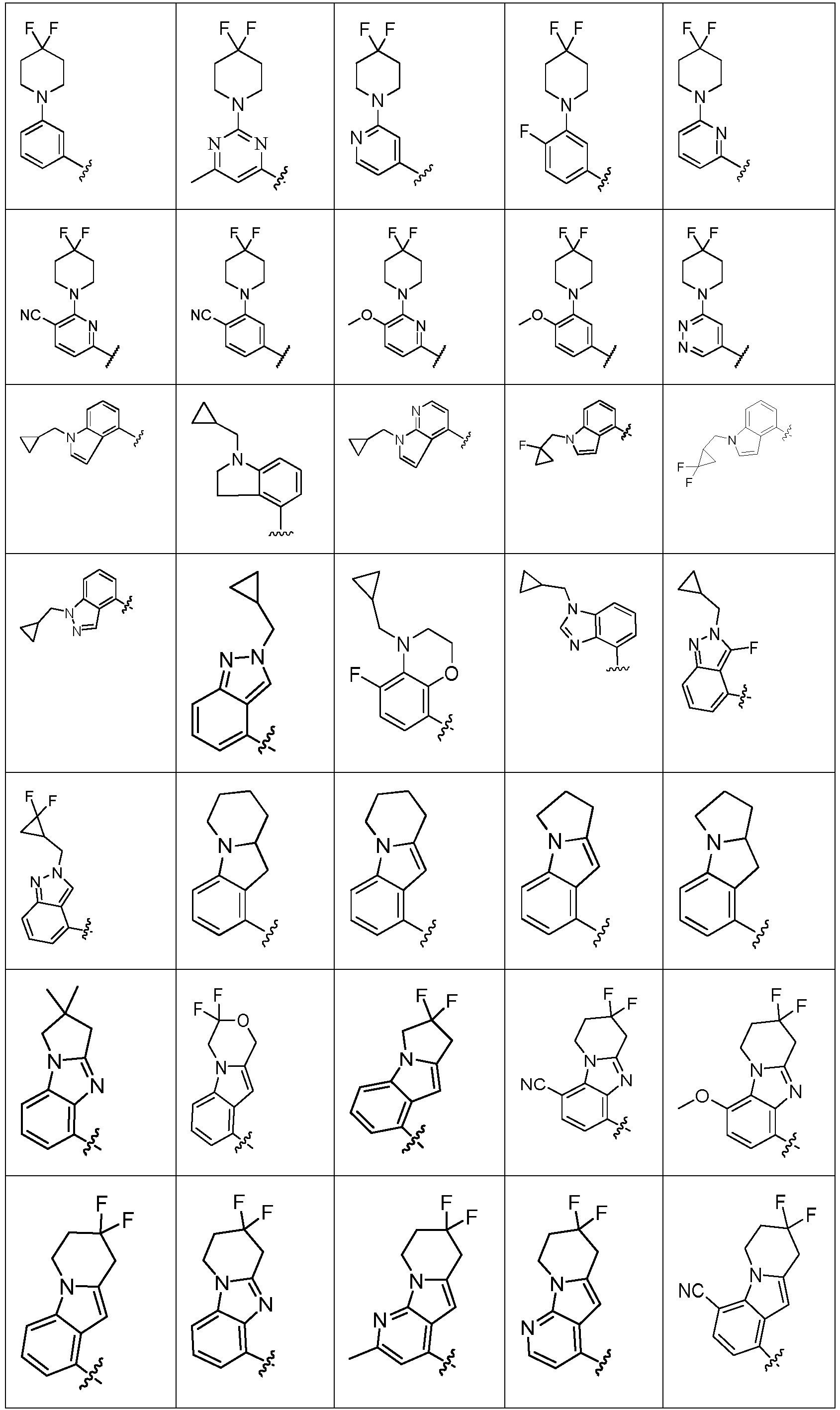

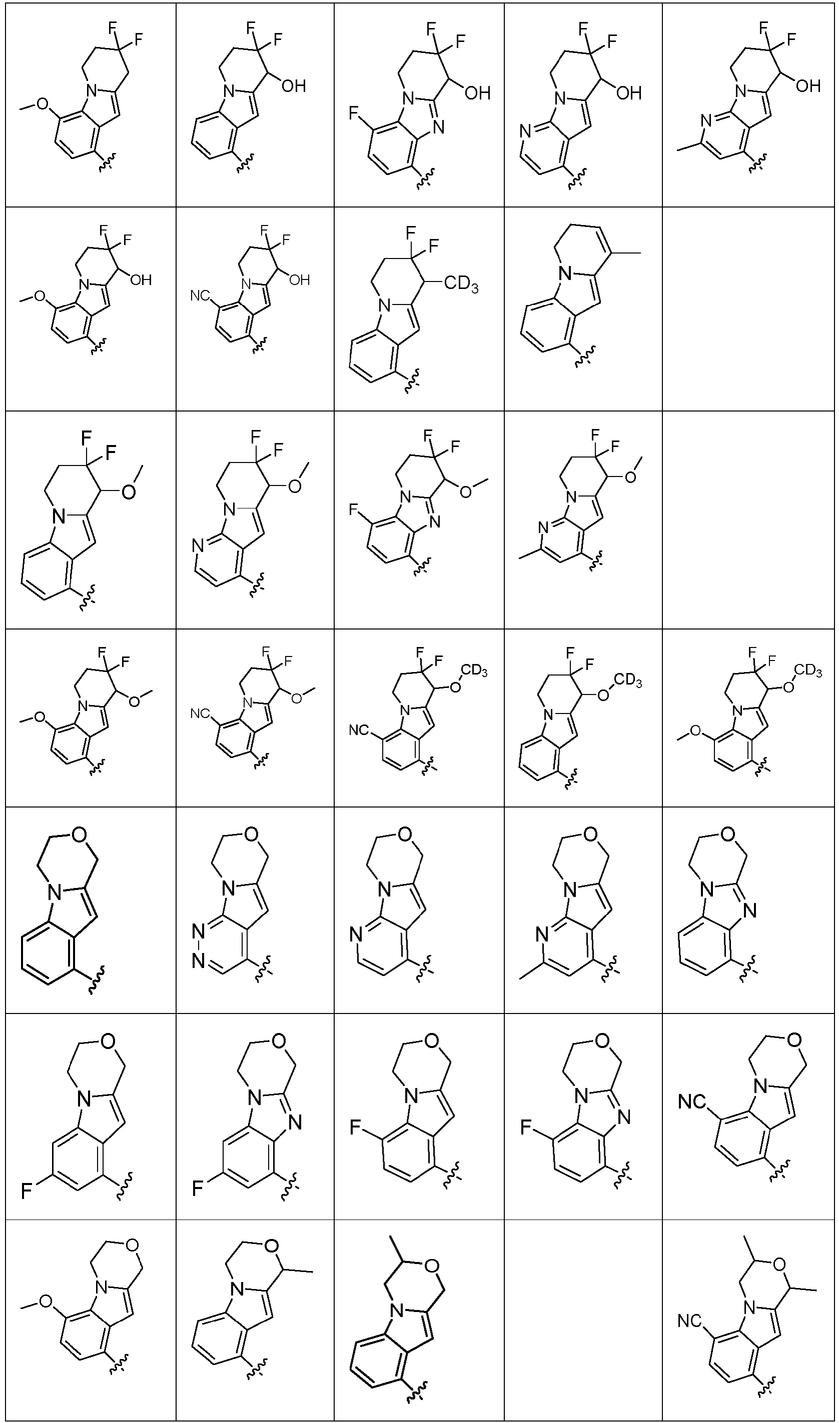

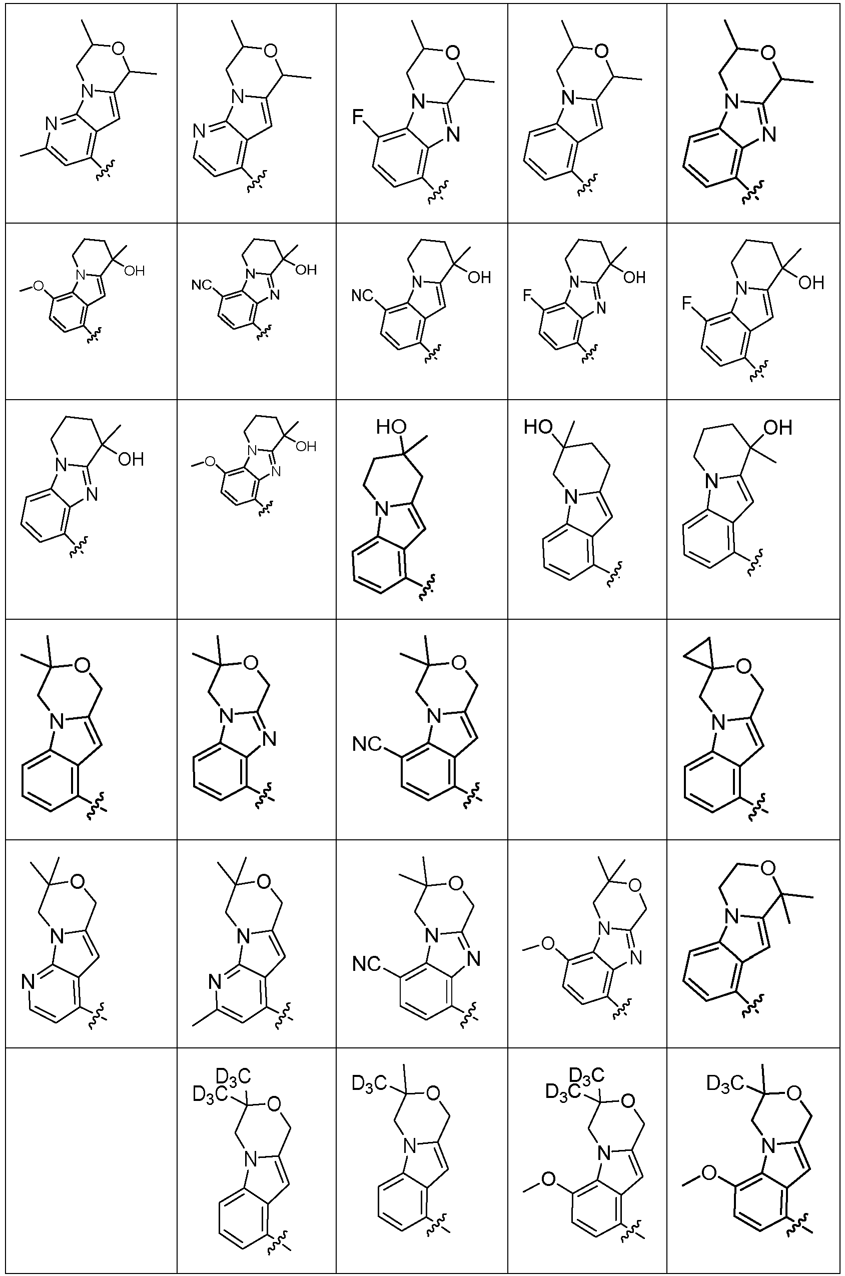

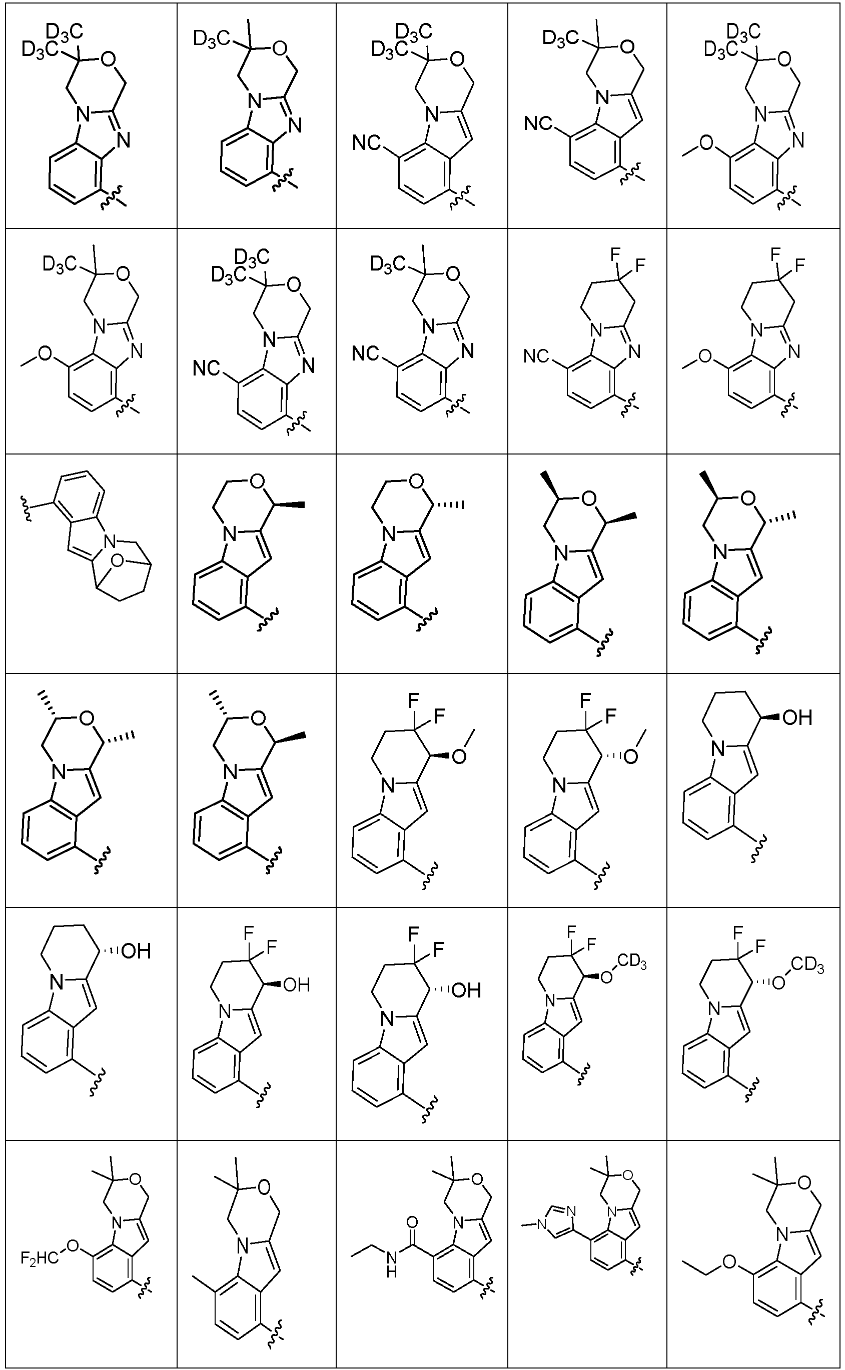

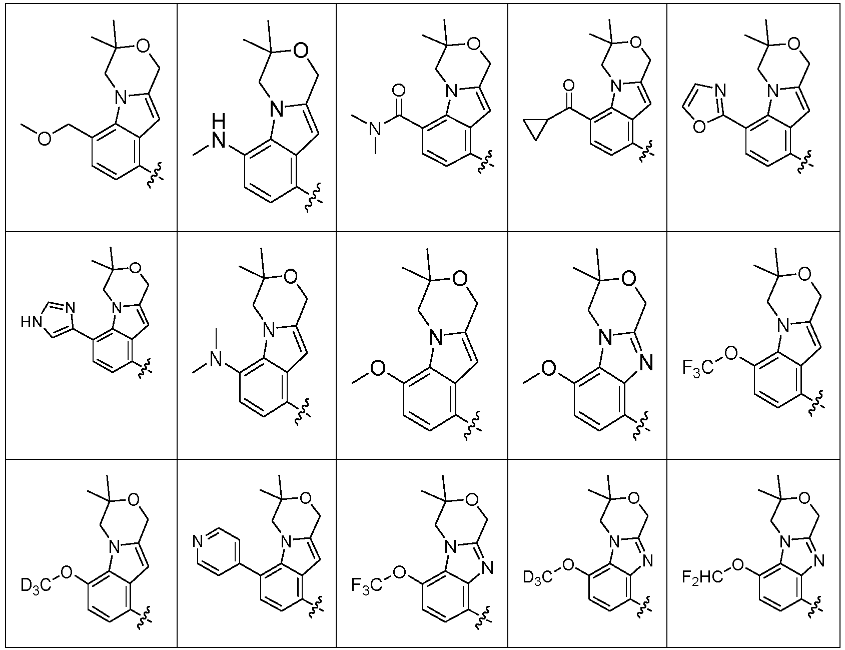

In some embodiments, A is selected from any one of the following groups:

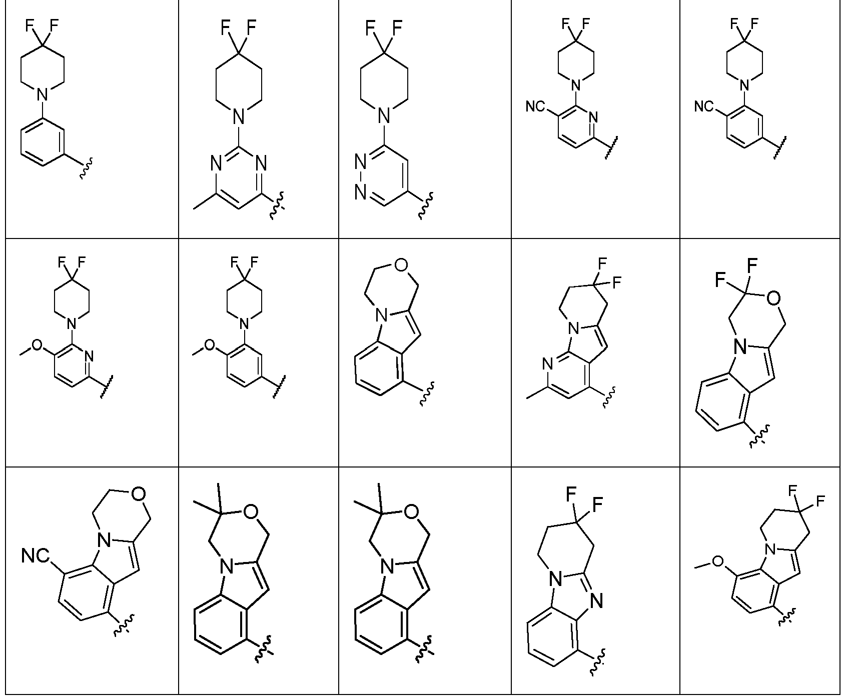

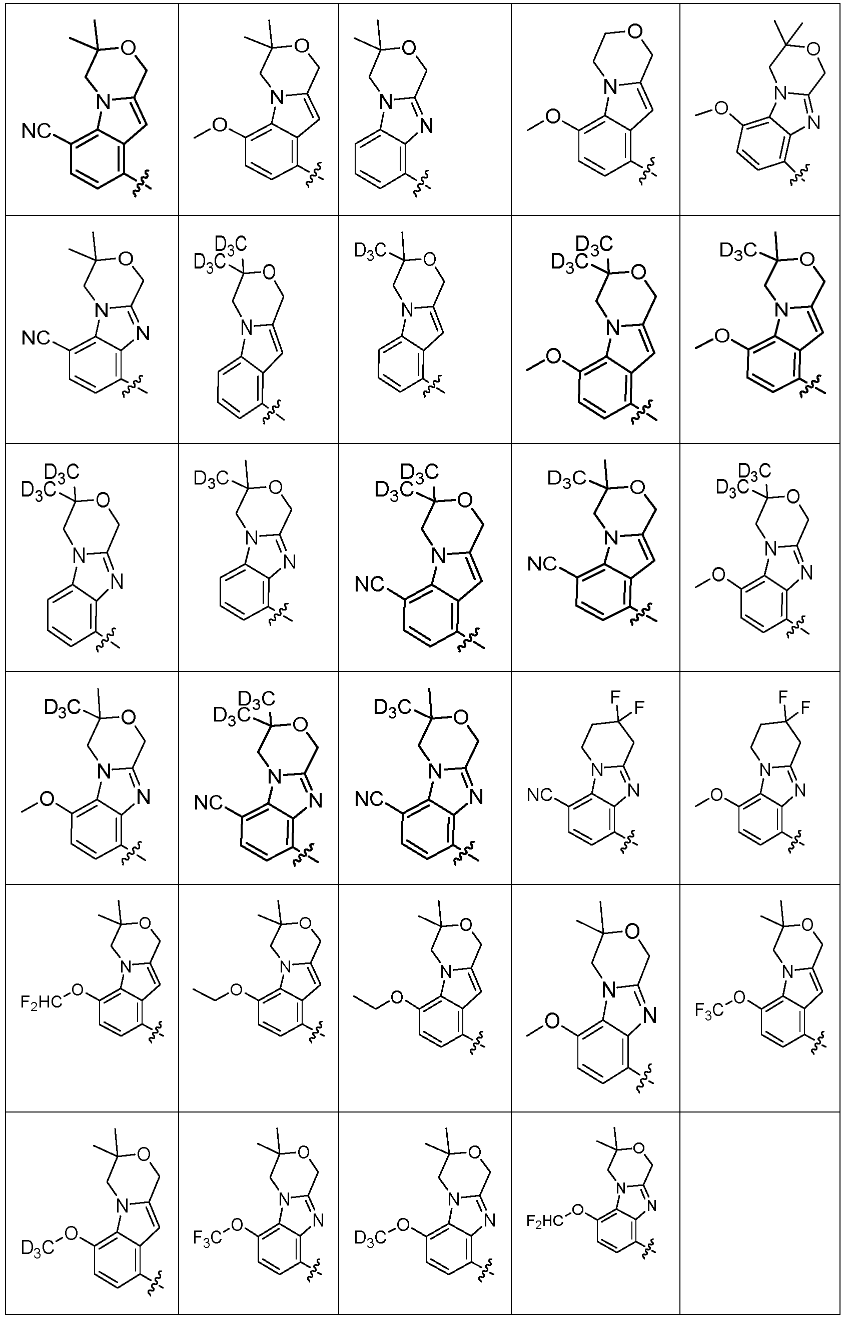

In some embodiments, A is selected from any one of the following groups:

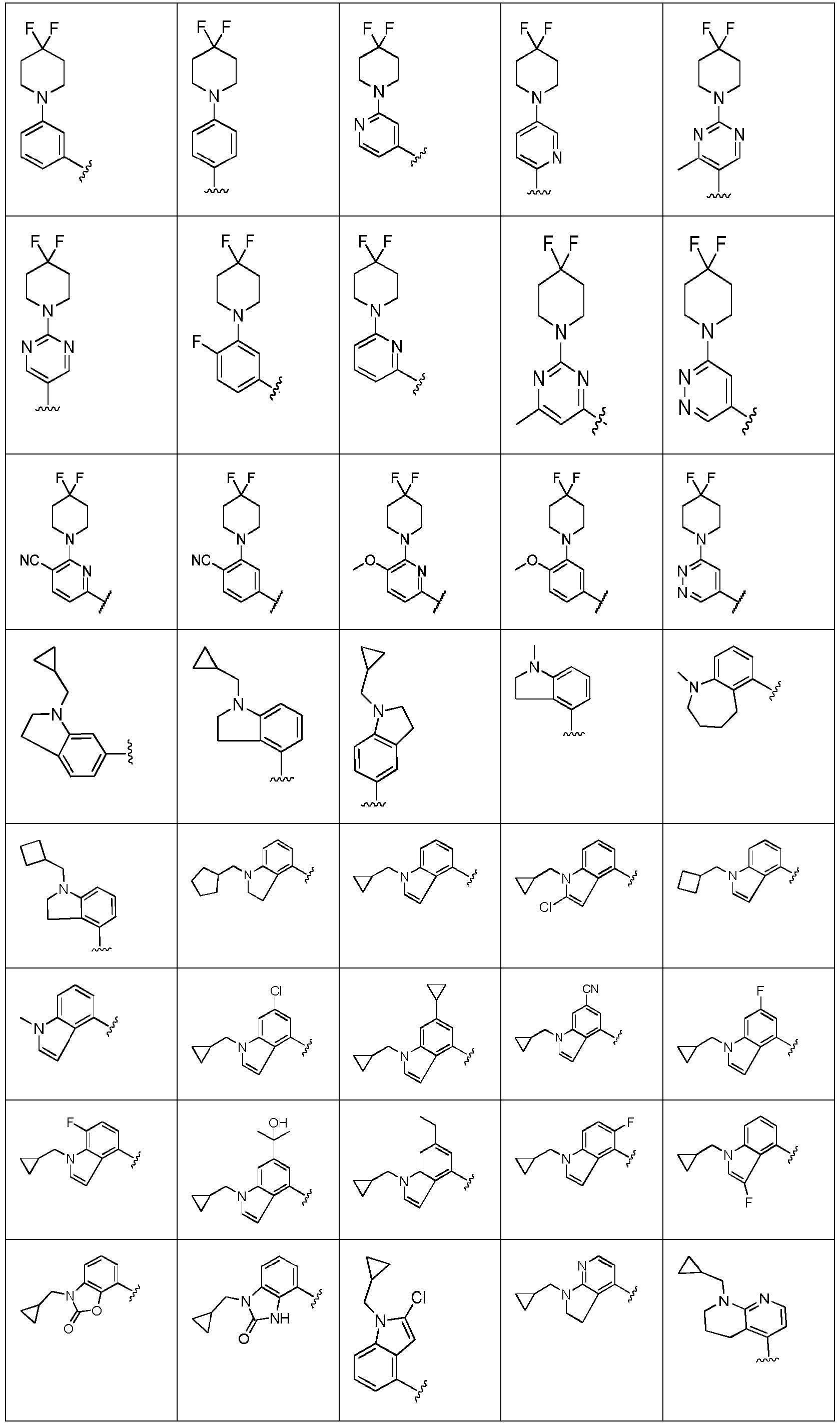

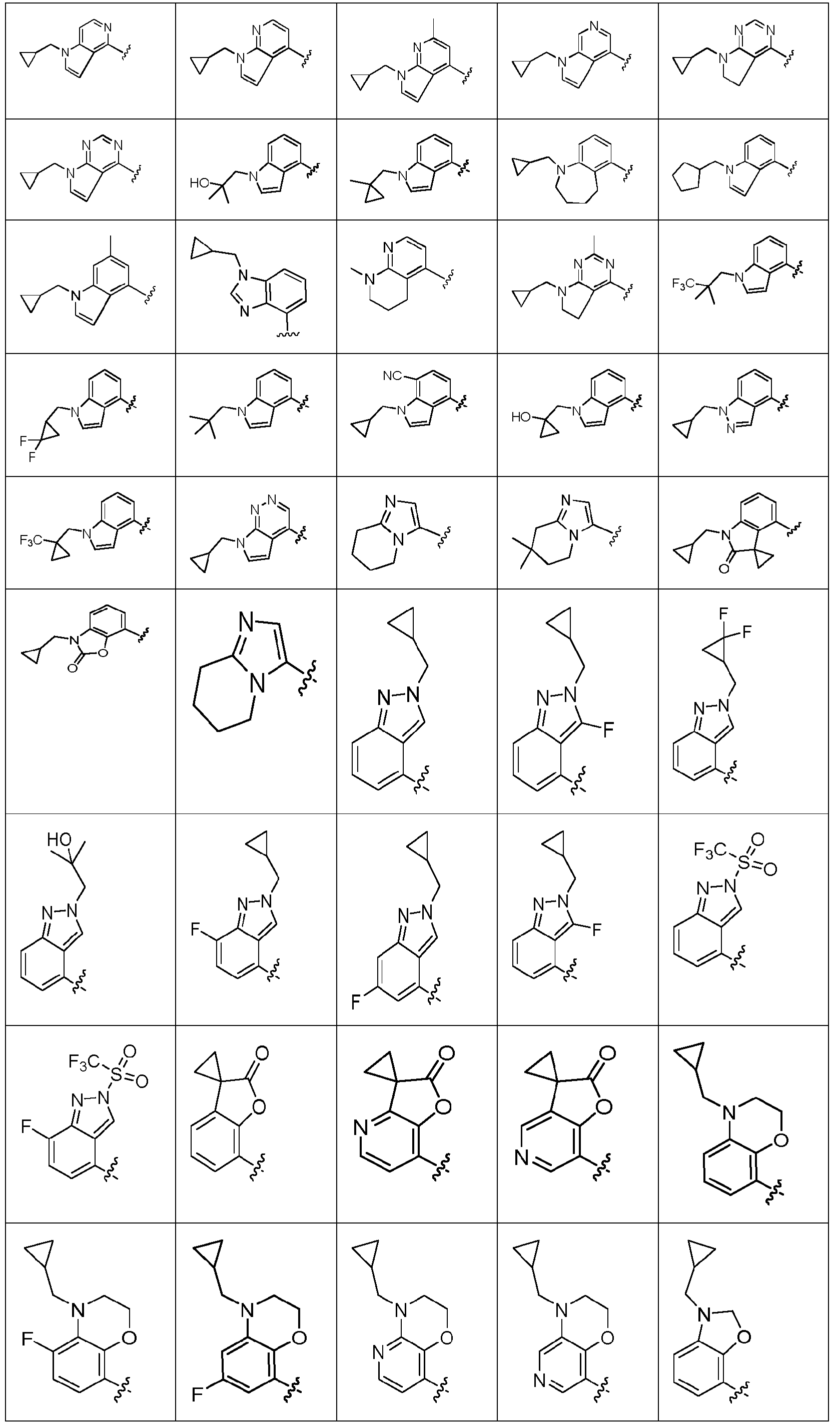

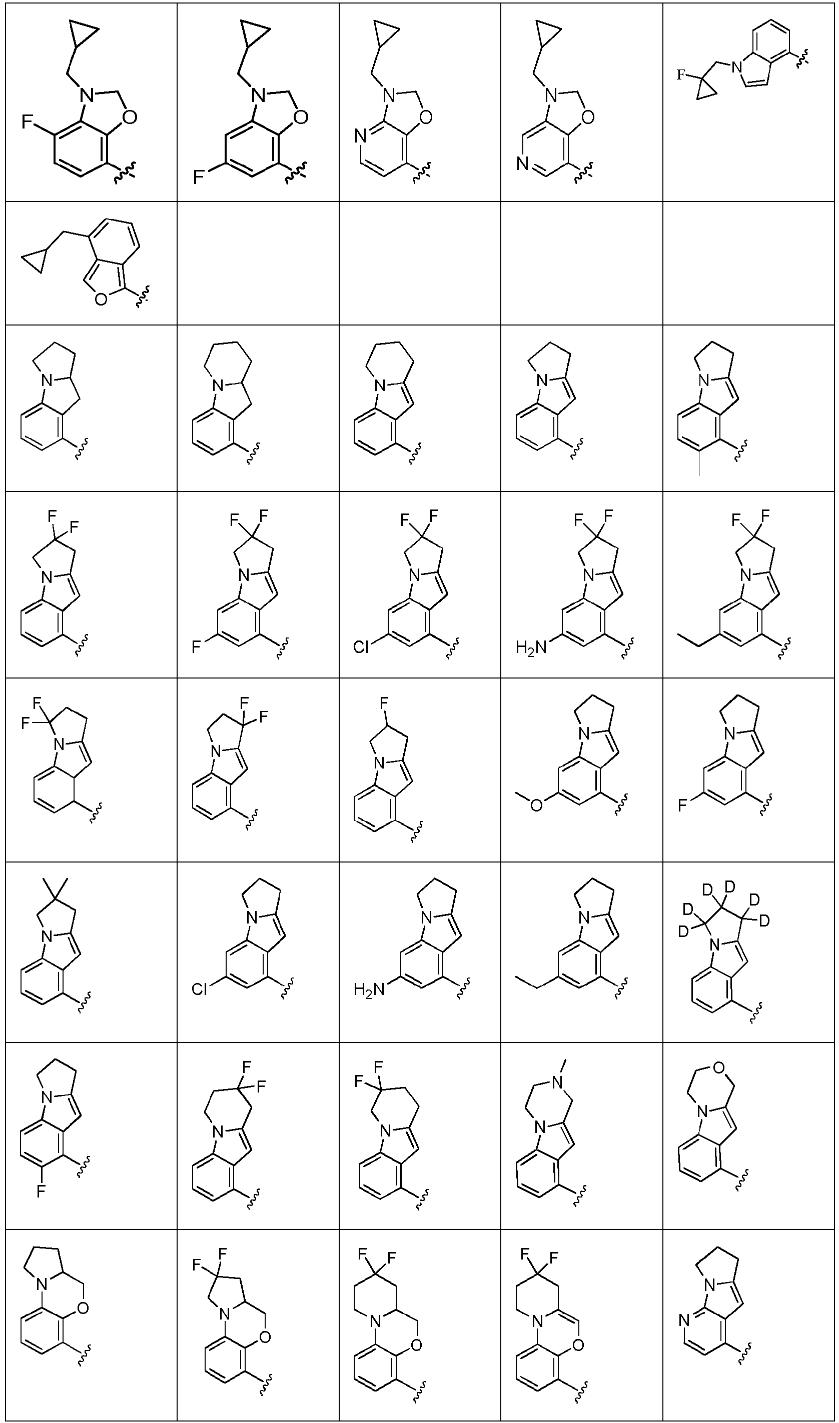

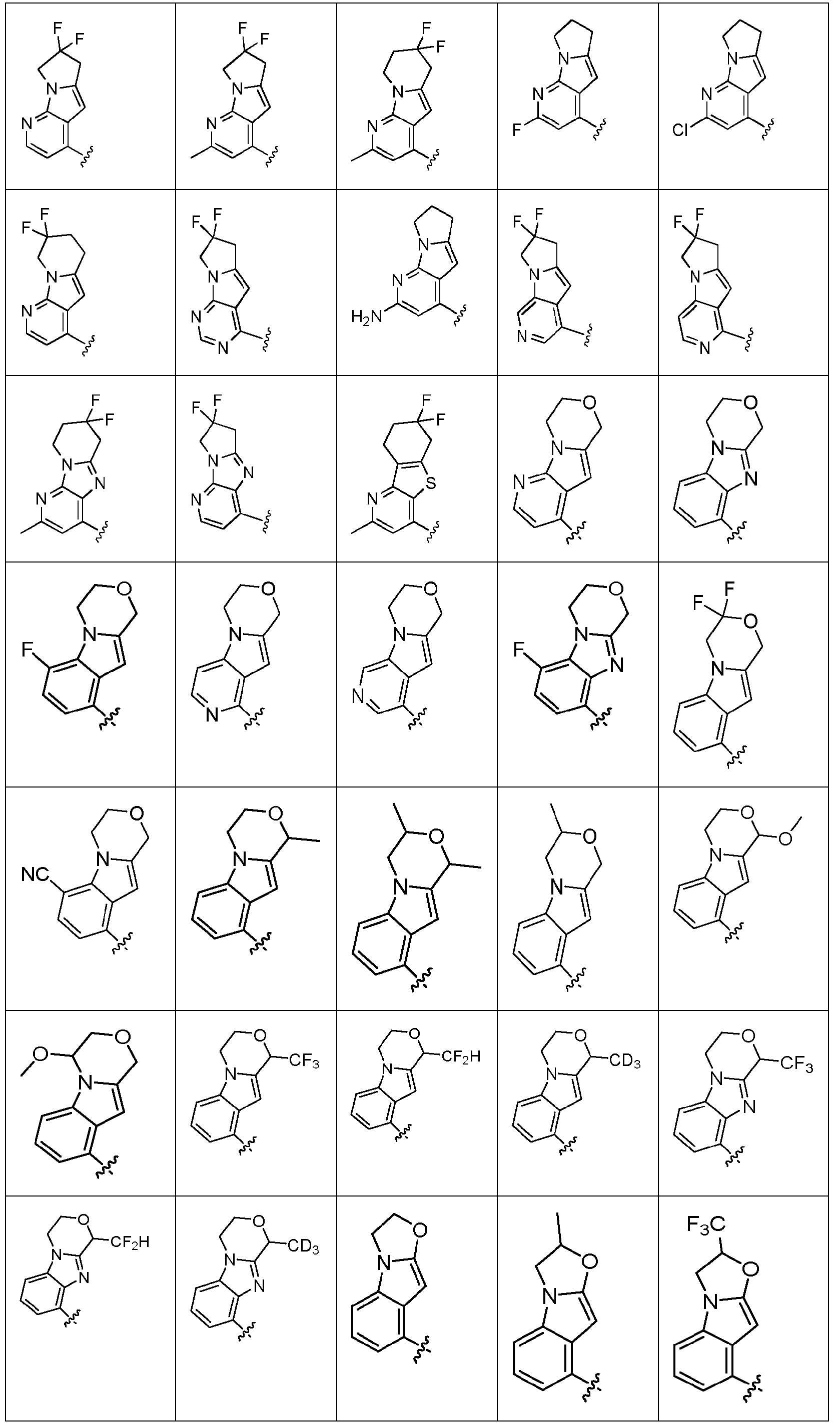

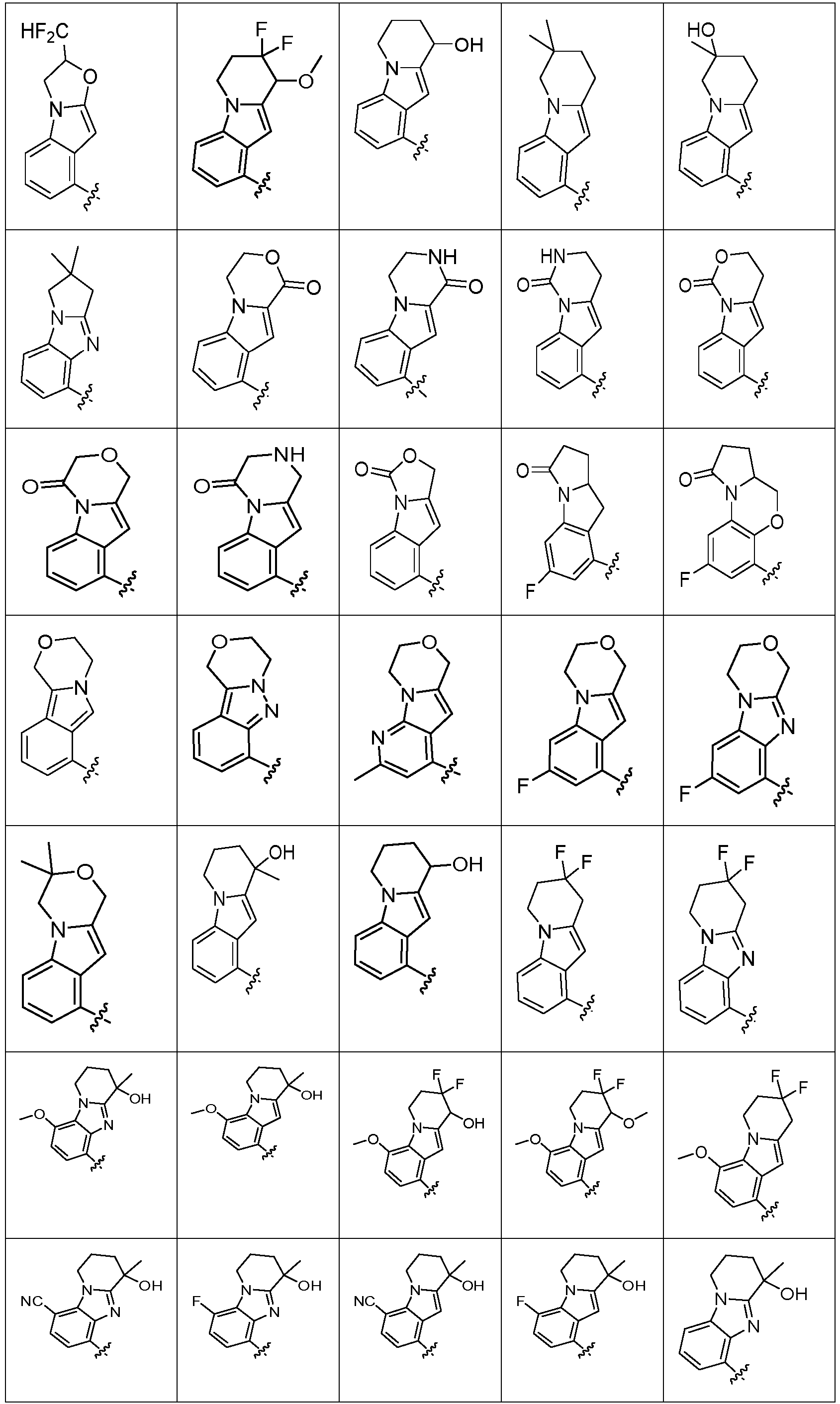

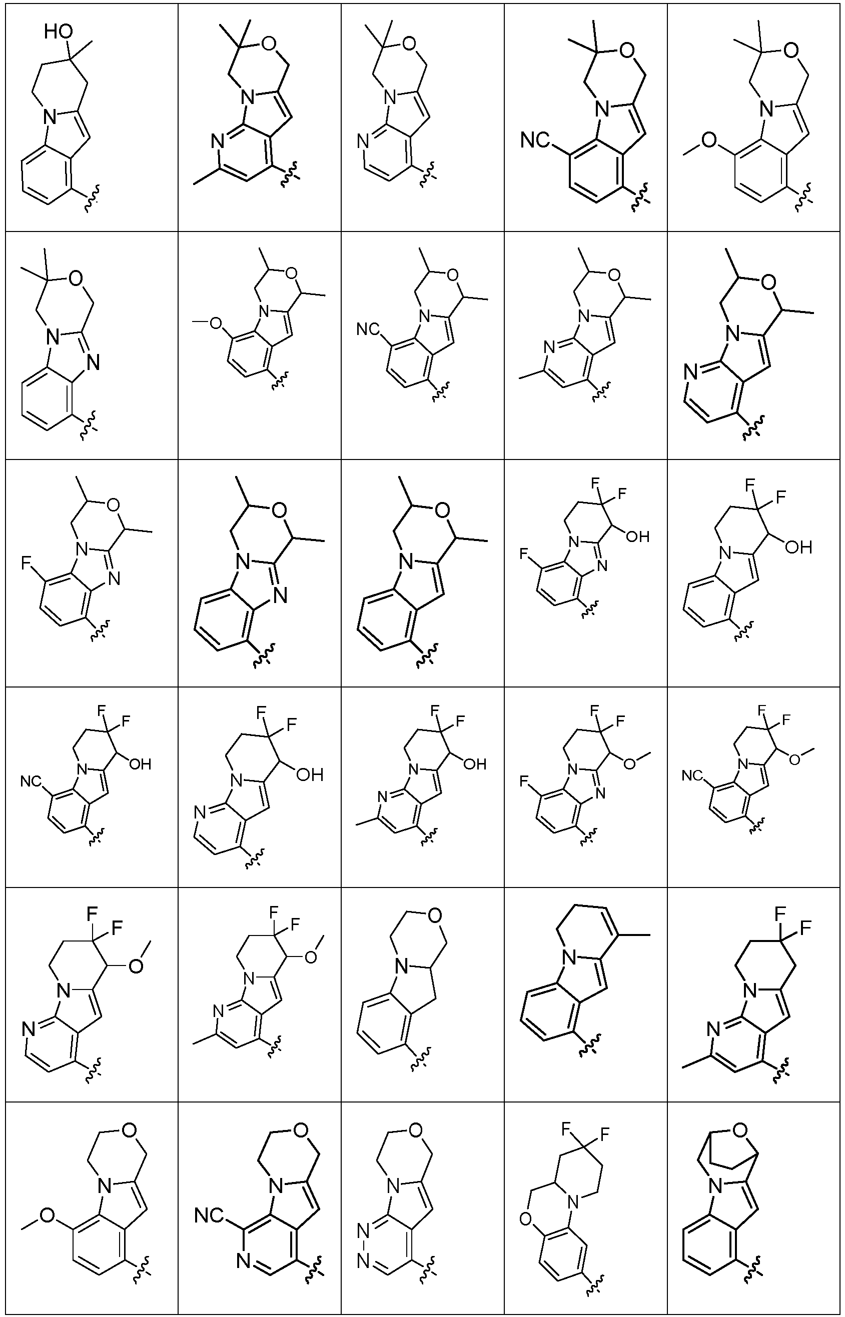

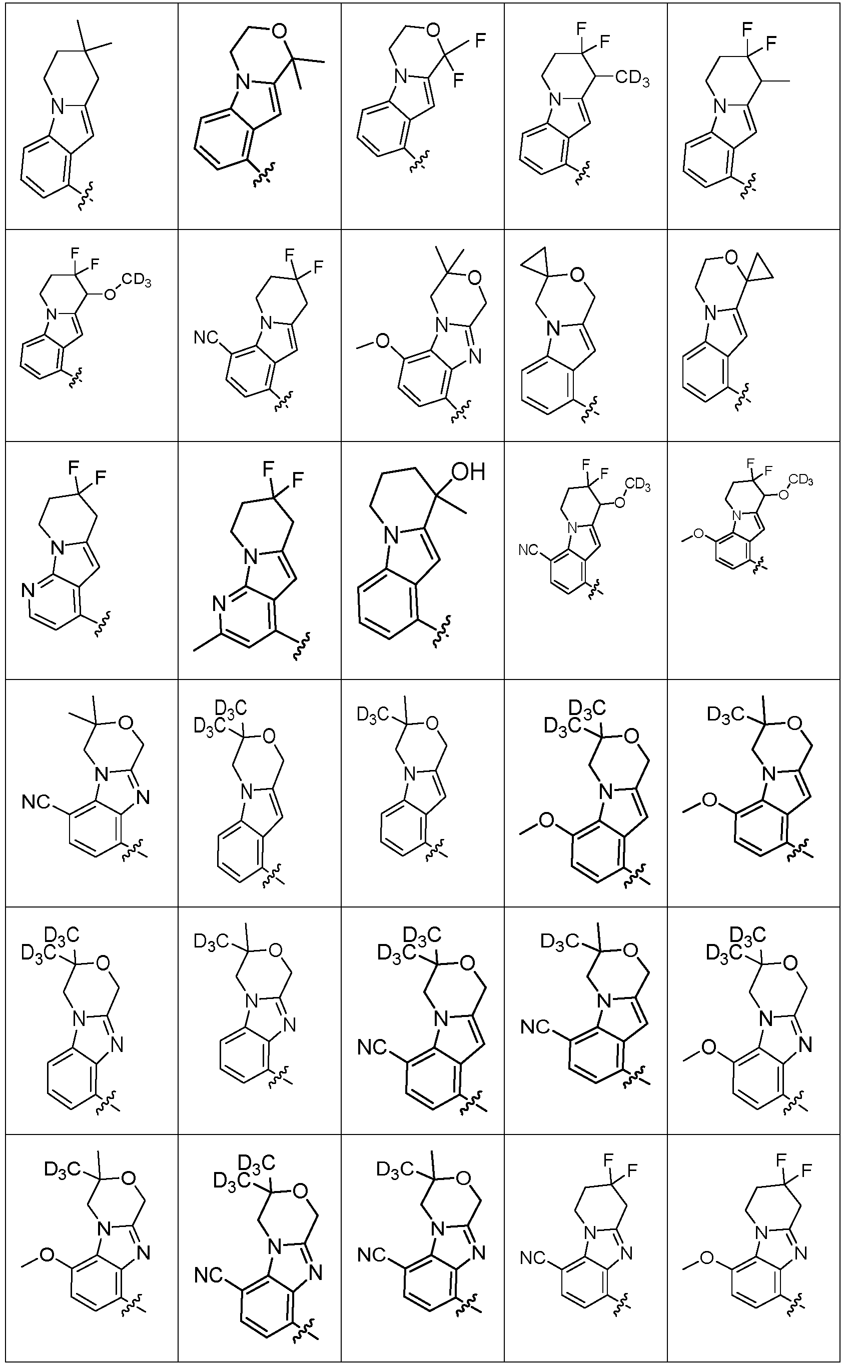

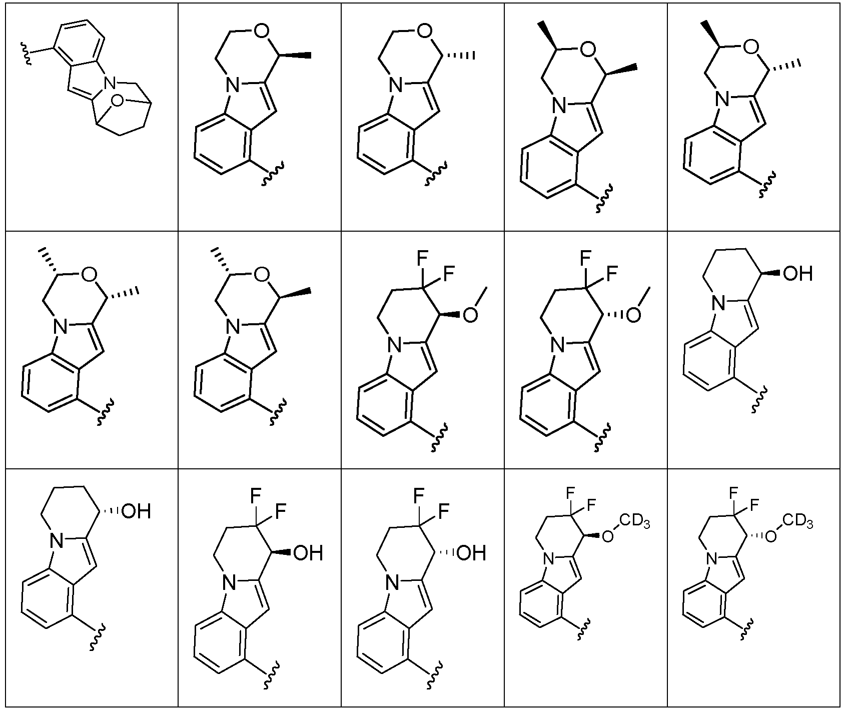

In some embodiments, A is selected from any one of the following groups:

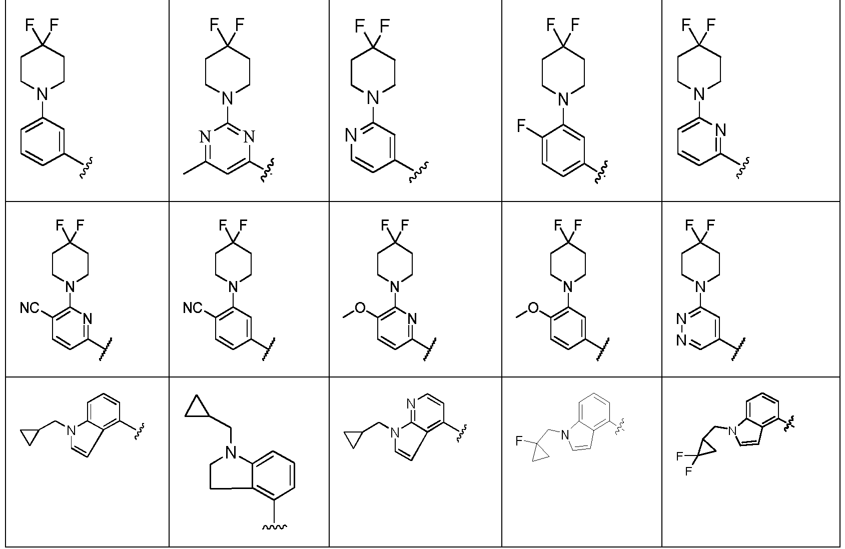

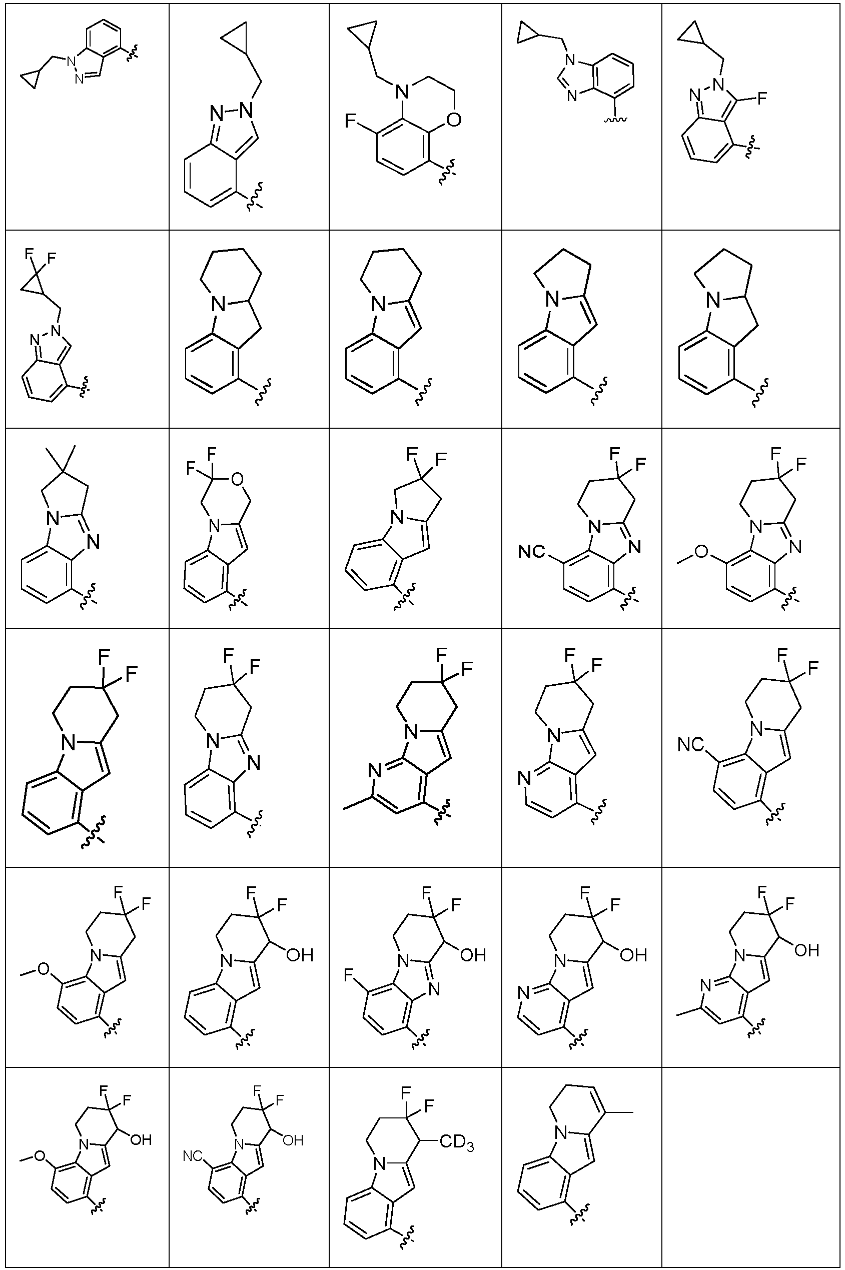

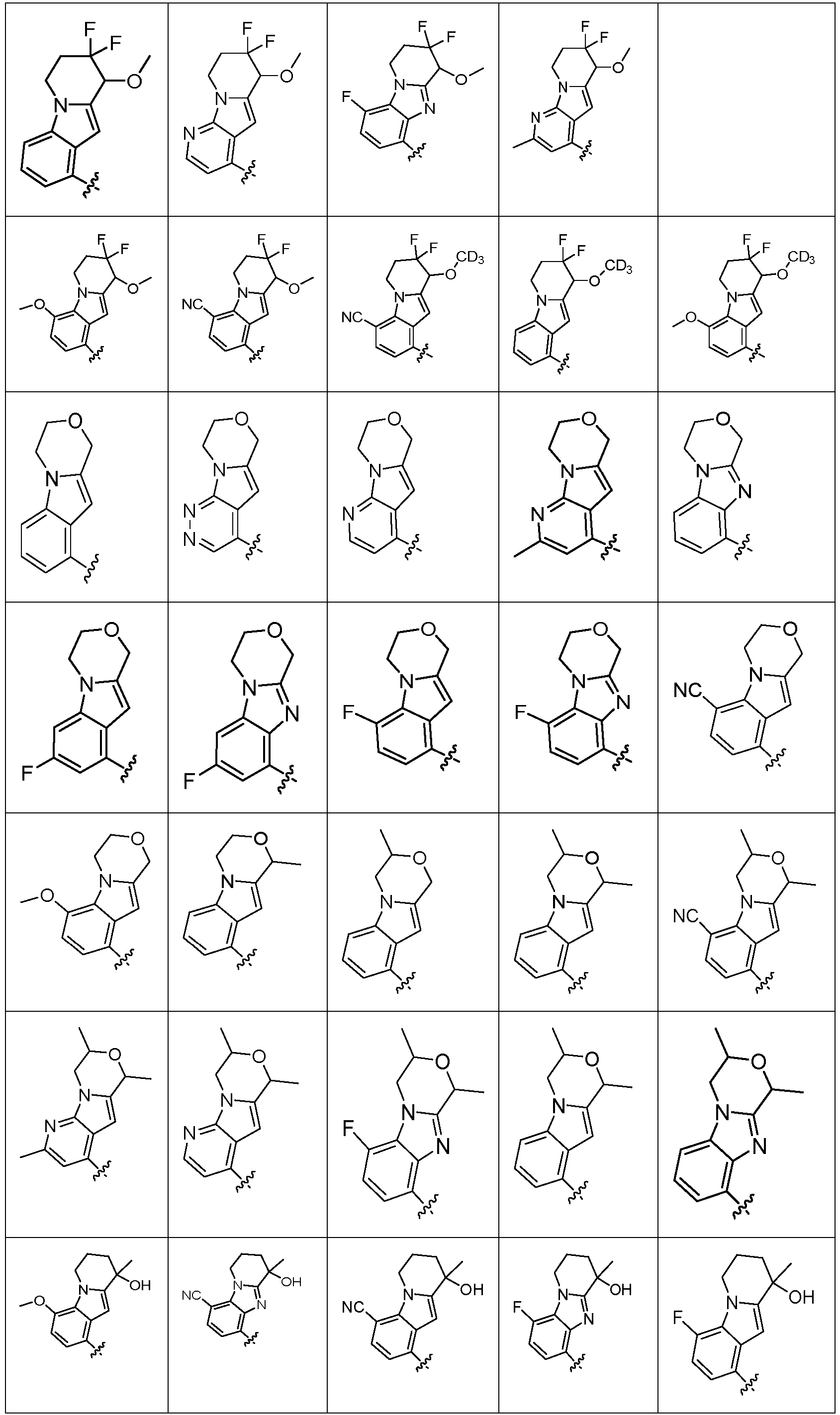

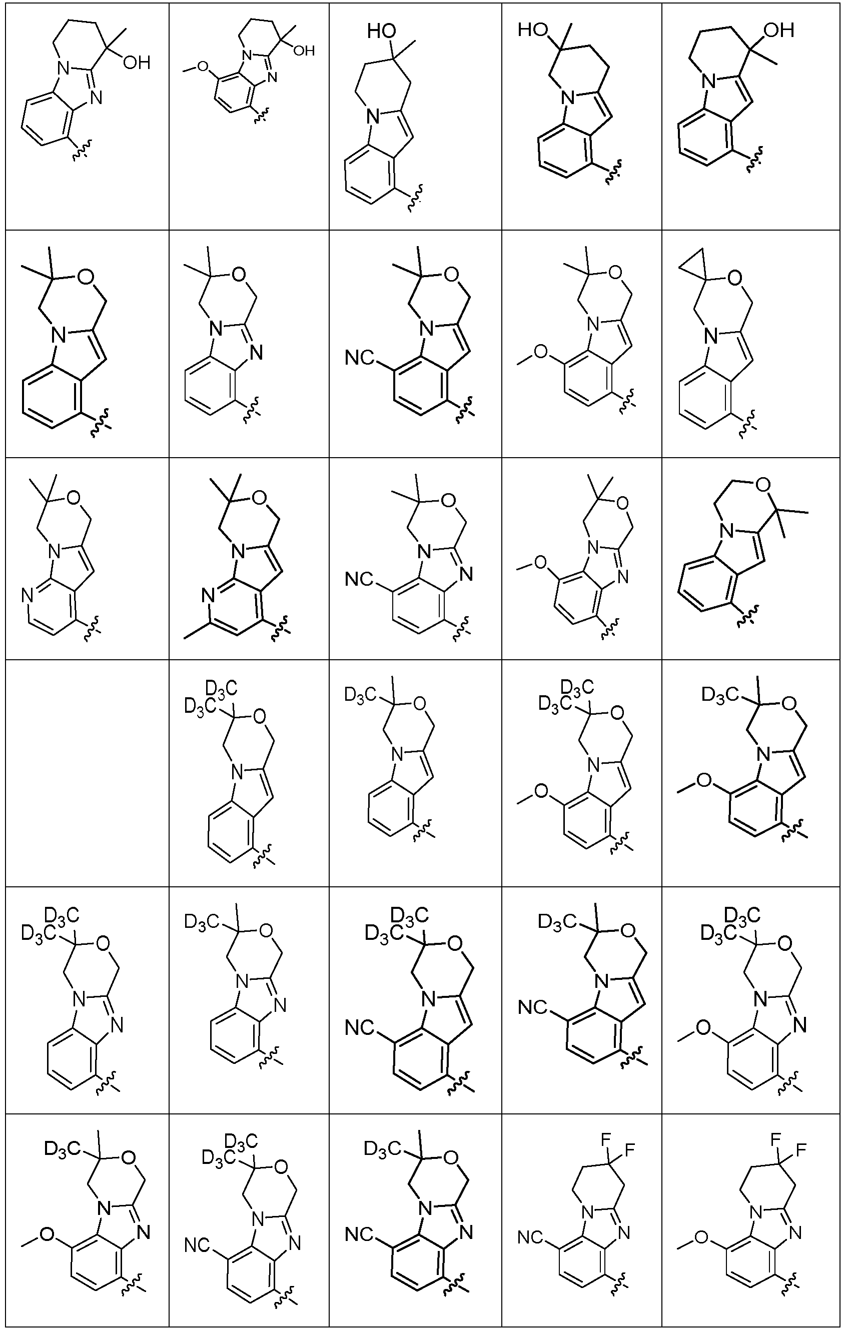

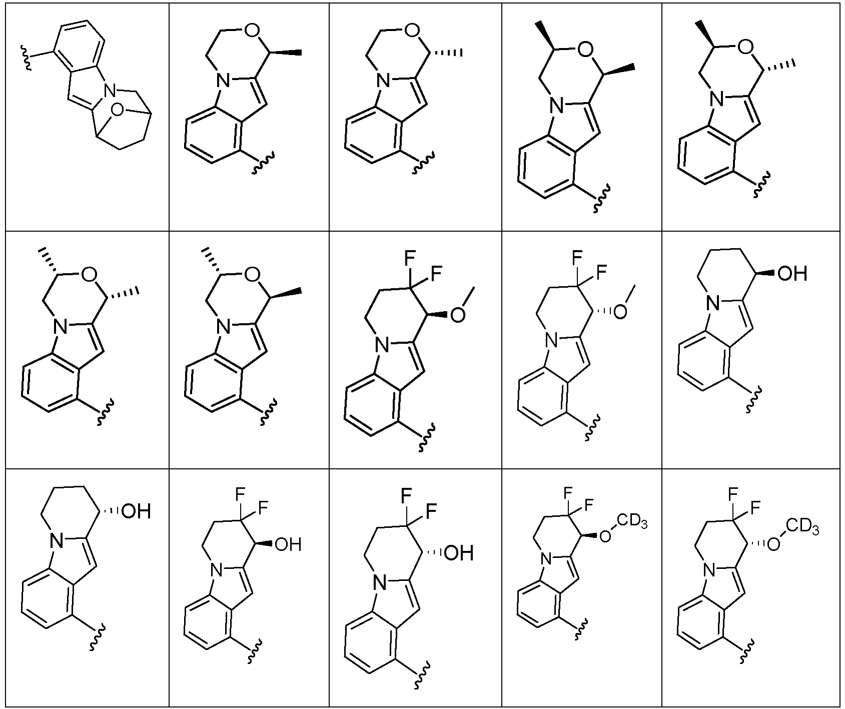

In some embodiments, A is selected from any one of the following groups:

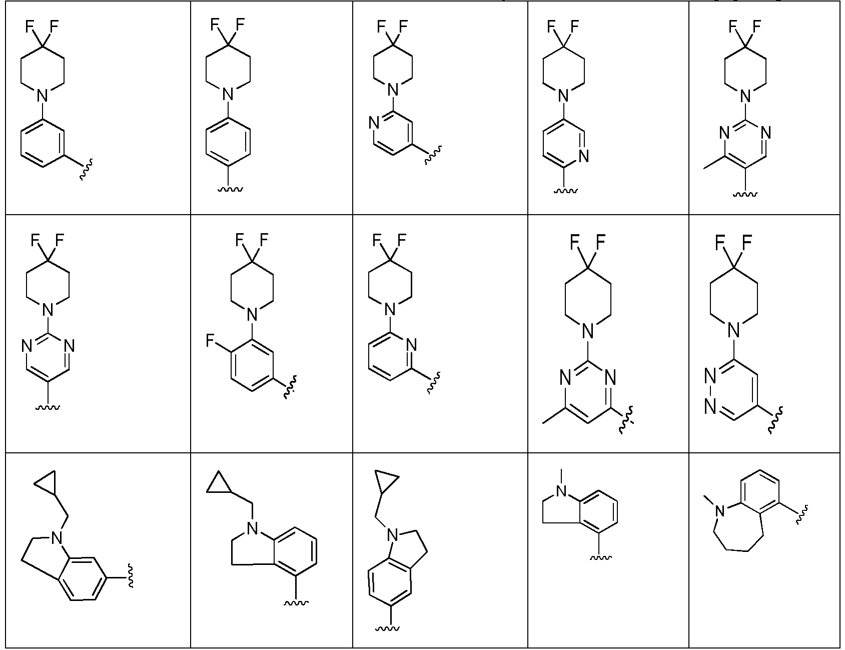

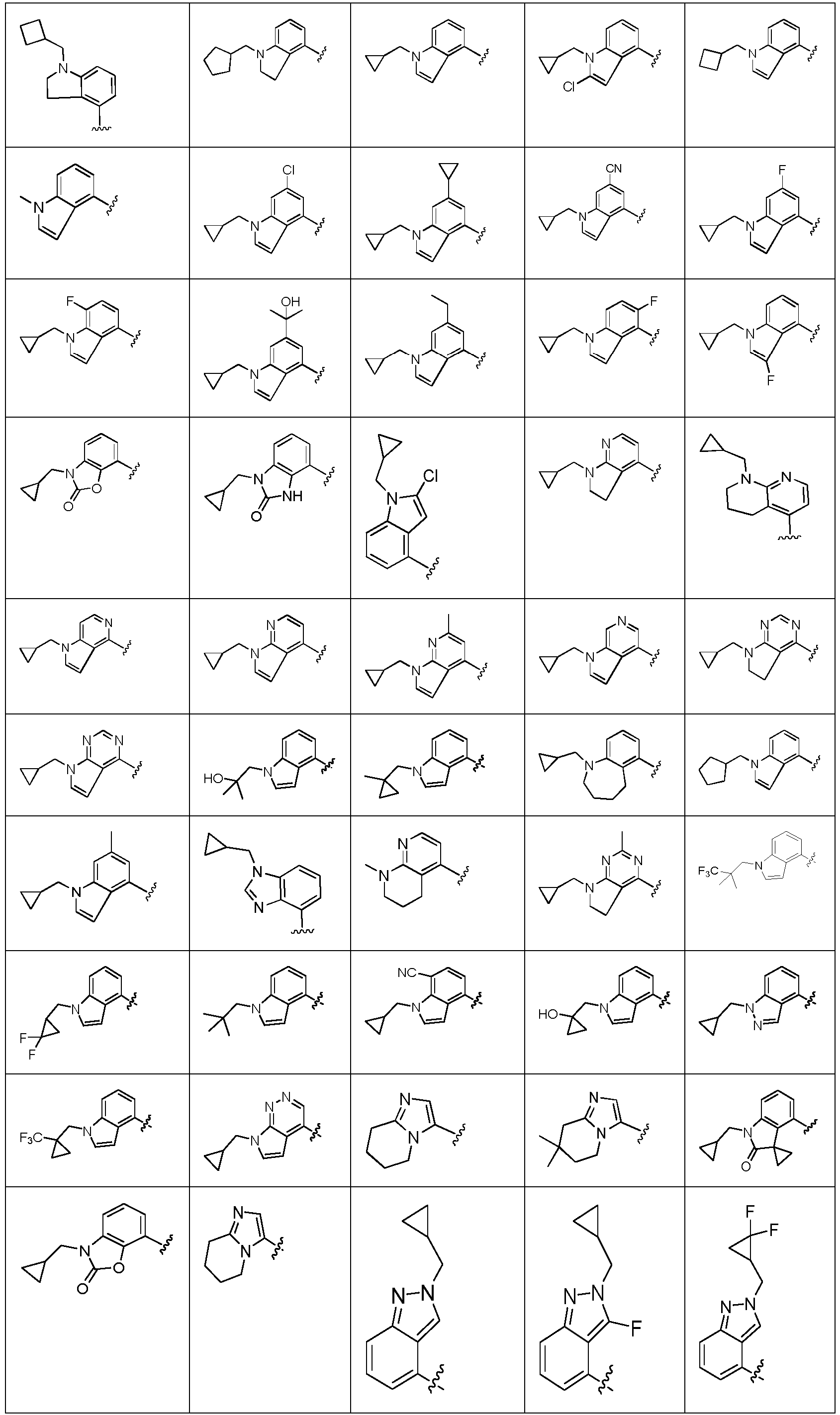

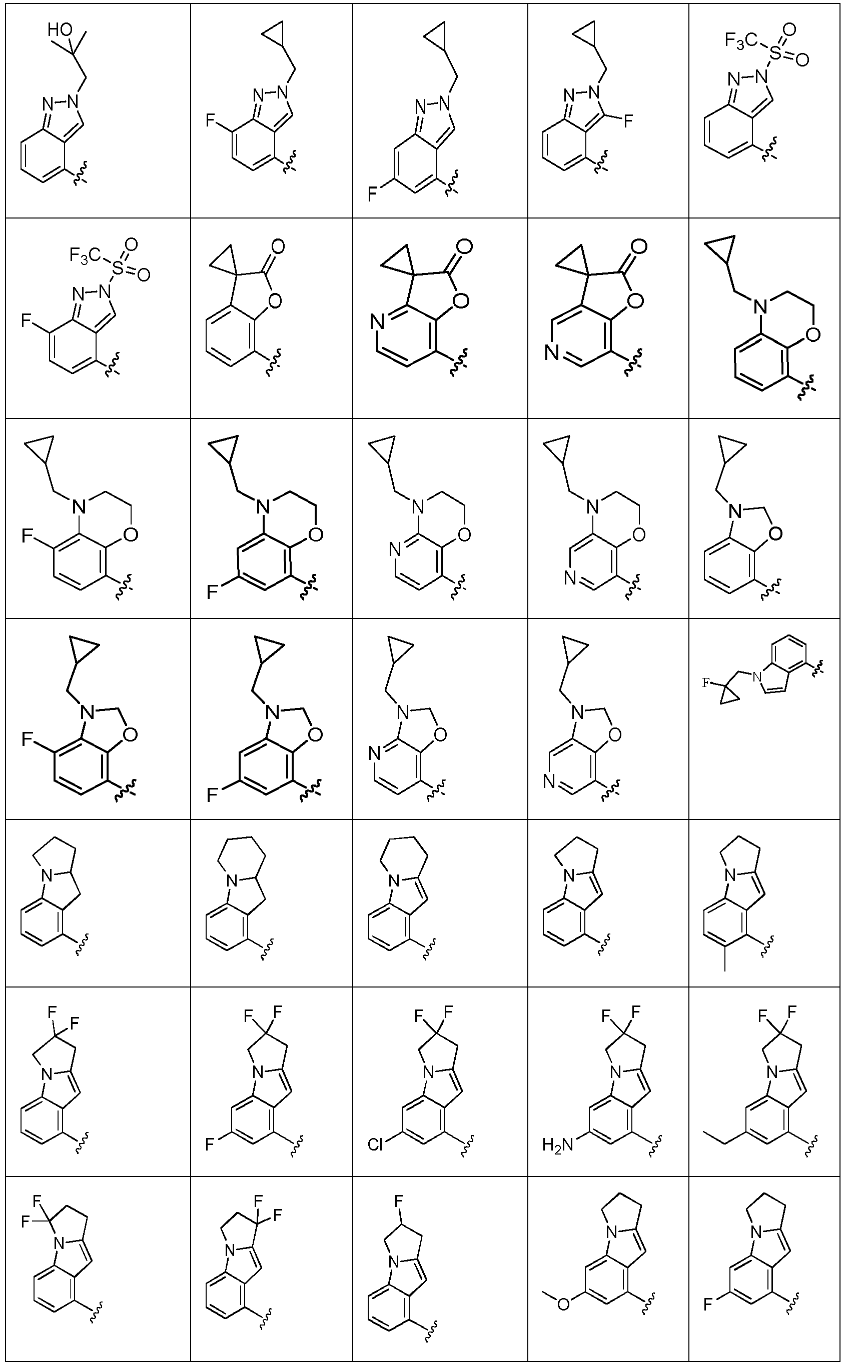

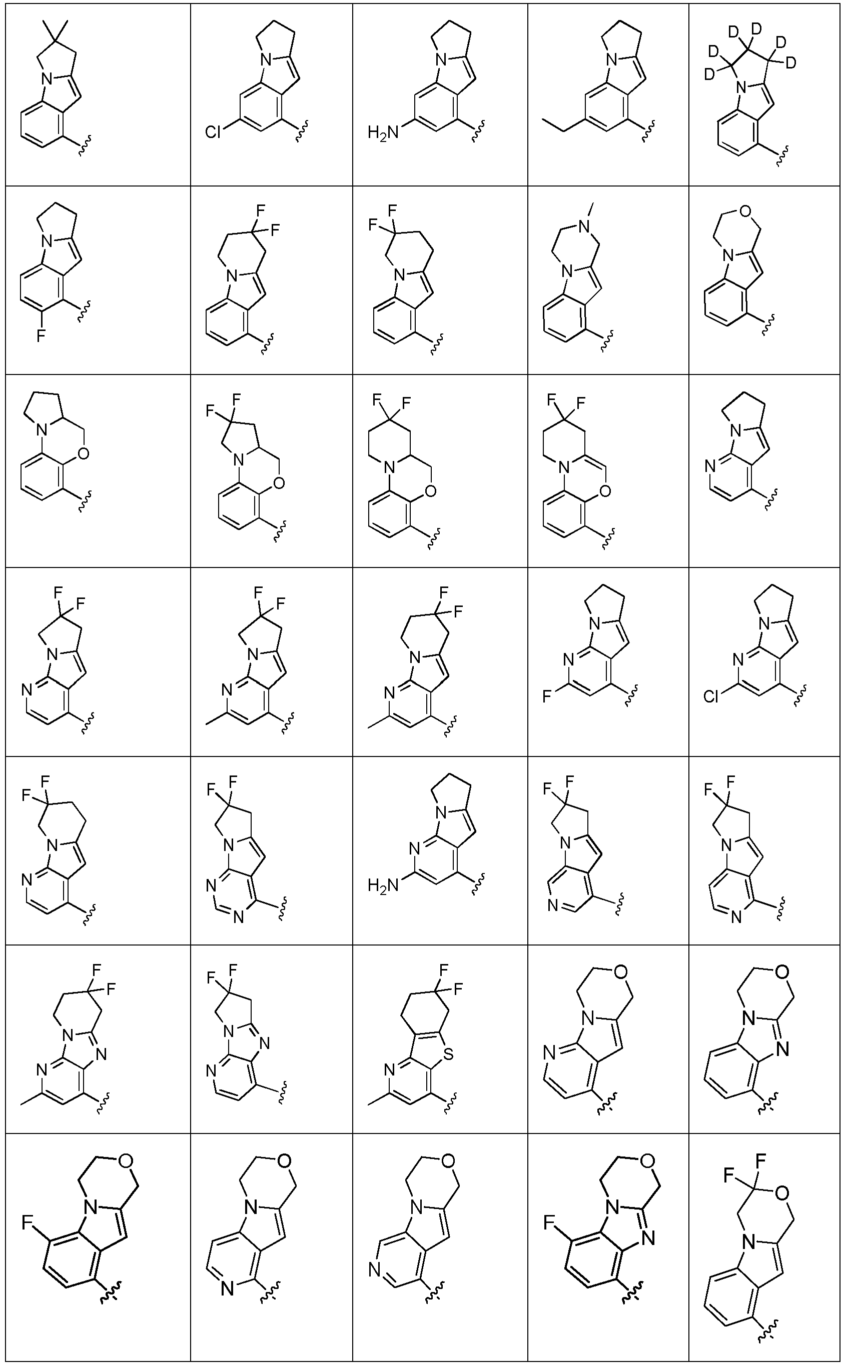

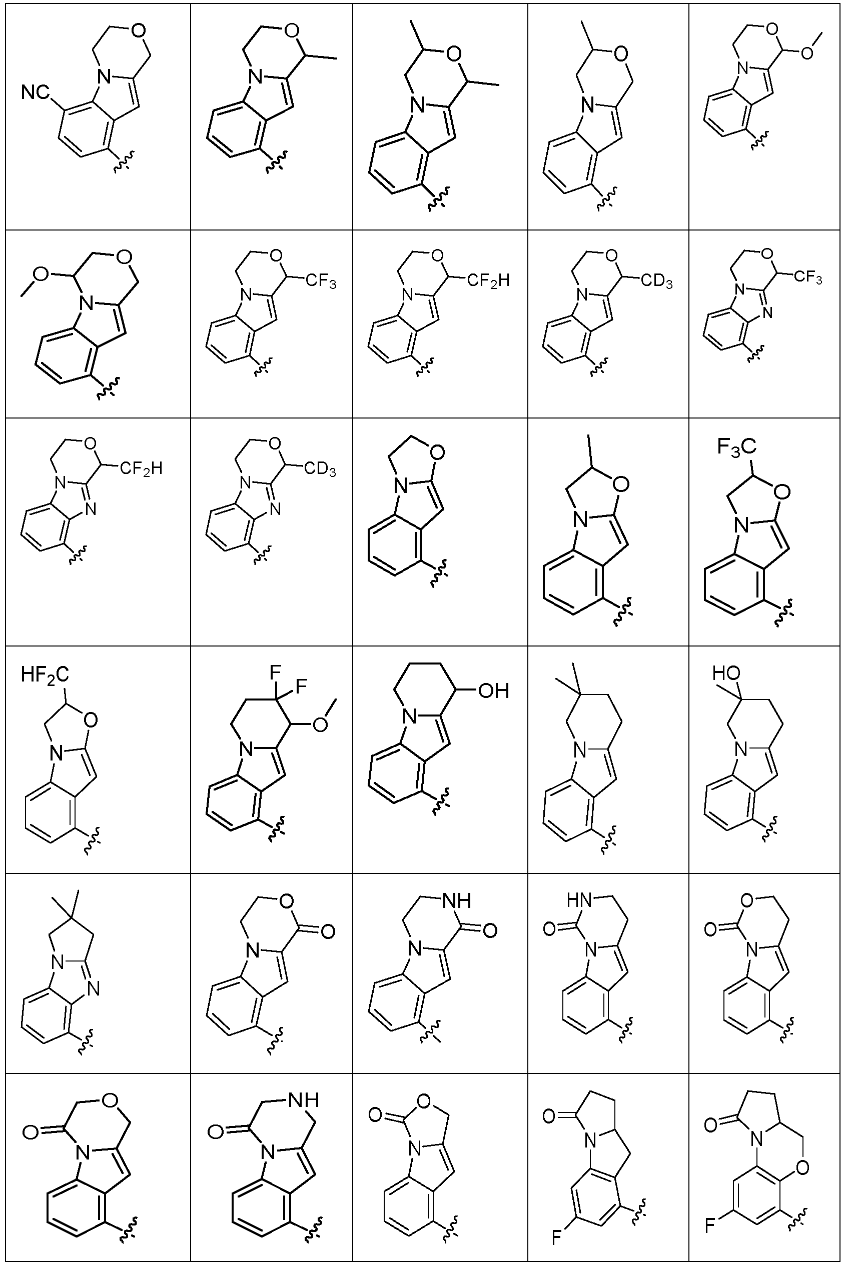

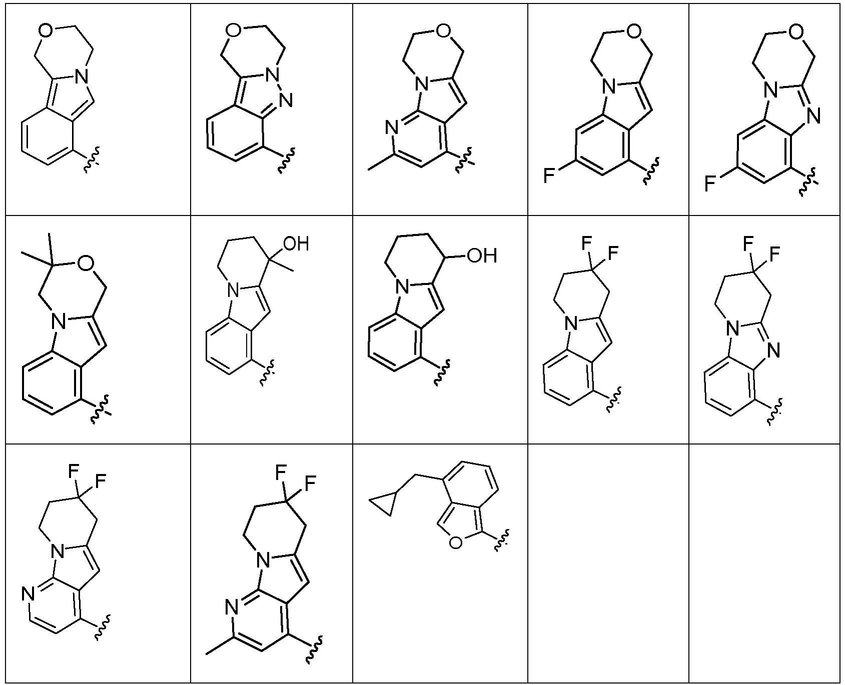

In some embodiments, A is selected from any one of the following groups:

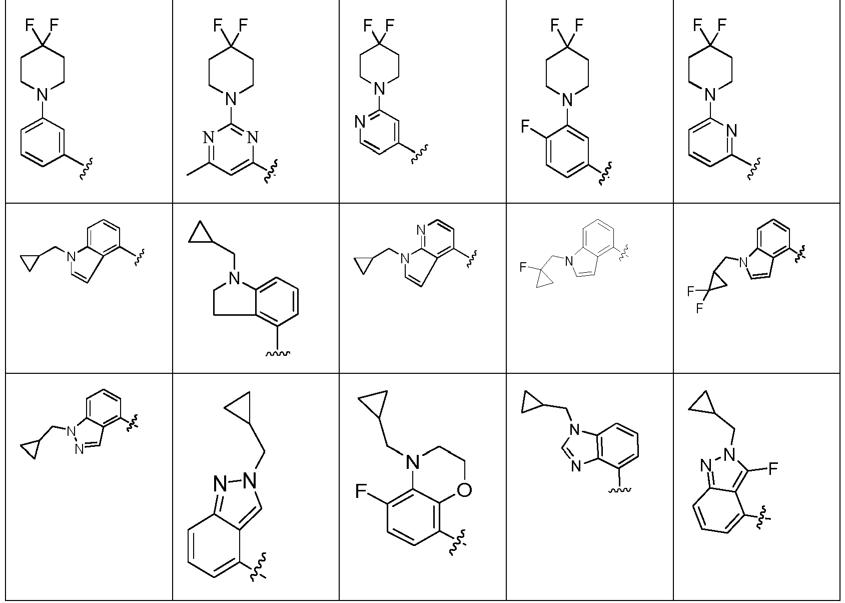

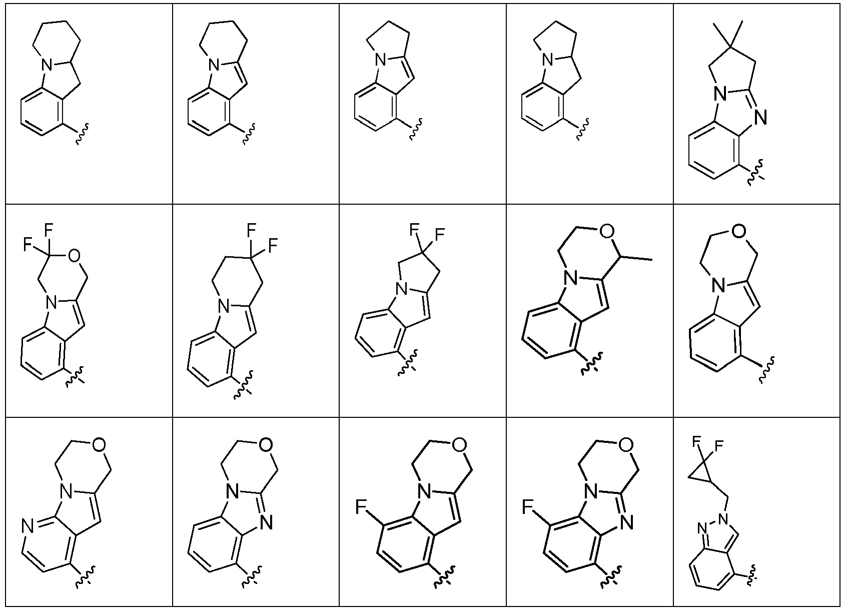

In some embodiments, A is selected from any one of the following groups:

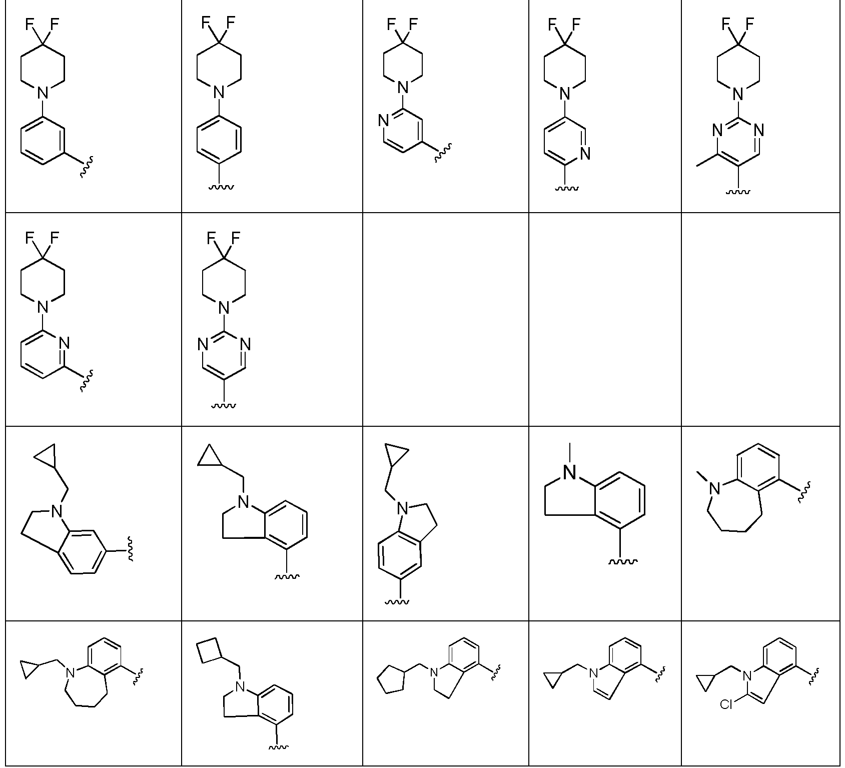

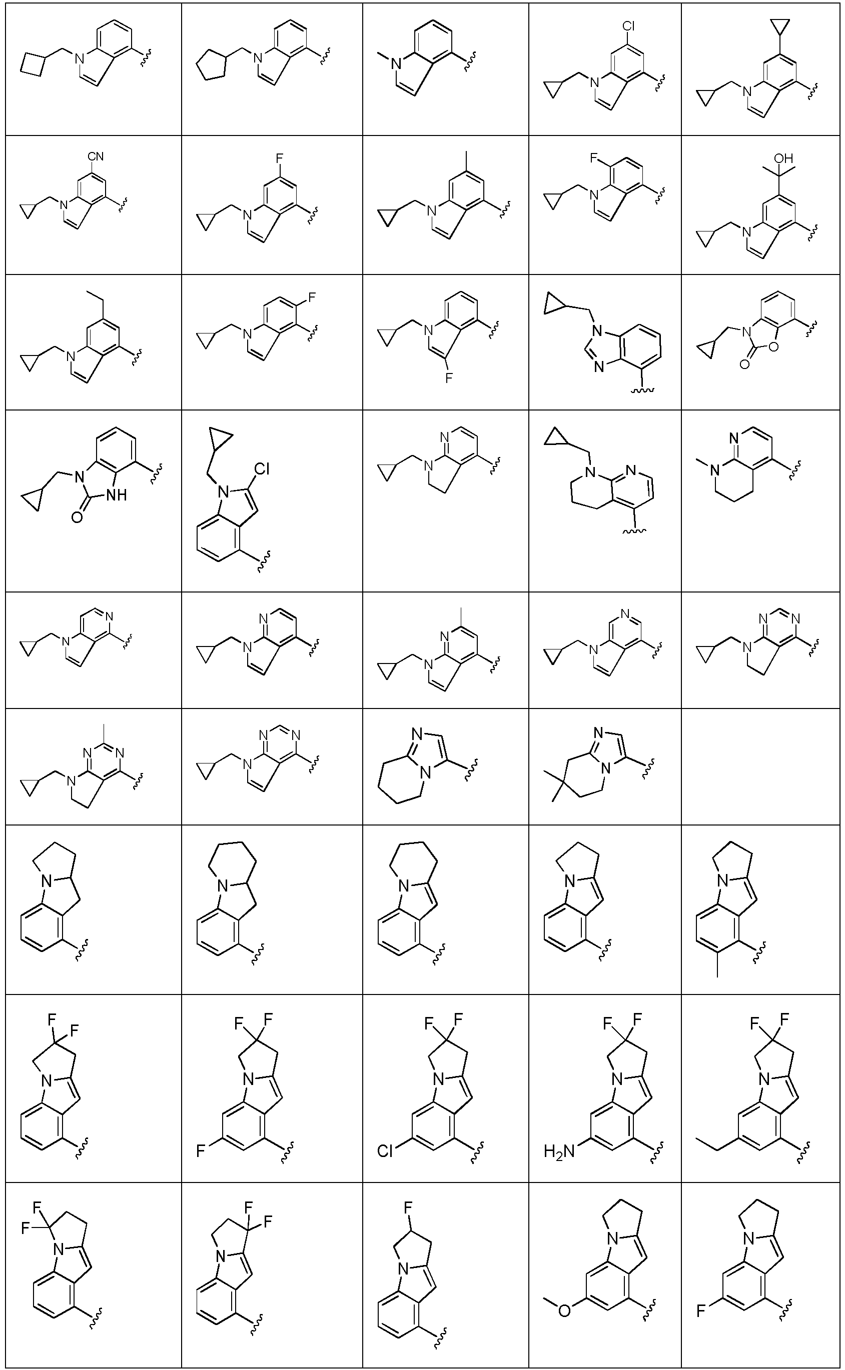

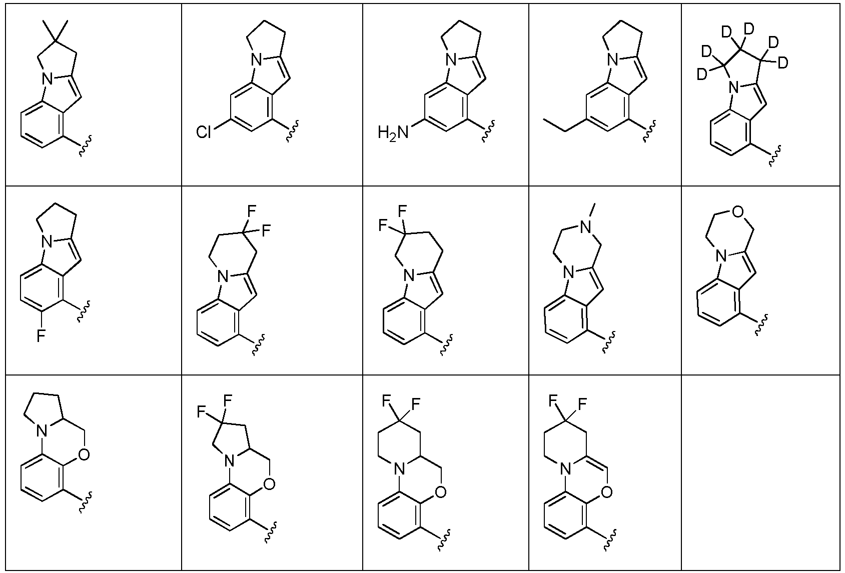

In some embodiments, L is selected from -NR³CONR³-; and/or X is CR₇, and R₇ is -NRₐRₐ.

In some embodiments, R₁ is group -Z-R₁₂, wherein -Z is a single bond, -C1-C4 alkyl-, -C1-C4 alkyl-O-, -O-, -S-, -S(=O)-, -SO₂-, -NH-, -NHSO₂-, -SO₂NH-, -NH-S(=O)(=NH)-, -S(=O)(=NH)-, -C1-C4 alkyl-SO₂-, -(C=O)-, -(C=O)NH-, - C=N(OH)- or -NH(C=O)-; and/or
(a) R₁₂ is H;
(b) R₁₂ is oxetanyl or cyclopropyl substituted with 0, 1, 2 or 3 groups selected from OH, halogen (such as F or Cl), or C1-C6 alkoxy; or
(c) R₁₂ is C1-C6 alkyl substituted with 0, 1, 2 or 3 groups selected from OH, halogen (such as F), or C1-C6 alkoxy.

In some embodiments, the group -Z-R₁₂ is -N=S(=O)-(R₁₂)₂, wherein a pair of two R₁₂, , alternatively together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
or in the group -Z-R₁₂, Z is a single bond, and R₁₂ is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₘ, -OC1-C6 haloalkyl, CN, -C(=O)Rₙ, -C(=O)ORₘ, -C(=O)NRₘRₘ, -C(=NRₘ)NRₘRₘ, - OC(=O)Rₙ, -OC(=O)NRₘRₘ, -OC2-C6 alkyl NRₘRₘ, -OC2-C6 alkyl ORₘ, -SRₘ, - S(=O)Rₙ, -S(=O)₂Rₙ, -S(=O)₂NRₘRₘ, -NRₘRₘ, -N(Rₘ)C(=O)Rₙ, -N(Rₘ)C(=O)ORₙ, -N(Rₘ)C(=O)NRₘRₘ, -N(Rₘ)C(=NRₘ)NRₘRₘ, -N(Rₘ)S(=O)₂Rₙ, - N(Rₘ)S(=O)₂NRₘRₘ, -NRₘC2-6 alkyl NRₘRₘ, -NRₘC2-C6 alkyl ORₘ, -C1-C6 alkyl NRₘRₘ, -C1-C6 alkyl ORₘ, -C1-C6 alkyl N(Rₘ)C(=O)Rₙ, -C1-C6 alkyl OC(=O)Rₙ, -C1-C6 alkyl C(=O)NRₘRₘ, -C1-C6 alkyl C(=O)ORₘ or oxo, and Rₘ and Rₙ are independently selected from H and -C1-C6 alkyl.

In some embodiments, R₁ is group -Z-R₁₂, wherein Z is -NHSO₂- or -SO₂NH-; and R₁₂ is oxetanyl or cyclopropyl substituted with 0, 1, 2 or 3 groups selected from OH, halogen (such as F), or C1-C6 alkoxy, or R₁₂ is C1-C6 alkyl substituted with 0, 1, 2 or 3 groups selected from OH, halogen (such as F), or C1-C6 alkoxy.

In some embodiments, R₁ is selected from the following groups:

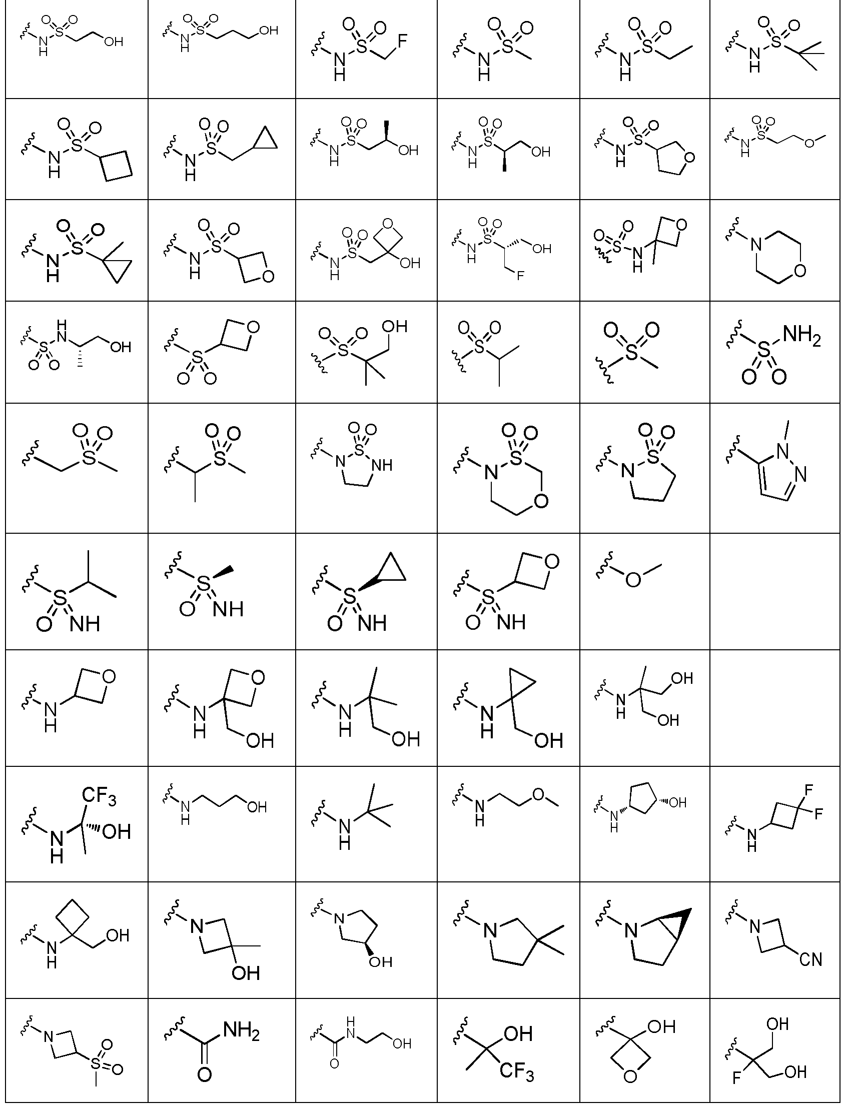

In some embodiments, Rₓ is selected from the group consisting of: 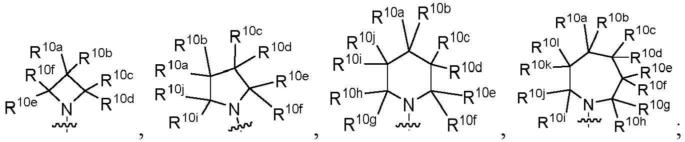
or alternatively, each of the R^{10a} and R^{10b} pair, R^{10c} and R^{10d} pair, R^{10e} and R^{10f} pair, R^{10g} and R^{10h} pair, or R¹⁰ⁱ and R^{10j} pair , independently together with the carbon atom to which they are each attached, may form saturated or partially saturated 3-, 4-, 5-, or 6-membered monocyclic ring spiro-connected to the Rₓ ring, wherein the 3-, 4-, 5-, or 6-membered monocyclic ring comprises 0, 1, 2 or 3 N atoms and 0, 1 or 2 atoms selected from O and S, and further, the 3-, 4-, 5-, or 6-membered monocyclic ring is substituted with 0, 1, 2, or 3 groups selected from: F, Cl, Br, C1-C6 alkyl, C1-C4 haloalkyl, -OR^{a}, -OC1-C4 haloalkyl, CN, -NR^{a}R^{a} or oxo;
R^{10k} is selected from the group consisting of: H, saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, - C(=NRₐ)NRₐRₐ, -OC(=O)R_{b}, -OC(=O)NRₐRₐ, -OC2-C6 alkyl NRₐRₐ, -OC2-C6 alkyl ORₐ, -SRₐ, -S(=O)R_{b}, -S(=O)₂R_{b}, -S(=O)₂NRₐRₐ, -NRₐRₐ, -N(Rₐ)C(=O)R_{d}, - N(Rₐ)C(=O)OR_{d}, -N(Rₐ)C(=O)NRₐRₐ, -N(Rₐ)C(=NRₐ)NRₐRₐ, -N(Rₐ)S(=O)₂R_{d}, - N(Rₐ)S(=O)₂NRₐRₐ, -NRₐC2-6 alkyl NRₐRₐ, -NRₐC2-C6 alkyl ORₐ, -C1-C6 alkyl NRₐRₐ, -C1-C6 alkyl ORₐ, -C1-C6 alkyl N(Rₐ)C(=O)R_{d}, -C1-C6 alkyl OC(=O)R_{b}, - C1-C6 alkyl C(=O)NRₐRₐ, -C1-C6 alkyl C(=O)ORₐ, or oxo, and Rₐ and R_{b} are independently selected from H or -C1-C6 alkyl;
R^{10l} is selected from the group consisting of: C1-C6 alkyl substituted with 0, 1, 2, 3, 4 or 5 groups selected from F, Cl, Br, C1-C6 alkoxy, -O-C1-C6 haloalkyl or CN.

In some embodiments, Rₓ is selected from:

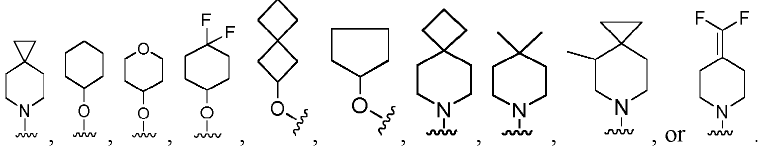

In some embodiments, the compound of formula (I) is a compound of formula (I-C-1) or formula (I-C-1'): 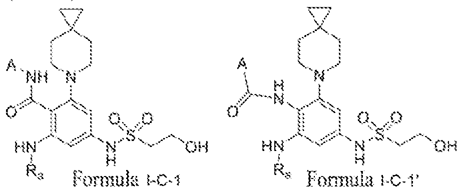 wherein A is a group selected from the A1 group, and Rₐ is H or C1-C4 alkyl.

In some embodiments, the compound of formula (I) is a compound of formula (I-C-2) or formula (I-C-2'): 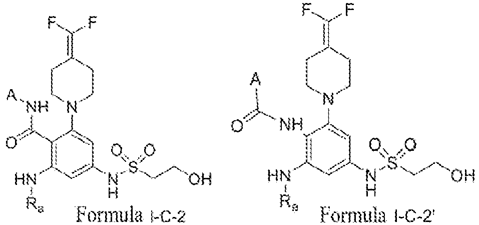 wherein A is a group selected from the A1 group, and Rₐ is H or C1-C4 alkyl.

In some embodiments, the compound of formula (I) is a compound of formula (I-A-1) 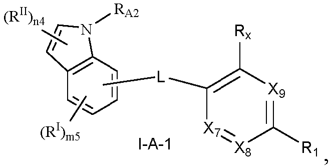 wherein the optional ranges of L, R_{X}, R₁, X₇, X₈, X₉, R_{A2}, R^{I}, R^{II}, n4, and m5 are as defined above; preferably, the compound as represented by formula (I-A-1) is

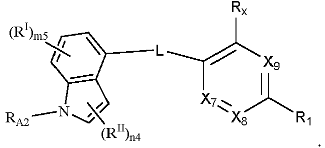

In some embodiments, the compound of formula (I) is a compound of formula (I-A-2) 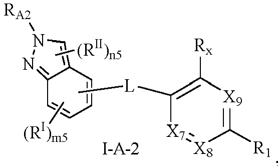 wherein the optional ranges of L, R_{X}, R₁, X₇, X₈, X₉, R_{A2}, R^{I}, R^{II}, n5, and m5 are as defined above; preferably, the compound as represented by formula (I-A-2) is

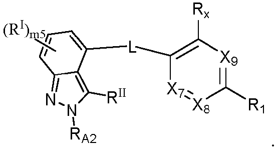

In some embodiments, the compound of formula (I) is a compound of formula (I-A-3) 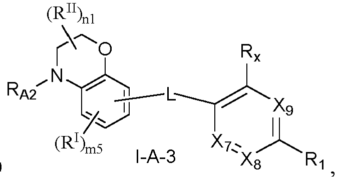 wherein the optional ranges of L, R_{X}, R₁, X₇, X₈, X₉, R_{A2}, R^{I}, R^{II}, n1, and m5 are as defined above; preferably, the compound as represented by formula (I-A-3) is

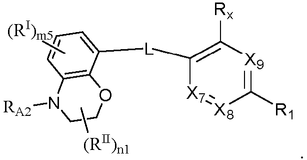

In some embodiments, the compound of formula (I) is a compound of formula (I-A-4) 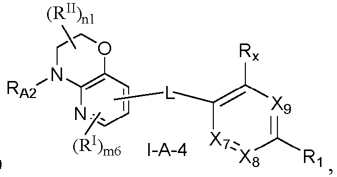 wherein the optional ranges of L, R_{X}, R₁, X₇, X₈, X₉, R_{A2}, R^{I}, R^{II}, n1, and m6 are as defined above; preferably, the compound as represented by formula (I-A-4) is

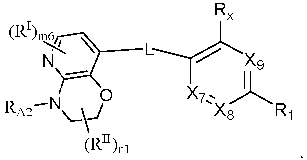

In some embodiments, the compound of formula (I) is a compound of formula (I-A-5) 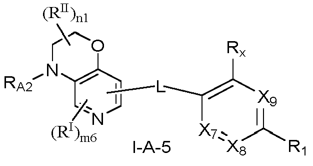 , wherein the optional ranges of L, R_{X}, R₁, X₇, X₈, X₉, R_{A2}, R^{I}, R^{II}, n1, and m6 are as defined above; preferably, the compound as represented by formula (I-A-5) is

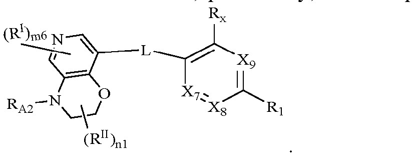

In some embodiments, the compound of formula (I) is a compound of formula (I-A-6) 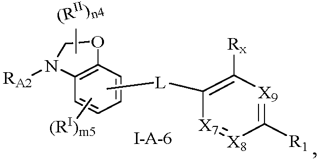 wherein the optional ranges of L, R_{X}, R₁, X₇, X₈, X₉, R_{A2}, R^{I}, R^{II}, n4, and m5 are as defined above; preferably, the compound as represented by formula (I-A-6) is

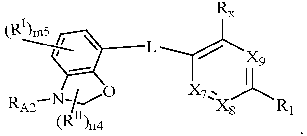

In some embodiments, the compound of formula (I) is a compound of formula (I-A-7) 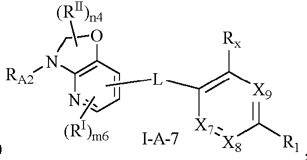 , wherein the optional ranges of L, R_{X}, R₁, X₇, X₈, X₉, R_{A2}, R^{I}, R^{II}, n4, and m6 are as defined above; preferably, the compound as represented by formula (I-A-7) is

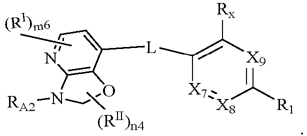

In some embodiments, the compound of formula (I) is a compound of formula (I-A-8) 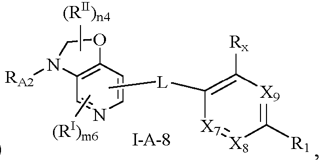 wherein the optional ranges of L, R_{X}, R₁, X₇, X₈, X₉, R_{A2}, R^{I}, R^{II}, n4, and m6 are as defined above; preferably, the compound as represented by formula (I-A-8) is

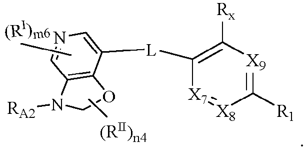

In some embodiments, the compound of formula (I) is a compound of formula (I-B-1) 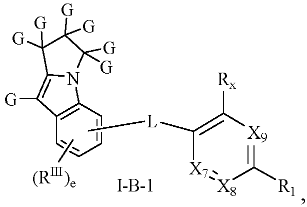 wherein the optional ranges of L, R_{X}, R₁, X₇, X₈, X₉, G, R^{III}, and e are as defined above; preferably, the compound as represented by formula (I-B-1) is

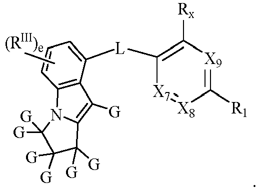

In some embodiments, the compound of formula (I) is a compound of formula (I-B-2) 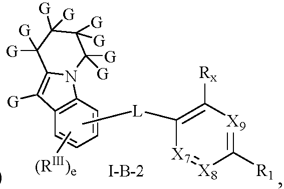 wherein the optional ranges of L, R_{X}, R₁, X₇, X₈, X₉, G, R^{III}, and e are as defined above; preferably, the compound as represented by formula (I-B-2) is

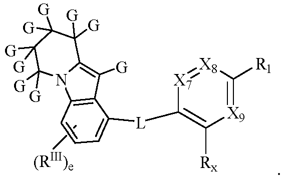

In some embodiments, the compound of formula (I-B-2) is

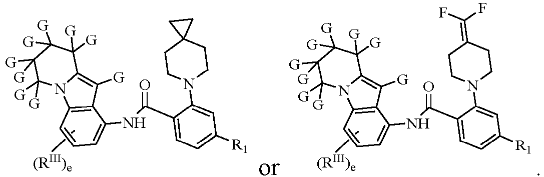

In other embodiments, the compound of formula (I-B-2) is

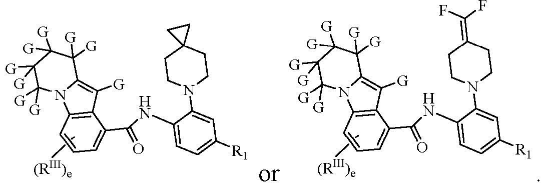

In some preferable embodiments, the compound of formula (I-B-2) is:

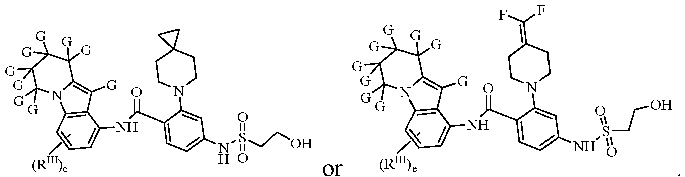

In some more preferable embodiments, the compound of formula (I-B-2) is:

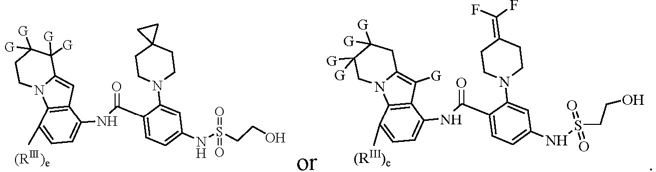

In other preferable embodiments, the compound of formula (I-B-2) is:

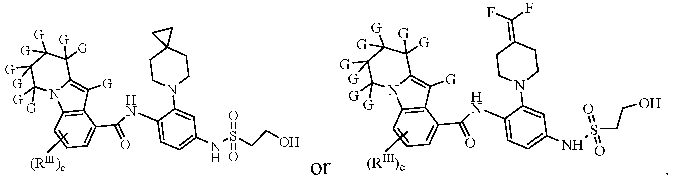

In some more preferable embodiments, the compound of formula (I-B-2) is:

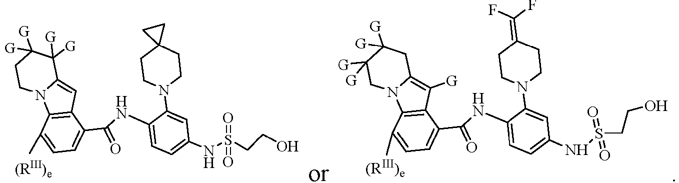

In some of such embodiments, preferably, R^{III} is selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, - NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl); and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or - N(C1-C6 alkyl)(C1-C6 alkyl), or two G attached to the same carbon atom form an oxo group (=O). In some further preferred embodiments, each e is 1.

In some embodiments, the compound of formula (I) is a compound of formula (I-B-3) 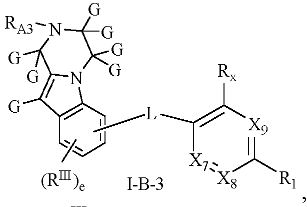 wherein the optional ranges of L, R_{X}, R₁, X₇, X₈, X₉, G, R^{III}, R_{A3}, and e are as defined above; preferably, the compound as represented by formula (I-B-3) is

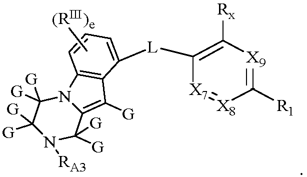

In some embodiments, the compound of formula (I) is a compound of formula (I-B-4) 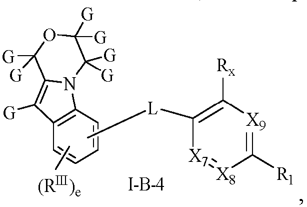 wherein the optional ranges of L, R_{X}, R₁, X₇, X₈, X₉, G, R^{III}, and e are as defined above; preferably, the compound as represented by formula (I-B-4) is

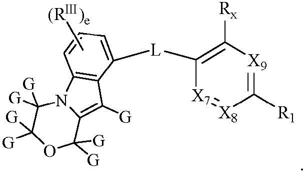

In some embodiments, the compound of formula (I-B-4) is:

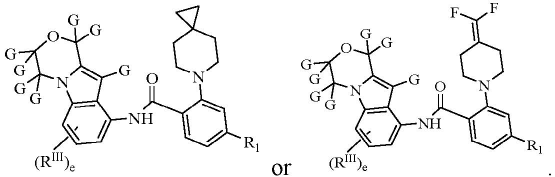

In some preferable embodiments, the compound of formula (I-B-4) is:

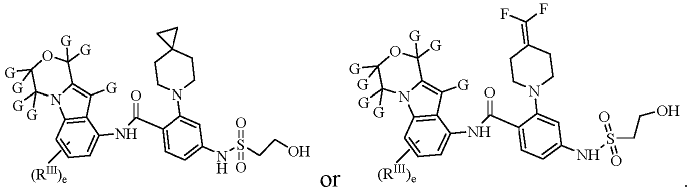

In some more preferable embodiments, the compound of formula (I-B-4) is:

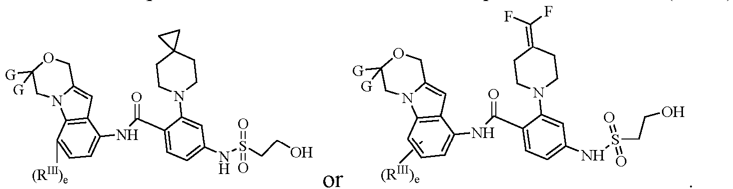

In other embodiments, the compound of formula (I-B-4) is:

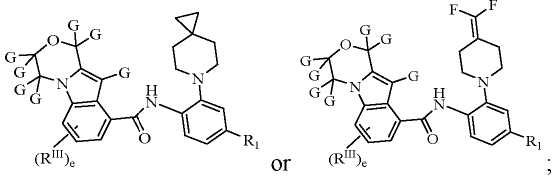

In some preferable embodiments, the compound of formula (I-B-4) is:

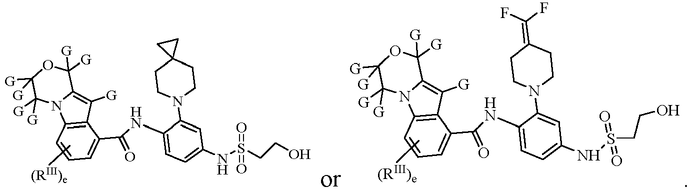

In some more preferred embodiments, the compound of formula (I-B-4) is:

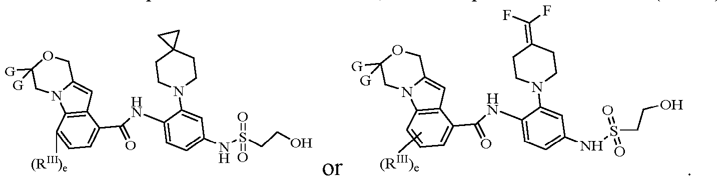

In some of such embodiments, preferably, R^{III} is selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, - NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl); and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or - N(C1-C6 alkyl)(C1-C6 alkyl), or two G attached to the same carbon atom form an oxo group (=O). In some further preferable embodiments, each e is 1.

In some embodiments, the compound of formula (I) is a compound of formula (I-B-5) 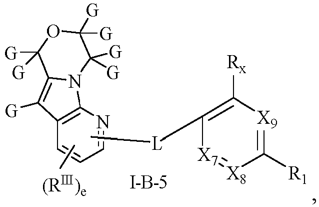 wherein the optional ranges of L, R_{X}, R₁, X₇, X₈, X₉, G, R^{III}, and e are as defined above; preferably, the compound as represented by formula (I-B-5) is

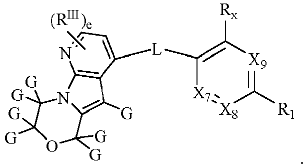

In some embodiments, the compound of formula (I) is a compound of formula (I-B-6) 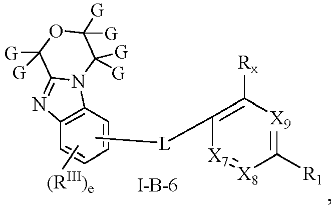 wherein the optional ranges of L, R_{X}, R₁, X₇, X₈, X₉, G, R^{III}, and e are as defined above; preferably, the compound as represented by formula (I-B-6) is

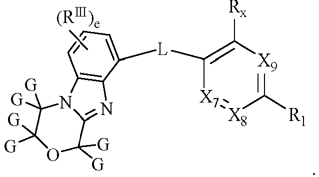

In some embodiments, the compound of formula (I-B-6) is:

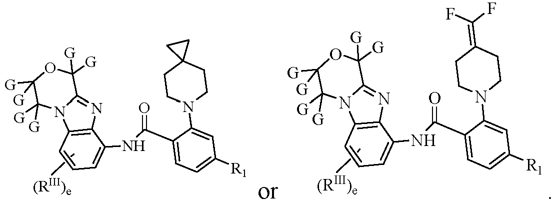

In some preferable embodiments, the compound of formula (I-B-6) is:

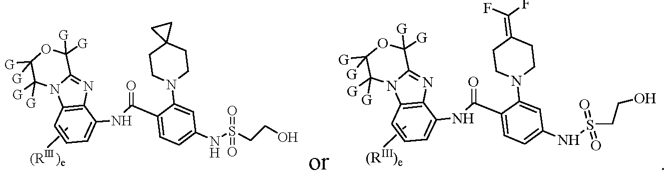

In some more preferable embodiments, the compound of formula (I-B-6) is:

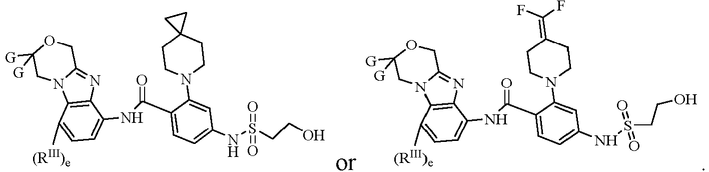

In other embodiments, the compound of formula (I-B-6) is:

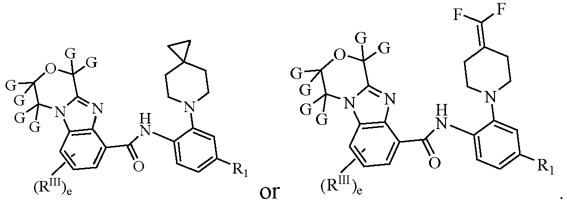

In some preferable embodiments, the compound of formula (I-B-6) is:

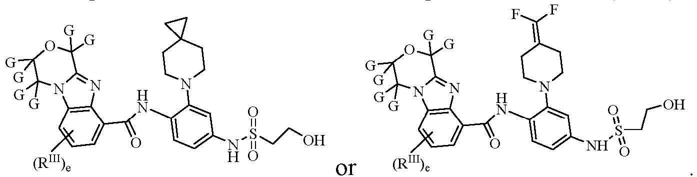

In some more preferable embodiments, the compound of formula (I-B-6) is:

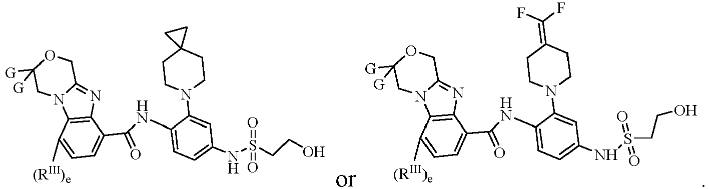

In some of such embodiments, preferably, R^{III} is selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, - NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl); and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or - N(C1-C6 alkyl)(C1-C6 alkyl), or two G attached to the same carbon atom form an oxo group (=O). In some further preferable embodiments, each e is 1.

In some embodiments, the compound of formula (I) is a compound of formula (I-B-7) 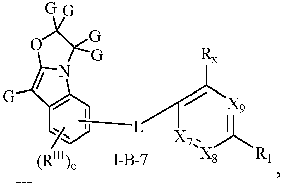 wherein the optional ranges of L, R_{X}, R₁, X₇, X₈, X₉, G, R^{III}, and e are as defined above; preferably, the compound as represented by formula (I-B-7) is

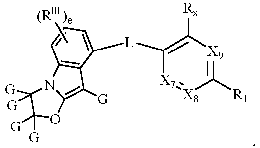

In some embodiments, the compound of formula (I) is a compound of formula (I-B-8) 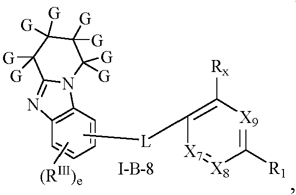 wherein the optional ranges of L, R_{X}, R₁, X₇, X₈, X₉, G, R^{III}, and e are as defined above; preferably, the compound as represented by formula (I-B-8) is

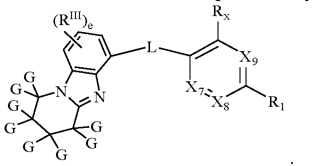

In some embodiments, the compound as represented by formula (I-B-8) is:

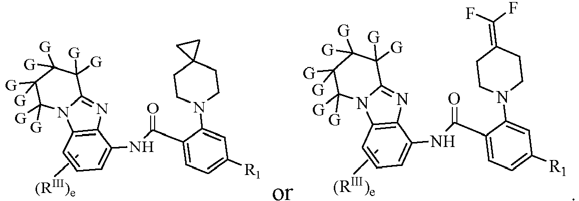

In some preferable embodiments, the compound as represented by formula (I-B-8) is:

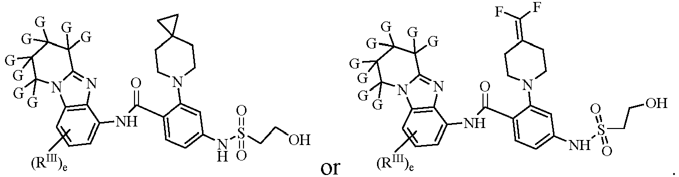

In some more preferable embodiments, the compound as represented by formula (I-B-8) is:

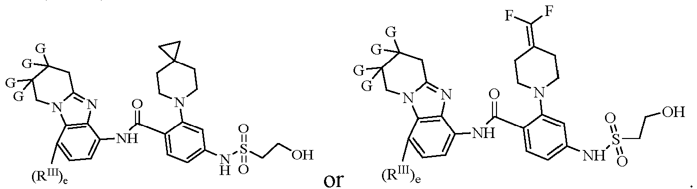

In other embodiments, the compound as represented by formula (I-B-8) is:

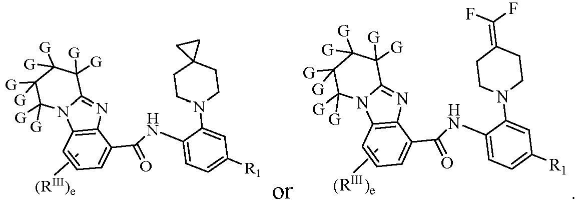

In some preferable embodiments, the compound as represented by formula (I-B-8) is:

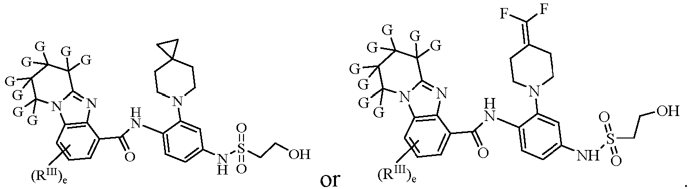

In some more preferable embodiments, the compound as represented by formula (I-B-8) is:

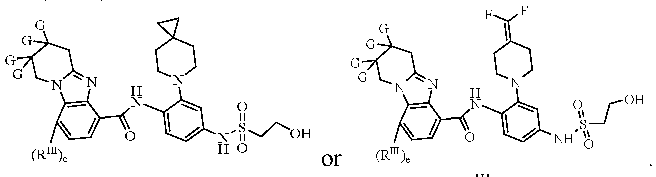

In some of such embodiments, preferably, R^{III} is selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, - NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl); and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or - N(C1-C6 alkyl)(C1-C6 alkyl), or two G attached to the same carbon atom form an oxo group (=O). In further preferable embodiments, each e is 1.

In some embodiments, the compound of formula (I) is a compound of formula (I-B-9) 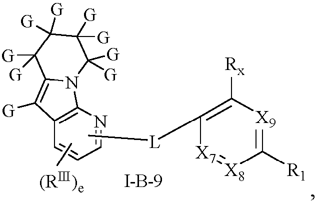 wherein the optional ranges of L, R_{X}, R₁, X₇, X₈, X₉, G, R^{III}, and e are as defined above; preferably, the compound as represented by formula (I-B-9) is 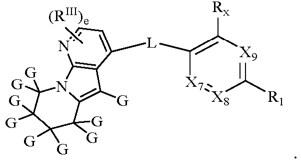 In some embodiments, the compound of formula (I) is a compound of formula (I-B-10) 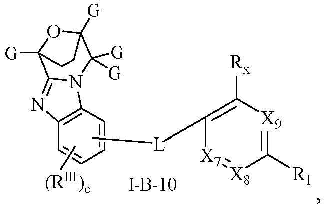 wherein the optional ranges of L, R_{X}, R₁, X₇, X₈, X₉, G, R^{III}, and e are as defined above; preferably, the compound as represented by formula (I-B-10) is

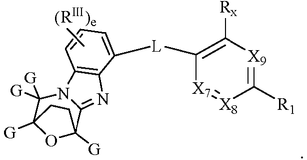

In some embodiments, the compound of formula (I) is a compound of formula (I-B-11) 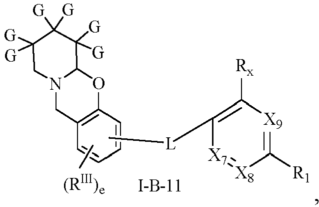 wherein the optional ranges of L, R_{X}, R₁, X₇, X₈, X₉, G, R^{III}, and e are as defined above; preferably, the compound as represented by formula (I-B-11) is

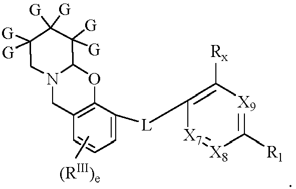

In some embodiments, the compound of formula (I) is a compound of formula (I-B-12) 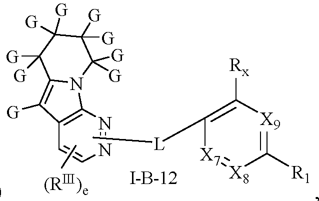 wherein the optional ranges of L, R_{X}, R₁, X₇, X₈, X₉, G, R^{III}, and e are as defined above; preferably, the compound as represented by formula (I-B-12) is

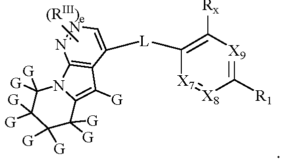

In some embodiments, the compound of formula (I) is a compound of formula (I-B-13) 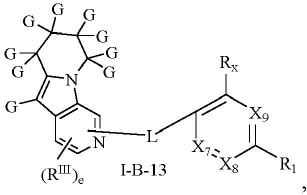 wherein the optional ranges of L, R_{X}, R₁, X₇, X₈, X₉, G, R^{III}, and e are as defined above; preferably, the compound as represented by formula (I-B-13) is

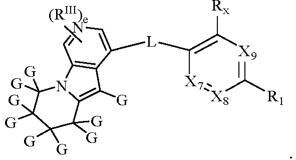

In some embodiments, the compound of formula (I) is a compound of formula (I-B-14) 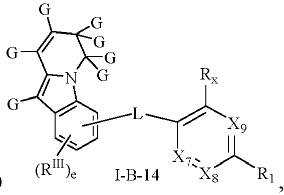 wherein the optional ranges of L, R_{X}, R₁, X₇, X₈, X₉, G, R^{III}, and e are as defined above; preferably, the compound as represented by formula (I-B-14) is

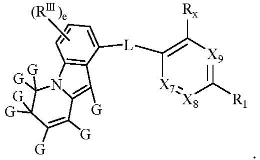

In some embodiments, the compound of formula (I) is a compound of formula (I-B-15) 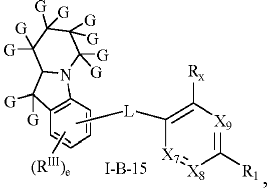 wherein the optional ranges of L, R_{X}, R₁, X₇, X₈, X₉, G, R^{III}, and e are as defined above; preferably, the compound as represented by formula (I-B-15) is

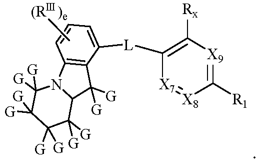

In some embodiments, the compound of formula (I) is a compound of formula (I-B-16) 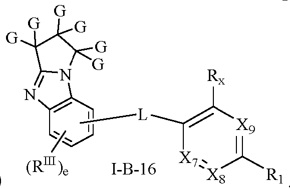 , wherein the optional ranges of L, R_{X}, R₁, X₇, X₈, X₉, G, R^{III}, and e are as defined above; preferably, the compound as represented by formula (I-B-16) is

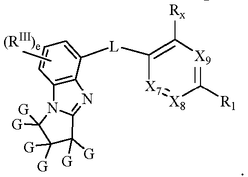

In some embodiments, the compound of formula (I) is selected from the group consisting of the following compounds:

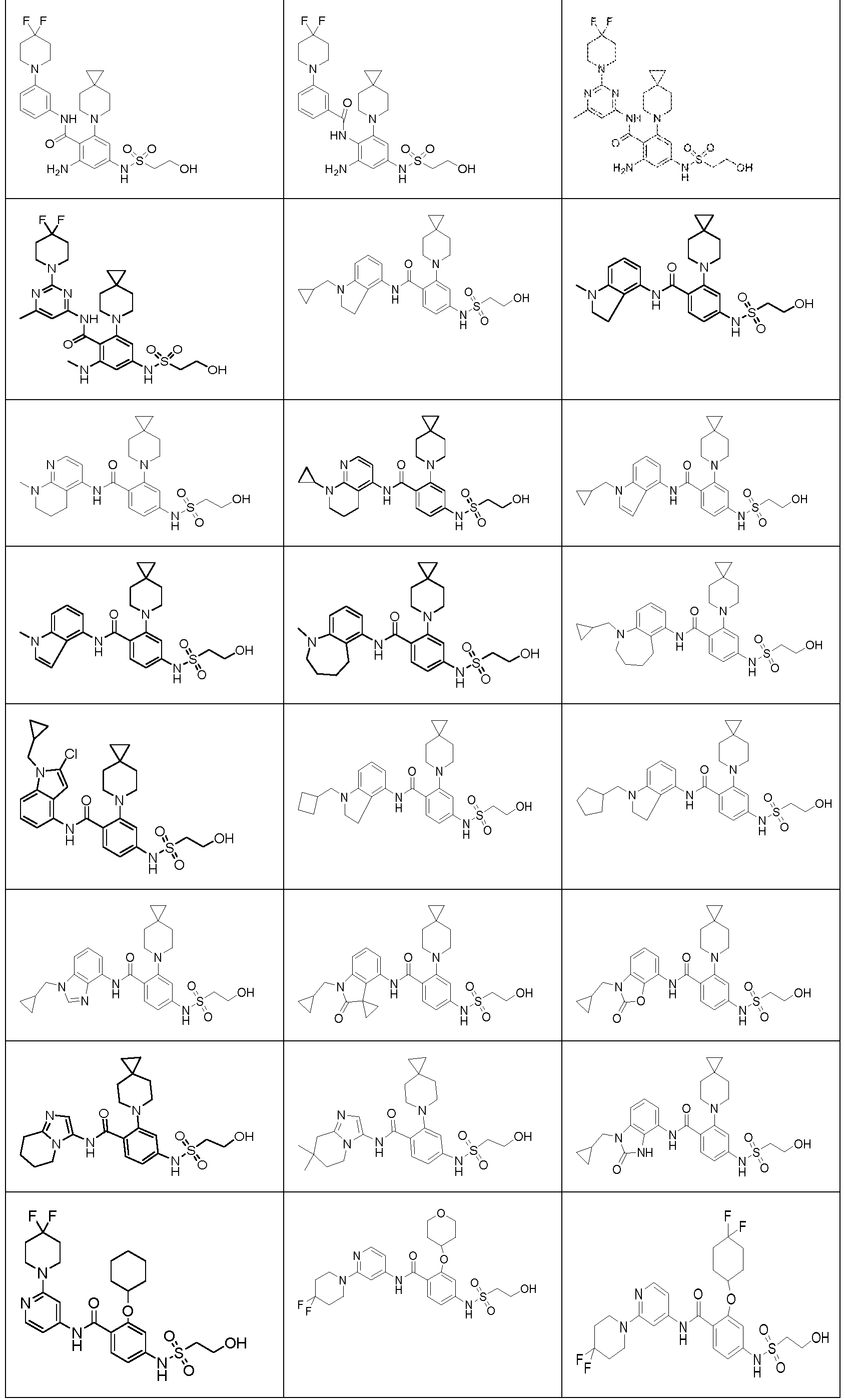

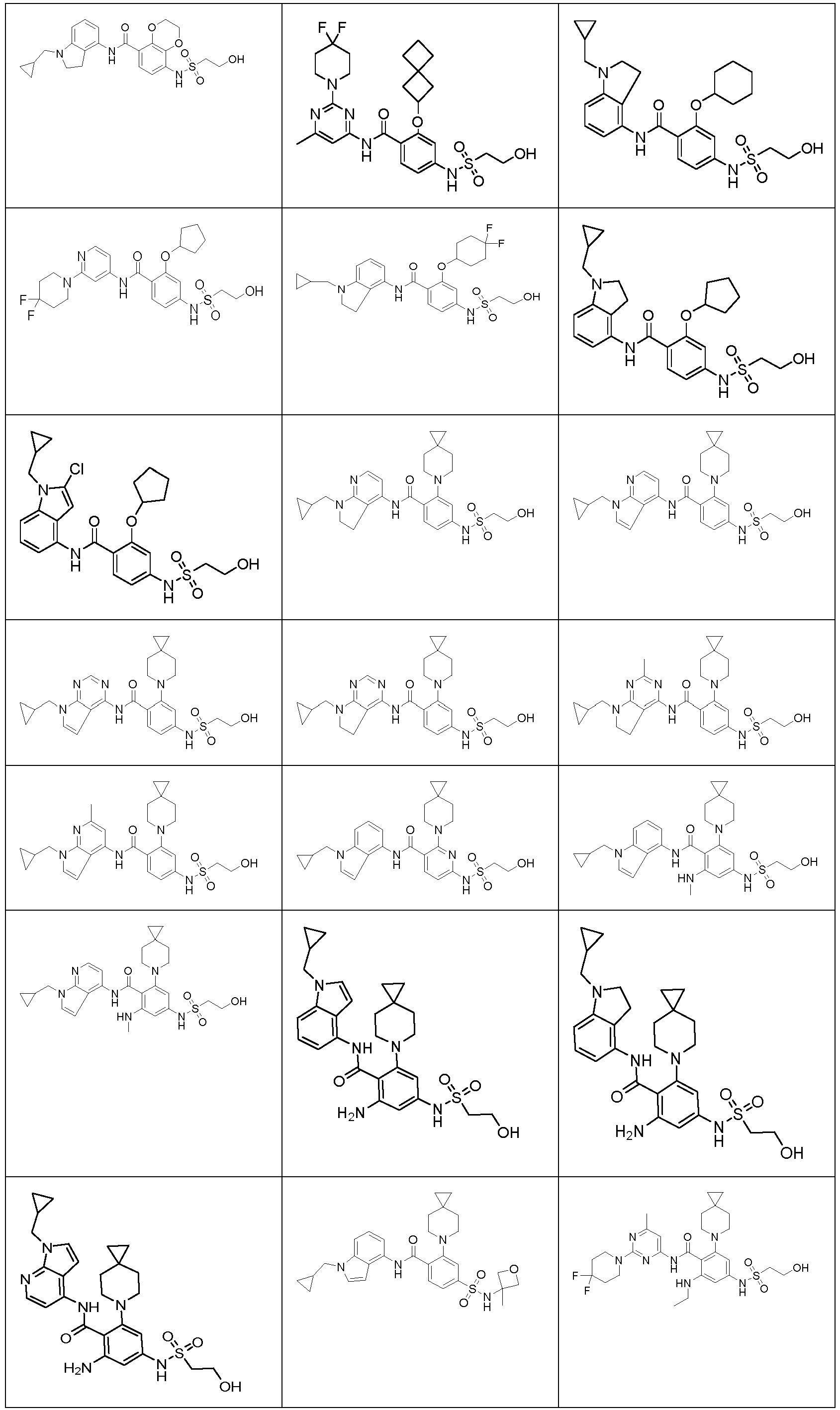

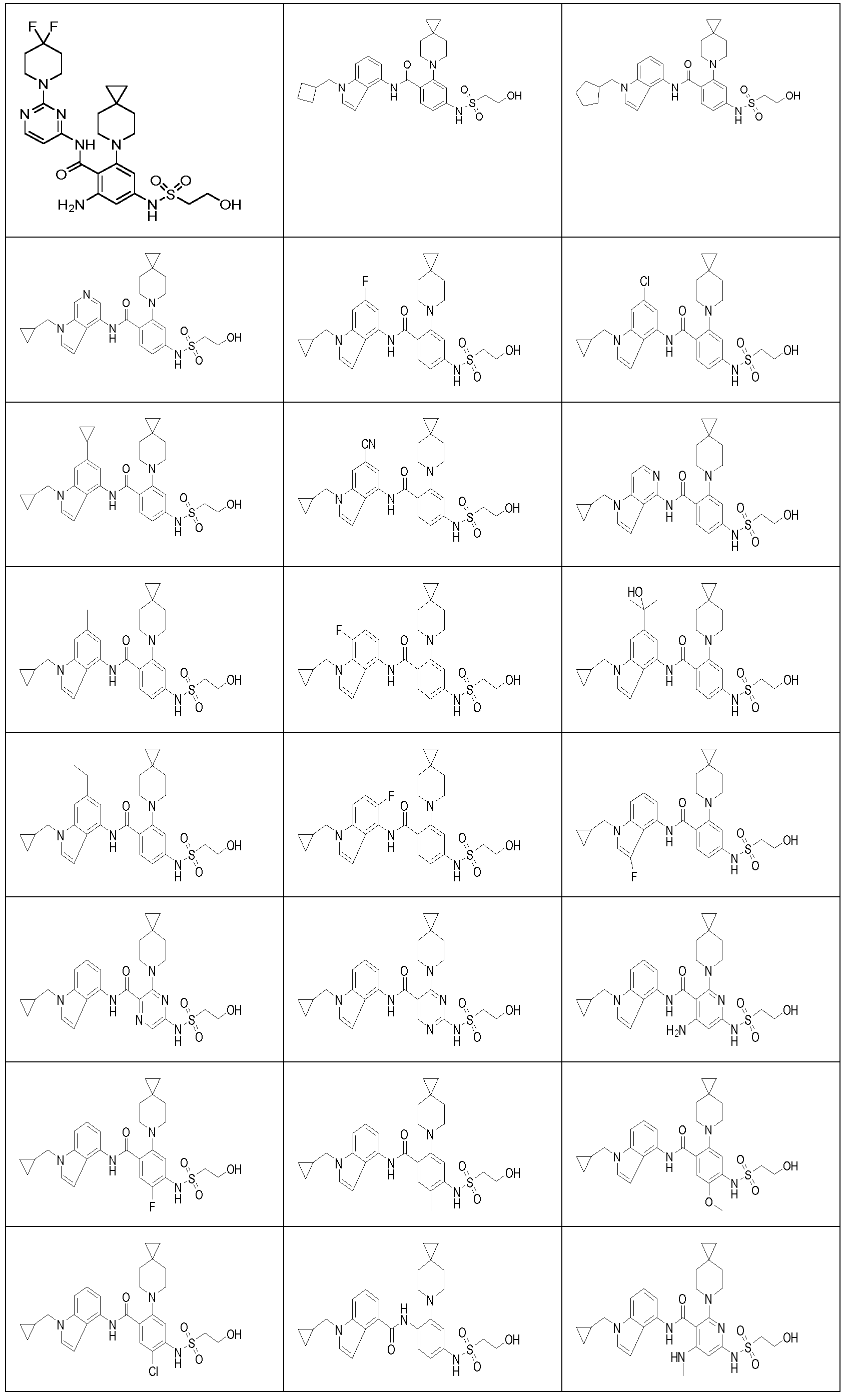

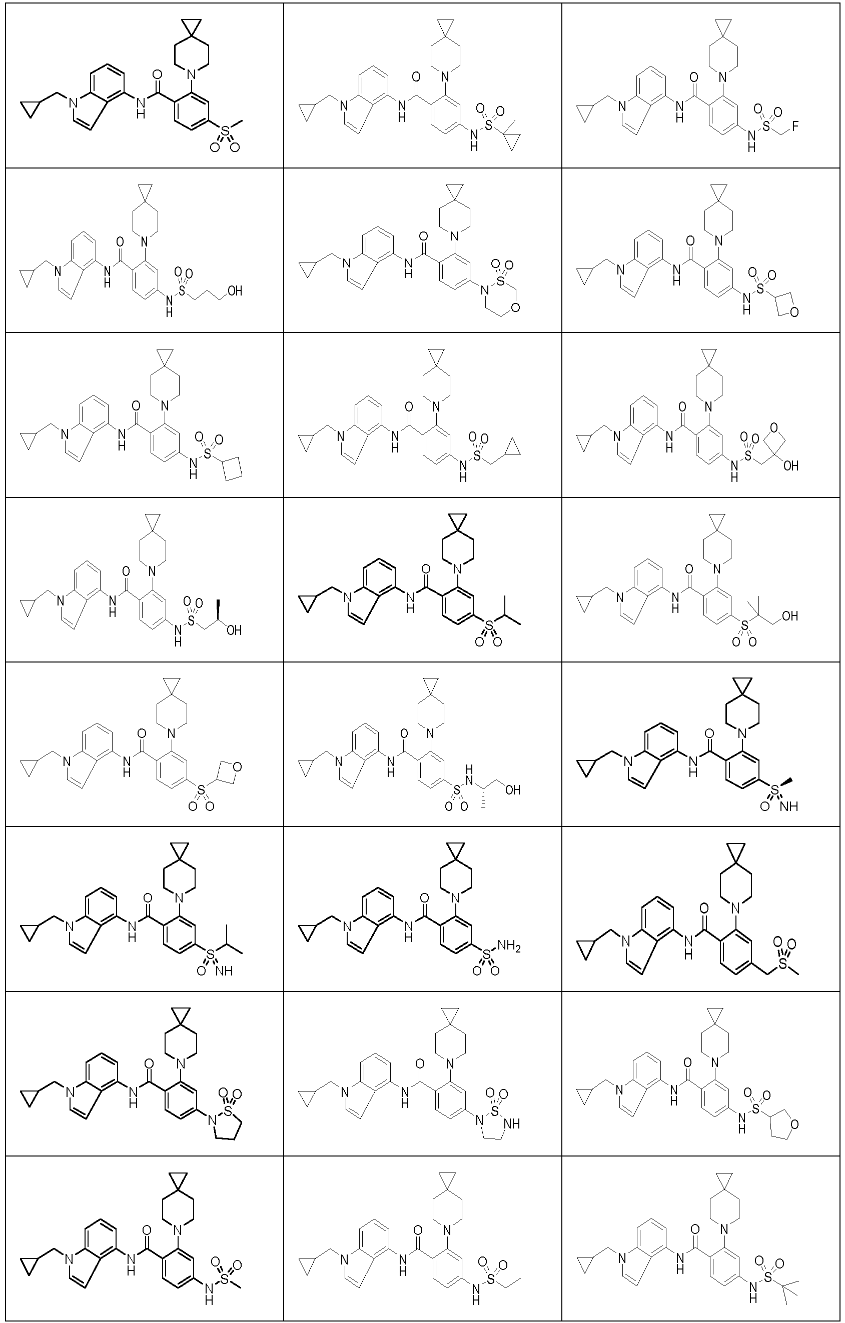

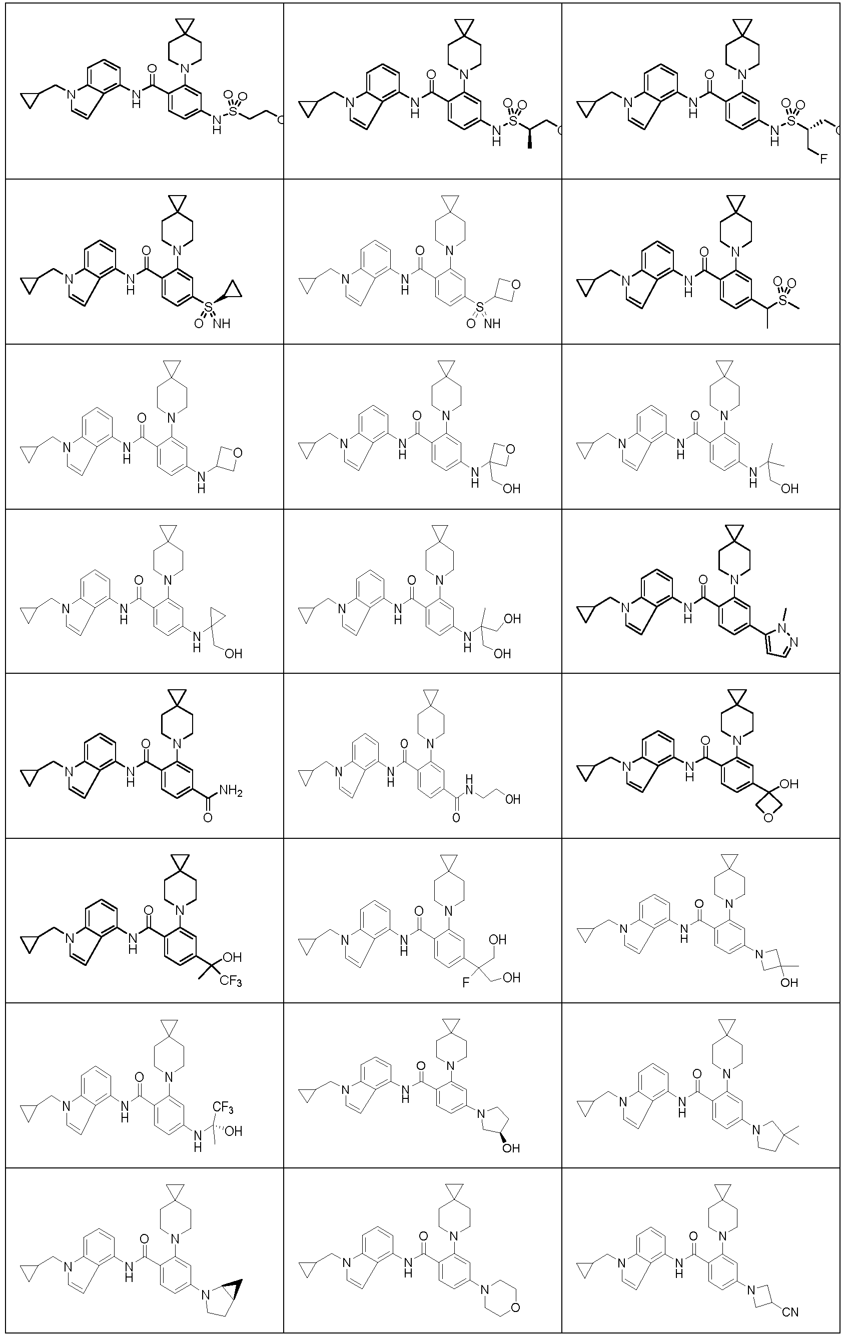

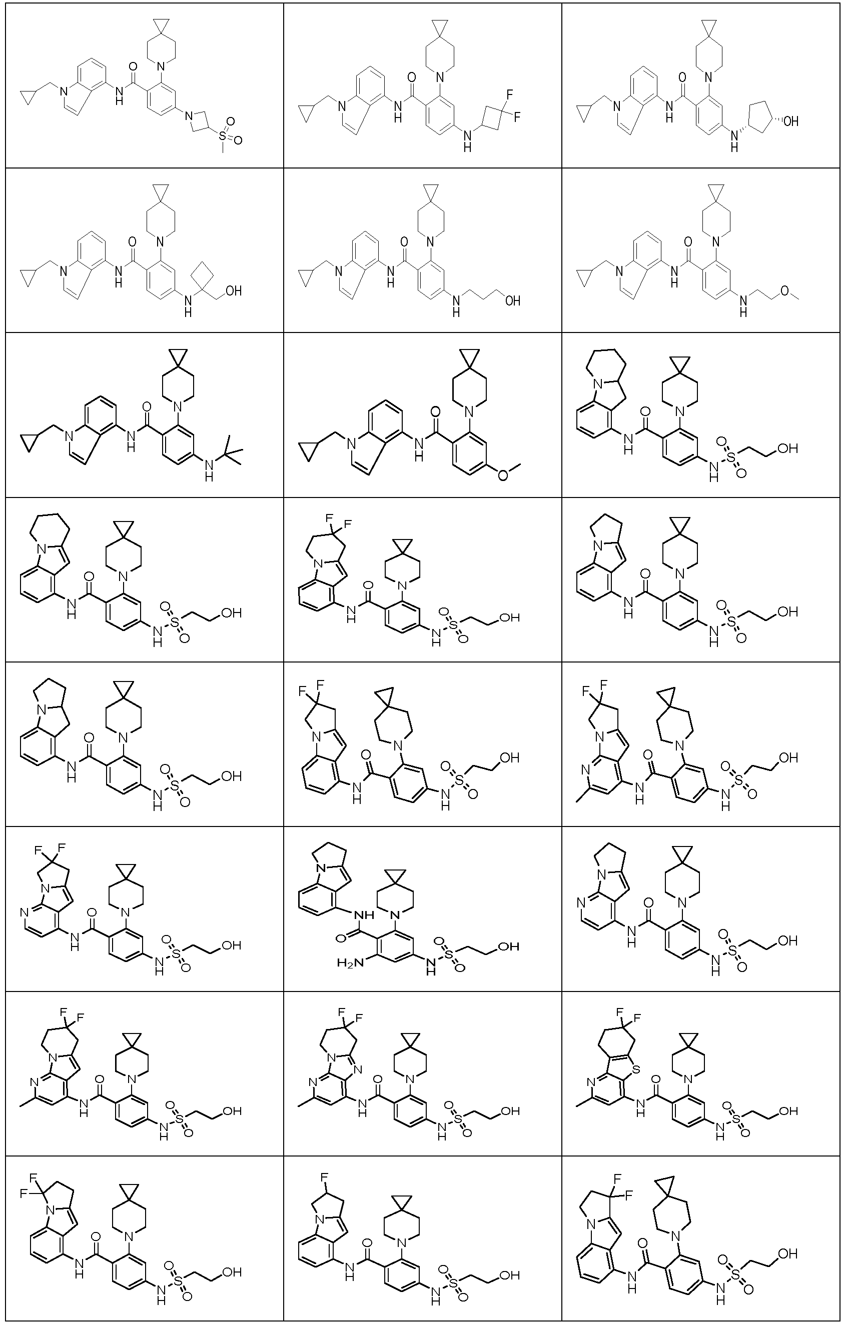

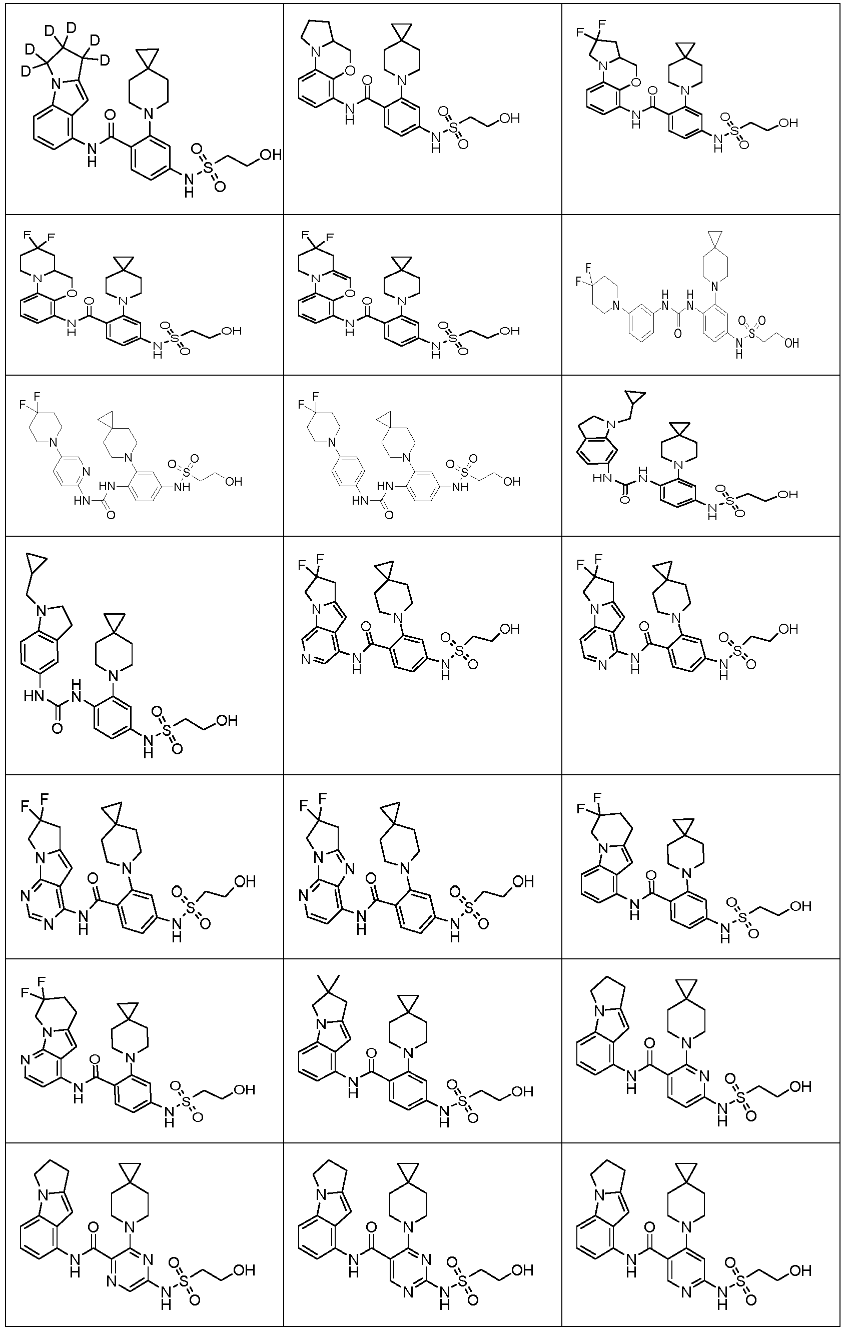

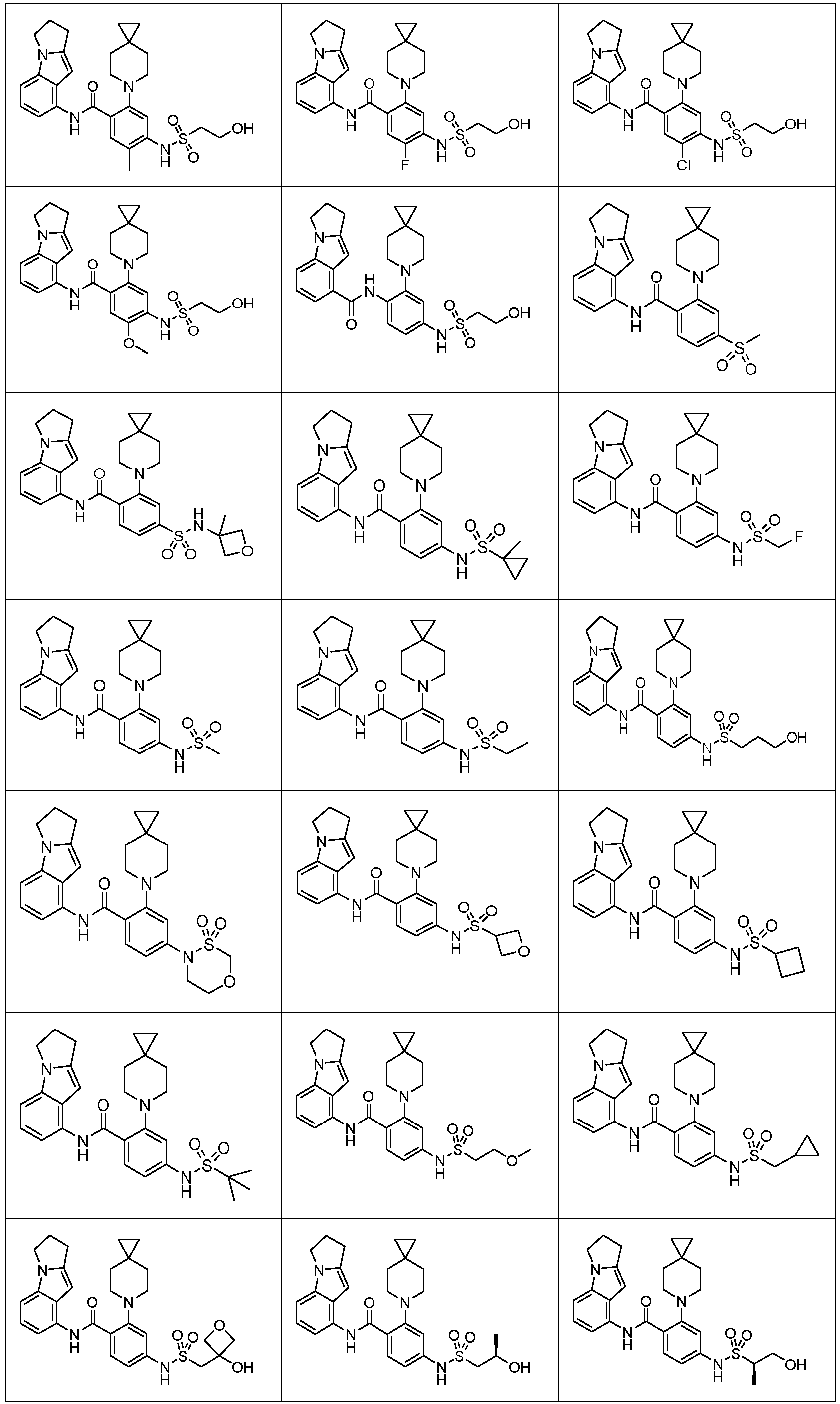

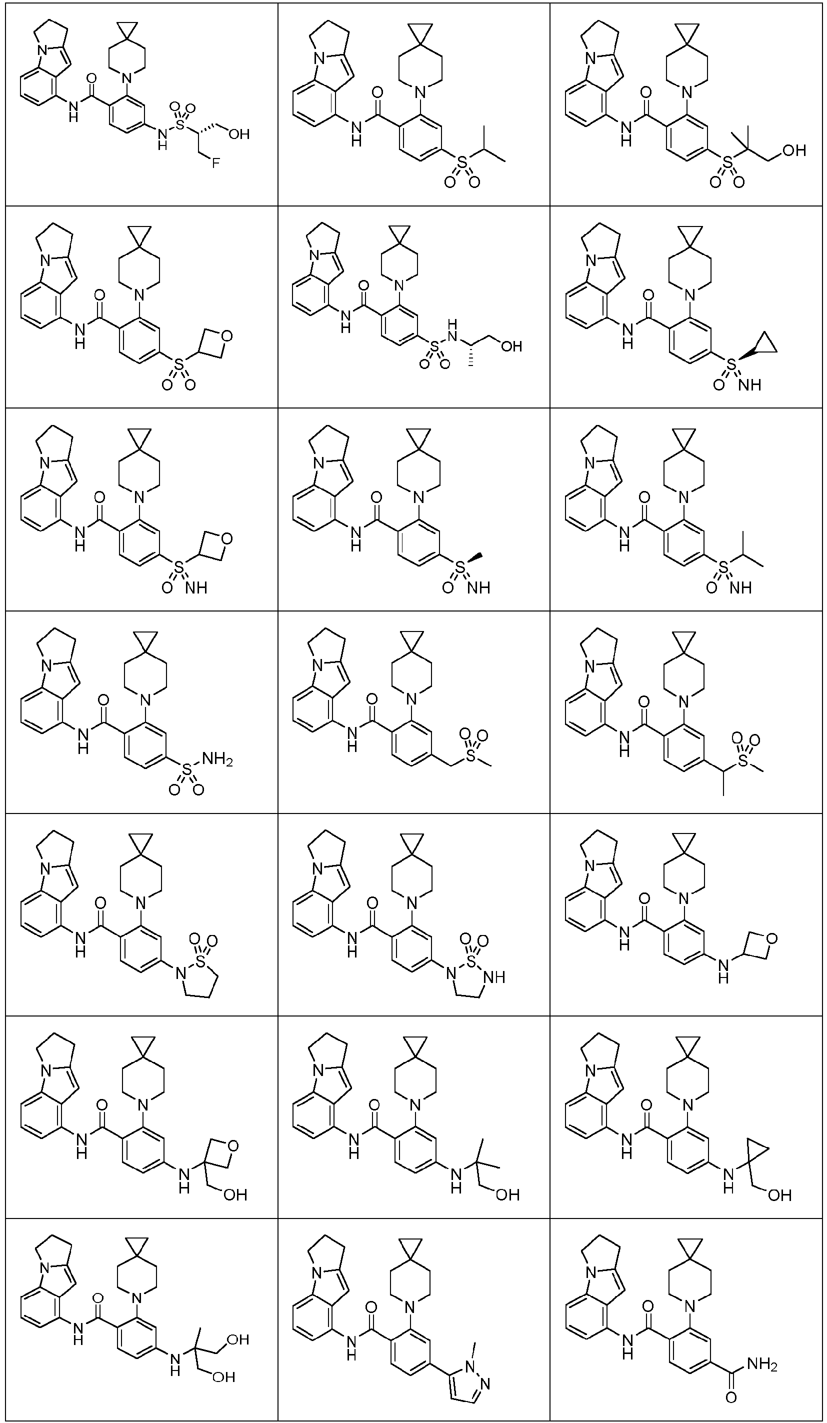

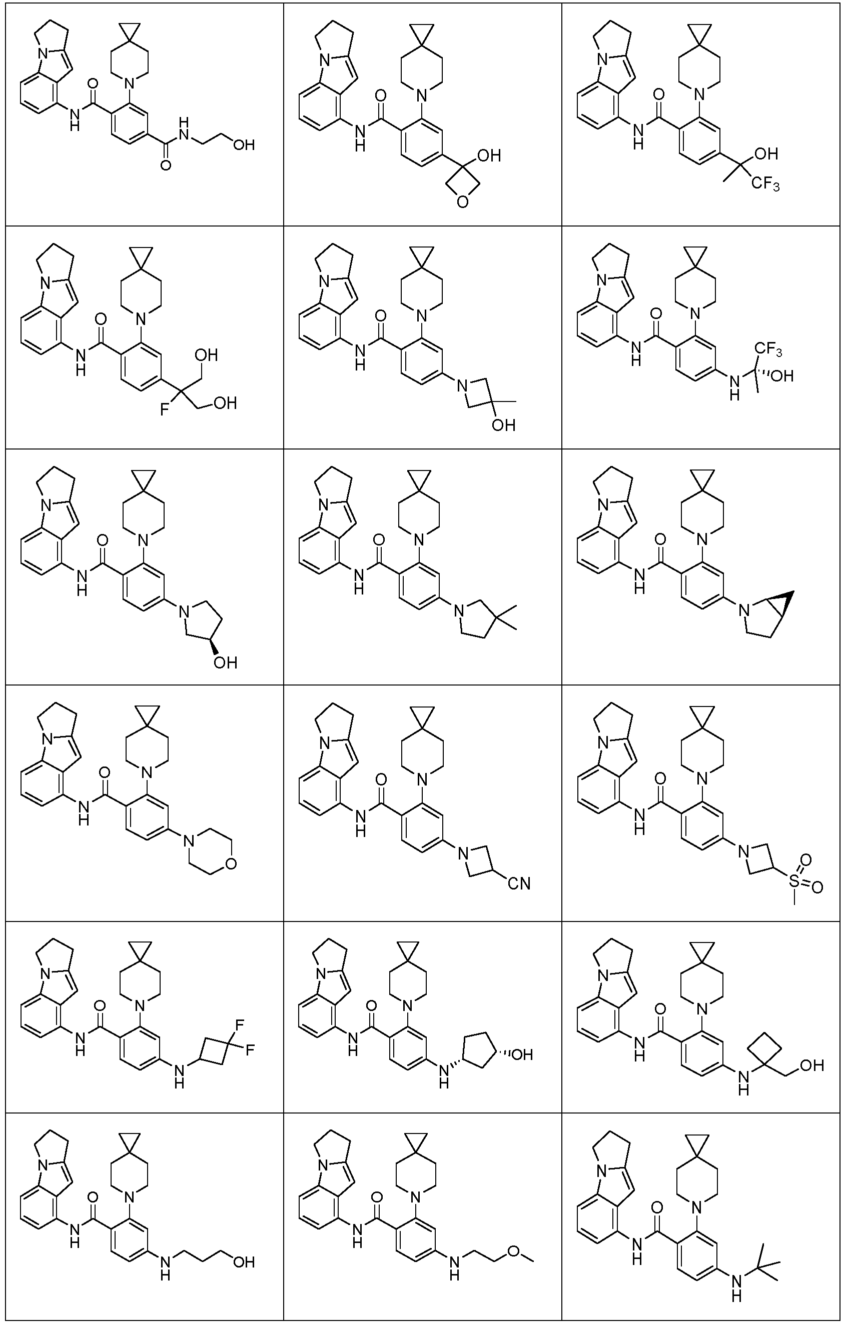

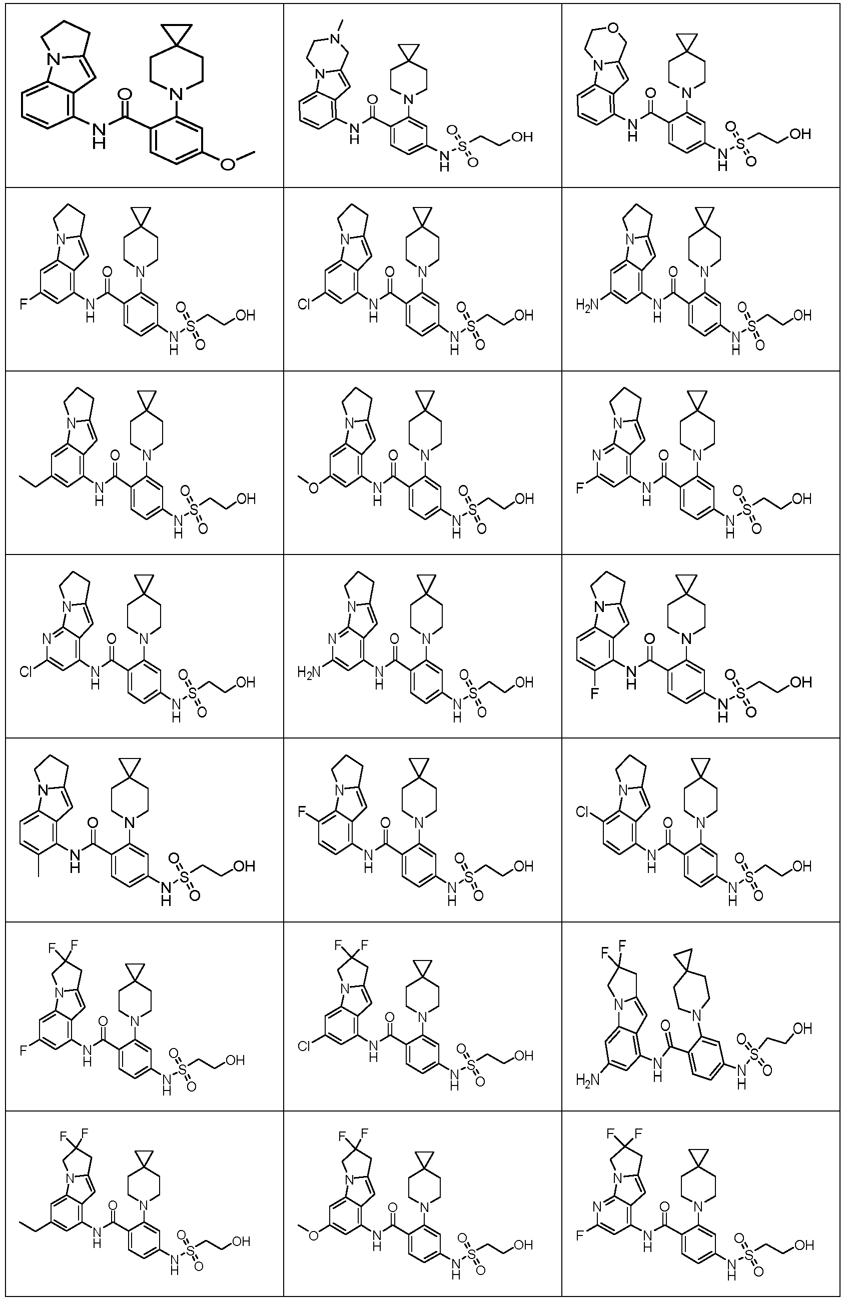

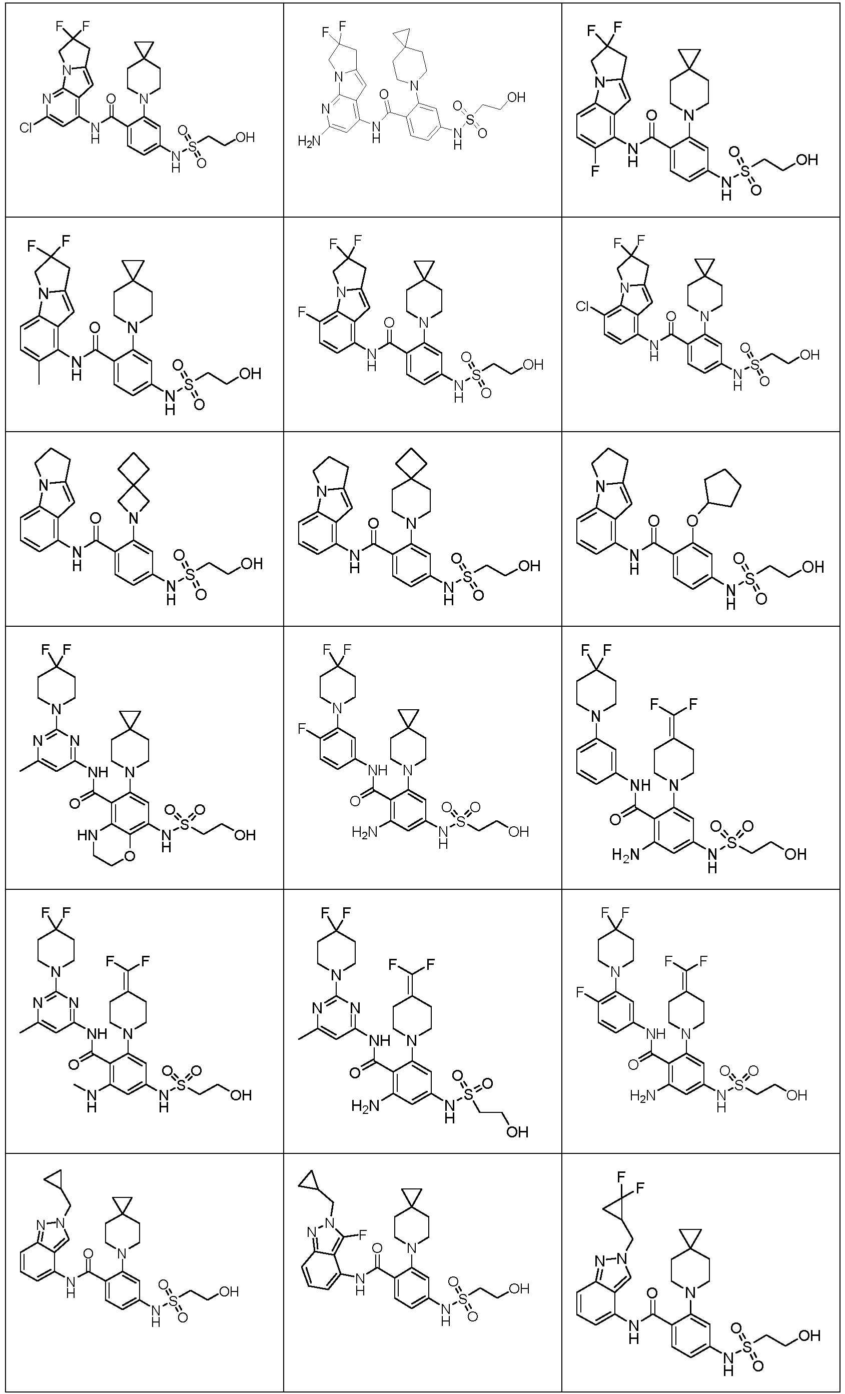

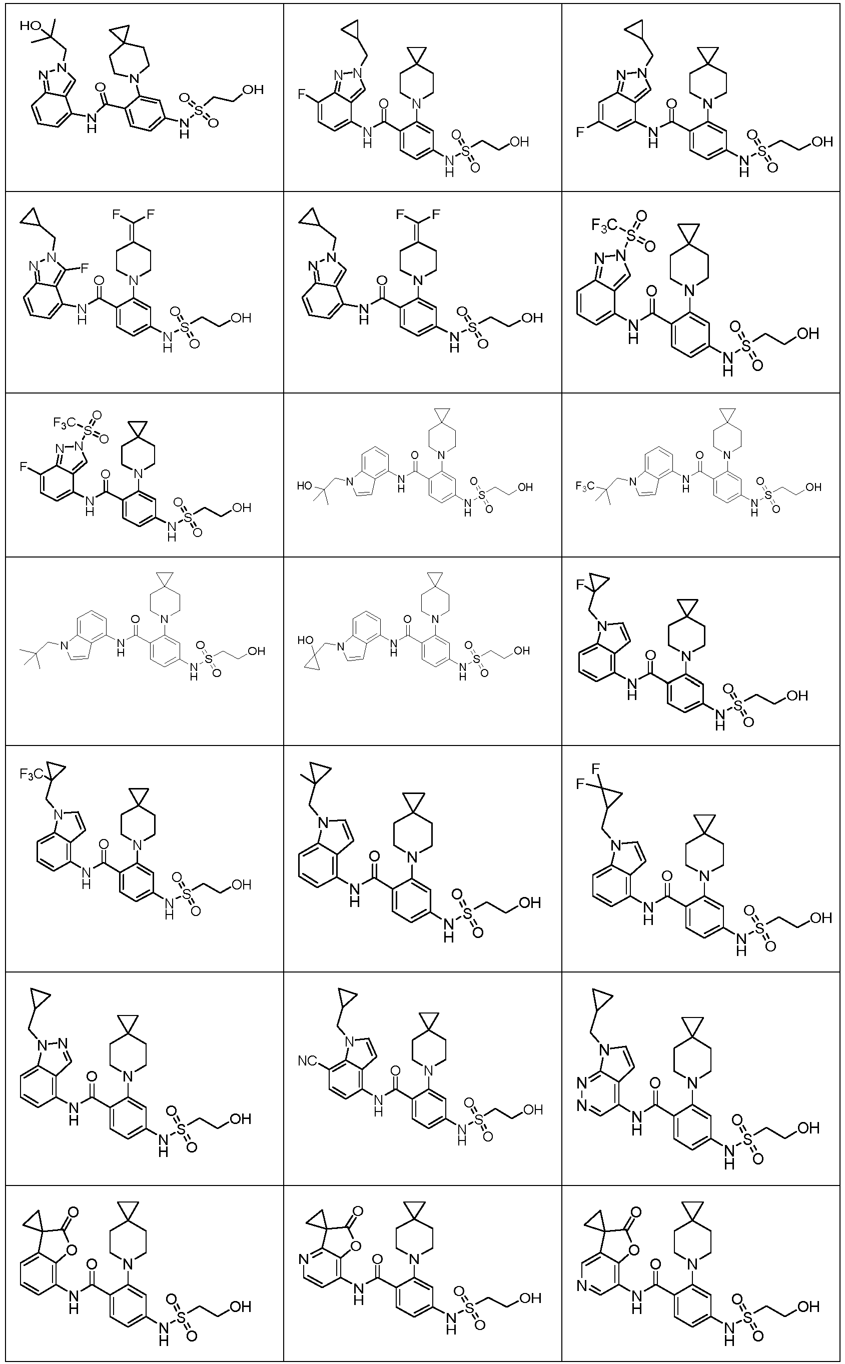

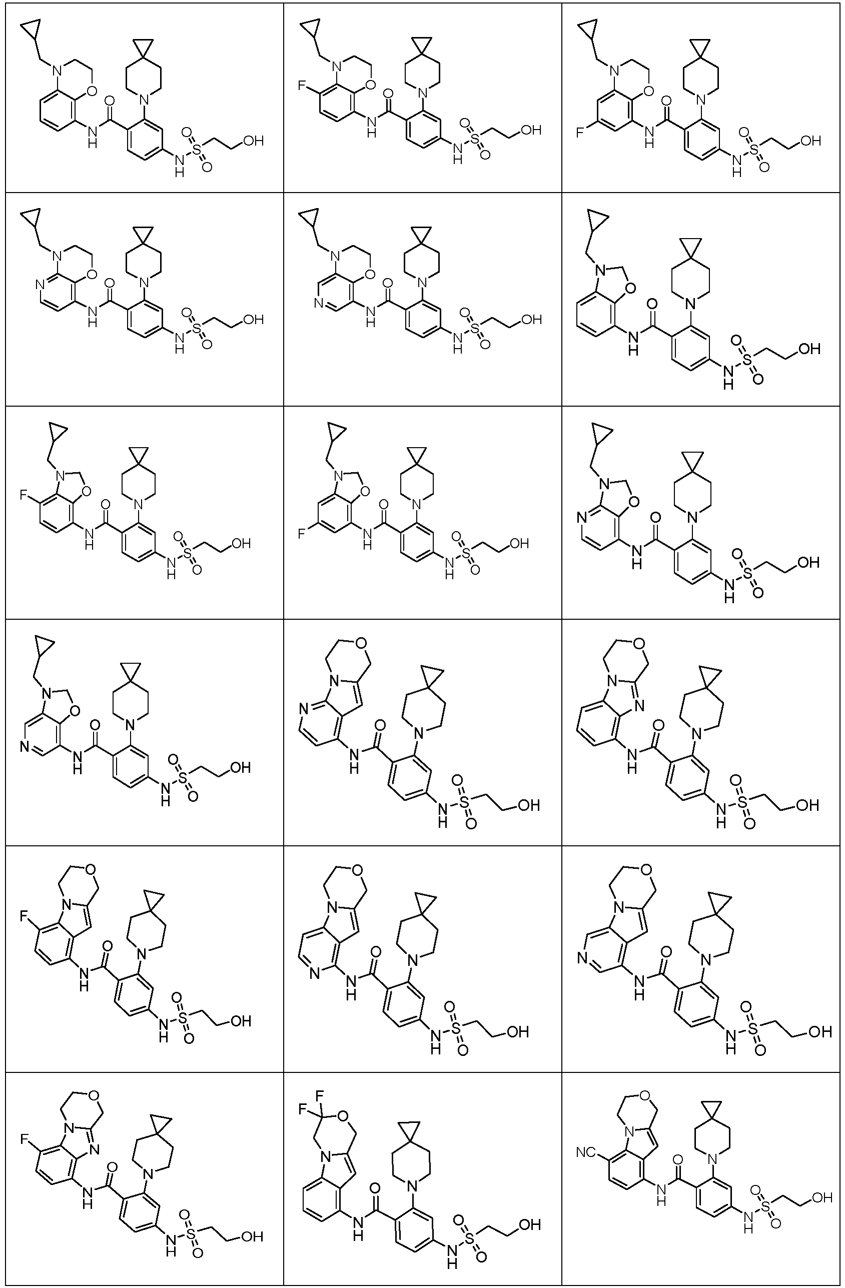

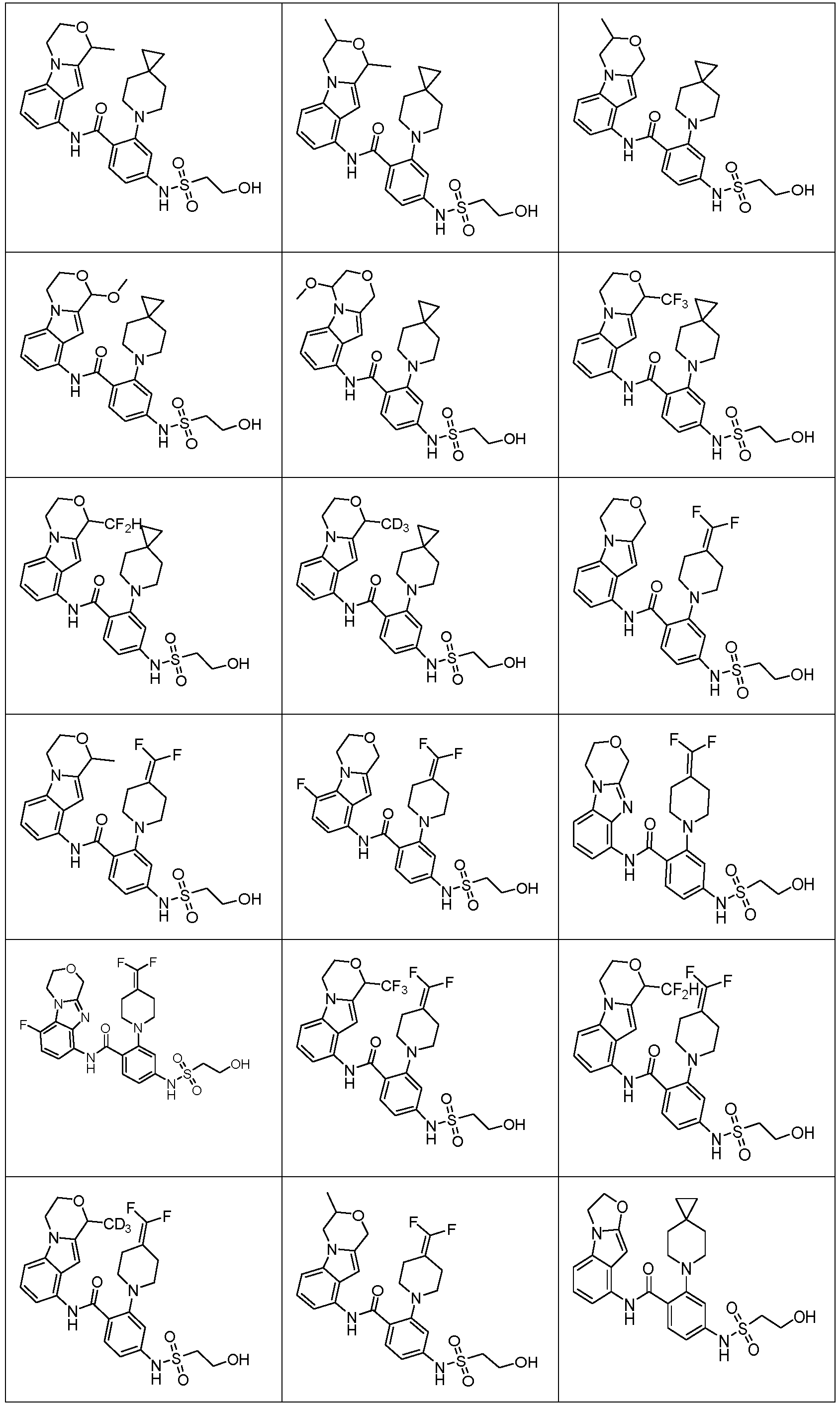

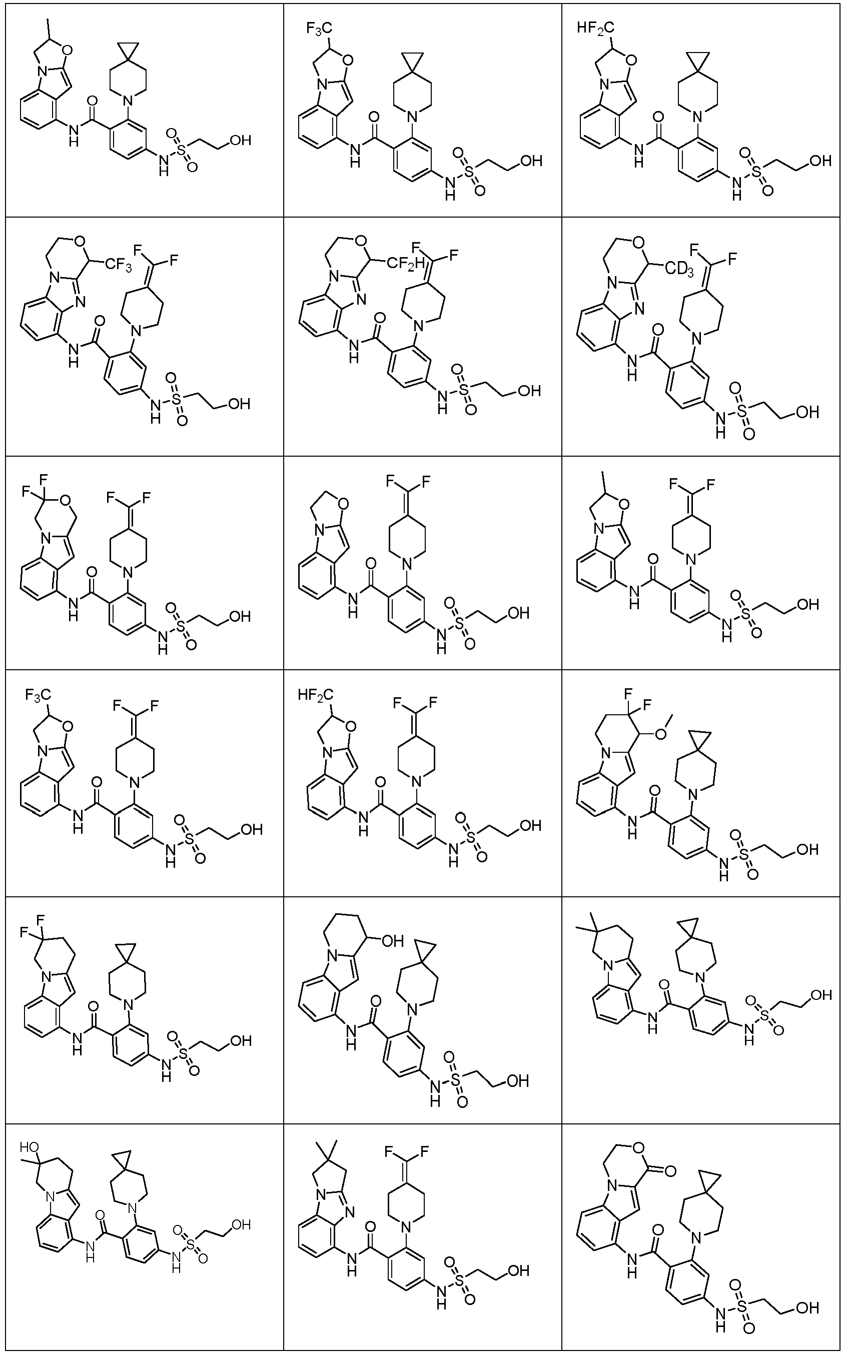

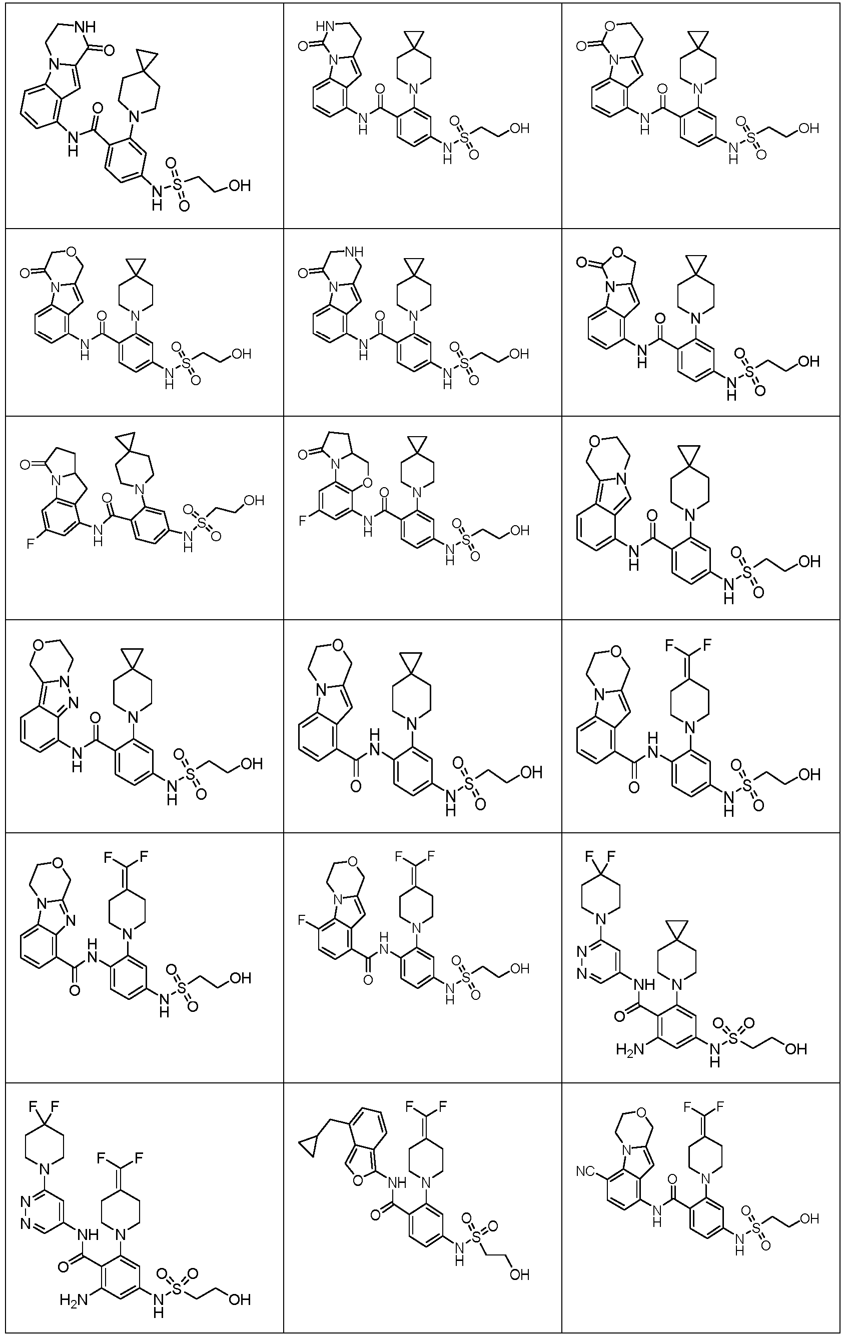

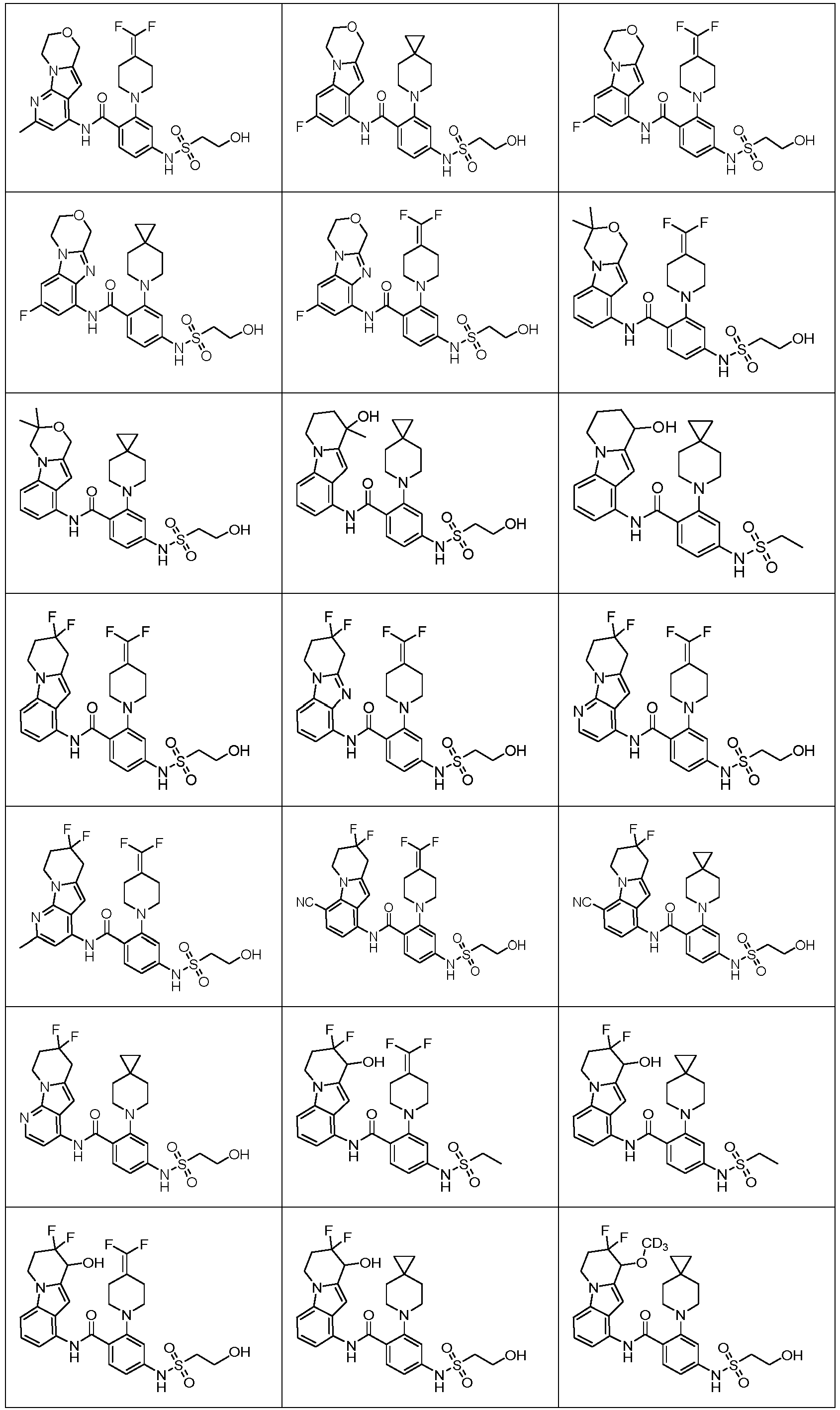

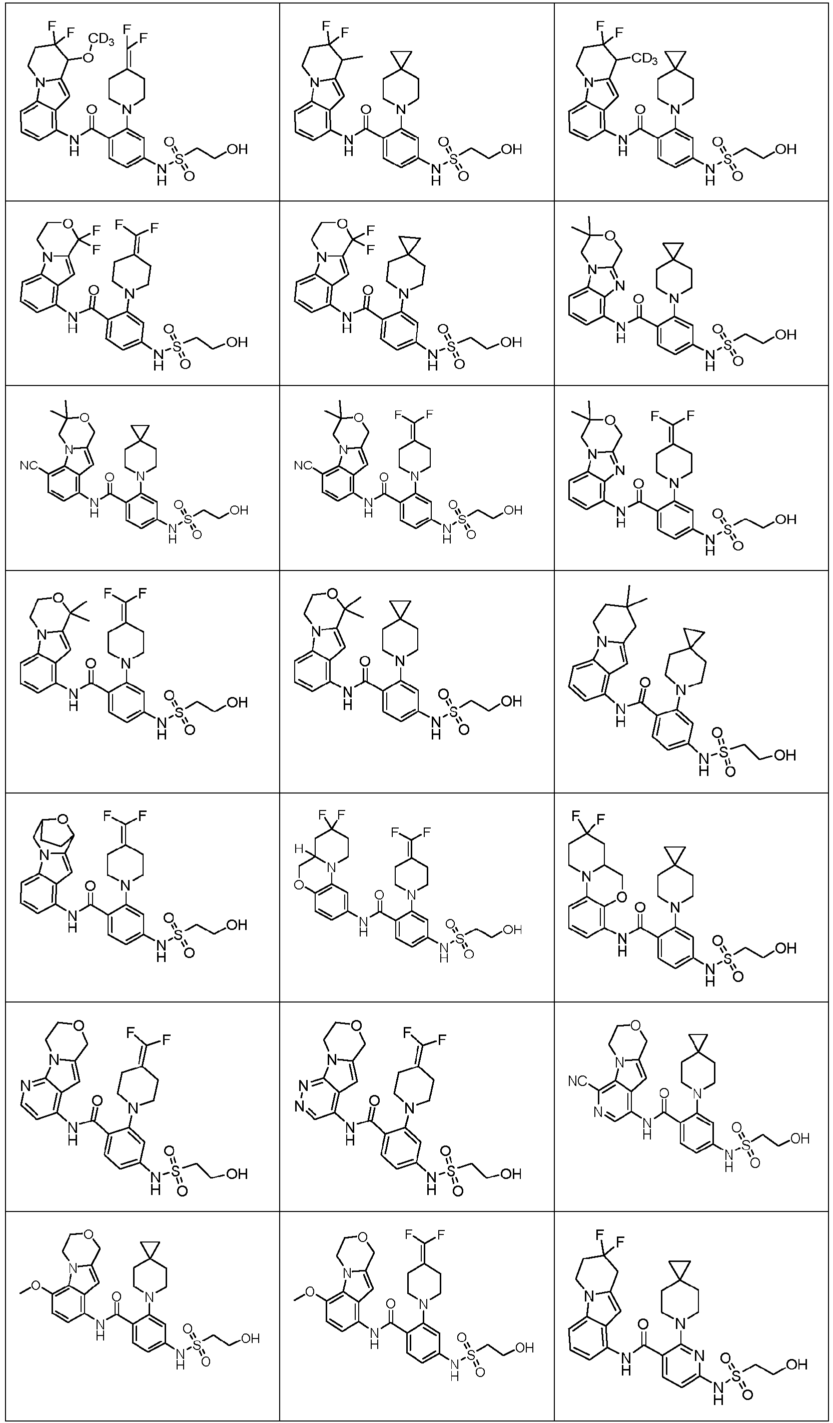

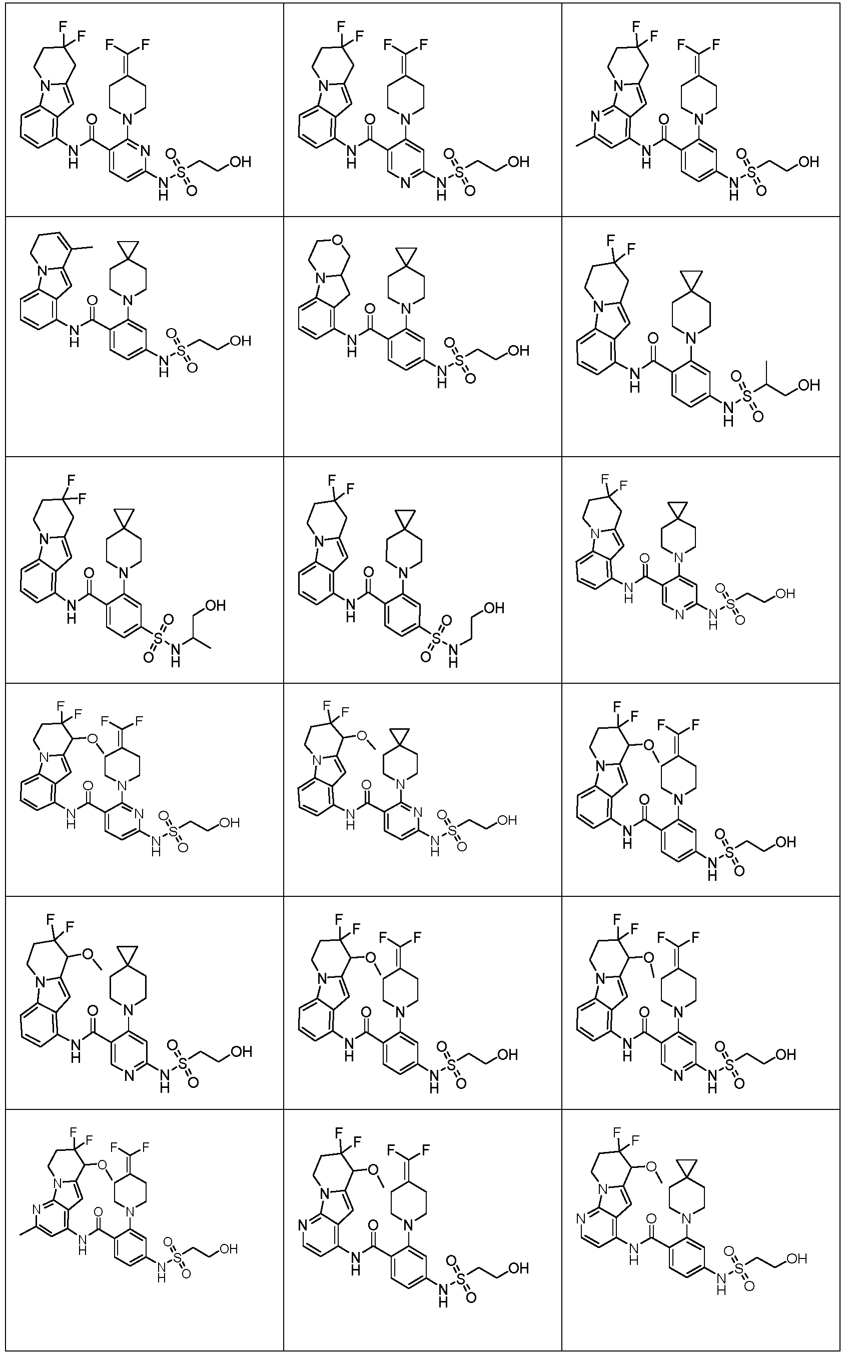

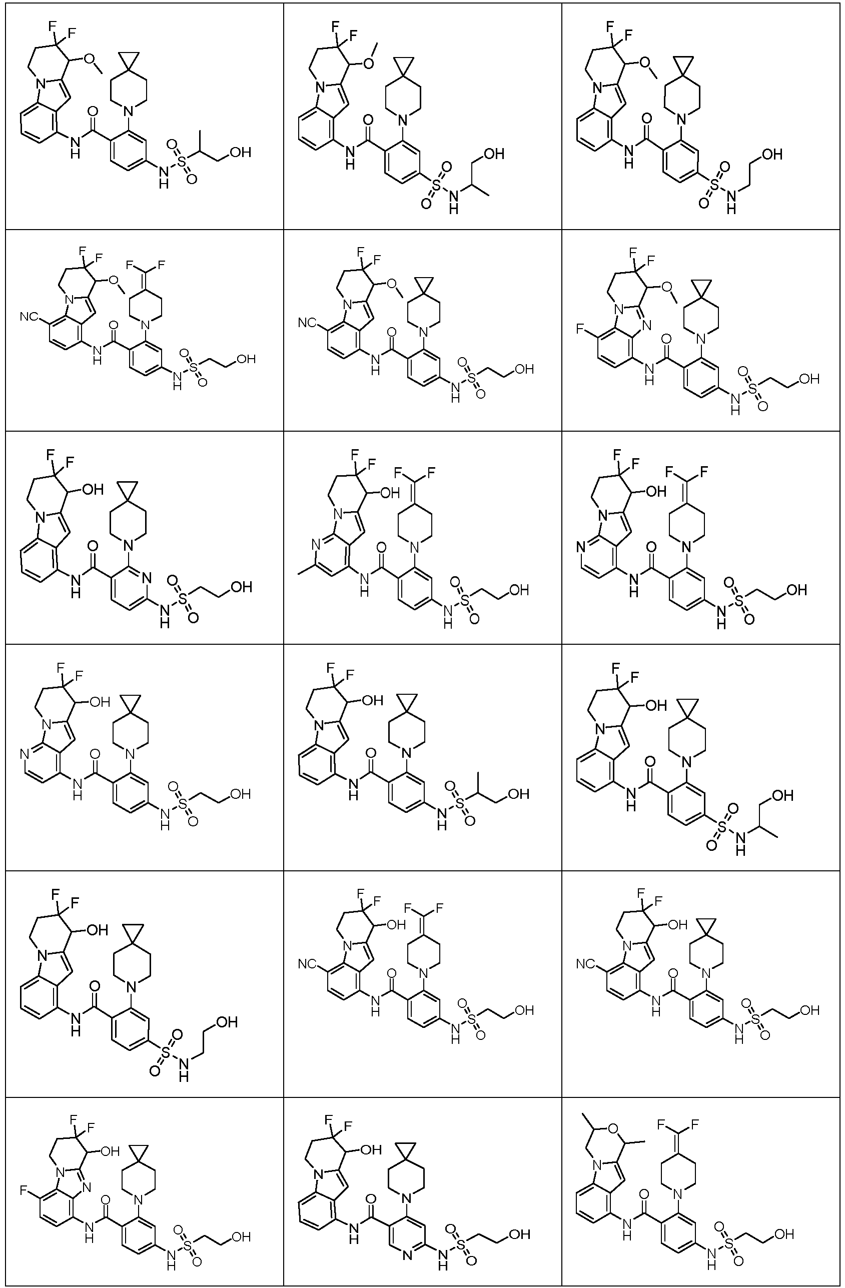

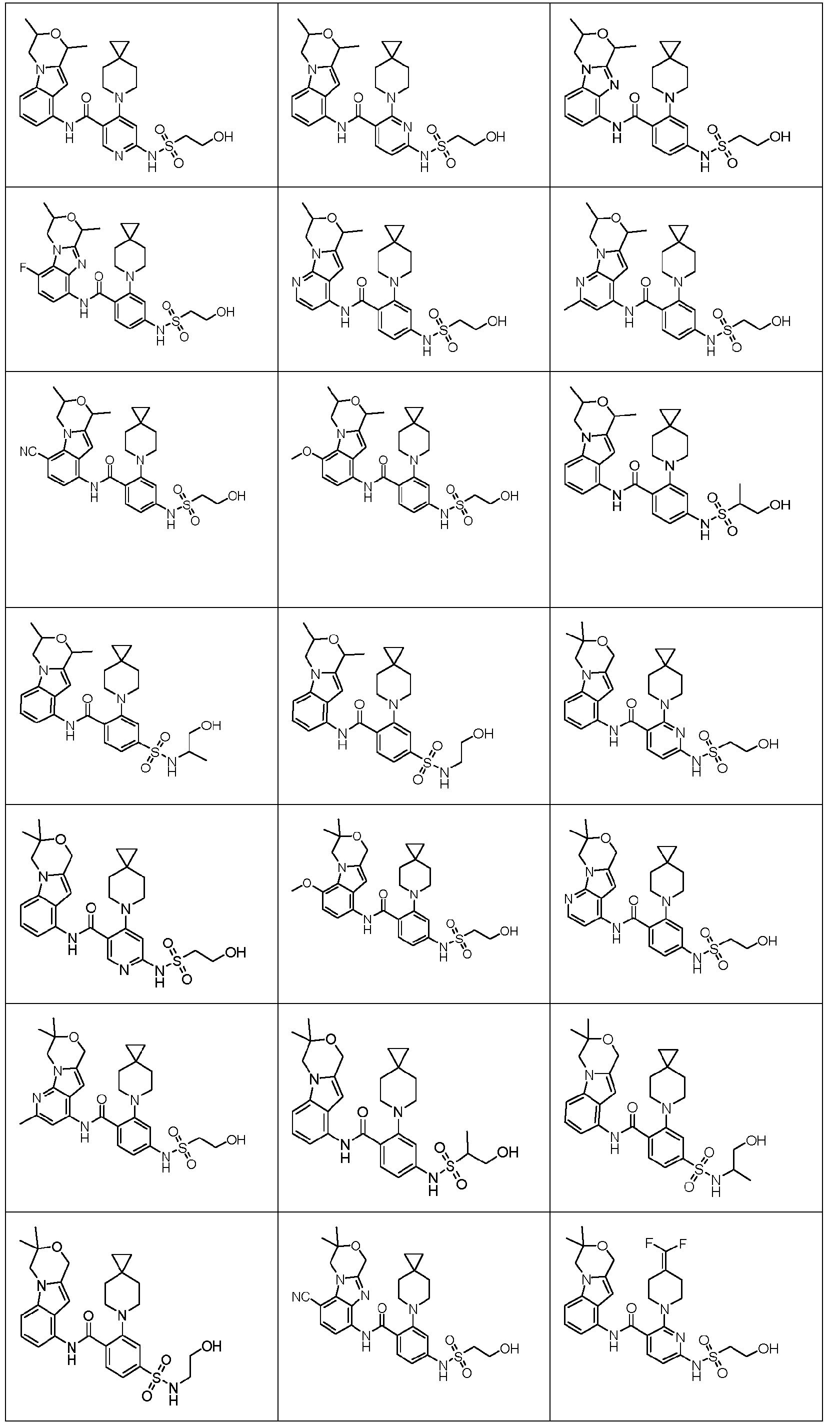

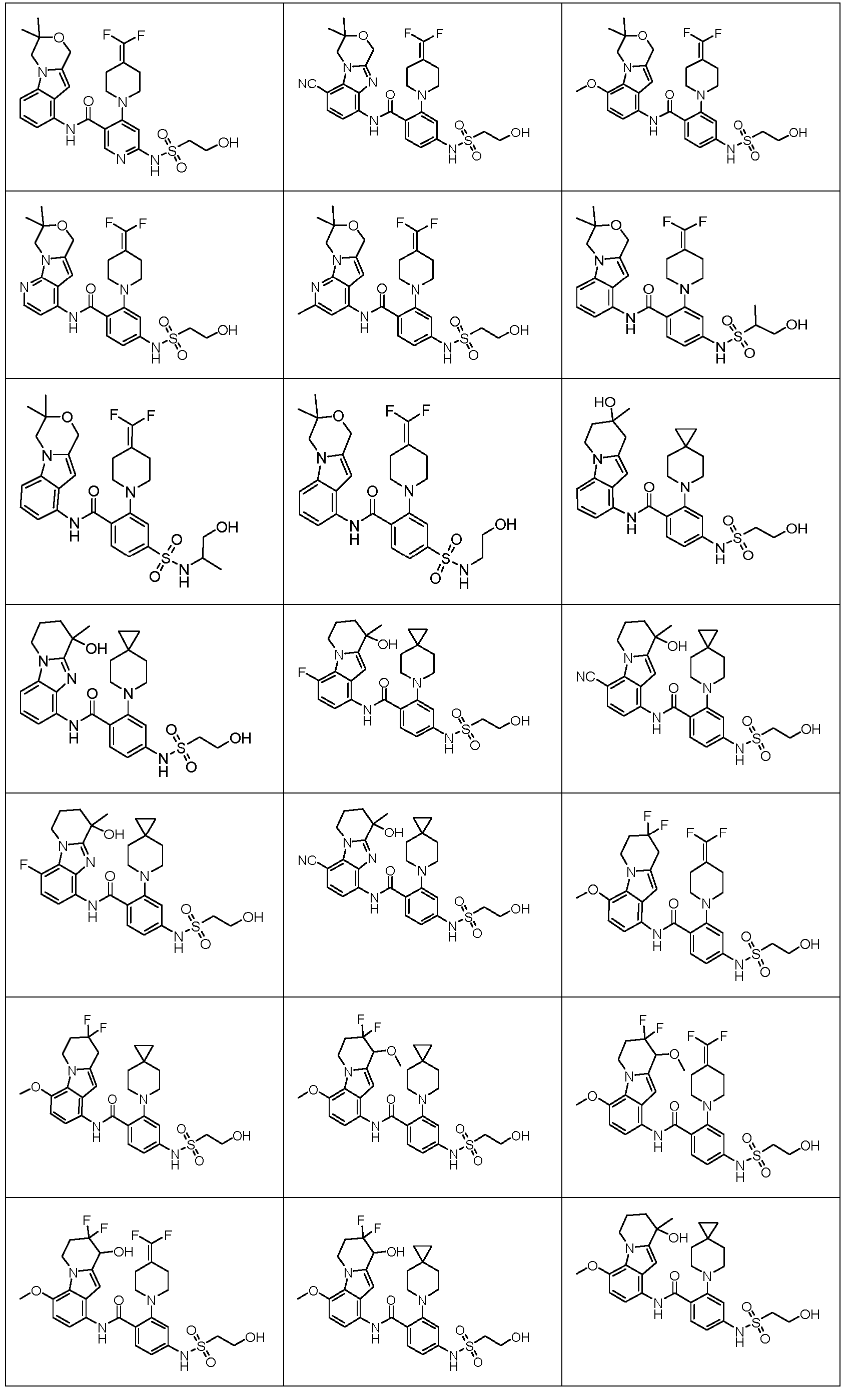

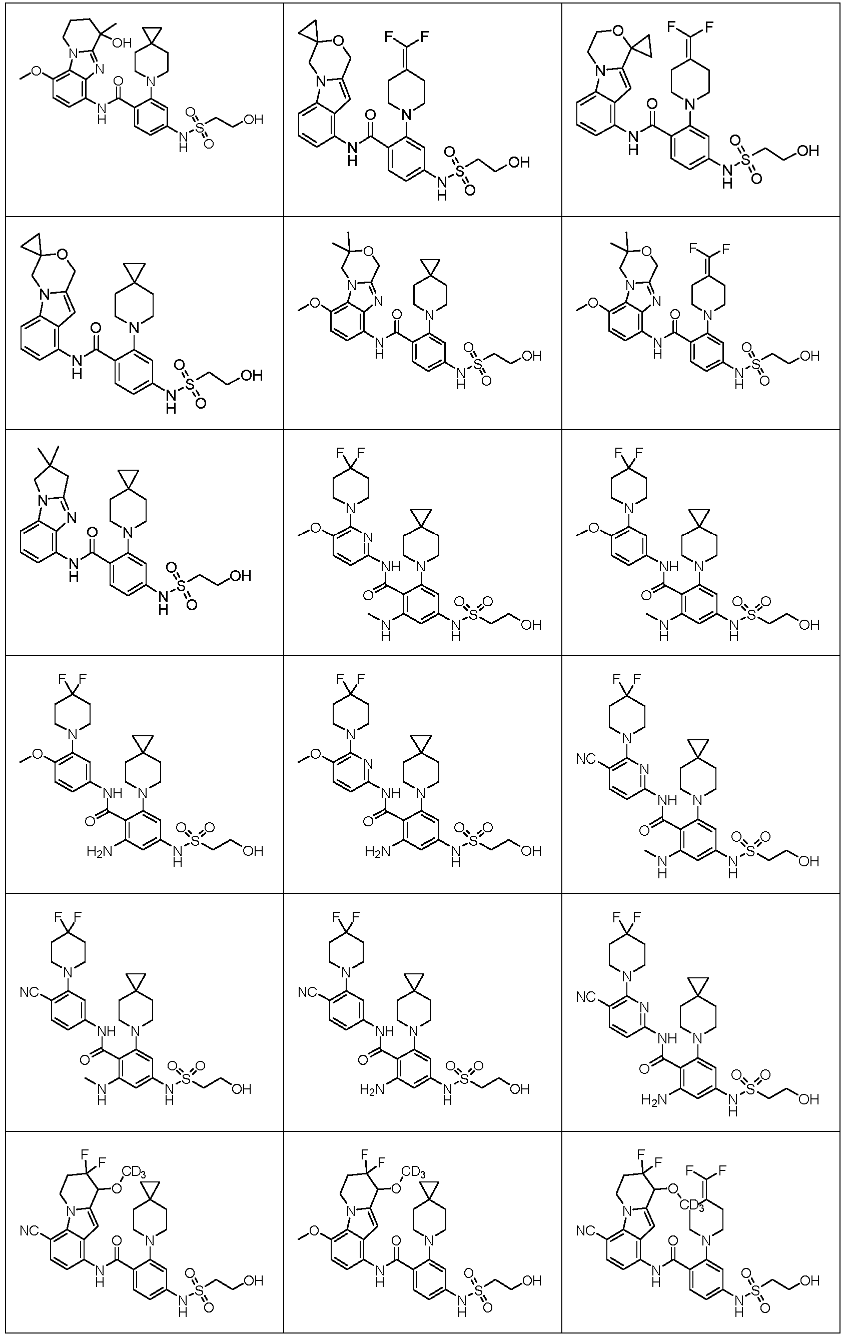

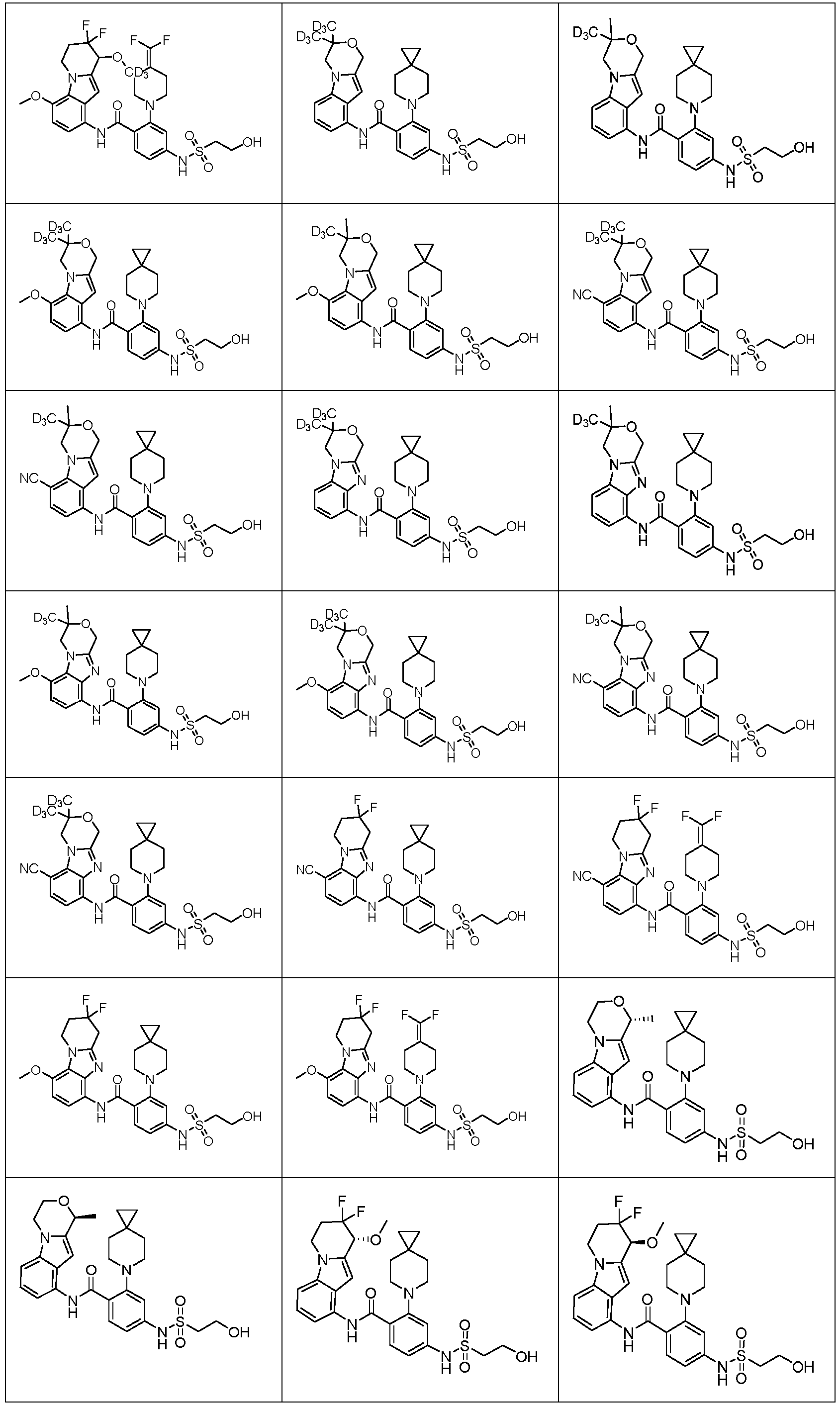

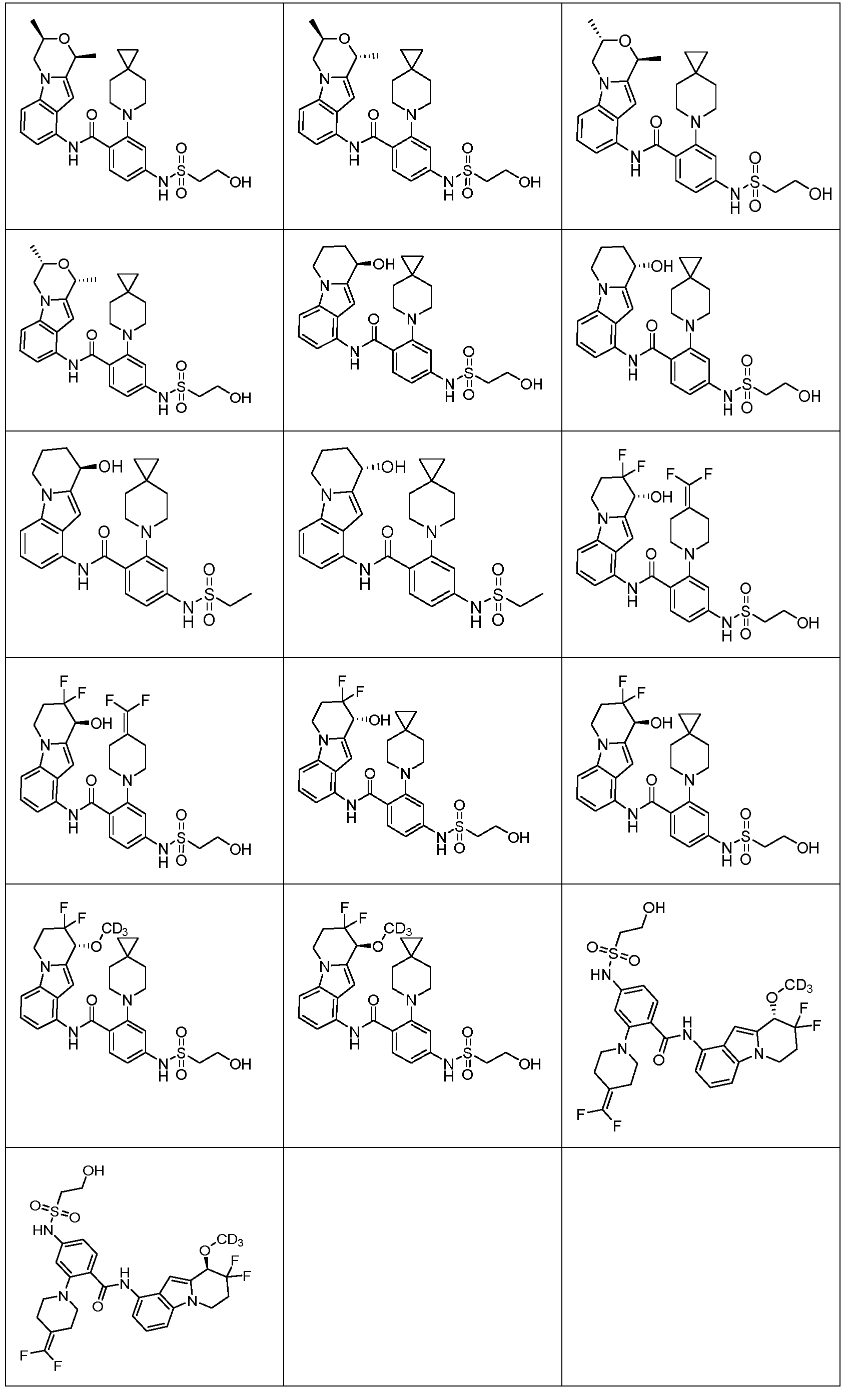

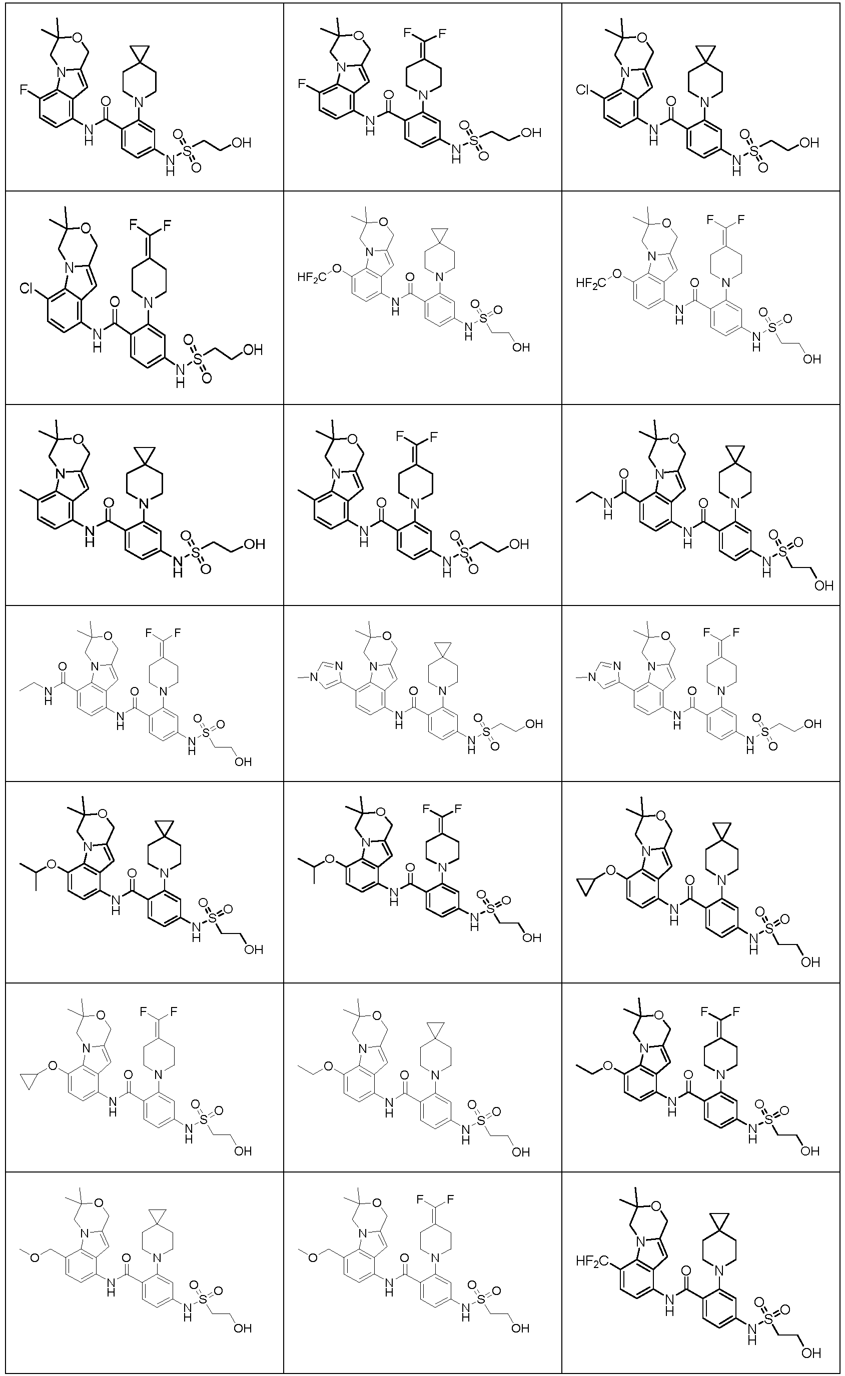

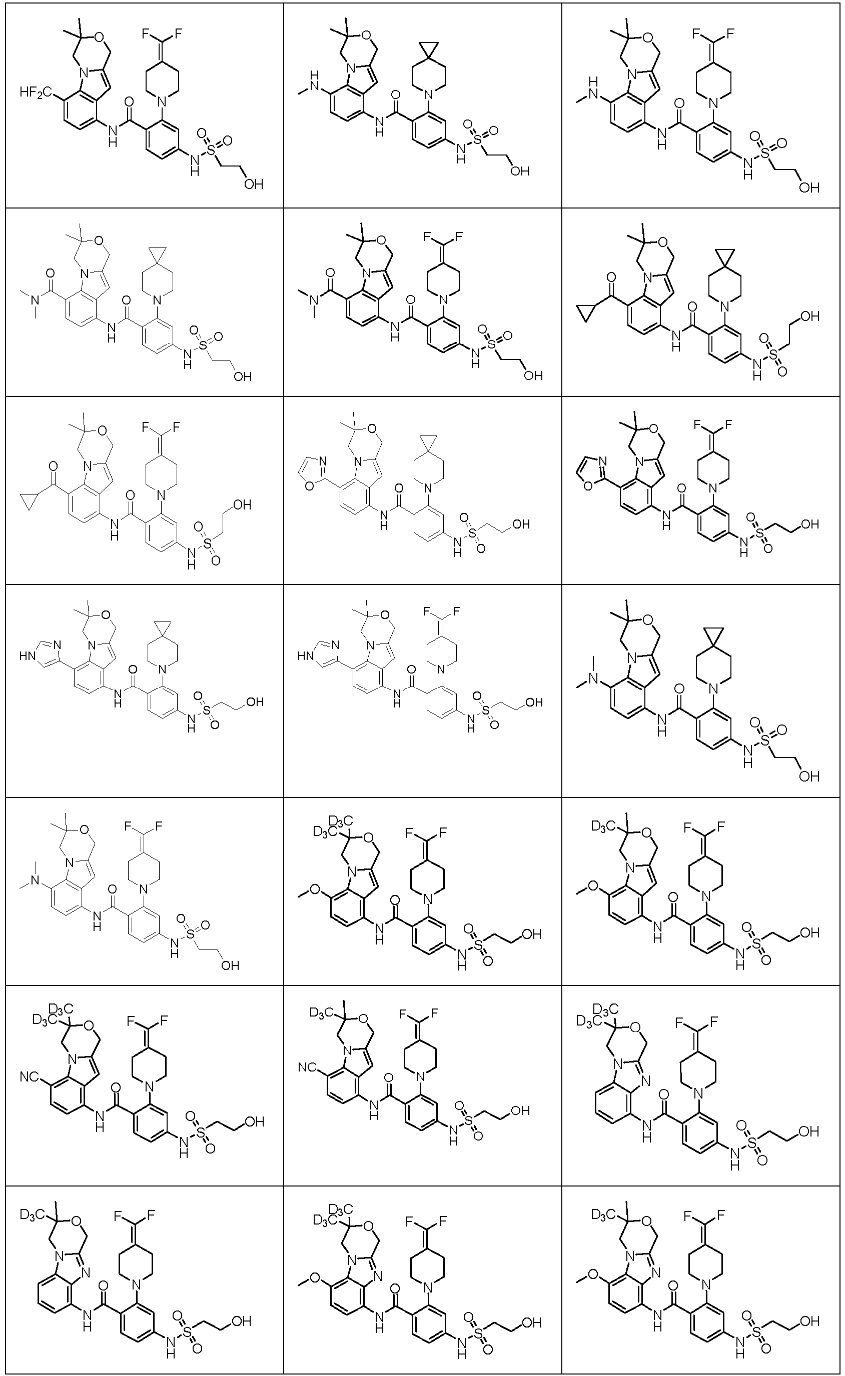

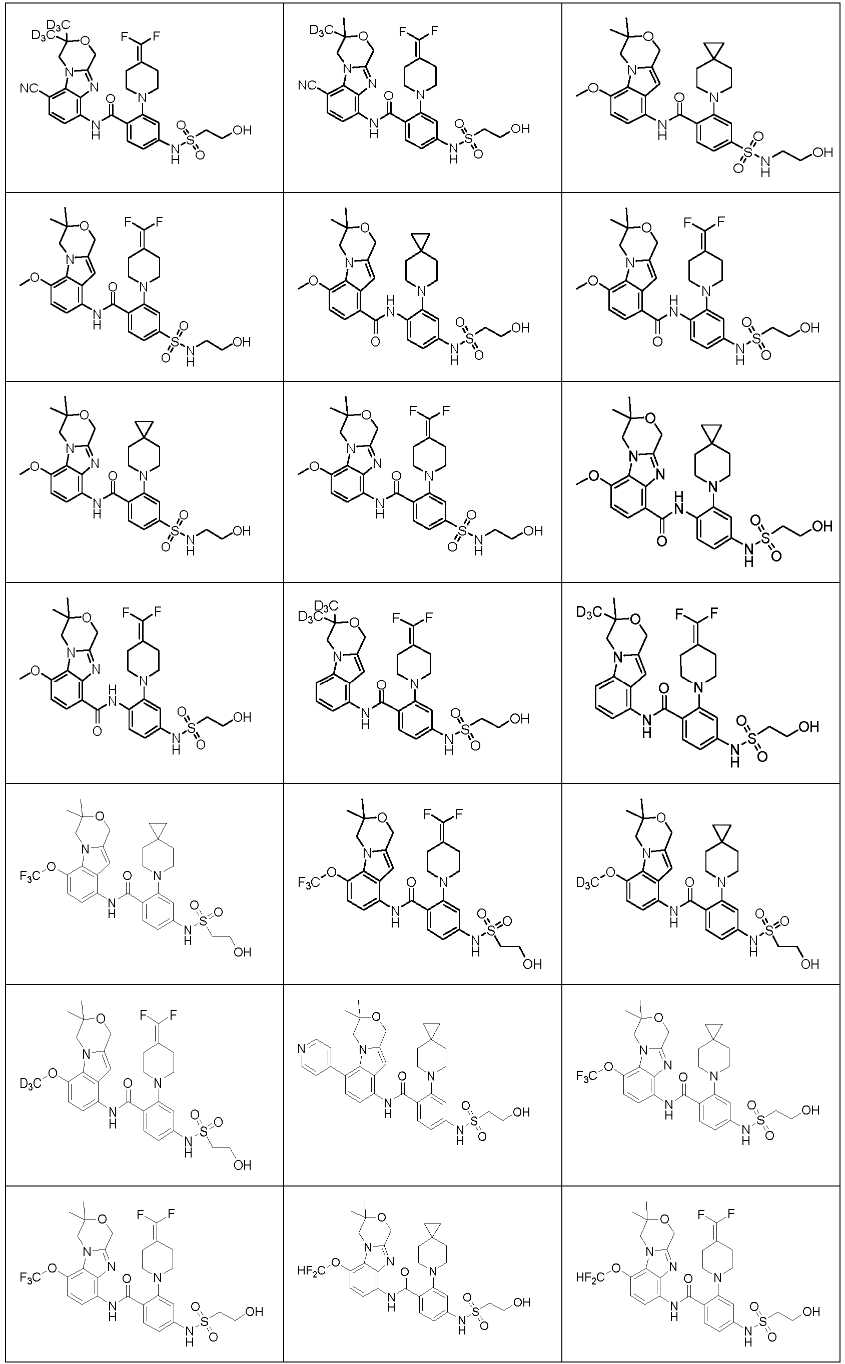

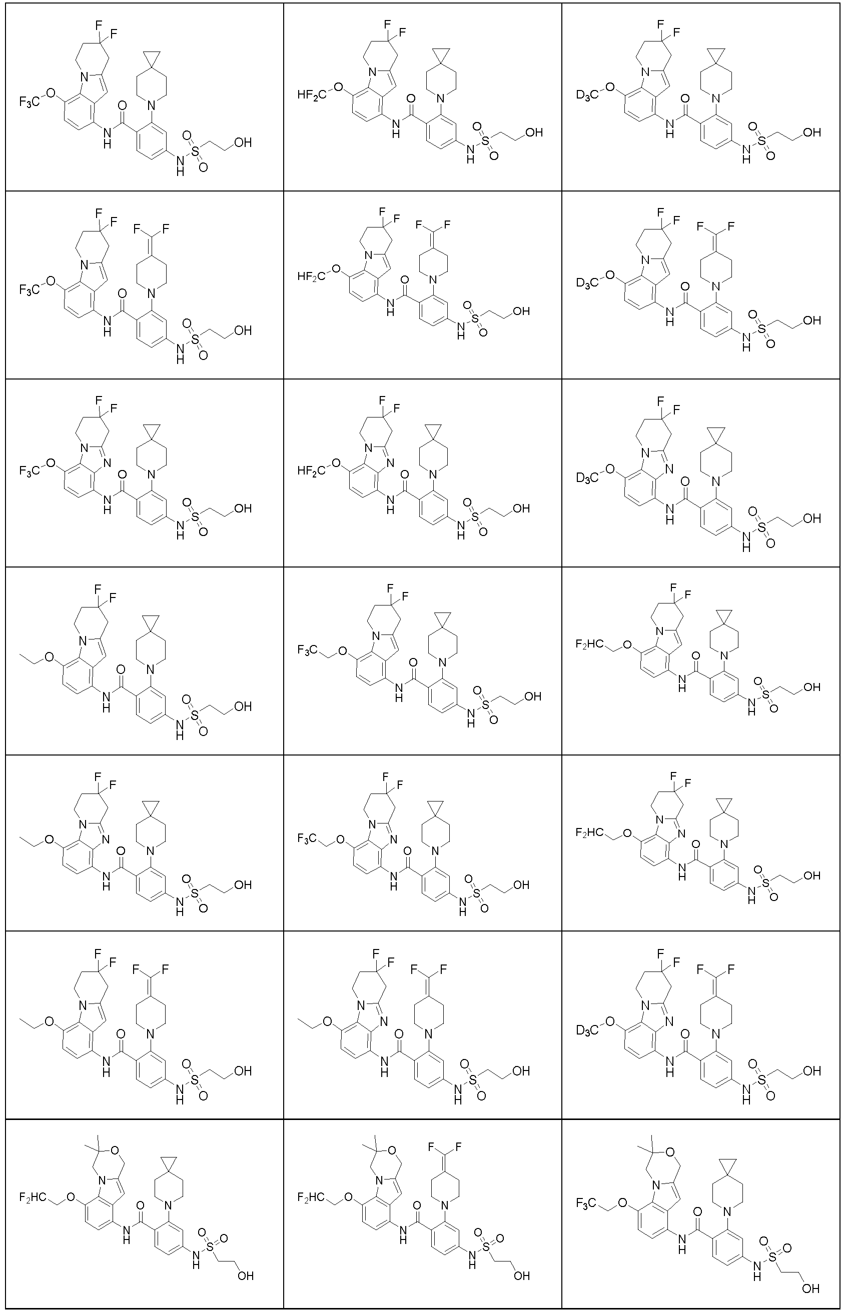

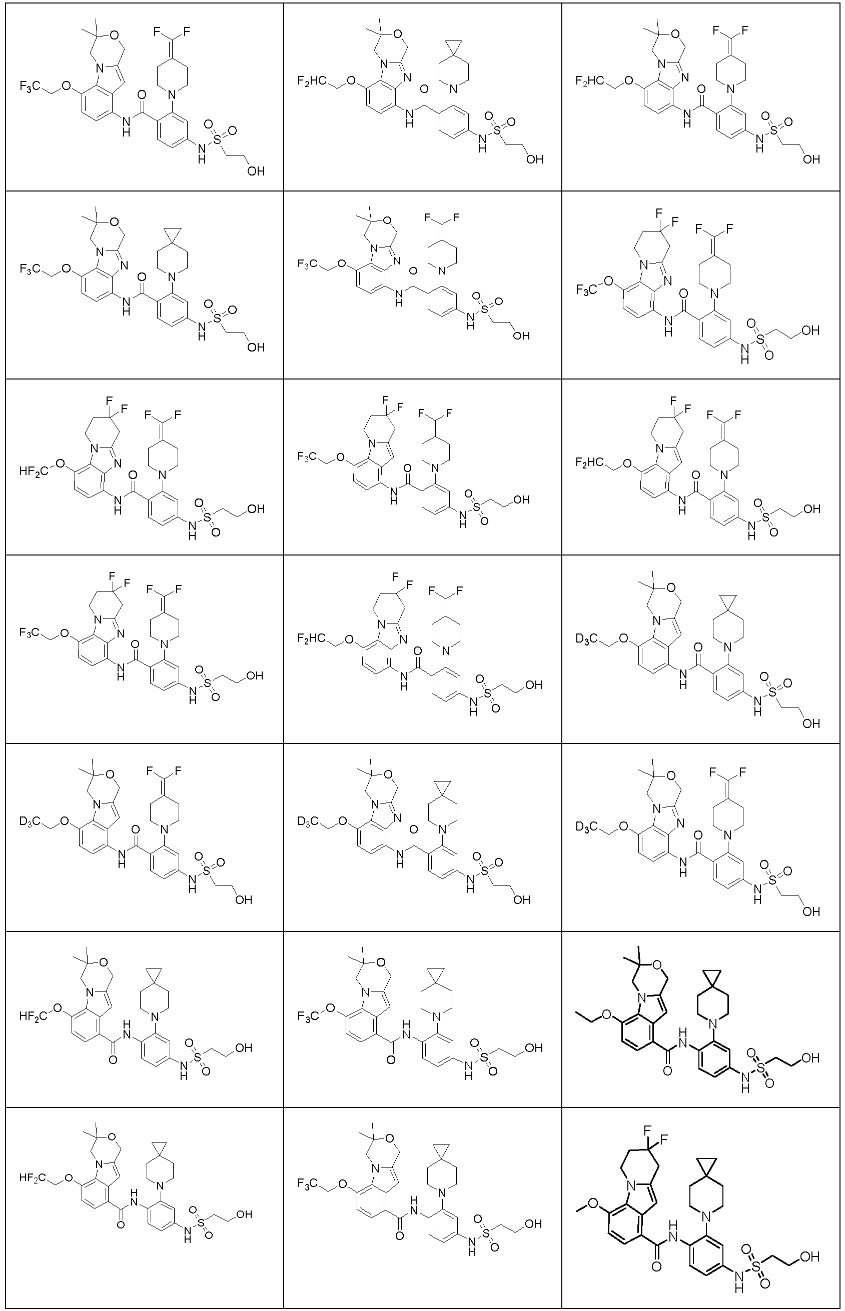

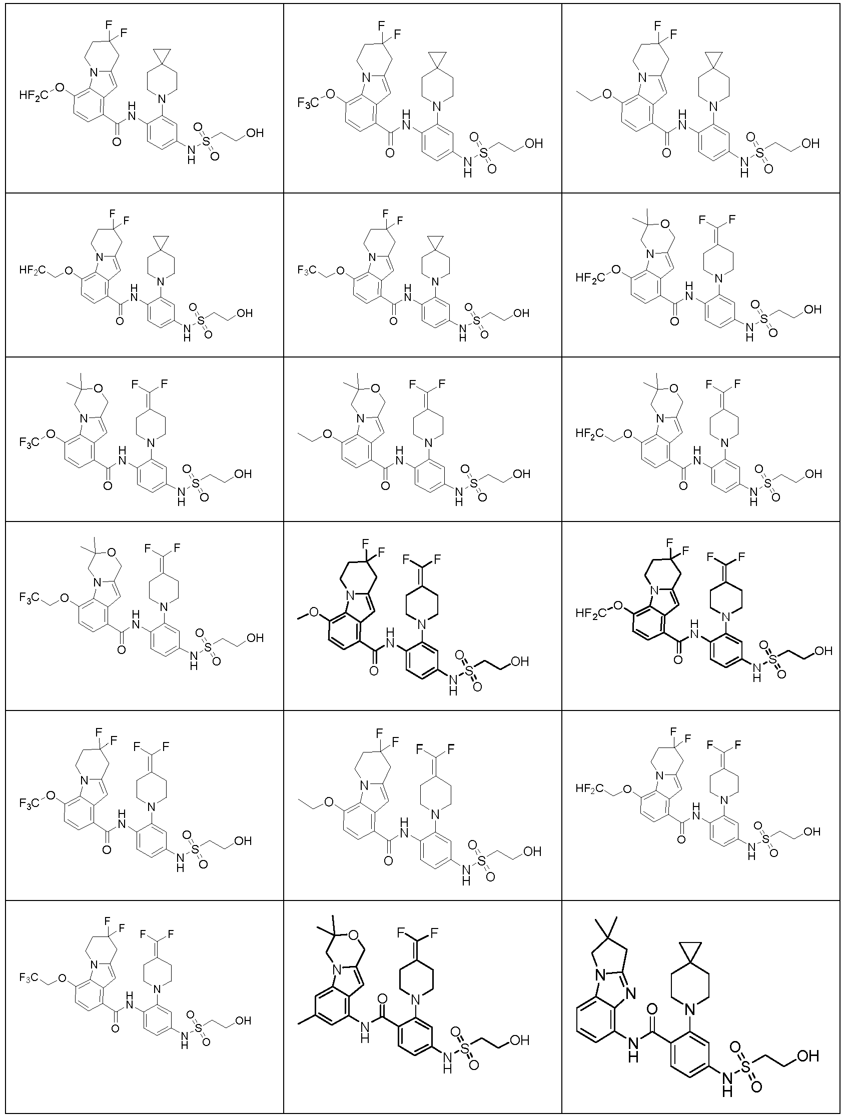

In some embodiments, the compound of formula (I) is selected from the group consisting of the following compounds:

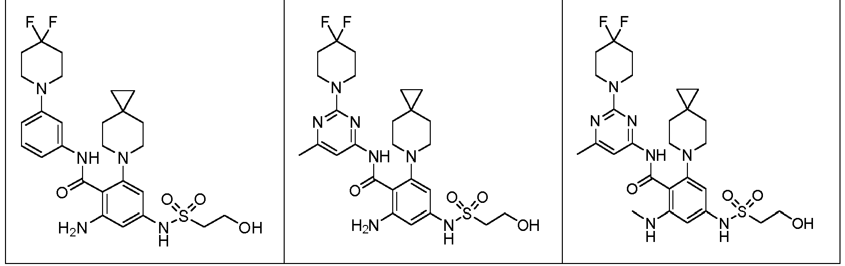

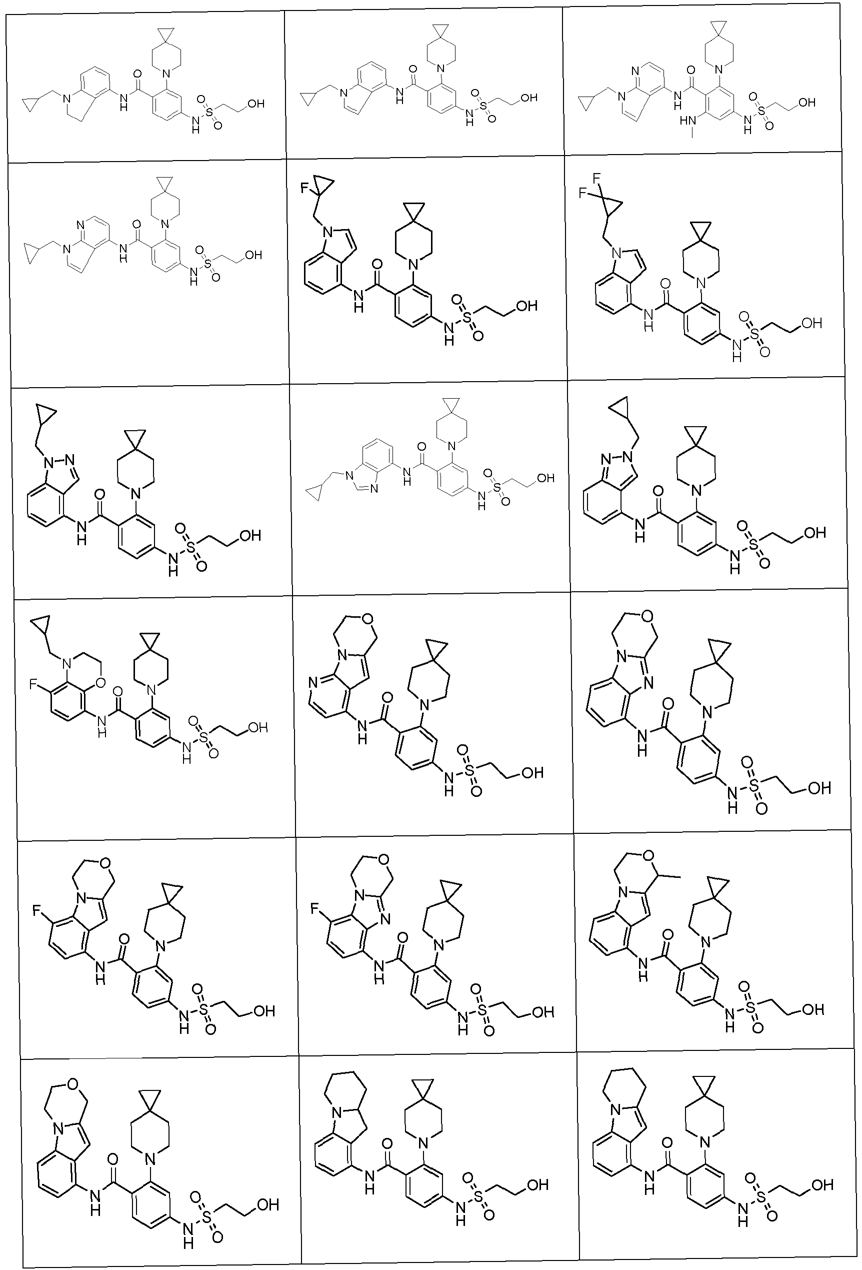

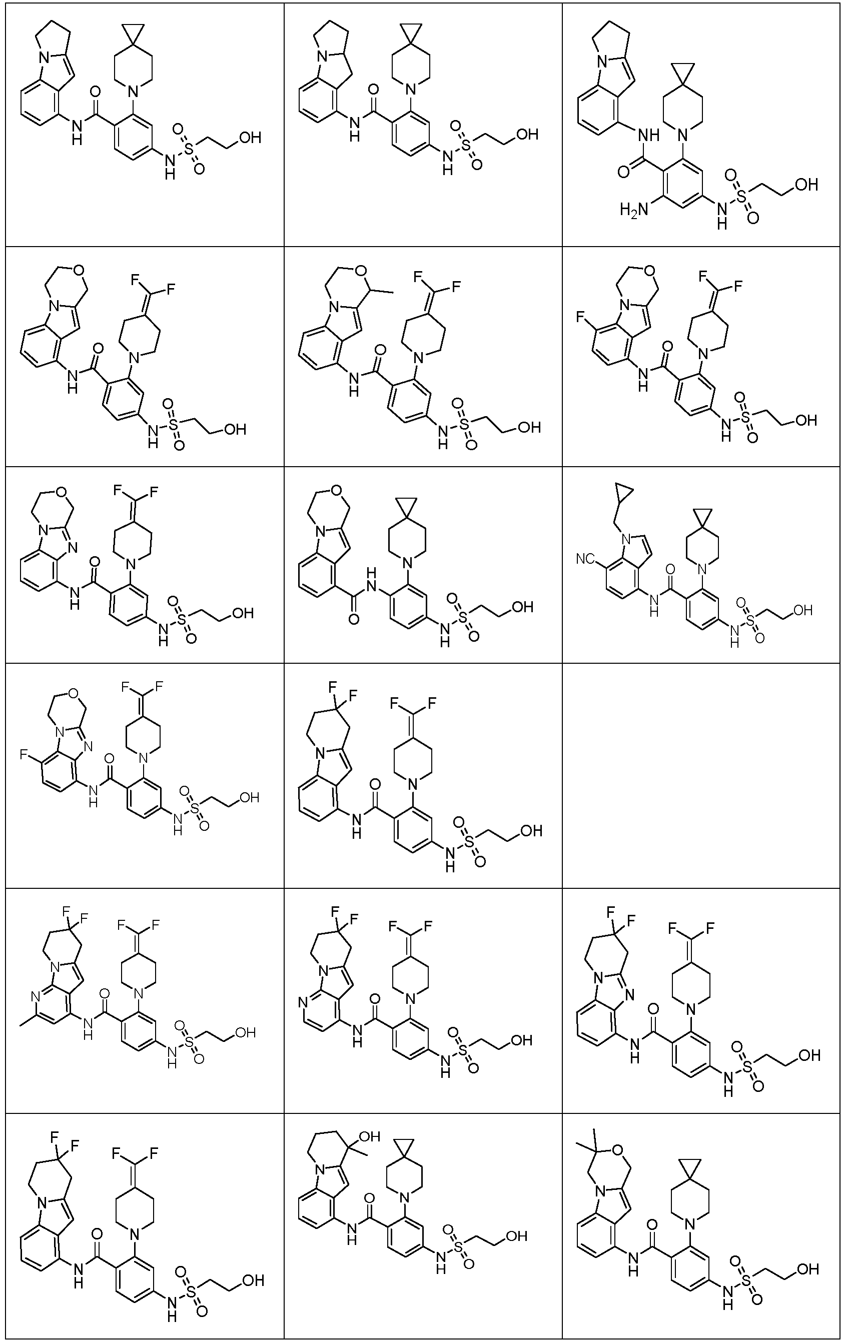

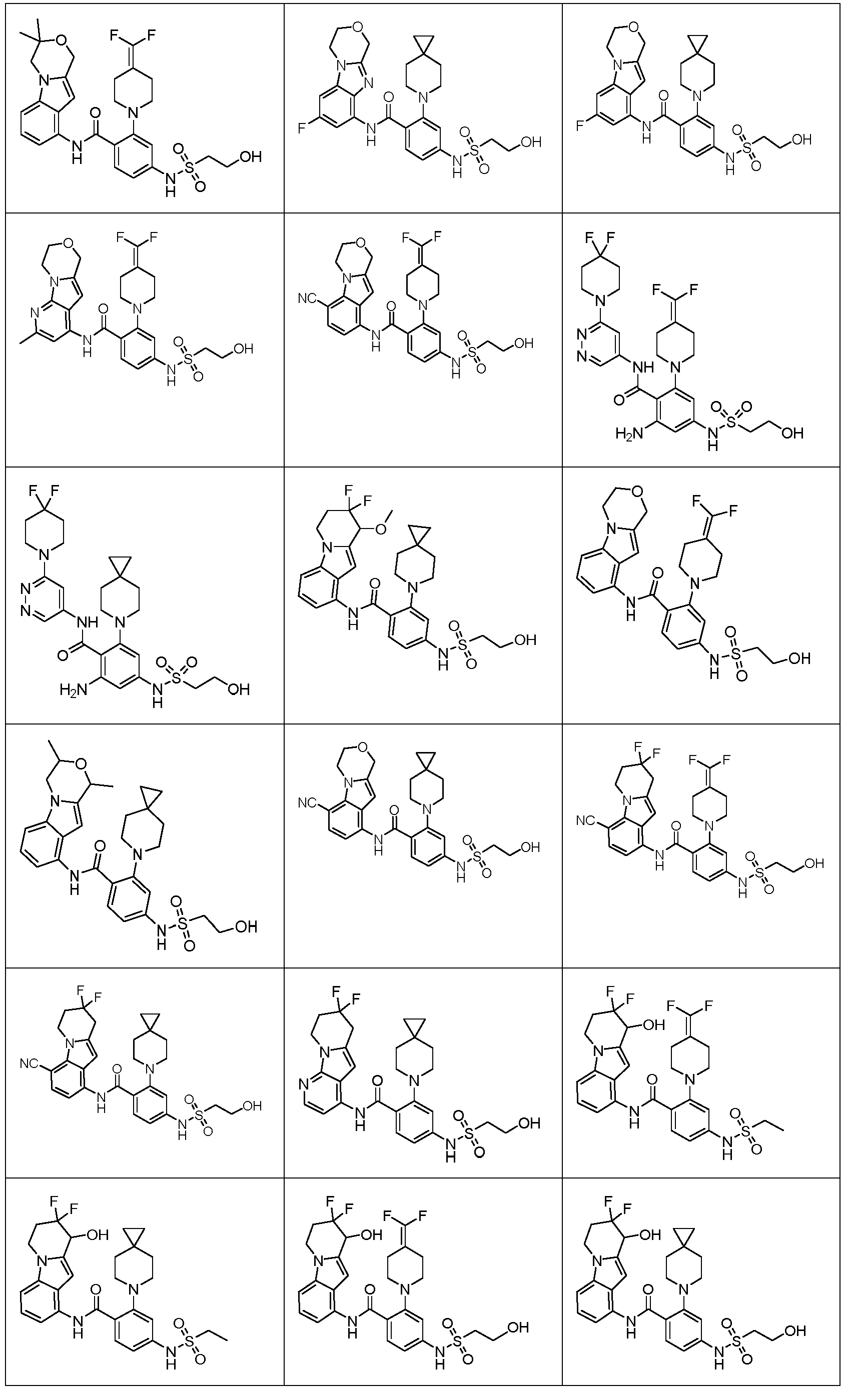

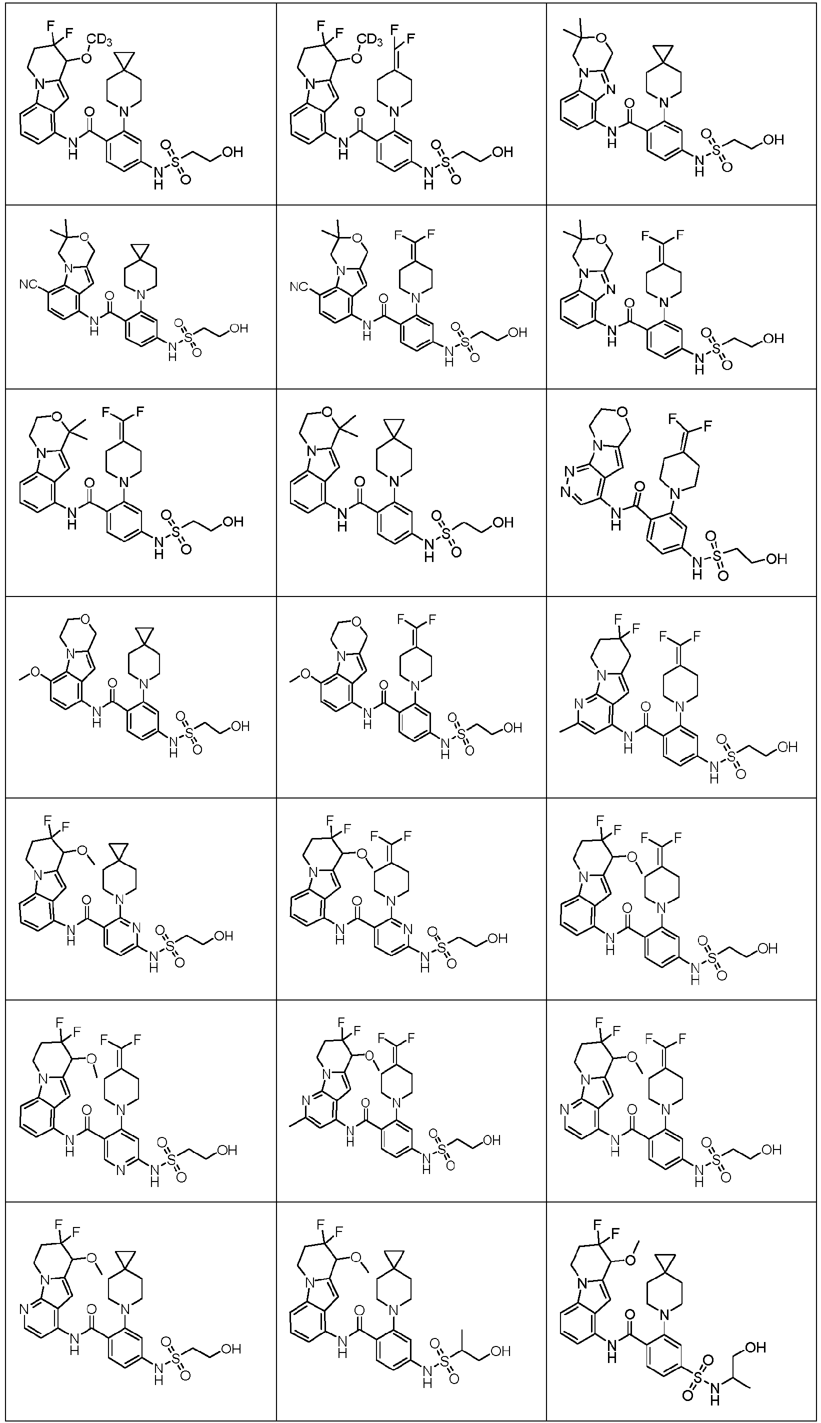

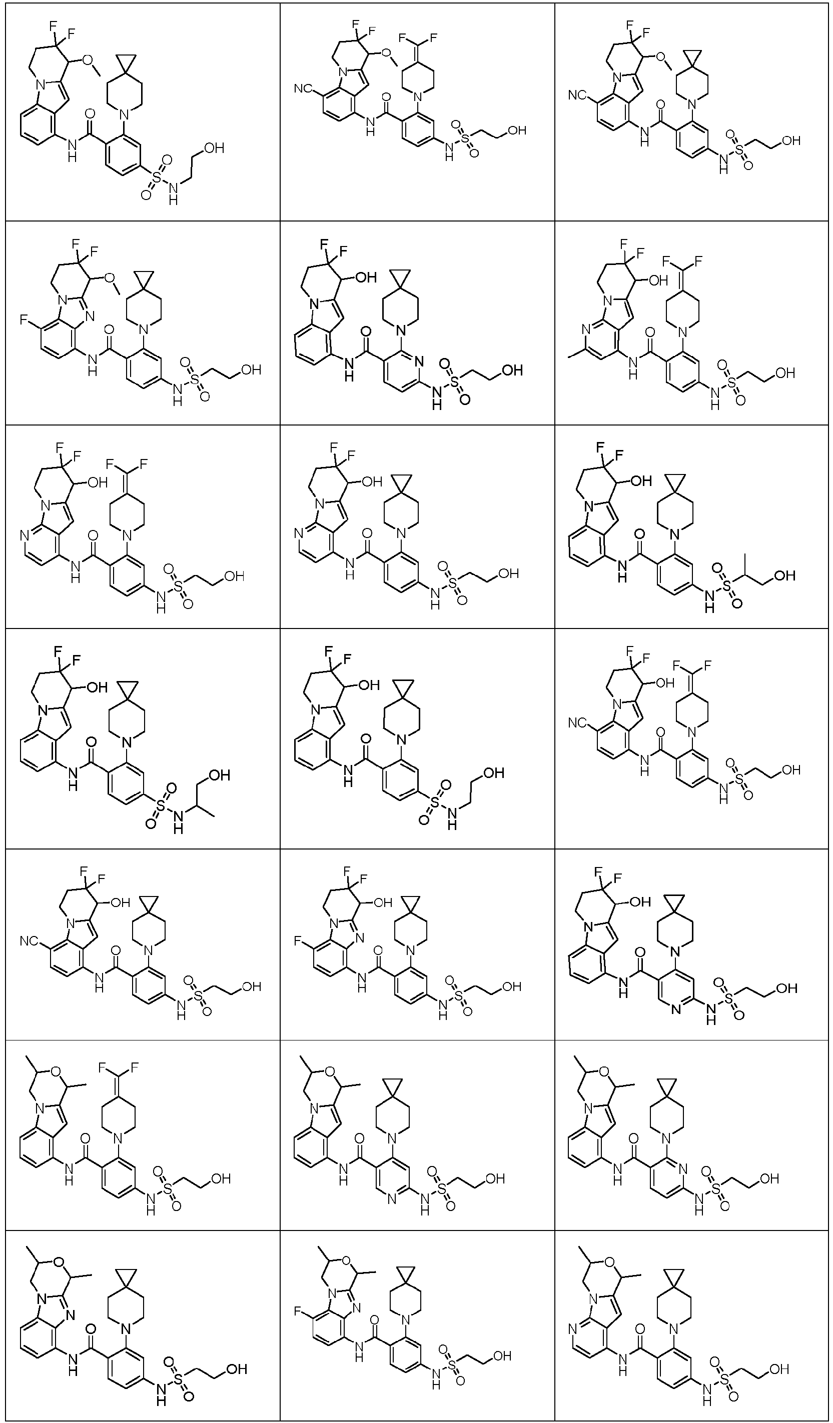

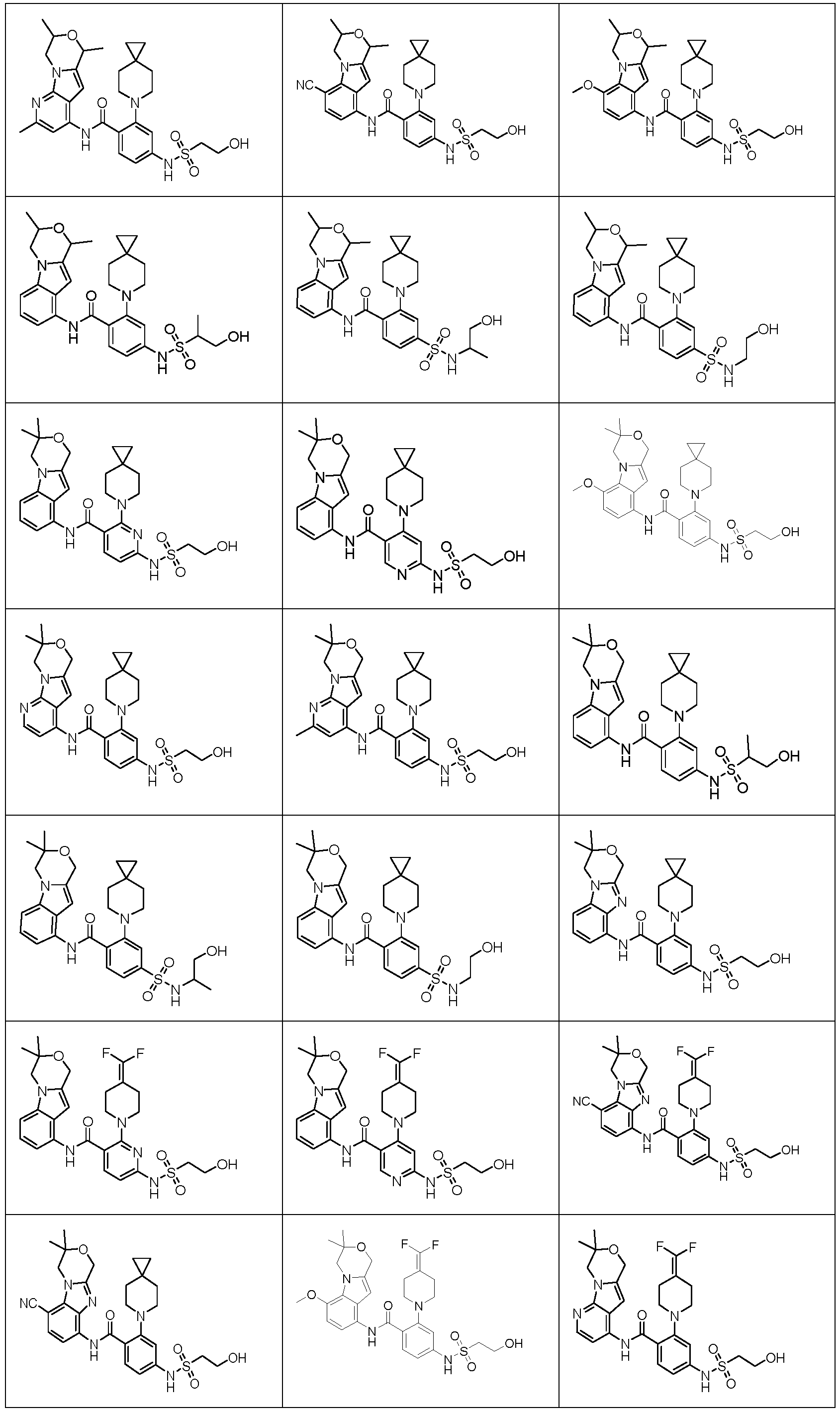

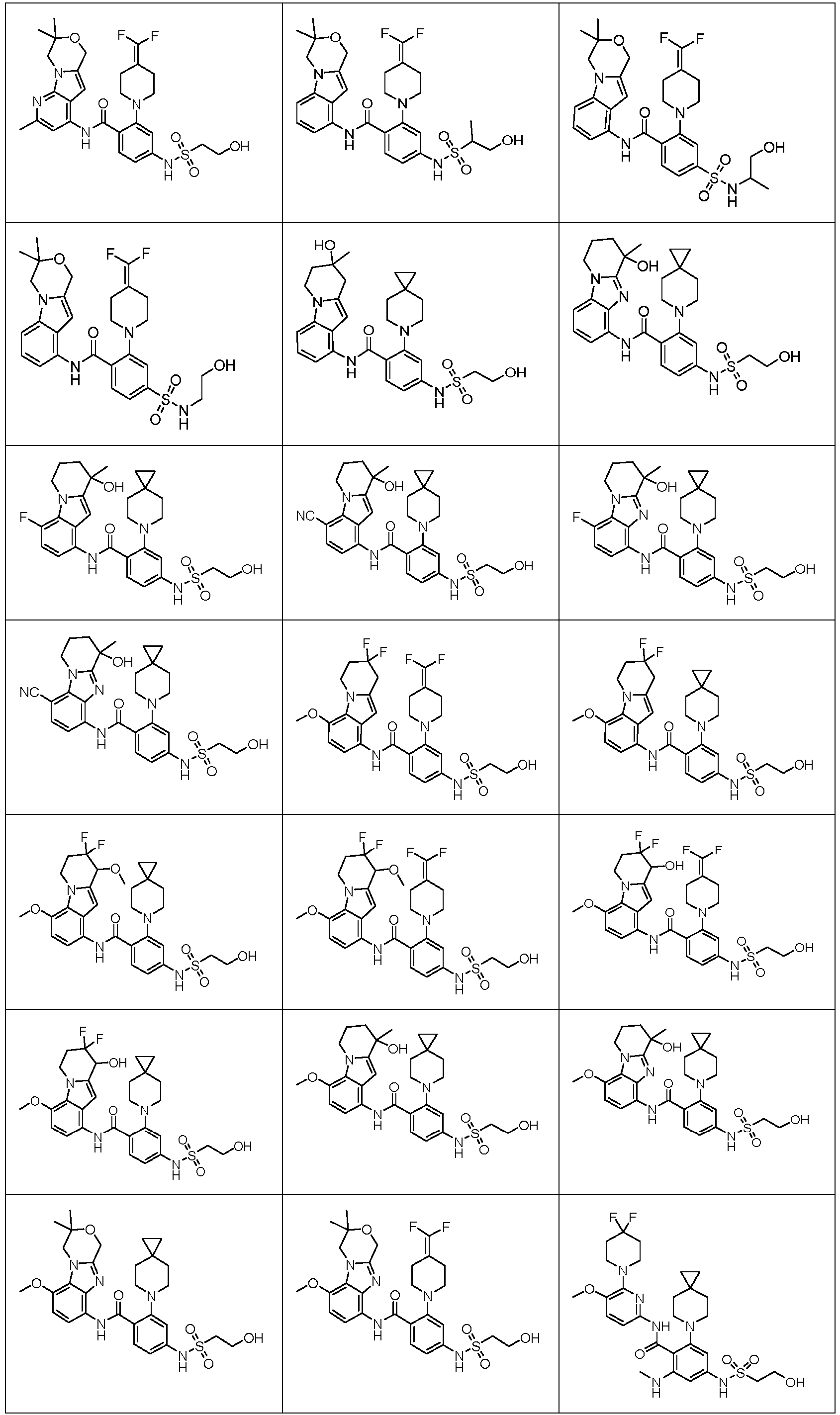

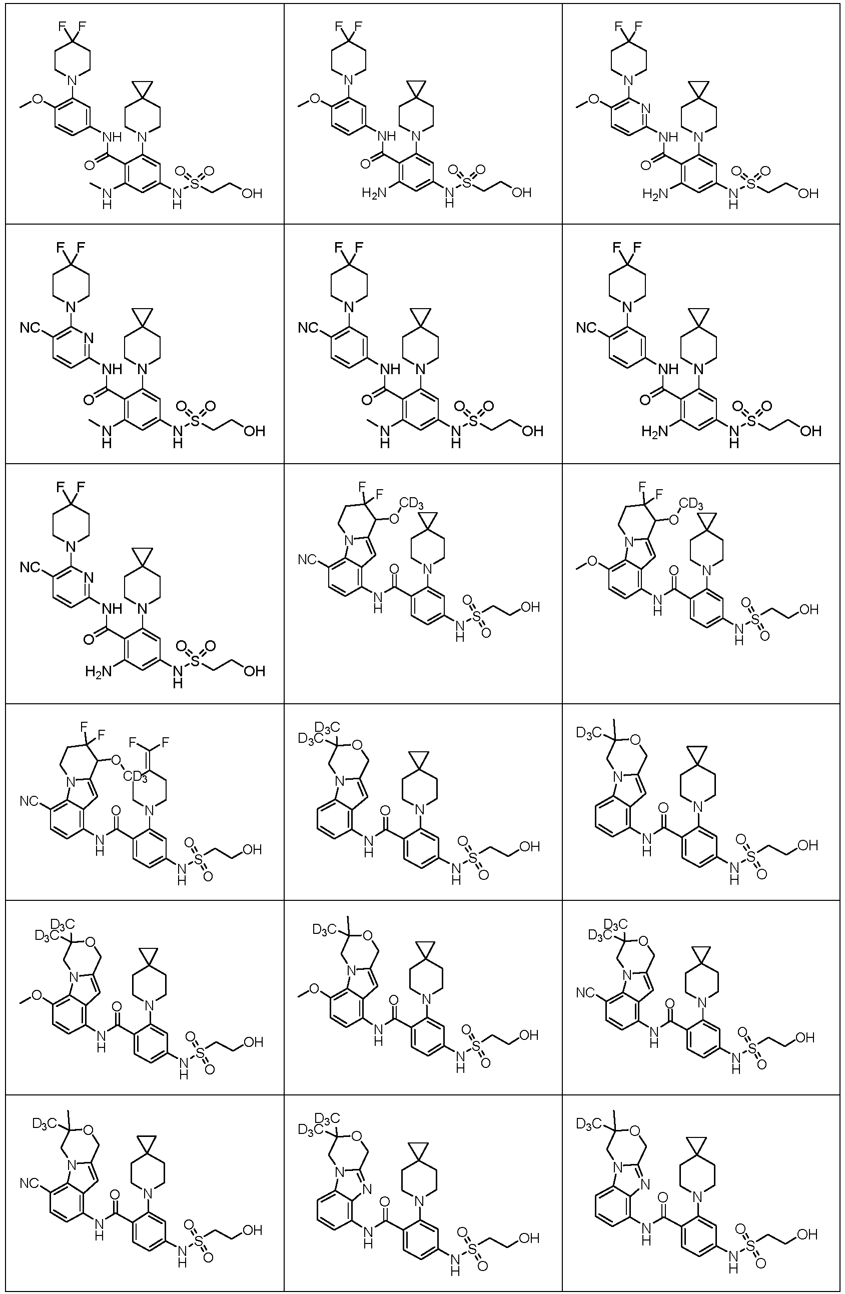

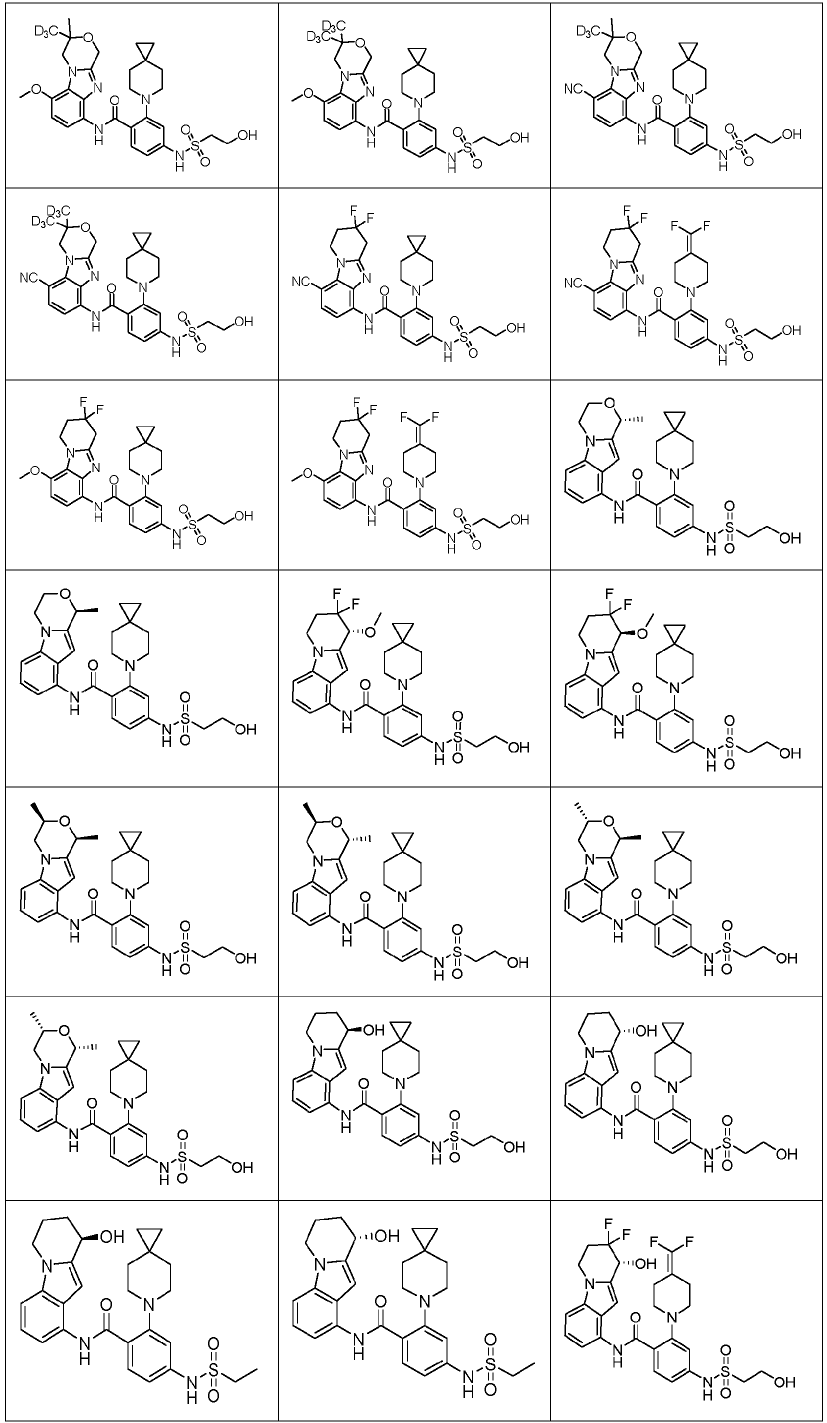

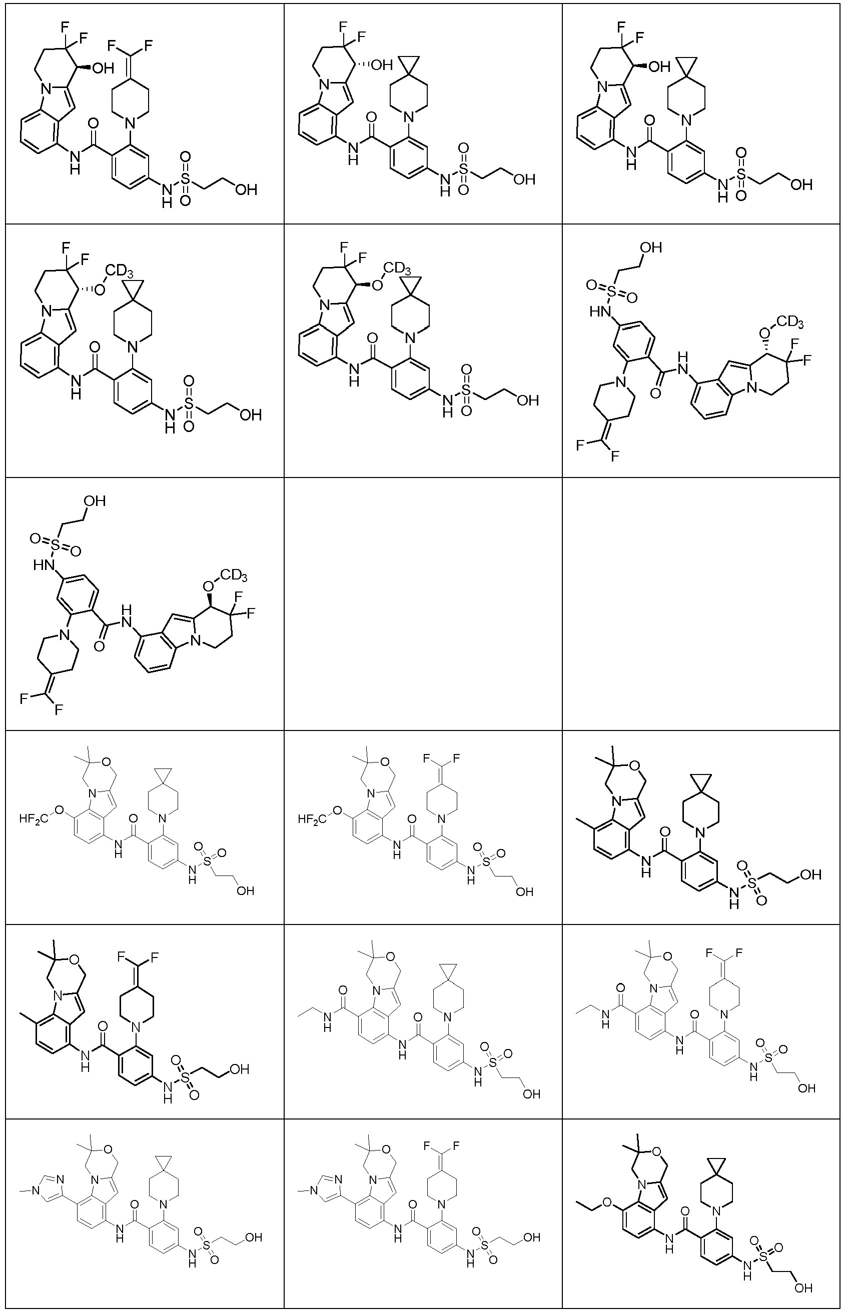

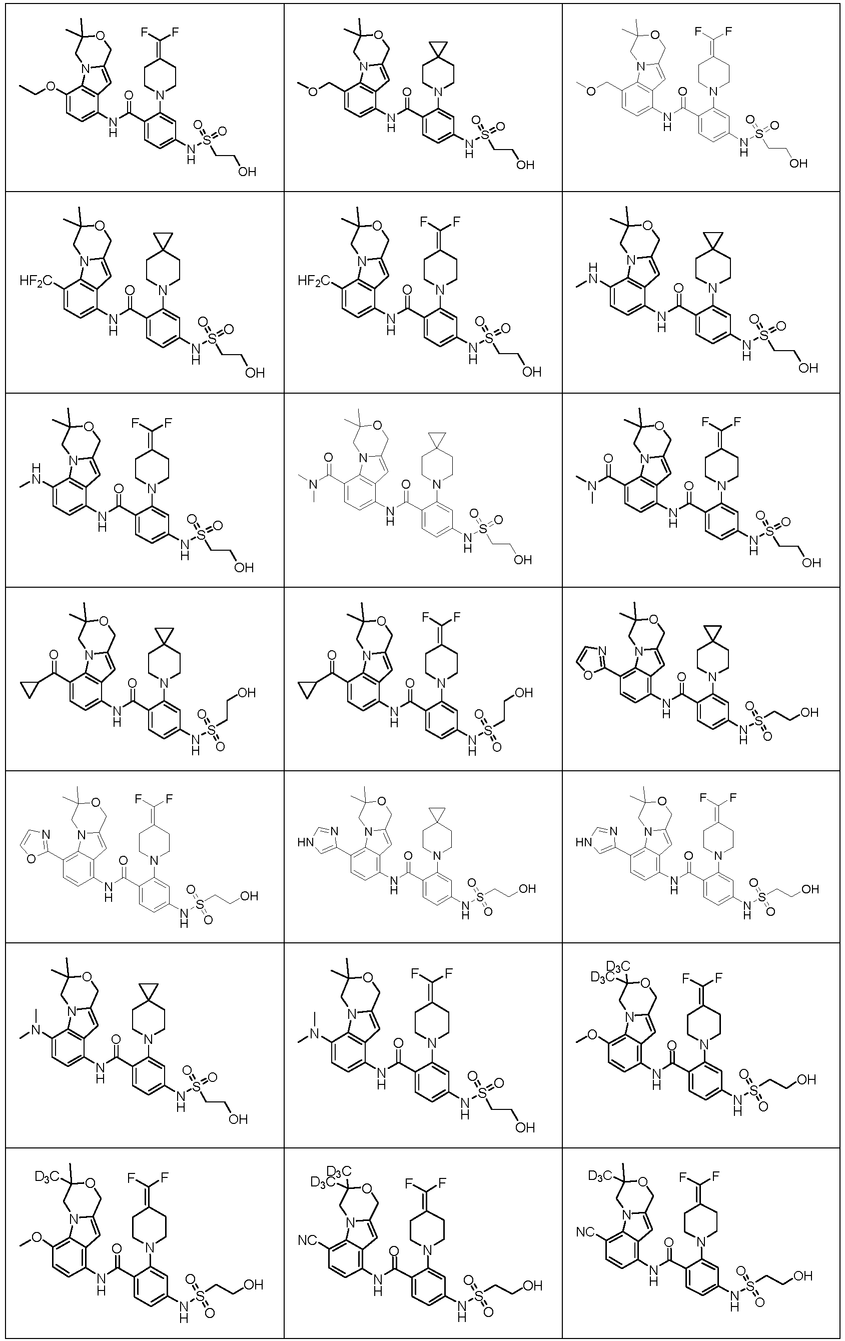

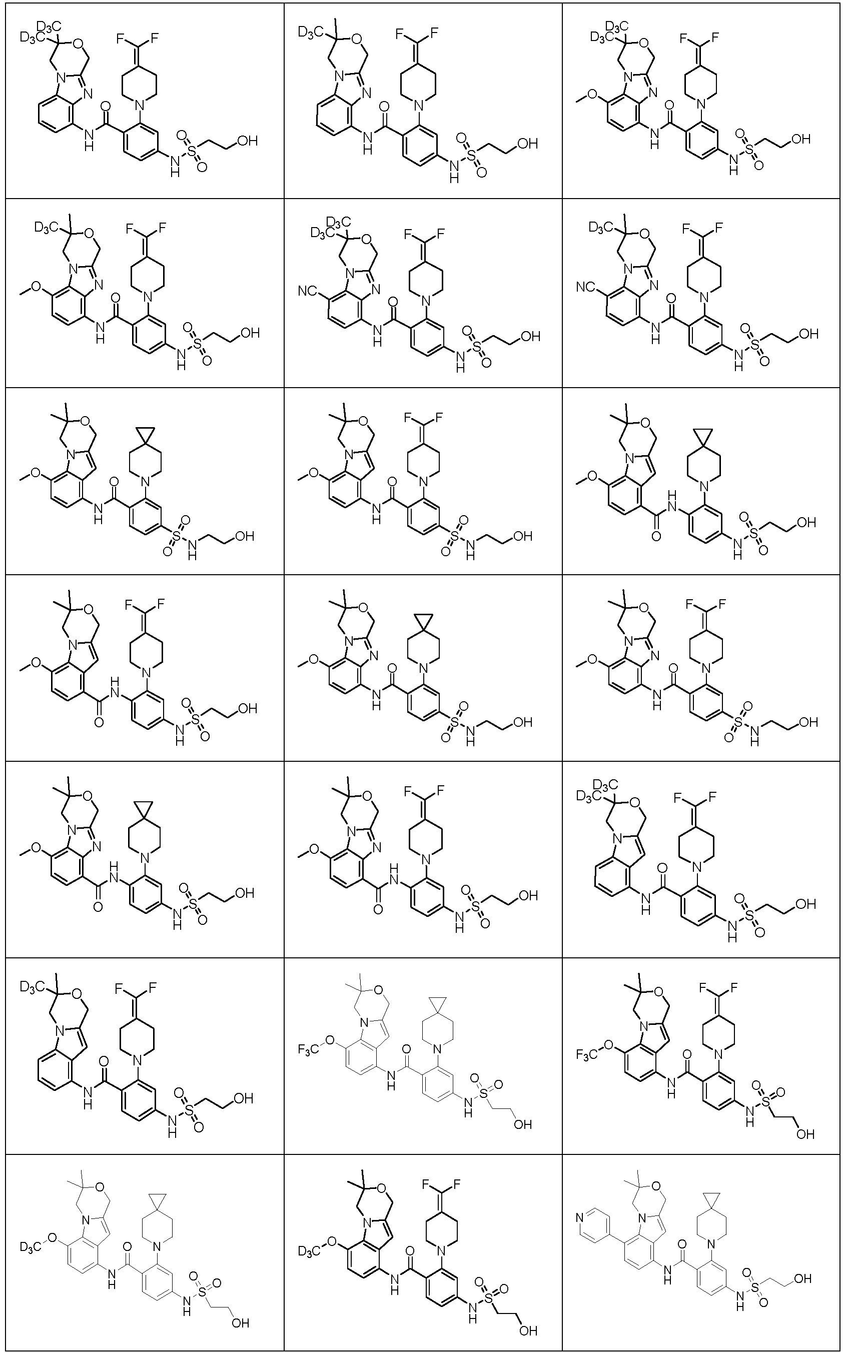

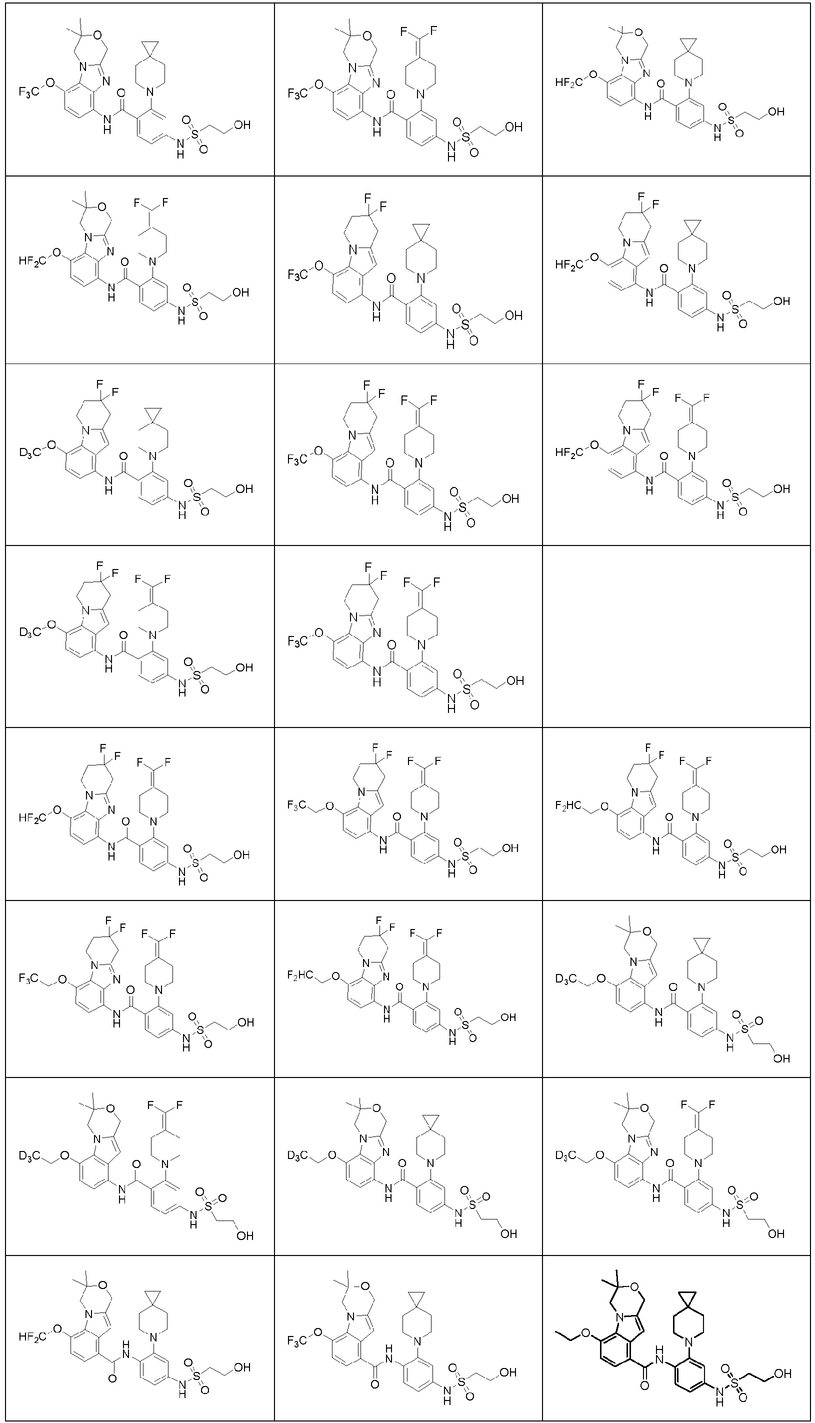

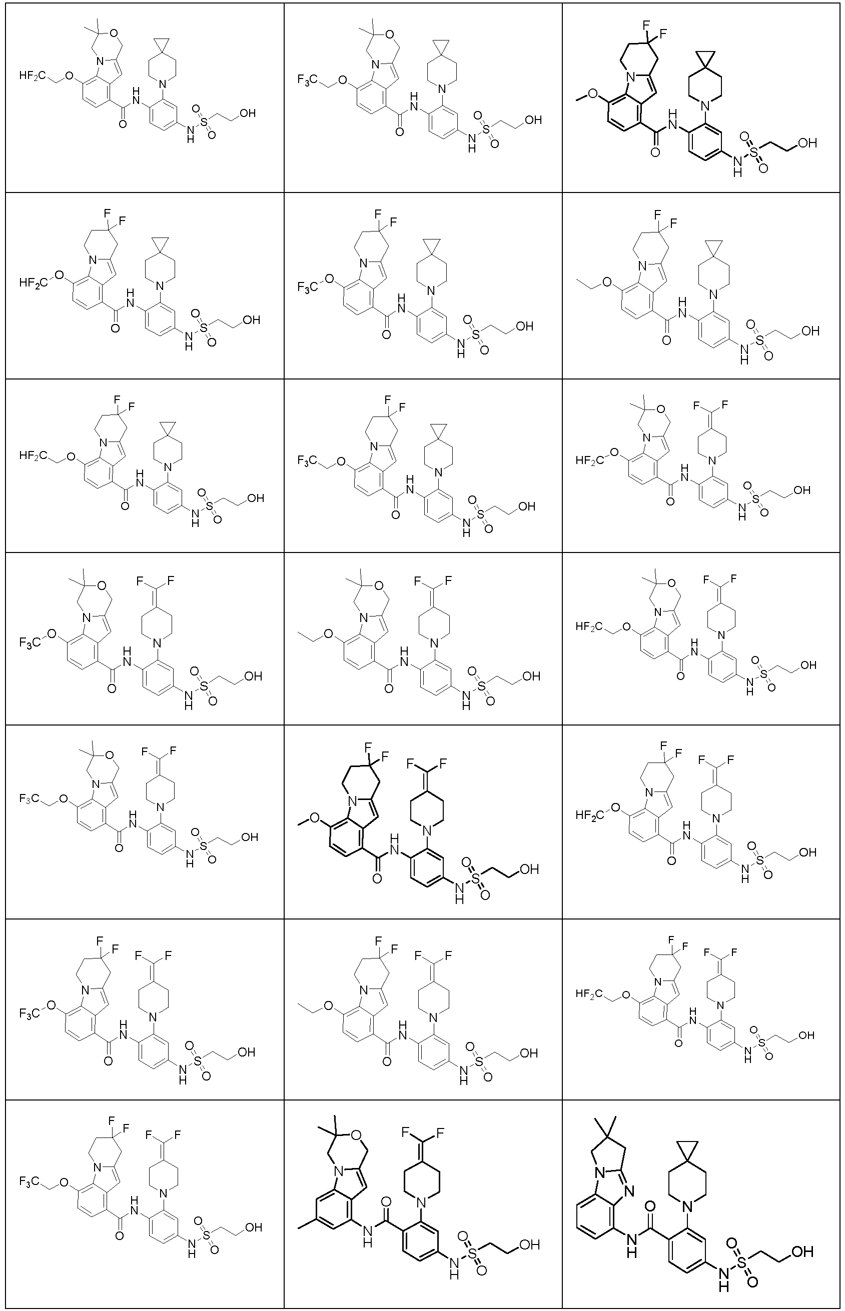

Further, the present application provides a pharmaceutical composition comprising the compound of formula (I), or the stereoisomer, tautomer, nitrogen oxide, solvate, metabolite, pharmaceutically acceptable salt or prodrug thereof, and a pharmaceutically acceptable diluent or carrier.

Further, the present application provides a method for treating a condition treatable with a KIF18A inhibitor, wherein the method comprises administering to a patient in need thereof a therapeutically effective amount of the compound of formula (I), or the stereoisomer, tautomer, nitrogen oxide, solvate, metabolite, pharmaceutically acceptable salt or prodrug thereof, or the pharmaceutical composition as described above.

In some embodiments, the condition is a cancer selected from the group consisting of: (a) a solid tumor or a blood-derived tumor selected from the following cancers: bladder cancer, endometrial cancer, squamous cell carcinoma of the lung, breast cancer, colon cancer, kidney cancer, liver cancer, lung cancer, small cell lung cancer, esophageal cancer, gallbladder cancer, brain cancer, head and neck cancer, ovarian cancer, pancreatic cancer, gastric cancer, cervical cancer, thyroid cancer, prostate cancer and skin cancer; (b) a hematopoietic tumor of lymphatic system selected from: leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkitt's lymphoma; (c) a hematopoietic tumor of bone marrow system selected from: acute and chronic myeloid leukemia, myelodysplastic syndrome and promyelocytic leukemia; (d) a tumor of interstitial origin selected from fibrosarcoma and rhabdomyosarcoma; (e) a tumor of the central and peripheral nervous system selected from astrocytoma, neuroblastoma, glioma and schwannoma; or (f) melanoma, seminoma, teratocarcinoma, osteosarcoma, xeroderma pigmentosum, keratoacanthoma, thyroid follicular carcinoma, or Kaposi's sarcoma.

Further, the present application provides a method for reducing the size of a solid tumor in a subject, wherein the method comprises administering to a patient in need thereof a therapeutically effective amount of the compound of formula (I), or the stereoisomer, tautomer, nitrogen oxide, solvate, metabolite, pharmaceutically acceptable salt or prodrug thereof, or the pharmaceutical composition as described above.

Further, the present application provides a method for treating a cell proliferation disorder in a subject, wherein the method comprises administering to a patient in need thereof a therapeutically effective amount of the compound of formula (I), or the stereoisomer, tautomer, nitrogen oxide, solvate, metabolite, pharmaceutically acceptable salt or prodrug thereof, or the pharmaceutical composition as described above.

Further, the cell proliferation disorder refers to abnormal cell proliferation, which is preferably mediated by affecting the cell cycle and mitosis.

Further, the present application provides a method for inhibiting KIF 18A in a cell, wherein the method comprises contacting the cell with the compound of formula (I), or the stereoisomer, tautomer, nitrogen oxide, solvate, metabolite, pharmaceutically acceptable salt or prodrug thereof, or the pharmaceutical composition as described above.

Further, the present application provides the use of the compound of formula (I), or the stereoisomer, tautomer, nitrogen oxide, solvate, metabolite, pharmaceutically acceptable salt or prodrug thereof, or the pharmaceutical composition in the preparation of a medicament for treating a condition treatable with a KIF 18A inhibitor.

In some embodiments, the condition is a cancer selected from the group consisting of: (a) a solid tumor or a blood-derived tumor selected from the following cancers: bladder cancer, endometrial cancer, squamous cell carcinoma of the lung, breast cancer, colon cancer, kidney cancer, liver cancer, lung cancer, small cell lung cancer, esophageal cancer, gallbladder cancer, brain cancer, head and neck cancer, ovarian cancer, pancreatic cancer, gastric cancer, cervical cancer, thyroid cancer, prostate cancer and skin cancer; (b) a hematopoietic tumor of lymphatic system selected from: leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkitt's lymphoma; (c) a hematopoietic tumor of bone marrow system selected from: acute and chronic myeloid leukemia, myelodysplastic syndrome and promyelocytic leukemia; (d) a tumor of interstitial origin selected from fibrosarcoma and rhabdomyosarcoma; (e) a tumor of the central and peripheral nervous system selected from astrocytoma, neuroblastoma, glioma and schwannoma; or (f) melanoma, seminoma, teratocarcinoma, osteosarcoma, xeroderma pigmentosum, keratoacanthoma, thyroid follicular carcinoma, or Kaposi's sarcoma.

Further, the present application provides the use of the compound of formula (I), or the stereoisomer, tautomer, nitrogen oxide, solvate, metabolite, pharmaceutically acceptable salt or prodrug thereof, or the pharmaceutical composition in the preparation of a medicament for reducing the size of a solid tumor in a subject.

Further, the present application provides the use of the compound of formula (I), or the stereoisomer, tautomer, nitrogen oxide, solvate, metabolite, pharmaceutically acceptable salt or prodrug thereof, or the pharmaceutical composition in the preparation of a medicament for treating a cell proliferation disorder in a subject.

Further, the present application provides the use of the compound of formula (I), or the stereoisomer, tautomer, nitrogen oxide, solvate, metabolite, pharmaceutically acceptable salt or prodrug thereof, or the pharmaceutical composition in the preparation of a medicament for inhibiting KIF18A in a cell.

The present disclosure provides a compound of formula (I) which is novel in structure and has good KIF18A inhibitory activity and is useful for therapeutic, prophylactic, acute or chronic treatment of KIF18A-mediated diseases and disorders, including but not limited to cancer.

### Detailed Description of Embodiments

### Definitions

Unless otherwise defined herein, all technical and scientific terms used herein are intended to have the same meanings as commonly understood by those skilled in the art. The techniques mentioned herein are intended to refer to those commonly understood in the art, including modifications of techniques or substitutions of equivalent techniques that would be obvious to a person skilled in the art. While it is believed that the following terms are well understood by those skilled in the art, the following definitions are set forth to better explain the present disclosure.

The terms "including", "comprising", "having", "containing" or "involving" and other variants thereof herein are inclusive or open-ended, and other unenumerated elements or method steps are not excluded (i.e., these terms also cover the terms "consisting essentially of" and "consisting of").

As used herein, the term "alkyl" is defined as a straight or branched saturated aliphatic hydrocarbon. In some embodiments, an alkyl group has 1 to 12, such as 1 to 6, carbon atoms. For example, as used herein, the term "C1-6 alkyl" refers to a straight or branched group of 1 to 6 carbon atoms (e.g., methyl, ethyl, n-propyl, isopropyl, n-butyl, isobutyl, sec-butyl, tert-butyl, n-pentyl or n-hexyl) optionally substituted with one or more (such as 1 to 3) suitable substituents such as halogen (in this case the group is referred to as "haloalkyl") (e.g. CF3, C2F5, CHF2, CH2F, CH2CF3, CH2Cl or -CH2CH2CF3). The term "C1-4 alkyl" refers to a straight or branched aliphatic hydrocarbon chain of 1 to 4 carbon atoms (i.e. methyl, ethyl, n-propyl, isopropyl, n-butyl, isobutyl, sec-butyl or tert-butyl).

As used herein, the term "alkylene" means a divalent straight or branched saturated aliphatic hydrocarbon group derived from the loss of 1 H of the alkyl group as defined above. In some embodiments, an alkylene group has 1 to 12, such as 1 to 6, carbon atoms. For example, as used herein, the term "C1-6 alkylene" refers to a straight or branched group of 1 to 6 carbon atoms (e.g., methylene, ethylene, n-propylene, isopropylene, n-butylene, isobutylene, sec-butylene, tert-butylene, n-pentylene or n-hexylene) optionally substituted with one or more (such as 1 to 3) suitable substituents such as halogen (in this case the group is referred to as "haloalkylene") (e.g. -CF2-, -C2F4, -CF2-, -CHF-, -CHCF2-, -CHCl- or - CHCH2CF2-). The term "C1-4 alkylene" refers to a divalent straight or branched aliphatic hydrocarbon chain of 1 to 4 carbon atoms (i.e., methylene, ethylene, n-propylene, isopropylene, n-butylene, isobutylene, or sec-butylene).

Those skilled in the art would readily understand that in the case of an alkyl group as part of a substituent and each of its two sides is attached to another group, if the term "alkyl" is still used, the term "alkyl" is actually the corresponding alkylene group. For example, "C2-C6 alkyl" in "-OC2-C6 alkyl NRₐRₐ" is actually "C2-C6 alkylene".

As used herein, the term "alkoxy" refers to an -O-alkyl group, where the alkyl group is as defined above. The term "C1-6 alkoxy" refers to a straight or branched alkoxy group having 1 to 6 carbon atoms (e.g. methoxy, ethoxy, n-propoxy, isopropoxy, tert-butoxy, n-pentyloxy or n-hexyloxy) optionally substituted with one or more (such as 1 to 3) suitable substituents such as halogen (in this case the group is referred to as "haloalkoxy") (e.g. -OCF3, -OC2F5, -OCHF2, -OCH2F, - OCH2CF3, -OCH2Cl or -OCH2CH2CF3). The term "C1-4 alkoxy" refers to a straight or branched alkoxy group of 1 to 4 carbon atoms (e.g. methoxy, ethoxy, n-propoxy, isopropoxy, or t-butoxy).

As used herein, the term "cycloalkyl" refers to a saturated monocyclic or polycyclic (such as bicyclic) hydrocarbon ring (e.g., monocyclic hydrocarbon ring, such as cyclopropyl, cyclobutyl, cyclopentyl, cyclohexyl, cycloheptyl, cyclooctyl, cyclonononyl, or bicyclic hydrocarbon ring, including spiro, fused or bridged systems (such as bicyclo[1.1.1]pentyl, bicyclo[2.2.1]heptyl, bicyclo[3.2.1]octyl or bicyclo[5.2.0]nonyl, or decahydronaphthyl) optionally substituted with one or more (such as 1 to 3) suitable substituents. The cycloalkyl group has 3 to 15 carbon atoms, suitably 3 to 10 carbon atoms. For example, the term "C3-6 cycloalkyl" refers to a saturated or partially unsaturated non-aromatic monocyclic or polycyclic (such as bicyclic) hydrocarbon ring of 3 to 6 ring-forming carbon atoms (e.g. cyclopropyl, cyclobutyl, cyclopentyl or cyclohexyl). The cycloalkyl group is optionally substituted with one or more (such as 1 to 3) suitable substituents, for example methyl-substituted cyclopropyl.

As used herein, the term "heterocyclyl" refers to a saturated or partially unsaturated monovalent monocyclic or bicyclic group having 2, 3, 4, 5, 6, 7, 8, or 9 carbon atoms and one or more (e.g., one, two, three or four) heteroatom-containing groups selected from C(=O), O, S, S(=O), S(=O)2 and NRa' in the ring, in which Ra' represents a hydrogen atom or a C1-6 alkyl group or a halo-C1-6 alkyl group. The heterocyclyl may be attached to the rest of the molecule through any of the carbon atoms, or the nitrogen atom (if present). In particular, 3- to 10-membered heterocyclyl is a group having 3-10 (e.g. 3-7, 4-6 or 5-6) carbon atoms, and heteroatoms in the ring, such as but not limited to oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl or trithianyl.

As used herein, the term "heterocyclyl" encompasses a fused ring, which may be attached to other groups at any ring in the fused ring. Therefore, heterocyclyl of the present disclosure further includes, but is not limited to heterocyclyl fused heterocyclyl, heterocyclyl fused cycloalkyl, monoheterocyclyl fused monoheterocyclyl, or monoheterocyclyl fused monocycloalkyl, such as 3- to 7-membered (mono)heterocyclyl fused 3- to 7-membered (mono)heterocyclyl, 3- to 7-membered (mono)heterocyclyl fused (mono)cycloalkyl, or 3- to 7-membered (mono)heterocyclyl fused C4-6 (mono)cycloalkyl, and the example thereof includes, but is not limited to pyrrolidinyl fused cyclopropyl, cyclopentyl fused aziridinyl, pyrrolidinyl fused cyclobutyl, pyrrolidinyl fused pyrrolidinyl, pyrrolidinyl fused piperidinyl, pyrrolidinyl fused piperazinyl, piperidinyl fused morpholinyl,

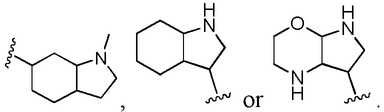

As used herein, the term "heterocyclyl" encompasses bridged and spiro heterocyclyl groups.

As used herein, the term "bridged heterocycle" refers to a cyclic structure containing one or more (e.g., 1, 2, 3, or 4) heteroatoms (e.g., oxygen, nitrogen, and/or sulphur atoms) formed by two saturated rings sharing two ring atoms that are not directly linked, including, but is not limited to, 7- to 10-membered bridged heterocycle, 8- to 10-membered bridged heterocycle, 7- to 10-membered nitrogen-containing bridged heterocycle, 7- to 10-membered oxygen-containing bridged heterocycle, and 7- to 10-membered sulphur-containing bridged heterocycles, such as 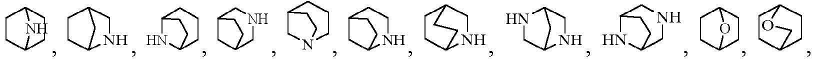 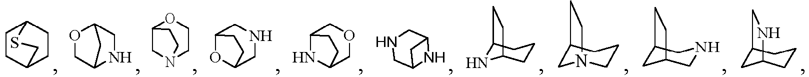 or 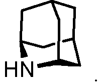 The "nitrogen-containing bridged heterocycle", "oxygen-containing bridged heterocycle", or "sulphur-containing bridged heterocycle" optionally contains one or more additional heteroatoms selected from oxygen, nitrogen and sulphur.

As used herein, the term "fused heterocyclyl" refers to two-membered or poly-membered heterocyclyl (e.g., two-membered heterocyclyl or three-membered heterocyclyl) encompassing an fused ring, which may be attached to other groups at any ring in the fused ring. Therefore, the fused heterocyclyl of the present disclosure includes, but is not limited to aryl fused heterocyclyl, heterocyclyl fused cycloalkyl, monoheterocyclyl fused monoheterocyclyl, monoheterocyclyl fused monocycloalkyl, or heteroaryl fused heterocyclyl, such as 3- to 7-membered (mono)heterocyclyl fused 3- to 7-membered (mono)heterocyclyl, 3- to 7-membered (mono)heterocyclyl fused (mono)cycloalkyl, 3- to 7-membered (mono)heterocyclyl fused C4-6(mono)cycloalkyl, 6- to 10-membered (mono)aryl fused 3- to 7-membered (mono)heterocyclyl, 6- to 10-membered (mono)aryl fused 6- to 10-membered (bis)heterocyclyl, 6- to 10-membered (bis)aryl fused 6- to 10-membered (mono)heterocyclyl, or 6- to 10-membered (bis)aryl fused 6- to 10-membered (bis)heterocyclyl.

As used herein, the term "aryl" refers to an all-carbon monocyclic or fused polycyclic aromatic group having a conjugated π electron system. For example, as used herein, the term "C6-14 aryl" refers to an aromatic group containing 6 to 14 (e.g. 6 to 12) carbon atoms, such as phenyl or naphthyl. The aryl group is optionally substituted with one or more (such as 1 to 3) suitable substituents (e.g. halogen, - OH, -CN, -NO2, or C1-6 alkyl).

As used herein, the term "heteroaryl" refers to a monovalent monocyclic, bicyclic, or tricyclic aromatic ring system, which has 5, 6, 8, 9, 10, 11, 12, 13, or 14 ring atoms, in particular 1 or 2 or 3 or 4 or 5 or 6 or 9 or 10 carbon atoms, and which contains at least one heteroatom (such as oxygen, nitrogen or sulphur), which may be the same or different. In addition, it can be benzo-fused in each case. In particular, heteroaryl is selected from thienyl, furanyl, pyrrolyl, oxazolyl, thiazolyl, imidazolyl, pyrazolyl, isoxazolyl, isothiazolyl, oxadiazolyl, triazolyl (including 1,2,3-triazolyl and 1,2,4-triazolyl), thiadiazolyl, etc., and benzo derivatives thereof; or pyridyl, pyridazinyl, pyrimidinyl, pyrazinyl, triazinyl, etc., and benzo derivatives thereof.

As used herein, the term "halo" or "halogen" group is defined to include F, Cl, Br or I.

As used herein, the term "haloalkyl" refers to an alkyl group substituted with one or more (such as 1 to 3) same or different halogen atoms, and the alkyl group is as defined herein. The terms "C1-8 haloalkyl," "C1-6 haloalkyl," and "C1-4 haloalkyl" refer to haloalkyl groups having 1 to 8 carbon atoms, 1 to 6 carbon atoms, and 1-4 carbon atoms, respectively, e.g. -CF₃, -C₂F₅, -CHF₂, -CH₂F, -CH₂CF₃, - CH₂Cl or -CH₂CH₂CF₃.

The term "substituted" means that one or more (e.g., one, two, three, or four) hydrogen atoms on a specified atom are replaced by selections from the indicated groups, provided that the substitution does not exceed the normal valence of the specified atom under current circumstances; and the substitution results in a stable compound. Combinations of substituents and/or variables are permitted only when such combinations result in a stable compound.

If a substituent is described as being "optionally substituted", the substituent may be (1) unsubstituted or (2) substituted. If the carbon of a substituent is described as being optionally substituted with one or more of the substituents in the list, one or more hydrogens on the carbon (to the extent of any hydrogen present) may be replaced, alone and/or together, by an independently selected optional substituent. If the nitrogen of a substituent is described as being optionally substituted with one or more of the substituents in the list, one or more hydrogens on the nitrogen (to the extent of any hydrogen present) may each be replaced by an independently selected optional substituent.

If a substituent is described as being "independently selected from" the group, each substituent is selected independently of the other. Thus, each substituent may be the same as or different from another (other) substituent.

As used herein, the term "one or more" refers to one or more than one under reasonable conditions, such as 2, 3, 4, 5 or 10.

Unless indicated, as used herein, the point of attachment of a substituent may be at any suitable position of the substituent.

When the bond of a substituent is depicted as crossing a bond connecting two atoms in a ring ("floating bond"), such substituent may be bonded to any ring atom of the substitutable ring, unless otherwise specified. In the case where an available ring member is shown to carry a substitutable hydrogen atom, the substitutable hydrogen atom has been substantially replaced (i.e., absent) when the floating bond is bonded to the available ring member.

As used herein, NRaRa groups and the like include substituents in which two Ra groups, together with the N to which they are attached, form a ring (optionally comprising an N, O or S atom), and include the following groups, for example: 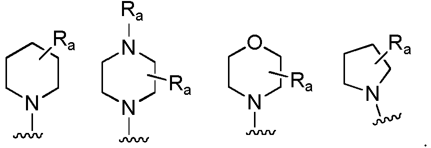 The group NCα-β alkyl)Cα-β alkyl (where α and β are as defined above, representing the number of carbon atoms in the alkyl group) includes substituents where the two Cα-β alkyl groups together form a ring (optionally containing N, O, or S atoms), and encompasses the following groups, for example: 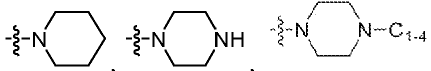 alkyl

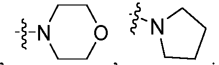

"Bicyclic ring" refers to a group having two connecting rings. Bicyclic rings can be carbocyclic (all ring atoms are carbon atoms) or heterocyclic (in addition to carbon atoms, ring atoms include, for example, 1, 2, or 3 heteroatoms, such as N, O, or S). Both rings can be aliphatic (e.g. decalin and norbornane), or aromatic (e.g. naphthalene), or a combination of aliphatic and aromatic rings (e.g. tetrahydronaphthalene).

Bicyclic rings include (a) spirocyclic compounds in which two rings share only one single atom (spiroatom, which is usually a quaternary carbon), and examples of spirocyclic compounds include, but are not limited to:

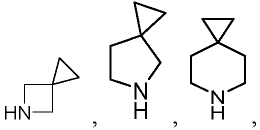

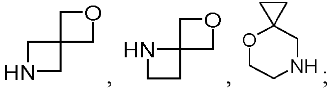

(b) fused bicyclic compounds in which two rings share two adjacent atoms, in other words, the rings share a covalent bond, i.e., the bridgehead atoms are directly linked (e.g., alpha-thujene and decalin), and examples of fused bicyclic rings include, but are not limited to: 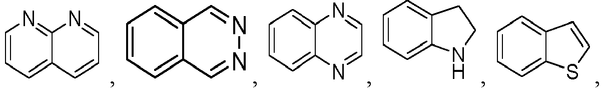 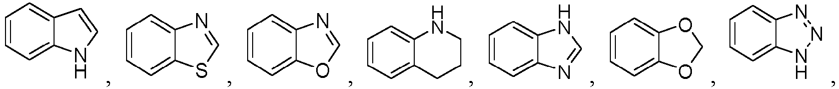 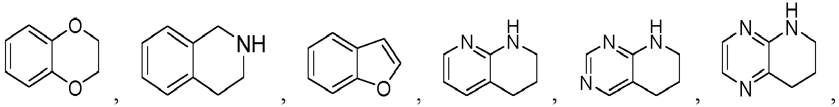 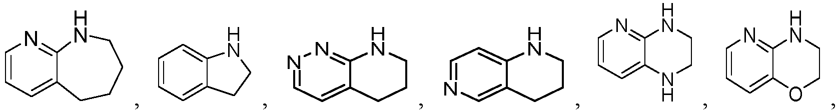 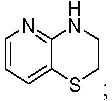 and (c) a bridged bicyclic compound wherein two rings share three or more atoms and separate two bridgehead atoms by a bridge comprising at least one atom, e.g., norbornane, also known as bicyclo[2.2.1]heptane, may be considered a pair of cyclopentane rings, each ring sharing three of their five carbon atoms, and examples of bridged bicyclic rings include, but are not limited to:

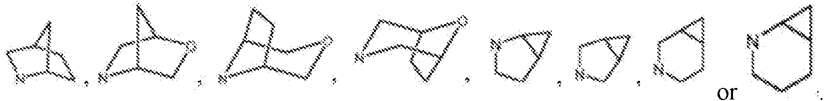

Benzomonoheterocycle refers to a bicyclic ring formed by fusion of a phenyl group with one (mono)heterocyclyl group.

Benzomonoheteroaromatic ring refers to a bicyclic ring formed by the fusion of a phenyl group with one (mono)heteroaryl group.

Benzobisheterocycle refers to a tricyclic ring formed by the fusion of a phenyl group with one bicyclic heterocyclyl group.

A nitrogen-containing heteroaryl fused monoheterocycle refers to a bicyclic ring formed by the fusion of an azaheteroaryl group with one (mono)heterocyclyl group.

A nitrogen-containing heteroaryl fused monoheteroaromatic ring refers to a bicyclic ring formed by the fusion of an azaheteroaryl group with one (mono)heteroaryl group.

A nitrogen-containing heteroaryl fused bisheterocycle refers to a tricyclic ring formed by the fusion of an azaheteroaryl group with one bicyclic heterocyclyl group.

The present disclosure also encompasses all pharmaceutically acceptable isotopically labeled compounds, which are identical to the compounds of the present disclosure except that one or more atoms are replaced by atoms having the same atomic number but having an atomic mass or mass number different from the atomic mass or mass number predominantly found in nature. Examples of isotopes suitable for inclusion in the compounds of the present disclosure include, but are not limited to, isotopes of hydrogen (e.g., deuterium (D, 2H), tritium (T, 3H)); isotopes of carbon (e.g., 11C, 13C, and 14C); isotopes of chlorine (e.g., 36Cl); isotopes of fluorine (e.g., 18F); isotopes of iodine (e.g., 123I and 125I); isotopes of nitrogen (e.g., 13N and 15N); isotopes of oxygen (e.g., 150, 170, and 180); isotopes of phosphorus (e.g., 32P); isotopes of sulphur (e.g., 35S). Certain isotopically labeled compounds of the present disclosure, such as those incorporating radioactive isotopes, may be used in drug and/or substrate tissue distribution studies (such as assays). The radioactive isotopes tritium (i.e., 3H) and carbon-14 (i.e., 14C) are particularly useful for this purpose because they are easy to incorporate and easily detectable. Substitution with positron-emitting isotopes (such as 11C, 18F, 150, and 13N) can be used to examine substrate receptor occupancy in positron emission tomography (PET) studies. Isotopically labeled compounds of the present disclosure may be prepared by methods similar to those described in the accompanying routes and/or embodiments and preparations by using an appropriate isotopically labeled reagent in place of the previously employed non-labeled reagent. Pharmaceutically acceptable solvates of the present disclosure include those in which the crystallization solvent may be isotopically substituted, for example, D2O, acetone-d6 or DMSO-d6.

The term "stereoisomer" refers to an isomer resulting from the presence of at least one asymmetric center. In a compound having one or more (e.g. 1, 2, 3 or 4) asymmetric centers, it can produce racemic mixtures, single enantiomers, diastereomeric mixtures and individual diastereomers. Specific individual molecules can also exist as geometric isomers (cis/trans). Similarly, the compounds of the present disclosure may exist as a mixture of two or more structurally distinct forms that are in rapid equilibrium (commonly referred to as tautomers). Representative examples of tautomers include keto-enol tautomers, phenol-keto tautomers, nitroso-oxime tautomers, imine-enamine tautomers, etc. It is to be understood that the scope of the present application covers all such isomers or mixtures thereof in any proportion (e.g., 60%, 65%, 70%, 75%, 80%, 85%, 90%, 95%, 96%, 97%, 98%, or 99%).

The solid line (-), solid wedge ( 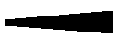 ) or dashed wedge ( 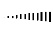 ) can be used herein to depict the chemical bonds of the compounds of the present disclosure. The use of solid lines to depict the bonds connected to asymmetric carbon atoms is intended to indicate that all possible stereoisomers at the carbon atoms (such as specific enantiomers and racemic mixtures) are included. The use of solid or dashed wedges to depict the bonds connected to asymmetric carbon atoms is intended to indicate the presence of the stereoisomers shown. When present in a racemic mixture, solid and dashed wedges are used to define the relative stereochemistry rather than the absolute stereochemistry. Unless otherwise indicated, the compounds of the present disclosure may exist in the form of stereoisomers, including cis and trans isomers, optical isomers (e.g., R- and S-enantiomers), diastereomers, geometric isomers, rotamers, conformers, atropisomers, and mixtures thereof. The compounds of the present disclosure may exhibit more than one type of isomerism, and consist of mixtures thereof (such as racemic mixtures and diastereomeric pairs).

It should also be understood that certain compounds of the present disclosure may exist in free form for therapeutic use, or where appropriate, in the form of pharmaceutically acceptable derivatives thereof. In the present disclosure, pharmaceutically acceptable derivatives include, but are not limited to, pharmaceutically acceptable salts, esters, solvates, metabolites or prodrugs, which, after administration to a patient in need thereof, are capable of providing, directly or indirectly, a compound of the present disclosure or a metabolite or residue thereof. Thus, when reference is made herein to "compounds of the present disclosure", it is also intended to encompass the various derivative forms of the compounds described above.

The wavy line ( 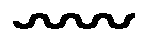 ) may be used herein to depict the chemical bonds of the compounds of the present disclosure. The use of a wavy line to depict a bond to an asymmetric carbon atom is intended to indicate that the absolute configuration at the carbon atom (e.g., R-configuration or S-configuration) is included.

Pharmaceutically acceptable salts of the compounds of the present disclosure include the acid addition salts and the base addition salts thereof.

Suitable acid addition salts are formed from acids that form pharmaceutically acceptable salts. Examples include aspartate, benzoate, bicarbonate/carbonate, bisulfate/sulfate, fumarate, glucoheptonate, gluconate, glucuronate, hexafluorophosphate, hydrobromide/bromide, hydroiodide/iodide, maleate, malonate, methyl sulfate, naphthylate, nicotinate, nitrate, orotate, oxalate, palmitate and other similar salts.

Suitable base addition salts are formed from bases that form pharmaceutically acceptable salts. Examples include aluminium salts, arginine salts, choline salts, diethylamine salts, lysine salts, magnesium salts, meglumine salts, potassium salts and other similar salts.

For a review of suitable salts, see "Handbook of Pharmaceutical Salts: Properties, Selection, and Use" by Stahl and Wermuth (Wiley-VCH, 2002). Methods for preparing pharmaceutically acceptable salts of the compounds of the present disclosure are known to those skilled in the art.

As used herein, the term "ester" refers to an ester derived from a compound of the general formula of the present application, which includes a physiologically hydrolyzable ester (capable of hydrolyzing under physiological conditions to release the free acid or alcohol form of the compound of the present disclosure). The compounds of the present disclosure may themselves be esters.

The present disclosure encompasses all possible crystalline forms or polymorphs of the compounds of the present disclosure, which may exist as a single polymorph or as a mixture of two or more polymorphs in any proportion.

The compounds of the present disclosure may exist in the form of solvates (preferably hydrates), wherein the compounds of the present disclosure contain polar solvents as structural elements of the crystal lattice of the compounds, in particular for example water, methanol or ethanol. The amount of polar solvent, particularly water, may be present in either stoichiometric or non-stoichiometric ratios.

Metabolites of the compounds of the present disclosure, i.e. substances formed in the body upon administration of the compounds of the present disclosure, are also included within the scope of the present disclosure. Such products may be produced through, for example, oxidation, reduction, hydrolysis, amidation, deamidation, esterification, de-esterification, enzymatic cleavage, etc. of the administered compound. Therefore, the present disclosure includes metabolites of the compounds of the present disclosure, including the compounds prepared by a method of contacting the compounds of the present disclosure with a mammal for a time sufficient to produce the metabolites thereof.

The present disclosure further includes within its scope prodrugs of the compounds of the present disclosure, which are certain derivatives of the compounds of the present disclosure which may themselves have little or no pharmacological activity, when administered to or onto the body, can be converted to the compounds of the present disclosure having the desired activity by, for example, hydrolytic cleavage. Usually, such prodrugs are functional derivatives of the compounds, which are readily converted in vivo to the desired therapeutically active compound. Additional information on the use of prodrugs can be found in "Pro-drugs as Novel Delivery Systems", Volume 14, ACS Symposium Series (T. Higuchi and V. Stella) and "Bioreversible Carriers in Drug Design," Pergamon Press, 1987 (E. B. Roche, ed. American Pharmaceutical Association). The prodrugs of the present disclosure may, for example, be prepared with certain moieties known to those skilled in the art as "pro-moieties" (as described in, e.g., "Design of Prodrugs" by H. Bundgaard (Elsevier, 1985)) instead of suitable functional groups present in the compounds of the present disclosure.

The present disclosure also encompasses compounds of the present disclosure containing protecting groups. During any preparation of the compounds of the present disclosure, it may be necessary and/or desirable to protect sensitive or reactive groups on any relevant molecules, thereby generating chemically protected forms of the compounds of the present disclosure. This can be achieved using conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which references are incorporated herein by reference. The protecting groups can be removed at an appropriate subsequent stage using methods known in the art.

As used herein, the term "about" means within ±10%, preferably within ±5%, more preferably within ±2% of the stated value.

The present disclosure is further described below in conjunction with specific examples. It should be understood that these examples are merely used for describing the present disclosure, rather than limiting the scope of the present disclosure. In the following examples, experimental methods in which no specific conditions are specified are carried out according to conventional conditions.

Some representative compounds of the present disclosure can be prepared by the following synthetic methods. In each reaction scheme below, the reagents and conditions for each step may adopt conventional reagents or conditions commonly used in the art for such preparation methods. After disclosure of the compound structures of the present disclosure, the above selections can be made by those skilled in the art based on the knowledge in the art.

### Example 1: synthesis of 2-amino-N-(3-(4,4-difluoropiperidin-1-yl)phenyl)-4-((2-hydroxyethyl)sulfonamide)-6-(6-azaspiro[2.5]octan-6-yl)benzamide (1)

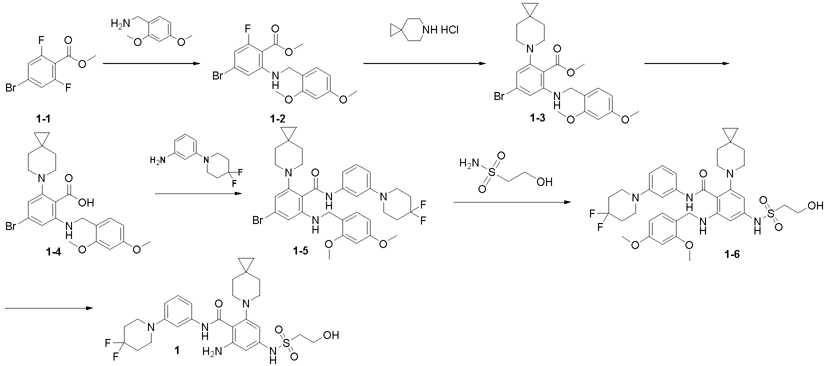

### Step 1: synthesis of compound 1-2

Compound 1-1 (2.0 g, 7.967 mmol) and (2,4-dimethoxyphenyl)methanamine (1.20 mL, 7.97 mmol) were dissolved in DMF (10 mL) solution, to which Cs₂CO₃ (5191.62 mg, 15.93 mmol) was added, and the reaction mixture was stirred in an oil bath at 60°C for 1 hour. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=40:1-20:1) to obtain compound 1-2.

### Step 2: synthesis of compound 1-3

6-Azaspiro[2.5]octane hydrochloride (714.04 mg, 4.84 mmol) was dissolved in DMSO (10 mL), and NaH (338.52 mg, 14.11 mmol) was added and stirred for a while. Compound 1-2 (1.6 g, 4.03 mmol) was then added and the reaction was stirred at 125°C overnight. After the reaction was completed, the reaction mixture was diluted with EA (60 mL), and the organic layer was washed with brine (80 mL), dried over Na₂SO₄, filtered and concentrated. The crude product was purified by column chromatography (PE: EA = 100:0-49:1) to obtain compound 1-3.

### Step 3: synthesis of compound 1-4

To a solution of compound 1-3 (325 mg, 0.66 mmol) in MeOH (2 mL) and H₂O (0.1 mL) was added NaOH (79.69 mg, 1.99 mmol) and the reaction was stirred at 60°C overnight. The reaction mixture was diluted with EA (50 mL). The organic layer was washed with brine (80 mL), dried over Na₂SO₄, filtered and concentrated. The crude product was purified by column chromatography (PE:EA=100:0-90:10-80:20) to obtain compound 1-4.

### Step 4: synthesis of compound 1-5

Compound 1-4 (150 mg, 0.32 mmol) and 3-(4,4-difluorohexahydropyridin-1-yl)aniline (73.67 mg, 0.35 mmol) were dissolved in DMF (2 mL), and HATU (143.98 mg, 0.38 mmol) and DIEA (0.10 mL, 0.63 mmol) were added, and the reaction was stirred at room temperature for 2 hours. The reaction mixture was diluted with EA (20 mL), and the organic layer was washed with brine (180 mL), dried over Na₂SO₄, filtered and concentrated. The residue was purified by column chromatography (PE:EA=100:0-95:5-90:10) to obtain compound 1-5.

### Step 5: synthesis of compound 1-6

Compound 1-5 (116 mg, 0.17 mmol), 2-hydroxyethyl-1-sulfonamide (85.10 mg, 0.68 mmol), a catalyst (CAS: 1599466-85-9; 14.46 mg, 0.02 mmol) and Cs₂CO₃ (110.78 mg, 0.34 mmol) were added to the reaction flask, anhydrous 1,4-dioxane (2 mL) was added under argon protection and the reaction was stirred at 100°C for 4 hours. The reaction mixture was diluted with EA (12 mL), and the organic layer was washed with brine (180 mL), dried over Na₂SO₄, filtered and concentrated. The residue was purified by column chromatography (PE:EA=100:0-90:10-60:40) to obtain product compound 1-6.

### Step 6: synthesis of compound 1

Compound 1-6 (85 mg, 0.13 mmol) was added to the reaction flask, followed by DCM (1 mL) and CF₃COOH (0.3 mL, 0.13 mmol), and the reaction was stirred at room temperature for 0.5 hours. The reaction mixture was diluted with DCM (30 mL). The organic layer was washed with brine (20 mL), dried over Na₂SO₄, filtered and concentrated. The crude product was purified by preparative chromatography to obtain product compound 1.

¹H NMR (400 MHz, DMSO-d₆) *δ* = 10.31-9.95 (m, 1H), 9.41-9.32 (m, 1H), 7.83 (br d, *J =* 8.4 Hz, 1H), 7.04 (br d, *J =* 8.1 Hz, 2H), 7.00-6.95 (m, 1H), 6.89 (br d, *J =* 8.5 Hz, 1H), 6.33 (br d, *J* = 7.6 Hz, 1H), 4.99-4.87 (m, 2H), 3.81-3.73 (m, 2H), 3.48-3.41 (m, 2H), 3.38-3.33 (m, 4H), 2.97-2.91 (m, 2H), 2.90-2.81 (m, 2H), 2.07-1.99 (m, 2H), 1.90-1.81 (m, 2H), 1.72 (br s, 2H), 1.67-1.58 (m, 2H), 0.50 (br d, *J* = 7.3 Hz, 2H), 0.21 (br d, *J* = 4.3 Hz, 2H).

### Example 3: synthesis of 2-amino-N-(2-(4, 4-difluoropiperidin-1-yl)-6-methylpyrimidin-4-yl)-4-(2-hydroxyethylsulfonamido)-6-(6-azaspiro[2.5]octan-6-yl)benzamide (3)

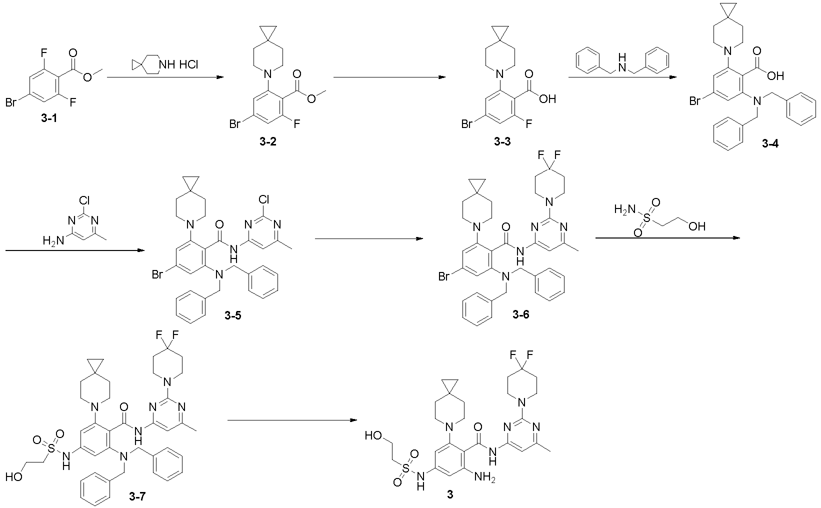

### Step 1: synthesis of compound 3-2

Compound 3-1 (5.0 g, 19.92 mmol) and 6-azaspiro[2.5]octane hydrochloride (3.53 g, 23.90 mmol) were dissolved in a mixed solvent of N,N-dimethylformamide (15 mL) and water (15 mL), to which N,N-diisopropyl-ethylamine (7.72 g, 59.75 mmol) was added and the reaction mixture was stirred in an oil bath at 100°C for 6 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=98:2-95:5) to obtain compound 3-2.

### Step 2: synthesis of compound 3-3

Compound 3-2 (5.9 g, 17.24 mmol) was dissolved in a mixed solvent of MeOH (32 mL) and H₂O (8 mL), to which NaOH (4.14 g, 103.45 mmol) was added and the reaction mixture was stirred in an oil bath at 60°C for 3 hours. After the reaction was completed, 2M HCl was added to adjust the reaction solution to an acidic system, and the reaction solution was diluted with water (100 mL), and extracted with ethyl acetate (30×3 mL). The organic layer was dried over Na₂SO₄, filtered and concentrated to obtain compound 3-3.

### Step 3: synthesis of compound 3-4

Compound 3-3 (3.0 g, 9.14 mmol) was dissolved in 1,4-dioxane (20 mL), to which dibenzylamine (9.02 g, 45.71 mmol) was added and the reaction mixture was stirred overnight in an oil bath at 100°C. After the reaction was completed, the reaction solution was diluted with water (100 mL), and extracted with ethyl acetate (60×3 mL). The organic layer was washed with brine (60×3 mL), dried over Na₂SO₄, filtered and concentrated. The crude product was purified by column chromatography (PE:EA=100:0-99:10-97:30) to obtain compound 3-4.

### Step 4: synthesis of compound 3-5

Compound 3-4 (159 mg, 0.31 mmol) was dissolved in DCM (2 mL), the reaction solution was cooled to 0°C, to which oxalyl chloride (119.78 mg, 0.94 mmol) was slowly added, and the reaction mixture was stirred at room temperature for half an hour. After the reaction was completed, the reaction solution was directly subjected to rotary evaporation to dryness for the next step. 2-Chloro-6-methylpyrimidin-4-amine (87.14 mg, 0.61 mmol) was dissolved in DMF (1 mL), the reaction solution was cooled to 0°C and NaH (14.57 mg, 0.61 mmol) was added. The reaction solution was stirred in an oil bath at 60°C for 30 minutes. The reaction solution was then cooled to 0°C, followed by addition of the acyl chloride obtained above. The mixed reaction solution was allowed to react at room temperature for 2 hours. After the reaction was completed, the reaction solution was diluted with water (30 mL), and extracted with ethyl acetate (30×3 mL). The organic layer was washed with brine (30×3 mL), dried over Na₂SO₄, filtered and concentrated. The crude product was purified by column chromatography (PE:EA=97:3-95:5) to obtain compound 3-5.

### Step 5: synthesis of compound 3-6

Compound 3-5 (53 mg, 0.08 mmol) was dissolved in 1-methyl-2-pyrrolidinone (1 mL), to which 4,4-difluoropiperidine hydrochloride (26.47 mg, 0.17 mmol) was added. The reaction mixture was stirred in an oil bath at 180°C for 2 hours. After the reaction was completed, the reaction solution was diluted with water (30 mL), and extracted with ethyl acetate (10×3 mL). The organic layer was washed with brine (10×3 mL), dried over Na₂SO₄, filtered and concentrated. The crude product was purified by column chromatography (PE:EA=100:0-97:3) to obtain compound 3-6.

### Step 6: synthesis of compound 3-7

Compound 3-6 (34 mg, 0.05 mmol) and 2-hydroxyethane-1-sulfonamide (23.78 g, 0.19 mmol) were dissolved in 1,4-dioxane (1 mL), to which a catalyst (CAS: 1599466-83-7; 8.74 mg, 0.01 mmol) and cesium carbonate (30.96 mg, 0.10 mmol) were added, and the reaction mixture was stirred in an oil bath at 95°C for 3 hours. After the reaction was completed, the reaction solution was diluted with water (30 mL), and extracted with ethyl acetate (10×3 mL). The organic layer was washed with brine (10×3 mL), dried over Na₂SO₄, filtered and concentrated. The crude product was purified by column chromatography (PE:EA=100:0-90:10-80:20) to obtain compound 3-7.

### Step 7: synthesis of compound 3

Compound 3-7 (28 mg, 0.04 mmol) was dissolved in methanol (1 mL), to which 10% palladium on carbon (0.43 mg) was added. The reaction mixture was stirred overnight at room temperature under hydrogen atmosphere. After the reaction was completed, the reaction solution was diluted with water (10 mL), and extracted with ethyl acetate (10×3 mL). The organic layer was washed with brine (10×3 mL), dried over Na₂SO₄, filtered and concentrated. The residue was purified to obtain compound 3. MS (ESI, pos.ion) m/z: 580.2 [M+1]⁺.

¹H NMR (400 MHz, DMSO-*d*₆): *δ* = 13.62-13.54 (m, 1H), 7.39-7.35 (m, 1H), 7.29-7.19 (m, 2H), 6.48-6.44 (m, 1H), 6.39-6.34 (m, 1H), 3.97-3.87 (m, 4H), 3.78-3.72 (m, 2H), 3.33-3.29 (m, 2H), 2.96-2.81 (m, 4H), 2.53-2.51 (m, 4H), 2.31-2.27 (m, 3H), 2.04-1.94 (m, 4H), 0.38-0.33 (m, 4H).

### Example 4: synthesis of N-(2-(4,4-difluoropiperidin-1-yl)-6-methylpyrimidin-4-yl)-4-(2-hydroxyethylsulfonamide)-2-methylamino-6-(6-azaspiro[2.5]octan-6-yl)benzamide (4)

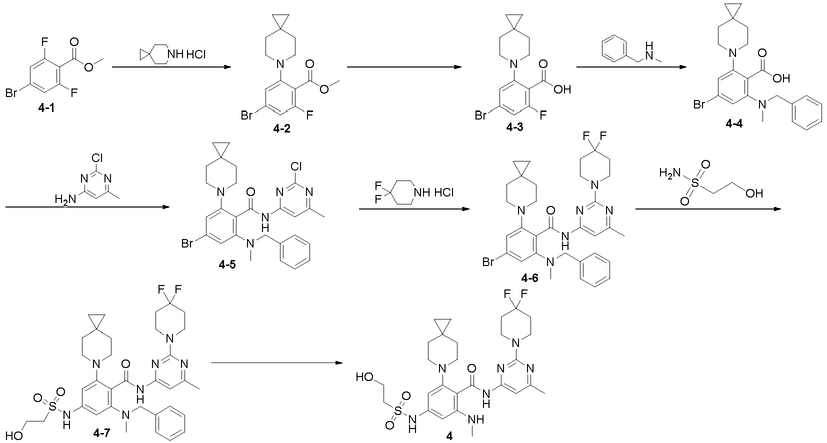

### Step 1: synthesis of compound 4-2

Compound 4-1 (5.0 g, 19.92 mmol) and 6-azaspiro[2.5]octane hydrochloride (3.53 g, 23.90 mmol) were dissolved in a mixed solvent of DMF (25 mL) and H₂O (25 mL), to which Cs₂CO₃ (7.72 g, 59.75 mmol) was added and the reaction mixture was stirred in an oil bath at 100°C for 3 hours. After the reaction was completed, the reaction solution was diluted with water (50 mL), and extracted with ethyl acetate (30×3 mL). The organic layer was washed with brine (30×3 mL), dried over Na₂SO₄, filtered and concentrated. The crude product was purified by column chromatography (PE:EA=98:2-95:5) to obtain compound 4-2.

### Step 2: synthesis of compound 4-3

Compound 4-2 (5.8 g, 16.95 mmol) was dissolved in a mixed solvent of MeOH (32 mL) and H₂O (8 mL), to which NaOH (4.07 g, 101.69 mmol) was added and the reaction mixture was stirred in an oil bath at 60°C for 3 hours. After the reaction was completed, 2M HCl was added to adjust the reaction solution to an acidic system, and the reaction solution was diluted with water (50 mL), and extracted with ethyl acetate (30×3 mL). The organic layer was dried over Na₂SO₄, filtered and concentrated to obtain compound 4-3.

### Step 3: synthesis of compound 4-4

Compound 4-3 (2.0 g, 6.09 mmol) was dissolved in N-methylbenzylamine (10 mL) and the reaction mixture was stirred in a microwave reactor at 150°C for 30 minutes. After the reaction was completed, the reaction solution was diluted with water (60 mL), and extracted with ethyl acetate (60×3 mL). The organic layer was washed with brine (30×3 mL), dried over Na₂SO₄, filtered and concentrated. The crude product was purified by column chromatography (DCM:MeOH=99:1-97:3) to obtain compound 4-4.

### Step 4: synthesis of compound 4-5

Compound 4-4 (400 mg, 0.93 mmol) was dissolved in DCM (6 mL), the reaction solution was cooled to 0°C, to which oxalyl chloride (355 mg, 2.79 mmol) was slowly added, and the reaction mixture was stirred at room temperature for half an hour. After the reaction was completed, the reaction solution was directly subjected to rotary evaporation to dryness for the next step. 2-Chloro-6-methylpyrimidin-4-amine (200.63 mg, 1.40 mmol) was dissolved in DMF (4 mL), the reaction solution was cooled to 0°C and NaH (33.54 mg, 1.40 mmol) was added. The reaction solution was stirred in an oil bath at 60°C for 30 minutes. The reaction solution was then cooled to 0°C, followed by addition of the acyl chloride obtained above. The mixed reaction solution was allowed to react at room temperature for 3 hours. After the reaction was completed, the reaction solution was diluted with water (30 mL), and extracted with ethyl acetate (30×3 mL). The organic layer was washed with brine (30×3 mL), dried over Na₂SO₄, filtered and concentrated. The crude product was purified by column chromatography (PE:EA=100:0-97:3) to obtain compound 4-5.

### Step 5: synthesis of compound 4-6

Compound 4-5 (140 mg, 0.25 mmol) and 4,4-difluoropiperidine hydrochloride (47.71 mg, 0.30 mmol) were dissolved in 1-methyl-2-pyrrolidinone (3 mL), to which N,N-diisopropylethylamine (97.82 mg, 0.76 mmol) was added. The reaction mixture was stirred in an oil bath at 180°C for 2 hours. After the reaction was completed, the reaction solution was diluted with water (30 mL), and extracted with ethyl acetate (10×3 mL). The organic layer was washed with brine (10×3 mL), dried over Na₂SO₄, filtered and concentrated. The crude product was purified by column chromatography (PE:EA=100:0-97:3) to obtain compound 4-6.

### Step 6: synthesis of compound 4-7

Compound 4-6 (90 mg, 0.14 mmol) and 2-hydroxyethane-1-sulfonamide (70.08 mg, 0.86 mmol) were dissolved in dioxane (2 mL), to which a catalyst (CAS: 1599466-89-3; 22.62 mg, 0.03 mmol) and Cs₂CO₃ (136.84 mg, 0.42 mmol) were added, and the reaction mixture was stirred in an oil bath at 95°C for 2 hours. After the reaction was completed, the reaction solution was diluted with water (30 mL), and extracted with ethyl acetate (30×3 mL). The organic layer was washed with brine (30×3 mL), dried over Na₂SO₄, filtered and concentrated. The crude product was purified by column chromatography (DCM:MeOH=95:5) to obtain compound 4-7.

### Step 7: synthesis of compound 4

Compound 4-7 (76 mg, 0.11 mmol) was dissolved in methanol (1 mL), to which 10% palladium on carbon (1.18 mg, 0.01 mmol) was added. The reaction mixture was stirred at room temperature under hydrogen atmosphere for 16 hours. After the reaction was completed, the reaction solution was diluted with water (10 mL), and extracted with ethyl acetate (10×3 mL). The organic layer was washed with brine (10×3 mL), dried over Na₂SO₄, filtered and concentrated. The crude product was purified to obtain compound 4. MS (ESI, pos.ion) m/z: 594.3 [M+1]⁺.

¹H NMR (400 MHz, DMSO-d₆) δ = 13.72 (s, 1H), 8.84 - 8.74 (m, 1H), 7.40 - 7.31 (m, 1H), 6.52 - 6.46 (m, 1H), 6.37 (s, 1H), 4.09 (q, *J =* 4.8 Hz, 1H), 3.89 (br s, 4H), 3.75 (t, *J =* 6.5 Hz, 2H), 3.37 -3.36 (m, 4H), 3.17-3.16 (d, *J =* 4.6 Hz, 2H), 2.92 (br s, 4H), 2.79 (br d, *J =* 4.6 Hz, 3H), 2.29 (s, 3H), 2.04 - 1.94 (m, 4H), 0.36 (s, 4H).

### Example 5: synthesis of N-(1-(cyclopropylmethyl)indoline-4-(2-hydroxyethyl)sulfonamido)-2-(6-azaspiro[2.5]octan-6-yl)benzamide (5)

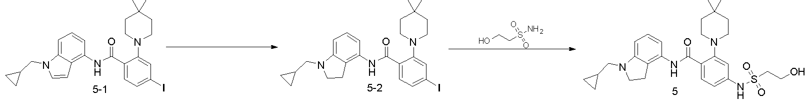

### Step 1: synthesis of compound 5-2

To a suspension of compound 5-1 (250 mg, 0.48 mmol) in AcOH (5 mL) was added NaBH₃CN (89.93 mg, 1.43 mmol), the reaction mixture was stirred at room temperature for 1 h, the reaction was monitored by LCMS, and after the reaction was completed, the reaction mixture was poured into NaOH solution (2N 40 mL) and extracted with EA (80 mL×3). The combined organic layers were washed with saturated sodium chloride aqueous solution (30 mL), dried over anhydrous Na₂SO₄, filtered, and concentrated. The yellow residue was purified by column chromatography (PE:EA = 95: 5-90: 10) to obtain compound 5-2.

### Step 2: synthesis of compound 5

To a solution of compound 5-2 (100 mg, 0.21 mmol) in DMSO (2 mL) were added copper iodide (79.28 mg, 0.42 mmol), a catalyst (CAS: 68737-65-5; 29.61 mg, 0.21 mmol), tripotassium phosphate (132.54 mg, 0.62 mmol) and 2-hydroxyethane-1-sulfonamide (52.09 mg, 0.42 mmol). The reaction mixture was stirred at 120°C overnight under N₂ atmosphere. The reaction mixture was poured into water (10 mL), and extracted with EA (30 mL×3). The combined organic layers were washed with brine (10 mL), dried over Na₂SO₄, filtered and concentrated. The crude product was purified to obtain compound 5.

**¹H NMR** (DMSO-d₆) δ: 11.18 (s, 1H), 9.99-10.21 (m, 1H), 7.94 (d, *J =* 7.6 Hz, 1H), 7.88 (d, *J =* 8.5 Hz, 1H), 7.47 (d, *J =* 3.1 Hz, 1H), 7.30 (d, *J =* 8.3 Hz, 1H), 7.20 (d, *J* = 1.9 Hz, 1H), 7.12 (t, *J* = 8.0 Hz, 1H), 7.04 (dd, *J* = 8.5, 1.9 Hz, 1H), 6.67 (d, *J =* 3.1 Hz, 1H), 4.96 (br s, 1H), 4.05 (d, *J =* 7.0 Hz, 2H), 3.77 (br t, *J =* 6.4 Hz, 2H), 3.33-3.42 (m, 4H), 3.03 (br t, *J =* 4.7 Hz, 4H), 1.47 (br s, 4H), 1.27 (br s, 1H), 0.47-0.59 (m, 2H), 0.35-0.46 (m, 2H), 0.28 (s, 4H).

### Example 6: synthesis of 4-(N-(2-hydroxyethyl)sulfonamido)-N-(1-methylindolin-4-yl)-2-(6-azaspiro[2.5]octan-6-yl)benzamide (6)

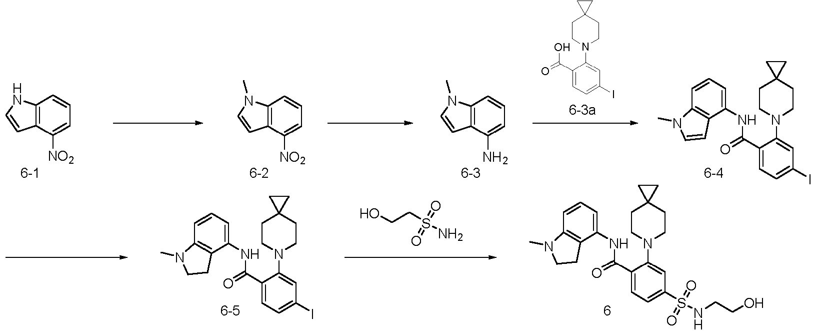

### Step 1: synthesis of compound 6-2

NaH (125 mg, 3 mmol) was added to a solution of compound 6-1 (500 mg, 3 mmol) in DMF, iodomethane (0.2 mL) was added dropwise and the reaction was stirred for 2 hours. The reaction was quenched with water and EA. The organic layer was dried over sodium sulfate and the solvent was removed. The organic layer was washed with water and brine, dried over Na₂SO₄, filtered and concentrated under reduced pressure. The residue was purified by silica gel column chromatography (PE/EA=1/0 to 10/1) to obtain compound 6-2.

### Step 2: synthesis of compound 6-3

A solution of compound 6-2 (520 mg, 2.95 mmol) and 10% Pd/C (63 mg, 0.6 mmol) in methanol (5 mL) was stirred overnight under hydrogen atmosphere. The solution was filtered and concentrated under reduced pressure to obtain compound 6-3.

### Step 3: synthesis of compound 6-4

The mixture of compound 6-3a (468 mg, 1.3 mmol), compound 6-3 (230 mg, 1.6 mmol), HATU (598 mg, 1.57 mmol) and DIPEA (340 mg, 2.6 mmol) in DMF (10 mL) was stirred at room temperature for 3 h. The reaction was then quenched with water and saturated NaHCO₃ solution. The resulting mixture was extracted with EtOAc. The organic layer was dried over Na₂SO₄, filtered and concentrated under reduced pressure. The residue was purified by silica gel column chromatography (PE/EA=1/0 to 4/1) to obtain compound 6-4.

### Step 4: synthesis of compound 6-5

Compound 6-4 (350 mg, 0.72 mmol) was stirred at ambient temperature in AcOH (1 mL), and NaBH₃CN (168 mg, 2.1 mmol) was slowly added. The stirring was continued at room temperature for 2 h. The reaction was then quenched with water and saturated NaHCO₃ solution. The resulting mixture was extracted with EtOAc. The organic layer was dried over Na₂SO₄, filtered and concentrated under reduced pressure. The residue was purified by silica gel column chromatography (PE/EA=1/0 to 4/1) to obtain compound 6-5.

### Step 5: synthesis of compound 6

Compound 6-5 (150 mg, 0.31 mmol) and 2-hydroxyethane-1-sulfonamide (154 mg, 1.23 mmol) were dissolved in DMSO (2 mL), and potassium phosphate (327 mg, 1.54 mmol), copper iodide (30 mg, 0.15 mmol) and sarcosine (14 mg, 0.15 mmol) were added. The reaction was heated at 120°C overnight under nitrogen protection. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 6. MS (ESI, pos.ion) m/z: 485.3 [M+1]⁺.

**¹H NMR** (DMSO-d6) δ: 10.74-10.80 (m, 1H), 10.02-10.11 (m, 1H), 7.79-7.85 (m, 1H), 7.18-7.22 (m, 1H), 7.14-7.17 (m, 1H),6.96-7.06 (m, 2H), 6.29-6.37 (m, 1H), 4.81-5.03 (m, 1H), 3.72-3.80 (m, 2H), 3.32-3.37 (m, 2H), 3.23-3.30 (m, 2H), 2.95-3.02(m, 4H), 2.88-2.95 (m, 2H), 2.68-2.73 (m, 3H), 1.41-1.54 (m, 4H), 0.25-0.40 (m, 4H).

### Example 7: synthesis of 4-(2-hydroxyethyl)sulfonamido-N-(8-methyl-5,6,7,8-tetrahydro-1,8-naphthyridin-4-yl)-2-(6-azaspiro[2.5]octan-6-yl)benzamide

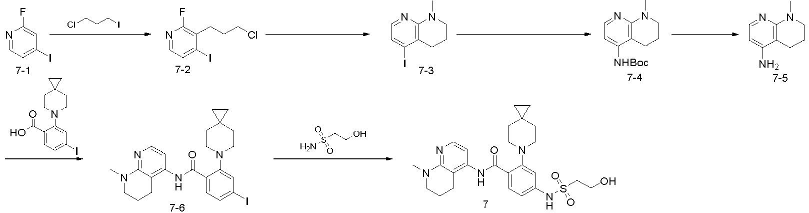

### Step 1: synthesis of compound 7-2

LDA (3.46 g, 32.3 mmol) was added to a solution of compound 7-1 (6 g, 26.9 mmol), cooled to -78°C in THF (80 mL). After 1 hour, 1-chloro-3-iodopropane (16 g, 80.72 mmol) was added dropwise to the solution and the reaction was stirred at room temperature overnight. The reaction was quenched with water and EA. Liquid separation was performed. The organic layer was washed with water and brine, dried over Na₂SO₄, filtered and concentrated under reduced pressure. The residue was purified by silica gel column chromatography (PE/EA=1/0 to 10/1) to obtain compound 7-2.

### Step 2: synthesis of compound 7-3

Compound 7-3 (600 mg, 2 mmol), methylamine hydrochloride (540 mg, 8 mmol), potassium iodide (665 mg, 4 mmol), potassium carbonate (1385 mg, 10 mmol) and DMF (6 mL) were added to a round bottom flask, and the mixture was reacted in an oil bath at 60°C for 5 hours under nitrogen protection. The reaction was cooled to room temperature, and then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 10/1) to obtain compound 7-3.

### Step 3: synthesis of compound 7-4

Compound 7-3 (2.5 g, 9.12 mmol), H₂NBoc (1.6 g, 13.68 mmol), Cs₂CO₃ (5.95 g, 18.2 mmol), Pd₂(dba)₃ (0.42 g, 0.46 mmol) and Xantphos (0.8 g, 1.37 mmol) were added to 1,4-dioxane. The mixture was then heated overnight at 110°C under N₂ atmosphere. The reaction was cooled to room temperature, then quenched with cold water and extracted with EtOAc. The organic layer was washed with brine, dried over Na₂SO₄, filtered and concentrated. The residue was purified by silica gel column chromatography (PE/EA=1/0 to 5/1) to obtain compound 7-4.

### Step 4: synthesis of compound 7-5

Compound 7-4 (2 g, 7.6 mmol) was dissolved in HCl-EA (10 mL) solution and the reaction was stirred at room temperature for 3 hours. HCl-EA was removed by rotary evaporation and the reaction mixture was adjusted to pH 8-9 with water and saturated NaHCO3 solution. The resulting mixture was extracted with EtOAc. The organic layer was dried over Na₂SO₄, filtered and concentrated under reduced pressure to obtain compound 7-5.

### Step 5: synthesis of compound 7-6

A: To a solution of 2-(6-azaspiro[2.5]octan-6-yl)-4-iodobenzoic acid (1.3 g, 3.6 mmol) in DCM (10 mL) was added oxalyl chloride (770 mg, 6 mmol) dropwise at 0°C, followed by DMF (0.10 mL). The reaction mixture was stirred at room temperature for 1 h. The reaction mixture was evaporated under reduced pressure and co-distilled with toluene to obtain crude 2-(6-azaspiro[2.5]oct-6-yl)-4-iodobenzoyl chloride. This product was used in the next step without further purification.

B: To a solution of compound 7-5 in DMF (5 mL) was added NaH (220 mg, 9.1 mmol) at 0°C, followed by 2-(6-azaspiro[2.5]oct-6-yl)-4-iodobenzoyl chloride at 0°C, and the reaction mixture was stirred at room temperature for 3 hours, then quenched with cold water and extracted with EtOAc. The organic layer was washed with water and brine, dried over Na2SO4, filtered and concentrated under reduced pressure and the residue was purified by silica gel column chromatography (PE/EA = 1/0 to 4/1) to obtain compound 7-6.

### Step 6: synthesis of compound 7

Compound 7-6 (170 mg, 1.35 mmol) was dissolved in DMSO (2 mL), and potassium phosphate (372 mg, 1.75 mmol), copper iodide (34 mg, 0.17 mmol) and sarcosine (32 mg, 0.34 mmol) were added. The reaction was heated at 120°C overnight under nitrogen protection. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 7.

MS (ESI, pos.ion) m/z: 500.3 [M+1]⁺.

**¹H NMR** (DMSO-d6) δ: 11.08-11.21 (m, 1H), 10.14-10.23 (m, 1H), 7.78-7.88 (m, 2H), 7.49-7.60 (m, 1H), 7.17-7.23 (m, 1H),7.00-7.09 (m, 1H), 4.82-5.12 (m, 1H), 3.76 (t, *J =* 6.5 Hz, 2H), 3.41-3.50 (m, 4H), 3.12 (br s, 3H), 2.99 (br t, *J =* 4.9 Hz, 4H),2.78 (br d, *J =* 12.3 Hz, 2H), 1.85-1.96 (m, 2H), 1.40-1.52 (m, 4H), 0.29-0.38 (m, 4H).

### Example 8: synthesis of 4-(2-hydroxyethyl)sulfonamido-N-(8-cyclopropylmethyl-5,6,7,8-tetrahydro-1,8-naphthyridin-4-yl)-2-(6-azaspiro[2.5]octan-6-yl)benzamide (8)

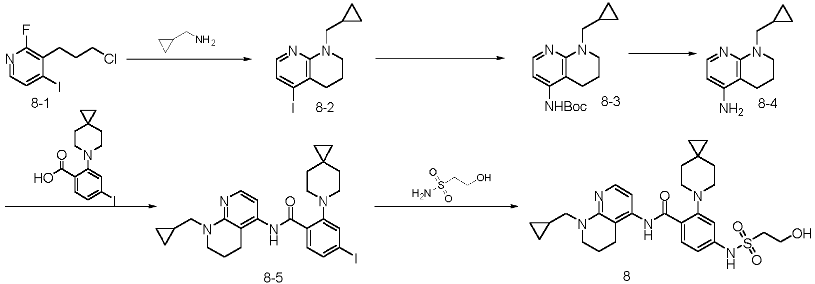

### Step 1: synthesis of compound 8-2

Compound 8-1 (1.2 g, 4 mmol), cyclopropyl methylamine hydrochloride (1.14 g, 16 mmol), potassium iodide (1.33 g, 8 mmol), potassium carbonate (2.77 g, 20 mmol) and DMF (30 mL) were added to a round bottom flask, and the mixture was reacted in an oil bath at 60°C for 5 hours under nitrogen protection. The reaction was cooled to room temperature, and then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 10/1) to obtain compound 8-2.

### Step 2: synthesis of compound 8-3

Compound 8-2 (1.1 g, 3.5 mmol), H₂NBoc (0.65 g, 5.25 mmol), Cs₂CO₃ (2.28 g, 7 mmol), Pd₂(dba)₃ (0.16 g, 0.18 mmol) and Xantphos (0.31 g, 0.53 mmol) were added to 1,4-dioxane. The mixture was then heated overnight at 110°C under N₂ atmosphere. The reaction was cooled to room temperature, then quenched with cold water and extracted with EtOAc. The organic layer was washed with brine, dried over Na₂SO₄, filtered and concentrated. The residue was purified by silica gel column chromatography (PE/EA=1/0 to 5/1) to obtain compound 8-3.

### Step 3: synthesis of compound 8-4

Compound 8-3 (1.5 g, 4.94 mmol) was dissolved in HCl-EA (10 mL) solution and the reaction was stirred at room temperature for 3 hours. HCl-EA was removed by rotary evaporation and the reaction mixture was adjusted to pH 8-9 with water and saturated NaHCO₃ solution. The resulting mixture was extracted with EtOAc. The organic layer was dried over Na₂SO₄, filtered and concentrated under reduced pressure to obtain compound 8-4.

### Step 4: synthesis of compound 8-5

step a: To a solution of 2-(6-azaspiro[2.5]octan-6-yl)-4-iodobenzoic acid (515 mg, 1.44 mmol) in DMF (4 mL) was added pentafluorophenyl trifluoroacetate (530 mg, 1.89 mmol) dropwise at 0°C, and the reaction mixture was stirred at room temperature for 1 hour to obtain crude perfluorophenyl 4-iodo-2-(6-azaspiro[2.5]octanyl)benzoate, which was used in the next step without further purification.

step b: To a solution of compound 8-4 in DMF (5 mL) was added NaHMDS (1.44 mL, 3.6 mmol) at 0°C, and after reaction for 10 minutes, perfluorophenyl 4-iodo-2-(6-azaspiro[2.5]octanyl)benzoate was added at 0°C. The reaction mixture was stirred at 60°C for 5 hours, then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 4/1) to obtain compound 8-5.

### Step 5: synthesis of compound 8

Compound 8-5 (85 mg, 0.16 mmol) and 2-hydroxyethane-1-sulfonamide (78 mg, 0.63 mmol) were dissolved in 1,4-dioxane (2 mL), and tBuXPhos Pd G4 (6 mg, 0.01 mmol) and cesium carbonate (102 mg, 0.31 mmol) were added. The reaction was heated at 100°C overnight under nitrogen protection. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 8. MS (ESI, pos.ion) m/z: 540.3 [M+1]⁺.

**¹H NMR** (DMSO-d6) δ: 10.84-10.91 (m, 1H), 10.07-10.13 (m, 1H), 7.79-7.87 (m, 2H), 7.20-7.26 (m, 1H), 7.16-7.20 (m, 1H), 7.00-7.06 (m, 1H), 4.90-4.97 (m, 1H), 3.72-3.79 (m, 2H), 3.42-3.48 (m, 4H), 3.32-3.37 (m, 2H), 2.95-3.02 (m, 4H), 2.70-2.77 (m, 2H), 1.83-1.92 (m, 2H), 1.42-1.52 (m, 4H), 1.00-1.09 (m, 1H), 0.40-0.46 (m, 2H), 0.34 (s, 4H), 0.21-0.26 (m, 2H).

### Example 9: synthesis of N-(1-(cyclopropylmethyl)-1H-indole-4-(2-hydroxyethyl)sulfonamido)-2-(6-azaspiro[2.5]octan-6-yl)benzamide (9)

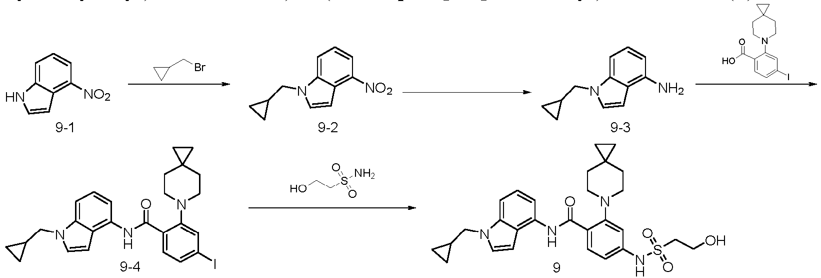

### Step 1: synthesis of compound 9-2

To a solution of compound 9-1 (0.23 mL, 1.85 mmol) in DMF (5 mL) was added NaH (118.41 mg, 2.96 mmol) at 0°C under N₂ atmosphere. The reaction mixture was stirred at room temperature for 15 min under N₂ atmosphere. To the above solution was added (bromomethyl)cyclopropane (0.20 mL, 2.04 mmol). The reaction mixture was stirred at room temperature for 2 h under N2 atmosphere. The reaction mixture was poured into water (50 mL), and extracted with EA (100 mL× 2). The combined organic layers were washed with saturated sodium chloride aqueous solution (50 mL), dried over anhydrous Na₂SO₄, filtered and concentrated. The residue was purified by column chromatography (PE:EA=90: 10) to obtain compound 9-2.

### Step 2: synthesis of compound 9-3

To a solution of compound 9-2 (35 mg, 1.62 mmol) in MeOH (5 mL) was added Pd/C (40 mg, 0.2 mmol), and the reaction system was subjected to H₂ replacement at room temperature. The reaction mixture was stirred overnight at room temperature. The filtrate was concentrated to obtain compound 9-3 which was used directly in the next step.

### Step 3: synthesis of compound 9-4

4-Iodo-2-(6-azaspiro[2.5]octan-6-yl)benzoic acid (675.03 mg, 1.89 mmol) and HATU (783.42 mg, 2.06 mmol) were added to DMF (5 mL), followed by compound 9-3 (320 mg, 1.72 mmol) and DIEA (443.25 mg, 3.44 mmol). The reaction mixture was stirred for 1 h at room temperature. The reaction mixture was poured into ice water (10 mL), and extracted with EA (30 mL×3). The combined organic layers were washed with brine (10 mL), dried over anhydrous Na₂SO₄, filtered and concentrated. The residue was purified by column chromatography (PE:EA=9: 1) to obtain compound 9-4.

### Step 4: synthesis of compound 9

To DMSO (1 mL) was added compound 9-4 (50 mg, 0.10 mmol), copper iodide (39.81 mg, 0.21 mmol), the catalyst: CAS: 68737-65-5 (14.87 mg, 0.10 mmol) and potassium phosphate (66.55 mg, 0.31 mmol) and 2-hydroxyethane-1-sulfonamide (26.16 mg, 0.21 mmol). The reaction mixture was stirred at 120°C overnight under N₂ atmosphere. The reaction mixture was poured into water (10 mL), and extracted with EA (30 mL×3). The combined organic layers were washed with brine (10 mL), dried over Na₂SO₄, filtered and concentrated. The crude product was purified to obtain compound 9.

**¹H NMR** (DMSO-d₆) δ: 11.18 (s, 1H), 9.96-10.23 (m, 1H), 7.94 (d, *J =* 7.8 Hz, 1H), 7.85-7.91 (m, 1H), 7.47 (d, *J =* 3.1 Hz, 1H), 7.30 (d, *J =* 8.3 Hz, 1H), 7.17-7.23 (m, 1H), 7.12 (t, *J* = 8.0 Hz, 1H), 6.99-7.09 (m, 1H), 6.66 (d, *J* = 3.1 Hz, 1H), 4.81-5.08 (m, 1H), 4.05 (d, *J =* 7.0 Hz, 2H), 3.77 (t, *J =* 6.5 Hz, 2H), 3.34-3.39 (m, 2H), 3.03 (br s, 4H), 1.47 (br s, 4H), 1.17-1.35 (m, 1H), 0.46-0.59 (m, 2H), 0.35-0.46 (m, 2H), 0.28 (s, 4H).

### Example 10: synthesis of 4-((2-hydroxyethyl)sulfonamido)-N-(1-methyl-1H-indol-4-yl)-2-(6-azaspiro[2.5]octan-6-yl)benzamide (10)

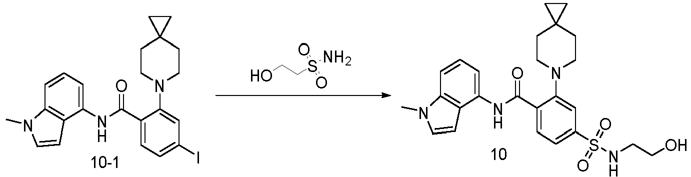

Compound 10-1 (50 mg, 0.1 mmol) and 2-hydroxyethane-1-sulfonamide (52 mg, 0.41 mmol) were dissolved in DMF (2 mL), and potassium phosphate (110 mg, 0.52 mmol), copper iodide (10 mg, 0.05 mmol) and sarcosine (5 mg, 0.05 mmol) were added. The reaction was heated at 100°C overnight under nitrogen protection. EtOAc (20 mL) and water (5 mL) were added to the system and the aqueous phase was washed with EtOAc (3 ×30 mL). The organic solution was separated and concentrated under vacuum. The residue was purified by silica gel column chromatography (PE/EA=1/0 to 1/1) to obtain compound 10. MS (ESI, pos.ion) m/z: 483.2 [M+1]⁺.

**¹H NMR** (DMSO-d6) δ: 11.15-11.23 (m, 1H), 10.06-10.14 (m, 1H), 7.95-8.01 (m, 1H), 7.84-7.90 (m, 1H), 7.33-7.37 (m, 1H), 7.10-7.25 (m, 3H), 7.01-7.06 (m, 1H), 6.64-6.69 (m, 1H), 4.93-4.99 (m, 1H), 3.73-3.83 (m, 5H), 3.33-3.39 (m, 2H), 2.97-3.07(m, 4H), 1.39-1.52 (m, 4H), 0.23-0.31 (m, 4H).

### Example 11: synthesis of 4-((2-hydroxyethyl)sulfonamido)-N-(1-methyl-2,3,4,5-tetrahydro-1H-benzo[b]azepin-6-yl)-2-(6-azaspiro[2.5]octan-6-yl)benzamide (11)

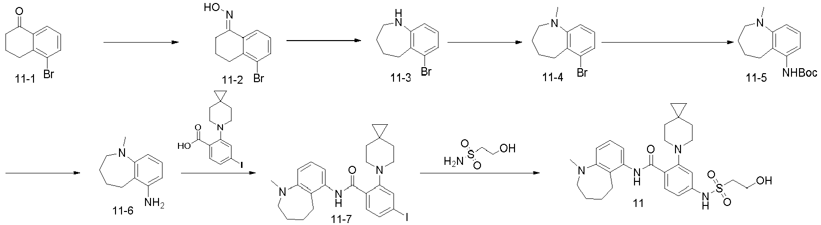

### Step 1: synthesis of compound 11-2

Compound 11-1 (4.5 g, 20 mmol) and hydroxylamine hydrochloride (1.32 g, 40 mmol) were dissolved in pyridine (20 mL) and allowed to react for 1h at room temperature. The reaction was quenched with water and EA. The organic layer was dried over sodium sulfate and the solvent was removed. The organic layer was washed with water and brine, dried over Na₂SO₄, filtered and concentrated under reduced pressure. The residue was purified by silica gel column chromatography (PE/EA=1/0 to 10/1) to obtain compound 11-2.

### Step 2: synthesis of compound 11-3

DIBAL-H (5.78 mL) was added to a solution of compound 11-2 (3 g, 12.5 mmol) in DCM (20 mL) over 10 minutes at 0-5°C under nitrogen atmosphere. Stirring was continued at 0°C for 5 minutes and then at room temperature for 2 hours. KF powder (9.4 g, 25 mmol) and water (10 mL) were added to the reaction mixture at 0°C. The resulting mixture was stirred for 30 minutes at 0°C. The reaction was cooled to room temperature, then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 10/1) to obtain compound 11-3.

### Step 3: synthesis of compound 11-4

Compound 11-3 (700 mg, 3.1 mmol) was dissolved in THF, NaH (186 mg, 4.64 mmol) was added and after reaction was carried out for 20 min at room temperature, iodomethane (660 mg, 4.64 mmol) was added dropwise at 0°C. The reaction mixture was then reacted at room temperature for 2 hours, quenched with water and extracted with EtOAc. The organic layer was washed with brine, dried over Na₂SO₄, filtered and concentrated. The residue was purified by silica gel column chromatography (PE/EA=1/0 to 20/1) to obtain compound 11-4.

### Step 4: synthesis of compound 11-5

Compound 11-4 (350 mg, 1.46 mmol) and H₂NBoc (256 mg, 2.19 mmol), a catalyst (CAS: 1599466-89-3; 59 mg, 0.07 mmol), and *^{t}*BuONa (280 mg, 2.91 mmol) were added to toluene. The mixture was then heated overnight at 105°C under N₂ atmosphere. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 11-5.

### Step 5: synthesis of compound 11-6

Compound 11-5 (300 mg, 1.09 mmol) was dissolved in HCl-EA (10 mL) solution and the reaction was stirred at room temperature for 3 hours. HCl-EA was removed by rotary evaporation and the reaction mixture was adjusted to pH 8-9 with water and saturated NaHCO₃ solution. The resulting mixture was extracted with EtOAc. The organic layer was dried over Na₂SO₄, filtered and concentrated under reduced pressure to obtain compound 11-6.

### Step 6: synthesis of compound 11-7

The mixture of 2-(6-azaspiro[2.5]oct-6-yl)-4-iodobenzoic acid (413 mg, 1.16 mmol), compound 11-6 (170 mg, 0.96 mmol), HATU (514 mg, 1.35 mmol) and DIPEA (250 mg, 1.93 mmol) in DMF (10 mL) was stirred at room temperature for 3 h. The reaction was then quenched with water and saturated NaHCO₃ solution. The resulting mixture was extracted with EtOAc. The organic layer was dried over Na₂SO₄, filtered and concentrated under reduced pressure. The residue was purified by silica gel column chromatography (PE/EA=1/0 to 4/1) to obtain compound 11-7.

### Step 7: synthesis of compound 11

Compound 11-7 (150 mg, 0.29 mmol) and 2-hydroxyethane-1-sulfonamide (150 mg, 1.2 mmol) were dissolved in DMSO (2 mL), and potassium phosphate (318 mg, 1.5 mmol), copper iodide (29 mg, 0.15 mmol) and sarcosine (29 mg, 0.3 mmol) were added. The reaction was heated at 120°C overnight under nitrogen protection. EtOAc (20 mL) and water (5 mL) were added to the system and the aqueous phase was washed with EtOAc (3 ×30 mL). The organic solution was separated and concentrated under vacuum. The residue was purified by silica gel column chromatography (PE/EA=1/0 to 1/1) to obtain compound 11. MS m/z (ESI): 513.3 [M+1]⁺.

¹H NMR (DMSO-d6) δ: 11.50-11.55 (m, 1H), 8.07-8.12 (m, 1H), 7.33-7.35 (m, 1H), 7.10-7.20 (m, 3H), 6.93-6.98(m, 1H), 6.84-6.88 (m, 1H), 3.98-4.03 (m, 2H), 3.21-3.27 (m, 2H), 3.04-3.10 (m, 4H), 2.95-3.00 (m, 2H), 2.87-2.91 (m, 3H),2.76-2.82 (m, 2H), 1.69-1.78 (m, 2H), 1.59-1.64 (m, 2H), 1.46-1.54 (m, 4H), 0.32-0.36 (m, 4H).

### Example 12: synthesis of N-(1-cyclopropylmethyl)-2,3,4,5-tetrahydro-1H-benzo[b]azepin-6-yl)-4-((2-hydroxyethyl)sulfonamido)-2-(6-azaspiro[2.5]octan -6-yl)benzamide (12)

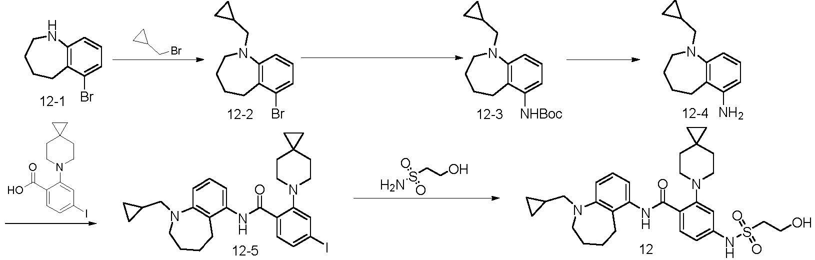

### Step 1: synthesis of compound 12-2

Compound 12-1 (1400 mg, 6.19 mmol) was dissolved in DMF and NaH (496 mg, 12.4 mmol) was added. The reaction mixture was allowed to react for 20 min at room temperature, and bromomethylcyclopropane (1254 mg, 9.29 mmol) was added dropwise at 0°C. The reaction mixture was then reacted at 70°C for 2 hours, quenched with water and extracted with EtOAc. The organic layer was washed with brine, dried over Na₂SO₄, filtered and concentrated. The residue was purified by silica gel column chromatography (PE/EA=1/0 to 20/1) to obtain compound 12-2.

### Step 2: synthesis of compound 12-3

Compound 12-2 (360 mg, 1.28 mmol) and H₂NBoc (226 mg, 1.93 mmol), a catalyst (CAS: 1599466-89-3; 52 mg, 0.06 mmol), and *^{t}*BuONa (247 mg, 2.57 mmol) were added to toluene. The mixture was then heated overnight at 105°C under N₂ atmosphere. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 12-3.

### Step 3: synthesis of compound 12-4

Compound 12-3 (310 mg, 0.98 mmol) was dissolved in HCl-EA (10 mL) solution and the reaction was stirred at room temperature for 3 hours. HCl-EA was removed by rotary evaporation and the reaction mixture was adjusted to pH 8-9 with water and saturated NaHCO₃ solution. The resulting mixture was extracted with EtOAc. The organic layer was dried over Na₂SO₄, filtered and concentrated under reduced pressure to obtain compound 12-4.

### Step 4: synthesis of compound 12-5

The mixture of 2-(6-azaspiro[2.5]oct-6-yl)-4-iodobenzoic acid (277 mg, 0.78 mmol), compound 12-4 (140 mg, 0.78 mmol), HATU (345 mg, 0.91 mmol) and DIPEA (168 mg, 1.29 mmol) in DMF (10 mL) was stirred at room temperature for 3 h. The reaction was then quenched with water and saturated NaHCO₃ solution. The resulting mixture was extracted with EtOAc. The organic layer was dried over Na₂SO₄, filtered and concentrated under reduced pressure. The residue was purified by silica gel column chromatography (PE/EA=1/0 to 4/1) to obtain compound 12-5.

### Step 5: Synthesis of compound 12

Compound 12-5 (150 mg, 0.27 mmol) and 2-hydroxyethane-1-sulfonamide (135 mg, 1.08 mmol) were dissolved in DMSO (2 mL), and potassium phosphate (287 mg, 1.35 mmol), copper iodide (26 mg, 0.14 mmol) and sarcosine (26 mg, 0.27 mmol) were added. The reaction was heated at 120°C overnight under nitrogen protection. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 12. MS m/z (ESI): 553.3 [M+1]⁺.

**¹H NMR** (DMSO-d6) δ: 11.34-11.51 (m, 1H), 8.14-8.20 (m, 1H), 7.32-7.36 (m, 1H), 7.13-7.25 (m, 3H), 6.96-7.04(m, 2H), 4.04-4.12 (m, 2H), 3.27-3.33 (m, 2H), 3.16-3.26 (m, 2H), 3.04-3.14 (m, 6H), 2.80-2.89 (m, 2H), 1.76-1.83 (m, 2H),1.64-1.71 (m, 4H), 1.17-1.37 (m, 2H), 0.97-1.08 (m, 1H), 0.51-0.58 (m, 2H), 0.32-0.39 (m, 4H), 0.19-0.25 (m, 2H).

### Example 13: synthesis of N-(2-chloro-1-(cyclopropylmethyl)-1H-indol-4-yl)-4-((2-hydroxyethyl)sulfonamide)-2-(6-azaspiro[2.5]octan-6-yl)benzamide (13)

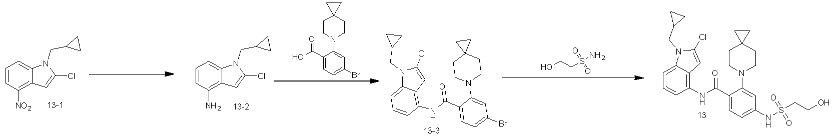

### Step 1: synthesis of compound 13-2

Compound 13-1 (450 mg, 1.80 mmol) was added to AcOH (9 mL), and the reaction mixture was cooled to 0°C. Zinc dust (0.35 g, 5.39 mmol) was added, and the reaction mixture was allowed to warm up to room temperature and stirred overnight. The reaction solution was extracted with EA (10 mL×3), the combined organic phases were washed with water (10 mL×2) and saturated sodium chloride aqueous solution (20 mL×1), respectively, dried over anhydrous Na₂SO₄, and concentrated under reduced pressure. The crude product was purified by silica gel column chromatography (PE:EA (v/v) = 10:1) to obtain compound 13-2. MS m/z (ESI): 221.0 [M+1]⁺.

### Step 2: synthesis of compound 13-3

Compound 13-2 (300 mg, 1.36 mmol), 4-bromo-2-(6-azaspiro[2.5]octyl-6-yl)benzoic acid (421.64 mg, 1.36 mmol), HATU (620.24 mg, 1.63 mmol) and DIEA (0.68 mL, 4.08 mmol) were added to DMF (9 mL) and the reaction solution was stirred at room temperature for 2 hours. After the reaction was completed, the reaction solution was extracted with EA (10 mL×3), the combined organic phases were successively washed with water (10 mL×2) and saturated sodium chloride aqueous solution (20 mL×1), respectively, dried over anhydrous Na₂SO₄, and concentrated under reduced pressure. The crude product was purified by silica gel column chromatography (PE:EA (v/v) = 10:1) to obtain compound 13-3. MS m/z (ESI): 512.0 [M+1]⁺.

### Step 3: synthesis of compound 13

Compound 13-3 (100 mg, 0.19 mmol), 2-hydroxyethane-1-sulfonamide (29.28 mg, 0.23 mmol), Cs₂CO₃ (127.06 mg, 0.39 mmol) and a catalyst (CAS: 1599466-89-3; 0.17 mg, 0.02 mmol) were added to 1,4-dioxane (5 mL) and stirred overnight at 80°C under N₂ protection. The reaction solution was extracted with EA (5 mL×3), the combined organic phases were successively washed with water (5 mL×2) and saturated sodium chloride aqueous solution (5 mL×1), respectively, dried over anhydrous Na₂SO₄, and concentrated under reduced pressure. The residue was purified by preparative chromatography to obtain compound 13. MS m/z (ESI): 557.0 [M+1]⁺

**¹HNMR** (DMSO-d6) δ: 11.09 (s, 1H), 9.94-10.22 (m, 1H), 7.85 (dd, *J =* 19.1, 8.1 Hz, 2H), 7.33 (d, *J =* 8.3 Hz, 1H), 7.12-7.22 (m, 2H), 7.02 (dd, J=8.4, 1.9 Hz, 1H), 6.81 (s, 1H), 4.85-5.11 (m, 1H), 4.14 (d, *J* = 6.9 Hz, 2H), 3.77 (t, *J* = 6.6 Hz, 2H), 3.33-3.37 (m, 2H), 3.01 (br t, *J* = 4.6 Hz, 4H), 1.44 (br s, 4H), 1.16-1.28 (m, 1H), 0.36-0.56 (m, 4H), 0.27 (s, 4H).

### Example 14: synthesis of N-(1-(cyclobutylmethyl)dihydroindol-4-yl)-4-((2-hydroxyethyl)sulfonamido)-2-(6-azaspiro[2.5]octan-6-yl)benzamide

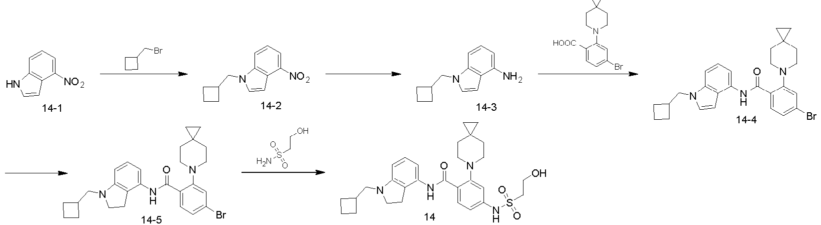

### Step 1: synthesis of compound 14-2.

Compound 14-1 (500 mg, 3.08 mmol) and Cs₂CO₃ (2009.37 mg, 6.17 mmol) were dissolved in DMF (10 mL), and (bromomethyl)cyclobutane (505.50 mg, 3.39 mmol) was slowly added dropwise. The reaction mixture was stirred at 90°C for 3 hours. The reaction solution was diluted with ethyl acetate (20 mL), then washed with saturated NaCl solution (20 mL), dried over anhydrous Na₂SO₄, and concentrated under reduced pressure. The crude product was purified by silica gel column chromatography (PE:EA=1/20-1/10) to obtain compound 14-2.

### Step 2: synthesis of compound 14-3.

Compound 14-2 (700 mg, 3.04 mmol) and Pd/C 10% (70 mg, 0.66 mmol) in MeOH (10 mL) was stirred at room temperature under H₂ for 18 hours. After the reaction mixture was subjected to suction filtration, the filtrate was concentrated under reduced pressure to obtain compound 14-3.

### Step 3: synthesis of compound 14-4.

Compound 14-3 (480 mg, 2.40 mmol), 2-(6-azaspiro[2.5]octan-6-yl)-4-bromobenzoic acid (676 mg, 2.18 mmol) and HATU (994.15 mg, 2.61 mol) were added to CH₃CN (5 mL) solution, a solution of diisopropylethylamine (DIEA) (563.21 mg, 4.36 mmol) was slowly added dropwise, and after stirring at room temperature for 18 hours, the reaction mixture was diluted with EA (20 mL). The organic layer was washed with NaCl (60 mL), dried over Na₂SO₄, filtered, concentrated, and purified by column chromatography (PE:EA=4:1) to obtain compound 14-4.

### Step 4: synthesis of compound 14-5.

Sodium cyanoborohydride (114.84 mg, 1.83 mmol) was added to a solution of compound 14-4 (300 mg, 0.61 mmol) in acetic acid (8 mL). The reaction mixture was stirred at room temperature for 3 hours, adjusted to pH of about 7 - 8 with an aqueous sodium bicarbonate solution, and the reaction mixture was diluted with EA (20 mL). The organic layer was washed with NaCl (60 mL), dried over Na₂SO₄, filtered, and concentrated to obtain compound 14-5.

### Step 5: synthesis of compound 14.

Compound 14-5 (200 mg, 0.4 mmol), 2-hydroxyethane-1-sulfonamide (202.46 mg, 1.62 mmol), Cs₂CO₃ (395.3 mg, 1.21 mmol) and palladium catalyst (CAS: 1599466-89-3; 16.17 mg, 0.02 mmol) were dissolved in 1,4-dioxane (2 mL). The reaction mixture was stirred at 95°C under condensation reflux for 18 hours, and diluted with EA (20 mL). The organic layer was washed with NaCl (60 mL), dried over Na₂SO₄, filtered, concentrated, and purified by column chromatography (DCM:MeOH=20:1) to obtain compound 14.

MS m/z (ESI):539.3 [M+1]⁺.

**¹H NMR** (400 MHz, DMSO-d6) δ = 10.77 (s, 1H), 7.82 (d, *J* = 8.4 Hz, 1H), 7.19 - 7.12 (m, 2H), 7.05 - 6.97 (m, 2H), 6.34 (d, *J =* 7.8 Hz, 1H), 3.82 - 3.72 (m, 2H), 3.35 - 3.33 (m, 2H), 3.31 - 3.26 (m, 2H), 3.05 (d, *J =* 7.1 Hz, 2H), 2.98 (br s, 4H), 2.94 - 2.88 (m, 2H), 2.69 - 2.57 (m, 1H), 2.11 - 2.02 (m, 2H), 1.94 - 1.82 (m, 2H), 1.80 - 1.70 (m, 2H), 1.49 (br s, 4H), 0.35 (s, 4H).

### Example 15: synthesis of N-(1-(cyclopentylmethyl)dihydroindol-4-yl)-4-((2-hydroxyethyl)sulfonamido)-2-(6-azaspiro[2.5]oct-6-yl)benzamide

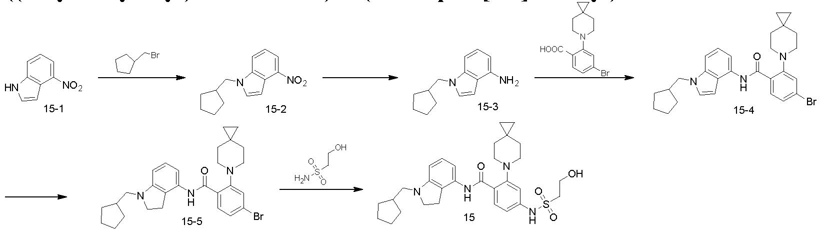

### Step 1: synthesis of compound 15-2.

Compound 15-1 (500 mg, 3.08 mmol) and Cs₂CO₃ (2007.05 mg, 6.16 mmol) were dissolved in DMF (10 mL), and (bromoethyl)cyclopentane (502.81 mg, 3.08 mmol) was slowly added dropwise. The reaction mixture was stirred at 90°C for 18 hours. The reaction solution was diluted with ethyl acetate (20 mL), then washed with saturated NaCl solution (20 mL), dried over anhydrous Na₂SO₄, and concentrated under reduced pressure to obtain compound 15-2.

### Step 2: synthesis of compound 15-3.

Compound 15-2 (570 mg, 2.33 mmol) and Pd/C 10% (57 mg, 0.54 mmol) in MeOH (10 mL) was stirred at room temperature under H₂ for 18 hours. After the reaction mixture was subjected to suction filtration, the filtrate was concentrated under reduced pressure to obtain compound 15-3.

### Step 3: synthesis of compound 15-4.

To a solution of compound 15-3 (320 mg, 1.49 mmol), 2-(6-azaspiro[2.5]octan-6-yl)-4-bromobenzoic acid (421.06 mg, 1.36 mmol) and HATU (625 mg, 1.63 mol) in acetonitrile (10 mL) was added dropwise a solution of DIEA (353 mg, 2.71 mmol), and after stirring at room temperature for 18 hours, the reaction mixture was diluted with EA (20 mL). The organic layer was washed with NaCl (60 mL), dried over Na₂SO₄, filtered, concentrated, and purified by column chromatography (PE:EA=4:1) to obtain compound 15-4.

### Step 4: synthesis of compound 15-5.

To a solution of compound 15-4 (400 mg, 0.79 mmol) in acetic acid (8 mL) was added sodium cyanoborohydride (148.88 mg, 2.37 mmol), and the reaction mixture was stirred at room temperature for 3 hours, and diluted with EA (20 mL). The organic layer was washed with NaCl (60 mL), dried over Na₂SO₄, filtered, and concentrated to obtain compound 15-5.

### Step 5: synthesis of compound 15.

Compound 15-5 (300 mg, 0.59 mmol), 2-hydroxyethane-1-sulfonamide (295.32 mg, 1.62 mmol), Cs₂CO₃ (576.70 mg, 1.77 mmol) and palladium catalyst (CAS: 1599466-89-3; 23.85 mg, 0.03 mmol) were dissolved in 1,4-dioxane (2 mL). The reaction mixture was stirred at 95°C under condensation reflux for 18 hours, and diluted with EA (20 mL). The organic layer was washed with NaCl (60 mL), dried over Na₂SO₄, filtered, concentrated, and purified by column chromatography (DCM:MeOH=20:1) to obtain compound 15. MS (ESI, pos.ion) m/z:553.4 [M+1]⁺.

**¹H NMR** (400 MHz, DMSO-d6) δ = 10.79 (s, 1H), 7.82 (d, *J* = 8.5 Hz, 1H), 7.18 - 7.10 (m, 2H), 7.05 - 6.96 (m, 2H), 6.32 (d, *J =* 7.9 Hz, 1H), 3.81 - 3.72 (m, 2H), 3.40 - 3.33 (m, 4H), 3.02 - 2.96 (m, 4H), 2.96 - 2.90 (m, 4H), 2.20 (s, 1H), 1.81 - 1.70 (m, 2H), 1.62 (br d, *J =* 6.4 Hz, 2H), 1.58 - 1.52 (m, 2H), 1.49 (br s, 4H), 1.26 (br dd, *J =* 6.6, 12.0 Hz, 2H), 0.35 (s, 4H).

### Example 16: synthesis of N-(1-cyclopropylmethyl)-1H-benzimidazol-4-yl)-4-(2-hydroxyethylsulfonamido)-2-(6-azaspiro[2.5]octan-6-yl)benzamide (16)

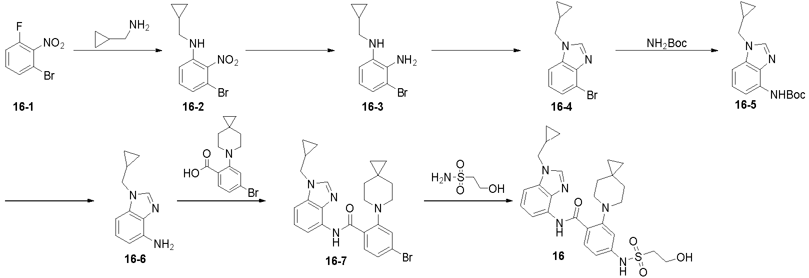

### Step 1: synthesis of compound 16-2

Compound 16-1 (1 g, 4.55 mmol) and cyclopropyl methylamine (0.47 mL, 5.45 mmol) were dissolved in 1,4-dioxane (5 mL), to which potassium carbonate (1.26 g, 9.09 mmol) was added, and the reaction mixture was stirred in an oil bath at 60°C for 4 hours. After the reaction was completed, the reaction solution was diluted with water (100 mL), and extracted with ethyl acetate (30 × 3 mL). The organic layer was washed with brine (30 × 3 mL), dried over Na₂SO₄, filtered and concentrated. The crude product was purified by column chromatography (PE:EA=100:0-98:2) to obtain compound 16-2.

### Step 2: synthesis of compound 16-3

Compound 16-2 (989 mg, 3.65 mmol) was dissolved in ethanol (5 mL) and water (5 mL), to which iron dust (1.02 g, 18.24 mmol) and ammonium chloride (975.65 mg, 18.24 mmol) were added, and the reaction mixture was stirred in an oil bath at 80°C for 3 hours. After the reaction was completed, the reaction solution was filtered and the filtrate was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-97:3) to obtain compound 16-3.

### Step 3: synthesis of compound 16-4

Compound 16-3 (735 mg, 3.05 mmol) was dissolved in isopropanol (5 mL), to which formic acid (701.53 mg, 15.24 mmol) was added. The reaction mixture was stirred in an oil bath at 70°C for 18 hours. After the reaction was completed, the reaction solution was filtered and the filtrate was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-95:5-80:20) to obtain compound 16-4.

### Step 4: synthesis of compound 16-5

Compound 16-4 (400 mg, 1.59 mmol) and tert-butyl carbamate (466.49 mg, 3.98 mmol) were dissolved in 1,4-dioxane (4 mL), to which BrettPhos Pd G4 (293.24 mg, 0.32 mmol) and cesium carbonate (2.08 g, 6.37 mmol) were added. The reaction mixture was stirred in an oil bath at 95°C for 2 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-80:20) to obtain compound 16-5.

### Step 5: synthesis of compound 16-6

Compound 16-5 (28 mg, 0.97 mmol) was dissolved in 1,4-dioxane (3 mL), to which hydrochloric acid (142.10 mg, 3.90 mmol) was added, and the reaction mixture was stirred at room temperature for 30 minutes. After the reaction was completed, the reaction solution was quenched with sodium bicarbonate, and then diluted with water (30 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20-0:100) to obtain compound 16-6.

### Step 6: synthesis of compound 16-7

Compound 16-6 (100 mg, 0.53 mmol) and 4-bromo-2-(6-azaspiro[2.5]octan-6-yl)benzoic acid (165.66 mg, 0.53 mmol) were dissolved in N,N-dimethylformamide (3 mL), to which tetramethyluronium hexafluorophosphate (297.41 mg, 1.06 mmol) and N,N-diisopropylethylamine (276.08 mg, 2.14 mmol) were added. The reaction mixture was stirred at room temperature for 2 hours. After the reaction was completed, the reaction solution was diluted with water (60 mL), and extracted with ethyl acetate (30 × 3 mL). The organic layer was washed with brine (30 × 3 mL), dried over Na₂SO₄, filtered and concentrated. The crude product was purified by column chromatography (PE:EA=100:0-95:5-90:10) to obtain compound 16-7.

### Step 7: synthesis of compound 16

Compound 16-7 (44 mg, 0.09 mmol) and 2-hydroxyethane-1-sulfonamide (45.94 mg, 0.37 mmol) were dissolved in 1,4-dioxane (2 mL), to which a catalyst (CAS: 1599466-83-7; 16.90 mg, 0.02 mmol) and cesium carbonate (59.81 mg, 0.18 mmol) were added. The reaction mixture was stirred in an oil bath at 95°C for 2 hours. After the reaction was completed, the reaction solution was diluted with water (60 mL), and extracted with ethyl acetate (30 × 3 mL). The organic layer was washed with brine (30×3 mL), dried over Na2SO4, filtered and concentrated. The crude product was purified by preparative chromatography to obtain compound 16. MS (ESI, pos.ion) m/z: 524.2[M+1]⁺ .

¹H NMR (400 MHz, DMSO-d6) δ = 12.34 - 12.23 (m, 1H), 8.37 - 8.30 (m, 1H), 8.30 - 8.24 (m, 1H), 8.02 - 7.93 (m, 1H), 7.36 (d, J = 8.1 Hz, 1H), 7.26 - 7.20 (m, 2H), 7.07 - 7.01 (m, 1H), 4.21 - 4.06 (m, 2H), 3.82 - 3.72 (m, 2H), 3.31 (br s, 2H), 3.07 - 2.94 (m, 4H), 1.93 - 1.46 (m, 4H), 1.36 - 1.30 (m, 1H), 0.59 - 0.49 (m, 2H), 0.47 - 0.39 (m, 2H), 0.33 - 0.22 (m, 4H).

### Example 17 Synthesis of N-(1'-(cyclopropylmethyl)-2'-oxospiro[cyclopropane-1,3'-indolin]-4'-yl)-4-((2-hydroxyethyl)sulfonamido)-2-(6-azaspiro[2.5]octan-6-yl)benzamide (17)

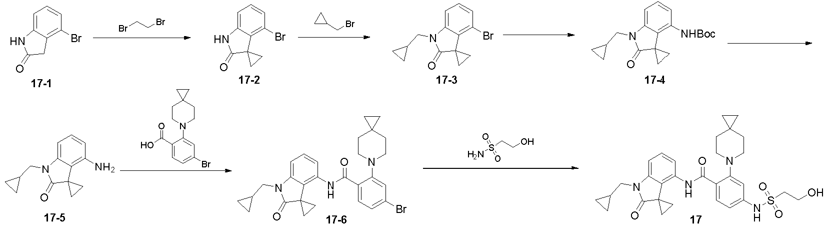

### Step 1: synthesis of compound 17-2

Compound 17-1 (1 g, 4.72 mmol) was dissolved in ultra-dry tetrahydrofuran (10 mL), to which LDA (4.72 mL, 9.44 mmol) was slowly added dropwise at -78°C under nitrogen protection, and the reaction mixture was stirred at -78°C for 1 hour. Then 1,2-dibromoethane (1.22 mL, 14.15 mmol) was added dropwise, slowly warmed to room temperature and stirred overnight. The reaction solution was quenched with ice water, and the reaction system was adjusted to a pH value of 5 with 2N HCl, and extracted with EA. The organic phase was washed with saturated NaCl solution, dried over anhydrous Na2SO4, and concentrated under reduced pressure. The crude product was purified by silica gel column chromatography (PE:EA=5:1-10:1) to obtain compound 17-2. MS (ESI, pos.ion) m/z:238.0/240.0 [M+1]⁺.

### Step 2: synthesis of compound 17-3

Compound 17-2 (310 mg, 1.30 mmol), and cesium carbonate (848 mg, 2.60 mmol) were dissolved in DMF (3mL), to which bromomethylcyclopropane (0.15 mL, 1.56 mmol) was added, and the reaction mixture was reacted at 70°C for 30 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 17-3.

### Step 3: synthesis of compound 17-4

Compound 17-3 (330 mg, 1.13 mmol), NH₂Boc (265 mg, 2.26 mmol), cesium carbonate (1.10 g, 3.39 mmol) and palladium catalyst (CAS: 1599466-85-9; 48 mg, 0.06 mmol) were dissolved in dioxane (5 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 17-4. MS (ESI, pos.ion) m/z:329.2 [M+1]⁺

### Step 4: synthesis of compound 17-5

Compound 17-4 (248 mg, 0.76 mmol) was dissolved in DCM (1 mL), to which HCl\EA (3 mL) was added, and the reaction mixture was allowed to react at 25°C overnight. The reaction solution was quenched with ice water, neutralized with saturated NaHCO₃ solution, and extracted with EA. The organic phase was washed with saturated NaCl solution, dried over anhydrous Na₂SO₄, concentrated under reduced pressure, and dried to obtain compound 17-5. MS (ESI, pos.ion) m/z:229.1 [M+1]⁺.

### Step 5: synthesis of compound 17-6

4-Bromo-2-(6-azaspiro[2.5]octan-6-yl)benzoic acid (181 mg, 0.58 mmol) was dissolved in DCM (1 mL), to which oxalyl chloride (0.10 mL, 1.16 mmol) and a catalytic amount of DMF were added, and the reaction mixture was allowed to react at 25°C for 10 minutes. The reaction solution was concentrated and dried with an oil pump to obtain 4-bromo-2-(6-azaspiro[2.5]octan-6-yl) benzoyl chloride. Compound 17-5 (90 mg, 0.39 mmol) was dissolved in pyridine (2 mL), to which 4-bromo-2-(6-azaspiro[2.5]octan-6-yl)benzoyl chloride was added, and the reaction mixture was allowed to react at 60°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 17-6.

### Step 6: synthesis of compound 17

Compound 17-6 (60 mg, 0.12 mmol), 2-hydroxylethane-1-sulfonamide (25 mg, 0.20 mmol), cesium carbonate (72 mg, 0.60 mmol) and palladium catalyst (CAS: 1599466-89-3; 5 mg) were dissolved in dioxane (2 mL), and the reaction mixture was reacted at 100°C for 2 h under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 17. MS (ESI, pos.ion) m/z:565.3 [M+1]⁺.

**¹H NMR** (400 MHz, DMSO-d6) δ = 10.78 (s, 1H), 10.11 (s, 1H), 7.78 (d, *J*= 8.5 Hz, 1H), 7.34 - 7.28 (m, 1H), 7.22 - 7.16 (m, 2H), 7.04 (br d, *J =* 8.4 Hz, 1H), 6.98 - 6.94 (m, 1H), 3.77 (s, 2H), 3.68 - 3.65 (m, 2H), 3.38 - 3.32 (m, 2H), 3.06 - 3.00 (m, 4H), 1.80 - 1.73 (m, 2H), 1.51 - 1.45 (m, 4H), 1.42 - 1.37 (m, 2H), 1.21 - 1.16 (m, 1H), 0.51 - 0.45 (m, 2H), 0.38 - 0.32 (m, 6H).

### Example 18 Synthesis of N-(3-(cyclopropylmethyl)-2-oxo-2,3-dihydrobenzo[d]oxazol-7-yl)-4-((2-hydroxyethyl)sulfonamido)-2-(6-azaspiro[2.5]oct-6-yl)benzamide (18)

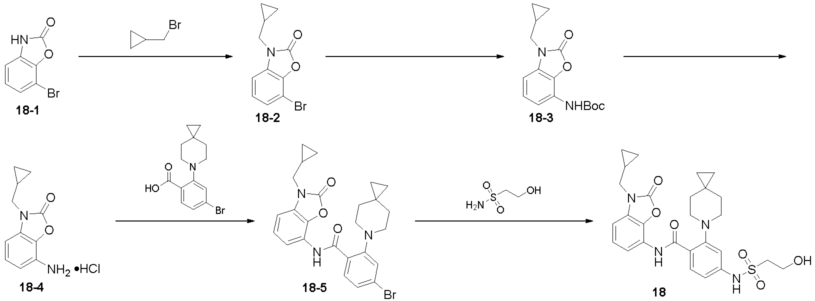

### Step 1: synthesis of compound 18-2

Compound 18-1 (500 mg, 2.34 mmol), and cesium carbonate (1.52 g, 4.68 mmol) were dissolved in ultra-dry DMF (5 mL), to which bromomethylcyclopropane (0.27 mL, 2.81 mmol) was added, and the reaction mixture was reacted at 70°C for 30 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 18-2.

**¹H NMR** (400 MHz, DMSO-d6) δ = 7.40 (dd, J = 0.8, 7.8 Hz, 1H), 7.35 (dd, J = 0.9, 8.3 Hz, 1H), 7.23 - 7.15 (m, 1H), 3.72 (d, J = 7.1 Hz, 2H), 1.24 - 1.17 (m, 1H), 0.55 - 0.49 (m, 2H), 0.43 - 0.37 (m, 2H).

### Step 2: synthesis of compound 18-3

Compound 18-2, NH₂Boc (538 mg, 4.60 mmol), cesium carbonate (2.25 g, 6.89 mmol) and palladium catalyst (CAS: 1599466-85-9; 195 mg, 0.23 mmol) were dissolved in dioxane (10 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 18-3.

### Step 3: synthesis of compound 18-4

Compound 18-3 (527 mg, 1.73 mmol) was dissolved in dichloromethane (2 mL), to which hydrogen chloride-ethyl acetate solution (5 mL) was added, and the reaction mixture was allowed to react at 25°C overnight. The reaction solution was filtered and dried to obtain compound 18-4.

### Step 4: synthesis of compound 18-5

Compound 18-4 (200 mg, 0.83 mmol), 4-bromo-2-(6-azaspiro[2.5]octan-6-yl)benzoic acid (310 mg, 1.00 mmol), and TCFH (351 mg, 1.25 mmol) were dissolved in ultra-dry acetonitrile (2 mL), to which N-methylimidazole (0.33 mL, 4.17 mmol) was added, and the reaction mixture was allowed to react at 25°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 18-5. MS (ESI, pos.ion) m/z:496.1/498.1 [M+1]⁺.

### Step 5: synthesis of compound 18

Compound 18-5 (261 mg, 0.53 mmol), 2-hydroxylethane-1-sulfonamide (329 mg, 2.65 mmol), cesium carbonate (514 mg, 1.59 mmol) and palladium catalyst (CAS: 1599466-87-1; 42 mg, 0.05 mmol) were dissolved in 1,4-dioxane (5 mL), and the reaction mixture was reacted at 100°C for 3 hours under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 18. MS (ESI, pos.ion) m/z:541.3 [M+1]⁺.

**¹H NMR** (400 MHz, DMSO-d6) δ = 12.31 (s, 1H), 8.07 (d, *J* = 8.4 Hz, 1H), 8.04 - 7.97 (m, 1H), 7.32 - 7.27 (m, 1H), 7.25 - 7.18 (m, 1H), 7.18 - 7.07 (m, 2H), 3.80 - 3.70 (m, 4H), 3.38 - 3.35 (m, 2H), 2.99 (br s, 4H), 1.64 (br s, 4H), 1.28 - 1.19 (m, 1H), 0.56 - 0.48 (m, 2H), 0.44 - 0.38 (m, 2H), 0.38 - 0.30 (m, 4H).

### Example 19: Synthesis of 4-((2-hydroxyethyl)sulfonamido)-2-(6-azaspiro[2.5]octan-6-yl)-N-(5,6,7,8-tetrahydroimidazo[1,2-a]pyridin-3-yl)benzamide (19)

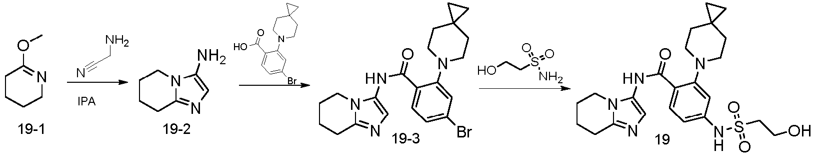

### Step 1: synthesis of compound 19-2

Compound 19-1 (900 mg, 7.95 mmol), and 2-aminoacetonitrile (800 mg, 8.65 mmol) were dissolved in IPA (10 mL) and stirred at 85°C for 3 h. The reaction solution was diluted with EA (20 mL), washed with sodium chloride aqueous solution (30 mL), dried over anhydrous Na₂SO₄, and concentrated under reduced pressure. The crude product was purified by silica gel column chromatography (DCM/MeOH = 1/0 to 20/1) to obtain compound 19-2.

### Step 2: synthesis of compound 19-3

Compound 19-2 (400 mg, 2.92 mmol), HATU (1330 mg, 3.50 mmol), DIEA (1130.5 mg, 8.75 mmol), and 4-bromo-2-(6-azaspiro[2.5]oct-6-yl)benzoic acid (995 mg, 3.21 mmol) were added to DMF (5 mL) and allowed to react overnight at ambient temperature. The reaction solution was diluted with EA (20 mL), washed with sodium chloride aqueous solution (30 mL), dried over anhydrous Na₂SO₄, and concentrated under reduced pressure. The crude product was purified by silica gel column chromatography (DCM/MeOH = 1/0 to 10/1) to obtain compound 19-3.

### Step 3: synthesis of compound 19

Compound 19-3 (200 mg, 0.47 mmol) and methanesulfonato(2-dicyclohexylphosphino-3,6-dimethoxy-2',4',6'-triisopropyl-1,1'-biphenyl)(2'-methylamino-1,1'-biphenyl-2-yl)palladium (II) [CAS: 1599466-83-7] (43 mg, 0.05 mmol), cesium carbonate (455.4 mg, 1.40 mmol), and 2-hydroxyethane-1-sulfonamide (233.17 mg, 1.86 mmol) were dissolved in tert-butanol (5 mL), and the mixture was stirred at 80°C for 3 hours under nitrogen protection. The reaction solution was diluted with EA (20 mL), washed with sodium chloride aqueous solution (30 mL), dried over anhydrous Na₂SO₄, and concentrated under reduced pressure. The crude product was purified by silica gel column chromatography (PE/EA= 1/0 to 5/1) to obtain compound 19.

MS m/z (ESI):474.6 [M+1]⁺.

**¹H NMR** (METHANOL-d 4) δ: 8.01 (d, *J =* 8.5 Hz, 1H), 7.30 (d, *J =* 2.0 Hz, 1H), 7.11 (dd, *J =* 8.5, 2.1 Hz, 1H), 6.88 (s, 1H),3.94-3.98 (m, 2H), 3.84 (t, *J =* 5.8 Hz, 2H), 3.34-3.39 (m, 2H), 3.09 (t, *J =* 5.3 Hz, 4H), 2.78-2.90 (m, 2H), 1.98-2.05 (m, 2H),1.92-1.98 (m, 2H), 1.60 (br s, 4H), 0.42 (s, 4H).

### Example 22: Synthesis of 2-(cyclohexyloxy)-N-(2-(4,4-difluoropiperidin-1-yl)pyridin-4-yl)-4-(2-hydroxyethyl)sulfonamido)benzamide (22)

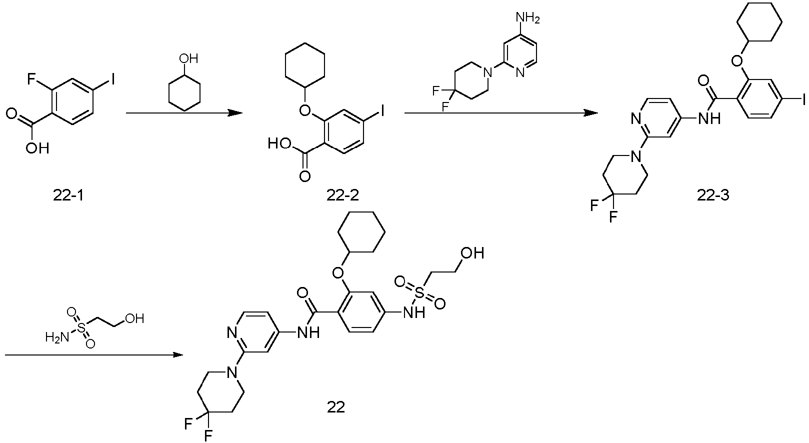

### Step 1: synthesis of compound 22-2

Cyclohexanol (4.5 g, 44.9 mmol) was dissolved in DMF (20 mL), cooled to 0°C, NaH (1.8 g, 44.9 mg) was slowly added, and stirred at ambient temperature for 30 min. Compound 22-1 (2 g, 9.13 mmol) was added, and the mixture was kept at 50°C overnight under nitrogen protection. The reaction solution was diluted with EA (50 mL), washed with sodium chloride aqueous solution (100 mL), dried over anhydrous Na₂SO₄, and concentrated under reduced pressure. The crude product was purified by silica gel column chromatography (PE/EA= 1/0 to 10/1) to obtain compound 22-2.

### Step 2: synthesis of compound 22-3

Compound 22-2 (500 mg, 1.67 mmol) was dissolved in DCM (5 mL), and was added dropwise to oxalyl chloride (350 mg, 2.7 mmol) at 0°C under nitrogen protection, and reacted at room temperature for 1 hour. The reaction solution was directly concentrated and subjected to rotary evaporation to dryness, sealed for later use. 2-(4,4-Difluoropiperidin-1-yl)pyridin-4-amine (270 mg, 1.27 mmol) was dissolved in pyridine (5 mL) and stirred at room temperature for 20 min under nitrogen protection. The crude product was dissolved in DCM (2 mL), added dropwise to the reaction solution, and reacted at room temperature for 3 h. The reaction solution was diluted with EA (20 mL), washed with sodium chloride aqueous solution (30 mL), dried over anhydrous Na₂SO₄, and concentrated under reduced pressure. The crude product was purified by silica gel column chromatography (PE/EA= 1/0 to 10/1) to obtain compound 22-3.

### Step 3: synthesis of compound 22

Compound 22-3 (150 mg, 0.30 mmol), cuprous iodide (28.8 mg, 0.15 mmol), potassium phosphate (321.5 mg, 1.5 mmol), sarcosine (64.3 mg, 0.3 mmol), and 2-hydroxyethane-1-sulfonamide (38.0 mg, 0.60 mmol) were dissolved in DMF (3 mL), and the mixture was reacted at 100°C overnight under nitrogen protection. The reaction solution was diluted with EA (20 mL), washed with sodium chloride aqueous solution (30 mL), dried over anhydrous Na₂SO₄, and concentrated under reduced pressure. The crude product was purified by silica gel column chromatography (PE/EA= 1/0 to 20/1) to obtain compound 22.

MS (ESI, pos.ion) m/z:539.1[M+1]⁺.

**¹H NMR** (DMSO-d6) δ: 10.00-10.23 (m, 2H), 7.99-8.08 (m, 1H), 7.62-7.77 (m, 1H), 7.34-7.50 (m, 1H), 6.96-7.06 (m, 1H),6.81-6.93 (m, 2H), 4.87-5.03 (m, 1H), 4.34-4.48 (m, 1H), 3.72-3.82 (m, 2H), 3.60-3.70 (m, 4H), 1.96-2.08 (m, 6H),1.66-1.77 (m, 2H), 1.54-1.65 (m, 2H), 1.21-1.52 (m, 6H).

### Example 23: synthesis of N-(2-(4,4-difluoropiperidin-1-yl)pyridin-4-yl)-4-((2-hydroxyethyl)sulfonamide)-2-((tetrahydro-2H-pyran-4-yl)oxy)benzamide (23)

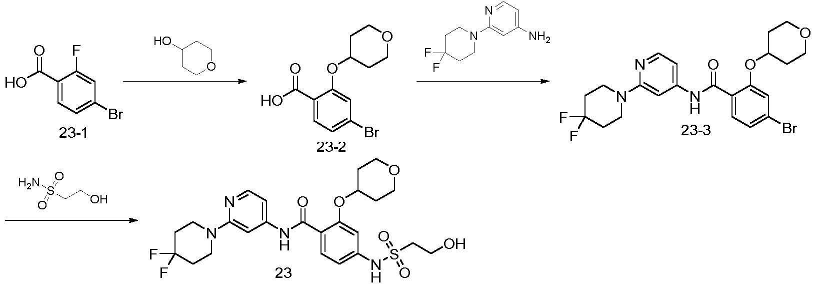

### Step 1: synthesis of compound 23-2

Tetrahydro-2H-pyran-4-ol (700 mg, 6.85 mmol) was dissolved in DMF (10 mL), sodium hydride (330 mg, 8.22 mmol) was added under ice bath conditions, and the reaction mixture was stirred at room temperature for 30 minutes. Then compound 23-1 (300 mg, 1.37 mmol) was added under ice bath conditions, and the reaction mixture was stirred at 80°C overnight. The reaction solution was cooled and poured into an appropriate amount of ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 23-2.

### Step 2: synthesis of compound 23-3

Compound 23-2 (280 mg, 0.93 mmol) was dissolved in dichloromethane, oxalyl chloride (0.16 mL, 1.86 mmol) was added dropwise under ice bath conditions, and the reaction mixture was reacted for 1 hour at room temperature. The reaction mixture was concentrated under reduced pressure, and the crude product was dissolved in dichloromethane (5 mL). This solution was then added dropwise to a solution of 2-(4,4-difluoropiperidin-1-yl)pyridin-4-amine (218 mg, 1.02 mmol) in pyridine (10 mL) under ice bath conditions. The resulting mixture was reacted at 50°C for 2 hours. The reaction solution was cooled and poured into an appropriate amount of ice water, extracted three times with ethyl acetate (20 mL), and washed twice with sodium chloride aqueous solution (60 mL). The organic phase was dried over anhydrous sodium sulfate and concentrated under reduced pressure, and the crude product was purified by silica gel column chromatography (EA/PE = 0-30%) to obtain compound 23-3.

### Step 3: synthesis of compound 23

Compound 23-3 (200 mg, 0.403 mmol), cesium carbonate (394 mg, 1.209 mmol), a catalyst (CAS: 1599466-85-9; 34 mg, 0.04 mmol) and 2-hydroxyethane-1-sulfonamide (252 mg, 2.016 mmol) were dissolved in 1,4-dioxane (10 mL), and the mixture was reacted at 105°C for 16 hours under nitrogen protection. The reaction solution was cooled and poured into ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 23. MS m/z (ESI): 541.2 [M+1]⁺.

**¹H NMR** (DMSO-d6) δ: 10.07 (s, 2H), 8.04 (d, *J =* 5.6 Hz, 1H), 7.66 (d, J=8.5 Hz, 1H), 7.36 (s, 1H), 7.00-7.03 (m, 1H),6.87-6.96 (m, 2H), 4.97 (br t, *J=* 5.0 Hz, 1H), 4.58-4.66 (m, 1H), 3.79-3.85 (m, 2H), 3.74-3.77 (m, 1H), 3.61-3.68 (m, 4H),3.46-3.53 (m, 2H), 3.35 (br s, 2H), 1.92-2.07 (m, 6H), 1.69-1.77 (m, 2H).

### Example 24: synthesis of 2-((4,4-difluorocyclohexyl)oxy)-N-(2-(4,4-difluoropiperidin-1-yl)pyridin-4-yl)-4-((2-hydroxyethyl)sulfonamide)benzamide (24)

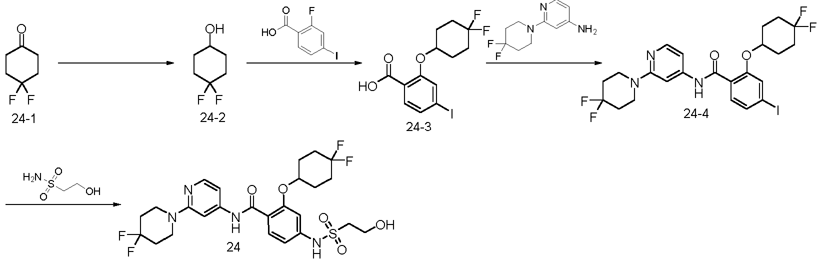

### Step 1: synthesis of compound 24-2

Compound 24-1 (1 g, 7.46 mmol) was dissolved in methanol (20 mL), sodium borohydride (851 mg, 22.39 mmol) was added under ice bath conditions, and the reaction mixture was reacted at room temperature for 2 hours. The reaction solution was diluted with ethyl acetate (50 mL), washed with sodium chloride aqueous solution (100 mL), dried over anhydrous Na₂SO₄, and concentrated under reduced pressure to obtain compound 24-2 which was used directly in the next reaction.

### Step 2: synthesis of compound 24-3

Compound 24-2 (800 mg, 5.88 mmol) was dissolved in DMF (10 mL), sodium hydride (282 mg, 7.06 mmol) was added under ice bath conditions, and the reaction mixture was stirred at room temperature for 30 minutes. Then 2-fluoro-4-iodobenzoic acid (300 mg, 1.13 mmol) was added under ice bath conditions, and the reaction mixture was stirred at 80°C overnight. The reaction solution was cooled and poured into an appropriate amount of ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 24-3.

### Step 3: synthesis of compound 24-4

Compound 24-3 (170 mg, 0.445 mmol) was dissolved in dichloromethane, oxalyl chloride (0.08 mL, 0.89 mmol) was added dropwise under ice bath conditions, and the reaction mixture was reacted for 1 hour at room temperature. The reaction mixture was concentrated under reduced pressure, and the crude product was dissolved in dichloromethane (5 mL). This solution was then added dropwise to a solution of 2-(4,4-difluoropiperidin-1-yl)pyridin-4-amine (218 mg, 1.02 mmol) in pyridine (10 mL) under ice bath conditions. The resulting mixture was reacted at 50°C for 2 hours. The reaction solution was cooled and poured into an appropriate amount of ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-30%) to obtain compound 24-4.

### Step 4: synthesis of compound 24

Compound 24-4 (120 mg, 0.208 mmol), cesium carbonate (203 mg, 0.624 mmol), a catalyst (CAS: 1599466-85-9; 18 mg, 0.02 mmol) and 2-hydroxyethane-1-sulfonamide (130 mg, 1.04 mmol) were dissolved in 1,4-dioxane (10 mL), and the mixture was reacted at 105°C for 16 hours under nitrogen protection. The reaction solution was cooled and poured into ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 24. MS (ESI) m/z: 575.2 [M+1]⁺.

**¹H NMR** (CHLOROFORM-d) δ: 9.64 (s, 1H), 8.17 (d, *J =* 8.5 Hz, 1H), 8.11 (d, *J* = 5.5 Hz, 1H), 7.58 (d, *J* = 1.3 Hz, 1H), 7.16 (d, *J* = 1.8 Hz, 1H), 6.86 (dd, *J* = 8.5, 2.0 Hz, 1H), 6.44 (dd, *J* = 5.5, 1.6 Hz, 1H), 4.67-4.75 (m, 1H), 4.14-4.19 (m, 2H), 3.74-3.80(m, 4H), 3.29-3.36 (m, 2H), 2.16-2.29 (m, 4H), 2.00-2.10 (m, 8H).

### Example 25: synthesis of N-(1-(cyclopropylmethyl)indolin-4-yl)-8-((2-hydroxyethyl)sulfonamido)-2,3-dihydrobenzo[b] [1,4]dioxine-5-carboxamide (25)

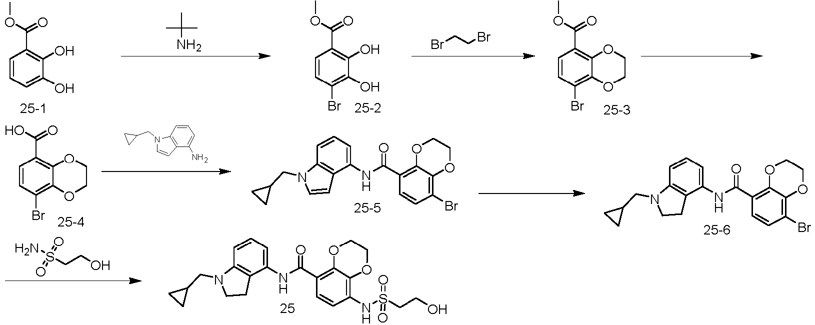

### Step 1: synthesis of compound 25-2

2-Methylpropan-2-amine (1.26 mL, 11.90 mmol) was dissolved in toluene (12 mL) and dichloromethane (7 mL) at 25°C, cooled to -78°C, and bromine (0.37 mL, 7.14 mmol) was slowly added dropwise and stirred for 10 minutes. Compound 25-2 (1 g, 5.95 mmol) dissolved in dichloromethane (7 mL) was added dropwise over 20 minutes. The reaction mixture was stirred at -78°C for 30 minutes and then slowly warmed to room temperature. Ethyl acetate (25 mL) was added and washed with 1M dilute hydrochloric acid (25 mL x 2). The organic phases were combined and then washed with saturated sodium chloride aqueous solution (50 mL), dried over anhydrous sodium sulfate, filtered and concentrated to dryness under reduced pressure to obtain a crude product. The crude product was purified by flash silica gel column chromatography (ethyl acetate/petroleum ether = 5%-10%) to obtain compound 25-2. MS m/z (ESI):246.9 [M+H]⁺

### Step 2: synthesis of compound 25-3

Compound 25-2 (190 mg, 0.77 mmol) was dissolved in DMF (3 mL) at 25°C, cesium carbonate (1002.30 mg, 3.08 mmol) was added, and the mixture was heated to 70°C and stirred for 1 hour. Then 1,2-dibromoethane (0.27 mL, 3.08 mmol) was added and the reaction was stirred at 110°C overnight. Then the reaction was slowly cooled to room temperature. Ethyl acetate (5 mL) was added and washed with water (5 mL×2). The organic phases were combined and then washed with saturated sodium chloride aqueous solution (10 mL), dried over anhydrous sodium sulfate, filtered and concentrated to dryness under reduced pressure to obtain a crude product. The crude product was purified by flash silica gel column chromatography (ethyl acetate/petroleum ether = 5%-10%) to obtain compound 25-3. MS m/z (ESI):272.9 [M+H]⁺

### Step 3: synthesis of compound 25-4

Compound 25-3 (145 mg, 0.53 mmol) was dissolved in tetrahydrofuran (1 mL) at 25°C, and lithium hydroxide (0.04 mL, 2.12 mmol) and water (0.25 mL) were added and stirred at ambient temperature for 2 hours. Then 1M dilute hydrochloric acid was added to adjust the pH to 5. Ethyl acetate (5 mL) was added to the reaction mixture, followed by washing with water (5 mL ×2). The organic phases were combined and then washed with saturated sodium chloride aqueous solution (10 mL), dried over anhydrous sodium sulfate, filtered and concentrated to dryness under reduced pressure to obtain compound 25-4 which was used directly in the next reaction. MS m/z (ESI):258.9 [M+H]⁺

### Step 4: synthesis of compound 25-5

Compound 25-4 (140 mg, 0.54 mmol) was dissolved in DMF (1.5 mL) at 25°C, and 1-(cyclopropylmethyl)indol-4-amine (120.79 mg, 0.65 mmol), HATU (410.98 mg, 1.08 mmol) and DIEA (174.61 mg, 1.35 mmol) were added. The mixture was stirred at 25°C for 2 hours, ethyl acetate (5 mL) was added and the mixture was washed with water (5 mL×2). The organic phases were combined and then washed with saturated sodium chloride aqueous solution (10 mL), dried over anhydrous sodium sulfate, filtered and concentrated to dryness under reduced pressure to obtain a crude product. The crude product was purified by flash silica gel column chromatography (ethyl acetate/petroleum ether = 5%-70%) to obtain compound 25-5. MS m/z (ESI):427.06 [M+H]⁺

### Step 5: synthesis of compound 25-6

Compound 25-5 (222 mg, 0.52 mmol) was dissolved in acetic acid (5 mL) at 25°C and sodium cyanoborohydride (97.94 mg, 1.56 mmol) was added, and the mixture was stirred for 1 hour. Sodium carbonate was added to adjust the pH to 8, ethyl acetate (5 mL) was added to the reaction mixture, followed by washing with water (5 mL×2). The organic phases were combined and then washed with saturated sodium chloride aqueous solution (10 mL), dried over anhydrous sodium sulfate, filtered and concentrated to dryness under reduced pressure to obtain a crude product, which was used directly in the next step. The crude product was purified by flash silica gel column chromatography (PE:EA=20%-50%) to obtain compound 25-6. MS m/z (ESI):429.07 [M+H]⁺

### Step 6: synthesis of compound 25

Compound 25-6 (80 mg, 0.19 mmol) was dissolved in 1,4-dioxane (5 mL) at 25°C, and 2-hydroxyethane-1-sulfonamide (93.28 mg, 0.75 mmol), a catalyst (CAS: 1599466-89-3; 15.06 mg, 0.02 mmol) and cesium carbonate (242.86 mg, 0.75 mmol) were added. The mixture was subjected to nitrogen replacement, and warmed to 100°C and stirred overnight. Ethyl acetate (5 mL) was added to the reaction mixture, followed by washing with water (5 mL×2). The organic phases were combined and then washed with saturated sodium chloride aqueous solution (10 mL), dried over anhydrous sodium sulfate, filtered and concentrated to dryness under reduced pressure to obtain a crude product. The crude product was purified by flash silica gel column chromatography (dichloromethane /methanol =2%-5%) to obtain compound 25. MS m/z (ESI):474.2 [M+H]⁺

H NMR (CHLOROFORM-d) δ: 9.08-9.22 (m, 1H), 7.79-7.89 (m, 1H), 7.40-7.48 (m, 1H), 7.09 (t, *J =* 7.9 Hz, 1H), 6.93-7.04 (m, 1H), 6.26-6.37 (m, 1H), 4.48-4.57 (m, 2H), 4.38-4.46 (m, 2H), 4.07 (br d, *J =* 4.8 Hz, 2H), 3.49-3.59 (m, 2H), 3.36-3.44 (m, 2H), 2.96 (d, *J =* 6.6 Hz, 2H), 2.88-2.94 (m, 2H), 2.54 (br s, 1H), 0.94-1.05 (m, 1H), 0.52-0.59 (m, 2H), 0.15-0.28 (m, 2H).

### Example 26: synthesis of N-(2-(4,4-difluoropiperidin-1-yl)-6-methylpyrimidin-4-yl)-4-((2-hydroxyethyl)sulfonamido)-2-(spiro[3.3]heptan-2-yloxy)benzamide (26)

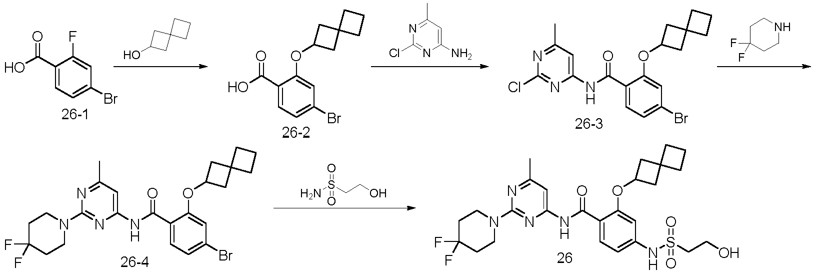

### Step 1: synthesis of compound 26-2

Spiro[3.3]heptan-2-ol (770 mg, 6.88 mmol) was dissolved in DMF (10 ml), sodium hydride (459 mg, 11.5 mmol) was added under ice bath conditions, and the reaction mixture was reacted at room temperature for 1 hour. Compound 26-1 (1 g, 4.59 mmol) was then added under ice bath conditions and the reaction was allowed to react at room temperature for 16 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-30%) to obtain compound 26-2.

### Step 2: synthesis of compound 26-3

Compound 26-2 (700 mg, 2.26 mmol) was dissolved in dichloromethane (10 mL), oxalyl chloride (711 mg, 5.65 mmol) was added dropwise under ice bath conditions, and the reaction was carried out for 1 hour at room temperature and the reaction solution was concentrated for later use. 2-Chloro-6-methylpyrimidin-4-amine (387 mg, 2.7 mmol) was dissolved in DMF (10ml), and sodium hydrogen (180 mg, 4.5 mmol) was added under ice bath conditions. The reaction was allowed to proceed for 1 hour at room temperature, and then the acyl chloride previously prepared was added to the reaction solution under ice bath conditions. The mixture was reacted at 70°C for 16 hours. The reaction solution was cooled and poured into an appropriate amount of ice water, extracted three times with ethyl acetate (50 ml), washed twice with sodium chloride aqueous solution (80 mL), the organic phase was dried over anhydrous sodium sulfate, concentrated under reduced pressure, and the crude product was purified by silica gel column chromatography (EA/PE = 0-70%) to obtain compound 26-3.

### Step 3: synthesis of compound 26-4

Compound 26-3 (300 mg, 0.690 mmol), 4,4-difluoropyridine (108 mg, 0.896 mmol) and DIEA (267 mg, 2.068 mmol) were dissolved in NMP (10 mL), and the mixture was reacted at 180°C for 5 hours under nitrogen protection. The reaction solution was cooled and poured into an appropriate amount of ice water, extracted three times with ethyl acetate (50 ml), washed twice with sodium chloride aqueous solution (80 mL), the organic phase was dried over anhydrous sodium sulfate, concentrated under reduced pressure, and the crude product was purified by silica gel column chromatography (EA/PE = 0-50%) to obtain compound 26-4.

### Step 4: synthesis of compound 26-5

Compound 26-4 (150 mg, 0.288 mmol), cesium carbonate (282 mg, 0.865 mmol), a catalyst (CAS: 1599466-89-3; 24 mg, 0.0288 mmol) and 2-hydroxyethane-1-sulfonamide (72 mg, 0.577 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 26. MS (ESI, pos.ion) m/z: 566.22 [M+1]⁺.

**¹H NMR** (DMSO-*d*6) *δ:* 10.46 (s, 1H), 10.28 (br s, 1H), 7.95 (d, *J =* 8.6 Hz, 1H), 7.34 (s, 1H), 6.94 (dd, *J =* 8.6, 1.9 Hz, 1H),6.84 (d, *J =* 1.6 Hz, 1H), 4.95 (br s, 1H), 4.81 (quin, *J* = 6.5 Hz, 1H), 3.89 (br t, *J =* 5.3 Hz, 4H), 3.71-3.82 (m, 2H), 3.35-3.42 (m,2H), 2.63-2.72 (m, 2H), 2.31 (s, 3H), 2.19-2.26 (m, 2H), 2.02-2.11 (m, 4H), 1.96-2.02 (m, 4H), 1.80-1.89 (m, 2H).

### Example 27: synthesis of 2-(cyclohexyloxy)-N-(1-(cyclopropylmethyl)indolin-4-yl)-4-((2-hydroxyethyl)sulfonamide)benzamide (27)

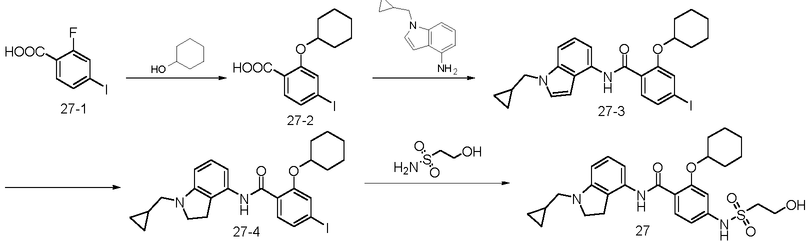

### Step 1: synthesis of compound 27-2

NaH (752 mg, 18.8 mmol) was added to a solution of cyclohexanol (1.5 g, 15 mmol) in DMF and after reaction for 30 min, compound 27-1 (1 g, 3.76 mmol) was added and the reaction was stirred at 80°C for 3 hours. The reaction was cooled to room temperature, then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 3/1) to obtain compound 27-2.

### Step 2: synthesis of compound 27-3

The mixture of compound 27-2 (161 mg, 4.51 mmol), 1-(cyclopropylmethyl)indol-4-amine (700 mg, 3.76 mmol), HATU (2 g, 5.26 mmol) and DIPEA (972 mg, 7.52 mmol) in DMF (10 mL) was stirred at room temperature for 1 h. Then the reaction was quenched with water and saturated NaHCO₃ solution, and the resulting mixture was extracted with EtOAc. The organic layer was dried over Na₂SO₄, filtered and concentrated under reduced pressure. The residue was purified by silica gel column chromatography (PE/EA=1/0 to 4/1) to obtain compound 27-3.

### Step 3: synthesis of compound 27-4

Compound 27-3 (110 mg, 0.21 mmol) was dissolved in acetic acid (3 mL) solution, NaBH₃CN (41 mg, 0.64 mmol) was added and the reaction was stirred at room temperature for 1 hour. Acetic acid was removed by rotary evaporation, water was added, and then the reaction solution was extracted with EtOAc. The organic layer was dried over Na₂SO₄, filtered and concentrated under reduced pressure. The residue was purified by silica gel column chromatography (PE/EA=1/0 to 4/1) to obtain compound 27-4.

### Step 4: synthesis of compound 27

Compound 27-4 (20 mg, 0.04mmol) and 2-hydroxyethane-1-sulfonamide (20 mg, 0.15 mmol) were dissolved in 1,4-dioxane (2 mL), and a catalyst (CAS:1599466-85-9; 2 mg, 0.001 mmol) and cesium carbonate (26 mg, 0.08 mmol) were added. The reaction was heated at 100°C overnight under nitrogen protection. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 27. MS m/z (ESI): 514.3 [M+1]⁺.

¹H NMR (CHLOROFORM-d) δ: 9.54 (s, 1H), 8.15 (d, *J =* 8.5 Hz, 1H), 7.16 (br d, *J =* 8.0 Hz, 1H), 7.05-7.20 (m, 1H), 7.02-7.20(m, 1H), 7.00-7.14 (m, 1H), 6.79 (dd, *J* = 8.5, 1.8 Hz, 1H), 6.34 (d, *J* = 7.8 Hz, 1H), 4.46-4.54 (m, 1H), 4.07 (q, *J* = 4.8 Hz, 2H),3.52 (t, *J* = 8.3 Hz, 2H), 3.23 (br t, *J* = 4.9 Hz, 2H), 2.91-2.98 (m, 4H), 2.14 (br dd, *J* = 12.0, 3.0 Hz, 2H), 1.75-1.85(m, 2H), 1.59-1.65 (m, 2H), 1.36-1.49 (m, 2H), 1.20-1.36 (m, 2H), 0.95-1.06 (m, 1H), 0.51-0.61 (m, 2H),0.19-0.25 (m, 2H),

### Example 28 Synthesis of 2-(cyclopentoxy)-N-(2-(4,4-difluoropiperidin-1-yl)pyridin-4-yl)-4-((2-hydroxyethyl)sulfonamide)benzamide (28)

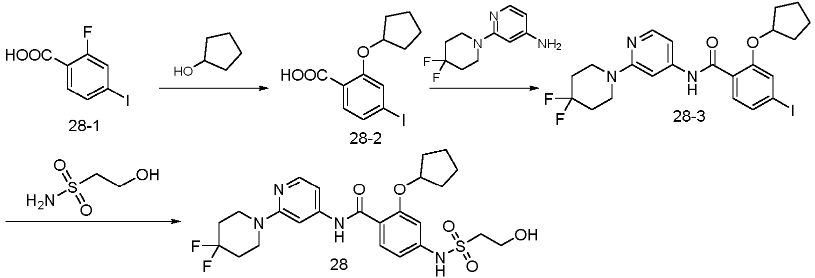

### Step 1: synthesis of compound 28-2

Sodium hydride (601 mg, 15 mmol) was added to a solution of cyclopentanol (972 mg, 11.28 mmol) in DMF. After reaction for 30 min, compound 28-1 (1 g, 3.76 mmol) was added and the reaction was stirred at 80°C for 3 hours. The reaction was cooled to room temperature, then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 3/1) to obtain compound 28-2.

### Step 2: synthesis of compound 28-3

A: To a solution of compound 28-2 (373 mg, 1.3 mmol) in DCM (6 mL) was added oxalyl chloride (286 mg, 2.25 mmol) dropwise at 0°C, followed by DMF (0.10 mL). The reaction mixture was stirred at room temperature for 1 hour, evaporated under reduced pressure and co-distilled with toluene to obtain crude 2-(cyclopentyloxy)-4-iodobenzoyl chloride, which was used in the next step without further purification.
B: To a solution of 2-(4,4-difluorohexahydropyridin-1-yl)pyridin-4-amine (200 mg, 0.94 mmol) in pyridine (2 mL) at 0°C was added the product from step A, the reaction mixture was stirred at 50°C overnight, then quenched with cold water and extracted with EtOAc. The organic layer was washed with water and brine, dried over Na₂SO₄, filtered and concentrated under reduced pressure. The residue was purified by silica gel column chromatography (PE/EA=1/0 to 4/1) to obtain compound 28-3.

### Step 3: synthesis of compound 28

Compound 28-3 (150 mg, 0.28 mmol) and 2-hydroxyethane-1-sulfonamide (143 mg, 1.14 mmol) were dissolved in 1,4-dioxane (2 mL), and a catalyst (CAS:1599466-85-9; 12 mg, 0.01 mmol) and cesium carbonate (186 mg, 0.57 mmol) were added. The reaction was heated at 100°C overnight under nitrogen protection. EtOAc (20 mL) and water (5 mL) were added to the system and the aqueous phase was washed with EtOAc (3x30 mL). The organic solution was separated and concentrated under vacuum. The residue was purified by silica gel column chromatography (PE/EA=1/0 to 1/1) to obtain compound 28. MS m/z (ESI): 525.2 [M+1]⁺.

**¹HNMR** (DMSO-d6) δ: 10.09-10.22 (m, 1H), 10.02 (s, 1H), 8.04 (d, *J =* 5.6 Hz, 1H), 7.71 (d, *J =* 8.5 Hz, 1H), 7.32 (s, 1H), 7.00 (d, *J =* 1.8 Hz, 1H), 6.83-6.93 (m, 2H), 4.92-5.00 (m, 1H), 4.85-4.91 (m, 1H), 3.76 (br t, *J =* 6.2 Hz, 2H), 3.65 (br t, *J =* 5.6 Hz, 4H), 3.34-3.37 (m, 2H), 1.91-2.06 (m, 6H), 1.83-1.91 (m, 2H), 1.70 (br d, *J =* 7.0 Hz, 2H), 1.63 (br d, *J =* 6.8 Hz, 2H).

### Example 29: synthesis of N-(1-(cyclopropylmethyl)indolin-4-yl)-2-((4,4-difluorocyclohexyl)oxy)-4-((2-hydroxyethyl)sulfonamide)benzamide (29)

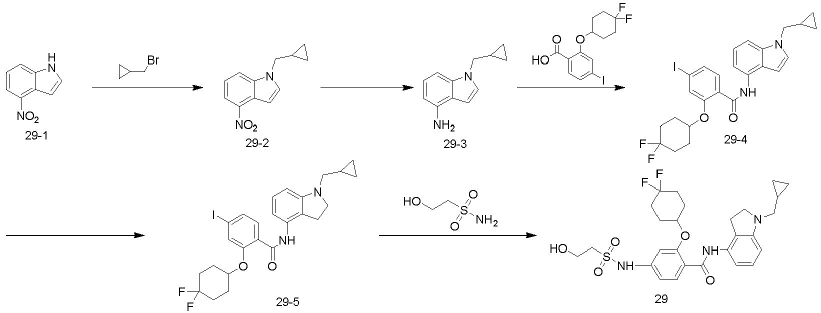

### Step 1: synthesis of compound 29-2

Compound 29-1 (2000 mg, 12.33 mmol) was dissolved in N,N-dimethylformamide (20 mL) at 25°C, and cooled to 0°C. Sodium hydride (986.74 mg, 24.67 mmol) was slowly added, and the mixture was warmed to room temperature, and stirred for 0.5 hours. To the above solution was added bromomethylcyclopropane (1.33 mL, 13.57 mmol) and stirred for 1 hour. Water (50 mL) was added, and the resulting mixture was extracted with ethyl acetate (50 mL× 3). The organic phases were combined and then washed with saturated sodium chloride aqueous solution (50 mL), dried over anhydrous sodium sulfate, filtered and concentrated to dryness under reduced pressure to obtain a crude product. The crude product was purified by flash silica gel column chromatography (ethyl acetate/petroleum ether = 5%-10%) to obtain compound 29-2. MS m/z (ESI):217.09 [M+H]⁺

### Step 2: synthesis of compound 29-3

Compound 29-2 (560 mg, 2.59 mmol) was dissolved in methanol (7 mL) at 25°C, 10% palladium on carbon (56 mg, 0.3 mmol) was added, and the mixture was subjected to hydrogen replacement and stirred overnight. The solution was filtered and concentrated under reduced pressure to dryness to obtain compound 29-3. MS m/z (ESI):187.12 [M+H]⁺

### Step 3: synthesis of compound 29-4

Compound 29-3 (243 mg, 1.30 mmol) and 2-[(4,4-difluorocyclohexyl)oxy]-4-iodobenzoic acid (498.56 mg, 1.30 mmol) were dissolved in N,N-dimethylformamide (7mL) at 25°C, 2-(7-azobenzotriazole)-N,N,N',N'-tetramethyluronium hexafluorophosphate (992.14 mg, 2.61 mmol) was added, and N,N-diisopropylethylamine (421.53 mg, 3.26 mmol) was added dropwise, and the mixture was stirred for 2 hours. Water (20 mL) was added, and the resulting mixture was extracted with ethyl acetate (20 mL×3). The organic phases were combined and then washed with saturated sodium chloride aqueous solution (50 mL), dried over anhydrous sodium sulfate, filtered and concentrated to dryness under reduced pressure to obtain a crude product. The crude product was purified by flash silica gel column chromatography (ethyl acetate/petroleum ether = 5%-13%) to obtain compound 29-4. MS m/z (ESI):551.09 [M+H]⁺

### Step 4: synthesis of compound 29-5

N-(1-(cyclopropylmethyl)-1H-indol-4-yl)-2-((4,4-difluorocyclohexyl)oxy)-4-iodobenzamide (18 mg, 0.03 mmol) was dissolved in acetic acid (0.5 mL) at 25°C and sodium cyanoborohydride (6.17 mg, 0.10 mmol) was added, and the mixture was stirred for 1 hour. Water (2 mL) was added, sodium carbonate was added to adjust the pH to 8, and the resulting mixture was extracted with ethyl acetate (2 mL ×3). The organic phases were combined and then washed with saturated sodium chloride aqueous solution (2 mL), dried over anhydrous sodium sulfate, filtered and concentrated to dryness under reduced pressure to obtain a crude product. The crude product was purified by flash silica gel column chromatography (ethyl acetate/petroleum ether = 10%-15%) to obtain compound 29-5. MS m/z (ESI):553.11 [M+H]⁺ .

### Step 5: synthesis of compound 29

Compound 29-5 (108 mg, 0.20 mmol) was dissolved in 1,4-dioxane (2 mL) at 25°C, and a catalyst (CAS: 1599466-85-9; 16.63 mg, 0.02 mmol) and cesium carbonate (191.10 mg, 0.59 mmol) were added. The mixture was subjected to nitrogen replacement, and warmed to 100°C and stirred overnight under nitrogen protection. The reaction solution was cooled to 25°C. Water (5 mL) was added, and the resulting mixture was extracted with ethyl acetate (5 mL×3). The organic phases were combined and then washed with saturated sodium chloride aqueous solution (20 mL), dried over anhydrous sodium sulfate, filtered and concentrated to dryness under reduced pressure to obtain a crude product. The crude product was purified by flash silica gel column chromatography (ethyl acetate/petroleum ether = 20%-50%) to obtain compound 29. MS m/z (ESI):550.21 [M+H]⁺.

**¹H NMR** (CHLOROFORM-d) δ: 9.09-9.30 (m, 1H), 8.11 (d, *J =* 8.5 Hz, 1H), 7.27-7.33 (m, 1H), 7.16 (*d, J =* 1.5 Hz, 1H),7.05-7.13 (m, 2H), 6.81 (dd, *J* = 8.4, 1.8 Hz, 1H), 6.36 (dd, *J =* 6.8, 1.6 Hz, 1H), 4.61-4.70 (m, 1H), 4.04-4.11 (m, 2H),3.50-3.56 (m, 2H), 3.18-3.25 (m, 2H), 2.97 (d, *J =* 6.6 Hz, 2H), 2.90 (t, *J =* 8.3 Hz, 2H), 2.08-2.22 (m, 4H), 1.94-2.05 (m, 4H),0.95-1.06 (m, 1H), 0.53-0.60 (m, 2H), 0.22 (q, *J =* 4.8 Hz, 2H).

### Example 30 synthesis of 2-(cyclopentoxy)-N-(1-(cyclopropylmethyl)indolin-4-yl)-4-((2-hydroxyethyl)sulfonamide)benzamide (30)

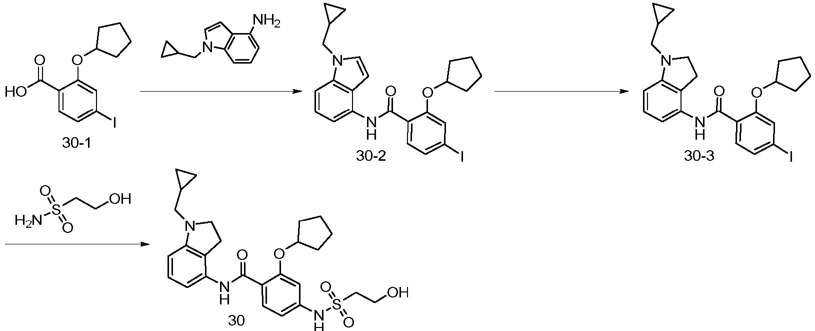

### Step 1: synthesis of compound 30-2

1-(Cyclopropylmethyl)indol-4-amine (123 mg, 0.66 mmol) and compound 30-1 (200 mg, 0.6 mmol) were dissolved in DMF (3 mL), HATU (273.77 mg, 0.72 mmol) and DIEA (0.2 mL, 1.2 mmol) were added, and the reaction was stirred at room temperature for 2 hours. The reaction mixture was diluted with EA (20 mL), and the organic layer was washed with brine (20 mL), dried over Na₂SO₄, filtered and concentrated. The crude product was purified by column chromatography (PE:EA=100:0-95:5-90:10) to obtain compound 30-2.

### Step 2: synthesis of compound 30-3

To a solution of compound 30-2 (200 mg, 0.40 mmol) in CH₃COOH (5 mL) was added NaBH₃CN (0.04 mL, 1.20 mmol). The reaction was stirred at room temperature overnight. The reaction mixture was diluted with EA (90 mL), and the organic layer was washed with brine (20 mL), dried over Na₂SO₄, filtered and concentrated. The crude product was purified by column chromatography (PE:EA=100:0-95:5-90:10) to obtain compound 30-3.

### Step 3: synthesis of compound 30

Compound 30-3 (100 mg, 0.20 mmol) and 2-hydroxyethyl-1-sulfonamide (99.63 mg, 0.80 mmol) were dissolved in 1,4-dioxane (3 mL), and Cs₂CO₃ (64.85 mg, 0.20 mmol) and a catalyst (CAS: 1599466-89-3; 8.89 mg, 0.01 mmol) were added, and the reaction was stirred overnight at 100°C. The reaction mixture was diluted with EA (90 mL), and the organic layer was washed with brine (20 mL), dried over Na₂SO₄, filtered and concentrated. The residue was purified by column chromatography (PE:EA=100:0-90:10-60:40) to obtain compound 30.

**¹H NMR** (400 MHz, DMSO-d6) δ = 11.22 - 11.18 (m, 1H), 7.38 - 7.35 (m, 1H), 7.28 - 7.24 (m, 1H), 7.21 - 7.16 (m, 1H), 6.74- 6.70 (m, 1H), 6.46 - 6.39 (m, 2H), 6.37 - 6.35 (m, 1H), 6.29 - 6.26 (m, 1H), 3.75 (t, ***J** =* 6.8 Hz, 2H), 3.35 (br d, ***J** =* 5.9 Hz,4H), 3.31 - 3.27 (m, 2H), 2.92 - 2.87 (m, 4H), 2.11 - 2.01 (m, 4H), 1.50 - 1.36 (m, 4H), 0.28 (s, 4H).

### Example 32 Synthesis of N-(1-(cyclopropylmethyl)-2,3-dihydro-1H-pyrrolo[2,3-b]pyridin-4-yl)-4-((2-hydroxyethyl)sulfanilamido)-2-(6-azaspiro[2.5]octan-6-yl)4-benzamide (32)

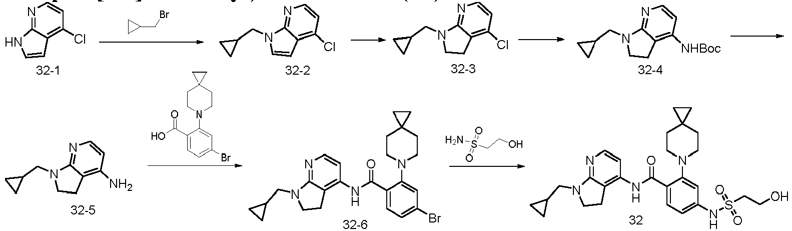

### Step 1: synthesis of compound 32-2

Compound 32-1 (1 g, 6.57 mmol), and cesium carbonate (6.4 g, 19.65 mmol) were dissolved in DMF (10 ml), and bromomethylcyclopropane (0.5 ml, 7.2 mmol) was added at room temperature. The mixture was reacted at 50°C for 2 hours under N₂ protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 32-2.

### Step 2: synthesis of compound 32-3

Compound 32-2 (1 g, 4.85 mmol) was dissolved in glacial acetic acid (5 ml), and sodium cyanoborohydride (1.5 g, 24.25 mmol) was added to the reaction system at 0°C, and the mixture was reacted at 50°C for 12 hours. The reaction solution was diluted with EA (5 mL), washed with sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 32-3.

### Step 3: synthesis of compound 32-4

Compound 32-3 (300 mg, 1.44 mmol), tert-butyl carbamate (338 mg, 2.88 mmol), cesium carbonate (1408 mg, 4.32 mmol) and a catalyst (CAS: 1599466-85-9; 61.3 mg, 0.07 mmol) were dissolved in dioxane (2 mL), and the reaction mixture was reacted at 90°C for 3 hours under nitrogen protection. The reaction solution was diluted with EA (10 mL), then washed with saturated NaCl solution (10 mL), dried over anhydrous Na₂SO₄, and concentrated under reduced pressure. The crude product was purified by silica gel column chromatography (PE/EA= 1/0 to 5/1) to obtain compound 32-4.

### Step 4: synthesis of compound 32-5

Compound 32-4 (400 mg, 1.38 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (10 mL), and the organic phase was washed with saturated NaCl solution (10 mL), dried over anhydrous Na₂SO₄, and concentrated under reduced pressure to obtain compound 32-5 which was used directly in the next step without purification.

### Step 5: synthesis of compound 32-6

4-Bromo-2-(6-azaspiro[2.5]octyl-6-yl)benzoic acid (246 mg, 0.79 mmol) was dissolved in DCM (3 ml), and oxalyl chloride (0.13 ml, 1.58 mmol) and a catalytic amount of DMF were added under nitrogen protection at 0°C. The reaction system was moved to room temperature and reacted for 30 minutes, concentrated under reduced pressure and dissolved in DMF (1 ml). Compound 32-5 (100 mg, 0.53 mmol) was dissolved in pyridine (5 ml) and the acyl chloride of 4-bromo-2-(6-azaspiro[2.5]octyl-6-yl)benzoic acid was added to the reaction mixture at 0°C and reacted at 80°C for 24 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 5/1) to obtain compound 32-6.

### Step 6: synthesis of compound 32

Compound 32-6 (30 mg, 0.062 mmol), 2-hydroxyethylsulfonamide (39 mg, 0.31 mmol), cesium carbonate (61 mg, 0.19 mmol) and a catalyst (CAS: 1599466-89-3; 5 mg, 0.0062 mmol) were dissolved in dioxane (3 mL), and the reaction mixture was reacted at 80°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 2/1) to obtain compound 32. MS (ESI, pos.ion) m/z: 526.3 [M+1]⁺.

**¹HNMR** (DMSO-d6 ) δ: 10.79-10.85 (m, 1H), 7.75-7.81 (m, 1H), 7.67-7.73 (m, 1H), 7.16-7.22 (m, 1H), 7.11-7.16 (m, 1H), 6.98-7.03 (m, 1H), 3.73-3.79 (m, 2H), 3.52-3.61 (m, 2H), 3.32-3.35 (m, 2H), 3.12-3.19 (m, 2H), 3.00-3.05 (m, 2H), 2.97 (br t, *J* = 5.0 Hz, 4H), 1.44-1.53 (m, 4H), 0.93-1.01 (m, 1H), 0.44-0.51 (m, 2H), 0.31-0.38 (m, 4H), 0.18-0.24 (m, 2H).

### Example 33: synthesis of N-(1-(cyclopropylmethyl)-1H-pyrrolo[2,3-b]pyridin-4-yl)-4-((2-hydroxyethyl)sulfanilamido)-2-(6-azaspiro[2.5]octan-6-yl)4-benzamide (33)

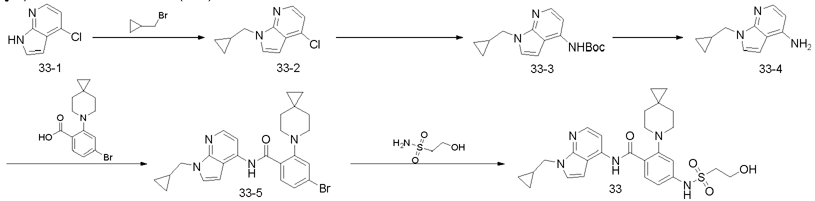

### Step 1: synthesis of compound 33-2

Compound 33-1 (500 mg, 3.27 mmol), and cesium carbonate (3.2 g, 9.8 mmol) were dissolved in DMF (10 ml), and bromomethylcyclopropane (0.35 ml, 3.6 mmol) was added at room temperature. The mixture was reacted at 50°C for 2 hours under N2 protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 33-2.

### Step 2: synthesis of compound 33-3

Compound 33-2 (300 mg, 1.45 mmol), tert-butyl carbamate (341 mg, 2.9 mmol), cesium carbonate (1400 mg, 4.35 mmol) and a catalyst (CAS: 1599466-85-9; 61.8 mg, 0.07 mmol) were dissolved in dioxane (2 mL), and the reaction mixture was reacted at 90°C for 3 hours under nitrogen protection. The reaction solution was diluted with EA (10 mL), then washed with saturated NaCl solution (10 mL), dried over anhydrous Na₂SO₄, and concentrated under reduced pressure. The crude product was purified by silica gel column chromatography (PE/EA= 1/0 to 5/1) to obtain compound 33-3.

### Step 3: synthesis of compound 33-4

Compound 33-3 (280 mg, 0.97 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (10 mL), and the organic phase was washed with saturated NaCl solution (10 mL), dried over anhydrous Na₂SO₄, and concentrated under reduced pressure to obtain compound 33-4 which was used directly in the next step without purification.

### Step 4: synthesis of compound 33-5

4-Bromo-2-(6-azaspiro[2.5]octyl-6-yl)benzoic acid (297 mg, 0.96 mmol) was dissolved in DCM (3 ml), and oxalyl chloride (0.16 ml, 1.92 mmol) and a catalytic amount of DMF were added under nitrogen protection at 0°C. The reaction system was moved to room temperature and reacted for 30 minutes, concentrated under reduced pressure and dissolved in DMF (1 ml). Compound 33-4 (150 mg, 0.8 mmol) was slowly added to NaH (35 mg, 0.8 mmol) at 0°C under nitrogen protection, and the reaction mixture was reacted at 60°C for 30 minutes. The acyl chloride of 4-bromo-2-(6-azaspiro[2.5]oct-6-yl)benzoic acid was added to the reaction mixture at 0°C and reacted at 80°C for 2 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 35-5.

### Step 5: synthesis of compound 33

Compound 33-5 (45 mg, 0.094 mmol), 2-hydroxyethylsulfonamide (58.8 mg, 0.47 mmol), cesium carbonate (92 mg, 0.28 mmol) and a catalyst (CAS: 1599466-89-3; 8 mg, 0.0094 mmol) were dissolved in dioxane (3 mL), and the reaction mixture was reacted at 80°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 33. MS (ESI, pos.ion) m/z: 524.3 [M+1]⁺.

**¹HNMR** (DMSO-d6 ) δ: 11.24-11.30 (m, 1H), 8.19 (d, *J =* 5.5 Hz, 1H), 8.02-8.09 (m, 1H), 7.80-7.86 (m, 1H), 7.58-7.63 (m,1H), 7.15-7.21 (m, 1H), 7.04 (d, *J =* 8.4 Hz, 1H), 6.75-6.80 (m, 1H), 4.12 (d, *J =* 7.1 Hz, 2H), 3.78 (t, *J =* 6.5 Hz, 2H), 3.33-3.38(m, 2H), 2.98-3.06 (m, 4H), 1.42-1.51 (m, 4H), 1.27-1.34 (m, 1H), 0.51 (br d, *J =* 8.0 Hz, 2H), 0.40-0.46 (m, 2H), 0.25-0.31(m, 4H).

### Example 34: synthesis of N-(7-(cyclopropylmethyl)-7H-pyrrolo[2,3-d]pyrimidin-4-yl)-4-((2-hydroxyethyl)sulfonamido)-2-(6-azaspiro[2.5]octan-6-yl)benzamide (34)

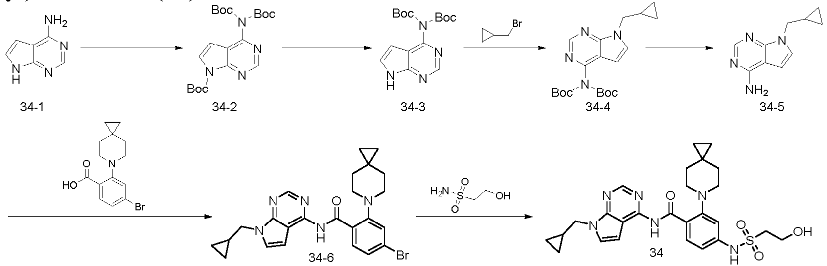

### Step 1: synthesis of compound 34-2

Compound 34-1 (500 mg, 3.73 mmol) and DMAP (683 mg, 5.59 mmol) were dissolved in THF (10 ml). Tert-butoxycarbonyl tert-butyl ester (3.19 ml, 14.91 mmol) was added under ice bath conditions. The mixture was reacted at room temperature for 2 hours under N₂ protection. The reaction solution was quenched by adding water (10 ml), and liquid separation was performed with EA (40 mL). The organic phase was washed with saturated sodium chloride aqueous solution (10 mL), dried over anhydrous Na2SO4, and concentrated under reduced pressure. The crude product was purified by silica gel column chromatography (PE/EA= 1/0 to 10/1) to obtain compound 34-2.

### Step 2: synthesis of compound 34-3

Compound 34-2 (1 g, 2.3 mmol) was dissolved in methanol (10 ml), and an aqueous saturated sodium bicarbonate solution (2.3 ml) was added. The mixture was reacted for 3 hours at room temperature under N₂ protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 34-3.

### Step 3: synthesis of compound 34-4

Compound 34-3 (670 mg, 2 mmol), and cesium carbonate (1.96 g, 6 mmol) were dissolved in DMF (10 ml), and bromomethylcyclopropane (0.22 ml, 2.2 mmol) was added at room temperature. The mixture was reacted at 50°C for 2 hours under N₂ protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 34-4.

### Step 4: synthesis of compound 34-5

Compound 34-4 (670 mg, 1.72 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (10 mL), and the organic phase was washed with saturated NaCl solution (10 mL), dried over anhydrous Na₂SO₄, and concentrated under reduced pressure to obtain compound 34-5 which was used directly in the next step without purification.

### Step 5: synthesis of compound 34-6

4-Bromo-2-(6-azaspiro[2.5]oct-6-yl)benzoic acid (297 mg, 0.96 mmol) was dissolved in DCM (3 ml), and oxalyl chloride (0.16 ml, 1.92 mmol) and a catalytic amount of DMF were added under nitrogen protection at 0°C. The reaction system was moved to room temperature and reacted for 30 minutes, concentrated under reduced pressure and dissolved in DMF (1 ml). Compound 34-5 (150 mg, 0.8 mmol) was slowly added to NaH (35 mg, 0.8 mmol) at 0°C under nitrogen protection, and the reaction mixture was reacted at 60°C for 30 minutes. The acyl chloride of 4-bromo-2-(6-azaspiro[2.5]octyl-6-yl)benzoic acid was added to the reaction mixture at 0°C and reacted at 80°C for 2 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 2/1) to obtain compound 34-6.

### Step 6: synthesis of compound 34

Compound 34-6 (45 mg, 0.094 mmol), 2-hydroxyethylsulfonamide (58.8 mg, 0.47 mmol), cesium carbonate (92 mg, 0.28 mmol) and a catalyst (CAS: 1599466-89-3; 8 mg, 0.0094 mmol) were dissolved in dioxane (3 mL), and the reaction mixture was reacted at 80°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 34. MS (ESI, pos.ion) m/z: 525.3 [M+1]⁺.

**¹HNMR** (ACETONITRILE-*d*₃) δ: 13.00-13.08 (m, 1H), 8.39-8.45 (m, 1H), 8.07-8.12 (m, 1H), 7.27-7.31 (m, 1H), 7.21-7.24(m, 1H), 7.07 (dd, *J* = 8.6, 2.1 Hz, 1H), 7.03-7.05 (m, 1H), 4.02-4.06 (m, 2H), 3.80-3.84 (m, 2H), 3.22-3.26 (m, 2H), 2.97-3.03(m, 4H), 1.59-1.72 (m, 4H), 0.78-0.80 (m, 1H), 0.44-0.50 (m, 2H), 0.34-0.37 (m, 2H), 0.32-0.34 (m, 4H).

### Example 37: synthesis of N-(1-(cyclopropylmethyl)-6-methyl-1H-pyrrolo[2,3-b]pyridin-4-yl)-4-((2-hydroxyethyl)sulfonamido)-2-(6-azaspiro[2.5]oct-6-yl)benzamide

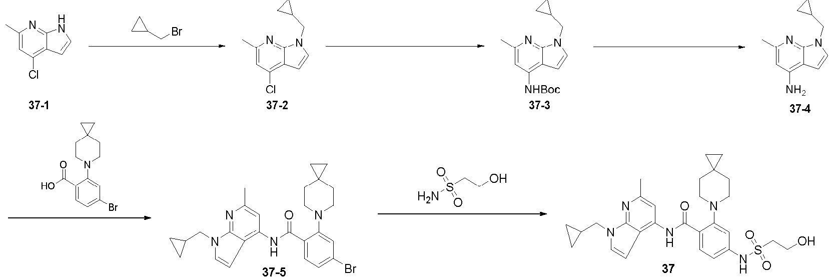

### Step 1: synthesis of compound 37-2

Compound 37-1 (500 mg, 3.00 mmol), and cesium carbonate (1.95 g, 6.00 mmol) were dissolved in DMF (5 mL), to which bromomethylcyclopropane (0.35 mL, 3.60 mmol) was added, and the reaction mixture was reacted at 70°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1-10:1) to obtain compound 37-2. MS (ESI, pos.ion) m/z: 221.0 [M+1]⁺.

### Step 2: synthesis of compound 37-3

Compound 37-2 (270 mg, 1.22 mmol), NH₂Boc (287 mg, 2.45 mmol), cesium carbonate (1.20 g, 3.67 mmol) and palladium catalyst (CAS: 1599466-85-9; 52 mg, 0.06 mmol) were dissolved in 1,4-dioxane (5 mL), and the reaction mixture was reacted at 90°C for 2 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1-10:1) to obtain compound 37-3. MS (ESI, pos.ion) m/z:302.2[M+1]⁺.

### Step 3: synthesis of compound 37-4

Compound 37-3 was dissolved in DCM (1 mL), to which HCl\EA (5 mL) was added, and the reaction mixture was allowed to react at 25°C for 2 h. The reaction solution was quenched with ice water, neutralized with saturated NaHCO₃ solution, and extracted with EA. The organic phase was washed with saturated NaCl solution, dried over anhydrous Na₂SO₄, concentrated under reduced pressure, and dried to obtain compound 37-4. MS (ESI, pos.ion) m/z: 202.1 [M+1]⁺.

### Step 4: synthesis of compound 37-5

4-Bromo-2-(6-azaspiro[2.5]octan-6-yl)benzoic acid (240 mg, 0.78 mmol) was dissolved in DCM (5 mL), to which oxalyl chloride (0.13 mL, 1.55 mmol) and a catalytic amount of DMF were added, and the reaction mixture was allowed to react at 25°C for 10 minutes. The reaction solution was concentrated and dried with an oil pump to obtain 4-bromo-2-(6-azaspiro[2.5]octan-6-yl) benzoyl chloride. Compound 37-4 (104 mg, 0.52 mmol) was dissolved in DMF (1 mL), to which NaH (21 mg, 0.52 mmol) was added, and the reaction mixture was reacted at 60°C for 30 minutes. Then 4-bromo-2-(6-azaspiro[2.5]octan-6-yl)benzoyl chloride was added, and the reaction mixture was allowed to react at 90°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 37-5 (4-bromo-N-(1-(cyclopropylmethyl)-6-methyl-1H-pyrrolo[2,3-b]pyridin-4-yl)-2-(6-azaspiro[2.5]octan-6-yl)benzamide). MS (ESI, pos.ion) m/z:493.2\495.2 [M+1]+.

### Step 5: synthesis of compound 37

Compound 37-5 (30 mg, 0.06 mmol), 2-hydroxyethanesulfonamide (38 mg, 0.30 mmol), cesium carbonate (59 mg, 0.18 mmol) and palladium catalyst (CAS: 1599466-89-3; 3 mg) were dissolved in 1,4-dioxane (2 mL), and the reaction mixture was reacted at 100°C for 2 h under nitrogen protection. The reaction solution was quenched with ice water, and extracted with EA. The organic phase was washed with saturated NaCl solution, dried over anhydrous Na2SO4, and concentrated under reduced pressure, and the crude product was purified to obtain compound 37. MS (ESI, pos.ion) m/z:538.3 [M+1]+.

**¹H NMR** (400 MHz, DMSO-d6) δ = 11.21 (s, 1H), 7.97 (s, 1H), 7.82 (d, *J*= 8.5 Hz, 1H), 7.48 (d, *J* = 3.5 Hz, 1H), 7.19 - 7.14 (m, 1H), 7.02 (dd, *J* = 1.7, 8.4 Hz, 1H), 6.69 (d, *J =* 3.5 Hz, 1H), 4.07 (d, *J =* 7.0 Hz, 2H), 3.77 (t, *J =* 6.6 Hz, 2H), 3.36 - 3.32 (m, 2H), 3.05 - 2.96 (m, 4H), 2.53 (s, 3H), 1.47 (br s, 4H), 1.31 - 1.26 (m, 1H), 0.54 - 0.39 (m, 4H), 0.28 (s, 4H).

### Example 38: synthesis of N-(1-(cyclopropylmethyl)-1H-indol-4-yl)-6-((2-hydroxyethyl)sulfonamido)-2-(6-azaspiro[2.5]oct-6-yl)nicotinamide

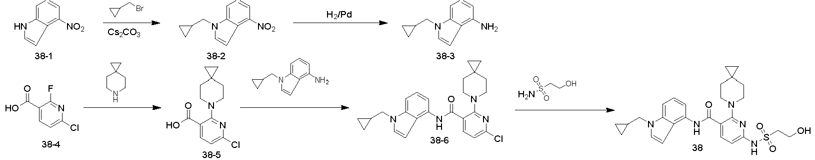

### Step 1: synthesis of compound 38-2

Compound 38-1 (800 mg, 4.93 mmol) and Cs₂CO₃ (3.2 g, 9.87 mmol) were dissolved in DMF (15 mL), and (bromomethyl)cyclobutane (733 mg, 5.43 mmol) was slowly added dropwise. The reaction mixture was stirred at 90°C for 3 hours. The reaction solution was diluted with ethyl acetate (30 mL), then washed with saturated NaCl solution (30 mL), dried over anhydrous Na₂SO₄, and concentrated under reduced pressure. The crude product was purified by silica gel column chromatography (PE:EA=1/20-1/10) to obtain compound 38-2.

### Step 2: synthesis of compound 38-3

Compound 38-2 (800 mg, 3.7 mmol) and Pd/C 10% (80 mg, 0.75 mmol) in MeOH (10 mL) was stirred at room temperature under H₂ for 18 hours. After the reaction mixture was subjected to suction filtration, the filtrate was concentrated under reduced pressure to obtain compound 38-3. MS m/z (ESI):187.1 [M+1]⁺.

### Step 3: synthesis of compound 38-5

Compound 38-4 (1 g, 5.21 mmol) and 6-azaspiro[2.5]octane (579 mg, 5.21 mmol) were dissolved in acetonitrile (10 mL), DIEA (2019.5 mg, 15.6 mmol) was slowly added dropwise, and the reaction mixture was stirred at 60°C for 3 hours. The reaction solution was diluted with ethyl acetate (20 mL), then washed with saturated NaCl solution (20 mL), dried over anhydrous Na₂SO₄, and concentrated under reduced pressure. The crude product was purified by silica gel column chromatography (PE:EA=1/20-1/10) to obtain compound 38-5. MS m/z (ESI):267.0 [M+1]⁺.

### Step 4: synthesis of compound 38-6

Compound 38-5 (90 mg, 0.34 mmol), 4-bromo-2-(6-azaspiro[2.5]octan-6-yl)benzoic acid (63, 0.34 mmol), and HATU (128 mg, 0.34 mmol) were dissolved in DMF (2 mL), to which DIEA (87 mg, 0.67 mmol) was added, and the reaction mixture was allowed to react at 25°C for 2 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 38-6. MS m/z (ESI):435.2 [M+1]⁺.

### Step 5: synthesis of compound 38

Compound 38-6 (100 mg, 0.23 mmol), 2-hydroxyethanesulfonamide (143.85 mg, 1.15 mmol), cesium carbonate (224.7 mg, 0.69 mmol) and palladium catalyst (1599466-85-9, 10 mg, 0.01 mmol) were dissolved in 1,4-dioxane (5 mL), and the reaction mixture was reacted at 80°C for 10 h under N₂ protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 38. MS m/z (ESI):524.3 [M+1]⁺.

¹H NMR (400 MHz, DMSO-d6) δ = 10.67 (s, 1H), 10.26 (s, 1H), 7.89 (d, *J =* 8.0 Hz, 1H), 7.83 (br d, *J =* 7.6 Hz, 1H), 7.43 (d, *J =* 3.1 Hz, 1H), 7.28 (d, *J =* 8.3 Hz, 1H), 7.14 - 7.08 (m, 1H), 6.71 (d, *J* = 3.1 Hz, 1H), 6.47 (d, *J =* 8.1 Hz, 1H), 4.04 (d, *J =* 7.0 Hz, 2H), 3.82 - 3.79 (m, 2H), 3.78 - 3.75 (m, 2H), 3.40 - 3.32 (m, 4H), 1.47 - 1.37 (m, 4H), 1.28 - 1.22 (m, 1H), 0.55 - 0.47 (m, 2H), 0.44 - 0.36 (m, 2H), 0.26 (s, 4H).

### Example 39: synthesis of N-(1-cyclopropylmethyl)-1H-indol-4-yl)-4-(2-hydroxyethyl)sulfonamido-2-methylamino-6-(6-azaspiro[2.5]octan-6-yl)benzamide (39)

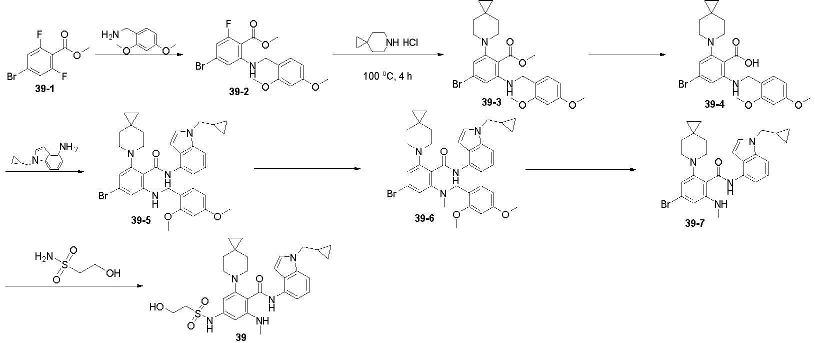

### Step 1: synthesis of compound 39-2

Compound 39-1 (10 g, 39.84 mmol) and (2,4-dimethoxyphenyl)methanamine (6.00 mL, 39.84 mmol) were dissolved in DMF (60 mL) solution, to which Cs₂CO₃ (25.96 g, 79.67 mmol) was added, and the reaction mixture was stirred in an oil bath at 60°C for 4 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-98:2-95:5) to obtain compound 39-2.

### Step 2: synthesis of compound 39-3

6-Azaspiro[2.5]octane hydrochloride (1.85 g, 12.56 mmol) was dissolved in DMF (15 mL) and water (15 mL), and N, N-diisopropylethylamine (3.25 g, 25.11 mmol) was added and stirred for a while. Compound 39-2 (2.5 g, 6.28 mmol) was then added and the mixture was stirred at 100°C for 4 hours. After the reaction was completed, the reaction mixture was diluted with EA (120 mL), and the organic layer was washed with brine (180 mL), dried over Na₂SO₄, filtered and concentrated. The crude product was purified by column chromatography (PE: EA = 100: 0-49:1) to obtain compound 39-3.

### Step 3: synthesis of compound 39-4

To a solution of compound 39-3 (1.38 g, 2.82 mmol) in methanol (10 mL) and water (2.5 mL) was added sodium hydroxide (0.68, 16.92 mmol) and the mixture was stirred at 60°C for 18 hours. The reaction mixture was diluted with EA (120 mL). The organic layer was washed with brine (180 mL), dried over Na₂SO₄, filtered and concentrated. The crude product was purified by column chromatography (PE:EA=100:0-90:10-80:20) to obtain compound 39-4.

### Step 4: synthesis of compound 39-5

Compound 39-4 (1.2 g, 2.52 mmol) and 1-cyclopropylmethyl-1H-indol-4-amine (517.19 mg, 2.78 mmol) were dissolved in DMF (10 mL), azabenzotriazolyltetramethyluronium hexafluorophosphate (1.15 g, 3.03 mmol) and N, N-diisopropylethylamine (0.84 mL, 5.05 mmol) were added, and the reaction was stirred at room temperature for 1 hour. The reaction mixture was diluted with EA (120 mL), and the organic layer was washed with brine (180 milliliter), dried over Na₂SO₄, filtered and concentrated. The residue was purified by column chromatography (PE:EA=100:0-90:10-80:20) to obtain compound 39-5.

### Step 5: synthesis of compound 39-6

Compound 39-5 (600 mg, 0.93 mmol) was dissolved in methanol (6 mL) and acetic acid (0.5 mL), to which formaldehyde (83.98 mg, 2.80 mmol) and cyano sodium borohydride (175.74 mg, 2.80 mmol) were added, and the reaction was stirred at room temperature for 18 hours. The reaction mixture was diluted with EA (120 mL), and the organic layer was washed with brine (180 mL), dried over Na₂SO₄, filtered and concentrated. The residue was purified by column chromatography (PE:EA=100:0-90:10-80:20) to obtain compound 39-6.

### Step 6: synthesis of compound 39-7

Compound 39-6 (497 mg, 0.76 mmol) was dissolved in dichloromethane (3 mL), to which trifluoroacetic acid (3 mL, 40.26 mmol) was added, and the reaction solution was reacted with stirring at room temperature for 10 minutes. The reaction mixture was diluted with EA (100 mL), and the organic layer was washed with brine (180 mL), dried over Na₂SO₄, filtered and concentrated. The residue was purified by column chromatography (PE:EA=100:0-90:10-80:20) to obtain compound 39-7.

### Step 7: synthesis of compound 39

Compound 39-7 (167 mg, 0.33 mmol) and 2-hydroxyethane-1-sulfonamide (164.72 mg, 1.32 mmol) were dissolved in 1,4-dioxane (2 mL), to which a catalyst (CAS: 1599466-83-7; 30.47 mg, 0.03 mmol) and cesium carbonate (214.44 mg, 0.66 mmol) were added, and the reaction solution was stirred in an oil bath at 95°C for 1.5 hours. The reaction mixture was diluted with EA (100 mL), and the organic layer was washed with brine (180 mL), dried over Na₂SO₄, filtered and concentrated. The residue was purified to obtain compound 39. MS (ESI, pos.ion) m/z: 552.3 [M+1]⁺.

**¹H NMR** (400 MHz, METHANOL-d4) δ = 7.75-7.75 (m, 1H), 7.76-7.71 (m, 1H), 7.33-7.29 (m, 1H), 7.28-7.24 (m, 1H), 7.19-7.13 (m, 1H), 6.55-6.52 (m, 1H), 6.51-6.48 (m, 1H), 6.41-6.38 (m, 1H), 4.08-4.03 (m, 2H), 3.98-3.94 (m, 2H), 3.41-3.37 (m, 2H), 3.09 (br d, *J =* 4.4 Hz, 4H), 2.88-2.83 (m, 3H), 1.52-1.41 (m, 4H), 1.29 (br s, 1H), 0.63-0.57 (m, 2H), 0.43-0.38 (m, 2H), 0.27-0.23 (m, 4H).

### Example 40: synthesis of N-(1-cyclopropylmethyl)-1H-pyrrolo[2,3-b]pyridin-4-yl)-4-(2-hydroxyethylsulfonamide)-2-methylamino-6-(6-azaspiro[2.5]octan-6-yl)benzamide (40)

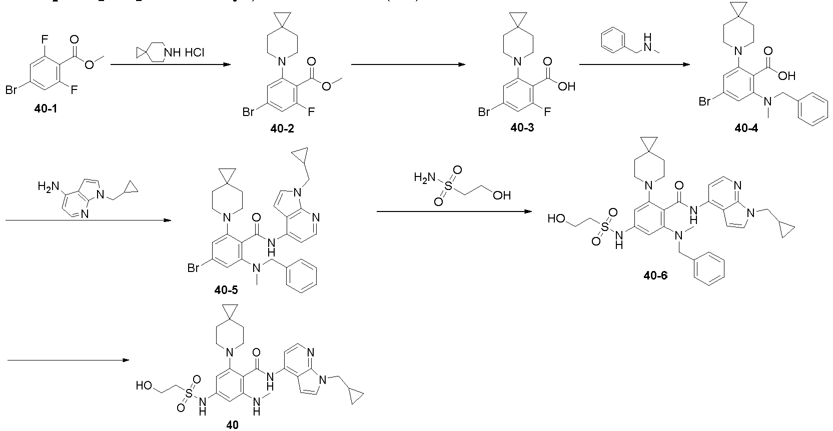

### Step 1: synthesis of compound 40-2

Compound 40-1 (5.0 g, 19.92 mmol) and 6-azaspiro[2.5]octane hydrochloride (3.53 g, 23.90 mmol) were dissolved in a mixed solvent of DMF (25 mL) and H2O (25 mL), to which Cs₂CO₃ (7.72 g, 59.75 mmol) was added and the reaction mixture was stirred in an oil bath at 100°C for 3 hours. After the reaction was completed, the reaction solution was diluted with water (50 mL), and extracted with ethyl acetate (30×3 mL). The organic layer was washed with brine (30×3 mL), dried over Na₂SO₄, filtered and concentrated. The residue was purified by column chromatography (PE:EA=98:2-95:5) to obtain compound 40-2.

### Step 2: synthesis of compound 40-3

Compound 40-2 (5.8 g, 16.95 mmol) was dissolved in a mixed solvent of MeOH (32 mL) and H₂O (8 mL), to which NaOH (4.07 g, 101.69 mmol) was added and the reaction mixture was stirred in an oil bath at 60°C for 3 hours. After the reaction was completed, 2M HCl was added to adjust the reaction solution to an acidic system, and the reaction solution was diluted with water (50 mL), and extracted with ethyl acetate (30×3 mL). The organic layer was dried over Na₂SO₄, filtered and concentrated to obtain compound 40-3.

### Step 3: synthesis of compound 40-4

Compound 40-3 (2.0 g, 6.09 mmol) was dissolved in N-methylbenzylamine (10 mL) and the reaction mixture was stirred in a microwave reactor at 150°C for 30 minutes. After the reaction was completed, the reaction solution was diluted with water (60 mL), and extracted with ethyl acetate (60×3 mL). The organic layer was washed with brine (30×3 mL), dried over Na₂SO₄, filtered and concentrated. The residue was purified by column chromatography (DCM:MeOH=99:1-97:3) to obtain compound 40-4.

### Step 4: synthesis of compound 40-5

Compound 40-4 (300 mg, 0.70 mmol) was dissolved in DCM (6 mL), the reaction solution was cooled to 0°C, to which oxalyl chloride (56.45 mg, 2.10 mmol) was slowly added, and the reaction mixture was stirred at room temperature for half an hour. After the reaction was completed, the reaction solution was directly subjected to rotary evaporation to dryness for the next step. 1-Cyclopropylmethyl-1H-pyrrolo[2,3-b]pyridine-4-amine (200.63 mg, 1.40 mmol) was dissolved in DMF (4 mL), the reaction solution was cooled to 0°C and NaH (33.54 mg, 1.40 mmol) was added. The reaction solution was stirred in an oil bath at 60°C for 30 minutes. The reaction solution was then cooled to 0°C, followed by addition of the acyl chloride obtained above. The mixed reaction solution was allowed to react at room temperature for 3 hours. After the reaction was completed, the reaction solution was diluted with water (30 mL), and extracted with ethyl acetate (30×3 mL). The organic layer was washed with brine (30×3 mL), dried over Na₂SO₄, filtered and concentrated. The residue was purified by column chromatography (PE:EA=100:0-97:3) to obtain compound 40-5.

### Step 5: synthesis of compound 40-6

Compound 40-5 (128 mg, 0.21 mmol) and 2-hydroxyethane-1-sulfonamide (107 mg, 0.86 mmol) were dissolved in dioxane (2 mL), to which a catalyst (CAS: 1599466-89-3; 35 mg, 0.04 mmol) and Cs₂CO₃ (139 mg, 0.43 mmol) were added, and the reaction mixture was stirred in an oil bath at 95°C for 2 hours. After the reaction was completed, the reaction solution was diluted with water (30 mL), and extracted with ethyl acetate (30×3 mL). The organic layer was washed with brine (30×3 mL), dried over Na2SO4, filtered and concentrated. The crude product was purified by column chromatography (DCM:MeOH=95:5) to obtain compound 40-6.

### Step 6: synthesis of compound 40

Compound 40-6 (56 mg, 0.09 mmol) was dissolved in methanol (1 mL), to which 10% palladium on carbon (0.93 mg, 0.01 mmol) was added. The reaction mixture was stirred for 16 hours at room temperature under hydrogen atmosphere. After the reaction was completed, the reaction solution was diluted with water (10 mL), and extracted with ethyl acetate (10×3 mL). The organic layer was washed with brine (10×3 mL), dried over Na₂SO₄, filtered and concentrated. The crude product was purified by preparative chromatography to obtain compound 40. MS (ESI, pos.ion) m/z: 553.3 [M+1]⁺.

**¹H NMR** (400 MHz, METHANOL-d4) *δ* = 8.18-8.13 (m, 1H), 8.10-8.04 (m, 1H), 7.48-7.45 (m, 1H), 6.70-6.67 (m, 1H), 6.50-6.45 (m, 1H), 6.42-6.39 (m, 1H), 4.15 (d, ***J*** = 7.1 Hz, 2H), 3.98-3.94 (m, 2H), 3.43-3.38 (m, 2H), 3.13-3.06 (m, 4H), 2.86 (s, 3H), 1.54-1.44 (m, 4H), 0.90 (br t, ***J*** = 6.8 Hz, 1H), 0.59 (br d, ***J*** = 7.6 Hz, 2H), 0.46-0.42 (m, 2H), 0.28-0.24 (m, 4H).

### Example 41: synthesis of 2-amino-N-(1-cyclopropylmethyl)-1H-indol-4-yl)-4-(2-hydroxyethyl)sulfonamido-6-(6-azaspiro[2.5]octan-6-yl)benzamide (41)

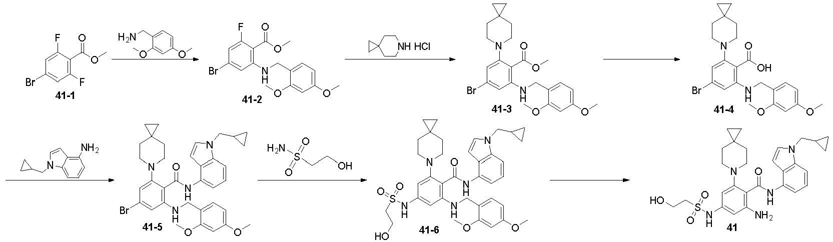

### Step 1: synthesis of compound 41-2

Methyl 4-bromo-2,6-difluorobenzoate (4.98 g, 19.84 mmol) and (2,4-dimethoxyphenyl)methanamine (3.35 g, 20.04 mmol) were dissolved in N,N-dimethylformamide (40 mL), to which cesium carbonate (12.93 g, 39.68 mmol) was added and the reaction mixture was stirred in an oil bath at 60°C for 3 hours. After the reaction was completed, the reaction solution was diluted with water (120 mL), and extracted with ethyl acetate (60 × 3 mL). The organic layer was washed with brine (60×3 mL), dried over Na₂SO₄, filtered and concentrated. The residue was purified by column chromatography (PE:EA=98:2-95:5) to obtain compound 41-2.

### Step 2: synthesis of compound 41-3

6-Azaspiro[2.5]octane hydrochloride (1.24 g, 8.37 mmol) and sodium hydride (0.50 g, 20.94 mmol) were dissolved in N,N-dimethylformamide (25 mL), stirred in an oil bath at 70°C for 10 minutes. To this mixture was then added compound 41-2 (2.78 g, 6.98 mmol) and the reaction mixture was stirred in an oil bath at 125°C for 18 hours. After the reaction was completed, the reaction solution was diluted with water (100 mL), and extracted with ethyl acetate (30×3 mL). The organic layer was dried over Na₂SO₄, filtered and concentrated to obtain compound 41-3.

### Step 3: synthesis of compound 41-4

Compound 41-3 (498 mg, 1.02 mmol) was dissolved in methanol (4 mL) and water (0.4 mL), LiOH (74 mg, 3.06 mmol) was then added, and the reaction mixture was stirred in an oil bath at 60°C for 7 hours. After the reaction was completed, the reaction solution was diluted with water (60 mL), and extracted with ethyl acetate (60×3 mL). The organic layer was washed with brine (30×3 mL), dried over Na₂SO₄, filtered and concentrated. The residue was purified by column chromatography (PE:EA=100:0-90:10-80:20) to obtain compound 41-4.

### Step 4: synthesis of compound 41-5

Compound 41-4 (170 mg, 0.36 mmol) and 1-cyclopropylmethyl-1H-indol-4-amine (66.61 mg, 0.36 mmol) were dissolved in N,N-dimethylformamide (3 mL), to which azabenzotriazolyltetramethyluronium hexafluorophosphate (135.98 mg, 0.36 mmol) and N, N-diisopropylethylamine (46.22mg, 0.36 mmol) were added, and the reaction mixture was stirred at room temperature for 3 hours. After the reaction was completed, the reaction solution was diluted with water (90 mL), and extracted with ethyl acetate (30 × 3 mL). The organic layer was washed with brine (30×3 mL), dried over Na₂SO₄, filtered and concentrated. The residue was purified by column chromatography (PE:EA=100:0-90:10-80:20) to obtain compound 41-5.

### Step 5: synthesis of compound 41-6

Compound 41-5 (118 mg, 0.18 mmol) and 2-hydroxyethane-1-sulfonamide (91.77 mg, 0.73 mmol) were dissolved in dioxane (3 mL), to which a catalyst (CAS: 1599466-89-3; 7.41 mg, 0.01 mmol) and Cs₂CO₃ (119.47 mg, 0.37 mmol) were added, and the reaction mixture was stirred in an oil bath at 95°C for 3 hours. After the reaction was completed, the reaction solution was diluted with water (90 mL), and extracted with ethyl acetate (30 × 3 mL). The organic layer was washed with brine (30×3 mL), dried over Na₂SO₄, filtered and concentrated. The residue was purified by column chromatography (PE:EA=100:0-90:10-80:20) to obtain compound 41-6.

### Step 6: synthesis of compound 41

Compound 41-6 (40 mg, 0.06 mmol) was dissolved in dichloromethane (1 mL), to which trifluoroacetic acid (1 mL, 13.42 mmol) was added, and the reaction mixture was stirred at room temperature for 6 minutes. After the reaction was completed, the reaction solution was diluted with water (10 mL), and extracted with ethyl acetate (10×3 mL). The organic layer was washed with brine (10×3 mL), dried over Na₂SO₄, filtered and concentrated. The residue was purified by preparative HPLC to obtain compound 41. MS (ESI, pos.ion) m/z: 538.3 [M+1]+.

**1H NMR** (400 MHz, DMSO-d6) δ = 11.12 - 11.05 (m, 1H), 7.92 - 7.86 (m, 1H), 7.43 (br s, 1H), 7.46 - 7.40 (m, 1H), 7.29 - 7.23 (m, 1H), 7.13 - 7.07 (m, 1H), 6.66 - 6.61 (m, 1H), 6.56 - 6.48 (m, 2H), 6.40 - 6.36 (m, 1H), 6.34 - 6.30 (m, 1H), 4.08 - 4.01 (m, 2H), 3.80 - 3.73 (m, 2H), 3.30 - 3.27 (m, 2H), 2.96 - 2.96 (m, 1H), 3.00 - 2.91 (m, 3H), 1.42 - 1.31 (m, 4H), 1.24 - 1.22 (m, 1H), 0.56 - 0.49 (m, 2H), 0.42 - 0.37 (m, 2H), 0.25 - 0.18 (m, 4H).

### Example 42: synthesis of 2-amino-N-(1-cyclopropylmethyl)indolin-4-yl)-4-(2-hydroxyethylsulfonamido)-6-(6-azaspiro[2.5]octan-6-yl)benzamide (42)

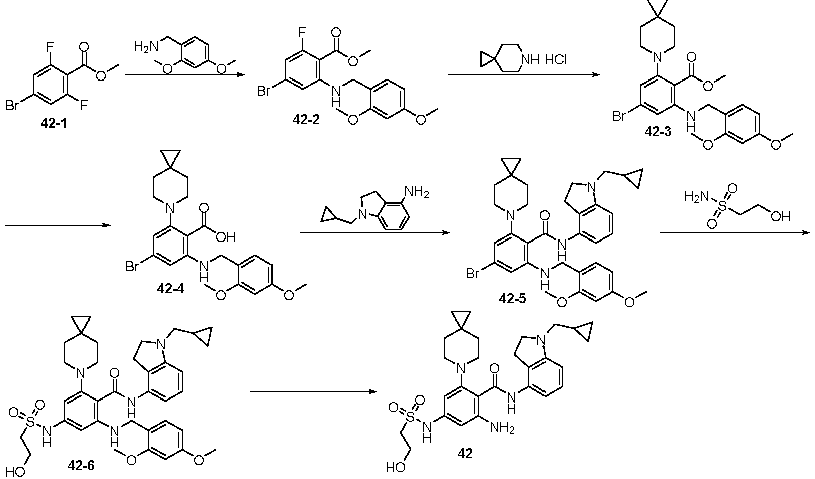

### Step 1: synthesis of compound 42-2

Methyl 4-bromo-2,6-difluorobenzoate (4.98 g, 19.84 mmol) and (2,4-dimethoxyphenyl)methanamine (3.35 g, 20.04 mmol) were dissolved in N,N-dimethylformamide (40 mL), to which cesium carbonate (12.93 g, 39.68 mmol) was added and the reaction mixture was stirred in an oil bath at 60°C for 3 hours. After the reaction was completed, the reaction solution was diluted with water (120 mL), and extracted with ethyl acetate (60 × 3 mL). The organic layer was washed with brine (60×3 mL), dried over Na₂SO₄, filtered and concentrated. The residue was purified by column chromatography (PE:EA=98:2-95:5) to obtain compound 42-2.

### Step 2: synthesis of compound 42-3

6-Azaspiro[2.5]octane hydrochloride (1.24 g, 8.37 mmol) and sodium hydride (0.50 g, 20.94 mmol) were dissolved in N,N-dimethylformamide (25 mL), stirred in an oil bath at 70°C for 10 minutes. To this mixture was then added compound 42-2 (2.78 g, 6.98 mmol) and the reaction mixture was stirred in an oil bath at 125°C for 18 hours. After the reaction was completed, the reaction solution was diluted with water (100 mL), and extracted with ethyl acetate (30×3 mL). The organic layer was dried over Na₂SO₄, filtered and concentrated to obtain compound 42-3.

### Step 3: synthesis of compound 42-4

Compound 42-3 (498 mg, 1.02 mmol) was dissolved in methanol (4 mL) and water (0.4 mL), and the reaction mixture was stirred in an oil bath at 60°C for 7 hours. After the reaction was completed, the reaction solution was diluted with water (60 mL), and extracted with ethyl acetate (60×3 mL). The organic layer was washed with brine (30×3 mL), dried over Na₂SO₄, filtered and concentrated. The residue was purified by column chromatography (PE:EA=100:0-90:10-80:20) to obtain compound 42-4.

### Step 4: synthesis of compound 42-5

Compound 42-4 (160 mg, 0.34 mmol) and 1-cyclopropylmethyl-1H-indol-4-amine (63.37 mg, 0.34 mmol) were dissolved in N,N-dimethylformamide (3 mL), to which azabenzotriazolyltetramethyluronium hexafluorophosphate (153.57 mg, 0.40 mmol) and N, N-diisopropylethylamine (87.00 mg, 0.67 mmol) were added, and the reaction mixture was stirred at room temperature for 3 hours. After the reaction was completed, the reaction solution was diluted with water (90 mL), and extracted with ethyl acetate (30 × 3 mL). The organic layer was washed with brine (30×3 mL), dried over Na₂SO₄, filtered and concentrated. The residue was purified by column chromatography (PE:EA=100:0-90:10-80:20) to obtain compound 42-5.

### Step 5: synthesis of compound 42-6

Compound 42-5 (97 mg, 0.15 mmol) and 2-hydroxyethane-1-sulfonamide (75.20 mg, 0.60 mmol) were dissolved in 1,4-dioxane (4 mL), to which a catalyst (CAS: 1599466-89-3; 6.07 mg, 0.01 mmol) and Cs₂CO₃ (97.90 mg, 0.30 mmol) were added, and the reaction mixture was stirred in an oil bath at 95°C for 3 hours. After the reaction was completed, the reaction solution was diluted with water (90 mL), and extracted with ethyl acetate (30 × 3 mL). The organic layer was washed with brine (30×3 mL), dried over Na₂SO₄, filtered and concentrated. The residue was purified by column chromatography (PE:EA=100:0-90:10-80:20) to obtain compound 42-6.

### Step 6: synthesis of compound 42

Compound 42-6 (70 mg, 0.10 mmol) was dissolved in dichloromethane (3 mL), to which trifluoroacetic acid (0.6 mL, 8.05 mmol) was added, and the reaction mixture was stirred at room temperature for 5 minutes. After the reaction was completed, the reaction solution was diluted with water (10 mL), and extracted with ethyl acetate (10×3 mL). The organic layer was washed with brine (10×3 mL), dried over Na₂SO₄, filtered and concentrated. The residue was purified to obtain compound 42. MS (ESI, pos.ion) m/z: 540.3 [M+1]⁺.

**¹H \NMR** (400 MHz, DMSO-d6) δ = 10.64 - 10.58 (m, 1H), 7.14 - 7.09 (m, 1H), 7.00 - 6.94 (m, 1H), 6.51 - 6.43 (m, 2H), 6.38 - 6.35 (m, 1H), 6.34 - 6.28 (m, 2H), 3.78 - 3.72 (m, 2H), 3.44 - 3.39 (m, 2H), 3.29 (br s, 2H), 2.96 - 2.88 (m, 8H), 1.45 - 1.35 (m, 4H), 0.88 - 0.84 (m, 1H), 0.53 - 0.48 (m, 2H), 0.34 - 0.28 (m, 4H), 0.24 - 0.19 (m, 2H).

### Example 44: synthesis of N-(1-(cyclopropylmethyl)-1H-indol-4-yl)-4-(N-(3-methyloxetan-3-yl)sulfamoyl)-2-(6-azaspiro[2.5]oct-6-yl)benzamide (44)

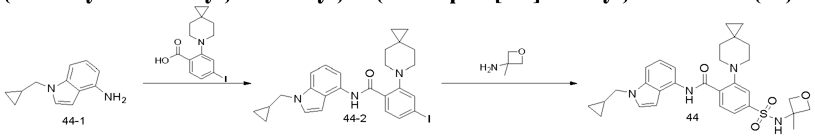

### Step 1: synthesis of compound 44-2

Compound 44-1 (150 mg, 0.81 mmol), 4-iodo-2-(6-azaspiro[2.5]oct-6-yl)benzoic acid (345 mg, 0.97 mmol), HATU (336 mg, 0.89 mmol) and N,N-diisopropylethylamine (0.53 ml, 3.22 mmol) were dissolved in DMF (5 ml), and the reaction mixture was reacted at room temperature for 2 hours under nitrogen protection at 0°C. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 20/1) to obtain compound 44-2.

### Step 2: synthesis of compound 44

Compound 44-2 (100 mg, 0.19 mmol), DABSO (137 mg, 0.57 mmol), palladium acetate (7 mg, 0.028 mmol), n-butyl di(1-adamantyl)phosphine (14 mg, 0.038 mmol) and triethylamine (0.05 ml, 0.38 mmol) were dissolved in isopropanol (5 ml), and the reaction mixture was reacted at 85°C for 1 hour under N₂. After the reaction was completed, the reaction system was cooled to room temperature, and 3-methyloxetan-3-amine (0.1 ml, 1.14 mmol) and aqueous sodium hypochlorite (0.3 ml, 3.8 mmol) were added, and the reaction mixture was allowed to react overnight at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 2/1) to obtain compound 44. MS (ESI, pos.ion) m/z: 549.3 [M+1]⁺.

¹HNMR (DMSO-d6) δ: 10.81-10.84 (m, 1H), 7.91-7.95 (m, 1H), 7.87-7.91 (m, 1H), 7.64-7.67 (m, 1H), 7.56-7.60 (m, 1H), 7.44-7.47 (m, 1H), 7.31-7.35 (m, 1H), 7.11-7.17 (m, 1H), 6.74-6.76 (m, 1H), 4.57-4.61 (m, 2H), 4.15-4.18 (m, 2H), 4.03-4.07 (m, 2H), 3.07-3.13 (m, 4H), 1.42-1.48 (m, 7H), 1.22-1.24 (m, 1H), 0.50-0.56 (m, 2H), 0.37-0.42 (m, 2H), 0.25-0.30 (m, 4H).

### Example 50: synthesis of N-(1-cyclopropylmethyl)-6-fluoro-1H-indol-4-yl)-4-(2-hydroxyethylsulfonamido)-2-(6-azaspiro[2.5]octan-6-yl)benzamide (50)

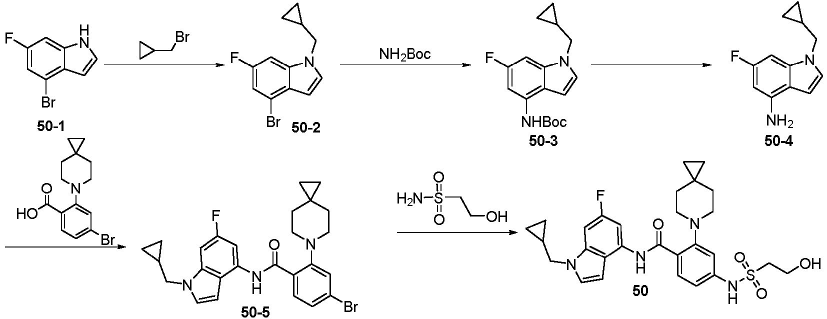

### Step 1: synthesis of compound 50-2

Compound 50-1 (500 mg, 2.34 mmol) and (bromomethyl)cyclopropane (379 mg, 2.81 mmol) were dissolved in N,N-dimethylformamide (5 mL), to which cesium carbonate (1.52 g, 4.67 mmol) was added. The reaction mixture was stirred in an oil bath at 70°C for 1.5 hours. After the reaction was completed, the reaction solution was diluted with water (100 mL), and extracted with ethyl acetate (30×3 mL). The organic layer was washed with brine (30 × 3 mL), dried over Na₂SO₄, filtered and concentrated. The residue was purified by column chromatography (PE:EA=100:0-90: 10) to obtain compound 50-2.

### Step 2: synthesis of compound 50-3

Compound 50-2 (260 mg, 0.97 mmol) and NH₂Boc (227 mg, 1.94 mmol) were dissolved in 1,4-dioxane (5 mL), to which a catalyst (CAS: 1599466-83-7; 179 mg, 0.19 mmol) and cesium carbonate (948 mg, 2.91 mmol) were added. The reaction mixture was stirred in an oil bath at 95°C for 1.5 hours. After the reaction was completed, the reaction solution was filtered and the filtrate was diluted with water (60 mL), and extracted with ethyl acetate (30×3 mL). The organic layer was washed with brine (30 × 3 mL), dried over Na₂SO₄, filtered and concentrated. The residue was purified by column chromatography (PE:EA=100:0-90:10-80:20) to obtain compound 50-3.

### Step 3: synthesis of compound 50-4

Compound 50-3 (195 mg, 0.64 mmol) was dissolved in dichloromethane (3 mL), to which trifluoroacetic acid (0.14 mL, 1.92 mmol) was added. The reaction mixture was stirred at room temperature for 2 hours. After the reaction was completed, the reaction solution was quenched with sodium bicarbonate, and then diluted with water (30 mL), and extracted with ethyl acetate (30 × 3 mL). The organic layer was washed with brine (30 × 3 mL), dried over Na₂SO₄, filtered and concentrated. The residue was purified by column chromatography (PE:EA=100:0-90:10-80:20) to obtain compound 50-4.

### Step 4: synthesis of compound 50-5

Compound 50-4 (60 mg, 0.29 mmol) and 4-bromo-2-(6-azaspiro[2.5]octan-6-yl)benzoic acid (100 mg, 0.32 mmol) were dissolved in N,N-dimethylformamide (2 mL), to which azabenzotriazolyltetramethyluronium hexafluorophosphate (134 mg, 0.35 mmol) and N,N-diisopropylethylamine (76 mg, 0.59 mmol) were added. The reaction mixture was stirred at room temperature for 1.5 hours. After the reaction was completed, the reaction solution was diluted with water (60 mL), and extracted with ethyl acetate (30 × 3 mL). The organic layer was washed with brine (30 × 3 mL), dried over Na₂SO₄, filtered and concentrated. The crude product was purified by column chromatography (PE:EA=100:0-95:5-90:10) to obtain compound 50-5.

### Step 5: synthesis of compound 50

Compound 50-5 (71 mg, 0.14 mmol) and 2-hydroxyethane-1-sulfonamide (72 mg, 0.57 mmol) were dissolved in 1,4-dioxane (3 mL), to which a catalyst (CAS: 1599466-83-7; 13 mg, 0.01 mmol) and cesium carbonate (93 mg, 0.29 mmol) were added. The reaction mixture was stirred in an oil bath at 95°C for 2 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 50. MS (ESI, pos.ion) m/z: 541.2 [M+1]⁺

**¹H NMR** (400 MHz, DMSO-d6) δ = 11.24-11.15 (m, 1H), 7.98-7.89 (m, 1H), 7.88-7.81 (m, 1H), 7.52-7.44 (m, 1H), 7.23-7.15 (m, 2H), 7.07-7.00 (m, 1H), 6.77-6.70 (m, 1H), 4.07-3.98 (m, 2H), 3.82-3.74 (m, 2H), 3.40-3.33 (m, 2H), 3.10-2.97 (m, 4H), 1.54-1.39 (m, 4H), 1.27-1.21 (m, 1H), 0.56-0.49 (m, 2H), 0.44-0.38 (m, 2H), 0.32-0.23 (m, 4H).

### Example 56: synthesis of N-(1-(cyclopropylmethyl)-7-fluoro-1H-indol-4-yl)-4-((2-hydroxyethyl)sulfonamido)-2-(6-azaspiro[2.5]octan-6-yl)benzamide (56)

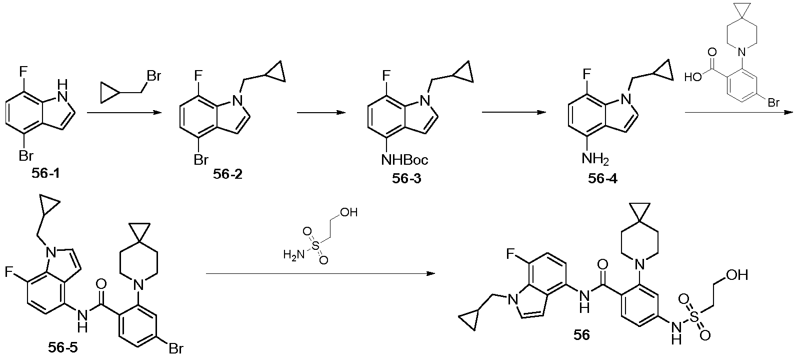

### Step 1: synthesis of compound 56-2

Compound 56-1 (300 mg, 1.40 mmol), and cesium carbonate (913 mg, 2.80 mmol) were dissolved in DMF (4 mL), to which (bromomethyl)cyclopropane (0.16 mL, 1.68 mmol) was added, and the reaction mixture was reacted at 70°C for 30 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 56-2. MS (ESI, pos.ion) m/z: 267.9/269.9 [M+1]⁺.

### Step 2: synthesis of compound 56-3

Compound 56-2 (350 mg, 1.31 mmol), NH₂Boc (306 mg, 2.62 mmol) and cesium carbonate (1.28 g, 3.93 mmol) were dissolved in dioxane (5 mL), to which a catalyst (CAS: 1599466-83-7; 241 mg, 0.26 mmol) was added, and the reaction mixture was reacted at 90°C for 3 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 56-3.

### Step 3: synthesis of compound 56-4

Compound 56-3 (369 mg, 1.21 mmol) was dissolved in dichloromethane (2 mL), to which hydrogen chloride-dioxane solution (3 mL) was added, and the reaction mixture was allowed to react at 25°C for 2 hours. The reaction solution was filtered and dried to obtain hydrochloride of compound 56-4. MS (ESI, pos.ion) m/z:205.0 [M+1-HCl]⁺.

### Step 4: synthesis of compound 56-5

Hydrochloride of compound 56-4 (155 mg, 0.65 mmol), 4-bromo-2-(6-azaspiro[2.5]octan-6-yl)benzoic acid (220 mg, 0.71 mmol), and HATU (270 mg, 0.71 mmol) were dissolved in ultra-dry DMF (2 mL), to which DIEA (0.43 mL, 2.58 mmol) was added, and the reaction mixture was allowed to react at 25°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 56-5.

### Step 5: synthesis of compound 56

Compound 56-5 (269 mg, 0.54 mmol), 2-hydroxylethanesulfonamide (101.72 mg, 0.81 mmol), cesium carbonate (530 mg, 1.63 mmol) and a catalyst (CAS: 1599466-83-7; 100 mg, 0.11 mmol) were dissolved in 1,4-dioxane (5 mL), and the reaction mixture was reacted at 100°C for 2 hours under N2 protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 56. MS (ESI, pos.ion) m/z:541.3 [M+1]⁺.

**¹H NMR** (400 MHz, DMSO-d6) δ = 11.16 (s, 1H), 10.09 (br s, 1H), 7.86 (d, *J* = 8.5 Hz, 1H), 7.80 (dd, *J* = 3.8, 8.5 Hz, 1H), 7.49 (d, *J* = 3.1 Hz, 1H), 7.22 - 7.16 (m, 1H), 7.03 (dd, *J* = 1.7, 8.4 Hz, 1H), 6.97 - 6.89 (m, 1H), 6.71 (t, *J =* 2.6 Hz, 1H), 5.04 - 4.86 (m, 1H), 4.16 (d, *J* = 7.0 Hz, 2H), 3.77 (br t, *J* = 6.5 Hz, 2H), 3.35 (t, *J* = 6.6 Hz, 2H), 3.08 - 2.95 (m, 4H), 1.45 (br s, 4H), 1.33 - 1.26 (m, 1H), 0.54 - 0.48 (m, 2H), 0.39 (q, *J* = 4.9 Hz, 2H), 0.28 (s, 4H).

### Example 68: synthesis of N-(1-(cyclopropylmethyl)-1H-indol-4-yl)-4-((2-hydroxyethyl)sulfonamido)-2-(6-azaspiro[2.5]oct-6-yl)benzamide

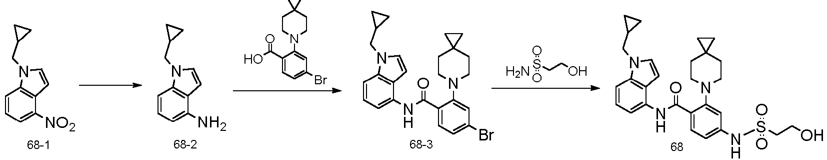

### Step 1: synthesis of compound 68-2

Compound 68-1 (1 g, 4.63 mmol) was dissolved in methanol (10 mL), palladium on carbon (200 mg) was added, and the reaction mixture was stirred overnight at room temperature under hydrogen atmosphere. The reaction solution was filtered and concentrated under reduced pressure to obtain compound 68-2.

### Step 2: synthesis of compound 68-3

Compound 68-2 (800 mg, 4.3 mmol), HATU (2.45 g, 6.45 mmol), DIEA (1.66 g, 12.9 mmol), and 4-bromo-2-(6-azaspiro[2.5]oct-6-yl)benzoic acid (1.33 g, 4.3 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 68-3.

### Step 3: synthesis of compound 68

Compound 68-3 (700 mg, 1.47 mmol), cesium carbonate (1.44 g, 4.4 mmol), tBuXPhos Pd G4 (119 mg, 0.147 mmol) and 2-hydroxyethane-1-sulfonamide (367 mg, 2.94 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 68.

MS (ESI, pos.ion) m/z: 523.2 [M+1]⁺.

¹H NMR (DMSO-d6) *δ*: 11.18 (s, 1H), 10.10 (br s, 1H), 7.94 (d, *J =* 7.6 Hz, 1H), 7.88 (d, *J =* 8.5 Hz, 1H), 7.47 (d, *J* = 3.1 Hz,1H), 7.29 (d, *J* = 8.3 Hz, 1H), 7.20 (d, *J* = 1.9 Hz, 1H), 7.12 (t, *J=* 8.0 Hz, 1H), 7.04 (dd, *J* = 8.5, 1.9 Hz, 1H), 6.67 (d, *J* = 3.0 Hz,1H),4.96 (br s, 1H), 4.05 (d, *J* = 7.0 Hz, 2H), 3.77 (br t, *J* = 6.2 Hz, 2H), 3.33-3.39 (m, 2H), 2.96-3.09 (m, 4H), 1.47 (br s, 4H),1.23-1.30 (m, 1H), 0.50-0.56 (m, 2H), 0.37-0.44 (m, 2H), 0.28 (s, 4H).

### Example 126: synthesis of 4-((2-hydroxyethyl)sulfonamide)-2-(6-azaspiro[2.5]octan-6-yl)-N-(6,7,8,9-tetrahydropyridine[1,2-a]indole-1)benzamide (126)

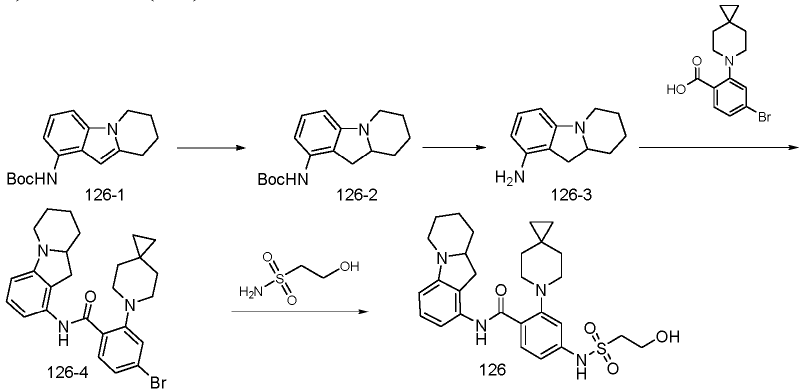

### Step 1: synthesis of compound 126-2

Compound 126-1 (100 mg, 0.350 mmol) and palladium on carbon (20 mg) were dissolved in methanol (10 mL), and the reaction mixture was stirred for 16 hours at room temperature under hydrogen atmosphere. The palladium on carbon was filtered off and the organic phase was concentrated under reduced pressure to obtain compound 126-2.

### Step 2: synthesis of compound 126-3

Compound 126-2 (95 mg, 0.33 mmol) was dissolved in ethyl acetate (5 mL), hydrogen chloride-ethyl acetate solution (0.5 mL, 1.98 mmol) was added dropwise under ice bath conditions, and the reaction solution was stirred at room temperature for 1 hour. The reaction solution was then concentrated under reduced pressure to obtain compound 126-3.

### Step 3: synthesis of compound 126-4

Compound 126-3 (60 mg, 0.319 mmol), 4-bromo-2-(6-azaspiro[2.5]octan-6-yl)benzoic acid (108 mg, 0.351 mmol), HATU (182 mg, 0.479 mmol) and DIPEA (0.51 mL, 0.957 mmol) were dissolved in DMF (10 mL), and the mixture was stirred at room temperature for two hours. The reaction solution was poured into an appropriate amount of ice water, extracted three times with ethyl acetate (10 mL), washed twice with sodium chloride aqueous solution (10 mL), the organic phase was dried over anhydrous sodium sulfate, concentrated under reduced pressure, and the crude product was purified by silica gel column chromatography (EA/PE = 0-20%) to obtain compound 126-4.

### Step 4: synthesis of compound 126

Compound 126-4 (40 mg, 0.083 mmol), cesium carbonate (81 mg, 0.25 mmol), a catalyst (CAS: 1599466-89-3; 6.7 mg, 0.008 mmol) and 2-hydroxyethane-1-sulfonamide (52 mg, 0.417 mmol) were dissolved in 1,4-dioxane (10 mL), and the mixture was reacted at 80°C for 16 hours under nitrogen protection. The reaction solution was cooled and poured into ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 126. MS (ESI, pos.ion) m/z: 525.2 [M+1]⁺.

**¹H NMR** (DMSO-d6) δ: 10.71-10.79 (m, 1H), 7.76-7.84 (m, 1H), 7.11-7.20 (m, 2H), 6.93-7.03 (m, 2H), 6.24-6.32 (m, 1H),3.72-3.79 (m, 2H), 3.58-3.67 (m, 1H), 3.30 (br s, 2H), 3.19-3.29 (m, 2H), 2.97 (br d, *J* = 6.5 Hz, 4H), 2.51-2.67 (m, 2H),1.72-1.82 (m, 2H), 1.59-1.66 (m, 1H), 1.36-1.54 (m, 4H), 0.28-0.37 (m, 4H).

### Example 127: synthesis of N-(1-(cyclopropylmethyl)-1H-indol-4-yl)-4-methoxy-2-(6-azaspiro[2.5]octan-6-yl)benzamide (127)

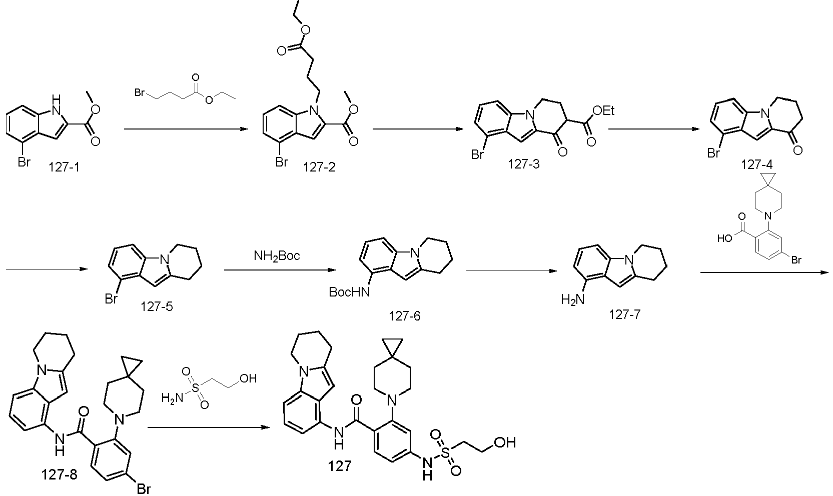

### Step 1: synthesis of compound 127-2

Compound 127-1 (6 g, 23.62 mmol), ethyl 4-bromobutyrate (5.5 g, 28.35 mmol) and potassium carbonate (9.8 g, 70.87 mmol) were dissolved in DMF (100 mL), and the mixture was stirred at room temperature for two hours. The reaction solution was poured into an appropriate amount of ice water, extracted three times with ethyl acetate (60 mL), washed twice with sodium chloride aqueous solution (60 mL), the organic phase was dried over anhydrous sodium sulfate, concentrated under reduced pressure, and the crude product was purified by silica gel column chromatography (EA/PE = 0-20%) to obtain compound 127-2.

### Step 2: synthesis of compound 127-3

Compound 127-2 (5 g, 13.59 mmol) and potassium tert-butoxide (3.04 g, 27.17 mmol) were dissolved in tetrahydrofuran (50 mL). The reaction mixture was stirred at room temperature for two hours. The reaction solution was poured into an appropriate amount of ice water, extracted three times with ethyl acetate (30 mL), washed twice with sodium chloride aqueous solution (30 mL), the organic phase was dried over anhydrous sodium sulfate, concentrated under reduced pressure, and the crude product was purified by silica gel column chromatography (EA/PE = 0-10%) to obtain compound 127-3.

### Step 3: synthesis of compound 127-4

Compound 127-3 (3.7 g, 11.01 mmol) was dissolved in ethanol (30 mL), concentrated hydrochloric acid (35 mL) was added under ice bath conditions, and the reaction mixture was refluxed at 80°C for 2 hours. The reaction solution was cooled and a solid was precipitated, and the reaction solution was filtered to obtain crude product 127-4, which was used directly in the next reaction.

### Step 4: synthesis of compound 127-5

Compound 127-4 (1 g, 3.79 mmol) was dissolved in tetrahydrofuran (10 mL), and then borane-tetrahydrofuran solution (11.36 mL, 11.36 mmol) was added under ice bath conditions. The reaction solution was stirred overnight at 70°C, cooled, then quenched with dilute hydrochloric acid under ice bath conditions, extracted three times with ethyl acetate (10 mL), and washed twice with sodium chloride aqueous solution (10 mL). The organic phase was dried over anhydrous sodium sulfate, concentrated under reduced pressure, and the crude product was purified by silica gel column chromatography (EA/PE = 0-10%) to obtain compound 127-5.

### Step 5: synthesis of compound 127-6

Compound 127-5 (100 mg, 0.4 mmol), cesium carbonate (391 mg, 1.2 mmol), a catalyst (CAS: 1599466-89-3; 32 mg, 0.04 mmol) and tert-butyl carbamate (140 mg, 1.2 mmol) were dissolved in 1,4-dioxane (10 mL), and the mixture was reacted at 80°C for 2 hours under nitrogen protection. The reaction solution was cooled and poured into an appropriate amount of ice water, extracted three times with ethyl acetate (10 mL), washed twice with sodium chloride aqueous solution (10 mL), the organic phase was dried over anhydrous sodium sulfate, concentrated under reduced pressure, and the crude product was purified by silica gel column chromatography (EA/PE = 0-20%) to obtain compound 127-6.

### Step 6: synthesis of compound 127-7

Compound 127-6 (110 mg, 11.01 mmol) was dissolved in ethyl acetate (5 mL), hydrogen chloride-ethyl acetate solution (1.92 mL, 3.85 mmol) was added under ice bath conditions, and the reaction mixture was stirred at room temperature for 2 hours. A solid was precipitated out from the reaction solution, and the reaction solution was concentrated to obtain the crude product 127-7, which was used directly in the next reaction.

### Step 7: synthesis of compound 127-8

Compound 127-7 (70 mg, 0.376 mmol), 4-bromo-2-(6-azaspiro[2.5]octan-6-yl)benzoic acid (140 mg, 0.452 mmol), HATU (215 mg, 0.565 mmol) and DIPEA (0.33 mL, 1.88 mmol) were dissolved in tetrahydrofuran (10 mL), and the mixture was stirred at room temperature for two hours. The reaction solution was poured into an appropriate amount of ice water, extracted three times with ethyl acetate (10 mL), washed twice with sodium chloride aqueous solution (10 mL), the organic phase was dried over anhydrous sodium sulfate, concentrated under reduced pressure, and the crude product was purified by silica gel column chromatography (EA/PE = 0-20%) to obtain compound 127-8.

### Step 8: synthesis of compound 127

Compound 127-8 (100 mg, 0.208 mmol), cesium carbonate (205 mg, 0.628 mmol), a catalyst (CAS: 1599466-89-3; 17 mg, 0.02 mmol) and 2-hydroxyethane-1-sulfonamide (130 mg, 1.05 mmol) were dissolved in 1,4-dioxane (10 mL), and the mixture was reacted at 105°C for 16 hours under nitrogen protection. The reaction solution was cooled and poured into ice water, extracted three times with ethyl acetate (20 mL), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 127. MS (ESI) m/z: 523.2 [M+1]⁺.

**¹H NMR** (CHLOROFORM-d) δ: 11.56 (s, 1H), 8.09 (d, *J* = 8.5 Hz, 1H), 7.79 (d, *J=* 7.4 Hz, 1H), 7.31 (d, *J =* 1.9 Hz, 1H), 7.10-7.18 (m, 2H), 6.95 (dd, *J =* 8.4, 1.9 Hz, 1H), 6.25 (s, 1H), 4.05-4.10 (m, 2H), 4.00-4.05 (m, 2H), 3.19-3.24 (m, 2H), 3.09 (br t, *J = 5.2* Hz, 4H), 2.95-3.00 (m, 2H), 2.06-2.15 (m, 2H), 1.88-1.95 (m, 2H), 1.54 (br s, 4H), 0.30 (s, 4H).

### Example 128: synthesis of 2-(6-azaspiro[2.5]oct-6-yl)-N-(8,8-difluoro-6,7,8,9-tetrahydropyridino[1,2-a]indol-1-yl)-4-{[(2-hydroxyethyl) sulfonamide]}benzamide (128)

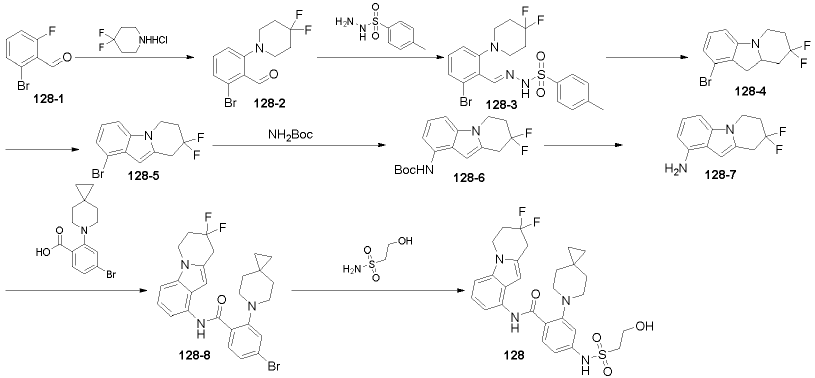

### Step 1: synthesis of compound 128-2

Compound 128-1 (1.50 g, 7.39 mmol) was dissolved in DMSO (15 mL), to which 4,4-difluorohexahydropyridine hydrochloride (1.40 g, 8.87 mmol) and potassium carbonate (2.55 g, 18.47 mmol) were added, and the reaction mixture was allowed to react at 95°C for 2 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 128-2.

### Step 2: synthesis of compound 128-3

Compound 128-2 (1.50 g, 4.93 mmol) was dissolved in toluene (30 mL), to which N-amino-4-tosylamide (1.10 g, 5.92 mmol) was added, and reaction was carried out at room temperature for 20 min to obtain the reaction solution containing compound 128-3, which was used directly in the next reaction.

### Step 3: synthesis of compound 128-4

To the reaction solution from step 2, sodium hydride (0.22 g, 5.43 mmol) was added and the reaction mixture was allowed to react at 135°C for 20 min. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 128-4.

### Step 4: synthesis of compound 128-5

Compound 128-4 (560 mg, 1.94 mmol) was dissolved in 1,2-dichloroethane (5 mL), DDQ (883.03 mg, 3.89 mmol) was added, and the reaction mixture was allowed to react at room temperature for 2 h. The reaction solution was quenched with water, and extracted with DCM. The organic phase was washed with saturated NaCl solution, dried over anhydrous Na₂SO₄, and concentrated under reduced pressure. The crude product was purified by silica gel column chromatography (PE:EA=1/20-1/10) to obtain compound 128-5.

### Step 5: synthesis of compound 128-6

Compound 128-5 (672 mg, 2.35 mmol), NH₂Boc (550.29 mg, 4.70 mmol), cesium carbonate (2.30 g, 7.05 mmol) and RuPhos Pd G4 (199.73 mg, 0.23 mmol) were dissolved in 1,4-dioxane (15 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/5) to obtain compound 128-6.

### Step 6: synthesis of compound 128-7.

Compound 128-6 (800 mg, 1.72 mmol) was dissolved in DCM (5 mL), to which hydrogen chloride-1,4-dioxane (5 mL) was added, and the reaction mixture was allowed to react at room temperature for 0.5 h. The reaction solution was concentrated under reduced pressure to obtain hydrochloride of compound 128-7, which was used directly in the next reaction.

### Step 7: synthesis of compound 128-8.

Hydrochloride of compound 128-7 (551.53 mg, 2.48 mmol), 4-bromo-2-(6-azaspiro[2.5]octan-6-yl)benzoic acid (923.75 mg, 2.98 mmol), and HATU (1133.12 mg, 2.98 mmol) were dissolved in DMF (10 mL), to which DIEA (1283.45 mg, 9.93 mmol) was added, and the reaction mixture was allowed to react at room temperature for 4 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 128-8.

### Step 8: synthesis of compound 128

Compound 128-8 (500 mg, 0.97 mmol), 2-hydroxylethanesulfonamide (486.54 mg, 3.89 mmol), cesium carbonate (633.39 mg, 1.94 mmol) and BrettPhos Pd G4 (89.47 mg, 0.10 mmol) were dissolved in 1,4-dioxane (15 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative high performance liquid chromatography to obtain compound 128. MS m/z (ESI):559.2 [M+1]⁺.

**¹H NMR** (DMSO-d6) δ = 11.25 (s, 1H), 7.89 - 7.80 (m, 2H), 7.24 - 7.18 (m, 1H), 7.17 - 7.10 (m, 2H), 6.98 (dd, *J=* 1.9, 8.5 Hz, 1H), 6.51 (s, 1H), 4.34 - 4.18 (m, 2H), 3.76 (t, *J* = 6.6 Hz, 2H), 3.63 - 3.48 (m, 2H), 3.30 - 3.26 (m, 2H), 3.01 (brt, *J* = 4.8 Hz, 4H), 2.72 - 2.59 (m, 2H), 1.47 (br s, 4H), 0.28 (s, 4H).

### Example 129: synthesis of N-(2,3-dihydro-1H-pyrrolo[1,2-a]indol-8-yl)-4-((2-hydroxyethyl)sulfonamide)-2-(6-azaspiro[2.5]octan-6-yl)benzamide (129)

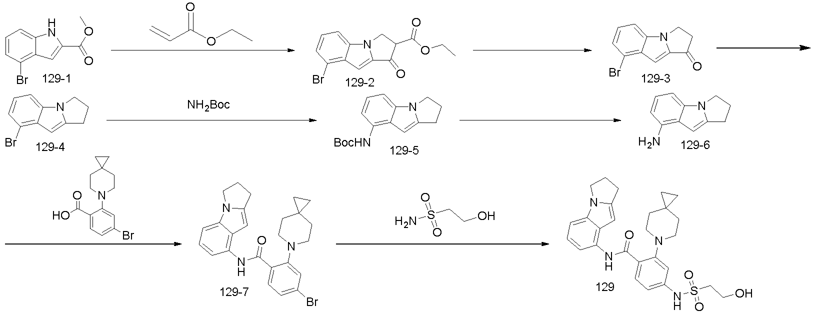

### Step 1: synthesis of compound 129-2

Compound 129-1 (3 g, 11.81 mmol) was dissolved in toluene (15 mL), to which potassium tert-butoxide (0.88 g, 11.81 mmol) and ethyl acrylate (2.57 mL, 23.61 mmol) were added, and the reaction mixture was allowed to react at 110°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 129-2. MS m/z (ESI):322.0 [M+1]⁺.

### Step 2: synthesis of compound 129-3

Compound 129-2 (2.28 g, 7.08 mmol) was dissolved in ultra-dry ethanol (30 mL), to which 12 mol/L hydrochloric acid (20 mL) was added, and the reaction mixture was allowed to react at 80°C for 1 h. The reaction solution was concentrated and slowly poured into ice water, and a solid was precipitated. The reaction solution was filtered, and dried to obtain compound 129-3, which was used directly in the next reaction.

### Step 3: synthesis of compound 129-4

Compound 129-3 (1.7 g, 6.8 mmol) was dissolved in trifluoroacetic acid (10 mL), to which triethylsilane (3.25 mL, 20.39 mmol) and trifluoromethanesulfonic acid (0.18 mL, 2.04 mmol) were added under ice bath conditions and under N₂ protection, and the reaction mixture was reacted at 25°C for 2h. The reaction solution was quenched with ice water, neutralized with saturated NaHCO₃ solution, and extracted with EA. The organic phase was washed with saturated NaCl solution, dried over anhydrous Na₂SO₄, and concentrated under reduced pressure. The crude product was purified by silica gel column chromatography (pure petroleum ether) to obtain compound 129-4.

### Step 4: synthesis of compound 129-5

Compound 129-4 (300 mg, 1.27 mmol), NH₂Boc (297 mg, 2.54 mmol), cesium carbonate (1.24 g, 3.81 mmol) and palladium catalyst (CAS: 1599466-89-3; 52 mg, 0.06 mmol) were dissolved in 1,4-dioxane (5 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 129-5. MS m/z (ESI):273.2 [M+1]⁺.

### Step 5: synthesis of compound 129-6

Compound 129-5 (260 mg, 0.95 mmol) was dissolved in DCM (1 mL), to which HCl\EA (2 mL) was added, and the reaction mixture was allowed to react at 25°C for 2 h. A solid was precipitated, and the reaction solution was filtered, and dried to obtain hydrochloride of compound 129-6, which was used directly in the next reaction.

### Step 6: synthesis of compound 129-7

Hydrochloride of compound 129-6 (130 mg, 0.75 mmol), 4-bromo-2-(6-azaspiro[2.5]octan-6-yl)benzoic acid (281 mg, 0.91 mmol), and HATU (316 mg, 0.83 mmol) were dissolved in DMF (1 mL), to which DIEA (0.5 mL, 3.02 mmol) was added, and the reaction mixture was allowed to react at 25°C for 1 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 129-7. MS m/z (ESI):464.1\466.1 [M+1]⁺.

### Step 7: synthesis of compound 129

Compound 129-7 (198 mg, 0.43 mmol), 2-hydroxyethanesulfonamide (267 mg, 2.13 mmol), cesium carbonate (417 mg, 1.28 mmol) and palladium catalyst (CAS: 1599466-89-3; 18 mg, 0.02 mmol) were dissolved in 1,4-dioxane (5 mL), and the reaction mixture was reacted at 80°C for 3 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=1/20-1/10) to obtain compound 129. MS m/z (ESI):509.2 [M+1]⁺.

**¹H NMR** (DMSO-d6) δ: 11.19 (s, 1H), 7.83-7.88 (m, 2H), 7.15 (s, 1H), 7.07-7.11 (m, 1H), 6.98-7.05 (m, 2H), 6.32 (s, 1H), 4.09 (t, *J=* 6.9 Hz, 2H), 3.76 (t, *J* = 6.6 Hz, 2H), 3.28-3.31 (m, 2H), 2.93-3.06 (m, 7H), 2.58 (quin, *J* = 7.2 Hz, 2H), 1.47 (br s, 4H), 0.28 (s, 4H).

### Example 130: synthesis of 4-((2-hydroxyethyl)sulfonamide)-2-(6-azaspiro[2.5]octan-6-yl)-N-(2,3,9,9a-tetrahydro-1H-pyrrolo[1,2a]indol-8-yl)benzamide (130)

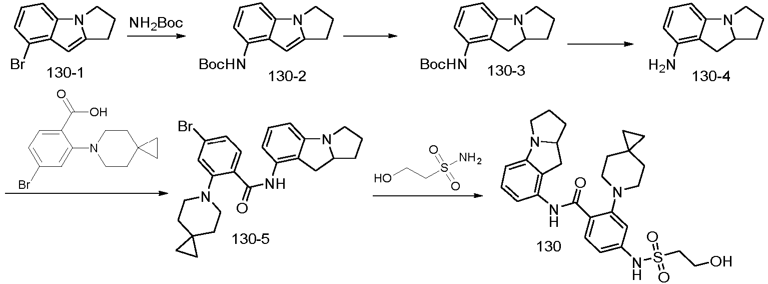

### Step 1: synthesis of compound 130-2

Compound 130-1 (220 mg, 0.93 mmol), NH₂Boc (218 mg, 1.86 mmol), cesium carbonate (911 mg, 2.80 mmol) and palladium catalyst (CAS: 1599466-89-3; 38 mg, 0.05 mmol) were dissolved in 1,4-dioxane (5 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 130-2. MS m/z (ESI):273.2 [M+1]⁺.

### Step 2: synthesis of compound 130-3

Compound 130-2 (215 mg, 0.79 mmol) was dissolved in MeOH (2 mL), to which Pd/C (50 mg) and 12 mol/L hydrochloric acid (0.02 mL) were added, and the reaction mixture was allowed to react at 25°C overnight. The reaction solution was filtered, and concentrated under reduced pressure. The crude product was purified by silica gel column chromatography (PE:EA=1/10- DCM:EA=1/10) to obtain compound 130-3. MS m/z (ESI):275.2 [M+1]⁺.

### Step 3: synthesis of compound 130-4

Compound 130-3 (86 mg, 0.32 mmol) was dissolved in DCM (1 mL), to which HCl\EA (1 mL) was added, and the reaction mixture was allowed to react at 25°C for 2 h. A solid was precipitated, and the reaction solution was filtered, and dried to obtain hydrochloride of compound 130-4, which was used directly in the next reaction.

### Step 4: synthesis of compound 130-5

Hydrochloride of compound 130-4 (67 mg, 0.38 mmol), 4-bromo-2-(6-azaspiro[2.5]octan-6-yl)benzoic acid (143 mg, 0.46 mmol), and HATU (161 mg, 0.42 mmol) were dissolved in DMF (1 mL), to which DIEA (0.25 mL, 1.54 mmol) was added, and the reaction mixture was allowed to react at 25°C for 1 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 130-5.

### Step 5: synthesis of compound 130

Compound 130-5 (85 mg, 0.18 mmol), 2-hydroxyethanesulfonamide (114 mg, 0.91 mmol), cesium carbonate (178 mg, 0.55 mmol) and palladium catalyst (CAS: 1599466-89-3; 8 mg, 0.01 mmol) were dissolved in 1,4-dioxane (2 mL), and the reaction mixture was reacted at 80°C for 2 h under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 130. MS m/z (ESI):511.2 [M+1]⁺.

**¹H NMR** (DMSO-d6) δ: 10.70 (br s, 1H), 7.79 (d, *J* = 8.5 Hz, 1H), 7.34-7.38 (m, 1H), 7.12 (s, 1H), 7.04 (t, *J* = 7.9 Hz, 1H), 6.94-7.01 (m, 1H), 6.38 (d, *J* = 7.8 Hz, 1H), 3.88 (br d, *J* = 8.9 Hz, 1H), 3.75 (t, *J* = 6.6 Hz, 2H), 3.03-3.30 (m, 6H), 2.98 (br t, *J= 4.8* Hz, 4H), 1.70-1.91 (m, 4H), 1.45-1.52 (m, 4H), 0.31-0.34 (m, 4H).

### Example 147: synthesis of N-(4-(3-(3-(4,4-difluoropiperidin-1-yl)phenyl)urea)-3-(6-azaspiro[2.5]octan-6-yl)phenyl)-2-hydroxyethane-1-sulfonamide (147)

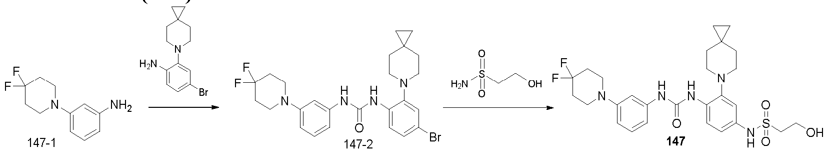

### Step 1: synthesis of compound 147-2

Compound 147-1 (100 mg, 0.472 mmol) was added slowly to a solution of triphosgene (42 mg, 0.142 mmol) in dichloromethane (5 mL) under ice bath conditions. Then triethylamine (0.195 mL, 1.42 mmol) was added dropwise, and the reaction solution was stirred at room temperature for 30 minutes. 4-Bromo-2-(6-azaspiro[2.5]octan-6-yl)aniline (133 mg, 472 mmol) was added under ice bath conditions, and the reaction solution was stirred at room temperature for 30 minutes, cooled and poured into an appropriate amount of ice water. The reaction solution was extracted three times with dichloromethane (20 mL), and washed twice with sodium chloride aqueous solution (50 mL). The organic phase was dried over anhydrous sodium sulfate, and concentrated under reduced pressure, and the crude product was purified by silica gel column chromatography (EA/PE = 0-20%) to obtain compound 147-2.

### Step 2: synthesis of compound 147

Compound 147-2 (80 mg, 0.154 mmol), cesium carbonate (150 mg, 0.462 mmol), a catalyst (CAS: 1599466-89-3; 13 mg, 0.015 mmol) and 2-hydroxyethane-1-sulfonamide (96 mg, 0.771 mmol) were dissolved in 1,4-dioxane (10 mL), and the mixture was reacted at 100°C for 16 hours under nitrogen protection. The reaction solution was cooled and poured into ice water, extracted three times with ethyl acetate (20 mL), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 147. MS (ESI) m/z: 564.2 [M+1]⁺.

**¹H NMR** (ACETONITRILE-d3) δ: 7.99 (br d, *J* = 8.8 Hz, 1H), 7.62-7.84 (m, 2H), 7.18-7.34 (m, 1H), 6.98-7.18 (m, 3H), 6.75-6.96 (m, 2H), 6.60 (br d, *J=* 7.1 Hz, 1H), 3.80 (q, *J* = 5.6 Hz, 2H), 3.17-3.38 (m, 4H), 3.01-3.17 (m, 3H), 2.73 (br t, *J =* 4.7 Hz, 4H), 1.91-2.05 (m, 4H), 1.32-1.59 (m, 4H), 0.17-0.38 (m, 4H).

### Example 150: synthesis of N-(4-(3-(1-(cyclopropylmethyl)indolin-6-yl)urea)-3-(6-azaspiro[2.5]octan-6-yl)phenyl)-2-hydroxyethane-1-sulfonamide (150)

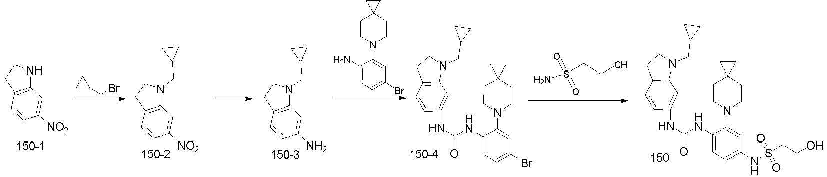

### Step 1: synthesis of compound 150-2

Compound 150-1 (2 g, 12.18 mmol), and potassium carbonate (5.05 g, 36.55 mmol) were dissolved in DMF (10 mL), to which (bromomethyl)cyclopropane (1.43 mL, 14.62 mmol) was added, and the reaction mixture was reacted at 80°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 150-2. MS m/z (ESI):219.1 [M+1]⁺.

### Step 2: synthesis of compound 150-3

Compound 150-2 ( 600 mg, 2.75 mmol), ammonium chloride (1.47 g, 27.49 mmol) and iron dust (1.54 g, 27.49 mmol) were dissolved in EtOH (15 mL) and H₂O (5 mL), and the reaction mixture was allowed to react at 80°C for 2 h. The reaction solution was filtered, and concentrated under reduced pressure. The crude product was purified by silica gel column chromatography (PE:EA=1/20-1/10) to obtain compound 150-3. MS m/z (ESI):189.1 [M+1]⁺.

### Step 3: synthesis of compound 150-4

Compound 150-3 (180 mg, 0.96 mmol), and triphosgene (113 mg, 0.38 mmol) were dissolved in DCM (10 mL) and stirred at 0°C for 10 minutes. To this mixture was added 4-bromo-2-(6-azaspiro[2.5]octan-6-yl)aniline (269 mg, 0.96 mmol) under ice bath conditions, and the reaction mixture was reacted at 25°C for 2 h. The reaction solution was quenched with ice water, and extracted with DCM. The organic phase was washed with saturated NaCl solution, dried over anhydrous Na₂SO₄, and concentrated under reduced pressure. The crude product was purified by silica gel column chromatography (PE:EA=1/10) to obtain compound 150-4.

### Step 4: synthesis of compound 150

Compound 150-4 (160 mg, 0.32 mmol), 2-hydroxyethanesulfonamide (202 mg, 1.61 mmol), cesium carbonate (316 mg, 0.97 mmol) and palladium catalyst (CAS: 1599466-89-3; 13 mg, 0.02 mmol) were dissolved in 1,4-dioxane (5 mL), and the reaction mixture was reacted at 80°C for 3 h under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 150. MS m/z (ESI):540.4 [M+1]⁺.

**¹H NMR** (DMSO-d6) δ: 9.20 (s, 2H), 7.94 (d, *J* = 8.8 Hz, 1H), 7.87 (s, 1H), 7.03 (d, *J=* 2.1 Hz, 1H), 6.85-6.94 (m, 2H), 6.74 (s, 1H), 6.58 (d, *J=* 7.9 Hz, 1H), 4.90 (br s, 1H), 3.73 (br d, *J* = 4.5 Hz, 2H), 3.37-3.45 (m, 2H), 3.13-3.20 (m, 2H), 2.88-2.91 (m, 2H), 2.79-2.85 (m, 2H), 2.73-2.78 (m, 4H), 1.57 (br s, 5H), 0.97 (br s, 2H), 0.47-0.53 (m, 2H), 0.35 (s, 4H), 0.18-0.23 (m, 2H).

### Example 151: synthesis of N-(4-(3-(1-(cyclopropylmethyl)indolin-5-yl)urea)-3-(6-azaspiro[2.5]octan-6-yl)phenyl)-2-hydroxyethane-1-sulfonamide (151)

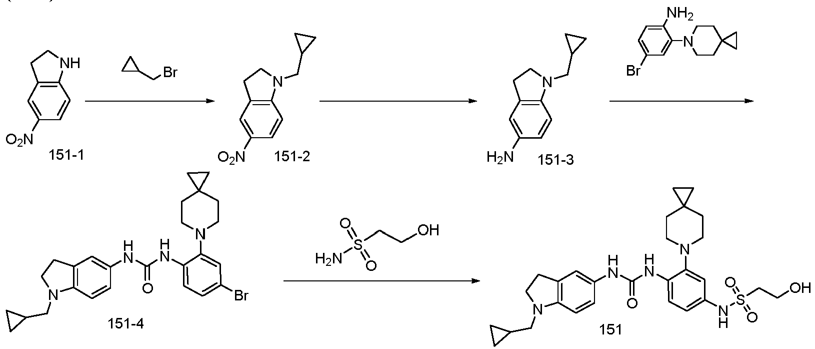

### Step 1: synthesis of compound 151-2

Compound 151-1 (2.0 g, 12.191 mmol) and NaH (585 mg, 24.375 mmol) were weighed into a reaction flask, DMF (10 mL) was added, and after the reaction solution was stirred at room temperature for 30 minutes, to the reaction solution was added (bromomethyl)cyclopropane (1.81 g, 13.410 mmol) and the reaction mixture was reacted in an oil bath at 70°C for 3 h. After the reaction was completed, the reaction solution was diluted with water (100 mL), and extracted with ethyl acetate (30×3 mL). The organic layer was washed with brine (30×3 mL), dried over Na₂SO₄, filtered and concentrated. After concentration, compound 151-2 was obtained and was used directly in the next step.

### Step 2: synthesis of compound 151-3

Compound 151-2 (1.5 g, 6.877 mmol), reduced iron dust (3.85 g, 68.77 mmol) and ammonium chloride (3.68 g, 68.77 mmol) were added to a reaction flask with EtOH:H₂O = 3: 1 (12 mL) as solvents, and the reaction mixture was allowed to react at 80°C for 3 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-70:30) to obtain compound 151-3.

### Step 3: synthesis of compound 151-4

Triphosgene (79 mg, 0.266 mmol) and compound 151-3 (100 mg, 0.532 mmol) were dissolved in DCM, followed by addition of triethylamine (162 mg, 1.601 mmol) under ice bath conditions. After the reaction solution was stirred at room temperature for half an hour, 4-bromo-2-(6-azaspiro[2.5]octan-6-yl)aniline (150 mg, 0.533 mmol) was added thereto and the reaction was stirred for another 3 hours. After the reaction was completed, the reaction solution was directly concentrated. The crude product was purified by column chromatography (PE:EA=100:0-90:10-70:30) to obtain compound 151-4.

### Step 4: synthesis of compound 151

Compound 151-4 (110 mg, 0.223 mmol), a catalyst (CAS: 1599466-89-3; 10 mg, 0.011 mmol) and Cs₂CO₃ (145 mg, 0.445 mmol) were added to a reaction flask with 1,4-dioxane as the solvent and the reaction solution was stirred in an oil bath at 90°C for 3 hours under argon protection. After the reaction was completed, the reaction solution was directly concentrated. The crude product was purified by column chromatography (PE:EA=100:0-70:30-50:50-0:100) to obtain compound 151.

¹H NMR (400 MHz, DMSO-*d*₆) *δ* = 9.43-9.30 (m, 1H), 9.00-8.87 (m, 1H), 8.01-7.93 (m, 1H), 7.84-7.76 (m, 1H), 7.21-7.15 (m, 1H), 7.04-6.96 (m, 2H), 6.90-6.86 (m, 1H), 6.50-6.43 (m, 1H), 4.95-4.89 (m, 1H), 3.78-3.72 (m, 2H), 3.42-3.34 (m, 2H), 3.20-3.14 (m, 2H), 2.92-2.84 (m, 4H), 2.79-2.69 (m, 4H), 1.59-1.38 (m, 4H), 1.00-0.94 (m, 1H), 0.54-0.48 (m, 2H), 0.36-0.30 (m, 4H), 0.23-0.18 (m, 2H).

### Example 234: synthesis of 4-(2-hydroxyethyl)sulfonamido-N-(2-methyl-1,2,3,4-tetrahydropyrazino [1,2-a] indol-9-yl)-2-(6-azaspiro [2.5] octan-6-yl)benzamide (234)

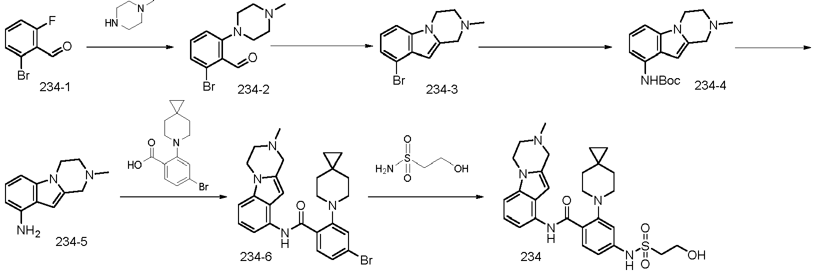

### Step 1: synthesis of compound 234-2

Compound 234-1 (2 g, 9.9 mmol), 1-methylpiperazine (1.19 g, 11.88 mmol) and potassium carbonate (4.1 g, 29.7 mmol) were dissolved in water (20 mL), and the mixture was stirred at 100°C for two hours. The reaction solution was poured into an appropriate amount of ice water, extracted three times with ethyl acetate (20 mL), washed twice with sodium chloride aqueous solution (20 mL), the organic phase was dried over anhydrous sodium sulfate, concentrated under reduced pressure, and the crude product was purified by silica gel column chromatography (EA/PE = 0-70%) to obtain compound 234-2.

### Step 2: synthesis of compound 234-3

Compound 234-2 (2 g, 7.09 mmol), dichlorobis(4-methylisopropylphenyl)ruthenium (II) (1.3 g, 2.13 mmol) and d-camphorsulfonic acid (987 mg, 4.26 mmol) were dissolved in toluene (20 mL) and molecular sieves (4 g) were added, and the mixture was stirred at 140°C for eight hours. The reaction solution was poured into an appropriate amount of ice water, extracted three times with ethyl acetate (30 mL), washed twice with sodium chloride aqueous solution (30 mL), the organic phase was dried over anhydrous sodium sulfate, concentrated under reduced pressure, and the crude product was purified by silica gel column chromatography (EA/PE = 0-90%) to obtain compound 234-3.

### Step 3: synthesis of compound 234-4

Compound 234-3 (80 mg, 0.3 mmol), cesium carbonate (296 mg, 0.91 mmol), a catalyst (CAS: 1599466-89-3; 24 mg, 0.03 mmol) and tert-butyl carbamate (53 mg, 0.455 mmol) were dissolved in 1,4-dioxane (2 mL), and the mixture was reacted at 80°C for 2 hours under nitrogen protection. The reaction solution was cooled and poured into an appropriate amount of ice water, extracted three times with ethyl acetate (10 mL), washed twice with sodium chloride aqueous solution (10 mL), the organic phase was dried over anhydrous sodium sulfate, concentrated under reduced pressure, and the crude product was purified by silica gel column chromatography (EA/PE = 0-90%) to obtain compound 234-4.

### Step 4: synthesis of compound 234-5

Compound 234-4 (20 mg, 0.07 mmol) was dissolved in ethyl acetate (1 mL), hydrogen chloride-ethyl acetate solution (0.5 mL, 0.797 mmol) was added under ice bath conditions, and the reaction mixture was stirred at room temperature for 2 hours. A solid was precipitated out from the reaction solution, and the reaction solution was concentrated to obtain the crude product compound 234-5, which was used directly in the next reaction.

### Step 5: synthesis of compound 234-6

Compound 234-5 (13 mg, 0.065 mmol), 4-bromo-2-(6-azaspiro[2.5]octan-6-yl)benzoic acid (34 mg, 0.077 mmol), HATU (37 mg, 0.097 mmol) and DIPEA (34 ul, 0.194 mmol) were dissolved in DMF (3 mL), and the mixture was stirred at room temperature for two hours. The reaction solution was poured into an appropriate amount of ice water, extracted three times with ethyl acetate (10 mL), washed twice with sodium chloride aqueous solution (10 mL), the organic phase was dried over anhydrous sodium sulfate, concentrated under reduced pressure, and the crude product was purified by silica gel column chromatography (EA/PE = 0-20%) to obtain compound 234-6.

### Step 6: synthesis of compound 234

Compound 234-6 (25 mg, 0.051 mmol), cesium carbonate (50 mg, 0.152 mmol), a catalyst (CAS: 1599466-89-3; 4 mg, 0.005 mmol) and 2-hydroxyethane-1-sulfonamide (32 mg, 0.254 mmol) were dissolved in 1,4-dioxane (3 mL), and the mixture was reacted at 80°C for 16 hours under nitrogen protection. The reaction solution was cooled and poured into ice water, extracted three times with ethyl acetate (10 mL), washed twice with sodium chloride aqueous solution (10 mL), the organic phase was dried over anhydrous sodium sulfate, concentrated under reduced pressure, and the crude product was purified by silica gel column chromatography (EA/PE = 0-80%) to obtain compound 234.

**¹H NMR** (CHLOROFORM-d, 400 MHz) *δ* 11.63 (s, 1H), 8.0-8.1 (m, 1H), 7.8-7.8 (m, 1H), 7.3-7.3 (m, 1H), 7.2-7.2 (m, 1H),7.1-7.2 (m, 1H), 6.9-6.9 (m, 1H), 6.2-6.3 (m, 1H), 4.1-4.2 (m, 2H), 3.9-4.0 (m, 2H), 3.79 (s, 2H), 3.2-3.2 (m, 2H), 3.0-3.1 (m,4H), 2.9-3.0 (m, 2H), 2.5-2.6 (m, 3H), 1.4-1.6 (m, 4H), 0.2-0.4 (m, 4H).

### Example 235: synthesis of N-(3, 4-dihydro-1H-[1, 4]oxazino[4, 3-a]indol-9-yl)-4-(2-hydroxyethylsulfonamido)-2-(6-azaspiro[2.5]octan-6-yl)benzamide (235)

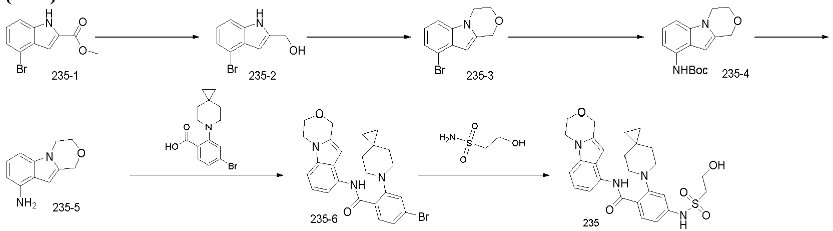

### Step 1: synthesis of compound 235-2

Compound 235-1 (1000 mg, 3.94 mmol) was dissolved in THF and diisobutylaluminium hydride (7.87 mL, 11.81 mmol) was slowly added dropwise at -78°C under nitrogen protection. The reaction system was reacted at -78°C for 3 h. The reaction solution was quenched by adding water (20 mL), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 5/1) to obtain compound 235-2.

### Step 2: synthesis of compound 235-3

Compound 235-2 (500 mg, 2.21 mmol) and potassium hydroxide (310 mg, 5.53 mmol) were dissolved in dichloromethane and diphenyl(vinyl)sulfonium trifluoromethanesulfonate was slowly added dropwise (962 mg, 2.65 mmol) at 0°C. The reaction system was moved to room temperature and reacted for 3 hours. The reaction solution was quenched by adding water (10 mL), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 235-3.

### Step 3: synthesis of compound 235-4

Compound 235-3 (120 mg, 0.48 mmol), tert-butyl carbamate (111 mg, 0.95 mmol), cesium carbonate (465 mg, 1.43 mmol) and a catalyst (CAS: 1599466-85-9; 20 mg, 0.02 mmol) were dissolved in 1,4-dioxane (5 mL), and the reaction mixture was reacted at 90°C for 2 hours under nitrogen protection. The reaction solution was quenched by adding water (10 mL), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 235-4.

### Step 4: synthesis of compound 235-5

Compound 235-4 (110 mg, 0.38 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 235-5 which was used directly in the next step without purification.

### Step 5: synthesis of compound 235-6

Compound 235-5 (60 mg, 0.32 mmol), 4-bromo-2-(6-azaspiro[2.5]oct-6-yl)benzoic acid (119 mg, 0.38 mmol), HATU (133 mg, 0.35 mmol) and *N,N-*diisopropylethylamine (0.21 mL, 1.28 mmol) were dissolved in DMF (4 mL), and the reaction mixture was reacted at room temperature for 1 hour under nitrogen protection at 0°C. The reaction solution was quenched by adding water (10 mL), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 235-6.

### Step 6: synthesis of compound 235

Compound 235-6 (110 mg, 0.23 mmol), 2-hydroxyethylsulfonamide (143 mg, 1.15 mmol), cesium carbonate (224 mg, 0.69 mmol) and a catalyst (CAS: 1599466-89-3; 18 mg, 0.023 mmol) were dissolved in 1,4-dioxane (3 mL), and the reaction mixture was reacted at 80°C for 3 hours under nitrogen protection. The reaction solution was quenched by adding water (10 mL), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 235. MS (ESI, pos.ion) m/z: 525.2 [M+1]⁺.

**¹HNMR** (DMSO-*d*₆ ) δ: 11.2-11.2 (m, 1H), 7.8-7.9 (m, 2H), 7.2-7.2 (m, 2H), 7.1-7.1 (m, 1H), 7.0-7.0 (m, 1H), 6.4-6.5 (m, 1H), 4.9-5.0 (m, 2H), 4.1-4.2 (m, 2H), 4.1-4.1 (m, 2H), 3.7-3.8 (m, 2H), 3.3-3.4 (m, 2H), 3.0-3.1 (m, 4H), 1.4-1.5 (m, 4H), 0.2-0.3 (m, 4H).

### Example 269: synthesis of N-(2-cyclopropylmethyl)-2H-indazol-4-yl)-4-(2-hydroxyethyl)sulfonamido-2-(6-azaspiro[2.5]octan-6-yl)benzamide (269)

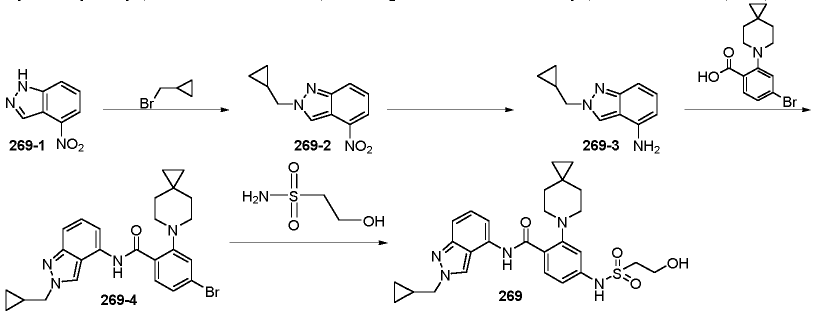

### Step 1: synthesis of compound 269-2

Compound 269-1 (1 g, 6.13 mmol) and (bromomethyl)cyclopropane (0.90 mL, 9.19 mmol) were dissolved in N,N-dimethylformamide (10 mL), to which cesium carbonate (3.99 g, 12.26 mmol) was added. The reaction mixture was stirred in an oil bath at 70°C for 1.5 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by preparative chromatography to obtain compound 269-2.

### Step 2: synthesis of compound 269-3

Compound 269-2 (240 mg, 1.10 mmol) was dissolved in ethanol (3 mL) and water (3 mL), to which iron dust (308 mg, 5.52 mmol) and ammonium chloride (295 mg, 5.52 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-80:20) to obtain compound 269-3.

### Step 3: synthesis of compound 269-4

Compound 269-3 (150 mg, 0.80 mmol) and 4-bromo-2-(6-azaspiro[2.5]octan-6-yl)benzoic acid (273 mg, 0.88 mmol) were dissolved in N,N-dimethylformamide (3 mL), to which azabenzotriazolyltetramethyluronium hexafluorophosphate (366 mg, 0.96 mmol) and N,N-diisopropylethylamine (207 mg, 1.60 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30 × 3 mL), dried over Na₂SO₄, filtered and concentrated. The crude product was purified by column chromatography (PE:EA=100:0-80:20) to obtain compound 269-4.

### Step 4: synthesis of compound 269

Compound 269-4 (100 mg, 0.21 mmol) and 2-hydroxyethane-1-sulfonamide (104 mg, 0.83 mmol) were dissolved in 1,4-dioxane (3 mL), to which a catalyst (CAS: 1599466-89-3; 17 mg, 0.02 mmol) and cesium carbonate (136 mg, 0.42 mmol) were added. The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 269. MS (ESI, pos.ion) m/z: 524.2 [M+1]⁺.

**1H NMR** (400 MHz, DMSO-d6) δ = 11.15-11.09 (m, 1H), 8.67-8.62 (m, 1H), 7.82-7.76 (m, 2H), 7.37-7.32 (m, 1H), 7.25-7.19 (m, 1H), 7.17-7.13 (m, 1H), 7.04-6.99 (m, 1H), 4.32-4.26 (m, 2H), 3.81-3.74 (m, 2H), 3.37-3.33 (m, 2H), 3.06-2.97 (m, 4H), 1.49-1.36 (m, 5H), 0.63-0.56 (m, 2H), 0.48-0.42 (m, 2H), 0.28-0.22 (m, 4H).

### Example 271: synthesis of N-(2-(2,2-difluorocyclopropyl)methyl-2H-indazol-4-yl)-4-(2-hydroxyethylsulfonamido)-2-(6-azaspiro[2.5]octan-6-yl)benzamide (271)

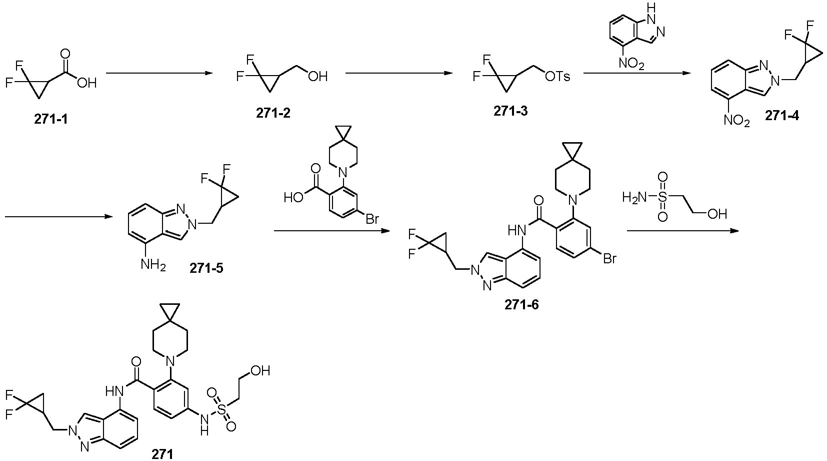

### Step 1: synthesis of compound 271-2

Compound 271-1 (2 g, 16.38 mmol) was dissolved in tetrahydrofuran (15 mL), to which lithium aluminium hydride (0.93 g, 24.57 mmol) was added at 0°C. The reaction mixture was stirred at room temperature overnight. After the reaction was completed, the reaction solution was quenched with ice sodium hydroxide solution (1 mL) and filtered, and the filter cake was washed with dichloromethane (60 × 3 mL). The filtrate was dried over Na₂SO₄, filtered and concentrated (the water bath temperature was maintained at 25°C) to obtain compound 271-2.

### Step 2: synthesis of compound 271-3

Compound 271-2 (1.60 g, 14.80 mmol) and 4-methylbenzenesulfonyl chloride (3.39 g, 17.76 mmol) were dissolved in dichloromethane (20 mL), to which triethylamine (4.49 g, 44.41 mmol) was added. The reaction mixture was stirred at room temperature for 3 hours. After the reaction was completed, the reaction solution was diluted with water (100 mL), and extracted with dichloromethane (30×3 mL). The organic layer was washed with brine (30×3 mL), dried over Na₂SO₄, filtered and concentrated. The crude product was purified by column chromatography (PE:EA=100:0-98:2-95:5) to obtain compound 271-3.

### Step 3: synthesis of compound 271-4

Compound 271-3 (1.29 g, 4.90 mmol) and 4-nitro-1H-indazole (800 mg, 4.90 mmol) were dissolved in N,N-dimethylformamide (12 mL), to which cesium carbonate (3.19 g, 9.80 mmol) was added. The reaction mixture was stirred in an oil bath at 75°C for 1.5 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The residue was purified by preparative HPLC to obtain compound 271-4.

### Step 4: synthesis of compound 271-5

Compound 271-4 (205 mg, 0.81 mmol) was dissolved in ethanol (5 mL) and water (5 mL), to which iron dust (226.04 mg, 4.05 mmol) and ammonium chloride (216.53 mg, 4.05 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The residue was purified by column chromatography (PE:EA=100:0-90:10-80:20) to obtain compound 271-5.

### Step 5: synthesis of compound 271-6

Compound 271-5 (77 mg, 0.34 mmol) and 4-bromo-2-(6-azaspiro[2.5]octan-6-yl)benzoic acid (107 mg, 0.34 mmol) were dissolved in acetonitrile (4 mL), to which azabenzotriazolyltetramethyluronium hexafluorophosphate (157.29 mg, 0.41 mmol) and N,N-diisopropylethylamine (89.16 mg, 0.69 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified by column chromatography (PE:EA=100:0-80:20) to obtain compound 271-6.

### Step 6: synthesis of compound 271

Compound 271-6 (130 mg, 0.25 mmol) and 2-hydroxyethane-1-sulfonamide (126.26 mg, 1.01 mmol) were dissolved in 1,4-dioxane (0.5 mL), to which a catalyst (CAS: 1599466-83-7; 46.44 mg, 0.05 mmol) and cesium carbonate (164.36 mg, 0.50 mmol) were added. The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 271. MS (ESI, pos.ion) m/z: 560.2 [M+1]⁺.

¹H NMR (400 MHz, DMSO-*d*₆) *δ* = 11.19 - 11.12 (m, 1H), 8.68 - 8.61 (m, 1H), 7.80 - 7.76 (m, 1H), 7.75 - 7.72 (m, 1H), 7.40 - 7.35 (m, 1H), 7.28 - 7.23 (m, 1H), 7.17 - 7.14 (m, 1H), 7.04 - 7.00 (m, 1H), 4.62 - 4.53 (m, 2H), 3.80 - 3.75 (m, 2H), 3.37 - 3.33 (m, 2H), 3.01 (br s, 4H), 2.44 - 2.36 (m, 1H), 1.79 - 1.68 (m, 1H), 1.59 - 1.51 (m, 1H), 1.45 - 1.39 (m, 4H), 0.27 - 0.24 (m, 4H).

### Example 272: synthesis of N-(2-(2-hydroxy-2-methylpropyl)-2H-indazol-4-yl)-4-((2-hydroxyethyl)sulfonamido)-2-(6-azaspiro[2.5]oct-6-yl)benzamide

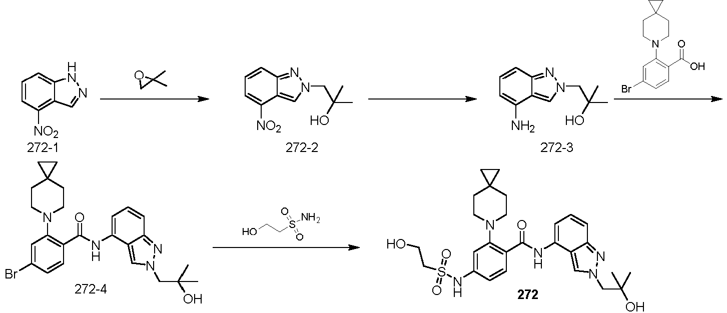

### Step 1: synthesis of compound 272-2

Compound 272-1 (500 mg, 3.06 mmol) and 2,2-dimethyloxirane (405 mg, 5.62 mmol) were dissolved in DMF (5 mL), cesium carbonate (1997.18 mg, 6.13 mmol) was added, and the mixture was stirred at 50°C for 3 hours. The reaction solution was poured into water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 272-2.

### Step 2: synthesis of compound 272-3

Compound 272-2 (120 mg, 0.51 mmol), iron dust (85.45 mg, 1.53 mmol) and ammonium chloride (81.86 mg, 1.53 mmol) were dissolved in tetrahydrofuran (2 mL), methanol (1 mL) and water (1 mL), and the reaction mixture was stirred at 60°C overnight. The reaction solution was filtered and the filtrate was concentrated under reduced pressure to obtain compound 272-3.

### Step 3: synthesis of compound 272-4

Compound 272-3 (50 mg, 0.24 mmol), HATU (182.52 mg, 0.48 mmol) and 2-(6-azaspiro[2.5]oct-6-yl)-4-bromobenzoic acid (90.67 mg, 0.29 mmol) were dissolved in DMF (1 mL). DIEA (93.06 mg, 0.72 mmol) was added, and the mixture was reacted at room temperature for 12 hours. The reaction solution was poured into water, extracted three times with ethyl acetate (10 ml), washed twice with sodium chloride aqueous solution (30 mL), the organic phase was dried over anhydrous sodium sulfate, concentrated under reduced pressure, and the crude product was purified by silica gel column chromatography (EA/PE = 0-50%) to obtain compound 272-4.

### Step 4: synthesis of compound 272

Compound 272-4 (120 mg, 0.24 mmol), 2-hydroxyethane-1-sulfonamide (30.19 mg, 0.24 mmol), tBuXPhosPdG4 (19.19 mg, 0.02 mmol) and cesium carbonate (117.90 mg, 0.36 mmol) were dissolved in dioxane (1 mL), and the mixture was stirred at 100°C for 12 hours. The reaction solution was poured into water, extracted three times with ethyl acetate (10 ml), washed twice with sodium chloride aqueous solution (30 mL), the organic phase was dried over anhydrous sodium sulfate, concentrated under reduced pressure, and the crude product was purified by silica gel column chromatography (EA/PE = 0-50%) to obtain compound 272. MS (ESI, pos.ion) m/z: 542.2 [M+1]⁺.

¹H NMR (DMSO-*d₆*, 400 MHz) *δ* 11.12 (s, 1H), 10.07 (br d, *J* = 0.8 Hz, 1H), 8.58 (s, 1H), 7.78 (d, *J* = 8.3 Hz, 2H), 7.35(d, *J* = 8.5 Hz, 1H), 7.22 (t, *J* = 7.9 Hz, 1H), 7.15 (d, *J =* 1.9 Hz, 1H), 7.01 (dd, *J =* 2.0, 8.4 Hz, 1H), 4.97 (br d, *J =* 8.0 Hz,1H), 4.84 (s, 1H), 4.33 (s, 2H), 3.77 (br t, *J* = 6.6 Hz, 2H), 3.37 - 3.33 (m, 2H), 3.00 (br t, *J=* 4.8 Hz, 4H), 1.42 (br s, 4H),1.12 (s, 6H), 0.24 (s, 4H).

### Example 277: synthesis of 4-((2-hydroxyethyl)sulfonamido)-2-(6-azaspiro[2.5]oct-6-yl)-N-(2-((trifluoromethyl)sulfonyl)-2H-indazol-4-yl)benzamide (277)

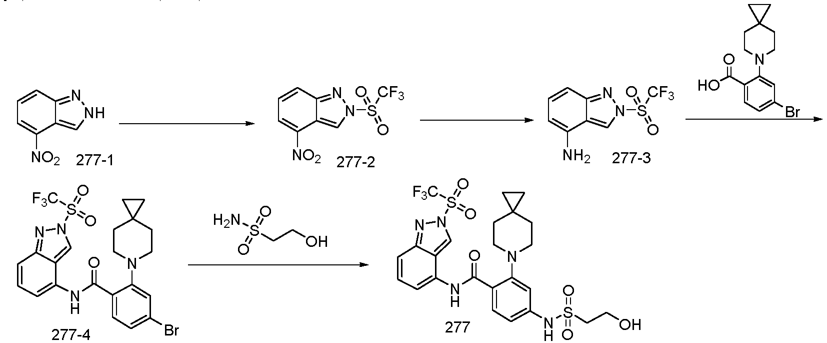

### Step 1: synthesis of compound 277-2

Compound 277-1 (100 mg, 0.61 mmol) was dissolved in THF (4 ml), sodium hydride (24 mg, 0.61 mmol) was added at 0°C, and after stirring at room temperature for half an hour, N-phenylbis(trifluoromethanesulfonimide) (382.47 mg, 0.92 mmol) was added. The mixture was reacted at room temperature for 2 hours under N₂ protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 277-2.

### Step 2: synthesis of compound 277-3

Compound 277-2 (80 mg, 0.27 mmol), iron dust (90 mg, 1.63 mmol), and ammonium chloride (29 mg, 0.54 mmol) were dissolved in ethanol (3 mL) and H₂O (1 mL), and the reaction mixture was allowed to react at 70°C for 1 hour under nitrogen protection. The reaction solution was diluted with EA (10 mL), then washed with saturated NaCl solution (10 mL), dried over anhydrous Na₂SO₄, and concentrated under reduced pressure. The crude product was purified by silica gel column chromatography (PE/EA= 1/0 to 8/1) to obtain compound 277-3.

### Step 3: synthesis of compound 277-4

Compound 277-3 (40 mg, 0.15 mmol), 4-bromo-2-(6-azaspiro[2.5]oct-6-yl)benzoic acid (51 mg, 0.17 mmol), TCFH (46 mg, 0.17 mmol) and *N,N-*diisopropylethylamine (0.05 ml, 0.3 mmol) were dissolved in THF (3 ml), and the reaction mixture was reacted at room temperature for 2 hours under nitrogen protection. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 277-4.

### Step 4: synthesis of compound 277

Compound 277-4 (40 mg, 0.07 mmol), 2-hydroxyethylsulfonamide (45 mg, 0.36 mmol), cesium carbonate (70 mg, 0.22 mmol) and a catalyst (CAS: 1599466-89-3; 6 mg, 0.01 mmol) were dissolved in dioxane (3 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na2SO4, and concentrated under reduced pressure. The crude product was purified by silica gel column chromatography (PE/EA= 1/0 to 1/1) to obtain compound 277. MS (ESI, pos.ion) m/z: 602.1 [M+1]+.

¹HNMR (DMSO-d6 ) δ: 11.39-11.46 (m, 1H), 10.13-10.22 (m, 1H), 7.86-7.94 (m, 1H), 7.62-7.73 (m, 1H), 7.34-7.45 (m, 1H), 7.16-7.24 (m, 2H), 6.93-7.10 (m, 2H), 3.70-3.82 (m, 2H), 3.32-3.41 (m, 2H), 2.93-3.07 (m, 4H), 1.48-1.62 (m, 4H), 0.28-0.39 (m, 4H).

### Example 279: synthesis of N-(1-(2-hydroxy-2-methylpropyl)-1H-indol-4-yl)-4-((2-hydroxyethyl)sulfonamido)-2-(6-azaspiro[2.5]oct-6-yl)benzamide (279)

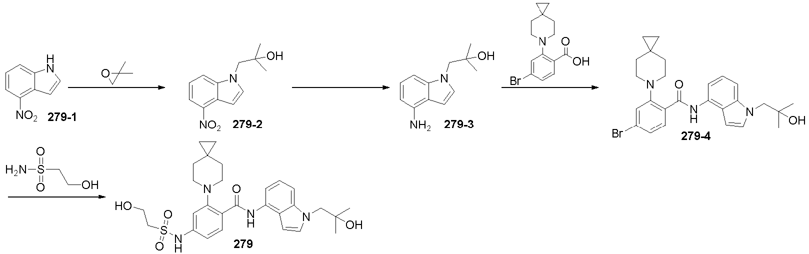

### Step 1: synthesis of compound 279-2

Compound 279-1 (1 g, 6.17 mmol) and sodium hydride (0.30 g, 12.33 mmol) were dissolved in N,N-dimethylformamide (16 mL), to which 2,2-dimethyloxirane (1.33 g, 18.50 mmol) was added. The reaction mixture was stirred at room temperature overnight. After the reaction was completed, the reaction solution was diluted with water (100 mL), and extracted with ethyl acetate (30×3 mL). The organic layer was washed with brine (30×3 mL), dried over Na₂SO₄, filtered and concentrated. The residue was purified by column chromatography (PE:EA=100:0-90:10-80:20) to obtain compound 279-2.

### Step 2: synthesis of compound 279-3

Compound 279-2 (595 mg, 2.54 mmol) was dissolved in ethanol (6 mL) and water (6 mL), to which iron dust (709 mg, 12.70 mmol) and potassium carbonate (679 mg, 12.70 mmol) were added. The reaction mixture was stirred in an oil bath at 80°C for 2 hours. The reaction solution was filtered and the filtrate was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-80:20) to obtain compound 279-3.

### Step 3: synthesis of compound 279-4

Compound 279-3 (140 mg, 0.69 mmol) and 2-(6-azaspiro[2.5]octan-6-yl)-4-bromobenzoic acid (234 mg, 0.75 mmol) were dissolved in N,N-dimethylformamide (4 mL), to which azabenzotriazolyltetramethyluronium hexafluorophosphate (313 mg, 0.82 mmol) and N,N-diisopropylethylamine (177 mg, 1.37 mmol) were added. The reaction mixture was stirred at room temperature for 1 hour. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-80:20) to obtain compound 279-4.

### Step 4: synthesis of compound 279

Compound 279-4 (105 mg, 0.21 mmol) and 2-hydroxyethane-1-sulfonamide (106 mg, 0.85 mmol) were dissolved in 1,4-dioxane (3 mL), to which a catalyst (CAS: 1599466-83-7; 19.47 mg, 0.02 mmol) and cesium carbonate (138 mg, 0.42 mmol) were added. The reaction mixture was stirred in an oil bath at 95°C for 2 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 279. MS (ESI, pos.ion) m/z: 541.2 [M+1]+.

¹H NMR (400 MHz, DMSO-d₆) δ = 11.17 (br s, 1H), 7.88 (br t, *J=* 6.1 Hz, 2H), 7.44 - 7.28 (m, 2H), 7.20 (br s, 1H), 7.14 - 7.00 (m, 2H), 6.65 (br s, 1H), 4.67 (br s, 1H), 4.08 (br s, 2H), 3.77 (br s, 2H), 3.46 - 3.38 (m, 2H), 3.03 (br s, 4H), 1.47 (br s, 4H), 1.09 (br s, 6H), 0.27 (br s, 4H).

### Example 280: synthesis of 4-((2-hydroxyethyl)sulfonamido)-2-(6-azaspiro[2.5]oct-6-yl)-N-(1-(3,3,3-trifluoro-2,2-dimethylpropyl)-1H-indol-4-yl)benzamide (280)

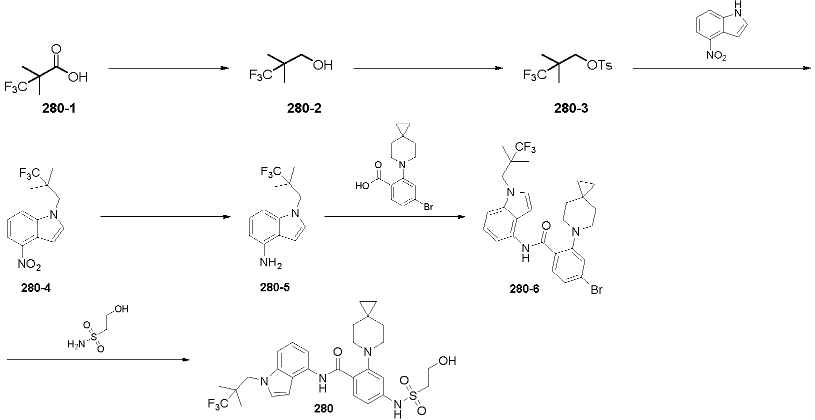

### Step 1: synthesis of compound 280-2

Compound 280-1 (960 mg, 6.15 mmol) was dissolved in ultra-dry tetrahydrofuran (10 mL), to which LiAlH₄ (350 mg, 9.22 mmol) was slowly added at 0°C, and the mixture was stirred at 25°C for 1 hour. The reaction solution was quenched with ice water, extracted with DCM, dried over anhydrous Na₂SO₄, and concentrated under reduced pressure to obtain compound 280-2, which was used directly in the next step.

### Step 2: synthesis of compound 280-3

Compound 280-2 (870 mg, 6.12 mmol), and triethylamine (2.55 mL, 18.36 mmol) were dissolved in DCM (6 mL) and stirred at 0°C for 10 minutes, to which TsCl (1.17 g, 6.12 mmol) was slowly added at 0°C, and the mixture was reacted overnight at 25°C. The reaction solution was quenched with ice water, and extracted with DCM. The organic phase was washed with saturated NaCl solution, dried over anhydrous Na₂SO₄, and concentrated under reduced pressure. The crude product was purified by silica gel column chromatography (PE:EA=49:1) to obtain compound 280-3.

¹H NMR (400 MHz, CHLOROFORM-d) δ = 7.71 (d, *J* = 8.0 Hz, 2H), 7.29 (d, *J* = 8.0 Hz, 2H), 3.85 (s, 2H), 2.38 (s, 3H), 1.06 (s, 6H).

### Step 3: synthesis of compound 280-4

4-Nitroindole (202 mg, 1.25 mmol), and cesium carbonate (814 mg, 2.50 mmol) were dissolved in DMF (5 mL), to which compound 280-3 (370 mg, 1.25 mmol) was added, and the reaction mixture was reacted at 120°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 280-4. MS (ESI, pos.ion) m/z:287.0 [M+1]⁺.

### Step 4: synthesis of compound 280-5

Compound 280-4 (216 mg, 0.75 mmol), and ammonium chloride (202 mg, 3.77 mmol) were dissolved in ethanol/water (3/1 mL), to which iron dust (211 mg, 3.77 mmol) was added, and the reaction mixture was allowed to react at 80°C for 2 hours. The reaction solution was quenched with ice water, and extracted with EA. The organic phase was washed with saturated NaCl solution, dried over anhydrous Na₂SO₄, and concentrated under reduced pressure and dried to obtain compound 280-5. MS (ESI, pos.ion) m/z: 257.0 [M+1]⁺.

### Step 5: synthesis of compound 280-6

Compound 280-5 (164 mg, 0.64 mmol), 4-bromo-2-(6-azaspiro[2.5]octan-6-yl)benzoic acid (238 mg, 0.77 mmol), and HATU (268 mg, 0.70 mmol) were dissolved in DMF (3 mL), to which DIEA (0.38 mL, 2.56 mmol) was added, and the reaction mixture was allowed to react at 25°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 280-6.

### Step 6: synthesis of compound 280

Compound 280-6 (320 mg, 0.58 mmol), 2-hydroxyethanesulfonamide (292 mg, 2.33 mmol), cesium carbonate (570 mg, 1.75 mmol) and the fourth-generation palladium catalyst t-BuXphos (CAS: 1599466-89-3; 47 mg, 0.06 mmol) were dissolved in dioxane (5 mL), and the reaction mixture was reacted at 100°C for 1 h under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 280. MS (ESI, pos.ion) m/z:593.3 [M+1]⁺.

¹H NMR (400 MHz, DMSO-d6) δ = 11.15 (s, 1H), 10.09 (br s, 1H), 7.93 (d, *J* = 7.8 Hz, 1H), 7.90 - 7.82 (m, 1H), 7.38 (d, *J* = 3.1 Hz, 1H), 7.32 - 7.28 (m, 1H), 7.20 - 7.12 (m, 2H), 7.06 - 7.01 (m, 1H), 6.75 (d, *J* = 3.1 Hz, 1H), 4.96 (br s, 1H), 4.37 (s, 2H), 3.77 (t, *J* = 6.5 Hz, 2H), 3.35 (t, *J* = 6.6 Hz, 2H), 3.02 (br s, 4H), 1.45 (br s, 4H), 1.15 (s, 6H), 0.26 (s, 4H).

### Example 281: synthesis of 4-((2-hydroxyethyl)sulfonamido)-N-(1-neopentyl-1H-indol-4-yl)-2-(6-azaspiro[2.5]octan-6-yl)benzamide (281)

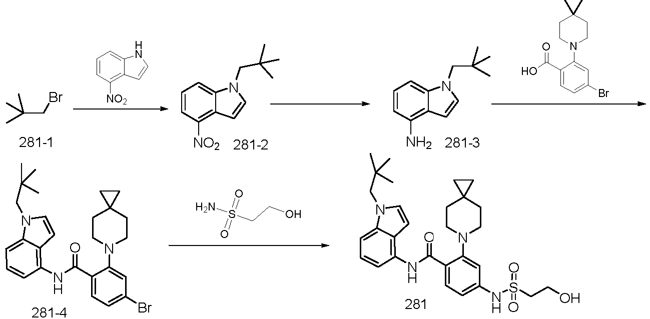

### Step 1: synthesis of compound 281-2

4-Nitroindole (1000 mg, 6.17 mmol), cesium carbonate (6000 mg, 18.51 mmol) and compound 281-1 (6.9 ml, 7.4 mmol) were dissolved in DMF (10 mL), and the reaction mixture was reacted at 120°C for 12 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 281-2.

### Step 2: synthesis of compound 281-3

Compound 281-2 (400 mg, 1.7 mmol), iron dust (579.3 mg, 10.3 mmol), and ammonium chloride (184.3 mg, 3.44 mmol) were dissolved in ethanol (9 mL) and H₂O (3 mL), and the reaction mixture was allowed to react at 70°C for 5 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 281-3.

### Step 3: synthesis of compound 281-4

Compound 281-3 (290 mg, 1.43 mmol), 4-bromo-2-(6-azaspiro[2.5]oct-6-yl)benzoic acid (488 mg, 1.57 mmol), HATU (600 mg, 1.57 mmol) and *N,N-*diisopropylethylamine (0.95 ml, 5.72 mmol) were dissolved in DMF (5 ml), and the reaction mixture was reacted at room temperature for 1 hour under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 281-4.

### Step 4: synthesis of compound 281

Compound 281-4 (300 mg, 0.6 mmol), 2-hydroxyethylsulfonamide (356 mg, 3.00 mmol), cesium carbonate (586 mg, 1.8 mmol) and a catalyst (CAS: 1599466-89-3; 48 mg, 0.06 mmol) were dissolved in dioxane (5 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 281. MS (ESI, pos.ion) m/z:539.3 [M+1]⁺.

¹HNMR (DMSO-d6 ) δ: 11.13-11.17 (m, 1H), 10.01-10.16 (m, 1H), 7.85-7.93 (m, 2H), 7.28-7.35 (m, 2H), 7.18-7.21 (m, 1H), 7.08-7.14 (m, 1H), 7.02-7.07 (m, 1H), 6.69 (s, 1H), 4.96 (br s, 1H), 3.97-4.03 (m, 2H), 3.74-3.82 (m, 2H), 3.36 (t, *J* = 6.6 Hz, 2H), 2.98-3.06 (m, 4H), 1.40-1.50 (m, 4H), 0.91-0.99 (m, 9H), 0.23-0.30 (m, 4H).

### Example 283: synthesis of N-(1-((1-fluorocyclopropyl)methyl)-1H-indol-4-yl)-4-((2-hydroxyethyl)sulfonamido)-2-(6-azaspiro[2.5]oct-6-yl)benzamide (283)

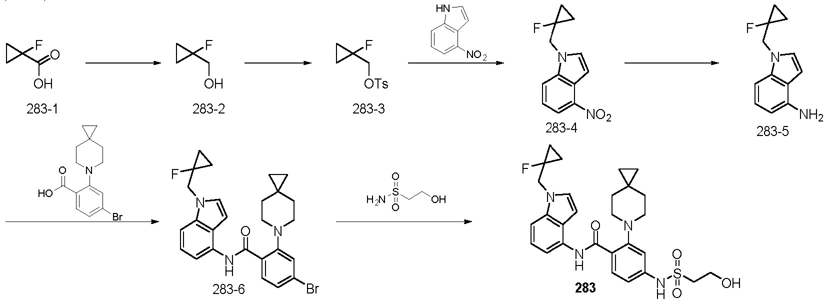

### Step 1: synthesis of compound 283-2

Compound 283-1 (1.5 g, 14.4 mmol) was dissolved in tetrahydrofuran (20 mL). Lithium aluminium hydride (1.64 mg, 43.27 mmol) was added under ice bath conditions, and the reaction mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 283-2.

### Step 2: synthesis of compound 283-3

Compound 283-2 (1.1 g, 12.22 mmol) and triethylamine (3.7 g, 36.67 mmol) were dissolved in dichloromethane (10 mL), paratoluensulfonyl chloride (3.02 g, 15.89 mmol) was added under ice bath conditions, and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 283-3.

### Step 3: synthesis of compound 283-4

Compound 283-3 (300 mg, 1.23 mmol), cesium carbonate (1.2 g, 3.69 mmol) and 4-nitro-1H-indole (199 mg, 1.23 mmol) were dissolved in DMF (10 mL), and the mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 283-4.

### Step 4: synthesis of compound 283-5

Compound 283-4 (230 mg, 0.983 mmol), iron dust (550 mg, 9.83 mmol) and ammonium chloride (521 mg, 9.83 mmol) were dissolved in ethanol (10 mL), and stirred at 80°C for 3 hours. The reaction solution was filtered and concentrated under reduced pressure to obtain compound 283-5.

### Step 5: synthesis of compound 283-6

Compound 283-5 (180 mg, 0.882 mmol), HATU (503 mg, 1.32 mmol), DIEA (341 mg, 2.65 mmol), and 4-bromo-2-(6-azaspiro[2.5]oct-6-yl)benzoic acid (273 mg, 0.882 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 283-6.

### Step 6: synthesis of compound 283

Compound 283-6 (200 mg, 0.404 mmol), cesium carbonate (395 mg, 1.21 mmol), a catalyst (CAS: 1599466-89-3; 33 mg, 0.04 mmol) and 2-hydroxyethane-1-sulfonamide (101 mg, 0.808 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 283. MS (ESI, pos.ion) m/z: 541.22 [M+1]⁺.

¹H NMR (CHLOROFORM-d, 400 MHz) δ 11.8-12.0 (m, 1H), 8.2-8.3 (m, 1H), 8.0-8.0 (m, 1H), 7.7-7.7 (m, 1H), 7.37 (s, 1H),7.2-7.3 (m, 3H), 6.9-7.0 (m, 1H), 6.58 (d, 1H, *J* = 3.0 Hz), 4.4-4.5 (m, 2H), 4.0-4.1 (m, 2H), 3.0-3.1 (m, 6H), 1.3-1.5 (m, 4H),1.1-1.2 (m, 2H), 0.8-0.9 (m, 2H), 0.25 (s, 4H).

### Example 284: synthesis of 4-((2-hydroxyethyl)sulfonamido)-2-(6-azaspiro[2.5]octan-6-yl)-N-(1-(trifluoromethyl)cyclopropylmethyl)-1H-indol-4-yl)benzamide (284)

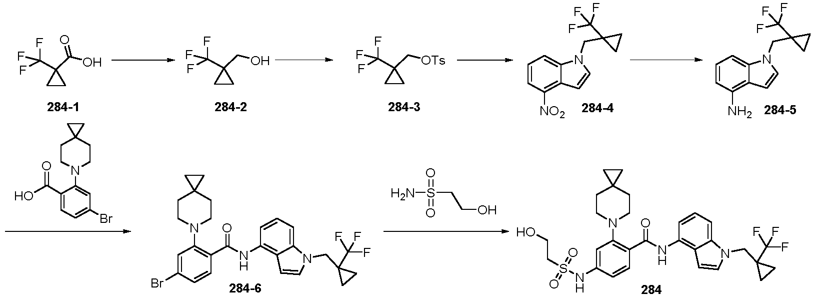

### Step 1: synthesis of compound 284-2

Compound 284-1 (1 g, 6.49 mmol) was dissolved in tetrahydrofuran (10 mL), to which lithium aluminium hydride (0.49 g, 12.98 mmol) was added at 0°C. The reaction mixture was stirred at room temperature for 2 hours. After the reaction was completed, the reaction solution was quenched with ice sodium hydroxide solution (0.5 mL) and filtered, and the filter cake was washed with dichloromethane (60 × 3 mL). The filtrate was dried over Na₂SO₄, filtered and concentrated to obtain compound 284-2.

### Step 2: synthesis of compound 284-3

Compound 284-2 (200 mg, 1.43 mmol) and 4-methylbenzenesulfonyl chloride (300 mg, 1.57 mmol) were dissolved in dichloromethane (3 mL), to which triethylamine (434 g, 4.29 mmol) was added. The reaction mixture was stirred at room temperature for 3 hours. After the reaction was completed, the reaction solution was diluted with water (60 mL), and extracted with dichloromethane (30×3 mL). The organic layer was washed with brine (30 × 3 mL), dried over Na₂SO₄, filtered and concentrated. The residue was purified by column chromatography (PE:EA=100:0-98:2) to obtain compound 284-3.

### Step 3: synthesis of compound 284-4

Compound 284-3 (200 mg, 0.68 mmol) and 4-nitro-1H-indole (121 mg, 0.75 mmol) were dissolved in N,N-dimethylformamide (6 mL), to which cesium carbonate (443 mg, 1.36 mmol) was added. The reaction mixture was stirred in an oil bath at 75°C for 2 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-80:20) to obtain compound 284-4.

### Step 4: synthesis of compound 284-5

Compound 284-4 (120 mg, 0.42 mmol) was dissolved in ethanol (3 mL) and water (3 mL), to which iron dust (118 mg, 2.11 mmol) and ammonium chloride (113 mg, 2.11 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30 × 3 mL), dried over Na₂SO₄, filtered and concentrated. The residue was purified by column chromatography (PE:EA=100:0-90:10-80:20) to obtain compound 284-5.

### Step 5: synthesis of compound 284-6

Compound 284-5 (53 mg, 0.21 mmol) and 4-bromo-2-(6-azaspiro[2.5]octan-6-yl)benzoic acid (71 mg, 0.23 mmol) were dissolved in N,N-dimethylformamide (3 mL), to which azabenzotriazolyltetramethyluronium hexafluorophosphate (95 mg, 0.25 mmol) and N,N-diisopropylethylamine (54 mg, 0.42 mmol) were added. The reaction mixture was stirred at room temperature for 1 hour. After the reaction was completed, the reaction solution was diluted with water (60 mL), and extracted with ethyl acetate (30×3 mL). The organic layer was washed with brine (30 × 3 mL), dried over Na₂SO₄, filtered and concentrated. The crude product was purified by column chromatography (PE:EA=100:0-90:10-80:20) to obtain compound 284-6.

### Step 6: synthesis of compound 284

Compound 284-6 (73 mg, 0.13 mmol) and 2-hydroxyethane-1-sulfonamide (67 mg, 0.53 mmol) were dissolved in 1,4-dioxane (3 mL), to which a catalyst (CAS: 1599466-83-7; 12.30 mg, 0.01 mmol) and cesium carbonate (87 mg, 0.27 mmol) were added. The reaction mixture was stirred in an oil bath at 95°C for 2 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified by HPLC to obtain compound 284. MS (ESI, pos.ion) m/z: 591.2 [M+1]⁺

1H NMR (400 MHz, DMSO-d6) δ = 7.99-7.92 (m, 1H), 7.66-7.59 (m, 1H), 7.37-7.33 (m, 1H), 7.29 (s, 1H), 7.30-7.26 (m, 1H), 7.26-7.22 (m, 1H), 7.19-7.10 (m, 2H), 6.60-6.56 (m, 1H), 4.45- 4.37 (m, 2H), 3.81-3.75 (m, 2H), 3.39-3.31 (m, 2H), 3.17-3.05 (m, 4H), 1.52-1.37 (m, 4H), 1.03-0.95 (m, 4H), 0.31-0.24 (m, 4H).

### Example 285: synthesis of 4-((2-hydroxyethyl)sulfonamido)-N-(1-((1-methylcyclopropyl)methyl)-1H-indol-4-yl)-2-(6-azaspiro[2.5]oct-6-yl)benzamide (285)

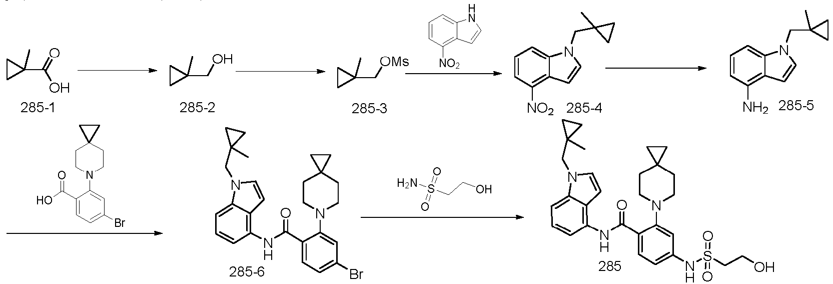

### Step 1: synthesis of compound 285-2

Compound 285-1 (1000 mg, 10 mmol) was dissolved in THF (10 mL) and lithium aluminium hydride (759 mg, 19.58 mmol) was slowly added at 0°C under nitrogen protection, and the reaction mixture was reacted at room temperature for 2 hours. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure to obtain compound 285-2 which was used directly in the next reaction.

### Step 2: synthesis of compound 285-3

Compound 285-2 (500 mg, 5.81 mmol) and methanesulfonic anhydride (1213 mg, 6.971 mmol) were dissolved in DCM (6 mL), followed by triethylamine (1762 mg, 17.42 mmol). The mixture was reacted at room temperature for 12 hours. The reaction solution was quenched by adding water (5 ml), and liquid separation was performed with DCM (20 mL). The organic phase was washed with saturated sodium chloride aqueous solution (10 mL), dried over anhydrous Na₂SO₄, and concentrated under reduced pressure to obtain compound 285-3 which was used directly in the next reaction.

### Step 3: synthesis of compound 285-4

4-Nitroindole (710 mg, 4.38 mmol), cesium carbonate (4764 mg, 14.61 mmol) and compound 285-3 (800 mg, 4.87 mmol) were dissolved in DMF (10 mL), and the reaction mixture was reacted at 90°C for 12 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 285-4.

### Step 4: synthesis of compound 285-5

Compound 285-4 (30 mg, 0.13 mmol), iron dust (42 mg, 0.78 mmol), and ammonium chloride (14 mg, 0.26 mmol) were dissolved in ethanol (3 mL) and H₂O (1 mL), and the reaction mixture was allowed to react at 70°C for 5 hours under nitrogen protection. The reaction solution was quenched by adding water (2 ml), and liquid separation was performed with EA (10 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 285-5.

### Step 5: synthesis of compound 285-6

Compound 285-5 (20 mg, 0.1 mmol), 4-bromo-2-(6-azaspiro[2.5]oct-6-yl)benzoic acid (34 mg, 0.11 mmol), HATU (42 mg, 0.11 mmol) and N,N-diisopropylethylamine (0.07 ml, 0.4 mmol) were dissolved in DMF (2 ml), and the reaction mixture was reacted at room temperature for 2 hours under nitrogen protection. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 285-6.

### Step 6: synthesis of compound 285

Compound 285-6 (35 mg, 0.07 mmol), 2-hydroxyethylsulfonamide (45 mg, 0.36 mmol), cesium carbonate (69 mg, 0.21 mmol) and a catalyst (CAS: 1599466-89-3; 6 mg, 0.01 mmol) were dissolved in dioxane (5 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 285. MS (ESI, pos.ion) m/z: 537.2 [M+1]⁺.

¹HNMR (DMSO-d6 ) δ: 11.15-11.20 (m, 1H), 7.90-7.94 (m, 1H), 7.85-7.89 (m, 1H), 7.42-7.45 (m, 1H), 7.31 (d, *J= 8.1* Hz, 1H), 7.17-7.20 (m, 1H), 7.08-7.14 (m, 1H), 7.00-7.04 (m, 1H), 6.67-6.71 (m, 1H), 4.04-4.09 (m, 2H), 3.74-3.80 (m, 2H), 3.33-3.36 (m, 2H), 3.00-3.06 (m, 4H), 1.42-1.51 (m, 4H), 0.89-0.93 (m, 3H), 0.64-0.68 (m, 2H), 0.34-0.39 (m, 2H), 0.24-0.28 (m, 4H).

### Example 286: synthesis of N-(1-((2,2-difluorocyclopropyl)methyl)-1H-indol-4-yl)-4-((2-hydroxyethyl)sulfonamido)-2-(6-azaspiro[2.5]oct-6-yl)benzamide (286)

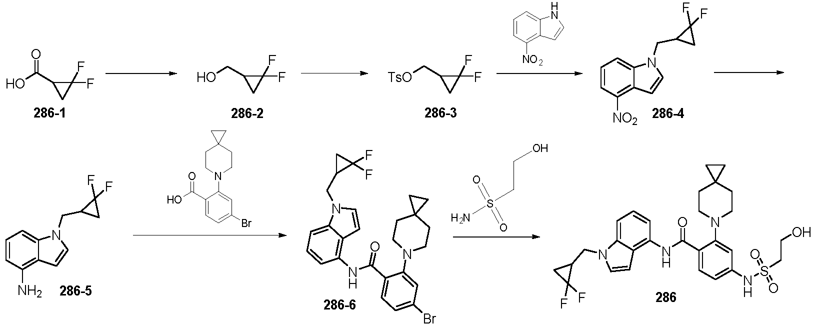

### Step 1: synthesis of compound 286-2

Compound 286-1 (2 g, 16.38 mmol) was dissolved in ultra-dry tetrahydrofuran (15 mL), to which LiAlH₄ (930 mg, 24.58 mmol) was slowly added at 0°C, and the mixture was stirred at 25°C for 1 hour. The reaction solution was quenched with ice water, extracted with DCM, dried over anhydrous Na₂SO₄, and concentrated under reduced pressure to obtain compound 286-2, which was used directly in the next step.

### Step 2: synthesis of compound 286-3

Compound 286-2 (1.77 g, 16.38 mmol), and triethylamine (0.69 mL, 49.13 mmol) were dissolved in DCM (10 mL) and stirred at 0°C for 10 minutes, to which TsCl (3.12 g, 16.38 mmol) was slowly added at 0°C, and the mixture was reacted for 1 hour at 25°C. The reaction solution was quenched with ice water, and extracted with DCM. The organic phase was washed with saturated NaCl solution, dried over anhydrous Na₂SO₄, and concentrated under reduced pressure. The crude product was purified by silica gel column chromatography (PE:EA=49:1) to obtain compound 286-3.

¹H NMR (400 MHz, CHLOROFORM-d) δ = 7.82 (d, *J* = 8.3 Hz, 2H), 7.38 (d, *J= 8.0* Hz, 2H), 4.14 - 4.09 (m, 2H), 2.47 (s, 3H), 2.02 - 1.88 (m, 1H), 1.60 - 1.50 (m, 1H), 1.24 - 1.11 (m, 1H).

### Step 3: synthesis of compound 286-4.

4-Nitroindole (1.63 g, 10.03 mmol), and cesium carbonate (6.53 g, 20.06 mmol) were dissolved in DMF (15 mL), to which compound 286-3 (2.63 g, 10.03 mmol) was added, and the reaction mixture was reacted at 90°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 286-4. MS (ESI, pos.ion) m/z: 253.0 [M+1]⁺.

### Step 4: synthesis of compound 286-5

Compound 286-4 (2.16 g, 8.56 mmol), and ammonium chloride (2.29 g, 42.82 mmol) were dissolved in ethanol/water (15/5 mL), to which iron dust (2.39 g, 42.82 mmol) was added, and the reaction mixture was allowed to react at 80°C for 1 hour. The reaction solution was quenched with ice water, and extracted with EA. The organic phase was washed with saturated NaCl solution, dried over anhydrous Na₂SO₄, and concentrated under reduced pressure and dried to obtain compound 286-5. MS (ESI, pos.ion) m/z: 223.0 [M+1]⁺.

### Step 5: synthesis of compound 286-6.

Compound 286-5 (300 mg, 1.35 mmol), 4-bromo-2-(6-azaspiro[2.5]octan-6-yl)benzoic acid (464 mg, 1.48 mmol), and HATU (565 mg, 1.48 mmol) were dissolved in DMF (5 mL), to which DIEA (0.89 mL, 5.40 mmol) was added, and the reaction mixture was allowed to react at 25°C for 1 hour. The reaction solution was quenched with ice water, and extracted with EA. The organic phase was washed with saturated NaCl solution, dried over anhydrous Na₂SO₄, and concentrated under reduced pressure. The residue was purified by silica gel column chromatography (PE:EA=10:1) to obtain compound 286-6.

### Step 6: synthesis of compound 286.

Compound 286-6 (240 mg, 0.47 mmol), 2-hydroxyethanesulfonamide (292 mg, 2.33 mmol), cesium carbonate (456 mg, 1.40 mmol) and a catalyst (CAS: 1621274-19-8; 90 mg, 0.09 mmol) were dissolved in dioxane (5 mL), and the reaction mixture was reacted at 95°C for 3 h under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 286. MS (ESI, pos.ion) m/z:559.2 [M+1]⁺.

¹H NMR (400 MHz, DMSO-d6) δ = 11.18 (s, 1H), 10.07 (br s, 1H), 7.95 (d, J = 7.6 Hz, 1H), 7.91 - 7.84 (m, 1H), 7.43 (d, *J =* 3.0 Hz, 1H), 7.33 (d, *J* = 8.3 Hz, 1H), 7.20 (s, 1H), 7.16 (t, *J* = 7.9 Hz, 1H), 7.11 - 6.97 (m, 1H), 6.71 (d, *J* = 3.0 Hz, 1H), 5.10 - 4.78 (m, 1H), 4.33 (br d, *J= 7.1* Hz, 2H), 3.78 (t, *J =* 6.6 Hz, 2H), 3.35 (t, *J* = 6.6 Hz, 2H), 3.03 (br s, 4H), 2.34 - 2.22 (m, 1H), 1.68 (ddt, *J* = 4.6, 7.8, 11.9 Hz, 1H), 1.57 - 1.50 (m, 1H), 1.50 - 1.43 (m, 4H), 0.27 (s, 4H).

### Example 287: synthesis of N-(1-(cyclopropylmethyl)-1H-indazol-4-yl)-4-(2-hydroxyethylsulfonamido)-2-(6-azaspiro[2.5]octan-6-yl)benzamide (287)

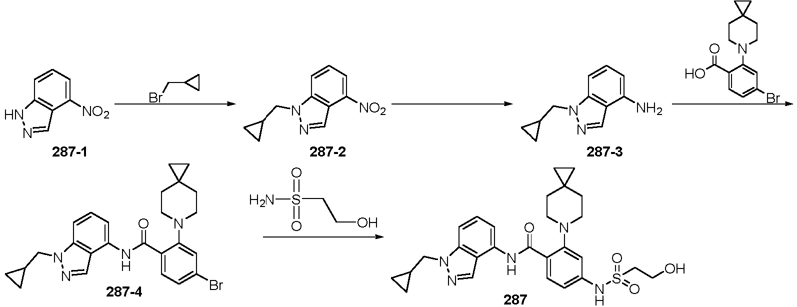

### Step 1: synthesis of compound 287-2

Compound 287-1 (1 g, 6.13 mmol) and (bromomethyl)cyclopropane (0.90 mL, 9.19 mmol) were dissolved in N,N-dimethylformamide (10 mL), to which cesium carbonate (3.99 g, 12.26 mmol) was added. The reaction mixture was stirred in an oil bath at 70°C for 1.5 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The residue was purified to obtain compound 287-2.

### Step 2: synthesis of compound 287-3

Compound 287-2 (300 mg, 1.38 mmol) was dissolved in ethanol (3 mL) and water (3 mL), to which iron dust (386 mg, 6.91 mmol) and ammonium chloride (369 mg, 6.91 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The residue was purified by column chromatography (PE:EA=100:0-90:10-80:20) to obtain compound 287-3.

### Step 3: synthesis of compound 287-4

Compound 287-3 (210 mg, 1.13 mmol) and 4-bromo-2-(6-azaspiro[2.5]octan-6-yl)benzoic acid (385 mg, 1.24 mmol) were dissolved in N,N-dimethylformamide (4 mL), to which azabenzotriazolyltetramethyluronium hexafluorophosphate (514 mg, 1.35 mmol) and N,N-diisopropylethylamine (291 mg, 2.25 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 287-4.

### Step 4: synthesis of compound 287

Compound 287-4 (150 mg, 0.31 mmol) and 2-hydroxyethane-1-sulfonamide (157 mg, 1.25 mmol) were dissolved in 1,4-dioxane (5 mL), to which a catalyst (CAS: 1599466-89-3; 25 mg, 0.03 mmol) and cesium carbonate (204 mg, 0.63 mmol) were added. The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 287. MS (ESI, pos.ion) m/z: 524.2 [M+1]⁺.

¹H NMR (400 MHz, DMSO-d6) δ = 11.15-11.09 (m, 1H), 8.67-8.62 (m, 1H), 7.82-7.76 (m, 2H), 7.37-7.32 (m, 1H), 7.25-7.19 (m, 1H), 7.17-7.13 (m, 1H), 7.04-6.99 (m, 1H), 4.32-4.26 (m, 2H), 3.81-3.74 (m, 2H), 3.37-3.33 (m, 2H), 3.06-2.97 (m, 4H), 1.49-1.36 (m, 5H), 0.63-0.56 (m, 2H), 0.48-0.42 (m, 2H), 0.28-0.22 (m, 4H).

### Example 288: synthesis of N-(7-cyano-1-(cyclopropylmethyl)-1H-indol-4-yl)-4-((2-hydroxyethyl)sulfonamido)-2-(6-azaspiro[2.5]oct-6-yl)benzamide (288)

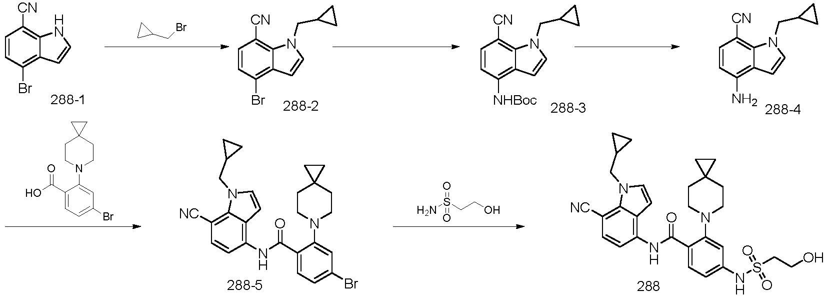

### Step 1: synthesis of compound 288-2

Compound 288-1 (200 mg, 0.909 mmol), potassium carbonate (376 mg, 2.73 mmol) and (bromomethyl)cyclopropane (134 mg, 1.00 mmol) were dissolved in DMSO (10 mL), and the mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 288-2.

### Step 2: synthesis of compound 288-3

Compound 288-2 (220 mg, 0.803 mmol), cesium carbonate (785 mg, 2.41 mmol), tBuXPhos Pd G4 (CAS: 1599466-89-3; 65 mg, 0.08 mmol) and tert-butoxycarbonyl amine (188 mg, 1.61 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 288-3.

### Step 3: synthesis of compound 288-4

Compound 288-3 (180 mg, 0.579 mmol) was dissolved in dichloromethane (10 ml), and hydrogen chloride-ethyl acetate solution (1.45 ml, 5.79 mmol) was added under ice bath conditions. The mixture was reacted at room temperature for 2 hours, a solid was precipitated and the reaction solution was concentrated to obtain compound 288-4.

### Step 4: synthesis of compound 288-5

Compound 288-4 (115 mg, 0.545 mmol), HATU (310 mg, 0.818 mmol), DIEA (211 mg, 1.63 mmol), and 4-bromo-2-(6-azaspiro[2.5]oct-6-yl)benzoic acid (168 mg, 0.545 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 288-5.

### Step 5: synthesis of compound 288

Compound 288-5 (120 mg, 0.404 mmol), cesium carbonate (233 mg, 0.717 mmol), a catalyst (CAS: 1599466-89-3; 19 mg, 0.023 mmol) and 2-hydroxyethane-1-sulfonamide (60 mg, 0.478 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 288. MS (ESI, pos.ion) m/z: 548.23 [M+1]⁺.

¹H NMR (CHLOROFORM-d, 400 MHz) δ 11.6-11.7 (m, 1H), 8.2-8.3 (m, 1H), 8.1-8.2 (m, 1H), 7.6-7.6 (m, 1H), 7.3-7.3 (m,2H), 7.0-7.0 (m, 1H), 6.9-7.0 (m, 1H), 6.7-6.7 (m, 1H), 4.4-4.4 (m, 2H), 4.16 (q, 2H, *J* = 4.8 Hz), 3.3-3.4 (m, 2H), 3.1-3.2 (m,4H), 2.5-2.6 (m, 1H), 1.56 (br s, 4H), 0.7-0.7 (m, 2H), 0.4-0.5 (m, 2H), 0.3-0.4 (m, 4H).

### Example 294: synthesis of N-(4-(cyclopropylmethyl)-5-fluoro-3,4-dihydro-2H-benzo[b][1,4]oxazin-8-yl)-4-((2-hydroxyethyl)sulfonamido)-2-(6-azaspiro[2.5]oct-6-yl)benzamide (294)

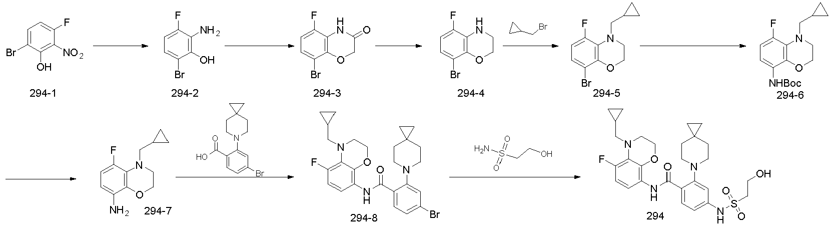

### Step 1: synthesis of compound 294-2

Compound 294-1 (1000 mg, 4.25 mmol), iron dust (1428 mg, 25.5 mmol), and ammonium chloride (459 mg, 8.5 mmol) were dissolved in ethanol (9 mL) and H₂O (3 mL), and the reaction mixture was allowed to react at 70°C for 3 hours under nitrogen protection. The reaction solution was quenched by adding water (8 ml), and liquid separation was performed with EA (30 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 294-2.

### Step 2: synthesis of compound 294-3

Compound 294-2 (400 mg, 1.94 mmol) and potassium carbonate (939 mg, 3.5 mmol) were dissolved in 2-butanone (6 mL), followed by chloroacetyl chloride (328 mg, 2.91 mmol), and the reaction mixture was allowed to react at 80°C for 2 hours. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 294-3.

### Step 3: synthesis of compound 294-4

Compound 294-3 (340 mg, 1.38 mmol) was dissolved in THF (6 mL), and BH₃-DMS (314 mg, 4.15 mmol) was slowly added at 0°C. The mixture was reacted at 70°C for 1 hour under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 294-4.

### Step 4: synthesis of compound 294-5

Compound 294-4 (250 mg, 1.08 mmol) was dissolved in DMF (10 mL) and sodium hydride (52 mg, 1.29 mmol) was slowly added at 0°C, and the reaction system was moved to room temperature and reacted for half an hour. Bromomethyl cyclopropane (174 mg, 1.29 mmol) was then added, and the mixture was reacted at 80°C for 2 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 294-5.

### Step 5: synthesis of compound 294-6

Compound 294-5 (100 mg, 0.35 mmol), tert-butyl carbamate (81 mg, 0.7 mmol), cesium carbonate (342 mg, 1.05 mmol) and a catalyst (CAS: 1599466-89-3; 28 mg, 0.03 mmol) were dissolved in dioxane (5 mL), and the reaction mixture was reacted at 90°C for 8 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 294-6.

### Step 6: synthesis of compound 294-7

Compound 294-6 (80 mg, 0.25 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (20 mL), and the organic phase was washed with saturated NaCl solution (10 mL), dried over anhydrous Na₂SO₄, and concentrated under reduced pressure to obtain compound 294-7 which was used directly in the next step without purification.

### Step 7: synthesis of compound 294-8

Compound 294-7 (40 mg, 0.18 mmol), 4-bromo-2-(6-azaspiro[2.5]oct-6-yl)benzoic acid (61 mg, 0.2 mmol), HATU (75 mg, 0.2 mmol) and *N,N-*diisopropylethylamine (0.12 ml, 0.72 mmol) were dissolved in DMF (4 ml), and the reaction mixture was reacted at room temperature for 2 hours under nitrogen protection. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 294-8.

### Step 8: synthesis of compound 294

Compound 294-8 (80 mg, 0.16 mmol), 2-hydroxyethylsulfonamide (97 mg, 0.78 mmol), cesium carbonate (152 mg, 0.47 mmol) and a catalyst (CAS: 1599466-89-3; 13 mg, 0.02 mmol) were dissolved in dioxane (5 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 294. MS (ESI, pos.ion) m/z: 559.2 [M+1]⁺.

¹HNMR (DMSO-d6 ) δ: 11.56-11.60 (m, 1H), 8.04-8.10 (m, 1H), 7.94-7.98 (m, 1H), 7.19-7.22 (m, 1H), 7.03-7.07 (m, 1H), 6.67-6.74 (m, 1H), 4.24-4.30 (m, 2H), 3.71-3.78 (m, 2H), 3.32-3.36 (m, 4H), 3.00-3.05 (m, 2H), 2.90-2.97 (m, 4H), 1.48-1.66 (m, 4H), 0.97-1.08 (m, 1H), 0.44-0.50 (m, 2H), 0.33-0.39 (m, 4H), 0.19-0.25 (m, 2H).

### Example 303: synthesis of N-(8,9-dihydro-6H-pyrido[3',2':4,5]pyrrolo[2,1-c][1,4]oxazin-4-yl)-4-((2-hydroxyethyl)sulfonamido)-2-(6-azaspiro[2.5]octan-6-yl)benzamide (303)

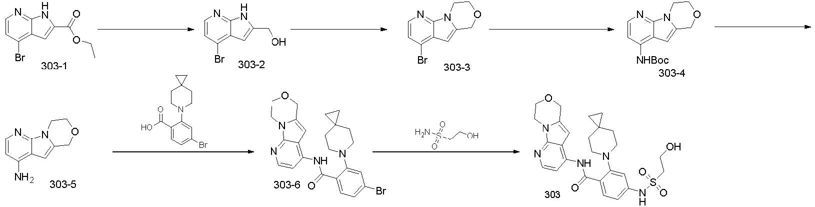

### Step 1: synthesis of compound 303-2

Compound 303-1 (900 mg, 3.53 mmol) was dissolved in THF and lithium aluminium hydride (7.87 ml, 11.81 mmol) was slowly added at 0°C under nitrogen protection. The reaction system was reacted at 0°C for 0.5 h. The reaction solution was quenched by adding water (1 ml) and 1N NaOH (1 ml), water (10 ml) was added, and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 303-2.

### Step 2: synthesis of compound 303-3

Compound 303-2 (580 mg, 2.55 mmol) and potassium hydroxide (358 mg, 6.39 mmol) were dissolved in dichloromethane, to which diphenyl(vinyl)sulfonium trifluoromethanesulfonate (1110 mg, 3.07 mmol) was slowly added dropwise at 0°C. The reaction system was moved to room temperature and reacted for 3 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 303-3.

### Step 3: synthesis of compound 303-4

Compound 303-3 (240 mg, 0.95 mmol), tert-butyl carbamate (222 mg, 1.90 mmol), cesium carbonate (927 mg, 2.84 mmol) and a catalyst (CAS: 1599466-85-9; 40 mg, 0.05 mmol) were dissolved in dioxane (5 mL), and the reaction mixture was reacted at 90°C for 2 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 4/1) to obtain compound 303-4.

### Step 4: synthesis of compound 303-5

Compound 303-4 (140 mg, 0.48 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 303-5 which was used directly in the next step without purification.

### Step 5: synthesis of compound 303-6

Compound 303-5 (50 mg, 0.26 mmol), 4-bromo-2-(6-azaspiro[2.5]oct-6-yl)benzoic acid (90 mg, 0.29 mmol), TCFH (81 mg, 0.29 mmol) and *N,N-*diisopropylethylamine (0.086 ml, 0.53 mmol) were dissolved in DMF (4 ml), and the reaction mixture was reacted at room temperature for 1 hour under nitrogen protection at 0°C. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 5/1) to obtain compound 303-6.

### Step 6: synthesis of compound 303

Compound 303-6 (35 mg, 0.07 mmol), 2-hydroxyethylsulfonamide (45 mg, 0.35 mmol), cesium carbonate (71 mg, 0.22 mmol) and a catalyst (CAS: 1599466-89-3; 6 mg, 0.01 mmol) were dissolved in dioxane (3 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 303. MS (ESI, pos.ion) m/z: 526.3 [M+1]⁺.

¹HNMR (DMSO-d6 ) δ: 11.24 (s, 1H), 8.12-8.16 (m, 1H), 7.99-8.04 (m, 1H), 7.79-7.83 (m, 1H), 7.14-7.18 (m, 1H), 7.02 (br d, *J=* 8.5 Hz, 1H), 6.55 (s, 1H), 4.96 (s, 2H), 4.15 (s, 4H), 3.77 (br d, *J* = 12.9 Hz, 2H), 3.35 (s, 2H), 3.00 (br s, 4H), 1.44 (br s, 4H), 0.28 (s, 4H).

### Example 304: synthesis of N-(3,4-dihydro-1H-benzo[4,5]imidazo[2,1-c][1,4]oxazin-9-yl)-4-((2-hydroxyethyl)sulfonamido)-2-(6-azaspiro[2.5]octan-6-yl)benzamide (304)

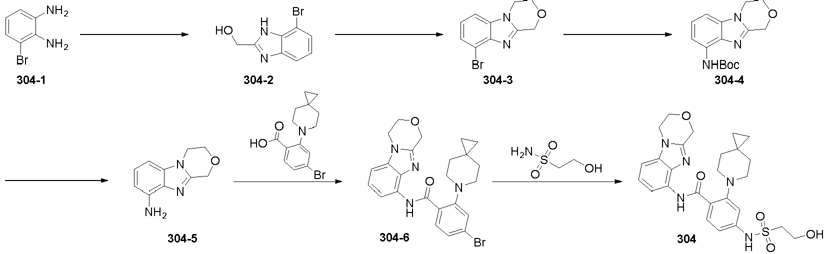

### Step 1: synthesis of compound 304-2

Compound 304-1 (1.5 g, 8.02 mmol) and 2-hydroxyacetic acid (0.96 mL, 16.04 mmol) were dissolved in 6 N hydrochloric acid (2 mL). The reaction mixture was stirred overnight in an oil bath at 100°C. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-95:5) to obtain compound 304-2.

### Step 2: synthesis of compound 304-3

Compound 304-2 (800 mg, 3.52 mmol) and (2-bromoethyl)diphenylsulfonium trifluoromethanesulfonate (3.12 g, 7.05 mmol) were dissolved in acetonitrile (4 mL) and water (2 mL), to which potassium hydroxide (790.77 mg, 14.09 mmol) was added. The reaction mixture was stirred at room temperature overnight. After the reaction was completed, the reaction solution was adjusted to be alkaline and then diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-90:10-80:20) to obtain compound 304-3.

### Step 3: synthesis of compound 304-4

Compound 304-3 (400 mg, 1.58 mmol) and tert-butyl carbamate (370.29 mg, 3.16 mmol) were dissolved in 1,4-dioxane (6 mL), to which a catalyst (CAS: 1599466-83-7; 73.17 mL, 0.08 mmol) and cesium carbonate (1.54 g, 4.74 mmol) were added. The reaction mixture was stirred in an oil bath at 95°C for 5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-90:10-80:20) to obtain compound 304-4.

### Step 4: synthesis of compound 304-5

Compound 304-4 (300 mg, 1.04 mmol) was dissolved in hydrogen chloride in 1,4-dioxane (6 mL). The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0; then DCM:MeOH=95:5) to obtain compound 304-5.

### Step 5: synthesis of compound 304-6

Compound 304-5 (120 mg, 0.63 mmol) and 4-bromo-2-(6-azaspiro[2.5]octan-6-yl)benzoic acid (196.72 mg, 0.63 mmol) were dissolved in acetonitrile (2 mL), to which tetramethyluronium hexafluorophosphate (355.88 mg, 1.27 mmol) and N-methyl imidazole (260.33 mg, 3.17 mmol) were added. The reaction mixture was stirred at room temperature for 2 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-90:10-80:20) to obtain compound 304-6.

### Step 6: synthesis of compound 304

Compound 304-6 (140 mg, 0.29 mmol) and 2-hydroxyethane-1-sulfonamide (145.58 mg, 1.16 mmol) were dissolved in 1,4-dioxane (3 mL), to which SPhos Pd G4 (23.10 mg, 0.03 mmol) and cesium carbonate (284.27 mg, 0.87 mmol) were added. The reaction mixture was stirred in an oil bath at 95°C for 2 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 304. MS (ESI, pos.ion) m/z: 526.2 [M+1]⁺.

¹H NMR (400 MHz, DMSO-d6) δ = 12.55 - 12.45 (m, 1H), 8.43 - 8.32 (m, 1H), 8.03 (br d, *J* = 8.5 Hz, 1H), 7.26 -7.17 (m, 3H), 7.07 (br d, *J* = 8.4 Hz, 1H), 5.03 - 4.92 (m, 2H), 4.30 - 4.17 (m, 4H), 3.81 - 3.72 (m, 2H), 3.48 - 3.36 (m, 2H), 3.04 - 2.94 (m, 4H), 1.97 - 1.46 (m, 4H), 0.39 - 0.28 (m, 4H).

### Example 305: synthesis of N-(6-fluoro-3,4-dihydro-1H-[1,4]oxazino[4,3-a]indol-9-yl)-4-((2-hydroxyethyl)sulfonamido)-2-(6-azaspiro[2.5]oct-6-yl)benzamide (305)

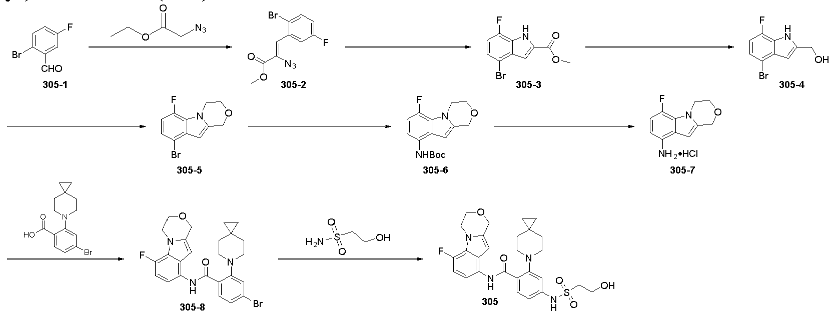

### Step 1: synthesis of compound 305-2

Sodium methoxide (5.4 mol/L, 11.0 mL, 59.11 mmol) was dissolved in methanol (40 mL), to which a solution of compound 305-1 (8.0 g, 39.41 mmol) in methanol (40 mL), and ethyl azidoacetate (6.8 mL, 59.11 mmol) were slowly added dropwise at -15°C, and the mixture was reacted overnight at 0°C. The reaction solution was slowly poured into ice water, and a solid was precipitated and filtered, and the solid was slurried with a small amount of methanol. The resulting slurry was filtered and dried to obtain compound 305-2.

¹H NMR (400 MHz, DMSO-d6) δ = 7.97 (dd, *J* = 3.0, 10.4 Hz, 1H), 7.76 (dd, *J=* 5.5, 8.9 Hz, 1H), 7.23 (dt, *J= 3.1,* 8.4 Hz, 1H), 7.01 (s, 1H), 3.89 (s, 3H).

### Step 2: synthesis of compound 305-3

Compound 305-2 (4.4 g, 14.66 mmol) was dissolved in o-dichlorobenzene (20 mL), and the mixture was reacted at 180°C for 5 h under nitrogen protection. The reaction solution was cooled to room temperature, and a solid was precipitated, and the reaction solution was filtered and dried to obtain compound 305-3.

¹H NMR (400 MHz, DMSO-d6) δ = 12.93 (br s, 1H), 7.32 - 7.26 (m, 1H), 7.13 - 7.05 (m, 2H), 3.90 (s, 3H).

### Step 3: synthesis of compound 305-4

Compound 305-3 (778 mg, 2.86 mmol) was dissolved in tetrahydrofuran (10 mL), to which LiAlH₄ (119 mg, 3.15 mmol) was slowly added at 0°C, and the mixture was reacted at 0°C for 40 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20:1) to obtain compound 305-4.

### Step 4: synthesis of compound 305-5

Compound 305-4 (510 mg, 2.09 mmol), and KOH (293 mg, 5.22 mmol) were dissolved in DCM (3 mL) and stirred at 0°C for 30 minutes, to which a solution of a catalyst (CAS: 247129-88-0; 909 mg, 2.51 mmol) in DCM (2 mL) was added, and stirring was continued at 0°C for 30 minutes. The reaction system was moved to room temperature and reacted overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 305-5.

¹H NMR (400 MHz, DMSO-d6) δ = 7.18 (dd, J = 3.9, 8.4 Hz, 1H), 6.88 (dd, *J* = 8.3, 12.1 Hz, 1H), 6.28 - 6.21 (m, 1H), 4.94 (s, 2H), 4.37 - 4.27 (m, 2H), 4.10 (t, *J* = 5.3 Hz, 2H).

### Step 5: synthesis of compound 305-6

Compound 305-5 (407 mg, 1.51 mmol), NH₂Boc (353 mg, 3.01 mmol), cesium carbonate (1.47 g, 4.52 mmol) and palladium catalyst (CAS: 1599466-83-7; 277 mg, 0.30 mmol) were dissolved in 1,4-dioxane (10 mL), and the reaction mixture was reacted at 90°C for 2 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1-10:1) to obtain compound 305-6.

### Step 6: synthesis of compound 305-7

Compound 305-6 (344 mg, 1.12 mmol) was dissolved in DCM (2 mL), to which HCl\EA (4 mL) was added, and the reaction mixture was allowed to react at 25°C overnight. The reaction solution was filtered and dried to obtain compound 305-7. MS (ESI, pos.ion) m/z:207.1 [M+1-HCl]⁺.

### Step 7: synthesis of compound 305-8

Compound 305-7 (222 mg, 0.92 mmol), 4-bromo-2-(6-azaspiro[2.5]octan-6-yl)benzoic acid (313 mg, 1.01 mmol), and HATU (384 mg, 1.01 mmol) were dissolved in DMF (3 mL), to which DIEA (0.61 mL, 3.67 mmol) was added, and the reaction mixture was allowed to react at 25°C for 1 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1-5:1) to obtain compound 305-8. MS (ESI, pos.ion) m/z:498.2/500.2 [M+1]⁺

### Step 8: synthesis of compound 305

Compound 305-8 (150 mg, 0.30 mmol), 2-hydroxyethanesulfonamide (188 mg, 1.50 mmol), cesium carbonate (294 mg, 0.90 mmol) and palladium catalyst (CAS: 1599466-89-3; 24 mg) were dissolved in 1,4-dioxane (5 mL), and the reaction mixture was reacted at 100°C for 3 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 305. MS (ESI, pos.ion) m/z: 543.3 [M+1]⁺.

¹H NMR (400 MHz, DMSO-d6) δ = 11.15 (s, 1H), 10.08 (br s, 1H), 7.85 (d, *J* = 8.4 Hz, 1H), 7.81 - 7.65 (m, 1H), 7.18 (s, 1H), 7.03 (dd, *J* = 1.6, 8.4 Hz, 1H), 6.98 - 6.82 (m, 1H), 6.49 (s, 1H), 4.93 (s, 2H), 4.33 (br s, 2H), 4.15 - 4.07 (m, 2H), 3.80 - 3.73 (m, 2H), 3.37 - 3.34 (m, 2H), 3.01 (br s, 4H), 1.44 (br s, 4H), 1.23 (br s, 4H).

### Example 306: synthesis of N-(3,4-dihydro-1H-pyridino[3',4':4,5]pyrrolo[2,1-c][1,4]oxazin-9-yl)-4-((2-hydroxyethyl)sulfonamido)-2-(6-azaspiro[2.5]oct-6-yl)benzamide (306)

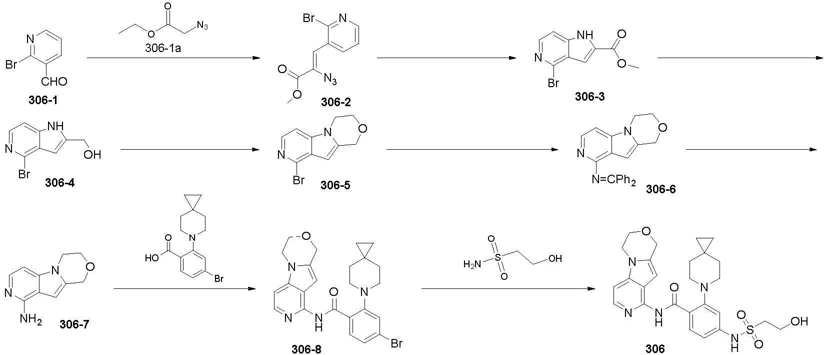

### Step 1: synthesis of compound 306-2.

Sodium methoxide (5.4 mol/L, 22.40 mL, 120.96 mmol) was dissolved in methanol (40 mL), to which a solution of compound 306-1 (15.0 g, 80.64 mmol) in methanol (40 mL), and compound 306-1a (13.82 mL, 120.96 mmol) were slowly added dropwise at -15°C, and the mixture was reacted for 6 hours at 0°C. The reaction solution was slowly poured into ice water, and a solid was precipitated and filtered, and the solid was slurried with a small amount of methanol. The resulting slurry was filtered and dried to obtain compound 306-2.

¹H NMR (400 MHz, DMSO-d6) δ = 8.50 - 8.42 (m, 1H), 8.40 - 8.27 (m, 1H), 7.56 - 7.48 (m, 1H), 7.01 - 6.92 (m, 1H), 3.89 (s, 3H).

### Step 2: synthesis of compound 306-3.

Compound 306-2 (8.36 g, 29.53 mmol) was dissolved in mesitylene (70 mL), and the mixture was reacted at 166°C for 1.5 h under nitrogen protection. The reaction solution was cooled to room temperature, and a solid was precipitated, and the reaction solution was filtered and dried to obtain compound 306-3.

### Step 3: synthesis of compound 306-4.

Compound 306-3 (2.97 g, 11.64 mmol) was dissolved in tetrahydrofuran (30 mL), to which LiAlH₄ (880 mg, 23.28 mmol) was slowly added at 0°C, and the mixture was reacted overnight at 25°C. The reaction solution was quenched with 10% aqueous sodium hydroxide solution and ice water, and extracted with EA. The organic phase was washed with saturated NaCl solution, dried over anhydrous Na₂SO₄, and concentrated under reduced pressure. The crude product was purified by silica gel column chromatography (DCM:MeOH=20:1) to obtain compound 306-4.

### Step 4: synthesis of compound 306-5.

Compound 306-4 (1.955 g, 8.61 mmol), and KOH (1.21 g, 21.53 mmol) were dissolved in DCM (10 mL) and stirred at 0°C for 30 minutes, to which a solution of a catalyst (CAS: 247129-88-0; 3.74 g, 10.33 mmol) in DCM (5 mL) was added, and stirring was continued at 0°C for 30 minutes. The reaction system was moved to room temperature and reacted overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=3:1) to obtain compound 306-5.

¹H NMR (400 MHz, DMSO-d6) δ = 7.97 (d, *J* = 5.6 Hz, 1H), 7.53 (d, *J* = 5.6 Hz, 1H), 6.27 (s, 1H), 4.96 (s, 2H), 4.19 - 4.12 (m, 4H).

### Step 5: synthesis of compound 306-6

Compound 306-5 (400 mg, 1.58 mmol), benzophenonimine (0.53 mL, 3.16 mmol), cesium carbonate (1.54 g, 4.74 mmol) and palladium catalyst (CAS: 1621274-19-8; 304 mg, 0.32 mmol) were dissolved in 1,4-dioxane (10 mL), and the reaction mixture was reacted at 100°C for 3 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1:2) to obtain compound 306-6.

MS (ESI, pos.ion) m/z:354.1 [M+1]⁺.

### Step 6: synthesis of compound 306-7

Compound 306-6 (272 mg, 0.77 mmol) was dissolved in DCM (1 mL), to which HCl\EA (5 mL) was added, and the reaction mixture was allowed to react at 25°C overnight. The reaction solution was filtered and dried to obtain compound 306-7. MS (ESI, pos.ion) m/z:190.1 [M+1-HCl]⁺.

### Step 7: synthesis of compound 306-8.

Compound 306-7 (120 mg, 0.53 mmol), 4-bromo-2-(6-azaspiro[2.5]octan-6-yl)benzoic acid (199 mg, 0.64 mmol), and HATU (223 mg, 0.59 mmol) were dissolved in DMF (2 mL), to which DIEA (0.35 mL, 2.13 mmol) was added, and the reaction mixture was allowed to react at 25°C for 1 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1:1) to obtain compound 306-8.

### Step 8: synthesis of compound 306.

Compound 306-8 (120 mg, 0.25 mmol), 2-hydroxyethanesulfonamide (156 mg, 1.25 mmol), cesium carbonate (244 mg, 0.75 mmol) and palladium catalyst (CAS: 1599466-87-1; 40 mg) were dissolved in 1,4-dioxane (3 mL), and the reaction mixture was reacted at 100°C for 2 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 306. MS (ESI, pos.ion) m/z:526.3 [M+1]⁺.

¹H NMR (400 MHz, DMSO-d6) δ = 12.36 (s, 1H), 10.27 - 10.04 (m, 1H), 8.05 - 7.98 (m, 2H), 7.31 - 7.28 (m, 1H), 7.28 - 7.24 (m, 1H), 7.10 (dd, *J* = 1.8, 8.5 Hz, 1H), 6.51 (s, 1H), 4.95 (s, 2H), 4.13 (s, 4H), 3.76 (br t, *J* = 6.4 Hz, 2H), 3.37 - 3.34 (m, 2H), 3.01 (br t, *J* = 4.9 Hz, 4H), 1.64 (br s, 4H), 0.36 (s, 4H).

### Example 308: synthesis of N-(6-fluoro-3,4-dihydro-1H-benzo[4,5]imidazo[2,1-c][1,4]oxazin-9-yl)-4-((2-hydroxyethyl)sulfonamido)-2-(6-azaspiro[2.5]octan-6-yl)benzamide (308)

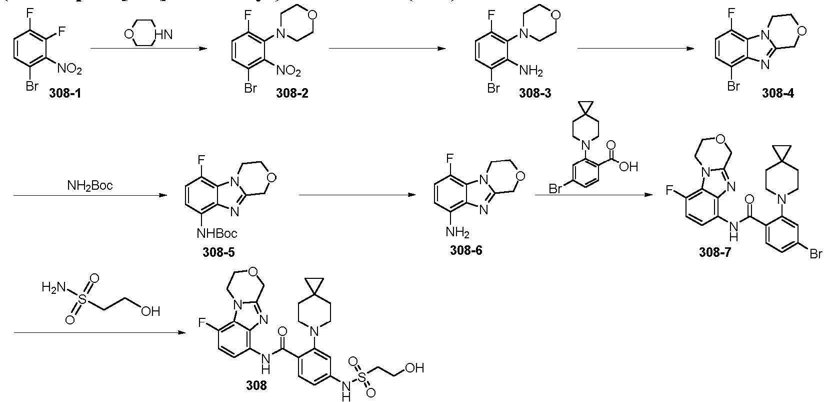

### Step 1: synthesis of compound 308-2

Compound 308-1 (1 g, 4.20 mmol) and morpholine (0.55 mL, 6.30 mmol) were dissolved in dimethyl sulfoxide (12 mL), to which potassium carbonate (1.16 g, 8.40 mmol) was added. The reaction mixture was stirred in an oil bath at 110°C for 2 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-95:5) to obtain compound 308-2.

### Step 2: synthesis of compound 308-3

Compound 308-2 (543 mg, 1.78 mmol) was dissolved in ethanol (5 mL) and water (5 mL), to which iron dust (497 mg, 8.90 mmol) and ammonium chloride (476 mg, 8.90 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30 × 3 mL), dried over Na₂SO₄, filtered and concentrated. The crude product was purified by column chromatography (PE:EA=100:0-90:10-80:20) to obtain compound 308-3.

### Step 3: synthesis of compound 308-4

Compound 308-3 (388 mg, 1.23 mmol) was dissolved in dichloromethane (3 mL), to which trifluoroacetic acid (1.05 mL, 14.10 mmol) and hydrogen peroxide (0.42 mL, 14.10 mmol) were added. The reaction mixture was stirred in an oil bath at 40°C for 24 hours. After the reaction was completed, the reaction solution was quenched with anhydrous sodium sulfite, and then diluted with water (30 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 308-4.

### Step 4: synthesis of compound 308-5

Compound 308-4 (210 mg, 0.77 mmol) and NH₂Boc (227 mg, 1.94 mmol) were dissolved in 1,4-dioxane (6 mL), to which a catalyst (CAS: 1621274-19-8; 200 mg, 0.23 mmol) and cesium carbonate (1.01 g, 3.10 mmol) were added. The reaction mixture was stirred in an oil bath at 95°C for 3 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 308-5.

### Step 5: synthesis of compound 308-6

Compound 308-5 (120 mg, 0.39 mmol) was dissolved in dichloromethane (3 mL), to which trifluoroacetic acid (0.29 mL, 3.90 mmol) was added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 308-6.

### Step 6: synthesis of compound 308-7

Compound 308-6 and 4-bromo-2-(6-azaspiro[2.5]octan-6-yl)benzoic acid (90 mg, 0.29 mmol) were dissolved in acetonitrile (3 mL), to which azabenzotriazolyltetramethyluronium hexafluorophosphate (132 mg, 0.35 mmol) and N,N-diisopropylethylamine (94 mg, 0.72 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 308-7.

### Step 7: synthesis of compound 308

Compound 308-7 (120 mg, 0.24 mmol) and 2-hydroxyethane-1-sulfonamide (120 mg, 0.96 mmol) were dissolved in 1,4-dioxane (3 mL), to which a catalyst (CAS: 1599466-83-7; 66 mg, 0.07 mmol) and cesium carbonate (157 mg, 0.48 mmol) were added. The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified to obtain compound 308. MS (ESI, pos.ion) m/z: 544.2 [M+1]+.

¹H NMR (400 MHz, DMSO-*d*₆) *δ* = 12.60-12.52 (m, 1H), 8.34-8.27 (m, 1H), 8.02-7.96 (m, 1H), 7.21-7.16 (m, 1H), 7.06-6.98 (m, 2H), 5.04-4.94 (m, 2H), 4.48-4.38 (m, 2H), 4.24-4.14 (m, 2H), 3.78-3.72 (m, 2H), 3.29 - 3.24 (m, 2H), 3.05-2.90 (m, 4H), 2.06-1.28 (m, 4H), 0.32 (s, 4H).

### Example 310: synthesis of N-(6-cyano-3,4-dihydro-1H-[1,4]oxazino[4,3-a]indol-9-yl)-4-((2-hydroxyethyl)sulfonamido)-2-(6-azaspiro[2.5]oct-6-yl)benzamide

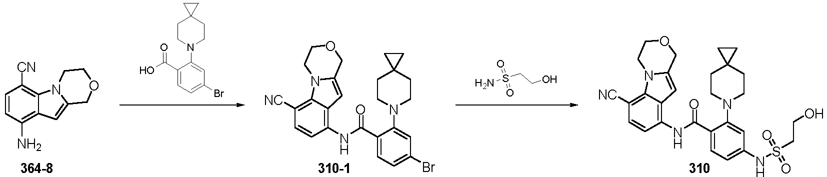

### Step 1: synthesis of compound 310-1.

Compound 364-8 (28 mg, 0.13 mmol), 4-bromo-2-(6-azaspiro[2.5]oct-6-yl)benzoic acid (49 mg, 0.16 mmol), and TCFH (55 mg, 0.20 mmol) were dissolved in THF (1 mL), to which DIEA (0.09 mL, 0.53 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 310-1.

### Step 2: synthesis of compound 310.

Compound 310-1 (35 mg, 0.07 mmol), 2-hydroxyethanesulfonamide (35 mg, 0.28 mmol), cesium carbonate (68 mg, 0.21 mmol) and the fourth-generation palladium catalyst (CAS: 1599466-89-3; 6 mg, 0.01 mmol) were dissolved in 1,4-dioxane (3 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 310. MS (ESI, pos.ion) m/z:550.2 [M+1]⁺.

¹H NMR (400 MHz, DMSO-d₆) δ = 11.30 (s, 1H), 10.14 (br s, 1H), 8.15 (d, *J* = 8.4 Hz, 1H), 7.82 (d, *J= 8.5* Hz, 1H), 7.60 (d, *J= 8.3* Hz, 1H), 7.18 (d, *J=* 1.8 Hz, 1H), 7.03 (dd, *J* = 1.9, 8.5 Hz, 1H), 6.73 (s, 1H), 4.96 (s, 2H), 4.48 (t, *J* = 5.3 Hz, 2H), 4.17 (t, *J* = 5.3 Hz, 2H), 3.77 (t, *J* = 6.5 Hz, 2H), 3.37 - 3.35 (m, 2H), 3.00 (br t, *J=* 4.8 Hz, 4H), 1.41 (br s, 4H), 0.27 (s, 4H).

### Example 311: synthesis of 4-((2-hydroxyethyl)sulfonamido)-N-(1-methyl-3,4-dihydro-1H-[1,4]oxazinyl[4,3-a]indol-9-yl)-2-(6-azaspiro[2.5]oct-6-yl)benzamide (311), compound 311-P1, and compound 311-P2

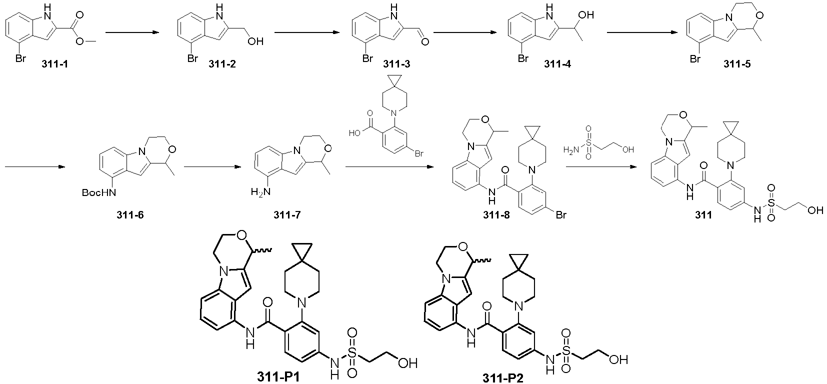

### Step 1: synthesis of compound 311-2

Compound 311-1 (5 g, 19.76 mmol) was dissolved in tetrahydrofuran (20 mL). DIBAL-H (39.5 ml, 59.29 mmol) was added under ice bath conditions, and the reaction mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 311-2.

### Step 2: synthesis of compound 311-3

Compound 311-2 (4.2 g, 19.11 mmol) was dissolved in dichloromethane (50 mL), manganese dioxide (16.2 g, 186.7 mmol) was added, and the reaction mixture was stirred overnight at room temperature. The reaction solution was filtered and the filtrate was concentrated under reduced pressure to obtain compound 311-3.

### Step 3: synthesis of compound 311-4

Compound 311-3 (1 g, 4.48 mmol) was dissolved in tetrahydrofuran (20 mL). Methyl magnesium bromide (5.98 ml, 8.97 mmol) was added under ice bath conditions, and the reaction mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 311-4.

### Step 4: synthesis of compound 311-5

Compound 311-4 (760 mg, 3.18 mmol) and potassium hydroxide (356 mg, 6.36 mmol) were dissolved in dichloromethane (10 ml), then diphenyl(vinyl)sulfonium trifluoromethanesulfonate (2.3 g, 6.36 mmol) was added under ice bath conditions. The reaction solution was stirred at 70°C for 16 hours. The reaction solution was poured into an appropriate amount of ice water, extracted three times with ethyl acetate (30 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 311-5.

### Step 5: synthesis of compound 311-6

Compound 311-5 (420 mg, 1.58 mmol), cesium carbonate (1.55 g, 4.75 mmol), a catalyst (CAS: 1599466-89-3; 128 mg, 0.158 mmol) and tert-butoxycarbonyl amine (370 mg, 3.17 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 311-6.

### Step 6: synthesis of compound 311-7

Compound 311-6 (180 mg, 0.596 mmol) was dissolved in dichloromethane (10 ml), and hydrogen chloride-ethyl acetate solution (1.49 ml, 5.96 mmol) was added under ice bath conditions. The mixture was reacted at room temperature for 2 hours, a solid was precipitated and the reaction solution was concentrated to obtain compound 311-7.

### Step 7: synthesis of compound 311-8

Compound 311-7 (100 mg, 0.495 mmol), HATU (282 mg, 0.743 mmol), DIEA (192 mg, 1.49 mmol), and 4-bromo-2-(6-azaspiro[2.5]oct-6-yl)benzoic acid (153 mg, 0.495 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 311-8.

### Step 8: synthesis of compound 311, compound 311-P1, and compound 311-P2

Compound 311-8 (150 mg, 0.304 mmol), cesium carbonate (298 mg, 0.913 mmol), a catalyst (CAS: 1599466-89-3; 25 mg, 0.030 mmol) and 2-hydroxyethane-1-sulfonamide (76 mg, 0.609 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 311.

¹H NMR (CHLOROFORM-d, 400 MHz) δ 11.56 (s, 1H), 8.1-8.2 (m, 1H), 8.0-8.0 (m, 1H), 7.3-7.3 (m, 1H), 7.23 (t, 1H, J= 7.9 Hz), 7.11 (d, 1H, J= 8.1 Hz), 6.96 (dd, 1H, *J* = 2.1, 8.4 Hz), 6.3-6.4 (m, 1H), 4.9-5.0 (m, 1H), 4.3-4.4 (m, 1H), 4.0-4.1(m, 5H), 3.3-3.3 (m, 2H), 3.1-3.2 (m, 4H), 1.64 (s, 2H), 1.60 (br s, 3H), 1.2-1.3 (m, 2H), 0.3-0.3 (m, 4H).

Compound 311 was subjected to resolution by preparative SFC to obtain compound 311-P1 and compound 311-P2; for purification by preparative SFC, the following conditions were used: column: ChiralPak AS, 250×30mm I.D., 10µm particles; mobile phase A: 0/30 CO₂/EtOH (0.1% NH₃H₂O); detector wavelength: 220 nm; flow rate: 150 mL/min.

Compound 311-P1 (41 mg) MS (ESI, pos.ion) m/z: 539.2 [M+1]⁺ retention time=1.859min;

¹H NMR (CHLOROFORM-d, 400 MHz) *δ* 11.56 (s, 1H), 8.1-8.2 (m, 1H), 8.0-8.0 (m, 1H), 7.3-7.3 (m, 1H), 7.23 (t, *J =* 7.9 Hz, 1H), 7.11 (d, *J =* 8.1 Hz, 1H), 6.96 (dd, *J* = 2.1, 8.4 Hz, 1H), 6.3-6.4 (m, 1H), 4.9-5.0 (m, 1H), 4.3-4.4 (m, 1H), 4.0-4.1(m, 5H), 3.3-3.3 (m, 2H), 3.1-3.2 (m, 4H), 1.64 (s, 2H), 1.60 (br s, 3H), 1.2-1.3 (m, 2H), 0.3-0.3 (m, 4H).

Compound 311-P2 (41 mg) MS (ESI, pos.ion) m/z: 539.2 [M+1]⁺; retention time = 2.323 min;

¹H NMR (CHLOROFORM-d, 400 MHz) *δ* 11.56 (s, 1H), 8.1-8.2 (m, 1H), 8.0-8.0 (m, 1H), 7.3-7.3 (m, 1H), 7.23 (t, *J =* 7.9 Hz, 1H), 7.11 (d, *J =* 8.1 Hz, 1H), 6.96 (dd, *J* = 2.1, 8.4 Hz, 1H), 6.3-6.4 (m, 1H), 4.9-5.0 (m, 1H), 4.3-4.4 (m, 1H), 4.0-4.1(m, 5H), 3.3-3.3 (m, 2H), 3.1-3.2 (m, 4H), 1.64 (s, 2H), 1.60 (br s, 3H), 1.2-1.3 (m, 2H), 0.3-0.3 (m, 4H).

### Example 312: synthesis of N-(1,3-dimethyl-3,4-dihydro-1H-[1,4] oxazino [4,3-a] indol-9-yl)-4-((2-hydroxyethyl)sulfonamido)-2-(6-azaspiro[2.5]oct-6-yl)benzamide (312)

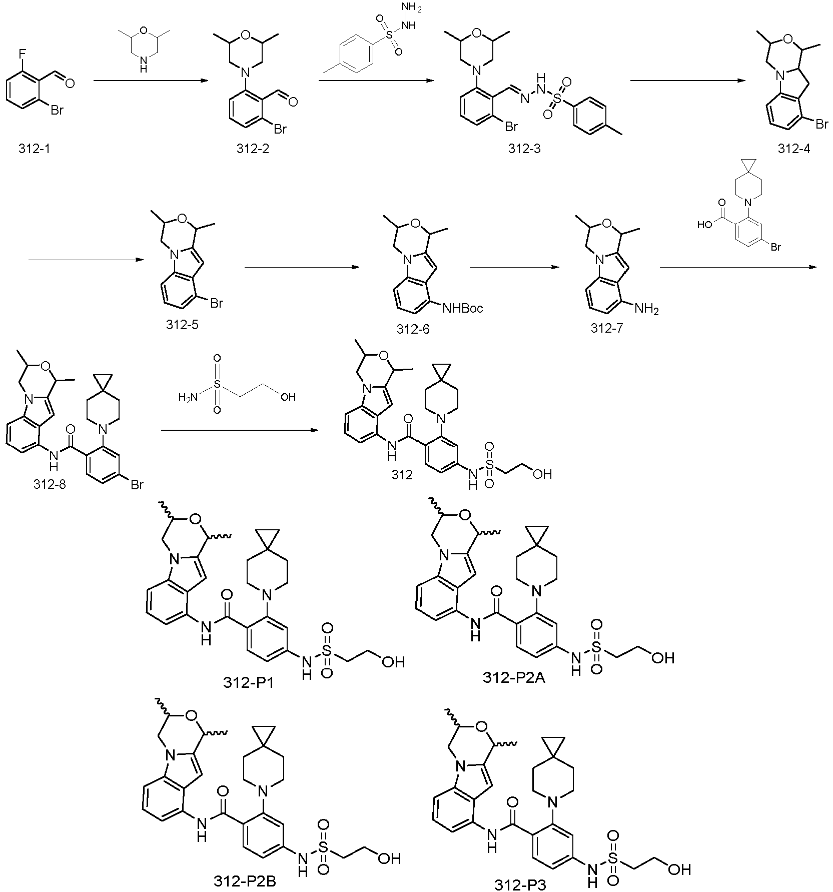

### Step 1: synthesis of compound 312-2

Compound 312-1 (3 g, 14.85 mmol), potassium carbonate (6.15 g, 44.55 mmol) and 2,6-dimethyl morpholine (2.05 g, 17.8 mmol) were dissolved in DMSO (10 mL), and the mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 312-2.

### Step 2: synthesis of compound 312-3

Compound 312-2 (1.2 g, 4.04 mmol) and 4-methylbenzenesulfonyl hydrazide (827 mg, 4.44 mmol) were dissolved in toluene (10 mL) and stirred at room temperature for 10 minutes to obtain the reaction solution containing compound 312-3, which was used directly in the next reaction without purification.

### Step 3: synthesis of compound 312-4

The reaction solution from the previous step was added to sodium hydride (194 mg, 4.85 mmol) under ice bath conditions and stirred at 135°C for 10 minutes under microwave irradiation. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 312-4.

### Step 4: synthesis of compound 312-5

Compound 312-4 (1 g, 19.11 mmol) was dissolved in dichloromethane (50 mL), manganese dioxide (3.1 g, 35.58 mmol) was added, and the reaction mixture was stirred overnight at room temperature. The reaction solution was filtered and the filtrate was concentrated under reduced pressure to obtain compound 312-5.

### Step 5: synthesis of compound 312-6

Compound 312-5 (700 mg, 2.51 mmol), cesium carbonate (2.45 g, 7.53 mmol), a catalyst (CAS: 1599466-89-3; 203 mg, 0.25 mmol) and tert-butoxycarbonyl amine (587 mg, 5.02 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 312-6.

### Step 6: synthesis of compound 312-7

Compound 312-6 (650 mg, 2.06 mmol) was dissolved in dichloromethane (10 ml), and hydrogen chloride-ethyl acetate solution (5.15 ml, 20.6 mmol) was added under ice bath conditions. The mixture was reacted at room temperature for 2 hours, a solid was precipitated and the reaction solution was concentrated to obtain compound 312-7.

### Step 7: synthesis of compound 312-8

Compound 312-7 (360 mg, 1.67 mmol), HATU (950 mg, 2.5 mmol), DIEA (645 mg, 5.0 mmol), and 4-bromo-2-(6-azaspiro[2.5]oct-6-yl)benzoic acid (515 mg, 1.67 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 312-8.

### Step 8: synthesis of compound 312, compound 312-P1, compound 312-P2A, compound 312-P2B, and compound 312-P3

Compound 312-8 (350 mg, 0.404 mmol), cesium carbonate (673 mg, 2.07 mmol), a catalyst (CAS: 1599466-89-3; 56 mg, 0.069 mmol) and 2-hydroxyethane-1-sulfonamide (172 mg, 1.38 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 312 (120 mg). MS (ESI, pos.ion) m/z: 553.24 [M+1]⁺.

¹H NMR (DMSO-d6, 400 MHz) δ 11.1-11.2 (m, 1H), 10.08 (br s, 1H), 8.0-8.0 (m, 1H), 7.9-7.9 (m, 1H), 7.2-7.2 (m, 1H), 7.1-7.2(m, 2H), 7.0-7.1 (m, 1H), 6.5-6.5 (m, 1H), 5.00 (br s, 1H), 4.2-4.3 (m, 1H), 4.1-4.2 (m, 1H), 3.7-3.8 (m, 2H), 3.58 (t, 1H, *J = 11.3* Hz), 3.3-3.4 (m, 2H), 3.0-3.1 (m, 4H), 1.5-1.6 (m, 3H), 1.4-1.5 (m, 4H), 1.4-1.4 (m, 1H), 1.3-1.4 (m, 2H), 0.2-0.3 (m, 4H).

Compound 312 was subjected to resolution by preparative SFC to obtain compound 283-P1 (45 mg), compound 283-P2A (5 mg), compound 283-P2B (3 mg) and compound 283-P3 (42 mg); for purification by preparative SFC, the following conditions were used: column: ChiralCel OD, 250×30mm I.D., 10µm particles; mobile phase A: 65/35 CO₂/EtOH(0.1% NH₃H₂O); detector wavelength: 220 nm; flow rate: 150 mL/min.

Compound 312-P1 MS (ESI, pos.ion) m/z: 553.2 [M+1]⁺; retention time = 2.331 min;
¹H NMR (DMSO-d6, 400 MHz) *δ* 11.18 (s, 1H), 10.08 (br s, 1H), 8.02 (d, 1H, *J* = 7.3 Hz), 7.89 (d, *J =* 8.5 Hz, 1H), 7.2-7.2 (m,1H), 7.1-7.2 (m, 2H), 7.04 (dd, *J* = 1.9, 8.5 Hz, 1H), 6.53 (s, 1H), 4.97 (q, *J* = 6.3 Hz, 2H), 4.27 (dd, *J* = 3.3, 11.7 Hz, 1H), 4.17(ddd, *J* = 3.6, 6.3, 10.3 Hz, 1H), 3.77 (t, *J* = 6.6 Hz, 2H), 3.58 (t, *J* = 11.3 Hz, 1H), 3.3-3.4 (m, 2H), 3.0-3.1 (m, 4H), 1.55 (d, *J=* 6.4 Hz, 3H), 1.5-1.5 (m, 4H), 1.3-1.4 (m, 3H), 0.2-0.3 (m, 4H).

Compound 312-P2A MS (ESI, pos.ion) m/z: 553.2 [M+1]⁺; retention time = 2.637 min;
¹H NMR (DMSO-d6, 400 MHz) *δ* 11.18 (s, 1H), 10.08 (br s, 1H), 8.02 (d, 1H, *J* = 7.3 Hz), 7.89 (d, *J =* 8.5 Hz, 1H), 7.2-7.2 (m,1H), 7.1-7.2 (m, 2H), 7.04 (dd, *J* = 1.9, 8.5 Hz, 1H), 6.53 (s, 1H), 4.97 (q, *J* = 6.3 Hz, 2H), 4.27 (dd, *J* = 3.3, 11.7 Hz, 1H), 4.17(ddd, *J* = 3.6, 6.3, 10.3 Hz, 1H), 3.77 (t, *J* = 6.6 Hz, 2H), 3.58 (t, *J* = 11.3 Hz, 1H), 3.3-3.4 (m, 2H), 3.0-3.1 (m, 4H), 1.55 (d, *J=* 6.4 Hz, 3H), 1.5-1.5 (m, 4H), 1.3-1.4 (m, 3H), 0.2-0.3 (m, 4H).

Compound 312-P2B MS (ESI, pos.ion) m/z: 553.2 [M+1]⁺; retention time = 2.683 min;
¹H NMR (DMSO-d6, 400 MHz) *δ* 11.18 (s, 1H), 10.08 (br s, 1H), 8.02 (d, 1H, *J* = 7.3 Hz), 7.89 (d, *J =* 8.5 Hz, 1H), 7.2-7.2 (m,1H), 7.1-7.2 (m, 2H), 7.04 (dd, *J* = 1.9, 8.5 Hz, 1H), 6.53 (s, 1H), 4.97 (q, *J* = 6.3 Hz, 2H), 4.27 (dd, *J* = 3.3, 11.7 Hz, 1H), 4.17(ddd, *J* = 3.6, 6.3, 10.3 Hz, 1H), 3.77 (t, *J* = 6.6 Hz, 2H), 3.58 (t, *J* = 11.3 Hz, 1H), 3.3-3.4 (m, 2H), 3.0-3.1 (m, 4H), 1.55 (d, *J=* 6.4 Hz, 3H), 1.5-1.5 (m, 4H), 1.3-1.4 (m, 3H), 0.2-0.3 (m, 4H).

Compound 312-P3 MS (ESI, pos.ion) m/z: 553.2 [M+1]⁺; retention time = 3.739 min;
¹H NMR (DMSO-d6, 400 MHz) *δ* 11.18 (s, 1H), 10.08 (br s, 1H), 8.02 (d, 1H, *J* = 7.3 Hz), 7.89 (d, *J =* 8.5 Hz, 1H), 7.2-7.2 (m,1H), 7.1-7.2 (m, 2H), 7.04 (dd, *J* = 1.9, 8.5 Hz, 1H), 6.53 (s, 1H), 4.97 (q, *J* = 6.3 Hz, 2H), 4.27 (dd, *J* = 3.3, 11.7 Hz, 1H), 4.17(ddd, *J* = 3.6, 6.3, 10.3 Hz, 1H), 3.77 (t, *J* = 6.6 Hz, 2H), 3.58 (t, *J* = 11.3 Hz, 1H), 3.3-3.4 (m, 2H), 3.0-3.1 (m, 4H), 1.55 (d, *J=* 6.4 Hz, 3H), 1.5-1.5 (m, 4H), 1.3-1.4 (m, 3H), 0.2-0.3 (m, 4H).

### Example 319: synthesis of 2-(4-difluoromethylene)piperidin-1-yl-N-(3,4-dihydro-1H-[1,4]oxazino[4,3-a]indol-9-yl)-4-((2-hydroxyethyl)sulfonamide) benzamide (319)

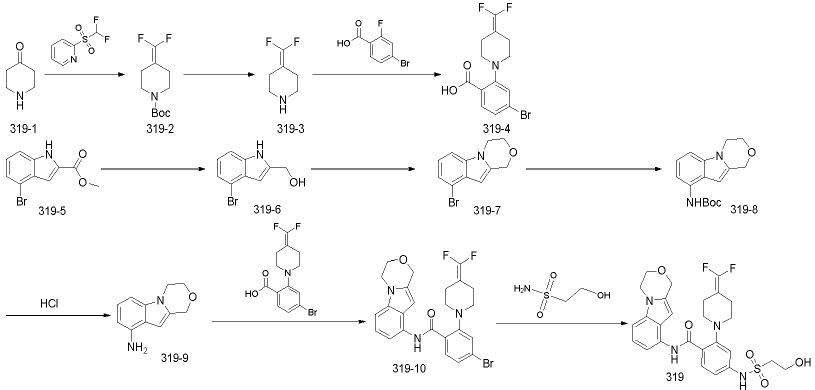

### Step 1: synthesis of compound 319-2

Compound 319-1 (4000 mg, 20.08 mmol) was dissolved in DMF (10 mL), and a solution of difluoromethyl(2-pyridyl)sulfone (3100 mg, 16.06 mmol) and potassium tert-butoxide (3380 mg, 30.11 mmol) in DMF (30 mL) was added portionwise at -40°C, and the mixture was stirred at -40°C for 15 min. Then 10 ml of saturated ammonium chloride and 26 ml of 3M hydrochloric acid were added to the reaction solution. The reaction mixture was slowly warmed to room temperature. The reaction was completed as monitored by TLC. The reaction solution was quenched by adding water (20 ml), and liquid separation was performed with EA (60 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 30/1) to obtain compound 319-2.

### Step 2: synthesis of compound 319-3

Compound 319-2 (3250 mg, 13.93 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 319-3 which was used directly in the next step without purification.

### Step 3: synthesis of compound 319-4

2-Fluoro-4-bromobenzoic acid (600 mg, 2.74 mmol), compound 319-3 (547 mg, 4.11 mmol) and cesium carbonate (2700 mg, 8.22 mmol) were dissolved in DMSO (8 mL), and the reaction mixture was reacted at 100°C for 16 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 6/1) to obtain compound 319-4.

### Step 4: synthesis of compound 319-6

Compound 319-5 (1000 mg, 3.94 mmol) was dissolved in THF (10 mL) and diisobutylaluminium hydride (7.87 ml, 11.81 mmol) was slowly added dropwise at -78°C under nitrogen protection. The reaction system was reacted at -78°C for 3 h. The reaction solution was quenched by adding water (20 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 5/1) to obtain compound 319-6.

### Step 5: synthesis of compound 319-7

Compound 319-6 (780 mg, 3.45 mmol) and potassium hydroxide (484 mg, 8.63 mmol) were dissolved in dichloromethane (10 mL) and diphenyl(vinyl)sulfonium trifluoromethanesulfonate was slowly added dropwise (1500 mg, 4.14 mmol) at 0°C. The reaction system was moved to room temperature and reacted for 3 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 319-7.

### Step 6: synthesis of compound 319-8

Compound 319-7 (600 mg, 2.38 mmol), tert-butyl carbamate (557 mg, 4.76 mmol), cesium carbonate (2320 mg, 7.14 mmol) and a catalyst (CAS: 1599466-85-9; 202 mg, 0.24 mmol) were dissolved in dioxane (10 mL), and the reaction mixture was reacted at 90°C for 2 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 319-8.

### Step 7: synthesis of compound 319-9

Compound 318-8 (570 mg, 0.38 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 319-9 which was used directly in the next step without purification.

### Step 8: synthesis of compound 319-10

Compound 319-9 (150 mg, 0.82 mmol), compound 319-4 (300 mg, 0.91 mmol), HATU (344 mg, 0.91 mmol) and N,N-diisopropylethylamine (0.54 ml, 3.29 mmol) were dissolved in DMF (8 ml), and the reaction mixture was reacted at room temperature for 1 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 319-10.

### Step 9: synthesis of compound 319

Compound 319-10 (170 mg, 0.34 mmol), 2-hydroxyethylsulfonamide (211 mg, 1.69 mmol), cesium carbonate (331 mg, 1.02 mmol) and a catalyst (CAS: 1599466-89-3; 27 mg, 0.03 mmol) were dissolved in dioxane (6 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 319. MS (ESI, pos.ion) m/z: 547.1 [M+1]⁺.

¹HNMR (DMSO-d6 ) δ: 10.7-10.8 (m, 1H), 7.9-7.9 (m, 1H), 7.8-7.8 (m, 1H), 7.2-7.2 (m, 1H), 7.1-7.1 (m, 2H),7.0-7.0 (m, 1H), 6.4-6.5 (m, 1H), 4.9-4.9 (m, 2H), 4.1-4.2 (m, 2H), 4.1-4.1 (m, 2H), 3.7-3.8 (m, 2H), 3.3-3.3 (m, 2H), 3.0-3.1(m, 4H), 2.2-2.3 (m, 4H).

### Example 320: synthesis of 2-(4-(difluoromethylene)piperidin-1-yl)-4-((2-hydroxyethyl)sulfonamido)-N-(1-methyl-3,4-dihydro-1H-[1,4]oxazino[4,3-a]indol-9-yl)benzamide (320)

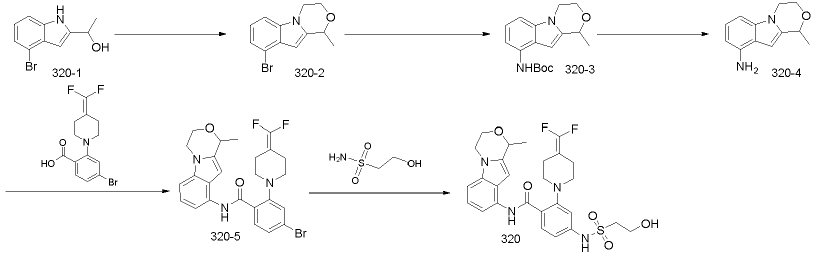

### Step 1: synthesis of compound 320-2

Compound 320-1 (900 mg, 3.75 mmol) and potassium hydroxide (525 mg, 9.37 mmol) were dissolved in dichloromethane and diphenyl(vinyl)sulfonium trifluoromethanesulfonate was slowly added dropwise (1475 mg, 4.5 mmol) at 0°C. The reaction system was moved to room temperature and reacted for 3 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 320-2.

### Step 2: synthesis of compound 320-3

Compound 320-2 (600 mg, 2.25 mmol), tert-butyl carbamate (528 mg, 4.51 mmol), cesium carbonate (2204 mg, 6.76 mmol) and a catalyst (CAS: 1599466-85-9; 191 mg, 0.23 mmol) were dissolved in dioxane (10 mL), and the reaction mixture was reacted at 90°C for 2 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 320-3.

### Step 3: synthesis of compound 320-4

Compound 320-3 (430 mg, 1.42 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 320-4 which was used directly in the next step without purification.

### Step 4: synthesis of compound 320-5

Compound 320-4 (280 mg, 1.38 mmol), 4-bromo-2-(4-(difluoromethylene)piperidin-1-yl)benzoic acid (505 mg, 1.52 mmol), HATU (578 mg, 1.52 mmol) and N,N-diisopropylethylamine (714 mg, 5.54 mmol) were dissolved in DMF (6 ml), and the reaction mixture was reacted at room temperature for 1 hour under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 320-5.

### Step 5: synthesis of compound 320

Compound 320-5 (320 mg, 0.62 mmol), 2-hydroxyethylsulfonamide (387 mg, 3.10 mmol), cesium carbonate (606 mg, 1.86 mmol) and a catalyst (CAS: 1599466-89-3; 50 mg, 0.06 mmol) were dissolved in dioxane (3 mL), and the reaction mixture was reacted at 80°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 320. MS (ESI, pos.ion) m/z: 561.1 [M+1]⁺.

¹H NMR (DMSO-d6 ) δ: 10.78 (s, 1H), 8.00-8.05 (m, 1H), 7.82 (d, *J=* 8.4 Hz, 1H), 7.16-7.20 (m, 1H), 7.11 (br d, *J* = 14.6 Hz, 2H), 7.04 (d, *J* = 10.1 Hz, 1H), 6.55 (s, 1H), 4.93 (br d, *J* = 6.5 Hz, 1H), 4.14-4.20 (m, 1H), 4.18 (br d, *J* = 10.3 Hz, 1H), 3.92-4.06 (m, 2H), 3.78 (br d, *J* = 13.0 Hz, 2H), 3.33-3.37 (m, 2H), 3.03 (br s, 4H), 2.27 (br s, 4H), 1.51(d, *J* = 6.4 Hz, 3H).

### Example 321: synthesis of 2-(4-(difluoromethylene)piperidin-1-yl)-N-(6-fluoro-3,4-dihydro-1H-[1,4] oxazino [4,3-a] indol-9-yl)-4-((2-hydroxyethyl)sulfonamido)benzamide (321)

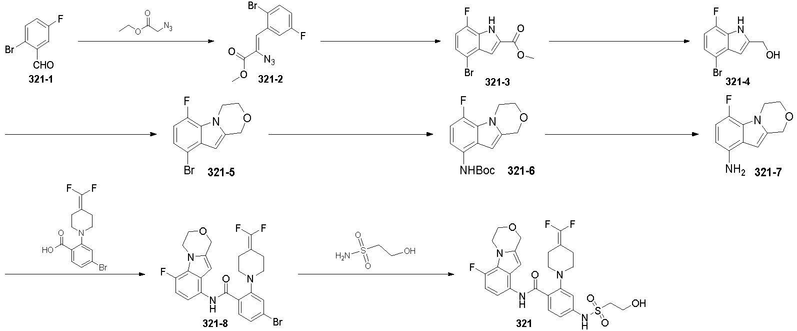

### Step 1: synthesis of compound 321-2

Sodium methoxide (5.4 mol/L, 11.0 mL, 59.11 mmol) was dissolved in methanol (40 mL), to which a solution of compound 321-1 (8.0 g, 39.41 mmol) in methanol (40 mL), and ethyl azidoacetate (6.8 mL, 59.11 mmol) were slowly added dropwise at -15°C, and the mixture was reacted overnight at 0°C. The reaction solution was slowly poured into ice water, and a solid was precipitated and filtered, and the solid was slurried with a small amount of methanol. The resulting slurry was filtered and dried to obtain compound 321-2.

¹H NMR (400 MHz, DMSO-d6) δ = 7.97 (dd, *J* = 3.0, 10.4 Hz, 1H), 7.76 (dd, *J=* 5.5, 8.9 Hz, 1H), 7.23 (dt, *J= 3.1,* 8.4 Hz, 1H), 7.01 (s, 1H), 3.89 (s, 3H).

### Step 2: synthesis of compound 321-3

Compound 321-2 (4.4 g, 14.66 mmol) was dissolved in o-dichlorobenzene (20 mL), and the mixture was reacted at 180°C for 5 h under nitrogen protection. The reaction solution was cooled to room temperature, and a solid was precipitated, and the reaction solution was filtered and dried to obtain compound 321-3.

¹H NMR (400 MHz, DMSO-d6) δ = 12.93 (br s, 1H), 7.32 - 7.26 (m, 1H), 7.13 - 7.05 (m, 2H), 3.90 (s, 3H).

### Step 3: synthesis of compound 321-4

Compound 321-3 (778 mg, 2.86 mmol) was dissolved in tetrahydrofuran (10 mL), to which LiAlH₄ (119 mg, 3.15 mmol) was slowly added at 0°C, and the mixture was reacted at 0°C for 40 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20:1) to obtain compound 321-4.

### Step 4: synthesis of compound 321-5

Compound 321-4 (510 mg, 2.09 mmol), and KOH (293 mg, 5.22 mmol) were dissolved in DCM (3 mL) and stirred at 0°C for 30 minutes, to which a solution of a catalyst (CAS: 247129-88-0; 909 mg, 2.51 mmol) in DCM (2 mL) was added, and stirring was continued at 0°C for 30 minutes. The reaction system was moved to room temperature and reacted overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 321-5.

¹H NMR (400 MHz, DMSO-d6) δ = 7.18 (dd, J = 3.9, 8.4 Hz, 1H), 6.88 (dd, *J* = 8.3, 12.1 Hz, 1H), 6.28 - 6.21 (m, 1H), 4.94 (s, 2H), 4.37 - 4.27 (m, 2H), 4.10 (t, *J* = 5.3 Hz, 2H).

### Step 5: synthesis of compound 321-6

Compound 321-5 (407 mg, 1.51 mmol), NH₂Boc (353 mg, 3.01 mmol), cesium carbonate (1.47 g, 4.52 mmol) and palladium catalyst (CAS: 1599466-83-7; 277 mg, 0.30 mmol) were dissolved in 1,4-dioxane (10 mL), and the reaction mixture was reacted at 90°C for 2 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1-10:1) to obtain compound 321-6.

### Step 6: synthesis of compound 321-7

Compound 321-6 (344 mg, 1.12 mmol) was dissolved in DCM (2 mL), to which HCl\EA (4 mL) was added, and the reaction mixture was allowed to react at 25°C overnight. The reaction solution was filtered, and dried to obtain compound 321-7. MS (ESI, pos.ion) m/z: 207.1 [M+1-HCl]⁺.

### Step 7: synthesis of compound 321-8

Compound 321-7 (92 mg, 0.38 mmol), 4-bromo-2-(4-(difluoromethylene)piperidin-1-yl)benzoic acid (105 mg, 0.32 mmol), and HATU (132 mg, 0.35 mmol) were dissolved in DMF (3 mL), to which DIEA (0.22 mL, 1.26 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 321-8.

### Step 8: synthesis of compound 321

Compound 321-8 (113 mg, 0.22 mmol), 2-hydroxyethanesulfonamide (109 mg, 0.88 mmol), cesium carbonate (212 mg, 0.66 mmol) and palladium catalyst (CAS: 1599466-89-3; 18 mg) were dissolved in 1,4-dioxane (5 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified to obtain compound 321. MS (ESI, pos.ion) m/z:565.2 [M+1]⁺.

¹H NMR (400 MHz, DMSO-d6) δ = 10.70 (s, 1H), 10.07 (br s, 1H), 7.78 - 7.73 (m, 2H), 7.08 - 7.04 (m, 1H), 7.01 (dd, *J =* 1.8, 8.4 Hz, 1H), 6.90 (dd, *J =* 8.5, 12.0 Hz, 1H), 6.51 (s, 1H), 4.93 - 4.91 (m, 2H), 4.32 (br s, 2H), 4.11 (t, *J =* 5.2 Hz, 2H), 3.76 (br t, *J =* 6.4 Hz, 2H), 3.36 - 3.32 (m, 2H), 3.01 (br t, *J =* 5.1 Hz, 4H), 2.22 (br s, 4H).

### Example 322: synthesis of 2-(4-(difluoromethylene)piperidin-1-yl)-N-(3,4-dihydro-1H-benzo[4,5]imidazo[2,1-c][1,4]oxazin-9-yl)-4-((2-hydroxyethyl) sulfonamido)benzamide (322)

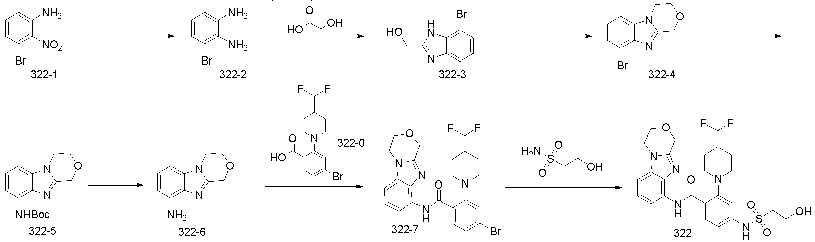

### Step 1: synthesis of compound 322-2

Compound 322-1 (1000 mg, 4.63 mmol), iron dust (1555 mg, 27.7 mmol), and ammonium chloride (495.3 mg, 9.26 mmol) were dissolved in ethanol (9 mL) and H₂O (3 mL), and the reaction mixture was allowed to react at 70°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 322-2.

### Step 2: synthesis of compound 322-3

Compound 322-2 (850 mg, 4.54 mmol) and hydroxyacetic acid (864 mg, 11.36 mmol) were dissolved in 6N HCl (8 mL), and the mixture was reacted at 100°C for 2 hours. The reaction solution was washed with an aqueous saturated sodium bicarbonate solution until the pH reached 9, and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 6/1) to obtain compound 322-3.

### Step 3: synthesis of compound 322-4

Compound 322-3 (800 mg, 3.54 mmol) and (2-bromoethyl)diphenylsulfonium trifluoromethanesulfonate (3.14 mg, 7.08 mmol) were dissolved in acetonitrile (8 mL) and H₂O (4 mL), followed by potassium hydroxide (793 mg, 14.16 mmol), and the reaction was allowed to proceed at 0°C for half an hour under nitrogen protection, and then moved to room temperature and reacted for 4 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 322-4.

### Step 4: synthesis of compound 322-5

Compound 322-4 (300 mg, 1.2 mmol), tert-butyl carbamate (279 mg, 2.38 mmol), cesium carbonate (1163 mg, 3.57 mmol) and a catalyst (CAS: 1599466-85-9; 102 mg, 0.12 mmol) were dissolved in dioxane (5 mL), and the reaction mixture was reacted at 90°C for 2 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 322-5.

### Step 5: synthesis of compound 322-6

Compound 322-5 (200 mg, 0.69 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 322-6 which was used directly in the next step without purification.

### Step 6: synthesis of compound 322-7

Compound 322-6 (54 mg, 0.24 mmol), compound 322-0 (80 mg, 0.24 mmol), HATU (100 mg, 0.26 mmol) and N,N-diisopropylethylamine (0.16 ml, 0.96 mmol) were dissolved in DMF (3 ml), and the reaction mixture was reacted at room temperature for 1 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 322-7.

### Step 7: synthesis of compound 322

Compound 322-7 (70 mg, 0.14 mmol), 2-hydroxyethylsulfonamide (87 mg, 0.7 mmol), cesium carbonate (136 mg, 0.42 mmol) and a catalyst (CAS: 1599466-89-3; 12 mg, 0.01 mmol) were dissolved in dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 322. MS (ESI, pos.ion) m/z: 548.1 [M+1]⁺.

¹HNMR (DMSO-d6 ) δ: 12.35-12.39 (m, 1H), 10.20 (br s, 1H), 8.37-8.41 (m, 1H), 8.04-8.08 (m, 1H), 7.19-7.29 (m, 3H), 7.09-7.14 (m, 1H), 4.88-4.92 (m, 2H), 4.16-4.25 (m, 4H), 3.74-3.80 (m, 2H), 3.34-3.39 (m, 2H), 2.96-3.03 (m, 4H), 2.58-2.65 (m, 4H).

### Example 323: synthesis of 2-(4-(difluoromethylene)piperidin-1-yl)-N-(6-fluoro-3,4-dihydro-1H-benzo[4,5]imidazo[2,1-c][1,4]oxazin-9-yl)-4-((2-hydroxyethyl)sulfonamide)benzamide (323)

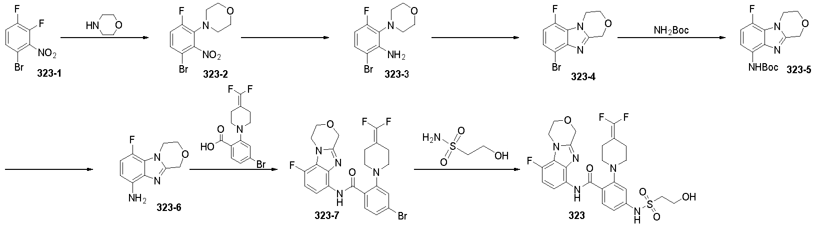

### Step 1: synthesis of compound 323-2

Compound 323-1 (1.5 g, 6.30 mmol) and morpholine (0.83 mL, 9.45 mmol) were dissolved in DMSO (15 mL) solution, to which K₂CO₃ (1.74 g, 12.61 mmol) was added, and the reaction mixture was stirred in an oil bath at 110°C for 2 hours. After the reaction was completed, the reaction solution was diluted with water (150 mL), and extracted with ethyl acetate (60 × 3 mL). The organic layer was washed with brine (60 × 3 mL), dried over Na₂SO₄, filtered and concentrated. The residue was purified by column chromatography (PE:EA=100:0-95:5) to obtain compound 323-2.

### Step 2: synthesis of compound 323-3

Compound 323-2 (1.38 g, 4.52 mmol) was dissolved in a mixed solvent of ethanol (5 mL) and water (5 mL), to which iron dust (1.26 g, 22.62 mmol) was added and the reaction mixture was stirred in an oil bath at 80°C for 2 hours. After the reaction was completed, the reaction solution was filtered and the filtrate was diluted with water (100 mL), and extracted with ethyl acetate (30×3 mL). The organic layer was washed with brine (30 × 3 mL), dried over Na₂SO₄, filtered and concentrated. The residue was purified by column chromatography (PE:EA=100:0-90:10-80:20) to obtain compound 323-3.

### Step 3: synthesis of compound 323-4

Compound 323-3 (600 mg, 2.18 mmol) was dissolved in dichloromethane (3 mL), to which trifluoroacetic acid (1.63 mL, 21.81 mmol) and hydrogen peroxide (0.66 mL, 21.81 mmol) were added. The reaction mixture was stirred in an oil bath at 40°C for 24 hours. After the reaction was completed, the reaction solution was quenched sequentially with anhydrous sodium sulfite solution and sodium bicarbonate solution. The resulting mixture was diluted with water (60 mL) and extracted with ethyl acetate (20 × 3 mL). The organic layer was washed with brine (20 × 3 mL), dried over Na₂SO₄, filtered and concentrated. The residue was purified by column chromatography (PE:EA=100:0-80:20) to obtain compound 323-4.

### Step 4: synthesis of compound 323-5

Compound 323-4 (238 mg, 0.88 mmol) and NH₂Boc (257 mg, 2.19 mmol) were dissolved in 1,4-dioxane (6 mL), to which a catalyst (CAS: 1621274-19-8; 227 mg, 0.26 mmol) and cesium carbonate (1.14 g, 3.51 mmol) were added, and the reaction mixture was stirred in an oil bath at 95°C for 3 hours. After the reaction was completed, the reaction solution was diluted with water (60 mL), and extracted with ethyl acetate (20 × 3 mL). The organic layer was washed with brine (20×3 mL), dried over Na₂SO₄, filtered and concentrated. The crude product was purified by column chromatography (PE:EA=100:0-80:20) to obtain compound 323-5.

### Step 5: synthesis of compound 323-6

Compound 323-5 (212 mg, 0.69 mmol) was dissolved in dichloromethane (3 mL), to which trifluoroacetic acid (0.51 mL, 6.90 mmol) was added, and the reaction mixture was stirred at room temperature for 1 hour. After the reaction was completed, sodium carbonate solution was added to the reaction solution until the solution became alkaline. The resulting mixture was diluted with water (60 mL) and extracted with ethyl acetate (20 × 3 mL). The organic layer was washed with brine (20 × 3 mL), dried over Na₂SO₄, filtered and concentrated. The residue was purified by column chromatography (PE:EA=100:0-80:20) to obtain compound 323-6.

### Step 6: synthesis of compound 323-7

Compound 323-6 (68 mg, 0.20 mmol) and 4-bromo-2-(4-(difluoromethylene)piperidin-1-yl)benzoic acid (47 mg, 0.23 mmol) were dissolved in acetonitrile (3 mL), to which tetramethyluronium hexafluorophosphate (115 mg, 0.41 mmol) and N,N-diisopropylethylamine (106 mg, 0.82 mmol) were added, and the reaction mixture was stirred at room temperature for 1 hour. After the reaction was completed, the reaction solution was diluted with water (60 mL), and extracted with ethyl acetate (20 × 3 mL). The organic layer was washed with brine (20 × 3 mL), dried over Na₂SO₄, filtered and concentrated. The residue was purified by column chromatography (PE:EA=100:0-20:80) to obtain compound 323-7.

### Step 7: synthesis of compound 323

Compound 323-7 (80 mg, 0.15 mmol) and 2-hydroxyethane-1-sulfonamide (77 mg, 0.61 mmol) were dissolved in 1,4-dioxane (3 mL), to which a catalyst (CAS: 1621274-19-8; 13 mg, 0.02 mmol) and cesium carbonate (100 mg, 0.31 mmol) were added, and the reaction mixture was stirred in an oil bath at 95°C for 3 hours. The reaction mixture was stirred at room temperature for 1.5 hours. After the reaction was completed, the reaction solution was filtered and concentrated, and the residue was purified to obtain compound 323. MS (ESI, pos.ion) m/z: 566.1 [M+1]⁺.

¹H NMR (400 MHz, DMSO-d6) δ = 12.40-12.33 (m, 1H), 8.34-8.27 (m, 1H), 8.08-8.02 (m, 1H), 7.23-7.18 (m, 1H), 7.12-7.01 (m, 2H), 4.94-.89 (m, 2H), 4.46-4.38 (m, 2H), 4.21-4.13 (m, 2H), 3.79 -3.72 (m, 2H), 3.40-3.33 (m, 2H), 3.05-2.95 (m, 4H), 2.65-2.56 (m, 4H).

### Example 327: synthesis of 2-(4-(difluoromethylene)piperidin-1-yl)-4-((2-hydroxyethyl)sulfonamido)-N-(3-methyl-3,4-dihydro-1H-[1,4]oxazinyl[4,3-a]indol-9-yl)benzamide (327)

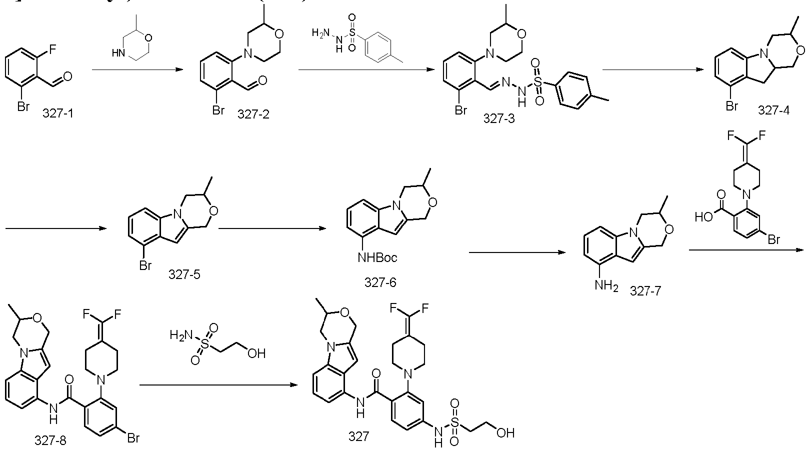

### Step 1: synthesis of compound 327-2

Compound 327-1 (3000 mg, 14.78 mmol), 2-methyl-1,4-oxazine (1790 mg, 17.73 mmol) and potassium carbonate (5000 mg, 36.94 mmol) were dissolved in DMSO (10 mL), and the reaction mixture was reacted at 95°C for 12 hours under nitrogen protection. The reaction solution was quenched by adding water (20 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 327-2.

### Step 2: synthesis of compound 327-3

Compound 327-2 (800 mg, 2.81 mmol) and N-amino-4-toluenesulfonamide (1328 mg, 3.38 mmol) were dissolved in toluene (10 mL) and reacted at room temperature for 0.5 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure to obtain compound 327-3 which was used directly in the next reaction.

### Step 3: synthesis of compound 327-4

Compound 327-3 (1270 mg, 7.89 mmol) and sodium hydride (378 mg, 9.47 mmol) were dissolved in toluene (10 mL) and reacted at room temperature for 0.5 hours, and then reacted at 135°C for 15 min under microwave irradiation. After the reaction was completed as monitored by TLC, the reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure and purified to obtain compound 327-4.

### Step 4: synthesis of compound 327-5

Compound 327-4 (220 mg, 0.82 mmol) and manganese dioxide (1069 mg, 12.31 mmol) were dissolved in DCM (8 mL) and reacted at 40°C for 12 hours. After the reaction was completed as monitored by TLC, the reaction solution was quenched by adding water (6 ml), and liquid separation was performed with DCM (30 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 327-5.

### Step 5: synthesis of compound 327-6

Compound 327-5 (100 mg, 0.38 mmol), tert-butyl carbamate (88 mg, 0.75 mmol), cesium carbonate (367 mg, 1.13 mmol) and a catalyst (CAS: 1599466-85-9; 31 mg, 0.04 mmol) were dissolved in dioxane (5 mL), and the reaction mixture was reacted at 90°C for 4 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 327-6.

### Step 6: synthesis of compound 327-7

Compound 327-6 (100 mg, 0.33 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 327-7 which was used directly in the next step without purification.

### Step 7: synthesis of compound 327-8

Compound 327-7 (70 mg, 0.29 mmol), 4-bromo-2-(4-(difluoromethylene)piperidin-1-yl)benzoic acid (107 mg, 0.32 mmol), HATU (123 mg, 0.32 mmol) and N,N-diisopropylethylamine (0.19 ml, 1.18 mmol) were dissolved in DMF (4 ml), and the reaction mixture was reacted at room temperature for 1 hour under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 327-8.

### Step 8: synthesis of compound 327

Compound 327-8 (100 mg, 0.19 mmol), 2-hydroxyethylsulfonamide (121 mg, 0.97 mmol), cesium carbonate (189 mg, 0.58 mmol) and a catalyst (CAS: 1599466-89-3; 15 mg, 0.02 mmol) were dissolved in dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 327. MS (ESI, pos.ion) m/z: 561.2 [M+1]⁺.

¹HNMR (DMSO-d6 ) δ: 10.71-10.76 (m, 1H), 7.90-7.95 (m, 1H), 7.76-7.81 (m, 1H), 7.15-7.20 (m, 1H), 7.05-7.13 (m, 2H), 6.99-7.04 (m, 1H), 6.43-6.48 (m, 1H), 4.99-5.06 (m, 1H), 4.84-4.91 (m, 1H), 4.25-4.32 (m, 1H), 4.05-4.13 (m, 1H), 3.73-3.80 (m, 2H), 3.54-3.62 (m, 1H), 3.32-3.36 (m, 2H), 2.98-3.05 (m, 4H), 2.19-2.27 (m, 4H), 1.35 (d, *J*=6.1 Hz, 3H).

### Example 340: N-(8,8-difluoro-9-methoxy-6,7,8,9-tetrahydropyridino[1,2-a]indol-1-yl)-4-((2-hydroxyethyl)sulfonamido)-2-(6-azaspiro[2.5]oct-6-yl)benzamide (340)

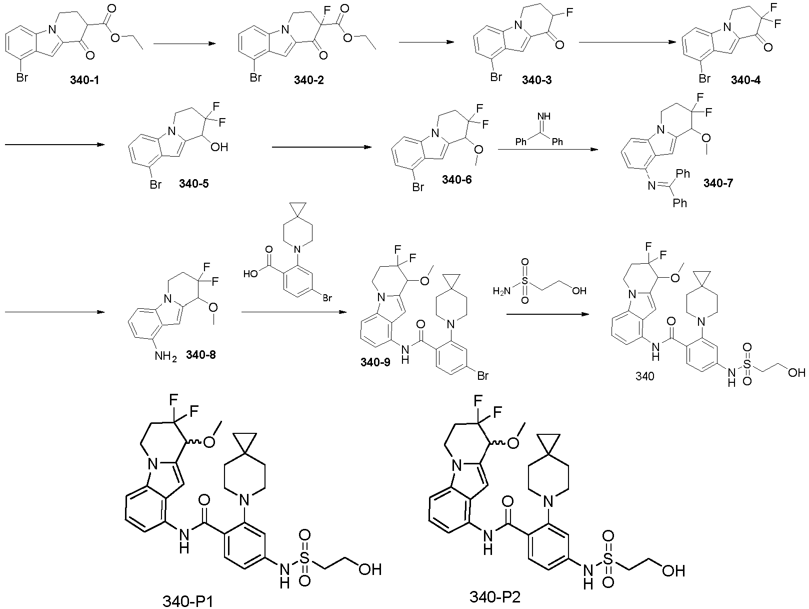

### Step 1: synthesis of compound 340-2

Compound 340-1 (4 g, 11.9 mmol) and potassium **tert-butoxide** (2.67 g, 23.89 mmol) were dissolved in tetrahydrofuran (50 mL). NFSI (7.52 g, 23.89 mmol) was added under ice bath conditions, and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-20%) to obtain compound 340-2.

### Step 2: synthesis of compound 340-3

Compound 340-2 (3.4 g, 9.63 mmol) was dissolved in ethanol (30 ml), concentrated hydrochloric acid (30 ml) was added, and the mixture was reacted at 80°C for 2 hours. After cooling, a solid was precipitated and the reaction solution was filtered to obtain compound 340-3.

### Step 3: synthesis of compound 340-4

Compound 340-3 (2.2 g, 7.83 mmol) was dissolved in tetrahydrofuran (30 ml), followed by the addition of lithium bis(trimethylsilyl)amide (7.8 ml, 15.65 mmol) under ice bath conditions. The reaction solution was stirred under ice bath conditions for 1 hour, and then NFSI (4.93 g, 15.65 mmol) was added under ice bath conditions. The resulting mixture was stirred at room temperature for 3 hours. The reaction solution was poured into an appropriate amount of ice water, extracted three times with ethyl acetate (30 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 340-4.

### Step 4: synthesis of compound 340-5

Compound 340-4 (1.5 g, 5.01 mmol) was dissolved in methanol (15 mL), sodium borohydride (572 mg, 15.05 mmol) was added, and the reaction mixture was stirred overnight at room temperature. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 340-5.

### Step 5: synthesis of compound 340-6

Compound 340-5 (1 g, 3.32 mmol) was dissolved in DMF (10 ml), followed by the addition of sodium hydride (266 mg, 6.64 mmol) under ice bath conditions. The reaction solution was stirred under ice bath conditions for 1 hour, and then iodomethane (566 mg, 3.99 mmol) was added under ice bath. The resulting mixture was stirred at room temperature for 3 hours. The reaction solution was poured into an appropriate amount of ice water, extracted three times with ethyl acetate (30 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 340-6.

### Step 6: synthesis of compound 340-7

Compound 340-6 (500 mg, 1.59 mmol), cesium carbonate (1.55 g, 4.76 mmol), a catalyst (CAS: 1599466-89-3; 128 mg, 0.159 mmol) and benzophenonimine (575 mg, 3.17 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 340-7.

### Step 7: synthesis of compound 340-8

Compound 340-7 (300 mg, 0.721 mmol) was dissolved in dichloromethane (5 ml), and hydrogen chloride-ethyl acetate solution (1.8 ml, 7.2 mmol) was added under ice bath conditions. The mixture was reacted at room temperature for 2 hours, a solid was precipitated and the reaction solution was concentrated to obtain compound 340-8.

### Step 8: synthesis of compound 340-9

Compound 340-8 (170 mg, 0.675 mmol), HATU (385 mg, 1.01 mmol), DIEA (261 mg, 2.02 mmol), and 4-bromo-2-(6-azaspiro[2.5]oct-6-yl)benzoic acid (208 mg, 0.675 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 340-9.

### Step 9: synthesis of compound 340, compound 340-P1, and compound 340-P2

Compound 340-9 (230 mg, 0.424 mmol), cesium carbonate (414 mg, 1.27 mmol), a catalyst (CAS: 1599466-89-3; 34 mg, 0.042 mmol) and 2-hydroxyethane-1-sulfonamide (417 mg, 3.33 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 340. MS (ESI, pos.ion) m/z:589.3 [M+1]⁺.

¹HNMR (CHLOROFORM-d, 400 MHz) δ 11.6-11.6 (m, 1H), 8.1-8.2 (m, 1H), 8.0-8.0 (m, 1H), 7.3-7.3 (m, 2H), 7.1-7.2 (m,2H), 6.9-7.0 (m, 1H), 6.7-6.8 (m, 1H), 4.5-4.6 (m, 1H), 4.4-4.4 (m, 1H), 4.1-4.1 (m, 3H), 3.50 (s, 3H), 3.3-3.3 (m, 2H),3.1-3.2 (m, 4H), 2.8-3.0 (m, 2H), 2.4-2.5 (m, 1H), 1.56 (br d, 4H, *J =* 4.5 Hz), 0.34 (br s, 4H).

For purification of compound 340 by preparative SFC, the following conditions were used: column: ChiralPak IC, 250×30mm I.D., 10µm particles; mobile phase A: 70/30 CO₂/EtOH (0.1% NH₃H₂O); detector wavelength: 220 nm; flow rate: 150 mL/min.

Compound 340-P1 (23 mg) MS (ESI, pos.ion) m/z: 589.2 [M+1]⁺; retention time = 2.051 min;
¹H NMR (CHLOROFORM-d, 400 MHz) *δ* 11.60 (s, 1H), 8.14 (d, *J =* 8.5 Hz, 1H), 8.02 (d, *J =* 7.6 Hz, 1H), 7.3-7.3 (m, 2H), 7.1-7.2 (m, 2H), 6.97 (dd, *J =* 2.0, 8.4 Hz, 1H), 6.73 (s, 1H), 4.56 (t, *J =* 5.8 Hz, 1H), 4.40 (ddd, *J* = 2.1, 6.8, 11.7 Hz, 1H), 4.0-4.1(m, 3H), 3.50 (s, 3H), 3.2-3.3 (m, 2H), 3.1-3.2 (m, 4H), 2.91 (br t, *J* = 4.8 Hz, 2H), 2.4-2.5 (m, 1H), 1.5-1.6 (m, 4H), 0.3-0.3 (m, 4H).

Compound 340-P2 (17 mg) MS (ESI, pos.ion) m/z: 589.2 [M+1]⁺; retention time = 2.554 min;
¹H NMR (CHLOROFORM-d, 400 MHz) δ 11.60 (s, 1H), 8.14 (d, *J =* 8.5 Hz, 1H), 8.02 (d, *J =* 7.6 Hz, 1H), 7.3-7.3 (m, 2H), 7.1-7.2 (m, 2H), 6.97 (dd, *J =* 2.0, 8.4 Hz, 1H), 6.73 (s, 1H), 4.56 (t, *J =* 5.8 Hz, 1H), 4.40 (ddd, *J* = 2.1, 6.8, 11.7 Hz, 1H), 4.0-4.1(m, 3H), 3.50 (s, 3H), 3.2-3.3 (m, 2H), 3.1-3.2 (m, 4H), 2.91 (br t, *J =* 4.8 Hz, 2H), 2.4-2.5 (m, 1H), 1.5-1.6 (m, 4H), 0.3-0.3 (m, 4H).

### Example 341: synthesis of N-(7,7-difluoro-6,7,8,9-tetrahydropyridino[1,2-a]indol-1-yl)-4-((2-hydroxyethyl)sulfonamido)-2-(6-azaspiro[2.5]oct-6-yl)benzamide (341)

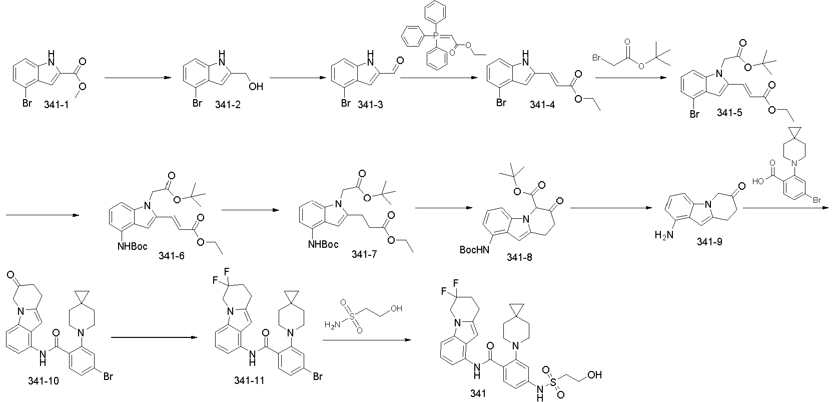

### Step 1: synthesis of compound 341-2

Compound 341-1 (5 g, 19.76 mmol) was dissolved in tetrahydrofuran (20 mL). DIBAL-H (39.5 ml, 59.29 mmol) was added under ice bath conditions, and the reaction mixture was stirred at room temperature for 3 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 341-2.

### Step 2: synthesis of compound 341-3

Compound 341-2 (4.3 g, 19.11 mmol) was dissolved in dichloromethane (50 mL), manganese dioxide (16.6 g, 191.11 mmol) was added, and the reaction mixture was stirred overnight at room temperature. The reaction solution was filtered and the filtrate was concentrated under reduced pressure to obtain compound 341-3.

### Step 3: synthesis of compound 341-4

Compound 341-3 (4 g, 17.94 mmol) and ethyl 2-(triphenyl-5-phosphinimide)acetate (9.36 g, 26.9 mmol) were dissolved in dichloromethane (50 mL), and the mixture was reacted at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-20%) to obtain compound 341-4.

### Step 4: synthesis of compound 341-5

Compound 341-4 (3.5 g, 11.95 mmol), tert-butyl 2-bromoacetate (3.01 g, 15.5 mmol) and cesium carbonate (11.68 g, 35.84 mmol) were dissolved in DMF (20 mL), and the mixture was stirred at 50°C for 5 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-10%) to obtain compound 341-5.

### Step 5: synthesis of compound 341-6

Compound 341-5 (3.8 g, 9.34 mmol), cesium carbonate (9.13 g, 28.01 mmol), a catalyst (CAS: 1599466-89-3; 754 mg, 0.934 mmol) and tert-butoxycarbonyl amine (2.18 g, 18.67 mmol) were dissolved in 1,4-dioxane (50 mL), and the mixture was reacted at 80°C for 16 hours under nitrogen protection. The reaction solution was cooled and poured into an appropriate amount of ice water, extracted three times with ethyl acetate (40 ml), washed twice with sodium chloride aqueous solution (60 mL), the organic phase was dried over anhydrous sodium sulfate, concentrated under reduced pressure, and the crude product was purified by silica gel column chromatography (EA/PE = 0-20%) to obtain compound 341-6.

### Step 6: synthesis of compound 341-7

Compound 341-6 (2.5 g, 5.63 mmol) was dissolved in methanol (30 mL), palladium on carbon (500 mg) was added, and the reaction mixture was stirred overnight at room temperature under hydrogen atmosphere. The reaction solution was filtered and the filtrate was concentrated under reduced pressure to obtain compound 341-7.

### Step 7: synthesis of compound 341-8

Compound 341-7 (2.3 g, 5.15 mmol) and potassium tert-butoxide (1.73 g, 15.47 mmol) were dissolved in tetrahydrofuran (50 mL). The reaction mixture was reacted at room temperature for 3 hours under nitrogen protection. The reaction solution was poured into an appropriate amount of ice water, extracted three times with ethyl acetate (50 ml), washed twice with sodium chloride aqueous solution (80 mL), the organic phase was dried over anhydrous sodium sulfate, concentrated under reduced pressure, and the crude product was purified by silica gel column chromatography (EA/PE = 0-10%) to obtain compound 341-8.

### Step 8: synthesis of compound 341-9

Compound 341-8 (1.2 g, 3.00 mmol) was dissolved in dichloromethane (10 ml), and hydrogen chloride-ethyl acetate solution (11.25 ml, 45.00 mmol) was added under ice bath conditions. The mixture was reacted at room temperature for 2 hours, a solid was precipitated and the reaction solution was concentrated to obtain compound 341-9.

### Step 9: synthesis of compound 341-10

Compound 341-9 (100 mg, 0.5 mmol), HATU (285 mg, 0.75 mmol), DIEA (193.5 mg, 1.5 mmol), and 4-bromo-2-(6-azaspiro[2.5]oct-6-yl)benzoic acid (154.5 mg, 0.5 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 341-10.

### Step 10: synthesis of compound 341-11

Compound 341-10 (130 mg, 0.265 mmol) and DAST (85 mg, 0.530 mmol) were dissolved in tetrahydrofuran (10 mL). The reaction mixture was reacted at room temperature for 3 hours under nitrogen protection. The reaction solution was poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-10%) to obtain compound 341-11.

### Step 11: synthesis of compound 341

Compound 341-11 (65 mg, 0.127 mmol), cesium carbonate (124 mg, 0.380 mmol), a catalyst (CAS: 1599466-89-3; 10 mg, 0.013 mmol) and 2-hydroxyethane-1-sulfonamide (32 mg, 0.253 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 341. MS (ESI, pos.ion) m/z: 559.21 [M+1]⁺.

¹H NMR (CHLOROFORM-d, 400 MHz) δ 11.6-11.7 (m, 1H), 8.1-8.2 (m, 1H), 7.8-7.8 (m, 1H), 7.3-7.3 (m, 1H), 7.2-7.2 (m,1H), 7.0-7.1 (m, 1H), 6.9-7.0 (m, 1H), 6.3-6.4 (m, 1H), 4.3-4.4 (m, 2H), 4.0-4.1 (m, 2H), 3.2-3.3 (m, 2H), 3.2-3.2 (m, 2H),3.1-3.1 (m, 4H), 2.34 (br s, 1H), 1.5-1.6 (m, 4H), 0.3-0.3 (m, 4H).

### Example 342: synthesis of N-(9-hydroxy-6,7,8,9-tetrahydropyridino[1,2-a]indol-1-yl)-4-((2-hydroxyethyl)sulfonamido)-2-(6-azaspiro[2.5]octan-6-yl)benzamide (342)

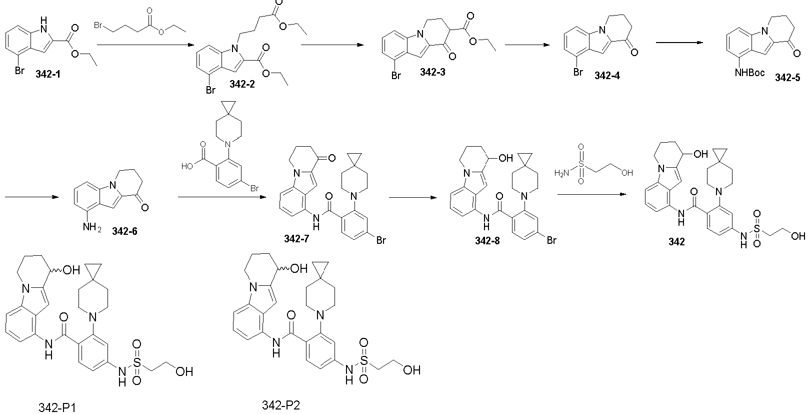

### Step 1: synthesis of compound 342-2

Compound 342-1 (2 g, 7.49 mmol), ethyl 4-bromobutyrate (1.74 g, 8.99 mmol) and cesium carbonate (4.88 g, 14.98 mmol) were dissolved in DMF (20 mL), and the mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-10%) to obtain compound 342-2.

### Step 2: synthesis of compound 342-3

Compound 342-2 (2.5 g, 6.56 mmol) and potassium tert-butoxide (2.2 g, 19.69 mmol) were dissolved in tetrahydrofuran (30 mL). The reaction mixture was reacted at room temperature for 3 hours under nitrogen protection. The reaction solution was poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-10%) to obtain compound 342-3.

### Step 3: synthesis of compound 342-4

Compound 342-3 (1.8 g, 5.37 mmol) was dissolved in ethanol (15 ml), concentrated hydrochloric acid (15 ml) was added, and the mixture was reacted at 80°C for 2 hours. After cooling, a solid was precipitated and the reaction solution was filtered to obtain compound 342-4.

### Step 4: synthesis of compound 342-5

Compound 342-4, cesium carbonate (744 mg, 2.28 mmol), a catalyst (CAS: 1599466-89-3; 61 mg, 0.076 mmol) and tert-butyl carbamate (178 mg, 1.52 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 342-5.

### Step 5: synthesis of compound 342-6

Compound 342-5 (135 mg, 0.45 mmol) was dissolved in dichloromethane (5 ml), and hydrogen chloride-ethyl acetate solution (1.13 ml, 4.5 mmol) was added under ice bath conditions. The mixture was reacted at room temperature for 2 hours, a solid was precipitated and the reaction solution was concentrated to obtain compound 342-6.

### Step 6: synthesis of compound 342-7

Compound 342-6 (70 mg, 0.35 mmol), HATU (199 mg, 0.525 mmol), DIEA (135 mg, 1.05 mmol), and 4-bromo-2-(6-azaspiro[2.5]oct-6-yl)benzoic acid (108 mg, 0.35 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-30%) to obtain compound 342-7.

### Step 7: synthesis of compound 342-8

Compound 342-7 (80 mg, 0.163 mmol) was dissolved in methanol (5 mL), sodium borohydride (31 mg, 0.815 mmol) was added, and the reaction mixture was stirred overnight at 70°C. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 342-8.

### Step 8: synthesis of compound 342, compound 342-P1, and compound 342-P2

Compound 342-8 (60 mg, 0.122 mmol), cesium carbonate (119 mg, 0.365 mmol), a catalyst (CAS: 1599466-89-3; 9.8 mg, 0.012 mmol) and 2-hydroxyethane-1-sulfonamide (30 mg, 0.243 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 342. MS (ESI, pos.ion) m/z: 539.2 [M+1]⁺.

¹H NMR (DMSO-d6, 400 MHz) δ 11.2-11.2 (m, 1H), 10.1-10.1 (m, 1H), 7.8-7.9 (m, 2H), 7.2-7.2 (m, 1H), 7.1-7.2 (m, 1H),7.1-7.1 (m, 1H), 7.0-7.1 (m, 1H), 6.6-6.6 (m, 1H), 4.8-4.9 (m, 1H), 4.0-4.1 (m, 2H), 3.97 (br dd, 1H, *J* = 4.8, 7.4 Hz), 3.7-3.8(m, 2H), 3.35 (t, 2H, *J =* 6.6 Hz), 3.04 (br s, 4H), 2.2-2.3 (m, 1H), 2.0-2.1 (m, 1H), 1.9-2.0 (m, 1H), 1.7-1.8 (m, 1H), 1.4-1.5(m, 4H), 0.2-0.3 (m, 4H).

For purification of compound 342 by preparative SFC, the following conditions were used: column: Whelk O1(S, S), 250×30mm I.D., 10µm particles; mobile phase A: 60/40 CO₂/EtOH (0.1% NH₃H₂O); detector wavelength: 220 nm; flow rate: 150 mL/min.

Compound 342-P1 MS (ESI, pos.ion) m/z: 539.2 [M+1]⁺; retention time = 5.910 min;
¹H NMR (DMSO-*d6*, 400 MHz) *δ* 11.2-11.2 (m, 1H), 10.1-10.1 (m, 1H), 7.8-7.9 (m, 2H), 7.2-7.2 (m, 1H), 7.1-7.2 (m, 1H),7.1-7.1 (m, 1H), 7.0-7.1 (m, 1H), 6.6-6.6 (m, 1H), 4.8-4.9 (m, 1H), 4.0-4.1 (m, 2H), 3.97 (br dd, *J* = 4.8*,* 7.4 Hz, 1H), 3.7-3.8(m, 2H), 3.35 (t, *J =* 6.6 Hz, 2H), 3.04 (br s, 4H), 2.2-2.3 (m, 1H), 2.0-2.1 (m, 1H), 1.9-2.0 (m, 1H), 1.7-1.8 (m, 1H), 1.4-1.5(m, 4H), 0.2-0.3 (m, 4H).

Compound 342-P2 MS (ESI, pos.ion) m/z: 539.2 [M+1]⁺; retention time = 7.214 min;
¹H NMR (DMSO-*d6*, 400 MHz) *δ* 11.2-11.2 (m, 1H), 10.1-10.1 (m, 1H), 7.8-7.9 (m, 2H), 7.2-7.2 (m, 1H), 7.1-7.2 (m, 1H),7.1-7.1 (m, 1H), 7.0-7.1 (m, 1H), 6.6-6.6 (m, 1H), 4.8-4.9 (m, 1H), 4.0-4.1 (m, 2H), 3.97 (br dd, *J* = 4.8, 7.4 Hz, 1H), 3.7-3.8(m, 2H), 3.35 (t, *J =* 6.6 Hz, 2H), 3.04 (br s, 4H), 2.2-2.3 (m, 1H), 2.0-2.1 (m, 1H), 1.9-2.0 (m, 1H), 1.7-1.8 (m, 1H), 1.4-1.5(m, 4H), 0.2-0.3 (m, 4H).

### Example 346: synthesis of 4-((2-hydroxyethyl)sulfonamido)-N-(1-oxo-3,4-dihydro-1H-[1,4]oxazino[4,3-a]indol-9-yl)-2-(6-azaspiro[2.5]oct-6-yl)benzamide (346)

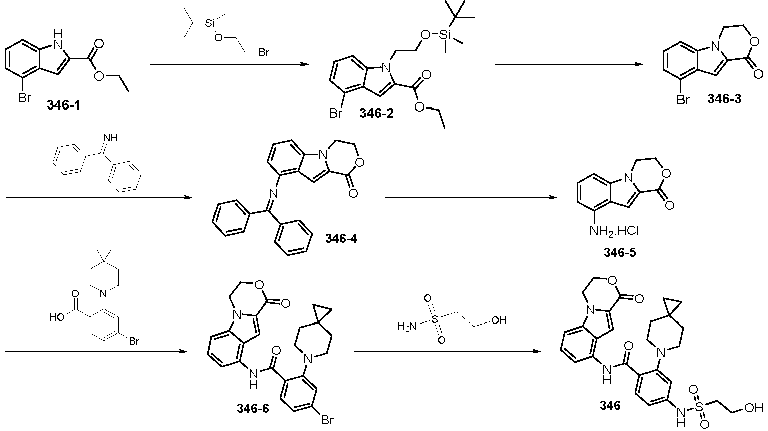

### Step 1: synthesis of compound 346-2

Compound 346-1 (720 mg, 2.69 mmol) was dissolved in ultra-dry DMF (5 mL), to which NaH (129 mg, 3.22 mmol) was slowly added at 0°C, and the mixture was stirred at room temperature for 10 minutes, to which (2-bromoethoxy)(tert-butyl)dimethylsilane (0.75 mL, 3.49 mmol) was added. The mixture was reacted at 52°C for 4 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 346-2.

### Step 2: synthesis of compound 346-3

Compound 346-2 (995 mg, 2.33 mmol) was dissolved in THF (2 mL), to which TBAF (4.66 mL, 4.66 mmol) was slowly added at 0°C, and the mixture was reacted at room temperature for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=3:1) to obtain compound 346-3.

### Step 3: synthesis of compound 346-4

Compound 346-3 (250 mg, 0.94 mmol), benzophenonimine (0.32 mL, 1.88 mmol), cesium carbonate (918 mg, 2.82 mmol) and palladium catalyst (CAS: 1599466-83-7) (166 mg, 0.18 mmol) were dissolved in dioxane (5 mL), and the reaction mixture was reacted at 90°C for 3 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 346-4. MS (ESI, pos.ion) m/z: 367.1 [M+1]⁺.

### Step 4: synthesis of compound 346-5

Compound 346-4 (212 mg, 0.58 mmol) was dissolved in dichloromethane (1 mL), to which hydrogen chloride-ethyl acetate solution (5 mL) was added, and the reaction mixture was allowed to react at 25°C overnight. The reaction solution was filtered and dried to obtain compound 346-5. MS (ESI, pos.ion) m/z:203.1 [M+1-HCl]⁺.

### Step 5: synthesis of compound 346-6

Compound 346-5 (82 mg, 0.34 mmol), 4-bromo-2-(6-azaspiro[2.5]octan-6-yl)benzoic acid (128 mg, 0.41 mmol), and TCFH (145 mg, 0.52 mmol) were dissolved in ultra-dry acetonitrile (2 mL), to which N-methylimidazole (0.14 mL, 1.72 mmol) was added, and the reaction mixture was allowed to react at 25°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 346-6.

### Step 6: synthesis of compound 346

Compound 346-6 (128 mg, 0.25 mmol), 2-hydroxylethanesulfonamide (162 mg, 1.29 mmol), cesium carbonate (253 mg, 0.78 mmol) and palladium catalyst (CAS: 1599466-85-9, 44 mg, 0.05 mmol) were dissolved in 1,4-dioxane (5 mL), and the reaction mixture was reacted at 100°C for 2 hours under N₂ protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 346. MS (ESI, pos.ion) m/z:539.2 [M+1]⁺.

¹H NMR (400 MHz, DMSO-d6) δ = 11.20 (s, 1H), 10.10 (s, 1H), 8.03 (d, *J* = 7.0 Hz, 1H), 7.81 (d, *J =* 8.4 Hz, 1H), 7.66 (s, 1H), 7.42 - 7.34 (m, 2H), 7.20 - 7.16 (m, 1H), 7.03 (dd, *J =* 1.8, 8.4 Hz, 1H), 4.82 - 4.75 (m, 2H), 4.48 - 4.45 (m, 2H), 3.78 (t*, J =* 6.6 Hz, 2H), 3.35 (t, *J =* 6.6 Hz, 2H), 3.04 (br s, 4H), 1.49 - 1.39 (m, 4H), 0.26 (s, 4H).

### Example 357: synthesis of N-(4-(2-hydroxyethyl)sulfonamido)-2-(6-azaspiro[2.5]octan-6-yl)phenyl)-3,4-dihydro-1H-[1,4]oxazino[4,3-a]indole-9-carboxamide (357)

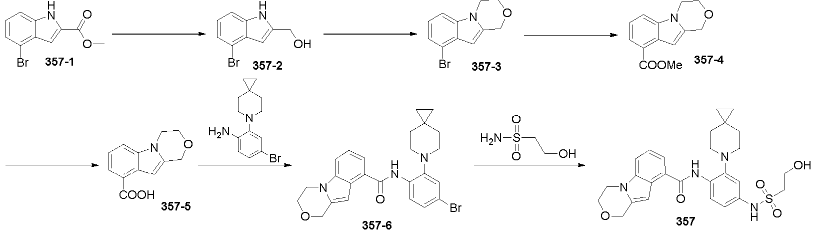

### Step 1: synthesis of compound 357-2

Compound 357-1 (5 g, 19.68 mmol) was dissolved in tetrahydrofuran (40 mL), to which diisobutylaluminium hydride (8.40 g, 59.04 mmol) was added at -20°C. The reaction mixture was stirred in a water bath at 0°C for 1 hour. After the reaction was completed, the reaction solution was quenched with ice saturated ammonium chloride solution (150 mL), and extracted with ethyl acetate (90 × 3 mL). The organic layer was washed with brine (90×3 mL), dried over Na₂SO₄, filtered and concentrated. The crude product was purified by column chromatography (PE:EA=100:0-90:10-80:20) to obtain compound 357-2.

### Step 2: synthesis of compound 357-3

Compound 357-2 (1 g, 4.42 mmol) was dissolved in dichloromethane (14 mL), to which vinyldiphenyl-1,1,1-sulfonium trifluoromethanesulfonate (1.92 g, 5.31 mmol) and potassium carbonate (0.62 g, 11.06 mmol) were added. The reaction mixture was stirred at room temperature for 1.5 hours. After the reaction was completed, the reaction solution was filtered and the filtrate was diluted with water (100 mL), and extracted with ethyl acetate (60×3 mL). The organic layer was washed with brine (60 × 3 mL), dried over Na₂SO₄, filtered and concentrated. The residue was purified by column chromatography (PE:EA=100:0-95:5) to obtain compound 357-3.

### Step 3: synthesis of compound 357-4

Compound 357-3 (300 mg, 1.19 mmol) was dissolved in dimethyl sulfoxide (4 mL) and methanol (4 mL), to which [1,1'-bis(diphenylphosphine) ferrocene]palladium dichloride (173 mg, 0.24 mmol) and triethylamine (241 mg, 2.38 mmol) were added. The reaction mixture was stirred overnight in an oil bath at 75°C under hydrogen atmosphere. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-95:5) to obtain compound 357-4.

### Step 4: synthesis of compound 357-5

Compound 357-4 (130 mg, 0.56 mmol) was dissolved in methanol (4 mL) and water (1 mL), to which sodium hydroxide (134 mg, 3.36 mmol) was added. The reaction mixture was stirred in an oil bath at 60°C for 2 hours. After the reaction was completed, 2M HCl was added to adjust the reaction solution to an acidic system, and then the reaction was filtered to obtain compound 357-5.

### Step 5: synthesis of compound 357-6

Compound 357-5 (50 mg, 0.23 mmol) and 2-(6-azaspiro[2.5]octan-6-yl)-4-bromoaniline (65 mg, 0.23 mmol) were dissolved in acetonitrile (3 mL), to which N,N,N',N'-tetramethylchloroformamidinium hexafluorophosphate (78 mg, 0.28 mmol) and N,N-diisopropylethylamine (74 mg, 0.58 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 357-6.

### Step 6: synthesis of compound 357

Compound 357-6 (60 mg, 0.12 mmol) and 2-hydroxyethane-1-sulfonamide (60 mg, 0.48 mmol) were dissolved in 1,4-dioxane (3 mL), to which a catalyst (CAS: 1599466-83-7; 33 mg, 0.04 mmol) and cesium carbonate (78 mg, 0.24 mmol) were added. The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The residue was purified by preparative chromatography to obtain compound 357. MS (ESI, pos.ion) m/z: 525.2 [M+1]⁺.

1H NMR (400 MHz, DMSO-d6) δ = 9.64-9.55 (m, 1H), 9.52-9.48 (m, 1H), 8.36-8.31 (m, 1H), 7.70-7.64 (m, 2H), 7.32-7.26 (m, 1H), 7.20-7.17 (m, 1H), 7.05-7.00 (m, 1H), 6.84-6.81 (m, 1H), 5.02-4.96 (m, 2H), 4.96 -4.88 (m, 1H), 4.17 (s, 4H), 3.79-3.72 (m, 2H), 3.22 (t, *J =* 6.7 Hz, 2H), 2.90-2.84 (m, 2H), 2.84-2.81 (m, 2H), 1.56-1.32 (m, 4H), 0.39-0.32 (m, 4H).

### Example 364: synthesis of N-(6-cyano-3,4-dihydro-1H-[1,4]oxazino[4,3-a]indol-9-yl)-2-(4-(difluoromethylene)piperidin-1-yl)-4-((2-hydroxyethyl) sulfonamido)benzamide (364)

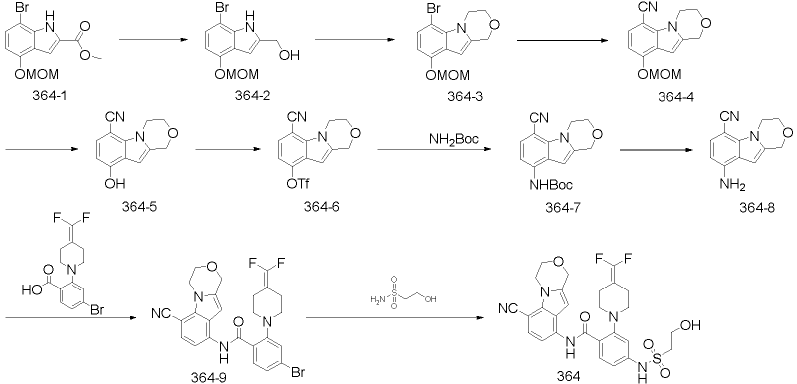

### Step 1: synthesis of compound 364-2

Compound 364-1 (2 g, 6.39 mmol) was dissolved in tetrahydrofuran (15 mL), to which LiAlH₄ (267 mg, 7 mmol) was slowly added at 0°C, and the mixture was reacted at 0°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 364-2 as a white solid.

### Step 2: synthesis of compound 364-3

Compound 364-2 (1.27 g, 4.45 mmol), and KOH (623 mg, 11.14 mmol) were dissolved in DCM (12 mL) and stirred at 0°C for 30 minutes, to which a solution of a compound (CAS: 247129-88-0; 1.93 g, 5.34 mmol) in DCM (5 mL) was added, and stirring was continued at 0°C for 30 minutes. The reaction system was moved to room temperature and reacted overnight. The reaction solution was quenched with ice water, and extracted with DCM. The organic phase was washed with saturated NaCl solution, dried over anhydrous Na₂SO₄, and concentrated under reduced pressure. The crude product was purified by silica gel column chromatography (PE:EA=10:1) to obtain compound 364-3 as a white solid.

### Step 3: synthesis of compound 364-4

Compound 364-3 (710 mg, 2.28 mmol), Zn(CN)₂ (2.6 g, 22.8 mmol), dppf (126 mg, 0.23 mmol) and tetrakis(triphenylphosphine)palladium (264 mg, 0.23 mmol) were dissolved in DMF (10 mL), and the reaction mixture was reacted at 100°C for 4 h under nitrogen. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 364-4.

### Step 4: synthesis of compound 364-5

Compound 364-4 (500 mg, 1.93 mmol) was dissolved in DCM (5 mL), to which HCl\EA (5 mL) was added, and the reaction mixture was allowed to react at 25°C for 2 hours. The reaction solution was subjected to rotary evaporation to dryness to obtain hydrochloride of compound 364-5, which was used directly in the next step.

### Step 5: synthesis of compound 364-6

Compound 364-5 (400 mg, 1.86 mmol), NPTF (1336 mg, 3.74 mmol), and DMAP (228 mg, 1.86 mmol) were dissolved in DCM (8 mL), and reacted at 25°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 364-6.

### Step 6: synthesis of compound 364-7

Compound 364-6 (260 mg, 1.2 mmol), NH₂Boc (281.7 mg, 2.4 mmol), cesium carbonate (1.17 g, 3.6 mmol) and palladium catalyst (CAS: 1599466-85-9; 102 mg, 0.12 mmol) were dissolved in 1,4-dioxane (8 mL), and the reaction mixture was reacted at 90°C for 4 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 364-7.

### Step 7: synthesis of compound 364-8

Compound 364-7 (300 mg, 0.95 mmol) was dissolved in DCM (3 mL), to which HCl\EA (4 mL) was added, and the reaction mixture was allowed to react at room temperature for 5 hours. The reaction solution was quenched with ice water, neutralized with saturated NaHCO₃, and extracted with EA. The organic phase was washed with saturated NaCl solution, dried over anhydrous Na₂SO₄, and concentrated under reduced pressure to obtain compound 364-8.

### Step 8: synthesis of compound 364-9

Compound 364-8 (100 mg, 0.47 mmol), 4-bromo-2-(4-(difluoromethylene)piperidin-1-yl)benzoic acid (171 mg, 0.52 mmol), and TCFH (144 mg, 0.52 mmol) were dissolved in THF (5 mL), to which DIEA (0.31 mL, 1.88 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 364-9.

### Step 9: synthesis of compound 364

Compound 364-9 (70 mg, 0.01 mmol), 2-hydroxyethanesulfonamide (83 mg, 0.05 mmol), cesium carbonate (130 mg, 0.03 mmol) and palladium catalyst (CAS: 1599466-89-3; 11 mg, 0.001 mmol) were dissolved in 1,4-dioxane (5 mL), and the reaction mixture was reacted at 90°C for 2 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 364 as a solid. MS (ESI, pos.ion) m/z:572.6 [M+1]⁺.

¹H NMR (400 MHz, DMSO-*d*₆) δ: 10.94 (s, 1H), 8.16 (d, *J*=1.0 Hz, 1H), 7.74 (d, *J*=1.0 Hz, 1H), 7.60 (d, *J=*1.0 Hz, 1H), 6.98-7.09 (m, 2H), 6.74 (s, 1H), 4.95 (s, 2H), 4.46 (t, *J*=1.0 Hz, 2H), 4.16 (t, *J*=1.0 Hz, 2H), 3.76 (t, *J*=1.0 Hz, 2H), 3.34 (br s, 2H), 2.97-3.05 (m, 4H), 2.15-2.22 (m, 4H).

### Example 366: synthesis of N-(7-fluoro-3,4-dihydro-1H-[1,4]oxazino[4,3-a]indol-9-yl)-4-((2-hydroxyethyl)sulfonamido)-2-(6-azaspiro[2.5]oct-6-yl)benzamide

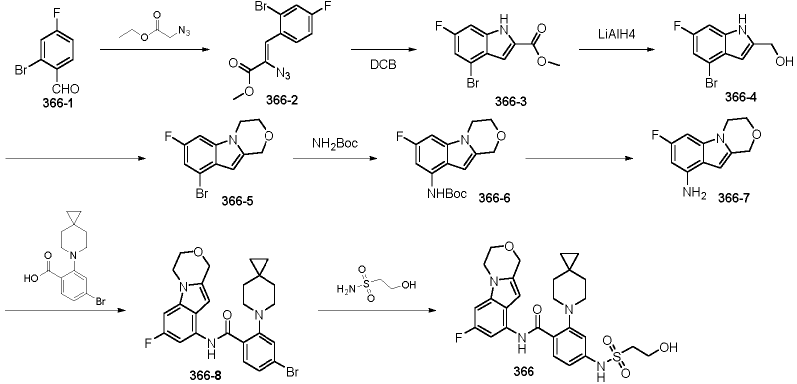

### Step 1: synthesis of compound 366-2

Sodium methoxide (5.4 mol/L, 16.4 mL, 88.67 mmol) was dissolved in methanol (20 mL), to which a solution of compound 366-1 (12.0 g, 59.11 mmol) in methanol (80 mL), and ethyl azidoacetate (10.16 mL, 88.67 mmol) were slowly added dropwise at -15°C, and the mixture was reacted overnight at 0°C. The reaction solution was slowly poured into ice water, and a solid was precipitated and filtered, and the solid was slurried with a small amount of methanol. The resulting slurry was filtered and dried to obtain compound 366-2.

¹H NMR (400 MHz, DMSO-d₆) δ = 8.21 (dd, *J =* 6.4, 8.9 Hz, 1H), 7.71 (dd, *J* = 2.7, 8.6 Hz, 1H), 7.36 (dt, *J=* 2.7, 8.6 Hz, 1H), 7.04 (s, 1H), 3.88 (s, 3H).

### Step 2: synthesis of compound 366-3

Compound 366-2 (6 g, 19.96 mmol) was dissolved in o-dichlorobenzene (30 mL), and the mixture was reacted at 180°C for 5 h under nitrogen protection. The reaction solution was cooled to room temperature, poured slowly into petroleum ether, a solid was precipitated, and the reaction solution was filtered. The crude product was purified by silica gel column chromatography (PE:EA=10:1) to obtain compound 366-3.

### Step 3: synthesis of compound 366-4

Compound 366-3 (955 mg, 3.51 mmol) was dissolved in tetrahydrofuran (10 mL), to which LiAlH₄ (160 mg, 4.21 mmol) was slowly added at 0°C, and the mixture was reacted at 0°C for 40 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 366-4.

### Step 4: synthesis of compound 366-5

Compound 366-4 (697 mg, 2.66 mmol), and KOH (401 mg, 7.15 mmol) were dissolved in DCM (5 mL) and stirred at 0°C for 30 minutes, to which a solution of a catalyst (CAS: 247129-88-0; 1.24 g, 3.43 mmol) in DCM (3 mL) was added, and stirring was continued at 0°C for 30 minutes. The reaction system was moved to room temperature and reacted overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 366-5.

¹H NMR (400 MHz, DMSO-d₆) δ = 7.39 (dd, *J=* 2.1, 9.7 Hz, 1H), 7.23 (dd, *J* = 2.1, 9.3 Hz, 1H), 6.21 - 6.18 (m, 1H), 4.91 (s, 2H), 4.13 - 4.04 (m, 4H).

### Step 5: synthesis of compound 366-6

Compound 366-5 (360 mg, 1.33 mmol), NH₂Boc (312 mg, 2.67 mmol), cesium carbonate (1.30 g, 4.00 mmol) and palladium catalyst (CAS: 1599466-83-7; 245 mg, 0.27 mmol) were dissolved in 1,4-dioxane (10 mL), and the reaction mixture was reacted at 90°C for 3 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1-10:1) to obtain compound 366-6. MS (ESI, pos.ion) m/z:251.0 [M+1-56]⁺.

### Step 6: synthesis of compound 366-7

Compound 366-6 (400 mg, 1.12 mmol) was dissolved in DCM (2 mL), to which HCl\dioxane (3 mL) was added, and the reaction mixture was allowed to react at 25°C for 1 h. The reaction solution was filtered and dried to obtain compound 366-7. MS (ESI, pos.ion) m/z:207.1 [M+1-HCl]⁺.

### Step 7: synthesis of compound 366-8

Compound 366-7 (110 mg, 0.45 mmol), 4-bromo-2-(6-azaspiro[2.5]oct-6-yl)benzoic acid (155 mg, 0.50 mmol), and HATU (190 mg, 0.50 mmol) were dissolved in DMF (3 mL), to which DIEA (0.30 mL, 1.82 mmol) was added, and the reaction mixture was allowed to react at 25°C for 1 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 366-8.

### Step 8: synthesis of compound 366

Compound 366-8 (70 mg, 0.14 mmol), 2-hydroxyethanesulfonamide (70.3 mg, 0.56 mmol), cesium carbonate (137 mg, 0.42 mmol) and palladium catalyst (CAS: 1599466-89-3; 11 mg, 0.01 mmol) were dissolved in 1,4-dioxane (3 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 366. MS (ESI, pos.ion) m/z:543.3 [M+1]⁺.

¹H NMR (400 MHz, DMSO-d₆) δ = 11.20 (s, 1H), 7.91 (dd, *J =* 2.1, 12.5 Hz, 1H), 7.83 (d, *J=* 8.5 Hz, 1H), 7.17 (d, *J=* 2.0 Hz, 1H), 7.09 - 7.00 (m, 2H), 6.51 (s, 1H), 4.92 - 4.89 (m, 2H), 4.16 - 4.11 (m, 2H), 4.09 - 4.04 (m, 2H), 3.77 (t, *J =* 6.5 Hz, 2H), 3.37 - 3.33 (m, 2H), 3.01 (br t, *J =* 4.8 Hz, 4H), 1.45 (br s, 4H), 0.28 (s, 4H).

### Example 367: synthesis of 2-(4-(difluoromethylene)piperidin-1-yl)-N-(7-fluoro-3,4-dihydro-1H-[1,4]oxazino[4,3-a]indol-9-yl)-4-((2-hydroxyethyl) sulfonamido)benzamide

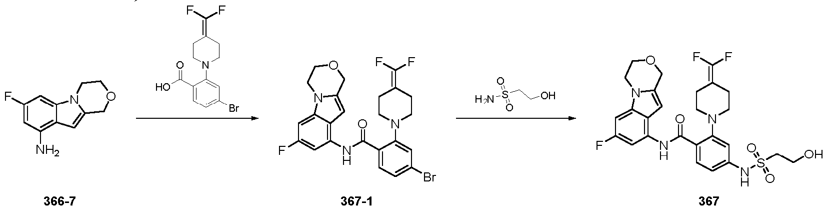

### Step 1: synthesis of compound 367-1

Compound 366-7 (180 mg, 0.74 mmol), 4-bromo-2-(4-(difluoromethylene)piperidin-1-yl)benzoic acid (272 mg, 0.82 mmol), and HATU (311 mg, 0.82 mmol) were dissolved in DMF (3 mL), to which DIEA (0.10 mL, 2.98 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 367-1.

### Step 2: synthesis of compound 367

Compound 367-1 (110 mg, 0.21 mmol), 2-hydroxyethanesulfonamide (106 mg, 0.85 mmol), cesium carbonate (207 mg, 0.63 mmol) and palladium catalyst (CAS: 1599466-89-3; 17 mg, 0.02 mmol) were dissolved in 1,4-dioxane (3 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 367. MS (ESI, pos.ion) m/z:565.2 [M+1]⁺.

¹H NMR (400 MHz, DMSO-d₆) δ = 10.80 (s, 1H), 7.94 (dd, *J =* 1.9, 12.6 Hz, 1H), 7.75 (d, *J =* 8.5 Hz, 1H), 7.09 - 7.05 (m, 2H), 7.03 - 7.00 (m, 1H), 6.52 (s, 1H), 4.89 (s, 2H), 4.14 - 4.10 (m, 2H), 4.07 - 4.03 (m, 2H), 3.77 (t, *J =* 6.5 Hz, 2H), 3.36 - 3.32 (m, 2H), 3.00 (br d, *J =* 5.0 Hz, 4H), 2.22 (br s, 4H).

### Example 368: synthesis of N-(7-fluoro-3,4-dihydro-1H-benzo[4,5]imidazo[2,1-c][1,4]oxazin-9-yl)-4-((2-hydroxyethyl)sulfonamido)-2-(6-azaspiro[2.5]oct-6-yl)benzamide

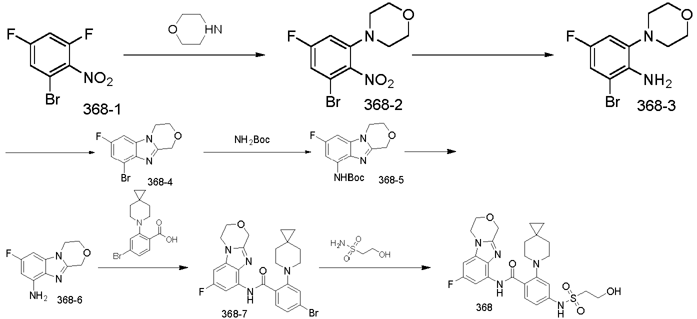

### Step 1: synthesis of compound 368-2

Compound 368-1 (1 g, 4.20 mmol) and morpholine (0.55 mL, 6.30 mmol) were dissolved in dimethyl sulfoxide (12 mL), to which potassium carbonate (1.16 g, 8.40 mmol) was added. The reaction mixture was stirred in an oil bath at 110°C for 2 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-95:5) to obtain compound 368-2.

### Step 2: synthesis of compound 368-3

Compound 368-2 (543 mg, 1.78 mmol) was dissolved in ethanol (5 mL) and water (5 mL), to which iron dust (497 mg, 8.90 mmol) and ammonium chloride (476 mg, 8.90 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by column chromatography (PE:EA=100:0-90:10-80:20) to obtain compound 368-3.

### Step 3: synthesis of compound 368-4

Compound 368-3 (450 mg, 1.63 mmol) was dissolved in dichloromethane (3 mL), to which trifluoroacetic acid (1.05 mL, 14.10 mmol) and hydrogen peroxide (0.42 mL, 14.10 mmol) were added. The reaction mixture was stirred in an oil bath at 40°C for 24 hours. After the reaction was completed, the reaction solution was quenched with anhydrous sodium sulfite, and then diluted with water (30 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain product compound 368-4.

### Step 4: synthesis of compound 368-5

Compound 368-4 (400 mg, 1.48 mmol) and 2-methylprop-2-ylcarbamate (346 mg, 2.95 mmol) were dissolved in 1,4-dioxane (6 mL), to which palladium catalyst (CAS: 1599466-83-7; 67.8 mg, 0.07 mmol) and cesium carbonate (1.45 g, 4.43 mmol) were added. The reaction mixture was stirred in an oil bath at 95°C for 3 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain product compound 368-5. MS m/z (ESI):308.0 [M+1]⁺.

### Step 5: synthesis of compound 368-6

Compound 368-5 (350 mg, 1.14 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the PH reached 9. The reaction solution was extracted with EA (30 mL), and the organic phase was washed with saturated NaCl solution (10 mL), dried over anhydrous Na₂SO₄, and concentrated under reduced pressure to obtain compound 368-6 MS m/z (ESI):208.0 [M+1]⁺.

### Step 6: synthesis of compound 368-7

Compound 368-6 (60 mg, 0.29 mmol) and 4-bromo-2-(6-azaspiro[2.5]octan-6-yl)benzoic acid (90 mg, 0.29 mmol) were dissolved in acetonitrile (3 mL), to which HATU (128 mg, 0.34 mmol) and N,N-diisopropylethylamine (94 mg, 0.72 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 368-7.

### Step 7: synthesis of compound 368

Compound 368-7 (60 mg, 0.12 mmol) and 2-hydroxyethane-1-sulfonamide (60 mg, 0.48 mmol) were dissolved in 1,4-dioxane (3 mL), to which palladium catalyst (CAS: 1599466-89-3; 4.85 mg, 0.05 mmol) and cesium carbonate (117.4 mg, 0.24 mmol) were added. The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by preparative chromatography to obtain compound 368. MS (ESI, pos.ion) m/z: 544.2 [M+1]⁺.

**¹H NMR** (400 MHz, DMSO-d6) δ = 12.76 - 12.71 (m, 1H), 8.23 (dd, *J=* 2.4, 12.8 Hz, 1H), 8.05 (d, *J =* 8.6 Hz, 1H), 7.31 - 7.26 (m, 1H), 7.20 - 7.15 (m, 1H), 7.13 - 7.08 (m, 1H), 4.97 - 4.93 (m, 2H), 4.23 - 4.18 (m, 4H), 3.79 - 3.73 (m, 2H), 3.39 - 3.34 (m, 2H), 3.01 - 2.92 (m, 4H), 1.97 - 1.55 (m, 4H), 0.38 - 0.30 (m, 4H).

### Example 369: synthesis of 2-(4-(difluoromethylene)piperidin-1-yl)-N-(7-fluoro-3,4-dihydro-1H-benzo[4,5]imidazo[2,1-c][1,4]oxazin-9-yl)-4-((2-hydroxyethyl)sulfonamido)benzamide

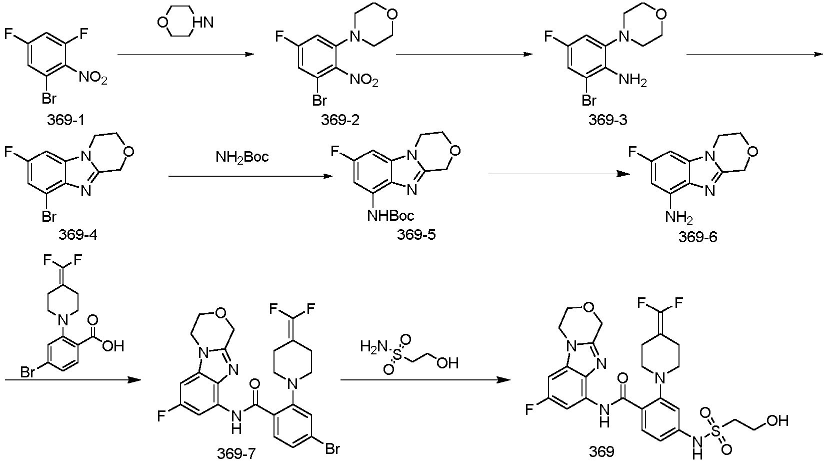

### Step 1: synthesis of compound 369-2

Compound 369-1 (1 g, 4.20 mmol) and morpholine (0.55 mL, 6.30 mmol) were dissolved in dimethyl sulfoxide (12 mL), to which potassium carbonate (1.16 g, 8.40 mmol) was added. The reaction mixture was stirred in an oil bath at 110°C for 2 hours. After the reaction was completed, the reaction solution was diluted with water (100 mL), and extracted with ethyl acetate (30×3 mL). The organic layer was washed with brine (30×3 mL), dried over Na₂SO₄, filtered and concentrated. The crude product was purified by column chromatography (PE:EA=100:0-95:5) to obtain compound 369-2.

### Step 2: synthesis of compound 369-3

Compound 369-2 (543 mg, 1.78 mmol) was dissolved in ethanol (5 mL) and water (5 mL), to which iron dust (497 mg, 8.90 mmol) and ammonium chloride (476 mg, 8.90 mmol) were added. The reaction mixture was stirred in an oil bath at 80°C for 2 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by column chromatography (PE:EA=4:1) to obtain compound 369-3.

### Step 3: synthesis of compound 369-4

Compound 369-3 (450 mg, 1.63 mmol) was dissolved in dichloromethane (3 mL), to which trifluoroacetic acid (1.05 mL, 14.10 mmol) and hydrogen peroxide (0.42 mL, 14.10 mmol) were added. The reaction mixture was stirred in an oil bath at 40°C for 24 hours. After the reaction was completed, the reaction solution was quenched with anhydrous sodium sulfite, and then diluted with water (30 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 369-4.

### Step 4: synthesis of compound 369-5

Compound 369-4 (400 mg, 1.48 mmol) and 2-methylprop-2-ylcarbamate (346 mg, 2.95 mmol) were dissolved in 1,4-dioxane (6 mL), to which palladium catalyst (CAS: 1599466-83-7; 67.8 mg, 0.07 mmol) and cesium carbonate (1.45 g, 4.43 mmol) were added. The reaction mixture was stirred in an oil bath at 95°C for 3 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain product compound 369-5. MS m/z (ESI):308.0 [M+1]⁺.

### Step 5: synthesis of compound 369-6

Compound 369-5 (350 mg, 1.14 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 369-6 MS m/z (ESI):208.0 [M+1]⁺.

### Step 6: synthesis of compound 369-7

Compound 369-6 (60 mg, 0.29 mmol) and 4-bromo-2-(4-(difluoromethylene)piperidin-1-yl)benzoic acid (96 mg, 0.29 mmol) were dissolved in DMF (3 mL), to which HATU (128 mg, 0.34 mmol) and N,N-diisopropylethylamine (94 mg, 0.72 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by column chromatography (PE:EA=100:0-80:20) to obtain compound 369-7.

### Step 7: synthesis of compound 369

Compound 369-7 (60 mg, 0.12 mmol) and 2-hydroxyethane-1-sulfonamide (60 mg, 0.48 mmol) were dissolved in 1,4-dioxane (3 mL), to which palladium catalyst (CAS: 1599466-89-3; 4.85 mg, 0.05 mmol) and cesium carbonate (117.4 mg, 0.24 mmol) were added. The reaction mixture was stirred in an oil bath at 95°C for 1.5 hours under N₂ protection. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by preparative chromatography to obtain compound 369. MS (ESI, pos.ion) m/z: 566.2 [M+1]⁺.

**¹H NMR** (400 MHz, DMSO-d6) δ = 10.88 (s, 1H), 8.14 (d, *J =* 5.4 Hz, 1H), 8.03 (br d, *J =* 5.4 Hz, 1H), 7.73 (d, *J =* 8.4 Hz,1H), 7.06 - 7.03 (m, 1H), 7.01 (dd, *J* = 1.9, 8.5 Hz, 1H), 6.55 (s, 1H), 4.94 (s, 2H), 4.13 (s, 4H), 3.80 - 3.74 (m, 2H), 3.37 -3.33 (m, 2H), 3.04 - 2.97 (m, 4H), 2.20 (br s, 4H).

### Example 370: synthesis of 2-(4-(difluoromethylene)piperidin-1-yl)-N-(3,3-dimethyl-3,4-dihydro-1H-[1,4]oxazino[4,3-a]indol-9-yl)-4-((2-hydroxyethyl)sulfonamido)benzamide

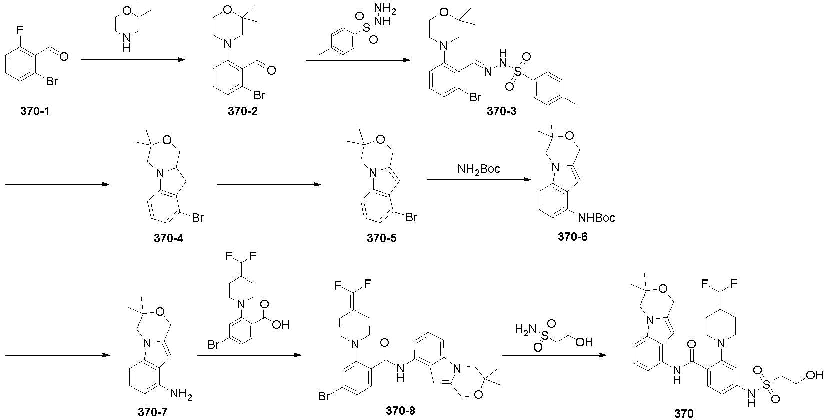

### Step 1: synthesis of compound 370-2

Compound 370-1 (6 g, 29.56 mmol) and 2,2-dimethyl-1,4-oxazinane (4.09 g, 35.47 mmol) were dissolved in N-methylpyrrolidinone (30 mL), to which N,N-diisopropylethylamine (7.64 g, 59.12 mmol) was added, and the reaction mixture was allowed to react at 100°C overnight. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=97:3-90:10) to obtain compound 370-2.

### Step 2: synthesis of compound 370-3

Compound 370-2 (4.10 g, 13.75 mmol) was dissolved in ultra-dry toluene (24 mL), to which N-amino-4-methylbenzenesulfonamide (3.07 g, 16.50 mmol) was added, and the mixture was reacted at room temperature for 1 h. The reaction solution was used directly in the next reaction.

### Step 3: synthesis of compound 370-4

To the reaction solution (reaction solution 370-3) from the previous step was added ultra-dry toluene (24 mL), to which sodium hydride (0.71 g, 25.59 mmol) was added under ice bath conditions and under N₂ protection. The resulting mixture was stirred at 25°C for 0.5 h, followed by reaction at 135°C for 10 minutes. The reaction solution was diluted with ice water, and extracted with EA. The organic phase was washed with saturated NaCl solution, dried over anhydrous Na₂SO₄, concentrated under reduced pressure and the crude product was purified by preparative chromatography to obtain compound 370-4.

### Step 4: synthesis of compound 370-5

Compound 370-4 (500 mg, 1.77 mmol) and manganese dioxide (1.54 g, 17.70 mmol) were dissolved in dioxane (10 mL) and the mixture was reacted at room temperature for 1.5 h. The reaction system was subjected to suction filtration, and the filtrate was diluted with water, and extracted with EA. The organic phase was washed with saturated NaCl solution, dried over anhydrous Na₂SO₄, and concentrated under reduced pressure. The crude product was purified by silica gel column chromatography (PE:EA=97:3-90:10) to obtain compound 370-5.

### Step 5: synthesis of compound 370-6

Compound 370-5 (427 mg, 1.52 mmol), 2-methylprop-2-ylcarbamate (357.09 mg, 3.09 mmol), cesium carbonate (1.49 g, 4.57 mmol) and palladium catalyst (CAS: 1599466-81-5; 65.57 mg, 0.08 mmol) were dissolved in 1,4-dioxane (8 mL), and the reaction mixture was reacted at 95°C for 1.5 h under N₂ protection. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=90:10) to obtain compound 370-6.

### Step 6: synthesis of compound 370-7

Compound 370-6 (450 mg, 1.42 mmol) was dissolved in hydrogen chloride-ethyl acetate solution (2 M, 6 mL) and the mixture was reacted at room temperature for 1 h. The reaction solution was quenched with ice-cold sodium bicarbonate solution, and extracted with EA. The organic phase was washed with saturated NaCl solution, dried over anhydrous Na₂SO₄, and concentrated under reduced pressure to obtain compound 370-7. MS m/z (ESI):217.0 [M+1]⁺.

### Step 7: synthesis of compound 370-8

Compound 370-7 (290 mg, 1.34 mmol), 4-bromo-2-[4-(difluoromethylene)hexahydropyridin-1-yl]benzoic acid (489.89 mg, 1.47 mmol), 2-(7-azabenzotriazole)-N,N,N',N'-tetramethyluronium hexafluorophosphate (611.82 mg, 1.61 mmol) and N,N-diisopropylethylamine (346.61 mg, 2.68 mmol) were dissolved in N,N-dimethylformamide (6 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=80:20) to obtain compound 370-8.

### Step 8: synthesis of compound 370

Compound 370-8 (270 mg, 0.51 mmol), 2-hydroxyethanesulfonamide (254.81 mg, 2.04 mmol), cesium carbonate (331.71 mg, 1.02 mmol) and palladium catalyst (CAS: 1599466-89-3; 20.57 mg, 0.03 mmol) were dissolved in 1,4-dioxane (5 mL), and the reaction mixture was reacted at 95°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 370. MS m/z (ESI):575.3 [M+1]⁺.

**¹H NMR** (400 MHz, DMSO-d₆) δ = 10.75 (s, 1H), 7.89 (br d, *J =* 7.6 Hz, 1H), 7.78 (d, *J =* 8.4 Hz, 1H), 7.18 (br d, *J =* 8.0 Hz, 1H), 7.13 - 7.06 (m, 2H), 7.02 (br d, *J* = 8.5 Hz, 1H), 6.46 (s, 1H), 4.95 (s, 2H), 3.93 (s, 2H), 3.77 (t, *J =* 6.5 Hz, 2H), 3.32 - 3.23 (m, 2H), 3.02 (m, 4H), 2.24 (m, 4H), 1.33 (s, 6H).

### Example 371: synthesis of N-(3,3-dimethyl-3,4-dihydro-1H-[1,4]oxazino[4,3-a]indol-9-yl)-4-(2-hydroxyethylsulfonamido)-2-(6-azaspiro[2.5]octan-6-yl)benzamide

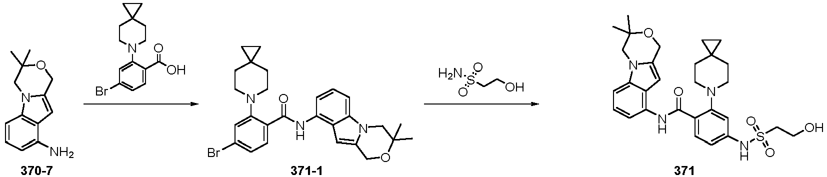

### Step 1: synthesis of compound 371-1

Compound 370-7 (53 mg, 0.25 mmol), 2-(6-azaspiro[2.5]octan-6-yl)-4-bromobenzoic acid (83.61 mg, 0.27 mmol), 2-(7-azabenzotriazole)-N,N,N',N'-tetramethyluronium hexafluorophosphate (111.81 mg, 0.29 mmol) and N,N-diisopropylethylamine (63.35 mg, 0.49 mmol) were dissolved in acetonitrile (3 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=80-20) to obtain compound 371-1.

### Step 2: synthesis of compound 371

Compound 371-1 (103 mg, 0.20 mmol), 2-hydroxyethanesulfonamide (101.40 mg, 0.81 mmol), cesium carbonate (132.00 mg, 0.41 mmol) and palladium catalyst (CAS: 1599466-89-3; 8.18 mg, 0.01 mmol) were dissolved in 1,4-dioxane (4 mL), and the reaction mixture was reacted at 95°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 371. MS m/z (ESI):553.3 [M+1]⁺.

**¹H NMR** (400 MHz, DMSO-d₆) δ = 11.21 (s, 1H), 7.86 (dd, *J =* 5.3, 7.9 Hz, 2H), 7.20 - 7.15 (m, 2H), 7.10 (d, *J =* 7.9 Hz, 1H), 7.01 (br d, *J =* 8.4 Hz, 1H), 6.45 (s, 1H), 4.96 (s, 2H), 3.95 (s, 2H), 3.77 (t, *J =* 1.0 Hz, 2H), 3.31 - 3.29 (m, 2H), 3.01 (m, 4H), 1.51 - 1.41 (m, 4H), 1.34 (s, 6H), 0.27 (s, 4H).

### Example 372: synthesis of N-(4-(cyclopropylmethyl)-5-fluoro-3,4-dihydro-2H-benzo[b][1,4]oxazin-8-yl)-4-((2-hydroxyethyl)sulfonamido)-2-(6-azaspiro[2.5]oct-6-yl)benzamide

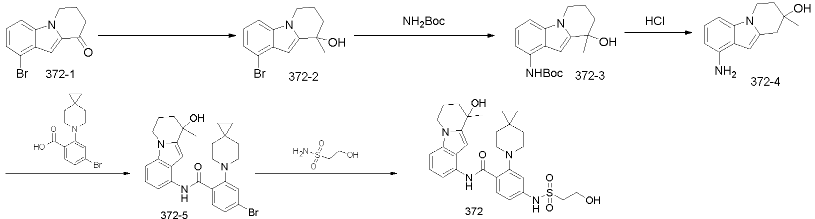

### Step 1: synthesis of compound 372-2

Compound 372-1 (420 mg, 1.59 mmol) was dissolved in tetrahydrofuran (5 mL). Methyl magnesium bromide (2.39 ml, 4.79 mmol) was added dropwise at 0°C under nitrogen protection, and the reaction solution was moved to room temperature and reacted for 1 hour. The reaction solution was quenched by adding water (8 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 372-2.

### Step 2: synthesis of compound 372-3

Compound 372-2 (230 mg, 0.82 mmol), tert-butyl carbamate (192 mg, 1.64 mmol), cesium carbonate (802 mg, 2.46 mmol) and palladium catalyst (CAS: 1599466-85-9; 69 mg, 0.08 mmol) were dissolved in dioxane (5 mL), and the reaction mixture was reacted at 90°C for 12 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 372-3.

### Step 3: synthesis of compound 372-4

Compound 372-3 (60 mg, 0.18 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (20 mL), and the organic phase was washed with saturated NaCl solution (10 mL), dried over anhydrous Na₂SO₄, and concentrated under reduced pressure to obtain compound 372-4 which was used directly in the next step without purification.

### Step 4: synthesis of compound 372-5

Compound 372-4 (61 mg, 0.2 mmol), HATU (75 mg, 0.2 mmol) and *N,N-*diisopropylethylamine (0.12 ml, 0.72 mmol) were dissolved in DMF (4 ml), and the reaction mixture was reacted at room temperature for 2 hours under nitrogen protection. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 372-5.

### Step 5: synthesis of compound 372

Compound 372-5 (40 mg, 0.08 mmol), 2-hydroxyethylsulfonamide (50 mg, 0.4 mmol), cesium carbonate (78 mg, 0.24 mmol) and palladium catalyst (CAS: 1599466-89-3; 7 mg, 0.008 mmol) were dissolved in 1,4-dioxane (5 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na2SO4, and concentrated under reduced pressure. The crude product was purified by silica gel column chromatography (PE/EA= 1/0 to 3/1) to obtain compound 372. MS (ESI, pos.ion) m/z: 553.2 [M+1]+.

¹H NMR (DMSO-*d*₆ ) δ: 11.59 (s, 1H), 8.13 (d, *J*=1.0 Hz, 1H), 7.96 (d, *J*=1.0 Hz, 1H), 7.34 (d, *J*=1.0 Hz, 1H), 7.23 (dd, *J*=1.0 Hz, 1H), 7.10 (d, *J*=1.0 Hz, 1H), 6.98 (dd, *J*=1.0 Hz, 1H), 6.54 (s, 1H), 5.75-5.81 (m, 1H), 4.04-4.13 (m, 4H), 3.24-3.31 (m, 2H), 3.09-3.16 (m, 4H), 2.59-2.68 (m, 2H), 2.09 (s, 3H), 1.52-1.63 (m, 6H), 0.29-0.34 (m, 4H).

### Example 373: synthesis of 4-(ethylsulfonamido)-N-(9-hydroxy-6,7,8,9-tetrahydropyridino[1,2-a]indol-1-yl)-2-(6-azaspiro[2.5]oct-6-yl)benzamide

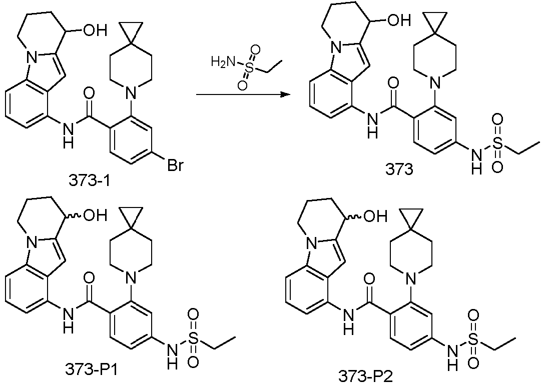

Compound 373-1 (100 mg, 0.34 mmol), ethanesulfonamide (211 mg, 1.69 mmol), cesium carbonate (331 mg, 1.02 mmol) and palladium catalyst (CAS: 1599466-89-3; 27 mg, 0.03 mmol) were dissolved in 1,4-dioxane (6 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 3/1) to obtain compound 373 (67 mg, 32%).

For purification of compound 373 by preparative SFC, the following conditions were used: column: ChiralPak IG, 250×30mm I.D., 10µm particles; mobile phase A: 60/40 CO₂/EtOH (0.1% NH₃H₂O); detector wavelength: 220 nm; flow rate: 150 mL/min.

Compound 373-P1(32 mg) MS m/z (ESI):523.2 [M+1]⁺ retention time=2.943 min;
Compound 373-P1: ¹H NMR (DMSO-*d*₆ ) δ: 11.21 (s, 1H), 10.10-10.17 (m, 1H), 7.88 (t, *J=1.0* Hz, 2H), 7.22 (d, *J=1.0* Hz, 1H), 7.16 (d, *J=1.0* Hz, 1H), 7.07 (dd, *J=1.0* Hz, 2H), 6.60 (s, 1H), 5.36 (d, *J=1.0* Hz, 1H), 4.80-4.87 (m, 1H), 3.91-4.09 (m, 2H), 3.22 (q, *J=1.0* Hz, 2H), 3.00-3.07 (m, 4H), 2.17-2.28 (m, 1H), 1.90-2.08 (m, 2H), 1.69-1.81 (m, 1H), 1.44-1.55 (m, 4H), 1.22 (t, *J=1.0* Hz, 3H), 0.28 (s, 4H).

Compound 373-P2(33 mg) MS m/z (ESI):523.2 [M+1]⁺ retention time=4.422 min;
Compound 373-P2: ¹HNMR (DMSO-*d*₆ ) δ: 11.3 (s, 1H), 10.10-10.17 (m, 1H), 7.85-7.91 (t, *J=1.0* Hz, 2H), 7.23 (d, *J=1.0* Hz, 1H), 7.16 (d, *J=1.0* Hz, 1H), 7.01-7.07 (dd, *J=1.0* Hz, 2H), 6.60 (s, 1H), 5.36 (d, *J=1.0* Hz, 1H), 4.80-4.87 (m, 1H), 3.91-4.09 (m, 2H), 3.18-3.25 (q, *J=1.0* Hz, 2H), 3.00-3.07 (m, 4H), 2.17-2.28 (m, 1H), 1.90-2.08 (m, 2H), 1.69-1.81 (m, 1H), 1.44-1.55 (m, 4H), 1.22 (t, *J=1.0* Hz, 3H), 0.28 (s, 4H).

### Example 374: synthesis of N-(8,8-difluoro-6,7,8,9-tetrahydropyridino[1,2-a]indol-1-yl)-2-(4-(difluoromethylene)piperidin-1-yl)-4-((2-hydroxyethyl)sulfonamido)benzamide

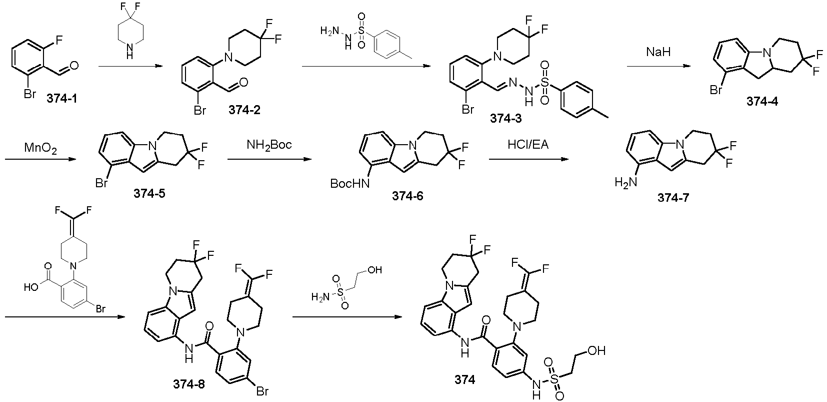

### Step 1: synthesis of compound 374-2

Compound 374-1 (1 g, 4.93 mmol), and granular potassium carbonate (1.7 g, 12.31 mmol) were dissolved in DMSO (8 mL), to which 4,4-difluoropiperidine hydrochloride (930 mg, 5.91 mmol) was added, and the reaction mixture was allowed to react at 95°C for 5 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 374-2.

### Step 2: synthesis of compound 374-3

Compound 374-2 (400 mg, 1.32 mmol) was dissolved in toluene (8 mL), to which p-toluenesulfonyl hydrazide (295 mg, 1.58 mmol) was added, and the mixture was reacted at room temperature for 10 minutes. The reaction solution was quenched with ice water, and extracted with EA. The organic phase was washed with saturated NaCl solution, dried over anhydrous Na₂SO₄, and concentrated under reduced pressure to obtain compound 374-3.

### Step 3: synthesis of compound 374-4

Compound 374-3 (1.3 g, 2.75 mmol) was dissolved in toluene (60 mL), to which NaH (120 mg, 3.03 mmol) was slowly added at 0°C, and after stirring for 30 minutes, the mixture was reacted at 135°C for 10 minutes under microwave irradiation. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 374-4.

### Step 4: synthesis of compound 374-5

Compound 374-4 (505 mg, 1.75 mmol) was dissolved in DCM (10 mL), to which manganese dioxide (1.52 g, 17.50 mmol) was added, and the mixture was reacted at room temperature for 2 days. The reaction solution was filtered, and concentrated under reduced pressure. The crude product was purified by silica gel column chromatography (PE:EA=10:1) to obtain compound 374-5.

### Step 5: synthesis of compound 374-6

Compound 374-5 (190 mg, 0.66 mmol), NH₂Boc (156 mg, 1.33 mmol), cesium carbonate (649 mg, 1.99 mmol) and the fourth-generation palladium catalyst (CAS: 1599466-85-9; 63 mg, 0.07 mmol) were dissolved in 1,4-dioxane (3 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 374-6.

### Step 6: synthesis of compound 374-7

Compound 374-6 (164 mg, 0.51 mmol) was dissolved in DCM (2 mL), to which HCl\EA (3 mL) was added, and the reaction mixture was allowed to react at 25°C for 1 hour. The reaction solution was filtered and dried to obtain compound 374-7 as a solid.

### Step 7: synthesis of compound 374-8

Compound 374-7 (110 mg, 0.43 mmol), 4-bromo-2-(4-(difluoromethylene)piperidin-1-yl)benzoic acid (170 mg, 0.51 mmol), and HATU (178 mg, 0.47 mmol) were dissolved in DMF (3 mL), to which DIEA (0.28 mL, 1.71 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 374-8 as a solid.

### Step 8: synthesis of compound 374

Compound 374-8 (100 mg, 0.19 mmol), 2-hydroxyethanesulfonamide (93 mg, 0.75 mmol), cesium carbonate (182 mg, 0.56 mmol) and the fourth-generation palladium catalyst (CAS: 1621274-19-8; 36 mg, 0.04 mmol) were dissolved in 1,4-dioxane (3 mL), and the reaction mixture was reacted at 100°C for 2 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 374. MS (ESI, pos.ion) m/z:581.3 [M+1]⁺.

¹H NMR (400 MHz, DMSO-d₆) δ = 10.74 (s, 1H), 10.09 (br s, 1H), 7.90 (d, *J* = 7.8 Hz, 1H), 7.78 (d, *J =* 8.4 Hz, 1H), 7.21 (d, *J =* 8.0 Hz, 1H), 7.16 - 7.09 (m, 1H), 7.09 - 7.06 (m, 1H), 7.05 - 6.98 (m, 1H), 6.53 (s, 1H), 4.96 (br s, 1H), 4.24 (t, *J =* 6.3 Hz, 2H), 3.77 (br t, *J =* 6.6 Hz, 2H), 3.56 (br t, *J =* 14.6 Hz, 2H), 3.36 - 3.32 (m, 2H), 3.05 - 2.98 (m, 4H), 2.68 - 2.58 (m, 2H), 2.27 - 2.21 (m, 4H).

### Example 376: synthesis of N-(7,7-difluoro-6,7,8,9-tetrahydropyridino[3,2-b]indolizin-4-yl)-2-(4-(difluoromethylene)piperidin-1-yl)-4-(((2-hydroxyethyl)sulfonamido)benzamide (376)

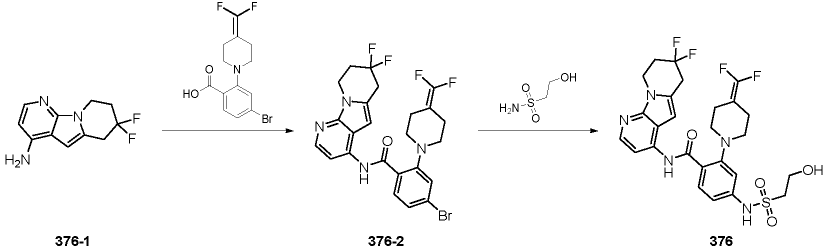

### Step 1: synthesis of compound 376-2

Compound 376-1 (200 mg, 0.77 mmol), 4-bromo-2-(4-(difluoromethylene)piperidin-1-yl)benzoic acid (256 mg, 0.77 mmol), and TCFH (325 mg, 1.16 mmol) were dissolved in THF (3 mL), to which DIEA (0.51 mL, 3.09 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 376-2.

### Step 2: synthesis of compound 376

Compound 376-2 (40 mg, 0.07 mmol), 2-hydroxyethanesulfonamide (37 mg, 0.28 mmol), cesium carbonate (73 mg, 0.21 mmol) and the fourth-generation palladium catalyst (CAS: 1599466-89-3; 6 mg, 0.01 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 376. MS (ESI, pos.ion) m/z:582.2 [M+1]⁺.

¹H NMR (400 MHz, DMSO-d₆) δ = 10.88 (s, 1H), 8.15 (d, *J =* 5.5 Hz, 1H), 8.05 - 7.98 (m, 1H), 7.73 (d, *J =* 8.5 Hz, 1H), 7.06 (s, 1H), 7.04 - 6.99 (m, 1H), 6.63 (s, 1H), 4.29 (br t, *J =* 6.3 Hz, 2H), 3.77 (t, *J =* 6.5 Hz, 2H), 3.59 (br t, *J =* 14.5 Hz, 2H), 3.38 - 3.34 (m, 2H), 3.00 (br s, 4H), 2.68 - 2.57 (m, 2H), 2.21 (br s, 4H).

### Example 378: synthesis of N-(4-cyano-8,8-difluoro-6,7,8,9-tetrahydropyridino[1,2-a]indol-1-yl)-2-(4-(difluoromethylene)piperidin-1-yl)-4-(((2-hydroxyethyl)sulfonamido)benzamide (378)

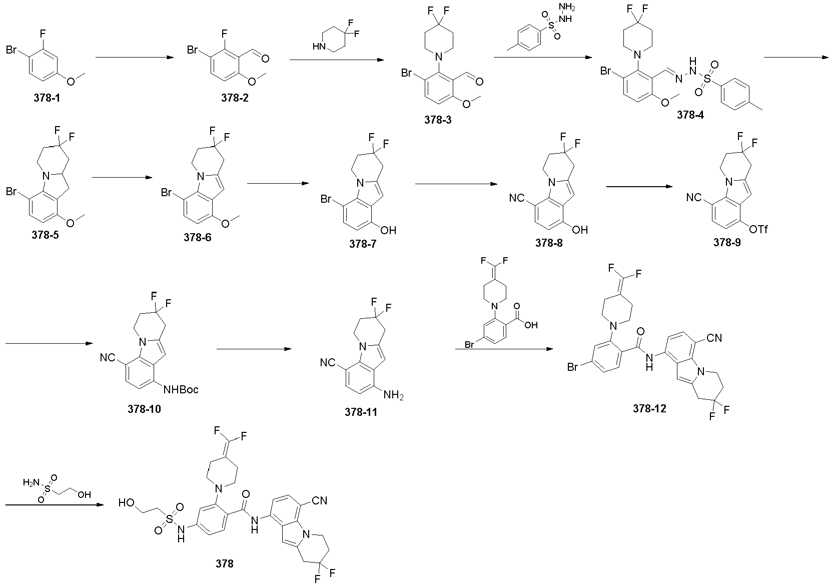

### Step 1: synthesis of compound 378-2

Compound 378-1 (20 g, 97.55 mmol) was dissolved in THF (200 mL), to which LDA (58.53 mL, 117.06 mmol, 2 M) was slowly added dropwise at -78°C, and the reaction mixture was allowed to react at -78°C for 20 min. DMF (9.10 mL, 117.06 mmol) was then slowly added dropwise to the reaction solution, and the reaction mixture was allowed to react at -78°C for 10 min. The reaction was quenched by dropwise addition of acetic acid (22.31 mL, 390.19 mmol) to the reaction solution at -78°C, followed by addition of water. The reaction solution was extracted with EA. The organic phase was washed with saturated NaCl solution, dried over anhydrous Na₂SO₄, and concentrated under reduced pressure, and the crude product was slurried with PE and purified to obtain compound 378-2.

### Step 2: synthesis of compound 378-3

Compound 378-2 (8.00 g, 34.33 mmol) and 4,4-difluorohexahydropyridine (8.15 g, 51.49 mmol) were dissolved in DMF (100 mL), K₂CO₃ (11.86 g, 85.83 mmol) was then added, and the mixture was reacted at 120°C for 24 h. The reaction solution was allowed to cool to room temperature and filtered, and the filter cake was washed with EA. The filtrate was poured into water and then extracted with EA. The organic phase was washed with saturated sodium chloride aqueous solution, then dried over anhydrous Na₂SO₄, and concentrated under reduced pressure. The crude product was purified by silica gel column chromatography (PE:EA = 100/0-10/1) to obtain compound 378-3.

### Step 3: synthesis of compound 378-4

Compound 378-3 (5.00 g, 14.96 mmol) and 4-methylbenzenesulfonyl hydrazide (2.845 g, 15.26 mmol) were dissolved in xylene (70 mL), and reacted at 25°C for 30 min. After monitoring the complete reaction of raw materials, the reaction solution containing compound 378-4 was obtained and used directly in the next reaction.

### Step 4: synthesis of compound 378-5

NaH (778.08 mg, 19.45 mmol, 60% purity) was added to the reaction solution containing the synthetic compound 378-4 at 25°C, and the reaction mixture was allowed to react at 140°C for 1.5 h. The reaction solution was quenched with water, and extracted with EA. The organic phase was dried over anhydrous Na₂SO₄, and concentrated under reduced pressure. The crude product was purified by silica gel column chromatography (PE:EA=100/1-20/1) to obtain compound 378-5.

### Step 5: synthesis of compound 378-6

Compound 378-5 (2400 mg, 7.54 mmol) was dissolved in dichloromethane (30 mL), to which active manganese dioxide (3279 mg, 37.72 mmol) was added, and the mixture was reacted at 45°C for 18 h. It should be detected whether the raw materials had reacted completely. If the reaction was not completed, the reaction solution was filtered through diatomaceous earth and concentrated, and active manganese dioxide was added for reaction. If the raw materials were completely consumed, the reaction solution was filtered through diatomaceous earth, the filter cake was rinsed several times with DCM, and the organic phase was concentrated under reduced pressure. The crude product was purified by silica gel column chromatography (PE:EA = 100/1-20/1) to obtain compound 378-6.

### Step 6: synthesis of compound 378-7

Compound 378-6 (860 mg, 2.72 mmol) was dissolved in DCM (30 mL), to which boron tribromide (852 uL, 9.0 mmol, previously dissolved in 3 mL of DCM) was added dropwise at 0°C, and the reaction mixture was allowed to react at 0°C for 3 h. After the raw materials were completely consumed, the reaction solution was cooled to -65°C, quenched by the dropwise addition of methanol, and then poured into saturated sodium bicarbonate solution. The mixture was extracted with EA. The organic phase was then washed with saturated NaCl solution, dried over anhydrous Na₂SO₄, and concentrated under reduced pressure to obtain crude product compound 378-7.

### Step 7: synthesis of compound 378-8

Compound 378-7 (910 mg, 3.01 mmol), Zn(CN)₂ (191.89 mg, 1.64 mmol), dppf (170 mg, 0.30 mmol) and palladium catalyst (CAS: 14221-01-3; 348 mg, 0.30 mmol) were dissolved in DMF (20 mL), and the reaction mixture was reacted at 130°C for 1.5 h under N₂ protection. The reaction solution was poured into water, and extracted with EA. The organic phase was washed with saturated NaCl solution, dried over anhydrous Na₂SO₄, and concentrated under reduced pressure. The crude product was purified by silica gel column chromatography (PE:EA=100/1-3/1) to obtain compound 378-8. MS m/z (ESI):249.1 [M+1]⁺.

### Step 8: synthesis of compound 378-9

Compound 378-8 (293 mg, 1.18 mmol) was dissolved in DCM (20 mL), and the reaction solution was cooled to 0°C. Triethylamine (0.33 mL, 2.36 mmol) and N-phenylbis(trifluoromethanesulfonyl)imide (548.17 mg, 1.53 mmol) were added, and the reaction mixture was reacted at 25°C for 3 h under N₂ protection. The reaction solution was poured into water, and extracted with DCM. The organic phase was washed with saturated NaCl solution, dried over anhydrous Na₂SO₄, and concentrated under reduced pressure. The crude product was purified by silica gel column chromatography (PE:EA=100/1-10/1) to obtain compound 378-9.

### Step 9: synthesis of compound 378-10

Compound 378-9 (302 mg, 0.79 mmol), NH₂Boc (139.5 mg, 1.19 mmol), Cs₂CO₃ (517.5 mg, 1.59 mmol) and palladium catalyst (CAS: 1599466-85-9; 67.5 mg, 0.08 mmol) were dissolved in 1,4-dioxane (10 mL), and the reaction mixture was reacted at 90°C for 2 h under N₂ protection. The reaction solution was poured into water, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 378-10.

### Step 10: synthesis of compound 378-11

Compound 378-10 (178 mg, 0.51 mmol) was dissolved in EA (2 mL), to which HCl\EA (8 mL, 2M) was added, and the reaction mixture was allowed to react at 25°C for 3 h. The reaction solution was directly subjected to rotary evaporation to dryness to obtain compound 378-11. MS m/z (ESI):248.0 [M+1]⁺.

### Step 11: synthesis of compound 378-12

Compound 378-11 (52 mg, 0.18 mmol) and 4-bromo-2-(4-(difluoromethylene)piperidin-1-yl)benzoic acid (72.9 mg, 0.22 mmol) were dissolved in THF (5 mL), to which (2,4-dimethyl-2,4-diazapentan-3-ylidene)hexafluoro-λ5-phosphazene (102.7 mg, 0.37 mmol) and DIEA (0.15 mL, 0.91 mmol) were added, and the reaction mixture was allowed to react at 25°C for 2 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 378-12. MS m/z (ESI):561.0\563.0 [M+1]⁺.

### Step 12: synthesis of compound 378

Compound 378-12 (80 mg, 0.14 mmol), 2-hydroxyethanesulfonamide (35.67 mg, 0.29 mmol), cesium carbonate (162.51 mg, 0.50 mmol) and palladium catalyst (CAS: 1599466-89-3; 11.52 mg, 0.01 mmol) were dissolved in 1,4-dioxane (5 mL), and the reaction mixture was reacted at 90°C for 2 h under N₂ protection. The reaction solution was poured into water, and extracted with EA. The organic phase was washed with saturated NaCl solution, dried over anhydrous Na₂SO₄, and concentrated under reduced pressure. The crude product was purified by silica gel column chromatography (PE:EA=100/1-1/1) to obtain compound 378. MS m/z (ESI):606.2 [M+1]⁺.

**¹H NMR** (CHLOROFORM-d) δ: 11.30 (s, 1H), 8.11-8.20 (m, 2H), 7.56 (d, *J* = 8.0 Hz, 1H), 7.24 (s, 1H), 6.98-7.08 (m, 1H), 6.96 (s, 1H), 6.42 (s, 1H), 4.78 (t, *J* = 6.4 Hz, 2H), 4.16 (br t, *J =* 4.8 Hz, 2H), 3.50 (t, *J =* 13.6 Hz, 2H), 3.29-3.39 (t, *J* = 4.8 Hz, 2H), 3.11 (br t, *J =* 5.2 Hz, 4H), 2.59 (tt, *J =* 12.8, 6.0 Hz, 2H), 2.39-2.49 (m, 1H), 2.35 (br s, 4H).

### Example 379: synthesis of 2-(4-(difluoromethylene)piperidin-1-yl)-4-((2-hydroxyethyl)sulfonamido)-N-(7,8,9,10-tetrahydro-6H-7,10-epoxyazeto[1,2-a]indol-1-yl)benzamide (379)

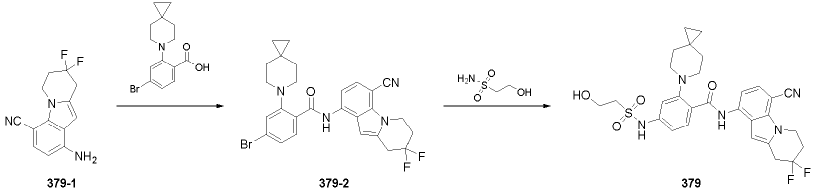

### Step 1: synthesis of compound 379-2

Compound 379-1 (70 mg, 0.25 mmol) and 2-(6-azaspiro[2.5]oct-6-yl)-4-bromobenzoic acid (91.68 mg, 0.30 mmol) were dissolved in THF (5 mL), to which (2,4-dimethyl-2,4-diazapentan-3-ylidene)hexafluoro-λ5-phosphazene (138.22 mg, 0.49 mmol) and DIEA (0.20 mL, 1.23 mmol) were added, and the reaction mixture was allowed to react at 25°C for 2 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 379-2. MS m/z (ESI):539.1\541.1 [M+1]⁺.

### Step 2: synthesis of compound 379

Compound 379-2 (103 mg, 0.19 mmol), 2-hydroxyethanesulfonamide (47.79 mg, 0.38 mmol), cesium carbonate (217.75 mg, 0.67 mmol) and palladium catalyst (CAS: 1599466-89-3; 15.44 mg, 0.02 mmol) were dissolved in 1,4-dioxane (5 mL), and the reaction mixture was reacted at 90°C for 2 h under N₂ protection. The reaction solution was poured into water, and extracted with EA. The organic phase was washed with saturated NaCl solution, dried over anhydrous Na₂SO₄, and concentrated under reduced pressure. The crude product was purified by silica gel column chromatography (PE:EA=100/1-1/1) to obtain compound 379. MS m/z (ESI):584.2 [M+1]⁺.

**¹H NMR** (CHLOROFORM-d) δ: 11.68 (br s, 1H), 8.16 (br dd, *J =* 8.0, 4.0 Hz, 2H), 7.56 (d, *J =* 8.0 Hz, 1H), 7.30-7.35 (m, 1H), 7.01 (br d, *J =* 8.0 Hz, 1H), 6.96 (br s, 1H), 6.54 (s, 1H), 4.79 (br t, *J =* 6.4 Hz, 2H), 4.16 (br s, 2H), 3.52 (br t, *J =* 13.6 Hz, 2H), 3.35 (br t, *J =* 4.8 Hz, 2H), 3.13 (br s, 4H), 2.59 (td, *J =* 12.8, 6.0 Hz, 2H), 2.44-2.54 (m, 1H), 1.52-1.58 (m, 4H), 0.36 (s, 4H).

### Example 380: synthesis of N-(7,7-difluoro-6,7,8,9-tetrahydropyridin[3,2-b]indolizin-4-yl)-4-((2-hydroxyethyl)sulfonamido)-2-(6-azaspiro[2.5]oct-6-yl)benzamide (380)

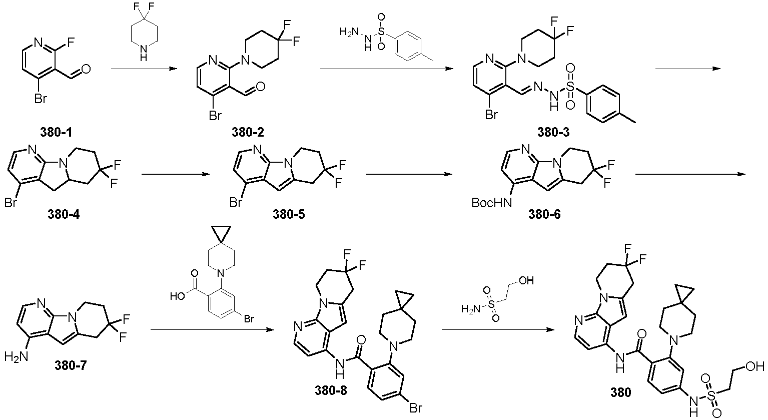

### Step 1: synthesis of compound 380-2

Compound 380-1 (4.78 g, 23.43 mmol), and granular potassium carbonate (8.1 g, 8.58 mmol) were dissolved in DMSO (30 mL), to which 4,4-difluoropiperidine hydrochloride (4.43 g, 28.12 mmol) was added, and the reaction mixture was allowed to react at 95°C for 3 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 380-2.

### Step 2: synthesis of compound 380-3.

Compound 380-2 (1.0 g, 3.28 mmol) was dissolved in toluene (6 mL), to which p-toluenesulfonyl hydrazide (730 mg, 3.94 mmol) was added, and the mixture was reacted at room temperature for 10 minutes. The reaction solution was quenched with ice water, and extracted with EA. The organic phase was washed with saturated NaCl solution, dried over anhydrous Na₂SO₄, and concentrated under reduced pressure to obtain compound 380-3.

### Step 3: synthesis of compound 380-4

Compound 380-3 (1.55 g, 3.29 mmol) was dissolved in toluene (25 mL), to which NaH (140 mg, 3.61 mmol) was slowly added at 0°C, and after stirring for 30 minutes, the mixture was reacted at 135°C for 10 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 380-4.

### Step 4: synthesis of compound 380-5

Compound 380-4 (861 mg, 2.98 mmol) was dissolved in dioxane (10 mL), to which DDQ (1.35 g, 5.96 mmol) was added, and the mixture was reacted at 100°C for 3 h. The reaction solution was quenched with saturated NaHCO₃ solution, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 380-5.

### Step 5: synthesis of compound 380-6

Compound 380-5 (527 mg, 1.84 mmol), NH₂Boc (430 mg, 3.67 mmol), cesium carbonate (1.79 g, 5.51 mmol) and the fourth-generation palladium catalyst (CAS: 1599466-85-9; 156 mg, 0.18 mmol) were dissolved in 1,4-dioxane (10 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 380-6.

### Step 6: synthesis of compound 380-7

Compound 380-6 (574 mg, 1.78 mmol) was dissolved in DCM (2 mL), to which HCl\dioxane (3 mL) was added, and the reaction mixture was allowed to react at 25°C for 1 hour. The reaction solution was filtered and dried to obtain compound 380-7.

### Step 7: synthesis of compound 380-8

Compound 380-7 (110 mg, 0.39 mmol), 4-bromo-2-(6-azaspiro[2.5]oct-6-yl)benzoic acid (122 mg, 0.39 mmol), and TCFH (166 mg, 0.59 mmol) were dissolved in THF (3 mL), to which DIEA (0.26 mL, 1.58 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 380-8.

### Step 8: synthesis of compound 380

Compound 380-8 (100 mg, 0.19 mmol), 2-hydroxyethanesulfonamide (97 mg, 0.78 mmol), cesium carbonate (190 mg, 0.58 mmol) and the fourth-generation palladium catalyst (CAS: 1599466-89-3; 15 mg, 0.02 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 380. MS (ESI, pos.ion) m/z:560.2 [M+1]⁺.

¹H NMR (400 MHz, DMSO-d₆) δ = 11.24 (s, 1H), 10.13 (br s, 1H), 8.16 (d, *J* = 5.4 Hz, 1H), 8.00 (br d, *J =* 5.4 Hz, 1H), 7.81 (d, *J =* 8.5 Hz, 1H), 7.17 (s, 1H), 7.08 - 6.98 (m, 1H), 6.63 (s, 1H), 4.97 (br t, *J* = 5.4 Hz, 1H), 4.31 (br t, *J* = 6.3 Hz, 2H), 3.77 (q, *J =* 6.1 Hz, 2H), 3.65 - 3.55 (m, 2H), 3.37 - 3.35 (m, 2H), 3.00 (br s, 4H), 2.70 - 2.59 (m, 2H), 1.45 (br s, 4H), 0.28 (s, 4H).

### Example 381: synthesis of N-(8,8-difluoro-9-hydroxy-6,7,8,9-tetrahydropyridino[1,2-a]indol-1-yl)-2-(4-(difluoromethylene)piperidin-1-yl)-4-ethyl sulfonamidobenzamide

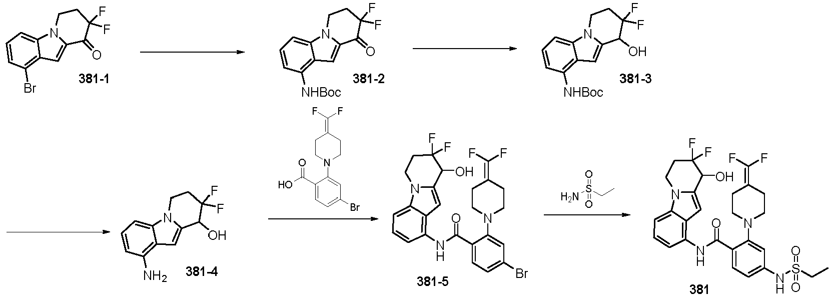

### Step 1: synthesis of compound 381-2

Compound 381-1 (600 mg, 2.01 mmol), cesium carbonate (1.96 g, 6.02 mmol), tBuXPhos Pd G4 (161 mg, 0.2 mmol) and tert-butyl carbamate (470 mg, 4.01 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 381-2.

### Step 2: synthesis of compound 381-3

Compound 381-2 (520 mg, 1.55 mmol) was dissolved in methanol (10 mL), sodium borohydride (118 mg, 3.10 mmol) was added, and the reaction mixture was stirred overnight at room temperature. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 381-3.

### Step 3: synthesis of compound 381-4

Compound 381-3 (500 mg, 1.48 mmol) was dissolved in dichloromethane (5 ml), and hydrogen chloride-ethyl acetate solution (7.5 ml, 14.8 mmol) was added under ice bath conditions. The mixture was reacted at room temperature for 2 hours, a solid was precipitated and the reaction solution was concentrated to obtain compound 381-4 as a solid.

### Step 4: synthesis of compound 381-5

Compound 381-4 (170 mg, 0.71 mmol), HATU (362 mg, 1.07 mmol), DIEA (276 mg, 2.14 mmol), and 4-bromo-2-(4-(difluoromethylene)piperidin-1-yl)benzoic acid (236 mg, 0.71 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-30%) to obtain compound 381-5.

### Step 5: synthesis of compound 381

Compound 381-5 (150 mg, 0.272 mmol), cesium carbonate (266 mg, 0.817 mmol), tBuXPhos Pd G4 (22 mg, 0.027 mmol) and ethanesulfonamide (59 mg, 0.544 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 381. MS (ESI, pos.ion) m/z: 581.2 [M+1]⁺.

¹H NMR (CHLOROFORM-d, 400 MHz) *δ* 11.18 (br s, 1H), 8.06 (br s, 1H), 7.81 (br d, *J* = 7.6 Hz, 1H), 7.1-7.2 (m, 1H), 7.06(s, 1H), 7.02 (br d, *J* = 8.1 Hz, 1H), 6.8-6.9 (m, 1H), 6.57 (s, 1H), 5.02 (br dd, *J =* 6.6, 12.3 Hz, 1H), 4.16 (br t, *J =* 6.2 Hz, 2H),3.13(q, *J =* 7.4 Hz, 2H), 2.98 (br dd, *J =* 5.5, 11.3 Hz, 4H), 2.7-2.8 (m, 1H), 2.4-2.5 (m, 1H), 2.28 (br s, 4H), 1.34 (t, *J =* 7.4Hz, 3H).

### Example 382: synthesis of N-(8,8-difluoro-9-hydroxy-6,7,8,9-tetrahydropyridino[1,2-a]indol-1-yl)-4-ethyl sulfonamido-2-(6-azaspiro[2.5]octan-6-yl)benzamide

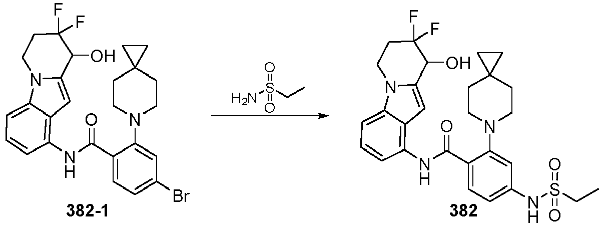

Compound 382-1 (130 mg, 0.272 mmol), cesium carbonate (240 mg, 0.737 mmol), tBuXPhos Pd G4 (20 mg, 0.024 mmol) and 2-hydroxyethane-1-sulfonamide (54 mg, 0.491 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 382. MS (ESI, pos.ion) m/z: 559.2 [M+1]⁺.

¹H NMR (CHLOROFORM-*d*, 400 MHz) *δ* 11.58 (br s, 1H), 7.96 (br s, 1H), 7.77 (br d, *J =* 7.8 Hz, 1H), 7.1-7.2 (m, 2H), 6.99(br d, *J =* 8.1 Hz, 1H), 6.7-6.9 (m, 1H), 6.62 (s, 1H), 5.02 (br dd, *J =* 6.4, 11.4 Hz, 1H), 4.0-4.3 (m, 2H), 3.11 (q, *J =* 7.2 Hz, 2H),2.9-3.1 (m, 4H), 2.7-2.8 (m, 1H), 2.4-2.5 (m, 1H), 1.45 (br s, 4H), 1.32 (br t, *J =* 7.3 Hz, 3H), 0.23 (s, 4H).

### Example 383: synthesis of N-(8,8-difluoro-9-hydroxy-6,7,8,9-tetrahydropyridino[1,2-a]indol-1-yl)-2-(4-(difluoromethylene)piperidin-1-yl)-4-((2-hydroxyethyl)sulfonamido)benzamide

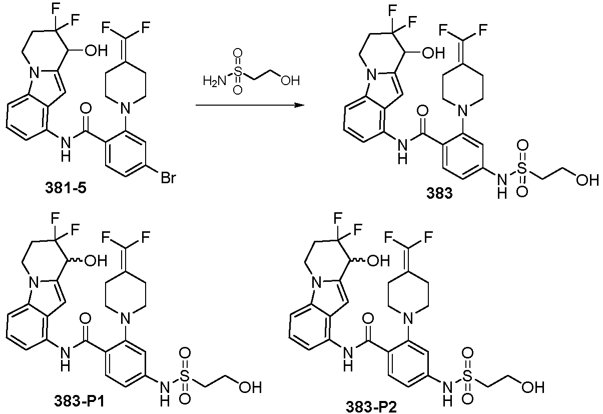

Compound 381-5 (150 mg, 0.272 mmol), cesium carbonate (266 mg, 0.817 mmol), tBuXPhos Pd G4 (22 mg, 0.027 mmol) and 2-hydroxyethane-1-sulfonamide (63 mg, 0.544 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 383.

Compound 383 was subjected to resolution by preparative SFC to obtain compound 383-P1 and compound 383-P2; for purification by preparative SFC, the following conditions were used: column: Whelk O1(S, S), 250×30mm I.D., 10µm particles; mobile phase A: 60/40 CO₂/EtOH (0.1% NH₃H₂O); detector wavelength: 220 nm; flow rate: 150 mL/min.

Compound 383-P1(28 mg) MS (ESI, pos.ion) m/z: 597.2 [M+1]⁺, retention time=5.910min;
Compound 383-P1 ¹H NMR (CHLOROFORM-*d*, 400 MHz) *δ* 11.47 (s, 1H), 7.7-7.9 (m, *J* = 8.4 Hz, 1H), 7.58 (d, *J =* 7.6 Hz, 1H), 7.15 (t, *J =* 7.8 Hz, 1H), 7.0-7.1 (m, 2H), 6.7-6.8 (m, *J =* 8.4 Hz, 1H), 6.52 (s, 1H), 4.97 (br dd, *J =* 6.3, 12.1 Hz, 1H), 4.1-4.2 (m, 2H), 3.88(br t, *J =* 4.8 Hz, 2H), 3.14 (br t, *J =* 4.6 Hz, 2H), 2.8-2.9 (m, 4H), 2.73 (br s, 1H), 2.3-2.4 (m, 1H), 2.19 (br s, 4H).

Compound 383-P2(23 mg) MS (ESI, pos.ion) m/z: 597.2 [M+1]⁺; retention time = 7.214 min;
Compound 383-P2 ¹H NMR (CHLOROFORM-*d*, 400 MHz) *δ* 11.47 (s, 1H), 7.7-7.9 (m, *J* = 8.4 Hz, 1H), 7.58 (d, *J =* 7.6 Hz, 1H), 7.15 (t, *J =* 7.8 Hz, 1H), 7.0-7.1 (m, 2H), 6.7-6.8 (m, *J =* 8.4 Hz, 1H), 6.52 (s, 1H), 4.97 (br dd, *J =* 6.3*,* 12.1 Hz, 1H), 4.1-4.2 (m, 2H), 3.88(br t, *J =* 4.8 Hz, 2H), 3.14 (br t, *J =* 4.6 Hz, 2H), 2.8-2.9 (m, 4H), 2.73 (br s, 1H), 2.3-2.4 (m, 1H), 2.19 (br s, 4H).

### Example 384: synthesis of N-(8,8-difluoro-9-hydroxy-6,7,8,9-tetrahydropyridino[1,2-a]indol-1-yl)-4-((2-hydroxyethyl)sulfonamido)-2-(6-azaspiro[2.5]oct-6-yl)benzamide

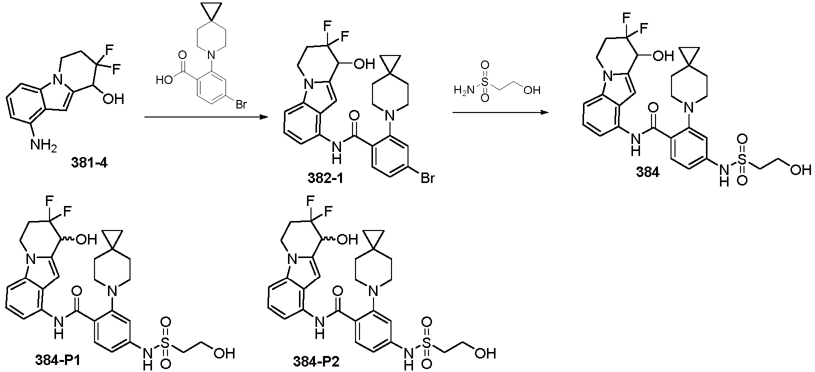

### Step 1: synthesis of compound 382-1

Compound 381-4 (150 mg, 0.71 mmol), HATU (359 mg, 0.945 mmol), DIEA (243 mg, 1.89 mmol), and 4-bromo-2-(6-azaspiro[2.5]oct-6-yl)benzoic acid (195 mg, 0.63 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-30%) to obtain compound 382-1.

### Step 2: synthesis of compound 384, compound 384-P1, and compound 384-P2

Compound 382-1 (160 mg, 0.302 mmol), cesium carbonate (296 mg, 0.907 mmol), tBuXPhos Pd G4 (24 mg, 0.030 mmol) and 2-hydroxyethane-1-sulfonamide (76 mg, 0.605 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 384 (65 mg).

For purification of compound 384 by preparative SFC, the following conditions were used: column: Whelk O1(S, S), 250×30mm I.D., 10µm particles; mobile phase A: 60/40 CO₂/ethanol (0.1% NH₃H₂O); detector wavelength: 220 nm; flow rate: 150 mL/min.

Compound 384-P1(30 mg): MS (ESI, pos.ion) m/z: 575.2 [M+1]⁺. Retention time = 6.572 min;
¹H NMR (CHLOROFORM-*d*, 400 MHz) δ 11.87 (br s, 1H), 7.90 (br s, 1H), 7.6-7.6 (m, 1H), 7.21 (br s, 1H), 7.17 (s, 1H),7.06 (d, *J =* 8.3 Hz, 1H), 6.7-6.9 (m, 1H), 6.60 (br s, 1H), 5.00 (br dd, *J =* 6.4, 11.7 Hz, 1H), 4.1-4.2 (m, 2H), 3.9-4.0 (m, 2H),3.16 (br s, 2H), 2.9-3.0 (m, 4H), 2.7-2.8 (m, 1H), 2.4-2.4 (m, 1H), 1.4-1.5 (m, 4H), 0.22 (s, 4H).

Compound 384-P2(30 mg): MS (ESI, pos.ion) m/z: 575.2 [M+1]⁺. Retention time = 7.576 min;
¹H NMR (CHLOROFORM-*d*, 400 MHz) *δ* 11.87 (br s, 1H), 7.90 (br s, 1H), 7.6-7.6 (m, 1H), 7.21 (br s, 1H), 7.17 (s, 1H),7.06 (d, *J =* 8.3 Hz, 1H), 6.7-6.9 (m, 1H), 6.60 (br s, 1H), 5.00 (br dd, *J =* 6.4, 11.7 Hz, 1H), 4.1-4.2 (m, 2H), 3.9-4.0 (m, 2H),3.16 (br s, 2H), 2.9-3.0 (m, 4H), 2.7-2.8 (m, 1H), 2.4-2.4 (m, 1H), 1.4-1.5 (m, 4H), 0.22 (s, 4H).

### Example 385: synthesis of N-(8,8-difluoro-9-(methoxy-d3)-6,7,8,9-tetrahydropyridino[1,2-a]indol-1-yl)-4-((2-hydroxyethyl)sulfonamido)-2-(6-azaspiro[2.5]oct-6-yl)benzamide

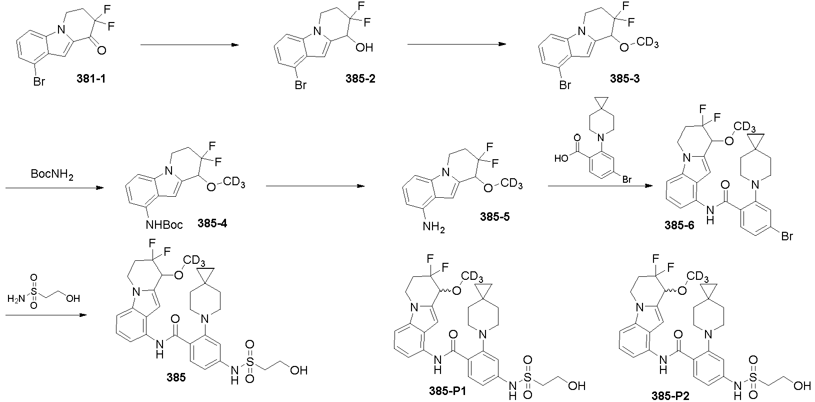

### Step 1: synthesis of compound 385-2

Compound 381-1 (2 g, 6.69 mmol) was dissolved in methanol (20 mL), sodium borohydride (508 mg, 13.38 mmol) was added, and the reaction mixture was stirred overnight at room temperature. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 385-2.

### Step 2: synthesis of compound 385-3

Compound 385-2 (1 g, 3.32 mmol) was dissolved in DMF (10 ml), followed by the addition of cesium carbonate (3.25 g, 9.97 mmol) and deuterated iodomethane (530 mg, 3.65 mmol). The reaction solution was stirred at room temperature for 1 hour. The reaction solution was poured into an appropriate amount of ice water, extracted three times with ethyl acetate (30 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 385-3.

### Step 3: synthesis of compound 385-4

Compound 385-3 (900 mg, 2.83 mmol), cesium carbonate (2.77 g, 8.49 mmol), tBuXPhos Pd G4 (229 mg, 0.283 mmol) and tert-butyl carbamate (662 mg, 5.66 mmol) were dissolved in 1,4-dioxane (10 mL), and the mixture was reacted at 90°C for 16 hours under nitrogen protection. The reaction solution was cooled and poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-80%) to obtain compound 385-4.

### Step 4: synthesis of compound 385-5

Compound 385-4 (350 mg, 0.986 mmol) was dissolved in dichloromethane (5 ml), and hydrogen chloride-ethyl acetate solution (4.9 ml, 9.86 mmol) was added under ice bath conditions. The mixture was reacted at room temperature for 2 hours, a solid was precipitated and the reaction solution was concentrated to obtain compound 385-5.

### Step 5: synthesis of compound 385-6

Compound 385-5 (250 mg, 0.98 mmol), HATU (559 mg, 1.47 mmol), DIEA (379 mg, 2.94 mmol), and 4-bromo-2-(6-azaspiro[2.5]oct-6-yl)benzoic acid (303 mg, 0.98 mmol) were dissolved in DMF (10 mL), and the reaction mixture was stirred at room temperature for 2 hours. The reaction solution was poured into an appropriate amount of ice water, extracted three times with ethyl acetate (20 ml), washed twice with sodium chloride aqueous solution (60 mL), the organic phase was dried over anhydrous sodium sulfate, concentrated under reduced pressure, and the crude product was purified by silica gel column chromatography (EA/PE = 0-30%) to obtain compound 385-6.

### Step 6: synthesis of compound 385

Compound 385-6 (230 mg, 0.421 mmol), cesium carbonate (412 mg, 1.26 mmol), tBuXPhos Pd G4 (34 mg, 0.042 mmol) and 2-hydroxyethane-1-sulfonamide (105 mg, 0.84 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 385.

Compound 385 was subjected to resolution by preparative SFC to obtain compound 385-P1 and compound 385-P2; for purification by preparative SFC, the following conditions were used: column: ChiralPak IC, 250×30mm I.D., 5µm particles; mobile phase A: 60/40 CO₂/EtOH (0.1% NH₃H₂O); detector wavelength: 220 nm; flow rate: 80 mL/min.

Compound 385-P1(33 mg): MS (ESI, pos.ion) m/z: 592.2 [M+1]⁺. retention time = 2.003 min;
¹H NMR (CHLOROFORM-*d*, 400 MHz) *δ* 11.76 (s, 1H), 8.08 (d, *J =* 8.4 Hz, 1H), 7.91 (d, *J* = 7.6 Hz, 1H), 7.36 (d, *J =* 1.8Hz, 1H), 7.3-7.3 (m, 1H), 7.16 (d, *J =* 8.3 Hz, 1H), 6.96 (dd, *J =* 1.9, 8.5 Hz, 1H), 6.72 (s, 1H), 4.56 (t, *J =* 6.0 Hz, 1H), 4.4-4.4 (m,1H), 4.1-4.1 (m, 1H), 4.0-4.1 (m, 2H), 3.24 (t, *J =* 5.1 Hz, 2H), 3.0-3.2 (m, 4H), 2.8-3.0 (m, 1H), 2.4-2.5 (m, 1H), 1.4-1.7 (m,4H), 0.2-0.3 (m, 4H).

Compound 385-P2(36 mg): MS (ESI, pos.ion) m/z: 592.2 [M+1]⁺. retention time = 2.556 min;
¹H NMR (CHLOROFORM-*d*, 400 MHz) δ 11.76 (s, 1H), 8.08 (d, *J =* 8.4 Hz, 1H), 7.91 (d, *J* = 7.6 Hz, 1H), 7.36 (d, *J =* 1.8Hz, 1H), 7.3-7.3 (m, 1H), 7.16 (d, *J =* 8.3 Hz, 1H), 6.96 (dd, *J =* 1.9, 8.5 Hz, 1H), 6.72 (s, 1H), 4.56 (t, *J =* 6.0 Hz, 1H), 4.4-4.4 (m,1H), 4.1-4.1 (m, 1H), 4.0-4.1 (m, 2H), 3.24 (t, *J =* 5.1 Hz, 2H), 3.0-3.2 (m, 4H), 2.8-3.0 (m, 1H), 2.4-2.5 (m, 1H), 1.4-1.7 (m,4H), 0.2-0.3 (m, 4H).

### Example 386: synthesis of N-(8,8-difluoro-9-(methoxy-d3)-6,7,8,9-tetrahydropyridino[1,2-a]indol-1-yl)-2-(4-(difluoromethylene)piperidin-1-yl)-4-((2-hydroxyethyl)sulfonamido)benzamide

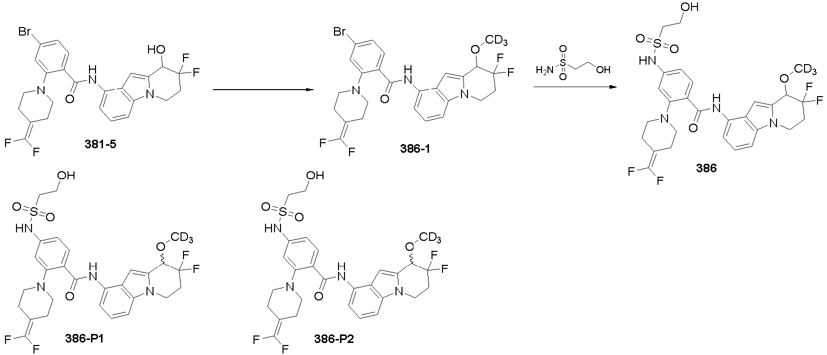

### Step 1: synthesis of compound 386-1

Compound 381-5 (400 mg, 3.32 mmol) was dissolved in DMF (10 ml), followed by the addition of cesium carbonate (710 mg, 2.18 mmol) and deuterated iodomethane (116 mg, 0.799 mmol). The reaction solution was stirred at room temperature for 1 hour. The reaction solution was poured into an appropriate amount of ice water, extracted three times with ethyl acetate (30 ml), washed twice with sodium chloride aqueous solution (30 mL), the organic phase was dried over anhydrous sodium sulfate, concentrated under reduced pressure, and the crude product was purified by silica gel column chromatography (EA/PE = 0-20%) to obtain compound 386-1.

### Step 2: synthesis of compound 386, compound 386-P1, and compound 386-P2

Compound 386-1 (200 mg, 0.421 mmol), cesium carbonate (344 mg, 1.06 mmol), tBuXPhos Pd G4 (28 mg, 0.035 mmol) and 2-hydroxyethane-1-sulfonamide (88 mg, 0.704 mmol) were dissolved in 1,4-dioxane (10 mL), and the mixture was reacted at 80°C for 16 hours under nitrogen protection. The reaction solution was cooled and poured into an appropriate amount of ice water, extracted three times with ethyl acetate (20 ml), washed twice with sodium chloride aqueous solution (50 mL), the organic phase was dried over anhydrous sodium sulfate, concentrated under reduced pressure, and the crude product was purified by silica gel column chromatography (EA/PE = 0-80%) to obtain compound 386.

Compound 386 was subjected to resolution by preparative SFC to obtain compound 386-P1 and compound 386-P2; for purification by preparative SFC, the following conditions were used: column: ChiralPak AS, 250×30mm I.D., 5µm particles; mobile phase A: 65/35 CO₂/isopropanol (0.1% NH₃H₂O); detector wavelength: 220 nm; flow rate: 80 mL/min.

Compound 386-P1(52 mg):MS (ESI, pos.ion) m/z: 614.2 [M+1]⁺. retention time = 4.085 min;
¹H NMR (CHLOROFORM-*d*, 400 MHz) *δ* 11.20 (s, 1H), 8.19 (d, *J =* 8.3 Hz, 1H), 8.05 (d, *J =* 7.8 Hz, 1H), 7.32 (t, *J =* 7.6Hz, 1H), 7.2-7.3 (m, 1H), 7.16 (d, *J =* 8.3 Hz, 1H), 7.03 (br d, *J=* 8.3 Hz, 1H), 6.87 (br s, 1H), 6.66 (s, 1H), 4.58 (t, *J =* 6.2 Hz, 1H),4.4-4.4 (m, 1H), 4.15 (br s, 2H), 4.1-4.1 (m, 1H), 3.33 (t, *J =* 5.2 Hz, 2H), 3.1-3.2 (m, 4H), 2.8-3.0 (m, 1H), 2.4-2.6 (m, 2H),2.41 (br s, 4H).

Compound 386-P2(49 mg):MS (ESI, pos.ion) m/z: 614.2 [M+1]⁺. retention time = 4.406 min;
¹H NMR (CHLOROFORM-d, 400 MHz) *δ* 11.20 (s, 1H), 8.19 (d, *J =* 8.3 Hz, 1H), 8.05 (d, *J =* 7.8 Hz, 1H), 7.32 (t, *J =* 7.6Hz, 1H), 7.2-7.3 (m, 1H), 7.16 (d, *J =* 8.3 Hz, 1H), 7.03 (br d, *J=* 8.3 Hz, 1H), 6.87 (br s, 1H), 6.66 (s, 1H), 4.58 (t, *J =* 6.2 Hz, 1H),4.4-4.4 (m, 1H), 4.15 (br s, 2H), 4.1-4.1 (m, 1H), 3.33 (t, *J =* 5.2 Hz, 2H), 3.1-3.2 (m, 4H), 2.8-3.0 (m, 1H), 2.4-2.6 (m, 2H),2.41 (br s, 4H).

### Example 391: synthesis of compound N-(3,3-dimethyl-3,4-dihydro-1H-benzo[4,5]imidazo[2,1-c][1,4]oxazin-9-yl)-4-((2-hydroxyethyl)sulfonamido)-2-(6-azaspiro [2.5] octan-6-yl)benzamide

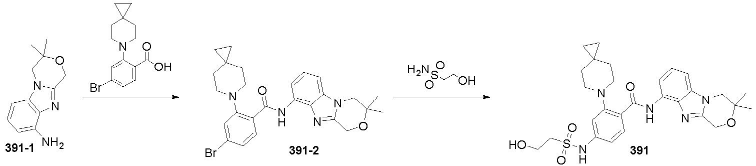

### Step 1: synthesis of compound 391-2

Compound 391-1 (80 mg, 0.67 mmol), 2-(6-azaspiro[2.5]octan-6-yl)-4-bromobenzoic acid (125.64 mg, 0.41 mmol), 2-(7-azabenzotriazole)-N,N,N',N'-tetramethyluronium hexafluorophosphate (167.90 mg, 0.44 mmol) and N,N-diisopropylethylamine (95.00 mg, 0.74 mmol) were dissolved in N,N-dimethylformamide (3 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=90-10) to obtain compound 391-2.

### Step 2: synthesis of compound 391

Compound 391-2 (105 mg, 0.21 mmol), 2-hydroxyethanesulfonamide (103.17 mg, 0.82 mmol), cesium carbonate (133.97 mg, 0.41 mmol) and palladium catalyst (CAS: 1599466-83-7; 18.96 mg, 0.02 mmol) were dissolved in 1,4-dioxane (3 mL), and the reaction mixture was reacted at 95°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 391. MS m/z (ESI):554.2 [M+1]⁺.

**¹H NMR** (400 MHz, DMSO-d₆) δ = 12.50 (s, 1H), 8.39-8.37 (m, 1H), 8.04 (d, *J = 8.6* Hz, 1H), 7.27 (d, *J =* 1.6 Hz, 1H), 7.27 - 7.20 (m, 2H), 7.09 (dd, *J =* 1.9, 8.6 Hz, 1H), 4.98 (s, 2H), 4.11 (s, 2H), 3.76 (t, *J =* 6.5 Hz, 2H), 3.37 - 3.35 (m, 2H), 2.98-2.96 (m, 4H), 1.85 - 1.69 (m, 4H), 1.37 (s, 6H), 0.31 (s, 4H).

### Example 392: synthesis of N-(6-cyano-3,3-dimethyl-3,4-dihydro-1H-[1,4] oxazino [4,3-a] indol-9-yl)-4-((2-hydroxyethyl)sulfonamido)-2-(6-azaspiro[2.5]oct-6-yl)benzamide (392).

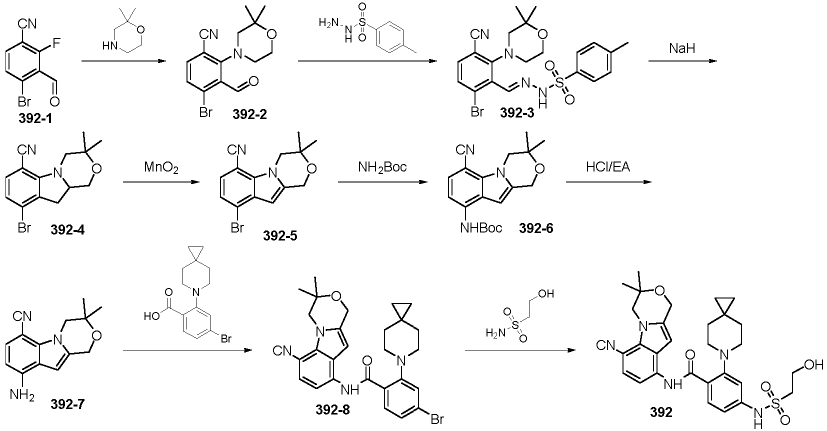

### Step 1: synthesis of compound 392-2

Compound 392-1 (2.2 g, 9.65 mmol), and granular potassium carbonate (1.73 g, 12.54 mmol) were dissolved in DMSO (15 mL), to which 2,2-dimethylmorpholine (1.22 g, 10.61 mmol) was added, and the reaction mixture was allowed to react at 60°C for 6 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 392-2.

### Step 2: synthesis of compound 392-3

Compound 392-2 (1.49 g, 4.61 mmol) was dissolved in toluene (15 mL), to which p-toluenesulfonyl hydrazide (1.03 g, 5.53 mmol) was added, and the mixture was reacted at room temperature for 20 minutes. The reaction solution was quenched with ice water, and extracted with EA. The organic phase was washed with saturated NaCl solution, dried over anhydrous Na₂SO₄, and concentrated under reduced pressure to obtain compound 392-3.

### Step 3: synthesis of compound 392-4

Compound 392-3 (2.26 g, 4.60 mmol) was dissolved in toluene (25 mL), to which NaH (204 mg, 5.06 mmol) was slowly added at 0°C, and after stirring for 30 minutes, the mixture was reacted at 135°C for 30 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 392-4.

### Step 4: synthesis of compound 392-5

Compound 392-4 (1048 mg, 3.41 mmol) was dissolved in DCM (10 mL), to which MnO₂ (2.97 g, 34.12 mmol) was added, and the reaction mixture was allowed to react at 25°C overnight. The reaction solution was filtered, and concentrated under reduced pressure. The crude product was purified by silica gel column chromatography (PE:EA=50:1) to obtain compound 392-5.

### Step 5: synthesis of compound 392-6

Compound 392-5 (180 mg, 0.59 mmol), NH₂Boc (138 mg, 1.18 mmol), cesium carbonate (576 mg, 1.77 mmol) and the fourth-generation palladium catalyst (CAS: 1599466-83-7; 108 mg, 0.12 mmol) were dissolved in 1,4-dioxane (3 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 392-6.

### Step 6: synthesis of compound 392-7

Compound 392-6 (195 mg, 0.57 mmol) was dissolved in DCM (2 mL), to which HCl\EA (3 mL) was added, and the reaction mixture was allowed to react at 25°C for 1 hour. The reaction solution was filtered, neutralized and dried to obtain compound 392-7.

### Step 7: synthesis of compound 392-8

Compound 392-7 (50 mg, 0.18 mmol), 4-bromo-2-(6-azaspiro[2.5]oct-6-yl)benzoic acid (112 mg, 0.36 mmol), and TCFH (152 mg, 0.54 mmol) were dissolved in THF (3 mL), to which DIEA (0.12 mL, 0.72 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 392-8.

### Step 8: synthesis of compound 392

Compound 392-8 (45 mg, 0.08 mmol), 2-hydroxyethanesulfonamide (42 mg, 0.32 mmol), cesium carbonate (82 mg, 0.24 mmol) and the fourth-generation palladium catalyst (CAS: 1599466-89-3; 7 mg, 0.01 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 392. MS (ESI, pos.ion) m/z:578.2 [M+1]⁺.

¹H NMR (400 MHz, DMSO-d₆) δ = 11.31 (s, 1H), 8.14 (br d, *J =* 8.3 Hz, 1H), 7.82 (d, *J=* 8.5 Hz, 1H), 7.59 (d, *J =* 8.3 Hz, 1H), 7.19 - 7.16 (m, 1H), 7.03 (dd, *J =* 1.8, 8.5 Hz, 1H), 6.76 (s, 1H), 5.00 (s, 2H), 4.31 (s, 2H), 3.78 (t, *J =* 6.5 Hz, 2H), 3.38 - 3.34 (m, 2H), 3.00 (br s, 4H), 1.44 - 1.39 (m, 4H), 1.39 - 1.37 (m, 6H), 0.26 (s, 4H).

### Example 393: synthesis of N-(6-cyano-3,3-dimethyl-3,4-dihydro-1H-[1,4]oxazino[4,3-a]indol-9-yl)-2-(4-(difluoromethylene)piperidin-1-yl)-4-((2-hydroxyethyl)sulfonamido)benzamide (393)

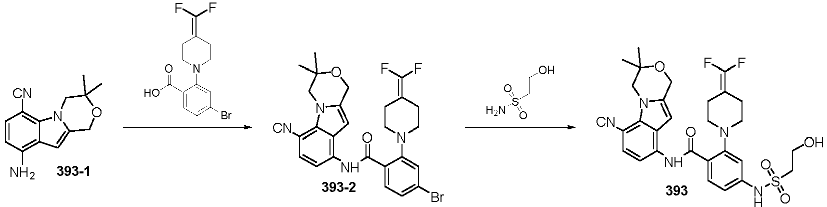

### Step 1: synthesis of compound 393-2

Compound 393-1 (60 mg, 0.22 mmol), 4-bromo-2-(4-(difluoromethylene)piperidin-1-yl)benzoic acid (143 mg, 0.44 mmol), and TCFH (182 mg, 0.66 mmol) were dissolved in THF (3 mL), to which DIEA (0.14 mL, 0.88 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 393-2.

### Step 2: synthesis of compound 393

Compound 393-2 (48 mg, 0.09 mmol), 2-hydroxyethanesulfonamide (43 mg, 0.36 mmol), cesium carbonate (84 mg, 0.27 mmol) and the fourth-generation palladium catalyst (CAS: 1599466-89-3; 7 mg, 0.01 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 393. MS (ESI, pos.ion) m/z:600.2 [M+1]⁺.

¹H NMR (400 MHz, DMSO-d₆) δ = 10.91 (s, 1H), 8.15 (br d, *J=* 8.0 Hz, 1H), 7.74 (d, *J =* 8.4 Hz, 1H), 7.59 (d, *J =* 8.3 Hz, 1H), 7.06 (s, 1H), 7.04 - 6.99 (m, 1H), 6.77 (s, 1H), 5.00 - 4.96 (m, 2H), 4.29 (s, 2H), 3.77 (t, *J =* 6.5 Hz, 2H), 3.35 (br s, 2H), 3.00 (br s, 4H), 2.18 (br s, 4H), 1.36 (s, 6H).

### Example 394: synthesis of 2-(4-(difluoromethylene)piperidin-1-yl)-N-(3,3-dimethyl-3,4-dihydro-1H-benzo[4,5]imidazo[2,1-c][1,4]oxazin-9-yl)-4-((2-hydroxyethyl)sulfonamido)benzamide

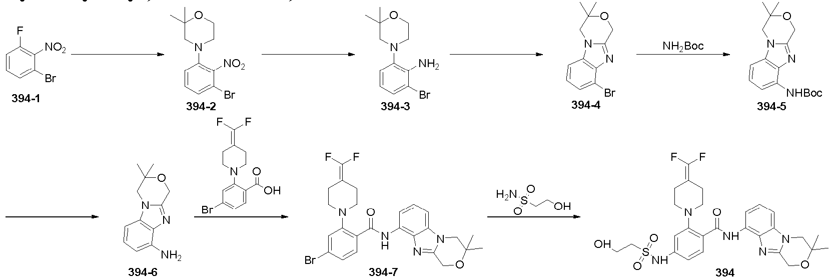

### Step 1: synthesis of compound 394-2

Compound 394-1 (2 g, 9.09 mmol) and 2,2-dimethyl-1,4-oxazinane (1.26 g, 10.91 mmol) were dissolved in N-methylpyrrolidinone (10 mL), to which N,N-diisopropylethylamine (2.35 g, 18.18 mmol) was added, and the reaction mixture was allowed to react at 100°C overnight. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=100:0-90:10) to obtain compound 394-2.

### Step 2: synthesis of compound 394-3

Compound 394-2 (2.3 g, 7.30 mmol) was dissolved in ethanol (5 mL) and water (5 mL), to which iron dust (2.04 g, 36.49 mmol) and ammonium chloride (1.95 g, 36.49 mmol) were added. The reaction mixture was allowed to react at 80°C for 3 h. The reaction solution was filtered under reduced pressure and then quenched with water, and extracted with EA. The organic phase was washed with saturated NaCl solution, dried over anhydrous Na₂SO₄, and concentrated under reduced pressure. The crude product was purified by silica gel column chromatography (PE:EA=100:0-80:20) to obtain compound 394-3.

### Step 3: synthesis of compound 394-4

Compound 394-3 (800 mg, 2.81 mmol) was dissolved in dichloromethane (6 mL), followed by the addition of hydrogen peroxide (0.84 mL, 28.05 mmol) and trifluoroacetic acid (2.09 mL, 28.05 mmol). The mixture was stirred overnight at 40°C under sealed conditions. The reaction solution was then diluted with ice-cold sodium thiosulfate solution and then extracted with EA. The organic phase was washed with saturated NaCl solution, dried over anhydrous Na₂SO₄, and concentrated under reduced pressure. The crude product was purified by silica gel column chromatography (PE:EA=100:0-80:20) to obtain compound 394-4.

### Step 4: synthesis of compound 394-5

Compound 394-4 (200 mg, 0.71 mmol), 2-methylprop-2-ylcarbamate (166.67 mg, 1.42 mmol), cesium carbonate (695.33 mg, 2.13 mmol) and palladium catalyst (CAS: 1599466-81-5; 61.18 mg, 0.07 mmol) were dissolved in 1,4-dioxane (3 mL), and the reaction mixture was reacted at 95°C for 2 h under N₂ protection. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=90-10) to obtain compound 394-5. MS m/z (ESI):318.1 [M+1]⁺

### Step 6: synthesis of compound 394-6

Compound 394-5 (255 mg, 0.80 mmol) was dissolved in hydrogen chloride-1,4-dioxane solution (4 M, 5 mL) and the mixture was reacted at room temperature for 0.5 h. The reaction solution was quenched with ice-cold sodium bicarbonate solution, and extracted with EA. The organic phase was washed with saturated NaCl solution, dried over anhydrous Na₂SO₄, and concentrated under reduced pressure to obtain compound 394-6.

### Step 7: synthesis of compound 394-7

Compound 394-6 (145 mg, 0.67 mmol), 4-bromo-2-[4-(difluoromethylene)cyclohexyl]benzoic acid (243.11 mg, 0.73 mmol), 2-(7-azabenzotriazole)-N,N,N',N'-tetramethyluronium hexafluorophosphate (304.51 mg, 0.80 mmol) and N,N-diisopropylethylamine (172.50 mg, 1.33 mmol) were dissolved in N,N-dimethylformamide (5 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=80-20) to obtain compound 394-7.

### Step 8: synthesis of compound 394

Compound 394-7 (300 mg, 0.56 mmol), 2-hydroxyethanesulfonamide (282.59 mg, 2.26 mmol), cesium carbonate (367.88 mg, 1.13 mmol) and palladium catalyst (CAS: 1599466-83-7; 51.97 mg, 0.06 mmol) were dissolved in 1,4-dioxane (5 mL), and the reaction mixture was reacted at 95°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 394. MS m/z (ESI):576.2 [M+1]⁺.

**¹H NMR** (400 MHz, DMSO-d₆) δ = 12.41 (s, 1H), 8.38 (br t, *J =* 4.1 Hz, 1H), 8.06 (br d, *J =* 8.5 Hz, 1H), 7.23 - 7.21 (m, 3H), 7.12 (br d, *J =* 8.4 Hz, 1H), 4.89 (s, 2H), 4.09 (s, 2H), 3.77 (br t, *J =* 6.3 Hz, 2H), 3.39 - 3.37 (m, 2H), 3.00 - 2.98 (m, 4H), 2.64 - 2.62 (m, 4H), 1.35 (s, 6H).

### Example 395: synthesis of 2-(4-(difluoromethylene)piperidin-1-yl)-N-(1,1-dimethyl-3,4-dihydro-1H-[1,4]oxazino[4,3-a]indol-9-yl)-4-((2-hydroxyethyl)sulfonamido)benzamide

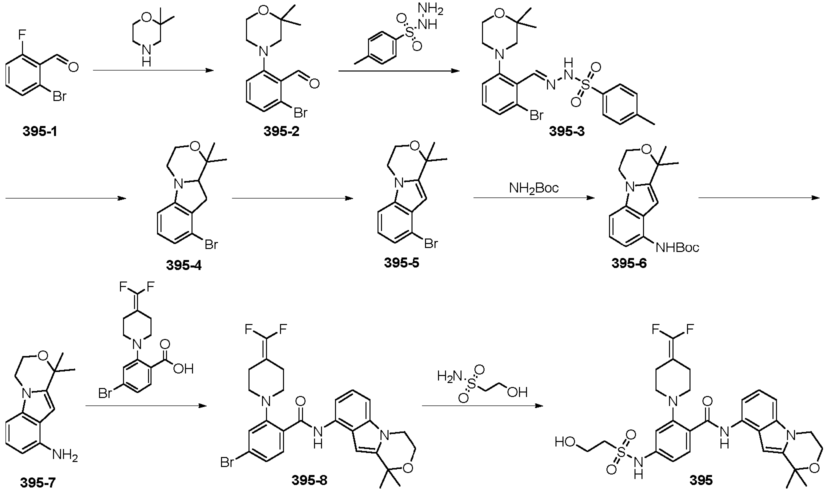

### Step 1: synthesis of compound 395-2

Compound 395-1 (6 g, 29.56 mmol) and 2,2-dimethyl-1,4-oxazinane (4.09 g, 35.47 mmol) were dissolved in N-methylpyrrolidinone (30 mL), to which N,N-diisopropylethylamine (7.64 g, 59.12 mmol) was added, and the reaction mixture was allowed to react at 100°C overnight. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=97:3-90:10) to obtain compound 395-2.

### Step 2: synthesis of compound 395-3

Compound 395-2 (4.10 g, 13.75 mmol) was dissolved in ultra-dry toluene (24 mL), to which N-amino-4-methylbenzenesulfonamide (3.07 g, 16.50 mmol) was added, and the mixture was reacted at room temperature for 1 h to obtain the reaction solution containing compound 395-3, which was used directly in the next reaction.

### Step 3: synthesis of compound 395-4

To the reaction solution from the previous step was added ultra-dry toluene (24 mL), to which sodium hydride (0.71 g, 25.59 mmol) was added under ice bath conditions and under N₂ protection. The resulting mixture was stirred at 25°C for 0.5 h, followed by reaction at 135°C for 10 minutes. The reaction solution was diluted with ice water, and extracted with EA. The organic phase was washed with saturated NaCl solution, dried over anhydrous Na₂SO₄, concentrated under reduced pressure and the crude product was purified by preparative chromatography to obtain compound 395-4.

### Step 4: synthesis of compound 395-5

Compound 395-4 (769 mg, 2.73 mmol), 4,5-dichloro-3,6-dioxocyclohexa-1,4-diene-1,2-dicarbonitrile (1.24 g, 5.45 mmol) were dissolved in dioxane (1 mL), and the mixture was reacted at room temperature for 2 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=97:3-90:10) to obtain compound 395-5.

### Step 5: synthesis of compound 395-6

Compound 395-5 (296 mg, 1.06 mmol), 2-methylprop-2-ylcarbamate (247.54 mg, 2.11 mmol), cesium carbonate (1.03 g, 3.17 mmol) and palladium catalyst (CAS: 1599466-81-5; 45.46 mg, 0.05 mmol) were dissolved in 1,4-dioxane (5 mL), and the reaction mixture was reacted at 95°C for 1.5 h under N₂ protection. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=90-10) to obtain compound 395-6 (290 mg). MS m/z (ESI):317.1 [M+1]⁺.

### Step 6: synthesis of compound 395-7

Compound 395-6 (280 mg, 0.88 mmol) was dissolved in hydrogen chloride-ethyl acetate solution (2 M, 5 mL) and the mixture was reacted at room temperature for 1 h. The reaction solution was quenched with ice-cold sodium bicarbonate solution, and extracted with EA. The organic phase was washed with saturated NaCl solution, dried over anhydrous Na₂SO₄, and concentrated under reduced pressure to obtain compound 395-7.

### Step 7: synthesis of compound 395-8

Compound 395-7 (55 mg, 0.25 mmol), 4-bromo-2-[4-(difluoromethylene)hexahydropyridin-1-yl]benzoic acid (92.91 mg, 0.28 mmol), 2-(7-azabenzotriazole)-N,N,N',N'-tetramethyluronium hexafluorophosphate (116.03 mg, 0.31 mmol) and N,N-diisopropylethylamine (65.73 mg, 0.51 mmol) were dissolved in acetonitrile (2 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=80-20) to obtain compound 395-8.

### Step 8: synthesis of compound 395

Compound 395-8 (83 mg, 0.16 mmol), 2-hydroxyethanesulfonamide (78.33 mg, 0.63 mmol), cesium carbonate (101.97 mg, 0.31 mmol) and palladium catalyst (CAS: 1599466-83-7; 7.20 mg, 0.01 mmol) were dissolved in 1,4-dioxane (3 mL), and the reaction mixture was reacted at 95°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 395. MS m/z (ESI):575.3 [M+1]⁺.

**¹H NMR** (400 MHz, DMSO-d₆) δ = 10.82 (s, 1H), 8.06 (d, *J =* 7.5 Hz, 1H), 7.83 (d, *J =* 1.0 Hz, 1H), 7.84 - 7.78 (m, 1H), 7.12 - 7.06 (m, 2H), 7.02 (dd, *J =* 1.7, 8.4 Hz, 1H), 6.56 (s, 1H), 4.15 - 4.09 (m, 2H), 4.07 - 4.02 (m, 2H), 3.77 (t, *J =* 6.6 Hz, 2H), 3.31 (m, 2H), 3.05 - 2.98 (m, 4H), 2.33 - 2.26 (m, 4H), 1.54 (s, 6H).

### Example 396: synthesis of N-(1,1-dimethyl-3,4-dihydro-1H-[1,4] oxazino [4,3-a] indol-9-yl)-4-((2-hydroxyethyl)sulfonamido)-2-(6-azaspiro[2.5]octan-6-yl)benzamide

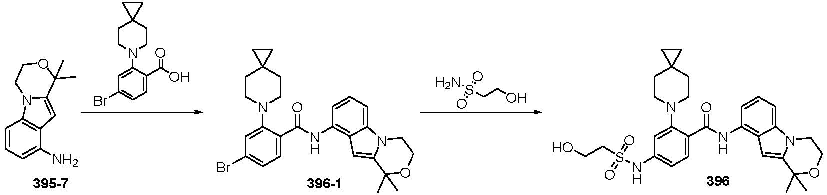

**Step 1: synthesis of compound 396-1**

Compound 395-7 (55 mg, 0.25 mmol), 2-(6-azaspiro[2.5]octan-6-yl)-4-bromobenzoic acid (86.77 mg, 0.28 mmol), 2-(7-azabenzotriazole)-N,N,N',N'-tetramethyluronium hexafluorophosphate (116.03 mg, 0.31 mmol) and N,N-diisopropylethylamine (65.73 mg, 0.51 mmol) were dissolved in acetonitrile (2 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=80-20) to obtain compound 396-1.

### Step 2: synthesis of compound 396

Compound 396-1 (25 mg, 0.05 mmol), 2-hydroxyethanesulfonamide (24.61 mg, 0.20 mmol), cesium carbonate (32.04 mg, 0.10 mmol) and palladium catalyst (CAS: 1599466-83-7; 2.26 mg, 0.00 mmol) were dissolved in 1,4-dioxane (3 mL), and the reaction mixture was reacted at 95°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 396. MS m/z (ESI):553.2 [M+1]⁺.

**¹H NMR** (400 MHz, DMSO-*d₆*) δ = 11.24 (s, 1H), 8.09 (d, *J* = 1.0 Hz, 1H), 7.89 (d, *J* = 1.0 Hz, 1H), 7.16 - 7.10 (m, 3H), 7.00 (br d, *J* = 7.5 Hz, 1H), 6.55 (br s, 1H), 4.17 - 4.11 (m, 2H), 4.08-4.04 (m, 2H), 3.76-3.74 (m, 4H), 3.06 - 2.98 (m, 4H), 1.63 - 1.48 (m, 10H), 0.26 (br s, 4H).

### Example 398: synthesis of 2-(4-(difluoromethylene)piperidin-1-yl)-4-((2-hydroxyethyl)sulfonamido)-N-(7,8,9,10-tetrahydro-6H-7,10-epoxyazeto[1,2-a]indol-1-yl)benzamide

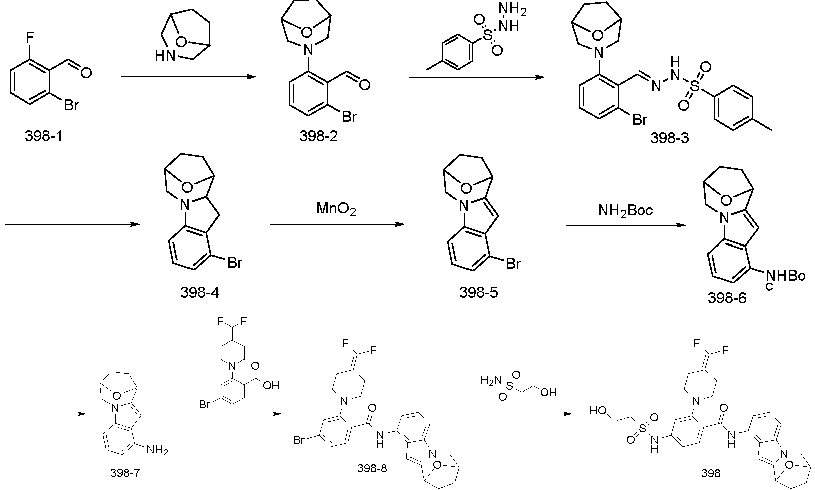

### Step 1: synthesis of compound 398-2

Compound 398-1 (2 g, 9.85 mmol) was dissolved in N-methylpyrrolidinone (10 mL), to which N,N-diisopropylethylamine (5.71 mL, 34.48 mmol) was added, and the reaction mixture was allowed to react at 150°C for 5 h. The reaction solution was allowed to cool to room temperature before being poured into the saturated NH₄Cl solution, and extracted with EA. The organic phase was washed with saturated NH₄Cl solution, dried over anhydrous Na₂SO₄, and concentrated under reduced pressure. The crude product was purified by silica gel column chromatography (PE:EA=100/1-10/1) to obtain compound 398-2.

### Step 2: synthesis of compound 398-3

Compound 398-2 (1.62 g, 5.47 mmol) and 4-methylbenzenesulfonyl hydrazide (1.07 g, 5.74 mmol) were dissolved in toluene (32 mL), and reacted at 25°C for 30 min. After monitoring the complete reaction of raw materials, a reaction solution containing compound 398-3 was obtained and used directly in the next reaction.

### Step 3: synthesis of compound 398-4

NaH (328.18 mg, 8.20 mmol, 60% purity) was added to the reaction solution containing compound 398-3 (2.54 g, 5.47 mmol) at 25°C, and the reaction mixture was allowed to react at 140°C for 3 h. The reaction solution was quenched with water, and extracted with EA. The organic phase was dried over anhydrous Na₂SO₄, and concentrated under reduced pressure. The crude product was purified by silica gel column chromatography (PE:EA=100/1-20/1) to obtain compound 398-4.

### Step 4: synthesis of compound 398-5

Compound 398-4 (980 mg, 3.50 mmol) was dissolved in dichloromethane (30 mL), to which active manganese dioxide (1.52 g, 17.49 mmol) was added, and the mixture was reacted at 40°C for 18 h. The reaction solution was filtered through diatomaceous earth, the filter cake was rinsed several times with DCM, and the organic phase was concentrated under reduced pressure. The crude product was purified by silica gel column chromatography (PE:EA = 100/1-20/1) to obtain compound 398-5.

### Step 5: synthesis of compound 398-6

Compound 398-5 (350 mg, 1.26 mmol), NH₂Boc (191.89 mg, 1.64 mmol), cesium carbonate (1.02 g, 3.15 mmol) and palladium catalyst (CAS: 1599466-85-9; 53.5 mg, 0.06 mmol) were dissolved in 1,4-dioxane (5 mL), and the reaction mixture was reacted at 90°C for 5 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=100/1-10/1) to obtain compound 398-6.

### Step 6: synthesis of compound 398-7

Compound 398-6 (200 mg, 0.64 mmol) was dissolved in EA (2 mL), to which HCl\EA (4 mL, 2M) was added, and the reaction mixture was allowed to react at 25°C for 2 h. A solid was precipitated, and the reaction solution was filtered, neutralized, and dried to obtain compound 398-7, which was used directly in the next reaction.

### Step 7: synthesis of compound 398-8

4-Bromo-2-(4-(difluoromethylene)piperidin-1-yl)benzoic acid (281 mg, 0.91 mmol), and HATU (265.12 mg, 0.70 mmol) were dissolved in dichloromethane (5 mL), to which DIEA (0.24 mL, 1.45 mmol) was added, and the reaction mixture was allowed to react at 25°C for 5 min. Compound 398-7 (150 mg, 0.58 mmol) was added to the reaction solution, and the reaction mixture was allowed to react at 25°C for 30 min. The reaction solution was quenched with saturated NH₄Cl solution, and extracted with EA. The organic phase was washed twice with saturated NH₄Cl solution, then dried over anhydrous Na₂SO₄, and concentrated under reduced pressure. The crude product was purified by silica gel column chromatography (PE:EA=100/1-4/1) to obtain compound 398-8.

### Step 7: synthesis of compound 398

Compound 398-8 (180 mg, 0.34 mmol), 2-hydroxyethanesulfonamide (63.94 mg, 0.51 mmol), cesium carbonate (277.48 mg, 0.85 mmol) and palladium catalyst (CAS: 1599466-89-3; 13.76 mg, 0.02 mmol) were dissolved in 1,4-dioxane (5 mL), and the reaction mixture was reacted at 80°C for 5 h under N₂ protection. The reaction solution was quenched with saturated NH₄Cl solution, and extracted with EA. The organic phase was washed with saturated NaCl solution, dried over anhydrous Na₂SO₄, and concentrated under reduced pressure. The crude product was purified by silica gel column chromatography (DCM:MeOH=100/1-10/1) to obtain compound 398. MS m/z (ESI):573.1 [M+1]⁺.

**¹H NMR** (CHLOROFORM-d) δ: 11.37 (br s, 1H), 8.03 (br d, J=8.3 Hz, 1H), 7.78 (br d, J=7.5 Hz, 1H), 7.72 (br s, 1H), 7.17-7.26 (m, 2H), 7.09 (br d, J=8.1 Hz, 1H), 6.95 (br s, 1H), 6.19 (s, 1H), 5.34 (br d, *J* =5.4 Hz, 1H), 4.87-4.97 (m, 1H), 4.25 (br s, 1H), 4.01 (br s, 2H), 3.90 (br d, *J* =11.0 Hz, 1H), 3.36-3.68 (m, 1H), 3.21 (br s, 2H), 3.04 (br s, 4H), 2.31 (br s, 6H), 2.01-2.11 (m, 1H), 1.90-2.00 (m, 1H).

### Example 399:

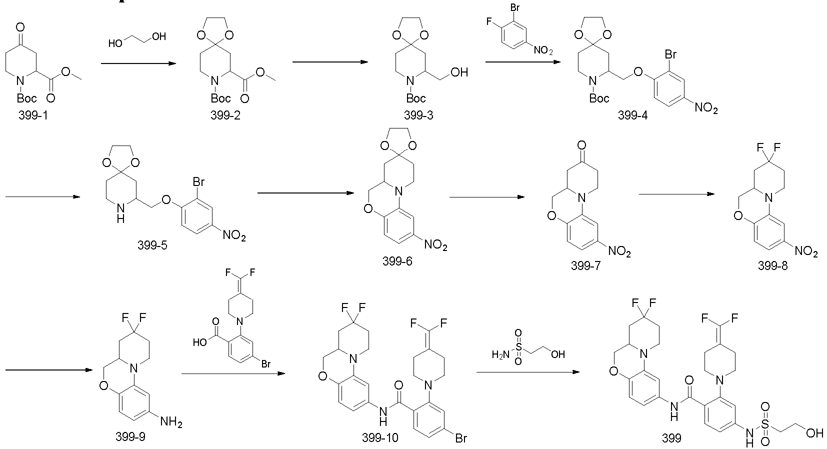

### Step 1: synthesis of compound 399-2

Compound 399-1 (3 g, 11.67 mmol), ethylene glycol (1.5 g, 24.5 mmol) and p-toluenesulfonic acid (200 mg, 1.17 mmol) were dissolved in THF (20 ml), and the mixture was stirred at reflux for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/5) to obtain compound 399-2.

### Step 2: synthesis of compound 399-3

Compound 399-2 (1.67 g, 5.55 mmol) was dissolved in THF (20 ml). Lithium aluminium hydride (422 mg, 11.1 mmol) was added under ice bath conditions, and the reaction mixture was stirred at room temperature for 2 hours. The reaction solution was poured into ice water, and extracted with ethyl acetate. The organic phase was washed with saturated sodium chloride aqueous solution, dried over anhydrous sodium sulfate, and concentrated. The crude product was not purified to obtain compound 399-3 which was used directly in the next reaction.

### Step 3: synthesis of compound 399-4

Compound 399-3 (1.01 g, 3.70 mmol), cesium carbonate (2.41 g, 7.4 mmol) and 2-bromo-1-fluoro-4-nitrobenzene (814 mg, 3.70 mmol) were dissolved in DMF (10 ml), and the mixture was stirred at room temperature for 3 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/8) to obtain compound 399-4.

### Step 4: synthesis of compound 399-5

Compound 399-4 (1.1 g, 2.33 mmol) was dissolved in dichloromethane (10 ml), and hydrogen chloride-ethyl acetate solution (11.6 ml, 2N) was added dropwise under ice bath conditions, and the reaction mixture was stirred at room temperature for 2 hours. A solid was precipitated out from the reaction solution, and the reaction solution was neutralized, extracted, and concentrated, to directly give compound 399-5.

### Step 5: synthesis of compound 399-6

Compound 399-5 (780 mg, 2.09 mmol), cesium carbonate (2.05 g, 6.27 mmol), and RuPhos Pd G4 (178 mg, 0.209 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/7) to obtain compound 399-6.

### Step 6: synthesis of compound 399-7

The compound 399-6 (370 mg, 1.27 mmol) and 3M hydrochloric acid (5 ml) were dissolved in tetrahydrofuran (5 ml), and stirred at room temperature for 3 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/8) to obtain compound 399-7.

### Step 7: synthesis of compound 399-8

The compound 399-7 (180 mg, 0.726 mmol) and bis(2-methoxyethyl)aminosulphur trifluoride (209 mg, 0.944 mmol) were dissolved in dichloromethane (5 ml) and stirred at room temperature for 2 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/15-1/10) to obtain compound 399-8.

### Step 8: synthesis of compound 399-9

Compound 399-8 (130 mg, 0.481 mmol), iron dust (135 mg, 2.4 mmol) and ammonium chloride (128 mg, 2.4 mmol) were dissolved in ethanol (5 ml) and water (2 ml), and the reaction mixture was stirred at 80°C for 3 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/8) to obtain compound 399-9.

### Step 9: synthesis of compound 399-10

Compound 399-9 (100 mg, 0.416 mmol), 4-bromo-2-(4-(difluoromethylene)piperidin-1-yl)benzoic acid (138 mg, 0.416 mmol), HATU (238 mg, 0.625 mmol) and DIEA (161 mg, 1.25 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 399-10.

### Step 10: synthesis of compound 399

Compound 399-10 (120 mg, 0.217 mmol), cesium carbonate (212 mg, 0.65 mmol), tBuXPhos Pd G4 (18 mg, 0.022 mmol) and 2-hydroxyethylsulfonamide (54 mg, 0.433 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 399. MS m/z (ESI):599.2 [M+1]⁺.

¹H NMR (400 MHz, CHLOROFORM-d) *δ* = 11.77 (s, 1H), 8.19 (d, *J =* 8.5 Hz, 1H), 7.90 (d, *J =* 1.9 Hz, 1H), 7.25 - 7.20 (m, 1H), 7.10 - 6.98 (m, 2H), 6.77 (d, *J* = 8.4 Hz, 1H), 6.56 (dd, *J* = 2.0, 8.4 Hz, 1H), 4.27 (dd, *J* = 2.3, 10.9 Hz, 1H), 4.13 (br t, *J* = 4.9 Hz, 2H), 4.08 - 3.97 (m, 2H), 3.42 - 3.35 (m, 1H), 3.31 (t, *J* = 5.1 Hz, 2H), 3.05 (t, *J* = 5.4 Hz, 4H), 3.03 - 2.96 (m, 1H), 2.46 (br s, 4H), 2.17 - 2.07 (m, 2H), 2.02 - 1.75 (m, 2H).

### Example 400: synthesis of N-(8,8-difluoro-6,6a,7,8,9,10-hexahydrobenzo[b]pyrido[1,2-d][1,4]oxazin-4-yl)-4-((2-hydroxyethyl)sulfonamido)-2-(6-azaspiro[2.5]oct-6-yl)benzamide (400)

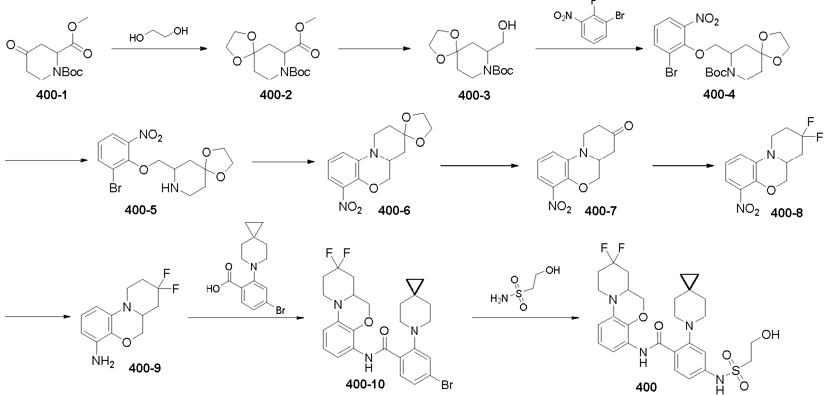

### Step 1: synthesis of compound 400-2

Compound 400-1 (3 g, 11.66 mmol), and p-toluenesulfonic acid (200 mg, 1.17 mmol) were dissolved in toluene (15 mL), to which ethylene glycol (1.78 mL, 34.98 mmol) was added, and the mixture was reacted at 100°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 400-2.

### Step 2: synthesis of compound 400-3

Compound 400-2 (1.052 g, 3.49 mmol) was dissolved in THF (5 mL), to which LiAlH₄ (159 mg, 4.19 mmol) was added under ice bath conditions, and the reaction mixture was reacted under ice bath conditions for 40 minutes. The reaction solution was quenched with ice water, and extracted with EA. The organic phase was washed with saturated NaCl solution, dried over anhydrous Na₂SO₄, and concentrated under reduced pressure to obtain compound 400-3.

### Step 3: synthesis of compound 400-4

Compound 400-3 (694 mg, 2.54 mmol), 1-bromo-2-fluoro-3-nitrobenzene (559 mg, 2.54 mmol), and cesium carbonate (1.65 g, 5.08 mmol) were dissolved in DMF (5 mL), and the mixture was reacted overnight at 50°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 400-4.

### Step 4: synthesis of compound 400-5

Compound 400-4 (521 mg, 1.10 mmol) was dissolved in DCM (2 mL), to which HCl/dioxane solution (3 mL) was added, and the reaction mixture was allowed to react at 25°C for 30 minutes. The reaction solution was neutralized, extracted and dried, and concentrated under reduced pressure to obtain compound 400-5.

### Step 5: synthesis of compound 400-6

Compound 400-5 (375 mg, 0.92 mmol), cesium carbonate (898 mg, 2.76 mmol) and the fourth-generation palladium catalyst (CAS: 1599466-85-9; 78 mg, 0.09 mmol) were dissolved in 1,4-dioxane (5 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 400-6.

### Step 6: synthesis of compound 400-7

Compound 400-6 (200 mg, 1.78 mmol) was dissolved in THF (3 mL), to which 3M HCl (3 mL) was added, and the reaction mixture was allowed to react at 25°C for 1 hour. The reaction solution was adjusted to pH 7 with saturated NaHCO₃ solution, and extracted with EA. The organic phase was washed with saturated NaCl solution, dried over anhydrous Na₂SO₄, and concentrated under reduced pressure to obtain compound 400-7.

### Step 7: synthesis of compound 400-8

Compound 400-7 (167 mg, 0.67 mmol) was dissolved in DCM (5 mL), to which Deoxo-Flour (0.62 mL, 3.36 mmol) was slowly added at -78°C under nitrogen protection, and the mixture was slowly warmed from -78°C to room temperature and reacted overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 400-8. MS (ESI, pos.ion) m/z:271.1 [M+1]⁺.

### Step 8: synthesis of compound 400-9

Compound 400-8 (123 mg, 0.46 mmol), and ammonium chloride (122 mg, 2.28 mmol) were dissolved in EtOH/H₂O (3 mL/1 mL), to which iron dust (127 mg, 2.28 mmol) was added, and the reaction mixture was allowed to react at 80°C for 1 hour. The reaction solution was concentrated under reduced pressure. The crude product was purified by silica gel column chromatography (PE:EA=10:1) to obtain compound 400-9. MS (ESI, pos.ion) m/z:241.3 [M+1]⁺.

### Step 9: synthesis of compound 400-10

Compound 400-9 (75 mg, 0.31 mmol), 4-bromo-2-(6-azaspiro[2.5]oct-6-yl)benzoic acid (97 mg, 0.31 mmol), and HATU (131 mg, 0.34 mmol) were dissolved in DMF (3 mL), to which DIEA (0.15 mL, 0.94 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 400-10.

### Step 10: synthesis of compound 400

Compound 400-10 (134 mg, 0.25 mmol), 2-hydroxyethanesulfonamide (126 mg, 1.01 mmol), cesium carbonate (246 mg, 0.76 mmol) and the fourth-generation palladium catalyst (CAS: 1599466-83-7; 46 mg, 0.05 mmol) were dissolved in 1,4-dioxane (5 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 400 (87.74 mg). MS (ESI, pos.ion) m/z:577.2 [M+1]⁺.

¹H NMR (400 MHz, DMSO-d₆) δ = 11.48 (s, 1H), 8.01 (d, *J* = 7.1 Hz, 1H), 7.92 (d, *J =* 8.6 Hz, 1H), 7.21 - 7.13 (m, 1H), 7.02 (dd, *J* = 1.8, 8.6 Hz, 1H), 6.86 - 6.74 (m, 2H), 4.41 (br d, *J* = 9.3 Hz, 1H), 4.20 - 4.14 (m, 1H), 4.09 (br d, *J* = 13.6 Hz, 1H), 3.75 (t, *J* = 6.5 Hz, 2H), 3.41 - 3.38 (m, 1H), 3.31 (br s, 2H), 2.99 - 2.90 (m, 4H), 2.89 - 2.82 (m, 1H), 2.26 - 2.05 (m, 2H), 2.00 - 1.78 (m, 2H), 1.54 (br s, 4H), 0.36 (s, 4H).

### Example 401: synthesis of 2-(4-(difluoromethylene)piperidin-1-yl)-N-(8,9-dihydro-6H-pyrido[3',2':4,5]pyrrolo[2,1-c][1,4]oxazin-4-yl)-4-((2-hydroxyethyl)sulfonamido)benzamide

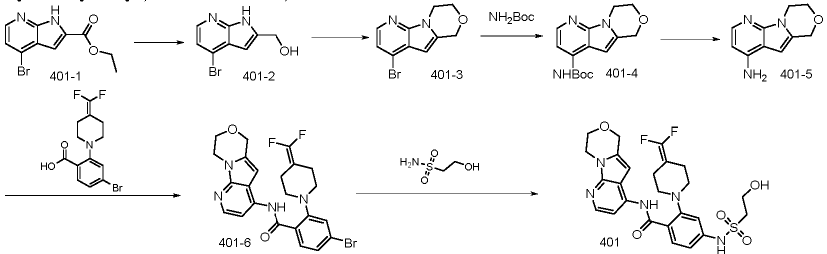

### Step 1: synthesis of compound 401-2

Compound 401-1 (900 mg, 3.34 mmol) was dissolved in THF and lithium aluminium hydride (152.3 mg, 4.01 mmol) was slowly added at 0°C under nitrogen protection. The reaction system was reacted at 0°C for 0.5 h. The reaction solution was quenched by adding water (1 ml) and 1N NaOH (1 ml), water (10 ml) was added, and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 401-2.

### Step 2: synthesis of compound 401-3

Compound 401-2 (600 mg, 2.64 mmol) and potassium hydroxide (370.67 mg, 6.61 mmol) were dissolved in dichloromethane, to which diphenyl(vinyl)sulfonium trifluoromethanesulfonate (1149.10 mg, 3.17 mmol) was slowly added dropwise at 0°C. The reaction system was moved to room temperature and reacted for 3 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 401-3.

### Step 3: synthesis of compound 401-4

Compound 401-3 (230 mg, 0.91 mmol), tert-butyl carbamate (425.83, 3.63 mmol), cesium carbonate (888 mg, 2.73 mmol) and palladium catalyst (CAS: 1599466-85-9; 41 mg, 0.05 mmol) were dissolved in dioxane (5 mL), and the reaction mixture was reacted at 90°C for 2 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 4/1) to obtain compound 401-4. MS m/z (ESI):290.2 [M+1]⁺.

### Step 4: synthesis of compound 401-5

Compound 401-4 (195 mg, 0.67 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 401-5 which was used directly in the next step without purification. MS m/z (ESI):190.0 [M+1]⁺.

### Step 5: synthesis of compound 401-6

Compound 401-5 (50 mg, 0.26 mmol), 4-bromo-2-(6-azaspiro[2.5]oct-6-yl)benzoic acid (96.54 mg, 0.29 mmol), TCFH (81.56 mg, 0.29 mmol) and N,N-diisopropylethylamine (0.13 mL, 0.79 mmol) were dissolved in DMF (4 ml), and the reaction mixture was reacted at room temperature for 1 hour under nitrogen protection at 0°C. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 5/1) to obtain compound 401-6.

### Step 6: synthesis of compound 401

Compound 401-6 (80 mg, 0.16 mmol), 2-hydroxyethylsulfonamide (40 mg, 0.32 mmol), cesium carbonate (155.4 mg, 0.48 mmol) and palladium catalyst (CAS: 1599466-89-3; 6.4 mg, 0.05 mmol) were dissolved in dioxane (3 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 401. MS m/z (ESI):548.1 [M+1]⁺.

**¹H NMR** (400 MHz, DMSO-d6) δ = 10.88 (s, 1H), 8.14 (d, *J* = 5.4 Hz, 1H), 8.03 (br d, *J =* 5.4 Hz, 1H), 7.73 (d, *J* = 8.4 Hz, 1H), 7.06 - 7.03 (m, 1H), 7.01 (dd, *J =* 1.9, 8.5 Hz, 1H), 6.55 (s, 1H), 4.94 (s, 2H), 4.13 (s, 4H), 3.80 - 3.74 (m, 2H), 3.37 - 3.33 (m, 2H), 3.04 - 2.97 (m, 4H), 2.20 (br s, 4H).

### Example 404: synthesis of 4-((2-hydroxyethyl)sulfonamido)-N-(6-methoxy-3,4-dihydro-1H-[1,4]oxazinyl[4,3-a]indol-9-yl)-2-(6-azaspiro[2.5]oct-6-yl)benzamide

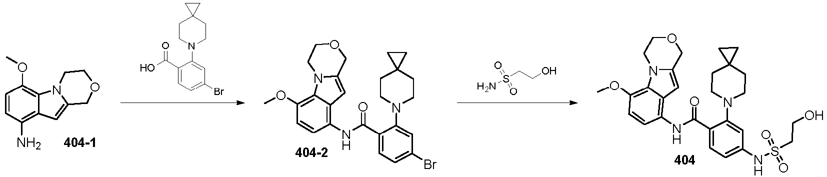

### Step 1: synthesis of compound 404-2

Compound 404-1 (30 mg, 0.12 mmol), 4-bromo-2-(6-azaspiro[2.5]oct-6-yl)benzoic acid (41 mg, 0.13 mmol), and HATU (49 mg, 0.13 mmol) were dissolved in DMF (3 mL), to which DIEA (0.08 mL, 0.47 mmol) was added, and the reaction mixture was allowed to react at 25°C for 1 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 404-2.

### Step 2: synthesis of compound 404

Compound 404-2 (30 mg, 0.68 mmol), 2-hydroxyethanesulfonamide (29 mg, 0.24 mmol), cesium carbonate (57 mg, 0.18 mmol) and the fourth-generation palladium catalyst (CAS: 1599466-89-3; 5 mg, 0.01 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 404. MS (ESI, pos.ion) m/z:555.2 [M+1]⁺.

¹H NMR (400 MHz, DMSO-d₆) δ = 11.08 (s, 1H), 7.85 (d, *J =* 8.5 Hz, 1H), 7.66 (d, *J* = 8.4 Hz, 1H), 7.17 (d, *J* = 1.9 Hz, 1H), 7.02 (dd, *J* = 1.9, 8.5 Hz, 1H), 6.63 (d, *J=* 8.5 Hz, 1H), 6.35 (s, 1H), 4.89 (s, 2H), 4.40 (t, *J* = 5.3 Hz, 2H), 4.07 (t, *J= 5.3* Hz, 2H), 3.87 (s, 3H), 3.76 (t, *J* = 6.6 Hz, 2H), 3.32 - 3.30 (m, 2H), 3.00 (br t, *J* = 4.9 Hz, 4H), 1.45 (br s, 4H), 0.28 (s, 4H).

### Example 405: synthesis of 2-(4-(difluoromethylene)piperidin-1-yl)-4-((2-hydroxyethyl)sulfonamido)-N-(6-methoxy-3,4-dihydro-1H-[1,4]oxazinyl[4,3-a]indol-9-yl)benzamide

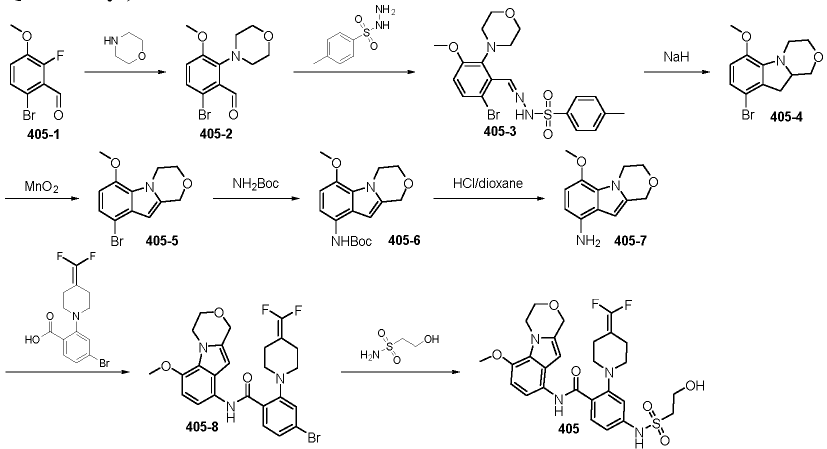

### Step 1: synthesis of compound 405-2

Compound 405-1 (4 g, 17.16 mmol), and granular potassium carbonate (3.08 g, 22.31 mmol) were dissolved in DMSO (20 mL), to which morpholine (3.02 mL, 34.33 mmol) was added, and the reaction mixture was allowed to react at 95°C for 5 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 405-2.

### Step 2: synthesis of compound 405-3

Compound 405-2 (1.98 g, 6.60 mmol) was dissolved in toluene (16 mL), to which p-toluenesulfonyl hydrazide (1.47 g, 7.92 mmol) was added, and the mixture was reacted at room temperature for 10 minutes. The reaction solution was quenched with ice water, and extracted with EA. The organic phase was washed with saturated NaCl solution, dried over anhydrous Na₂SO₄, and concentrated under reduced pressure to obtain compound 405-3.

### Step 3: synthesis of compound 405-4

Compound 405-3 (3.09 g, 6.60 mmol) was dissolved in toluene (30 mL), to which NaH (290 mg, 7.26 mmol) was slowly added at 0°C, and after stirring for 30 minutes, the mixture was reacted at 135°C for 10 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 405-4.

### Step 4: synthesis of compound 405-5

Compound 405-4 (395 mg, 1.39 mmol) was dissolved in DCM (8 mL), to which manganese dioxide (1.2 g, 13.90 mmol) was added, and the mixture was reacted at 40°C for 3 h. The reaction solution was filtered, and concentrated under reduced pressure. The crude product was purified by silica gel column chromatography (PE:EA=10:1) to obtain compound 405-5.

### Step 5: synthesis of compound 405-6

Compound 405-5 (130 mg, 0.46 mmol), NH₂Boc (108 mg, 0.92 mmol), cesium carbonate (450 mg, 1.38 mmol) and the fourth-generation palladium catalyst (CAS: 1599466-83-7; 85 mg, 0.09 mmol) were dissolved in 1,4-dioxane (3 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 405-6. MS (ESI, pos.ion) m/z:263.0 [M+1-56]⁺.

### Step 6: synthesis of compound 405-7

Compound 405-6 (130 mg, 0.41 mmol) was dissolved in DCM (2 mL), to which HCl\dioxane (3 mL) was added, and the reaction mixture was allowed to react at 25°C for 1 hour. The reaction solution was filtered, neutralized, extracted and dried, and concentrated to obtain compound 405-7. MS (ESI, pos.ion) m/z:219.1 [M+1-HCl]⁺.

### Step 7: synthesis of compound 405-8

Compound 405-7 (35 mg, 0.11 mmol), 4-bromo-2-(4-(difluoromethylene)piperidin-1-yl)benzoic acid (40 mg, 0.12 mmol), and HATU (46 mg, 0.12 mmol) were dissolved in DMF (3 mL), to which DIEA (0.07 mL, 0.44 mmol) was added, and the reaction mixture was allowed to react at 25°C for 2 h. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 405-8.

### Step 8: synthesis of compound 405

Compound 405-8 (40 mg, 0.08 mmol), 2-hydroxyethanesulfonamide (38 mg, 0.30 mmol), cesium carbonate (73 mg, 0.23 mmol) and the fourth-generation palladium catalyst (CAS: 1599466-89-3; 6 mg, 0.01 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 405. MS (ESI, pos.ion) m/z:577.2 [M+1]⁺.

¹H NMR (400 MHz, DMSO-d₆) δ = 10.62 (s, 1H), 7.77 (d, *J =* 8.5 Hz, 1H), 7.73 - 7.66 (m, 1H), 7.07 (s, 1H), 7.05 - 6.98 (m, 1H), 6.63 (d, *J* = 8.4 Hz, 1H), 6.37 (s, 1H), 4.91 - 4.85 (m, 2H), 4.38 (br t, *J* = 4.9 Hz, 2H), 4.06 (br t, *J* = 5.1 Hz, 2H), 3.87 (s, 3H), 3.80 - 3.72 (m, 2H), 3.32 - 3.30 (m, 2H), 3.06 - 2.96 (m, 4H), 2.23 (br s, 4H).

### Example 410: synthesis of compound 4-(2-hydroxyethyl)sulfonamido-N-(9-methyl-6,7-dihydropyridino[1,2-a]indol-1-yl)-2-(6-azaspiro[2.5]octan-6-yl)benzamide

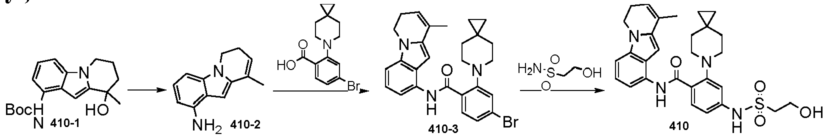

### Step 1: synthesis of compound 410-2

Compound 410-1 (150 mg, 0.47 mmol) was dissolved in hydrogen chloride-ethyl acetate solution and the mixture was reacted at room temperature for 1 hour. The reaction solution was concentrated under reduced pressure and then washed with an aqueous saturated sodium bicarbonate solution until the pH reached 9, and extracted with EA (20 mL). The organic phase was washed with saturated NaCl solution (10 mL), dried over anhydrous Na₂SO₄, and concentrated under reduced pressure. The crude product was purified by silica gel column chromatography (PE:EA=100:0-50:50) to obtain compound 410-2. MS m/z (ESI):199.1 [M+1]⁺.

### Step 2: synthesis of compound 410-3

Compound 410-2 (60 mg, 0.30 mmol), 2-(6-azaspiro[2.5]octan-6-yl)-4-bromobenzoic acid (103.26 mg, 0.33 mmol), HATU (137.99 mg, 0.36 mmol) and N,N-diisopropylethylamine (78.08 ml, 0.61 mmol) were dissolved in N,N-dimethylformamide (3 ml), and the reaction mixture was reacted at room temperature for 1 hour under nitrogen protection. The reaction solution was quenched by adding water (5 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 100:0 - 90:10) to obtain compound 410-3. MS m/z (ESI): 490.1/492.1 [M+1]⁺.

**¹H NMR** (400 MHz, DMSO-*d*₆) δ = 10.97 (s, 1H), 7.97 (d, *J =* 8.0 Hz, 1H), 7.78 (d, *J =* 8.4 Hz, 1H), 7.47 (s, 1H), 7.41 (d, *J* = 8.4 Hz, 1H), 7.20 (d, *J* = 8.0 Hz, 1H), 7.13 (t, *J* = 8.0 Hz, 1H), 6.76 (s, 1H), 5.86 - 5.84 (m, 1H), 4.11 (t, *J =* 6.7 Hz, 2H), 3.10 - 3.07 (m, 4H), 2.60 - 2.57 (m, 2H), 2.08 - 2.05 (m, 3H), 1.52-1.48 (m, 4H), 0.27 (s, 4H).

### Step 3: synthesis of compound 410

Compound 410-3 (70 mg, 0.14 mmol), 2-hydroxyethylsulfonamide (71.44 mg, 0.57 mmol), cesium carbonate (93.01 mg, 0.29 mmol) and palladium catalyst (CAS: 1599466-89-3; 12.27 mg, 0.01 mmol) were dissolved in 1,4-dioxane (3 mL), and the reaction mixture was reacted at 90°C for 1.5 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by preparative chromatography to obtain compound 410. MS m/z (ESI):535.2 [M+1]⁺.

**¹H NMR** (400 MHz, DMSO-*d*₆) δ = 11.22 (s, 1H), 8.01 (d, *J* = 7.4 Hz, 1H), 7.90 (d, *J =* 8.5 Hz, 1H), 7.22 - 7.11 (m, 3H), 7.05 (dd, *J =* 1.9, 8.4 Hz, 1H), 6.72 (s, 1H), 5.85 (br d, *J =* 1.4 Hz, 1H), 4.11 (t, *J =* 7.1 Hz, 2H), 3.77 (t, *J =* 6.6 Hz, 2H), 3.36 - 3.34 (m, 2H), 3.04 - 3.03 (m, 4H), 2.61 - 2.59 (m, 2H), 2.07 (s, 3H), 1.55-1.53 (m, 4H), 0.29 (s, 4H).

### Example 411: synthesis of 4-((2-hydroxyethyl)sulfonamido)-2-(6-azaspiro[2.5]oct-6-yl)-N-(3,4,10,10-tetrahydro-1H-[1,4]oxazino[4,3-a]indol-9-yl)benzamide

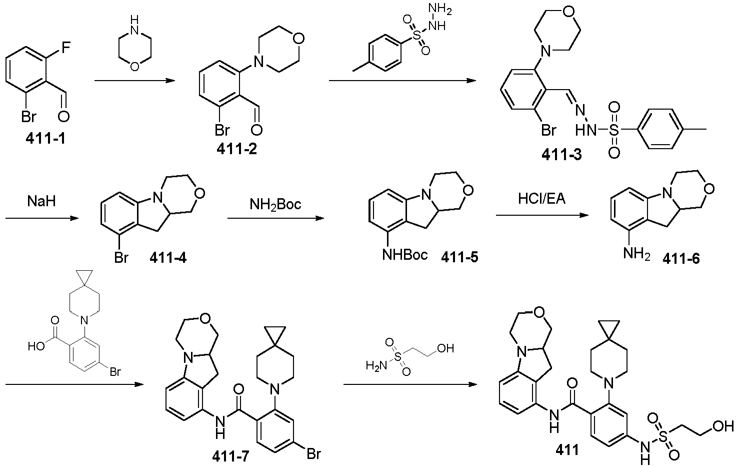

### Step 1: synthesis of compound 411-2

Compound 411-1 (2 g, 9.85 mmol), and granular potassium carbonate (1.77 g, 12.81 mmol) were dissolved in DMSO (12 mL), to which morpholine (1.04 mL, 11.82 mmol) was added, and the reaction mixture was allowed to react at 95°C for 5 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 411-2.

### Step 2: synthesis of compound 411-3

Compound 411-2 (1.968 g, 7.29 mmol) was dissolved in toluene (10 mL), to which p-toluenesulfonyl hydrazide (1.63 g, 8.74 mmol) was added, and the mixture was reacted at room temperature for 10 minutes. The reaction solution was quenched with ice water, and extracted with EA. The organic phase was washed with saturated NaCl solution, dried over anhydrous Na₂SO₄, and concentrated under reduced pressure to obtain compound 411-3.

### Step 3: synthesis of compound 411-4

Compound 411-3 (739 g, 1.69 mmol) was dissolved in toluene (20 mL), to which NaH (445 mg, 1.85 mmol) was slowly added at 0°C, and after stirring for 30 minutes, the mixture was reacted at 135°C for 10 minutes under microwave irradiation. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 411-4.

### Step 4: synthesis of compound 411-5

Compound 411-4 (170 mg, 0.67 mmol), NH₂Boc (157 mg, 1.34 mmol), cesium carbonate (654 mg, 2.01 mmol) and the fourth-generation palladium catalyst (CAS: 1599466-83-7; 123 mg, 0.13 mmol) were dissolved in 1,4-dioxane (3 mL), and the reaction mixture was reacted at 95°C for 4 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 411-5.

### Step 5: synthesis of hydrochloride of compound 411-6

Compound 411-5 (170 mg, 0.59 mmol) was dissolved in DCM (1 mL), to which HCl\EA (3 mL) was added, and the reaction mixture was allowed to react at 25°C overnight. The reaction solution was filtered, neutralized, extracted and dried, and concentrated to obtain compound 411-6.

### Step 6: synthesis of compound 411-7

Compound 411-6 (114 mg, 0.50 mmol), 4-bromo-2-(6-azaspiro[2.5]oct-6-yl)benzoic acid (172 mg, 0.55 mmol), and TCFH (212 mg, 0.76 mmol) were dissolved in ACN (3 mL), to which NMI (0.20 mL, 2.52 mmol) was added, and the reaction mixture was allowed to react at 25°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 411-7.

### Step 7: synthesis of compound 411

Compound 411-7 (140 mg, 0.29 mmol), 2-hydroxyethanesulfonamide (145 mg, 1.16 mmol), cesium carbonate (284 mg, 0.87 mmol) and the fourth-generation palladium catalyst (CAS: 1599466-89-3; 24 mg, 0.03 mmol) were dissolved in 1,4-dioxane (3 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 411.

MS (ESI, pos.ion) m/z:527.2 [M+1]⁺. ¹H NMR (400 MHz, METHANOL-d₄) δ = 8.11 (br d, *J* = 8.8 Hz, 1H), 7.51 (br s, 1H), 7.31 (br d, *J* = 8.3 Hz, 1H), 7.12 (t, *J* = 7.9 Hz, 1H), 6.85 (br d, *J* = 7.5 Hz, 1H), 6.47 (d, *J* = 7.8 Hz, 1H), 3.97 (t, *J* = 6.1 Hz, 2H), 3.81 (dd, *J* = 2.8, 10.8 Hz, 2H), 3.67 - 3.60 (m, 1H), 3.60 - 3.54 (m, 2H), 3.49 - 3.38 (m, 7H), 3.19 - 3.09 (m, 1H), 2.97 (dd, *J* = 7.9, 15.6 Hz, 1H), 2.53 (dd, *J* = 7.1, 15.4 Hz, 1H), 1.73 (br s, 4H), 0.51 (s, 4H).

### Example 427: synthesis of N-(4-cyano-8,8-difluoro-9-methoxy-6,7,8,9-tetrahydropyridino[1,2-a]indol-1-yl)-2-(4-(difluoromethylene)piperidin-1-yl)-4-((2-hydroxyethyl)sulfonamide)benzamide (427)

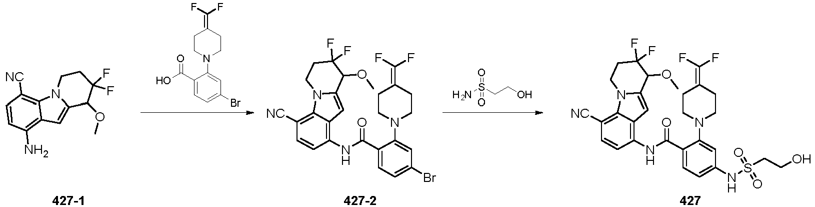

### Step 1: synthesis of compound 427-2

Compound 427-1 (141 mg, 0.51 mmol) and 4-bromo-2-(4-(difluoromethylene)piperidin-1-yl)benzoic acid (219.57 mg, 0.66 mmol) were dissolved in THF (5 mL), to which (2,4-dimethyl-2,4-diazapentan-3-ylidene)hexafluoro-λ5-phosphazene (285.37 mg, 1.02 mmol) and DIEA (0.42 mL, 2.54 mmol) were added, and the reaction mixture was allowed to react at 45°C for 18 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 427-2.

### Step 2: synthesis of compound 427

Compound 427-2 (233 mg, 0.39 mmol), 2-hydroxyethanesulfonamide (73.95 mg, 0.59 mmol), potassium carbonate (107.8 mg, 0.78 mmol) and palladium catalyst (CAS: 1599466-89-3; 31.85 mg, 0.04 mmol) were dissolved in 1,4-dioxane (5 mL), and the reaction mixture was reacted at 82°C for 1.5 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-1/1) to obtain compound 427. MS m/z (ESI):636.2 [M+1]⁺.

**¹H NMR** (CHLOROFORM-d) δ: 10.98 (s, 1H), 10.14 (br s, 1H), 8.27 (br d, *J* = 8.4 Hz, 1H), 7.76 (d, *J* = 8.4 Hz, 1H), 7.71 (d, *J* = 8.4 Hz, 1H), 7.22 (s, 1H), 7.07 (s, 1H), 7.03 (dd, *J* = 8.4, 1.8 Hz, 1H), 4.94-5.03 (m, 1H), 4.90 (br dd, *J* = 9.2, 5.8 Hz, 1H), 4.71-4.83 (m, 1H), 4.45 (ddd, *J=* 12.0, 8.4, 6.2 Hz, 1H), 3.77 (br t, *J =* 6.4 Hz, 2H), 3.47 (s, 3H), 3.33-3.37 (m, 2H), 3.01 (br t, *J* = 5.4 Hz, 4H), 2.72-2.92 (m, 1H), 2.60-2.72 (m, 1H), 2.13-2.31 (m, 4H).

### Example 428: synthesis of N-(4-cyano-8,8-difluoro-9-methoxy-6,7,8,9-tetrahydropyridino[1,2-a]indol-1-yl)-4-((2-hydroxyethyl)sulfonamido)-2-(6-azaspiro[2.5]oct-6-yl)benzamide (428)

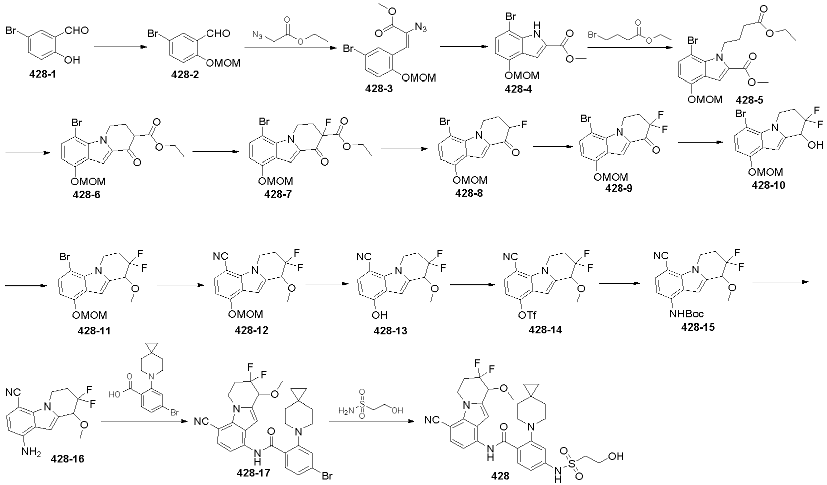

### Step 1: synthesis of compound 428-2

Compound 428-1 (30 g, 149.24 mmol) and K₂CO₃ (61.87 g, 447.72 mmol) were dissolved in DMF (250 mL), to which bromo(methoxy)methane (24.22 mL, 298.48 mmol) was added dropwise at 50°C-60°C, and the mixture was reacted for 20 min. After monitoring the complete reaction of raw materials, the reaction solution was poured into water and extracted with EA. The organic phase was washed with saturated NaCl solution, dried over anhydrous Na₂SO₄, and concentrated under reduced pressure to obtain compound 428-2.

### Step 2: synthesis of compound 428-3

Compound 428-2 (38 g, 155.06 mmol) and ethyl azidoacetate (30.12 mL, 263.60 mmol) were dissolved in MeOH (114 mL). In addition, sodium methoxide (16.75 g, 310.12 mmol) was dissolved in MeOH (76 mL), and a solution of sodium methoxide in methanol was added dropwise to the reaction of compound 428-2 at 0°C, and the reaction was allowed to naturally warm to 25°C and react for 3 h. After monitoring the complete reaction of raw materials, the reaction solution was poured into ice water and filtered to obtain the filter cake. The filter cake was slurried with methanol to obtain compound 428-3.

### Step 3: synthesis of compound 428-4

Compound 428-3 (25.4 g, 74.24 mmol) was dissolved in 1,2-dichlorobenzene (160 mL), and the mixture was reacted at 170°C for 1.5 h. The reaction solution was cooled to room temperature and then directly purified by silica gel column chromatography (PE:EA=100/1-6/1) to obtain compound 428-4.

### Step 4: synthesis of compound 428-5

Compound 428-4 (11.93 g, 37.98 mmol), and Cs₂CO₃ (24.75 g, 75.96 mmol) were dissolved in DMF (100 mL). Ethyl 4-bromobutyrate (6.54 mL, 45.57 mmol) was added dropwise at 0°C. The mixture was reacted at 25°C for 2 h, and then at 50°C for 0.5 h. After monitoring the complete reaction of raw materials, the reaction solution was poured into ice water, and extracted with EA. The organic phase was dried over anhydrous Na₂SO₄, and concentrated under reduced pressure to obtain crude compound 428-5.

### Step 5: synthesis of compound 428-6

Compound 428-5 (13.9 g, 32.46 mmol) was dissolved in THF (150 mL), to which potassium tert-butoxide (7283 mg, 64.91 mmol) was added, and the mixture was reacted at 25°C for 2 h. After monitoring the complete reaction of raw materials, the reaction solution was poured into ice water, and extracted with EA. The organic phase was concentrated under reduced pressure to obtain compound 428-6.

### Step 6: synthesis of compound 428-7

Compound 428-6 (12.1 g, 30.54 mmol) was dissolved in MeCN (130 mL), to which N-(dioxophenyl-λ6-sulfanyl)-N-fluorobenzenesulfonamide (11.56 g, 36.64 mmol) was added, and the mixture was reacted at 50°C for 1.5 h. After monitoring the complete reaction of raw materials, the reaction solution was concentrated, and the residue was dissolved in DCM. The organic phase was washed with water and saturated NaCl solution, dried over anhydrous Na₂SO₄, and concentrated under reduced pressure. The crude product was purified by silica gel column chromatography (PE:EA=100/1-4/1) to obtain compound 428-7.

### Step 7: synthesis of compound 428-8

Compound 428-7 (13.6 g, 32.83 mmol) was dissolved in THF (68 mL), to which NaOH (68 mL, 136 mmol, 2 M) was added, and the reaction mixture was allowed to react at 25°C for 2 h. H₂O (150 mL) was added into the reaction, and then extracted with DCM. The organic phase was washed with saturated NaCl solution, dried over anhydrous Na₂SO₄, and concentrated under reduced pressure to obtain compound 428-8.

### Step 8: synthesis of compound 428-9

Compound 428-8 (7200 mg, 21.04 mmol) was dissolved in THF (70 mL), and the reaction solution was cooled to -65°C. LiHMDS (52.61 mL, 52.61 mmol, 1 M) were added dropwise, and the reaction mixture was reacted at -65°C for 1 h under N₂ protection. The reaction solution was then slowly warmed to 0°C. N-(Dioxyphenyl-λ6-sulfanyl)-N-fluorobenzenesulfonamide (9953 mg, 31.56 mmol) was added to the reaction solution at 0-10°C, and the mixture was reacted at 25°C for 1 h. The reaction was quenched with water, and extracted with EA. The organic phase was washed with saturated NaCl solution, dried over anhydrous Na₂SO₄, and concentrated under reduced pressure. The crude product was purified by silica gel column chromatography (PE:EA=100/1-4/1) to obtain compound 428-9.

### Step 9: synthesis of compound 428-10

Compound 428-9 (560 mg, 1.55 mmol) was dissolved in MeOH (2 mL) and THF (2 mL), and NaBH₄ (88.23 mg, 2.33 mmol) was added to the reaction at 0°C, and the mixture was reacted at 0°C for 20 min. Water was added into the reaction solution, and extracted with EA. The organic phase was washed with saturated NaCl solution, dried over anhydrous Na₂SO₄, and concentrated under reduced pressure to obtain compound 428-10.

### Step 10: synthesis of compound 428-11

Compound 428-10 (563 mg, 1.55 mmol) and Cs₂CO₃ (1012.99 mg, 3.11 mmol) were dissolved in DMF (6 mL), and iodomethane (0.12 mL, 1.87 mmol) was added to the reaction solution, and the mixture was reacted at 45°C for 3 h. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-33/1) to obtain compound 428-11.

**¹H NMR** (CHLOROFORM-d) δ: 7.27 (d, *J* **=** 8.4 Hz, 1H), 6.75 (s, 1H), 6.67 (d, *J* = 8.4 Hz, 1H), 5.22-5.37 (m, 3H), 4.51-4.57 (m, 1H), 4.41 (ddd, *J* = 12.2, 11.1, 5.6 Hz, 1H), 3.56 (s, 3H), 3.55 (s, 3H), 2.80-2.96 (m, 1H), 2.36-2.47 (m, 1H).

### Step 11: synthesis of compound 428-12

Compound 428-11 (465 mg, 1.24 mmol), and Zn(CN)₂ (725.6 mg, 6.18 mmol) were dissolved in DMF (5 mL), to which dppf (69.77 mg, 0.12 mmol) and palladium catalyst (CAS: 14221-01-3; 71.42 mg, 0.06 mmol) were added, and the reaction mixture was reacted at 130°C for 3 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 428-12.

### Step 12: synthesis of compound 428-13

Compound 428-12 (302 mg, 0.94 mmol) was dissolved in EA (2 mL), to which HCl\dioxane (2 mL, 4 M) was added dropwise, and the reaction mixture was allowed to react at 25°C for 2 h. Water was added into the reaction solution to adjust the pH to about 8, and extracted with EA. The organic phase was washed with saturated NaCl solution, dried over anhydrous Na₂SO₄, and concentrated under reduced pressure to obtain compound 428-13.

### Step 13: synthesis of compound 428-14

Compound 428-13 ( 272 mg, 0.98 mmol) and DIEA (0.32 mL, 1.96 mmol) were dissolved in DCM (5 mL), to which N-phenylbis(trifluoromethanesulfonyl)imide (453.96 mg, 1.27 mmol) was added at 0°C, and the mixture was reacted at 25°C for 2 h. Water was added into the reaction solution, and extracted with DCM. The organic phase was washed with saturated NaCl solution, dried over anhydrous Na₂SO₄, and concentrated under reduced pressure. The crude product was purified by silica gel column chromatography (PE:EA=100/1-10/1) to obtain compound 428-14. MS m/z (ESI):411.0 [M+1]⁺.

### Step 14: synthesis of compound 428-15

Compound 428-14 (360 mg, 0.88 mmol), and 2-methylprop-2-ylcarbamate (154.17 mg, 1.32 mmol) were dissolved in 1,4-dioxane (5 mL), to which Cs₂CO₃ (571.73 mg, 1.75 mmol) and palladium catalyst (CAS: 1599466-85-9; 37.31 mg, 0.04 mmol) were added, and the reaction mixture was reacted at 25°C for 1.5 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 428-15.

### Step 15: synthesis of compound 428-16

Compound 428-15 (371 mg, 0.98 mmol) was dissolved in EA (2 mL), to which HCl\dioxane (3.5 mL, 4 M) was added, and the reaction mixture was allowed to react at 25°C for 1 h, and then at 40°C for 1 h. The reaction solution was neutralized, extracted and dried, and concentrated to obtain compound 428-16.

### Step 16: synthesis of compound 428-17

Compound 428-16 (141 mg, 0.51 mmol) and 2-(6-azaspiro[2.5]oct-6-yl)-4-bromobenzoic acid (205.06 mg, 0.66 mmol) were dissolved in THF (5 mL), to which (2,4-dimethyl-2,4-diazapentan-3-ylidene)hexafluoro-λ5-phosphazene (285.37 mg, 1.02 mmol) and DIEA (0.42 mL, 2.54 mmol) were added, and the reaction mixture was allowed to react at 45°C for 18 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 428-17. MS m/z (ESI): 569.1\571.1 [M+1]⁺.

### Step 17: synthesis of compound 428

Compound 428-17 (157 mg, 0.28 mmol), 2-hydroxyethanesulfonamide (51.75 mg, 0.41 mmol), potassium carbonate (77.39 mg, 0.56 mmol) and palladium catalyst (CAS: 1599466-89-3; 22.29 mg, 0.03 mmol) were dissolved in 1,4-dioxane (5 mL), and the reaction mixture was reacted at 82°C for 1.5 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-1/1) to obtain compound 428. MS m/z (ESI):614.3 [M+1]⁺.

**¹H NMR** (CHLOROFORM-d) δ: 11.69 (s, 1H), 8.31 (d, *J* = 8.4 Hz, 1H), 8.16 (d, *J* = 8.4 Hz, 1H), 7.62 (d, *J* = 8.4 Hz, 1H), 7.34 (d, *J*=2.0 Hz, 1H), 7.05 (s, 1H), 7.00 (dd, *J* = 8.4 Hz, 2.0 Hz, 1H) 6.86 (s, 1H), 4.98-5.04 (m, 1H), 4.44-4.60 (m, 2H), 4.12-4.19 (m, 2H), 3.55 (s, 3H), 3.32-3.40 (m, 2H), 3.09-3.22 (m, 4H), 2.85-3.05 (m, 1H), 2.58 (br t, *J* = 5.0 Hz, 1H), 2.50 (br s, 1H), 1.49-1.60 (m, 4H), 0.29-0.38 (m, 4H).

### Example 438: synthesis of N-(4-cyano-8,8-difluoro-9-hydroxy-6,7,8,9-tetrahydropyridino[1,2-a]indol-1-yl)-4-((2-hydroxyethyl)sulfonamido)-2-(6-azaspiro[2.5]oct-6-yl)benzamide (438)

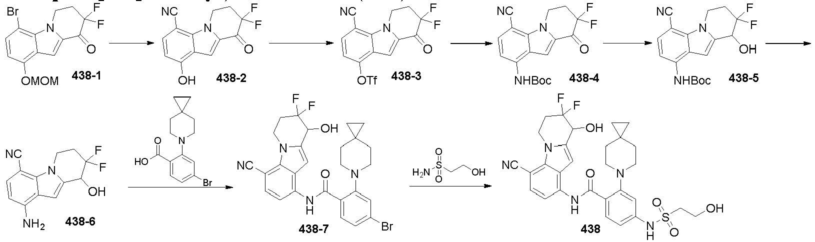

### Step 1: synthesis of compound 438-2

Compound 438-1 (200 mg, 0.56 mmol), and Zn(CN)₂ (326.03 mg, 2.78 mmol) were dissolved in DMF (5 mL), to which dppf (62.69 mg, 0.11 mmol) and palladium catalyst (CAS: 14221-01-3; 64.17 mg, 0.06 mmol) were added, and the reaction mixture was reacted at 130°C for 3 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 438-2.

### Step 2: synthesis of compound 438-3

Compound 438-2 (57 mg, 0.22 mmol) and DIEA (0.07 mL, 0.43 mmol) were dissolved in DCM (3 mL), to which N-phenylbis(trifluoromethanesulfonyl)imide (100.95 mg, 0.28 mmol) was added at 0°C, and the mixture was reacted at 25°C for 2 h. Water was added into the reaction solution, and extracted with DCM. The organic phase was washed with saturated NaCl solution, dried over anhydrous Na₂SO₄, and concentrated under reduced pressure. The crude product was purified by silica gel column chromatography (PE:EA=100/1-6/1) to obtain compound 438-3.

**¹H NMR** (CHLOROFORM-d) δ: 7.88 (d, *J* = 8.3 Hz, 1H), 7.68 (s, 1H), 7.30 (d, *J* = 8.3 Hz, 1H), 5.04 (t, *J* = 6.1 Hz, 2H), 2.89-3.00 (m, 2H).

### Step 3: synthesis of compound 438-4

Compound 438-3 (53 mg, 0.13 mmol), and 2-methylprop-2-ylcarbamate (31.5 mg, 0.27 mmol) were dissolved in 1,4-dioxane (5 mL), to which K₂CO₃ (37.16 mg, 0.27 mmol) and palladium catalyst (CAS: 1599466-85-9; 11.43 mg, 0.01 mmol) were added, and the reaction mixture was reacted at 75°C for 2 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-2/1) to obtain compound 438-4. MS m/z (ESI): 362.1 [M+1]⁺.

### Step 4: synthesis of compound 438-5

Compound 438-4 (86 mg, 0.24 mmol) was dissolved in MeOH (2 mL) and THF (2 mL), to which NaBH₄ (18.0 mg, 0.48 mmol) was added at 0°C, and the mixture was reacted at 25°C for 0.5 h. The reaction was quenched with water, and then extracted with EA. The organic phase was washed with saturated NaCl solution, dried over anhydrous Na₂SO₄, and concentrated under reduced pressure. The crude product was purified by silica gel column chromatography (PE:EA=100/1-2/1) to obtain compound 438-5. MS m/z (ESI): 364.1 [M+1] ⁺.

### Step 5: synthesis of compound 438-6

Compound 438-5 (41 mg, 0.11 mmol) was dissolved in EA (2 mL), to which HCl\EA (2 mL, 4 M) was added, and the reaction mixture was allowed to react at 40°C for 2 h. The reaction solution was concentrated, neutralized, extracted and dried, and concentrated to obtain compound 438-6.

### Step 6: synthesis of compound 438-7

Compound 438-6 (30 mg, 0.11 mmol) and 2-(6-azaspiro[2.5]oct-6-yl)-4-bromobenzoic acid (42.42 mg, 0.14 mmol) were dissolved in THF (3 mL), to which (2,4-dimethyl-2,4-diazapentan-3-ylidene)hexafluoro-λ5-phosphazene (63.95 mg, 0.23 mmol) and DIEA (0.09 mL, 0.57 mmol) were added, and the reaction mixture was allowed to react at 25°C for 3 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 438-7. MS m/z (ESI): 555.2\557.2 [M+1]⁺.

### Step 7: synthesis of compound 438

Compound 438-7 (39 mg, 0.07 mmol), 2-hydroxyethanesulfonamide (17.57 mg, 0.14 mmol), potassium carbonate (19.41 mg, 0.14 mmol) and palladium catalyst (CAS: 1599466-89-3; 5.65 mg, 0.007 mmol) were dissolved in 1,4-dioxane (3 mL), and the reaction mixture was reacted at 82°C for 1.5 h under N₂ protection. Water was added into the reaction solution, and extracted with EA. The organic phase was washed with saturated NaCl solution, dried over anhydrous Na₂SO₄, and concentrated under reduced pressure, and the crude product was purified by preparative chromatography to obtain compound 438. MS m/z (ESI):600.3 [M+1]⁺.

**¹H NMR** (CHLOROFORM-d) δ: 11.36 (s, 1H), 10.12 (s, 1H), 8.16 (d, *J* = 8.3 Hz, 1H), 7.81 (d, *J* = 8.5 Hz, 1H), 7.67 (d, *J* = 8.4 Hz, 1H), 7.18 (d, *J* = 2.0 Hz, 1H), 7.00-7.06 (m, 2H), 6.57 (d, *J =* 6.9 Hz, 1H), 4.82-5.16 (m, 2H), 4.67 (dt, *J =* 12.1, 5.9 Hz, 1H), 4.54 (dt, *J =* 12.7, 6.5 Hz, 1H), 3.77 (t, *J =* 6.6 Hz, 2H), 3.34-3.38 (m, 2H), 2.96-3.06 (m, 4H), 2.70-2.87 (m, 2H), 1.43 (br s, 4H), 0.27 (s, 4H).

### Example 444: synthesis of 2-(6-azaspiro[2.5]oct-6-yl)-N-(1,3-dimethyl-3,4-dihydro-1H-benzo[4,5]imidazo[2,1-c][1,4]oxazin-9-yl)-4-{[(2-hydroxyethyl)sulfonamide]}benzamide (444)

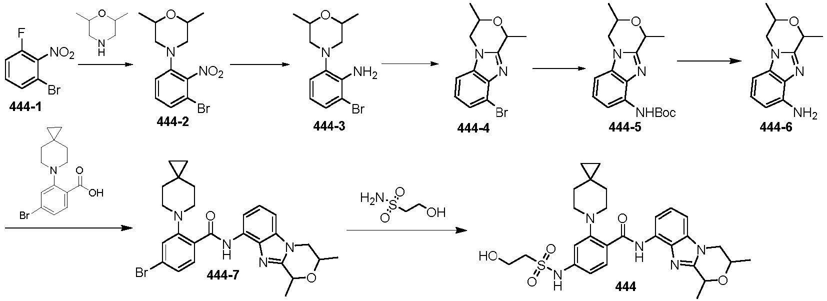

### Step 1: synthesis of compound 444-2

Compound 444-1 (3 g, 13.64 mmol) was dissolved in NMP (20 mL), to which 2,6-dimethylmorpholine (1.88 g, 16.36 mmol) and DIEA (3.52 g, 27.27 mmol) were added, and the reaction mixture was allowed to react at 110°C overnight. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 444-2.

### Step 2: synthesis of compound 444-3

Compound 444-2 (3.30 g, 10.47 mmol) was dissolved in ethanol (27 mL) and water (27 mL), to which iron dust (2.92 g, 52.35 mmol) and ammonium chloride (2.80 g, 52.35 mmol) were added. The reaction mixture was allowed to react at 80°C for 3 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 444-3.

### Step 3: synthesis of compound 444-4

Compound 444-3 (1.77 g, 6.20 mmol) was dissolved in DCM (12 mL), TFA (4.62 mL, 61.69 mmol) and hydrogen peroxide (1.44 mL, 61.69 mmol) were added, and the reaction mixture was allowed to react at 40°C overnight. The reaction solution was quenched with aqueous sodium thiosulfate solution, and extracted with DCM. The organic phase was washed with saturated NaCl solution, dried over anhydrous Na₂SO₄, and concentrated under reduced pressure. The crude product was purified by silica gel column chromatography (PE:EA=1/10-1/4) to obtain compound 444-4.

### Step 4: synthesis of compound 444-5

Compound 444-4 (900 mg, 3.20 mmol), NH₂Boc (750.03 mg, 6.40 mmol), cesium carbonate (2.09 g, 6.40 mmol) and XPhos Pd G4 (137.72 mg, 0.16 mmol) were dissolved in 1,4-dioxane (10 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/5) to obtain compound 444-5.

### Step 5: synthesis of compound 444-6

To compound 444-5 (546 mg, 1.72 mmol), HCl\EA (30 mL) was added, and the reaction mixture was allowed to react at room temperature for 2 h. The reaction solution was concentrated, neutralized, extracted and dried, and concentrated to obtain compound 444-6, which was used directly in the next reaction.

### Step 6: synthesis of compound 444-7

Compound 444-6 (373.77 mg, 1.72 mmol), 4-bromo-2-(6-azaspiro[2.5]octan-6-yl)benzoic acid (640.23 mg, 2.06 mmol), and HATU (783.29 mg, 2.06 mmol) were dissolved in acetonitrile (30 mL), to which DIEA (1.14 mL, 6.88 mmol) was added, and the reaction mixture was allowed to react at room temperature for 2 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 444-7.

### Step 7: synthesis of compound 444

Compound 444-7 (350 mg, 0.69 mmol), 2-hydroxylethanesulfonamide (343.89 mg, 2.75 mmol), cesium carbonate (446.37 mg, 1.37 mmol) and BrettPhos Pd G4 (27.62 mg, 0.03 mmol) were dissolved in 1,4-dioxane (10 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative high performance liquid chromatography (NH₄HCO₃ system) to obtain compound 444. MS m/z (ESI):554.2 [M+1]⁺.

**¹H NMR** (DMSO-d6) δ = 12.10 (s, 1H), 10.15 (br s, 1H), 8.48 - 8.41 (m, 1H), 8.02 (d, J = 8.5 Hz, 1H), 7.27 - 7.19 (m, 3H), 7.08 (dd, *J* = 2.1, 8.6 Hz, 1H), 5.04 (q, *J =* 6.6 Hz, 1H), 4.93 (br s, 1H), 4.37 - 4.18 (m, 2H), 3.85 - 3.73 (m, 3H), 3.38 - 3.33 (m, 2H), 3.08 - 2.92 (m, 4H), 1.94 - 1.48 (m, 7H), 1.38 (d, *J =* 6.1 Hz, 3H), 0.34 - 0.25 (m, 4H).

### Example 445: synthesis of 2-(6-azaspiro[2.5]oct-6-yl)-N-(6-fluoro-1,3-dimethyl-3,4-dihydro-1H-benzo[4,5]imidazo[2,1-c][1,4]oxazin-9-yl)-4-{[(2-hydroxyethyl)sulfonamide]}benzamide (445)

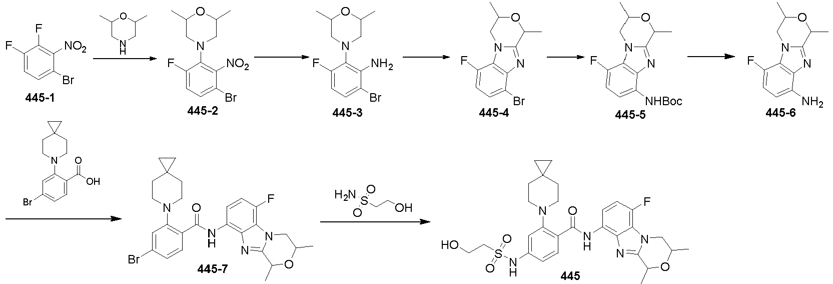

### Step 1: synthesis of compound 445-2

Compound 445-1 (3.9 g, 16.39 mmol) was dissolved in NMP (20 mL), to which 2,6-dimethylmorpholine (2.98 g, 19.67 mmol) and DIEA (5.43 mL, 32.77 mmol) were added, and the reaction mixture was allowed to react at 110°C overnight. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 445-2.

### Step 2: synthesis of compound 445-3

Compound 445-2 (2.00 g, 6.00 mmol) was dissolved in ethanol (20 mL) and water (20 mL), to which iron dust (2.68 g, 48.02 mmol) and ammonium chloride (2.57 g, 48.02 mmol) were added. The reaction mixture was allowed to react at 80°C for 3 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/5) to obtain compound 445-3.

### Step 3: synthesis of compound 445-4

Compound 445-3 (1.60 g, 5.28 mmol) was dissolved in DCM (12 mL), TFA (3.97 mL, 52.77 mmol) and hydrogen peroxide (1.23 mL, 52.77 mmol) were added, and the reaction mixture was allowed to react at 40°C overnight. The reaction solution was quenched with aqueous sodium thiosulfate solution, and extracted with DCM. The organic phase was washed with saturated NaCl solution, dried over anhydrous Na₂SO₄, and concentrated under reduced pressure. The crude product was purified by silica gel column chromatography (PE:EA=1/10-1/4) to obtain compound 445-4.

### Step 4: synthesis of compound 445-5

Compound 445-4 (500 mg, 1.67 mmol), NH₂Boc (391.62 mg, 3.34 mmol), cesium carbonate (1.09 g, 3.34 mmol) and XPhos Pd G4 (71.79 mg, 0.08 mmol) were dissolved in 1,4-dioxane (10 mL), and the reaction mixture was reacted at 95°C for 4 h under N₂ protection. The reaction solution was quenched with water, and extracted with EA. The organic phase was dried over anhydrous Na₂SO₄, and concentrated under reduced pressure. The crude product was purified by silica gel column chromatography (PE:EA=1/20-1/5) to obtain compound 445-5.

### Step 5: synthesis of compound 445-6

To compound 444-5 (200 mg, 0.60 mmol), HCl\EA (10 mL) was added, and the reaction mixture was allowed to react at room temperature for 2 h. The reaction solution was concentrated under reduced pressure, neutralized, extracted and dried, and concentrated to obtain compound 445-6, which was used directly in the next reaction.

### Step 6: synthesis of compound 445-7

Compound 445-6 (140.29 mg, 0.60 mmol), 4-bromo-2-(6-azaspiro[2.5]octan-6-yl)benzoic acid (223.34 mg, 0.72 mmol), and HATU (273.77 mg, 0.72 mmol) were dissolved in acetonitrile (10 mL), to which DIEA (0.40 mL, 2.40 mmol) was added, and the reaction mixture was allowed to react at room temperature for 2 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 445-7.

### Step 7: synthesis of compound 445

Compound 445-7 (90 mg, 0.17 mmol), 2-hydroxylethanesulfonamide (85.41 mg, 0.68 mmol), cesium carbonate (111.19 mg, 0.34 mmol) and XPhos Pd G4 (14.68 mg, 0.02 mmol) were dissolved in 1,4-dioxane (6 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative high performance liquid chromatography (TFA system) to obtain compound 445. MS m/z (ESI):572.3 [M+1]⁺.

**1H NMR** (DMSO-d6) δ = 12.09 (s, 1H), 10.16 (s, 1H), 8.38 (dd, *J* = 4.3, 8.8 Hz, 1H), 8.02 (d, *J* = 8.5 Hz, 1H), 7.29 - 7.23 (m, 1H), 7.12 - 7.01 (m, 2H), 5.05 (q, *J* = 6.5 Hz, 1H), 4.50 (br d, *J* = 11.6 Hz, 1H), 4.29 - 4.18 (m, 1H), 4.11 - 4.03 (m, 1H), 3.87 - 3.71 (m, 3H), 3.40 - 3.28 (m, 3H), 3.00 (br s, 4H), 1.82 - 1.61 (m, 6H), 1.36 (d, *J* = 6.0 Hz, 3H), 0.36 - 0.21 (m, 4H).

### Example 449: synthesis of compound 4-(2-hydroxyethyl)sulfonamido)-N-(6-methoxy-1,3-dimethyl-3,4-dihydro-1H-[1,4]oxazino[4,3-a]indol-9-yl)-2-(6-azaspiro[2.5]octan-6-yl)benzamide

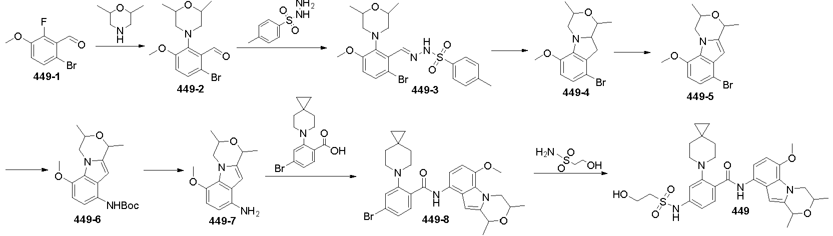

### Step 1: synthesis of compound 449-2

Compound 449-1 (3.5 g, 10.52 mmol) and 2,6-dimethylmorpholine hydrochloride (2.73 g, 18.02 mmol) were dissolved in N-methylpyrrolidinone (15 mL), to which N,N-diisopropylethylamine (4.85 g, 37.55 mmol) was added, and the reaction mixture was allowed to react at 95°C for 18 hours. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=100:0-98:2) to obtain compound 449-2. MS m/z (ESI):328.0/330.0 [M+1]⁺.

### Step 2: synthesis of compound 449-3

Compound 449-2 (2.23 g, 6.79 mmol) was dissolved in ultra-dry toluene (15 mL), to which N-amino-4-methylbenzenesulfonamide (1.52 g, 8.15 mmol) was added, and the mixture was reacted at room temperature for 30 minutes to obtain the reaction solution containing compound 449-3, which was used directly in the next reaction.

### Step 3: synthesis of compound 449-4

To the reaction solution (reaction solution 449-3) from the previous step was added ultra-dry toluene (15 mL). Sodium hydride (0.31 g, 12.97 mmol) was added to the mixture under ice bath conditions and under N₂ protection. The resulting mixture was stirred at 25°C for 0.5 h, followed by reaction at 135°C for 10 minutes. The reaction solution was diluted with ice water, and extracted with EA. The organic phase was washed with saturated NaCl solution, dried over anhydrous Na₂SO₄, concentrated under reduced pressure and the crude product was purified by silica gel column chromatography (PE:EA=100:0-90:10) to obtain compound 449-4. MS m/z (ESI):312.1/314.1 [M+1]⁺.

### Step 4: synthesis of compound 449-5

Compound 449-4 (200 mg, 0.64 mmol) and manganese dioxide (556.93 mg, 6.41 mmol) were dissolved in dioxane (3 mL) and the mixture was reacted overnight at room temperature. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=100:0-95:5) to obtain compound 449-5. MS m/z (ESI):310.0/312.0 [M+1]⁺.

### Step 5: synthesis of compound 449-6

Compound 449-5 (140 mg, 0.45 mmol), 2-methylprop-2-ylcarbamate (105.75 mg, 0.90 mmol), cesium carbonate (441.16 mg, 1.35 mmol) and palladium catalyst (CAS: 1599466-89-3; 41.52 mg, 0.05 mmol) were dissolved in 1,4-dioxane (3 mL), and the reaction mixture was reacted at 95°C for 1.5 h under N₂ protection. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=100:0-80:20) to obtain compound 449-6.

### Step 6: synthesis of compound 449-7

Compound 449-6 (270 mg, 0.78 mmol) was dissolved in dichloromethane solution (4 mL), trifluoroacetic acid (0.17 mL) was then added, and the mixture was reacted overnight at room temperature. The reaction solution was quenched with ice-cold sodium bicarbonate solution, and extracted with EA. The organic phase was washed with saturated NaCl solution, dried over anhydrous Na₂SO₄, and concentrated under reduced pressure to obtain compound 449-7. MS m/z (ESI):247.0 [M+1]⁺.

### Step 7: synthesis of compound 449-8

Compound 449-7 (125 mg, 0.51 mmol), 2-(6-azaspiro[2.5]octan-6-yl)-4-bromobenzoic acid (173.16 mg, 0.56 mmol), 2-(7-azabenzotriazole)-N,N,N',N'-tetramethyluronium hexafluorophosphate (231.42 mg, 0.61 mmol) and N,N-diisopropylethylamine (327.33 mg, 2.54 mmol) were dissolved in N,N-dimethylformamide (2 mL), and the mixture was allowed to react at room temperature for 1 h. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=4:1) to obtain compound 449-8. MS m/z (ESI):538.2/540.2 [M+1]⁺.

### Step 8: synthesis of compound 449

Compound 449-8 (180 mg, 0.33 mmol), 2-hydroxyethanesulfonamide (167.32 mg, 1.34 mmol), cesium carbonate (217.82 mg, 0.67 mmol) and palladium catalyst (CAS: 1599466-89-3; 61.51 mg, 0.07 mmol) were dissolved in 1,4-dioxane (3 mL), and the reaction mixture was reacted at 110°C for 1.5 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative chromatography to obtain compound 449. MS m/z (ESI):583.2 [M+1]⁺.

**¹H NMR** (400 MHz, DMSO-*d*₆) δ = 11.06 (br s, 1H), 10.10 (br s, 1H), 7.89 - 7.82 (m, 2H), 7.20 (s, 1H), 7.10 - 7.00 (m, 1H), 6.70 - 6.60 (m, 1H), 6.46 (s, 1H), 5.02 - 4.91 (m, 1H), 4.73 - 4.63 (m, 1H), 4.32 - 4.02 (m, 4H), 3.35 (s, 3H), 3.02 - 2.75 (m, 6H), 1.58 - 1.47 (m, 6H), 1.39 - 1.25 (m, 4H), 0.28 (s, 4H).

### Example 453: synthesis of N-(3,3-dimethyl-3,4-dihydro-1H-[1,4]oxazino[4,3-a]indol-9-yl)-6-((2-hydroxyethyl)sulfonamido)-2-(6-azaspiro[2.5]octan-6-yl)nicotinamide (453)

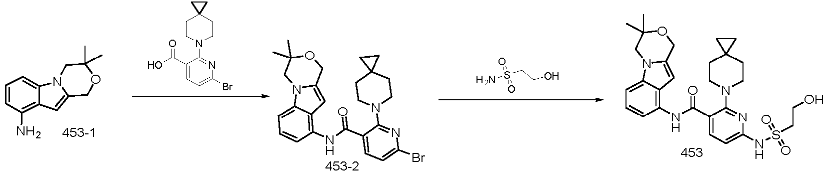

### Step 1: synthesis of compound 453-2

Compound 453-1 (70 mg, 0.32 mmol), 6-bromo-2-(6-azaspiro[2.5]octan-6-yl)nicotinic acid (110 mg, 0.36 mmol), HATU (135 mg, 0.36 mmol) and *N,N-*diisopropylethylamine (167 mg, 1.29 mmol) were dissolved in DMF (4 ml), and the reaction mixture was reacted at room temperature for 3 hours under nitrogen protection at room temperature. The reaction solution was quenched by adding water (10 ml), and liquid separation was performed with EA (20 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 15/1) to obtain compound 453-2.

### Step 2: synthesis of compound 453

Compound 453-2 (60 mg, 0.12 mmol), 2-hydroxyethylsulfonamide (73.7 mg, 0.6 mmol), cesium carbonate (115 mg, 0.35 mmol) and palladium catalyst (CAS: 1599466-89-3; 10 mg, 0.01 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 1/1) to obtain compound 453. MS (ESI, pos.ion) m/z:554.2 [M+1]⁺.

¹H NMR (400 MHz, DMSO-*d*₆) δ: 10.23 (s, 1H), 7.85 (d, *J* = 8.0 Hz, 1H), 7.76 (br d, *J* = 7.4 Hz, 1H), 7.17 (d, *J* = 8.1 Hz, 1H), 7.07 (t, *J* = 7.9 Hz, 1H), 6.48 (s, 1H), 6.44 (d, *J* = 8.1 Hz, 1H), 4.96 (s, 3H), 3.93 (s, 2H), 3.69-3.85 (m, 4H), 3.33-3.35 (m, 4H), 1.39 (br s, 4H), 1.34 (s, 6H), 1.23 (s, 1H), 0.26 (s, 4H).

### Example 454: synthesis of N-(3,3-dimethyl-3,4-dihydro-1H-[1,4]oxazino[4,3-a]indol-9-yl)-6-(2-hydroxyethylsulfonamide)-4-(6-azaspiro[2.5]octan-6-yl)nicotinamide (454)

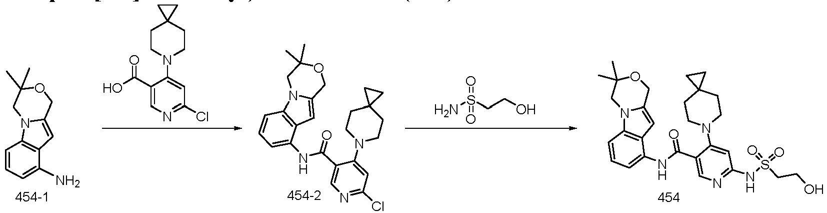

### Step 1: synthesis of compound 454-2

Compound 454-1 (100 mg, 0.46 mmol), HATU (263 mg, 0.69 mmol), 6-chloro-4-(6-azaspiro[2.5]octan-6-yl)nicotinic acid (123 mg, 0.46 mmol) were dissolved in DMF (5 mL), to which DIEA (3 mL, 1.39 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 454-2. MS m/z (ESI):465.2 [M+1]⁺.

### Step 2: synthesis of compound 454

Compound 454-2 (137 mg, 0.29 mmol), 2-hydroxyethanesulfonamide (74 mg, 0.59 mmol), cesium carbonate (290 mg, 0.88 mmol) and palladium catalyst (CAS: 1599466-89-3; 24 mg, 0.029 mmol) were dissolved in 1,4-dioxane (5 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=1/20-1/10) to obtain compound 454. MS m/z (ESI):554.3 [M+1]⁺.

**¹H NMR (400 MHz, DMSO-d₆)** δ = 10.08 (s, 1H), 8.02 (s, 1H) 7.71 (br d, *J =* 7.5 Hz, 1H), 7.18 (d, *J* = 8.1 Hz, 1H), 7.07 (t, *J* = 7.9 Hz, 1H), 6.59 (s, 1H), 6.50 (s, 1H), 4.96 (s, 2H), 3.93 (s, 2H), 3.77 (t, *J* = 6.9 Hz, 2H), 3.52 (m, 2H)3.24 - 3.14 (m, 4H), 1.39 (br s, 4H), 1.34 (s, 6H), 0.28 (s, 4H).

### Example 455: synthesis of 4-((2-hydroxyethyl)sulfonamido)-N-(6-methoxy-3,3-dimethyl-3,4-dihydro-1H-[1,4]oxazinyl[4,3-a]indol-9-yl)-2-(6-azaspiro[2.5]oct-6-yl)benzamide (455)

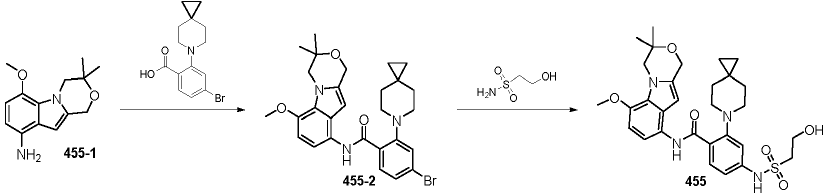

### Step 1: synthesis of compound 455-2

Compound 455-1 (53 mg, 0.19 mmol), 4-bromo-2-(6-azaspiro[2.5]oct-6-yl)benzoic acid (64 mg, 0.21 mmol), and HATU (79 mg, 0.21 mmol) were dissolved in DMF (3 mL), to which DIEA (0.12 mL, 0.76 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 455-2.

### Step 2: synthesis of compound 455

Compound 455-2 (95 mg, 0.18 mmol), 2-hydroxyethanesulfonamide (88 mg, 0.72 mmol), cesium carbonate (172 mg, 0.54 mmol) and the fourth-generation palladium catalyst (CAS: 1599466-89-3; 14 mg, 0.02 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 455. MS (ESI, pos.ion) m/z:583.2 [M+1]⁺.

¹H NMR (400 MHz, DMSO-d₆) δ = 11.07 (s, 1H), 7.86 (d, *J =* 8.5 Hz, 1H), 7.64 (d, *J* = 8.4 Hz, 1H), 7.18 (d, *J* = 1.9 Hz, 1H), 7.03 (dd, *J* = 1.9, 8.5 Hz, 1H), 6.62 (d, *J* = 8.5 Hz, 1H), 6.37 (s, 1H), 4.92 (s, 2H), 4.26 (s, 2H), 3.88 (s, 3H), 3.80 - 3.73 (m, 2H), 3.37 - 3.34 (m, 2H), 3.00 (br t, *J* = 4.7 Hz, 4H), 1.45 (br s, 4H), 1.32 (s, 6H), 0.28 (s, 4H).

### Example 465: synthesis of 2-(4-(difluoromethylene)piperidin-1-yl)-4-((2-hydroxyethyl)sulfonamido)-N-(6-methoxy-3,3-dimethyl-3,4-dihydro-1H-[1,4]oxazinyl[4,3-a]indol-9-yl)benzamide (465)

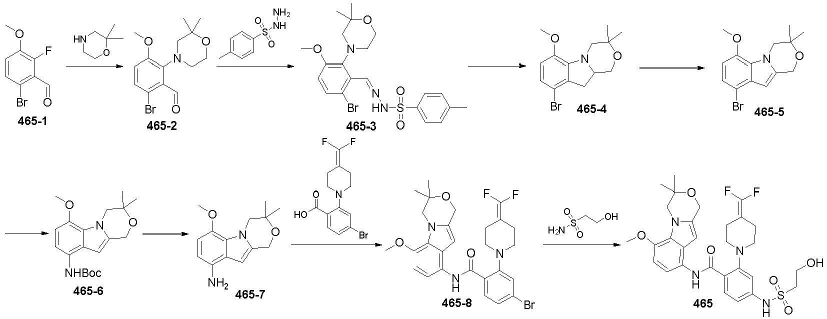

### Step 1: synthesis of compound 465-2

Compound 465-1 (9.7 g, 41.62 mmol), and granular potassium carbonate (7.48 g, 54.11 mmol) were dissolved in DMSO (60 mL), to which 2,2-dimethylmorpholine (5.75 g, 49.95 mmol) was added, and the reaction mixture was allowed to react at 60°C for 3 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 465-2.

### Step 2: synthesis of compound 465-3

Compound 465-2 (950 mg, 2.89 mmol) was dissolved in toluene (10 mL), to which p-toluenesulfonyl hydrazide (647 mg, 3.47 mmol) was added, and the mixture was reacted at room temperature for 20 minutes. The reaction solution was quenched with ice water, and extracted with EA. The organic phase was washed with saturated NaCl solution, dried over anhydrous Na₂SO₄, and concentrated under reduced pressure to obtain compound 465-3.

### Step 3: synthesis of compound 465-4

Compound 465-3 (1.43 g, 2.88 mmol) was dissolved in toluene (20 mL), to which NaH (130 mg, 3.17 mmol) was slowly added at 0°C, and after stirring for 30 minutes, the mixture was reacted at 135°C for 30 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 465-4.

### Step 4: synthesis of compound 465-5

Compound 465-4 (366 mg, 1.17 mmol) was dissolved in DCM (5 mL), to which MnO₂ (1.02 g, 11.70 mmol) was added, and the reaction mixture was allowed to react at 25°C for 2 hours. The reaction solution was filtered, and concentrated under reduced pressure. The crude product was purified by silica gel column chromatography (PE:EA=20:1) to obtain compound 465-5.

¹H NMR (400 MHz, CHLOROFORM-d) δ = 7.04 (d, *J* = 8.3 Hz, 1H), 6.37 (d, *J* = 8.4 Hz, 1H), 6.14 (s, 1H), 4.93 (s, 2H), 4.20 (s, 2H), 3.82 (s, 3H), 1.33 - 1.30 (m, 6H).

### Step 5: synthesis of compound 465-6

Compound 465-5 (180 mg, 0.58 mmol), NH₂Boc (136 mg, 1.16 mmol), cesium carbonate (567 mg, 1.74 mmol) and the fourth-generation palladium catalyst (CAS: 1599466-83-7; 107 mg, 0.12 mmol) were dissolved in 1,4-dioxane (3 mL), and the reaction mixture was reacted at 90°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 465-6. MS (ESI, pos.ion) m/z:347.1 [M+1]⁺.

### Step 6: synthesis of compound 465-7

Compound 465-6 (146 mg, 0.42 mmol) was dissolved in DCM (2 mL), to which HCl\dioxane (2 mL) was added, and the reaction mixture was allowed to react at 25°C for 1 hour. The reaction solution was filtered, neutralized, extracted and dried, and concentrated to obtain compound 465-7. MS (ESI, pos.ion) m/z:247.0 [M+1]⁺.

### Step 7: synthesis of compound 465-8

Compound 465-7 (52 mg, 0.18 mmol), 4-bromo-2-(4-(difluoromethylene)piperidin-1-yl)benzoic acid (67 mg, 0.20 mmol), and HATU (77 mg, 0.20 mmol) were dissolved in DMF (2 mL), to which DIEA (0.12 mL, 0.74 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 465-8.

### Step 8: synthesis of compound 465

Compound 465-8 (85 mg, 0.15 mmol), 2-hydroxyethanesulfonamide (76 mg, 0.61 mmol), cesium carbonate (148 mg, 0.45 mmol) and the fourth-generation palladium catalyst (CAS: 1599466-89-3; 12 mg, 0.02 mmol) were dissolved in 1,4-dioxane (3 mL), and the reaction mixture was reacted at 100°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified by preparative chromatography to obtain compound 465. MS (ESI, pos.ion) m/z:605.2 [M+1]⁺.

¹H NMR (400 MHz, DMSO-d₆) δ = 10.62 (s, 1H), 7.77 (d, *J* = 8.4 Hz, 1H), 7.66 (d, *J* = 8.4 Hz, 1H), 7.06 (s, 1H), 7.04 - 6.98 (m, 1H), 6.62 (d, *J* = 8.5 Hz, 1H), 6.38 (s, 1H), 4.91 (s, 2H), 4.24 (s, 2H), 3.87 (s, 3H), 3.77 (t, *J* = 6.6 Hz, 2H), 3.32 - 3.30 (m, 2H), 3.06 - 2.95 (m, 4H), 2.28 - 2.20 (m, 4H), 1.32 (s, 6H).

### Example 467: synthesis of 2-(4-(difluoromethylene)piperidin-1-yl)-4-((2-hydroxyethyl)sulfonamide)-N-(3,3,7-trimethyl-3,4-dihydro-1H-[1,4]oxazino[4,3-a]indol-9-yl)benzamide (467)

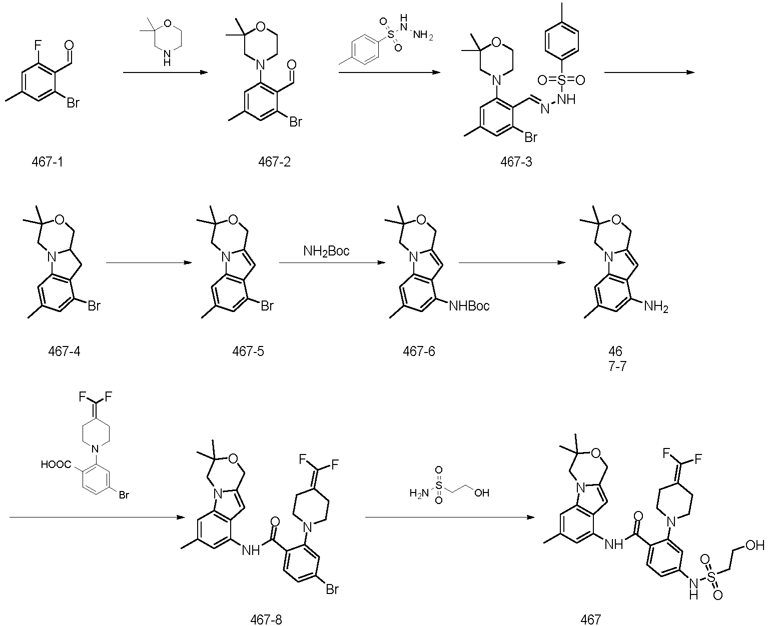

### Step 1: synthesis of compound 467-2

Compound 467-1 (2 g, 9.21 mmol), and K₂CO₃ (3.82 g, 27.63 mmol) were dissolved in DMSO, to which 2,2-dimethylmorpholine (1.77 mL, 13.82 mmol) was added, and the reaction mixture was allowed to react overnight at 90°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 467-2.

### Step 2: synthesis of compound 467-3

Compound 467-2 (1 g, 3.20 mmol) was dissolved in toluene (10 mL), to which p-toluenesulfonyl hydrazide (715 mg, 3.84 mmol) was added. The mixture was stirred at 25°C for 10 minutes to obtain the reaction solution containing compound 467-3, which was used without further post-treatments.

### Step 3: synthesis of compound 467-4

To the reaction solution containing compound 467-3 (1.53 g, 3.20 mmol) was slowly added NaH (153 mg, 3.84 mmol), stirred at room temperature for 10 minutes, and then the reaction temperature was raised to 130°C and the mixture was reacted for half an hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 467-4.

### Step 4: synthesis of compound 467-5

Compound 467-4 (800 g, 2.7 mmol) was dissolved in dichloromethane (15 mL), to which manganese dioxide (2.35 g, 27 mmol) was added, and the mixture was reacted overnight at 40°C. The reaction solution was filtered, and the filtrate was collected, and concentrated under reduced pressure. The crude product was purified by silica gel column chromatography (PE:EA=20/1-10/1) to obtain compound 467-5.

### Step 5: synthesis of compound 467-6

Compound 467-5 (450 mg, 1.53 mmol) was dissolved in 1,4-dioxane (10 mL), to which tert-butyl carbamate (537 mg, 4.6 mmol), cesium carbonate (1500 mg, 4.6 mmol) and palladium catalyst (CAS: 1599466-89-3; 137 mg, 0.15 mmol) were added, and the reaction mixture was reacted at 90°C overnight under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 467-6. MS m/z (ESI):331.2 [M+1]⁺.

### Step 6: synthesis of compound 467-7

Compound 467-6 (396 mg, 1.2 mmol) was dissolved in dichloromethane (5 mL), to which HCl/EA (6 mL) was added, and the mixture was stirred at 25°C for 1 hour. The solvent was removed by concentration under reduced pressure, and the residue was neutralized. The reaction system was subjected to extraction, drying and concentration to obtain product 467-7, which was used directly in the next reaction. MS m/z (ESI):231.1 [M+1]⁺.

### Step 7: synthesis of compound 467-8

Compound 467-7 (276 mg, 1.2 mmol), HATU (684 mg, 1.8 mmol), 4-bromo-2-(4-(difluoromethylene)piperidin-1-yl)benzoic acid (398 mg, 1.2 mmol) were dissolved in DMF (5 mL), to which DIEA (6 mL, 3.6 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 467-8.

### Step 8: synthesis of compound 467

Compound 467-8 (209 mg, 0.38 mmol), 2-hydroxyethanesulfonamide (95 mg, 0.76 mmol), cesium carbonate (374 mg, 1.14 mmol) and palladium catalyst (CAS: 1599466-89-3; 32 mg, 0.04 mmol) were dissolved in 1,4-dioxane (5 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=1/20-1/10) to obtain compound 467. MS m/z (ESI):589.2 [M+1]⁺.

**¹H NMR (400 MHz, CHLOROFORM-d)** δ = 11.05 (s, 1H), 8.16 (d, *J =* 8.4 Hz, 1H), 7.85 (s, 1H), 7.25 - 7.21 (m, 1H), 7.01 (br d, *J* = 8.0 Hz, 1H), 6.94 (s, 1H), 6.86 (br s, 1H), 6.18 (s, 1H), 5.01 (s, 2H), 4.15 (br t, *J=* 5.1 Hz, 2H), 3.86 (s, 2H), 3.33 (t, *J =* 5.1 Hz, 2H), 3.12 (br t, *J =* 4.9 Hz, 4H), 2.54 - 2.51 (m, 3H), 2.38 (br s, 4H), 1.45 (s, 6H).

### Example 477: synthesis of N-(8,8-difluoro-4-methoxy-6,7,8,9-tetrahydropyridino[1,2-a]indol-1-yl)-2-(4-(difluoromethylene)piperidin-1-yl)-4-((2-hydroxyethyl)sulfonamido)benzamide (477)

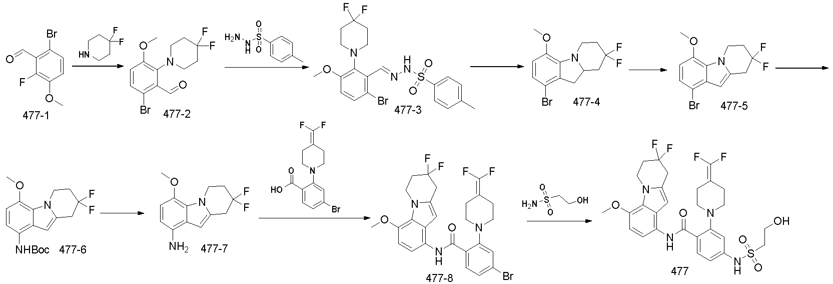

### Step 1: synthesis of compound 477-2

2-Bromo-5-methoxy-6-fluorobenzaldehyde (2000 mg, 8.5 mmol), 4,4-difluoropiperidine (1.25 g, 10.3 mmol) and *N,N* diisopropylethylamine (2.21 g, 17.16 mmol) were dissolved in NMP (10 mL) and reacted at 100°C for 12 hours under nitrogen protection. The reaction solution was quenched by adding water (20 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 477-2.

### Step 2: synthesis of compound 477-3

Compound 477-2 (1100 mg, 3.29 mmol) and *N*-amino-4-toluenesulfonamide (613 mg, 3.29 mmol) were dissolved in toluene (10 mL) and reacted at room temperature for 0.5 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was not purified and used directly in the next reaction to obtain compound 477-3.

### Step 3: synthesis of compound 477-4

Compound 477-3 (1653 mg, 3.29 mmol) and sodium hydride (158 mg, 3.95 mmol) were dissolved in toluene (40 mL) and reacted at room temperature for 0.5 hours, and then reacted at 135°C for 25 min. After the reaction was completed as monitored by TLC, the reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by preparative chromatography to obtain compound 477-4.

### Step 4: synthesis of compound 477-5

The compound 477-4 (480 mg, 1.51 mmol) and manganese dioxide (1311 mg, 15.1 mmol) were dissolved in DCM (15 mL) and reacted at room temperature for 12 hours. After the reaction was completed as monitored by TLC, the reaction solution was filtered and the filtrate was quenched by adding water (6 ml), and liquid separation was performed with DCM (30 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 477-5.

### Step 5: synthesis of compound 477-6

Compound 477-5 (330 mg, 1.04 mmol), tert-butyl carbamate (244 mg, 2.08 mmol), cesium carbonate (1000 mg, 3.13 mmol) and palladium catalyst (CAS: 1599466-85-9; 88 mg, 0.1 mmol) were dissolved in 1,4-dioxane (8 mL), and the reaction mixture was reacted at 90°C for 12 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 10/1) to obtain compound 477-6.

### Step 6: synthesis of compound 477-7

Compound 477-6 (160 mg, 0.32 mmol) was dissolved in hydrogen chloride-dioxane solution (1 ml) and the mixture was reacted at room temperature for 3 hours. The reaction solution was concentrated under reduced pressure, neutralized, extracted and dried, and concentrated to obtain compound 477-7, which was used directly in the next step.

### Step 7: synthesis of compound 477-8

Compound 477-7 (60 mg, 0.24 mmol), 4-bromo-2-(4-(difluoromethylene)piperidin-1-yl)benzoic acid (87 mg, 0.26 mmol), HATU (99 mg, 0.26 mmol) and *N,N-*diisopropylethylamine (0.17 ml, 0.95 mmol) were dissolved in DMF (4 ml), and the reaction mixture was reacted at room temperature for 2 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 10/1) to obtain compound 477-8.

### Step 8: synthesis of compound 477

Compound 477-8 (100 mg, 0.18 mmol), 2-hydroxyethylsulfonamide (66 mg, 0.53 mmol), cesium carbonate (173 mg, 0.53 mmol) and palladium catalyst (CAS: 1599466-89-3; 14 mg, 0.02 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 1/1) to obtain compound 477. MS (ESI, pos.ion) m/z:611.2 [M+1]⁺.

¹H NMR (400 MHz, DMSO-*d*₆) δ: 10.62 (s, 1H), 7.77 (d, *J*=8.5 Hz, 1H), 7.68 (d, *J*=8.5 Hz, 1H), 7.06 (s, 1H), 7.01 (dd, *J*=8.4, 1.7 Hz, 1H), 6.65 (d, *J*=8.5 Hz, 1H), 6.45 (s, 1H), 4.56 (t, *J*=6.3 Hz, 2H), 3.88 (s, 3H), 3.76 (t, *J*=6.6 Hz, 2H), 3.52 (br t, *J*=14.6 Hz, 2H), 3.33-3.33 (m, 1H), 3.34 (br s, 1H), 3.01 (br t, *J*=5.2 Hz, 4H), 2.53-2.63 (m, 2H), 2.24 (br s, 4H).

### Example 478: synthesis of N-(8,8-difluoro-4-methoxy-6,7,8,9-tetrahydropyridino[1,2-a]indol-1-yl)-4-(2-hydroxyethylsulfonamido)-2-(6-azaspiro[2.5]octan-6-yl)benzamide (478)

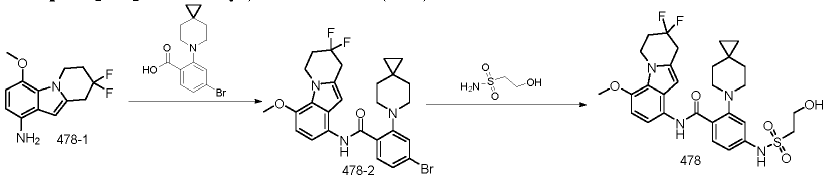

### Step 1: synthesis of compound 478-2

Compound 478-1 (55 mg, 0.22 mmol), 4-bromo-2-(6-azaspiro[2.5]octan-6-yl)benzoic acid (75 mg, 0.24 mmol), HATU (91 mg, 0.24 mmol) and *N,N-*diisopropylethylamine (112 mmol, 0.87 mmol) were dissolved in DMF (4 ml), and the reaction mixture was reacted at room temperature for 2 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 10/1) to obtain compound 478-2.

### Step 2: synthesis of compound 478-3

Compound 478-2 (80 mg, 0.15 mmol), 2-hydroxyethylsulfonamide (55 mg, 0.44 mmol), cesium carbonate (143 mg, 0.44 mmol) and palladium catalyst (CAS: 1599466-89-3; 12 mg, 0.01 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na2SO4, and concentrated under reduced pressure. The crude product was purified by silica gel column chromatography (PE/EA= 1/0 - 1/1) to obtain compound 478. MS (ESI, pos.ion) m/z:599.3 [M+1]⁺.

¹H NMR (400 MHz, DMSO-*d*₆) δ: 11.08 (s, 1H), 7.86 (d, *J* = 8.5 Hz, 1H), 7.64 (d, *J* = 8.4 Hz, 1H), 7.18 (s, 1H), 7.03 (d, *J* = 8.5 Hz, 1H), 6.66 (d, *J* = 8.5 Hz, 1H), 6.43 (s, 1H), 4.96 (br s, 1H), 4.57 (t, *J* = 6.4 Hz, 2H), 3.88 (s, 3H), 3.77 (t, *J* = 6.5 Hz, 2H), 3.45-3.57 (m, 2H), 3.34 (s, 2H), 3.01 (br t, *J* = 4.7 Hz, 4H), 2.54-2.65 (m, 2H), 1.46 (br s, 4H), 0.29 (s, 4H).

### Example 479: synthesis of N-(8,8-difluoro-4,9-dimethoxy-6,7,8,9-tetrahydropyridino[1,2-a]indol-1-yl)-4-((2-hydroxyethyl)sulfonamido)-2-(6-azaspiro[2.5]oct-6-yl)benzamide (479)

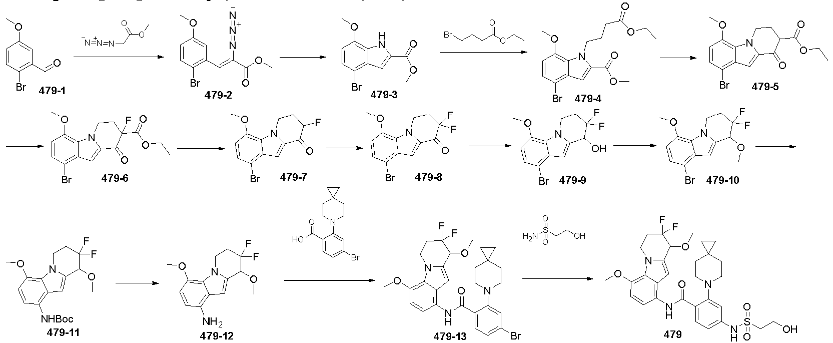

### Step 1: synthesis of compound 479-2

Compound 479-1 (25 g, 116.279 mmol) was dissolved in methanol (150 ml), A solution of methyl azidoacetate (20.1 g, 174.419 mmol) and sodium methoxide (12.56 g, 232.558 mmol) in methanol (100 ml) was added dropwise under ice bath conditions and the reaction was allowed to warm to room temperature and stirred overnight at room temperature. the reaction solution was quenched with ice water, and extracted with ethyl acetate. The organic phase was washed with saturated sodium chloride aqueous solution, dried over anhydrous sodium sulfate, and concentrated under reduced pressure. The crude product was subjected to recrystallization from methanol to obtain compound 479-2.

### Step 2: synthesis of compound 479-3

Compound 479-2 (15 g, 48.1 mmol) was dissolved in o-dichlorobenzene (80 ml) and refluxed for 3 hours. The reaction solution was cooled and then directly purified by silica gel column chromatography (PE:EA=1/10-1/8) to obtain compound 479-3.

### Step 3: synthesis of compound 479-4

Compound 479-3 (4.5 g, 15.85 mmol), cesium carbonate (10.33 g, 31.69 mmol) and ethyl 4-bromobutyrate (3.7 g, 19.01 mmol) were dissolved in DMF (50 ml), and the mixture was stirred at room temperature for 3 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/15-1/10) to obtain compound 479-4.

### Step 4: synthesis of compound 479-5

Compound 479-4 (5.2 g, 13.06 mmol) and potassium tert-butoxide (2.93 g, 26.13 mmol) were dissolved in THF (50 ml). The reaction mixture was stirred at room temperature for 2 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/15-1/10) to obtain compound 479-5.

### Step 5: synthesis of compound 479-6

Compound 479-5 (4.1 g, 11.2 mmol), potassium tert-butoxide (2.51 g, 22.4 mmol) and NFSI (7.06 g, 22.4 mmol) were dissolved in THF (50 ml). The reaction mixture was stirred at room temperature for 2 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/5) to obtain compound 479-6.

### Step 6: synthesis of compound 479-7

The compound 479-6 (2.1 g, 5.47 mmol) and concentrated hydrochloric acid (20 ml, 12N) were dissolved in ethanol (20 ml), and the mixture was stirred at 80°C at reflux for 2 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/15-1/10) to obtain compound 479-7.

### Step 7: synthesis of compound 479-8

Compound 479-7 was dissolved in THF, and cooled to -78°C. LiHMDS was added dropwise under nitrogen protection. The reaction solution was stirred at this low temperature for 1 hour, and then a solution of NFSI in THF was added dropwise. After the reaction was completed, the reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/5-1/3) to obtain compound 479-8.

### Step 8: synthesis of compound 479-9

Compound 479-8 (800 mg, 2.42 mmol) and sodium borohydride (184 mg, 4.85 mmol) were dissolved in ethanol (10 ml). The reaction mixture was stirred at room temperature for 2 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 479-9.

### Step 9: synthesis of compound 479-10

Compound 479-9 (200 mg, 0.6 mmol), cesium carbonate (393 mg, 1.2 mmol) and iodomethane (111 mg, 0.783 mmol) were dissolved in DMF (10 ml), and the mixture was stirred at room temperature for 2 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/8) to obtain compound 479-10.

### Step 10: synthesis of compound 479-11

Compound 479-10 (195 mg, 0.564 mmol), cesium carbonate (367 mg, 1.13 mmol), tBuXPhos Pd G4 (46 mg, 0.056 mmol) and tert-butyl carbamate (132 mg, 1.13 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/15-1/10) to obtain compound 479-11. MS m/z (ESI):383.2 [M+1]⁺.

### Step 11: synthesis of compound 479-12

The compound 479-11 (140 mg, 0.366 mmol) and concentrated hydrochloric acid (1.83 ml, 2N) were dissolved in dichloromethane (5 ml) and stirred at room temperature for 2 hours. A solid was precipitated out from the reaction solution, the solid was neutralized, and the reaction solution was extracted, dried, and concentrated, to directly give compound 479-12. MS m/z (ESI):283.2 [M+1]⁺.

### Step 12: synthesis of compound 479-13

Compound 479-12 (98 mg, 0.346 mmol), 4-bromo-2-(6-azaspiro[2.5]oct-6-yl)benzoic acid (129 mg, 0.416 mmol), HATU (197 mg, 0.519 mmol) and DIEA (134 mg, 1.04 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 479-13.

### Step 13: synthesis of compound 479

Compound 479-13 (120 mg, 0.209 mmol), cesium carbonate (136 mg, 0.418 mmol), tBuXPhos Pd G4 (17 mg, 0.021 mmol) and 2-hydroxyethylsulfonamide (52 mg, 0.418 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 479. MS m/z (ESI):619.2 [M+1]⁺.

¹H NMR (CHLOROFORM-d, 400 MHz) δ 11.56 (s, 1H), 8.11 (d, *J* = 8.5 Hz, 1H), 7.75 (d, *J* = 8.4 Hz, 1H), 7.42 (br s, 1H), 7.34 (d, *J =* 1.9 Hz, 1H), 6.96 (dd, *J* = 1.8, 8.4 Hz, 1H), 6.67 (d, *J =* 8.5 Hz, 1H), 6.64 (s, 1H), 4.9-5.0 (m, 1H), 4.52 (t, *J* = 6.0 Hz, 1H), 4.36 (ddd, *J =* 5.4, 11.0, 12.8 Hz, 1H), 4.0-4.1 (m, 2H), 3.93 (s, 3H), 3.48 (s, 3H), 3.2-3.3 (m, 2H), 3.11 (tt, *J* = 5.6, 10.8 Hz, 4H), 2.8-2.9 (m, 1H), 2.3-2.4 (m, 1H), 1.5-1.6 (m, 4H), 0.2-0.4 (m, 4H).

### Example 482: synthesis of N-(8,8-difluoro-9-hydroxy-4-methoxy-6,7,8,9-tetrahydropyridino[1,2-a]indol-1-yl)-4-((2-hydroxyethyl)sulfonamido)-2-(6-azaspiro[2.5]oct-6-yl)benzamide (482)

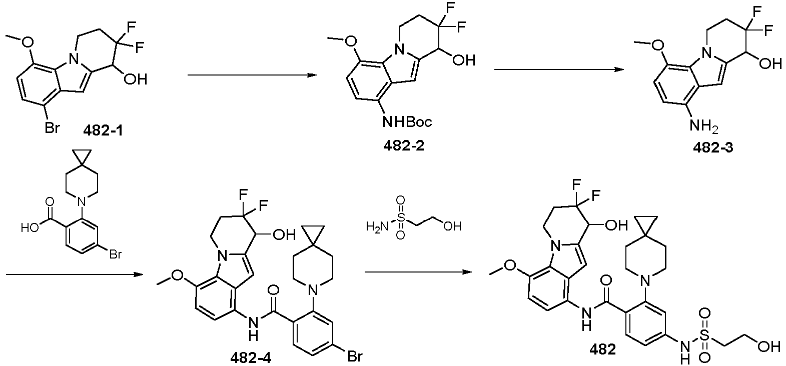

### Step 1: synthesis of compound 482-2

Compound 482-1 (200 mg, 0.604 mmol), cesium carbonate (394 mg, 1.2 mmol), tBuXPhos Pd G4 (49 mg, 0.06 mmol) and tert-butyl carbamate (141 mg, 1.2 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/8) to obtain compound 482-2.

### Step 2: synthesis of compound 482-3

The compound 482-2 (180 mg, 0.366 mmol) and concentrated hydrochloric acid (2.45 ml, 2N) were dissolved in dichloromethane (5 ml) and stirred at room temperature for 2 hours. A solid was precipitated out from the reaction solution, the solid was neutralized, and the reaction solution was extracted, dried, and concentrated, to directly give compound 482-3.

### Step 3: synthesis of compound 482-4

Compound 482-3 (120 mg, 0.448 mmol), 4-bromo-2-(6-azaspiro[2.5]oct-6-yl)benzoic acid (167 mg, 0.537 mmol), HATU (255 mg, 0.672 mmol) and DIEA (173 mg, 1.34 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/3) to obtain compound 482-4.

### Step 4: synthesis of compound 482

Compound 482-4 (150 mg, 0.268 mmol), cesium carbonate (176 mg, 0.535 mmol), tBuXPhos Pd G4 (22 mg, 0.027 mmol) and 2-hydroxyethylsulfonamide (62 mg, 0.536 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 482. MS m/z (ESI):605.2 [M+1]⁺.

¹H NMR (CHLOROFORM-d, 400 MHz) δ 11.89 (s, 1H), 7.91 (d, *J* = 8.5 Hz, 1H), 7.36 (d, *J* = 8.3 Hz, 1H), 7.25 (d, *J =* 1.6 Hz, 1H), 6.80 (dd, *J =* 1.4, 8.4 Hz, 1H), 6.59 (d, *J* = 8.4 Hz, 1H), 6.54 (s, 1H), 4.98 (br dd, *J* = 6.8, 12.3 Hz, 1H), 4.64 (br t, *J=* 6.3 Hz, 2H), 3.91 (s, 3H), 3.8-3.9 (m, 2H), 3.19 (br t, *J* = 4.9 Hz, 2H), 2.9-3.0 (m, 4H), 2.74 (br s, 1H), 2.3-2.5 (m, 1H), 1.46 (br s,4H), 0.27 (s, 4H).

### Example 485: synthesis of 2-(4-(difluoromethylene)piperidin-1-yl)-4-((2-hydroxyethyl)sulfonamido)-N-(1'H,4'H-spiro[cyclopropane-1,3'-[1,4]oxazino[4,3-a]indole]-9'-yl)benzamide (485)

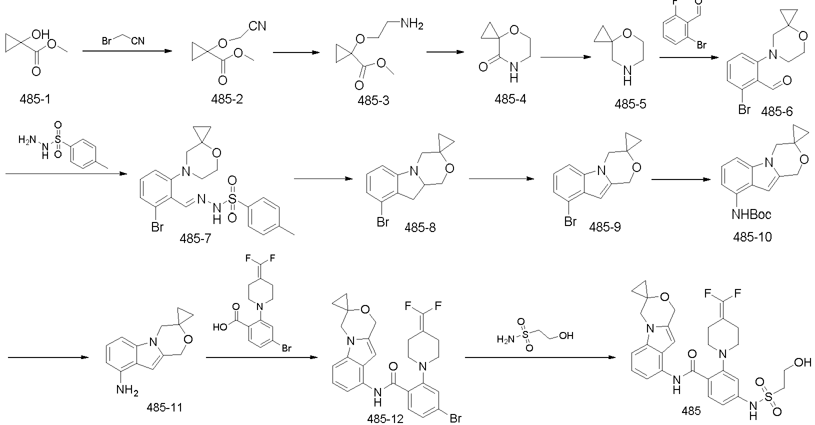

### Step 1: synthesis of compound 485-2

Compound 485-1 (20 g, 172.4 mmol) was dissolved in tetrahydrofuran (30 mL), to which sodium hydride (8.96 g, 224.12 mmol) was slowly added at 0°C. After reaction at 0°C for 2 hours, bromoacetonitrile (32.82 g, 275 mmol) was slowly added dropwise, and the reaction was continued at 0°C for 3 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=5:1) to obtain compound 485-2 as a white solid.

### Step 2: synthesis of compound 485-3

Compound 485-2 (9.7 g, 62.58 mmol) and Pd/C (1332 mg, 12.51 mmol) were dissolved in MeOH (50 mL), concentrated hydrochloric acid (8 ml) was added and the reaction solution was stirred under hydrogen at room temperature for 24 hours. The reaction solution was filtered through diatomaceous earth, concentrated under reduced pressure, and the organic phase was slurried with EA solution and filtered to obtain compound 485-3 as a white solid.

### Step 3: synthesis of compound 485-4

The compound 485-3 (6 g, 37.73 mmol) and triethylamine (7.63 g, 75.47 mmol) were dissolved in MeOH (50 mL) and reacted at room temperature for 18 hours. The reaction solution was concentrated under reduced pressure. The crude product was purified by silica gel column chromatography (EA) to obtain compound 485-4.

### Step 4: synthesis of compound 485-5

Compound 485-4 (3.33 g, 12.33 mmol) was dissolved in TFH (40 mL), to which lithium aluminium hydride (1.55 g, 3.93 mmol) was slowly added at 0°C, and then the mixture was reacted overnight at room temperature. The reaction solution was quenched with ice water and 1M NaOH aqueous solution, and extracted with EA. The organic phase was washed with saturated NaCl solution, dried over anhydrous Na₂SO₄, and concentrated under reduced pressure to obtain compound 485-5.

### Step 5: synthesis of compound 485-6

2-Bromo-6-fluorobenzaldehyde (1796 mg, 8.85 mmol), compound 485-5 (1 g, 8.85 mmol) and potassium carbonate (3660 mg, 26.54 mmol) were dissolved in DMSO (20 mL), and the reaction mixture was reacted at 100°C for 12 hours under nitrogen protection. The reaction solution was quenched by adding water (20 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 485-6.

### Step 6: synthesis of compound 485-7

Compound 485-6 (720 mg, 2.44 mmol) and N-amino-4-toluenesulfonamide (454 mg, 2.44 mmol) were dissolved in toluene (10 mL) and reacted at room temperature for 0.5 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was not purified and used directly in the next step to obtain compound 485-7.

### Step 7: synthesis of compound 485-8

Compound 485-7 (1129 mg, 2.44 mmol) and sodium hydride (117 mg, 2.93 mmol) were dissolved in toluene (10 mL) and reacted at room temperature for 0.5 hours, and then reacted at 135°C for 25 min. After the reaction was completed as monitored by TLC, the reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by preparative chromatography to obtain compound 485-8.

### Step 8: synthesis of compound 485-9

The compound 485-8 (180 mg, 0.65 mmol) and manganese dioxide (837 mg, 9.67 mmol) were dissolved in DCM (8 mL) and reacted at 40°C for 12 hours. After the reaction was completed as monitored by TLC, the reaction solution was quenched by adding water (6 ml), and liquid separation was performed with DCM (30 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 485-9.

### Step 9: synthesis of compound 485-10

Compound 485-9 (110 mg, 0.4 mmol), tert-butyl carbamate (93 mg, 0.79 mmol), cesium carbonate (386 mg, 1.19 mmol) and palladium catalyst (CAS: 1599466-85-9; 34 mg, 0.04 mmol) were dissolved in 1,4-dioxane (5 mL), and the reaction mixture was reacted at 90°C for 4 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 485-10.

### Step 10: synthesis of compound 485-11

Compound 485-10 (100 mg, 0.32 mmol) was dissolved in hydrogen chloride-ethyl acetate solution and the mixture was reacted at room temperature for 12 hours. The reaction solution was concentrated under reduced pressure, then washed with an aqueous saturated sodium bicarbonate solution until the pH reached 9. The reaction solution was extracted with EA (30 mL), and the organic phase was washed with saturated NaCl solution (10 mL), dried over anhydrous Na₂SO₄, and concentrated under reduced pressure to obtain compound 485-11 (75 mg), which was used directly in the next step without purification.

### Step 11: synthesis of compound 485-12

Compound 485-11 (75 mg, 0.35 mmol), 4-bromo-2-(4-(difluoromethylene)piperidin-1-yl)benzoic acid (127 mg, 0.39 mmol), HATU (146 mg, 0.39 mmol) and *N,N-diisopropylethylamine* (0.17 ml, 1.05 mmol) were dissolved in DMF (4 ml), and the reaction mixture was reacted at room temperature for 2 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 8/1) to obtain compound 485-12.

### Step 12: synthesis of compound 485

Compound 485-12 (105 mg, 0.2 mmol), 2-hydroxyethylsulfonamide (124 mg, 0.99 mmol), cesium carbonate (194 mg, 0.6 mmol) and palladium catalyst (CAS: 1599466-89-3; 16 mg, 0.02 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 485. MS (ESI, pos.ion) m/z:573.1 [M+1]⁺.

¹H NMR (400 MHz, DMSO-*d*₆) δ: 10.75 (s, 1H), 10.09 (br s, 1H), 7.93 (dd, *J=1.0* Hz, 1H), 7.79 (d, *J=1.0* Hz, 1H), 7.09-7.14 (m, 2H), 7.08 (d, *J=1.0* Hz, 1H), 7.02 (dd, *J=1.0* Hz, 1H), 6.50 (s, 1H), 4.92 (s, 2H), 4.12 (s, 2H), 3.73-3.80 (m, 2H), 3.33-3.37 (m, 2H), 3.02 (br s, 4H), 2.24 (br s, 4H), 1.04 (t, *J=1.0* Hz, 2H), 0.81 (t, *J=1.0* Hz, 2H).

### Example 488:

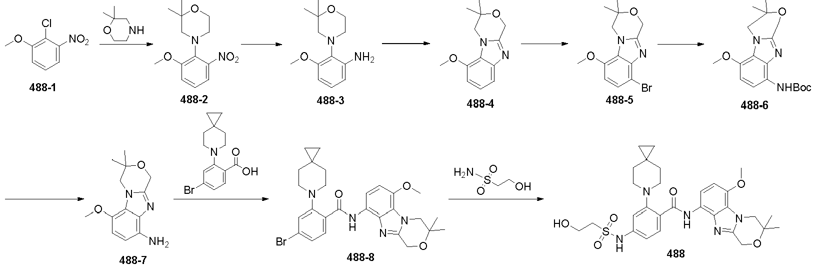

### Step 1: synthesis of compound 488-2

Compound 488-1 (4 g, 21.32 mmol) and 2,2-dimethylmorpholine (5.63 mL, 42.65 mmol) were dissolved in N-methylpyrrolidinone (20 mL), to which N,N-diisopropylethylamine (11.02 g, 85.30 mmol) was added. The reaction mixture was stirred overnight in an oil bath at 160°C. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=100:0-90:10) to obtain compound 488-2.

### Step 2: synthesis of compound 488-3

Compound 488-2 (2.3 g, 8.64 mmol) was dissolved in ethanol (10 mL) and water (10 mL), to which iron dust (2.41 g, 43.18 mmol) and ammonium chloride (2.31 g, 43.18 mmol) were added. The reaction mixture was stirred in an oil bath at 80°C for 3 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by silica gel column chromatography (PE:EA=100:0-90:10) to obtain compound 488-3.

### Step 3: synthesis of compound 488-4

Compound 488-3 (1.2 g, 5.08 mmol) was dissolved in dichloromethane (10 mL), to which trifluoroacetic acid (3.78 mL, 50.78 mmol) and hydrogen peroxide (1.53 mL, 50.78 mmol) were added. The reaction mixture was stirred in an oil bath at 40°C for 4 hours. After the reaction was completed, the reaction solution was quenched with anhydrous sodium sulfite, and then diluted with water (30 mL), and extracted with ethyl acetate (30 × 3 mL). The organic layer was washed with brine (30×3 mL), dried over Na₂SO₄, filtered and concentrated. The crude product was purified by silica gel column chromatography (PE:EA=100:0-80:20) to obtain compound 488-4.

### Step 4: synthesis of compound 488-5

Compound 488-4 (650 mg, 2.80 mmol) was dissolved in dichloromethane (10 mL), to which N-bromosuccinimide (448.27 mg, 2.52 mmol) was added. The reaction mixture was stirred at room temperature for half an hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by silica gel column chromatography (PE:EA=100:0-90:10) to obtain compound 488-5.

### Step 5: synthesis of compound 488-6

Compound 488-5 (240 mg, 0.77 mmol) and tert-butyl carbamate (180.71 mg, 1.54 mmol) were dissolved in 1,4-dioxane (5 mL). To this mixture was added palladium catalyst (CAS: 1599466-81-5; 66.33 mg, 0.08 mmol) and cesium carbonate (753.87 mg, 2.31 mmol). The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by silica gel column chromatography (PE:EA=100:0-90:10) to obtain compound 488-6.

### Step 6: synthesis of compound 488-7

Compound 488-6 (211 mg, 0.61 mmol) was dissolved in hydrogen chloride-ethyl acetate solution (3 mL) and the reaction mixture was stirred at room temperature for half an hour. After the reaction was completed, the reaction solution was neutralized with aqueous sodium bicarbonate solution, and then diluted with water (60 mL), and extracted with ethyl acetate (30 × 3 mL). The organic layer was washed with brine (30×3 mL), dried over Na₂SO₄, filtered and concentrated. The crude product was purified by silica gel column chromatography (PE:EA=100:0-80:20) to obtain compound 488-7.

### Step 7: synthesis of compound 488-8

Compound 488-7 (60 mg, 0.24 mmol) and 4-bromo-2-(6-azaspiro[2.5]octan-6-yl)benzoic acid (82.78 mg, 0.27 mmol) were dissolved in acetonitrile (3 mL), to which azabenzotriazolyltetramethyluronium hexafluorophosphate (110.70 mg, 0.29 mmol) and N,N-diisopropylethylamine (62.71 mg, 0.49 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by silica gel column chromatography (PE:EA=100:0-90:10) to obtain compound 488-8.

### Step 8: synthesis of compound 488

Compound 488-8 (100 mg, 0.19 mmol) and 2-hydroxyethane-1-sulfonamide (46.39 mg, 0.37 mmol) were dissolved in 1,4-dioxane (3 mL). To this mixture was added palladium catalyst (CAS: 1599466-83-7; 34.11 mg, 0.04 mmol) and cesium carbonate (120.49 mg, 0.37 mmol). The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by preparative chromatography to obtain compound 488. MS (ESI, pos.ion) m/z: 584.2 [M+1]⁺.

**¹H NMR (400 MHz, DMSO-*d*₆) δ** = **12.37 (s, 1H), 8.25 (d, *J* = 8.8 Hz, 1H), 8.01 (d, *J* = 8.6 Hz, 1H), 7.24 - 7.18 (m, 1H), 7.04 (br d, *J* = 8.6 Hz, 1H), 6.74 (d,** *J =* **8.6 Hz, 1H), 4.95 (s, 2H), 4.31 (s, 2H), 3.89 (s, 3H), 3.75 (t, *J =* 6.4 Hz, 2H), 3.31 - 3.27 (m, 2H), 3.02 - 2.90 (m, 4H), 1.83 - 1.76 (m, 4H), 1.36 (s, 6H), 0.31 (s, 4H).**

### Example 489:

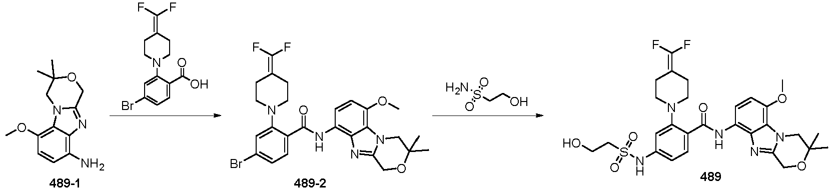

### Step 1: synthesis of compound 489-2

Compound 489-1 (60 mg, 0.24 mmol) and 4-bromo-2-[4-(difluoromethylene)hexahydropyridin-1-yl]benzoic acid (88.64 mg, 0.27 mmol) were dissolved in acetonitrile (2 mL), and azabenzotriazolyltetramethyluronium hexafluorophosphate (110.70 mg, 0.29 mmol) and N,N-diisopropylethylamine (62.71 mg, 0.49 mmol) were added. The reaction mixture was stirred at room temperature for 1 hour. After the reaction was completed, the reaction solution was diluted with water (60 mL), and extracted with ethyl acetate (30×3 mL). The organic layer was washed with brine (30×3 mL), dried over Na₂SO₄, filtered and concentrated. The crude product was purified by silica gel column chromatography (PE:EA=100:0-90:10) to obtain compound 489-2.

### Step 2: synthesis of compound 489

Compound 489-2 (100 mg, 0.18 mmol) and 2-hydroxyethane-1-sulfonamide (89.16 mg, 0.71 mmol) were dissolved in 1,4-dioxane (3 mL). To this mixture was added palladium catalyst (CAS: 1599466-83-7; 16.39 mg, 0.02 mmol) and cesium carbonate (116.07 mg, 0.36 mmol). The reaction mixture was stirred in an oil bath at 95°C for 1 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by preparative chromatography to obtain compound 489. MS m/z (ESI):606.2 [M+1]⁺.

¹H NMR (400 MHz, DMSO-*d*₆) δ = 12.29 (br s, 1H), 8.27 - 8.25 (m, 1H), 8.00 - 7.97 (m, 1H), 7.09 - 7.02 (m, 2H), 6.75 - 6.73 (m, 1H), 4.85 (br s, 2H), 4.29 (br s, 2H), 3.89 (br s, 3H), 3.77 - 3.72 (m, 2H), 3.27 - 3.24 (m, 2H), 3.00 - 2.90 (m, 4H), 2.64 - 2.56 (m, 4H), 1.39 - 1.29 (m, 6H).

### Example 490: synthesis of N-(2,2-dimethyl-2,3-dihydro-1H-benzo[d]pyrrolo[1,2-a]imidazol-5-yl)-4-((2-hydroxyethyl)sulfonamido)-2-(6-azaspiro[2.5]oct-6-yl)benzamide

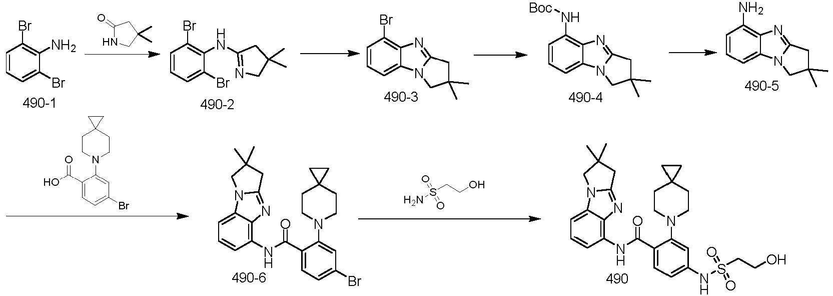

### Step 1: synthesis of compound 490-2

4,4-Dimethyltetrahydropyrrol-2-one (1200 mg, 10.60 mmol) and compound 490-1 (2659.75 mg, 10.60 mmol) were dissolved in DCE (15 mL), phosphorus oxychloride (1625.87 mg, 10.60 mmol) was added, and the mixture was stirred at 60°C for 3 hours. The reaction solution was poured into an aqueous saturated sodium bicarbonate solution, extracted three times with ethyl acetate (60 ml), washed twice with sodium chloride aqueous solution (60 mL), the organic phase was dried over anhydrous sodium sulfate, concentrated under reduced pressure, and the crude product was purified by silica gel column chromatography (EA/PE = 0-50%) to obtain compound 490-2.

### Step 2: synthesis of compound 490-3

The compound 490-2 (50 mg, 0.14 mmol) and cesium carbonate (94.15 mg, 0.29 mmol) were dissolved in DMAC (1 mL), and the reaction was stirred at 160°C for 2 hours under microwave irradiation. The reaction solution was poured into water, extracted three times with ethyl acetate (10 ml), washed twice with sodium chloride aqueous solution (20 mL), the organic phase was dried over anhydrous sodium sulfate, concentrated under reduced pressure, and the crude product was purified by silica gel column chromatography (EA/PE = 0-50%) to obtain compound 490-3.

### Step 3: synthesis of compound 490-4

Compound 490-3 (30 mg, 0.11 mmol), NH₂Boc (26.51 mg, 0.23 mmol), Brettphos Pd G3 (10.27 mg, 0.01 mmol) and sodium tert-butoxide (21.75 mg, 0.23 mmol) were dissolved in dioxane (1 mL). The reaction was stirred at 100°C for 12 hours. The reaction solution was poured into water, extracted three times with ethyl acetate (10 ml), washed twice with sodium chloride aqueous solution (30 mL), the organic phase was dried over anhydrous sodium sulfate, concentrated under reduced pressure, and the crude product was purified by silica gel column chromatography (EA/PE = 0-50%) to obtain compound 490-4.

### Step 4: synthesis of compound 490-5

Compound 490-4 (30 mg, 0.10 mmol) was dissolved in DCM (1 mL), trifluoroacetic acid (0.5 mL) was added dropwise under ice bath conditions, and the reaction was stirred at room temperature for 1 hour. The reaction solution was poured into an aqueous saturated sodium bicarbonate solution, extracted three times with ethyl acetate (10 ml), washed twice with sodium chloride aqueous solution (20 mL), and the organic phase was dried over anhydrous sodium sulfate, and concentrated under reduced pressure to obtain compound 490-5.

### Step 5: synthesis of compound 490-6

Compound 490-5 (20 mg, 0.10 mmol), HATU (75.57 mg, 0.20 mmol) and 2-(6-azaspiro[2.5]oct-6-yl)-4-bromobenzoic acid (30.82 mg, 0.10 mmol) were dissolved in DMF (1 mL). DIEA (38.53 mg, 0.30 mmol) was added and the reaction was stirred at room temperature for 12 hours. The reaction solution was poured into water, extracted three times with ethyl acetate (10 ml), washed twice with sodium chloride aqueous solution (30 mL), the organic phase was dried over anhydrous sodium sulfate, concentrated under reduced pressure, and the crude product was purified by silica gel column chromatography (EA/PE = 0-50%) to obtain compound 490-6.

### Step 6: synthesis of compound 490

Compound 490-6 (29 mg, 0.06 mmol), 2-hydroxyethane-1-sulfonamide (8.83 mg, 0.07 mmol), tBuXPhosPdG4 (8.08 mg, 0.01 mmol) and cesium carbonate (28.72 mg, 0.09 mmol) were dissolved in dioxane (1 mL), and the mixture was stirred at 100°C for 12 hours. The reaction solution was poured into water, extracted three times with ethyl acetate (10 ml), washed twice with sodium chloride aqueous solution (30 mL), the organic phase was dried over anhydrous sodium sulfate, concentrated under reduced pressure, and the crude product was purified by silica gel column chromatography (EA/PE = 0-50%) to obtain compound 490. MS (ESI, pos.ion) m/z: 538.2 [M+1]⁺.

¹H NMR (DMSO-*d*₆, 400 MHz) *δ* 12.34 (s, 1H), 10.21 (br s, 1H), 8.31 - 8.26 (m, 1H), 8.01 (d, J = 8.5 Hz, 1H), 7.24 (d, *J* =2.0 Hz, 1H), 7.16 - 7.12 (m, 2H), 7.07 (dd, *J* = 2.1, 8.6 Hz, 1H), 4.95 (br s, 1H), 3.92 (s, 2H), 3.76 (t, *J* = 6.5 Hz, 2H), 3.39 -3.36 (m, 2H), 3.01 - 2.94 (m, 4H), 2.82 (s, 2H), 1.77 (br s, 4H), 1.29 (s, 6H), 0.28 (s, 4H).

### Example 491: synthesis of N-(6-(4,4-difluoropiperidin-1-yl)-5-methoxypyridin-2-yl)-4-((2-hydroxyethyl)sulfonamido)-2-(methylamino)-6-(6-azaspiro[2.5]oct-6-yl)benzamide (491)

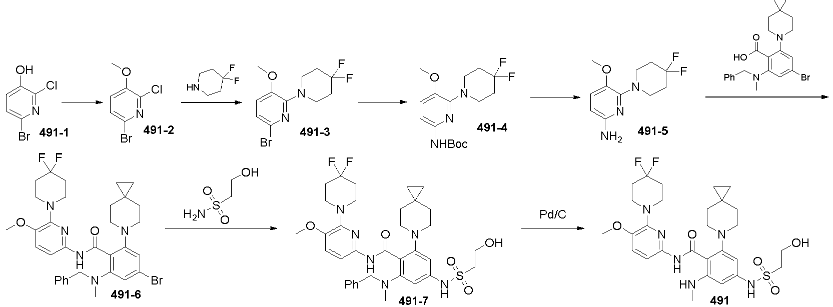

### Step 1: synthesis of compound 491-2

Compound 491-1 (950 mg, 4.56 mmol) and sodium methoxide (258 mg, 4.79 mmol) were dissolved in DMF (5 mL), to which iodomethane (4.43 g, 28.12 mmol) was added under ice bath conditions, and the reaction mixture was allowed to react at 25°C for 1 hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1:1) to obtain compound 491-2.

### Step 2: synthesis of compound 491-3

Compound 491-2 (878 mg, 3.95 mmol), and granular potassium carbonate (1.36 g, 9.87 mmol) were dissolved in DMSO (10 mL), to which 4,4-difluoropiperidine hydrochloride (746 mg, 4.74 mmol) was added, and the reaction mixture was allowed to react at 110°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=50:1) to obtain compound 491-3.

### Step 3: synthesis of compound 491-4

Compound 491-3 (297 mg, 0.97 mmol), NH₂Boc (226 mg, 1.93 mmol), cesium carbonate (945 mg, 2.90 mmol) and the fourth-generation palladium catalyst (CAS: 1599466-85-9; 82 mg, 0.10 mmol) were dissolved in 1,4-dioxane (5 mL), and the reaction mixture was reacted at 90°C for 2 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 491-4. MS (ESI, pos.ion) m/z:344.1 [M+1]⁺.

### Step 4: synthesis of compound 491-5

Compound 491-4 (289 mg, 0.84 mmol) was dissolved in DCM (1 mL), to which HCl\dioxane (2 mL) was added, and the reaction mixture was allowed to react at 25°C for 1 hour. The reaction solution was filtered, neutralized, extracted and dried, and concentrated to obtain compound 491-5. MS (ESI, pos.ion) m/z:244.1 [M+1]⁺.

### Step 5: synthesis of compound 491-6

Compound 491-5 (224 mg, 0.92 mmol), 2-(benzyl(methyl)amino)-4-bromo-6-(6-azaspiro[2.5]oct-6-yl)benzoic acid (395 mg, 0.92 mmol), and TCFH (775 mg, 2.76 mmol) were dissolved in THF (5 mL), to which DIEA (0.61 mL, 3.68 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 491-6.

### Step 6: synthesis of compound 491-7

Compound 491-6 (75 mg, 0.11 mmol), 2-hydroxyethanesulfonamide (57 mg, 0.46 mmol), cesium carbonate (112 mg, 0.34 mmol) and the fourth-generation palladium catalyst (CAS: 1599466-89-3; 10 mg, 0.01 mmol) were dissolved in 1,4-dioxane (3 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=10:1) to obtain compound 491-7. MS (ESI, pos.ion) m/z:699.3 [M+1]⁺.

### Step 7: synthesis of compound 491

Compound 491-7 (58 mg, 0.08 mmol) and Pd/C (30 mg) were dissolved in anhydrous methanol (3 mL), and the mixture was reacted at 25°C for 2 h under hydrogen protection. The reaction solution was filtered, and concentrated under reduced pressure, and the crude product was purified by preparative chromatography to obtain compound 491. MS (ESI, pos.ion) m/z:609.3 [M+1]⁺.

¹H NMR (400 MHz, DMSO-d₆) δ = 13.00 (s, 1H), 9.91 (br s, 1H), 8.74 (br d, *J* = 4.9 Hz, 1H), 7.75 (d, *J* = 8.5 Hz, 1H), 7.33 (d, *J =* 8.6 Hz, 1H), 6.48 - 6.42 (m, 1H), 6.37 - 6.31 (m, 1H), 4.94 (br t, *J* = 5.4 Hz, 1H), 3.80 (s, 3H), 3.78 - 3.72 (m, 2H), 3.49 (br s, 4H), 3.36 (br s, 2H), 2.91 (br s, 4H), 2.80 - 2.76 (m, 3H), 2.12 - 2.02 (m, 4H), 1.23 (s, 4H), 0.34 (s, 4H).

### Example 519: synthesis of N-(6-fluoro-3,3-dimethyl-3,4-dihydro-1H-[1,4] oxazinyl[4,3-a]indol-9-yl)-4-((2-hydroxyethyl)sulfanilamido)-2-(6-azaspiro[2.5]oct-6-yl)benzamide (519)

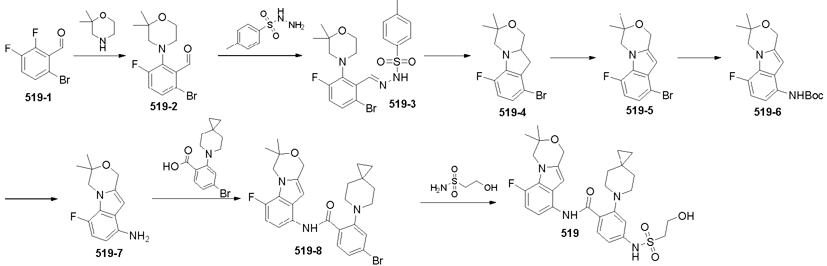

### Step 1: synthesis of compound 519-2

Compound 519-1 (2 g, 9.05 mmol) was dissolved in N-methylpyrrolidinone (10 mL), to which 2,2-dimethylmorpholine (1.25 g, 10.86 mmol) and N,N-diisopropylethylamine (3.00 mL, 18.10 mmol) were added, and the reaction mixture was allowed to react at 100°C overnight. The reaction solution was quenched with ice water, and extracted with ethyl acetate. The organic phase was washed with saturated NaCl solution, dried over anhydrous Na₂SO₄, and concentrated under reduced pressure. The crude product was purified by silica gel column chromatography (PE:EA=20/1-10/1) to obtain compound 519-2.

### Step 2: synthesis of compound 519-3

Compound 519-2 (2 g, 6.33 mmol) was dissolved in toluene (20 mL), to which p-toluenesulfonyl hydrazide (1.4 g, 7.6 mmol) was added. The mixture was stirred at 25°C for 10 minutes to obtain compound 519-3 which was used directly in the next reaction.

### Step 3: synthesis of compound 519-4

To the reaction solution containing compound 519-3 (3.0 g, 6.3 mmol) was slowly added sodium hydride (305 mg, 7.63 mmol), stirred at room temperature for 10 minutes, and then the reaction temperature was raised to 130°C and the mixture was reacted for half an hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 519-4.

### Step 4: synthesis of compound 519-5

Compound 519-4 (1.6 g, 5.33 mmol) was dissolved in dichloromethane (10 mL), to which manganese dioxide (4.6 g, 53 mmol) was slowly added, and the mixture was reacted overnight at 38°C. The reaction solution was filtered, and the filtrate was collected, and concentrated under reduced pressure. The crude product was purified by silica gel column chromatography (PE:EA=20/1-10/1) to obtain compound 519-5.

### Step 5: synthesis of compound 519-6

Compound 519-5 (1 g, 3.37 mmol) and Cs₂CO₃ (2.2 g, 10.11 mmol) were dissolved in 1,4-dioxane (10 mL), to which tert-butyl carbamate (789 mg, 6.74 mmol), and palladium catalyst (CAS: 1599466-89-2; 75 mg, 0.34 mmol) were added, and the reaction mixture was reacted at 90°C overnight under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 519-6.

### Step 6: synthesis of compound 519-7

Compound 519-6 (257 mg, 0.77 mmol) was dissolved in dichloromethane (4 mL), to which HCl/EA (4 mL) was added, and the mixture was stirred at 25°C for 1 hour. The solvent was removed by concentration under reduced pressure, and solids were neutralized. The reaction system was subjected to extraction, drying and concentration to obtain compound 519-7, which was used directly in the next reaction.

### Step 7: synthesis of compound 519-8

Compound 519-7 (100 mg, 0.42 mmol), HATU (240 mg, 0.63 mmol), 4-bromo-2-(6-azaspiro[2.5]octan-6-yl)benzoic acid (132 mg, 0.42 mmol) were dissolved in DMF (5 mL), to which DIEA (225 uL, 1.26 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 519-8.

### Step 8: synthesis of compound 519

Compound 519-8 (143 mg, 0.27 mmol), 2-hydroxyethanesulfonamide (68 mg, 0.54 mmol), cesium carbonate (266 mg, 0.81 mmol) and palladium catalyst (CAS: 1599466-89-3; 22 mg, 0.03 mmol) were dissolved in 1,4-dioxane (5 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20/1-10/1) to obtain compound 519. MS m/z (ESI):571.3 [M+1]⁺.

**¹H NMR (400 MHz, CHLOROFORM-d)** δ = 11.53 (s, 1H), 8.07 (d, *J* = 8.4 Hz, 1H), 7.54 (dd, *J* = 3.8, 8.4 Hz, 1H), 7.24 (d, *J* = 2.0 Hz, 1H), 7.06 - 7.02 (m, 1H), 6.90 (dd, *J* = 1.9, 8.4 Hz, 1H), 6.77 (dd, *J* = 8.5, 12.0 Hz, 1H), 6.20 (s, 1H), 4.93 (s, 2H), 4.11 (s, 2H), 4.02 (br d, *J* = 5.0 Hz, 2H), 3.24 - 3.21 (m, 2H), 3.03 (br t, *J* = 5.2 Hz, 4H), 1.49 - 1.42 (m, 4H), 1.36 (s, 6H), 0.25 (s, 4H).

### Example 520: synthesis of 2-(4-(difluoromethylene)piperidin-1-yl)-N-(6-fluoro-3,3-dimethyl-3,4-dihydro-1H-[1,4] oxazino [4,3-a] indol-9-yl)-4-((2-hydroxyethyl)sulfonamido)benzamide (520)

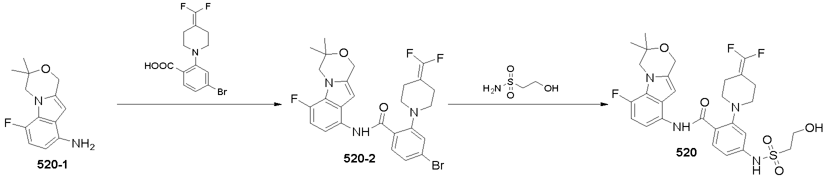

### Step 1: synthesis of compound 520-2

Compound 520-1 (100 mg, 0.42 mmol), HATU (240 mg, 0.63 mmol), 4-bromo-2-(4-(difluoromethylene)piperidin-1-yl)benzoic acid (139 mg, 0.42 mmol) were dissolved in DMF (4 mL), to which DIEA (225 uL, 1.26 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 520-2.

### Step 2: synthesis of compound 520

Compound 520-2 (70 mg, 0.12 mmol), 2-hydroxyethanesulfonamide (32 mg, 0.24 mmol), cesium carbonate (118 mg, 0.36 mmol) and palladium catalyst (CAS: 1599466-89-3; 10 mg, 0.01 mmol) were dissolved in 1,4-dioxane (2 mL), and the reaction mixture was reacted at 90°C for 2 h under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20/1-10/1) to obtain compound 520. MS m/z (ESI):593.3 [M+1]⁺.

¹H NMR (400 MHz, CHLOROFORM-d) δ = 11.25 (s, 1H), 8.13 (d, *J=* 8.4 Hz, 1H), 7.65 (dd, *J* = 3.8, 8.4 Hz, 1H), 7.24 (d, *J* = 2.0 Hz, 1H), 7.19 (br s, 1H), 7.00 (dd, *J =* 1.8, 8.4 Hz, 1H), 6.86 (dd, *J =* 8.5, 12.0 Hz, 1H), 6.21 (s, 1H), 5.01 (s, 2H), 4.19 (s, 2H), 4.11 (br t, *J =* 4.9 Hz, 2H), 3.34 - 3.27 (m, 2H), 3.10 (br t, *J =* 5.4 Hz, 4H), 2.35 (br s, 4H), 1.45 (s, 6H).

### Example 521: synthesis of 2-(6-azaspiro[2.5]oct-6-yl)-N-(6-chloro-3,3-dimethyl-3,4-dihydro-1H-[1,4]oxazino[4,3-a]indol-9-yl)-4-{[(2-hydroxyethyl)sulfonamide]}benzamide (521)

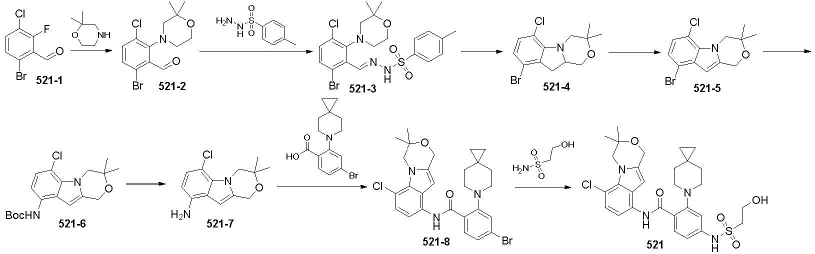

### Step 1: synthesis of compound 521-2

Compound 521-1 (3.00 g, 12.63 mmol) was dissolved in NMP (15 mL), to which 2,2-dimethyl-1,4-oxazine (1.40 g, 15.16 mmol) and DIEA (6.53 g, 50.52 mmol) were added, and the reaction mixture was allowed to react at 100°C overnight. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/10-1/5) to obtain compound 521-2.

### Step 2: synthesis of compound 521-3

Compound 521-2 (2.50 g, 7.52 mmol) was dissolved in toluene (25 mL), to which N-amino-4-tosylamide (1.68 g, 9.02 mmol) was added, and reaction was carried out at room temperature for 20 min to obtain the reaction solution containing compound 521-3, which was used directly in the next reaction.

### Step 3: synthesis of compound 521-4

To the reaction solution from step 2, sodium hydride (0.33 g, 8.27 mmol) was added and the reaction mixture was allowed to react at 135°C for 20 min. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 521-4.

### Step 4: synthesis of compound 521-5

Compound 521-4 (700 mg, 2.21 mmol) was dissolved in DCM (10 mL), to which manganese dioxide (1922.24 mg, 22.11 mmol) was added, and the mixture was reacted overnight at room temperature. The reaction solution was filtered, and concentrated under reduced pressure. The crude product was purified by silica gel column chromatography (PE:EA=1/100-1/50) to obtain compound 521-5.

### Step 5: synthesis of compound 521-6

Compound 521-5 (300 mg, 0.95 mmol), NH₂Boc (223.42 mg, 1.91 mmol), cesium carbonate (931.85 mg, 2.86 mmol) and XantPhos Pd G4 (91.43 mg, 0.10 mmol) were dissolved in 1,4-dioxane (8 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 521-6.

### Step 6: synthesis of compound 521-7

Compound 521-6 (210 mg, 0.60 mmol) was dissolved in DCM (3 mL), to which hydrogen chloride-1,4-dioxane (3 mL) was added, and the reaction mixture was allowed to react at room temperature for 0.5 h. The reaction solution was concentrated under reduced pressure to obtain solids, neutralized, extracted and dried, and concentrated to obtain compound 521-7, which was used directly in the next reaction.

### Step 7: synthesis of compound 521-8

Compound 521-7 (80 mg, 0.32 mmol), 4-bromo-2-(6-azaspiro[2.5]octan-6-yl)benzoic acid (104.22 mg, 0.34 mmol), and HATU (129.28 mg, 0.34 mmol) were dissolved in DMF (5 mL), to which DIEA (217.14 mg, 1.68 mmol) was added, and the reaction mixture was allowed to react at room temperature for 4 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 521-8.

### Step 8: synthesis of compound 521

Compound 521-8 (98 mg, 0.18 mmol), 2-hydroxylethanesulfonamide (90.10 mg, 0.72 mmol), cesium carbonate (117.30 mg, 0.36 mmol) and BrettPhos Pd G4 (18.41 mg, 0.02 mmol) were dissolved in 1,4-dioxane (5 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative high performance liquid chromatography to obtain compound 521. MS m/z (ESI):587.8 [M+1]⁺.

**¹H NMR** (DMSO-d6) δ = 11.18 (s, 1H), 7.85 (dd, *J* = 8.4, 11.6 Hz, 2H), 7.18 (d, *J* = 1.8 Hz, 1H), 7.09 (d, *J* = 8.3 Hz, 1H), 7.03 (dd, *J* = 1.9, 8.5 Hz, 1H), 6.60 (s, 1H), 4.96 (s, 2H), 4.42 (s, 2H), 3.77 (t, *J* = 6.6 Hz, 2H), 3.37 - 3.34 (m, 2H), 3.06 - 2.95 (m, 4H), 1.43 (br s, 4H), 1.35 (s, 6H), 0.27 (s, 4H).

### Example 522: synthesis of N-(6-chloro-3,3-dimethyl-3,4-dihydro-1H-[1,4]oxazino[4,3-a]indol-9-yl)-2-[4-(difluoromethylene)hexahydropyridin-1-yl]-4-{[(2-hydroxyethyl)sulfonamide]}benzamide (522)

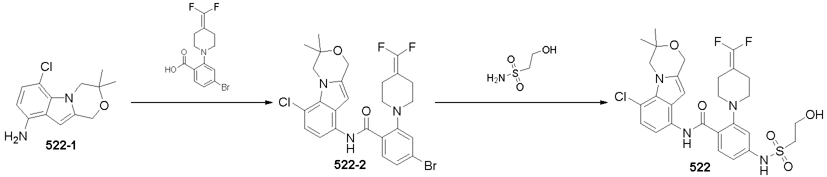

### Step 1: synthesis of compound 522-2

Compound 522-1 (60 mg, 0.24 mmol), 4-bromo-2-[4-(difluoromethylene)hexahydropyridin-1-yl]benzoic acid (95.38 mg, 0.29 mmol), and HATU (110.27 mg, 0.29 mmol) were dissolved in DMF (5 mL), to which DIEA (186.12 mg, 1.44 mmol) was added, and the reaction mixture was allowed to react at room temperature for 6 h. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=1/20-1/10) to obtain compound 522-2.

### Step 2: synthesis of compound 522

Compound 522-2 (100 mg, 0.18 mmol), 2-hydroxylethanesulfonamide (88.62 mg, 0.71 mmol), cesium carbonate (115.36 mg, 0.35 mmol) and BrettPhos Pd G4 (16.30 mg, 0.02 mmol) were dissolved in 1,4-dioxane (5 mL), and the reaction mixture was reacted at 95°C for 4 h under N₂ protection. The reaction solution was quenched with water, and extracted with EA. The organic phase was washed with saturated NaCl solution, dried over anhydrous Na₂SO₄, and concentrated under reduced pressure, and the crude product was purified by preparative high performance liquid chromatography to obtain compound 522. MS m/z (ESI):609.1 [M+1]⁺.

**¹H NMR** (DMSO-d6) δ = 10.74 (br s, 1H), 10.30 - 9.88 (m, 1H), 7.88 (br d, *J* = 7.8 Hz, 1H), 7.75 (br d, *J* = 7.8 Hz, 1H), 7.15 - 7.04 (m, 2H), 7.03 - 6.96 (m, 1H), 6.61 (br s, 1H), 4.95 (br s, 2H), 4.40 (br s, 2H), 3.77 (br s, 2H), 3.41 - 3.33 (m, 2H), 3.01 (br s, 4H), 2.22 (br s, 4H), 1.34 (br s, 6H).

### Example 523: synthesis of N-(6-difluoromethoxy)-3,3-dimethyl-3,4-dihydro-1H-[1,4]oxazino[4,3-a]indol-9-yl)-4-((2-hydroxyethyl)sulfonamido)-2-(6-azaspiro[2.5]octan-6-yl)benzamide (523)

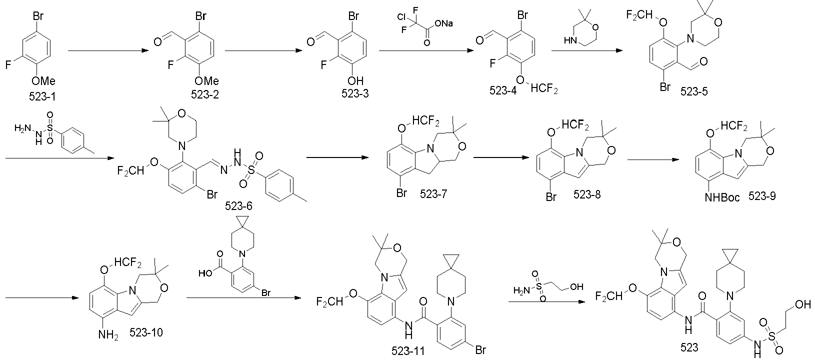

### Step 1: synthesis of compound 523-2

Compound 523-1 (1000 mg, 4.88 mmol) was dissolved in tetrahydrofuran (10 mL), lithium diisopropylamide (2.95 ml, 5.85 mmol) was slowly added dropwise at -78°C. After reaction at -78°C for 1 hour, DMF (0.49 ml, 6.34 mmol) was added and the reaction system was allowed to react at -78°C for 2 hours. The reaction solution was quenched by an aqueous saturated ammonium chloride solution (20 ml), water (10 ml) was added, and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 523-2.

### Step 2: synthesis of compound 523-3

Compound 523-2 (5000 mg, 21.46 mmol) was dissolved in dichloromethane (20 mL), 1M boron tribromide solution (64 ml, 64.37 mmol) was slowly added dropwise at 0°C, and the reaction mixture was allowed to react at 0°C for 2 hours. The reaction solution was quenched by an aqueous saturated ammonium chloride solution (20 ml), water (10 ml) was added, and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 523-3.

### Step 3: synthesis of compound 523-4

Compound 523-3 (4500 mg, 20.55 mmol) was dissolved in DMF (16 mL) and H₂O (2 mL), followed by sodium [(2-chloro-2,2-difluoroacetyl)oxy] (6270 mg, 41 mmol) and potassium carbonate (2840 mg, 20.55 mmol), and the reaction mixture was allowed to react at 100°C for 16 hours. The reaction solution was quenched by an aqueous saturated ammonium chloride solution (20 ml), water (10 ml) was added, and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 523-4.

### Step 4: synthesis of compound 523-5

Compound 523-4 (1200 mg, 4.48 mmol), 2,2-dimethyl-1,4-oxazinane (618 mg, 5.37 mmol) and *N,N* diisopropylethylamine (1.15 g, 8.96 mmol) were dissolved in NMP (10 mL) and reacted at 100°C for 16 hours under nitrogen protection. The reaction solution was quenched by adding water (20 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 523-5.

### Step 5: synthesis of compound 523-6

Compound 523-5 (870 mg, 2.4 mmol) and *N*-amino-4-toluenesulfonamide (535.6 mg, 2.88 mmol) were dissolved in toluene (10 mL) and reacted at room temperature for 0.5 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was not purified and used directly in the next step to obtain compound 523-6.

### Step 6: synthesis of compound 523-7

Compound 523-6 (1270 mg, 2.39 mmol) and sodium hydride (114.8 mg, 2.87 mmol) were dissolved in toluene (40 mL) and reacted at room temperature for 0.5 hours, and then reacted at 135°C for 25 min. After the reaction was completed as monitored by TLC, the reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by preparative chromatography to obtain compound 523-7.

### Step 7: synthesis of compound 523-8

The compound 523-7 (300 mg, 0.82 mmol) and manganese dioxide (710 mg, 8.2 mmol) were dissolved in DCM (10 mL) and reacted at room temperature for 12 hours. After the reaction was completed as monitored by TLC, the reaction solution was subjected to suction filtration and the filtrate was quenched by adding water (6 ml), and liquid separation was performed with DCM (30 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 523-8.

### Step 8: synthesis of compound 523-9

Compound 523-8 (250 mg, 0.72 mmol), tert-butyl carbamate (170 mg, 1.45 mmol), cesium carbonate (708 mg, 2.17 mmol) and palladium catalyst (CAS: 1599466-85-9; 62 mg, 0.07 mmol) were dissolved in 1,4-dioxane (8 mL), and the reaction mixture was reacted at 90°C for 12 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 10/1) to obtain compound 523-9.

### Step 9: synthesis of compound 523-10

Compound 523-9 (120 mg, 0.31 mmol) was dissolved in hydrogen chloride-ethyl acetate solution and the mixture was reacted at room temperature for 3 hours. The reaction solution was concentrated under reduced pressure, neutralized, extracted and dried, and concentrated to obtain compound 523-10, which was used directly in the next step without purification.

### Step 10: synthesis of compound 523-11

Compound 523-10 (50 mg, 0.16 mmol), 4-bromo-2-(6-azaspiro[2.5]octan-6-yl)benzoic acid (54 mg, 0.17 mmol), HATU (66 mg, 0.17 mmol) and *N,N-*diisopropylethylamine (0.11 ml, 0.63 mmol) were dissolved in DMF (4 ml), and the reaction mixture was reacted at room temperature for 12 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 20/1) to obtain compound 523-11.

### Step 11: synthesis of compound 523

Compound 523-11 (40 mg, 0.07 mmol), 2-hydroxyethylsulfonamide (26 mg, 0.21 mmol), cesium carbonate (68 mg, 0.21 mmol) and palladium catalyst (CAS: 1599466-89-3; 6 mg, 0.01 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO4, and concentrated under reduced pressure. The crude product was purified by silica gel column chromatography (PE/EA= 1/0 - 1/1) to obtain compound 523.

MS (ESI, pos.ion) m/z:619.2 [M+1]⁺.

¹H NMR (400 MHz, DMSO-*d*₆) δ: 11.20 (s, 1H), 7.85 (d, *J =* 8.5 Hz, 1H), 7.79 (d, *J* = 8.4 Hz, 1H), 7.26 (t, *J =* 76 Hz, 1H), 7.18 (d, *J =* 1.9Hz, 1H), 7.02 (dd, *J =* 8.5, 2.0 Hz, 1H), 6.90 (d, *J =* 8.4 Hz, 1H), 6.53 (s, 1H), 4.96 (s, 2H), 4.19 (s, 2H), 3.77 (t, *J =* 6.6 Hz, 2H),3.33-3.36 (m, 2H), 2.96-3.04 (m, 4H), 1.42-1.51 (m, 4H), 1.34 (s, 6H), 0.28 (s, 4H).

### Example 524: synthesis of N-(8,8-difluoro-4-methoxy-6,7,8,9-tetrahydropyridino[1,2-a]indol-1-yl)-4-(2-hydroxyethylsulfonamido)-2-(6-azaspiro[2.5]octan-6-yl)benzamide (524)

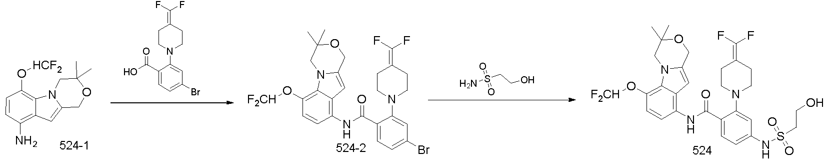

### Step 1: synthesis of compound 524-2

Compound 524-1 (50 mg, 0.16 mmol), 4-bromo-2-(4-(difluoromethylene)piperidin-1-yl)benzoic acid (57 mg, 0.17 mmol), HATU (66 mg, 0.17 mmol) and *N,N-*diisopropylethylamine (0.11 ml, 0.63 mmol) were dissolved in DMF (4 ml), and the reaction mixture was reacted at room temperature for 2 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 - 10/1) to obtain compound 524-2.

### Step 2: synthesis of compound 524-3

Compound 524-2 (40 mg, 0.07 mmol), 2-hydroxyethylsulfonamide (25 mg, 0.2 mmol), cesium carbonate (66 mg, 0.2 mmol) and palladium catalyst (CAS: 1599466-89-3; 6 mg, 0.01 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 524. MS (ESI, pos.ion) m/z:641.2 [M+1]⁺.

¹H NMR (400 MHz, DMSO-*d*₆) δ: 10.74 (s, 1H), 7.80 (d, *J =* 8.5 Hz, 1H), 7.76 (d, *J* = 8.4 Hz, 1H), 7.25 (t, *J* = 76 Hz, 1H), 7.05-7.07 (m, 1H), 7.01 (dd, *J* = 8.4, 1.9 Hz, 1H), 6.90 (d, *J =* 8.4 Hz, 1H), 6.54 (s, 1H), 4.94 (s, 2H), 4.17 (s, 2H), 3.77 (t, *J* = 6.6 Hz, 2H),3.33-3.36 (m, 2H), 3.01 (br t, *J =* 5.0 Hz, 4H), 2.17-2.30 (m, 4H), 1.33 (s, 6H).

### Example 525:

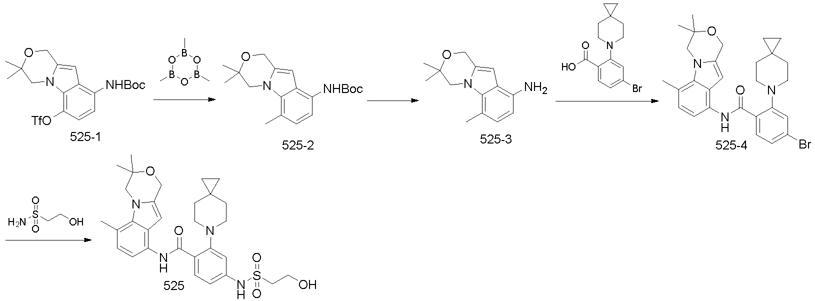

### Step 1: synthesis of compound 525-2

Compound 525-1 (300 mg, 0.647 mmol), cesium carbonate (632 mg, 1.94 mmol), and tetra(triphenylphosphine)palladium (75 mg, 0.065 mmol) were dissolved in 1,4-dioxane (5 ml) and water (1 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/7) to obtain compound 525-2.

### Step 2: synthesis of compound 525-3

Compound 525-2 (190 mg, 0.576 mmol) was dissolved in dichloromethane (5 ml), and hydrogen chloride-ethyl acetate solution (2.88 ml, 2N) was added dropwise under ice bath conditions, and the reaction mixture was stirred at room temperature for 2 hours. A solid was precipitated out from the reaction solution, the solid was neutralized, and the reaction solution was extracted, and concentrated, to obtain compound 525-3.

### Step 3: synthesis of compound 525-4

Compound 525-3 (60 mg, 0.26 mmol), 4-bromo-2-(6-azaspiro[2.5]oct-6-yl)benzoic acid (81 mg, 0.26 mmol), HATU (137 mg, 0.391 mmol) and DIEA (101 mg, 0.783 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 525-4.

### Step 4: synthesis of compound 525

Compound 525-4 (120 mg, 0.217 mmol), cesium carbonate (225 mg, 0.69 mmol), tBuXPhos Pd G4 (19 mg, 0.023 mmol) and 2-hydroxyethylsulfonamide (58 mg, 0.46 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 525. MS m/z (ESI):567.3 [M+1]⁺.

¹H NMR (400 MHz, CHLOROFORM-*d*) *δ* = 11.69 (s, 1H), 8.02 (d, *J* = 8.5 Hz, 1H), 7.73 (br s, 1H), 7.59 - 7.54 (m, 1H), 7.36 - 7.31 (m, 1H), 6.92 (br d, *J* = 8.5 Hz, 1H), 6.88 (br d, *J =* 7.9 Hz, 1H), 6.25 (s, 1H), 5.01 (s, 2H), 4.27 (s, 2H), 4.00 (br s, 2H), 3.79 - 3.62 (m, 1H), 3.22 - 3.17 (m, 2H), 3.07 (br t, *J =* 4.5 Hz, 4H), 2.72 (s, 3H), 1.49 (br s, 4H), 1.43 (s, 6H), 0.29 (s, 4H).

### Example 526:

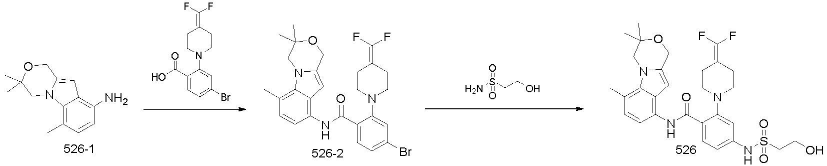

### Step 1: synthesis of compound 526-2

Compound 526-1 (60 mg, 0.26 mmol), 4-bromo-2-(4-(difluoromethylene)piperidin-1-yl)benzoic acid (88 mg, 0.26 mmol), HATU (137 mg, 0.391 mmol) and DIEA (101 mg, 0.783 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 526-2.

### Step 2: synthesis of compound 526

Compound 526-2 (110 mg, 0.202 mmol), cesium carbonate (198 mg, 0.61 mmol), tBuXPhos Pd G4 (16 mg, 0.02 mmol) and 2-hydroxyethylsulfonamide (51 mg, 0.405 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 526. MS m/z (ESI):589.2 [M+1]⁺.

¹H NMR (400 MHz, CHLOROFORM-d) *δ* = 11.40 (s, 1H), 7.94 (d, *J* = 8.5 Hz, 1H), 7.52 (d, *J =* 7.6 Hz, 1H), 7.25 (d, *J =* 1.3 Hz, 1H), 6.98 - 6.80 (m, 2H), 6.16 (s, 1H), 4.98 (s, 2H), 4.26 (s, 2H), 4.03 - 3.94 (m, 2H), 3.21 - 3.12 (m, 2H), 2.99 (br t, *J= 5.0* Hz, 4H), 2.71 (s, 3H), 2.25 (br s, 4H), 1.42 (s, 6H).

### Example 527:

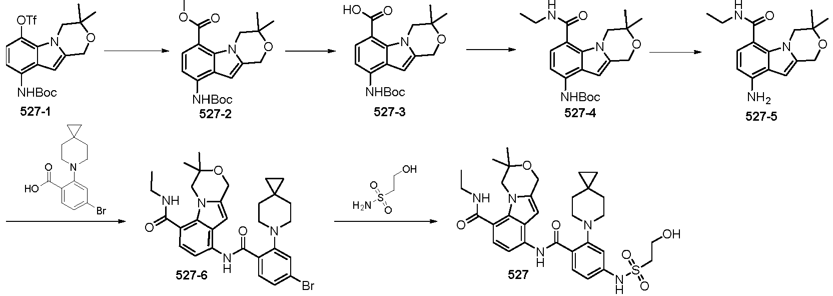

### Step 1: synthesis of compound 527-2

Compound 527-1 (200 mg, 0.43 mmol), palladium acetate (19 mg, 0.09 mmol), and DPPP (178 mg, 0.43 mmol) were dissolved in DMSO (3 mL) and MeOH (3 mL), and the reaction mixture was reacted overnight at 60°C under CO protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 527-2.

### Step 2: synthesis of compound 527-3

Compound 527-2 (125 mg, 0.33 mmol) was dissolved in MeOH/THF/H₂O=2: 2: 1 (3 mL), to which NaOH (54 mg, 1.32 mmol) was added, and the reaction mixture was reacted at 50°C for 1 hour. The reaction solution was quenched with ice water, the pH was adjusted to 7 with 2N HCl, and the reaction solution was extracted with EA. The organic phase was washed with saturated NaCl solution, dried over anhydrous Na₂SO₄, and concentrated under reduced pressure to obtain compound 527-3.

### Step 3: synthesis of compound 527-4

Compound 527-3 (115 mg, 0.32 mmol), ethylamine hydrochloride (130 mg, 1.60 mmol), and HATU (134 mg, 0.35 mmol) were dissolved in DMF (3 mL), to which DIEA (0.26 mL, 1.60 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=1:1) to obtain compound 527-4.

### Step 4: synthesis of compound 527-5

Compound 527-4 (88 mg, 023 mmol) was dissolved in DCM (2 mL), to which hydrogen chloride-ethyl acetate solution (1 mL) was added at 0°C, and the reaction mixture was allowed to react at 0°C for 1 hour. The reaction solution was filtered, the solid was neutralized, and the liquid separation was performed. The resulting liquid was dried, and concentrated, to obtain compound 527-5.

### Step 5: synthesis of compound 527-6

Compound 527-5 (30 mg, 0.09 mmol), 4-bromo-2-(6-azaspiro[2.5]oct-6-yl)benzoic acid (32 mg, 0.10 mmol), and HATU (39 mg, 0.10 mmol) were dissolved in DMF (3 mL), to which DIEA (0.06 mL, 0.46 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 527-6.

### Step 6: synthesis of compound 527

Compound 527-6 (40 mg, 0.07 mmol), 2-hydroxyethanesulfonamide (35 mg, 0.28 mmol), cesium carbonate (67 mg, 0.21 mmol) and the fourth-generation palladium catalyst (CAS: 1599466-89-3; 6 mg, 0.01 mmol) were dissolved in 1,4-dioxane (3 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 527. MS (ESI, pos.ion) m/z:624.0 [M+1]⁺.

¹H NMR (400 MHz, DMSO-*d*₆) δ = 11.24 (s, 1H), 10.28 - 9.94 (m, 1H), 8.51 (br t, *J* = 5.0 Hz, 1H), 7.94 (br d, *J =* 7.9 Hz, 1H), 7.86 (br d, *J =* 8.4 Hz, 1H), 7.19 (s, 1H), 7.11 (br d, *J* = 8.0 Hz, 1H), 7.04 (br d, *J* = 8.5 Hz, 1H), 6.61 (s, 1H), 4.97 (s, 2H), 3.93 (s, 2H), 3.77 (br t, *J =* 6.4 Hz, 2H), 3.39 - 3.35 (m, 2H), 3.01 (br s, 4H), 1.44 (br s, 4H), 1.28 (s, 6H), 1.26 - 1.21 (m, 2H), 1.15 (br t, *J* = 7.1 Hz, 3H), 0.26 (s, 4H).

### Example 528:

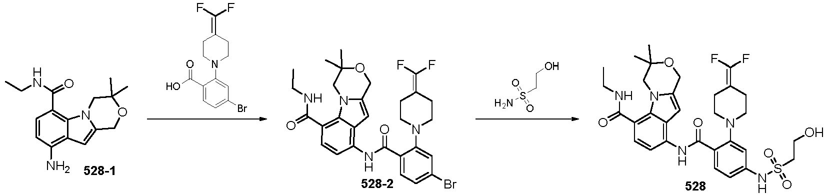

### Step 1: synthesis of compound 528-2

Compound 528-1 (30 mg, 0.09 mmol), 4-bromo-2-(4-(difluoromethylene)piperidin-1-yl)benzoic acid (33 mg, 0.10 mmol), and HATU (39 mg, 0.10 mmol) were dissolved in DMF (3 mL), to which DIEA (0.06 mL, 0.46 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 528-2.

### Step 2: synthesis of compound 528

Compound 528-2 (40 mg, 0.07 mmol), 2-hydroxyethanesulfonamide (36 mg, 0.29 mmol), cesium carbonate (70 mg, 0.21 mmol) and the fourth-generation palladium catalyst (CAS: 1599466-89-3; 6 mg, 0.01 mmol) were dissolved in 1,4-dioxane (3 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 528. MS (ESI, pos.ion) m/z:646.3 [M+1]⁺.

¹H NMR (400 MHz, DMSO-*d*₆) δ = 10.82 (s, 1H), 8.49 (br t, *J =* 5.4 Hz, 1H), 7.95 (br d, *J =* 7.9 Hz, 1H), 7.78 (d, *J =* 8.4 Hz, 1H), 7.14 - 7.09 (m, 1H), 7.09 - 7.06 (m, 1H), 7.02 (br d, *J =* 8.3 Hz, 1H), 6.62 (s, 1H), 4.95 (s, 2H), 3.92 (s, 2H), 3.77 (br t, *J* = 6.5 Hz, 2H), 3.31 - 3.27 (m, 2H), 3.02 (br s, 4H), 2.26 - 2.19 (m, 4H), 1.28 - 1.26 (m, 6H), 1.24 - 1.23 (m, 2H), 1.15 (t, *J =* 7.2 Hz, 3H).

### Example 529:

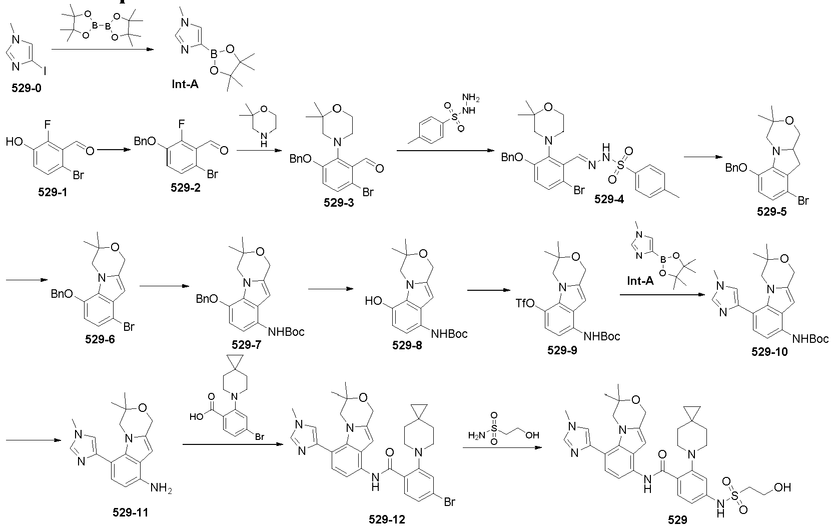

### Step 1: synthesis of compound Int-A

Compound 529-0 (500 mg, 2.40 mmol), bis(pinacolato)diboron (915.65 mg, 3.61 mmol), potassium acetate (471.83 mg, 4.81 mmol) and palladium catalyst (CAS: 72287-26-4; 178.31 mg, 0.24 mmol) were dissolved in 1,4-dioxane (10 mL), and the reaction mixture was reacted at 90°C for 18 h under N₂ protection. The reaction solution was cooled to room temperature and then poured into water, and extracted with EA. The organic phase was washed with saturated NaCl solution, dried over anhydrous Na₂SO₄, and concentrated under reduced pressure to obtain crude product Int-A.

### Step 2: synthesis of compound 529-2

Compound 529-1 ( 3 g, 13.70 mmol) and K₂CO₃ were dissolved in DMF (20 mL), to which benzyl bromide (2.46 mL, 20.55 mmol) was added dropwise at 45°C-55°C. The mixture was reacted at 50°C for 10 min. The reaction was quenched by pouring the reaction solution into ice water, and then extracted with EA. The organic phase was washed with saturated NaCl solution, dried over anhydrous Na₂SO₄, and concentrated under reduced pressure, and the crude product was slurried with PE and purified to obtain compound 529-2.

### Step 3: synthesis of compound 529-3

Compound 529-2 (3.48 g, 34.33 mmol) and 2,2-dimethyl-1,4-oxazine (1.56 g, 13.51 mmol) were dissolved in NMP (18 mL), NaHCO₃(1892 mg, 22.51 mmol) was then added, and the mixture was reacted at 95°C for 18 h. The reaction solution was quenched by pouring into ice water and then extracted with EA. The organic phase was washed with saturated sodium chloride aqueous solution, then dried over anhydrous Na₂SO₄, and concentrated under reduced pressure. The crude product was purified by silica gel column chromatography (PE:EA = 0-15%) to obtain compound 529-3.

### Step 4: synthesis of compound 529-4

Compound 529-3 (3.46 g, 8.56 mmol) and 4-methylbenzenesulfonyl hydrazide (1594 mg, 8.56 mmol) were dissolved in toluene (25 mL), and reacted at 25°C for 30 min. After the complete reaction of raw materials, the reaction solution containing compound 529-4 was obtained and used directly in the next reaction.

### Step 5: synthesis of compound 529-5

NaH (410.82 mg, 10.27 mmol, 60% purity) was added to the reaction solution containing the synthetic compound 529-4 at 0°C, and the reaction mixture was stirred at 25°C for 20 min. The mixture was then reacted at 135°C for 30 min. The reaction solution was quenched with water, and extracted with EA. The organic phase was dried over anhydrous Na₂SO₄, and concentrated under reduced pressure. The crude product was purified by silica gel column chromatography (PE:EA=0-5%) to obtain compound 529-5.

### Step 6: synthesis of compound 529-6

Compound 529-5 (1830 mg, 4.71 mmol) was dissolved in dichloromethane (20 mL). At 0°C, active manganese dioxide (4097 mg, 47.1 mmol) was added to the mixture, and the mixture was reacted at 25°C for 18 h. After monitoring the complete reaction of raw materials, the reaction solution was filtered through diatomaceous earth, the filter cake was rinsed several times with DCM, and the organic phase was concentrated under reduced pressure. The crude product was purified by silica gel column chromatography (PE:EA = 0-5%) to obtain compound 529-6.

### Step 7: synthesis of compound 529-7

Compound 529-6 (870 mg, 2.25 mmol), NH₂Boc (395.38 mg, 3.38 mmol), Cs₂CO₃ (1467 mg, 4.5 mmol) and palladium catalyst (CAS: 1470372-59-8; 204.39 mg, 0.23 mmol) were dissolved in 1,4-dioxane (10 mL), and the reaction mixture was reacted at 105°C for 18 h under N₂ protection. The reaction solution was poured into water, and extracted with EA. The organic phase was washed with saturated NaCl solution, dried over anhydrous Na₂SO₄, and concentrated under reduced pressure. The crude product was purified by silica gel column chromatography (PE:EA=0-15%) to obtain compound 529-7.

### Step 8: synthesis of compound 529-8

Compound 529-7 (629 mg, 1.49 mmol) was dissolved in THF (5 mL) and MeOH (5 mL), to which 10% wet palladium on carbon (300 mg, 0.24 mmol) was added and the reaction mixture was reacted at 25°C for 2h under hydrogen atmosphere at 15 psi. After monitoring the complete reaction of raw materials, the reaction solution was filtered through diatomaceous earth, the filter cake was rinsed several times with EA, and the organic phase was concentrated under reduced pressure. The crude product was purified by silica gel column chromatography (PE:EA = 0-20%) to obtain compound 529-8.

### Step 9: synthesis of compound 529-9

Compound 529-8 (520 mg, 1.56 mmol) and DIEA (606.59 mg, 4.69 mmol) were dissolved in DCM ( 5 mL), to which N-[dioxo(trifluoromethyl)-λ6-sulfanyl]-1,1,1-trifluoro-N-phenylmethanesulfonamide (1117.72 mg, 3.13 mmol) was added at 0°C, and the mixture was reacted at 25°C for 18 h. After treatment, the reaction solution was directly purified by column chromatography (PE:EA=0-15%) to obtain compound 529-9.

### Step 10: synthesis of compound 529-10

Compound 529-9 (500 mg, 1.08 mmol) and Int-A (2239.91 mg, 3.23 mmol, 30% purity) were dissolved in DMF (6 mL) and H₂O (1.5 mL), to which Cs₂CO₃ (2104.51 mg, 6.46 mmol) and palladium catalyst (CAS: 72287-26-4; 159.71 mg, 0.22 mmol) were added, and the reaction mixture was reacted at 90°C for 18 h under nitrogen protection. The reaction solution was poured into water, and extracted with EA. The organic phase was washed with saturated NaCl solution, dried over anhydrous Na₂SO₄, and concentrated under reduced pressure. The crude product was purified by silica gel column chromatography (DCM:MeOH=0-3%) to obtain compound 529-10.

### Step 11: synthesis of compound 529-11

Compound 529-10 (50 mg, 0.13 mmol) was dissolved in EA (1.5 mL), to which HCl\EA (1.5 mL, 4M) was added, and the reaction mixture was allowed to react at 25°C for 2 h. The reaction solution was directly subjected to rotary evaporation to dryness, and the solid was neutralized, extracted and dried, and concentrated to obtain compound 529-11. MS m/z (ESI):297.0 [M+1]⁺.

### Step 12: synthesis of compound 529-12

Compound 529-11 (37 mg, 0.12 mmol) and 2-(6-azaspiro[2.5]oct-6-yl)-4-bromobenzoic acid (48.39 mg, 0.16 mmol) were dissolved in DCM (3 mL), to which 2-(7-azabenzotriazole)-N,N,N',N'-tetramethyluronium hexafluorophosphate (71.21 mg, 0.19 mmol) and DIEA (80.68 mg, 0.62 mmol) were added, and the reaction mixture was allowed to react at 25°C for 3 h under N₂ protection. The reaction solution was quenched with water, and extracted with EA. The organic phase was washed with saturated NaCl solution, dried over anhydrous Na₂SO₄, and concentrated under reduced pressure. The crude product was purified by silica gel column chromatography (PE:EA=0-35%) to obtain compound 529-12.

### Step 13: synthesis of compound 529

Compound 529-12 (30 mg, 0.05 mmol), 2-hydroxyethanesulfonamide (12.76 mg, 0.10 mmol), potassium carbonate (14.09 mg, 0.10 mmol) and palladium catalyst (CAS: 1599466-89-3; 4.12 mg, 0.01 mmol) were dissolved in 1,4-dioxane (3 mL), and the reaction mixture was reacted at 90°C for 1.5 h under N₂ protection. The reaction solution was filtered, and then purified by preparative chromatography to obtain compound 529. MS m/z (ESI):633.2 [M+1]⁺.

¹H NMR (METHANOL-d4) δ: 8.03 (d, *J =* 8.5 Hz, 1H), 7.85 (d, *J =* 7.9 Hz, 1H), 7.74 (s, 1H), 7.30 (d, *J =* 1.9 Hz, 1H), 7.24 (s, 1H), 7.12 (dd, *J =* 8.6, 1.9 Hz, 1H), 7.05 (d, *J =* 7.9 Hz, 1H), 6.50 (s, 1H), 5.04 (s, 2H), 3.98 (t, *J =* 6.3 Hz, 2H), 3.85 (s, 3H), 3.68 (s, 2H), 3.35-3.44 (m, 2H), 3.10-3.19 (m, 4H), 1.55 (br s, 4H), 1.25 (s, 6H), 0.33 (s, 4H).

### Example 531: synthesis of 4-((2-hydroxyethyl)sulfonamido)-N-(6-isopropoxy-3,3-dimethyl-3,4-dihydro-1H-[1,4]oxazinyl[4,3-a]indol-9-yl)-2-(6-azaspiro[2.5]oct-6-yl)benzamide (531)

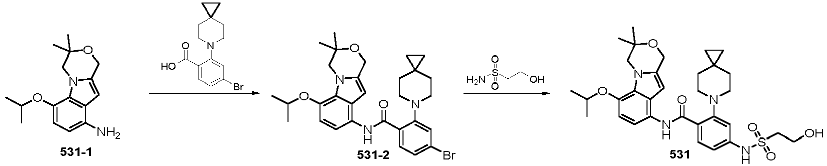

### Step 1: synthesis of compound 531-2

Compound 531-1 (100 mg, 0.28 mmol), HATU (139.63 mg, 0.37 mmol) and 2-(6-azaspiro[2.5]oct-6-yl)-4-bromobenzoic acid (105.15 mg, 0.34 mmol) were dissolved in DCM (5 mL) and THF (5 mL), to which DIEA (0.23 mL, 1.41 mmol) was added, and the reaction mixture was allowed to react at 25°C for 2 h. Water was added into the reaction solution, and extracted with DCM. The organic phase was washed with saturated NaCl solution, dried over anhydrous Na₂SO₄, and concentrated under reduced pressure. The crude product was purified by silica gel column chromatography (PE:EA=100/1-4/1) to obtain compound 531-2. MS m/z (ESI): 566.2\568.2 [M+1]⁺.

### Step 2: synthesis of compound 531

Compound 531-2 (180 mg, 0.32 mmol), 2-hydroxyethanesulfonamide (79.52 mg, 0.64 mmol), potassium carbonate (87.82 mg, 0.64 mmol) and palladium catalyst (CAS: 1599466-89-3; 25.68 mg, 0.03 mmol) were dissolved in 1,4-dioxane (5 mL), and the reaction mixture was reacted at 90°C for 1.5 h under N₂ protection. Water was added into the reaction solution, and extracted with EA. The organic phase was washed with saturated NaCl solution, dried over anhydrous Na₂SO₄, and concentrated under reduced pressure, and the crude product was purified by preparative chromatography to obtain compound 531. MS m/z (ESI):611.3 [M+1]⁺.

**¹H NMR** (CHLOROFORM-d) δ: 11.06 (s, 1H), 10.06 (br s, 1H), 7.86 (d, *J =* 8.4 Hz, 1H), 7.62 (d, *J =* 8.4 Hz, 1H), 7.18 (d, *J =* 1.8 Hz, 1H), 7.02 (dd, *J* = 8.4, 1.8 Hz, 1H), 6.65 (d, *J =* 8.4 Hz, 1H), 6.35 (s, 1H), 4.95 (s, 1H), 4.91 (s, 2H), 4.67 (dt, *J =* 12.0, 6.0 Hz, 1H), 4.26 (s, 2H), 3.76 (t, *J =* 6.5 Hz, 2H), 3.33-3.38 (m, 2H), 3.01 (br s, 4H), 1.46 (br s, 4H), 1.36 (s, 3H), 1.34 (s, 3H), 1.32 (s, 6H), 0.29 (s, 4H).

### Example 532: synthesis of 2-(4-(difluoromethylene)piperidin-1-yl)-4-((2-hydroxyethyl)sulfonamido)-N-(6-isopropoxy-3,3-dimethyl-3,4-dihydro-1H-[1,4]oxazinyl[4,3-a]indol-9-yl)benzamide (532)

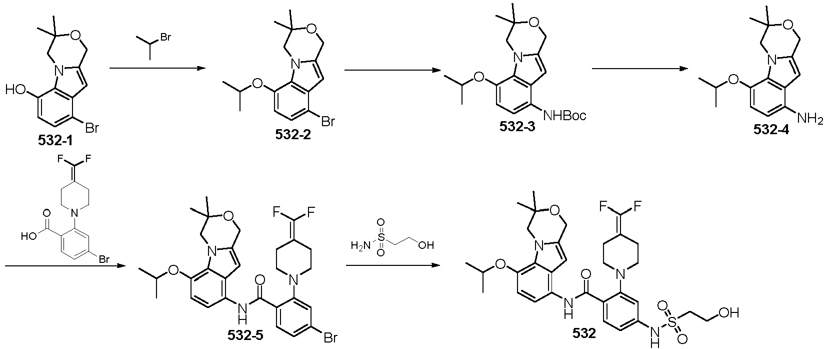

### Step 1: synthesis of compound 532-2

Compound 532-1 (350 mg, 1.18 mmol) and 2-bromopropane (0.22 mL, 2.36 mmol) were dissolved in DMF (5 mL), to which K₂CO₃ (489.97 mg, 3.55 mmol) was added, and the mixture was reacted at 60°C for 5 h. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-20/1) to obtain compound 532-2.

### Step 2: synthesis of compound 532-3

Compound 532-2 (327 mg, 0.97 mmol), and 2-methylprop-2-ylcarbamate (227.27 mg, 1.94 mmol) were dissolved in 1,4-dioxane (5 mL), to which Cs₂CO₃ (663.70 mg, 2.04 mmol) and palladium catalyst (CAS: 1470372-59-8; 88.03 mg, 0.10 mmol) were added, and the reaction mixture was reacted at 100°C for 5 h under N₂ protection. Water was added into the reaction solution, and extracted with EA. The organic phase was washed with saturated NaCl solution, dried over anhydrous Na₂SO₄, and concentrated under reduced pressure. The crude product was purified by silica gel column chromatography (PE:EA=100/1-6/1) to obtain compound 532-3. MS m/z (ESI): 375.2 [M+1]⁺.

### Step 3: synthesis of compound 532-4

Compound 532-3 (327 mg, 0.87 mmol) was dissolved in EA (4 mL), to which HCl\EA (8 mL, 4 M) was added, and the reaction mixture was allowed to react at 40°C for 2 h. The reaction solution was directly concentrated under reduced pressure, and the solid was neutralized, extracted and dried, and concentrated to obtain compound 532-4. MS m/z (ESI): 275.2 [M+1]⁺.

### Step 4: synthesis of compound 532-5

Compound 532-4 (90 mg, 0.25 mmol), HATU (125.67 mg, 0.33 mmol) and 4-bromo-2-[4-(difluoromethylene)hexahydropyridin-1-yl]benzoic acid (101.33 mg, 0.31 mmol) were dissolved in DCM(10 mL), to which DIEA (0.21 mL, 1.27 mmol) was added, and the reaction mixture was allowed to react at 25°C for 2 h. Water was added into the reaction solution, and extracted with DCM. The organic phase was washed with saturated NaCl solution, dried over anhydrous Na₂SO₄, and concentrated under reduced pressure. The crude product was purified by silica gel column chromatography (PE:EA=100/1-4/1) to obtain compound 532-5.

### Step 5: synthesis of compound 532

Compound 532-5 (172 mg, 0.29 mmol), 2-hydroxyethanesulfonamide (73.15 mg, 0.58 mmol), potassium carbonate (80.78 mg, 0.58 mmol) and palladium catalyst (CAS: 1599466-89-3; 23.63 mg, 0.03 mmol) were dissolved in 1,4-dioxane (5 mL), and the reaction mixture was reacted at 90°C for 1.5 h under N₂ protection. Water was added to the reaction solution, and extracted with EA. The organic phase was washed with saturated NaCl solution, dried over anhydrous Na₂SO₄, and concentrated under reduced pressure, and the crude product was purified by preparative chromatography to obtain compound 532. MS m/z (ESI):633.3 [M+1]⁺.

**¹H NMR** (CHLOROFORM-d) δ: 10.60 (s, 1H), 10.06 (s, 1H), 7.77 (d, *J =* 8.4 Hz, 1H), 7.64 (d, *J =* 8.4 Hz, 1H), 7.07 (s, 1H), 7.01 (dd, *J =* 8.5, 1.8 Hz, 1H), 6.65 (d, *J= 8.6* Hz, 1H), 6.36 (s, 1H), 4.95 (t, *J =* 5.6 Hz, 1H), 4.90 (s, 2H), 4.66 (dt, *J =* 12.1, 6.0 Hz, 1H), 4.24 (s, 2H), 3.76 (q, *J =* 6.2 Hz, 2H), 3.33-3.37 (m, 2H), 3.01 (br t, *J =* 5.3 Hz, 4H), 2.20-2.29 (m, 4H), 1.36 (s, 3H), 1.34 (s, 3H), 1.32 (s, 6H).

### Example 535: synthesis of N-(6-ethoxy-3,3-dimethyl-3,4-dihydro-1H-[1,4] oxazino [4,3-a] indol-9-yl)-4-((2-hydroxyethyl)sulfonamido)-2-(6-azaspiro[2.5]octan-6-yl)benzamide (535)

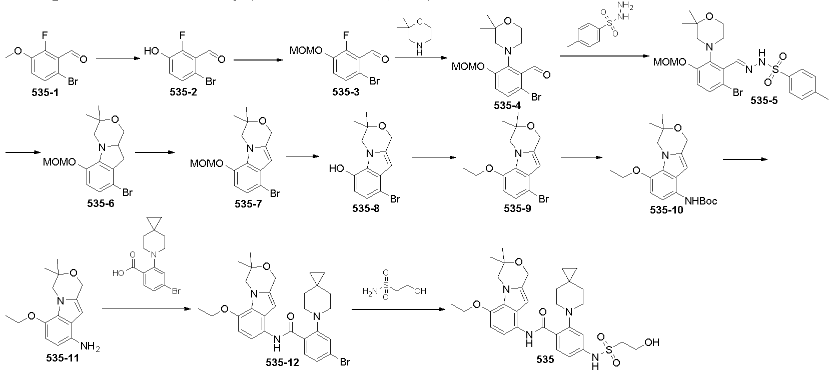

### Step 1: synthesis of compound 535-2

Compound 535-1 (10 g, 43.3 mmol) was dissolved in dichloromethane (50 mL), and BBr₃ (120 mL, 130 mmol) was slowly added at 0 °C, and stirred at 0 °C for 10 minutes. The temperature was raised to room temperature and the reaction was allowed to proceed for 2 hours. The reaction solution was quenched with ice water, and extracted with dichloromethane. The organic phase was washed with saturated NaCl solution, dried over anhydrous Na₂SO₄, and concentrated under reduced pressure to obtain compound 535-2.

### Step 2: synthesis of compound 535-3

The crude product 535-2 (9.3 g, 42.5 mmol) and potassium carbonate (17.7 g, 127.5 mmol) were dissolved in N,N-dimethylformamide (80 mL). Bromomethyl methyl ether (5.2 mL, 63.75 mmol) was slowly added dropwise at 50°C under nitrogen atmosphere, and reaction was carried out at 50°C for 10 minutes after addition was completed. The reaction solution was quenched with ice water, and extracted with ethyl acetate. The organic phase was washed with saturated NaCl solution, dried over anhydrous Na₂SO₄, and concentrated under reduced pressure to obtain compound 535-3.

### Step 3: synthesis of compound 535-4

Compound 535-3 (11 g, 42.1 mmol) and sodium bicarbonate (7.1 g, 84.2 mmol) were dissolved in N-methylpyrrolidinone (40 mL), to which 2,2-dimethylmorpholine (6.7 mL, 50.6 mmol) was added, and the reaction mixture was allowed to react at 95°C overnight. The reaction solution was quenched with ice water, and extracted with ethyl acetate. The organic phase was washed with saturated NaCl solution, dried over anhydrous Na₂SO₄, and concentrated under reduced pressure. The crude product was purified by silica gel column chromatography (PE:EA=20/1-10/1) to obtain compound 535-4.

### Step 4: synthesis of compound 535-5

Compound 535-4 (12.4 g, 34.6 mmol) was dissolved in toluene (50 mL), to which p-toluenesulfonyl hydrazide (7.7 g, 41.6 mmol) was added. The mixture was stirred at 25°C for 30 minutes to obtain the reaction solution containing compound 535-5.

### Step 5: synthesis of compound 535-6

To the reaction solution containing compound 535-5 (18.2 g, 34.6 mmol) was slowly added sodium hydride (1.7 g, 41.5 mmol), stirred at room temperature for 10 minutes, and then the reaction temperature was raised to 130°C and the mixture was reacted for half an hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 535-6.

### Step 6: synthesis of compound 535-7

Compound 535-6 (3.2 g, 9.36 mmol) was dissolved in dichloromethane (20 mL), to which manganese dioxide (4.1 g, 46.8 mmol) was slowly added, and the mixture was reacted overnight at room temperature. The reaction solution was filtered, and the filtrate was collected, and concentrated under reduced pressure. The crude product was purified by silica gel column chromatography (PE:EA=20/1-10/1) to obtain compound 535-7.

### Step 7: synthesis of compound 535-8

Compound 535-7 (500 mg, 1.47 mmol) was dissolved in anhydrous ethanol (6 mL), and stirred at 0°C for 15 minutes, to which HCl/EtOH (6 mL, 60 mmol) was added, and the reaction mixture was allowed to react at 0°C for 2 hours. The reaction solution was quenched with saturated sodium bicarbonate solution, and extracted with EA. The organic phase was washed with saturated NaCl solution, dried over anhydrous Na₂SO₄, and concentrated under reduced pressure to obtain compound 535-8.

### Step 8: synthesis of compound 535-9

Compound 535-8 (435 mg, 1.47 mmol), and potassium carbonate (579.6 mg, 4.2 mmol) were dissolved in DMF (4 mL), to which bromoethane (1.3 mL, 14.7 mmol) was added, and the reaction mixture was reacted at 60°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=30/1-20/1) to obtain compound 535-9.

### Step 9: synthesis of compound 535-10

Compound 535-9 (273 mg, 0.84 mmol) and Cs₂CO₃ (832 mg, 2.52 mmol) were dissolved in 1,4-dioxane (4 mL), to which tert-butyl carbamate (198 mg, 1.7 mmol), and palladium catalyst (CAS: 1599466-89-3; 68.2 mg, 0.08 mmol), and the reaction mixture was reacted at 90°C for 2 hours under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 535-10.

### Step 10: synthesis of compound 535-11

Compound 535-10 (278 mg, 0.77 mmol) was dissolved in dichloromethane (4 mL), to which HCl/EA (4 mL) was added, and the mixture was stirred at 25°C for 1 hour. The solvent was removed by concentration under reduced pressure, and solids were neutralized. The reaction system was subjected to extraction, drying and concentration to obtain compound 535-11, which was used directly in the next reaction.

### Step 11: synthesis of compound 535-12

Compound 535-11 (110 mg, 0.39 mmol), HATU (219 mg, 0.59 mmol), 4-bromo-2-(6-azaspiro[2.5]octan-6-yl)benzoic acid (119 mg, 0.39 mmol) were dissolved in DMF (5 mL), to which DIEA (207 uL, 1.17 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 535-12.

### Step 12: synthesis of compound 535

Compound 535-12 (108 mg, 0.2 mmol), 2-hydroxylethanesulfonamide (49 mg, 0.4 mmol), cesium carbonate (266 mg, 0.81 mmol) and palladium catalyst (CAS: 1599466-85-0; 17 mg, 0.02 mmol) were dissolved in 1,4-dioxane (4 mL), and the reaction mixture was reacted at 90°C for 4 hours under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20/1-10/1) to obtain compound 535. MS m/z (ESI):597.3 [M+1]⁺.

**¹H NMR (400 MHz, CHLOROFORM-d)** δ = 11.46 (s, 1H), 8.13 (d, *J* = 8.4 Hz, 1H), 7.61 (br d, *J* = 8.3 Hz, 1H), 7.41 - 7.29 (m, 1H), 7.20 (br s, 1H), 7.00 (br d, *J* = 8.3 Hz, 1H), 6.59 (d, *J =* 8.4 Hz, 1H), 6.23 (s, 1H), 5.01 (s, 2H), 4.35 (s, 2H), 4.18 (q, *J =* 7.0 Hz, 2H), 4.10 (br s, 2H), 3.31 (br t, *J =* 4.9 Hz, 2H), 3.12 (br s, 4H), 1.58 - 1.48 (m, 7H), 1.43 (s, 6H), 0.33 (s, 4H).

### Example 536: synthesis of 2-(4-(difluoromethylene)piperidin-1-yl)-N-(6-ethoxy-3,3-dimethyl-3,4-dihydro-1H-[1,4]oxazino[4,3-a]indol-9-yl)-4-((2-hydroxyethyl)sulfonamido)benzamide (536)

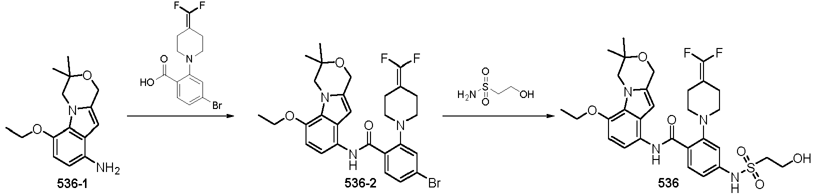

### Step 1: synthesis of compound 536-2

Compound 536-1 (110 mg, 0.39 mmol), HATU (219 mg, 0.59 mmol), 4-bromo-2-(4-(difluoromethylene)piperidin-1-yl)benzoic acid (128 mg, 0.39 mmol) were dissolved in DMF (5 mL), to which DIEA (207 uL, 1.17 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 536-2.

### Step 2: synthesis of compound 536

Compound 536-2 (190 mg, 0.33 mmol), 2-hydroxylethanesulfonamide (83 mg, 0.66 mmol), cesium carbonate (325 mg, 0.99 mmol) and palladium catalyst (CAS: 1599466-85-0; 28 mg, 0.03 mmol) were dissolved in 1,4-dioxane (4 mL), and the reaction mixture was reacted at 90°C for 4 hours under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20/1-10/1) to obtain compound 536.

MS m/z (ESI):619.3 [M+1]⁺.

**¹H NMR (400 MHz, CHLOROFORM-d)** δ = 11.01 (br s, 1H), 8.01 (d, *J* = 8.4 Hz, 1H), 7.53 (br d, *J* = 7.4 Hz, 1H), 7.16 (br d, *J =* 11.6 Hz, 2H), 6.95 - 6.84 (m, 1H), 6.50 (d, *J* = 8.4 Hz, 1H), 6.06 (s, 1H), 4.90 (s, 2H), 4.25 (s, 2H), 4.11 - 4.06 (m, 2H), 4.00 (br t, *J =* 4.8 Hz, 2H), 3.22 - 3.17 (m, 2H), 3.00 (br s, 4H), 2.26 (br s, 4H), 1.42 - 1.38 (m, 3H), 1.33 (s, 6H).

### Example 565:

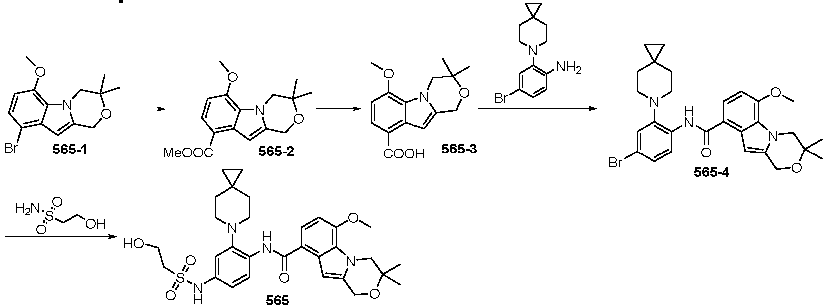

### Step 1: synthesis of compound 565-2

Compound 565-1 (400 mg, 1.29 mmol), [1,1'-bis(diphenylphosphino)ferrocene]palladium dichloride (187.15 mg, 0.26 mmol) and triethylamine (1.87 g, 2.58 mmol) were dissolved in dimethyl sulfoxide (4 mL) and methanol (4 mL), and carbon monoxide gas was introduced into the reaction. The reaction mixture was stirred overnight in an oil bath at 75°C. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=100:0-90:10) to obtain compound 565-2.

### Step 2: synthesis of compound 565-3

Compound 565-2 (88 mg, 0.30 mmol) was dissolved in methanol (4 mL) and water (1 mL), to which sodium hydroxide (73 mg, 1.82 mmol) was added. The reaction mixture was stirred in an oil bath at 60°C for 3 hours. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by silica gel column chromatography (PE:EA=100:0-80:20) to obtain compound 565-3.

### Step 3: synthesis of compound 565-4

Compound 565-3 (33 mg, 0.12 mmol) and 4-bromo-2-(6-azaspiro[2.5]octan-6-yl)benzoic acid (40.45 mg, 0.14 mmol) were dissolved in acetonitrile (1 mL), to which N,N,N',N'-tetramethylchloroformamidinium hexafluorophosphate (67.27 mg, 0.24 mmol) and N,N-diisopropylethylamine (61.97 mg, 0.48 mmol) were added. The reaction mixture was stirred at room temperature for 1 hour. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by silica gel column chromatography (PE:EA=100:0-90:10) to obtain compound 565-4.

### Step 8: synthesis of compound 565

Compound 565-4 (40 mg, 0.07 mmol) and 2-hydroxyethanesulfonamide (37.18 mg, 0.30 mmol) were dissolved in 1,4-dioxane (2 mL). To this mixture was added palladium catalyst (CAS: 1599466-83-7; 3.42 mg) and cesium carbonate (48.40 mg, 0.15 mmol). The reaction mixture was stirred in an oil bath at 95°C for 1.5 hours. After the reaction was completed, the reaction solution was diluted with water (60 mL), and extracted with ethyl acetate (30 × 3 mL). The organic layer was washed with brine (30×3 mL), dried over Na₂SO₄, filtered and concentrated. The crude product was purified by preparative chromatography to obtain compound 565. MS (ESI, pos.ion) m/z: 583.0 [M+1]⁺.

¹H NMR (400 MHz, DMSO-d6) δ = 9.40 (s, 1H), 8.34 (d, *J* = 8.8 Hz, 1H), 7.61 (d, *J* = 8.3 Hz, 1H), 7.17 (s, 1H), 7.06 - 6.97 (m, 1H), 6.89 - 6.78 (m, 2H), 5.00 (s, 2H), 4.32 (s, 2H), 3.97 (s, 3H), 3.75(t, *J =* 6.8 Hz, 2H), 3.21 (t, *J =* 6.8 Hz, 2H), 2.90 - 2.78 (m, 4H), 1.58 - 1.42 (m, 4H), 1.34 (s, 6H), 0.34 (s, 4H).

### Example 566:

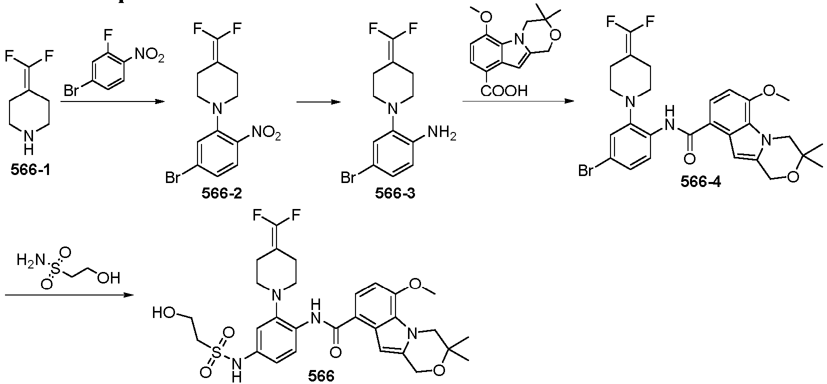

### Step 1: synthesis of compound 566-2

Compound 566-1 (92.51 mg, 0.55 mmol), 4-bromo-2-fluoro-1-nitrobenzene (100 mg, 0.45 mmol) and cesium carbonate (296.20 mg, 0.91 mmol) were dissolved in N,N-dimethylformamide (3 mL). The reaction mixture was stirred in an oil bath at 110°C for 1 hour. The reaction solution was diluted with water, and extracted with EA. The organic phase was washed with saturated NaCl solution, dried over anhydrous Na₂SO₄, and concentrated under reduced pressure. The crude product was purified by silica gel column chromatography (PE:EA=100:0-95:5) to obtain compound 566-2.

### Step 2: synthesis of compound 566-3

Compound 566-2 (120 mg, 0.36 mmol) was dissolved in ethanol (2 mL) and water (2 mL), to which iron dust (100.57 mg, 1.80 mmol) and ammonium chloride (96.34 mg, 1.80 mmol) were added. The reaction mixture was stirred in an oil bath at 100°C for 1 hour. After the reaction was completed, the reaction solution was filtered and the filtrate was diluted with water (60 mL), and extracted with ethyl acetate (30×3 mL). The organic layer was washed with brine (30×3 mL), dried over Na₂SO₄, filtered and concentrated. The crude product was purified by silica gel column chromatography (PE:EA=100:0-90:10) to obtain compound 565-3.

### Step 3: synthesis of compound 566-4

Compound 566-3 (62.99 mg, 0.21 mmol) and 6-methoxy-3,3-dimethyl-3,4-dihydro-1H-[1,4]oxazino[4,3-a]indole-9-carboxylic acid (52 mg, 0.19 mmol) were dissolved in acetonitrile (3 mL), to which N,N,N',N'-tetramethylchloroformamidine hexafluorophosphate (121.89 mg, 0.43 mmol) and N,N-diisopropylethylamine (97.65 mg, 0.76 mmol) were added. The reaction mixture was stirred at room temperature for 1 hour. After the reaction was completed, the reaction solution was diluted with water (60 mL), and extracted with ethyl acetate (30×3 mL). The organic layer was washed with brine (30×3 mL), dried over Na₂SO₄, filtered and concentrated. The crude product was purified by silica gel column chromatography (PE:EA=100:0-90:10) to obtain compound 566-4.

### Step 4: synthesis of compound 566

Compound 566-4 (39 mg, 0.07 mmol) and 2-hydroxyethanesulfonamide (34.83 mg, 0.28 mmol) were dissolved in 1,4-dioxane (2 mL). To this mixture was added palladium catalyst (CAS: 1599466-83-7; 12.81 mg, 0.01 mmol) and cesium carbonate (45.35 mg, 0.14 mmol). The reaction mixture was stirred in an oil bath at 95°C for 1.5 hours. After the reaction was completed, the reaction solution was diluted with water (60 mL), and extracted with ethyl acetate (30×3 mL). The organic layer was washed with brine (30×3 mL), dried over Na₂SO₄, filtered and concentrated. The crude product was purified by preparative chromatography to obtain compound 566. MS m/z (ESI):605.2 [M+1]⁺.

¹H NMR (400 MHz, DMSO-*d*₆) δ = 9.55 (s, 1H), 9.31(s, 1H), 8.26 (d, *J =* 8.8 Hz, 1H), 7.60 (d, *J* = 8.3 Hz, 1H), 7.11 (d, *J =* 2.3 Hz, 1H), 7.02 (d, *J =* 6.4 Hz, 1H), 6.85 - 6.78 (m, 2H), 4.96 (s, 2H), 4.30 (s, 2H), 3.96 (s, 3H), 3.75 (t, *J =* 6.5 Hz, 2H), 3.21 (t, *J =* 6.7 Hz, 2H), 2.86 - 2.82 (m, 4H), 2.33 - 2.25 (m, 4H), 1.32 (s, 6H).

### Example 573:

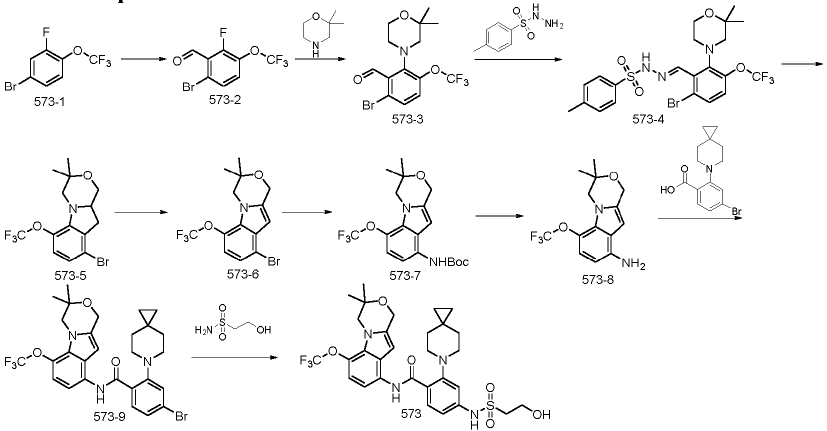

### Step 1: synthesis of compound 573-2

Compound 573-1 (25 g, 96.5 mmol) was dissolved in THF (200 ml), and LDA (72.4 ml, 144.79 mmol) was added dropwise at -78°C under nitrogen. The reaction solution was stirred at -78°C for one hour, and then DMF (11.2 ml, 144.79 mmol) was added dropwise under equivalent conditions, and stirring was continued for one hour. After the reaction was completed, the reaction was quenched with saturated ammonium chloride solution, and extracted three times with ethyl acetate, and the organic phase was washed three times with saturated sodium chloride aqueous solution. The resulting reaction solution was dried over anhydrous sodium sulfate, concentrated and purified by column chromatography (PE:EA=1/20-1/10) to obtain compound 573-2.

### Step 2: synthesis of compound 573-3

Compound 573-2 (6 g, 20.9 mmol), DIEA (11.2 ml, 62.7 mmol) and 2,2-dimethylmorpholine (4.8 g, 41.8 mmol) were dissolved in NMP (60 ml), and the reaction mixture was stirred at 150°C for 3 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/15-1/8) to obtain compound 573-3.

### Step 3: synthesis of compound 573-4

The compound 573-3 (5.2 g, 13.6 mmol) and p-toluenesulfonyl hydrazide (2.79 g, 14.97 mmol) were dissolved in toluene (50 ml) and stirred at room temperature for 1 hour. After monitoring the completion of the reaction, the reaction solution containing compound 573-4 was obtained and used directly in the next step without treatments.

### Step 4: synthesis of compound 573-5

To the reaction solution from the previous step was added sodium hydride (653 mg, 16.34 mmol), and the mixture was stirred at room temperature for 10 minutes, followed by stirring at 135°C for half an hour. After monitoring the completion of the reaction, the reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/7) to obtain compound 573-5.

### Step 5: synthesis of compound 573-6

The compound 573-5 (2.2 g, 6.01 mmol) and manganese dioxide (5.23 g, 60.1 mmol) were dissolved in dichloromethane (30 ml) and stirred at room temperature for 16 hours. After monitoring the completion of the reaction, manganese dioxide was filtered off, and the filtrate was concentrated and purified by silica gel column chromatography (PE:EA=1/15-1/10) to obtain compound 573-6.

### Step 6: synthesis of compound 573-7

Compound 573-6 (570 mg, 1.57 mmol), cesium carbonate (1.53 g, 4.7 mmol), tBuXPhos Pd G4 (127 mg, 0.157 mmol) and tert-butyl carbamate (391 mg, 3.13 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/10-1/7) to obtain compound 573-7.

### Step 7: synthesis of compound 573-8

The compound 573-7 (300 mg, 0.75 mmol) and hydrogen chloride-ethyl acetate solution (1.9 ml, 4N) were dissolved in dichloromethane (5 ml) and stirred at room temperature for 2 hours. A solid was precipitated out from the reaction solution, concentrated and neutralized, extracted, dried, and then concentrated to obtain compound 573-8.

### Step 8: synthesis of compound 573-9

Compound 573-8 (100 mg, 0.33 mmol), 4-bromo-2-(6-azaspiro[2.5]oct-6-yl)benzoic acid (103 mg, 0.33 mmol), HATU (190 mg, 0.5 mmol) and DIEA (0.18 ml, 1.0 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 573-9.

### Step 9: synthesis of compound 573

Compound 573-9 (180 mg, 0.304 mmol), cesium carbonate (297 mg, 0.912 mmol), tBuXPhos Pd G4 (25 mg, 0.03 mmol) and 2-hydroxyethylsulfonamide (76 mg, 0.608 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 573. MS m/z (ESI):637.2 [M+1]⁺.

¹H NMR (400 MHz, CHLOROFORM-d) *δ* = 11.68 (s, 1H), 8.13 (d, *J =* 8.4 Hz, 1H), 7.77 (d, *J =* 8.5 Hz, 1H), 7.33 (d, *J =* 1.9 Hz, 1H), 7.26 - 7.19 (m, 1H), 7.06 (dd, *J =* 1.4, 8.5 Hz, 1H), 6.99 (dd, *J =* 1.9, 8.5 Hz, 1H), 6.33 (s, 1H), 5.02 (s, 2H), 4.17 (s, 2H), 4.09 (br d, *J =* 4.0 Hz, 2H), 3.35 - 3.27 (m, 2H), 3.11 (br t, *J =* 4.9 Hz, 4H), 2.96 (br s, 1H), 1.60 - 1.48 (m, 4H), 1.43 (s, 6H), 0.33 (s, 4H).

### Example 574:

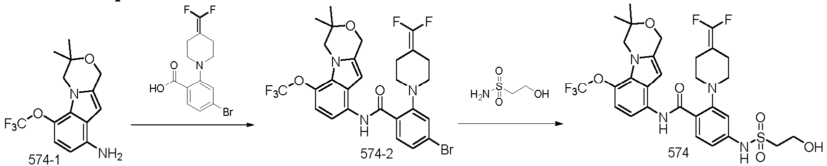

### Step 1: synthesis of compound 574-2

Compound 574-1 (100 mg, 0.33 mmol), 4-bromo-2-(4-(difluoromethylene)piperidin-1-yl)benzoic acid (110 mg, 0.33 mmol), HATU (190 mg, 0.5 mmol) and DIEA (0.18 ml, 1.0 mmol) were dissolved in DMF (5 ml), and the mixture was stirred at room temperature for 5 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/8-1/5) to obtain compound 574-2.

### Step 2: synthesis of compound 574

Compound 574-2 (150 mg, 0.304 mmol), cesium carbonate (239 mg, 0.733 mmol), tBuXPhos Pd G4 (20 mg, 0.024 mmol) and 2-hydroxyethylsulfonamide (61 mg, 0.489 mmol) were dissolved in 1,4-dioxane (10 ml), and the mixture was stirred at 80°C for 16 hours. The reaction solution was poured into ice water, and extracted with ethyl acetate. The organic phase was washed with saturated sodium chloride aqueous solution, dried over anhydrous sodium sulfate, and concentrated. The crude product was purified by silica gel column chromatography (PE:EA=1/3-1/1) to obtain compound 574. MS m/z (ESI):659.2 [M+1]⁺.

¹H NMR (400 MHz, CHLOROFORM-d) *δ* = 11.33 (s, 1H), 8.09 (d, *J* = 8.5 Hz, 1H), 7.77 (d, *J =* 8.6 Hz, 1H), 7.44 (s, 1H), 7.24 (s, 1H), 7.05 (dd, *J =* 1.4, 8.4 Hz, 1H), 6.99 (dd, *J=* 1.9, 8.5 Hz, 1H), 6.24 (s, 1H), 5.01 (s, 2H), 4.16 (s, 2H), 4.08 (br s, 2H), 3.34 - 3.25 (m, 2H), 3.07 (br t, *J =* 5.4 Hz, 5H), 2.33 (br s, 4H), 1.43 (s, 6H).

### Example 575:

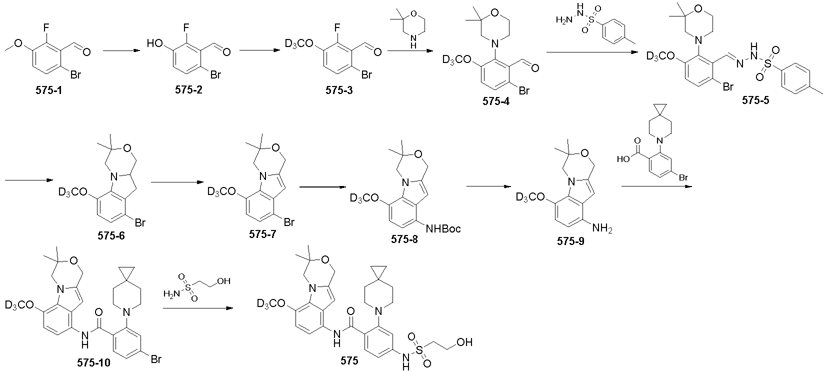

### Step 1: synthesis of compound 575-2

Compound 575-1 (25 g, 107.3 mmol) was dissolved in dichloromethane (100 mL), and BBr₃ (161 mL, 321.8 mmol) was slowly added at 0°C, and stirred at 0°C for 10 minutes. The temperature was raised to room temperature and the reaction was allowed to proceed for 2 hours. The reaction solution was quenched with ice water, and extracted with dichloromethane. The organic phase was washed with saturated NaCl solution, dried over anhydrous Na₂SO₄, and concentrated under reduced pressure to obtain compound 575-2.

### Step 2: synthesis of compound 575-3

Compound 575-2 (6 g, 27.4 mmol) and potassium carbonate (11.3 g, 82.2 mmol) were dissolved in N,N-dimethylformamide (20 mL), to which deuterated iodomethane (2.1 mL, 32.9 mmol) was added, and the mixture was reacted at 50°C for 1 hour. The reaction solution was quenched with ice water, and extracted with ethyl acetate. The organic phase was washed with saturated NaCl solution, dried over anhydrous Na₂SO₄, and concentrated under reduced pressure to obtain compound 575-3.

### Step 3: synthesis of compound 575-4

Compound 575-3 (3 g, 12.8 mmol), and N,N-diisopropylethylamine (6.3 g, 38.4 mmol) were dissolved in N-methylpyrrolidinone (10 mL), to which 2,2-dimethylmorpholine (2.1 mL, 15.4 mmol) was added, and the reaction mixture was allowed to react at 100°C overnight. The reaction solution was quenched with ice water, and extracted with ethyl acetate. The organic phase was washed with saturated NaCl solution, dried over anhydrous Na₂SO₄, and concentrated under reduced pressure. The crude product was purified by silica gel column chromatography (PE:EA=20/1-10/1) to obtain compound 575-4.

### Step 4: synthesis of compound 575-5

Compound 575-4 (1.9 g, 5.8 mmol) was dissolved in toluene (10 mL), to which p-toluenesulfonyl hydrazide (1.3 g, 7.0 mmol) was added. The mixture was stirred at 25°C for 30 minutes to obtain the reaction solution containing compound 575-5, which was used without further post-treatments.

### Step 5: synthesis of compound 575-6

To the reaction solution containing compound 575-5 (2.9 g, 5.8 mmol) was slowly added sodium hydride (280 g, 7.0 mmol), stirred at room temperature for 10 minutes, and then the reaction temperature was raised to 135°C and the mixture was reacted for half an hour. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 575-6.

### Step 6: synthesis of compound 575-7

Compound 575-6 (757 mg, 2.4 mmol) was dissolved in dichloromethane (5 mL), to which manganese dioxide (1.9 g, 24 mmol) was slowly added, and the mixture was reacted overnight at room temperature. The reaction solution was filtered, and the filtrate was collected, and concentrated under reduced pressure. The crude product was purified by silica gel column chromatography (PE:EA=20/1-10/1) to obtain compound 575-7.

### Step 7: synthesis of compound 575-8

Compound 575-7 (239 mg, 0.76 mmol) and Cs₂CO₃ (748 mg, 2.28 mmol) were dissolved in 1,4-dioxane (4 mL), to which tert-butyl carbamate (179 mg, 1.5 mmol), and palladium catalyst (CAS: 1599466-89-3; 61 mg, 0.08 mmol), and the reaction mixture was reacted at 100°C for 2 hours under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20/1-10/1) to obtain compound 575-8.

### Step 10: synthesis of compound 575-9

Compound 575-8 (260 mg, 0.74 mmol) was dissolved in dichloromethane (4 mL), to which HCl/EA (4 mL) was added, and the mixture was stirred at 25°C for 1 hour. The solvent was removed by concentration under reduced pressure, and the residue was redissolved and neutralized. The reaction system was subjected to extraction, drying and concentration to obtain compound 575-9, which was used directly in the next reaction without further purification.

### Step 11: synthesis of compound 575-10

Compound 575-9 (90 mg, 0.36 mmol), HATU (205 mg, 0.54 mmol), 4-bromo-2-(6-azaspiro[2.5]octan-6-yl)benzoic acid (111 mg, 0.36 mmol) were dissolved in DMF (5 mL), to which DIEA (194 uL, 1.08 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 575-10.

### Step 12: synthesis of compound 575

Compound 575-10 (122 mg, 0.21 mmol), 2-hydroxylethanesulfonamide (54 mg, 0.43 mmol), cesium carbonate (213 mg, 0.63 mmol) and palladium catalyst (CAS: 1599466-85-0; 18 mg, 0.02 mmol) were dissolved in 1,4-dioxane (4 mL), and the reaction mixture was reacted at 100°C for 4 hours under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20/1-10/1) to obtain compound 575.

MS m/z (ESI):586.0 [M+1]⁺.

¹H NMR (400 MHz, DMSO-d6) δ = 11.06 (s, 1H), 10.40 - 9.60 (m, 1H), 7.86 (d, *J* = 8.5 Hz, 1H), 7.64 (d, *J =* 8.4 Hz, 1H), 7.21 - 7.15 (m, 1H), 7.03 (dd, *J =* 1.6, 8.5 Hz, 1H), 6.62 (d, *J =* 8.4 Hz, 1H), 6.37 (s, 1H), 4.92 (s, 2H), 4.26 (s, 2H), 3.77 (t, *J =* 6.5 Hz, 2H), 3.38 - 3.34 (m, 2H), 3.01 (br s, 4H), 1.45 (br s, 4H), 1.33 (s, 6H), 0.28 (s, 4H).

### Example 576:

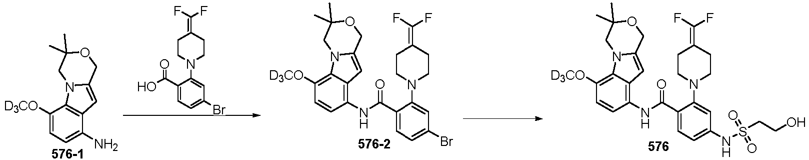

### Step 1: synthesis of compound 576-2

Compound 576-1 (90 mg, 0.36 mmol), HATU (205 mg, 0.54 mmol), 4-bromo-2-(4-(difluoromethylene)piperidin-1-yl)benzoic acid (119 mg, 0.36 mmol) were dissolved in DMF (5 mL), to which DIEA (194 uL, 1.08 mmol) was added, and the reaction mixture was stirred at 25°C for 2 hours. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10/1-5/1) to obtain compound 576-2.

### Step 2: synthesis of compound 576

Compound 576-2 (93 mg, 0.17 mmol), 2-hydroxylethanesulfonamide (43 mg, 0.34 mmol), cesium carbonate (213 mg, 0.51 mmol) and palladium catalyst (CAS: 1599466-85-0; 14 mg, 0.17 mmol) were dissolved in 1,4-dioxane (4 mL), and the reaction mixture was reacted at 100°C for 4 hours under N₂ protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (DCM:MeOH=20/1-10/1) to obtain compound 576.

MS m/z (ESI): 608.0 [M+1]⁺.

¹H NMR (400 MHz, DMSO-d6) δ = 10.60 (s, 1H), 10.06 (br s, 1H), 7.77 (d, *J* = 8.4 Hz, 1H), 7.66 (d, *J =* 8.4 Hz, 1H), 7.07(d, *J =* 1.8 Hz, 1H), 7.01 (dd, *J =* 1.9, 8.4 Hz, 1H), 6.61 (d, *J =* 8.4 Hz, 1H), 6.38 (s, 1H), 5.05 - 4.93 (m, 1H), 4.91 (s, 2H), 4.24 (s, 2H), 3.77 (br t, *J =* 6.3 Hz, 2H), 3.36 - 3.33 (m, 2H), 3.01 (br t, *J =* 5.2 Hz, 4H), 2.24 (br s, 4H), 1.32 (s, 6H).

### Example 582:

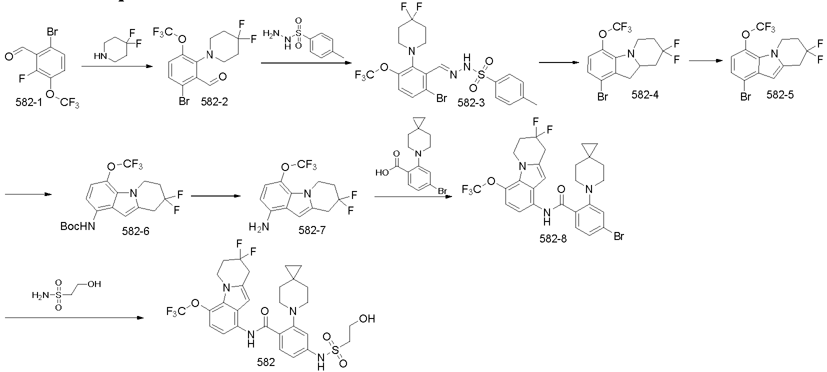

### Step 1: synthesis of compound 582-2

Compound 582-1 (1000 mg, 3.48 mmol), 4,4-difluoropiperidine hydrochloride (659 mg, 4.18 mmol) and *N,N* diisopropylethylamine (1.35 g, 10.45 mmol) were dissolved in NMP (10 mL) and reacted at 100°C for 16 hours under nitrogen protection. The reaction solution was quenched by adding water (20 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 582-2.

### Step 2: synthesis of compound 582-3

Compound 582-2 (500 mg, 1.29 mmol) and N-amino-4-toluenesulfonamide (240 mg, 1.29 mmol) were dissolved in toluene (4 mL) and reacted at room temperature for 0.5 hours. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was not purified and used directly in the next step to obtain compound 582-3.

### Step 3: synthesis of compound 582-4

Compound 582-3 (710 mg, 1.28 mmol) and sodium hydride (61 mg, 1.53 mmol) were dissolved in toluene (5 mL) and reacted at room temperature for 0.5 hours, and then reacted at 135°C for 25 min. After the reaction was completed as monitored by TLC, the reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 582-4.

### Step 4: synthesis of compound 582-5

The compound 582-4 (550 mg, 1.48 mmol) and manganese dioxide (1280 mg, 14.8 mmol) were dissolved in DCM (10 mL) and reacted at 40°C for 12 hours. After the reaction was completed as monitored by TLC, the reaction solution was quenched by adding water (6 ml) and then subjected to suction filtration, and liquid separation was performed with DCM (30 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 20/1) to obtain compound 582-5.

### Step 5: synthesis of compound 582-6

Compound 582-5 (270 mg, 0.73 mmol), tert-butyl carbamate (171 mg, 1.46 mmol), cesium carbonate (713 mg, 2.19 mmol) and palladium catalyst (CAS: 1599466-81-5; 62 mg, 0.07 mmol) were dissolved in 1,4-dioxane (8 mL), and the reaction mixture was reacted at 90°C for 4 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 10/1) to obtain compound 582-6.

### Step 6: synthesis of compound 582-7

Compound 582-6 (290 mg, 0.71 mmol) was dissolved in hydrogen chloride-ethyl acetate solution and the mixture was reacted at room temperature for 3 hours. The reaction solution was concentrated under reduced pressure, and the solids were neutralized, extracted and dried, and concentrated to obtain compound 582-7, which was used directly in the next step without purification.

### Step 7: synthesis of compound 582-8

Compound 582-7 (100 mg, 0.33 mmol), 4-bromo-2-(6-azaspiro[2.5]octan-6-yl)benzoic acid (121 mg, 0.39 mmol), HATU (136 mg, 0.36 mmol) and *N,N-*diisopropylethylamine (0.23 ml, 1.31 mmol) were dissolved in DMF (6 ml), and the reaction mixture was reacted at room temperature for 12 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 30/1) to obtain compound 582-8.

### Step 8: synthesis of compound 582

Compound 582-8 (150 mg, 0.25 mmol), 2-hydroxyethylsulfonamide (94 mg, 0.75 mmol), cesium carbonate (245 mg, 0.75 mmol) and palladium catalyst (CAS: 1599466-89-3; 20 mg, 0.03 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 582. MS (ESI, pos.ion) m/z:643.0 [M+1]⁺.

¹H NMR (400 MHz, DMSO-*d*₆) δ: 11.23 (s, 1H), 10.10 (br s, 1H), 7.82-7.89 (m, 2H), 7.19 (d, *J*=1.9 Hz, 1H), 7.12 (d, *J*=8.8 Hz, 1H), 7.03 (d, *J*=8.1 Hz, 1H), 6.66 (s, 1H), 4.96 (br t, *J*=5.4 Hz, 1H), 4.46 (t, *J*=6.4 Hz, 2H), 3.77 (br t, *J*=6.5 Hz, 2H), 3.59 (br t, *J=*14.3 Hz, 2H), 3.33-3.36 (m, 2H), 3.02 (br t, *J*=4.6 Hz, 4H), 2.63-2.74 (m, 2H), 1.45 (br s, 4H), 0.29 (s, 4H).

### Example 583:

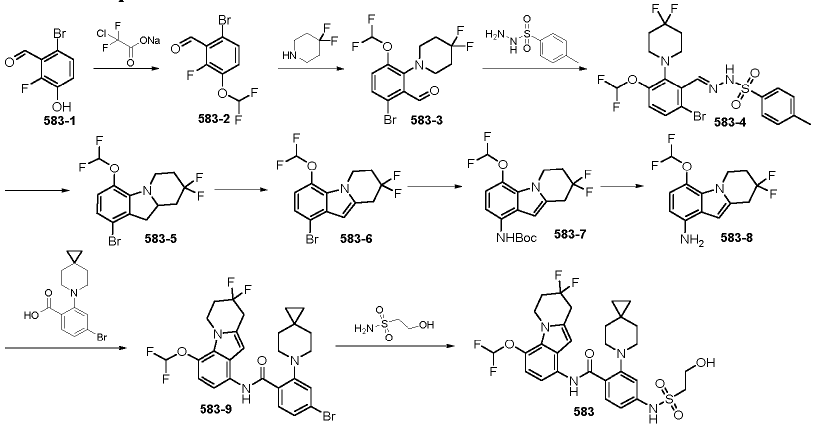

### Step 1: synthesis of compound 583-2

Compound 583-1 (5 g, 22.83 mmol), and potassium carbonate (3.16 g, 22.83 mmol) were dissolved in DMF (32 mL) and water (8 mL), to which sodium 2-chloro-2,2-difluoroacetate (6.96 g, 45.66 mmol) was added, and the reaction mixture was allowed to react at 100°C overnight. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 583-2.

### Step 2: synthesis of compound 583-3

Compound 583-2 (893 mg, 3.32 mmol), and 4,4-difluoropiperidine hydrochloride (628 mg, 3.98 mmol) were dissolved in NMP (8 mL), to which DIEA (1.65 mL, 9.96 mmol) was added, and the reaction mixture was allowed to react overnight at 100°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 583-3.

### Step 3: synthesis of compound 583-4

Compound 583-3 (954 mg, 2.58 mmol) was dissolved in toluene (8 mL), to which p-toluenesulfonyl hydrazide (528 mg, 2.84 mmol) was added, and the mixture was reacted at room temperature for 20 minutes. The reaction solution was quenched with ice water, and extracted with EA. The organic phase was washed with saturated NaCl solution, dried over anhydrous Na₂SO₄, and concentrated under reduced pressure to obtain compound 583-4.

### Step 4: synthesis of compound 583-5

Compound 583-4 (1387 mg, 2.58 mmol) was dissolved in toluene (20 mL), to which NaH (114 mg, 2.83 mmol) was slowly added at 0°C, and after stirring for 30 minutes, the mixture was sealed, and reacted at 135°C for 20 minutes. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=20:1) to obtain compound 583-5.

### Step 5: synthesis of compound 583-6

Compound 583-5 (600 mg, 1.69 mmol) was dissolved in DCM (10 mL), to which manganese dioxide (1.48 g, 16.90 mmol) was added, and the mixture was reacted overnight at room temperature. The reaction solution was filtered, and concentrated under reduced pressure. The crude product was purified by silica gel column chromatography (PE:EA=20:1) to obtain compound 583-6.

### Step 6: synthesis of compound 583-7

Compound 583-6 (470 mg, 1.33 mmol), NH₂Boc (312 mg, 2.66 mmol), cesium carbonate (1.30 g, 3.99 mmol) and the fourth-generation palladium catalyst (CAS: 1599466-83-7; 245 mg, 0.27 mmol) were dissolved in 1,4-dioxane (10 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 583-7.

### Step 7: synthesis of compound 583-8

Compound 583-7 (352 mg, 0.91 mmol) was dissolved in DCM (2 mL), to which hydrogen chloride-dioxane solution (3 mL) was added, and the reaction mixture was allowed to react at 25°C for 1 hour. The reaction solution was filtered, and the solid was dissolved, neutralized, extracted and dried, and concentrated to obtain compound 583-8.

### Step 8: synthesis of compound 583-9

Compound 583-8 (130 mg, 0.40 mmol), 4-bromo-2-(6-azaspiro[2.5]oct-6-yl)benzoic acid (137 mg, 0.44 mmol), and HATU (168 mg, 0.44 mmol) were dissolved in DMF (3 mL), to which DIEA (0.27 mL, 1.60 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 583-9.

### Step 9: synthesis of compound 583

Compound 583-9 (155 mg, 0.27 mmol), 2-hydroxyethanesulfonamide (134 mg, 1.07 mmol), cesium carbonate (261 mg, 0.80 mmol) and the fourth-generation palladium catalyst (CAS: 1599466-89-3; 22 mg, 0.03 mmol) were dissolved in 1,4-dioxane (5 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 583. MS (ESI, pos.ion) m/z:624.9 [M+1]⁺.

¹H NMR (400 MHz, DMSO-*d*₆) δ = 11.19 (s, 1H), 10.09 (s, 1H), 7.85 (d, *J* = 8.4 Hz, 1H), 7.79 (d, *J =* 8.4 Hz, 1H), 7.47 - 7.26 (m, 1H), 7.19 (d, *J =* 2.0 Hz, 1H), 7.03 (dd, *J =* 2.0*,* 8.5 Hz, 1H), 6.92 (d, *J =* 8.4 Hz, 1H), 6.59 (s, 1H), 4.95 (t, *J =* 5.6 Hz, 1H), 4.52 (t, *J =* 6.4 Hz, 2H), 3.80 - 3.74 (m, 2H), 3.57 (br t, *J =* 14.3 Hz, 2H), 3.37 - 3.33 (m, 2H), 3.01 (br s, 4H), 2.69 - 2.61 (m, 2H), 1.45 (br s, 4H), 0.29 (s, 4H).

### Example 585:

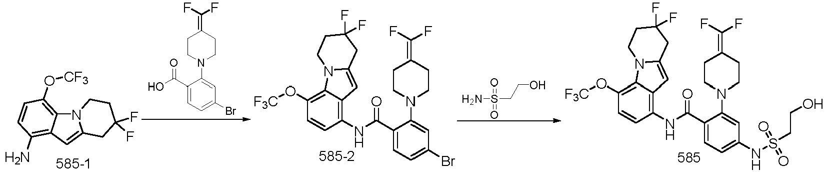

### Step 1: synthesis of compound 585-2

Compound 585-1 (100 mg, 0.33 mmol), 4-bromo-2-[4-(difluoromethylene)hexahydropyridin-1-yl]benzoic acid (130 mg, 0.39 mmol), HATU (136 mg, 0.36 mmol) and N,N-diisopropylethylamine (0.23 ml, 1.31 mmol) were dissolved in DMF (6 ml), and the reaction mixture was reacted at room temperature for 12 hours under nitrogen protection at room temperature. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 30/1) to obtain compound 585-2.

### Step 2: synthesis of compound 585

Compound 585-2 (140 mg, 0.23 mmol), 2-hydroxyethanesulfonamide (84 mg, 0.68 mmol), cesium carbonate (220 mg, 0.68 mmol) and palladium catalyst (CAS: 1599466-89-3; 18 mg, 0.02 mmol) were dissolved in 1,4-dioxane (4 mL), and the reaction mixture was reacted at 90°C for 3 hours under nitrogen protection. The reaction solution was quenched by adding water (10 ml), and liquid separation was performed with EA (40 mL). The organic phase was washed with saturated sodium chloride aqueous solution (10 mL), dried over anhydrous Na₂SO₄, and concentrated under reduced pressure. The crude product was purified by silica gel column chromatography (PE/EA= 1/0 to 1/1) to obtain compound 585. MS (ESI, pos.ion) m/z:664.9 [M+1]⁺.

¹H NMR (400 MHz, DMSO-*d*₆) δ: 10.80 (s, 1H), 7.89 (d, *J*=8.6 Hz, 1H), 7.75 (d, *J*=8.4 Hz, 1H), 7.11 (br d, *J*=7.6 Hz, 1H), 7.07 (s, 1H), 7.01 (d, *J*=8.4 Hz, 1H), 6.68 (s, 1H), 4.44 (t, *J*=6.4 Hz, 2H), 3.77 (t, *J*=6.6 Hz, 2H), 3.60 (br t, *J*=14.4 Hz, 2H), 3.35 (br s, 2H), 2.95-3.08 (m, 4H), 2.61-2.72 (m, 2H), 2.23 (br s, 4H).

### Example 586:

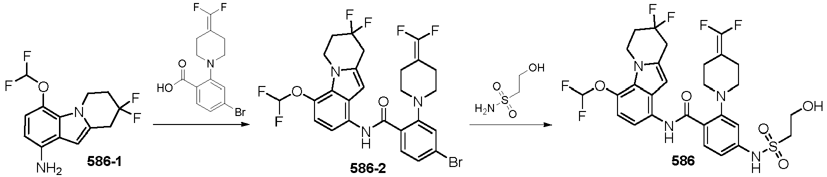

### Step 1: synthesis of compound 586-2

Compound 586-1 (130 mg, 0.40 mmol), 4-bromo-2-(4-(difluoromethylene)piperidin-1-yl)benzoic acid (146 mg, 0.44 mmol), and HATU (168 mg, 0.44 mmol) were dissolved in DMF (3 mL), to which DIEA (0.27 mL, 1.60 mmol) was added, and the reaction mixture was allowed to react overnight at 25°C. The reaction solution was quenched with ice water, and extracted with EA. The organic phase was washed with saturated NaCl solution, dried over anhydrous Na₂SO₄, and concentrated under reduced pressure. The crude product was purified by silica gel column chromatography (PE:EA=10:1) to obtain compound 586-2.

### Step 9: synthesis of compound 586

Compound 586-2 (219 mg, 0.36 mmol), 2-hydroxyethanesulfonamide (182 mg, 1.45 mmol), cesium carbonate (355 mg, 1.09 mmol) and the fourth-generation palladium catalyst (CAS: 1599466-89-3; 29 mg, 0.04 mmol) were dissolved in 1,4-dioxane (5 mL), and the reaction mixture was reacted at 95°C for 1 h under nitrogen protection. The reaction solution was quenched with ice water, and extracted with EA. The organic phase was washed with saturated NaCl solution, dried over anhydrous Na₂SO₄, and concentrated under reduced pressure, and the crude product was purified to obtain compound 586. MS (ESI, pos.ion) m/z:646.9 [M+1]⁺.

¹H NMR (400 MHz, DMSO-*d*₆) δ = 10.75 (s, 1H), 7.84 - 7.79 (m, 1H), 7.77 (d, *J =* 8.5 Hz, 1H), 7.46 - 7.24 (m, 1H), 7.10 - 7.05 (m, 1H), 7.02 (br d, *J =* 8.4 Hz, 1H), 6.96 - 6.88 (m, 1H), 6.61 (s, 1H), 4.51 (br t, *J =* 6.3 Hz, 2H), 3.77 (br t, *J =* 6.5 Hz, 2H), 3.62 - 3.52 (m, 2H), 3.37 - 3.34 (m, 2H), 3.02 (br s, 4H), 2.69 - 2.58 (m, 2H), 2.23 (br s, 4H).

### Test example 1: Assay of the inhibitory effects on KIF18A activity in vitro by the compound of the present disclosure (ADP-Glo assay)

### I. Test objective

This experiment assesses the inhibitory effect of the compounds of the present disclosure on the KIF18A target according to the IC50 values by measuring the KIF18A ATPase activity under tubulin stimulation with the presence of the compounds.

### II. Experimental method

1. Preparation of 30 mL of reaction buffer:
   (1) 450 µL 1M Tris-HCl (pH=7.5) + 300 µL 1M MgCl₂ + 30 µL 10% Pluronic F-68 (ThermoFisher, 24040032-100ml);
   (2) adjusting pH to 7.5, and adding water to adjust the volume to 30 mL.
2. Preparation of a 5 mg/mL pig microtubule (Microtubules; MT) (Cytoskeleton Inc, MT002) stock solution:
   (1) 10 mL PM buffer = 150 µL 1 M PIPES (pH 7.0) + 20 µL 0.5 mM MgCl₂ + 100 µL 2 mM Taxol (Cytoskeleton Inc, TXD01) + 9730 µL water.
   (2) MT (500 µg/tube): Adding 100 µL PM buffer to prepare a 5 mg/mL stock solution. Keeping the MT (Cytoskeleton Inc, MT002) at room temperature for 10-15 minutes, gently mixing and storing same at -80°C.
3. Preparation of 2 mL of kinase buffer: 2 mL reaction buffer + 1 µL 2 mM Taxol (Cytoskeleton Inc, TXD01) + 12 µL 5 mg/mL MT (Cytoskeleton Inc, MT002).
4. Preparation of 2X KIF18A (30 nM) (ChemPartner, CPB-P22-31645) working solution: 2.4 µL 10 µM KIF18A (ChemPartner, CPB-P22-31645) + 800 µL 1x kinase buffer.
5. Preparation of 2X ATP (150 µM) (Promega, G9101) working solution: 12 µL 10 mM ATP + 800 µL 1x kinase buffer.
6. 25 nL of compound was transferred to a 384-well plate using Echo650.
7. 2.5 µL of 2X KIF18A from Step 4 (final concentration of 30 nM) was added to the 384-well plate and the plate was incubated at 25°C for 15 minutes.
8. 2.5 µL of 2X ATP from Step 5 (final concentration of 150 µM) was added to the 384-well plate and the plate was incubated at 25°C for 15 minutes.
9. 5 µL ADP-Glo^{™} Reagent (Promega, G9101) was added to the 384-well plate and the plate was incubated at 25°C for 40 minutes.
10. 10 µL of kinase detection reagent (Promega, G9101) was added to the 384-well plate and the plate was incubated at 25°C for 40 minutes.
11. Luminescence values were measured using the EnVision Xcite Multilabel Reader (PerkinElmer, 2105-0020).

### III. Data analysis

IC50 values were calculated for inhibitory activity of compounds using Graphpad Prism software, and the results of some of the compounds are shown in Table 1 below.

**Table 1**

| Compound No. | ADP-Glo IC₅₀ | Compound No. | ADP-Glo IC₅₀ | Compound No. | ADP-Glo IC₅₀ |
|---|---|---|---|---|---|
| Reference compound | A | 11 | C | 23 | D |
| 1 | A | 12 | C | 24 | C |
| 3 | B | 13 | B | 25 | D |
| 4 | B | 14 | B | 26 | B |
| 5 | A | 15 | C | 27 | C |
| 6 | B | 16 | A | 28 | C |
| 7 | B | 17 | D | 29 | D |
| 8 | B | 18 | A | 30 | C |
| 9 | A | 19 | D | 32 | B |
| 10 | B | 22 | B | 33 | A |
| 34 | C | 50 | B | 148 | B |
| 37 | B | 56 | B | 149 | C |
| 38 | B | 126 | A | 150 | D |
| 39 | B | 127 | A | 151 | D |
| 40 | B | 128 | A | 234 | B |
| 41 | B | 129 | A | 235 | A |
| 42 | B | 130 | A | 269 | A |
| 44 | B | 134 | A | 279 | B |
| 47 | B | 145 | A | 281 | C |
| 48 | C | 147 | C | 283 | B |
| 284 | C | 308 | A | 313 | A |
| 285 | B | 310 | A | 313-P1 | A |
| 286 | A | 311 | A | 313-P2 | B |
| 287 | B | 311-P1 | A | 319 | A |
| 294 | A | 311-P2 | A | 320 | B |
| 303 | A | 312 | B | 320-P1 | C |
| 304 | A | 312-P1 | B | 320-P2 | A |
| 305 | A | 312-P2A | A | 321 | A |
| 306 | D | 312-P3 | B | 322 | A |
| 307 | D | 312-P2B | B | 323 | A |
| 340 | B | 364 | A | 373-P2 | B |
| 340-P1 | B | 366 | A | 374 | A |
| 340-P2 | A | 367 | A | 376 | A |
| 341 | B | 368 | A | 378 | B |
| 342 | A | 369 | A | 379 | A |
| 342-P1 | A | 370 | B | 380 | A |
| 342-P2 | A | 371 | A | 381 | A |
| 345 | B | 372 | A | 382 | A |
| 346 | B | 373 | A | 383 | A |
| 357 | A | 373-P1 | B | 384 | B |
| 384-P1 | A | 393 | B | 410 | B |
| 384-P2 | B | 394 | A | 411 | B |
| 385 | B | 395 | A | 427 | A |
| 385-P1 | B | 396 | B | 428 | B |
| 385-P2 | B | 398 | B | 438 | B |
| 386 | B | 399 | B | 444 | A |
| 386-P1 | C | 400 | A | 445 | A |
| 386-P2 | A | 401 | A | 449 | B |
| 391 | B | 404 | A | 453 | B |
| 392 | A | 405 | A | 454 | A |
| 455 | B | 490 | B | 527 | B |
| 465 | B | 491 | B | 528 | B |
| 467 | B | 519 | B | 529 | B |
| 477 | B | 520 | B | 531 | B |
| 478 | A | 521 | B | 532 | B |
| 479 | B | 522 | A | 535 | B |
| 482 | A | 523 | B | 536 | B |
| 485 | A | 524 | B | 565 | B |
| 488 | B | 525 | B | 566 | A |
| 489 | B | 526 | B | 573 | A |
| 574 | B | 592 | B | 615 | B |
| 575 | B | 593 | B | 618 | B |
| 576 | B | 597 | B | 619 | B |
| 582 | A | 600 | B | 620 | B |
| 583 | B | 601 | B | 621 | B |
| 584 | B | 602 | B | 622 | B |
| 585 | B | 603 | B | 623 | B |
| 586 | B | 610 | B | 624 | B |
| 587 | B | 611 | B | 625 | B |
| 591 | B | 614 | B | 626 | B |
| 627 | B | 628 | B | | |

Conclusions: The compounds of the present disclosure have better KIF18A inhibitory activity. For the ADP-Glo IC50 values in Table 1, A represents IC50 ≤ 50 nM; B represents 50 nM < IC50 ≤ 500 nM; C represents 500 nM < IC50 ≤ 5000 nM; D represents IC50 >5000 nM.

### Test example 2: Determination of the inhibitory effect of the compounds of the present disclosure on OVCAR-3 cell proliferation

### I. Test objective

The inhibitory effect on the KIF18A target by the compounds of the present disclosure was evaluated by testing the inhibitory effect on proliferation of OVCAR-3 cells by the compounds of the present disclosure.

### II. Experimental method

The following method was used to determine the effect of the compounds of the present disclosure on the proliferation of human ovarian cancer cells Ovcar-3 (HTB-161, ATCC). Cells were cultured in RPMI-1640 complete medium containing 20% fetal bovine serum, 0.01 mg/mL bovine insulin, 100 U penicillin, and 100 µg/mL streptomycin. The Promega kit Cell Titer-Glo (Cat. No. G7572) was used in this method for test.

For specific steps, reference was made to the kit instructions, and the procedure was outlined below: Test compounds were dissolved in DMSO to prepare a 10 mM stock solution, which was diluted at the predetermined test concentrations, and the test compounds at different concentrations were added to a 384-well cell test plate using ECHO. In the plate pre-added with the test compounds, 400 OVCAR-3 cells in the logarithmic growth phase were seeded per well, ensuring the final concentration of the test compounds in the reaction system ranged between 0.12 nM and 10 µM. The co-culture was maintained in a cell culture incubator for 10 days. Before testing, the cell culture plate was equilibrated for 10 minutes at room temperature, 25 µL of Cell Titer-Glo was added to each well, and the mixture was shaken and mixed well and let stand for 5 minutes. The luminescence values for samples in various wells were read using a microplate reader in Luminescence mode.

### III. Data analysis

The percentage of inhibition of cell proliferation by the test compound at each concentration was calculated by comparing with the values of the control group (0.1% DMSO). The IC50 values of the compounds were obtained by concentration-inhibition rate nonlinear regression analysis of the test compounds using GraphPad Prism 9.

**Table 2**

| Compound No. | OVCAR-3 IC₅₀ | Compound No. | OVCAR-3 IC₅₀ | Compound No. | OVCAR-3 IC₅₀ |
|---|---|---|---|---|---|
| Reference compound | A | 50 | B | 279 | C |
| 1 | A | 56 | B | 283 | B |
| 3 | A | 126 | B | 286 | B |
| 4 | A | 127 | A | 287 | B |
| 5 | B | 128 | A | 294 | C |
| 9 | B | 130 | B | 303 | B |
| 13 | B | 145 | B | 304 | A |
| 16 | B | 234 | B | 305 | B |
| 18 | B | 235 | A | 308 | A |
| 41 | B | 269 | B | 310 | A |
| 311 | A | 313-P2 | B | 341 | B |
| 311-P1 | B | 319 | A | 342 | B |
| 311-P2 | A | 320 | A | 342-P1 | B |
| 312 | A | 320-P2 | A | 342-P2 | B |
| 312-P1 | B | 321 | B | 345 | B |
| 312-P2A | A | 322 | A | 346 | D |
| 312-P3 | A | 323 | A | 357 | B |
| 312-P2B | B | 340 | A | 364 | B |
| 313 | B | 340-P1 | B | 366 | A |
| 313-P1 | B | 340-P2 | A | 367 | B |
| 368 | A | 378 | A | 385-P1 | B |
| 369 | B | 379 | A | 385-P2 | A |
| 370 | A | 380 | A | 386 | B |
| 371 | A | 381 | A | 386-P1 | C |
| 372 | A | 382 | A | 386-P2 | B |
| 373 | A | 383 | A | 391 | A |
| 373-P1 | B | 384 | A | 392 | A |
| 373-P2 | B | 384-P1 | A | 393 | B |
| 374 | A | 384-P2 | A | 394 | A |
| 376 | A | 385 | A | 395 | B |
| 396 | A | 428 | B | 477 | A |
| 398 | A | 438 | A | 478 | A |
| 399 | C | 444 | A | 479 | B |
| 400 | B | 445 | A | 482 | A |
| 401 | B | 449 | A | 485 | B |
| 404 | A | 453 | C | 488 | A |
| 405 | A | 454 | B | 489 | A |
| 410 | A | 455 | A | 490 | B |
| 411 | B | 465 | A | 491 | A |
| 427 | B | 467 | B | 519 | B |
| 520 | B | 531 | B | 582 | B |
| 521 | B | 532 | B | 583 | A |
| 522 | B | 535 | A | 584 | A |
| 523 | A | 536 | B | 585 | B |
| 524 | B | 565 | A | 586 | B |
| 525 | A | 566 | B | 587 | A |
| 526 | B | 573 | B | 591 | A |
| 527 | A | 574 | B | 592 | A |
| 528 | A | 575 | A | 593 | A |
| 529 | A | 576 | A | 597 | A |
| 600 | A | 620 | A | | |
| 601 | A | 621 | A | | |
| 602 | A | 622 | A | | |
| 603 | A | 623 | A | | |
| 610 | A | 624 | A | | |
| 611 | A | 625 | A | | |
| 614 | A | 626 | A | | |
| 615 | A | 627 | A | | |
| 618 | A | 628 | A | | |
| 619 | A | | | | |

Conclusions: The compounds of the present disclosure have a significant inhibitory effect on the proliferation of OVCAR-3 cells. The results of some of the compounds are shown in Table 2. For the OVCAR-3 IC50 values in Table 2, A represents IC50 ≤ 50 nM; B represents 50 nM< IC50 ≤ 500 nM; C represents 500 nM < IC50 ≤ 5000 nM; D represents IC50 > 5000 nM.

### Test example 3: Determination of the inhibitory effect of the compounds of the present disclosure on HCC1954 and HCC1395 cell proliferation

### I. Test objective

The inhibitory effect on the KIF18A target by the compounds of the present disclosure was evaluated by testing the inhibitory effect on proliferation of HCC1954 and HCC1395 cells by the compounds of the present disclosure.

### II. Experimental method

HCC1954 (ATCC, CRL-2388) and HCC1395 (ATCC, CRL-2324) cells were cultured separately in RPMI-1640 complete medium (Gibco, A1049101) containing 20% fetal bovine serum (Gibco, 10099141C), 100 U penicillin, and 100 µg/mL streptomycin (Gibco, 15140-122). The Promega kit Cell Titer-Glo (Promega: G7572) was used in this method for test.
1. HCC1954 (KIF18A-sensitive tumor cells) and HCC1395 (KIF18A-insensitive tumor cells) were cultured separately in T75 cell culture flasks (Corning, 430641), and the medium was placed in a 37°C, 5% CO₂ incubator.
2. When the cell density reached 70% to 80% (about 2 to 3 times per week), cells are passaged with trypsin (0.25% EDTA).
3. The cell culture flask was taken out and the cells were separated by adding 2 mL of trypsin (0.25% EDTA source) for 5 minutes.
4. Digestion was stopped by adding 8 mL of RPMI-1640 medium and the cell suspension was transferred to a centrifuge tube and centrifuged at 1000 rpm for 5 minutes.
5. The cells were resuspended with 5 mL of RPMI-1640 medium. Cell density was adjusted to 5×10³/mL (HCC1954), and 2.5×10⁴/mL (HCC1395), respectively.
6. 40 µL of cell solution was added to the 384-well plate (Corning, 3765).
7. The 384-well plate was centrifuged at 1000 rpm for 1 minute.
8. The cells were cultured overnight in a 37°C, 5% CO₂ incubator.
9. The next day, 40 nL of compound solution was added to the 384-well plate using Echo650. The plate was then placed in a 37°C, 5% CO₂ incubator for 6 days.
10. After 6 days, 20 µL of Cell-Titer-Glo (Promega, G7572) reagent was added per well.
11. Luminescence values were measured using the EnVision Xcite Multilabel Reader (PerkinElmer, 2105-0020).

### III. Data analysis

The percentage of inhibition of cell proliferation by the test compound at each concentration was calculated by comparing with the values of the control group (0.1% DMSO). The IC50 values of the compounds were obtained by concentration-inhibition rate nonlinear regression analysis of the test compounds using GraphPad Prism 9.

**Table 3**

| Compound No. | HCC1954 IC₅₀ | HCC1395 IC₅₀ | Compound No. | HCC1954 IC₅₀ | HCC1395 IC₅₀ |
|---|---|---|---|---|---|
| Reference compound | B | D | 126 | B | D |
| 1 | A | D | 129 | A | D |
| 3 | A | D | 134 | B | D |
| 4 | A | D | 234 | B | D |
| 9 | B | D | 235 | A | D |
| 13 | B | D | 269 | B | D |
| 16 | B | D | 303 | B | D |
| 18 | D | D | 304 | B | D |
| 41 | B | | 305 | B | D |
| 50 | B | D | 311 | B | D |
| 56 | B | D | 319 | B | D |
| 320 | B | D | 490 | B | |
| 340 | B | D | | | |

Conclusions: The compounds of the present disclosure have a significant inhibitory effect on the proliferation of HCC1954 cells. The results of some of the compounds are shown in Table 3. For the HCC1954 and HCC1395 IC50 values in Table 3, A represents IC50 ≤ 10 nM; B represents 10 nM < IC50≤500 nM; C represents 500 nM < IC50 ≤ 5000 nM; D represents IC50 > 5000 nM.

### Test example 4: PK study

1. Experiment animals
6 ICR mice (male/female) were divided into two groups, administered orally and intravenously, respectively, with 3 mice/group. The mice were fasted for 10-14 hours prior to dosing with free access to water.
2. Preparation of dose formulation
The test compound was weighed according to the administration dose, and prepared into a dose formulation at appropriate concentrations (for rats and mice, intravenous and oral administration at a volume of 5 mL/kg and 10 mL/kg, respectively), wherein the intravenous injection was a clear solution, and the oral preparation was a clear solution or a homogeneous suspension.
3. Administration to animals and blood sample collection
Animals were dosed by intravenous injection and oral gavage, and blood samples were collected at 0.033, 0.083, 0.25, 0.5, 1, 2, 4, 8, and 24 h after intravenous administration and 0.083, 0.25, 0.5, 1, 2, 4, 6, 8, and 24 h after oral administration. Whole blood was centrifuged at 6800 g for 6 min at 4°C and the top plasma was stored in a -80°C freezer for later analysis.
4. Plasma sample testing
The DMSO stock solution of the test compound was diluted into a series of working solutions with a methanol or an acetonitrile solution, and added to a blank plasma matrix, and standard curves were generated and quality control samples were prepared. An appropriate volume of the plasma sample was taken and a suitable multiple of methanol or acetonitrile containing an internal standard was added for protein precipitation based on the response. After centrifugation of all samples at 18000 g for 10 min at 4°C, an appropriate amount of supernatant was taken for LC-MS/MS injection analysis.
5. Parameter calculation
Drug concentration-time curves in plasma were plotted according to test concentrations, and pharmacokinetic parameters were calculated using noncompartmental analysis by WinNonlin software, including: half-life (T1/2), area under the concentration-time curve from time zero to time t (AUC0-t), clearance (CL), volume of distribution at steady state (Vss) and bioavailability (F), with some of the test results shown in Table 4.

**Table 4**

| Compound No. | AUC (h*ng/mL) | Cmax (ng/mL) | Compound No. | AUC (h*ng/mL) | Cmax (ng/mL) |
|---|---|---|---|---|---|
| 235 | 5976 | 2990 | 371 | 51207 | 3747 |
| 308 | 5646 | 1097 | 374 | 26457 | 3697 |
| 310 | 12620 | 3027 | 378 | 48150 | 5253 |
| 312-P3 | 32450 | 2980 | 379 | 36456 | 4043 |
| 319 | 16534 | 3717 | 384-P1 | 1293 | 366 |
| 321 | 16039 | 4141 | 391 | 37979 | 4373 |
| 322 | 12358 | 2423 | 392 | 50841 | 5078 |
| 323 | 4113 | 762 | 393 | 22301 | 3837 |
| 370 | 52443 | 3577 | 394 | 47849 | 4220 |
| 398 | 6992 | 1410 | 485 | 33327 | 3739 |
| 405 | 27572 | 4205 | 491 | 24792 | 2296 |
| 410 | 16409 | 2786 | 523 | 99384 | 7088 |
| 455 | 71046 | 5591 | 535 | 100625 | 8366 |
| 465 | 72111 | 5780 | 583 | 43268 | 4406 |
| 478 | 48468 | 6021 | | | |

Tests have demonstrated that the compounds disclosed herein exhibit better pharmacokinetic properties.

### Test example 5. Liver microsomal stability test

I. Experimental method: A solution of liver microsomes from different species and compounds in acetonitrile was mixed with 100 mM phosphate buffer (pH 7.4) to prepare a reaction stock solution, ensuring a final concentration of 1 µM of the compound in the reaction system. After mixing well, 30 µL of each sample was aliquoted into a new 96-deep-well plate to establish reaction groups (with NADPH at 0, 5, 15, 30, and 45 min; n = 2) and control groups (without NADPH at 0 and 45 min; n = 1). After pre-incubation of the above sample for 5 min with shaking in a 37°C water bath, the reaction was initiated by the addition of 15 µL of NADPH, ensuring a final concentration of 2 mM of NADPH in the reaction system, and 15 µL of phosphate buffer solution was added to the control group without NADPH. After mixing well, incubation was continued for the corresponding time. The reaction was stopped with 200 µL of glacial acetonitrile containing an internal standard. The reaction mixture was vortexed and mixed well, centrifuged at 4000 rpm for 50 min at 4°C, and the supernatant was diluted with ultrapure water for UPLC-MS/MS analysis.

### II. Data analysis

The logarithm of the test compound's remaining percentage was plotted versus incubation time, with the elimination half-life calculated using T1/2 = 0.693/K.

Tests have demonstrated that the compounds disclosed herein exhibit better stability in liver microsomes.

### Test example 6. hERG test

### I. Experimental method:

The cells used in this test were the HEK293 cell line transfected with hERG cDNA and stably expressing the hERG channel (#60187, supplied by BPS, passages P3-P23 for experimental studies). The cells were cultured in a medium containing the following components: MEM medium containing 10% (v/v) inactivated fetal calf serum, 1 mM sodium pyruvate, 500 µg/ml geneticin, 0.1 mM non-essential amino acids, and 100 U/ml penicillin/streptomycin. HEK293 hERG cells were grown in a culture dish containing the above-mentioned medium, cultured in a 37°C, 5% CO2 incubator, and passaged approximately three times per week, with the cell fusion rate between 40% and 80%. 24 to 48 hours prior to electrophysiological experiments, HEK293 hERG cells were transferred to 0.05 mg/ml PDL-pretreated slides, seeded at a density of 1×10⁴ cells per well in a 48-well plate, and grown in the same medium and under the same culture conditions. The density of HEK293 hERG cells on each circular slide needs to reach the requirement that most cells are independent and individual. The extracellular fluid components used in the hERG assay were as follows (mM): 145 NaCl, 4 KCl, 2 CaCl2, 1 MgCl2, 10 glucose, and 10 HEPES (pH adjusted to 7.40 with NaOH). The intracellular fluid components were as follows (mM): 130 KCl, 2 MgCl2, 5 EGTA, 10 HEPES and 5 Na2ATP (pH adjusted to 7.25 with KOH). To obtain the IC50 of the compound, the following concentrations (30, 10, 3, 1, 0.3 and 0.1 µM) were used for test (if the compound exhibited a strong effect, the test concentration could be adjusted appropriately). Prior to the experiment, the compound was first serially diluted with DMSO to prepare stock solutions at 10, 3, 1, 0.3, and 0.1 mM, which were then further diluted with extracellular fluid to achieve the final test concentrations (in µM). The final concentration of DMSO in compound solutions at various concentrations was between 0.1% and 0.3%. All compound solutions are routinely sonicated for 5 to 10 min and shaken sufficiently to ensure complete dissolution of the compound, and all test solutions are mixed well by rotation for at least 10 min.

Electrophysiological tests were performed using a manual patch clamp system (HEKA EPC-10 signal amplifier and digital conversion system, HEKA Electronics, Germany) for the recording of whole cell currents. The specific test method was described as follows: Circular slides with CHO hERG cells growing on the surface were placed in an electrophysiological recording chamber under an inverted microscope. The recording chamber was continuously perfused with extracellular fluid (at approximately 1 mL/min). The conventional whole-cell patch-clamp technique was employed for current recordings throughout the experiment. If not specified, the experiments were carried out at normal room temperature (about 25°C). The cells were clamped at -80 mV. The cell clamp voltage was depolarized to +30 mV to activate the hERG potassium channel, and then clamped to -50 mV after 5 seconds to eliminate inactivation and produce a tail current. The peak tail current value was used as a value for the magnitude of the hERG current. The potassium current of hERG recorded in the above steps was stabilized by continuous extracellular fluid perfusion in the recording chamber, and the drug to be tested was continuously perfused until the inhibitory effect of the drug on hERG currents had stabilized. The coincidence of the most recent three consecutive current recording traces was typically taken as the criteria for determining whether steady-state conditions were achieved.

### II. Data analysis

Dose-response curves of the test compounds were plotted with % hERG inhibition rate as the y-axis and test compound concentration as the x-axis, to calculate the IC₅₀ values. The results are as shown in Table 5.

**Table 5**

| hERG | |
|---|---|
| Example number | IC50 (µM) |
| 235 | 9.84 |
| 319 | > 10 |
| 370 | > 10 |
| 371 | > 10 |
| 455 | > 10 |
| 465 | > 10 |
| 478 | 8 |
| 523 | > 10 |

Tests have demonstrated that the compounds disclosed herein exhibit a lower risk of hERG cardiotoxicity.

### Test example 7: Determination of the inhibitory effect of the compounds of the present disclosure on HT-29 cell proliferation

### I. Test objective

The inhibitory effect on the KIF18A target by the compounds of the present disclosure was evaluated by testing the inhibitory effect on proliferation of HT-29 cells by the compounds of the present disclosure.

### II. Experimental method

HT-29 (ATCC, HTB-38) cells were cultured in McCoy's 5a complete medium (Gibco, 12330-031) containing 10% fetal bovine serum (Gibco, 10099-141C), 100 U penicillin and 100 100 L streptomycin (Gibco, 15140-122). The Promega kit Cell Titer-Glo (Promega: G7572) was used in this method for test.
1. HT-29 cells were cultured in T75 cell flasks (Corning, 430641) and the medium was placed in a 37°C, 5% CO2 incubator. Cells were passaged when reaching a cell density of 70%-80%, approximately twice per week.
2. To perform the experiment, the test compounds were dissolved in DMSO to prepare a 10 mM stock solution, and the test compounds were added to the 384-well cell test plate (Corning, 3765) at the predetermined concentration using ECHO.
3. The cell culture flask was taken out, the medium was removed, and the cells were rinsed with PBS. Then 2 mL of trypsin (with 0.25% EDTA) was added, and the flask was placed in the incubator for digestion for 4 minutes.
4. Digestion was stopped by adding 4 mL of complete medium and the cell suspension was transferred to a centrifuge tube and centrifuged at 1000 rpm for 4 minutes.
5. The cells were resuspended in 6 mL of complete medium, followed by cell counting. The cell density was adjusted to 4,170 cells/mL, and the cells were added to a 384-well plate at 60 µL/well.
6. The 384-well plate was centrifuged at 500 rpm for 1 minute, and then placed in a 37°C, 5% CO2 incubator for 6 days of cultivation.
7. Six days later, the cell culture plate was equilibrated for 10 minutes at room temperature, 25 µL of Cell Titer-Glo was added to each well, and the mixture was shaken and mixed well and let stand for 5 minutes. The luminescence values for samples in various wells were read using a microplate reader in Luminescence mode.

### III. Data analysis

The percentage of inhibition of cell proliferation by the test compound at each concentration was calculated by comparing with the values of the control group (0.3% DMSO). The IC50 values of the compounds were obtained by concentration-inhibition rate nonlinear regression analysis of the test compounds using GraphPad Prism 9. The results are shown in Table 6.

**Table 6**

| Compound No. | HT-29 IC₅₀ | Compound No. | HT-29 IC₅₀ | Compound No. | HT-29 IC₅₀ |
|---|---|---|---|---|---|
| Reference compound | A | 340-P2 | A | 384-P1 | A |
| 1 | A | 364 | B | 384-P2 | A |
| 4 | A | 370 | B | 385 | A |
| 235 | A | 371 | A | 385-P2 | A |
| 308 | A | 372 | A | 386 | B |
| 310 | A | 374 | A | 386-P2 | A |
| 312-P2A | A | 376 | B | 391 | A |
| 312-P3 | A | 378 | A | 392 | A |
| 319 | B | 379 | A | 393 | A |
| 321 | B | 380 | A | 394 | A |
| 395 | B | 405 | A | 465 | A |
| 396 | A | 410 | A | 485 | B |
| 404 | A | 455 | A | | |

Conclusions: The compounds of the present disclosure have a significant inhibitory effect on the proliferation of HT-29 cells. For the HT-29 IC50 values in Table 6, A represents IC50 ≤ 200 nM; B represents 200 nM < IC50 ≤ 1000 nM; C represents IC50 > 1000 nM.

### Test example 8: HCT116 cell anti-proliferation assay

HCT116 (ATCC, CCL-247) cells were cultured in McCoy's 5a complete medium (Gibco, 12330-031) containing 10% fetal bovine serum (Gibco, 10099-141C), 100 U penicillin and 100 µg/mL streptomycin (Gibco, 15140-122). The Promega kit Cell Titer-Glo (Promega: G7572) was used in this method for test.

HCT116 cells were cultured in T75 cell flasks (Corning, 430641) and the cells were placed in a 37°C, 5% CO₂ incubator. Cells were passaged when reaching a cell density of 70%-80%, approximately twice per week. To perform the experiment, the test compounds were dissolved in DMSO to prepare a 10 mM stock solution, and the test compounds were added to the 384-well cell test plate (Corning, 3765) at the predetermined concentration (19.5 nM-10 mM) using ECHO. HCT1169 cells were digested and 150 cells (60 µL medium) were seeded per well in the test plate. The 384-well plate was then centrifuged at 500 rpm for 1 minute, and then placed in a 37°C, 5% CO2 incubator for 6 days of cultivation. Six days later, the cell culture plate was equilibrated for 10 minutes at room temperature, 25 µL of Cell Titer-Glo was added to each well, and the mixture was shaken and mixed well and let stand for 5 minutes. The luminescence values for samples in various wells were read using a microplate reader in Luminescence mode.

The percentage of inhibition of cell proliferation by the test compound at each concentration was calculated by comparing with the values of the control group (0.2% DMSO). The IC₅₀ values of the compounds were obtained by concentration-inhibition rate nonlinear regression analysis of the test compounds using GraphPad Prism 9. The results are shown in Table 7.

**Table 7**

| HCT116 | | | |
|---|---|---|---|
| Compound No. | IC50 (µM) | Compound No. | IC50 (µM) |
| 4 | 2.6 | 378 | >10 |
| 235 | >10 | 379 | 9.8 |
| 308 | >10 | 391 | >10 |
| 310 | 9 | 392 | >10 |
| 312-P2A | 6.7 | 393 | >10 |
| 312-P3 | >10 | 394 | >10 |
| 319 | >10 | 395 | >10 |
| 321 | >10 | 398 | >10 |
| 340-P2 | 8.5 | 404 | >10 |
| 364 | 8.2 | 405 | >10 |
| 370 | >10 | 455 | >10 |
| 371 | >10 | 465 | >10 |
| 372 | >10 | Reference compound | >10 |
| 374 | 8.7 | | |

The results show that the compounds disclosed herein have no anti-proliferative effect on HCT116 cells and have better target selectivity.

### Test example 9: A2780 cell anti-proliferation assay

A2780 (ECACC) cells were cultured in RPMI-1640 complete medium (Gibco, A1049101) containing 10% fetal bovine serum (Gibco, 10099-141C), 100 U penicillin and 100 µg/mL streptomycin (Gibco, 15140-122). The Promega kit Cell Titer-Glo (Promega: G7572) was used in this method for test.

A2780 cells were cultured in T75 cell flasks (Corning, 430641) and the cells were placed in a 37°C, 5% CO₂ incubator. Cells were passaged when reaching a cell density of 70%-80%, once every five days. To perform the experiment, the test compounds were dissolved in DMSO to prepare a 10 mM stock solution, and the test compounds were added to the 384-well cell test plate (Corning, 3765) at the predetermined concentration (19.5 nM-10 mM) using ECHO. A2780 cells were digested and 4000 cells (60 µL medium) were seeded per well in the test plate. The 384-well plate was then centrifuged at 500 rpm for 1 minute, and then placed in a 37°C, 5% CO2 incubator for 6 days of cultivation. Six days later, the cell culture plate was equilibrated for 10 minutes at room temperature, 25 µL of Cell Titer-Glo was added to each well, and the mixture was shaken and mixed well and let stand for 5 minutes. The luminescence values for samples in various wells were read using a microplate reader in Luminescence mode.

The percentage of inhibition of A2780 cell proliferation by the test compound at each concentration was calculated by comparing with the values of the control group (0.2% DMSO). The IC₅₀ values of the compounds were obtained by concentration-inhibition rate nonlinear regression analysis of the test compounds using GraphPad Prism 9. The results are shown in Table 8.

**Table 8**

| A2780 | | | |
|---|---|---|---|
| Compound No. | IC₅₀(µM) | Compound No. | IC₅₀(µM) |
| 4 | 8.3 | 378 | 7.7 |
| 235 | >10 | 379 | 5.3 |
| 308 | >10 | 391 | >10 |
| 310 | >10 | 392 | 6.1 |
| 312-P2A | 6.5 | 393 | >10 |
| 312-P3 | >10 | 394 | >10 |
| 319 | >10 | 395 | >10 |
| 321 | >10 | 398 | >10 |
| 340-P2 | >10 | 404 | >10 |
| 364 | 9.4 | 405 | >10 |
| 370 | >10 | 455 | >10 |
| 371 | >10 | 465 | >10 |
| 372 | >10 | Reference compound | >10 |
| 374 | 8.5 | | |

The results show that the compounds disclosed herein have no anti-proliferative effect on A2780 cells and have better target selectivity.

### Test example 10: In vivo efficacy studies in the OVCAR3 human ovarian cancer subcutaneous transplant tumor model

I. Experimental objective: evaluation of the pharmacological effects of the reference compounds and the compounds of the present disclosure in the OVCAR3 human ovarian cancer subcutaneous transplant tumor model.
II. Experimental material: experimental female balb/c nude mice, 6-8-week old or 18-20 g, provided by Vital River Laboratory Animal Technology Co., Ltd.
III. Experimental method: Experimental animals were housed within a specific pathogen-free animal barrier facility, and the experiment was started 3 days after the animals were acclimated. The human ovarian cancer OVCAR3 tumor cells were cultured in vitro as monolayers in RPMI 1640 complete medium (supplemented with a final concentration of 20% fetal bovine serum, 1% penicillin-streptomycin dual antibiotics, and 0.01 mg/ml insulin) at 37°C in an atmosphere containing 5% CO2. The tumor cells were routinely passaged 2-3 times per week, and the cells in the exponential growth phase were collected and counted for tumor cell inoculation. 10x10⁶ OVCAR3 tumor cells were resuspended in 0.1 ml PBS and mixed with 0.1 ml matrigel and then subcutaneously inoculated into the right flank of each mouse to establish tumors. When the mean tumor volume of the tumor-bearing mice grew to around 100 mm³, the appropriate tumor-bearing mice were selected for randomization and the groups were set as: blank control groups, reference compound groups, and groups of compounds of the present disclosure. Vehicles were administered orally in the blank control group, and the corresponding compounds were administered orally in the other groups for 28 days. Tumor volumes and animal weights were monitored weekly for each group during the experiment, twice weekly. This experiment examined the inhibitory effect of the reference compound and the compound of the present disclosure on the growth of OVCAR3 human ovarian cancer cells by monitoring the size of the tumor volume of each group after administration of the compounds.

The results show that oral administration of compound 465 of the present invention (at a dose of 10 mg/kg, QD) exhibits an inhibition rate of 60%-70% on OVCAR3 tumour growth; oral administration of compounds 371 and 455 of the present invention (at a dose of 10 mg/kg, QD) exhibit an inhibition rate of 94%-98% on OVCAR3 tumour growth; and oral administration of compound 371 of the present invention (at a dose of 30 mg/kg, QD) exhibits an inhibition rate of about 110% on OVCAR3 tumour growth. The compounds of the present disclosure show a certain anti-tumor efficacy in vivo.

The compounds of the present disclosure have been found to have superior KIF18A inhibitory activity and anti-tumor efficacy in vivo as measured by the above test examples. Furthermore, the compounds of the present disclosure have better physicochemical properties (e.g. solubility, physical and/or chemical stability), improved pharmacokinetic properties (e.g. improved bioavailability, improved metabolic stability, and appropriate half-life and duration of action), improved safety (lower toxicity (e.g. reduced cardiotoxicity and/or less side effects), less susceptibility to drug resistance), and other superior properties.

While some exemplary embodiments of the present application have been illustrated and described, the present application is not limited to the disclosed embodiments. Rather, those of ordinary skill in the art will recognize that some modifications and changes may be made to the described embodiments without departing from the spirit and scope of the present application as described in the appended claims.

## Claims

1. A compound represented by formula (I), or a stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof, **characterized in that** 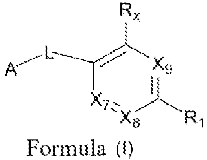
A is monocyclic aryl, monocyclic heteroaryl, bicyclic aryl, bicyclic heteroaryl or fused heterocyclyl, and optionally, the monocyclic aryl, monocyclic heteroaryl, bicyclic aryl, bicyclic heteroaryl or fused heterocyclyl is optionally substituted with one or more R_{z};
L is selected from -NR³-CO- or -NR³CONR³-, and each R³ is independently H, deuterium, C1-C6 alkylene or C1-C6 haloalkylene;
Rₓ is selected from -OR₄ or -NR₅R₆, wherein R₄ is selected from 3- to 8-membered cycloalkyl, 3- to 8-membered cycloalkenyl or 3- to 8-membered heterocyclyl, and the 3- to 8-membered heterocyclyl comprises at least one heteroatom selected from O, S and N; R₅ is selected from 3- to 8-membered cycloalkyl, 3- to 8-membered cycloalkenyl or 3- to 8-membered heterocyclyl, R₆ is selected from H or C1-C6 alkyl, or R₅ and R₆, together with the N atom to which they are attached, form 4- to 8-membered heterocyclyl; the 3- to 8-membered cycloalkyl, 3- to 8-membered cycloalkenyl, 3- to 8-membered heterocyclyl or 4- to 8-membered heterocyclyl comprises 0, 1, 2 or 3 heteroatoms selected from O, S and N, and is optionally substituted with one or more R_{z};
R₁ is -CN or -Z-R₁₂, wherein Z is a direct bond, -C1-C6 alkylene-, -C1-C6 alkylene-O-, -O-, -S-, -S(=O)-, -SO₂-, -NR₁₁-, -NR₁₁SO₂-, -SO₂NR₁₁-, -NR₁₁-S(=O)(=NH)-, -S(=O)(=NH)-, -C1-C6 alkylene-SO₂-, -C1-C6 alkylene-SO₂R₁₁-, - (C=O)-, -(C=O)NR₁₁-, -C=N(OH)- or -NR₁₁(C=O)-; or -Z-R₁₂ is -N=S(=O)-(R₁₂)₂, wherein a pair of two R₁₂, together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
X₇ is N or CR₇; X₈ is N or CR₈; X₉ is N or CR₉;
R₇, R₈ and R₉ are independently H, halogen, C1-C8 alkyl, C1-C6 haloalkyl, - OH, -O-R₈ₐ, -O-R_{8b} or -NRₐRₐ; where both R₇ and R₈ are present, R₇ and R₈, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S; where both Rₓ and R₉ are present, Rₓ and R₉, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
R₁₁ is H, R₁₁, or R_{11b}; R₁₂ is H, R₁₂ₐ or R_{12b};
R₈ₐ, R₁₁ₐ and R₁₂ₐ are independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, - S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, - N(Rₐ)C(=NRₐ)NRₐRₐ, -N(Rₐ)S(=O)₂R_{d}, -N(Rₐ)S(=O)₂NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, - C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, R₁₄ or oxo;
R_{8b}, R_{11b} and R_{12b} are independently selected from the group consisting of: C1-C6 alkyl substituted with 0, 1, 2, 3, 4 or 5 groups selected from F, Cl, Br, -ORₐ, - OC1-C6 haloalkyl or CN;
R₁₄, in each case, is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0 or 1 atom selected from O and S, wherein the monocyclic ring or bicyclic ring is substituted with 0, 1, 2 or 3 groups selected from: F, Cl, Br, C1-C6 alkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-6 alkylene NRₐRₐ, -OC2-6 alkylene ORₐ, -SRₐ, -S(=O)R_{b}, -S(=O)₂R_{b}, -S(=O)2NRₐRₐ, -NRₐRₐ, -N(Rₐ)C(=O)R_{b}, -N(Rₐ)C(=O)OR_{d}, -N(Rₐ)C(=O)NRₐRₐ, -N(Rₐ)C(=NRₐ)NRₐRₐ, -N(Rₐ)S(=O)₂R_{d}, - N(Rₐ)S(=O)₂NRₐRₐ, -NRₐC2-C6 alkylene NR^{a}R^{a}, -NR^{a}C2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ or oxo;
each Rₐ is independently H or R_{b};
each R_{b} is independently C1-C6 alkyl, phenyl or benzyl, wherein the C1-C6 alkyl is substituted with 0, 1, 2, or 3 substituents selected from: halogen, -OH, -O-C1-C6 alkyl, -NH₂, -NH-C1-C6 alkyl, -OC(=O)C1-C6 alkyl or -N(C1-C6 alkyl)(C1-C6 alkyl); and the phenyl or benzyl is substituted with 0, 1, 2, or 3 substituents selected from: halogen, C1-C6 alkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl);
each R_{z} is independently selected from the following groups: deuterium, halogen, C1-C6 alkyl, C3-C8 cycloalkyl, -C1-C6 alkylene -O-C1-C6 alkyl, C3-C8 cycloalkyl-C1-C6 alkylene, -OH, -CN, -O-C1-C6 alkyl, -O-C3-C8 cycloalkyl, - NH₂, -NH-C1-C6 alkyl, -OC(=O)C1-C6 alkyl, -N(C1-C6 alkyl)(C1-C6 alkyl), - S(O)-R_{z1}, -S(O)₂-R_{z1}, -C(O)-R_{z2}, -C(O)-NR_{z1}R_{z2}, phenyl, 5- to 6-membered monocyclic heteroaryl comprising 0, 1, 2, or 3 N atoms, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, - NH₂, -NH-C1-C6 alkyl, -OC(=O)C1-C6 alkyl or -N(C1-C6 alkyl)(C1-C6 alkyl); and two R_{z} attached to the same carbon atom may form 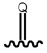 or two R_{z} attached to the same carbon atom, together with the carbon atom to which they are attached, may form C3-C8 cycloalkyl; the C1-C6 alkyl, C3-C8 cycloalkyl, -C1-C6 alkylene-O-C1-C6 alkyl, -O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl;
each R_{z1} is independently selected from the following groups: H, C1-C6 alkyl, or C1-C6 haloalkyl;
each R_{z2} is independently selected from the following groups: H, C1-C6 alkyl, C1-C6 haloalkyl, C1-C6 deuteroalkyl, or C3-C8 cycloalkyl;
Q is O or CR_{Q1}R_{Q2};
R_{Q1} and R_{Q2} are independently selected from the following groups: H, deuterium, halogen, C1-C6 alkyl, or C1-C6 haloalkyl.

2. The compound represented by formula (I), or the stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof according to claim 1, **characterized in that**
A is monocyclic aryl, monocyclic heteroaryl, bicyclic aryl, bicyclic heteroaryl or fused heterocyclyl, and optionally, the monocyclic aryl, monocyclic heteroaryl, bicyclic aryl, bicyclic heteroaryl or fused heterocyclyl is optionally substituted with one or more R_{z};
L is selected from -NR³-CO- or -NR³CONR³-, and each R³ is independently H, deuterium, C1-C6 alkylene or C1-C6 haloalkylene;
Rₓ is selected from -OR₄ or -NR₅R₆, wherein R₄ is selected from 3- to 8-membered cycloalkyl, 3- to 8-membered cycloalkenyl or 3- to 8-membered heterocyclyl, and the 3- to 8-membered heterocyclyl comprises at least one heteroatom selected from O, S and N; R₅ is selected from 3- to 8-membered cycloalkyl, 3- to 8-membered cycloalkenyl or 3- to 8-membered heterocyclyl, R₆ is selected from H or C1-C6 alkyl, or R₅ and R₆, together with the N atom to which they are attached, form 4- to 8-membered heterocyclyl; the 3- to 8-membered cycloalkyl, 3- to 8-membered cycloalkenyl, 3- to 8-membered heterocyclyl or 4- to 8-membered heterocyclyl comprises 0, 1, 2 or 3 heteroatoms selected from O, S and N, and is optionally substituted with one or more R_{z};
R₁ is -CN or -Z-R₁₂, wherein Z is a direct bond, -C1-C6 alkylene-, -C1-C6 alkylene-O-, -O-, -S-, -S(=O)-, -SO₂-, -NR₁₁-, -NR₁₁SO₂-, -SO₂NR₁₁-, -NR₁₁-S(=O)(=NH)-, -S(=O)(=NH)-, -C1-C6 alkylene-SO₂-, -C1-C6 alkylene-SO₂R₁₁-, - (C=O)-, -(C=O)NR₁₁-, -C=N(OH)- or -NR₁₁(C=O)-; or -Z-R₁₂ is -N=S(=O)-(R₁₂)₂, wherein a pair of two R₁₂, together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
X₇ is N or CR₇; X₈ is N or CR₈; X₉ is N or CR₉;
R₇, R₈ and R₉ are independently H, halogen, C1-C8 alkyl, C1-C6 haloalkyl, - OH, -O-R₈ₐ, -O-R_{8b} or -NRₐRₐ; where both R₇ and R₈ are present, R₇ and R₈, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S; where both Rₓ and R₉ are present, Rₓ and R₉, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
R₁₁ is H, R₁₁ₐ or R_{11b}; R₁₂ is H, R₁₂, or R_{12b};
R₈ₐ, R₁₁ₐ and R₁₂ₐ are independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, - S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, - N(Rₐ)C(=NRₐ)NRₐRₐ, -N(Rₐ)S(=O)₂R_{d}, -N(Rₐ)S(=O)₂NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, - C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, R₁₄ or oxo;
R_{8b}, R_{11b} and R_{12b} are independently selected from the group consisting of: C1-C6 alkyl substituted with 0, 1, 2, 3, 4 or 5 groups selected from F, Cl, Br, -ORₐ, - OC1-C6 haloalkyl or CN;
R₁₄, in each case, is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0 or 1 atom selected from O and S, wherein the monocyclic ring or bicyclic ring is substituted with 0, 1, 2 or 3 groups selected from: F, Cl, Br, C1-C6 alkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-6 alkylene NRₐRₐ, -OC2-6 alkylene ORₐ, -SRₐ, -S(=O)R_{b}, -S(=O)₂R_{d}, -S(=O)2NRₐRₐ, -NRₐRₐ, -N(Rₐ)C(=O)R_{b}, -N(Rₐ)C(=O)OR_{d}, -N(Rₐ)C(=O)NRₐRₐ, -N(Rₐ)C(=NRₐ)NRₐRₐ, -N(Rₐ)S(=O)₂R_{d}, - N(Rₐ)S(=O)₂NRₐRₐ, -NRₐC2-C6 alkylene NR^{a}R^{a}, -NR^{a}C2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ or oxo;
each Rₐ is independently H or R_{b};
each R_{b} is independently C1-C6 alkyl, phenyl or benzyl, wherein C1-C6 alkyl is substituted with 0, 1, 2, or 3 substituents selected from: halogen, -OH, -O-C1-C6 alkyl, -NH₂, -NH-C1-C6 alkyl, -OC(=O)C1-C6 alkyl or -N(C1-C6 alkyl)(C1-C6 alkyl); and phenyl or benzyl is substituted with 0, 1, 2, or 3 substituents selected from: halogen, C1-C6 alkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl);
each R_{z} is independently selected from the following groups: deuterium, halogen, C1-C6 alkyl, C3-C8 cycloalkyl, -C1-C6 alkylene -O-C1-C6 alkyl, C3-C8 cycloalkyl-C1-C6 alkylene, -OH, -CN, -O-C1-C6 alkyl, -O-C3-C8 cycloalkyl, - NH₂, -NH-C1-C6 alkyl, -OC(=O)C1-C6 alkyl, -N(C1-C6 alkyl)(C1-C6 alkyl), - S(O)-R_{z1}, -S(O)₂-R_{z1}, -C(O)-R_{z2}, -C(O)-NR_{z1}R_{z2}, 5- to 6-membered monocyclic heteroaryl comprising 0, 1, 2, or 3 N atoms, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two R_{z} attached to the same carbon atom may form 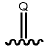 or two R_{z} attached to the same carbon atom, together with the carbon atom to which they are attached, may form C3-C8 cycloalkyl; the C1-C6 alkyl, C3-C8 cycloalkyl, -C1-C6 alkylene-O-C1-C6 alkyl, - O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl;
each R_{z1} is independently selected from the following groups: H, C1-C6 alkyl, or C1-C6 haloalkyl;
each R_{z2} is independently selected from the following groups: H, C1-C6 alkyl, C1-C6 haloalkyl, C1-C6 deuteroalkyl, or C3-C8 cycloalkyl;
Q is O or CR_{Q1}R_{Q2};
R_{Q1} and R_{Q2} are independently selected from the following groups: H, deuterium, halogen, C1-C6 alkyl, or C1-C6 haloalkyl.

3. The compound represented by formula (I), or the stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof according to claim 1,
**characterized in that** A is monocyclic aryl, monocyclic heteroaryl, bicyclic aryl, bicyclic heteroaryl or fused heterocyclyl, and optionally, the monocyclic aryl, monocyclic heteroaryl, bicyclic aryl, bicyclic heteroaryl or fused heterocyclyl is optionally substituted with one or more R_{z};
L is selected from -NR³-CO- or -NR³CONR³-, and each R³ is independently H, deuterium, C1-C6 alkylene or C1-C6 haloalkylene;
Rₓ is selected from -OR₄ or -NR₅R₆, wherein R₄ is selected from 3- to 8-membered cycloalkyl, 3- to 8-membered cycloalkenyl or 3- to 8-membered heterocyclyl, and the 3- to 8-membered heterocyclyl comprises at least one heteroatom selected from O, S and N; R₅ is selected from 3- to 8-membered cycloalkyl, 3- to 8-membered cycloalkenyl or 3- to 8-membered heterocyclyl, R₆ is selected from H or C1-C6 alkyl, or R₅ and R₆, together with the N atom to which they are attached, form 4- to 8-membered heterocyclyl; the 3- to 8-membered cycloalkyl, 3- to 8-membered cycloalkenyl, 3- to 8-membered heterocyclyl or 4- to 8-membered heterocyclyl comprises 0, 1, 2 or 3 heteroatoms selected from O, S and N, and is optionally substituted with one or more R_{z};
R₁ is -CN or -Z-R₁₂, wherein Z is a direct bond, -C1-C6 alkylene-, -C1-C6 alkylene-O-, -O-, -S-, -S(=O)-, -SO₂-, -NR₁₁-, -NR₁₁SO₂-, -SO₂NR₁₁-, -NR₁₁-S(=O)(=NH)-, -S(=O)(=NH)-, -C1-C6 alkylene-SO₂-, -C1-C6 alkylene-SO₂R₁₁-, - (C=O)-, -(C=O)NR₁₁-, -C=N(OH)- or -NR₁₁(C=O)-; or -Z-R₁₂ is -N=S(=O)-(R₁₂)₂, wherein a pair of two R₁₂, together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
X₇ is N or CR₇; X₈ is N or CR₈; X₉ is N or CR₉;
R₇, R₈ and R₉ are independently H, halogen, C1-C8 alkyl, C1-C6 haloalkyl, - OH, -O-R₈ₐ, -O-R_{8b} or -NRₐRₐ; where both R₇ and R₈ are present, R₇ and R₈, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S; where both Rₓ and R₉ are present, Rₓ and R₉, together with the atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
R₁₁ is H, R₁₁ₐ or R_{11b}; R₁₂ is H, R₁₂ₐ or R_{12b};
R₈ₐ, R₁₁ₐ and R₁₂ₐ are independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-C6 alkylene NRₐRₐ, -OC2-C6 alkylene ORₐ, -SRₐ, -S(=O)R_{b}, -S(=O)₂R_{b}, - S(=O)₂NRₐRₐ, -NRₐRₐ, -N(Rₐ)C(=O)R_{b}, -N(Rₐ)C(=O)OR_{b}, -N(Rₐ)C(=O)NRₐRₐ, - N(Rₐ)C(=NRₐ)NRₐRₐ, -N(Rₐ)S(=O)₂R_{d}, -N(Rₐ)S(=O)₂NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, - C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, R₁₄ or oxo;
R_{8b}, R_{11b} and R_{12b} are independently selected from the group consisting of: C1-C6 alkyl substituted with 0, 1, 2, 3, 4 or 5 groups selected from F, Cl, Br, -ORₐ, - OC1-C6 haloalkyl or CN;
R₁₄, in each case, is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0 or 1 atom selected from O and S, wherein the monocyclic ring or bicyclic ring is substituted with 0, 1, 2 or 3 groups selected from: F, Cl, Br, C1-C6 alkyl, -ORₐ, -OC1-C6 haloalkyl, CN, -C(=O)R_{b}, -C(=O)ORₐ, -C(=O)NRₐRₐ, -C(=NRₐ)NRₐRₐ, -OC(=O)R_{b}, -OC(=O)NRₐRₐ, -OC2-6 alkylene NRₐRₐ, -OC2-6 alkylene ORₐ, -SRₐ, -S(=O)R_{b}, -S(=O)₂R_{b}, -S(=O)2NRₐRₐ, -NRₐRₐ, -N(Rₐ)C(=O)R_{b}, -N(Rₐ)C(=O)OR_{d}, -N(Rₐ)C(=O)NRₐRₐ, -N(Rₐ)C(=NRₐ)NRₐRₐ, -N(Rₐ)S(=O)₂R_{d}, - N(Rₐ)S(=O)2NRₐRa, -NRₐC2-C6 alkylene NRₐRₐ, -NRₐC2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ or oxo;
each Rₐ is independently H or R_{b};
each R_{b} is independently C1-C6 alkyl, phenyl or benzyl, wherein C1-C6 alkyl is substituted with 0, 1, 2, or 3 substituents selected from: halogen, -OH, -O-C1-C6 alkyl, -NH₂, -NH-C1-C6 alkyl, -OC(=O)C1-C6 alkyl or -N(C1-C6 alkyl)(C1-C6 alkyl); and phenyl or benzyl is substituted with 0, 1, 2, or 3 substituents selected from: halogen, C1-C6 alkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl);
each R_{z} is independently selected from the following groups: deuterium, halogen, C1-C6 alkyl, C3-C8 cycloalkyl, C1-C6 haloalkyl, C3-C8 cycloalkyl-C1-C6 alkylene, -OH, -CN, -O-C1-C6 alkyl, -NH₂, -NH-C1-C6 alkyl, -OC(=O)C1-C6 alkyl, -N(C1-C6 alkyl)(C1-C6 alkyl), -S(O)-R_{z1}, -S(O)₂-R_{z1}, or saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, - NH₂, -NH-C1-C6 alkyl, -OC(=O)C1-C6 alkyl or -N(C1-C6 alkyl)(C1-C6 alkyl); and two R_{z} attached to the same carbon atom may form 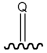 or two R_{z} attached to the same carbon atom, together with the carbon atom to which they are attached, may form C3-C8 cycloalkyl; the C1-C6 alkyl, C3-C8 cycloalkyl, -O-C1-C6 alkyl, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy, C1-C4 haloalkyl, or C3-C8 halocycloalkyl;
each R_{z1} is independently selected from the following groups: C1-C6 alkyl, or C1-C6 haloalkyl;
Q is O or CR_{Q1}R_{Q2};
R_{Q1} and R_{Q2} are independently selected from the following groups: H, deuterium, halogen, C1-C6 alkyl, or C1-C6 haloalkyl.

4. The compound, or the stereoisomer, tautomer, diastereomer, racemate, cis-trans isomer, isotopically labeled compound (preferably deuterated compound), nitrogen oxide, solvate, hydrate, crystal form, ester, metabolite, pharmaceutically acceptable salt or prodrug thereof according to claim 1, **characterized in that**
each R_{z} is independently selected from the following groups: deuterium, halogen, C1-C6 alkyl, C3-C8 cycloalkyl, C1-C6 haloalkyl, -OH, -O-C1-C6 alkyl, - NH₂, -NH-C1-C6 alkyl, -OC(=O)C1-C6 alkyl or -N(C1-C6 alkyl)(C1-C6 alkyl); saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); and two or more of the substituents, together with the carbon atom to which they are attached, may form C3-C8 cycloalkyl; the C1-C6 alkyl, C3-C8 cycloalkyl, or -O-C1-C6 alkyl may be optionally substituted with substituents selected from deuterium, F, Cl, Br, I, C1-C6 alkyl, C3-C8 cycloalkyl, C1-C6 alkoxy or C1-C4 haloalkyl.

5. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-4, **characterized in that** formula (I) is as represented by formula (I)-1, formula (I)-2, or formula (I)-3: 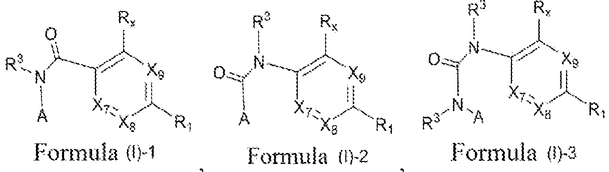 the symbols of formula (I)-1 to formula (I)-3 are as defined in any one of claims 1-4.

6. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-5, **characterized in that** Rₓ is selected from the group consisting of: 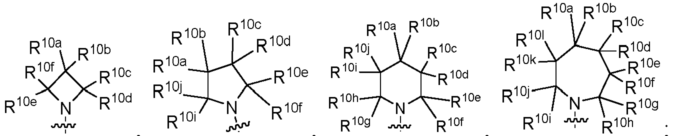
each of R^{10a} , R^{10b}, R^{10c}, R^{10d}, R^{10e}, R^{10f} , R^{10g}, R^{10h}, R¹⁰ⁱ, R^{10j}, R^{10k} and R^{10l} is H, deuterium, halogen, -CN, -OH, C1-C6 alkyl, C1-C6 alkoxy, C1-C6 haloalkyl, C3-C8 cycloalkyl, -NH₂, -NH(C1-C6 alkyl), -N(C1-C6 alkyl)(C1-C6 alkyl), or saturated, partially saturated or unsaturated 3-, 4-, 5-, 6- or 7-membered monocyclic ring or 5-, 6-, 7-, 8-, 9-, 10-, 11- or 12-membered bicyclic ring, which comprises 1-3 heteroatoms selected from N, O and S, and the C1-C6 alkyl, C1-C6 alkoxy, C1-C6 haloalkyl, or C3-C8 cycloalkyl is optionally substituted with 1-5 substituents selected from deuterium, halogen, -OH, -CN, C1-C6 alkyl, C1-C6 haloalkyl, C1-C6 alkoxy, or -O-C1-C6 haloalkyl; the monocyclic ring or bicyclic ring is substituted with 0, 1, 2, or 3 groups selected from: F, Cl, Br, C1-C6 alkyl, C1-C6 haloalkyl, - ORₘ, -OC1-C6 haloalkyl, CN, -C(=O)Rₙ, -C(=O)ORₘ, -C(=O)NRₘRₘ, - C(=NRₘ)NRₘRₘ, -OC(=O)Rₙ, -OC(=O)NRₘRₘ, -OC2-C6 alkylene NRₘRₘ, -OC2-C6 alkylene ORₘ, -SRₘ, -S(=O)Rₙ, -S(=O)₂Rₙ, -S(=O)₂NRₘRₘ, -NRₘRₘ, - N(Rₘ)C(=O)Rₙ, -N(Rₘ)C(=O)ORₙ, -N(Rₘ)C(=O)NRₘRₘ, - N(Rₘ)C(=NRₘ)NRₘRₘ, -N(Rₘ)S(=O)₂Rₙ, -N(Rₘ)S(=O)₂NRₘRₘ, -NRₘC2-6 alkylene NRₘRₘ, -NRₘC2-C6 alkylene ORₘ, -C1-C6 alkylene NRₘRₘ, -C1-C6 alkylene ORₘ, -C1-C6 alkylene N(Rₘ)C(=O)Rₙ, -C1-C6 alkylene OC(=O)Rₙ, -C1-C6 alkylene C(=O)NRₘRₘ, -C1-C6 alkylene C(=O)ORₘ or oxo;
or alternatively, each of the R^{10a} and R^{10b} pair, R^{10c} and R^{10d} pair, R^{10c} and R^{10f} pair, R^{10g} and R^{10h} pair, R¹⁰ⁱ and R^{10j} pair, and R^{10k} and R^{10l} pair, independently together with the carbon atom to which they are each attached, may form saturated or partially saturated 3-, 4-, 5-, or 6-membered monocyclic ring spiro-connected to the Rₓ ring, wherein the 3-, 4-, 5-, or 6-membered monocyclic ring comprises 0, 1, 2 or 3 N atoms and 0, 1 or 2 atoms selected from O and S, and further, the 3-, 4-, 5-, or 6-membered monocyclic ring is substituted with 0, 1, 2, or 3 groups selected from: F, Cl, Br, C1-C6 alkyl, C1-C4 haloalkyl, -ORₘ, -OC1-C4 haloalkyl, CN, - NRₘRₘ or oxo;
Rₘ and Rₙ are each independently selected from H or -C1-C6 alkyl;
or alternatively, the R^{10a} and R^{10b} pair, R^{10c} and R^{10d} pair, R^{10c} and R^{10f} pair, R^{10g} and R^{10h} pair, R¹⁰ⁱ and R^{10j} pair, and R^{10k} and R^{10l} pair may form 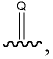 wherein Q is CR_{Q1}R_{Q2}; R_{Q1} and R_{Q2} are independently selected from the following groups: H, deuterium, halogen, C1-C6 alkyl, or C1-C6 haloalkyl.

7. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-6, **characterized in that** Rₓ is

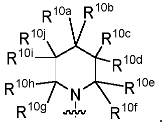

8. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-7, **characterized in that** each of R^{10c}, R^{10d}, R^{10e}, R^{10f}, R^{10g}, R^{10h}, R¹⁰ⁱ and R^{10j} is H, deuterium, halogen, -CN, -OH, C1-C6 alkyl, C1-C6 alkoxy, C1-C6 haloalkyl, -NH₂, -NH(C1-C6 alkyl) or -N(C1-C6 alkyl)(C1-C6 alkyl), and the C1-C6 alkyl, C1-C6 alkoxy, or C1-C6 haloalkyl is optionally substituted with 1-5 substituents selected from deuterium, halogen, -OH, -CN, C1-C6 alkyl, C1-C6 haloalkyl, C1-C6 alkoxy, or -O-C1-C6 haloalkyl; the R^{10a} and R^{10b} pair forms 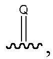 , and Q is CR_{Q1}R_{Q2}; R_{Q1} and R_{Q2} are independently selected from the following groups: H, deuterium, halogen, C1-C6 alkyl, or C1-C6 haloalkyl, or the R^{10a} and R^{10b} pair, together with the carbon atom to which they are attached, forms C3-C8 cycloalkyl spiro-connected to the Rₓ ring, and further, the C3-C8 cycloalkyl is substituted with 0, 1, 2, or 3 groups selected from: F, Cl, Br, C1-C6 alkyl, C1-C4 haloalkyl, -OH, C1-C6 alkoxy, -OC1-C4 haloalkyl, -CN, -NH₂, -NH(C1-C6 alkyl), -N(C1-C6 alkyl)(C1-C6 alkyl) or oxo.

9. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 8, **characterized in that** Rₓ is selected from:

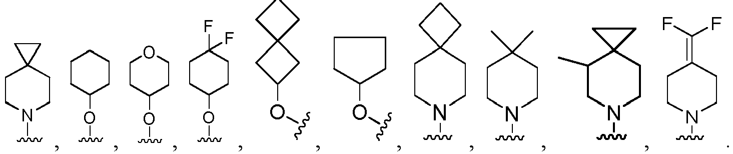

10. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-9, **characterized in that** formula (I) is as represented by formula (I)-4-1, formula (I)-4-2, formula (I)-5-1, formula (I)-5-2, formula (I)-6-1 or formula (I)-6-2:

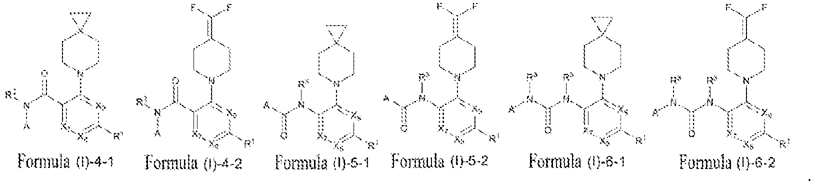

11. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-10, **characterized in that** R₁ is group -Z-R₁₂, wherein -Z is a single bond, -C1-C4 alkylene-, -C1-C4 alkylene-O-, -O-, -S-, - S(=O)-, -SO₂-, -NH-, -NHSO₂-, -SO₂NH-, -NH-S(=O)(=NH)-, -S(=O)(=NH)-, -C1-C4 alkylene-SO₂-, -(C=O)-, -(C=O)NH-, -C=N(OH)- or -NH(C=O)-; and/or
(a) R₁₂ is H;
(b) R₁₂ is oxetanyl or cyclopropyl substituted with 0, 1, 2 or 3 groups selected from OH, halogen (such as F or Cl), or C1-C6 alkoxy; or
(c) R₁₂ is C1-C6 alkyl substituted with 0, 1, 2 or 3 groups selected from OH, halogen (such as F), or C1-C6 alkoxy.

12. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-10, **characterized in that** the group -Z-R₁₂ is - N=S(=O)-(R₁₂)₂, wherein a pair of two R₁₂, alternatively together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
or in the group -Z-R₁₂, Z is a single bond, and R₁₂ is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, -C(=NRₐ)NRₐRₐ, - OC(=O)R_{b}, -OC(=O)NRₐRₐ, -OC2-C6 alkylene NRₐRₐ, -OC2-C6 alkylene ORₐ, - SRₐ, -S(=O)R_{b}, -S(=O)₂R_{b}, -S(=O)₂NRₐRₐ, -NRₐRₐ, -N(Rₐ)C(=O)R_{b}, - N(Rₐ)C(=O)OR_{b}, -N(Rₐ)C(=O)NRₐRₐ, -N(Rₐ)C(=NRₐ)NRₐRₐ, -N(Rₐ)S(=O)₂R_{b}, - N(Rₐ)S(=O)2NRₐRₐ, -NRₐC2-6 alkylene NRₐRₐ, -NRₐC2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, or oxo, and Rₐ and R_{b} are independently selected from H or -C1-C6 alkyl.

13. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-12, **characterized in that** R₁ is group -Z-R₁₂, wherein Z is -NHSO₂- or -SO₂NH-; and R₁₂ is oxetanyl or cyclopropyl substituted with 0, 1, 2 or 3 groups selected from OH, halogen (such as F), or C1-C6 alkoxy, or R₁₂ is C1-C6 alkyl substituted with 0, 1, 2 or 3 groups selected from OH, halogen (such as F), or C1-C6 alkoxy.

14. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-13, **characterized in that** R₁ is selected from the following groups:

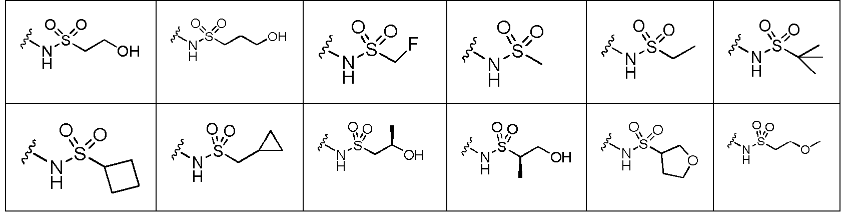

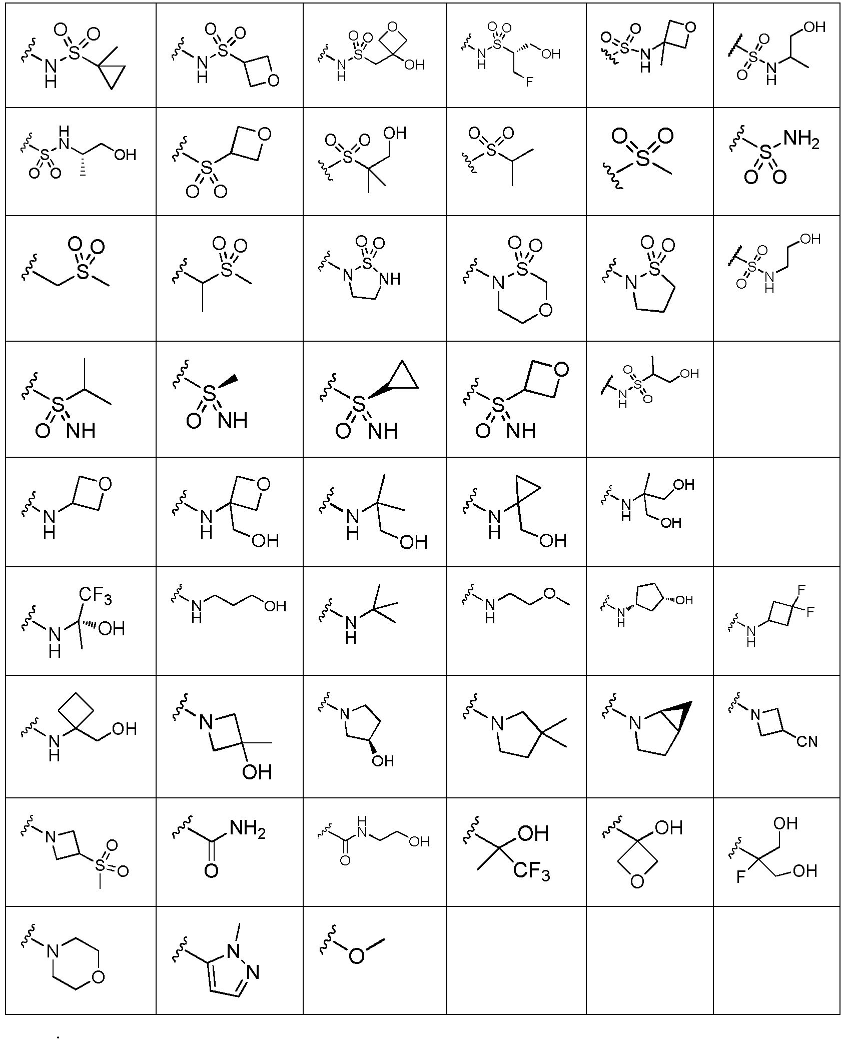

15. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-14, **characterized in that** formula (I) is as represented by formula (I)-1-1, formula (I)-1-2, formula (I)-2-1, formula (I)-2-2, formula (I)-3-1 or formula (I)-3-2:

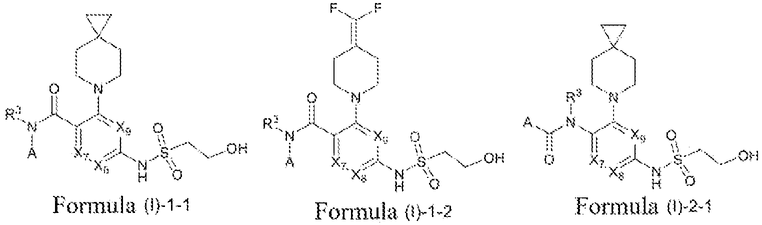

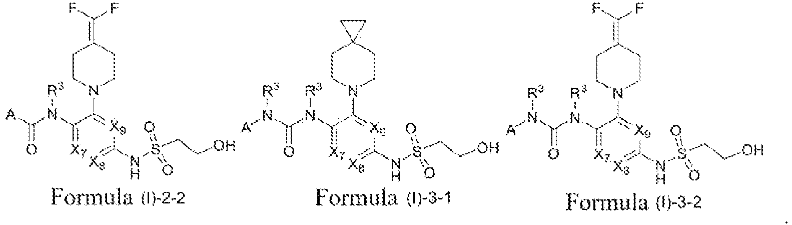

16. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-15, **characterized in that** each R³ is independently H, C1-C4 alkyl or C1-C4 haloalkyl.

17. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-16, **characterized in that** each R³ is independently H.

18. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-17, **characterized in that** X₇ is CR₇.

19. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-18, **characterized in that** R₇ is -NRₐRₐ.

20. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-19, **characterized in that** R₇ is -NH₂ or -NH-C1-C6 alkyl.

21. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-20, **characterized in that** X₇ is CR₇, X₈ is CR₈, and X₉ is CR₉.

22. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-21, **characterized in that** X₇ is CR₇ and R₇ is -NH₂ or -NH-C1-C6 alkyl, X₈ is CH, and X₉ is CH.

23. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-22, **characterized in that** X₇ is CH, X₈ is N or CR₈, and X₉ is N or CR₉; preferably, X₇ is CH, X₈ is CR₈, and X₉ is CR₉; more preferably, X₇ is CH, X₈ is CH, and X₉ is CH.

24. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-23, **characterized in that** X₉ is N.

25. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-17, **characterized in that** X₇ is N and/or X₈ is N.

26. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-25, **characterized in that** A is selected from phenyl, 5- to 6-membered nitrogen-containing heteroaryl, benzomonoheterocyclyl, benzobisheterocyclyl, 5- to 6-membered nitrogen-containing heteroaryl fused monoheterocyclyl, 5- to 6-membered nitrogen-containing heteroaryl fused bisheterocyclyl, benzomonoheteroaryl, or 5- to 6-membered nitrogen-containing heteroaryl fused monoheteroaryl;
optionally, the phenyl, 5- to 6-membered nitrogen-containing heteroaryl, benzomonoheterocyclyl, benzobisheterocyclyl, 5- to 6-membered nitrogen-containing heteroaryl fused monoheterocyclyl, 5- to 6-membered nitrogen-containing heteroaryl fused bisheterocyclyl, benzomonoheteroaryl, or 5- to 6-membered nitrogen-containing heteroaryl fused monoheteroaryl is substituted with one or more R_{z},
monoheterocyclyl in the benzomonoheterocyclyl, or 5- to 6-membered nitrogen-containing heteroaryl fused monoheterocyclyl contains 0, 1, 2 or 3 atoms selected from N, O and S;
monoheteroaryl in the benzomonoheteroaryl, or 5- to 6-membered nitrogen-containing heteroaryl fused monoheteroaryl contains 0, 1, 2 or 3 atoms selected from N, O and S;
bisheterocyclyl in the benzobisheterocyclyl and 5- to 6-membered nitrogen-containing heteroaryl fused bisheterocyclyl contains 0, 1, 2 or 3 atoms selected from N, O and S.

27. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-26, **characterized in that** A is a group selected from group A1, group A2 or group A3,
the group A1 includes the following groups: 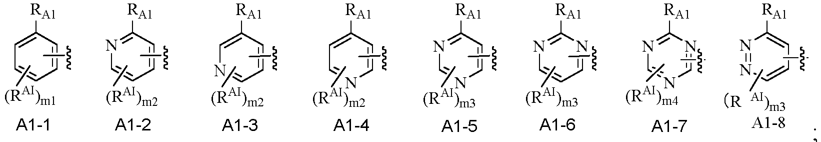
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -CN, -O-C1-C6 alkyl, C1-C6 alkylene-O-C1-C6 alkyl, C1-C6 haloalkoxy, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 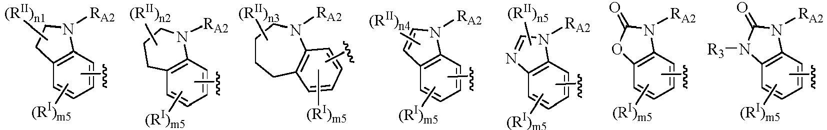 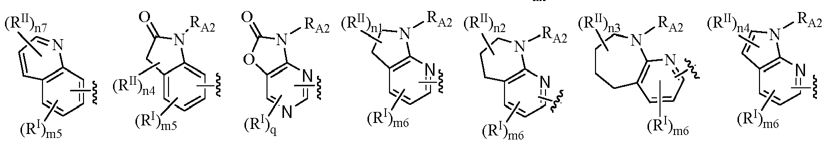 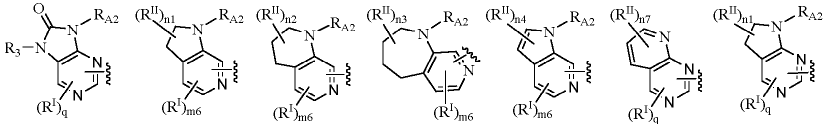 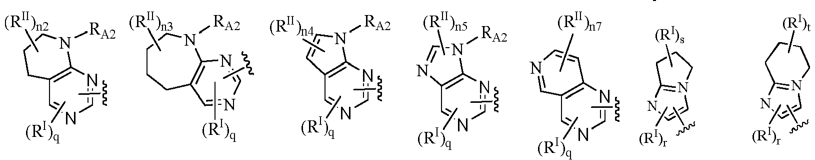 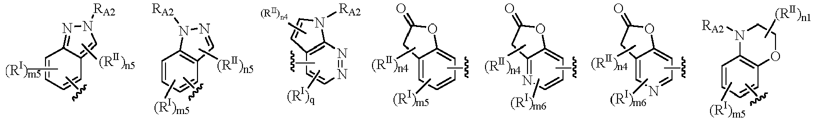 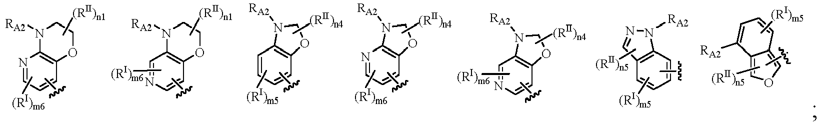
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, C3-C8 cycloalkyl-C1-C6 alkylene, -OH, C1-C6 alkoxy, -NH₂, -NH-C1-C6 alkyl, -OC(=O)C1-C6 alkyl, -N(C1-C6 alkyl)(C1-C6 alkyl), -S(O)-R_{z1} or -S(O)₂-R_{z1}, and the C1-C6 alkyl, C3-C8 cycloalkyl, C1-C6 haloalkyl, C1-C6 alkoxy, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkyl, C1-C6 alkoxy or C1-C4 haloalkyl;
each R_{z1} is independently selected from the following groups: C1-C6 alkyl, or C1-C6 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 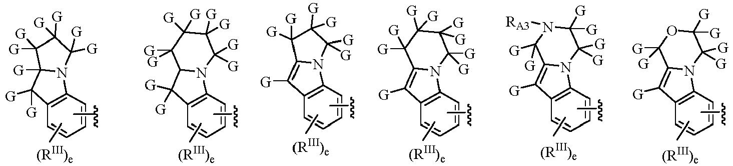 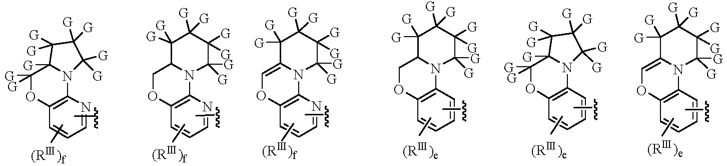 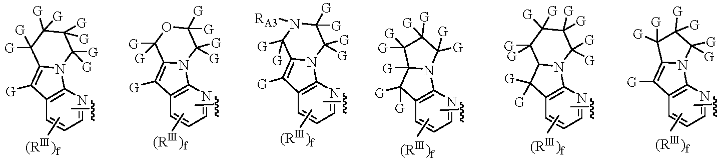 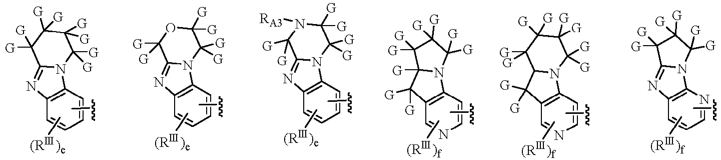 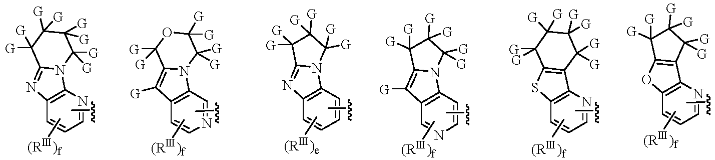 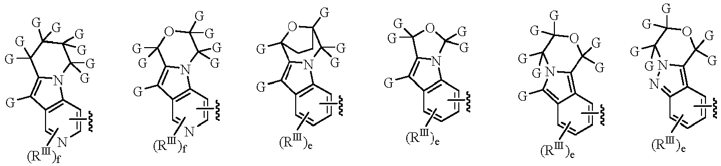 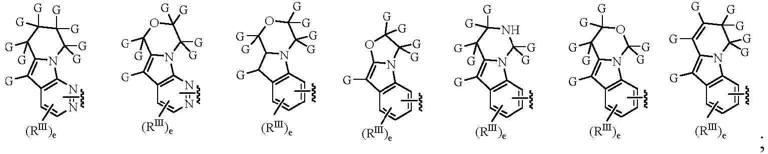
in the group A3,
each G is independently selected from hydrogen, deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl), and the C1-C6 alkyl, C3-C8 cycloalkyl, C1-C6 haloalkyl, and C1-C6 alkoxy may be optionally substituted with substituents selected from deuterium, halogen, C1-C6 alkyl, C3-C8 cycloalkyl, C1-C6 alkoxy or C1-C4 haloalkyl, or two G attached to the same carbon atom form an oxo group (=O) or two G attached to the same carbon atom, together with the carbon atom to which they are attached, may form C3-C8 cycloalkyl;
R_{A3} is independently selected from H, deuterium, halogen, C1-C6 alkyl, C3-C8 cycloalkyl, C1-C6 haloalkyl, -OH, -CN, -O-C3-C8 cycloalkyl, C1-C6 alkoxy, -NH₂, -NH-C1-C6 alkyl, -C(=O)NH-C1-C6 alkyl, -OC(=O)C1-C6 alkyl, -C(=O)C3-C8 cycloalkyl, -N(C1-C6 alkyl)(C1-C6 alkyl), -C(=O)N(C1-C6 alkyl)(C1-C6 alkyl), phenyl, 5- to 6-membered monocyclic heteroaryl comprising 1, 2 or 3 N atoms, or 5- to 6-membered monocyclic heteroaryl comprising 0, 1, 2 or 3 N atoms and 1 or 2 atoms selected from O, and the C1-C6 alkyl, C3-C8 cycloalkyl, C1-C6 alkoxy, or 5-to 6-membered monocyclic heteroaryl may be optionally substituted with substituents selected from deuterium, F, Cl, Br, I, C1-C6 alkyl, C3-C8 cycloalkyl, C1-C6 alkoxy or C1-C4 haloalkyl;
R^{III} is independently selected from deuterium, halogen, C1-C6 alkyl, C3-C8 cycloalkyl, C1-C6 haloalkyl, -OH, -CN, -O-C3-C8 cycloalkyl, C1-C6 alkoxy, -NH₂, -NH-C1-C6 alkyl, -C(=O)NH-C1-C6 alkyl, -OC(=O)C1-C6 alkyl, -C(=O)C3-C8 cycloalkyl, -N(C1-C6 alkyl)(C1-C6 alkyl), -C(=O)N(C1-C6 alkyl)(C1-C6 alkyl), phenyl, -5- to 6-membered monocyclic heteroaryl-C1-C6 alkyl comprising 1, 2 or 3 N atoms, 5- to 6-membered monocyclic heteroaryl comprising 1, 2 or 3 N atoms, or 5- to 6-membered monocyclic heteroaryl comprising 0, 1, 2 or 3 N atoms and 1 or 2 atoms selected from O, and the C1-C6 alkyl, C3-C8 cycloalkyl, C1-C6 alkoxy, or 5-to 6-membered monocyclic heteroaryl may be optionally substituted with substituents selected from deuterium, halogen, C1-C6 alkyl, C3-C8 cycloalkyl, C1-C6 alkoxy or C1-C4 haloalkyl;
e is selected from 0, 1, 2 or 3; f is selected from 0, 1 or 2.

28. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-27, **characterized in that** A is a group selected from group A1, group A2 or group A3,
the group A1 includes the following groups: 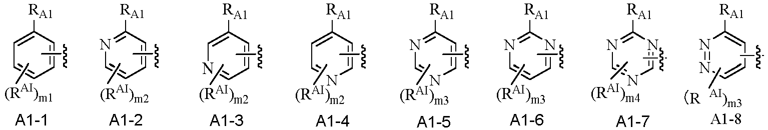
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -CN, -O-C1-C6 alkyl, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 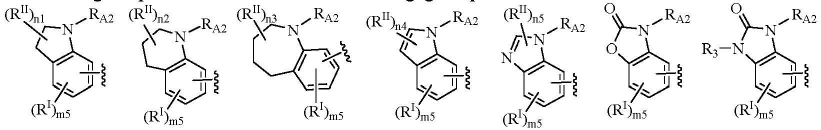 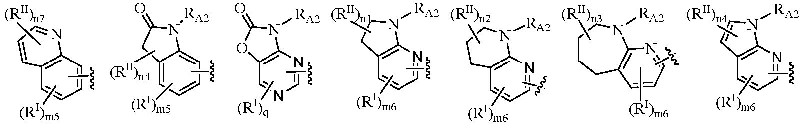 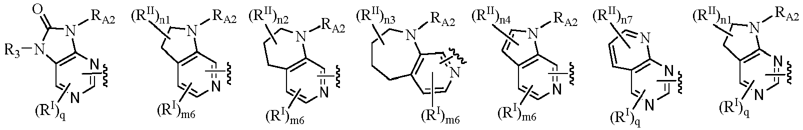 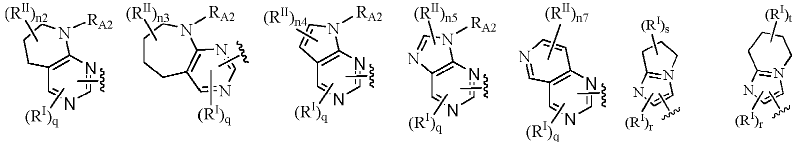 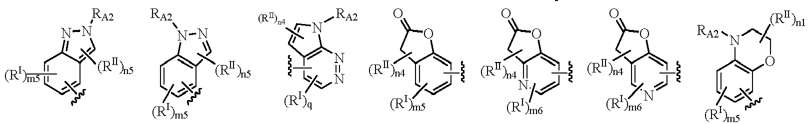 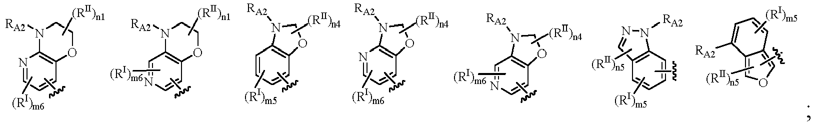
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, C3-C8 cycloalkyl-C1-C6 alkylene, -OH, C1-C6 alkoxy, -NH₂, -NH-C1-C6 alkyl, -OC(=O)C1-C6 alkyl, -N(C1-C6 alkyl)(C1-C6 alkyl), -S(O)-R_{z1} or -S(O)₂-R_{z1}, and the C1-C6 alkyl, C3-C8 cycloalkyl, C1-C6 haloalkyl, C1-C6 alkoxy, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy or C1-C4 haloalkyl;
each R_{z1} is independently selected from the following groups: C1-C6 alkyl, or C1-C6 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 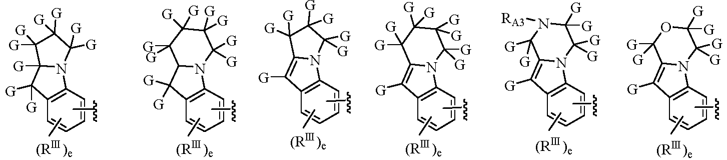 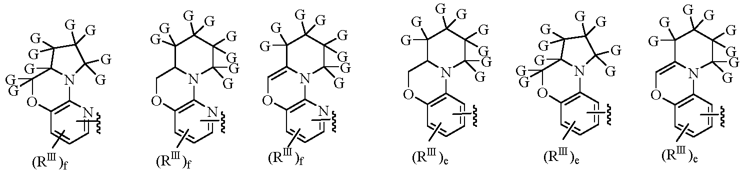 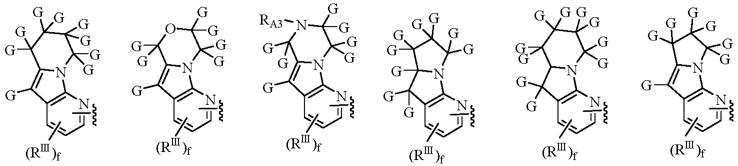 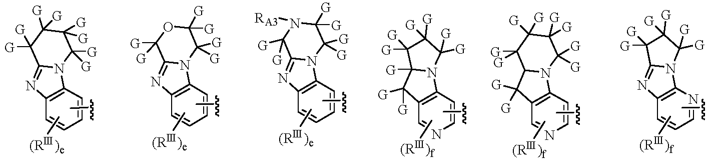 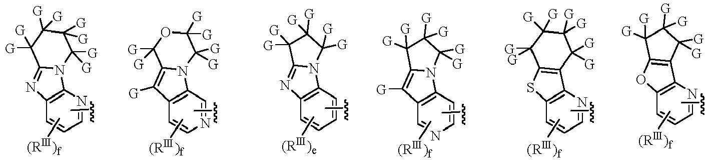 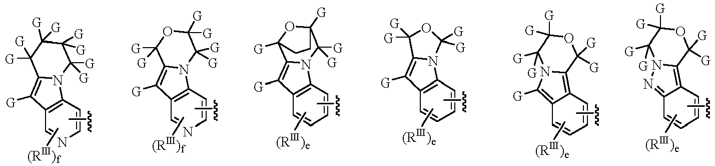 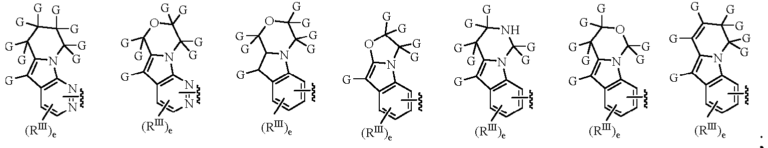
in the group A3, G, R_{A3} and R^{III} are each independently as defined in claim 27;
e is selected from 0, 1, 2 or 3; f is selected from 0, 1 or 2.

29. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-28, **characterized in that** A is a group selected from group A1, group A2 or group A3,
the group A1 includes the following groups: 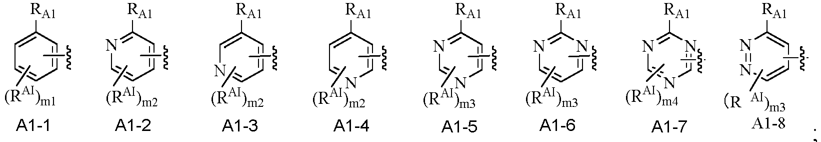
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -CN, -O-C1-C6 alkyl, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 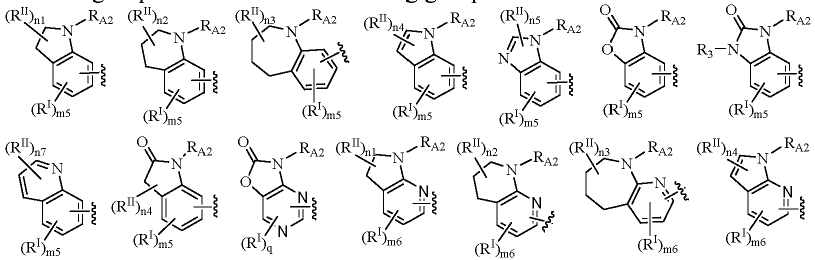 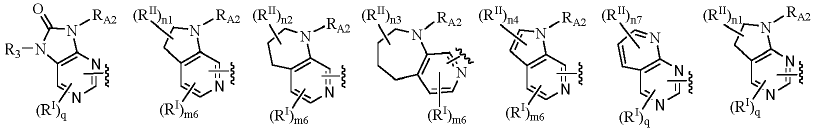 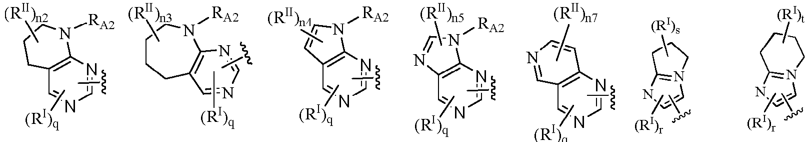 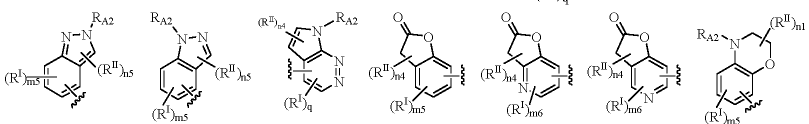 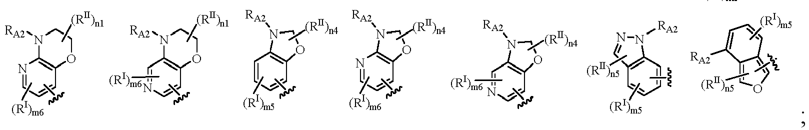
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, C3-C8 cycloalkyl-C1-C6 alkylene, -OH, C1-C6 alkoxy, -NH₂, -NH-C1-C6 alkyl, -OC(=O)C1-C6 alkyl, -N(C1-C6 alkyl)(C1-C6 alkyl), -S(O)-R_{z1} or -S(O)₂-R_{z1}, and the C1-C6 alkyl, C3-C8 cycloalkyl, C1-C6 haloalkyl, C1-C6 alkoxy, or C3-C8 cycloalkyl-C1-C6 alkylene may be optionally substituted with one or more substituents selected from deuterium, F, Cl, Br, I, -OH, C1-C6 alkyl, C3-C8 cycloalkyl, C1-C6 alkoxy or C1-C4 haloalkyl;
each R_{z1} is independently selected from the following groups: C1-C6 alkyl, or C1-C6 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 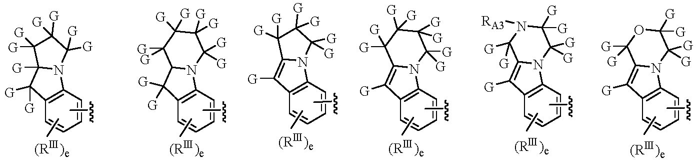 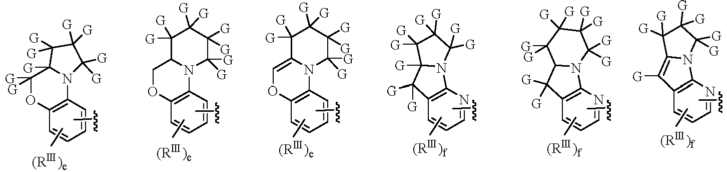 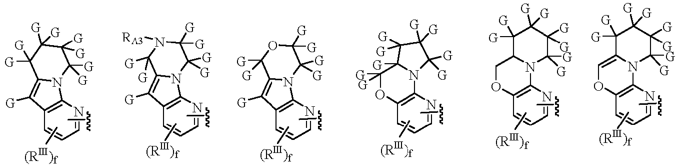 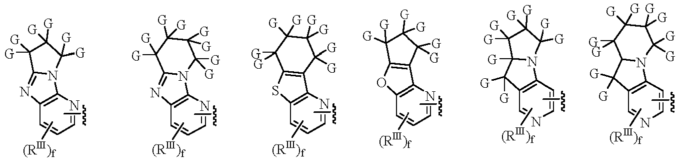 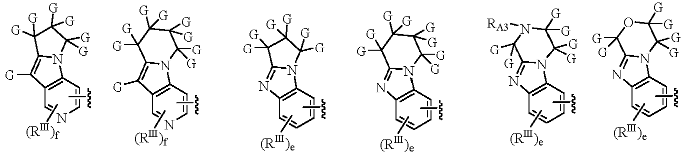 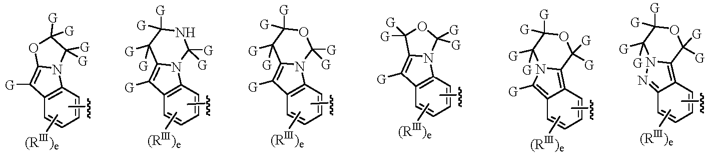 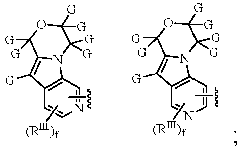
in the group A3,
G, R_{A3} and R^{III} are each independently as defined in claim 27; e is selected from 0, 1, 2 or 3; f is selected from 0, 1 or 2.

30. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-29, **characterized in that** A is a group selected from group A1, group A2 or group A3,
the group A1 includes the following groups: 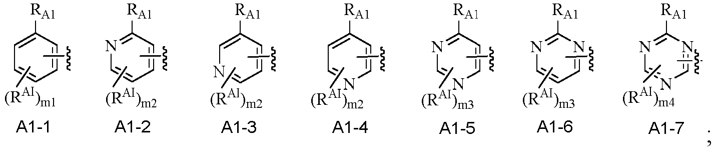
in the group A1, each R_{A1} is independently selected from saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl); each R^{AI} is independently selected from deuterium, halogen, C1-C6 alkyl, C3-C8 cycloalkyl, C1-C6 haloalkyl, -OH, -O-C1-C6 alkyl, -NH₂, -NH-C1-C6 alkyl, -OC(=O)C1-C6 alkyl or -N(C1-C6 alkyl)(C1-C6 alkyl);
m1 is selected from 0, 1, 2, 3 or 4; m2 is selected from 0, 1, 2 or 3; m3 is selected from 0, 1 or 2; m4 is selected from 0 or 1;
the group A2 includes the following groups: 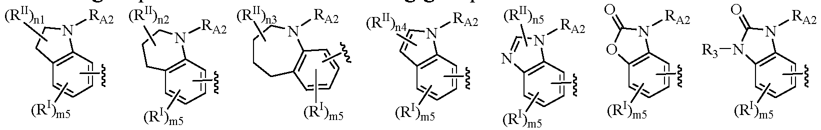 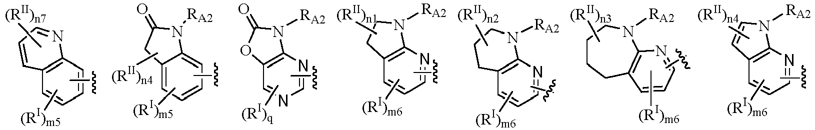 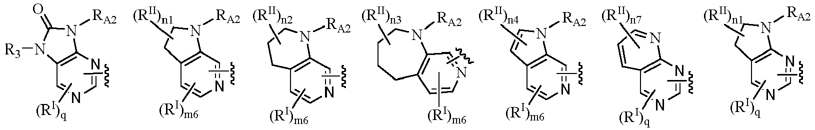 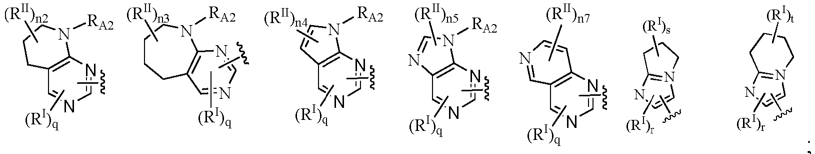
in the group A2, R^{I} and R^{II} are each independently selected from deuterium, halogen, C1-C6 alkyl, C3-C8 cycloalkyl, C1-C6 haloalkyl, -OH, -CN, C1-C6 alkoxy, -NH₂, -NH-C1-C6 alkyl, -OC(=O)C1-C6 alkyl or -N(C1-C6 alkyl)(C1-C6 alkyl); and two R^{II}, together with the carbon atom to which they are attached, may form C3-C8 cycloalkyl; the C1-C6 alkyl, C3-C8 cycloalkyl, C1-C6 haloalkyl, or C1-C6 alkoxy may be optionally substituted with substituents selected from deuterium, F, Cl, Br, I, C1-C6 alkyl, C3-C8 cycloalkyl, C1-C6 alkoxy or C1-C4 haloalkyl;
each R_{A2} is independently selected from hydrogen, deuterium, halogen, C1-C6 alkyl, C3-C8 cycloalkyl, C1-C6 haloalkyl, -OH, C1-C6 alkoxy, -NH₂, -NH-C1-C6 alkyl, -OC(=O)C1-C6 alkyl, -N(C1-C6 alkyl)(C1-C6 alkyl) or C3-C8 cycloalkyl-C1-C6 alkylene, and the C1-C6 alkyl, C3-C8 cycloalkyl, C1-C6 haloalkyl, C1-C6 alkoxy, or C3-C8 cycloalkyl-C1-C6 alkylene may be optionally substituted with substituents selected from deuterium, F, Cl, Br, I, -OH, C1-C6 alkyl, C3-C8 cycloalkyl, C1-C6 alkoxy or C1-C4 haloalkyl;
m5 is selected from 0, 1, 2 or 3; m6 is selected from 0, 1 or 2; n1 is an integer selected from 0-4; n2 is an integer selected from 0-6; n3 is an integer selected from 0-8; n4 is an integer selected from 0-2; n5 is selected from 0 or 1; n7 is an integer selected from 0-3; q is 0 or 1; s is an integer selected from 0-6; t is an integer selected from 0-8; r is selected from 0 or 1;
the group A3 includes the following groups: 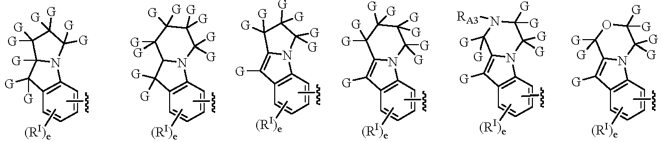 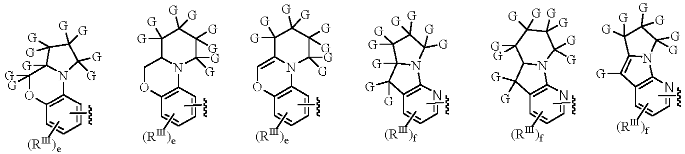 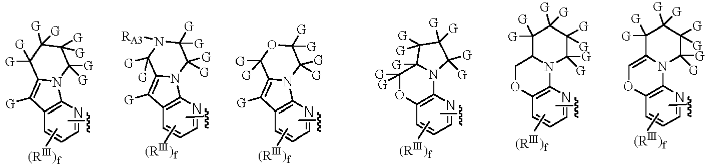 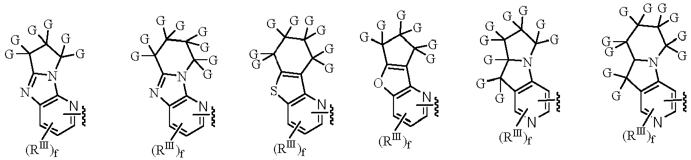 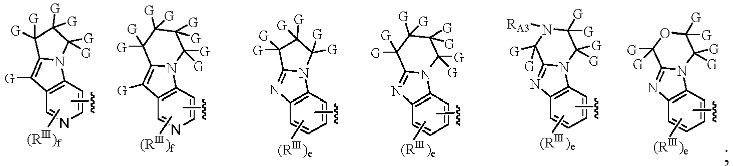
in the group A3,
G, R_{A3} and R^{III} are each independently as defined in claim 27; e is selected from 0, 1, 2 or 3; f is selected from 0, 1 or 2.

31. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is a group selected from the A1 group, and in the A1 group, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, -CN, C1-C6 alkyl, C3-C8 cycloalkyl, C1-C6 haloalkyl, -OH, -O-C1-C6 alkyl, -NH₂, -NH-C1-C6 alkyl, or -N(C1-C6 alkyl)(C1-C6 alkyl);
each R^{AI} is independently selected from deuterium, halogen, -CN, C1-C6 alkyl, C1-C6 haloalkyl, -OH, -O-C1-C6 alkyl, -C1-C6 alkylene-O-C1-C6 alkyl, C1-C6 haloalkoxy, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

32. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is a group selected from the A1 group, and in the A1 group, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, -CN, C1-C6 alkyl, C3-C8 cycloalkyl, C1-C6 haloalkyl, -OH, -O-C1-C6 alkyl, -NH₂, -NH-C1-C6 alkyl, or -N(C1-C6 alkyl)(C1-C6 alkyl);
each R^{AI} is independently selected from deuterium, halogen, -CN, C1-C6 alkyl, C1-C6 haloalkyl, -OH, -O-C1-C6 alkyl, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

33. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 26-29, **characterized in that** A is a group selected from the A1 group, and in the A1 group, each R_{A1} is independently selected from saturated, partially saturated or unsaturated 4-, 5-, 6- or 7-membered monocyclic ring, which comprises 0 or 1 N atom, which monocyclic ring is optionally substituted with 0, 1, 2 or 3 groups selected from: deuterium, halogen, C1-C6 alkyl, C3-C8 cycloalkyl, C1-C6 haloalkyl, -OH, -O-C1-C6 alkyl, -NH₂, -NH-C1-C6 alkyl, or - N(C1-C6 alkyl)(C1-C6 alkyl);
each R^{AI} is independently selected from deuterium, halogen, C1-C6 alkyl, C1-C6 haloalkyl, -OH, -O-C1-C6 alkyl, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

34. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is a group selected from the A1 group, and in the A1 group, each R_{A1} is independently selected from saturated 5- to 6-membered monocyclic ring, which comprises 0 or 1 N atom, which monocyclic ring is optionally substituted with 0, 1, 2 or 3 groups selected from: deuterium, halogen, C1-C6 alkyl, C1-C6 haloalkyl, -OH, -O-C1-C6 alkyl, -NH₂, - NH-C1-C6 alkyl, or -N(C1-C6 alkyl)(C1-C6 alkyl); each R^{AI} is independently selected from deuterium, halogen, -CN, C1-C6 alkyl, -OH, -O-C1-C6 alkyl, -NH₂, - NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

35. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is a group selected from the A1 group, and in the A1 group, each R_{A1} is independently selected from saturated 5- to 6-membered monocyclic ring, which comprises 0 or 1 N atom, which monocyclic ring is optionally substituted with 0, 1, 2 or 3 groups selected from: deuterium, halogen, C1-C6 alkyl, C1-C6 haloalkyl, -OH, -O-C1-C6 alkyl, -NH₂, - NH-C1-C6 alkyl, or -N(C1-C6 alkyl)(C1-C6 alkyl); each R^{AI} is independently selected from deuterium, halogen, C1-C6 alkyl, -OH, -O-C1-C6 alkyl, -NH₂, -NH-C1-C6 alkyl, or -N(C1-C6 alkyl)(C1-C6 alkyl);
m1 is selected from 0, 1 or 2; m2 is selected from 0, 1 or 2; m3 is selected from 0, 1 or 2; m4 is selected from 0 or 1.

36. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-35, **characterized in that** A is selected from:

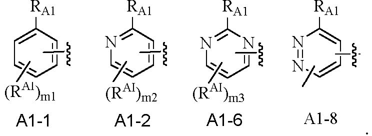

37. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-36, **characterized in that** A is selected from:

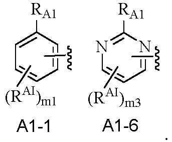

38. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-37, **characterized in that** A is selected from:

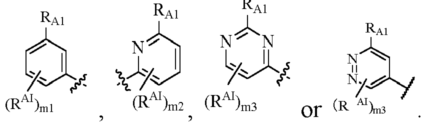

39. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-38, **characterized in that** A is selected from:

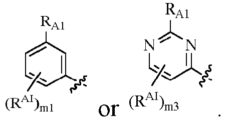

40. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is a group selected from the A2 group.

41. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 40, **characterized in that** A is selected from

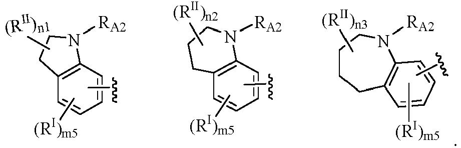

42. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 41, **characterized in that** A is selected from

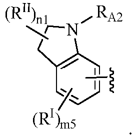

43. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 42, **characterized in that** A is selected from

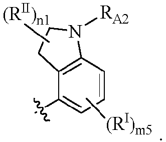

44. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 41-43, **characterized in that** R^{I} and R^{II} are each independently selected from deuterium, halogen, C1-C6 alkyl, C1-C6 haloalkyl, - OH, C1-C6 alkoxy, -NH₂, -NH-C1-C6 alkyl, or -N(C1-C6 alkyl)(C1-C6 alkyl); and two R^{II} , together with the carbon atom to which they are attached, may form C3-C6 cycloalkyl;
each R_{A2} is independently selected from hydrogen, deuterium, C1-C6 alkyl, C3-C6 cycloalkyl, or C1-C6 haloalkyl, and the C1-C6 alkyl may be optionally substituted with substituents selected from deuterium, F, Cl, Br, C1-C6 alkyl, C3-C6 cycloalkyl, or C1-C6 alkoxy;
m5 is selected from 0, 1, 2 or 3; n1 is an integer selected from 0-2; n2 is an integer selected from 0-2; n3 is an integer selected from 0-2.

45. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from

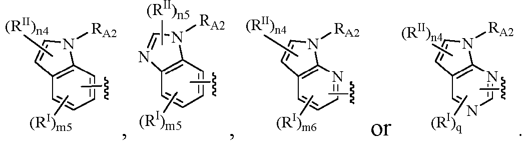

46. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 45, **characterized in that** A is selected from

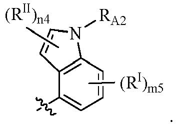

47. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 45 or 46, **characterized in that** R^{I} and R^{II} are each independently selected from deuterium, halogen, C1-C6 alkyl, -OH, -CN, C1-C6 alkoxy, -NH₂, - NH-C1-C6 alkyl or -N(C1-C6 alkyl)(C1-C6 alkyl);
each R_{A2} is independently selected from C1-C6 alkyl, C3-C6 cycloalkyl, C1-C6 haloalkyl, or C3-C6 cycloalkyl-C1-C4 alkyl, and the C1-C6 alkyl, C3-C6 cycloalkyl, C1-C6 haloalkyl, or C3-C6 cycloalkyl-C1-C4 alkyl may be optionally substituted with one or more substituents selected from deuterium, F, Cl, Br, **I,** -OH, C1-C6 alkyl, C3-C6 cycloalkyl, C1-C6 alkoxy or C1-C4 haloalkyl;
m5 is selected from 0 or 1; m6 is selected from 0 or 1; n4 is selected from 0 or 1; n5 is selected from 0 or 1; q is selected from 0 or 1.

48. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 47, **characterized in that** each R_{A2} is independently selected from C1-C6 alkyl or C3-C6 cycloalkyl-C1-C2 alkyl, and the C1-C6 alkyl or C3-C6 cycloalkyl-C1-C2 alkyl may be optionally substituted with 1-3 substituents selected from deuterium, F, Cl, Br, -OH, C1-C4 alkyl, C3-C6 cycloalkyl, or C1-C4 haloalkyl.

49. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 48, **characterized in that** each R_{A2} is independently selected from methyl,

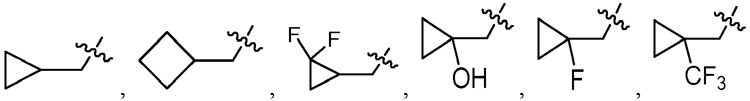

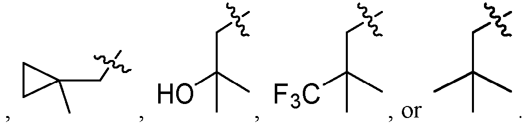

50. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 49, **characterized in that** each R_{A2} is independently selected from

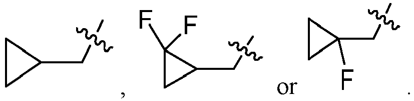

51. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 40-50, **characterized in that** A is selected from

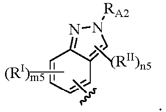

52. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 51, **characterized in that** A is selected from

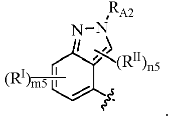

53. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 51 or 52, **characterized in that** R^{I} and R^{II} are each independently selected from deuterium, halogen, C1-C6 alkyl, -OH, -CN, C1-C6 alkoxy, -NH₂, - NH-C1-C6 alkyl or -N(C1-C6 alkyl)(C1-C6 alkyl);
each R_{A2} is independently selected from C1-C6 alkyl, C3-C6 cycloalkyl, C1-C6 haloalkyl, C3-C6 cycloalkyl-C1-C4 alkyl, -S(O)-R_{z1} or -S(O)₂-R_{z1}, and the C1-C6 alkyl, C3-C6 cycloalkyl, C1-C6 haloalkyl, or C3-C6 cycloalkyl-C1-C4 alkyl may be optionally substituted with 1-3 substituents selected from deuterium, F, Cl, Br, I, -OH, C1-C6 alkyl, C3-C6 cycloalkyl, C1-C6 alkoxy or C1-C4 haloalkyl; each R_{z1} is independently selected from C1-C6 alkyl or C1-C6 haloalkyl; m5 is selected from 0 or 1, and n5 is selected from 0 or 1.

54. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 53, **characterized in that** each R_{A2} is independently selected from 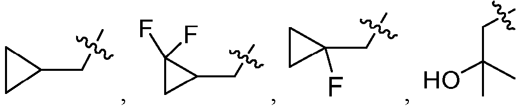 or -S(O)₂-CF₃.

55. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 40-50, **characterized in that** A is selected from

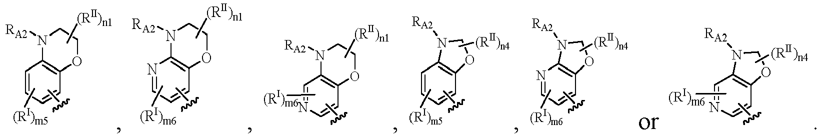

56. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 55, **characterized in that** A is selected from

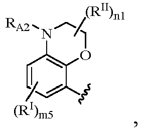

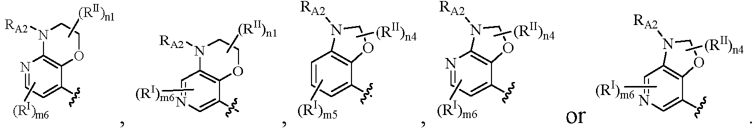

57. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 40-50, **characterized in that** A is selected from

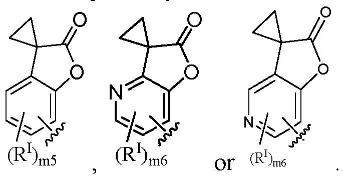

58. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 57, **characterized in that** A is selected from 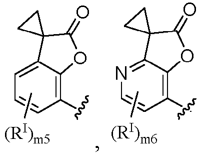 or

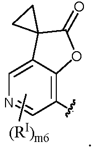

59. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is a group selected from the A3 group.

60. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 59, **characterized in that** A is a group selected from the A3 group, and wherein each G is independently selected from hydrogen, deuterium, halogen, C1-C6 alkyl, deuterated C1-C6 alkyl, C1-C6 haloalkyl, -OH, C1-C6 alkoxy, C1-C6 haloalkoxy, C1-C6 deuteroalkoxy, -NH₂, -NH-C1-C6 alkyl or -N(C1-C6 alkyl)(C1-C6 alkyl), or two G attached to the same carbon atom form an oxo group (=O);
R_{A3} is independently selected from hydrogen, deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH₂, -NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
R^{III} is independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH₂, -NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
e is selected from 0, 1 or 2; f is selected from 0 or 1.

61. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 60, **characterized in that** A is a group selected from the A3 group, and wherein each G is independently selected from hydrogen, deuterium, halogen, C1-C6 alkyl, deuterated C1-C6 alkyl, C1-C6 haloalkyl, -OH, C1-C6 alkoxy, C1-C6 haloalkoxy, C1-C6 deuteroalkoxy, -NH₂, -NH-C1-C6 alkyl or -N(C1-C6 alkyl)(C1-C6 alkyl), or two G attached to the same carbon atom form an oxo group (=O);
R_{A3} and R^{III} are each independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH₂, -NH-C1-C6 alkyl, - C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5-to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
e is selected from 0, 1 or 2; f is selected from 0 or 1.

62. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 61, **characterized in that** A is a group selected from the A3 group, and wherein each G is independently selected from hydrogen, deuterium, halogen, C1-C6 alkyl, deuterated C1-C6 alkyl, C1-C6 haloalkyl, -OH, C1-C6 alkoxy, -NH₂, -NH-C1-C6 alkyl or -N(C1-C6 alkyl)(C1-C6 alkyl), or two G attached to the same carbon atom form an oxo group (=O);
R_{A3} is independently selected from H, deuterium, halogen, C1-C6 alkyl, C1-C6 haloalkyl, -OH, C1-C6 alkoxy, -NH₂, -NH-C1-C6 alkyl or -N(C1-C6 alkyl)(C1-C6 alkyl);
R^{III} is independently selected from deuterium, halogen, C1-C6 alkyl, C1-C6 haloalkyl, -OH, C1-C6 alkoxy, -NH₂, -NH-C1-C6 alkyl or -N(C1-C6 alkyl)(C1-C6 alkyl);
e is selected from 0, 1 or 2; f is selected from 0 or 1.

63. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 62, **characterized in that** A is a group selected from the A3 group, and wherein each G is independently selected from hydrogen, deuterium, halogen, C1-C6 alkyl, deuterated C1-C6 alkyl, C1-C6 haloalkyl, -OH, C1-C6 alkoxy, -NH₂, -NH-C1-C6 alkyl or -N(C1-C6 alkyl)(C1-C6 alkyl), or two G attached to the same carbon atom form an oxo group (=O);
R_{A3} and R^{III} are each independently selected from deuterium, halogen, C1-C6 alkyl, C1-C6 haloalkyl, -OH, C1-C6 alkoxy, -NH₂, -NH-C1-C6 alkyl, or -N(C1-C6 alkyl)(C1-C6 alkyl);
e is selected from 0, 1 or 2; f is selected from 0 or 1.

64. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that**
A is selected from any one of the following groups: 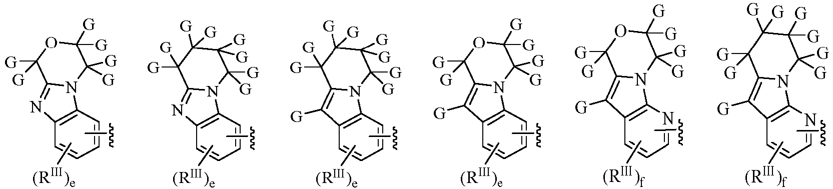 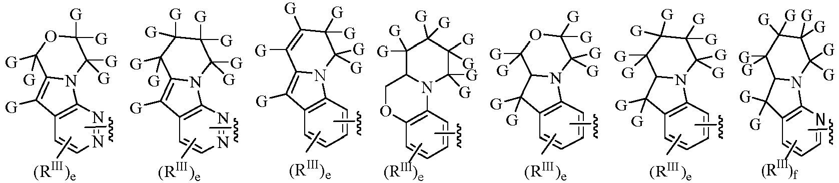 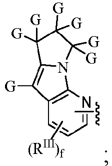
A is preferably selected from any one of the following groups:

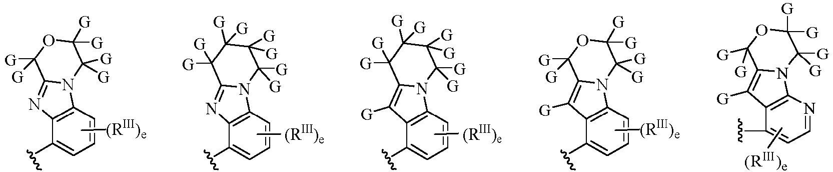

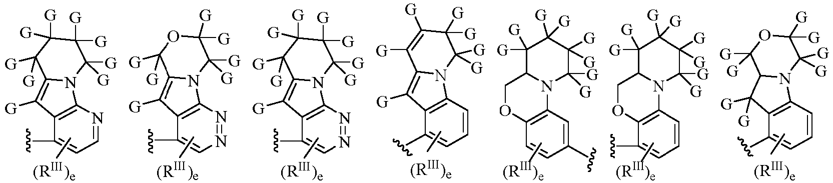

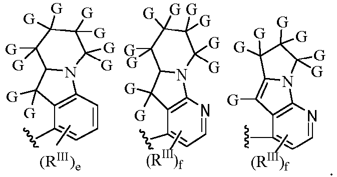

65. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 64, **characterized in that**
A is selected from any one of the following groups: 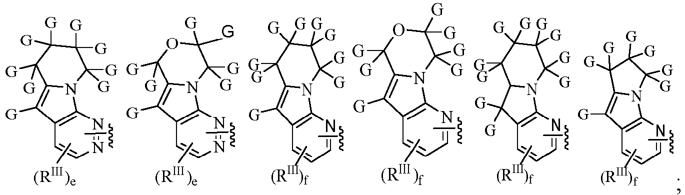
A is preferably selected from any one of the following groups:

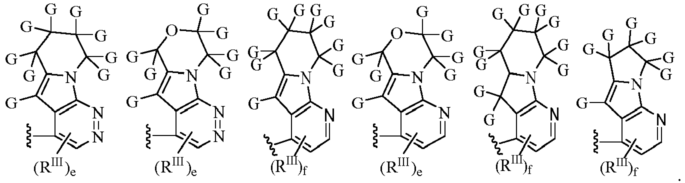

66. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 64, **characterized in that**
A is selected from any one of the following groups: 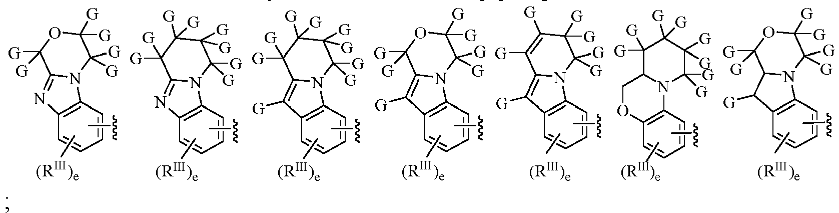
A is preferably selected from any one of the following groups:

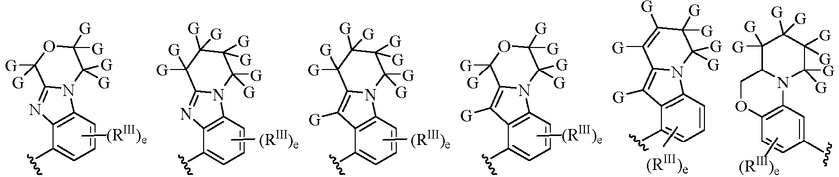

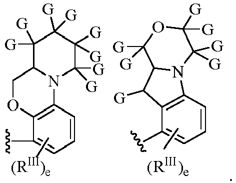

67. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that**
A is selected from any one of the following groups: 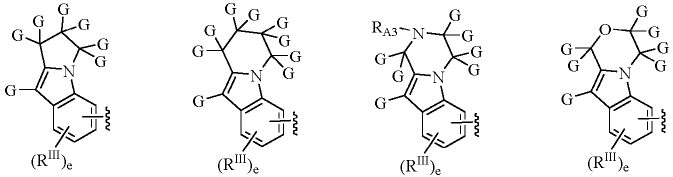 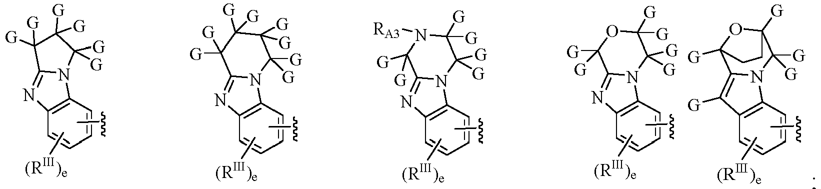
A is preferably selected from any one of the following groups:

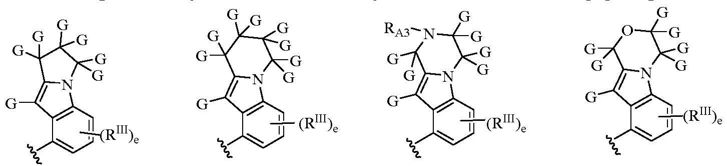

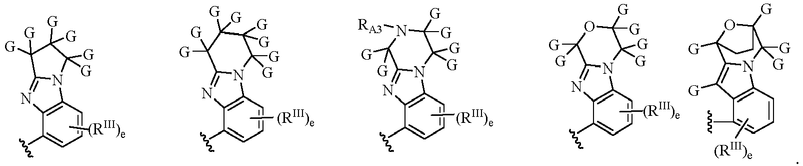

68. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups: 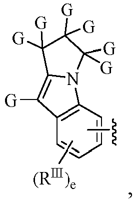 preferably

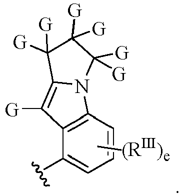

69. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups: 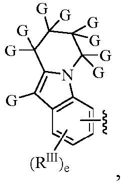 preferably

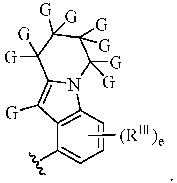

70. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups: 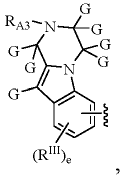 preferably

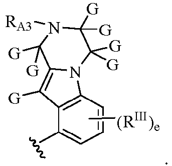

71. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

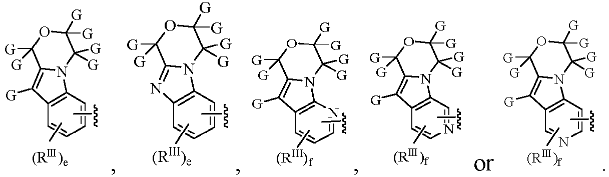

72. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 71, **characterized in that** A is selected from the following groups:

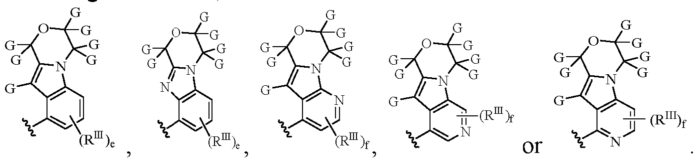

73. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups: 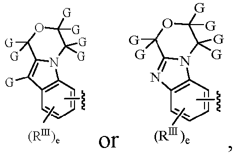 preferably

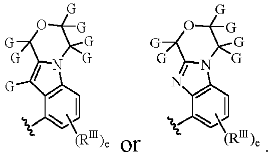

74. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 62-73, **characterized in that**
each G is independently selected from hydrogen, deuterium, halogen, C1-C6 alkyl, C1-C6 haloalkyl, C1-C6 deuteroalkyl, -OH, or C1-C6 alkoxy, or two G attached to the same carbon atom form 3- to 6-membered cycloalkyl, and the C1-C6 alkyl, C1-C6 haloalkyl, C1-C6 alkoxy, or 3- to 6-membered cycloalkyl may be optionally substituted with 1-2 substituents selected from deuterium, F, Cl, or Br;
each R^{III} is independently selected from deuterium, halogen, -CN, C1-C6 alkyl, C1-C6 alkoxy, C1-C6 haloalkyl, C1-C6 deuteroalkyl, -OH, -O-C3-C8 cycloalkyl, C1-C6 haloalkoxy, C1-C6 deuteroalkoxy, -NH₂, -NH-C1-C6 alkyl, -C(=O)NH-C1-C6 alkyl, -C(=O)N(C1-C6 alkyl)(C1-C6 alkyl), phenyl, -5- to 6-membered monocyclic heteroarylene-C1-C6 alkyl comprising 1, 2 or 3 N atoms, 5- to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
e is selected from 0, 1 or 2; f is selected from 0 or 1.

75. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 62-74, **characterized in that**
each G is independently selected from hydrogen, deuterium, halogen, C1-C6 alkyl, C1-C6 haloalkyl, -OH, or C1-C6 alkoxy, and the C1-C6 alkyl, C1-C6 haloalkyl, or C1-C6 alkoxy may be optionally substituted with 1-2 substituents selected from deuterium, F, Cl, or Br;
each R^{III} is independently selected from deuterium, halogen, -CN, C1-C6 alkyl or C1-C6 alkoxy;
e is selected from 0, 1 or 2; f is selected from 0 or 1.

76. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

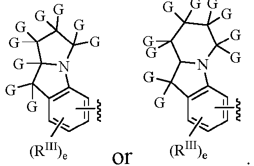

77. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

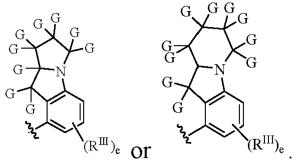

78. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

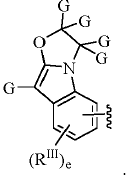

79. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

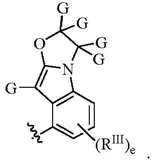

80. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

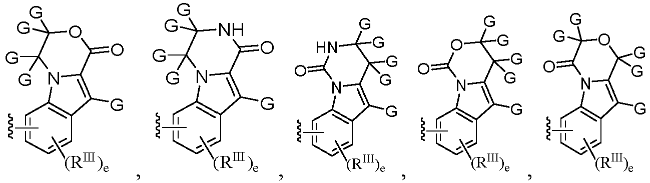

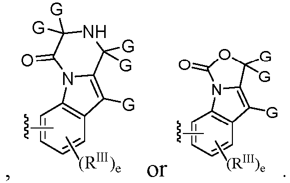

81. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

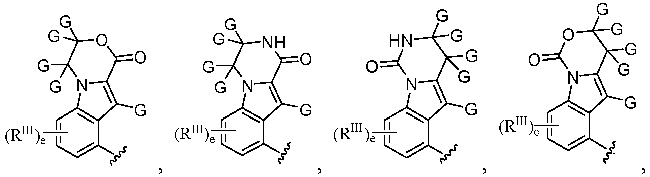

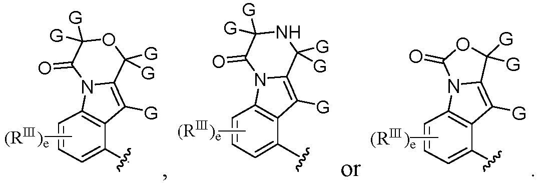

82. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

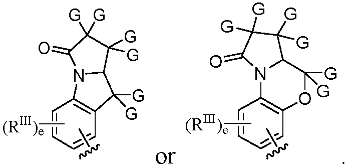

83. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

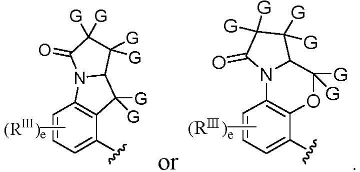

84. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

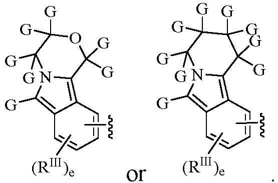

85. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 27-30, **characterized in that** A is selected from the following groups:

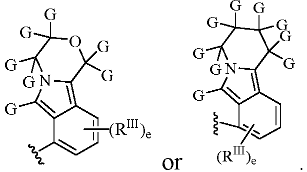

86. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

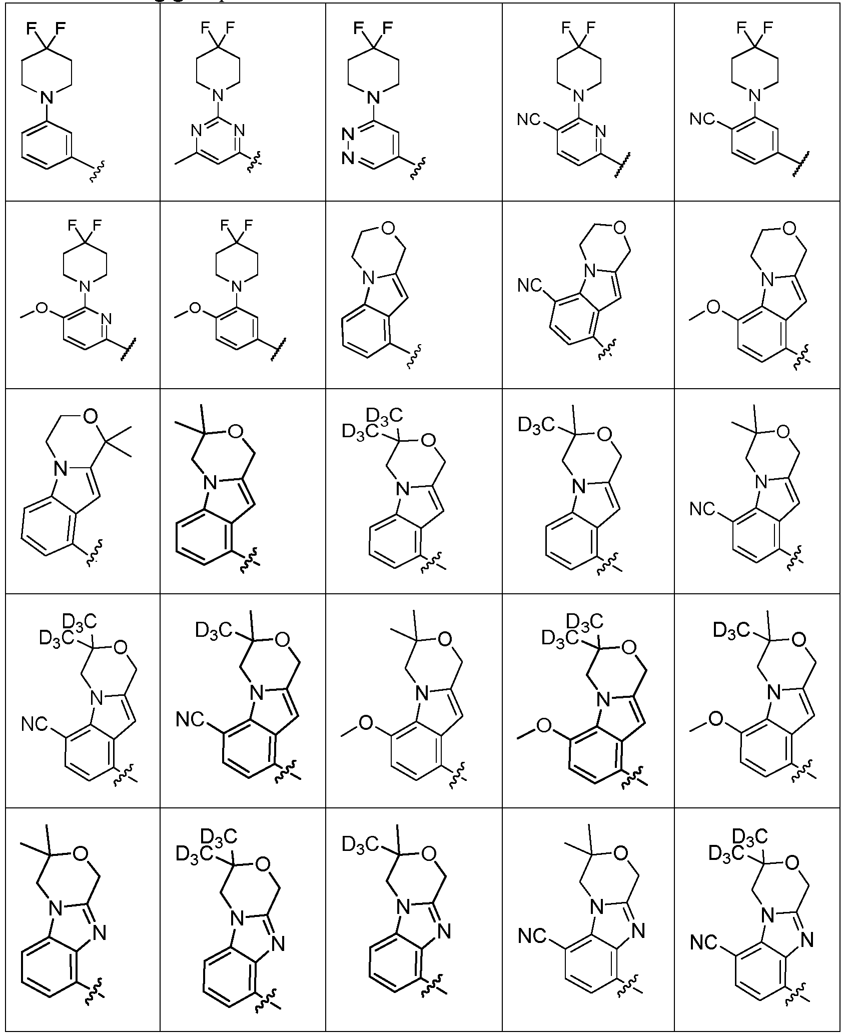

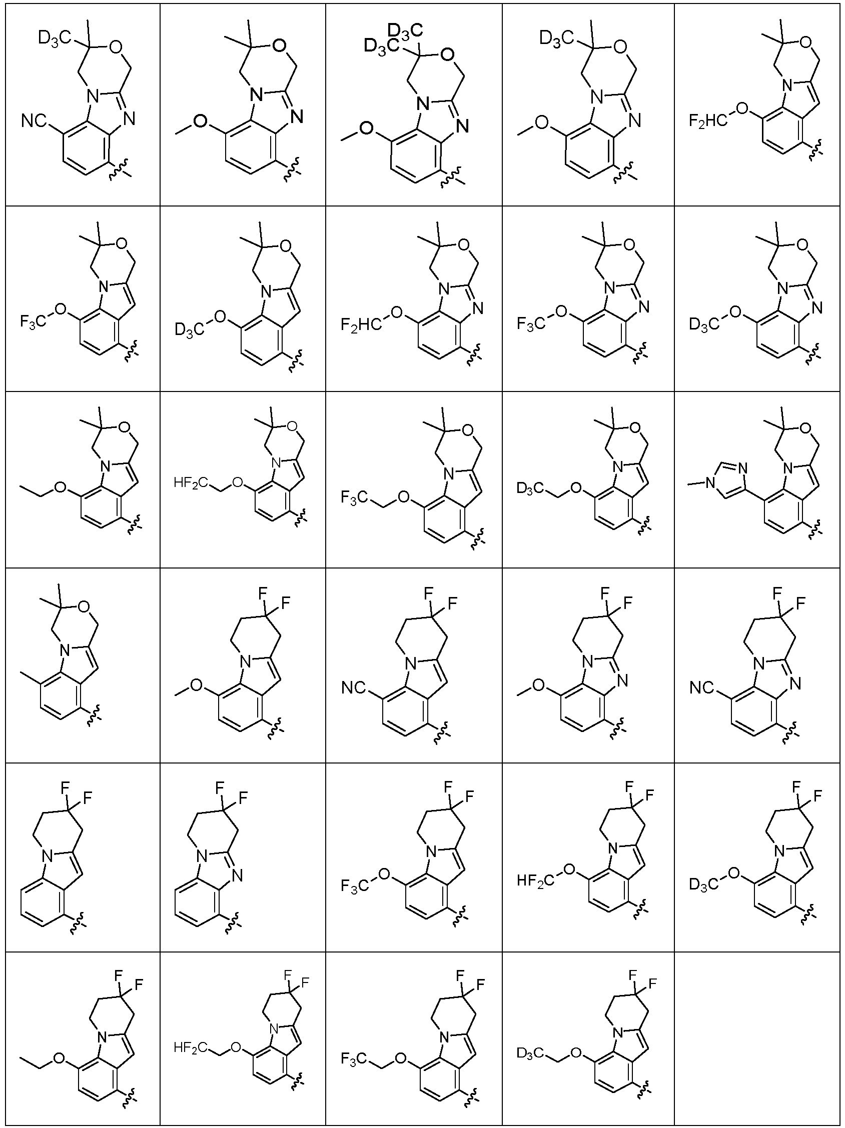

87. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-86, **characterized in that** A is selected from any one of the following groups:

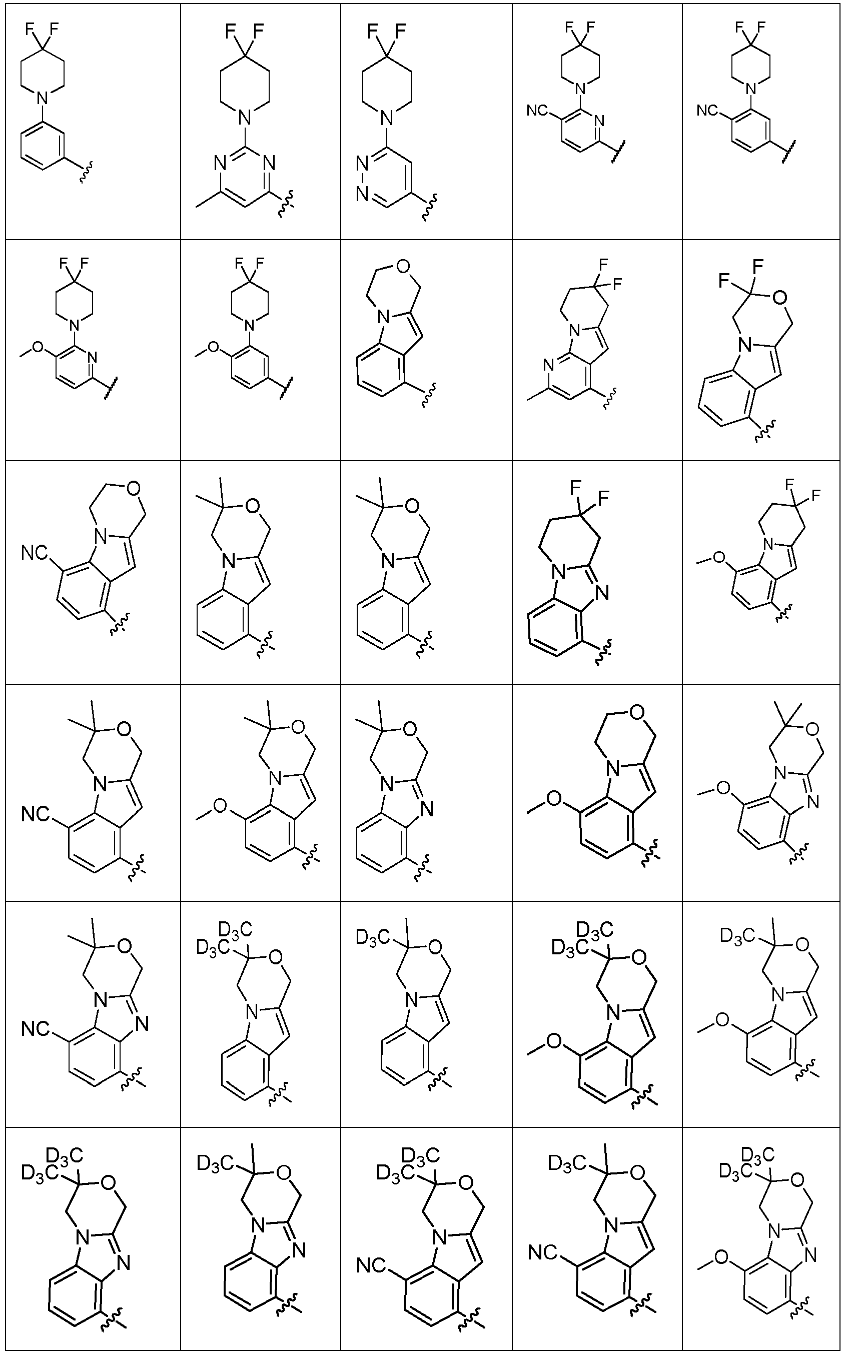

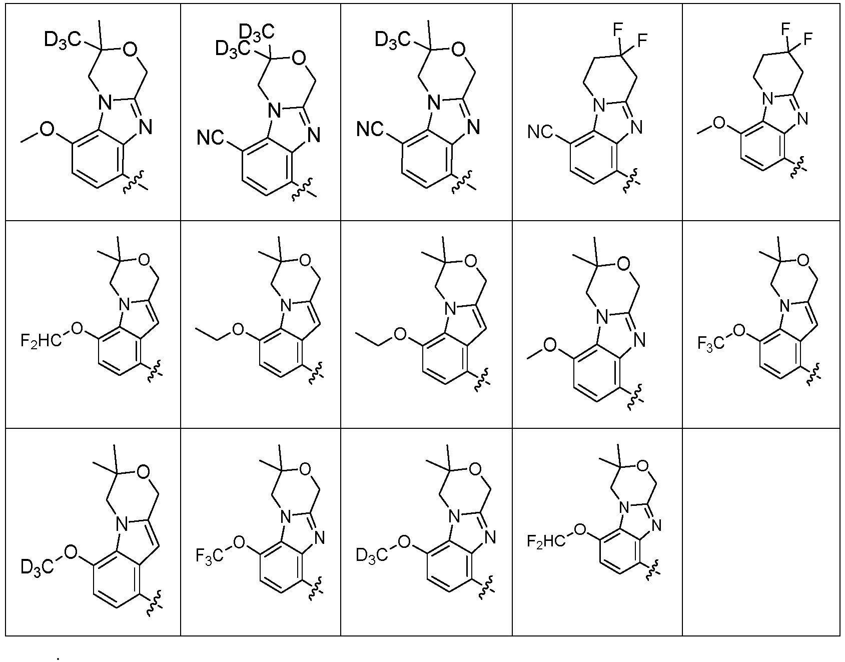

88. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-87, **characterized in that** A is selected from any one of the following groups:

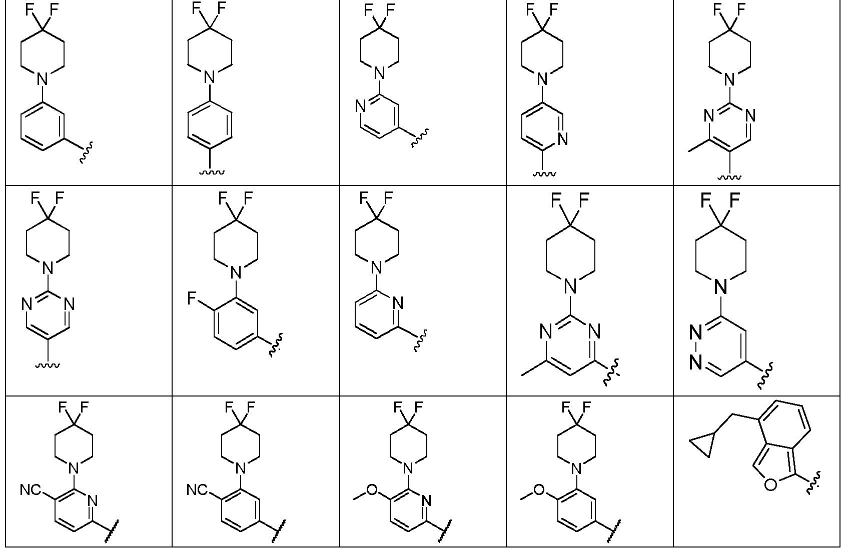

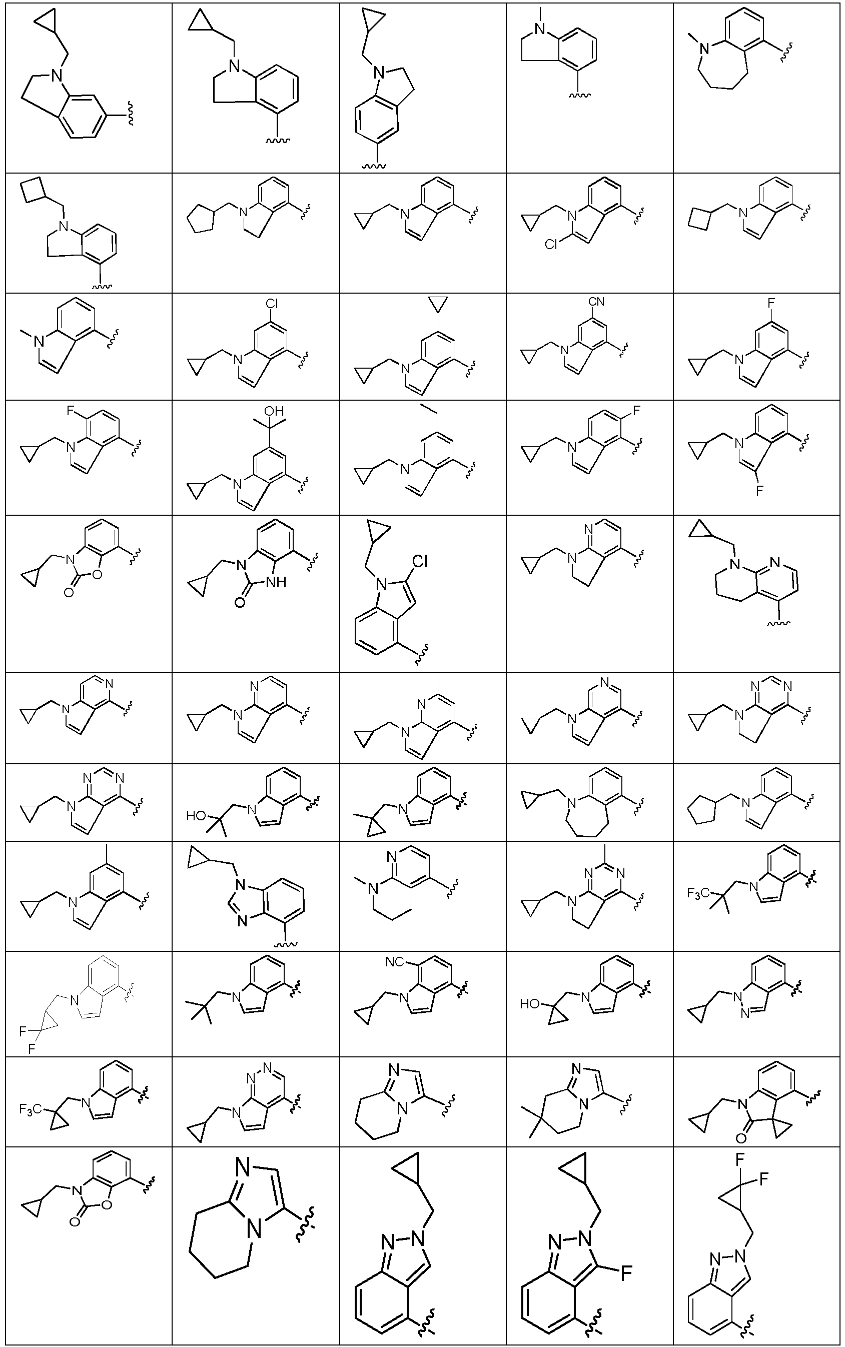

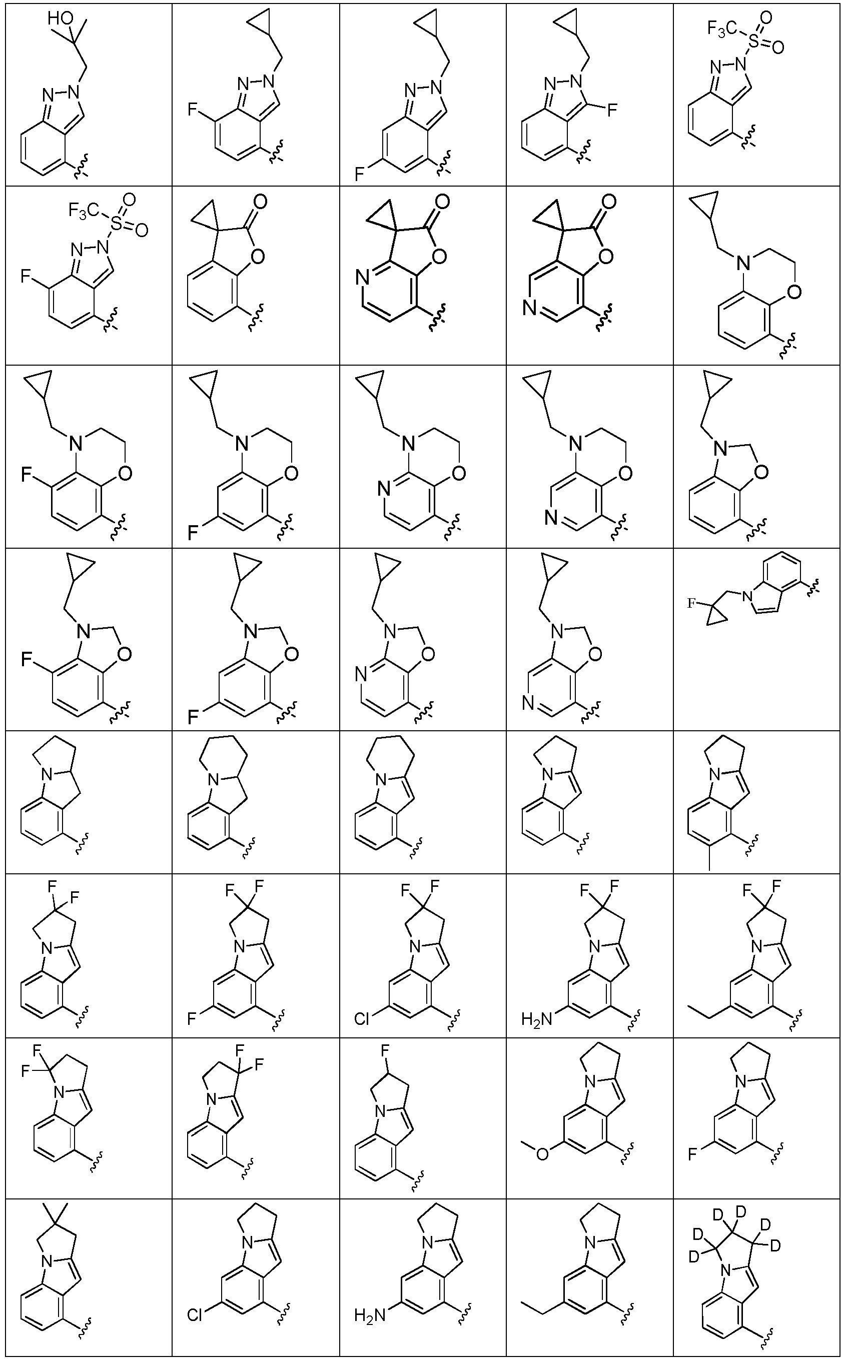

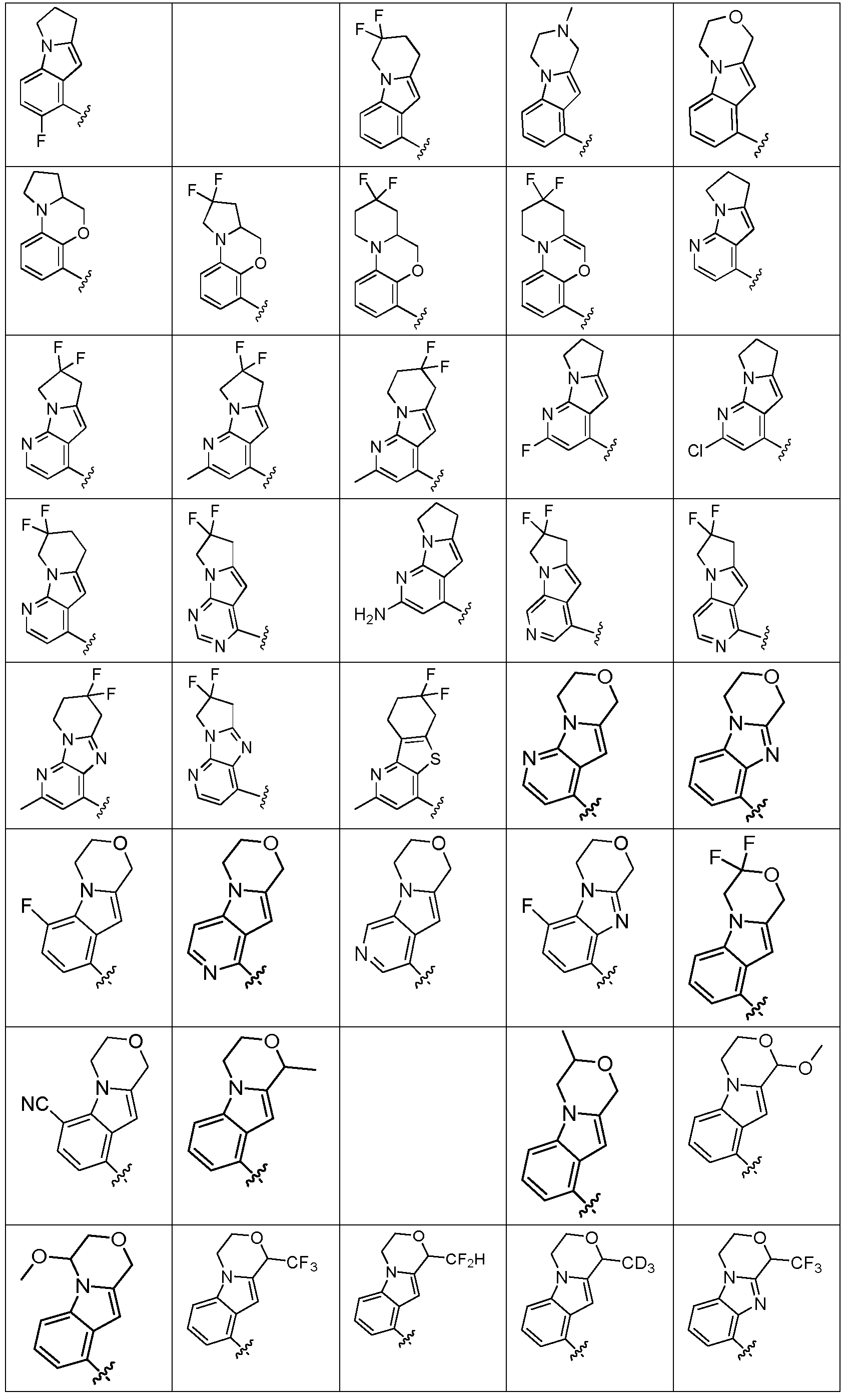

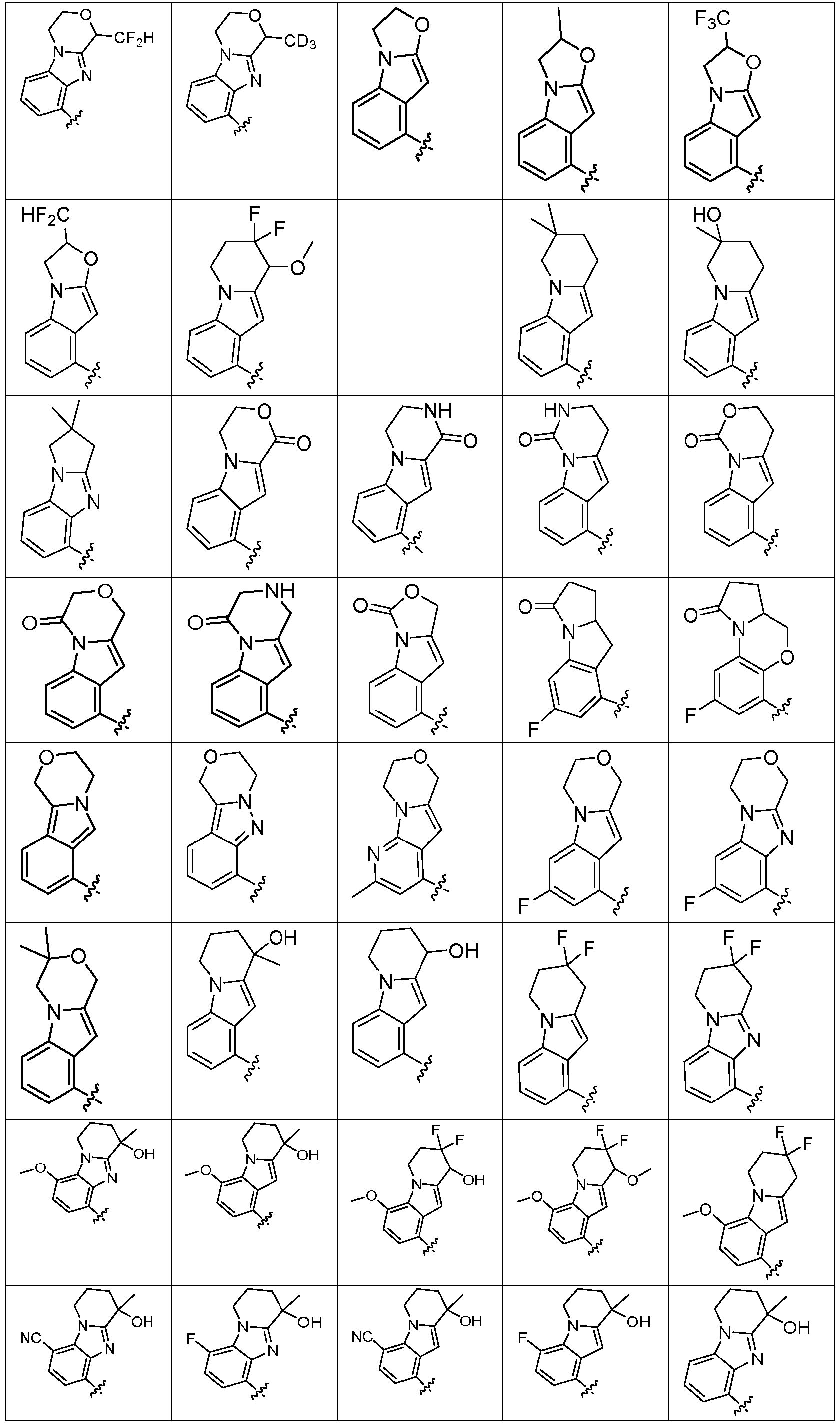

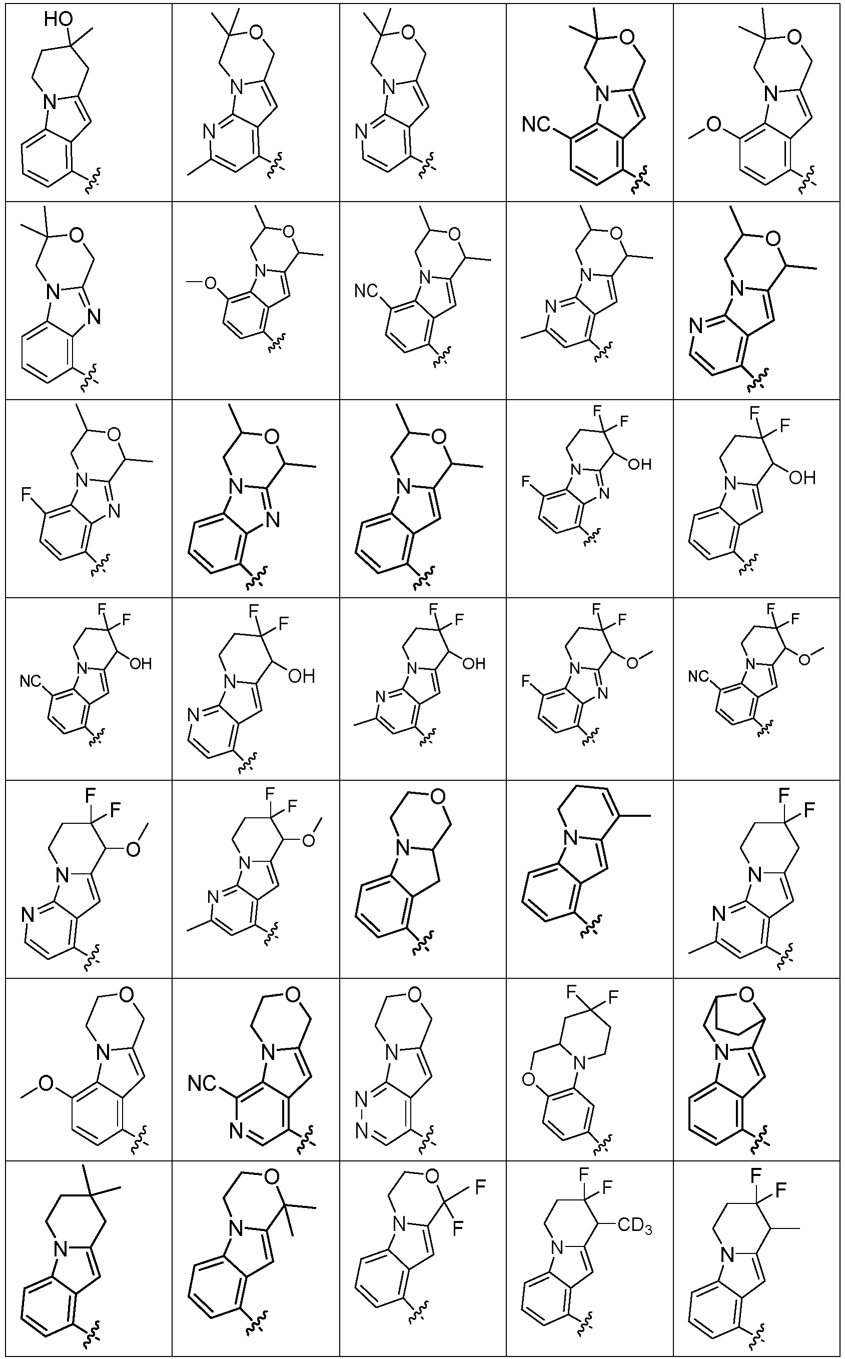

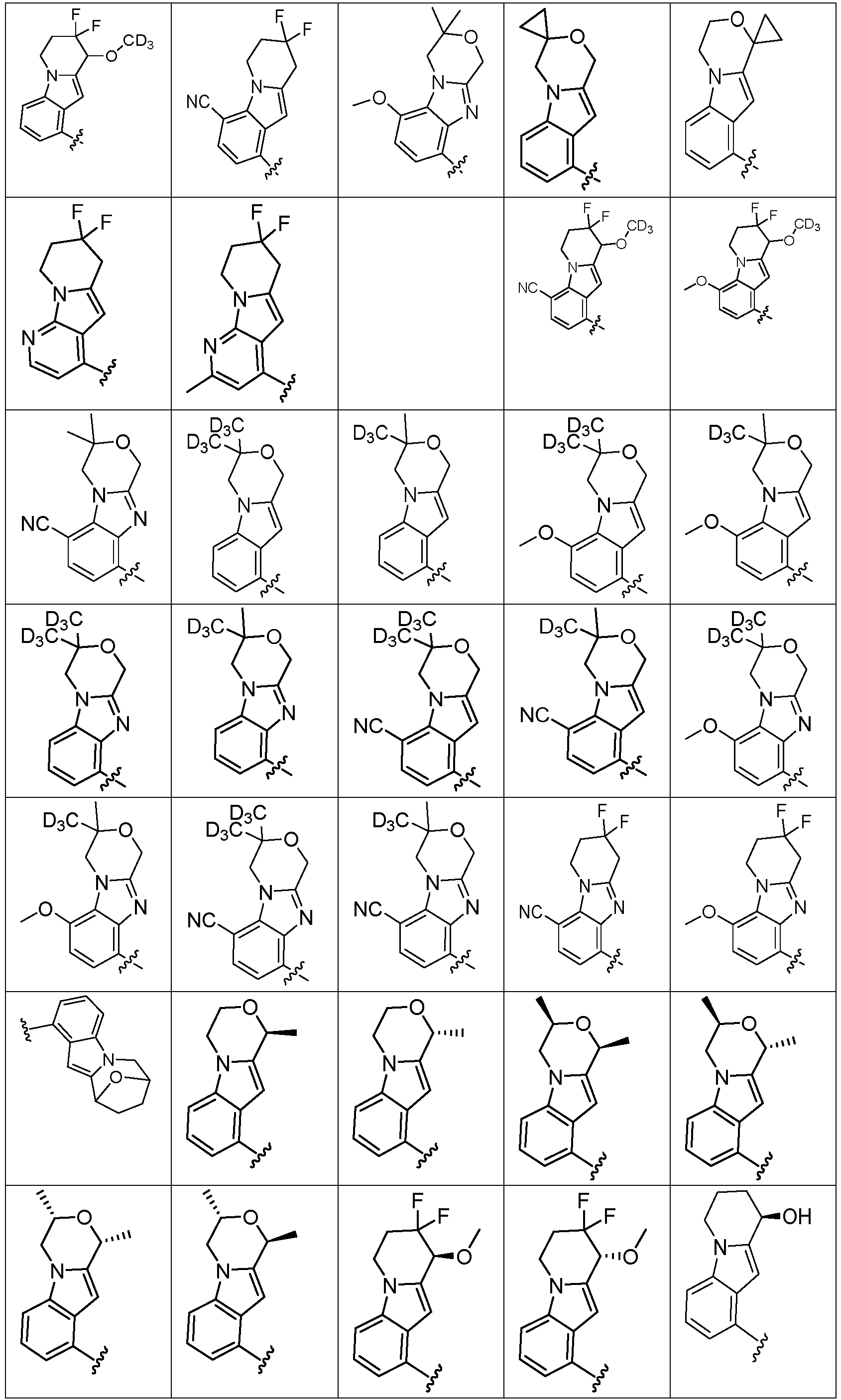

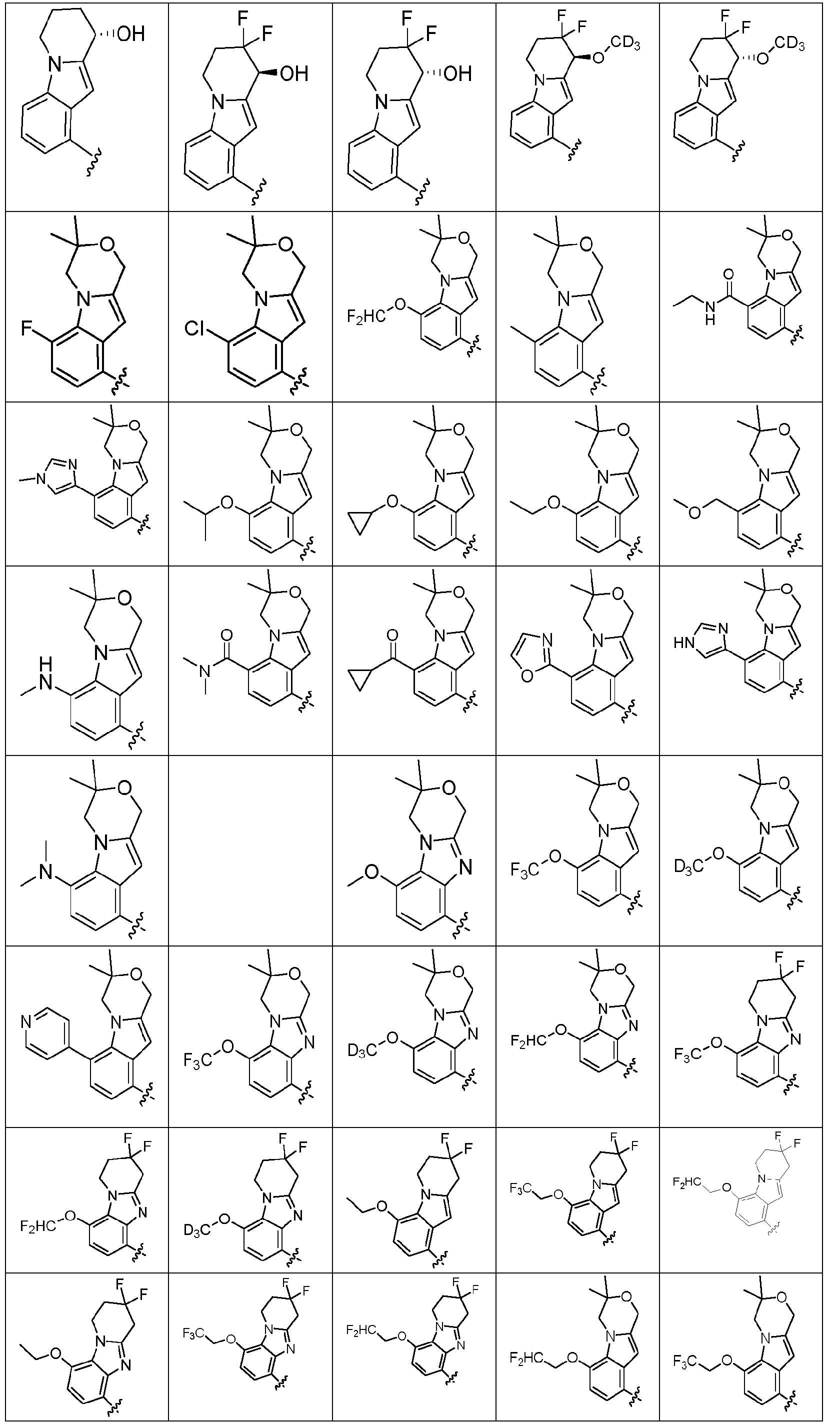

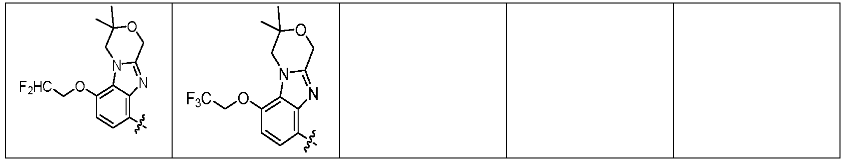

89. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

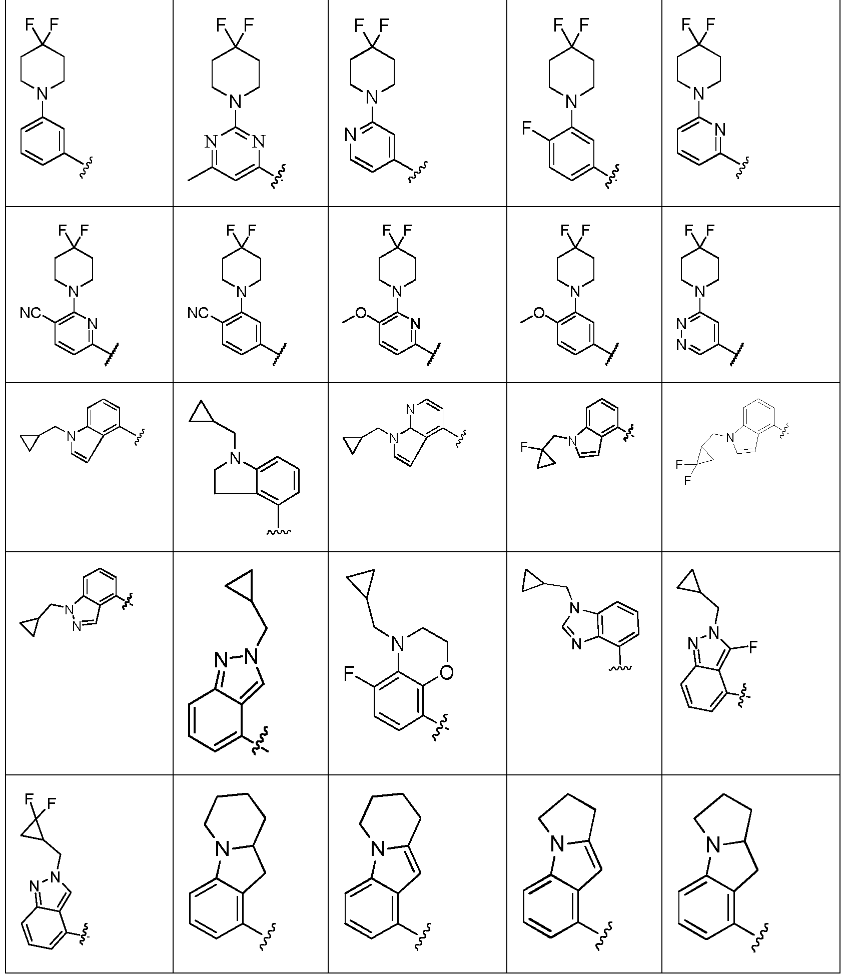

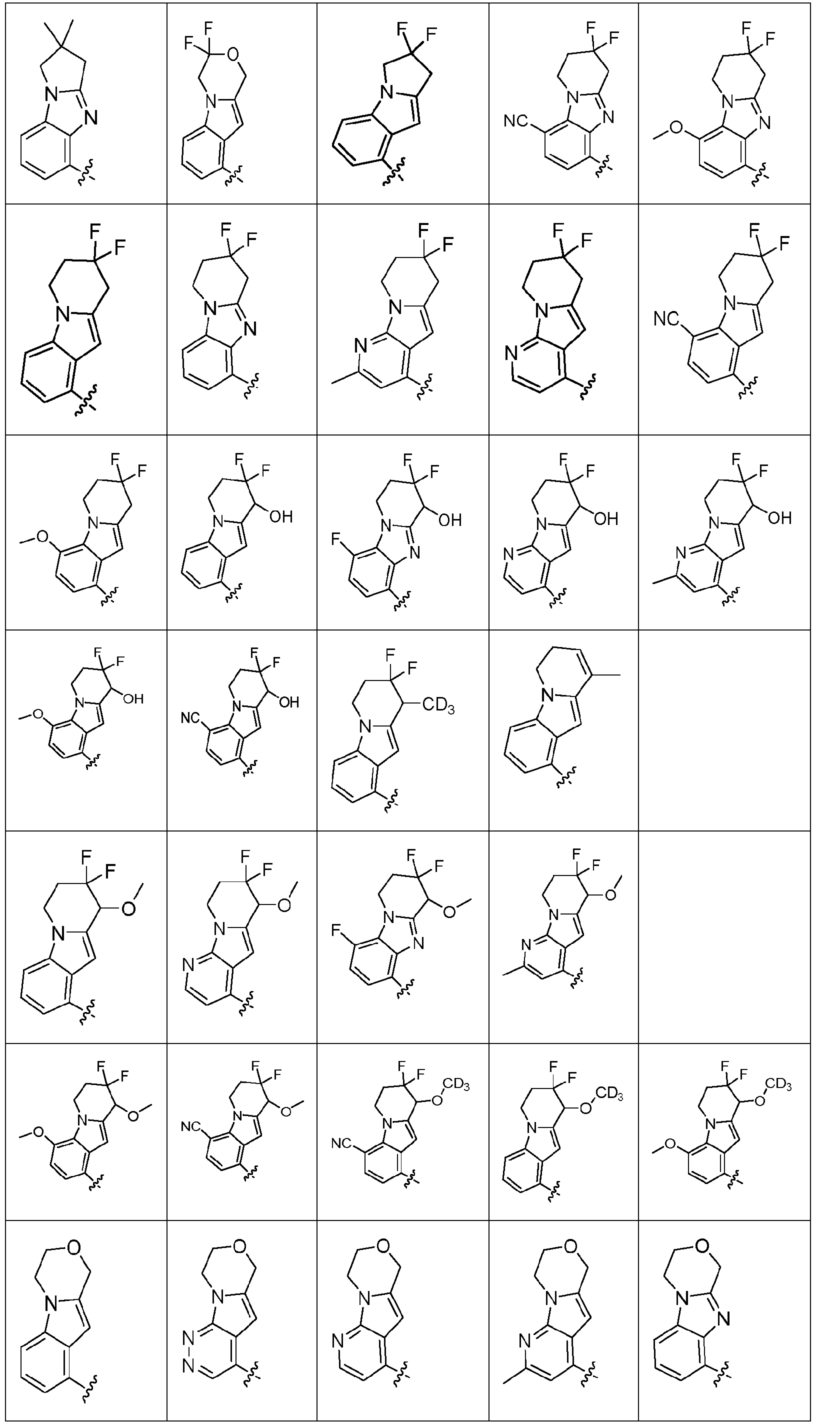

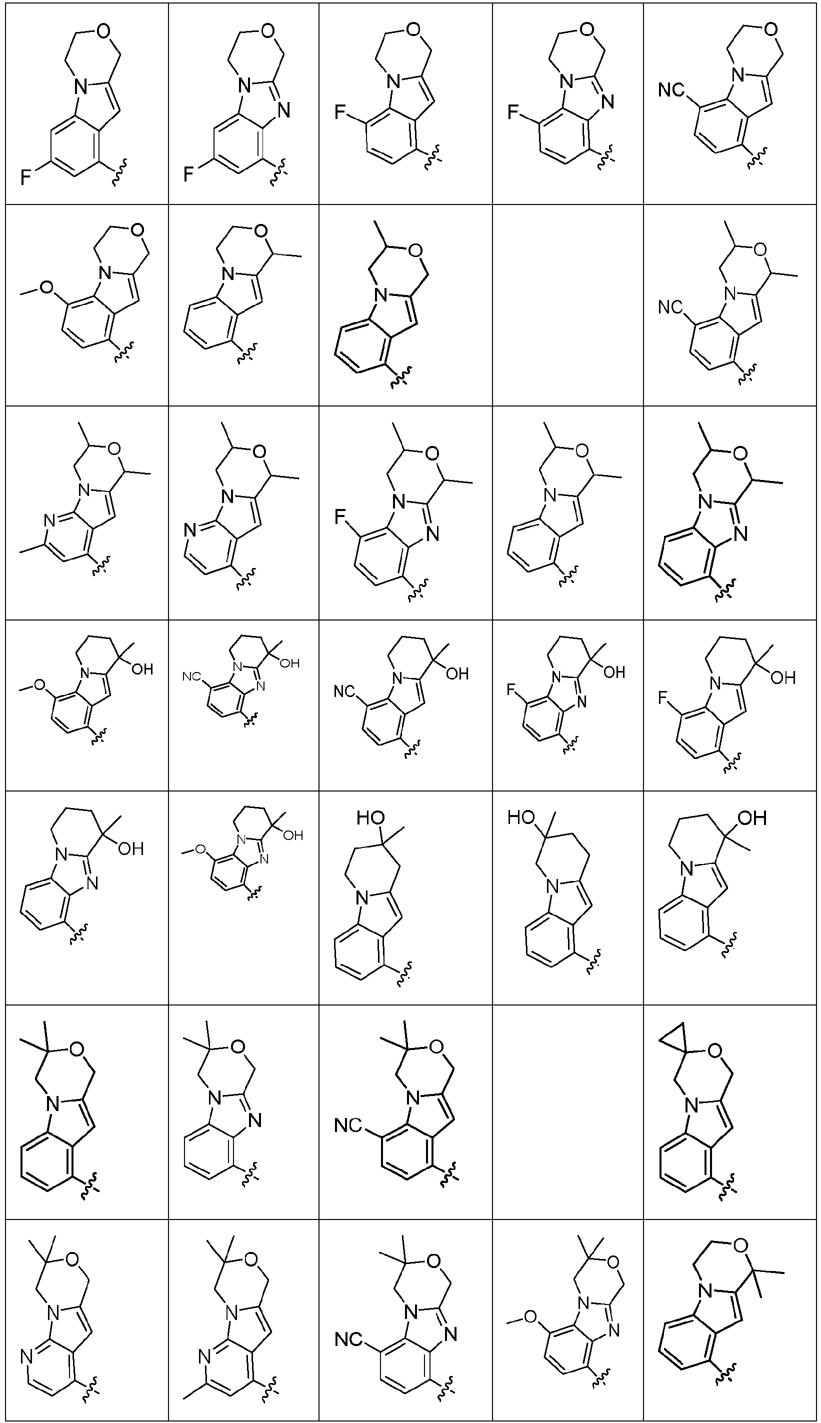

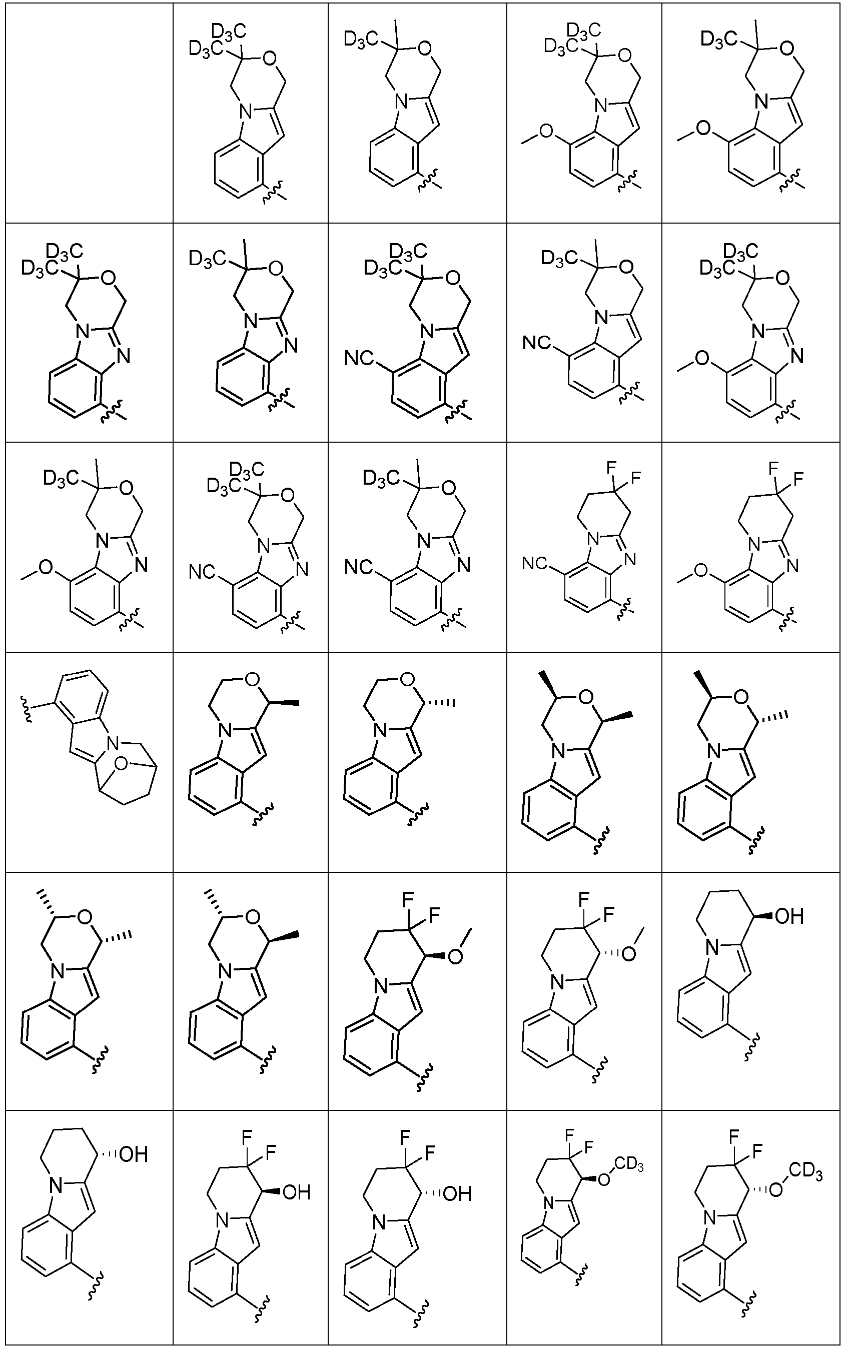

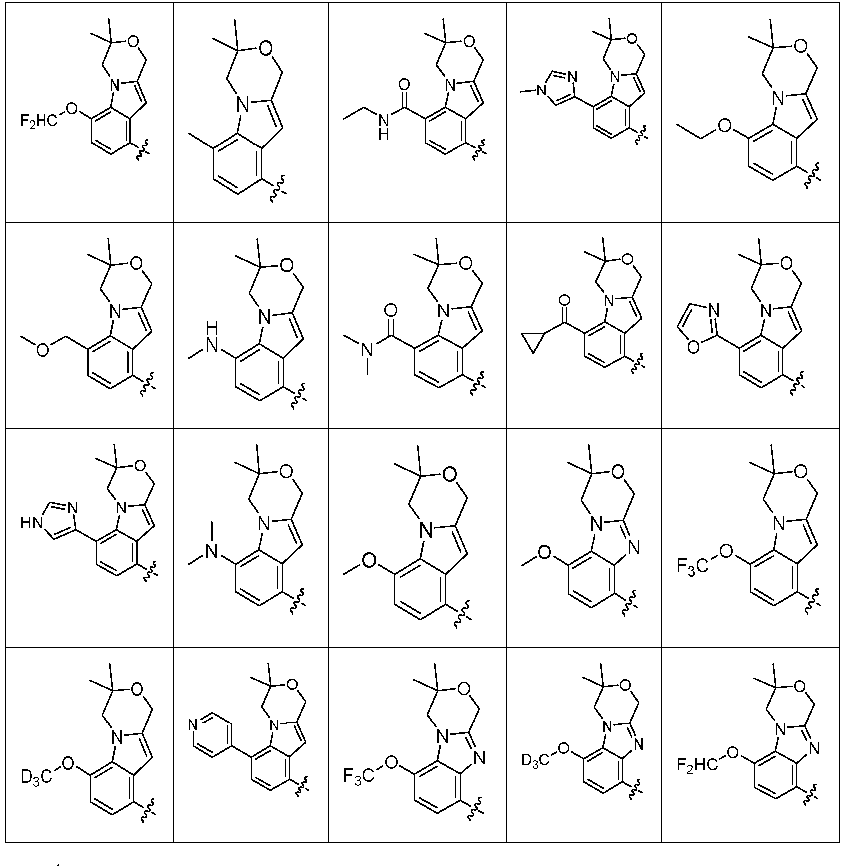

90. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

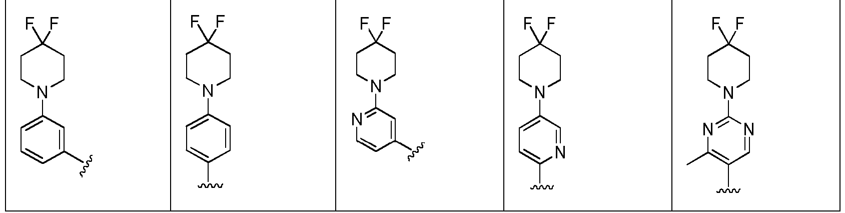

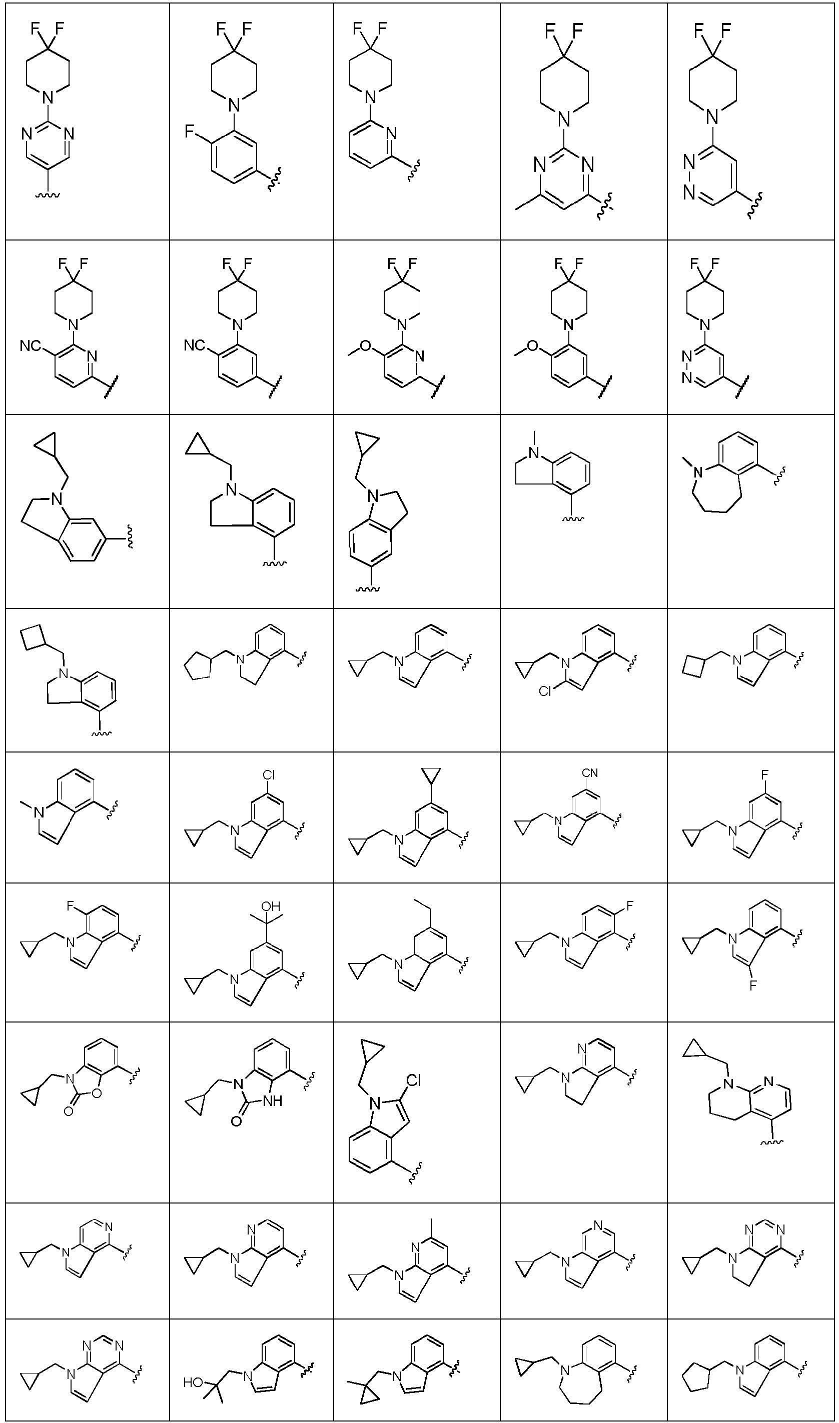

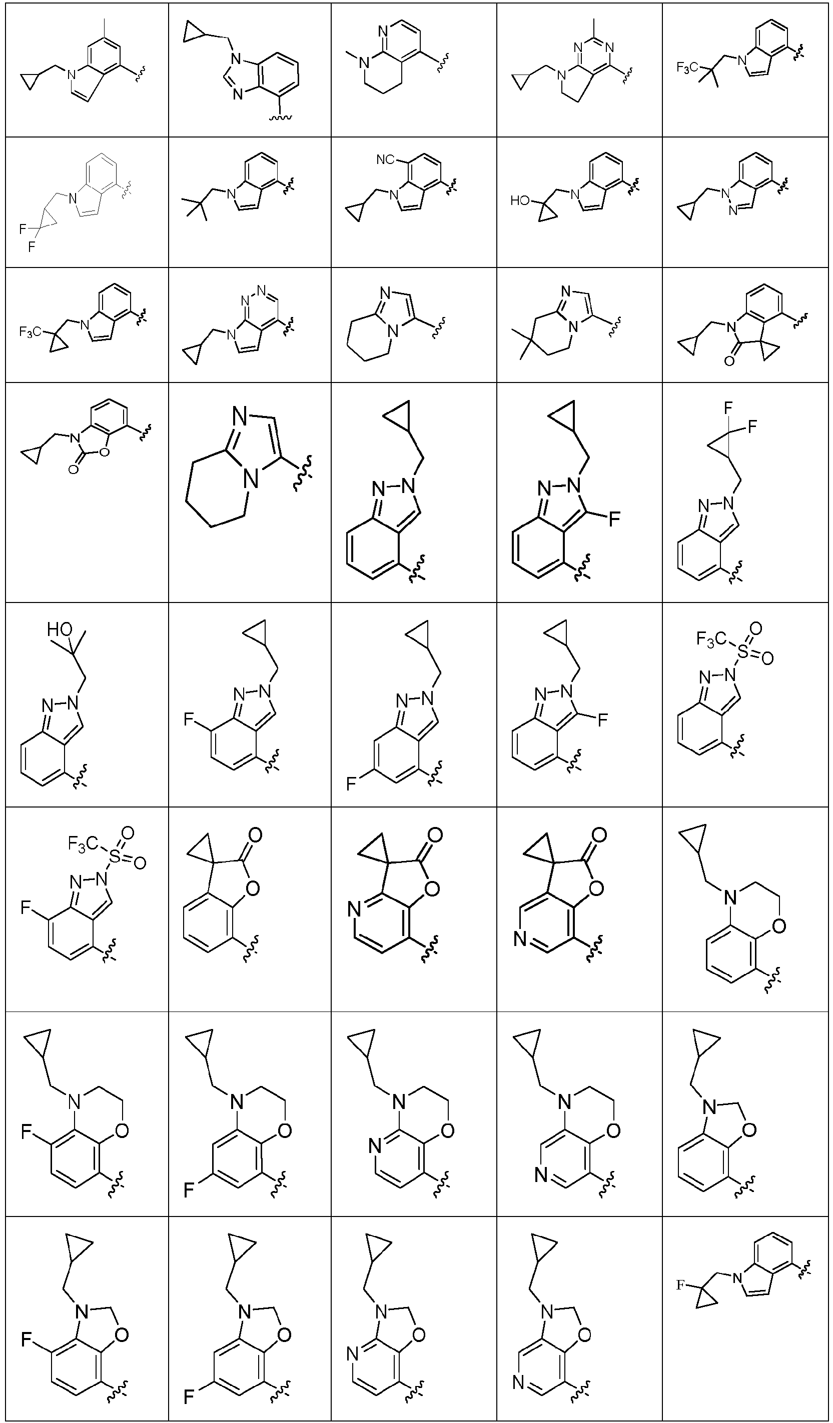

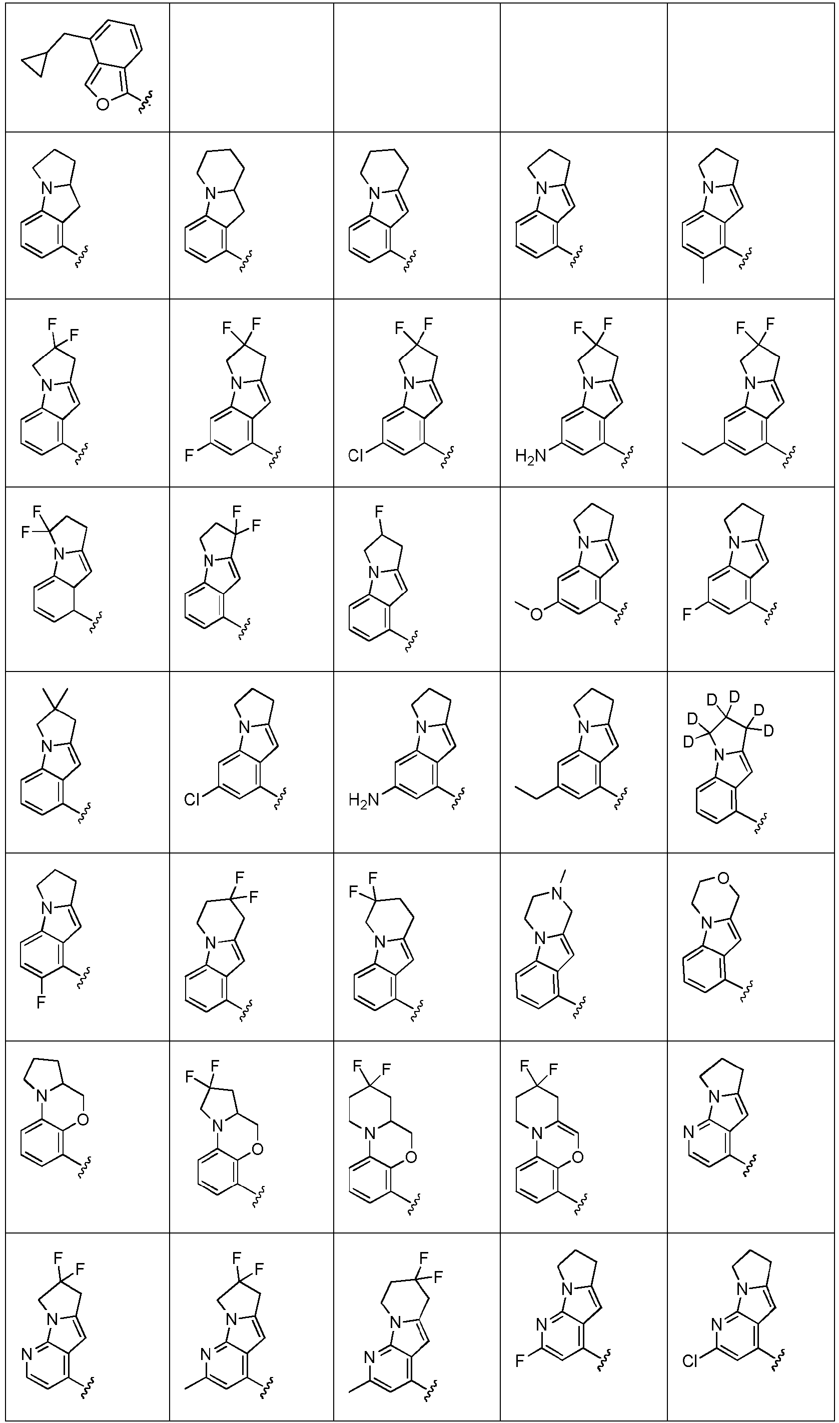

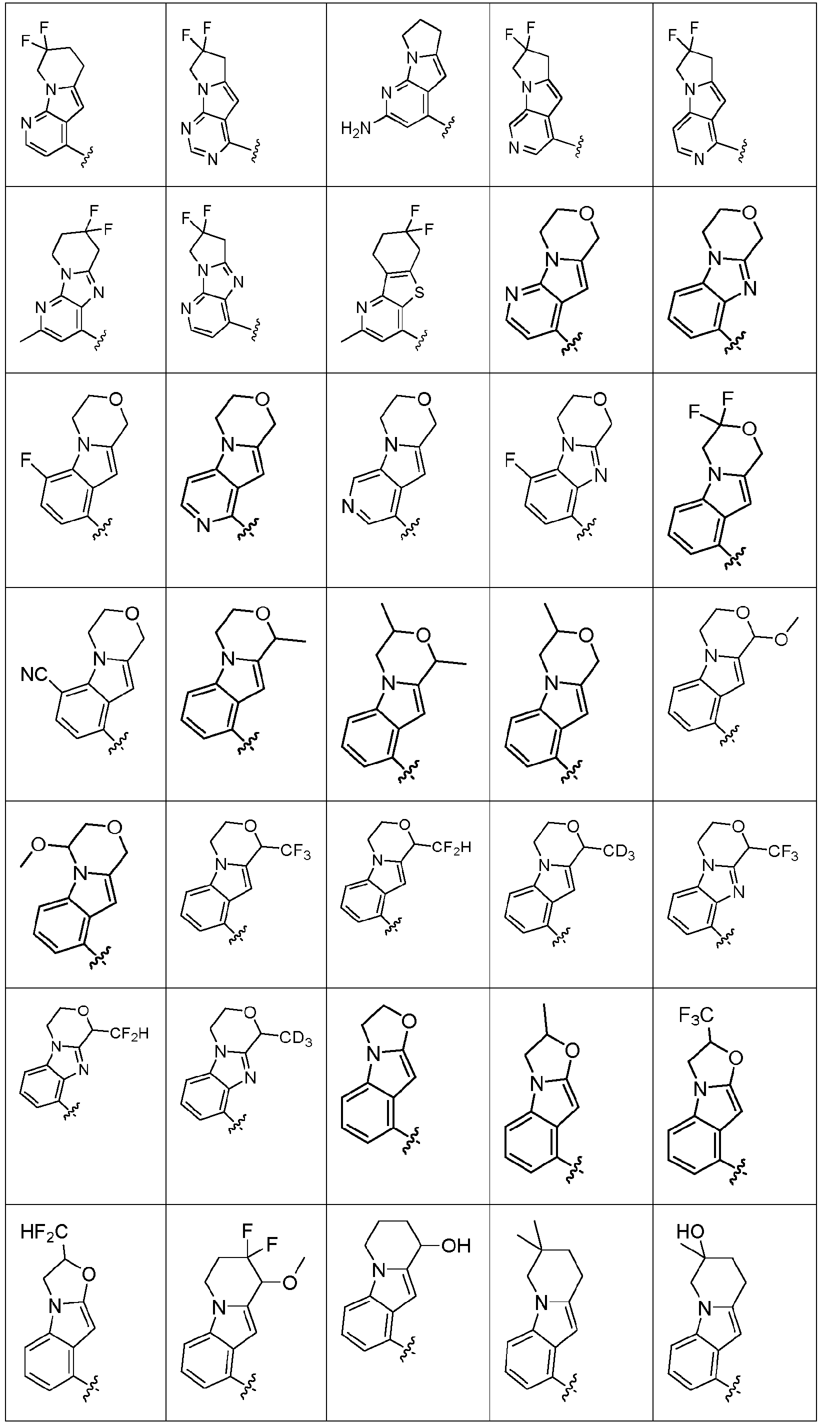

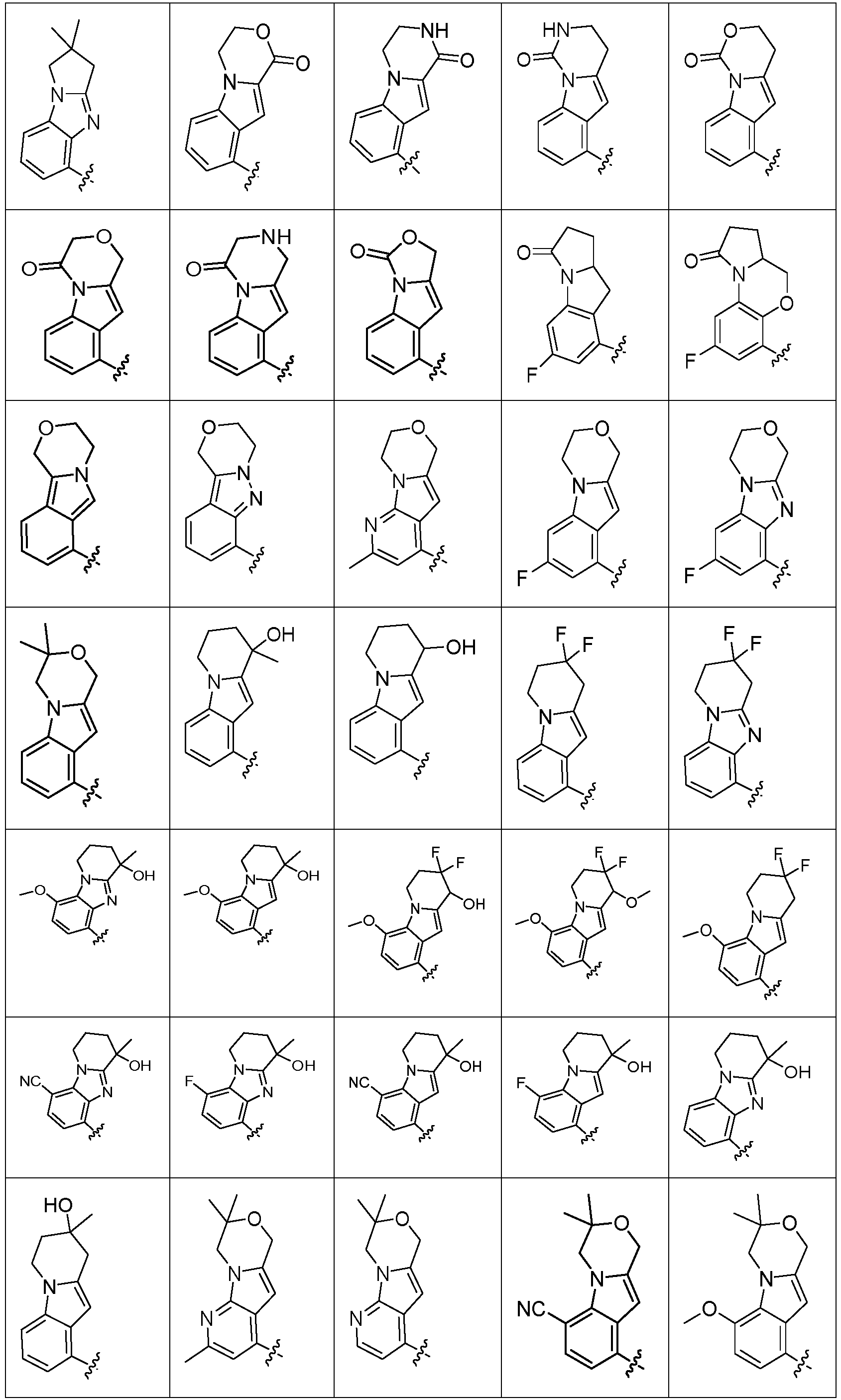

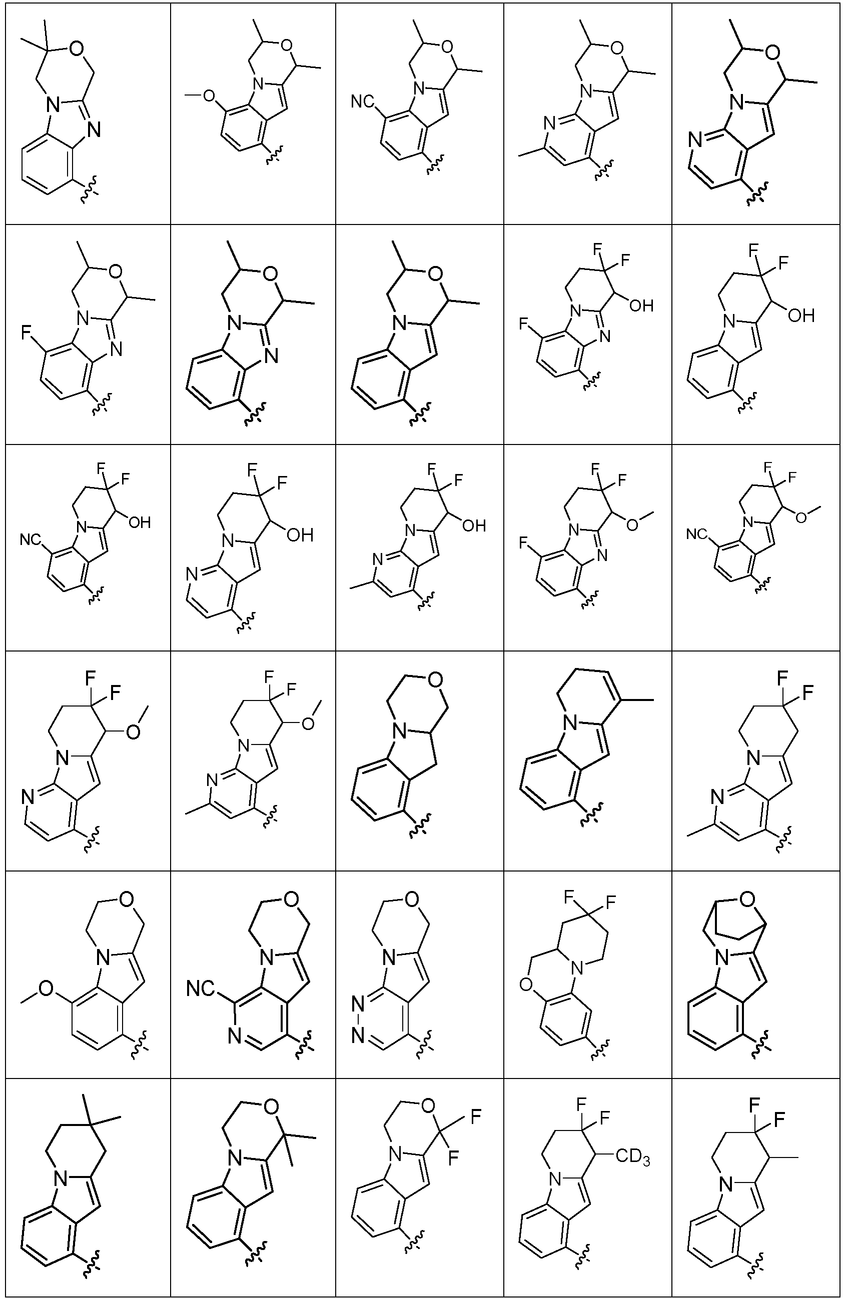

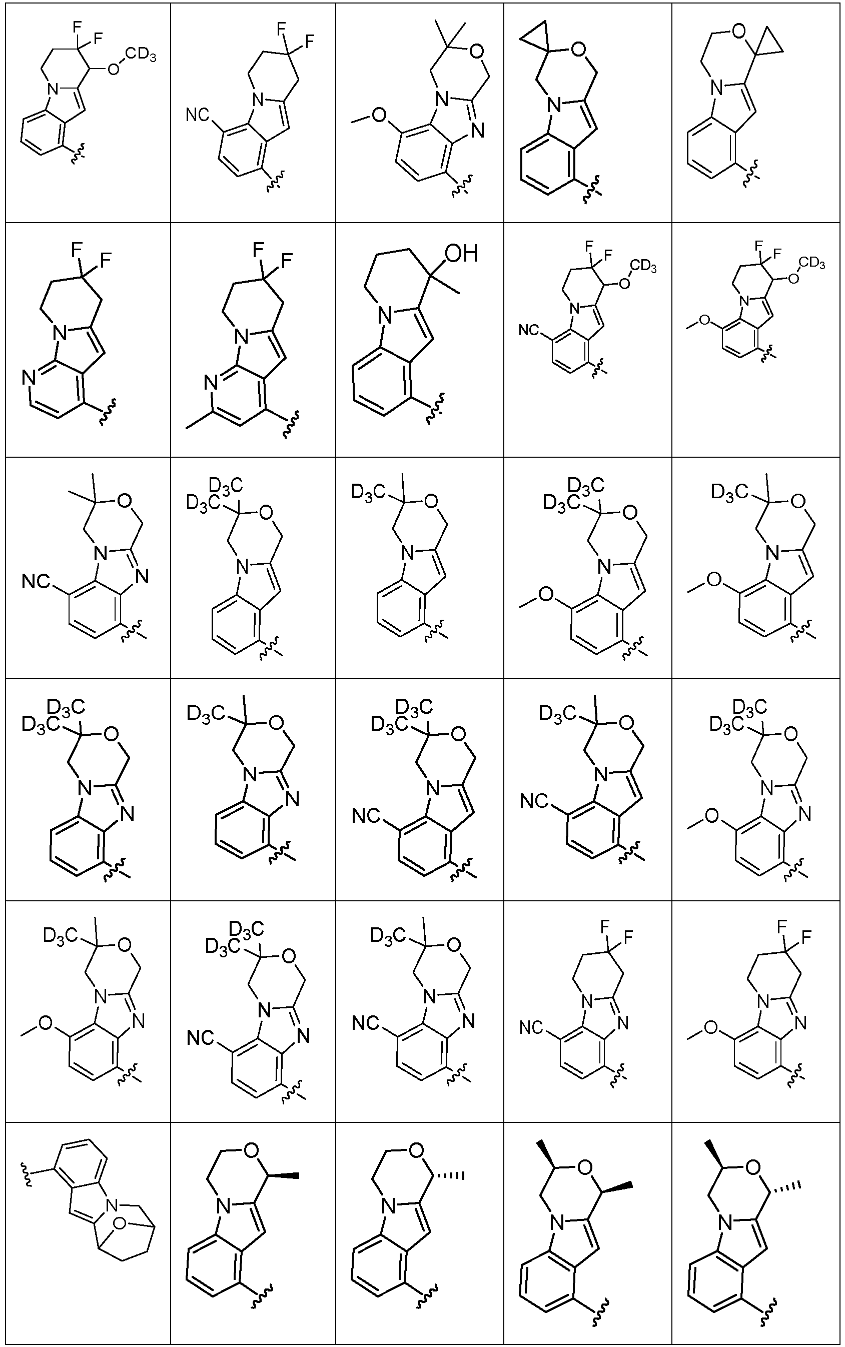

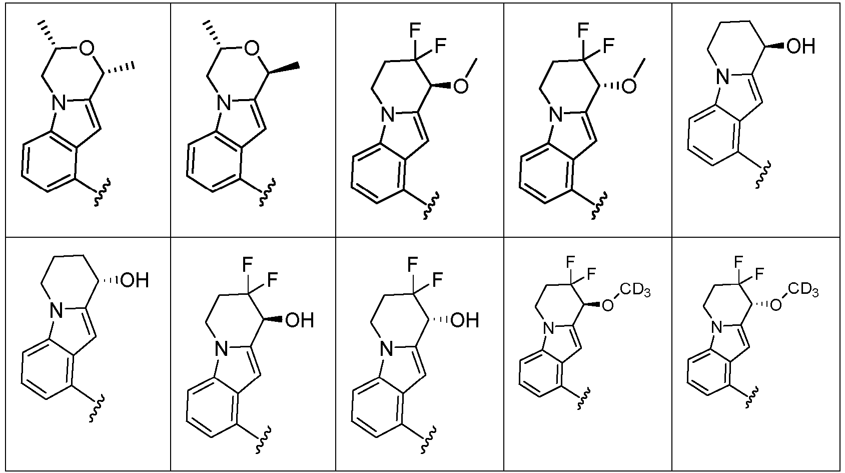

91. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

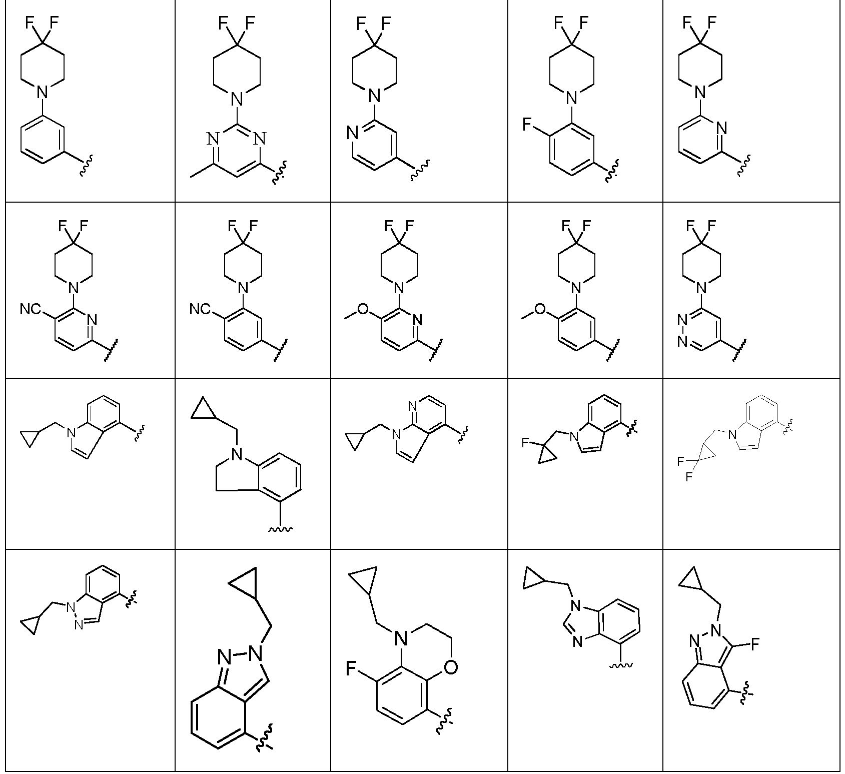

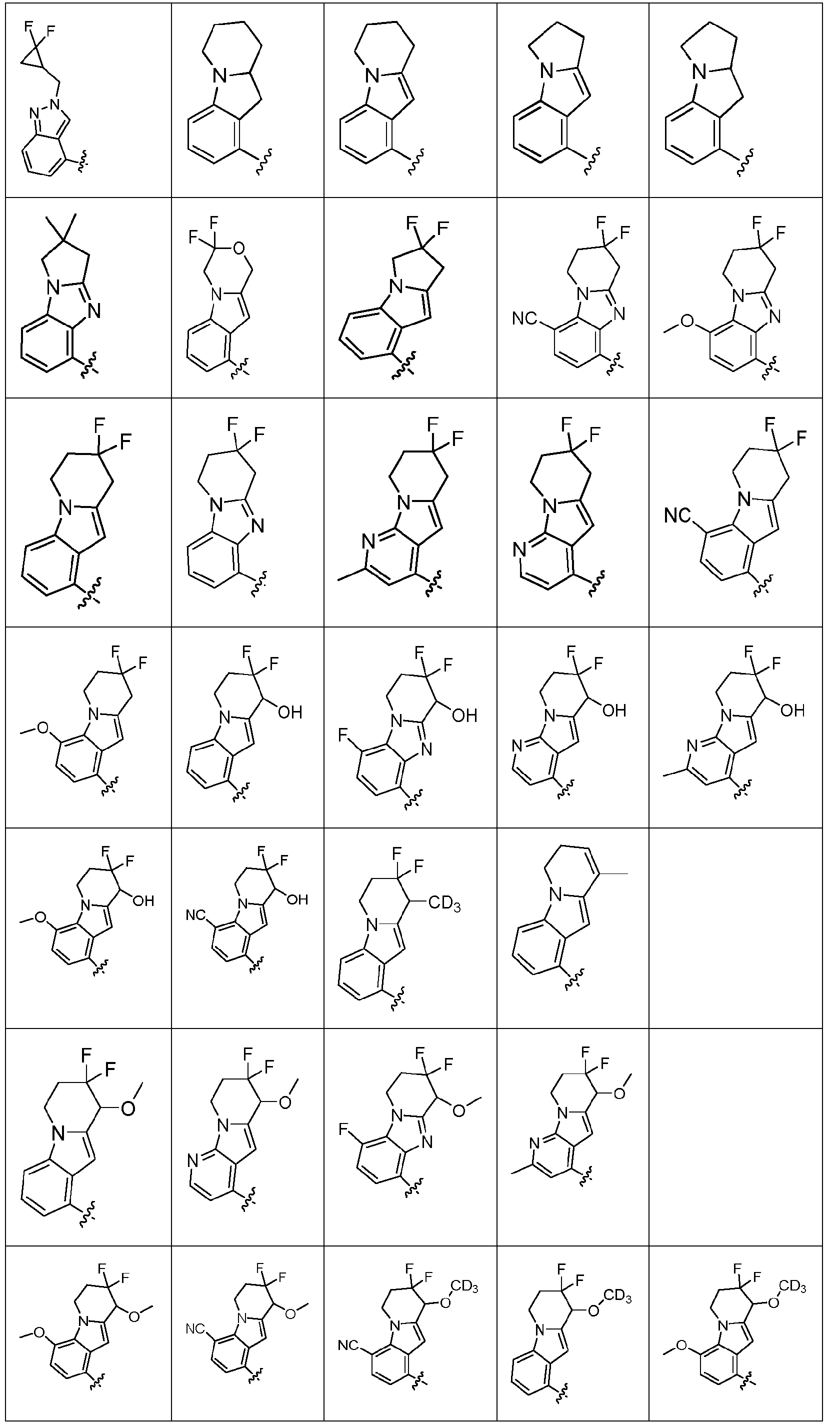

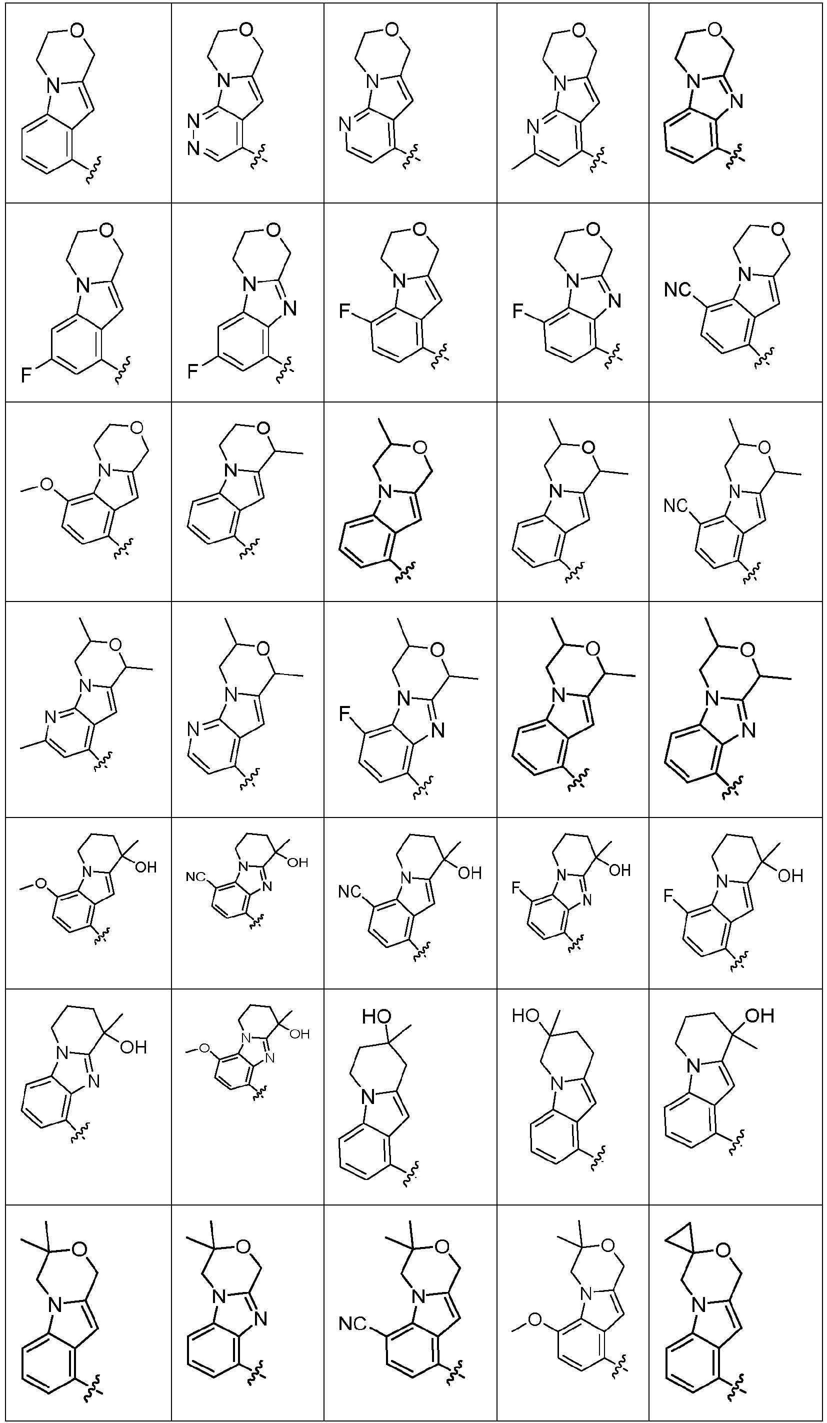

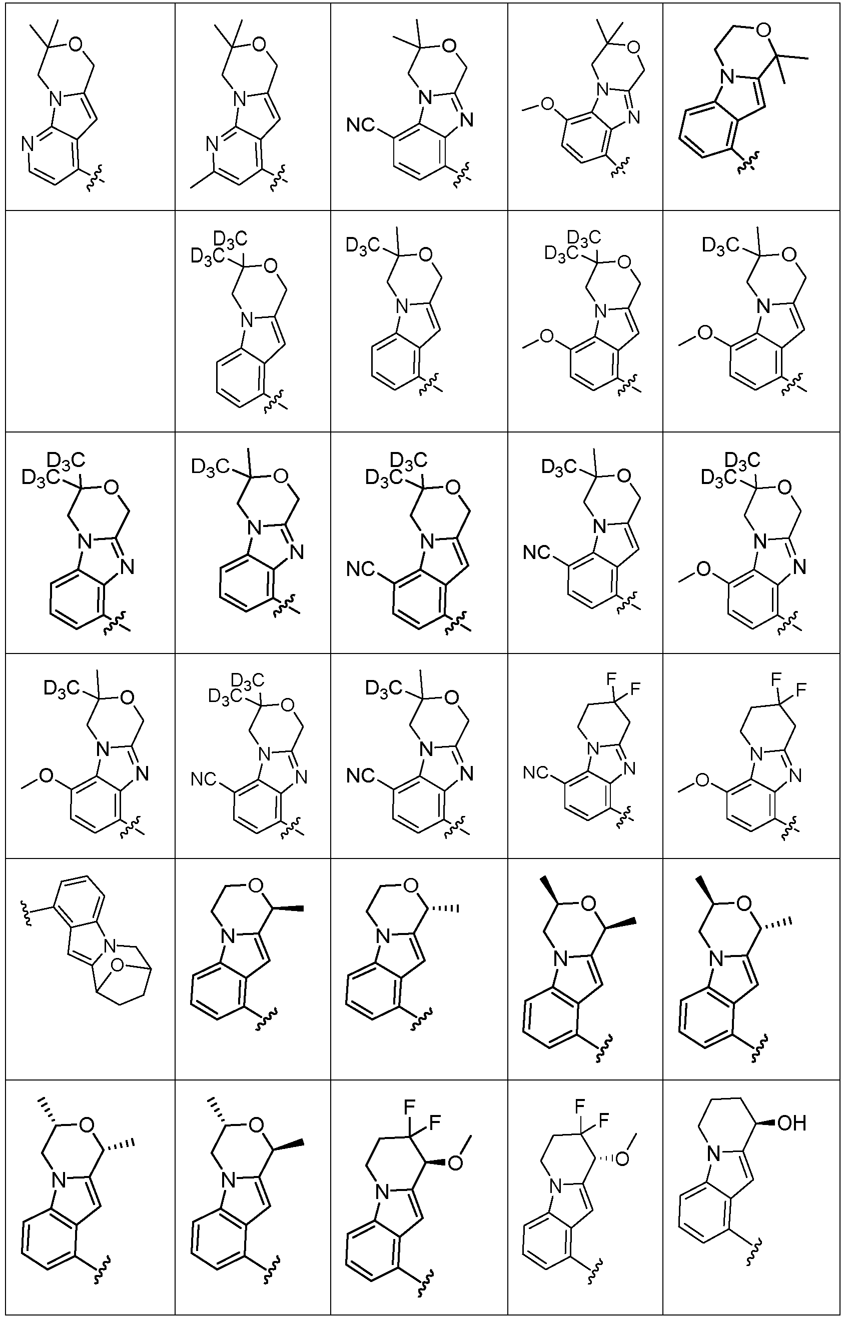

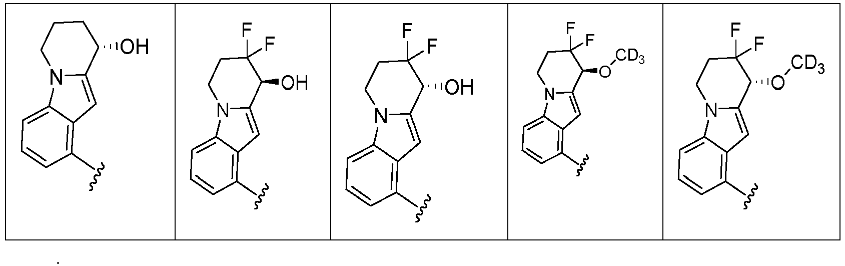

92. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

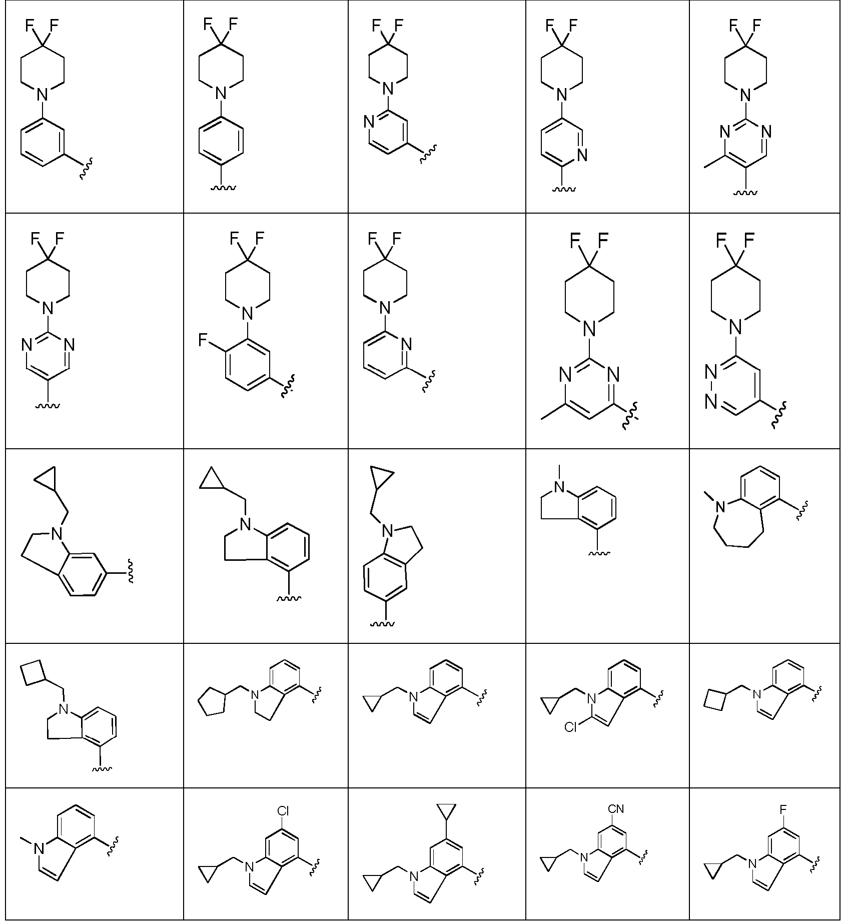

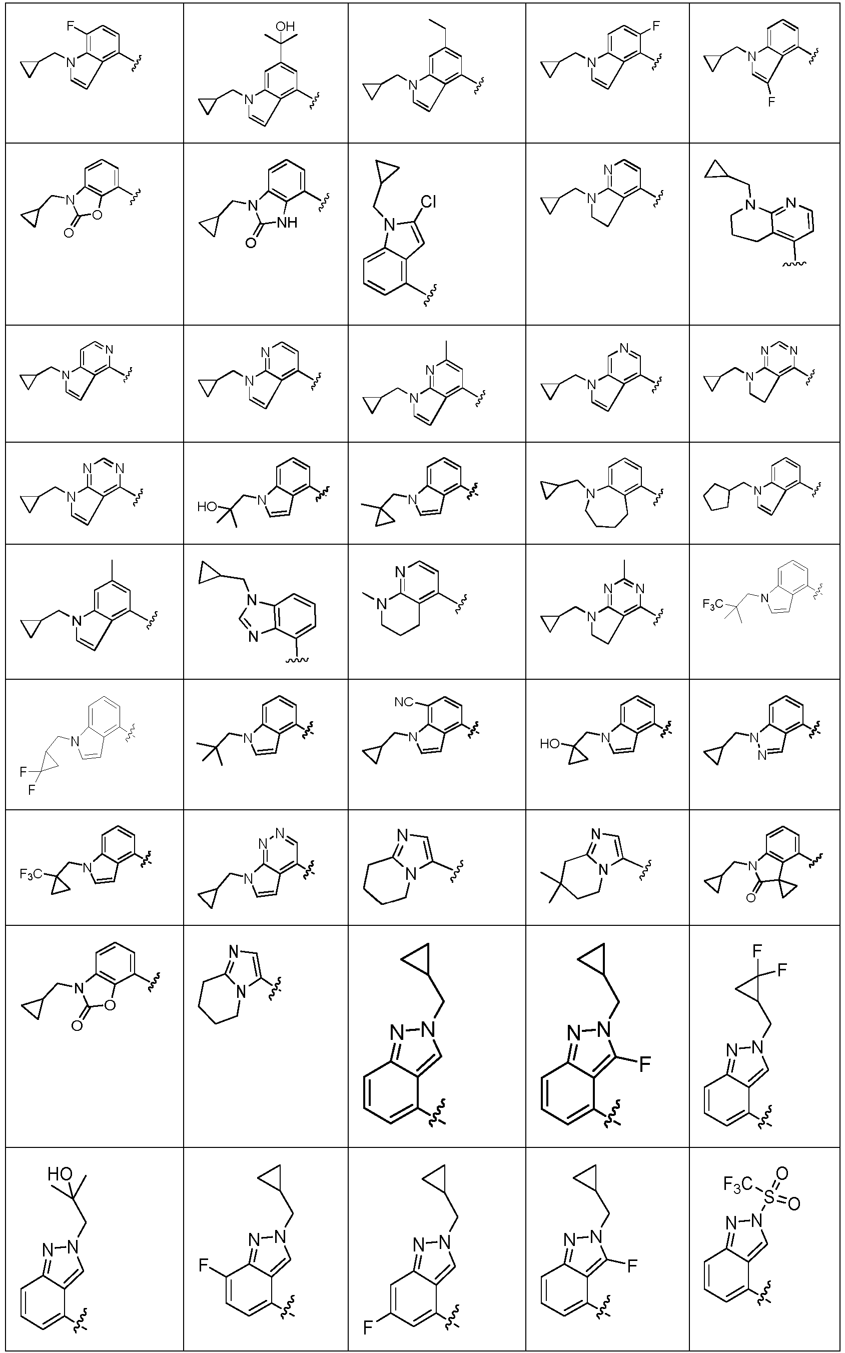

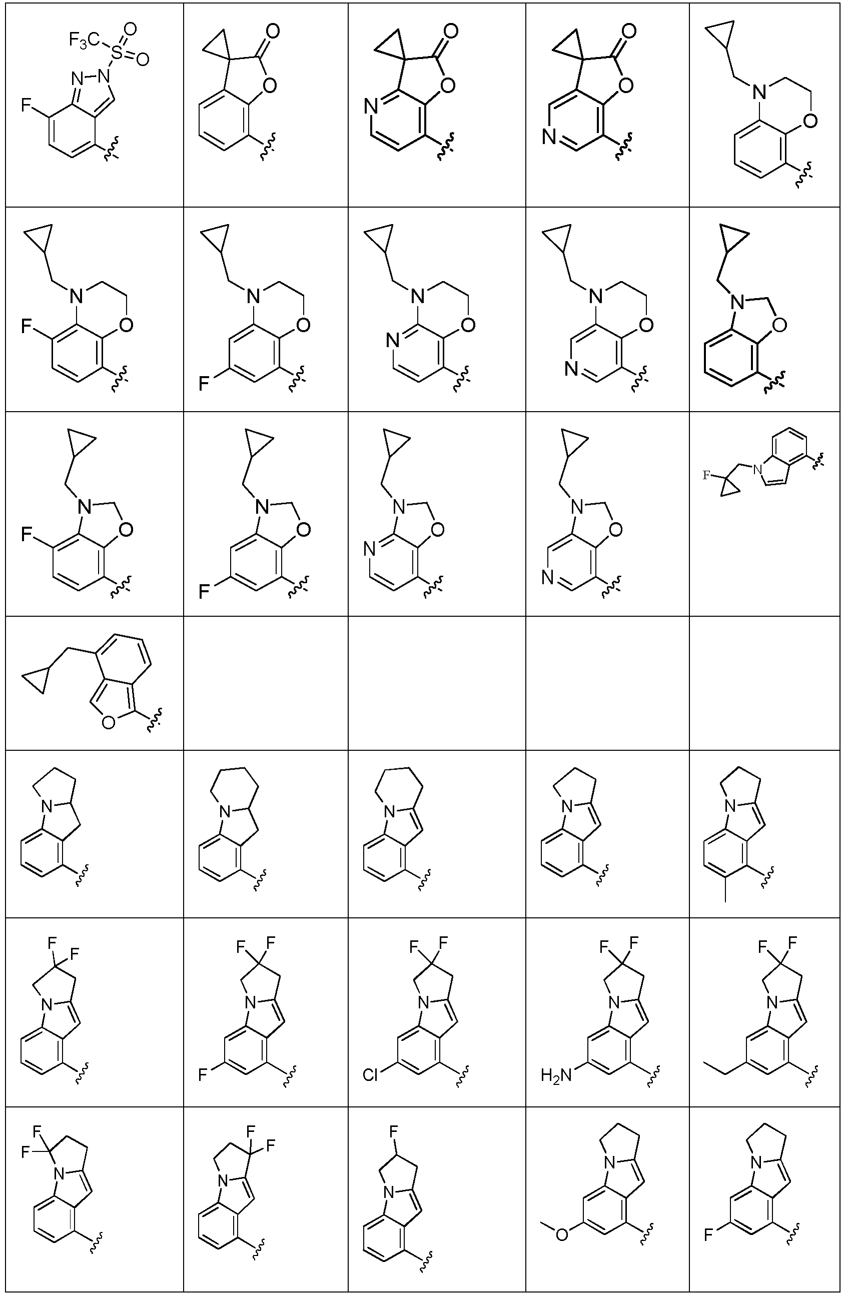

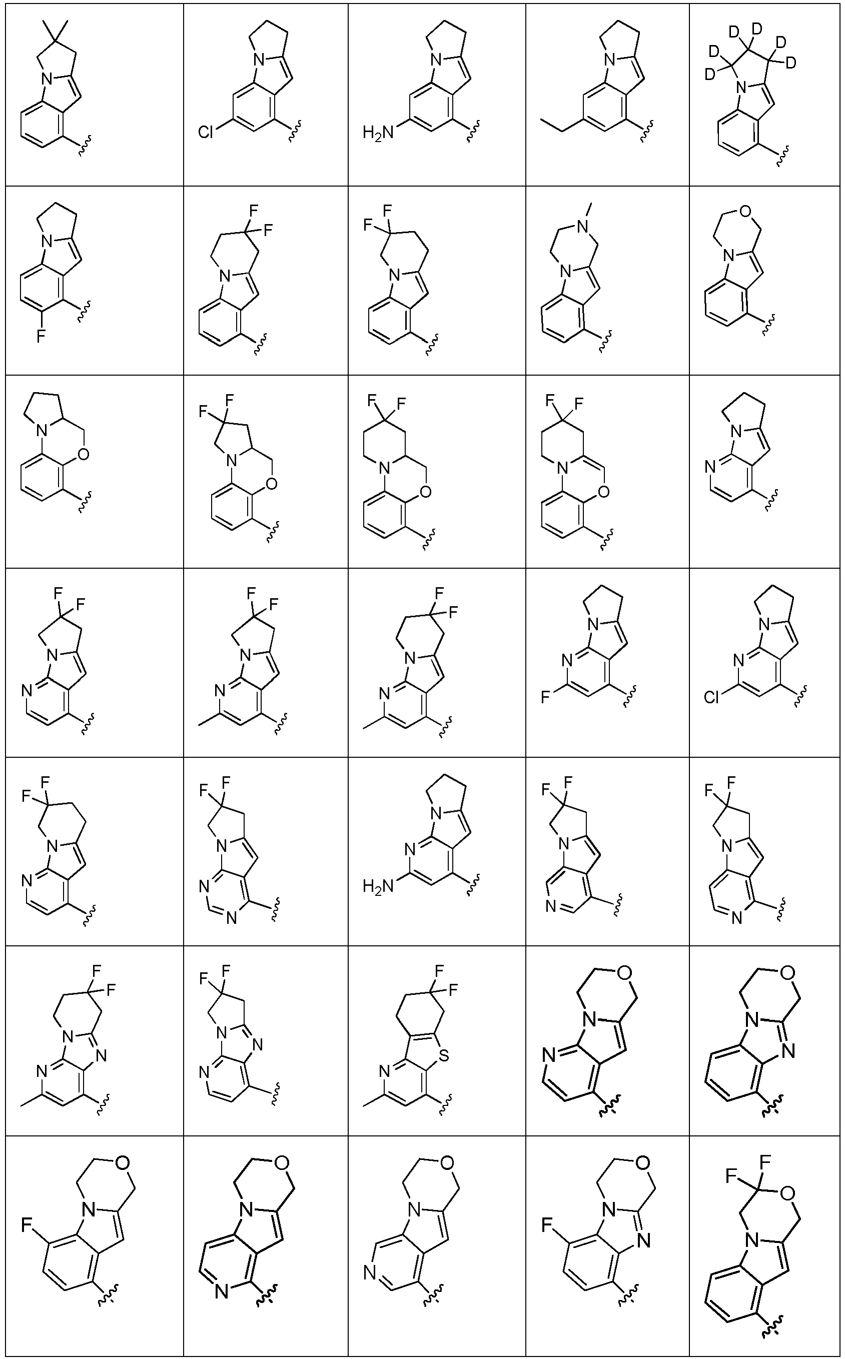

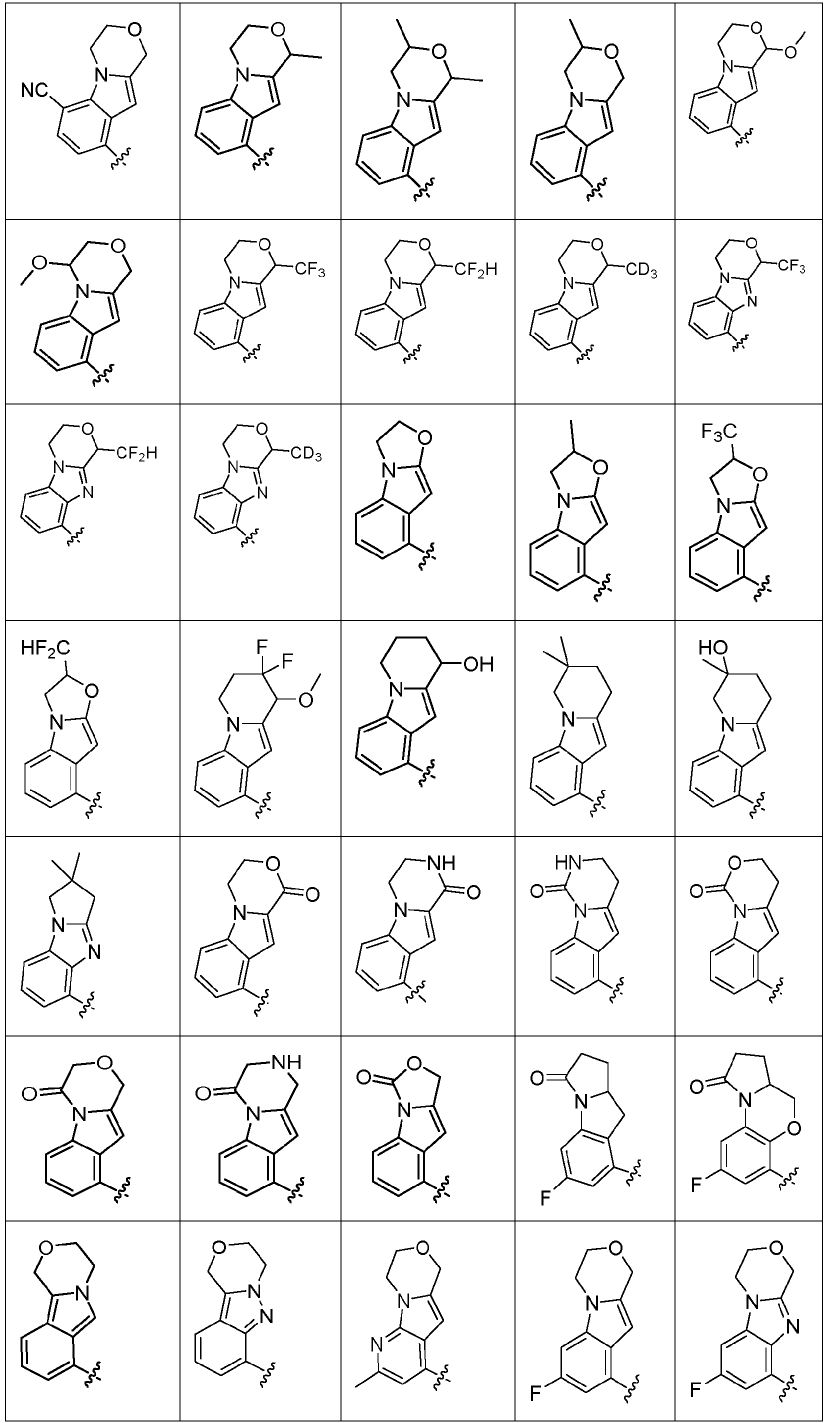

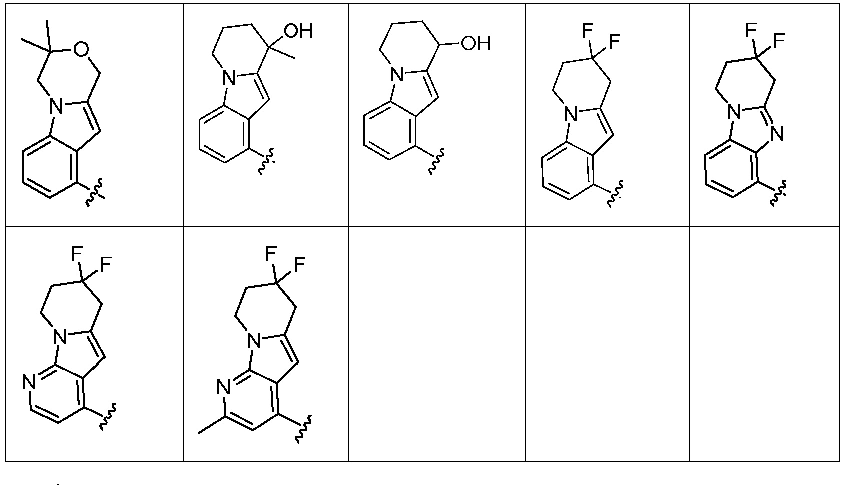

93. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

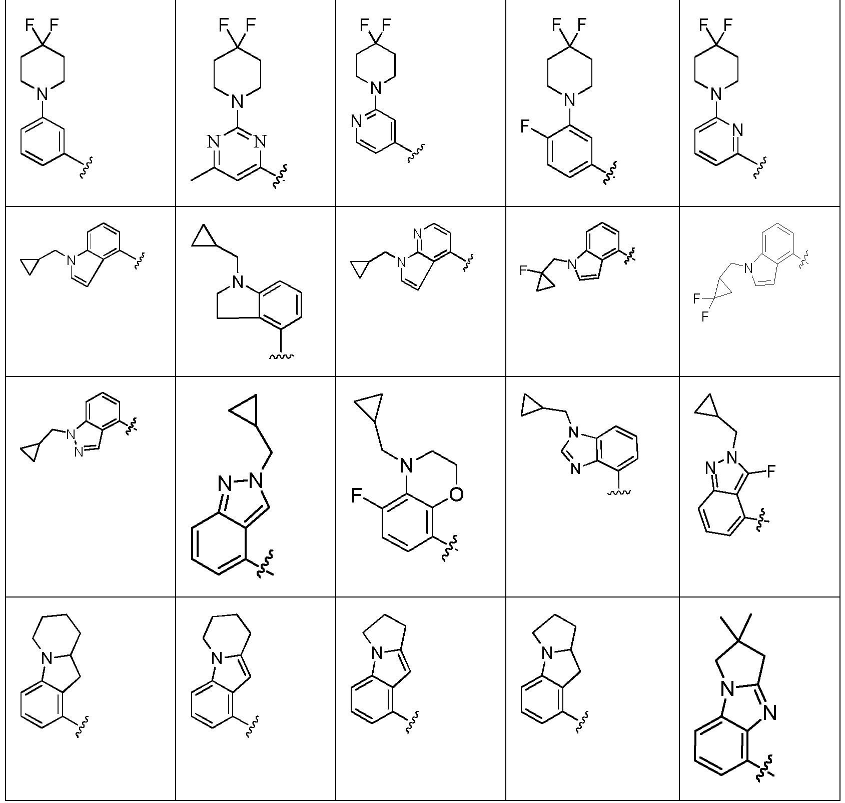

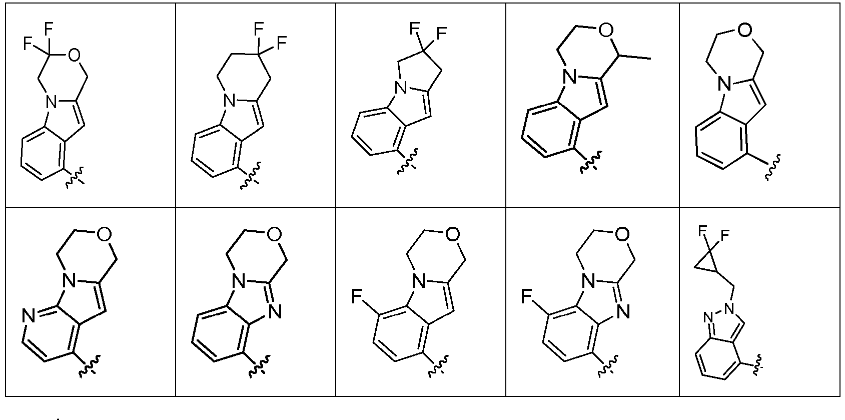

94. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-85, **characterized in that** A is selected from any one of the following groups:

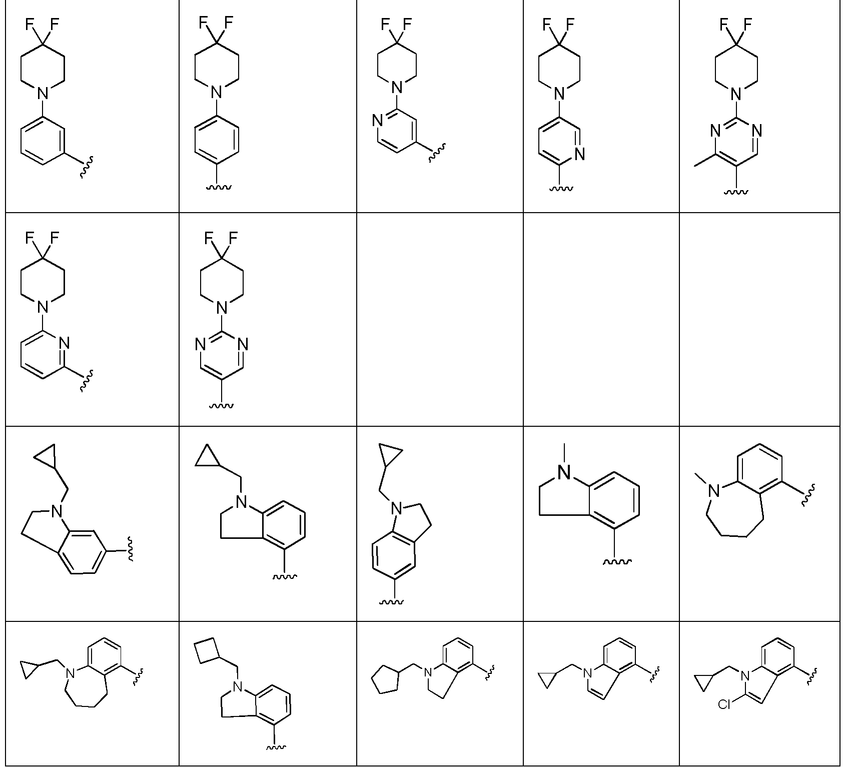

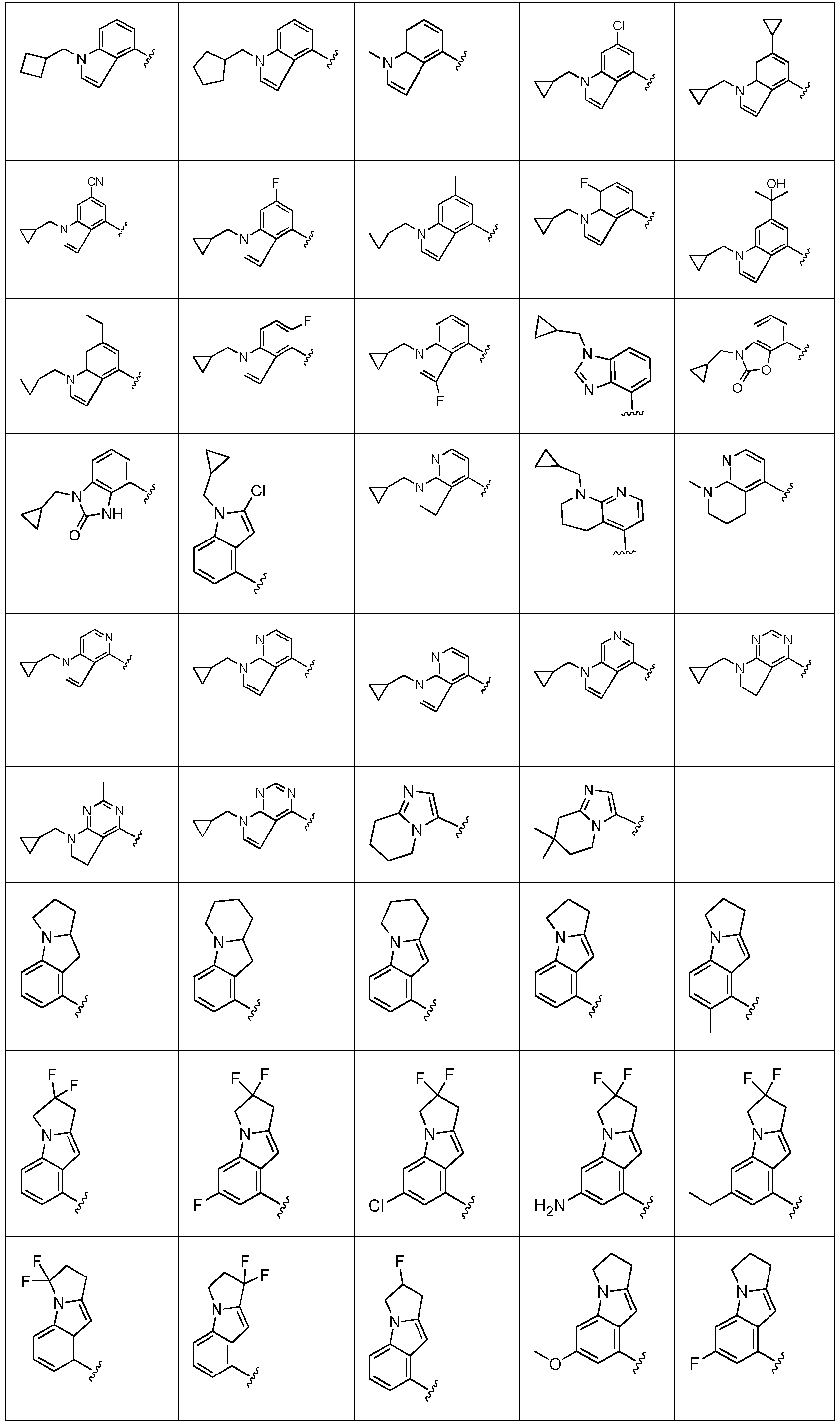

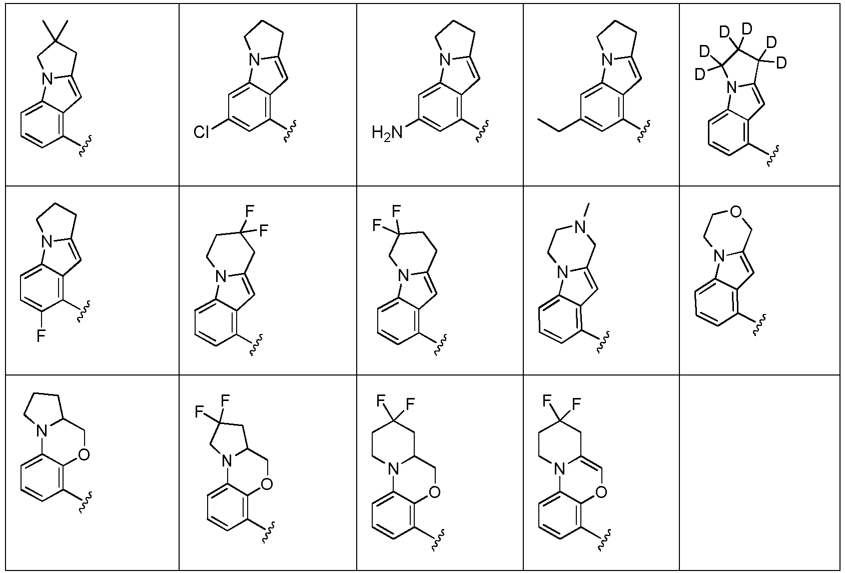

95. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-94, **characterized in that** L is selected from - NR³CONR³-; and/or X₇ is CR₇, and R₇ is -NRₐRₐ.

96. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-95, **characterized in that** R₁ is group -Z-R₁₂, wherein -Z is a single bond, -C1-C4 alkyl-, -C1-C4 alkylene-O-, -O-, -S-, -S(=O)-, -SO₂-, -NH-, -NHSO₂-, -SO₂NH-, -NH-S(=O)(=NH)-, -S(=O)(=NH)-, -C1-C4 alkylene-SO₂-, -(C=O)-, -(C=O)NH-, -C=N(OH)- or -NH(C=O)-; and/or
(a) R₁₂ is H;
(b) R₁₂ is oxetanyl or cyclopropyl substituted with 0, 1, 2 or 3 groups selected from OH, halogen (such as F or Cl), or C1-C6 alkoxy; or
(c) R₁₂ is C1-C6 alkyl substituted with 0, 1, 2 or 3 groups selected from OH, halogen (such as F), or C1-C6 alkoxy.

97. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-96, **characterized in that** the group -Z-R₁₂ is - N=S(=O)-(R₁₂)₂, wherein a pair of two R₁₂, alternatively together with the sulphur atom to which they are each attached, may form saturated or partially saturated 3-, 4-, 5-, or 6-membered monocyclic ring, which comprises 0, 1, 2, or 3 N atoms and 0, 1, or 2 atoms selected from O and S;
or in the group -Z-R₁₂, Z is a single bond, and R₁₂ is independently selected from the group consisting of: saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₘ, -OC1-C6 haloalkyl, CN, -C(=O)Rₙ, -C(=O)ORₘ, -C(=O)NRₘRₘ, -C(=NRₘ)NRₘRₘ, - OC(=O)Rₙ, -OC(=O)NRₘRₘ, -OC2-C6 alkylene NRₘRₘ, -OC2-C6 alkylene ORₘ, -SRₘ, -S(=O)Rₙ, -S(=O)₂Rₙ, -S(=O)₂NRₘRₘ, -NRₘRₘ, -N(Rₘ)C(=O)Rₙ, - N(Rₘ)C(=O)ORₙ, -N(Rₘ)C(=O)NRₘRₘ, -N(Rₘ)C(=NRₘ)NRₘRₘ, - N(Rₘ)S(=O)₂Rₙ, -N(Rₘ)S(=O)₂NRₘRₘ, -NRₘC2-6 alkylene NRₘRₘ, -NRₘC2-C6 alkylene ORₘ, -C1-C6 alkylene NRₘRₘ, -C1-C6 alkylene ORₘ, -C1-C6 alkylene N(Rₘ)C(=O)Rₙ, -C1-C6 alkylene OC(=O)Rₙ, -C1-C6 alkylene C(=O)NRₘRₘ, -C1-C6 alkylene C(=O)ORₘ or oxo, and Rₘ and Rₙ are independently selected from H and -C1-C6 alkyl.

98. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-97, **characterized in that** R₁ is group -Z-R₁₂, wherein Z is -NHSO₂- or -SO₂NH-; and R₁₂ is oxetanyl or cyclopropyl substituted with 0, 1, 2 or 3 groups selected from OH, halogen (such as F), or C1-C6 alkoxy, or R₁₂ is C1-C6 alkyl substituted with 0, 1, 2 or 3 groups selected from OH, halogen (such as F), or C1-C6 alkoxy.

99. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-95, **characterized in that** R₁ is selected from the following groups:

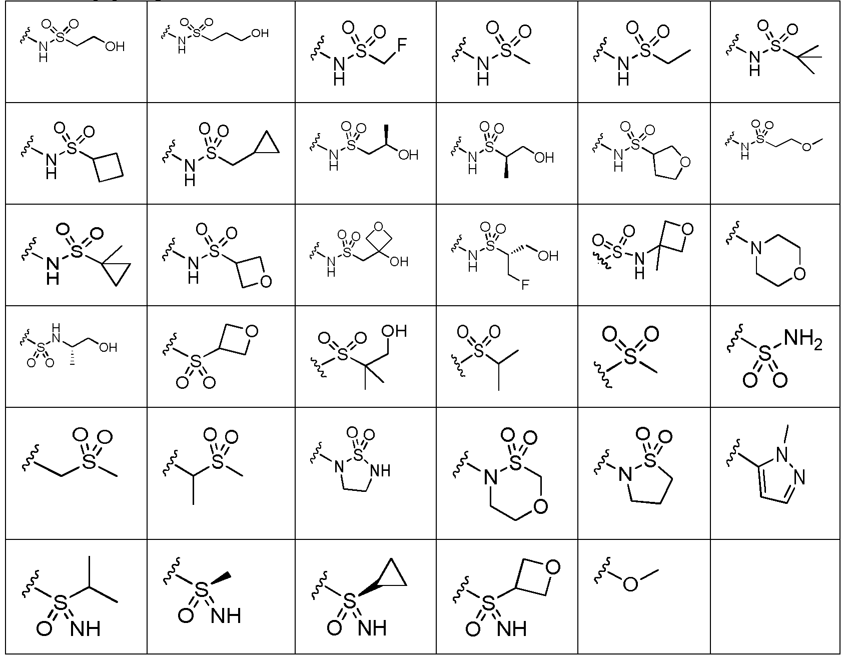

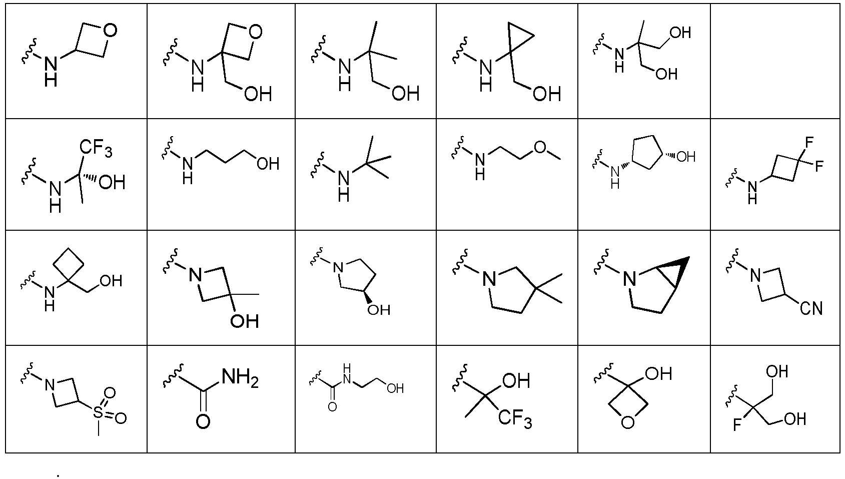

100. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-99, **characterized in that** Rₓ is selected from the group consisting of: 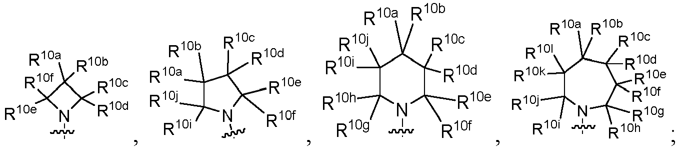
or alternatively, each of the R^{10a} and R^{10b} pair, R^{10c} and R^{10d} pair, R^{10e} and R^{10f} pair, R^{10g} and R^{10h} pair, or R¹⁰ⁱ and R^{10j} pair, independently together with the carbon atom to which they are each attached, may form saturated or partially saturated 3-, 4-, 5-, or 6-membered monocyclic ring spiro-connected to the Rₓ ring, wherein the 3-, 4-, 5-, or 6-membered monocyclic ring comprises 0, 1, 2 or 3 N atoms and 0, 1 or 2 atoms selected from O and S, and further, the 3-, 4-, 5-, or 6-membered monocyclic ring is substituted with 0, 1, 2, or 3 groups selected from: F, Cl, Br, C1-C6 alkyl, C1-C4 haloalkyl, -ORₐ, -OC1-C4 haloalkyl, CN, -NR^{a}R^{a} or oxo;
R^{10k} is selected from the group consisting of: H, saturated, partially saturated or unsaturated 3-, 4-, 5-, 6- or 7-membered monocyclic ring or 4-, 5-, 6-, 7-, 8-, 9-, 10-, 11- or 12-membered bicyclic ring, which comprises 0, 1, 2 or 3 N atoms and 0, 1 or 2 atoms selected from O and S, wherein the monocyclic ring or bicyclic ring is substituted with 0, 1, 2 or 3 groups selected from: F, Cl, Br, C1-C6 alkyl, C1-C6 haloalkyl, -ORₐ, -OC1-C6 haloalkyl, CN, -C(=O)R_{b}, -C(=O)ORₐ, -C(=O)NRₐRₐ, - C(=NRₐ)NRₐRₐ, -OC(=O)R_{b}, -OC(=O)NRₐRₐ, -OC2-C6 alkyl NRₐRₐ, -OC2-C6 alkyl ORₐ, -SRₐ, -S(=O)R_{b}, -S(=O)₂R_{b}, -S(=O)₂NRₐRₐ, -NRₐRₐ, -N(Rₐ)C(=O)R_{d}, - N(Rₐ)C(=O)OR_{d}, -N(Rₐ)C(=O)NRₐRₐ, -N(Rₐ)C(=NRₐ)NRₐRₐ, -N(Rₐ)S(=O)₂R_{d}, - N(Rₐ)S(=O)2NRₐRₐ, -NRₐC2-C6 alkylene NRₐRₐ, -NRₐC2-C6 alkylene ORₐ, -C1-C6 alkylene NRₐRₐ, -C1-C6 alkylene ORₐ, -C1-C6 alkylene N(Rₐ)C(=O)R_{b}, -C1-C6 alkylene OC(=O)R_{b}, -C1-C6 alkylene C(=O)NRₐRₐ, -C1-C6 alkylene C(=O)ORₐ, or oxo, and Rₐ and R_{b} are independently selected from H or -C1-C6 alkyl;
R¹⁰¹ is selected from the group consisting of: C1-C6 alkyl substituted with 0, 1, 2, 3, 4 or 5 groups selected from F, Cl, Br, C1-C6 alkoxy, -O-C1-C6 haloalkyl or CN.

101. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-100, **characterized in that** Rₓ is selected from:

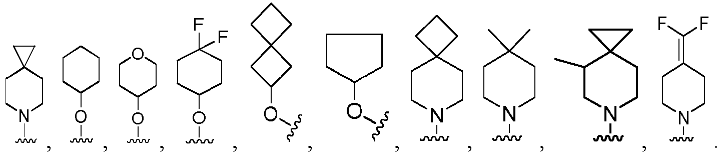

102. A compound represented by formula (I-C-1) or formula (I-C-1'), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 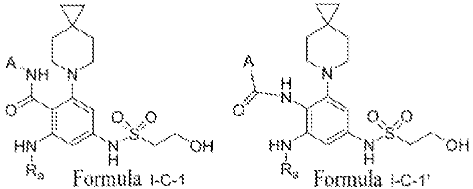 **characterized in that** A is a group selected from the A1 group according to any one of claims 27-30, and Rₐ is H or C1-C4 alkyl.

103. A compound represented by formula (I-C-2) or formula (I-C-2'), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 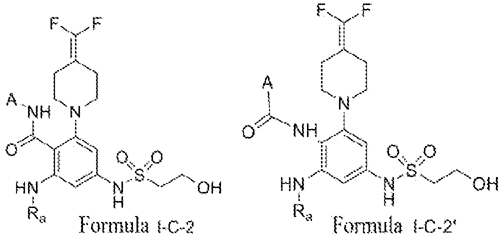 **characterized in that** A is a group selected from the A1 group according to any one of claims 27-30, and Rₐ is H or C1-C4 alkyl.

104. A compound represented by formula (I-A-1), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 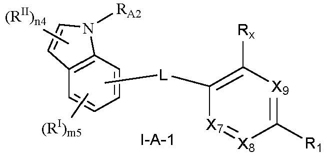 **characterized in that** L, R_{X}, R₁, X₇, X₈, and X₉ are as defined in any one of claims 1-26, and R_{A2}, R^{I}, R^{II}, n4, and m5 are as defined in any one of claims 27-30 and 47-50.

105. The compound represented by formula (I-A-1), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 104, **characterized in that** the compound is

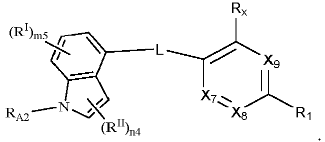

106. A compound represented by formula (I-A-2), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 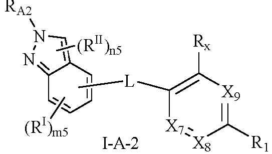 **characterized in that** L, R_{X}, R₁, X₇, X₈, and X₉ are as defined in any one of claims 1-26, and R_{A2}, R^{I}, R^{II}, n5, and m5 are as defined in any one of claims 27-30 and 53-54.

107. The compound represented by formula (I-A-2), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 106, **characterized in that** the compound is

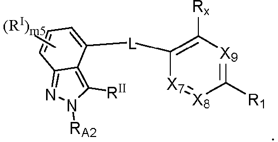

108. A compound represented by formula (I-A-3), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 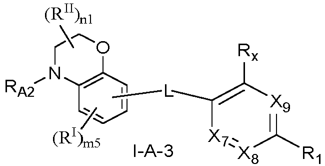 **characterized in that** L, R_{X}, R₁, X₇, X₈, and X₉ are as defined in any one of claims 1-26, and R_{A2}, R^{I}, R^{II}, n1, and m5 are as defined in any one of claims 27-30.

109. The compound represented by formula (I-A-3), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 108, **characterized in that** the compound is

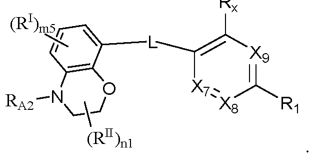

110. A compound represented by formula (I-A-4), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 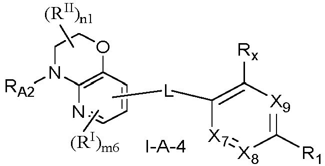 **characterized in that** L, R_{X}, R₁, X₇, X₈, and X₉ are as defined in any one of claims 1-26, and R_{A2}, R^{I}, R^{II}, n1, and m6 are as defined in any one of claims 27-30.

111. The compound represented by formula (I-A-4), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 110, **characterized in that** the compound is

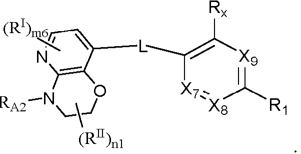

112. A compound represented by formula (I-A-5), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 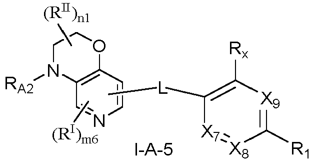 **characterized in that** L, R_{X}, R₁, X₇, X₈, and X₉ are as defined in any one of claims 1-26, and R_{A2}, R^{I}, R^{II}, n1, and m6 are as defined in any one of claims 27-30.

113. The compound represented by formula (I-A-5), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 112, **characterized in that** the compound is

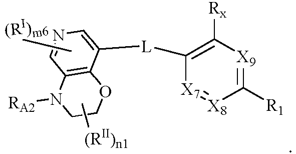

114. A compound represented by formula (I-A-6), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 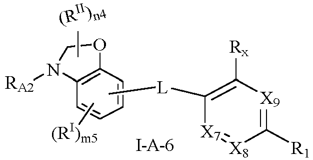 **characterized in that** L, R_{X}, R₁, X₇, X₈, and X₉ are as defined in any one of claims 1-26, and R_{A2}, R^{I}, R^{II}, n4, and m5 are as defined in any one of claims 27-30.

115. The compound represented by formula (I-A-6), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 114, **characterized in that** the compound is

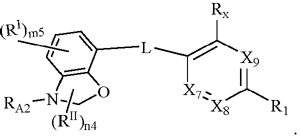

116. A compound represented by formula (I-A-7), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 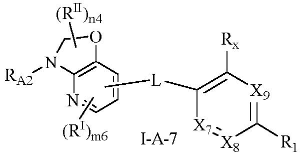 **characterized in that** L, R_{X}, R₁, X₇, X₈, and X₉ are as defined in any one of claims 1-26, and R_{A2}, R^{I}, R^{II}, n4, and m6 are as defined in any one of claims 27-30.

117. The compound represented by formula (I-A-7), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 116, **characterized in that** the compound is

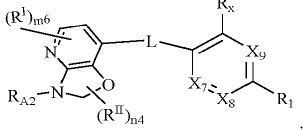

118. A compound represented by formula (I-A-8), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 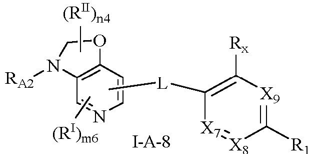 **characterized in that** L, R_{X}, R₁, X₇, X₈, and X₉ are as defined in any one of claims 1-26, and R_{A2}, R^{I}, R^{II}, n4, and m6 are as defined in any one of claims 27-30.

119. The compound represented by formula (I-A-8), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 118, **characterized in that** the compound is

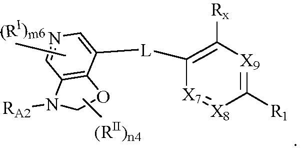

120. A compound represented by formula (I-B-1), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 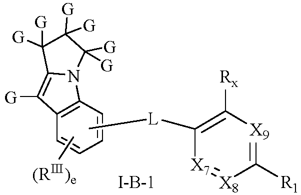 **characterized in that** L, R_{X}, R₁, X₇, X₈, and X₉ are as defined in any one of claims 1-26, and G, R^{III}, and e are as defined in any one of claims 27-30.

121. The compound represented by formula (I-B-1), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 120, **characterized in that** the compound is

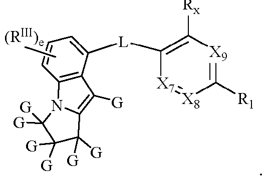

122. A compound represented by formula (I-B-2), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 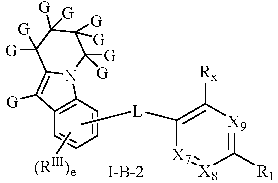 **characterized in that** L, R_{X}, R₁, X₇, X₈, and X₉ are as defined in any one of claims 1-26, and R^{III}, G, and e are as defined in any one of claims 27-30.

123. The compound represented by formula (I-B-2), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 122, **characterized in that** the compound is

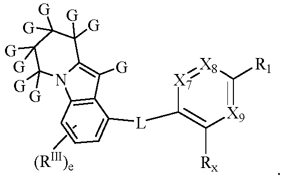

124. The compound represented by formula (I-B-2), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 123, **characterized in that** the compound is 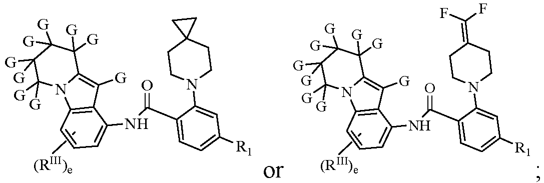 or
the compound is 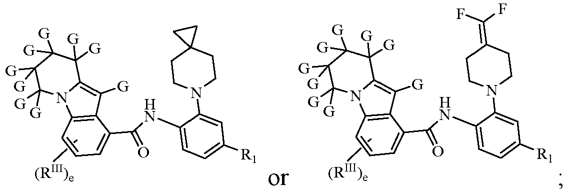
preferably, the compound is 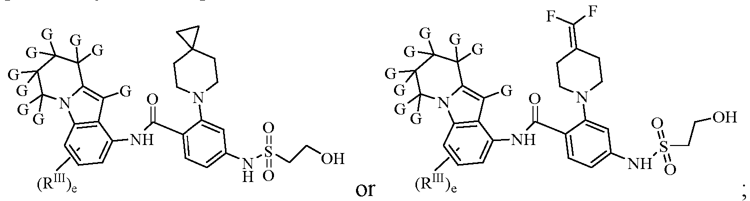
more preferably, the compound is 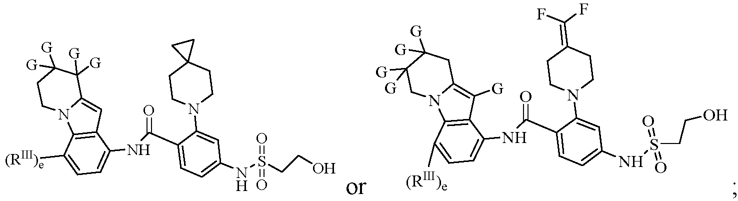 or
preferably, the compound is 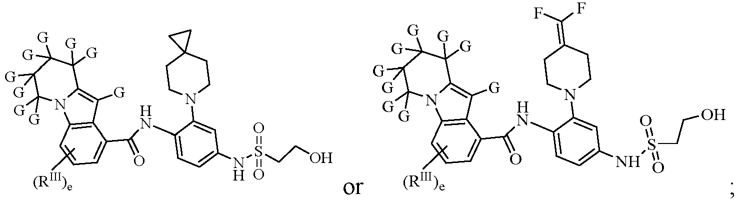 more preferably, the compound is 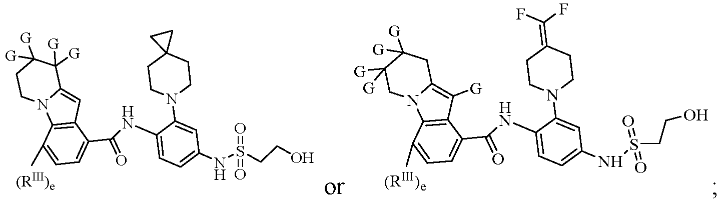
further preferably, each R^{III} is independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, -NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5-to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or -N(C1-C6 alkyl)(C1-C6 alkyl), or two G attached to the same carbon atom form an oxo group (=O);
further preferably, each e is 1.

125. A compound represented by formula (I-B-3), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 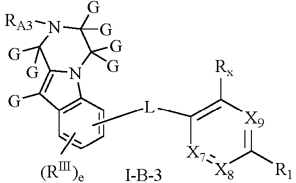 **characterized in that** L, R_{X}, R₁, X₇, X₈, and X₉ are as defined in any one of claims 1-26, and G, R^{III}, R_{A3}, and e are as defined in any one of claims 27-30.

126. The compound represented by formula (I-B-3), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 125, **characterized in that** the compound is

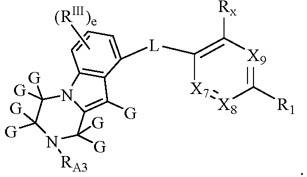

127. A compound represented by formula (I-B-4), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 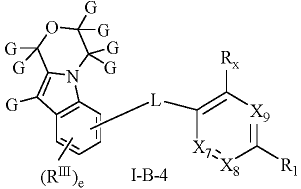 **characterized in that** L, R_{X}, R₁, X₇, X₈, and X₉ are as defined in any one of claims 1-26, and G, R^{III}, and e are as defined in any one of claims 27-30.

128. The compound represented by formula (I-B-4), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 127, **characterized in that** the compound is

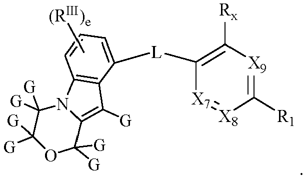

129. The compound represented by formula (I-B-4), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 128, **characterized in that**
the compound is 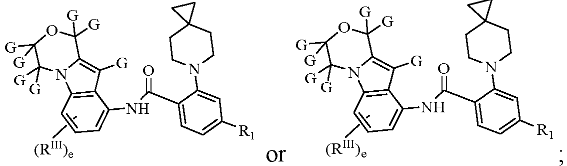
preferably, the compound is 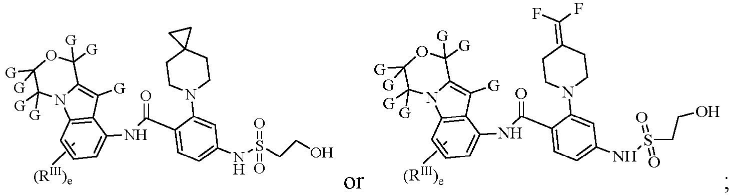
more preferably, the compound is 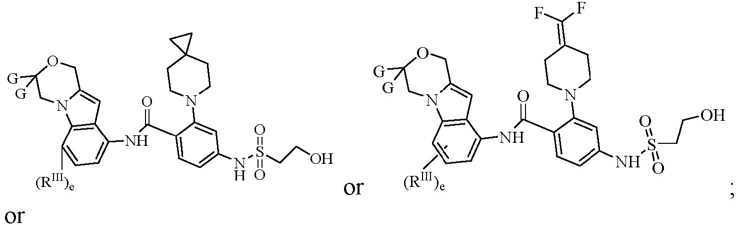
the compound is 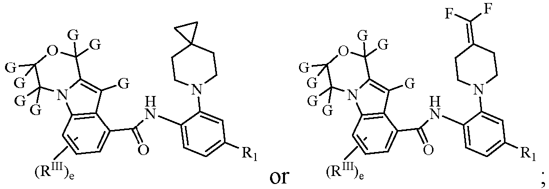
preferably, the compound is 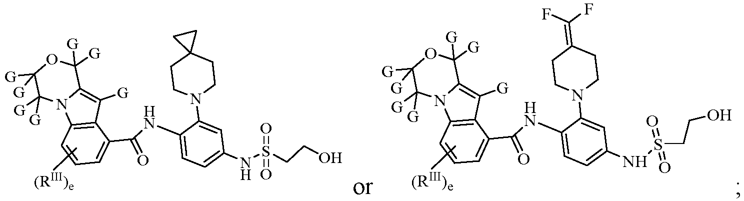
more preferably, the compound is 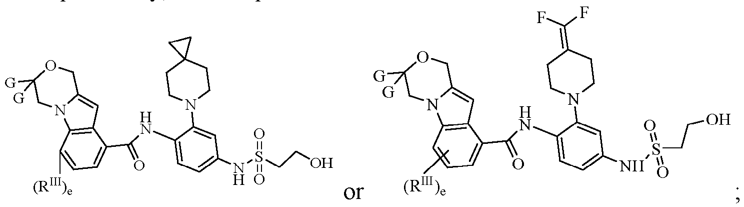
further preferably, each R^{III} is independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, -NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5-to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or -N(C1-C6 alkyl)(C1-C6 alkyl), or two G attached to the same carbon atom form an oxo group (=O);
further preferably, each e is 1.

130. A compound represented by formula (I-B-5), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 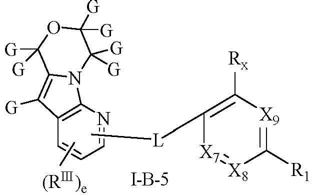 **characterized in that** L, R_{X}, R₁, X₇, X₈, and X₉ are as defined in any one of claims 1-26, and G, R^{III}, and e are as defined in any one of claims 27-30.

131. The compound represented by formula (I-B-5), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 130, **characterized in that** the compound is

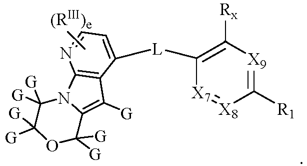

132. A compound represented by formula (I-B-6), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 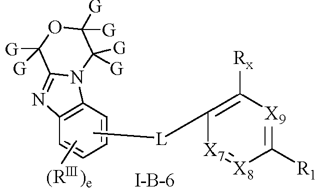 **characterized in that** L, R_{X}, R₁, X₇, X₈, and X₉ are as defined in any one of claims 1-26, and G, R^{III}, and e are as defined in any one of claims 27-30.

133. The compound represented by formula (I-B-6), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 132, **characterized in that** the compound is

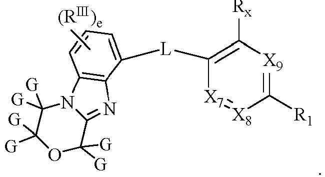

134. The compound represented by formula (I-B-6), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 133, **characterized in that**
the compound is 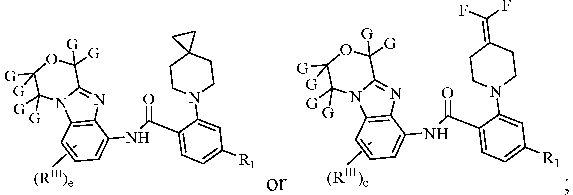
preferably, the compound is 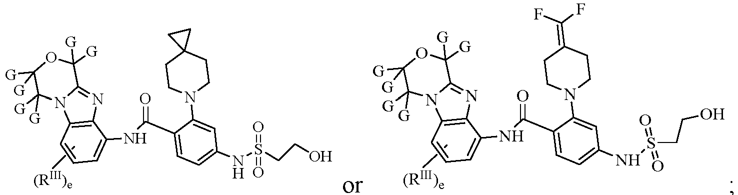 more preferably, the compound is 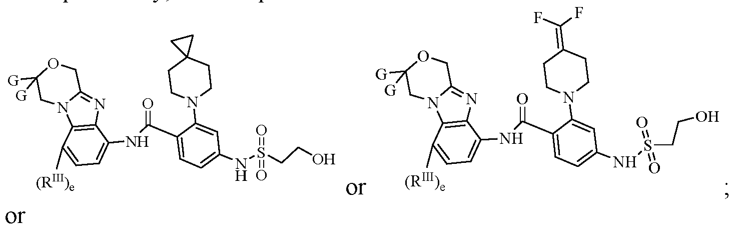
the compound is 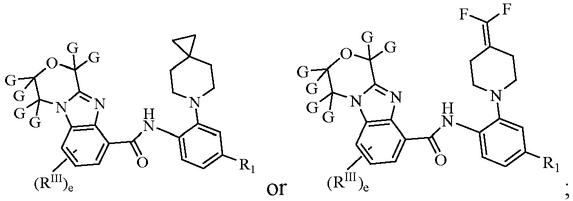
preferably, the compound is 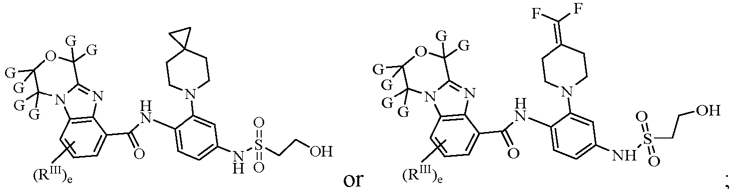
more preferably, the compound is 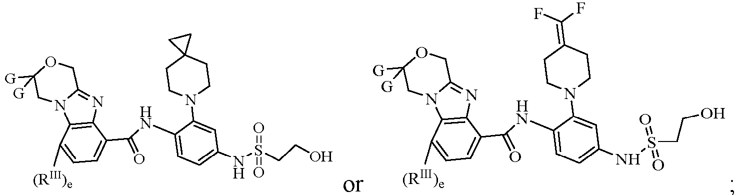
further preferably, each R^{III} is independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, -NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5-to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
and/or, each G is selected from hydrogen, deuterium, halogen, C1-C6 alkyl, C1-C6 deuteroalkyl, C1-C6 haloalkyl, -OH, C1-C6 alkoxy, C1-C6 haloalkoxy, - NH2, -NH-C1-C6 alkyl or -N(C1-C6 alkyl)(C1-C6 alkyl), or two G attached to the same carbon atom form an oxo group (=O);
further preferably, each e is 1.

135. A compound represented by formula (I-B-7), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 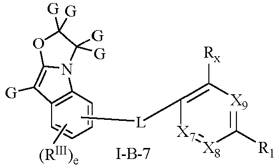 **characterized in that** L, R_{X}, R₁, X₇, X₈, and X₉ are as defined in any one of claims 1-26, and G, R^{III}, and e are as defined in any one of claims 27-30.

136. The compound represented by formula (I-B-7), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 135, **characterized in that** the compound is

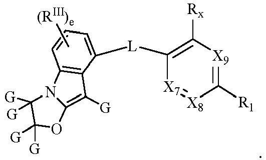

137. A compound represented by formula (I-B-8), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 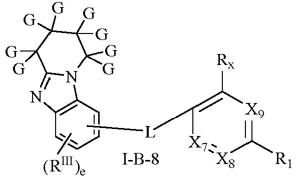 **characterized in that** L, R_{X}, R₁, X₇, X₈, and X₉ are as defined in any one of claims 1-26, and G, R^{III}, and e are as defined in any one of claims 27-30.

138. The compound represented by formula (I-B-8), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 137, **characterized in that** the compound is

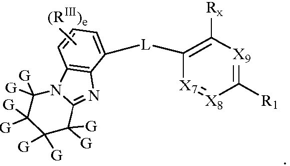

139. The compound represented by formula (I-B-8), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 138, **characterized in that**
the compound is 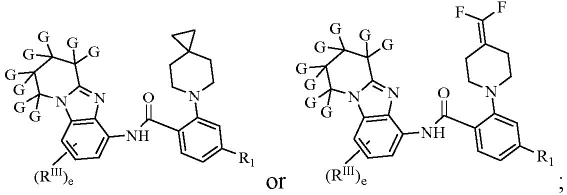
preferably, the compound is 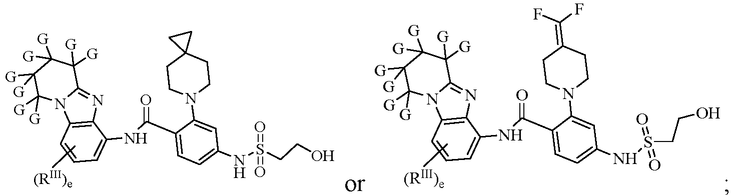
more preferably, the compound is 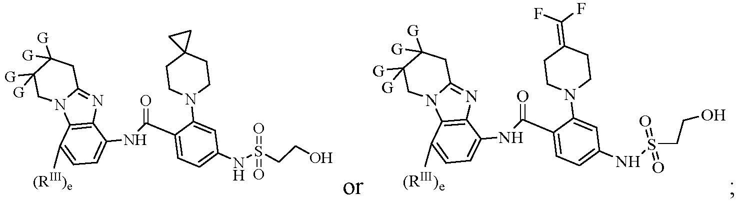 or
the compound is 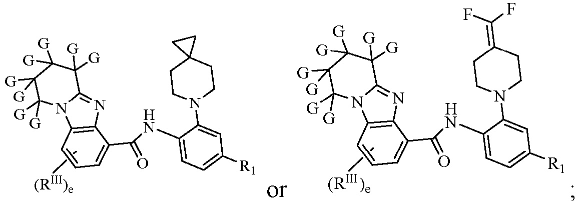
preferably, the compound is 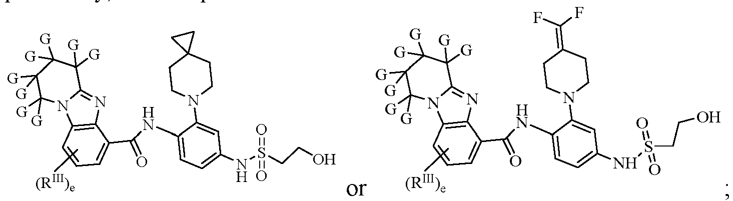
more preferably, the compound is 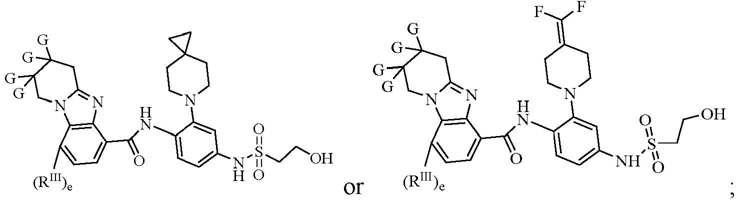
further preferably, each R^{III} is independently selected from deuterium, halogen, -CN, C1-C6 alkyl, C1-C6 haloalkyl, C1-C6 deuteroalkyl, OH, C1-C6 alkoxy, C1-C6 haloalkoxy, C1-C6 deuteroalkoxy, -O-C3-C8 cycloalkyl, -NH2, -NH-C1-C6 alkyl, -C(=O)NH-C1-C6 alkyl, -C(=O)N(C1-C6 alkyl)(C1-C6 alkyl), phenyl, -5- to 6-membered monocyclic heteroaryl-C1-C6 alkyl comprising 1, 2 or 3 N atoms, 5-to 6-membered monocyclic heteroaryl comprising 1 or 2 N atoms and 1 or 2 atoms selected from O, 5- to 6-membered monocyclic heteroaryl comprising 1, 2 or 3 N atoms, or -N(C1-C6 alkyl)(C1-C6 alkyl);
and/or, each G is independently selected from hydrogen, deuterium, halogen, C1-C6 alkyl, C1-C6 deuteroalkyl, C1-C6 haloalkyl, -OH, C1-C6 alkoxy, C1-C6 haloalkoxy, -NH2, -NH-C1-C6 alkyl or -N(C1-C6 alkyl)(C1-C6 alkyl), or two G attached to the same carbon atom form an oxo group (=O);
further preferably, each e is 1.

140. A compound represented by formula (I-B-9), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 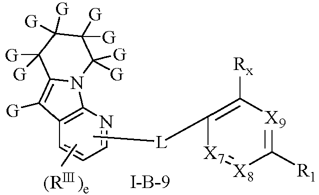 **characterized in that** L, R_{X}, R₁, X₇, X₈, and X₉ are as defined in any one of claims 1-26, and G, R^{III}, and e are as defined in any one of claims 27-30.

141. The compound represented by formula (I-B-9), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 140, **characterized in that** the compound is

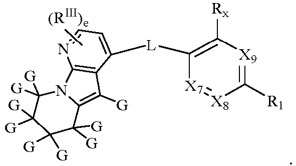

142. A compound represented by formula (I-B-10), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 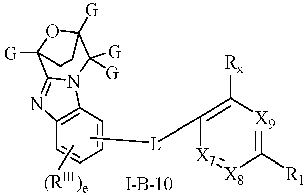 **characterized in that** L, R_{X}, R₁, X₇, X₈, and X₉ are as defined in any one of claims 1-26, and G, R^{III}, and e are as defined in any one of claims 27-30.

143. The compound represented by formula (I-B-10), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 142, **characterized in that** the compound is

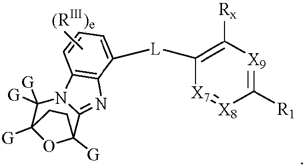

144. A compound represented by formula (I-B-11), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 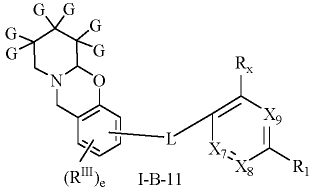 **characterized in that** L, R_{X}, R₁, X₇, X₈, and X₉ are as defined in any one of claims 1-26, and G, R^{III}, and e are as defined in any one of claims 27-30.

145. The compound represented by formula (I-B-11), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 144, **characterized in that** the compound is

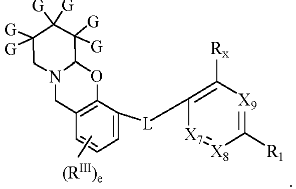

146. A compound represented by formula (I-B-12), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 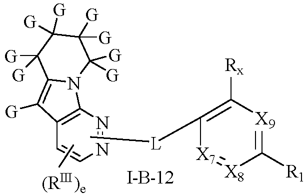 **characterized in that** L, R_{X}, R₁, X₇, X₈, and X₉ are as defined in any one of claims 1-26, and G, R^{III}, and e are as defined in any one of claims 27-30.

147. The compound represented by formula (I-B-12), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 146, **characterized in that** the compound is

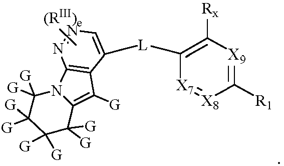

148. A compound represented by formula (I-B-13), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 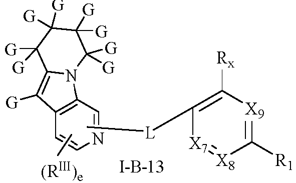 **characterized in that** L, R_{X}, R₁, X₇, X₈, and X₉ are as defined in any one of claims 1-26, and G, R^{III}, and e are as defined in any one of claims 27-30.

149. The compound represented by formula (I-B-13), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 148, **characterized in that** the compound is

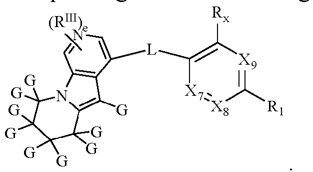

150. A compound represented by formula (I-B-14), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 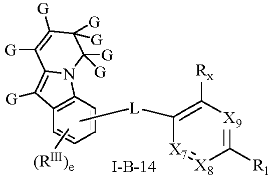 **characterized in that** L, R_{X}, R₁, X₇, X₈, and X₉ are as defined in any one of claims 1-26, and R^{III}, G, and e are as defined in any one of claims 27-30.

151. The compound represented by formula (I-B-14), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 150, **characterized in that** the compound is

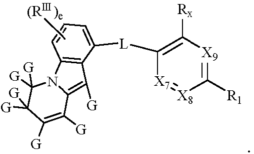

152. A compound represented by formula (I-B-15), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 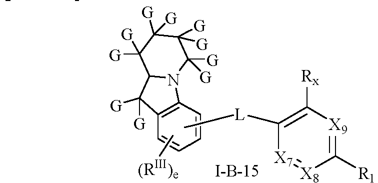 **characterized in that** L, R_{X}, R₁, X₇, X₈, and X₉ are as defined in any one of claims 1-26, and R^{III}, G, and e are as defined in any one of claims 27-30.

153. The compound represented by formula (I-B-15), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 152, **characterized in that** the compound is

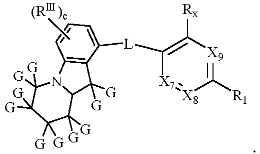

154. A compound represented by formula (I-B-16), or a stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof, 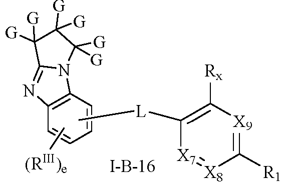 **characterized in that** L, R_{X}, R₁, X₇, X₈, and X₉ are as defined in any one of claims 1-26, and G, R^{III}, and e are as defined in any one of claims 27-30.

155. The compound represented by formula (I-B-16), or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to claim 154, **characterized in that** the compound is

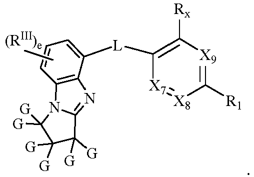

156. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-155, **characterized in that** the compound is selected from the group consisting of:

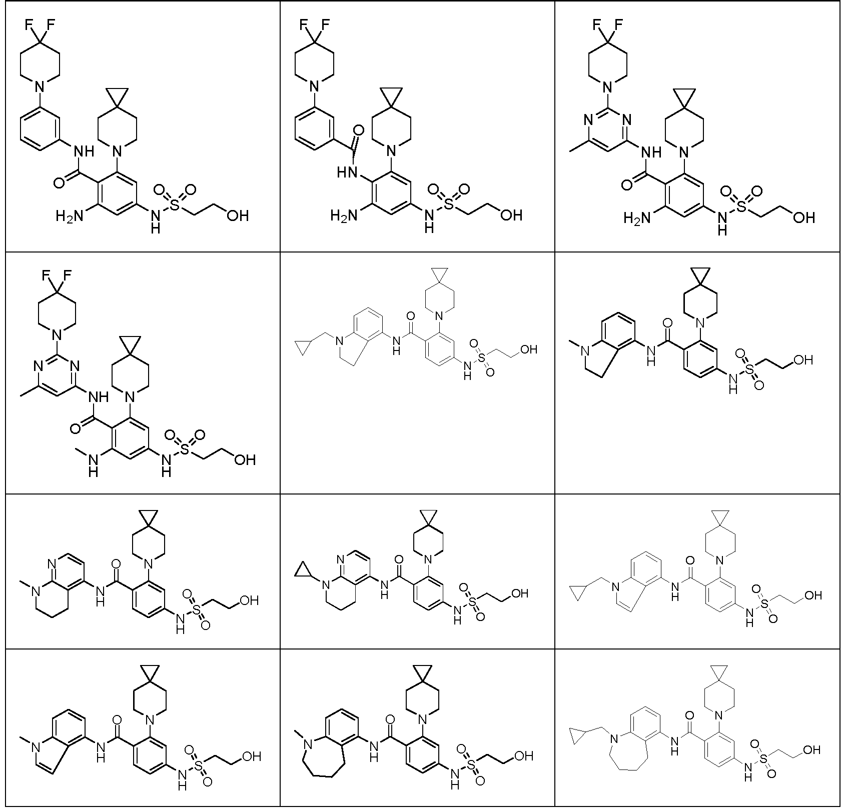

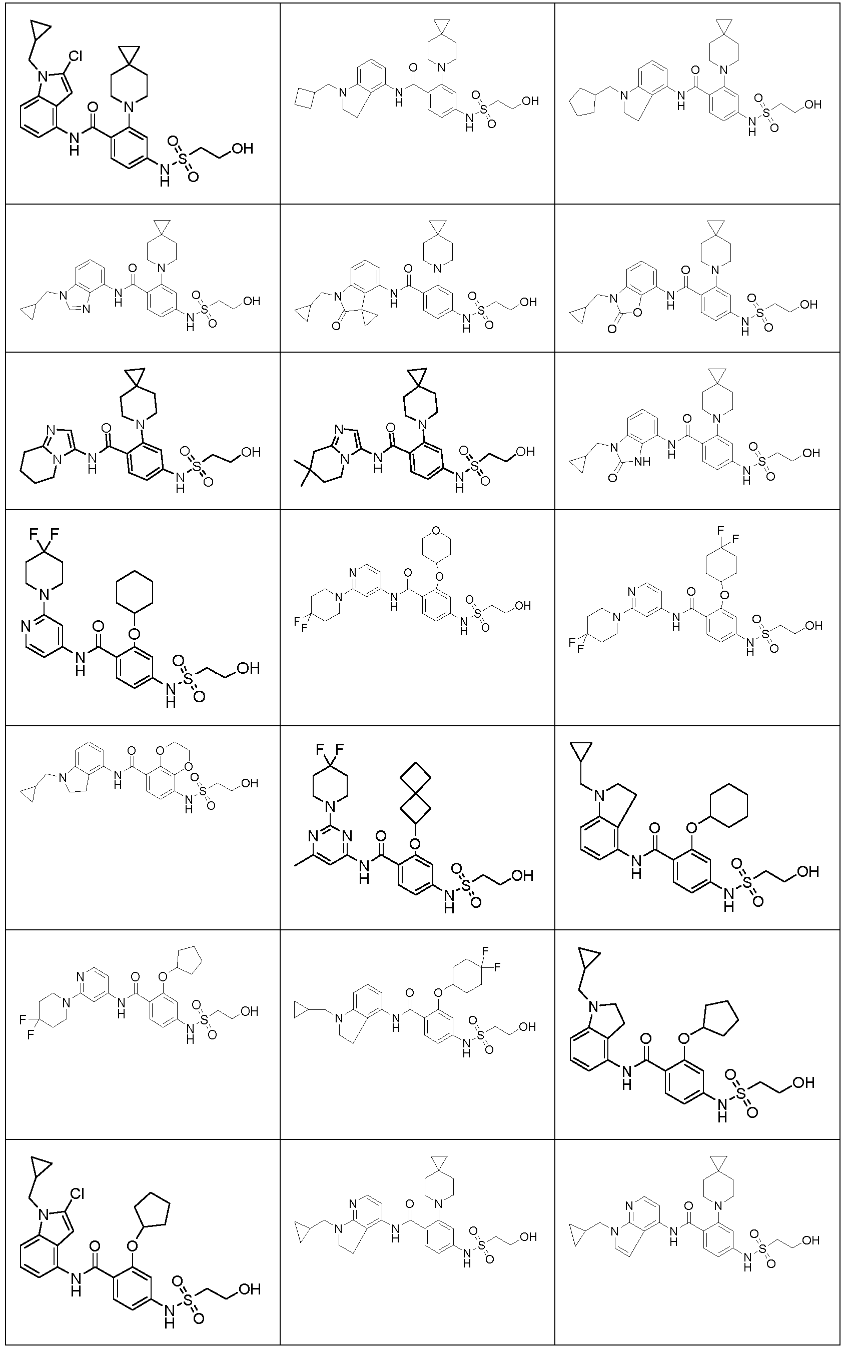

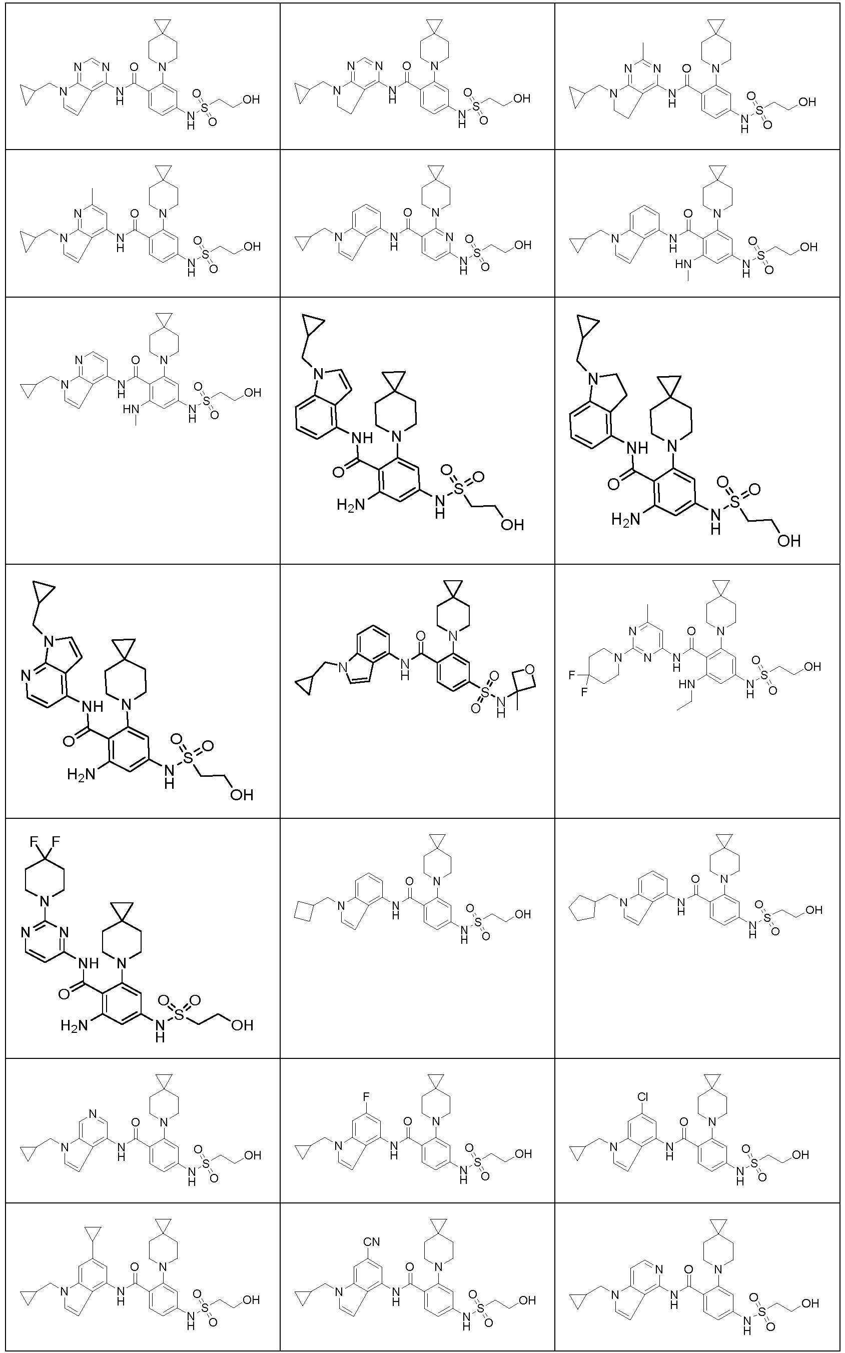

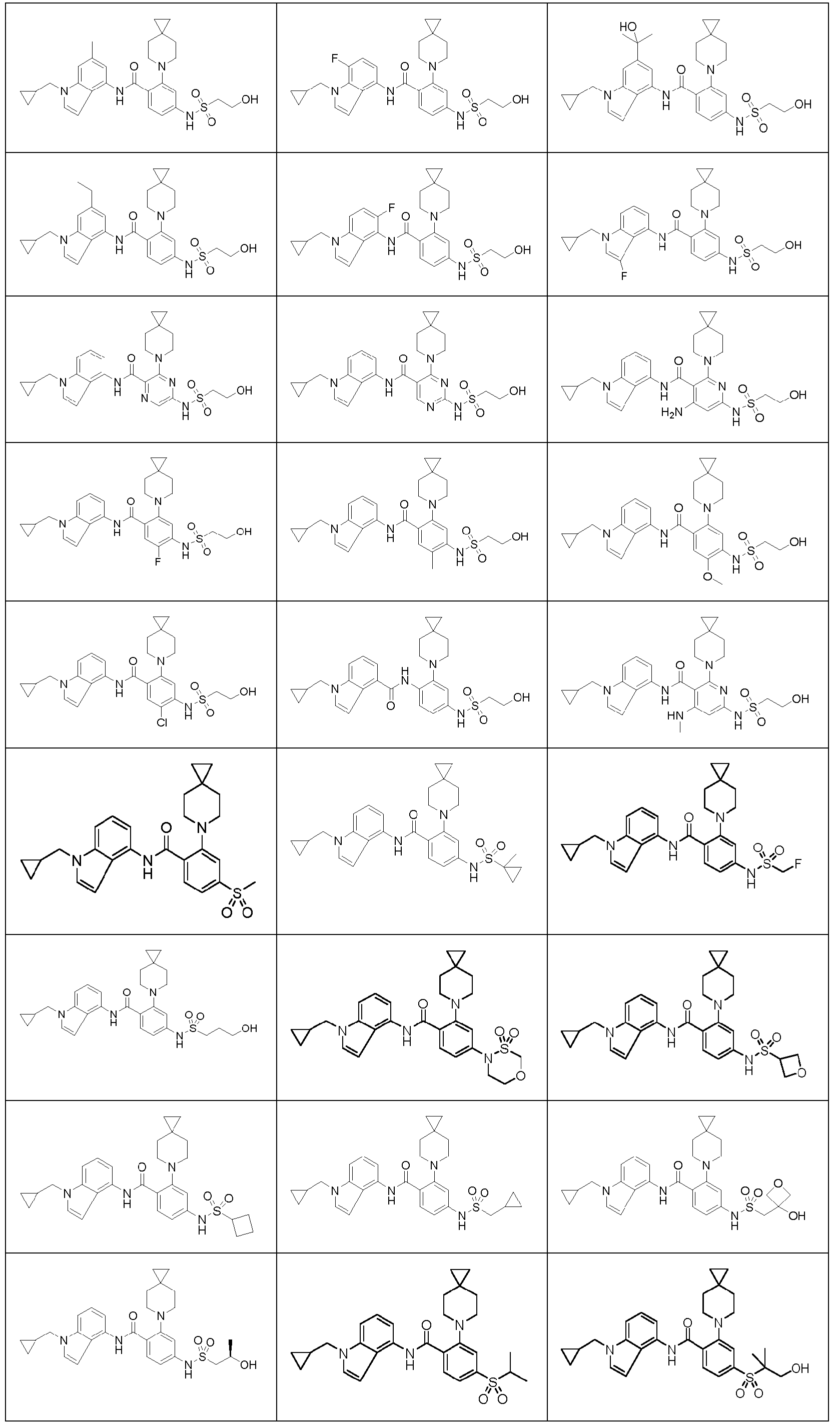

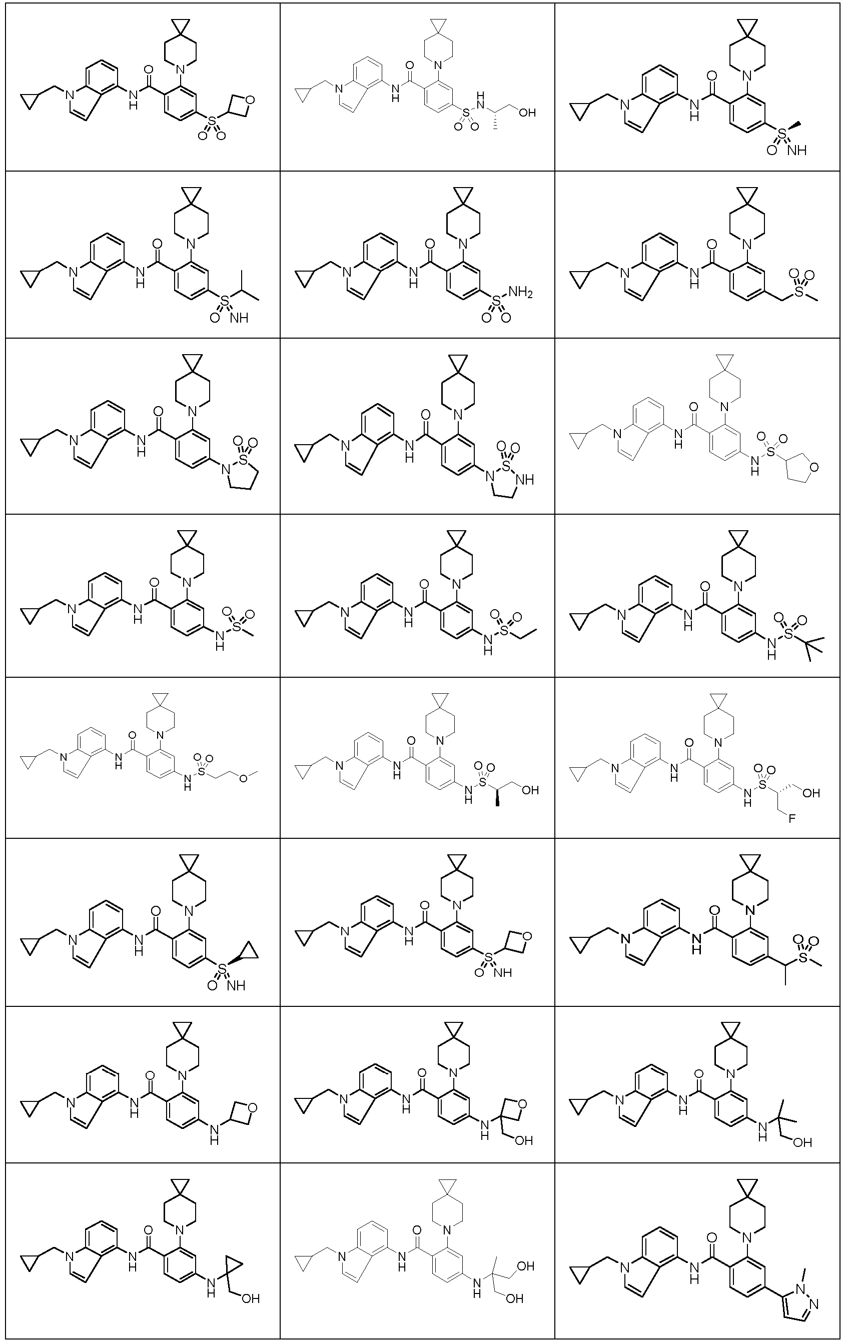

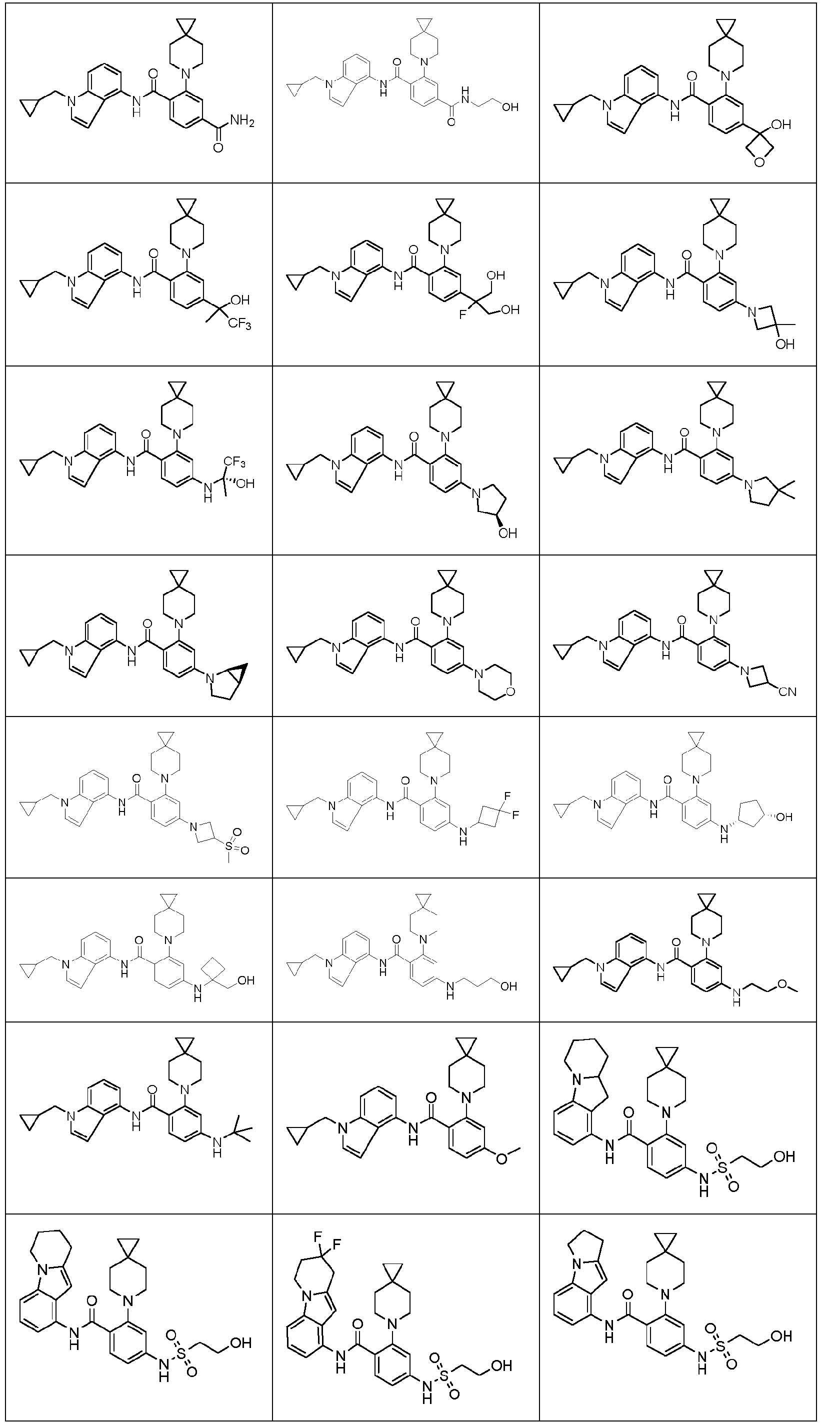

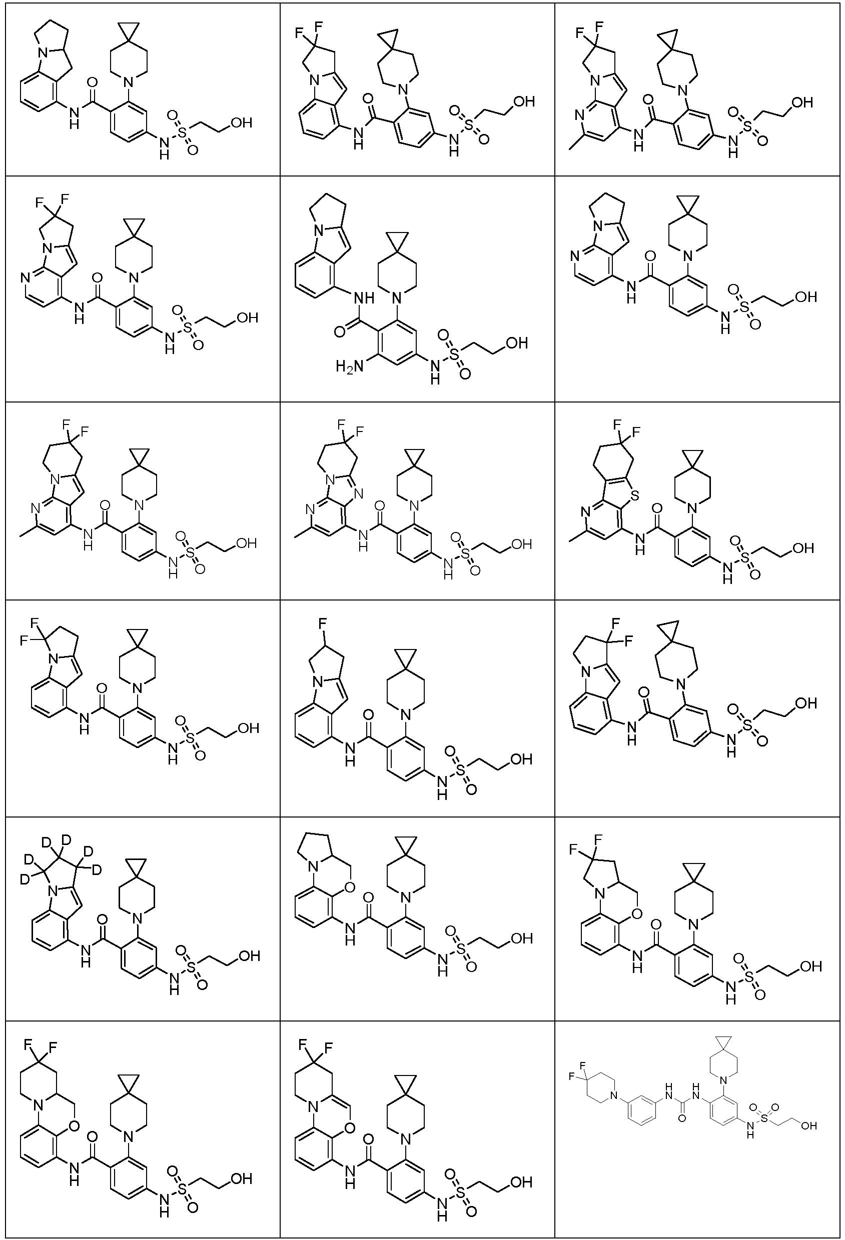

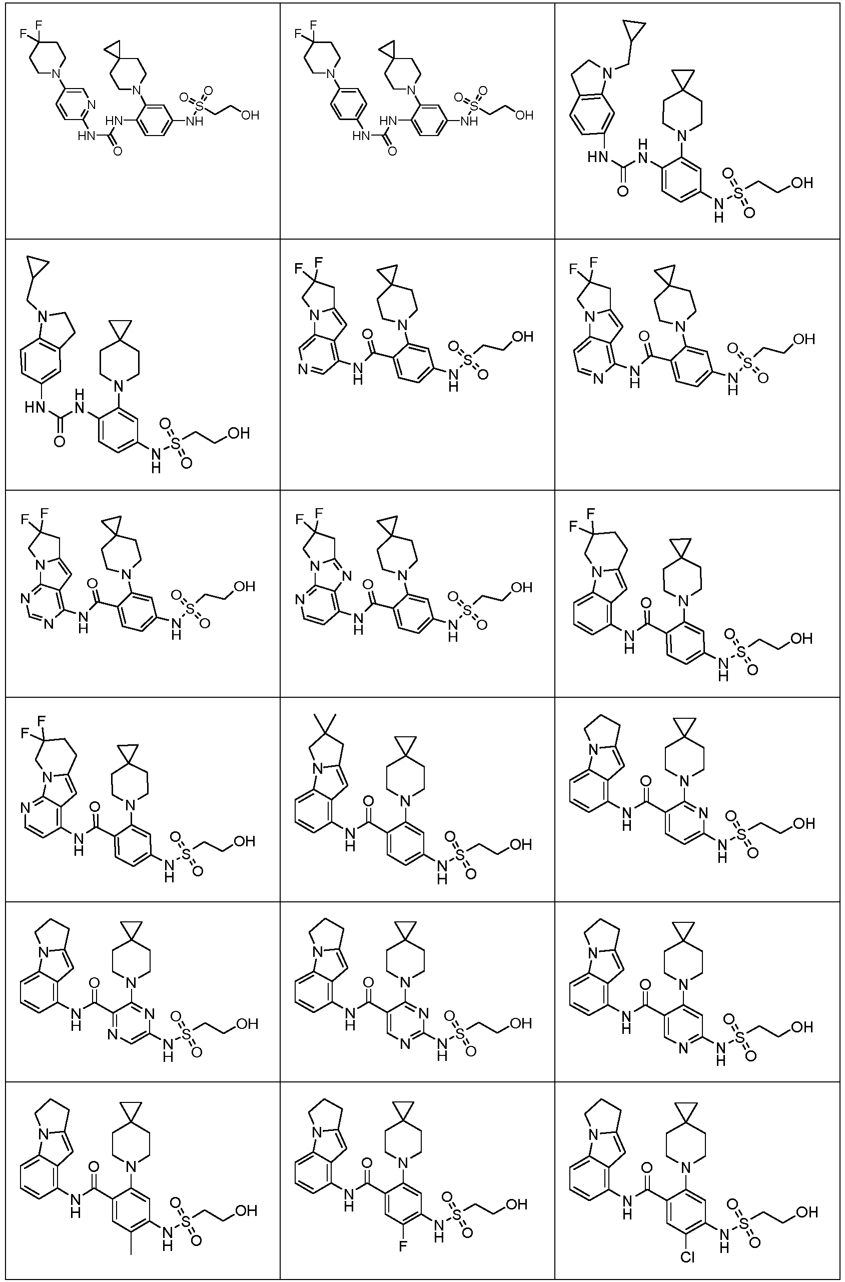

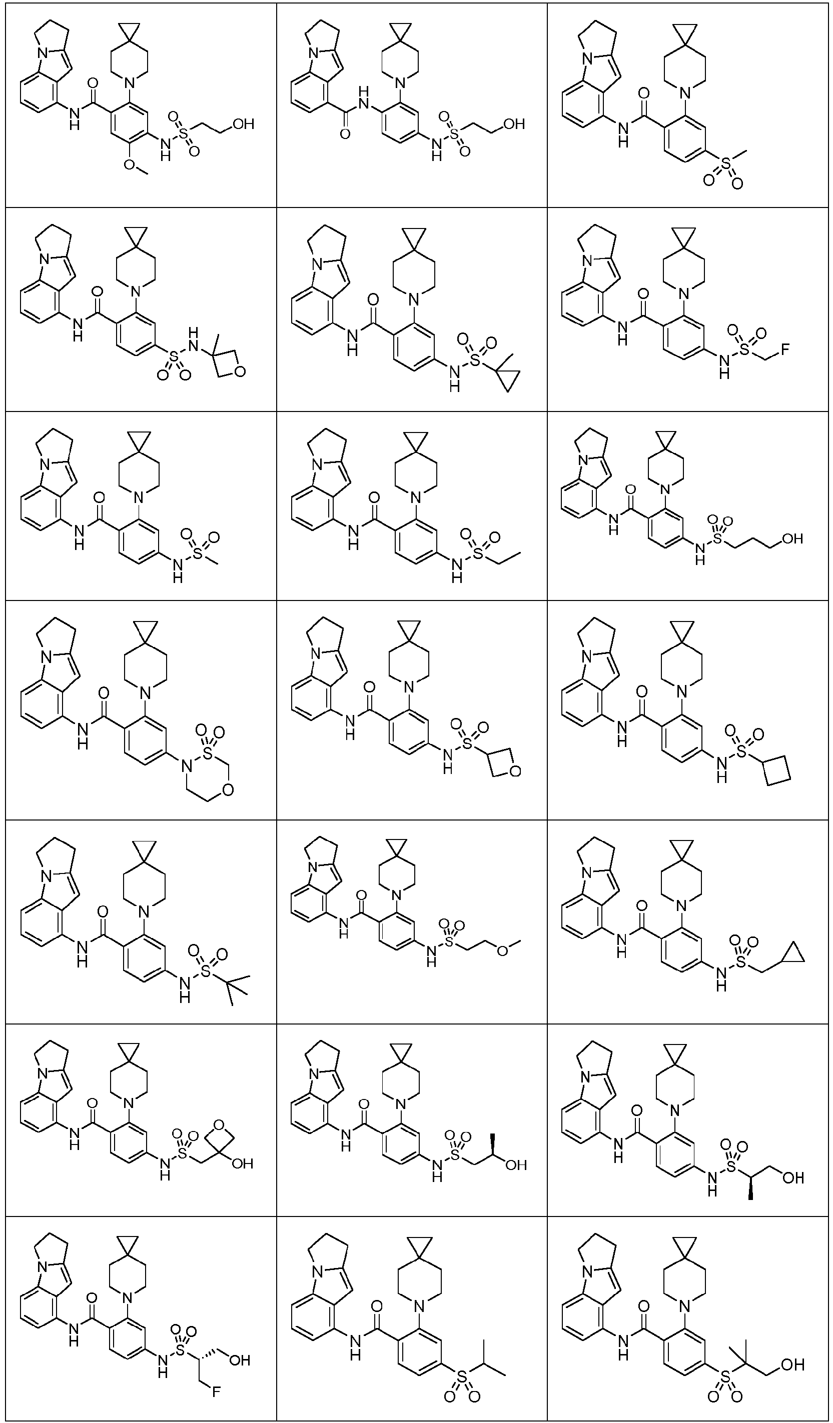

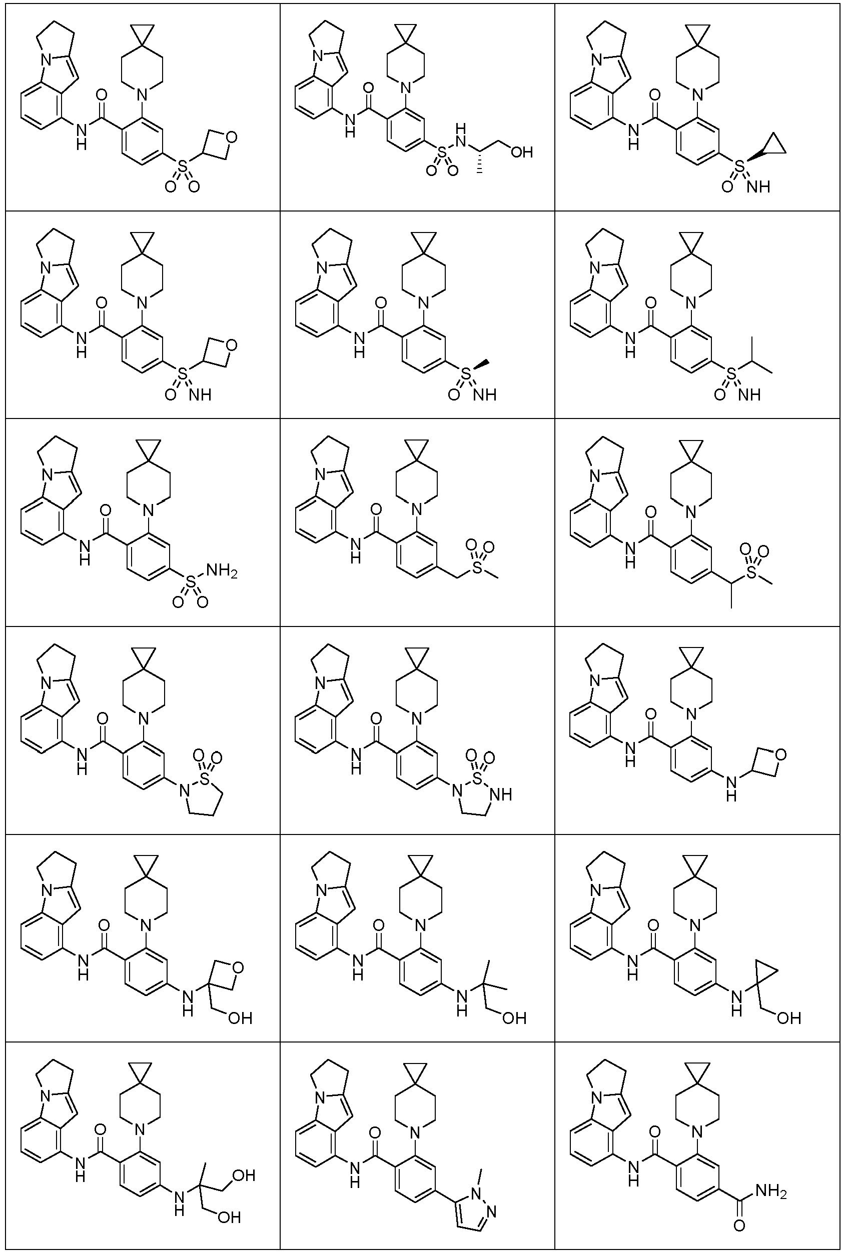

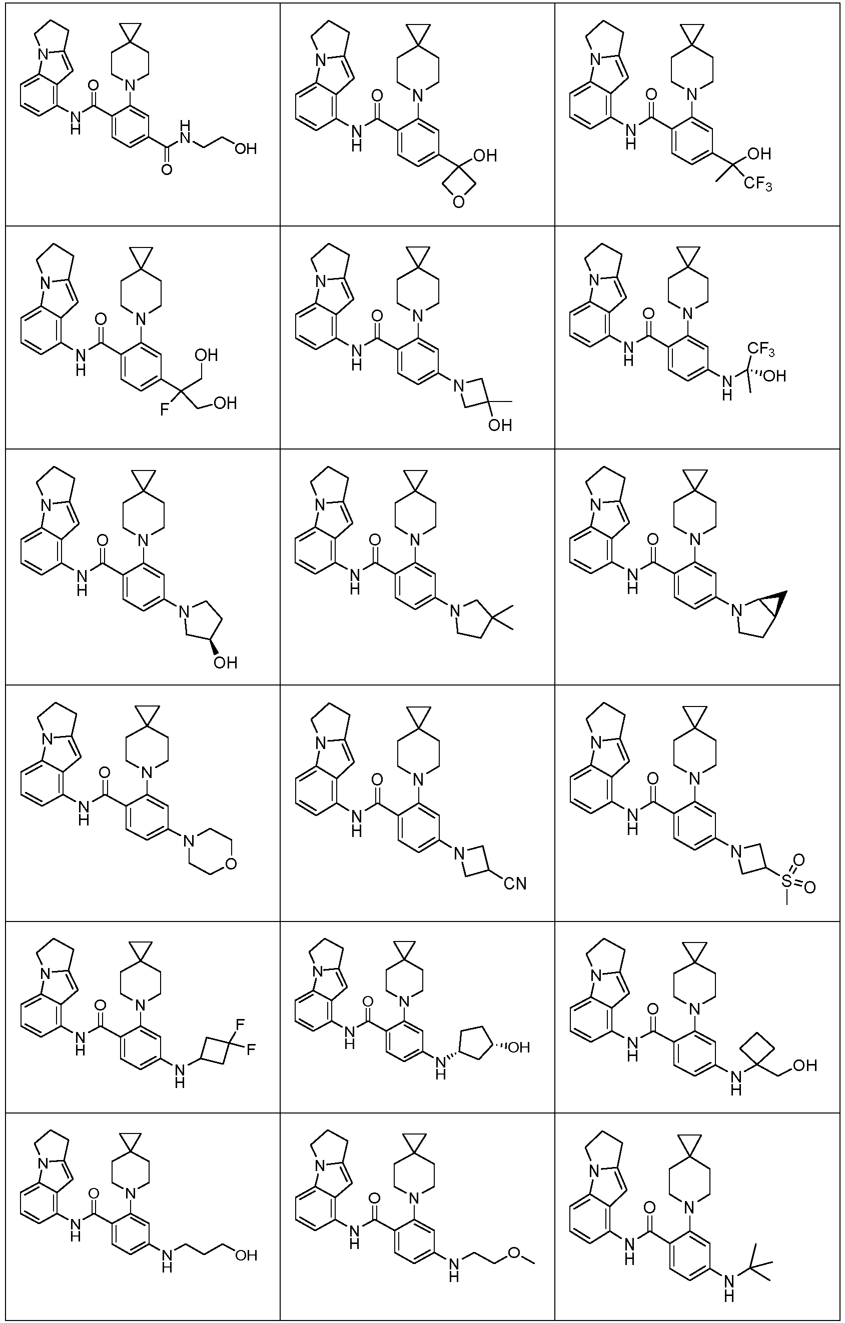

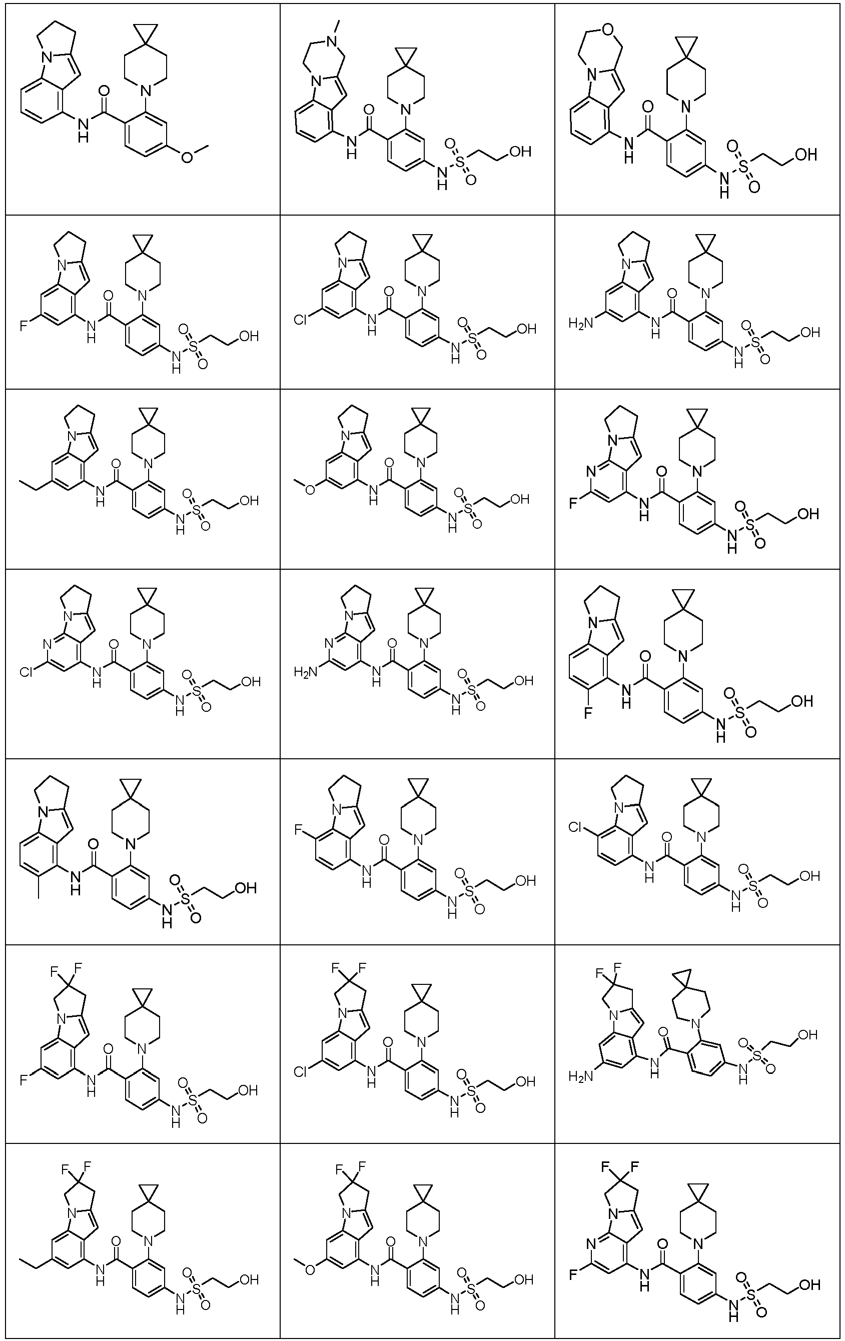

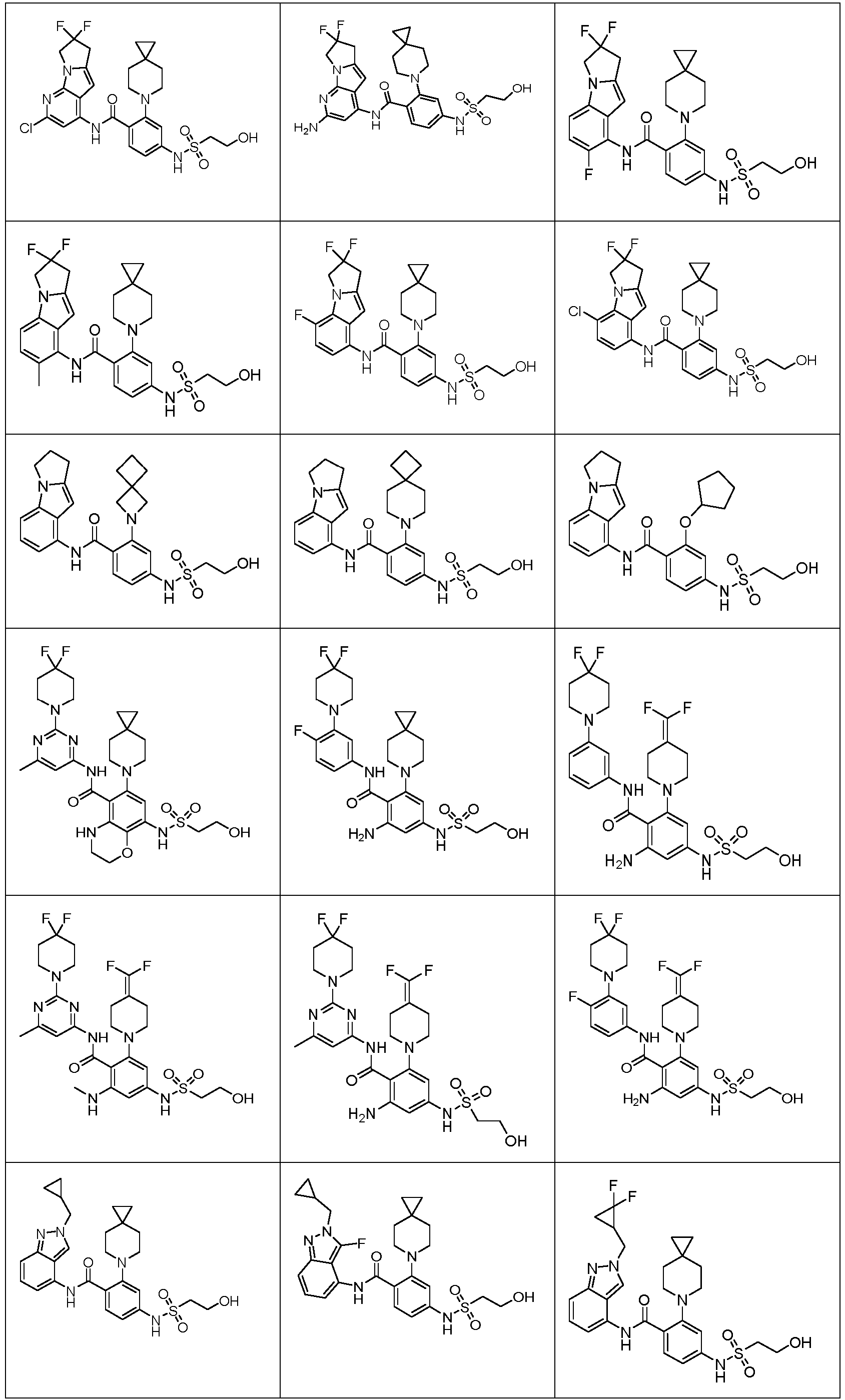

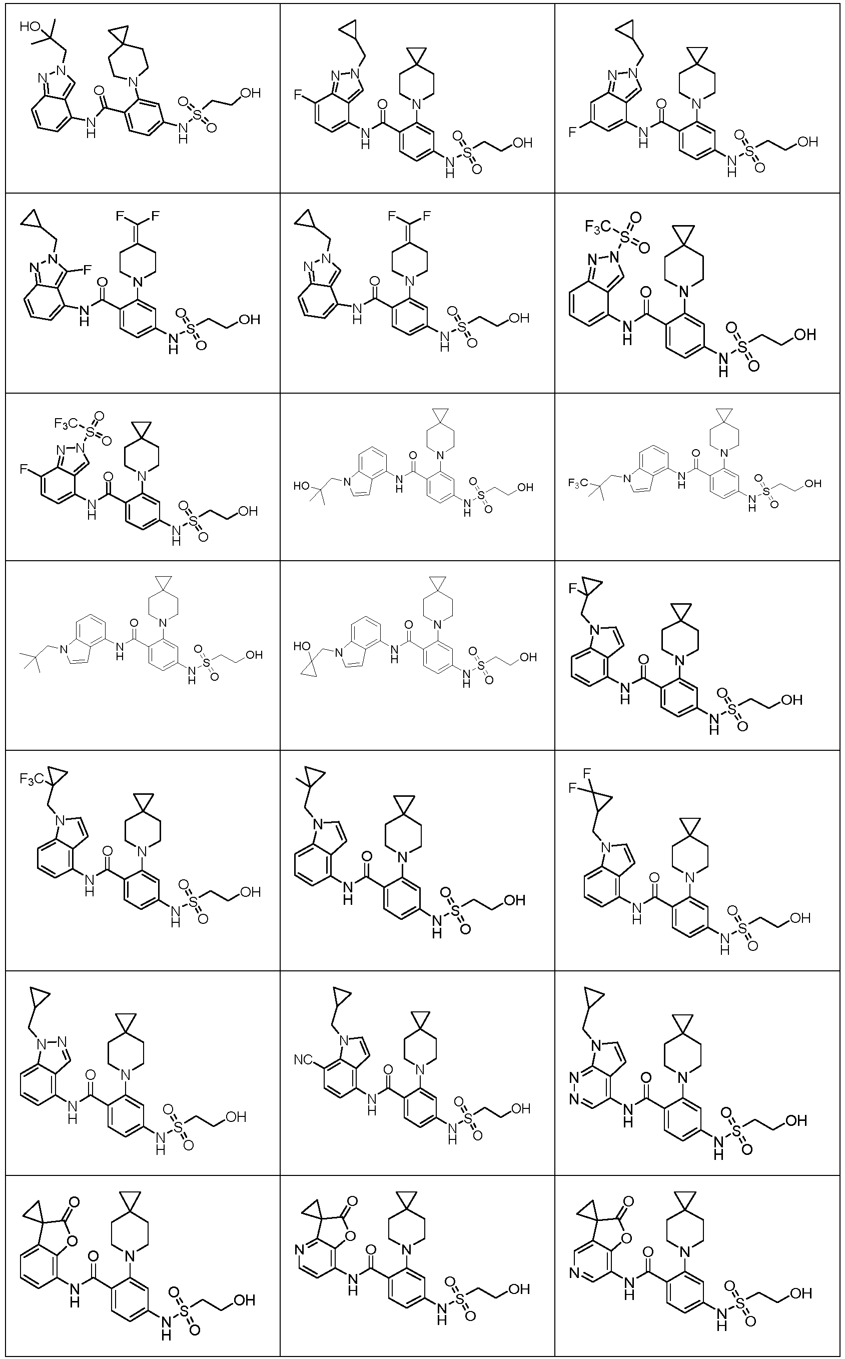

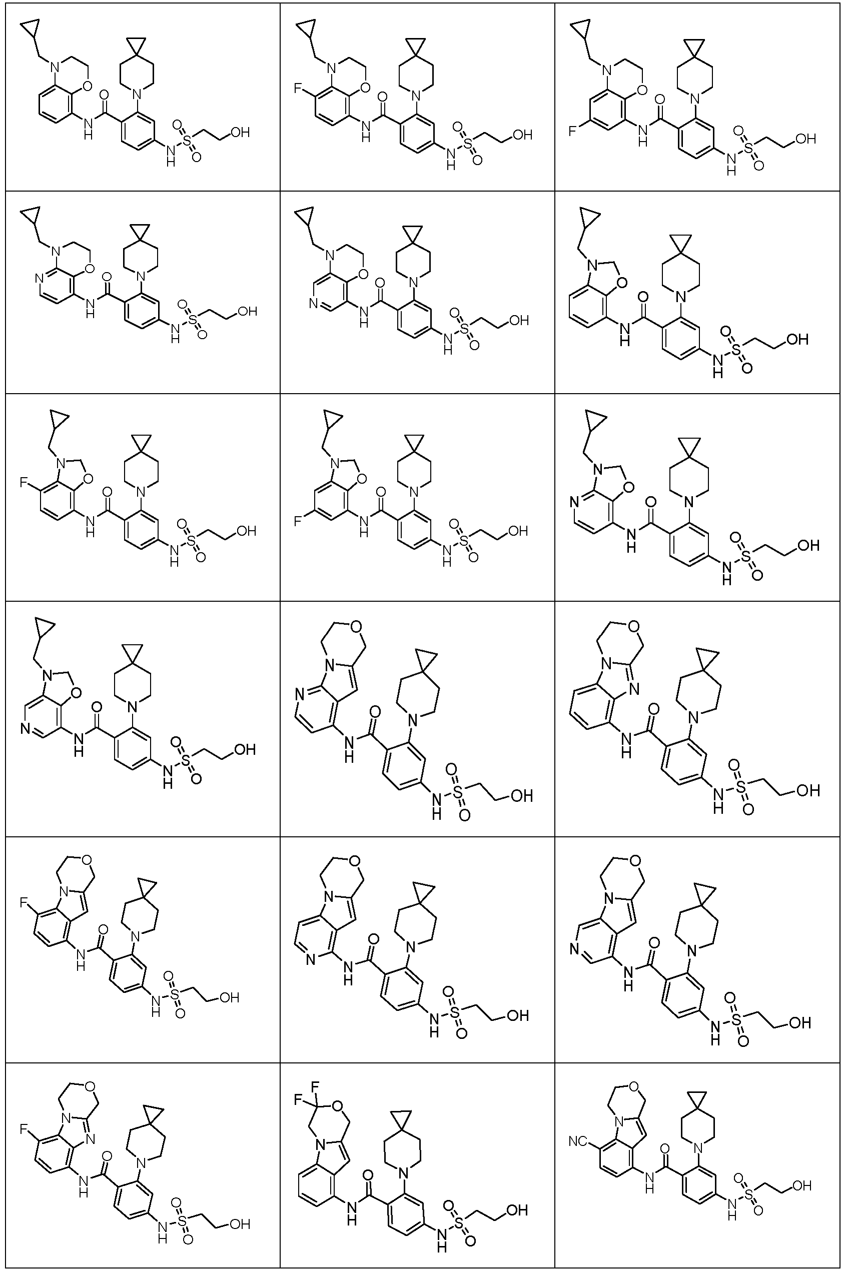

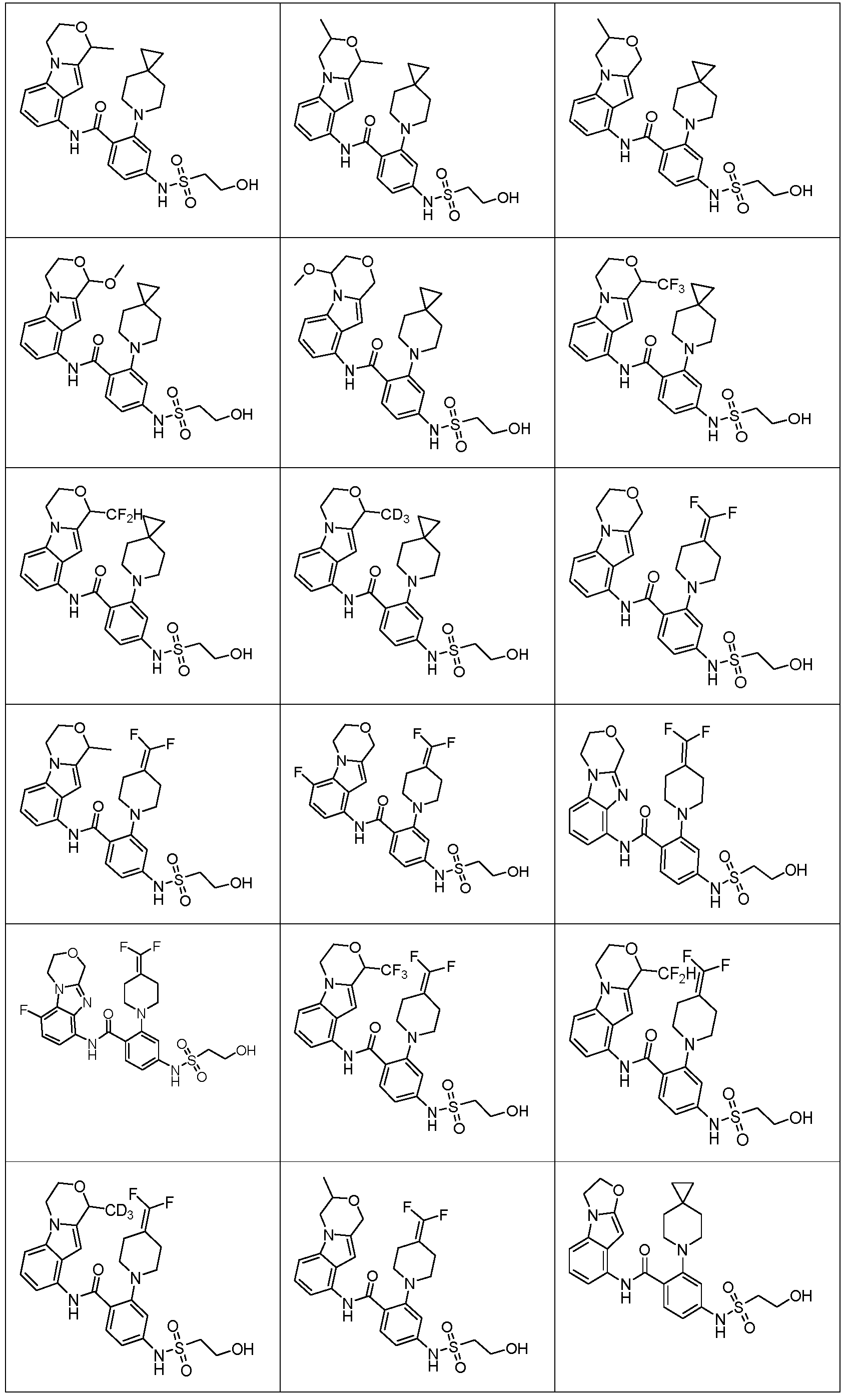

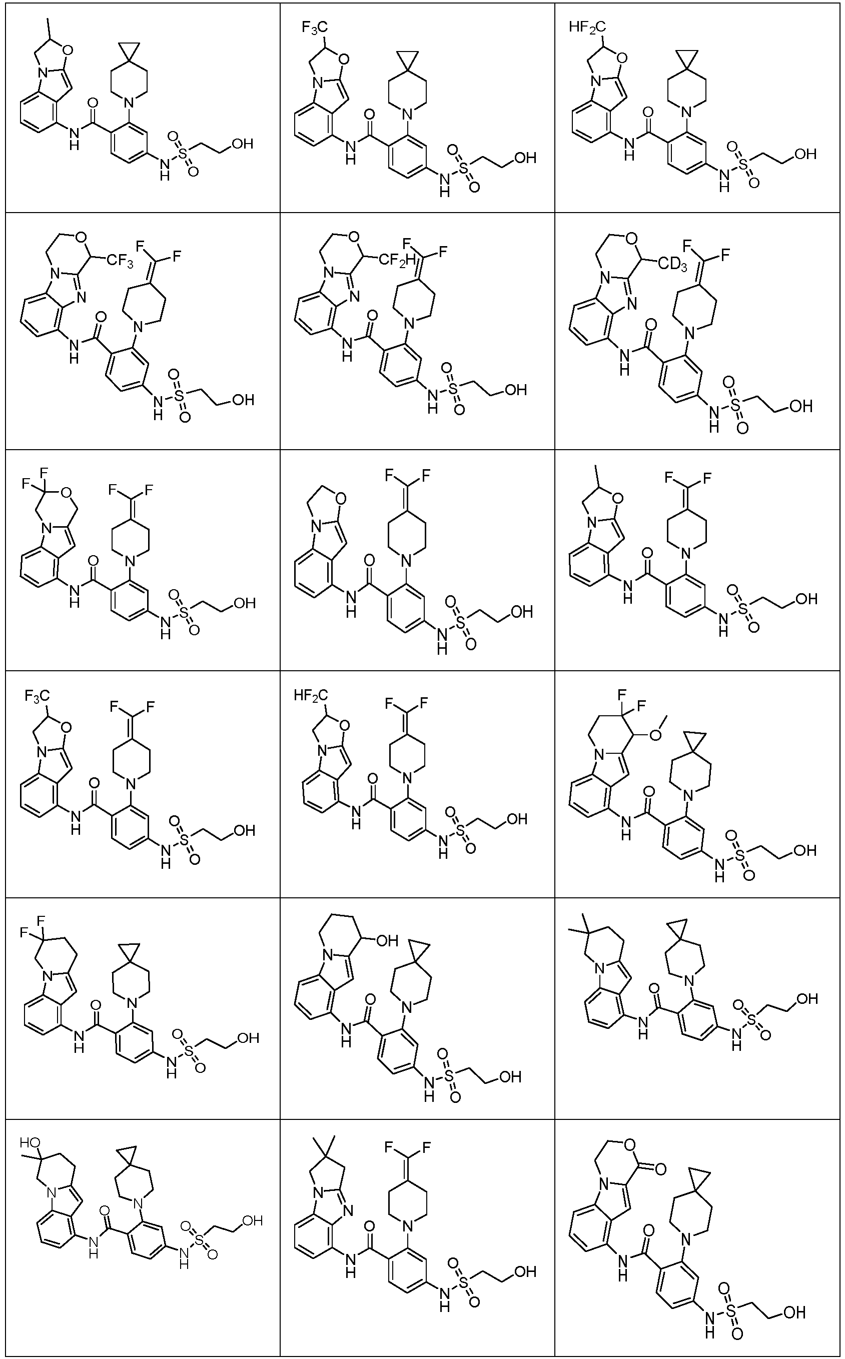

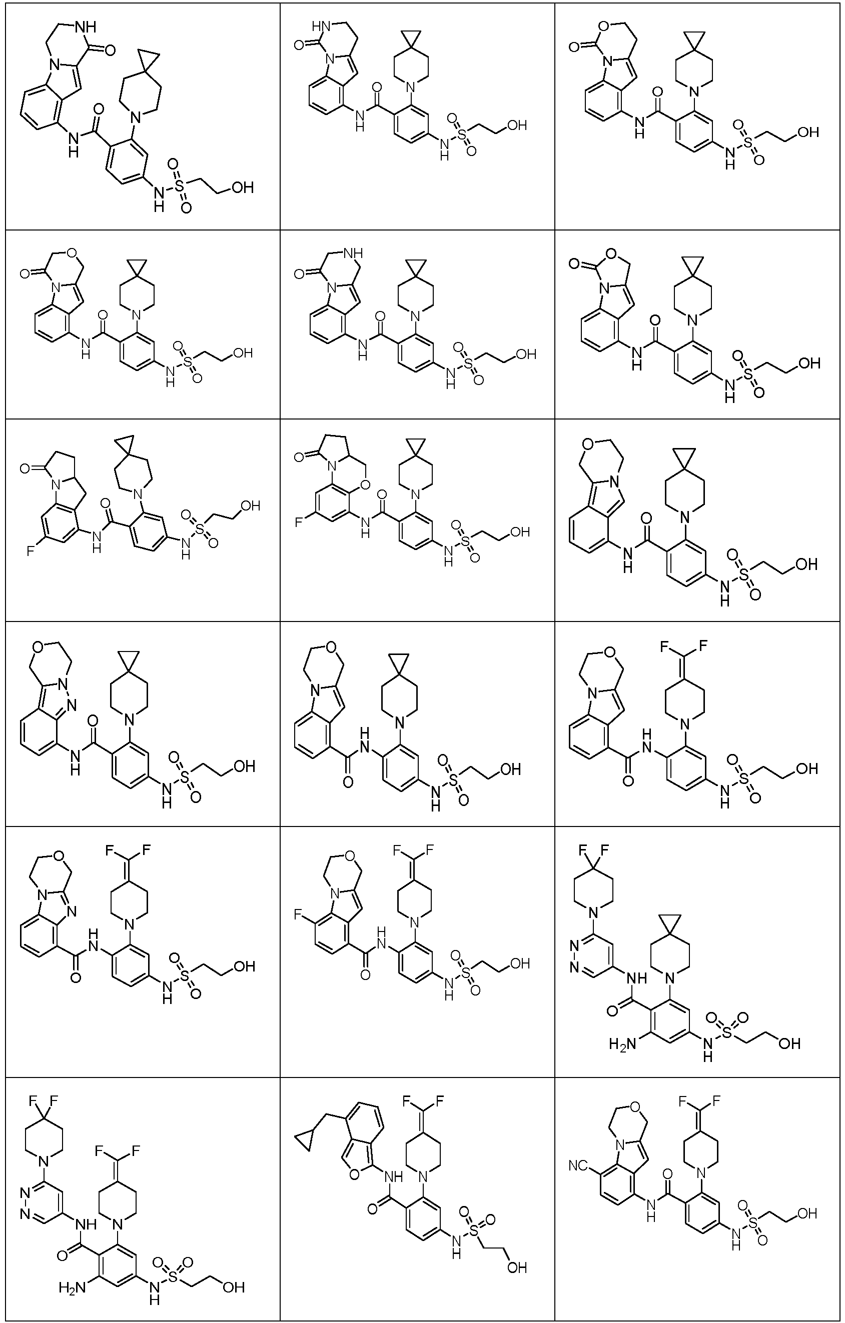

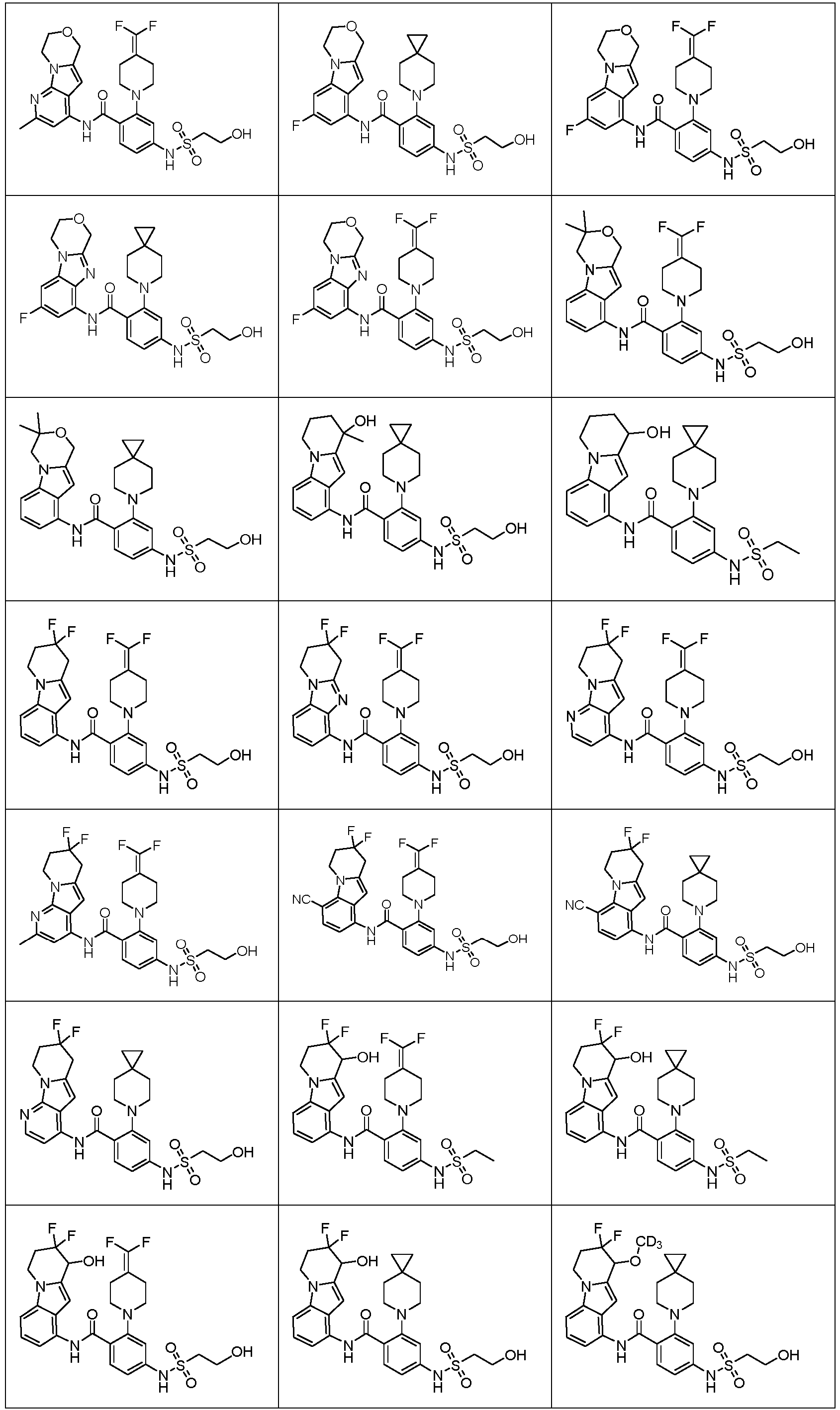

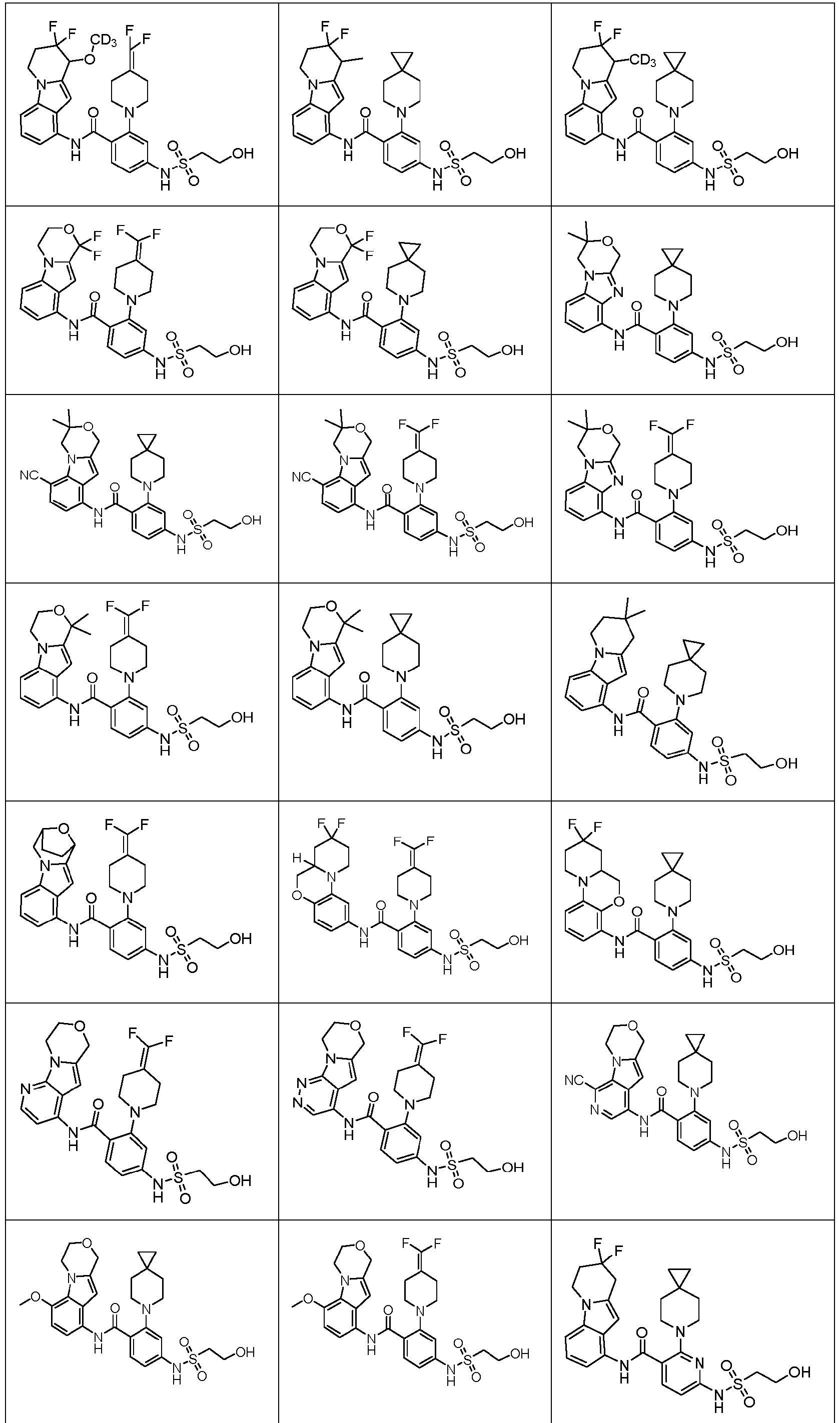

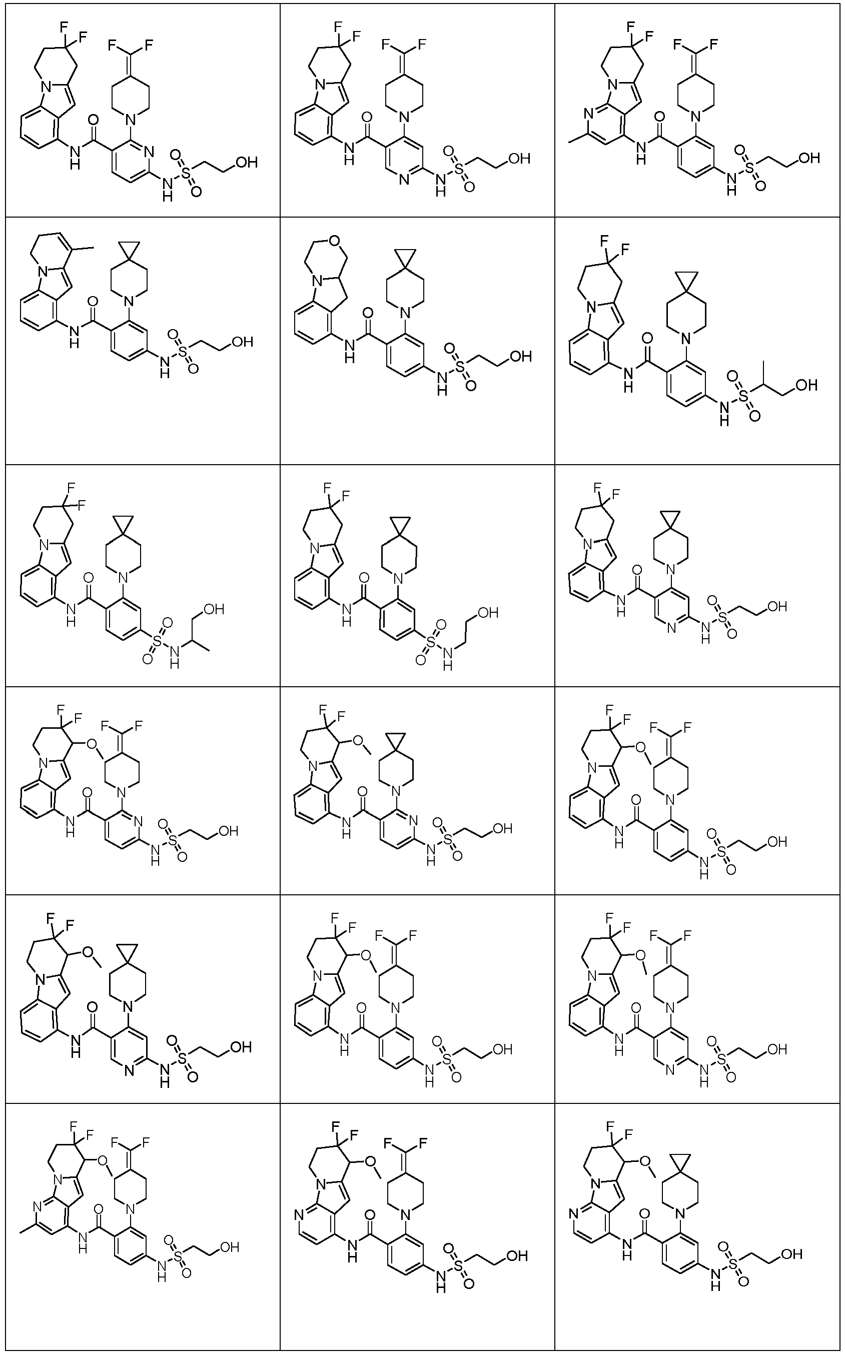

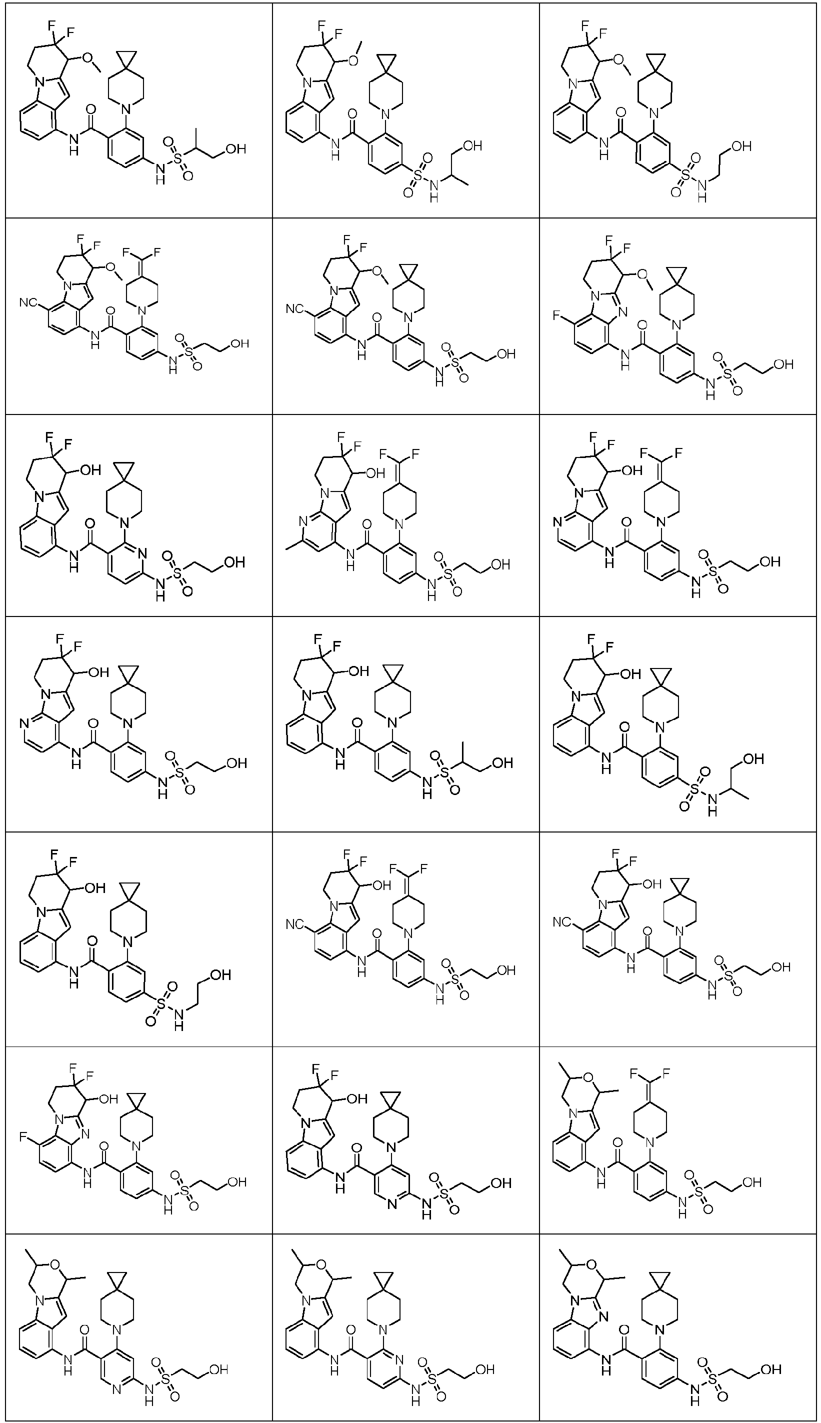

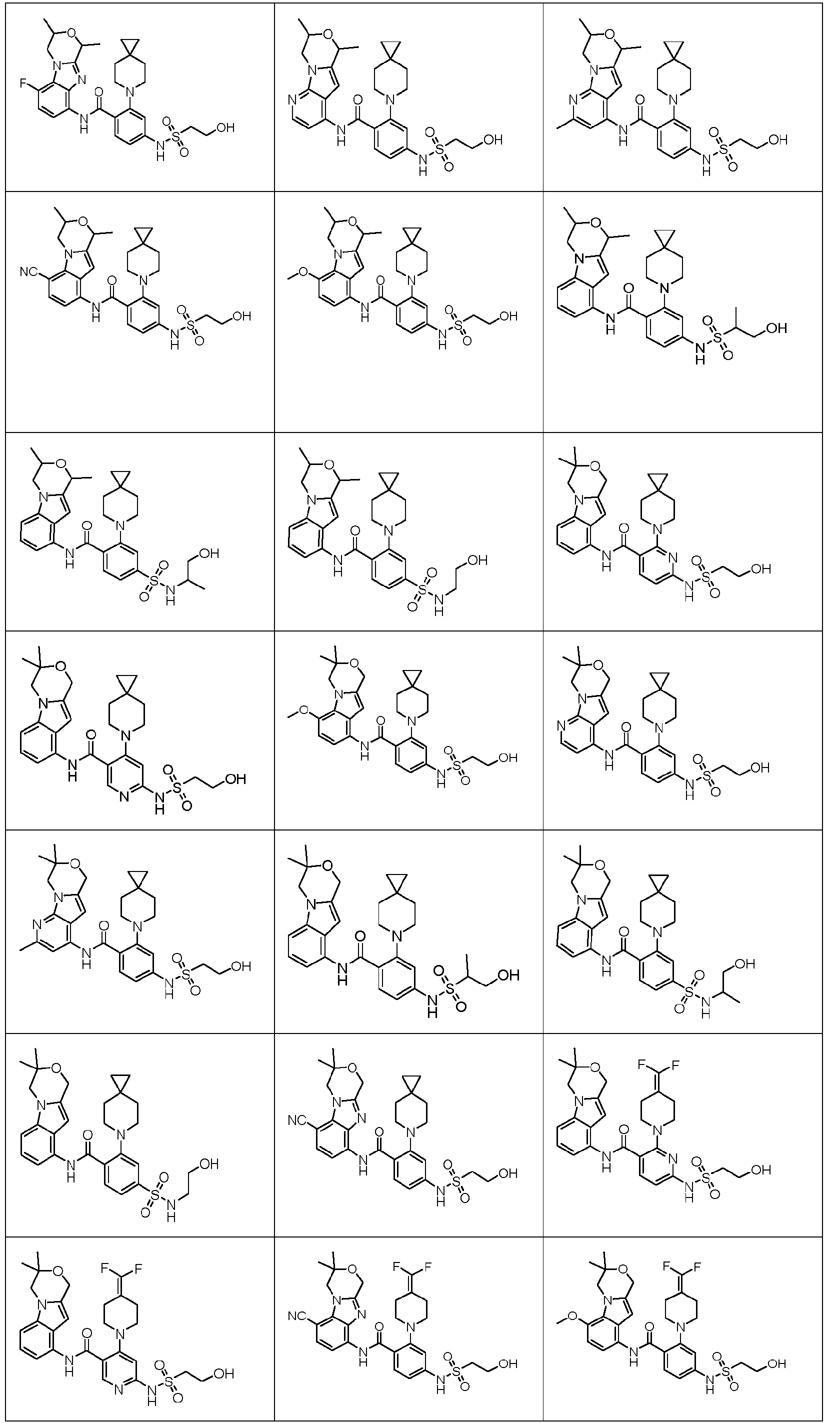

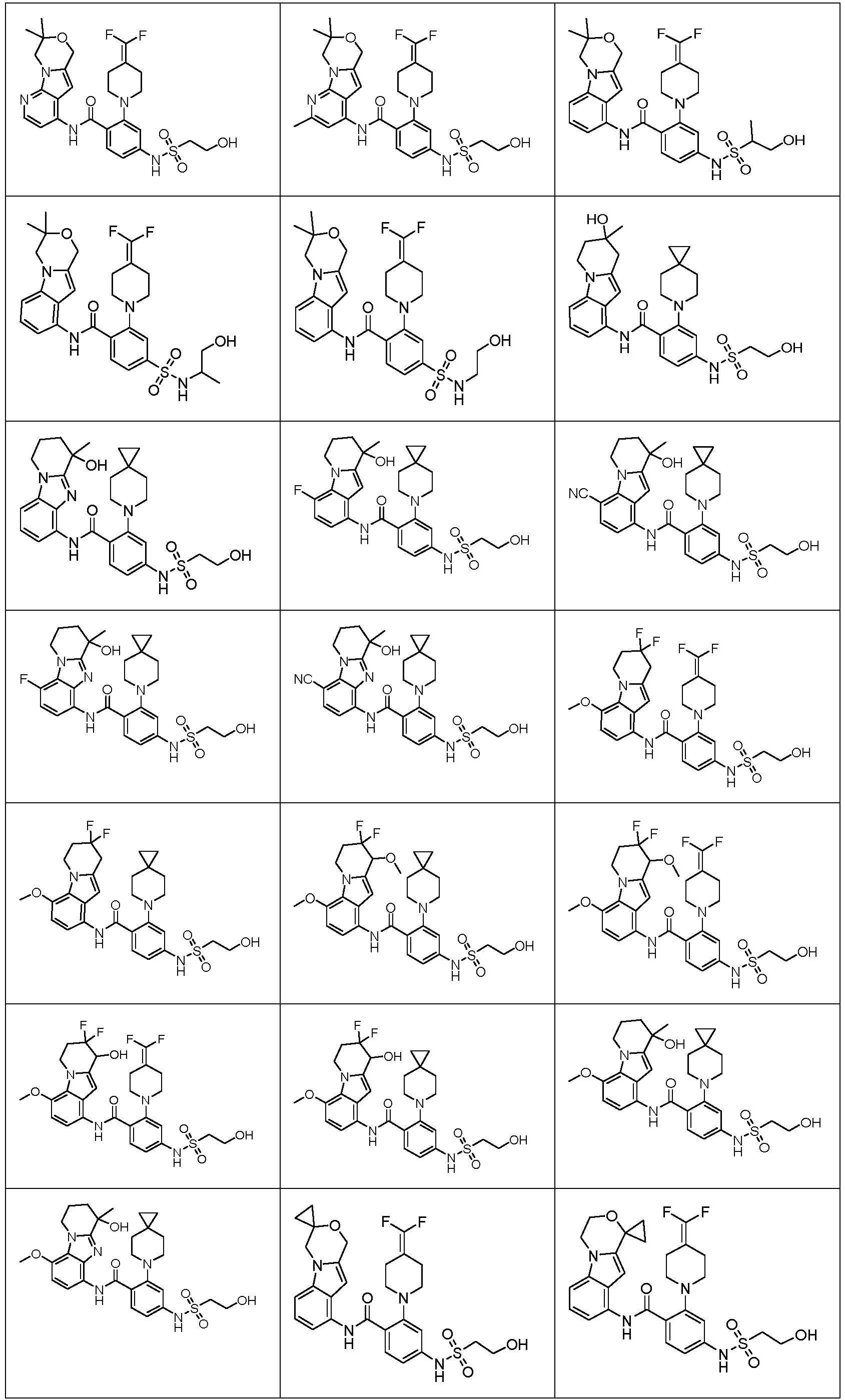

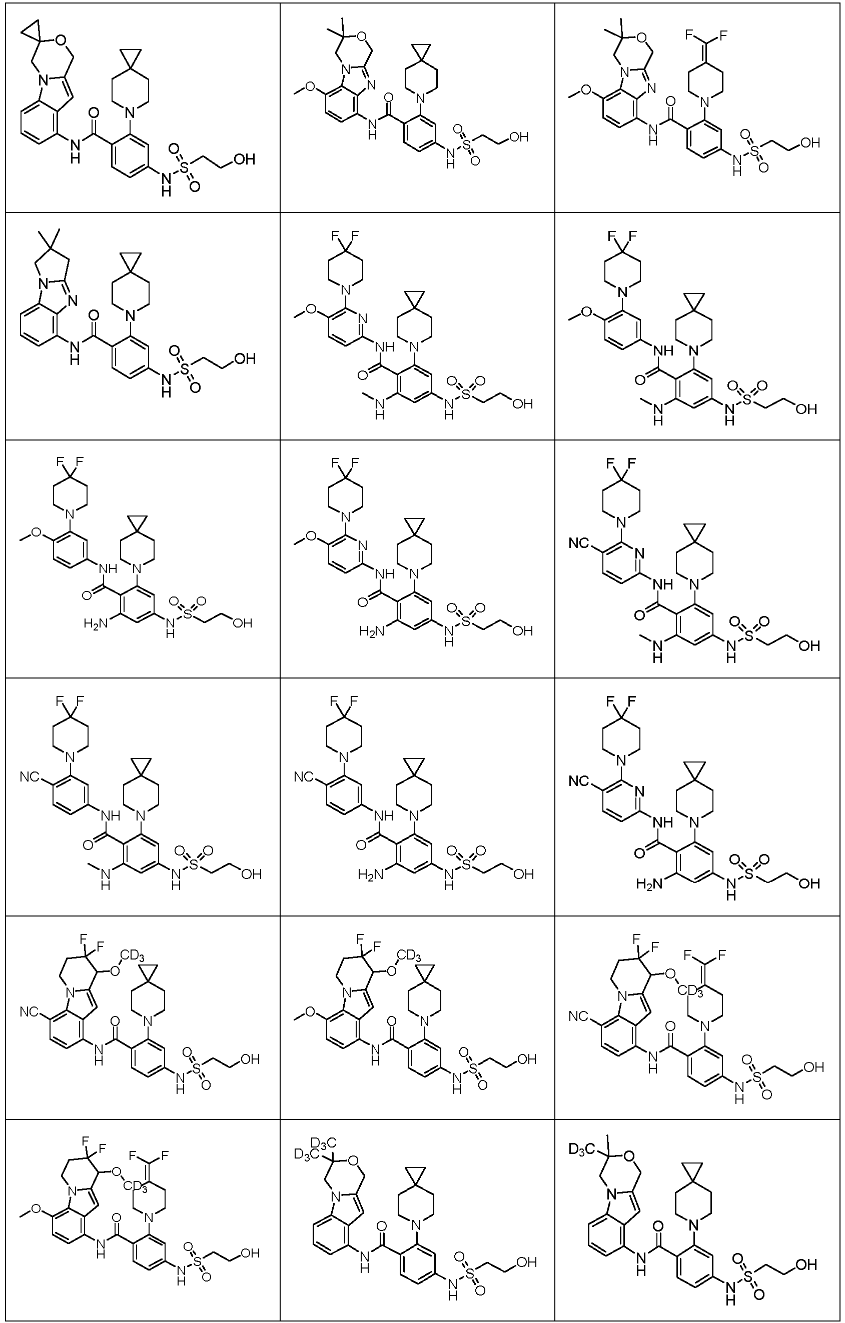

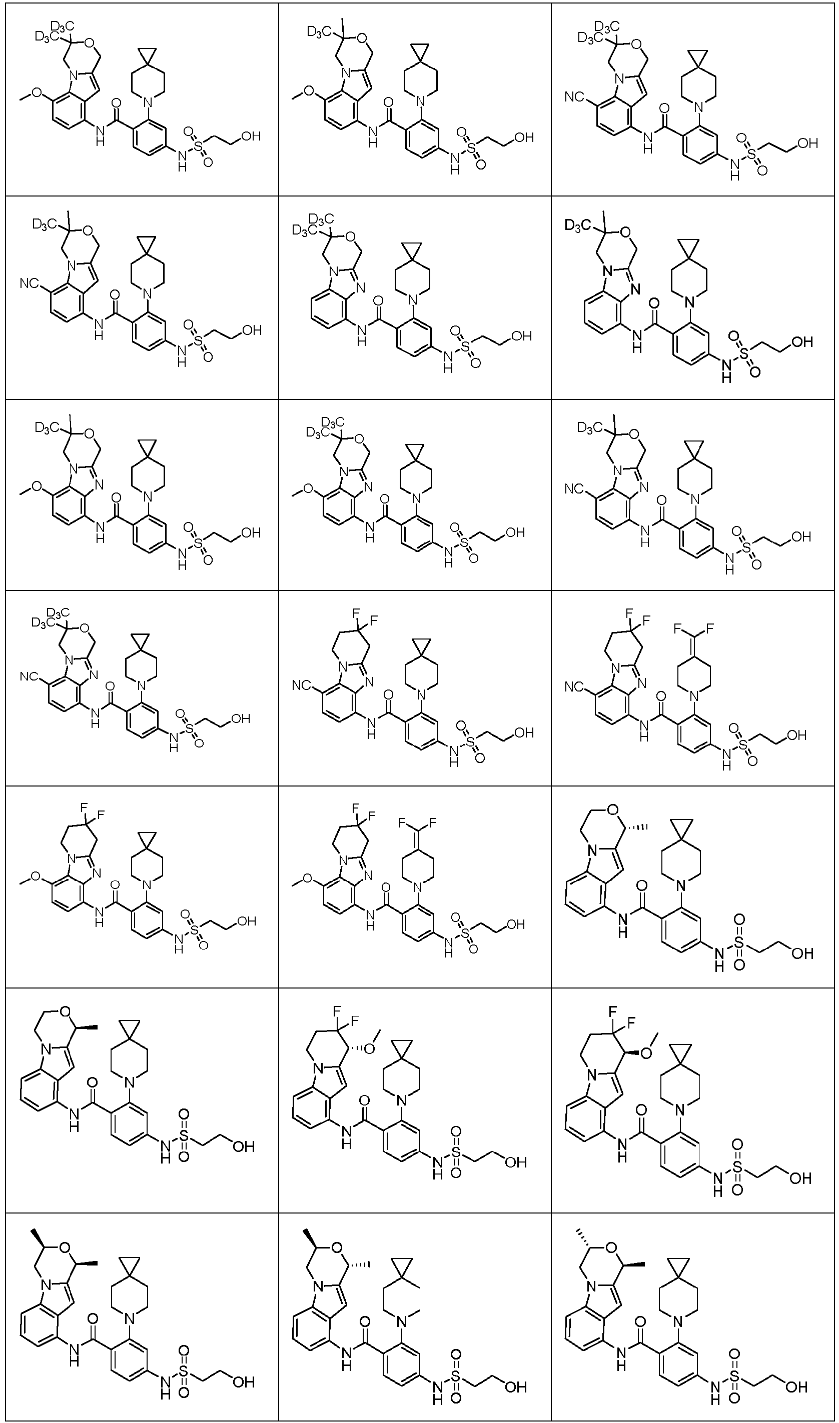

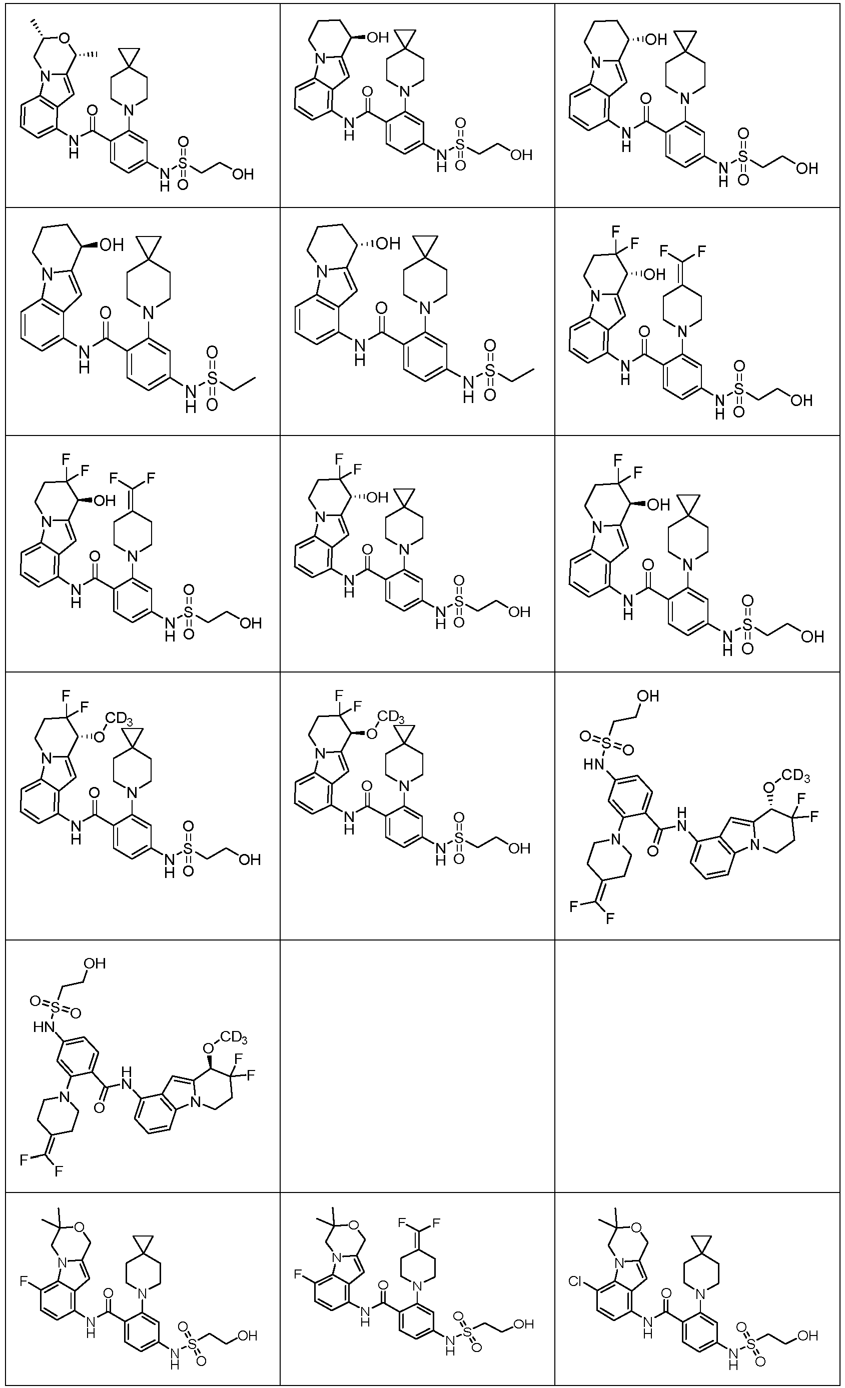

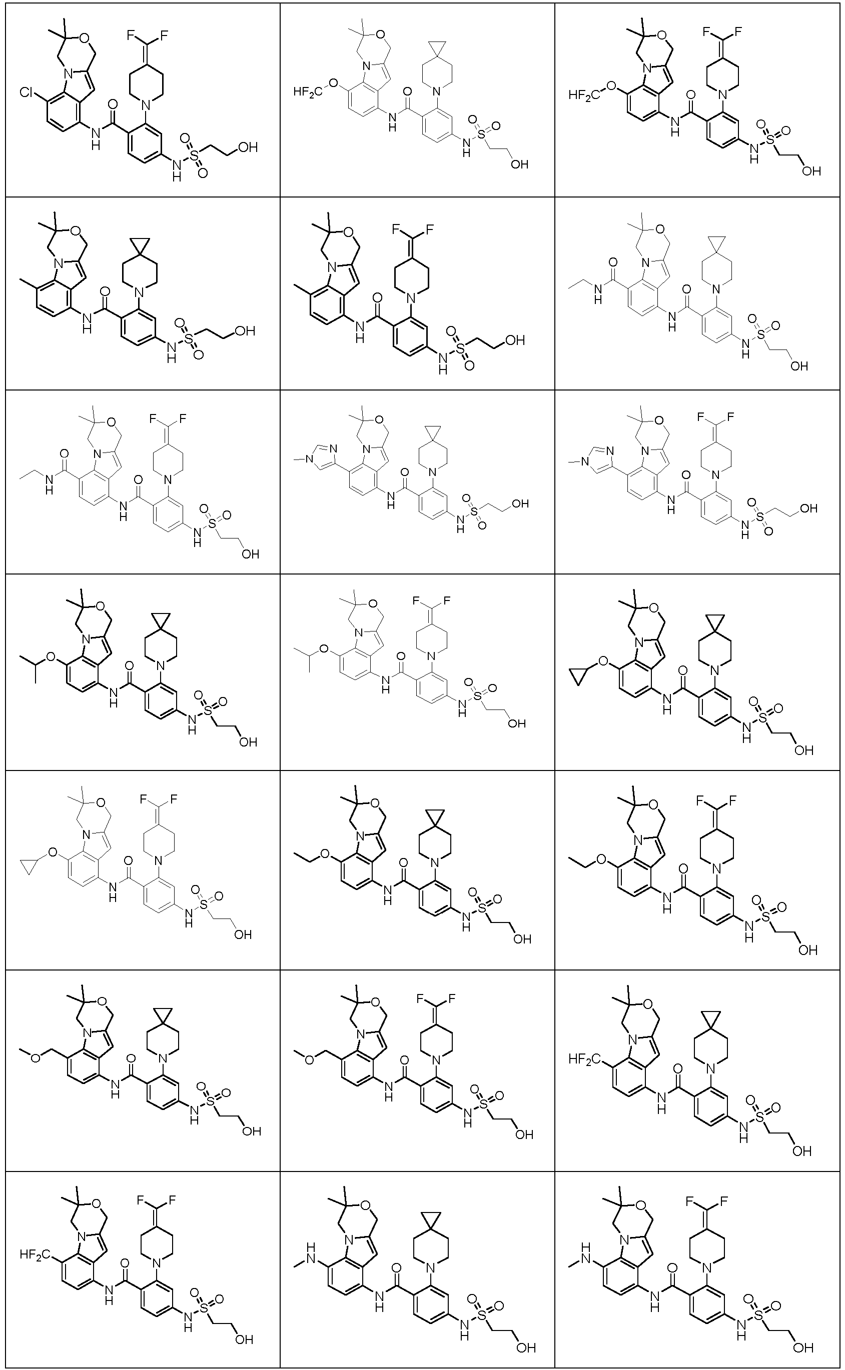

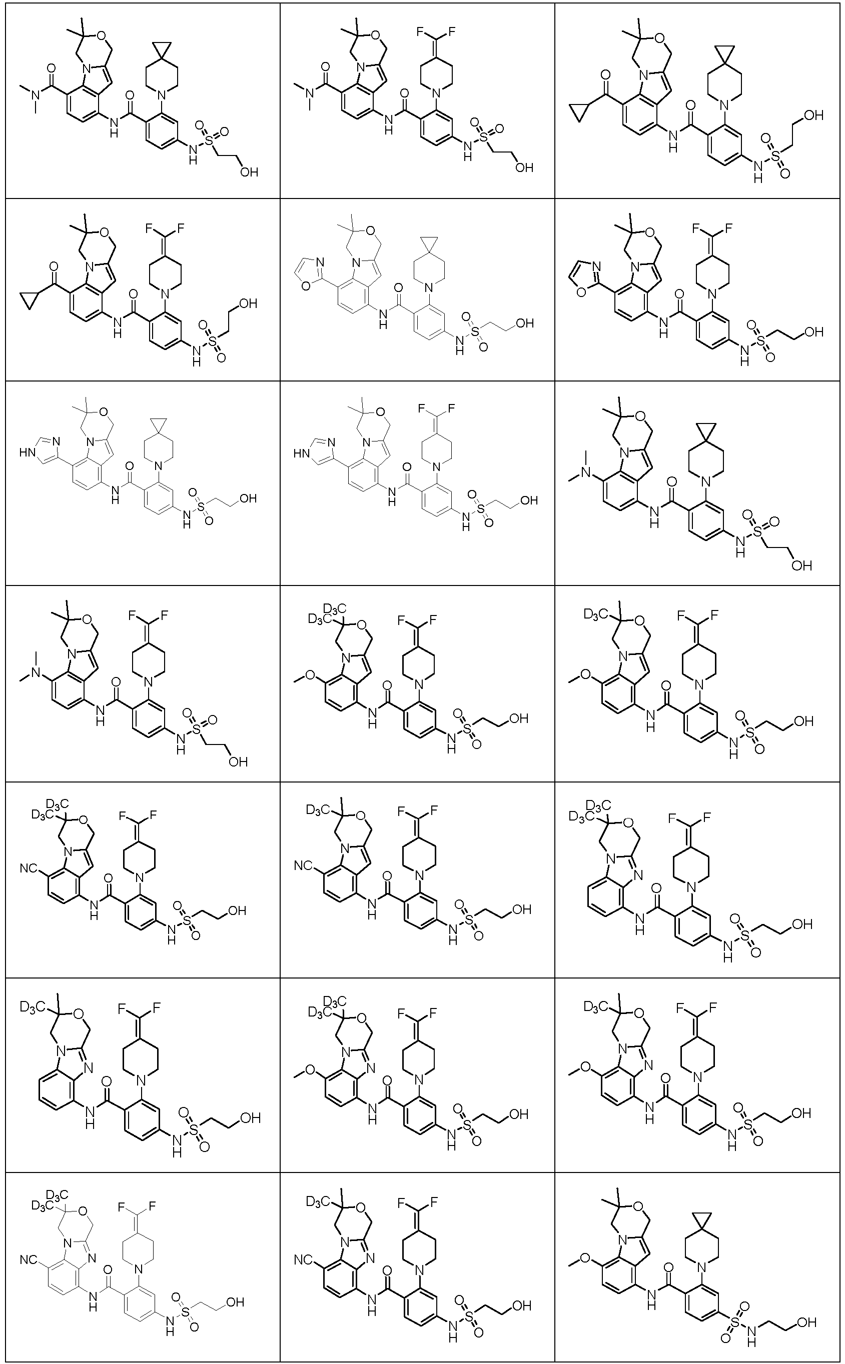

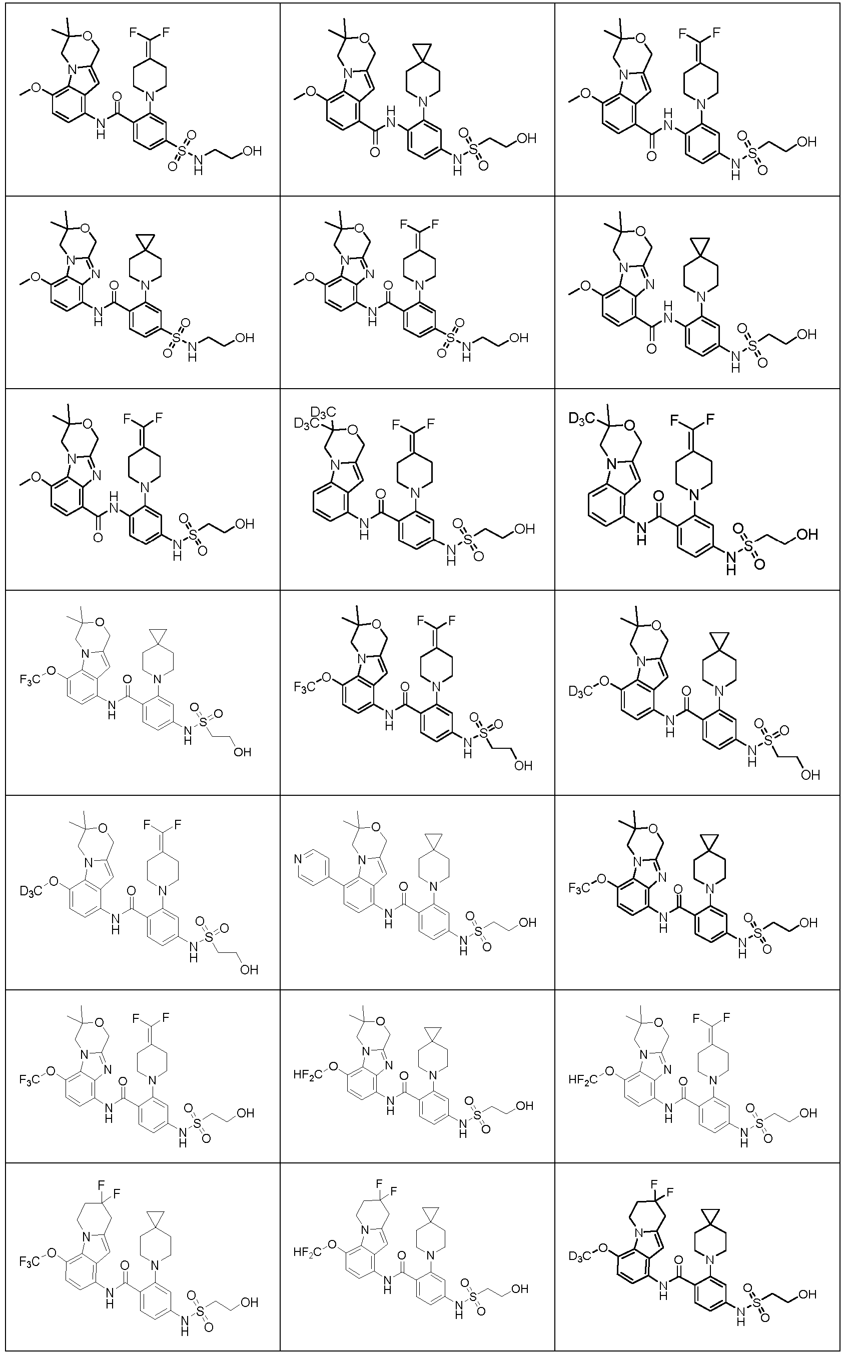

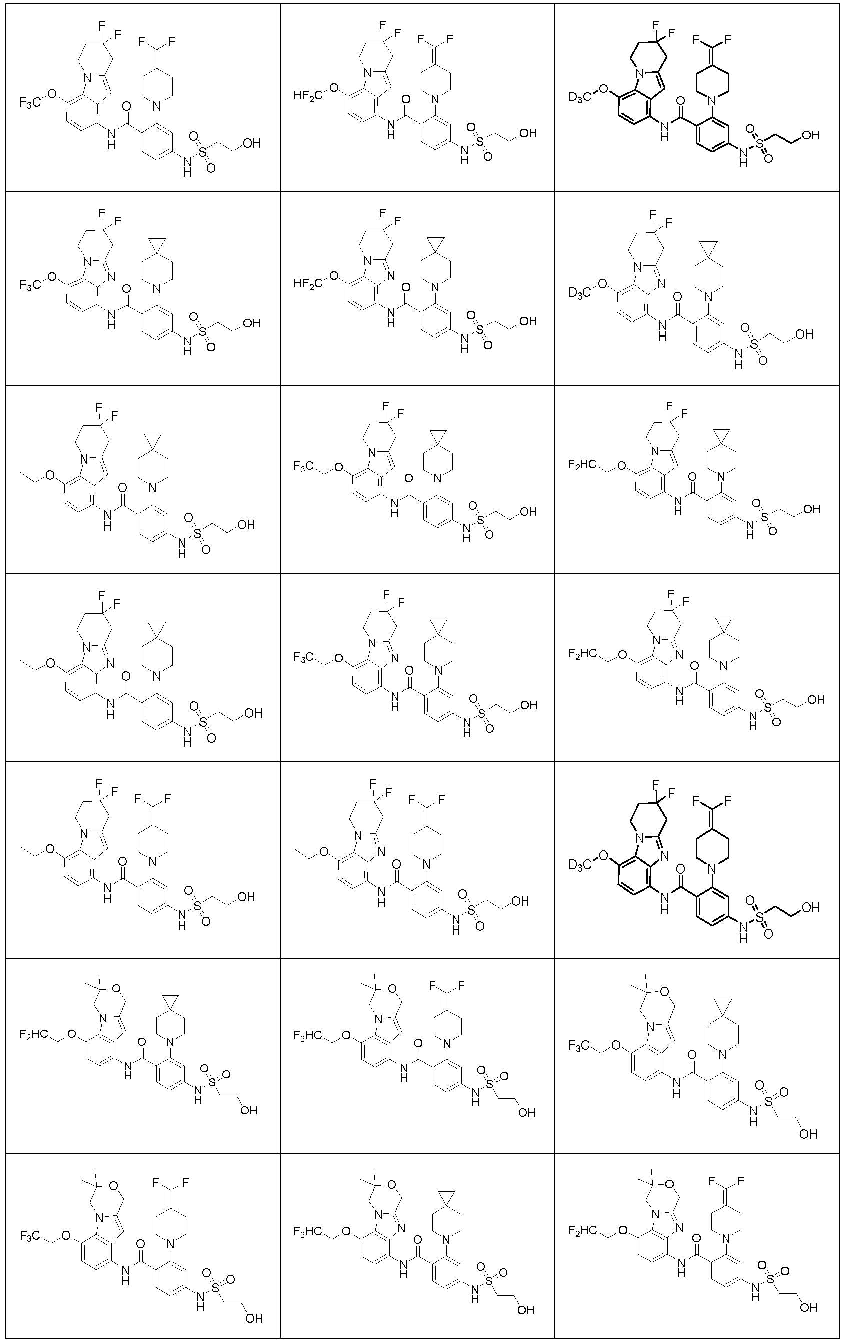

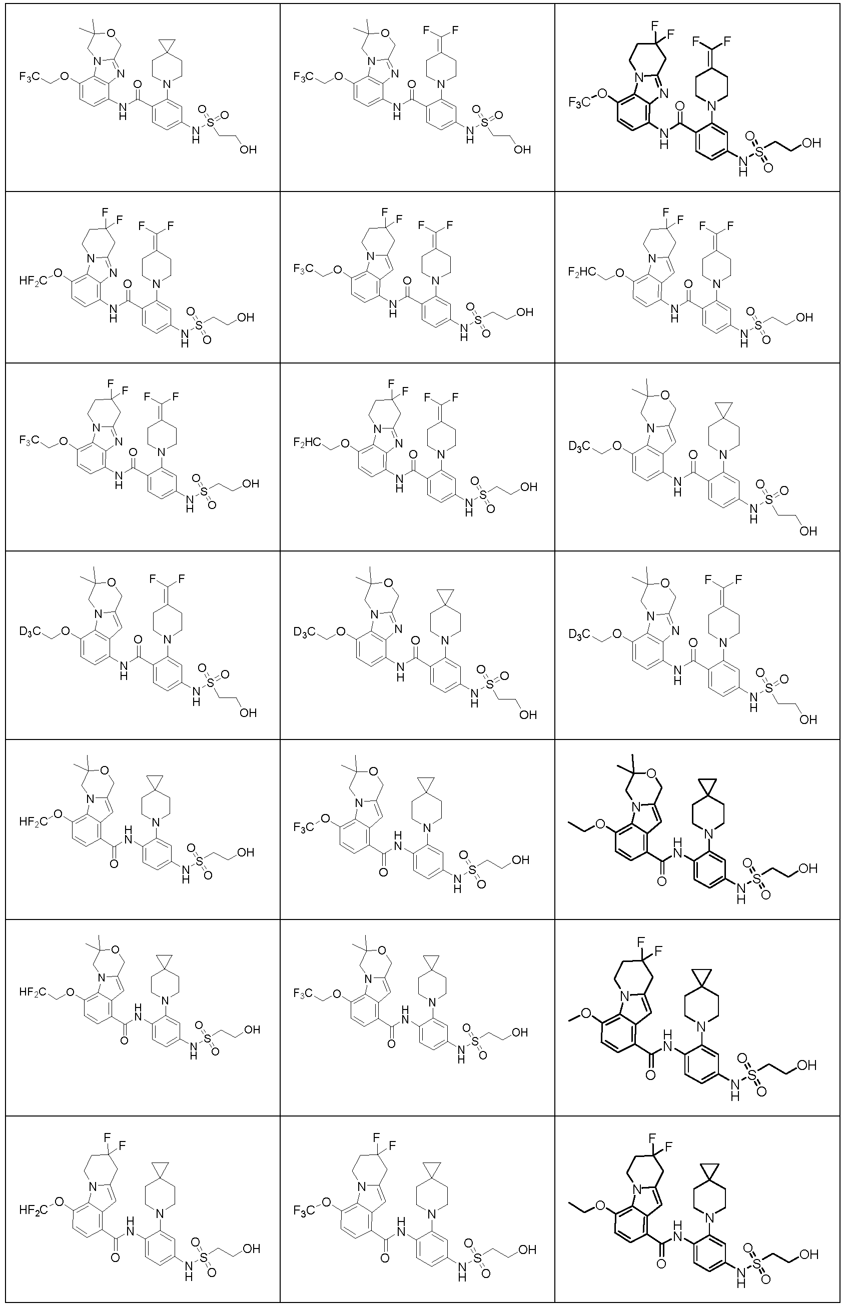

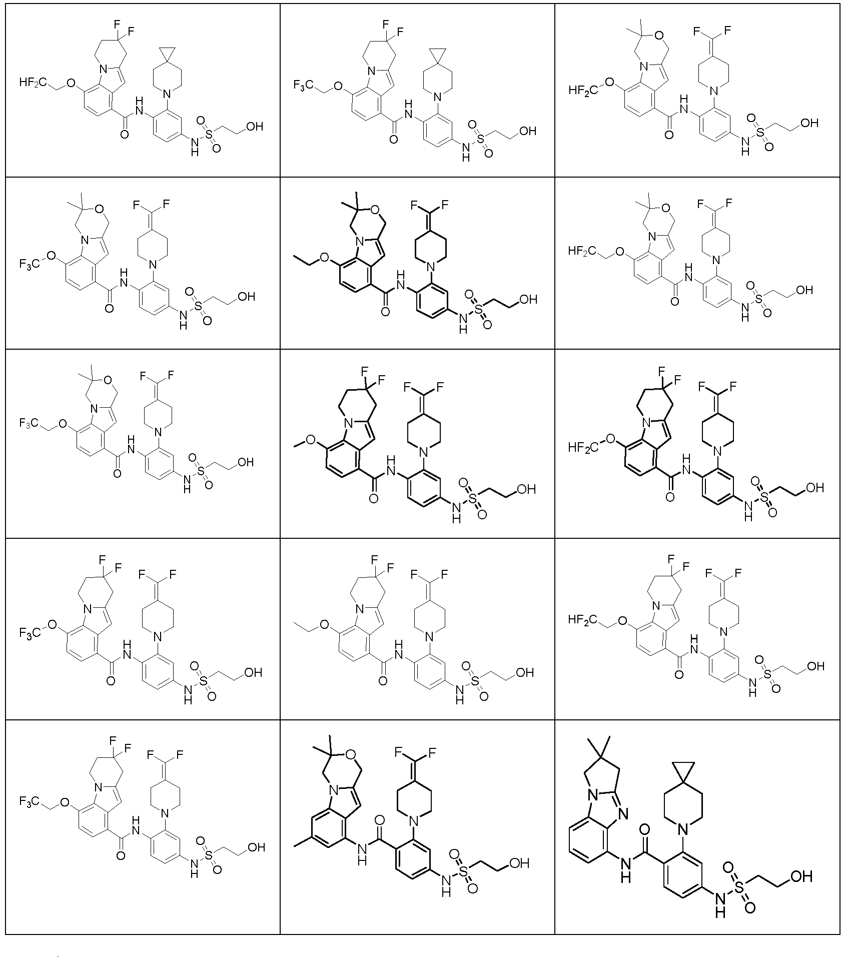

157. The compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-155, **characterized in that** the compound is selected from the group consisting of:

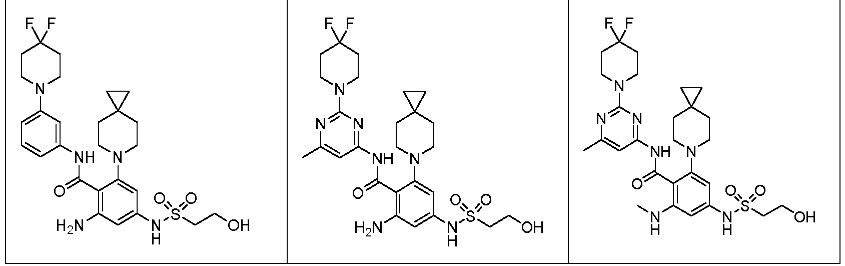

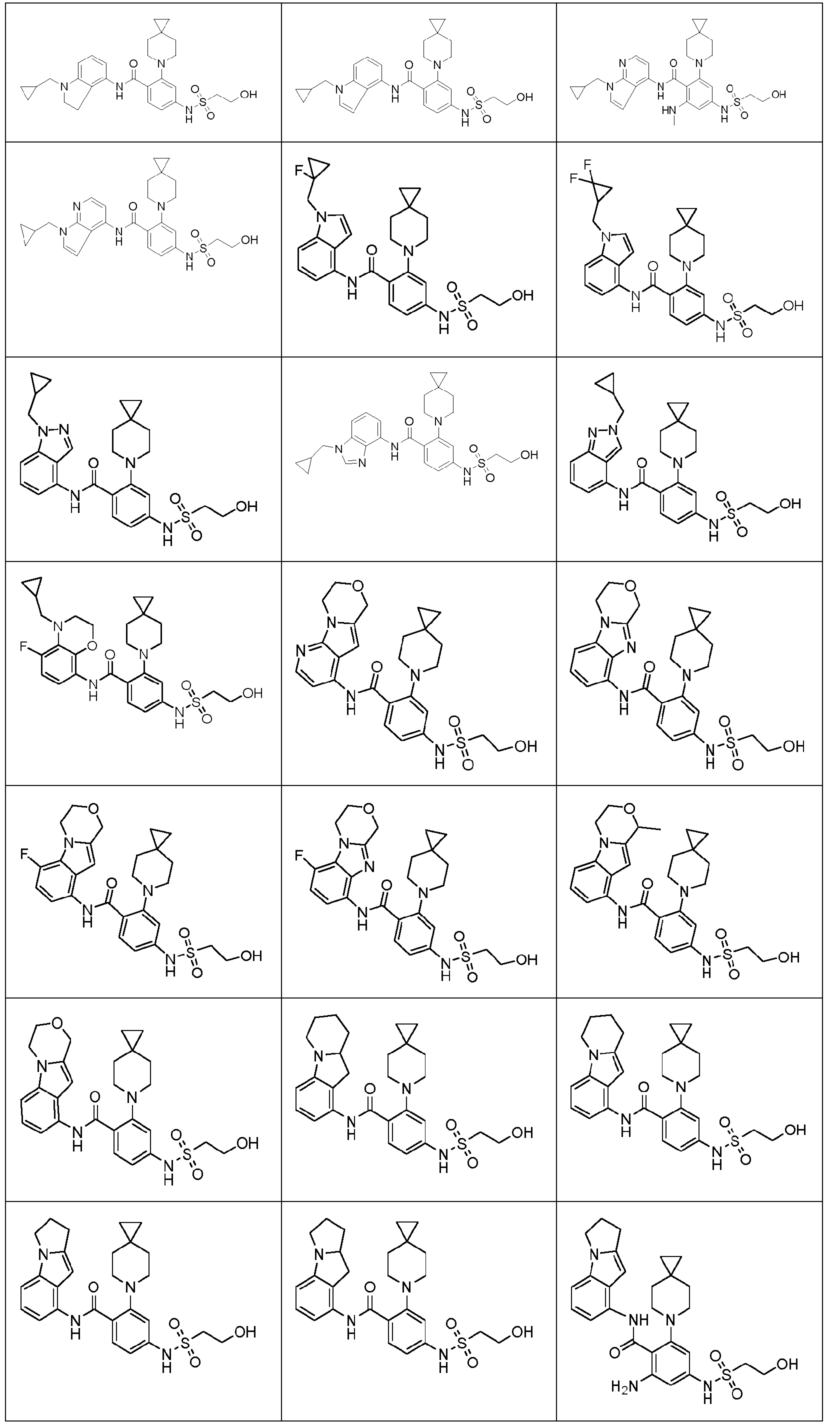

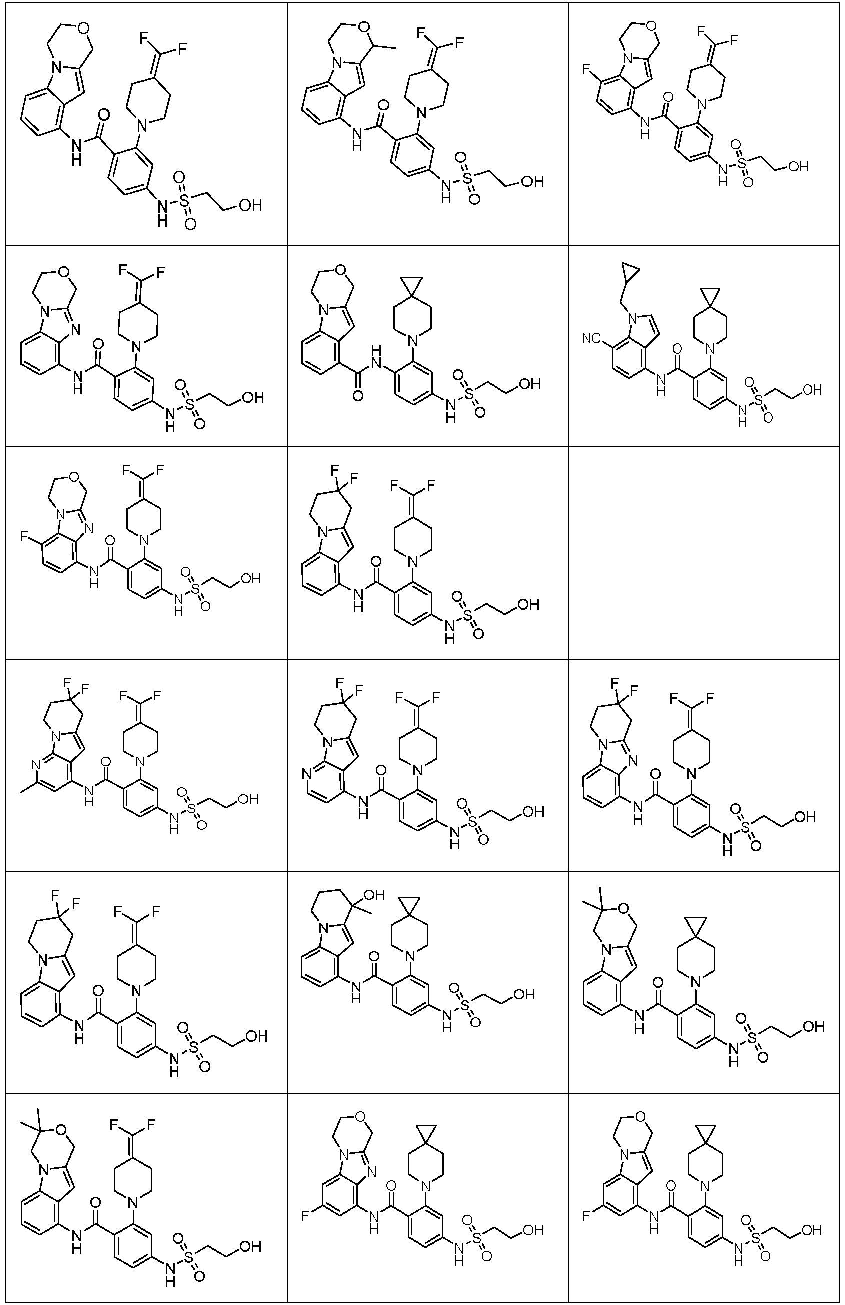

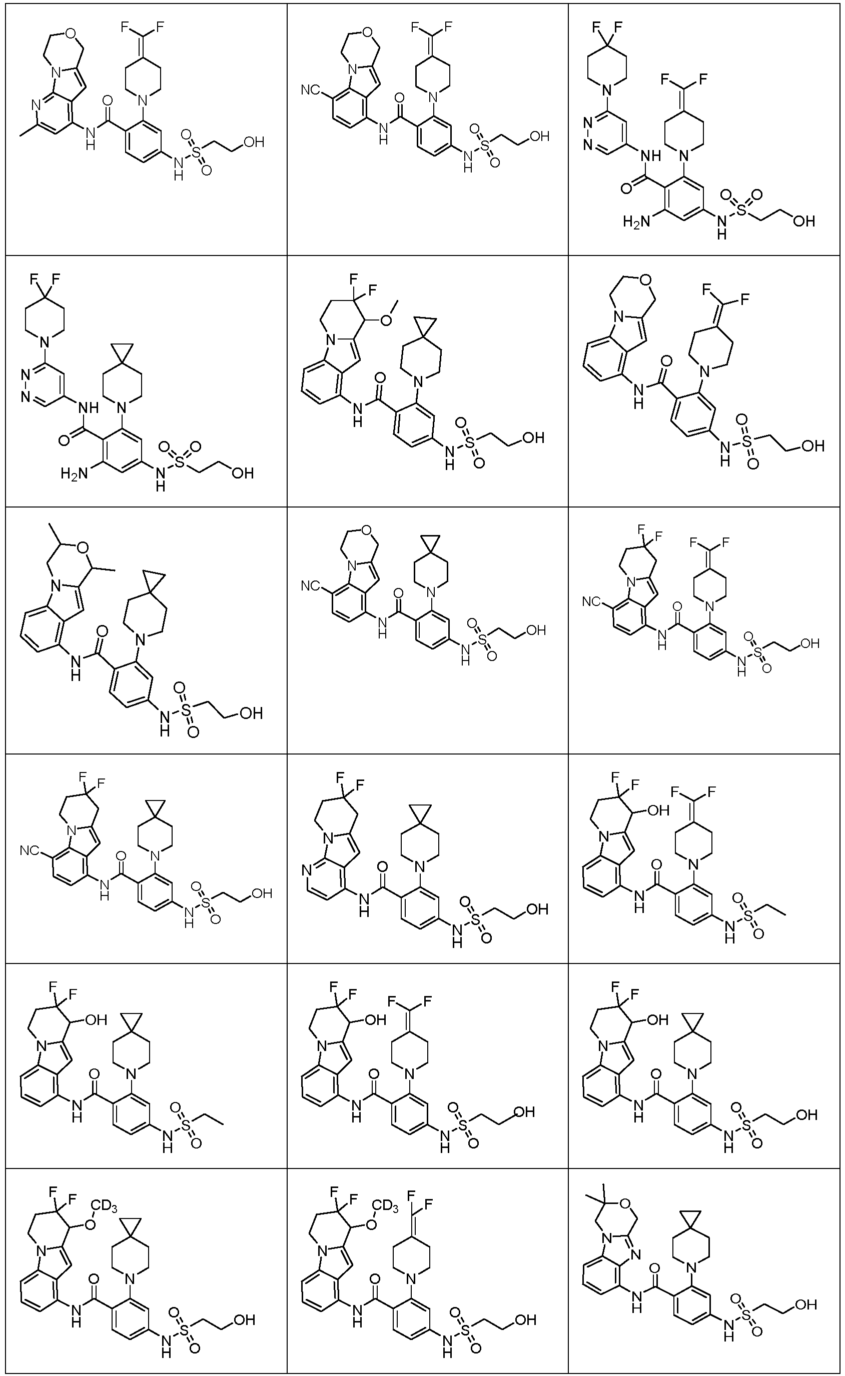

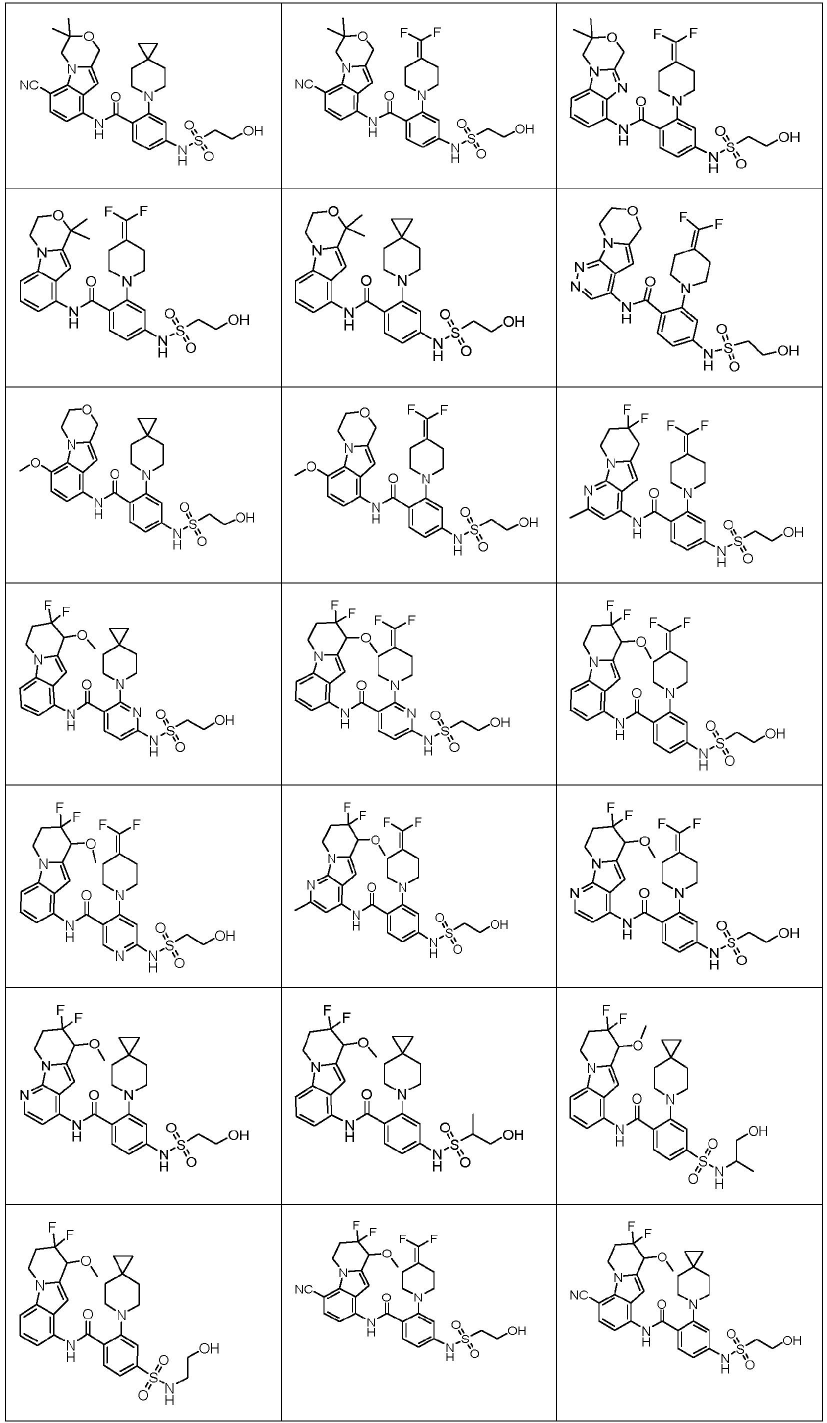

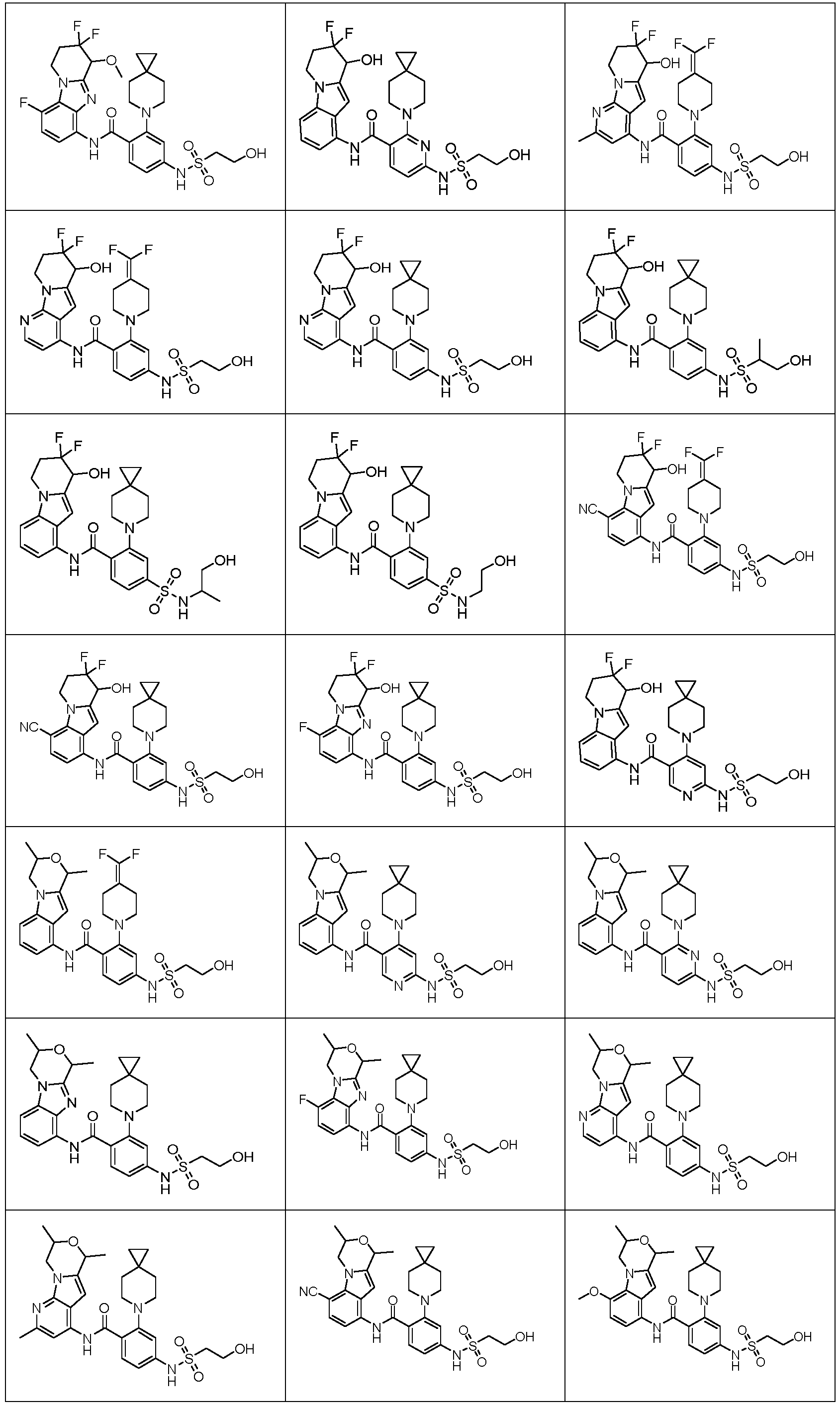

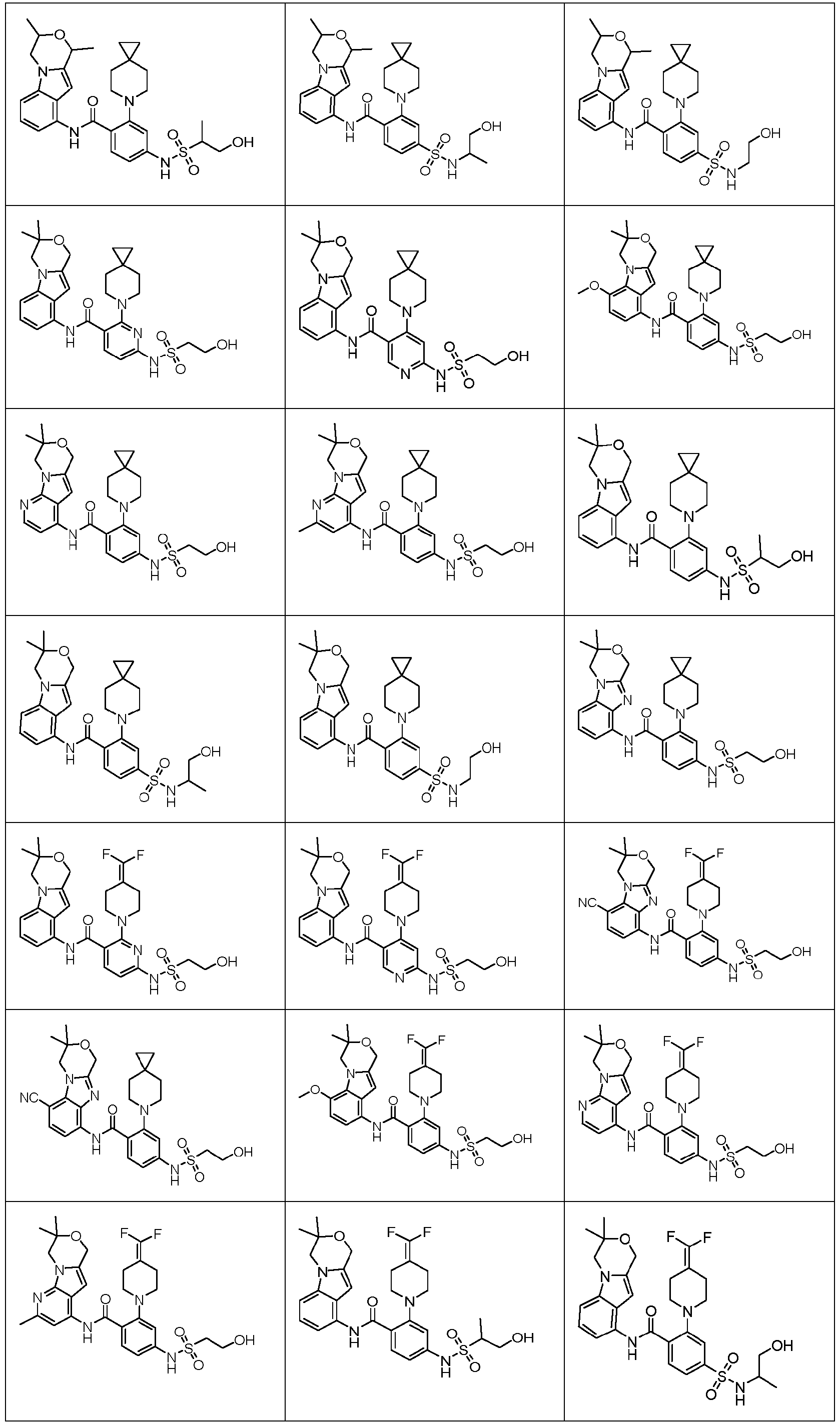

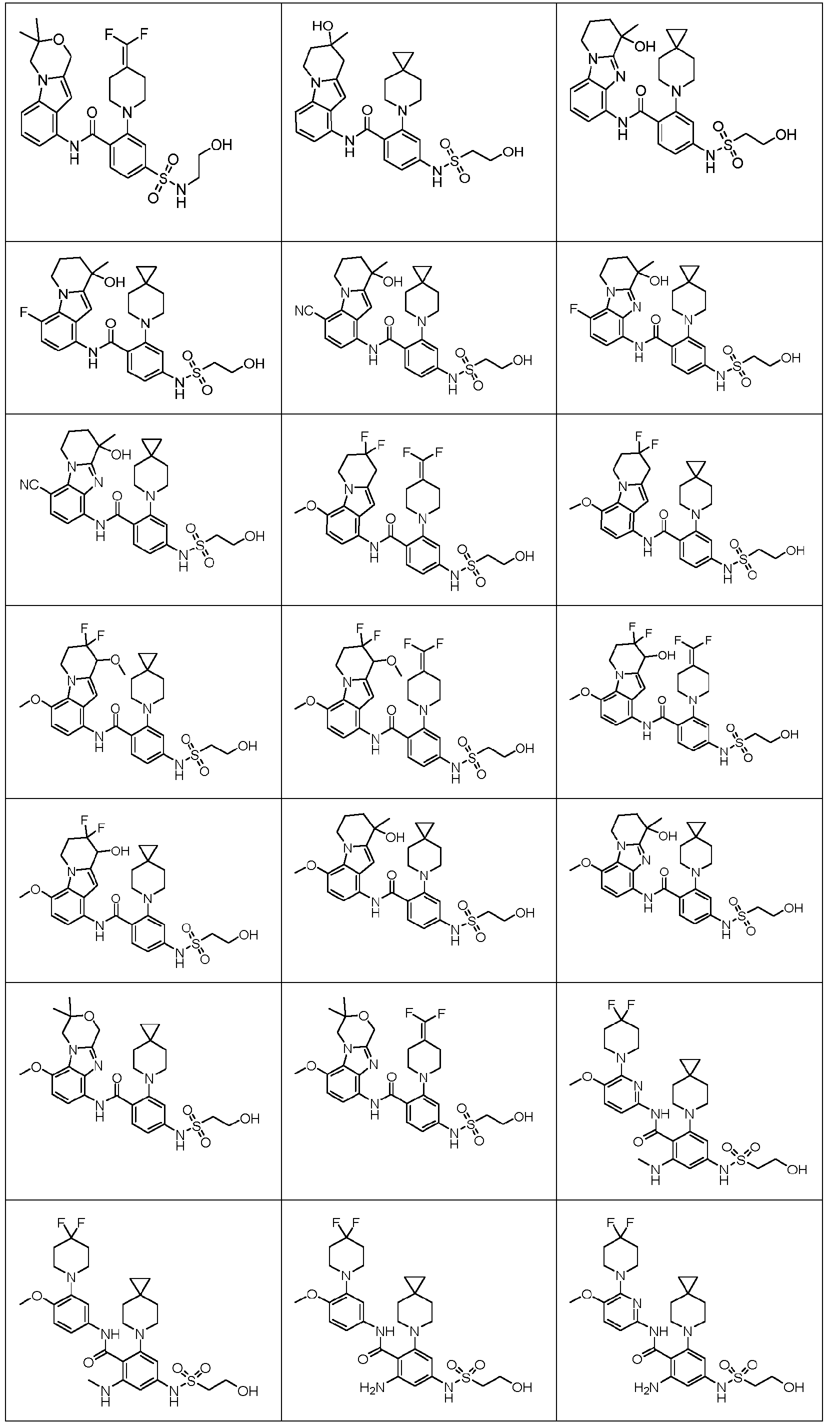

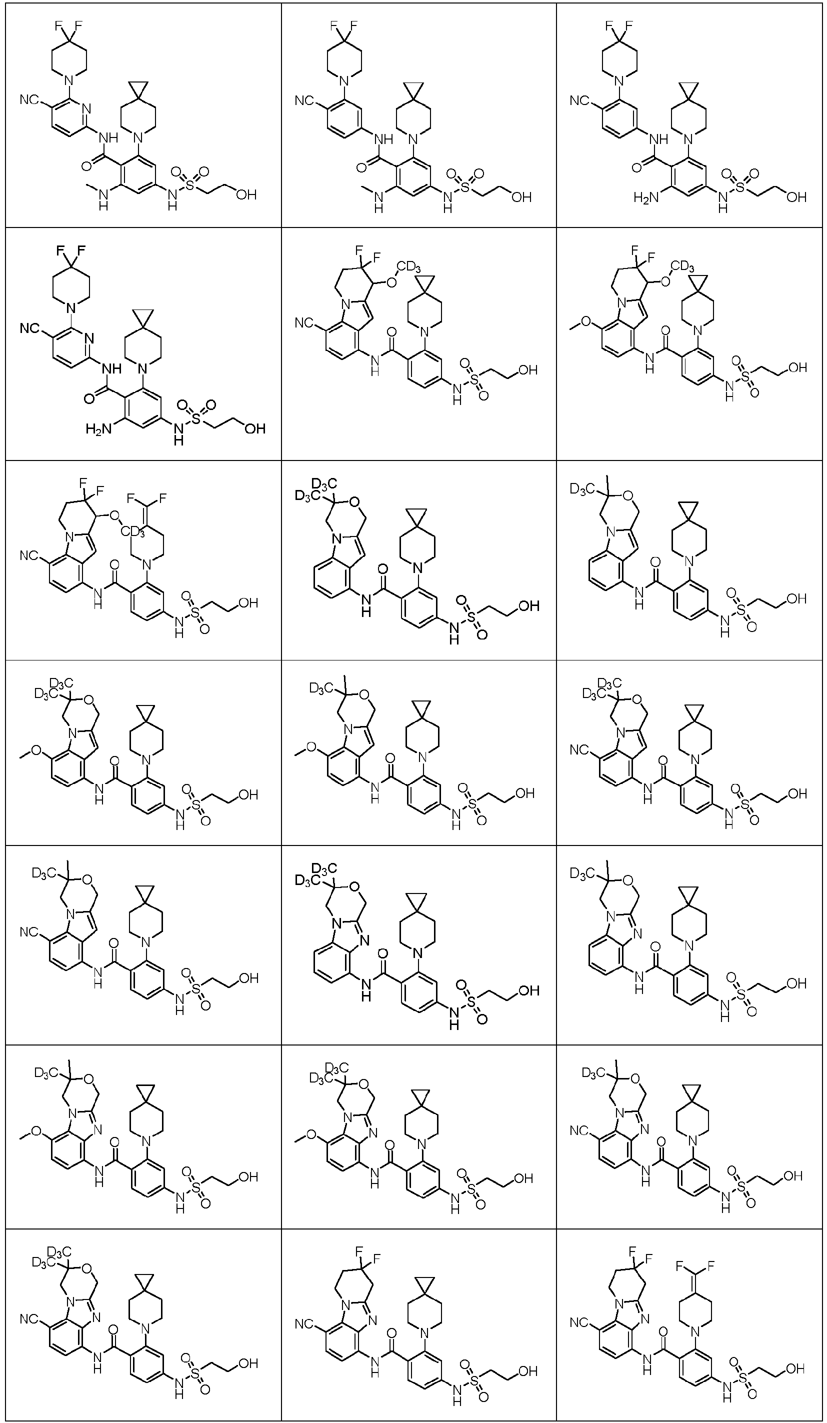

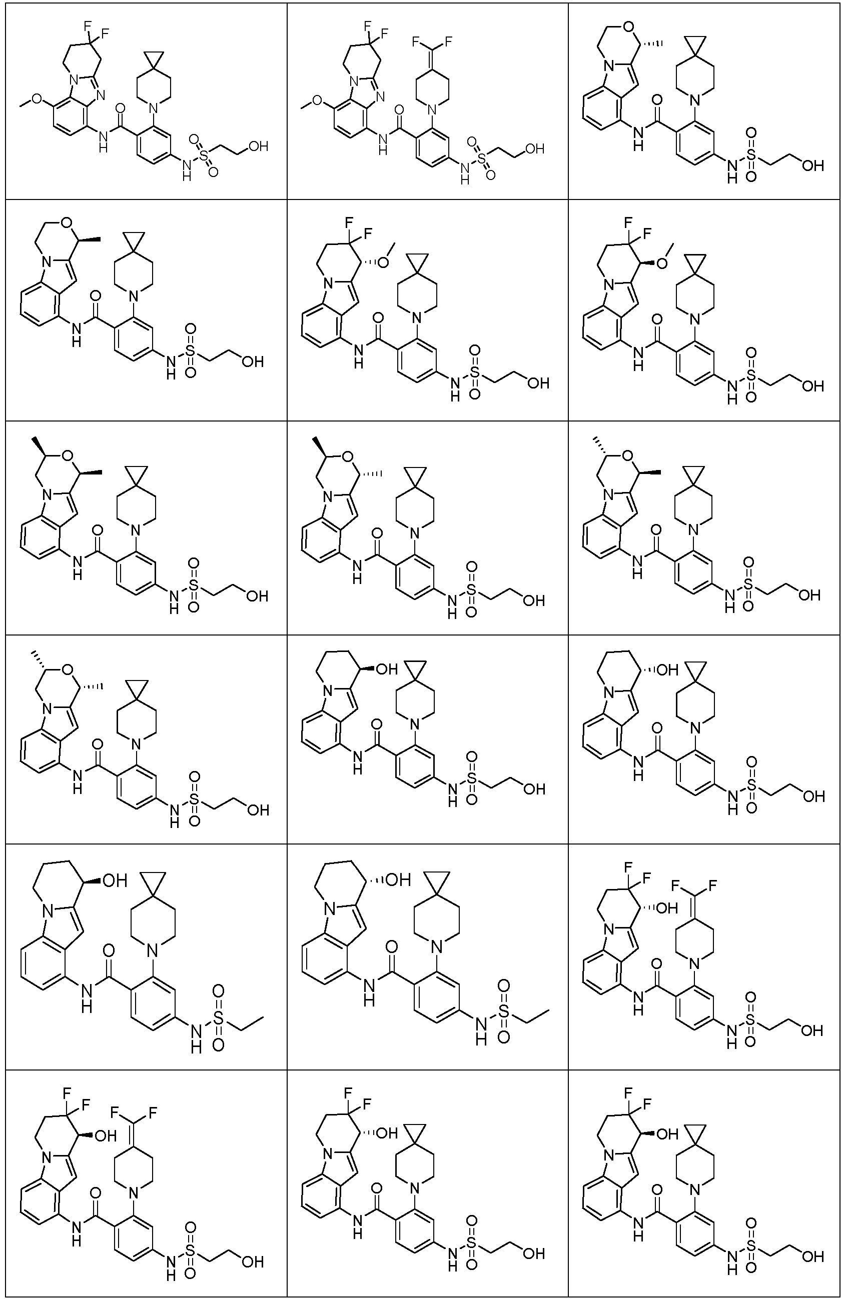

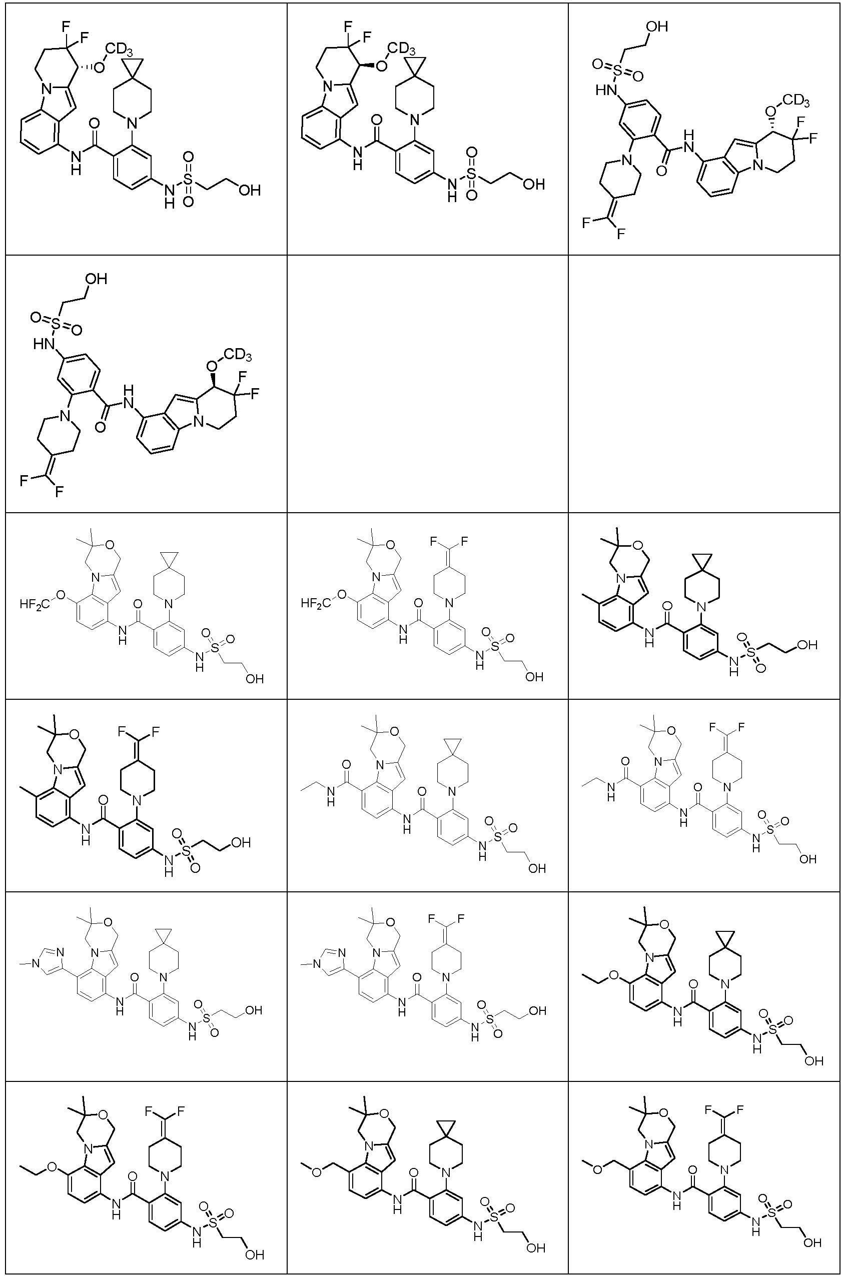

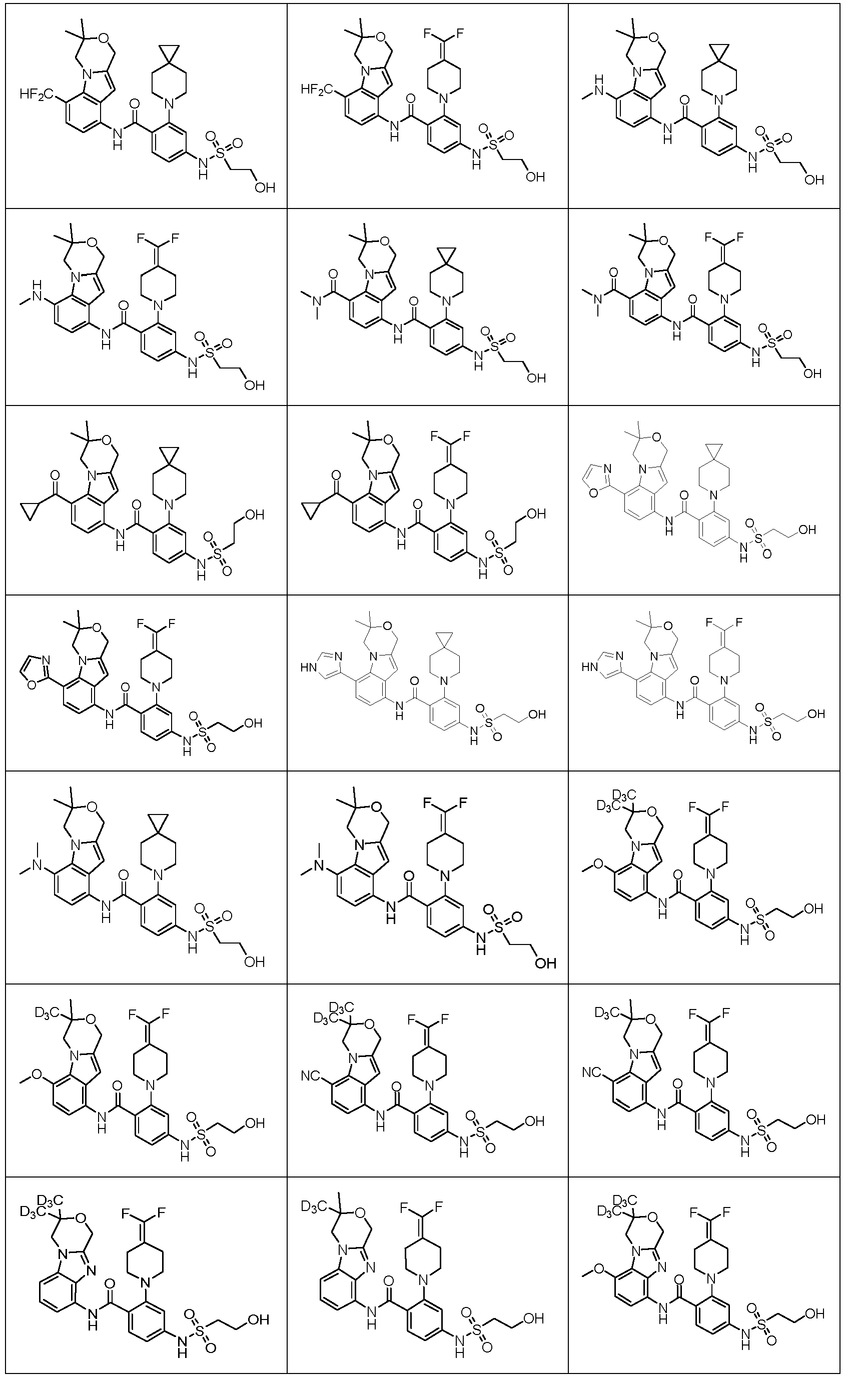

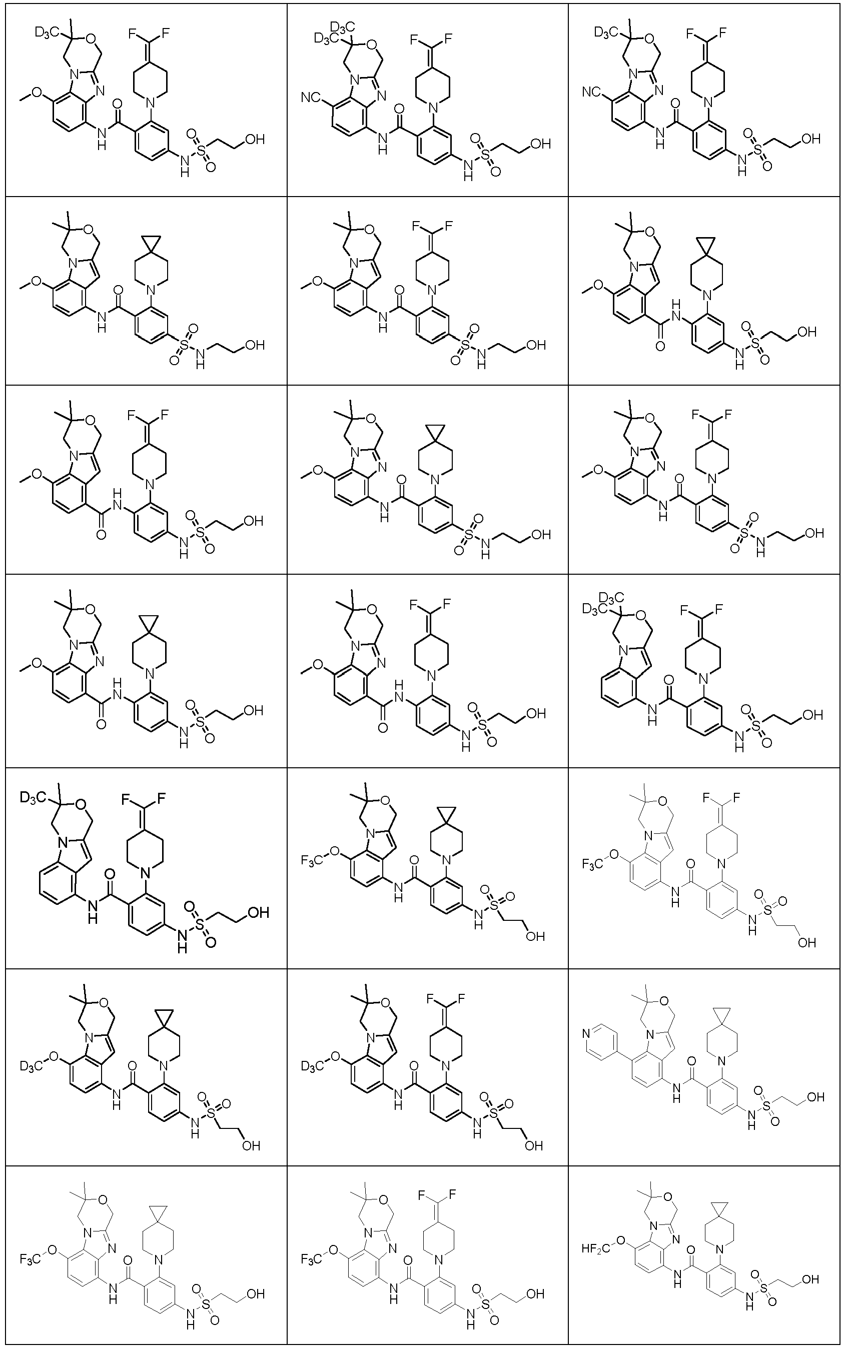

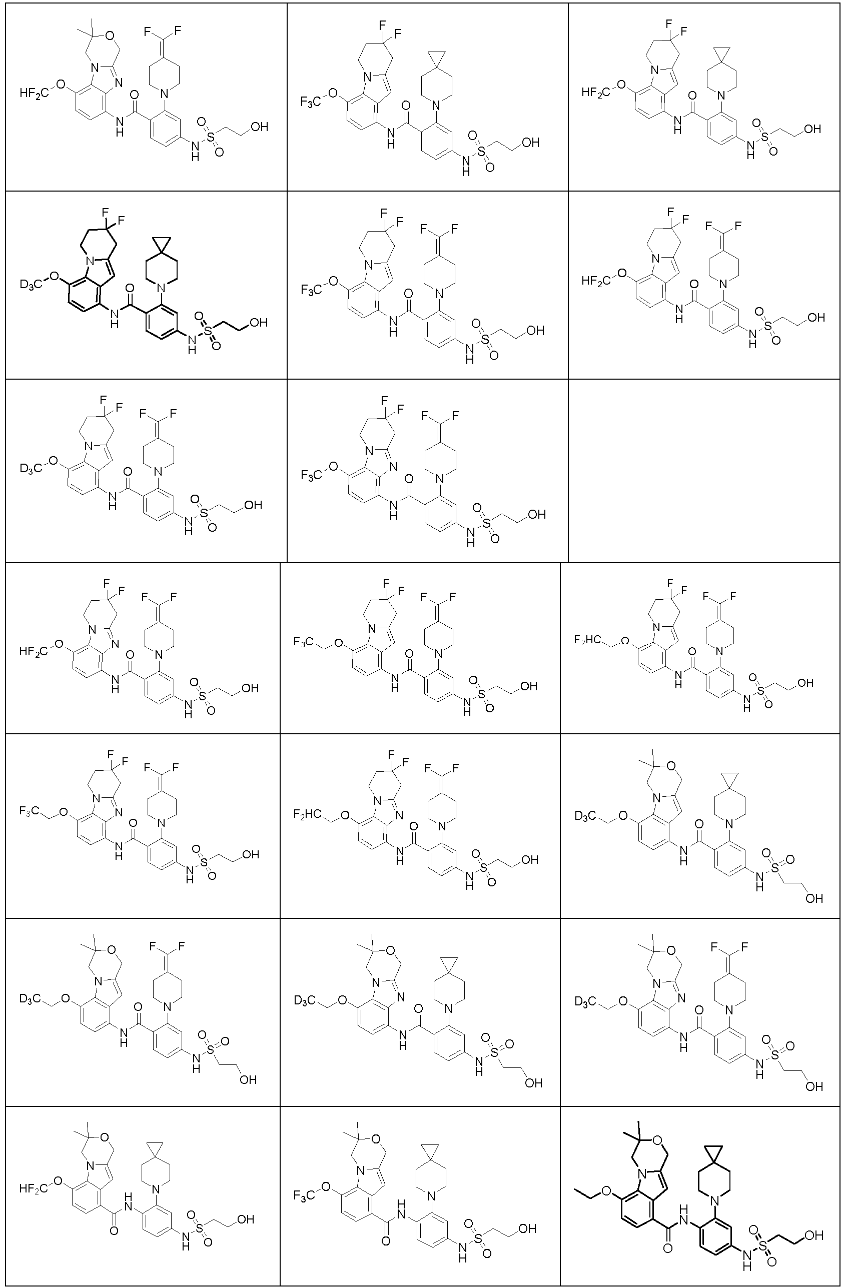

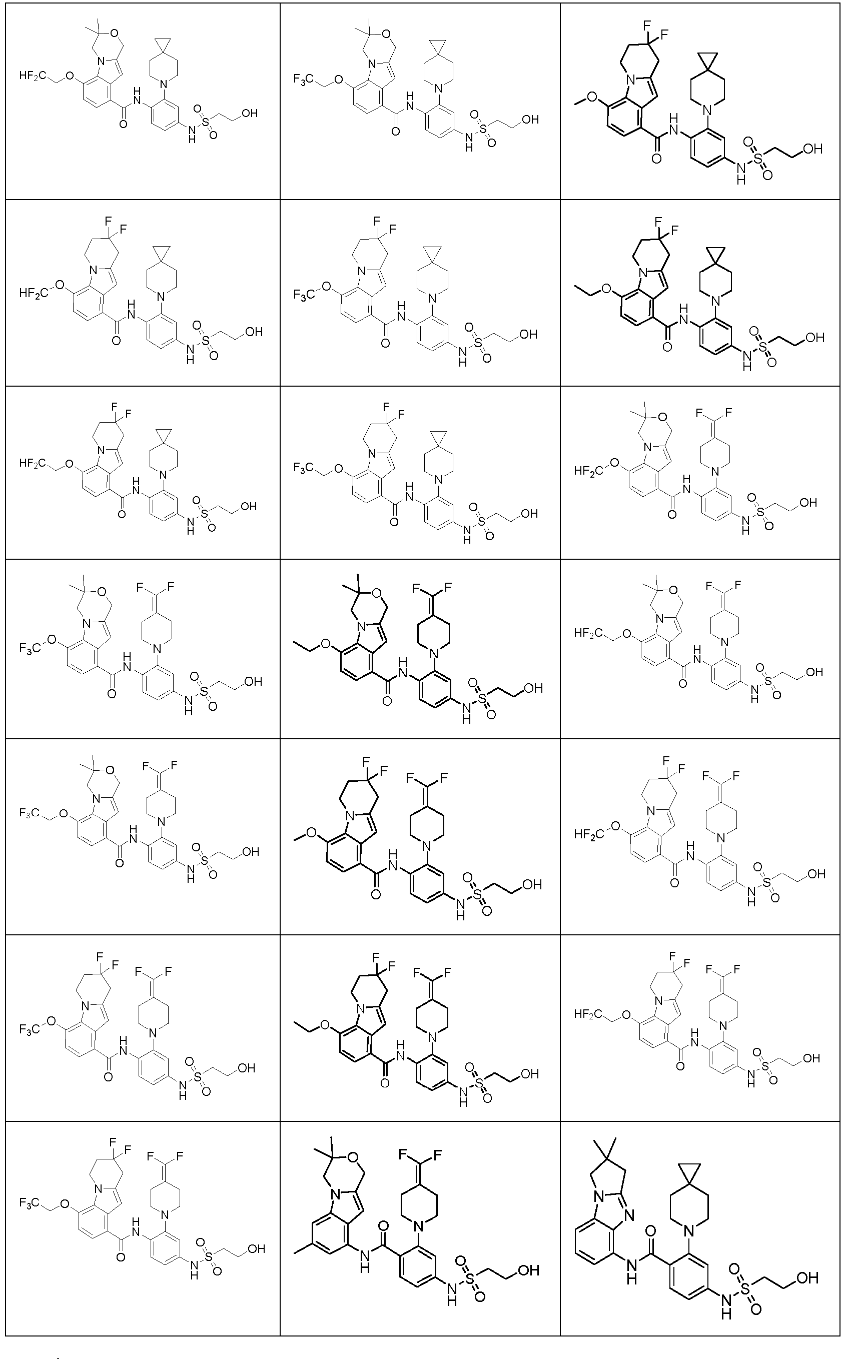

158. A pharmaceutical composition, **characterized by** comprising the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, and a pharmaceutically acceptable diluent or carrier.

159. A method for treating a condition treatable with a KIF18A inhibitor, **characterized in that** the method comprises administering to a patient in need thereof a therapeutically effective amount of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158.

160. The method according to claim 159, **characterized in that** the condition is a cancer selected from the group consisting of: (a) a solid tumor or a blood-derived tumor selected from the following cancers: bladder cancer, endometrial cancer, squamous cell carcinoma of the lung, breast cancer, colon cancer, kidney cancer, liver cancer, lung cancer, small cell lung cancer, esophageal cancer, gallbladder cancer, brain cancer, head and neck cancer, ovarian cancer, pancreatic cancer, gastric cancer, cervical cancer, thyroid cancer, prostate cancer and skin cancer; (b) a hematopoietic tumor of lymphatic system selected from: leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkitt's lymphoma; (c) a hematopoietic tumor of bone marrow system selected from: acute and chronic myeloid leukemia, myelodysplastic syndrome and promyelocytic leukemia; (d) a tumor of interstitial origin selected from fibrosarcoma and rhabdomyosarcoma; (e) a tumor of the central and peripheral nervous system selected from astrocytoma, neuroblastoma, glioma and schwannoma; or (f) melanoma, seminoma, teratocarcinoma, osteosarcoma, xeroderma pigmentosum, keratoacanthoma, thyroid follicular carcinoma, or Kaposi's sarcoma.

161. A method for reducing the size of a solid tumor in a subject, **characterized in that** the method comprises administering to a subject in need thereof a therapeutically effective amount of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158.

162. A method for treating a cell proliferation disorder in a subject, **characterized in that** the method comprises administering to a subject in need thereof a therapeutically effective amount of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158.

163. A method for inhibiting KIF18A in a cell, **characterized in that** the method comprises contacting the cell with the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158.

164. Use of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158 in the preparation of a medicament for treating a condition treatable with a KIF18A inhibitor.

165. The use according to claim 164, **characterized in that** the condition is a cancer selected from the group consisting of: (a) a solid tumor or a blood-derived tumor selected from the following cancers: bladder cancer, endometrial cancer, squamous cell carcinoma of the lung, breast cancer, colon cancer, kidney cancer, liver cancer, lung cancer, small cell lung cancer, esophageal cancer, gallbladder cancer, brain cancer, head and neck cancer, ovarian cancer, pancreatic cancer, gastric cancer, cervical cancer, thyroid cancer, prostate cancer and skin cancer; (b) a hematopoietic tumor of lymphatic system selected from: leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, and Burkitt's lymphoma; (c) a hematopoietic tumor of bone marrow system selected from: acute and chronic myeloid leukemia, myelodysplastic syndrome and promyelocytic leukemia; (d) a tumor of interstitial origin selected from fibrosarcoma and rhabdomyosarcoma; (e) a tumor of the central and peripheral nervous system selected from astrocytoma, neuroblastoma, glioma and schwannoma; or (f) melanoma, seminoma, teratocarcinoma, osteosarcoma, xeroderma pigmentosum, keratoacanthoma, thyroid follicular carcinoma, or Kaposi's sarcoma.

166. Use of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158 in the preparation of a medicament for reducing the size of a solid tumor in a subject.

167. Use of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158 in the preparation of a medicament for treating a cell proliferation disorder in a subject.

168. Use of the compound, or the stereoisomer, tautomer, diastereomer, racemate, cis-trans isomer, isotopically labeled compound, nitrogen oxide, solvate, hydrate, crystal form, ester, metabolite, pharmaceutically acceptable salt or prodrug thereof according to any one of claims 1-157, or the composition according to claim 158 in the preparation of a medicament for inhibiting KIF18A in a cell.
